# EUROPEAN PATENT APPLICATION

(11) **EP 1 067 182 A2**
(43) Date of publication of application: **10.01.2001**
(21) Application number: 00114090.4
(22) Date of filing: 07.07.2000
(51) Int. Cl.: C12N 15/12, C12N 15/10, C12N 15/85, C12N 5/10, C07K 14/47, C07K 16/18, C12Q 1/68

(54) **Secretory protein or membrane protein**

(30) Priority: 08.07.1999 JP 19417999; 11.01.2000 JP 2000118775; 02.05.2000 JP 2000183766
(71) Applicant: Helix Research Institute, Kisarazu-shi, Chiba 292-0812 (JP)
(72) Inventor: Ota, Toshio, Fujisawa-shi, Kanagawa 251-0042 (JP); Isogai, Takao, Inashiki-gun, Ibaraki 300-0303 (JP); Nishikawa, Tetsuo, Tokyo 173-0013 (JP); Kawai, Yuri, Kisarazu-shi, Chiba 292-0812 (JP); Sugiyama, Tomoyasu, Kisarazu-shi, Chiba 292-0045 (JP); Hayashi, Koji, Ichihara-shi, Chiba 299-0125 (JP)
(74) Representative: VOSSIUS & PARTNER

(57) **Abstract**

Novel human secretory proteins or membrane proteins, and full length cDNAs encoding the proteins are provided.

173 kinds of novel proteins and polynucleotides encoding these proteins have been isolated. The proteins of the present invention are useful as candidates for medicines or as target molecules for developing medicines. The polynucleotides of the present invention are used to produce these proteins.

## Description

### FIELD OF THE INVENTION

The present invention relates to a polynucleotide encoding a novel protein, a protein encoded by the polynucleotide, and novel usages of these.

### BACKGROUND OF THE INVENTION

Currently, sequencing projects, the determination and analysis of the genomic DNA of various living organisms are in progress all over the world. The whole genomic sequences of more than 10 species of prokaryotes, a lower eukaryote, yeast, and a multicellular eukaryote, C. elegans have been already determined. As to the human genome, which is supposed to be composed of three thousand million base pairs, world wide cooperative projects are under way to analyze it, and the whole structure is predicted to be determined by the years 2002-2003. The aim of the determination of genomic sequence is to reveal the functions of all genes and their regulation and to understand living organisms as a network of interactions between genes, proteins, cells or individuals through deducing the information in a genome, which is viewed as a blueprint of the highly complicated living organisms. To understand living organisms by utilizing the genomic information from various species is not only important as an academic subject, but also socially significant from the viewpoint of industrial application. However, determination of genomic sequences itself cannot identify the functions of all genes. For example, for yeast, the function of only approximately half of the 6000 genes, which is predicted based on the genomic sequence, has been deduced. As for humans, the number of genes is predicted to be approximately one hundred thousand. Therefore, it is desirable to establish "a high throughput analysis system of gene functions" which allows us to identify rapidly and efficiently the functions of vast amounts of the genes obtained by the genomic sequencing.

Many genes in the eukaryotic genome are split by introns into multiple exons. Thus, it is difficult to predict correctly the structure of encoded proteins solely based on genomic information. In contrast, cDNA, which is produced from mRNA that lacks introns, encodes a protein as a single continuous amino acid sequence and allows us to identify the primary structure of the protein easily. In human cDNA research, to date, more than one million ESTs (Expression Sequence Tags) are available from public domains (public databases), and the ESTs presumably cover not less than 80% of all human genes.

The information of ESTs is utilized for analyzing the structure of human genome, or for predicting the exon-regions of genomic sequences or their expression profile. However, many human ESTs have been derived from proximal regions to the 3'-end of cDNA, and information around the 5'-end of mRNA is extremely little. Among these human cDNAs, the number of the corresponding mRNAs whose encoding protein sequences are deduced is approximately 7000, and further, the number of full-length clones is only 5500. Thus, even including cDNA registered as EST, the percentage of human cDNA obtained so far is estimated to be 10-15% of all the genes.

It is possible to identify the transcription start site of mRNA on the genomic sequence based on the 5'-end sequence of a full-length cDNA, and to analyze factors involved in the stability of mRNA that is contained in the cDNA, or in its regulation of expression at the translation stage. Also, since a full-length cDNA contains ATG, the translation start site, in the 5'-region, it can be translated into a protein in a correct frame. Therefore, it is possible to produce a large amount of the protein encoded by the cDNA or to analyze biological activity of the expressed protein by utilizing an appropriate expression system. Thus, analysis of a full-length cDNA provides valuable information that complements the information from genome sequencing. Also, full-length cDNA clones that can be expressed are extremely valuable in empirical analysis of gene function and in industrial application.

In particular, human secretory proteins or membrane proteins would be useful by itself as a medicine like tissue plasminogen activator (TPA), or as a target of medicines like membrane receptors.

Therefore, it has great significance to isolate novel full-length cDNA clones of humans, of which only a few have been isolated. Especially, isolation of a novel cDNA clone encoding a secretory protein or membrane protein is desired since the protein itself, or a molecule that interacts with the membrane protein would be useful as a medicine, and also the clones potentially include a gene associated with diseases. Thus, identification of the full-length cDNA clones encoding those proteins has great significance.

### SUMMARY OF THE INVENTION

An objective of the present invention is to provide a polynucleotide encoding a novel protein, a protein encoded by said polynucleotide, and novel usages of these.

The inventors have developed a method for efficiently cloning a human full-length cDNA that is predicted by the ATGpr etc. to be a full-length cDNA clone, from a full-length-enriched cDNA library that is synthesized by the oligo-capping method [K. Maruyama and S. Sugano, Gene, 138: 171-174 (1994); Y. Suzuki et al., Gene, 200: 149-156 (1997)]. Then, the inventors determined the nucleotide sequence of the obtained cDNA clones from both 5'- and 3'- ends. By utilizing the sequences, the inventors selected clones that were expected to contain a signal by the PSORT (Nakai K. and Kanehisa M. (1992) Genomics 14: 897-911), and obtained clones that contain a cDNA encoding a secretory protein or membrane protein. The inventors found that it is possible to synthesize a novel full-length cDNA by using the combination of a primer that is designed based on the nucleotide sequence of the 5'-ends of the selected full-length cDNA clones and any of an oligo-dT primer or a 3'-primer that is designed based on the nucleotide sequence of the 3'-ends of the selected clones.

The full-length cDNA clones of the present invention have high fullness ratio since these were obtained by the combination of (1) construction of a full-length-enriched cDNA library that is synthesized by the oligo-capping method, and (2) a system in which fullness ratio is evaluated from the nucleotide sequence of the 5'-end.

Furthermore, the inventors have analyzed the nucleotide sequence of the full-length cDNA clones obtained by the method, and deduced the amino acid sequence encoded by the nucleotide sequence. Then, the inventors have performed the BLAST search (Altschul S.F., Gish W., Miller W., Myers E.W., and Lipman D.J. (1990) J. Mol. Biol. 215: 403-410; Gish W., and States D.J. (1993) Nature Genet. 3: 266-272; http://www.ncbi.nlm.nih.gov/BLAST/) of the GenBank (http://www.ncbi.nlm.nih.gov/Web/GenBank/index.html) and SwissProt (http://www.ebi.ac.uk/ebi_docs/swissprot_db/swisshome.html) using the deduced amino acid sequence to accomplish the present invention.

Homology analysis in which the analysis is carried out against a non-full-length cDNA fragment to postulate the function of a protein encoded by said fragment, is being commonly performed. However, since such analysis is based on the information of the fragment, it is not clear as to whether this fragment corresponds to a part that is functionally important in the protein. In other words, the reliability of the homology analysis based on the information of a fragment is doubtful, as information relating to the structure of the whole protein is not available. However, the homology analysis of the present invention is conducted based on the information of a full-length cDNA comprising the whole coding region of the cDNA, and therefore, the homology of various portions of the protein can be analyzed. Hence, the reliability of the homology analysis has been dramatically improved in the present invention.

The present invention relates to the polynucleotide mentioned below, a protein encoded by the polynucleotide, and their usage.

First, the present invention relates to
(1) an isolated polynucleotide selected from the group consisting of
(a) a polynucleotide comprising a coding region of the nucleotide sequence set forth in any one of the SEQ ID NOs in Table 1;
(b) a polynucleotide comprising a nucleotide sequence encoding a protein comprising the amino acid sequence set forth in any one of the SEQ ID NOs in Table 1;
(c) a polynucleotide comprising a nucleotide sequence encoding a protein comprising an amino acid sequence selected from the amino acid sequences set forth in the SEQ ID NOs in Table 1, in which one or more amino acids are substituted, deleted, inserted, and/or added, wherein said protein is functionally equivalent to the protein comprising said amino acid sequence selected from the amino acid sequences set forth in the SEQ ID NOs in Table 1;
(d) a polynucleotide that hybridizes with a polynucleotide comprising a nucleotide sequence selected from the nucleotide sequences set forth in the SEQ ID NOs in Table 1, and that comprises a nucleotide sequence encoding a protein functionally equivalent to the protein encoded by the nucleotide sequence selected from the nucleotide sequences set forth in the SEQ ID NOs in Table 1;
(e) a polynucleotide comprising a nucleotide sequence encoding a partial amino acid sequence of a protein encoded by the polynucleotide of (a) to (d);
(f) a polynucleotide comprising a nucleotide sequence with at least 70% identity to the nucleotide sequence set forth in any one of the SEQ ID NOs in Table 1.

Table 1 shows the name of the cDNA clones isolated in the examples described later, comprising the full-length cDNA of the present invention, the corresponding SEQ ID NOs. of the nucleotide sequences of the cDNA clones, and the corresponding SEQ ID NOs. of the amino acid sequences deduced from the cDNA nucleotide sequences.

**Table 1**

| Amino acid sequence | Nucleotide sequence | Clone Name |
|---|---|---|
| SEQ ID NO: 2 | SEQ ID NO: 1 | PSEC0001 |
| SEQ ID NO: 4 | SEQ ID NO: 3 | nnnnnnnn |
| SEQ ID NO: 6 | SEQ ID NO: 5 | PSEC0005 |
| SEQ ID NO: 8 | SEQ ID NO: 7 | PSEC0007 |
| SEQ ID NO: 10 | SEQ ID NO: 9 | PSEC0008 |
| SEQ ID NO: 12 | SEQ ID NO: 11 | PSEC0012 |
| SEQ ID NO: 14 | SEQ ID NO: 13 | PSEC0017 |
| SEQ ID NO: 16 | SEQ ID NO: 15 | PSEC0019 |
| SEQ ID NO: 18 | SEQ ID NO: 17 | PSEC0020 |
| SEQ ID NO: 20 | SEQ ID NO: 19 | PSEC0021 |
| SEQ ID NO: 22 | SEQ ID NO: 21 | PSEC0028 |
| SEQ ID NO: 24 | SEQ ID NO: 23 | PSEC0029 |
| SEQ ID NO: 26 | SEQ ID NO: 25 | PSEC0030 |
| SEQ ID NO: 28 | SEQ ID NO: 27 | PSEC0031 |
| SEQ ID NO: 30 | SEQ ID NO: 29 | PSEC0035 |
| SEQ ID NO: 32 | SEQ ID NO: 31 | PSEC0038 |
| SEQ ID NO: 34 | SEQ ID NO: 33 | PSEC0040 |
| SEQ ID NO: 36 | SEQ ID NO: 35 | PSEC0041 |
| SEQ ID NO: 38 | SEQ ID NO: 37 | PSEC0045 |
| SEQ ID NO: 40 | SEQ ID NO: 39 | PSEC0048 |
| SEQ ID NO: 42 | SEQ ID NO: 41 | PSEC0049 |
| SEQ ID NO: 44 | SEQ ID NO: 43 | PSEC0051 |
| SEQ ID NO: 46 | SEQ ID NO: 45 | PSEC0052 |
| SEQ ID NO: 48 | SEQ ID NO: 47 | PSEC0053 |
| SEQ ID NO: 50 | SEQ ID NO: 49 | PSEC0055 |
| SEQ ID NO: 52 | SEQ ID NO: 51 | PSEC0059 |
| SEQ ID NO: 54 | SEQ ID NO: 53 | PSEC0061 |
| SEQ ID NO: 56 | SEQ ID NO: 55 | PSEC0068 |
| SEQ ID NO: 58 | SEQ ID NO: 57 | PSEC0070 |
| SEQ ID NO: 60 | SEQ ID NO: 59 | PSEC0071 |
| SEQ ID NO: 62 | SEQ ID NO: 61 | PSEC0072 |
| SEQ ID NO: 64 | SEQ ID NO: 63 | PSEC0073 |
| SEQ ID NO: 66 | SEQ ID NO: 65 | PSEC0074 |
| SEQ ID NO: 68 | SEQ ID NO: 67 | PSEC0075 |
| SEQ ID NO: 70 | SEQ ID NO: 69 | PSEC0076 |
| SEQ ID NO: 72 | SEQ ID NO: 71 | PSEC0077 |
| SEQ ID NO: 74 | SEQ ID NO: 73 | PSEC0079 |
| SEQ ID NO: 76 | SEQ ID NO: 75 | PSEC0080 |
| SEQ ID NO: 78 | SEQ ID NO: 77 | PSEC0081 |
| SEQ ID NO: 80 | SEQ ID NO: 79 | PSEC0082 |
| SEQ ID NO: 82 | SEQ ID NO: 81 | PSEC0085 |
| SEQ ID NO: 84 | SEQ ID NO: 83 | PSEC0086 |
| SEQ ID NO: 86 | SEQ ID NO: 85 | PSEC0087 |
| SEQ ID NO: 88 | SEQ ID NO: 87 | PSEC0088 |
| SEQ ID NO: 90 | SEQ ID NO: 89 | PSEC0090 |
| SEQ ID NO: 92 | SEQ ID NO: 91 | PSEC0094 |
| SEQ ID NO: 94 | SEQ ID NO: 93 | PSEC0095 |
| SEQ ID NO: 96 | SEQ ID NO: 95 | PSEC0098 |
| SEQ ID NO: 98 | SEQ ID NO: 97 | PSEC0099 |
| SEQ ID NO: 100 | SEQ ID NO: 99 | PSEC0100 |
| SEQ ID NO: 102 | SEQ ID NO: 101 | PSEC0101 |
| SEQ ID NO: 104 | SEQ ID NO: 103 | PSEC0104 |
| SEQ ID NO: 106 | SEQ ID NO: 105 | PSEC0105 |
| SEQ ID NO: 108 | SEQ ID NO: 107 | PSEC0106 |
| SEQ ID NO: 110 | SEQ ID NO: 109 | PSEC0107 |
| SEQ ID NO: 112 | SEQ ID NO: 111 | PSEC0108 |
| SEQ ID NO: 114 | SEQ ID NO: 113 | PSEC0109 |
| SEQ ID NO: 116 | SEQ ID NO: 115 | PSEC0110 |
| SEQ ID NO: 118 | SEQ ID NO: 117 | PSEC0111 |
| SEQ ID NO: 120 | SEQ ID NO: 119 | PSEC0112 |
| SEQ ID NO: 122 | SEQ ID NO: 121 | PSEC0113 |
| SEQ ID NO: 124 | SEQ ID NO: 123 | PSEC0119 |
| SEQ ID NO: 126 | SEQ ID NO: 125 | PSEC0120 |
| SEQ ID NO: 128 | SEQ ID NO: 127 | PSEC0121 |
| SEQ ID NO: 130 | SEQ ID NO: 129 | PSEC0124 |
| SEQ ID NO: 132 | SEQ ID NO: 131 | PSEC0125 |
| SEQ ID NO: 134 | SEQ ID NO: 133 | PSEC0126 |
| SEQ ID NO: 136 | SEQ ID NO: 135 | PSEC0127 |
| SEQ ID NO: 138 | SEQ ID NO: 137 | PSEC0128 |
| SEQ ID NO: 140 | SEQ ID NO: 139 | PSEC0129 |
| SEQ ID NO: 142 | SEQ ID NO: 141 | PSEC0130 |
| SEQ ID NO: 144 | SEQ ID NO: 143 | PSEC0131 |
| SEQ ID NO: 146 | SEQ ID NO: 145 | PSEC0133 |
| SEQ ID NO: 148 | SEQ ID NO: 147 | PSEC0134 |
| SEQ ID NO: 150 | SEQ ID NO: 149 | PSEC0135 |
| SEQ ID NO: 152 | SEQ ID NO: 151 | PSEC0136 |
| SEQ ID NO: 154 | SEQ ID NO: 153 | PSEC0137 |
| SEQ ID NO: 156 | SEQ ID NO: 155 | PSEC0139 |
| SEQ ID NO: 158 | SEQ ID NO: 157 | PSEC0143 |
| SEQ ID NO: 160 | SEQ ID NO: 159 | PSEC0144 |
| | | |
| SEQ ID NO: 162 | SEQ ID NO: 161 | nnnnnnnn |
| SEQ ID NO: 164 | SEQ ID NO: 163 | PSEC0147 |
| SEQ ID NO: 166 | SEQ ID NO: 165 | PSEC0149 |
| SEQ ID NO: 168 | SEQ ID NO: 167 | PSEC0150 |
| SEQ ID NO: 170 | SEQ ID NO: 169 | PSEC0151 |
| SEQ ID NO: 172 | SEQ ID NO: 171 | PSEC0152 |
| SEQ ID NO: 174 | SEQ ID NO: 173 | PSEC0158 |
| SEQ ID NO: 176 | SEQ ID NO: 175 | PSEC0159 |
| SEQ ID NO: 178 | SEQ ID NO: 177 | PSEC0161 |
| SEQ ID NO: 180 | SEQ ID NO: 179 | PSEC0162 |
| SEQ ID NO: 182 | SEQ ID NO: 181 | PSEC0163 |
| SEQ ID NO: 184 | SEQ ID NO: 183 | PSEC0164 |
| SEQ ID NO: l16 | SEQ ID NO: 185 | PSEC0165 |
| SEQ ID NO: 188 | SEQ ID NO: 187 | PSEC0167 |
| SEQ ID NO: 190 | SEQ ID NO: 189 | PSEC0168 |
| SEQ ID NO: 192 | SEQ ID NO: 191 | PSEC0169 |
| SEQ ID NO: 194 | SEQ ID NO: 193 | PSEC0170 |
| SEQ ID NO: 196 | SEQ ID NO: 195 | PSEC0171 |
| SEQ ID NO: 198 | SEQ ID NO: 197 | PSEC0172 |
| SEQ ID NO: 200 | SEQ ID NO: 199 | PSEC0173 |
| SEQ ID NO: 202 | SEQ ID NO: 201 | PSEC0178 |
| SEQ ID NO: 204 | SEQ ID NO: 203 | PSEC0181 |
| SEQ ID NO: 206 | SEQ ID NO: 205 | PSEC0182 |
| SEQ ID NO: 208 | SEQ ID NO: 207 | PSEC0183 |
| SEQ ID NO: 210 | SEQ ID NO: 209 | PSEC0190 |
| SEQ ID NO: 212 | SEQ ID NO: 211 | PSEC0191 |
| SEQ ID NO: 214 | SEQ ID NO: 213 | PSEC0192 |
| SEQ ID NO: 216 | SEQ ID NO: 215 | PSEC0197 |
| SEQ ID NO: 218 | SEQ ID NO: 217 | PSEC0198 |
| SEQ ID NO: 220 | SEQ ID NO: 219 | PSEC0199 |
| SEQ ID NO: 222 | SEQ ID NO: 221 | PSEC0200 |
| SEQ ID NO: 224 | SEQ ID NO: 223 | PSEC0203 |
| SEQ ID NO: 226 | SEQ ID NO: 225 | PSEC0204 |
| SEQ ID NO: 228 | SEQ ID NO: 227 | PSEC0205 |
| SEQ ID NO : 230 | SEQ ID NO: 229 | PSEC0207 |
| SEQ ID NO: 232 | SEQ ID NO: 231 | PSEC0209 |
| SEQ ID NO: 234 | SEQ ID NO: 233 | PSEC0210 |
| SEQ ID NO: 236 | SEQ ID NO: 235 | PSEC0213 |
| SEQ ID NO: 238 | SEQ ID NO: 237 | PSEC0214 |
| SEQ ID NO: 240 | SEQ ID NO: 239 | PSEC0215 |
| SEQ ID NO: 242 | SEQ ID NO: 241 | PSEC0216 |
| SEQ ID NO : 244 | SEQ ID NO: 243 | PSEC0218 |
| SEQ ID NO: 246 | SEQ ID NO: 245 | PSEC0220 |
| SEQ ID NO: 248 | SEQ ID NO: 247 | PSEC0222 |
| SEQ ID NO: 250 | SEQ ID NO: 249 | PSEC0223 |
| SEQ ID NO: 252 | SEQ ID NO: 251 | PSEC0224 |
| SEQ ID NO: 254 | SEQ ID NO: 253 | PSEC0226 |
| SEQ ID NO: 256 | SEQ ID NO: 255 | PSEC0227 |
| SEQ ID NO: 258 | SEQ ID NO: 257 | PSEC0228 |
| SEQ ID NO: 260 | SEQ ID NO: 259 | PSEC0230 |
| SEQ ID NO: 262 | SEQ ID NO: 261 | PSEC0232 |
| SEQ ID NO: 264 | SEQ ID NO: 263 | PSEC0233 |
| SEQ ID NO: 266 | SEQ ID NO: 265 | PSEC0235 |
| SEQ ID NO: 268 | SEQ ID NO: 267 | PSEC0236 |
| SEQ ID NO: 270 | SEQ ID NO: 269 | PSEC0240 |
| SEQ ID NO: 272 | SEQ ID NO: 271 | PSEC0241 |
| SEQ ID NO: 274 | SEQ ID NO: 273 | PSEC0243 |
| SEQ ID NO: 276 | SEQ ID NO: 275 | PSEC0244 |
| SEQ ID NO: 278 | SEQ ID NO: 277 | PSEC0245 |
| SEQ ID NO: 280 | SEQ ID NO: 279 | PSEC0246 |
| SEQ ID NO: 282 | SEQ ID NO: 281 | PSEC0247 |
| SEQ ID NO: 284 | SEQ ID NO: 283 | PSEC0248 |
| SEQ ID NO: 286 | SEQ ID NO: 285 | PSEC0249 |
| SEQ ID NO: 288 | SEQ ID NO: 287 | PSEC0250 |
| SEQ ID NO: 290 | SEQ ID NO: 289 | PSEC0252 |
| SEQ ID NO: 292 | SEQ ID NO: 291 | PSEC0253 |
| SEQ ID NO: 294 | SEQ ID NO: 293 | PSEC0255 |
| SEQ ID NO: 296 | SEQ ID NO: 295 | PSEC0258 |
| SEQ ID NO: 298 | SEQ ID NO: 297 | PSEC0259 |
| SEQ ID NO: 300 | SEQ ID NO: 299 | PSEC0260 |
| SEQ ID NO: 302 | SEQ ID NO: 301 | PSEC0261 |
| SEQ ID NO: 304 | SEQ ID NO: 303 | PSEC0263 |
| SEQ ID NO: 306 | SEQ ID NO: 305 | PSEC0027 |
| SEQ ID NO: 308 | SEQ ID NO: 307 | PSEC0047 |
| SEQ ID NO: 310 | SEQ ID NO: 309 | PSEC0066 |
| | | |
| SEQ ID NO: 312 | SEQ ID NO: 311 | nnnnnnnn |
| SEQ ID NO: 314 | SEQ ID NO: 313 | PSEC0069 |
| SEQ ID NO: 316 | SEQ ID NO: 315 | PSEC0092 |
| SEQ ID NO: 318 | SEQ ID NO: 317 | PSEC0103 |
| SEQ ID NO: 320 | SEQ ID NO: 319 | PSEC0117 |
| SEQ ID NO: 322 | SEQ ID NO: 321 | PSEC0142 |
| SEQ ID NO: 324 | SEQ ID NO: 323 | PSEC0212 |
| SEQ ID NO: 326 | SEQ ID NO: 325 | PSEC0239 |
| SEQ ID NO: 328 | SEQ ID NO: 327 | PSEC0242 |
| SEQ ID NO: 330 | SEQ ID NO: 329 | PSEC0251 |
| SEQ ID NO: 332 | SEQ ID NO: 331 | PSEC0256 |
| SEQ ID NO: 334 | SEQ ID NO: 333 | PSEC0195 |
| SEQ ID NO: 336 | SEQ ID NO: 335 | PSEC0206 |
| SEQ ID NO: 342 | SEQ ID NO: 341 | PSEC0078 |
| SEQ ID NO: 344 | SEQ ID NO: 343 | PSEC0084 |
| SEQ ID NO: 346 | SEQ ID NO: 345 | PSEC0237 |
| SEQ ID NO: 348 | SEQ ID NO: 347 | PSEC0264 |
| SEQ ID NO: 350 | SEQ ID NO: 349 | PSEC0265 |

Furthermore, the present invention relates to the above polynucleotide, a protein encoded by the polynucleotide, and the use of them as described below.
(2) A substantially pure protein encoded by the polynucleotide of (1).
(3) Use of an oligonucleotide as a primer for synthesizing the polynucleotide comprising the nucleotide sequence set forth in any one of SEQ ID NOs: 370-540 or the complementary strand thereof, wherein said oligonucleotide is complementary to said polynucleotide or the complementary strand thereof and comprises at least 15 nucleotides.
(4) A primer set for synthesizing polynucleotides, the primer set comprising an oligo-dT primer and an oligonucleotide complementary to the complementary strand of the polynucleotide comprising the nucleotide sequence set forth in any one of SEQ ID NOs: 370-540, wherein said oligonucleotide comprises at least 15 nucleotides.
(5) A primer set for synthesizing polynucleotides, the primer set comprising a combination of an oligonucleotide comprising a nucleotide sequence complementary to the complementary strand of the polynucleotide comprising a 5'-end nucleotide sequence and an oligonucleotide comprising a nucleotide sequence complementary to the polynucleotide comprising a 3'-end nucleotide sequence, wherein said oligonucleotides comprise at least 15 nucleotides and wherein said combination of 5'-end nucleotide sequence/3'-end nucleotide sequence is selected from the combinations of 5'-end nucleotide sequence/3'-end nucleotide sequence set forth in the SEQ ID NOs in Table 342.
(6) A polynucleotide that can be synthesized with the primer set of (4) or (5).
(7) A polynucleotide comprising a coding region in the polynucleotide of (6).
(8) A protein encoded by polynucleotide of (7).
(9) A partial peptide of the protein of (8).
(10) An antibody against the protein or peptide of any one of (2), (8), and (9).
(11) A vector comprising the polynucleotide of (1) or (7).
(12) A transformant carrying the polynucleotide of (1) or (7), or the vector of (11).
(13) A transformant expressively carrying the polynucleotide of (1) or (7), or the vector of (11).
(14) A method for producing the protein or peptide of any one of (2), (8), and (9), comprising culturing the transformant of (13) and recovering the expression product.
(15) An oligonucleotide comprising the nucleotide sequence set forth in any one of the SEQ ID NOs in Table 1 or the nucleotide sequence complementary to the complementary strand thereof, wherein said oligonucleotide comprises 15 nucleotides or more.
(16) Use of the oligonucleotide of (15) as a primer for synthesizing a polynucleotide.
(17) Use of the oligonucleotide of (15) as a probe for detecting a gene.
(18) An antisense polynucleotide against the polynucleotide of (1), or the portion thereof.
(19) A method for synthesizing a polynucleotide, the method comprising:
   a) synthesizing a complementary strand using a cDNA library as a template, and using the primer set of (4) or (5), or the primer of (16); and
   b) recovering the synthesized product.
(20) The method of (19), wherein the cDNA library is obtainable by oligo-capping method.
(21) The method of (19), wherein the complementary strand is obtainable by PCR.
(22) A method for detecting the polynucleotide of (1), the method comprising:
   a) incubating a target polynucleotide with the oligonucleotide of (15) under the conditions where hybridization occurs, and
   b) detecting the hybridization of the target polynucleotide with the oligonucleotide of (15).
(23) A database of polynucleotides and/or proteins, the database comprising information on at least one sequence selected from the nucleotide sequences set forth in the SEQ ID NOs in Table 1 and/or the amino acid sequences set forth in the SEQ ID NOs in Table 1, or a medium on which the database is stored.

Table 342 shows a SEQ IDs of the nucleotide sequences defining 5'- and 3'-ends in the full-length cDNA of the present invention (173 clones), and the corresponding plasmid clones obtained in the examples described later, which contain the polynucleotides as an insert. Blank shows that the sequence of the 3'- end corresponding to the 5'-end has not been determined within the same clone. The SEQ ID of the 5'-sequence are shown on the right side of the name of the 5'-sequence, and the SEQ ID of the 3'- sequence are shown on the right side of the name of the 3'- sequence.

Any patents, patent applications, and publications cited herein are incorporated by reference.

### BRIEF DESCRIPTION OF THE DRAWINGS

Figure 1 shows the restriction maps of vectors pME18SFL3 and pUC19FL3.

Figure 2 shows the reproducibility of gene expression analysis. The ordinate and the abscissa show the intensities of gene expression obtained in experiments different from each other.

Figure 3 shows the detection limit in gene expression analysis. The intensity of expression is shown in the ordinate. and the concentration (µg/ml) of the probe used is shown in the abscissa.

Figure 4 is a photograph showing results of analyzing temporal expression of PSEC clones in NT cells at a pre-differentiation stage and at 1, 3, or 5 weeks after retinoic acid-treatment using RT-PCR.

PCR conditions (annealing temperature and 4 kinds of cycle numbers) used are indicated under the respective clone names or gene names. RA(-) and RA(+) represent undifferentiated NT2 cells and NT2 cells respectively cultured in the presence of retinoic acid. Each sample was analyzed by PCR with 4 types of conditions with different number of cycles (as mentioned above).

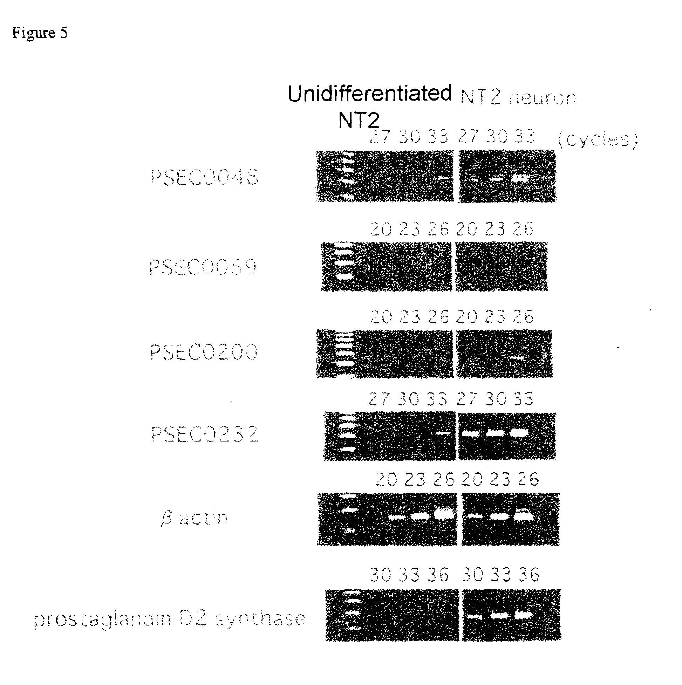

Figure 5 is a photograph showing results of analyzing gene expression of PSEC clones in undifferentiated NT2 cells and NT2 neurons using RT-PCR.

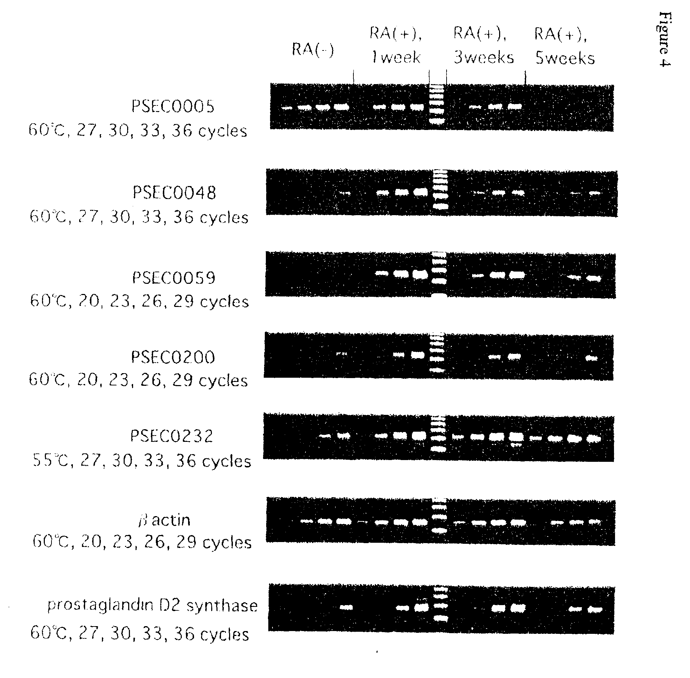

In the PCR experiment, the annealing temperature was the same as that used in Figure 4. Each sample was analyzed by PCR with 3 types of conditions with different number of cycles as indicated in the figure.

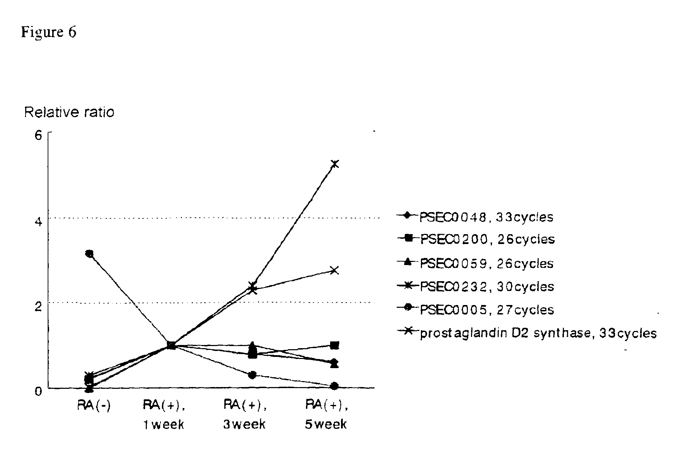

Figure 6 is a diagram showing temporal change in the expression level of the RT-PCR amplification products derived from PSEC clones. PCR conditions (the number of cycles) used are indicated adjacent to the respective clone names or gene names. RA(-) and RA(+) represent undifferentiated NT2 cells and NT2 cells respectively cultured in the presence of retinoic acid. Each point presented on the diagram was determined as a ratio obtained as follows. First, 3 independent data were averaged. Next, the average value was normalized by the corresponding average value representing the expression level of actin. Finally, the ratio was determined taking the amount of the products in NT2 cells cultured in the presence of retinoic acid for 1 week as 1.

### DETAILED DESCRIPTION OF THE INVENTION

Herein, "polynucleotide" is defined as a molecule in which multiple nucleotides are polymerized such as DNA or RNA. There are no limitations in the number of the polymerized nucleotides. In case that the polymer contains relatively low number of nucleotides, it is also described as an "oligonucleotide". The polynucleotide or the oligonucleotide of the present invention can be a natural or chemically synthesized product. Alternatively, it can be synthesized using a template DNA by an enzymatic reaction such as PCR.

All the cDNA provided by the invention are full-length cDNA. Herein, a "full-length cDNA" is defined as a cDNA that contains both ATG codon (the translation start site) and the stop codon. Accordingly, the untranslated regions, which are originally found in the upstream or downstream of the protein coding region in natural mRNA, may or may not be contained.

An "isolated polynucleotide" is a polynucleotide the structure of which is not identical to that of any naturally occurring nucleic acid or to that of any fragment of a naturally occurring genomic nucleic acid spanning more than three separate genes. The term therefore covers, for example,
(a) a DNA which has the sequence of part of a naturally occurring genomic DNA molecule but is not flanked by both of the coding sequences that flank that part of the molecule in the genome of the organism in which it naturally occurs;
(b) a nucleic acid incorporated into a vector or into the genomic DNA of a prokaryote or eukaryote in a manner such that the resulting molecule is not identical to any naturally occurring vector or genomic DNA;
(c) a separate molecule such as a cDNA, a genomic fragment, a fragment produced by polymerase chain reaction (PCR), or a restriction fragment; and
(d) a recombinant nucleotide sequence that is part of a hybrid gene, i.e., a gene encoding a fusion protein. Specifically excluded from this definition are nucleic acids present in mixtures of different (i) DNA molecules, (ii) transfected cells, or (iii) cell clones: e.g., as these occur in a DNA library such as a cDNA or genomic DNA library.
The term "substantially pure" as used herein in reference to a given polypeptide means that the protein or polypeptide is substantially free from other biological macromolecules. The substantially pure protein or polypeptide is at least 75% (e.g., at least 80, 85, 95, or 99%) pure by dry weight. Purity can be measured by any appropriate standard method, for example, by column chromatography, polyacrylamide gel electrophoresis, or HPLC analysis.

The present invention provides substantially pure human secretory protein or membrane protein comprising the amino acid sequence as shown in any SEQ ID NO: 2-336 and SEQ ID NO: 342-350; the ID number is also in Table 1. The 156 proteins out of 173 proteins of the present invention are encoded by the cDNA clones, shown in List 1. These clones were "the clones isolated from the full-length-enriched human cDNA libraries constructed by the oligo-capping method, using the programs such as ATGpr, and predicted by the PSORT to be a secretory protein or membrane protein which has a signal sequence in the N-terminus".

The list shown below indicates, in order, the following information separating each of these with a double-slash mark, //.
clone name (PSEC number),
length of cDNA,
length of amino acid sequence,
ATG No. from the 5' end,
ATGpr1 value,
definition of annotation data,
Accession No. of annotation data,
P value,
length of compared sequence,
homology

The annotation data are not shown for clones that did not exhibit explicit homology as a result of BLAST analysis of GenBank (http://www.ncbi.nm.nih.gov/Web/GenBank/index.html) and SwissProt (http://www.ebi.ac.uk/ebi_docs/swissprot_db/swisshome.html). The ATG No. from the 5' end means the position of ATG of the translation frame of the compared sequence counted from the 5' end. In other words, for example, when comparing with the translation frame from the first ATG, it is shown as "1^{st}", and when comparing with the translation frame beginning with the second ATG, it is shown as the "2^{nd}". The P value indicates similarity between two sequences as a score by considering the probability that the two sequences are accidentally similar. In general, as the value is lower, the similarity is higher. In general, as the value is lower, the homology is higher.

(Altschul, S.F., Gish, W., Miller, W., Myers, E. W. & Lipman, D.J. (1990) "Basic local alignment search tool." J. Mol. Biol. 215:403-410; Gish, W. & States, D.J. (1993) "Identification of protein coding regions by database similarity search." Nature Genet. 3:266-272)

### List 1

PSEC0001//1992bp//226aa//1st//0.94//GOLGI 4-TRANSMEMBRANE SPANNING TRANSPORTER MTP (KIAA0108).//Q15012//3.90E-53//221aa//46%
nnnnnnnn//1883bp//326aa//1st//0.94//Homo sapiens death effector domain-containing testicular molecule mRNA, complete cds.//AF043733//3.10E-37//852bp//62%
PSEC0005//1366bp//220aa//1st//0.94//Homo sapiens CLDN6 gene for claudin-6.//AJ249735//5.00E-285//1295bp//99%
PSEC0007//3425bp//570aa//1st//0.94//Homo sapiens FK506-binding protein (FKBP63) mRNA, partial cds.//AF089745//0//1580bp//99%
PSEC0008//978bp//215aa//1st//0.94//HYPOTHETICAL 72.5 KD PROTEIN C2F7.10 IN CHROMOSOME I.//Q09701//1.60E-13//119aa//36%
PSEC0012//1499bp//183aa//1st//0.82
PSEC0017//3125bp//273aa//1st//0.33//Mus musculus membrane protein TMS-2 mRNA, complete cds.//AF181685//3.00E-303//1949bp//82%
PSEC0019//1927bp//339aa//1st//0.9//Homo sapiens NPD003 mRNA, complete cds.//AF078855//0//1904bp//99%
PSEC0020//1483bp//393aa//1st//0.69
PSEC0021//1851bp//116aa//3rd//0.82
PSEC0028//2395bp//348aa//2nd//0.56//VESICULAR INTEGRAL-MEMBRANE PROTEIN VIP36 PRECURSOR (VIP36).//P49256//9.30E-100//355aa//54%
PSEC0029//1683bp//300aa//1st//0.9//OXIDOREDUCTASE UCPA (EC 1.-.-.-).//P37440//1.00E-21//217aa//32%
PSEC0030//1584bp//406aa//1st//0.26
PSEC0031//1336bp//136aa//2nd//0.2
PSEC0035//1729bp//406aa//1st//0.93//NEURONAL OLFACTOMEDIN-RELATED ER LOCALIZED PROTEIN PRECURSOR (NOEL) (1B426B).//Q62609//6.30E-33//373aa//28%
PSEC0038//1883bp//223aa//1st//0.9//TRIOSE PHOSPHATE/PHOSPHATE TRANSLOCATOR, NON-GREEN PLASTID PRECURSOR (CTPT).//P52178//6.60E-13//157aa//33%
PSEC0040//2027bp//216aa//2nd//0.82
PSEC0041//2518bp//240aa//2nd//0.51
PSEC0045//1631bp//372aa//1st//0.85
PSEC0048//3707bp//383aa//2nd//0.71//Homo sapiens serine protease mRNA, complete cds.//AF015287//0//1638bp//99%
PSEC0049//2652bp//131aa//1st//0.35//Homo sapiens brain my047 protein mRNA, complete cds.//AF063605//0//2651bp//99%
PSEC0051//3293bp//227aa//3rd//0.63

PSEC0052//3635bp//578aa//2nd//0.94//AQUALYSIN I PRECURSOR (EC 3.4.21.-).//P08594//1.60E-46//348aa//36%
PSEC0053//2366bp//285aa//1st//0.94//COLLAGEN ALPHA 1(XII) CHAIN PRECURSOR (FIBROCHIMERIN).//P13944//1.50E-37//227aa//31%
PSEC0055//2147bp//331aa//2nd//0.92//UDP N-ACETYLGLUCOSAMINE TRANSPORTER (GOLGI UDP-GLCNAC TRANSPORTER).//Q00974//4.80E-42//314aa//31%
PSEC0059//2863bp//230aa//3rd//0.72//Mus musculus claudin-2 mRNA, complete cds.//AF072128//4.50E-127//777bp//86%
PSEC0061//1931bp//464aa//1st//0.94//BETA-MANNOSYLTRANSFERASE (EC 2.4.1.-).//P16661//6.00E-42//356aa//35%
PSEC0068//1717bp//194aa//1st//0.64
PSEC0070//2510bp//286aa//3rd//0.94//OLIGOSACCHARYL TRANSFERASE STT3 SUBUNIT HOMOLOG.//P46975//2.50E-99//301aa//63%
PSEC0071//3558bp//875aa//1st//0.94//INTER-ALPHA-TRYPSIN INHIBITOR HEAVY CHAIN H3 PRECURSOR (ITI HEAVY CHAIN H3) (SERUM-DERIVED HYALURONAN-ASSOCIATED PROTEIN) (SHAP).//Q06033//9.30E-141//576aa//37%
PSEC0072//2092bp//350aa//1st//0.94//Homo sapiens mRNA for putative vacuolar proton ATPase membrane sector associated protein M8-9.//Y17975//2.10E-133//622bp//99%
PSEC0073//2341bp//523aa//1st//0.94//UDP-GLUCURONOSYLTRANSFERASE 2C1 MICROSOMAL (EC 2. 4. 1. 17) (UDPGT) (FRAGMENT).//P36514//7.90E-71//477aa//36%
PSEC0074//2971bp//770aa//1st//0.89//Mus musculus mRNA for semaphorin W, complete cds.//AB021291//0//2579bp//85%
PSEC0075//2244bp//633aa//2nd//0.79
PSEC0076//3253bp//860aa//1st//0.94//MITOCHONDRIAL PRECURSOR PROTEINS IMPORT RECEPTOR (72 KD MITOCHONDRIAL OUTER MEMBRANE PROTEIN) (MITOCHONDRIAL IMPORT RECEPTOR FOR THE ADP/ATP CARRIER) (TRANSLOCASE OF OUTER MEMBRANE TOM70).//P23231//3.80E-11//194aa//28%
PSEC0077//2195bp//483aa//1st//0.94//TROPONIN T, CARDIAC MUSCLE ISOFORMS (TNTC).//P02642//0.00000018//120aa//28%
PSEC0079//1290bp//189aa//2nd//0.94
PSEC0080//3171bp//740aa//2nd//0.94//Homo sapiens mRNA for NAALADase II protein.//AJ012370//0//3131bp//99%
PSEC0081//2890bp//172aa//1st//0.94
PSEC0082//1878bp//331aa//1st//0.94//PROBABLE OXIDOREDUCTASE (EC 1.-.-.-).//Q03326//7.30E-30//269aa//34%
PSEC0085//2392bp//280aa//1st//0.85//PROBABLE PROTEIN DISULFIDE ISOMERASE P5 PRECURSOR (EC 5.3.4.1).//P38660//5.60E-10//105aa//39%
PSEC0086//1821bp//390aa//1st//0.83//CELL SURFACE A33 ANTIGEN PRECURSOR.//Q99795//2.30E-23//259aa//32%

PSEC0087//1808bp//441aa//1st//0.94//Homo sapiens G protein-coupled receptor mRNA, complete cds.//AF181862//5.40E-27//1114bp//60%
PSEC0088//2015bp//467aa//1st//0.94//CATHEPSIN B PRECURSOR (EC 3.4.22.1).//P07688//1.10E-39//315aa//34%
PSEC0090//1722bp//543aa//1st//0.92//Homo sapiens heparanase (HPA) mRNA, complete cds.//AF144325//0//1722bp//99%
PSEC0094//2291bp//564aa//1st//0.93//PROTEIN PTM1 PRECURSOR.//P32857//7.10E-15//284aa//28%
PSEC0095//2080bp//349aa//1st//0.94
PSEC0098//2185bp//208aa//1st//0.94
PSEC0099//1627bp//350aa//2nd//0.91
PSEC0100//1391bp//172aa//1st//0.77//Homo sapiens clone 24952 mRNA sequence, complete cds.//AF131758//7.70E-308//1391bp//99%
PSEC0101//2547bp//258aa//2nd//0.92
PSEC0104//1430bp//418aa//2nd//0.79
PSEC0105//2506bp//494aa//1st//0.94
PSEC0106//2465bp//326aa//2nd//0.94
PSEC0107//2557bp//130aa//2nd//0.89
PSEC0108//3099bp//267aa//3rd//0.86//HYPOTHETICAL 49.3 KD PROTEIN C30D11.06C IN CHROMOSOME I.//Q09906//9.80E-17//307aa//28%
PSEC0109//2563bp//736aa//1st//0.94//Rattus norvegicus leprecan (lepre1) mRNA, complete cds.//AF087433//0//2501bp//84%
PSEC0110//2179bp//344aa//1st//0.94
PSEC0111//3362bp//208aa//1st//0.83
PSEC0112//3598bp//349aa//4th//0.74
PSEC0113//2451bp//423aa//1st//0.79//36 KD NUCLEOLAR PROTEIN HNP36 (DELAYED-EARLY RESPONSE PROTEIN 12) (DER12).//Q61672//4.20E-22//169aa//34%
PSEC0119//2518bp//555aa//1st//0.87//HYPOTHETICAL 63.9 KD PROTEIN C1F12.09 IN CHROMOSOME I.//Q10351//4.50E-26//240aa//30%
PSEC0120//2250bp//302aa//2nd//0.94//Human alpha-1,3-mannosyl-glycoprotein beta-1, 2-N-acetylglucosaminyltransferase (MGAT) gene, complete cds.//M61829//0//2235bp//92%
PSEC0121//1666bp//358aa//1st//0.94//HYPOTHETICAL 39.9 KD PROTEIN T15H9.1 IN CHROMOSOME II PRECURSOR.//Q10005//4.10E-106//351aa//58%
PSEC0124//1686bp//476aa//1st//0.91//VITELLOGENIC CARBOXYPEPTIDASE PRECURSOR (EC 3.4.16.-).//P42660//1.10E-103//444aa//45%
PSEC0125//1999bp//256aa//1st//0.74//Homo sapiens mRNA for type II membrane protein, complete cds, clone:HP10328.//AB015630//4.50E-306//1433bp//98%
PSEC0126//1906bp//102aa//1st//0.89//Homo sapiens mRNA for leukotriene B4 omega-hydroxylase, complete cds.//AB002454//3.90E-251//970bp//86%

PSEC0127//1773bp//218aa//1st//0.94
PSEC0128//2134bp//306aa//1st//0.94
PSEC0129//1828bp//135aa//1st//0.94
PSEC0130//2934bp//265aa//1st//0.68
PSEC0131//1658bp//297aa//1st//0.94
PSEC0133//2023bp//240aa//1st//0.94
PSEC0134//1898bp//144aa//6th//0.71
PSEC0135//1755bp//322aa//3rd//0.75//Homo sapiens lymphatic endothelium-specific hyaluronan receptor LYVE-1 mRNA, complete cds.//AF118108//0//1640bp//99%
PSEC0136//1907bp//392aa//1st//0.93
PSEC0137//2981bp//571aa//1st//0.94
PSEC0139//1361bp//218aa//2nd//0.89
PSEC0143//1976bp//125aa//1st//0.74//ENDOSOMAL P24A PROTEIN PRECURSOR (70 KD ENDOMEMBRANE PROTEIN) (PHEROMONE ALPHA-FACTOR TRANSPORTER) (ACIDIC 24 KD LATE ENDOCYTIC INTERMEDIATE COMPONENT).//P32802//1.00E-19//129aa//38%
PSEC0144//2067bp//247aa//1st//0.94//Homo sapiens CGI-78 protein mRNA, complete cds.//AF151835//0//1961bp//99%
nnnnnnnn//2807bp//346aa//7th//0.79//PUTATIVE G PROTEIN-COUPLED RECEPTOR GPR17 (R12).//Q13304//3.00E-44//308aa//36%
PSEC0147//1964bp//520aa//1st//0.91//HYPOTHETICAL 52.8 KD PROTEIN T05E11.5 IN CHROMOSOME IV.//P49049//3.60E-19//203aa//38%
PSEC0149//1988bp//432aa//1st//0.94
PSEC0150//2259bp//217aa//1st//0.94//Homo sapiens T-box protein TBX3 (TBX3) mRNA, complete cds.//AF170708//2.60E-140//673bp//98%
PSEC0151//1688bp//467aa//1st//0.93//TISSUE ALPHA-L-FUCOSIDASE PRECURSOR (EC 3.2.1.51) (ALPHA-L-FUCOSIDASE I) (ALPHA-L-FUCOSIDE FUCOHYDROLASE).//P04066//5.20E-145//459aa//55%
PSEC0152//2130bp//374aa//2nd//0.86
PSEC0158//1836bp//137aa//4th//0.94//Homo sapiens lifeguard (LFG) mRNA, complete cds.//AF190461//2.50E-44//591bp//68%
PSEC0159//2198bp//372aa//1st//0.8//Homo sapiens mRNA for type II membrane protein, complete cds, clone:HP10328.//AB015630//0//2186bp//99%
PSEC0161//2222bp//496aa//1st//0.89//GLUCOSE TRANSPORTER TYPE 5, SMALL INTESTINE (FRUCTOSE TRANSPORTER).//P22732//8.10E-101//479aa//42%
PSEC0162//1320bp//271aa//1st//0.83
PSEC0163//2167bp//578aa//1st//0.94//HYPOTHETICAL 67.8 KD PROTEIN IN IKI1-ERG9 INTERGENIC REGION.//P38875//3.10E-48//228aa//36%
PSEC0164//1877bp//463aa//1st//0.93//GLIOMA PATHOGENESIS-RELATED PROTEIN (RTVP-1 PROTEIN).//P48060//1.80E-27//169aa//39%
PSEC0165//2111bp//242aa//1st//0.83
PSEC0167//874bp//103aa//7th//0.73

PSEC0168//2533bp//269aa//1st//0.94//HYPOTHETICAL 42. 5 KD PROTEIN IN TSM1-ARE1 INTERGENIC REGION.//P25625//2.50E-18//179aa//30%
PSEC0169//1792bp//204aa//1st//0.75//Homo sapiens transmembrane 4 superfamily protein mRNA, complete cds.//AF100759//0//1771bp//99%
PSEC0170//2622bp//353aa//1st//0.94//Homo sapiens E2IG4 (E2IG4) mRNA, complete cds.//AF191019//0//2542bp//99%
PSEC0171//2005bp//301aa//2nd//0.91
PSEC0172//2012bp//415aa//1st//0.92//Homo sapiens procollagen C-terminal proteinase enhancer protein 2 (PCOLCE2) mRNA, complete cds.//AF098269//0//1741bp//99%
PSEC0173//1740bp//406aa//1st//0.91//NEURONAL OLFACTOMEDIN-RELATED ER LOCALIZED PROTEIN PRECURSOR (NOEL) (1B426B).//Q62609//6.60E-33//373aa//28%
PSEC0178//2308bp//222aa//3rd//0.94
PSEC0181//1890bp//165aa//3rd//0.66
PSEC0182//2153bp//657aa//2nd//0.82//Homo sapiens mRNA for UDP-GalNAc:polypeptide N-acetylgalactosaminyltransferase 7.//AJ002744//0//2006bp//99%
PSEC0183//2031bp//451aa//1st//0.88//CARTILAGE MATRIX PROTEIN PRECURSOR (MATRILIN-1).//P05099//5.50E-63//228aa//54%
PSEC0190//1841bp//194aa//1st//0.87
PSEC0191//1493bp//472aa//1st//0.87//ELASTIN PRECURSOR (TROPOELASTIN).//P15502//5.00E-113//367aa//67%
PSEC0192//1557bp//153aa//1st//0.93
PSEC0197//3555bp//576aa//2nd//0.85//PEROXISOMAL-COENZYME A SYNTHETASE (EC 6.-.-.-).//P38137//1.30E-33//169aa//32%
PSEC0198//2083bp//343aa//1st//0.94
PSEC0199//2586bp//283aa//1st//0.94
PSEC0200//1548bp//443aa//1st//0.94//Mus musculus immunosuperfamily protein B12 mRNA, complete cds.//AF061260//4.30E-243//1297bp//89%
PSEC0203//1457bp//323aa//1st//0.87
PSEC0204//1484bp//142aa//1st//0.74
PSEC0205//1656bp//435aa//1st//0.94//CELL DIVISION CONTROL PROTEIN 91.//P41733//7.70E-41//290aa//33%
PSEC0207//1754bp//262aa//3rd//0.94//Homo sapiens multispanning nuclear envelope membrane protein nurim (NRM29) mRNA, partial cds.//AF143676//0.00E+00//1399bp//99%
PSEC0209//2144bp//186aa//1st//0.93//Homo sapiens Pancreas-specific TSA305 mRNA , complete cds.//AB020335//0//1770bp//99%

PSEC0210//1689bp//349aa//1st//0.71
PSEC0213//1824bp//323aa//1st//0.94
PSEC0214//1959bp//141aa//1st//0.94
PSEC0215//2112bp//551aa//2nd//0.94//Homo sapiens emilin precursor, mRNA, complete cds and 3' UTR.//AF088916//0//1470bp//98%
PSEC0216//1765bp//410aa//2nd//0.89
PSEC0218//1369bp//242aa//1st//0.69//Homo sapiens torsinA (DYT1) mRNA, complete cds.//AF007871//3.10E-26//619bp//61%
PSEC0220//1584bp//365aa//1st//0.94//Mouse Wnt-6 mRNA, complete cds.//M89800//5.50E-198//1310bp//82%
PSEC0222//899bp//139aa//2nd//0.94
PSEC0223//1874bp//221aa//1st//0.94
PSEC0224//1463bp//170aa//1st//0.89//UROMODULIN PRECURSOR (TAMM-HORSFALL URINARY GLYCOPROTEIN) (THP).//P48733//8.30E-10//141aa//36%
PSEC0226//2103bp//477aa//1st//0.94//Mus musculus carboxypeptidase X2 mRNA, complete cds.//AF017639//1.00E-114//1057bp//66%
PSEC0227//1410bp//379aa//2nd//0.81//Cricetulus griseus SREBP cleavage activating protein (SCAP) mRNA, complete cds.//U67060//2.50E-231//1099bp//84%
PSEC0228//1483bp//146aa//1st//0.92//COP-COATED VESICLE MEMBRANE PROTEIN P24 PRECURSOR (P24A) (RNP21.4).//Q63524//5.90E-21//110aa//32%
PSEC0230//1784bp//271aa//1st//0.76//SIGNAL RECOGNITION PARTICLE RECEPTOR BETA SUBUNIT (SR-BETA).//P47758//5.80E-123//271aa//90%
PSEC0232//1709bp//246aa//1st//0.75//30 KD ADIPOCYTE COMPLEMENT-RELATED PROTEIN PRECURSOR (ACRP30) (ADIPOCYTE SPECIFIC PROTEIN ADIPOQ).//Q60994//3.30E-24//242aa//32%
PSEC0233//2499bp//267aa//1st//0.82
PSEC0235//1601bp//211aa//1st//0.94
PSEC0236//1906bp//529aa//1st//0.94//LAMININ GAMMA-1 CHAIN PRECURSOR (LAMININ B2 CHAIN).//P11047//5.00E-181//472aa//62%
PSEC0240//1638bp//253aa//1st//0.94//WNT-11 PROTEIN PRECURSOR.//096014//3.40E-109//220aa//93%

PSEC0241//3593bp//622aa//1st//0.85//Homo sapiens cerebral cell adhesion molecule mRNA, complete cds.//AF177203//2.50E-121//1541bp//68%
PSEC0243//2835bp//743aa//3rd//0.77
PSEC0244//2063bp//287aa//1st//0.91
PSEC0245//2896bp//418aa//3rd//0.91//INTEGRAL MEMBRANE GLYCOPROTEIN GP210 PRECURSOR.//P11654//3.40E-205//483aa//78%
PSEC0246//2969bp//345aa//1st//0.94//LOW-DENSITY LIPOPROTEIN RECEPTOR-RELATED PROTEIN 2 PRECURSOR (MEGALIN) (GLYCOPROTEIN 330).//P98158//1.60E-22//126aa//42%
PSEC0247//2872bp//236aa//1st//0.94//PLATELET-ENDOTHELIAL TETRASPAN ANTIGEN 3 (PETA-3) (GP27) (MEMBRANE GLYCOPROTEIN SFA-1) (CD151 ANTIGEN).//035566//3.30E-28//237aa//29%
PSEC0248//2694bp//172aa//1st//0.84
PSEC0249//3320bp//534aa//1st//0.94//BUTYROPHILIN PRECURSOR (BT).//Q62556//1.10E-21//276aa//32%
PSEC0250//2179bp//223aa//2nd//0.74//TWISTED GASTRULATION PROTEIN PRECURSOR.//P54356//1.50E-34//231aa//35%
PSEC0252//2617bp//491aa//3rd//0.89//HYPOTHETICAL 56. 2 KD PROTEIN IN ERG8-UBP8 INTERGENIC REGION.//Q04991//2.40E-15//208aa//29%
PSEC0253//2872bp//265aa//1st//0.69//PHOSPHATIDYLINOSITOL-4-PHOSPHATE 5-KINASE TYPE II ALPHA (EC 2.7.1.68) (PIP5KII-ALPHA) (1-PHOSPHATIDYLINOSITOL-4-PHOSPHATE KINASE) (PTDINS(4)P-5-KINASE B ISOFORM) (DIPHOSPHOINOSITIDE KINASE).//070172//1.30E-139//240aa//62%
PSEC0255//3774bp//687aa//2nd//0.89//Homo sapiens mRNA for TM7XN1 protein.//AJ011001//0//3700bp//99%
PSEC0258//3791bp//349aa//1st//0.94
PSEC0259//2583bp//242aa//2nd//0.89//CYTOCHROME B561 (CYTOCHROME B-561).//Q95245//3.70E-44//211aa//17%
PSEC0260//2492bp//496aa//1st//0.94
PSEC0261//3080bp//806aa//2nd//0.76//MITOCHONDRIAL PRECURSOR PROTEINS IMPORT RECEPTOR (72 KD MITOCHONDRIAL OUTER MEMBRANE PROTEIN) (MITOCHONDRIAL IMPORT RECEPTOR FOR THE ADP/ATP CARRIER) (TRANSLOCASE OF OUTER MEMBRANE TOM70).//P23231//4.60E-07//175aa//23%
PSEC0263//4144bp//971aa//2nd//0.94
PSEC0084//2788bp//335aa//1st//0.86//IMPLANTATION-ASSOCIATED PROTEIN.//035777//1.80E-167//335aa//92%
PSEC0237//1419bp//248aa//1st//0.81//Homo sapiens CTG1a mRNA, complete cds.//U80744//8.30E-22//556bp//61%
PSEC0264//2617bp//157aa//1st//0.94
PSEC0265//2646bp//192aa//1st//0.76
(Annotation 1) Clones with relatively low score in the ATGpr1 (PSEC0017, ATGpr1 0.33; PSEC0030, ATGpr1 0.26; PSEC0031, ATGpr1 0.20; PSEC0049, ATGpr1 0.35): These clones, in which data of the 5'-end sequence (one pass sequencing) was not sorted by the ATGpr, were selected as a clone having both the signal sequence and long ORF based on the data of the 5'-end sequence, and the sequence of their full-length cDNA clones was analyzed. All the clones have the signal sequence in the N-terminus. In addition, the above 4 clones except PSEC0049 had portions not contained in known EST in the 5'-end when compared to known EST. PSEC0049 had portions not contained in EST in the 5'-end within the ORF of the cDNA when compared with known EST. Thus, it turned out that these clones were full-length cDNA clones.

The next 15 proteins out of the 173 proteins of the present invention were encoded by the cDNA clones as shown in List 2 (PSEC0027, PSEC0047, PSEC0066, nnnnnnnn, PSEC0069, PSEC0078, PSEC0092, PSEC0103, PSEC0117, PSEC0142, PSEC0212, PSEC0239, PSEC0242, PSEC0251, and PSEC0256). These clones were predicted to encode a membrane protein (containing the transmembrane helix) by the MEMSAT (Jones D.T., Taylor W.R., and Thornton J.M. (1994) Biochemistry 33: 3038-3049). Similarly, the clones were predicted to encode a membrane protein by the SOSUI (Hirokawa T. et al. (1998) Bioinformatics 14: 378-379) (Mitsui Information Development Inc.). Thus, the clones were those "isolated from the human cDNA libraries constructed by the oligo-capping method, predicted to be a full-length cDNA clone by ATGpr etc., and predicted to encode a membrane protein by both MEMSAT and SOSUI". The proteins encoded by the clones are also classified into the category of a secretory proteins or membrane proteins described above. Two clones among the 15 clones (PSEC0242, and PSEC0251) were predicted to encode a membrane protein without a signal sequence in the N-terminus. However, in both clones; if translation starts from the third ATG (having high score in the ATGpr1), the resulting protein will contain a signal sequence in the N-terminus. Accordingly, it is possible that the two clones are classified into the category of secretory proteins or membrane proteins that contains a signal sequence in N-terminus.

The list shown below indicates PSEC number, length of cDNA, length of amino acid sequence, ATG No. from the 5' end, ATGpr1 value, predicted result for signal sequence by PSORT, predicted result for membrane protein by MEMSAT and SOSUI, definition of annotation data, Accession No. of annotation data, P value, length of compared sequence, and homology in this order, separating each of these with a double-slash mark, //.
The annotation data are not shown for clones that did not exhibit explicit homology as a result of BLAST analysis of GenBank (http://www.ncbi.nlm.nih.gov/Web/GenBank/index.html) and SwissProt (http://www.ebi.ac.uk/ebi_docs/swissprot_db/swisshome.html).

### List 2

PSEC0027//1085bp//271aa//1st//0.94//No//transmembrane
PSEC0047//2048bp//267aa//1st//0.94//No//transmembrane//INTEGRAL MEMBRANE PROTEIN 2B (TRANSMEMBRANE PROTEIN E3-16).//042204//1.80E-55//264aa//44%
PSEC0092//3624bp//465aa//1st//0.94//No//transmembrane//Homo sapiens mRNA for heparan-sulfate 6-sulfotransferase, complete cds.//AB006179//2.70E-102//1057bp//71%
PSEC0066//2682bp//474aa//1st//0.79//No//transmembrane//TETRACYCLINE RESISTANCE PROTEIN, CLASS E (TETA(E)).//Q07282//7.50E-19//173aa//31%
nnnnnnnn//2105bp//730aa//1st//0.26//No//transmembrane//VERY-LONG-CHAIN ACYL-COA SYNTHETASE (EC 6.2.1.-) (VERY-LONG-CHAIN- FATTY-ACID-COA LIGASE).//035488//2.50E-140//520aa//45%
PSEC0069//2568bp//433aa//2nd//0.94//No//transmembrane
PSEC0103//2530bp//236aa//1st//0.94//No//transmembrane//Homo sapiens neuroendocrine-specific protein-like protein 1 (NSPL1) mRNA, complete cds.//AF119297//0//2524bp//99%
PSEC0117//1873bp//583aa//1st//0.94//No//transmembrane//Rattus norvegicus lipolysis-stimulated remnant receptor beta subunit mRNA, complete cds.//AF119669//2.00E-221//1048bp//76%
PSEC0142//2153bp//343aa//2nd//0.94//No//transmembrane//PROBABLE G PROTEIN-COUPLED RECEPTOR RTA.//P23749//1.20E-159//343aa//84%
PSEC0212//1677bp//111aa//1st//0.94//No//transmembrane//Homo sapiens NJAC protein (NJAC) mRNA, complete cds.//AF144103//1.40E-237//1303bp//91%
PSEC0239//1712bp//423aa//2nd//0.18//No//transmembrane//Homo sapiens aspartyl protease mRNA, complete cds.//AF050171//0//1712bp//93%
PSEC0242//3017bp//401aa//1st//0.9//No//transmembrane
PSEC0251//2372bp//393aa//1st//0.78//No//transmembrane
PSEC0256//3520bp//612aa//1st//0.89//No//transmembrane//Homo sapiens protocadherin alpha 12 (PCDH-alpha12) mRNA, complete cds.//AF152308//0//3520bp//99%
PSEC0078//2194bp//333aa//2nd//0.24//No//transmembrane//M-Sema F=a factor in neural network development [mice, neonatal brain, mRNA, 3503 nt].//S79463//1.50E-282//1945bp//83%

### (Annotation 1)

Clones with relatively low score in the ATGpr1 (PSEC0239, ATGpr1 0.18): PSEC0239 was selected as a clone having high score in the ATGpr based on the 5'-end sequence data (one pass sequencing), and also was predicted to be a membrane protein (containing the transmembrane helix) by the MEMSAT and SOSUI. In addition, the comparison with known ESTs revealed that the clone has a portion not contained in ESTs in the 5'-end of the cDNA.

### (Annotation 2)

PSEC0242 and PSEC0251: The clones are classified into the category of the cDNA encoding the polypeptide "containing the signal sequence in the N-terminus", if translation starts from the third ATG.
PSEC0242: No.3 ATG, ATGpr1 0.82, SP-Yes, ORF 171-1343, 391 aa, Signal peptide 24 aa;
PSEC0251: No.3 ATG, ATGpr1 0.77, SP-Yes, ORF 116-1256, 380 aa, Signal peptide 28 aa.

Herein, "SP-Yes" means that a signal sequence is present at the N-terminus, predicted by the PSORT.

### (Annotation 3)

The ATGpr1 value for PSEC0078 was 0.24. This is a clone exhibited high ATGpr1 value based on the 5'-end sequence data (one pass sequencing), and also has been predicted to be a membrane protein (having a transmembrane helix) by MEMSAT and SOSUI analyses. In addition, in comparison with EST sequences, the cDNA sequence was not found to be 50 bp or more shorter than any EST sequence at their 5'-end, and therefore the clone was not judged to be a incomplete cDNA clone by using ESTs as criteria for the judgment.

The last 2 proteins among the 173 proteins of the present invention were encoded by the cDNA clones shown in List 3 (PSEC0195, and PSEC0206). As a result of the homology search of the SwissProt, PSEC0195, and PSEC0206 were found to have relatively high homology with mouse plasma membrane adapter HA2/AP2 adaptin alpha C subunit, and human carboxypeptidase H precursor (prohormone processing carboxypeptidase) in the secretory granule, respectively. Accordingly, the proteins are classified into the category of secretory proteins or membrane proteins.

### List 3

The list shown below indicates PSEC number, length of cDNA, length of amino acid sequence, ATG No. from the 5' end, ATGpr1 value, predicted result for signal sequence by PSORT, predicted result for membrane protein by MEMSAT and SOSUI, definition of annotation data, Accession No. of annotation data, P value, length of compared sequence, and homology in this order, separating each of these with a double-slash mark, //.
PSEC0195//1979bp//467aa//2nd//0.80//No//No//ALPHA-ADAPTIN C (CLATHRIN ASSEMBLY PROTEIN COMPLEX 2 ALPHA-C LARGE CHAIN) (100 KD COATED VESICLE PROTEIN C) (PLASMA MEMBRANE ADAPTOR HA2/AP2 ADAPTIN ALPHA C SUBUNIT).//P17427//1.8E-144//281aa//98%
PSEC0206//1606bp//430aa//3rd//0.90//No//No//CARBOXYPEPTIDASE H PRECURSOR (EC 3.4.17.10) (CPH) (CARBOXYPEPTIDASE E) (CPE) (ENKEPHALIN CONVERTASE) (PROHORMONE PROCESSING CARBOXYPEPTIDASE).//P15087//1.8E-103//397aa//49%

Since the amino acid sequence of the secretory protein or membrane protein of the present invention has been determined, it is possible to analyze its biological function(s) by expressing it as a recombinant protein utilizing an appropriate expression system, or by using a specific antibody against it.

For example, the biological activity of a secretory protein or membrane protein can be analyzed according to the methods described in "Glycobiology" (Fukuda M., and Kobata A. edit., (1993)), "Growth Factors" (McKay I., and Leigh I. edit., (1993)), and "Extracellular Matrix" (Haralson M.A., Hassell J.R. edit., (1995)) in the series of "The Practical Approach" (IRL PRESS), or "Glycoprotein Analysis in Biomedicine" (Hounsell E.F. edit., (1993)) in the series of "Method in Molecular Biology" (Humana Press). Alternatively, the methods disclosed in "New protocols in biochemical experiments Vol.7: Growth and differentiation factors and their receptors" (Japan Biochemistry Society edit. (1991)) (Tokyo Kagaku-Dojin), or "Vol.296: Neurotransmitter Transporters", "Vol.294: Ion Channels (Part C)", "Vol.293: Ion Channels (Part B)", "Vol.292: ABC Transporters", "Vol.288: Chemokine Receptors", "Vol.287: Chemokines", "Vol.248: Proteolytic Enzymes", "Vol.245: Extracellular Matrix Components", "Vol.244: Proteolytic Enzymes", "Vol.230: Guide to Techniques in Glycobiology", "Vol.198: Peptide Growth Factors". "Vol.192: Biomembranes", "Vol.191: Biomembranes", and "Vol.149: Drug and Enzyme Targeting" in the series of "Methods in Enzymology" (Academic Press) may be used to analyze the biological activity of a secretory protein or membrane protein. As for secretory proteins and membrane proteins, in the search of the Online Mendelian Inheritance in Man (OMIM) (http://www.ncbi.nlm.nih.gov/Omim/) using the following keywords, the results obtained with each keyword, suggest the association of the proteins with many diseases, as described below. Therefore, the secretory proteins and membrane proteins are useful as a target in the medicinal industry.

New information is constantly updated in the OMIM database. Therefore, it is possible for one skilled in the art to find a new relationship between a particular disease and a gene of the present invention in the updated database.

Keywords used in the search of the OMIM
(1) secretion protein
(2) membrane protein

Shown in the search result are only the accession numbers in the OMIM. Using the number, data showing the relationship between a disease and a gene or protein can be seen. The OMIM data has been renewed everyday.
1) Secretion protein
   268 entries found, searching for "secretion protein"
   104760, 176860, 160900, 107400, 118910, 139320, 603850, 147572, 176880, 600946, 603215, 157147, 600174, 151675, 170280, 179512, 179513, 138120, 179509, 246700, 179510, 600626, 179511, 600998, 109270, 601489, 154545, 179490, 185860, 603216, 122559, 601746, 147290, 602672, 146770, 603062, 179508, 131230, 601591, 602421, 139250, 167805, 167770, 600041, 600564, 118825, 601146, 300090, 600753, 601652, 600759, 600768, 602434, 182590, 603166, 308230, 602534. 603489, 107470, 150390, 104610, 173120, 158106, 143890, 306900, 308700, 134797, 137350, 227500, 176300, 107730, 600760, 138079, 120180, 120160, 120150, 124092, 138160, 101000, 227600, 600509, 601199, 142110, 104311, 193400, 201910, 107300, 122560, 272800, 217000, 590050, 147670, 133170, 176730, 300300, 134370, 274600, 120140, 162151, 158070, 152790, 120120, 106100, 300200, 192340, 190160, 138040, 147470, 147620, 173350, 147380, 152200, 152760, 157145, 153450, 264080, 113811, 600937, 600840, 188545, 202110, 600514, 186590, 603372, 136435, 137241, 252800, 214500, 207750, 138850, 139191, 142640, 138130, 189907, 603692, 600633, 603355, 107270, 600377, 147892, 232200, 600281, 232800, 602358, 137035, 601771, 601769, 253200, 601933, 118444, 600270, 120700, 600945, 603732, 147660, 600761, 172400, 600823, 600877, 130080, 171060, 107740, 307800, 602843, 130660, 152780, 124020, 601124, 601340, 601604, 601610, 171050, 312060, 232700, 300159, 142703, 600734, 125255, 168450, 123812, 188540, 147940, 188450, 600839, 182452, 188400, 182280, 176760, 263200, 600264, 188826. 252650, 601185, 162641, 137216, 601398, 601538, 118888, 118445, 601745, 190180, 601922, 182098, 602008, 147440, 602384, 600031, 109160, 602663, 151670, 602682, 602730, 602779, 146880, 603061, 142704, 603140, 106150, 600732, 153620, 603318, 139392, 600042, 102200, 603493, 182100, 264300, 603795, 184600
2) Membrane protein
   1017 entries found, searching for "membrane protein"
   130500, 305360, 153330, 173610, 170995, 109270, 170993, 309060, 120920, 602333, 133740, 133710, 602690, 133730, 159430, 600897, 133090, 601178, 602413, 602003, 109280, 603237, 602173, 107776, 602334, 125305, 602335, 182879, 154045, 309845, 600594, 603718, 603241, 603214, 603657, 603177, 600182, 601476, 602879, 136950, 600723, 601114, 185880, 185881, 300096, 602257, 160900, 177070, 603062, 603344, 602977, 310200, 600959, 300100, 186945, 600039, 600267, 128240, 182900, 601097, 136430, 600946, 602534, 601047, 143450, 603141, 603700, 600579, 256540, 159440, 602414, 600403, 602048, 188860, 137290, 158343, 184756, 602910, 603179, 600279, 108733, 107770, 173335, 602625, 154050, 219800, 603850, 601028, 600447, 104225, 186946, 601767, 603143, 121015, 603215, 227400, 603735, 600179, 602421, 180721,
   176801, 176860, 600753, 603142, 176790, 600266, 601239, 115501, 143890, 121014, 121011, 125950, 603534, 304040, 601134, 600754, 601510, 601595, 190315, 300172, 602216, 602261, 602262, 602461, 131560, 179514, 179512, 176981, 142461, 139310, 312080, 176640, 128239, 185470, 310300, 601403, 601757, 273800, 151460, 176943, 104311, 168468, 120130, 602887, 600164, 601531, 601832, 104775, 600040, 603583, 176894, 602631, 166945, 182180, 120620, 141180, 601014, 139150, 182860, 177061, 600174, 180069, 191275, 104760, 601693, 300017, 603518, 601009, 134651, 601107, 603868, 600168, 136425, 603531, 603291, 600917, 603216, 102720, 300118, 179590, 135630, 602285, 107150, 602296, 303630, 176878, 120090, 600322, 138160, 601212, 603293, 131230, 112205, 600763, 600718, 300187, 170715, 601966, 300051, 602474,
   120070, 600691, 600855, 182309, 602101, 602857, 194355, 162230, 600874, 113730, 155550, 602701, 306400, 601789, 231200, 107271, 175100, 182870, 305100, 301000, 601313, 157147, 147670, 139200, 603593, 157655, 600934, 155970, 602049, 155960, 155760, 118990, 135620, 308230, 602694, 162060, 300023, 160993, 153619, 153432, 120131, 603823, 603167, 601023, 600816, 165040, 601681, 166490, 300112, 120190, 300145, 163970, 600772, 602926, 602933, 602202, 400015, 151510, 600759, 602672, 602654, 603821, 116952, 151430, 602632, 155975, 602217, 150370, 600752, 601119, 600932, 603048, 603234, 601805, 603822, 603869, 601717, 601181, 313440, 139130, 107777, 109190, 603452, 191163, 191164, 602370, 176877, 103195, 600523, 191328, 601275, 204200, 602426, 603810, 600551, 600695, 600552, 600553, 602306, 601523,
   602507, 602299, 600583, 114070, 600632, 603498, 185430, 600587, 235200, 173470, 603199, 601633, 602500, 208900, 180297, 156225, 516020, 190195, 141900, 102680, 193300, 101000, 193400, 300011, 107400, 257220, 107741, 180380, 203200, 111700, 600024, 304800, 600065, 110750, 179605, 113705, 601638, 222900, 120120, 602509, 602469, 600930, 601383, 176261, 602574, 602997, 311770, 131550, 603616, 308700, 603372, 256100, 224100, 276903, 305900, 516000, 131195, 314555, 601567, 603866, 306900, 103390, 186720, 173850, 601050, 602505, 186590, 246530, 602689, 194380, 300041, 162643, 152790, 120150, 600682, 600106, 272750, 188040, 602382, 601497, 113811, 182138, 212138, 601309, 109690, 114760, 176805, 601253, 123900, 602581, 189980, 191190, 110700, 600163, 137167, 600580, 601610, 190000, 123825, 603491,
   600135, 186591, 173910, 138140, 107266, 120950, 601081, 603690, 244400, 312700, 171060, 601199, 601758, 170500, 277900, 601997, 314850, 601880, 603009, 120220, 603126, 164920, 602934, 164730, 163890, 603434, 107269, 602909, 600877, 256550, 164761, 602872, 120110, 126150, 158010, 266200, 223360, 250800, 269920, 252650, 603355, 154582, 138190, 300035, 602640, 227650, 158120, 153700, 182380, 155740, 204500, 603401, 601975, 300135, 136350, 602924, 300167, 185050, 176100, 300189, 151525, 300200, 165180, 230800, 602158, 602676, 603411, 193245, 120325, 601848, 192500, 603102, 147795, 245900, 137060, 147557, 120650, 602317, 307800, 120930, 308100, 142800, 191092, 232300, 173510, 602225, 180470, 190930, 186357, 134638, 600544, 601373, 600509, 600359, 603784, 600395, 600653, 603754, 601597, 601066,
   600185, 601295, 600978, 205400, 603274, 600418, 600839, 516050, 601691, 601007, 600650, 600308, 603261, 601193, 600004, 600017, 516040, 253800, 276901, 600019, 257200, 108780, 300037, 300104, 300126, 255125, 203300, 300191, 426000, 302060, 304700, 201475, 252010, 193210, 311030, 306250, 248600, 191740, 108360, 131244, 600423, 232200, 191305, 231680, 103320, 190180, 600493, 111200, 226200, 312600, 600170, 111680, 186910, 203100, 600536, 600238, 186830, 186760, 186745, 186711, 106180, 112203, 103180, 182530, 182160, 600644, 307030, 192321, 600667, 125647, 179080, 114207, 114860, 176000, 116930, 600748, 173515, 173325, 600377, 171760, 171050, 118425, 170260, 191315, 600798, 600821, 600823, 600444, 600840, 159465, 600857, 158380, 600867, 154360, 152427, 150330, 110900, 147840, 147360, 147280,
   146880, 312610, 120940, 142871, 142790, 600937, 142600, 134390, 111250, 600979, 600997, 142460, 186845, 134635, 601017, 139191, 139090, 138850, 601040, 138720, 122561, 131100, 123610, 217070, 100500, 603377, 602354, 603302, 603207, 603086, 602188, 602095, 603867, 603842, 603798, 602602, 601194, 602607, 603713, 603681, 601252, 603648, 603646, 603644, 601282, 601284, 603667, 603712, 603594, 601872, 603425, 601843, 603263, 603208, 601411, 603201, 603189, 601463, 603164, 603152, 603087, 602874, 601492, 602893, 602057, 602859, 602746, 603879, 603510, 602458, 603380, 601581, 603765, 603283, 601599, 601733, 601852, 602316, 601615, 601617, 602184, 602894, 603005, 603030, 603861, 602835, 602136, 600153, 600074, 600046, 600023, 601625, 516006, 600018, 600016, 516002, 601590, 313475, 313470, 600244,
   600528, 601611, 600282, 600327, 601568, 600368, 601130, 601535, 601745, 601929, 300169, 300150, 300132, 601533, 600385, 600464, 600421, 600429, 601156, 601488, 516005, 251100, 516004, 600918, 516003, 602192, 516001, 240500, 600465, 602241, 602243, 230200, 601485, 601478, 601416, 602297, 601459, 601839, 602314, 193065, 193001, 191306, 600504, 601020, 191191, 602372, 190181, 600534, 188380, 186854, 186360, 600530, 185250, 182331, 600535, 182305, 601296, 600582, 600732, 600734, 600742, 600782, 176802, 176266, 600769, 601883, 600864, 601901, 176260, 173490, 600910, 601905, 171890, 600916, 601987, 602679, 162651, 161555, 160994, 602714, 602715, 602724, 602736, 300007, 602783, 275630, 602836, 270200, 602871, 159460, 602876, 154540, 153900, 602890, 601153, 602190, 602905, 153634, 153337, 602914,
   152310, 151690, 151625, 602935, 602974, 150325, 602992, 150320, 250790, 603006, 603007, 603008, 150292, 233690, 603046, 150210, 603061, 147940, 603063, 221770, 223300, 603097, 147880, 603118, 147730, 146928, 146630, 142622, 603149, 603150, 603151, 600923, 138981, 138590, 138330, 216950, 603192, 138297, 603202, 601002, 602343, 138230, 136131, 603217, 603220, 134660, 131390, 131235, 603242, 603243, 130130, 602345, 126455, 601123, 126064, 125240, 602359, 603312, 602380, 603318, 123890, 123836, 603356, 603361, 603366, 123830, 179610, 188060, 123620, 120980, 186355, 118510, 114835, 114217, 113810, 603499, 182310, 111740, 109610, 603548, 603564, 108740, 603598, 603613, 107273, 603626, 602518, 179410, 603647, 602515, 603652, 106195, 602573, 178990, 105210, 104615, 167055, 603717, 104614, 603728,
   104210, 603749, 603750, 103850, 602608, 603787, 603788, 603796, 173445, 103220, 102910, 102681, 102670, 102642, 603833, 173391, 102576, 102575, 171833, 102573, 101800, 603875, 601108

There are several methods for analyzing the expression levels of genes associated with diseases. Differences in gene expression levels between diseased and normal tissues are studied by the analytical methods, for example, Northern hybridization and differential display. Other examples include a method with high-density cDNA filter, a method with DNA microarray and methods with PCR amplification (Experimental Medicine, Vol.17, No. 8, 980-1056 (1999); Cell Engineering (additional volume) DNA Microarray and Advanced PCR Methods, Muramatsu & Naba (eds.), Shujunsya). The levels of gene expression between diseased tissues and normal tissues can be studied by any of these analytical methods. When explicit difference in expression level is observed for a gene, it can be concluded that the gene is closely associated with a disease or disorder. Instead of diseased tissues, cultured cells can be used for the assessment. Similarly, when gene expression is explicitly different between normal cells and cells reproducing disease-associated specific features, it can be concluded that the gene is closely associated with a disease or disorder. When the expression levels of genes are evidently varied during major cellular events (such as differentiation and apoptosis), the genes are involved in the cellular events and accordingly are candidates for disease- and/or disorder-associated genes. Further, genes exhibiting tissue-specific expression are genes playing important parts in the tissue functions and, therefore, can be candidates for genes associated with diseases and/or disorders affecting the tissues.

For example, non-enzymic protein glycation reaction is believed to be a cause for a variety of chronic diabetic complications. Accordingly, genes, of which expression levels are elevated or decreased in a glycated protein-dependent manner, are associated with diabetic complications caused by glycated proteins (Diabetes 1996, 45 (Suppl. 3), S67-S72; Diabetes 1997, 46 (Suppl. 2), S19-S25). The onset of rheumatoid arthritis is thought to be involved in the proliferation of synovial cells covering inner surfaces of joint cavity and in inflammatory reaction resulted from the action of cytokines produced by leukocytes infiltrating into the joint synovial tissues (Rheumatism Information Center, http://www.rheuma-net.or.jp/). Recent studies have also revealed that tissue necrosis factor (TNF)-α participates in the onset (Current opinion in immunology 1999, 11, 657-662). When the expression of a gene exhibits responsiveness to the action of TNF on synovial cells, the gene is considered to be involved in rheumatoid arthritis. Genes associated with neural differentiation can be candidates for causative genes for neurological diseases as well as candidates for genes usable for treating the diseases.

Clones exhibiting differences in the expression levels thereof can be selected by using gene expression analysis. The selection comprises, for example; analyzing cDNA clones by using high-density cDNA filter; and statistically treating the multiple signal values (signal values of radioisotope in the radiolabeled probes or values obtained by measuring fluorescence intensities emitted from the fluorescent labels) for the respective clones by two-sample t-test, where the signal values are determined by multiple experiments of hybridization. The clones of interest are selectable based on the statistically significant differences in the signal distribution at p<0.05. However, selectable clones with significant difference in the expression levels thereof may be changed depending on the partial modification of statistical treatment. For example, the clones may be selected by conducting statistical treatment with two-sample t-test at p<0.01; or genes exhibiting more explicit differences in the expression levels thereof can be selected by performing statistical treatment with a pre-determined cut-off value for the significant signal difference. An alternative method is that the expression levels are simply compared with each other, and then, the clones of interest are selected based on the ratio of the expression levels thereof.

Clones exhibiting differences in the expression levels thereof can also be selected by comparing the expression levels by PCR analysis, for example, by using the method of determining the band intensities representing the amounts of PCR products with ethidium bromide staining; or the method of determining the values of radioisotope signals or fluorescence intensities of the probes hybridized to the PCR products when radiolabeled or fluorescent dye-labeled probes, respectively, are used in the hybridization. If the expression level ratios obtained in multiple PCR experiments are constantly at least 2-fold, such a clone can be judged to exhibit the difference in the expression level thereof. When the ratios are several-fold or not less than 10-fold, the clone can be selected as a gene exhibiting the explicit difference in the expression level thereof.

A survey of genes of which expression levels are varied in response to TNF α (Tumor Necrosis Factor-alpha) in the primary cell culture of synovial tissue detected the following clones with elevated expression levels in the presence of TNF α :
PSEC0070, PSEC0073, PSEC0084, PSEC0100, PSEC0109, PSEC0120, PSEC0131, PSEC0161, PSEC0183, PSEC0192, PSEC0197, PSEC0205, PSEC0207, PSEC0210, PSEC0213, PSEC0222, PSEC0230, PSEC0241, PSEC0252, PSEC0259.

On the other hand, clones with decreased expression levels in the presence of TNF α are PSEC0105 and PSEC0245. These clones are candidates for rheumatoid arthritis-associated genes.

A survey of genes of which expression levels are varied in response to the stimulation for inducing cell differentiation (stimulation using retinoic acid (RA)) in cultured cells of neural strain, NT2, detected the following clones with varied expression levels: PSEC0005, PSEC0048, PSEC0059, PSEC0200, and PSEC0232. These are important genes associated with neural differentiation. The following clones also had varied their expression levels: PSEC0017, PSEC0019, PSEC0021, PSEC0030, PSEC0041, PSEC0047, PSEC0049, PSEC0055, PSEC0066, PSEC0070, PSEC0071, PSEC0072, PSEC0074, PSEC0075, PSEC0076, PSEC0080, PSEC0081, PSEC0084, PSEC0088, PSEC0094, PSEC0103, PSEC0104, PSEC0105, PSEC0112, PSEC0113, PSEC0117, PSEC0119, PSEC0120, PSEC0127, PSEC0129, PSEC0136, PSEC0139, PSEC0143, PSEC0144, PSEC0152, PSEC0161, PSEC0169, PSEC0171, PSEC0181, PSEC0182, PSEC0192, PSEC0195, PSEC0203, PSEC0215, PSEC0223, PSEC0235, PSEC0239, PSEC0243, PSEC0251, PSEC0255, PSEC0265.

These clones are also associated with neural differentiation and, therefore, are candidates for genes associated with neurological diseases.

Based on the functional analyses using a secretory protein or membrane protein, it is possible to develop a medicine.
In case of a membrane protein, it is most likely to be a protein that functions as a receptor or ligand on the cell surface. Therefore, it is possible to reveal a new relationship between a ligand and receptor by screening the membrane protein of the invention based on the binding activity with the known ligand or receptor. Screening can be performed according to the known methods.

For example, a ligand against the protein of the invention can be screened in the following manner. Namely, a ligand that binds to a specific protein can be screened by a method comprising the steps of: (a) contacting a test sample with the protein of the invention or a partial peptide thereof, or cells expressing these, and (b) selecting a test sample that binds to said protein, said partial peptide, or said cells.

On the other hand, for example, screening using cells expressing the protein of the present invention that is a receptor protein can also be performed as follows. It is possible to screen receptors that is capable of binding to a specific protein by using procedures (a) attaching the sample cells to the protein of the invention or its partial peptide, and (b) selecting cells that can bind to the said protein or its partial peptide.

In a following screening as an example, first the protein of the invention is expressed, and the recombinant protein is purified. Next, the purified protein is labeled, binding assay is performed using a various cell lines or primary cultured cells, and cells that are expressing a receptor are selected (Growth and differentiation factors and their receptors, Shin-Seikagaku Jikken Kouza Vol.7 (1991) Honjyo, Arai, Taniguchi, and Muramatsu edit, p203-236, Tokyo-Kagaku-Doujin). A protein of the invention can be labeled with RI such as ¹²⁵I, and enzyme (alkaline phosphatase etc.). Alternatively, a protein of the invention may be used without labeling and then detected by using a labeled antibody against the protein. The cells that are selected by the above screening methods, which express a receptor of the protein of the invention, can be used for the further screening of an agonists or antagonists of the said receptor.

Once the ligand binding to the protein of the invention, the receptor of the protein of the invention or the cells expressing the receptor are obtained by screening, it is possible to screen a compound that binds to the ligand and receptor. Also it is possible to screen a compound that can inhibit both bindings (agonists or antagonists of the receptor, for example) by utilizing the binding activities.

When the protein of the invention is a receptor, the screening method comprises the steps of (a) contacting the protein of the invention or cells expressing the protein of the invention with the ligand, in the presence of a test sample, (b) detecting the binding activity between said protein or cells expressing said protein and the ligand, and (c) selecting a compound that reduces said binding activity when compared to the activity in the absence of the test sample. Furthermore, when the protein of the invention is a ligand, the screening method comprises the steps of (a) contacting the protein of the invention with its receptor or cells expressing the receptor in the presence of samples, (b) detecting the binding activity between the protein and its receptor or the cells expressing the receptor, and (c) selecting a compound that can potentially reduce the binding activity compared to the activity in the absence of the sample.

Samples to screen include cell extracts, expressed products from a gene library, synthesized low molecular compound, synthesized peptide, and natural compounds, for example, but are not construed to be listed here. A compound that is isolated by the above screening using a binding activity of the protein of the invention can also be used as a sample.

A compound isolated by the screening may be a candidate to be an agonist or an antagonist of the receptor of the protein. By utilizing an assay that monitors a change in the intracellular signaling such as phosphorylation that results from reduction of the binding between the protein and its receptor, it is possible to identify whether the obtained compound is an agonist or antagonist of the receptor. Also, the compound may be a candidate of a molecule that can inhibit the interaction between the protein and its associated proteins (including a receptor) in vivo. Such compounds can be used for developing drugs for precaution or cures of a disease with which the protein is associated.

Secretory proteins may regulate cellular conditions such as growth and differentiation. It is possible to find out a novel factor that regulates cellular conditions by adding the secretory protein of the invention to a certain kind of cell, and performing a screening by utilizing the cellular changes in growth or differentiation, or activation of a particular gene.

The screening can be performed, for example, as follows. First, the protein of the invention is expressed and purified in a recombinant form. Then, the purified protein is added to a various kind of cell lines or primary cultured cells, and the change in the cell growth and differentiation is monitored. The induction of a particular gene that is known to be involved in a certain cellular change is detected with the amounts of mRNA and protein. Alternatively, the amount of an intracellular molecule (low molecular compounds, etc.) that is changed by the function of a gene product (protein) that is known to be functioning in a certain cellular change is used for the detection.

Once the screening reveals that the protein of the invention can regulate cellular conditions or the functions, it is possible to apply the protein as a pharrnaceutical and diagnostic medicine for associated diseases by itself or by altering a part of it into an appropriate composition.

As is above described for membrane proteins, the secretory protein provided by the invention may be used to explore a novel ligand-receptor interaction using a screening based on the binding activity to a known ligand or receptor. A similar method can be used to identify an agonist or antagonist. The resulting compounds obtained by the methods can be a candidate of a compound that can inhibit the interaction between the protein of the invention and an interacting molecule (including a receptor). The compounds may be able to use as a preventive, therapeutic, and diagnostic medicine for the diseases, in which the protein may play a certain role.

If the protein or gene of the invention is associated with diseases, it is possible to screen a gene or compound that can regulate its expression and/or activity either directly or indirectly by utilizing the protein of the present invention.
For example, the protein of the invention is expressed and purified as a recombinant protein. Then, the protein or gene that interacts with the protein of the invention is purified, and screened based on the binding. Alternatively, the screening can be performed by adding with a compound of a candidate of the inhibitor added in advance and monitoring the change of binding activity. The compound obtained by the screening can be used for developing pharmaceutical and diagnostic medicines for the diseases with which the protein of the present invention is associated. Similarly, if the regulatory factor obtained by the screening is a protein, the protein itself can be used as a pharmaceutical, and if there is a compound that affects the original expression level and/or activity of the protein, it also can be used for the same purpose.

If the secrete or membrane protein of the present invention has an enzymatic activity, it is possible to identify the activity by adding a compound to the protein of the present invention under an appropriate condition, and monitoring the change of the compound. It is also possible to screen a compound that inhibits the activity of the protein of the invention by utilizing the activity as an index.

In a screening given as an example, the protein of the invention is expressed and the recombinant protein is purified. Then, compounds are contacted with the purified protein, and the amount of the compound and the reaction products is examined. Alternatively, compounds that are candidates of an inhibitor are pretreated, then a compound (substrate) that can react with the purified protein is added, and the amount of the substrate and the reaction products is examined.

The compounds obtained in the screening may be used as a medicine for diseases with which the protein of the invention is associated. Also they can be applied for tests that examine whether the protein of the invention functions normally *in vivo.*

Whether the secretory or membrane protein of the present invention is a novel protein associated with diseases or not is determined in another method than described above, by obtaining a specific antibody against the protein of the invention, and examining the relationship between the expression or activity of the protein and a certain disease. In an alternative way, it may be analyzed referred to the methods in "Molecular Diagnosis of Genetic Diseases" (Elles R. edit, (1996) in the series of "Method in Molecular Biology" (Humana Press).

The secrete or membrane protein of the present invention can be prepared as a recombinant protein or a natural protein. For example, a recombinant protein can be prepared by introducing a vector containing a DNA insert encoding the protein of the invention into an appropriate host cell, and purifying the expressed products from the transformant, as described below. On the other hand, a natural protein can be prepared , for example, by utilizing an affinity column which is bound with the antibody against the protein of the invention, as described below ("Current Protocols in Molecular Biology" Ausubel et al. edit. (1987) John Wily & Sons, Section 16.1-16.19). The antibody used in the preparation of an affinity column can be a monoclonal antibody or polyclonal antibody. Alternatively, it is possible to prepare the protein of the invention by in vitro translation (See "On the fidelity of mRNA translation in the nuclease-treated rabbit reticulocyte lysate system." Dasso M.C., and Jackson R.J. (1989) Nucleic Acids Res. 17:3129-3144).

Proteins functionally equivalent to the proteins of the present invention can be prepared based on the activities, which were clarified in the above-mentioned manner, of the proteins of the present invention. Using the biological activity possessed by the protein of the invention as an index, it is possible to verify whether or not a particular protein is functionally equivalent to the protein of the invention by examining whether or not the protein has said activity.

Proteins functionally equivalent to the proteins of the present invention can be prepared by those skilled in the art, for example, by using a method for introducing mutations into an amino acid sequence of a protein (for example, site-directed mutagenesis (Current Protocols in Molecular Biology, edit, Ausubel et al., (1987) John Wiley & Sons, Section 8.1-8.5). Besides, such proteins can be generated by spontaneous mutations. The present invention comprises the proteins having one or more amino acid substitutions, deletions, insertions and/or additions in the amino acid sequences of the proteins of the present invention (Table 1), as far as the proteins have the equivalent functions to those of the proteins identified in the present Examples described later.

There are no limitations in the number and sites of amino acid mutations, as far as the proteins maintain the functions thereof. The number of mutations is typically 30% or less, or 20% or less, or 10% or less, preferably within 5% or less, or 3% or less of the total amino acids, more preferably within 2% or less or 1 % or less of the total amino acids. From the viewpoint of maintaining the protein function, it is preferable that a substituted amino has a similar property to that of the original amino acid. For example, Ala, Val, Leu, Ile, Pro, Met, Phe and Trp are assumed to have similar properties to one another because they are all classified into a group of non-polar amino acids. Similarly, substitution can be performed among non-charged amino acid such as Gly, Ser, Thr, Cys, Tyr, Asn, and Gln, acidic amino acids such as Asp and Glu, and basic amino acids such as Lys, Arg, and His.

In addition, proteins functionally equivalent to the proteins of the present invention can be isolated by using techniques of hybridization or gene amplification known to those skilled in the art. Specifically, using the hybridization technique (Current Protocols in Molecular Biology, edit, Ausubel et al., (1987) John Wiley & Sons, Section 6.3-6.4)), those skilled in the art can usually isolate a DNA highly homologous to the DNA encoding the protein identified in the present Example based on the identified nucleotide sequence (Table 1) or a portion thereof and obtain the functionally equivalent protein from the isolated DNA. The present invention include proteins encoded by the DNAs hybridizing with the DNAs encoding the proteins identified in the present Example, as far as the proteins are functionally equivalent to the proteins identified in the present Example. Organisms from which the functionally equivalent proteins are isolated are illustrated by vertebrates such as human, mouse, rat, rabbit, pig and bovine, but are not limited to these animals.

Washing conditions of hybridization for the isolation of DNAs encoding the functionally equivalent proteins are usually "1 × SSC, 0.1% SDS, 37°C"; more stringent conditions are "0.5 × SSC, 0.1% SDS, 42°C"; and still more stringent conditions are "0.1 × SSC, 0.1% SDS, 65°C". Alternatively, the following conditions can be given as hybridization conditions of the present invention. Namely, conditions in which the hybridization is done at "6 × SSC, 40% Formamide, 25°C", and the washing at "1 × SSC, 55°C" can be given. More preferable conditions are those in which the hybridization is done at "6 × SSC, 40% Formamide, 37°C", and the washing at "0.2 × SSC, 55°C". Even more preferable are those in which the hybridization is done at "6 × SSC, 50% Formamide, 37°C", and the washing at "0.1 × SSC, 62°C". The more stringent the conditions of hybridization are, the more frequently the DNAs highly homologous to the probe sequence are isolated. Therefore, it is preferable to conduct hybridization under stringent conditions. Examples of stringent conditions in the present invention are, washing conditions of "0.5 × SSC, 0.1% SDS, 42°C", or alternatively, hybridization conditions of "6 × SSC, 40% Formamide, 37°C", and the washing at "0.2 × SSC, 55°C". However, the above-mentioned combinations of SSC, SDS and temperature conditions are indicated just as examples. Those skilled in the art can select the hybridization conditions with similar stringency to those mentioned above by properly combining the above-mentioned or other factors (for example, probe concentration, probe length and duration of hybridization reaction) that determines the stringency of hybridization.

The amino acid sequences of proteins isolated by using the hybridization techniques usually exhibit high homology to those of the proteins of the present invention, which are shown in Table 1. The present invention encompasses a polynucleotide comprising a nucleotide sequence that has a high identity to the nucleotide sequence of claim 1 (a). Furthermore, the present invention encompasses a peptide, or protein comprising an amino acid sequence that has a high identity to the amino acid sequence encoded by the polynucleotide of claim 1(b). The term "high identity" indicates sequence identity of at least 40% or more; preferably 60% or more; and more preferably 70% or more. Alternatively, more preferable is identity of 90% or more, or 93% or more, or 95% or more, furthermore, 97% or more, or 99% or more. The identity can be determined by using the BLAST search algorithm.

With the gene amplification technique (PCR) (Current Protocols in Molecular Biology, edit, Ausubel et al., (1987) John Wiley & Sons, Section 6.3-6.4)) using primers designed based on the DNA sequence (Table 1) or a portion thereof identified in the present Example, it is possible to isolate a DNA fragment highly homologous to the DNA sequence or a portion thereof and to obtain functionally equivalent protein to a particular protein identified in the present Example based on the isolated DNA fragment.

The "percent identity" of two amino acid sequences or of two nucleic acids is determine using the algorithm of Karlin and Altschul (Proc. Natl. Acad. Sei. USA 87:2264-2268, 1990), modified as in Karlin and Altschul (Proc. Natl. Acad. Sei. USA 90:5873-5877, 1993). Such an algorithm is incorporated into the BLASTN and BLASTX programs of Altschul et al. (J. Mol. Biol.215:403-410, 1990), BLAST nucleotide searches are performed with the BLASTN program, score = 100, wordlength = 12. BLAST protein searches are performed with the BLASTX program, score = 50, wordlength = 3. When gaps exist between two sequences, Gapped BLAST is utilized as described in Altschul et al. (Nucleic Acids Res.25:3389-3402,1997). When utilizing BLAST and Gapped BLAST programs, the default parameters of the respective programs (e.g., BLASTX and BLASTN) are used. See http://www.ncbi.nlm.nih.gov.

The present invention also includes a partial peptide of the proteins of the invention. The partial peptide comprises a protein generated as a result that a signal peptide has been removed from a secretory protein. If the protein of the present invention has an activity as a receptor or a ligand, the partial peptide may function as a competitive inhibitor of the protein and may bind to the receptor (or ligand). In addition, the present invention comprises an antigen peptide for raising antibodies. For the peptides to be specific for the protein of the invention, the peptides comprise at least 7 amino acids, preferably 8 amino acids or more, more preferably 9 amino acids or more, and even more preferably 10 amino acids or more. The peptide can be used for preparing antibodies against the protein of the invention, or competitive inhibitors of them, and also screening for a receptor that binds to the protein of the invention. The partial peptides of the invention can be produced, for example, by genetic engineering methods, known methods for synthesizing peptides, or digesting the protein of the invention with an appropriate peptidase.

The present invention also relates to a polynucleotide encoding the protein of the invention. The polynucleotide of the invention can be provided in any form as far as it encodes the protein of the invention, and thus includes cDNA, genomic DNA, and chemically synthesized DNA, etc. The polynucleotide also includes a DNA comprising any nucleotide sequence that is obtained based on the degeneracy of the genetic code, as far as it encodes the protein of the invention. The polynucleotide of the invention can be isolated by the standard methods such as hybridization using a probe DNA comprising the nucleotide sequence set forth in odd SEQ ID NOs of SEQ ID NO: 1 to SEQ ID NO: 335, or the portions of them, or by PCR using primers that are synthesized based on the nucleotide sequence.

For example, all the clones provided by the present invention, which were isolated in the example mentioned below, (173 clones) are novel and full-length, and encode a secretory protein or membrane protein. All the cDNA clones provided by the invention are characterized as follows.

A full-length-enriched cDNA library that is obtained by the oligo-capping method, and selected based on the features of the 5'-end sequence: by the score in the ATGpr (or described as ATGpr1), which predicts the fullness ratio of the 5'-end, and by the PSORT, which predicts the presence of the signal sequence, as those containing the signal sequence in the 5'-end, or transmembrane region in the protein coding region. Furthermore, as a result of the homology search using the 5'-end sequences, the clones were found to be not identical to any of the known human mRNA (therefore to be novel).

The present invention also relates to a vector into which the polynucleotide of the invention is inserted. The vector of the invention is not limited as long as it contains the inserted polynucleotide stably. For example, if E. coli is used as a host, vectors such as pBluescript vector (Stratagene) are preferable as a cloning vector. To produce the protein of the invention, expression vectors are especially useful. Any expression vector can be used as far as it is capable of expressing the protein in vitro, in E. coli, in cultured cells, or in vivo. For example, pBEST vector (Promega) is preferable for in vitro expression, pET vector (Invitrogen) for E. coli, pME18S-FL3 vector (GenBank Accession No. AB009864) for cultured cells, and pME18S vector (Mol. Cell. Biol. (1988) 8: 466-472) for in vivo expression. To insert the polynucleotide of the invention, ligation utilizing restriction sites can be performed according to the standard method (Current Protocols in Molecular Biology (1987) Ausubel et al. edit, John Wily & Sons, Section 11.4-11.11).

The present invention also relates to a transformant carrying the polynucleotide or the vector of the invention. Any cell can be used as a host into which the vector of the invention is inserted, and various kinds of host cells can be used depending on the purposes. For strong expression of the protein in eukaryotic cells, COS cells or CHO cells can be used, for example.

Introduction of the vector into host cells can be performed, for example, by calcium phosphate precipitation method, electroporation method (Current Protocols in Molecular Biology (1987) Ausubel et al. edit, John Wily & Sons, Section 9.1-9.9), lipofectamine method (GIBCO-BRL), or microinjection method, etc.

The present invention also relates to a oligonucleotide having a length of at least 15 nucleotides, comprising a nucleotide sequence that is complementary to a polynucleotide comprising the nucleotide sequence set forth in odd SEQ ID NOs of SEQ ID NO: 1 to SEQ ID NO: 335, or its complementary strand. The oligonucleotide of the present invention hybridizes with a polynucleotide of odd SEQ ID NOs of SEQ ID NO: 1 to SEQ ID NO: 335 encoding the protein of the invention, or its complementary strand, under the standard conditions for hybridization, or preferably under stringent conditions, and in principle does not preferably hybridize with DNA encoding other proteins. Such oligonucleotide can be used as a probe for isolation and detection of the polynucleotide of the invention, and as a primer for amplifying the polynucleotide of the present invention. As a primer, the DNA usually has a length of 15-100 bp, preferably 15-50 bp, and more preferably has a length of 15-35 bp. As a probe, the DNA contains the entire sequence of the DNA of the invention, or at least the portion of it, and has a length of at least 15 bp, preferably 30 bp or more, and more preferably 50 bp or more.

Any sequence shown in SEQ ID NOs: 370-540 and that shown in SEQ ID NOs: 541-679 can be chosen as the nucleotide sequence comprising the 5'-end primer and the 3'-end primer, respectively, to synthesize the full-length cDNAs of the present invention. Although, among these nucleotide sequences, some nucleotide sequences have already been known as EST sequences, the primers designed based on the present invention is novel in that they make it possible to synthesize full-length cDNA. The known EST sequences do not serve to design such primers because the EST sequences lack the crucial information about the location thereof within the corresponding cDNAs.

Each of the full-length cDNAs of the present inventions can be synthesized by PCR (Current Protocols in Molecular Biology, ed., Ausubel et al., (1987) John Wiley & Sons, Section 6.1-6.4) using a pair of primers selected from the 5'-end sequences and the 3'-end sequences or using a primer pair consisting of a primer selected from the 5'-end sequences and a primer with oligo(dT) sequence complementary to the poly(A) sequence.

Specifically, PCR can be performed using an oligonucleotide that has 15 nucleotides longer, and specifically hybridizes with the complementary strand of the polynucleotide that contains the nucleotide sequence selected from the 5'-end sequences shown in Table 342 (SEQ ID NO: 370-540), and an oligo-dT primer as a 5'-, and 3'-primer, respectively. The length of the primers is usually 15-100 bp, and favorably between 15-35 bp. In case of LA PCR, which is described below, the primer length of 25-35 bp may provide a good result.
A method to design a primer that enables a specific amplification based on the given nucleotide sequence is known to those skilled in the art (Current Protocols in Molecular Biology, Ausubel et al. edit, (1987) John Wiley & Sons, Section 6.1-6.4). In designing a primer based on the 5'-end sequence, the primer is designed so as that, in principle, the amplification products will include the translation start site. Accordingly, in case that a given 5'-end nucleotide sequence is the 5'- untranslated region (5'UTR), any part of the sequence can be used as a 5'-primer as far as the specificity toward the target cDNA is insured. The translation start site can be predicted using a known method such as the ATGpr as described below.

When synthesizing a full-length cDNA, the target nucleotide sequence to be amplified can extend to several thousand bp in some cDNA. However, it is possible to amplify such a long nucleotides by using such as LA PCR (Long and Accurate PCR). It is advantageous to use LA PCR when synthesizing long DNA. In LA PCR, in which a special DNA polymerase having 3'→5' exonuclease activity is used, misincorporated nucleotides can be removed. Accordingly, accurate synthesis of the complementary strand can be achieved even with a long nucleotide sequence. By using LA PCR, it is reported that amplification of a nucleotide with 20 kb longer can be achieved under desirable condition (Takeshi Hayashi (1996) Jikken-Igaku Bessatsu, "Advanced Technologies in PCR" Youdo-sha).

A template DNA for synthesizing the cDNA of the present invention can be obtained by using cDNA libraries that are prepared by various methods. The full-length cDNA clones obtained here are those with high fullness ratio, which were obtained using a combination of (1) a method to prepare a full-length-enriched cDNA library using the oligo-capping method, and (2) an estimation system for fullness using the 5'-end sequence (selection based on the estimation by the ATGpr after removing clones that are non-full-length compared to the ESTs). However, it is possible to easily obtain a full-length cDNA by using the primers that are provided by the present invention, not by the above described specialized method.

The problem with the cDNA libraries prepared by the known methods or commercially available is that mRNA contained in the libraries has very low fullness ratio. Thus, it is difficult to screen full-length cDNA clone directly from the library using ordinary cloning methods. The present invention has revealed a primer that is capable of synthesizing a full-length cDNA. If provided with primers, it is possible to synthesize a target full-length cDNA by using enzymatic reactions such as PCR. In particular, a full-length-enriched cDNA library, synthesized by methods such as oligo-capping, is desirable to synthesize a full-length cDNA with more reliability.

Transcriptional regulatory regions including promoters in the genome can be isolated by utilizing the 5'-end sequences of the full-length cDNA clones of the present invention. The rough draft (slightly inaccurate sequencing result obtained in the analysis of human genome) covering 90% or more of the entire human genome is expected to be achieved in the spring of 2000, and the entire analysis of human genome sequence is expected to be completed by 2003. Because of the presence of long introns, it is hard to determine the transcription initiation sites in human genome by using analytical software. The utilization of the 5'-end sequences of the full-length cDNA sequences of the present invention makes it easy to isolate promoter-containing genomic regions that are located upstream of transcription initiation sites and are involved in mRNA transcription regulation. This is because the mRNA transcription initiation sites in the genome can be identified easily based on the 5'-end sequences of the full-length cDNAs.

The polynucleotide of the present invention can be used for examination and diagnosis of the abnormality of the protein of the invention. For example, it is possible to examine the abnormal expression of the gene encoding the protein using the polynucleotide of the invention as a probe for Northern hybridization or as a primer for RT-PCR. Also, the polynucleotide of the invention can be used as a primer for polymerase chain reaction (PCR) such as the genomic DNA-PCR, and RT-PCR to amplify the polynucleotide encoding the protein of the invention, or the regulatory region of the expression, with which it is possible to examine and diagnose the abnormality of the sequence by RFLP analysis, SSCP, and direct sequencing, etc.

Furthermore, the "polynucleotide having a length of at least 15 nucleotides, comprising a nucleotide sequence that is complementary to a polynucleotide comprising the nucleotide sequence set forth in odd SEQ ID NOs of SEQ ID NO: 1 to SEQ ID NO: 335, or its complementary strand" includes an antisense polynucleotide for suppressing the expression of the protein of the invention. To exert the antisense effect, the antisense polynucleotide has a length of at least 15 bp or more, for example, 50 bp or more, preferably 100 bp or more, and more preferably 500 bp or more, and has a length of usually 3000 bp or less and preferably 2000 bp or less. The antisense DNA can be used in the gene therapy of the diseases that are caused by the abnormality of the protein of the invention (abnormal function or abnormal expression). Said antisense DNA can be prepared, for example, by the phosphorothioate method ("Physicochemical properties of phosphorothioate oligodeoxynucleotides." Stein (1988) Nucleic Acids Res. 16: 3209-3221) based on the nucleotide sequence of the DNA encoding the protein (for example, the DNA set forth in odd SEQ ID NOs of SEQ ID NO: 1 to SEQ ID NO: 335).

The polynucleotide or antisense DNA of the present invention can be used in gene therapy, for example, by administrating it into a patient by the in vivo or ex vivo method with virus vectors such as retrovirus vectors, adenovirus vectors, and adeno-associated virus vectors, or non-virus vectors such as liposome.

The present invention also relates to antibodies that bind to the protein of the invention. There are no limitations in the form of the antibodies of the invention. They include polyclonal antibodies, monoclonal antibodies, or their portions that can bind to the antigen. They also include antibodies of all classes. Furthermore, special antibodies such as humanized antibodies are also included.

The polyclonal antibody of the invention can be obtained according to the standard method by synthesizing an oligopeptide corresponding to the amino acid sequence and immunizing rabbits with the peptide (Current Protocols in Molecular Biology (1987) Ausubel et al. edit, John Wily & Sons, Section 11.12-11.13). The monoclonal antibody of the invention can be obtained according to the standard method by purifying the protein expressed in E. coli, immunizing mice with the protein, and producing a hybridoma cell by fusing the spleen cells and myeloma cells (Current Protocols in Molecular Biology (1987) Ausubel et al. edit, John Wily & Sons, Section 11.4-11.11).

The antibody binding to the protein of the present invention can be used for purification of the protein of the invention, and also for detection and/or diagnosis of the abnormalities of the expression and structure of the protein. Specifically, proteins can be extracted, for example, from tissues, blood, or cells, and the protein of the invention is detected by Western blotting, immunoprecipitation, or ELISA, etc. for the above purpose.

Furthermore, the antibody binding to the protein of the present invention can be utilized for treating the diseases that associates with the protein of the invention. If the antibodies are used for treating patients, human antibodies or humanized antibodies are preferable in terms of their low antigenicity. The human antibodies can be prepared by immunizing a mouse whose immune system is replaced with that of human ("Functional transplant of megabase human immunoglobulin loci recapitulates human antibody response in mice" Mendez M.J. et al. (1997) Nat. Genet. 15:146-156, for a reference). The humanized antibodies can be prepared by recombination of the hypervariable region of a monoclonal antibody (Methods in Enzymology (1991) 203: 99-121).

The present invention further relates to databases comprising at least a sequence of polynucleotides and/or protein, or a medium recorded in such databases, selected from the sequence data of the nucleotide and/or the amino acids indicated in Table 1. The term "database" means a set of accumulated information as machine-searchable and readable information of nucleotide sequence. The databases of the present invention comprise at least one of the novel nucleotide sequences of polynucleotides provided by the present invention. The databases of the present invention can consist of only the sequence data of the novel polynucleotides provided by the present invention or can comprise other information on nucleotide sequences of known full-length CDNAs or ESTs. The databases of the present invention can be comprised of not only the information on the nucleotide sequences but also the information on the gene functions revealed by the present invention. Additional information such as names of DNA clones carrying the full-length cDNAs can be recorded or linked together with the sequence data in the databases.

The database of the present invention is useful for gaining complete gene sequence information from partial sequence information of a gene of interest. The database of the present invention comprises nucleotide sequence information of full-length cDNAs. Consequently, by comparing the information in this database with the nucleotide sequence of a partial gene fragment yielded by differential display method or subtraction method, the information on the full-length nucleotide sequence of interest can be gained from the sequence of the partial fragment as a starting clue.

The sequence information of the full-length cDNAs constituting the database of the present invention contains not only the information on the complete sequences but also, extra information on expression frequency of the genes as well as homology of the genes to known genes and known proteins. Thus the extra information facilitates rapid functional analyses of partial gene fragments. Further, the information on human genes is accumulated in the database of the present invention, and therefore, the database is useful for isolating a human homologue of a gene originating from other species. The human homologue can be isolated based on the nucleotide sequence of the gene from the original species.

At present, information on a wide variety of gene fragments can be obtained by differential display method and subtraction method. In general, these gene fragments are utilized as tools for isolating the full-length sequences thereof. When the gene fragment corresponds to an already-known gene, the full-length sequence is easily obtained by comparing the partial sequence with the information in known databases. However, when there exists no information corresponding to the partial sequence of interest in the known databases, cDNA cloning should be carried out for the full-length CDNA. It is often difficult to obtain the full-length nucleotide sequence using the partial sequence information as an initial clue. If the full-length of the gene is not available, the amino acid sequence of the protein encoded by the gene remains unidentified. Thus the database of the present invention can contribute to the identification of full-length cDNAs corresponding to gene fragments. which cannot be revealed by using databases of known genes. The present invention has provided 173 proteins that are novel secretory proteins or membrane proteins, and full-length cDNA clones encoding the proteins. It has great significance to provide a novel full-length cDNA clone of humans, as only few a of which have been isolated. It was found that the secretory proteins and membrane proteins of the present invention are associated with many diseases. Those genes and proteins associated with diseases are useful for developing medicines as they can be used as a diagnostic marker, or a target for gene therapy or developing medicines that is capable of regulating their expression and activity. Especially, the cDNA clones encoding a secretory protein are extremely important for medicinal industry since the protein itself is expected to be effective as a medicine, and also the gene may have potential to be associated with many diseases. Moreover, those proteins such as membrane proteins and the genes encoding the proteins may be used as a disease marker. These cDNA clones are also important for medicinal industry as they may be effective for treating diseases through the regulation of the expression and activity of their encoded proteins.

The invention is illustrated more specifically with reference to the following examples, but is not to be construed as being limited thereto.

### EXAMPLE 1

### Construction of a cDNA library by the oligo-capping method.

The NT-2 neuron progenitor cells (Stratagene), a teratocarcinoma cell line from human embryo testis, which can differentiate into neurons by treatment with retinoic acid were used. The NT-2 cells were cultured according to the manufacturer's instructions as follows.
(1) NT-2 cells were cultured without induction by retinoic acid treatment (NT2RM1).
(2) After cultured, NT-2 cells were induced by adding retinoic acid, and then were cultured for 48 hours (NT2RP1).
(3) After cultured, NT-2 cells were induced by adding retinoic acid, and then were cultured for 2 weeks (NT2RP2).

The cells were harvested separately, from which mRNA was extracted by the method described in the literature (Molecular Cloning 2nd edition. Sambrook J., Fritsch, E.F., and Maniatis T. (1989) Cold Spring Harbor Laboratory Press). Furthermore, poly(A)⁺RNA was purified from the mRNA using oligo-dT cellulose.
Similarly, human placenta tissues (PLACE1), human ovary cancer tissues (OVARC1), and human embryo-derived tissues that were enriched with brain (HEMBA1) were used to extract mRNA by the method described in the literature (Molecular Cloning 2nd edition. Sambrook J., Fritsch, E.F., and Maniatis T. (1989) Cold Spring Harbor Laboratory Press). Furthermore, poly(A)⁺RNA was purified from the mRNA using oligo-dT cellulose.

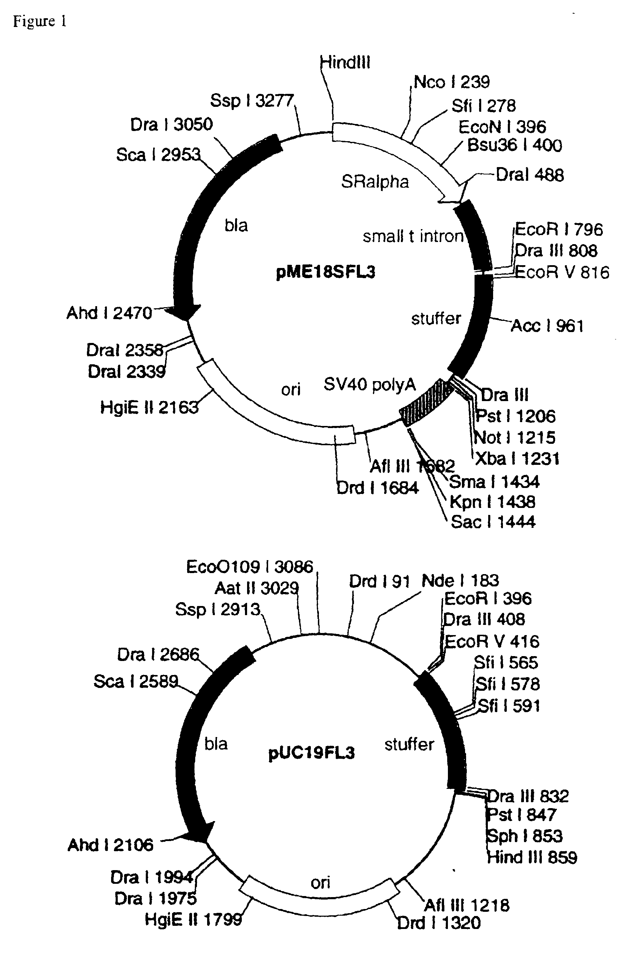

Each poly(A)⁺RNA was used to construct a cDNA library by the oligo-capping method (Maruyama M. and Sugano S. (1994) Gene 138: 171-174). Using the Oligo-cap linker (SEQ ID NO: 337) and the Oligo-dT primer (SEQ ID NO: 338), BAP (bacterial alkaline phosphatase) treatment, TAP (tobacco acid phosphatase) treatment, RNA ligation, the first strand cDNA synthesis, and removal of RNA were performed as described in the reference (Suzuki and Kanno (1996) Protein Nucleic acid and Enzyme. 41: 197-201; Suzuki Y. et al. (1997) Gene 200: 149-156). Next, 5'- and 3'-PCR primers (SEQ ID NO: 339, and 340, respectively) were used for performing PCR (polymerase chain reaction) to convert the cDNA into double stranded cDNA, which was then digested with SfiI. Then, the DraIII-cleaved pUC19FL3 vector (Figure 1; for NT2RM1, and NT2RP1), or the DraIII-cleaved pME18SFL3 (Figure 1) (GenBank AB009864, expression vector; for NT2RP2, NT2RP3, PLACE1, OVARC1, and HEMBA1) was used for cloning the cDNA in an unidirectional manner, and cDNA libraries were obtained. The clones having an insert cDNA with a length of 1 kb or less were discarded from NT2RM1, NT2RP1, NT2RP2, PLACE1, OVARC1, and HEMBA1, and the clones having an insert cDNA with a length of 2 kb or less were discarded from NT2RP3. Then, the nucleotide sequence of the 5'- and 3'- ends of the cDNA clones was analyzed with a DNA sequencer (ABI PRISM 377, PE Biosystems) after sequencing reactions were performed with the DNA sequencing reagents (Dye Terminator Cycle Sequencing FS Ready Reaction Kit, dRhodamine Terminator Cycle Sequencing FS Ready Reaction Kit, or BigDye Terminator Cycle Sequencing FS Ready Reaction Kit, from by PE Biosystems) according to the instructions.

The so analyzed 5'-end and 3'-end nucleotide sequences of the clones are shown in SEQ ID NOs: 370-540 and in SEQ ID NOs: 541-679, respectively. The SEQ IDs and the corresponding PSEC clones are as indicated in Table 342.

The cDNA libraries of NT2RP2 and HEMBA1 were constructed using eukaryotic expression vector pME18SFL3. The vector contains SRα promoter and SV40 small t intron in the upstream of the cloning site, and SV40 polyA added signal sequence site in the downstream. As the cloning site of pME18SFL3 has asymmetrical DraIII sites, and the ends of cDNA fragments contain SfiI sites complementary to the DraIII sites, the cloned cDNA fragments can be inserted into the downstream of the SRα promoter unidirectionally. Therefore, clones containing full-length cDNA can be expressed transiently by introducing the obtained plasmid directly into COS cells. Thus, the clones can be analyzed very easily in terms of the proteins that are the gene products of the clones, or in terms of the biological activities of the proteins.

The fullness ratio at the 5'-end sequences of the cDNA clones in the libraries constructed by the oligo-capping method was determined as follows. Of all the clones whose 5'-end sequences were found in those of known human mRNA in the public database, a clone was judged to be "full-length", if it had a longer 5'-end sequence than that of the known human mRNA, or, even though the 5'-end sequence was shorter, it it contained the translation initiation codon. A clone that did not contain the translation initiation codon was judged to be "non-full-length". The fullness ratio ((the number of full-length clones)/(the number of full-length and non-full-length clones)) at the 5'-end of the cDNA clones from each library was determined by comparing with the known human mRNA (NT2RM1: 69%; NT2RP1: 75%; NT2RP2: 62%; NT2RP3: 61%; PLACE1: 68%; OVARC1: 59%; and HEMBA1: 53%). The result indicates that the fullness ratio at the 5'-end sequence was extremely high.

The relationship between the cDNA libraries and the clones is shown below.
- NT2RM1 :: PSEC0001-PSEC0017
- NT2RP1 :: PSEC0019-PSEC0047
- NT2RP2 :: PSEC0048-PSEC0085,
PSEC0092-PSEC0109,
PSEC0111-PSEC0113, PSEC0173
- NT2RP3 :: PSEC0241-PSEC0265
- PLACE1 :: PSEC0086-PSEC0090, PSEC0110,
PSEC0117-PSEC0172
- OVARC1 :: PSEC0178-PSEC0183, PSEC0239-PSEC0240
- HEMBA1 :: PSEC0190-PSEC0237

### EXAMPLE 2

### Estimation of the fullness ratio at the 5'-end of the cDNA by the ATGpr and the ESTiMateFL.

The ATGpr, developed by Salamov A.A., Nishikawa T., and Swindells M.B. in the Helix Research Institute, is a program for prediction of the translation initiation codon based on the characteristics of the sequences in the vicinity of the ATG codon [A. A. Salamov, T. Nishikawa, M. B. Swindells, Bioinformatics, 14: 384-390 (1998); http://www.hri.co.jp/atgpr/]. The results are shown with expectations (also described as ATGpr1 below) that an ATG is a true initiation codon (0.05-0.94). When the program was applied to the 5'-end sequences of the clones from the cDNA library that was obtained by the oligo-capping method and that had 65% fullness ratio, the sensitivity and specificity of estimation of a full-length clone (clone containing the N-terminal end of ORF) were improved to 82-83% by selecting only clones having the ATGpr1 score 0.6 or higher. Furthermore, the 17,365 clones in which the 5'-end sequence is identical to a known human mRNA and which were cloned from the human cDNA libraries constructed by the oligo-capping method, were estimated by the program. Briefly, the maximal ATGpr1 score of the clones was determined, and then their 5'-end sequence was compared with the known human mRNA to estimate whether the clone is full-length or not. The result was summarized in Table 2. It is indicated that the method for the selection through the combination of the ATGpr and the clones isolated from the human cDNA library that was constructed by the oligo-capping method was very efficient.

**Table 2**

| maximal ATGpr1 Score | number of full-length and not-full-length clones | number of full-length clones | fullness ratio |
|---|---|---|---|
| >=0.70 | 10,226 | 8,428 | 82.4% |
| >=0.50 | 12,171 | 9,422 | 77.4% |
| >=0.30 | 14,102 | 10,054 | 71.3% |
| >=0.17 | 15,647 | 10,385 | 66.4% |
| >=0.05 | 17,365 | 10,608 | 61.1% |
| * number of full-length clones, the number of the clones which contain the N-terminus of the ORF; the number of not-full-length clones, number of the clones which does not contain the N-terminus of the ORF; fullness ratio, the resulting number of (the number of full-length clones)/(the number of full-length and not-full-length clones) | | | |

The ESTiMateFL, developed by Nishikawa and Ota in the Helix Research Institute, is a method for the selection of a clone with high fullness ratio by comparing with the 5'-end or 3'-end sequences of ESTs in the public database.

By the method, a cDNA clone is judged presumably not to be full-length if there are any ESTs that have longer 5'-end or 3'-end sequences than the clone. The method is systematized for high throughput analysis. A clone is judged to be full-length if the clone has a longer 5'-end sequence than ESTs in the public database. Even if a clone has a shorter 5'-end, the clone is judged to be full-length if the difference in length is within 50 bases, and otherwise judged not to be full-length, for convenience. The precision of the prediction by comparing cDNA clones with ESTs is improved with increasing number of ESTs to be compared. However, when only a limited number of ESTs are available, the reliability becomes low. Thus, the method is effective in excluding clones with high probability of being not-full-length, from the cDNA clones that is synthesized by the oligo-capping method and that have the 5'-end sequences with about 60 % fullness ratio. In particular, the ESTiMateFL is efficiently used to estimate the fullness ratio at the 3'-end sequence of cDNA of a human unknown mRNA that has a significant number of ESTs in the public database.

The results were summarized in Tables 3 and 4. It was confirmed that, in estimating the fullness ratio at the 5'-end sequence of the clones of the human cDNA library that was constructed by the oligo-capping method, the fullness ratio was improved even for the clones having low score in the ATGpr by combining the ATGpr and ESTiMateFL. The result was applied to the estimation of the fullness ratio at the 5'-end sequence of the clones whose complete cDNA sequences were determined. The number of full-length clones, the number of not-full-length clones, and the fullness ratio indicate the number of the clones which contain the N-terminus of the ORF, the number of the clones which does not contain the N-terminus of the ORF, and the resulting number of (the number of full-length clones)/(the number of full-length and not-full-length clones), respectively.

**Table 3**

| | | | |
|---|---|---|---|
| The fullness ratio at the 5'-end sequence of the cDNA clones that were judged to be full-length by comparing the ORF of the known human mRNA and that were obtained by the oligo-capping method, wherein the ratio was evaluated by comparing the cDNA clones with ESTs. | | | |

| maximal ATGpr1 Score | number of full-length clones | number of not-full-length clones | fullness ratio |
|---|---|---|---|
| >=0.30 | 8,646 | 907 | 90.5% |
| >=0.17 | 10,158 | 1,150 | 89.8% |
| >=0.05 | 15,351 | 2,728 | 84.9% |

**Table 4**

| | | | |
|---|---|---|---|
| The fullness ratio at the 5'-end sequence of the cDNA clones that were judged to be not-full-length by comparing the ORF of the known human mRNA and that were obtained by the oligo-capping method, wherein the ratio was evaluated by comparing the cDNA clones with ESTs. | | | |

| maximal ATGpr1 Score | number of full-length clones | number of not-full-length clones | fullness ratio |
|---|---|---|---|
| >=0.30 | 1,271 | 2,156 | 37.1% |
| >=0.17 | 1,678 | 2,907 | 36.6% |
| >=0.05 | 2,512 | 4,529 | 35.7% |

### EXAMPLE 3

### Selection of the clones containing the signal sequence and the full-length-enriched clones.

From the clones in each library constructed by the oligo-capping method, those clones predicted to contain the signal sequence (most likely to be a secretory protein or membrane protein) were specifically selected by analyzing the amino acid sequence that are predicted by all the ATG codons within the 5'-end sequence, for the presence of the signal peptide, which is characteristic in the N-terminus of many secretory proteins, by using the PSORT, developed by Nakai and Kanehisa, which predicts the localization of a protein.

PSEC0001-PSEC0066 were not selected by the ATGpr score of the 5'-end sequence (one pass sequencing), but selected by the presence of both the signal sequence (analyzed by the PSORT), and the ORF (Open reading frame; a region translated to be amino acids) in the 5'-end sequence. PSEC0068-PSEC0265 were selected as those having the maximal ATGpr1 score of the 5'-end sequence (one pass sequencing) 0.7 or higher, in which both the signal sequence (analyzed by the PSORT) and the ORF exist in the 5'-end sequence.

### EXAMPLE 4

### Analysis of the complete cDNA sequence and classification by categories.

For the 173 clones selected in Example 3, the nucleotide sequences of the full-length cDNA and the deduced ammo acid sequences were determined. The nucleotide sequences were finally determined by overlapping completely the partial nucleotide sequences determined by the following three methods. The amino acid sequences were deduced from the determined cDNA sequences. The results were shown in SEQUENCE LISTING (Only the results of the 173 clones that were classified into a secretory protein or membrane protein were shown).
(1) Long-read sequencing from both ends of the cDNA inserts using a Licor DNA sequencer (After sequence reactions were performed according to the manual for the Licor sequencer (Aroka), DNA sequence was determined by the sequencer.)
(2) Nested sequencing by the Primer Island method which utilizes the in vitro transfer of AT2 transposon (Devine S.E., and Boeke J.D. (1994) Nucleic Acids Res. 22: 3765-3772) (After clones were obtained using a kit from PE Biosystems, sequence reactions were performed using the DNA sequencing reagents from the company, according to the manufacturer's instructions, and DNA sequence was determined using an ABI PRISM 377 sequencer.)
(3) Primer walking by the dideoxy terminator method using custom synthesized DNA primers (After sequence reactions were performed using the DNA sequencing reagents from PE Biosystems and custom synthesized DNA primers according to the manufacturer's instructions, DNA sequence was determined using an ABI PRISM 377 sequencer).

These sequences were subjected to the analysis by the ATGpr and PSORT and also to the BLAST search of the GenBank and SwissProt. As a result, most clones (152 clones out of 173) were predicted to be a secretory protein or membrane protein that contains a signal sequence in the N-terminus. Furthermore, those clones, in which a signal sequence was not found by the PSORT, (PSEC0027, PSEC0047, PSEC0066, nnnnnnnn, PSEC0069, PSEC0092, PSEC0103, PSEC0117, PSEC0142, PSEC0212, PSEC0239, PSEC0242, PSEC0251, PSEC0256, PSEC0006, PSEC0043, PSEC0058, PSEC0195, PSEC0206, and PSEC0211) were subjected to the analysis by the MEMSAT and SOSUI for the identity as a membrane protein (containing the transmembrane helix). As a result, 14 clones among the 20 clones were predicted to contain the transmembrane helix (PSEC0027, PSEC0047, PSEC0066, nnnnnnnn, PSEC0069, PSEC0092, PSEC0103, PSEC0117, PSEC0142, PSEC0212, PSEC0239, PSEC0242, PSEC0251, and PSEC0256). Thus, the clones were predicted to be a membrane protein. As a result of the homology search of the SwissProt, PSEC0195 and PSEC0206 were found to have relatively high homology with mouse plasma membrane adapter HA2/AP2 adaptin α C subunit, and human carboxypeptidase H precursor (prohormone processing carboxypeptidase) in the secretory granule, respectively.

The above results were shown in List 1, List 2, and List 3. Therein, the function of each cDNA clone (annotation) was shown as well. The categories of the 168 clones out of 173 clones were shown in the followings.
1. Clones that are predicted to be a full-length cDNA clone encoding a secretory protein or membrane protein (168 clones)
   (Most clones have the ATGpr1 score 0.5 or higher).
   1) Clones that are predicted to be a full-length cDNA clone encoding a secretory protein or membrane protein, in which a signal sequence is present in the N-terminus (152 clones, List 1). PSEC0001 PSEC0049 PSEC0085 PSEC0113
      nnnnnnnn PSEC0051 PSEC0086 PSEC0119
      PSEC0005 PSEC0052 PSEC0087 PSEC0120
      PSEC0007 PSEC0053 PSEC0088 PSEC0121
      PSEC0008 PSEC0055 PSEC0090 PSEC0124
      PSEC0012 PSEC0059 PSEC0094 PSEC0125
      PSEC0017 PSEC0061 PSEC0095 PSEC0126
      PSEC0019 PSEC0068 PSEC0098 PSEC0127
      PSEC0020 PSEC0070 PSEC0099 PSEC0128
      PSEC0021 PSEC0071 PSEC0100 PSEC0129
      PSEC0028 PSEC0072 PSEC0101 PSEC0130
      PSEC0029 PSEC0073 PSEC0104 PSEC0131
      PSEC0030 PSEC0074 PSEC0105 PSEC0133
      PSEC0031 PSEC0075 PSEC0106 PSEC0134
      PSEC0035 PSEC0076 PSEC0107 PSEC0135
      PSEC0038 PSEC0077 PSEC0108 PSEC0136
      PSEC0040 PSEC0079 PSEC0109 PSEC0137
      PSEC0041 PSEC0080 PSEC0110 PSEC0139
      PSEC0045 PSEC0081 PSEC0111 PSEC0143
      PSEC0048 PSEC0082 PSEC0112 PSEC0144
      nnnnnnnn PSEC0178 PSEC0216 PSEC0247
      PSEC0147 PSEC0181 PSEC0218 PSEC0248
      PSEC0149 PSEC0182 PSEC0220 PSEC0249
      PSEC0150 PSEC0183 PSEC0222 PSEC0250
      PSEC0151 PSEC0190 PSEC0223 PSEC0252
      PSEC0152 PSEC0191 PSEC0224 PSEC0253
      PSEC0158 PSEC0192 PSEC0226 PSEC0255
      PSEC0159 PSEC0197 PSEC0227 PSEC0258
      PSEC0161 PSEC0198 PSEC0228 PSEC0259
      PSEC0162 PSEC0199 PSEC0230 PSEC0260
      PSEC0163 PSEC0200 PSEC0232 PSEC0261
      PSEC0164 PSEC0203 PSEC0233 PSEC0263
      PSEC0165 PSEC0204 PSEC0235
      PSEC0167 PSEC0205 PSEC0236
      PSEC0168 PSEC0207 PSEC0240
      PSEC0169 PSEC0209 PSEC0241
      PSEC0170 PSEC0210 PSEC0243
      PSEC0171 PSEC0213 PSEC0244
      PSEC0172 PSEC0214 PSEC0245
      PSEC0173 PSEC0215 PSEC0246
      (Annotation 1)
      Clones that have the ATGpr1 score 0.5 or lower (PSEC0017, ATGpr1 0.33; PSEC0030, ATGpr1 0.26; PSEC0031, ATGpr1 0.20; PSEC0049, ATGpr1 0.35): These clones, in which data of the 5'-end sequence (one pass sequencing) was not sorted by the ATGpr, were selected as a clone having both the signal sequence and long ORF based on the data of the 5'-end sequence, and the sequence of their full-length cDNA clones was determined. All the clones have a signal sequence in the N-terminus. In addition, the above 4 clones except PSEC0049 have longer 5'-end compared to the corresponding EST. PSEC0049 has an ORF that has longer 5'-end than that of EST. Thus, these clones turned out to be full-length cDNA clones.
   2) Clones that are predicted to be a full-length cDNA encoding a secretory protein or membrane protein, in which the signal sequence is not present in the N-terminus, and predicted to be a membrane protein (14 clones, List 2).
      PSEC0027
      PSEC0047
      PSEC0066
      nnnnnnnn
      PSEC0069
      PSEC0092
      PSEC0103
      PSEC0117
      PSEC0142
      PSEC0212
      PSEC0239
      PSEC0242
      PSEC0251
      PSEC0256
      (Annotation 3)
         Clones that have the ATGpr1 score 0.5 or lower (PSEC0239, ATGpr1 0.18): PSEC0239 was selected as a clone having high ATGpr1 score of the 5'-end sequence (one pass sequencing), in which the signal sequence was predicted to be present. Although this clone was predicted to be without the signal sequence in the N-terminus according to the predicted ORF after complete sequencing, the clone was predicted to be a membrane protein (having the transmembrane helix) by the MEMSAT and SOSUI. In addition, the clone was found to contain a longer 5'-sequence than ESTs by comparing with them.
      (Annotation 4)
         PSEC0242 and PSEC0251: Both clones are classified into the cDNA encoding the polypeptide "containing a signal sequence in the N-terminus", if translation starts from their third ATG codon.
         PSEC0242: No.3 ATG, ATGpr1 0.82, SP-Yes, ORF 171-1343 391 aa, Signal peptide 24;
         PSEC0251: No.3 ATG, ATGpr1 0.77, SP-Yes, ORF 116-1256 380 aa, Signal peptide 28.
2. Clones that are predicted to be neither of a secretory protein or membrane protein by the PSORT, MEMSAT, and SOSUI, but predicted to be full-length by the ATGpr, which were isolated from the full-length-enriched human cDNA libraries constructed by the oligo-capping method (2 clones)
   (Both clones have the ATGpr score 0.5 or higher).
   PSEC0195, and PSEC0206.

According to the result of the homology search of the SwissProt, PSEC0195 and PSEC0206 were found to have relatively high homology with mouse plasma membrane adapter HA2/AP2 adaptin α C subunit, and human carboxypeptidase H precursor (prohormone processing carboxypeptidase) in the secretory granule, respectively. Thus, the proteins are classified into the category of "a secretory protein or membrane protein" (see List3).

### EXAMPLE 5

### Selection of clones predicted to have signal sequences

Specific selection was carried out for clones predicted to have signal sequences (having high probability of being secretory and/or membrane proteins) by testing the presence of a sequence predicted as a characteristic signal peptide found in amino-terminal sequences of many secretory proteins. The selection was performed by surveying all the possible amino acid sequences that are initiated with distinct ATG codons located in the 5'-end sequence and that are encoded by a cDNA isolated from each library prepared by oligo-capping method, by using a computer program, "PSORT" developed for predicting domain localization in a protein by Nakai and Kanehisa. Specifically, based on the 5'-end sequence data (one pass sequencing), the clones were selected under the conditions that the signal sequence (analyzed by PSORT) had a maximal ATGpr1 value of 0.7 or higher and the corresponding ORF was found in the 5'-end sequence.

The correspondence between the clones and the cDNA libraries is as follows:
NT2RP2: PSEC0078, PSEC0084
NT2RP3: PSEC0264, PSEC0265
HEMBA1: PSEC0237

### EXAMPLE 6

### Sequencing of the full-length cDNAs and categorization thereof

Nucleotide sequences were determined for the 5 full-length cDNAs selected in Example 5 by assembling the sequence data derived from both strands. Amino acid sequences were then deduced from the full-length nucleotide sequences. The sequences were subjected to the analyses with ATGpr and PSORT programs. Furthermore, databases such as GenBank and SwissProt were searched for the full-length sequences by BLAST. There were 4 clones (PSEC0084, PSEC0237, PSEC0264, and PSEC0265) that were predicted to encode secretory proteins having signal sequences at their N-termini. As for another clone (PSEC0078), no signal sequence was detected in the deduced amino acid sequence thereof by PSORT. By using MEMSAT and SOSUl programs, this clone was further analyzed to assess whether or not the protein encoded by this clone was a membrane protein (having a transmembrane helix). The result showed that a transmembrane helix was predicted to be present in this protein. In other words, the protein was presumed to be a membrane protein.

From the matching data obtained by BLAST analysis, matching data including information on proteins whose functions were relatively easy to be predicted were chosen to present them herein. Some clones were, however, selected simply because of the high homology in the matching data. These results are shown in List 1 and List 2 together with the annotation of the function of each cDNA clone. The categorization of the 5 clones is described below.

Results obtained by BLAST analysis are presented herein for the above-mentioned clones other than the 5 clones based on the same criterion as mentioned above for the selection.
Clones predicted to cover the full-length cDNA sequences and to encode secretory and/or membrane proteins (5 clones)
clones predicted to cover the full-length cDNA sequences and to encode secretory and/or membrane proteins with signal sequences at the N-terminal ends thereof (4 clones) (List 1) (ATGpr1 value is 0.5 or higher)
PSEC0084, PSEC0237, PSEC0264, PSEC0265
a clone predicted to cover the full-length cDNA sequence and to encode secretory and/or membrane protein without signal sequence at the N-terminal end thereof (1 clones) (List 2) PSEC0078
(Annotation) The ATGpr1 value was 0.24. This is a clone exhibiting high ATGpr1 value and selected as having a signal sequence in the prediction based on the 5'-end sequence data (one pass sequencing). However, based on the ORF deduced from the full-length sequence determined later, this clone has been finally judged not to have the signal sequence at the N-terminus thereof. Nonetheless, the clone has been predicted to encode a membrane protein (having a transmembrane helix) by MEMSAT and SOSUI analyses. In addition, in comparison with EST sequences, the cDNA sequence was not found to be 50 bp or more shorter than any EST sequence at their 5'-end, and therefore the clone was not judged to be a incomplete cDNA clone by using ESTs as criteria for the judgment.

### EXAMPLE 7

### Gene expression analysis with hybridization using high density DNA filter

Nylon membrane for DNA spotting was prepared according to the following procedure. E. coli was cultured in each well of a 96-well plate (in a LB medium at 37°C for 16 hours). A sample of each culture was suspended in 10 µl of sterile water in a well of a 96-well plate. The plate was heated at 100°C for 10 minutes. Then, the boiled samples were analyzed by PCR. PCR was performed in a 20 µl solution by using TaKaRa PCR Amplification Kit (Takara) according to the supplier's protocol. Primers used for the amplification of an insert cDNA in a plasmid were a pair of sequencing primers, ME761FW (5' tacggaagtgttacttctgc 3') and ME1250RV (5' tgtgggaggttttttctcta 3'), or a pair of primers, M13M4 (5' gttttcccagtcacgac 3') and M13RV (5' caggaaacagctatgac 3'). PCR was performed using a thermal cycler, GeneAmp System 9600 (PE Biosystems) at 95°C for 5 minutes; at 95°C for 10 seconds and at 68°C for 1 minute for 10 cycles; at 98°C for 20 seconds and at 60°C for 3 minutes for 20 cycles; and at 72°C for 10 minutes. After the PCR, the 20 µl reaction solution was loaded onto a 1% agarose gel and fractionated by electrophoresis. DNA on the gel was stained with ethidium bromide to confirm the amplification of cDNA. When cDNAs were not amplified by PCR, plasmids containing the corresponding insert cDNAs were prepared by the alkali-extraction method (J. Sambrook, E.F., Fritsh, & T. Maniatis, "Molecular Cloning, A laboratory manual/ 2nd edition, Cold Spring Harbor Laboratory Press, 1989).

Preparation of DNA array was carried out by the following procedure. A sample of a DNA solution was added in each well of a 384-well plate. DNA was spotted onto a nylon membrane (Boehringer) by using a 384-pin tool of Biomek 2000 Laboratory Automation System (Beckman-Coulter). Specifically, the 384-well plate containing the DNA was placed under the 384-pin tool. The independent 384 needles were simultaneously dipped into the DNA solution for DNA deposition. The needles were gently pressed onto a nylon membrane and the DNA deposited at the tips of needles was spotted onto the membrane. Denaturation of the spotted DNA and immobilization of the DNA on the nylon membrane were carried out according to standard methods (J. Sambrook, E.F., Fritsh, & T. Maniatis, "Molecular Cloning, A laboratory manual/ 2nd edition, Cold Spring Harbor Laboratory Press, 1989).

A probe for hybridization was radioisotope-labeled first strand cDNA. Synthesis of the first strand cDNA was performed by using Thermoscript™ RT-PCR System (GIBCO). Specifically, the first strand cDNA was synthesized by using 1.5 µg of mRNAs from various human tissues (Clontech), 1 µl of 50 µM Oligo(dT)20 and 50 µ Ci [α ³³P]dATP according to an attached protocol. Purification of a probe was carried out by using ProbeQuant™ G-50 micro column (Amersham-Pharmacia Biotech) according to an attached protocol. In the next step, 2 units of E. coli RNase H were added to the reaction mixture. The mixture was incubated at room temperature for 10 minutes, and then, 100 µg of human COT-1 DNA (GIBCO) was added thereto. The mixture was incubated at 97°C for 10 minutes and then was allowed to stand on ice to give hybridization probe.

Hybridization of the radioisotope-labeled probe to the DNA array was performed according to standard methods (J. Sambrook, E.F., Fritsh, & T. Maniatis, Molecular Cloning, A laboratory manual/ 2nd edition, Cold Spring Harbor Laboratory Press, 1989). The membrane was washed as follows: the nylon membrane was washed 3 times by incubating it in Washing solution 1 (2 × SSC, 1% SDS) at room temperature (about 26°C) for 20 minutes; then the membrane was washed 3 times by incubating it in Washing solution 2 (0.1 × SSC, 1% SDS) at 65°C for 20 minutes.

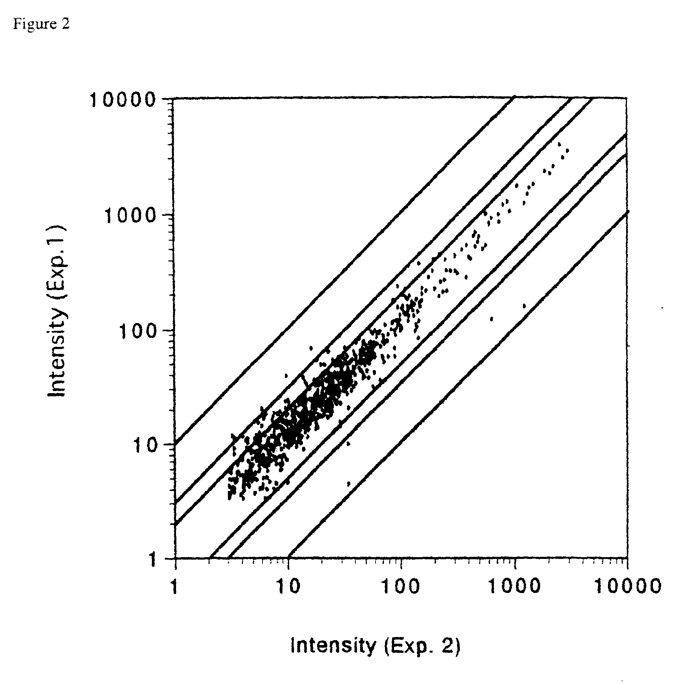

Autoradiography was performed by using an image plate for BAS2000 (Fuji Photo Film Co., Ltd.). Specifically, the nylon membrane with probe hybridized thereon was wrapped with a piece of Saran Wrap and brought into tight contact with the image plate on the light-sensitive surface. The membrane with the image plate was placed in an imaging cassette for radioisotope and allowed to stand in dark place for 4 hours. The radioactivity recorded on the image plate was analyzed by using BAS2000 (Fuji Photo Film Co., Ltd.). The activity was subjected to electronic conversion and recorded as an image file of autoradiogram. The signal intensity of each DNA spot was analyzed by using Visage High Density Grid Analysis Systems (Genomic Solutions Inc.). The signal intensity was converted into numerical data. The data were taken in duplicate. The reproducibility was assessed by comparing the signal intensities of the corresponding spots on the duplicated DNA filters that were hybridized to a single DNA probe (Figure 2). In 95% of entire spots, the ratio between the corresponding spots falls within a range of 2 or less, and the correlation coefficient is r=1.97. Thus, the reproducibility is satisfactory.

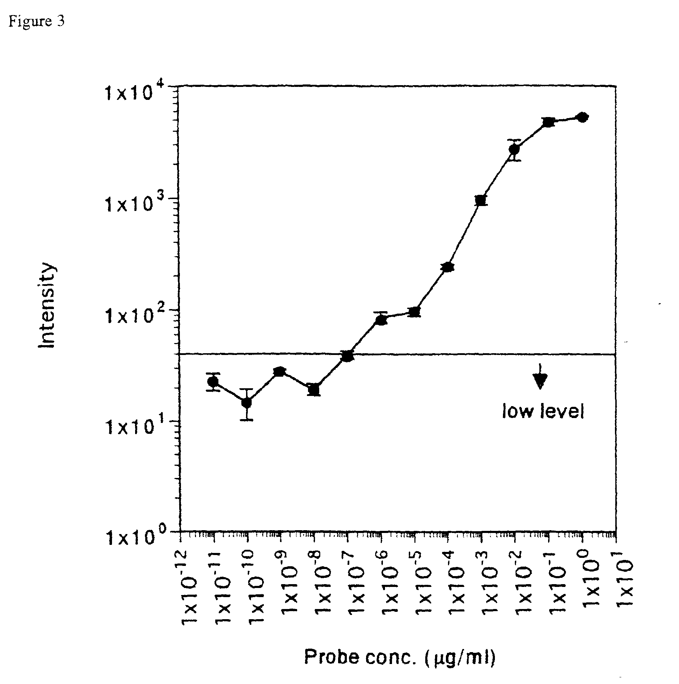

The detection sensitivity in gene expression analysis was estimated by examining increases in the signal intensity of probe concentration-dependent spot in hybridization using a probe complementary to the DNA spotted on the nylon membrane. DNA used was PLACE1008092 (the same as DNA deposited in GenBank under an Accession No. AF107253). The DNA array with DNA of PLACE1008092 was prepared according to the above-mentioned method. The probe used was prepared as follows: mRNA was synthesized in vitro from the clone, PLACE1008092. By using this mRNA as a template, radioisotope-labeled first strand cDNA was synthesized in the same manner as described above, and the cDNA was used as the probe. In order to synthesize mRNA from PLACE1008092 in vitro, a plasmid in which the 5' end of the cDNA PLACE 1008092 was ligated to the T7 promoter of pBluescript SK(-) was constructed. Specifically, the PLACE1008092 insert was cut out from pME18SFL3 carrying the cDNA at a DraIII site thereof by XhoI digestion. The resulting PLACE1008092 fragment was ligated to XhoI-predigested pBluescript SK(-) by using DNA ligation kit ver.2 (Takara). The in vitro mRNA synthesis from PLACE1008092 inserted into pBluescript SK(-) was carried out by using Ampliscribe™ T7 high yield transcription kit (Epicentre technologies). Hybridization and the analysis of signal intensity of each DNA spot were performed by the same methods as described above. When the probe concentration is 1 × 10⁷ µg/ml or less, there was no increase of signal intensity proportional to the probe concentration. Therefore, it was assumed to be difficult to compare the signals with one another in this concentration range. Thus, the spots with the intensity of 40 or less were uniformly taken as low level signals (Figure 3). Within a concentration of the probe ranging from 1 × 10⁷ µg/ml to 0.1 µg/ml, the signal was found to increase in a probe concentration-dependent manner. The detection limit represented as the ratio of the expression level of test mRNA to that of total mRNA in a sample was 1:100,000.

Tables 5-161 (also containing clones without description in Examples) show the expression of each cDNA in human normal tissues (heart, lung, pituitary gland, thymus, brain, kidney, liver and spleen). The expression levels are indicated with numerical values of 0-10,000. Genes that were expressed in at least a single tissue are indicated below by the corresponding clone names:
clone: HEMBA1000446, HEMBA1000675, HEMBA1001322, HEMBA1001552, HEMBA1001680, HEMBA1001879, HEMBA1002441, HEMBA1002706, HEMBA1002715, HEMBA1002913, HEMBA1002981, HEMBA1003280, HEMBA1003702, HEMBA1003764, HEMBA1004100, HEMBA1004633, HEMBA1005096, HEMBA1005452, HEMBA1005628, HEMBA1005833, HEMBA1006099, HEMBA1006391, HEMBA1006813, HEMBA1007104, HEMBA1007186, NT2RM1000558, NT2RP1000125, NT2RP1000279, NT2RP1000837, NT2RP1001023, NT2RP2000396, NT2RP2000428, NT2RP2000557, NT2RP2000601, NT2RP2000720, NT2RP2001087, NT2RP2001142, NT2RP2001270, NT2RP2001341, NT2RP2001499, NT2RP2001508, NT2RP2001768, NT2RP2002429, NT2RP2002695, NT2RP2002907, NT2RP2002927, NT2RP2002934, NT2RP2003050, NT2RP2003115, NT2RP2003227, NT2RP2003902, NT2RP2004130, NT2RP2004755, NT2RP2004795, NT2RP2004966, NT2RP2005219, NT2RP2005322, NT2RP2005671, NT2RP2005970, NT2RP2006435, NT2RP3000234, NT2RP3000266, NT2RP3000326, NT2RP3000638, NT2RP3000719, NT2RP3001359, NT2RP3001613, NT2RP3001861, NT2RP3003097, NT2RP3003235, NT2RP3003258, NT2RP3003368, NT2RP3003549, NT2RP3003731, NT2RP3003789, NT2RP3004541, OVARC1000636, OVARC1001849, PLACE1000456, PLACE1001098, PLACE1001300, PLACE1001904, PLACE1002376, PLACE1002379, PLACE1003405, PLACE1003724, PLACE1004113, PLACE1004273, PLACE1004757, PLACE1004850, PLACE1005047, PLACE1005760, PLACE1006472, PLACE1006610, PLACE1007635, PLACE1009580, PLACE1010330, PLACE1010482, PLACE1011134, PLACE1011146, PLACE1011360, PLACE1011386, PLACE1011514, PLACE1011835.

Genes that were expressed in all the tissues tested are indicated below by the corresponding clone names:
clone: HEMBA1002715, NT2RP1001023, NT2RP2000396, NT2RP21103902, NT2RP2005970, NT2RP30113258, NT2RP3003731, PLACE1003405, PLACE1003724,.

Genes that were expressed at low levels in any of the tissues tested are indicated below by the corresponding clone names:
clone: HEMBA1000296, HEMBA1001490, HEMBA1004078, HEMBA1004149, HEMBA1005301, HEMBA1005703, HEMBA1006019, HEMBA1006549, HEMBA1007053, NT2RM1000066, NT2RM1000566, NT2RM1000634, NT2RM1000726, NT2RM1000853, NT2RM1001103, NT2RP1000255, NT2RP1000477, NT2RP1000533, NT2RP1000544, NT2RP1000567, NT2RP1000593, NT2RP1000769, NT2RP1000905, NT2RP1000921, NT2RP1001042, NT2RP2000028, NT2RP2000116, NT2RP2000168, NT2RP2000279, NT2RP2000358, NT2RP2002115, NT2RP2003471, NT2RP2004036, NT2RP2004049, NT2RP2004076, NT2RP2004974, NT2RP2005670, NT2RP2006028, NT2RP2006400, NT2RP2006476, NT2RP3001619, NT2RP3001874, NT2RP3002337, NT2RP3003536, NT2RP3004059, NT2RP3004063, OVARC1000363, OVARC1001499, OVARC1001510, OVARC1001636, PLACE1001022, PLACE1003085, PLACE1003378, PLACE1003549, PLACE1004170, PLACE 1004322, PLACE1004507, PLACE1004904, PLACE 1006269, nnnnnnnnnnnn, PLACE1007190, PLACE1007338, PLACE1007878, PLACE1007885, PLACE1008738, PLACE1008994, PLACE1009772, PLACE1010021, PLACE1010978.

Genes exhibiting characteristic features in the expression thereof were selected by statistical analysis of these data. Two examples are shown below to describe the selection of genes of which expression is varied greatly among tissues. The β-actin gene is used frequently as a control in gene expression analysis. Genes of which expression is varied greatly among tissues as compared that of the β-actin gene were determined as follows. Specifically, sum of squared deviation was calculated in the signal intensity of β-actin observed in each tissue, which was divided by 7 degrees of freedom to determine a variance Sₐ². Next, sum of squared deviation was calculated in the signal intensity of a compared gene in each tissue, which was divided by 7 degrees of freedom to determine a variance S_{b}². By taking variance ratio F as F=S_{b}²/Sₐ², genes with a significance level of 5% or more were extracted in the F distribution. Genes extracted are indicated below by the corresponding clone names: NT2RP1001023(PSEC0045).

Gene of OVARC1000037 (heterogeneous nuclear ribonucleoprotein (hnRNP)) which expression is varied little. Genes of which expression is varied greatly among tissues as compared that of the OVARC1000037 gene were determined as follows. Specifically, sum of squared deviation was calculated in the signal intensity of β-actin observed in each tissue, which was divided by 7 degrees of freedom to determine a variance Sₐ². Next, sum of squared deviation was calculated in the signal intensity of a gene to be compared observed in each tissue, which was divided by 7 degrees of freedom to determine a variance S_{b}². By taking variance ratio F as F=S_{b}²/Sₐ², genes with a significance level of 5% or more were extracted in the F distribution. Genes extracted are indicated below by the corresponding clone names: clone: NT2RP1001023 (PSEC0045), NT2RP2005970 (PSEC0084),

Thus, characteristic features in the expression of a gene are illustrated by comparing and statistically analyzing the expression of many genes.

### Analysis of genes associated with neural cell differentiation

Genes involved in neural cell differentiation are useful for treating neurological diseases. It is possible that genes with varying expression levels in response to induction of cellular differentiation in neural cells are associated with neurological diseases.

A survey was performed for genes of which expression levels are varied in response to induction of differentiation (stimulation by retinoic acid (RA)) in cultured cells of a neural strain, NT2.

The NT2 cells were treated basically according to supplier's instruction manual. "Undifferentiated NT2 cells" means NT2 cells successively cultured in an Opti-MEM I (GIBCO-BRL; catalog No. 31985) containing 10%(v/v) fetal bovine serum and 1%(v/v) penicillin-streptomycin (GIBCO BRL). "NT2 cells cultured in the presence of retinoic acid" means the cells resulted from transferring undifferentiated NT2 cells into a retinoic acid-containing medium, which consists of D-MEM (GIBCO BRL; catalog No. 11965), 10%(v/v) fetal bovine serum, 1%(v/v) penicillin-streptomycin and 10 µM retinoic acid (GIBCO-BRL), and the subsequent successive culture therein for 5 weeks. "NT2 cells that were cultured in the presence of retinoic acid and then further cultured in the presence of cell-division inhibitor added" means NT2 cells resulted from transferring NT2 cells cultured in the presence of retinoic acid for 5 weeks into a cell-division inhibitor-containing medium, which consisted of D-MEM(GIBCO BRL; catalog No.11965), 10%(v/v) fetal bovine serum, 1%(v/v) penicillin-streptomycin, 10 µM retinoic acid, 10 µM FudR (5-fluoro-2'-deoxyuridine: GIBCO BRL), 10 µM Urd (Uridine: GIBCO BRL) and 1 µM araC (Cytosine β-D-Arabinofuranoside: GIBCO BRL), and the subsequence successive culture for 2 weeks. Each of the cells were treated with trypsin and then harvested. Total RNAs were extracted from the cells by using S.N.A.P.⁽™⁾ Total RNA Isolation kit (Invitrogen^{(r)}). The labeling of probe used for hybridization was carried out by using 10 µg of the total RNA according to the same methods as described above. The data were obtained in triplicate (n=3). The data of signal value representing gene expression level in the cells in the presence of stimulation for inducing differentiation were compared with those in the absence of the stimulation. The comparison was performed by statistical treatment of two-sample t-test. Clones with significant difference in the signal distribution were selected under the condition of p<0.05. In this analysis, clones with the difference can be statistically detected even when the signals were low. Accordingly, clones with signal value of 40 or less were also assessed for the selection.

Tables 162-341 show the expression level of each cDNA in undifferentiated NT2 cells, NT2 cells cultured in the presence of RA, and NT2 cells that were cultured in the presence of RA and that were further cultured in the presence of cell-division inhibitor added.

Averaged signal values (M₁, M₂) and sample variances (s₁², s₂²) were calculated for each gene in each of the cells, and then. the pooled sample variances s² were obtained from the sample variances of the two types of cells to be compared. The t values were determined according to the following formula: t=(M1-M2)/s/(1/3+1/3)^{1/2}. When the determined t-value was greater than a t-value at P, which means the probability of significance level, of 0.05 or 0.01 in the t-distribution table with 4 degrees of freedom, the difference was judged to be found in the expression level of the gene between the two types of cells at p<0.05 or p<0.01, respectively. The tables also include the information on an increase (+) or decrease (-) in the expression level of a gene in the treated cells when the level is compared with that of untreated undifferentiated cells.

Clones of which expression levels increased by RA are as follows:
PSEC0017, PSEC0021, PSEC0041, PSEC0047, PSEC0049, PSEC0055, PSEC0066, PSEC0070, PSEC0071, PSEC0072, PSEC0074, PSEC0075, PSEC0076, PSEC0080, PSEC0084, PSEC0088, PSEC0094, PSEC0103, PSEC0105, PSEC0112, PSEC0113, PSEC0119, PSEC0127, PSEC0129, PSEC0139, PSEC0143, PSEC0144, PSEC0152, PSEC0171, PSEC0181, PSEC0182, PSEC0192, PSEC0195, PSEC0200, PSEC0203, PSEC0215, PSEC0223, PSEC0235, PSEC0239, PSEC0243, PSEC0255, PSEC0265.

Clones of which expression levels increase by RA/inhibitor are as follows:
PSEC0017, PSEC0019, PSEC0030, PSEC0041, PSEC0047, PSEC0048, PSEC0049, PSEC0059, PSEC0066, PSEC0072, PSEC0081, PSEC0084, PSEC0094, PSEC0104, PSEC0117, PSEC0119, PSEC0120, PSEC0129, PSEC0136, PSEC0139, PSEC0143, PSEC0152, PSEC0161, PSEC0169, PSEC0181, PSEC0182, PSEC0192, PSEC0203, PSEC0223, PSEC0235, PSEC0251, PSEC0265.

Clones of which expression levels increase in the presence of both RA and RA/inhibitor are as follows:
PSEC0017, PSEC0041, PSEC0047, PSEC0049, PSEC0066, PSEC0072, PSEC0084, PSEC0094, PSEC0119, PSEC0129, PSEC0139, PSEC0143, PSEC0152, PSEC0181, PSEC0182, PSEC0192, PSEC0203, PSEC0223, PSEC0235, PSEC0265.

These are neurological disease-associated clones.

Analysis of rheumatoid arthritis-associated genes

The onset of rheumatoid arthritis is thought to be involved in the proliferation of synovial cells covering inner surfaces of joint cavity and in inflammatory reaction resulted from the action of cytokines produced by leukocytes infiltrating into the joint synovial tissues (Rheumatism Information Center http://www.rheuma-net.or.jp/). Recent studies have also revealed that tissue necrosis factor (TNF)-α participates in the onset (Current opinion in immunology 1999, 11, 657-662). When the expression of a gene exhibits responsiveness to the action of TNF on synovial cells, the gene is considered to be involved in rheumatoid arthritis.

A survey was performed for genes of which expression levels are varied in response to TNF-α in the primary cell culture of synovial tissue. The primary cultured cells of the smooth muscle (Cell Applications) were grown to be confluent in a culture dish, and then, human TNF-α (Boehringer-Mannheim) was added at a final concentration of 10 ng/ml thereto. The culture was further continued for 24 hours.

Total RNA was extracted from the cells by using S.N.A.P.⁽™⁾ Total RNA Isolation kit (Invitrogen). The labeling of probe used for hybridization was carried out by using 10 µg of the total RNA according to the same methods as described above. The data were obtained in triplicates (n=3). The data of signal value representing gene expression level in the cells in the presence of TNF stimulation were compared with those in the absence of the stimulation. The comparison was performed by statistical treatment of two-sample t-test. Clones with significant difference in the signal distribution were selected under the condition of p<0.05. In this analysis, clones with the difference can be statistically detected even when the signals were low. Accordingly, clones with signal value of 40 or less were also assessed for the selection.

Table 343 shows the expression level of each cDNA in synovial cells cultured in the absence or presence of TNF.

Averaged signal values (M₁, M₂) and sample variances (s₁², s₂²) for each gene were calculated in each of the cells, and then, the pooled sample variances s² were obtained from the sample variances of the two types of cells to be compared. The t-values were determined according to the following formula: t=(M₁-M₂)/s/(1/3+1/3)^{1/2}. When the determined t-value was greater than a t-value at P, which means the probability of significance level, of 0.05 or 0.01 in the t-distribution table with 4 degrees of freedom, the difference was judged to be found in the expression level of the gene between the two types of cells at p<0.05 or p<0.01, respectively. The tables also include the information of an increase (+) or decrease (-) in the expression level of a gene in the stimulated cells when the level is compared with that of unstimulated cells.

PSEC clones of which expression levels are elevated by TNF-α are as follows:
PSEC0070, PSEC0073, PSEC0084, PSEC0100, PSEC0109, PSEC0120, PSEC0131, PSEC0161, PSEC0183, PSEC0192, PSEC0197, PSEC0205, PSEC0207, PSEC0210, PSEC0213, PSEC0222, PSEC0230, PSEC0241, PSEC0252, PSEC0259.

PSEC clones of which expression levels decrease by TNF-α are as follows:
PSEC0105, PSEC0245.

These are rheumatoid arthritis-associated clones.

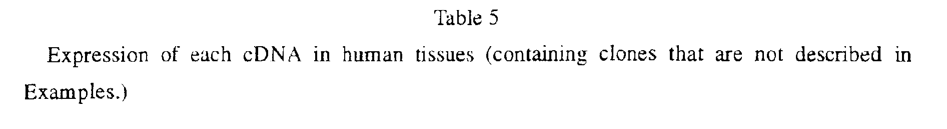

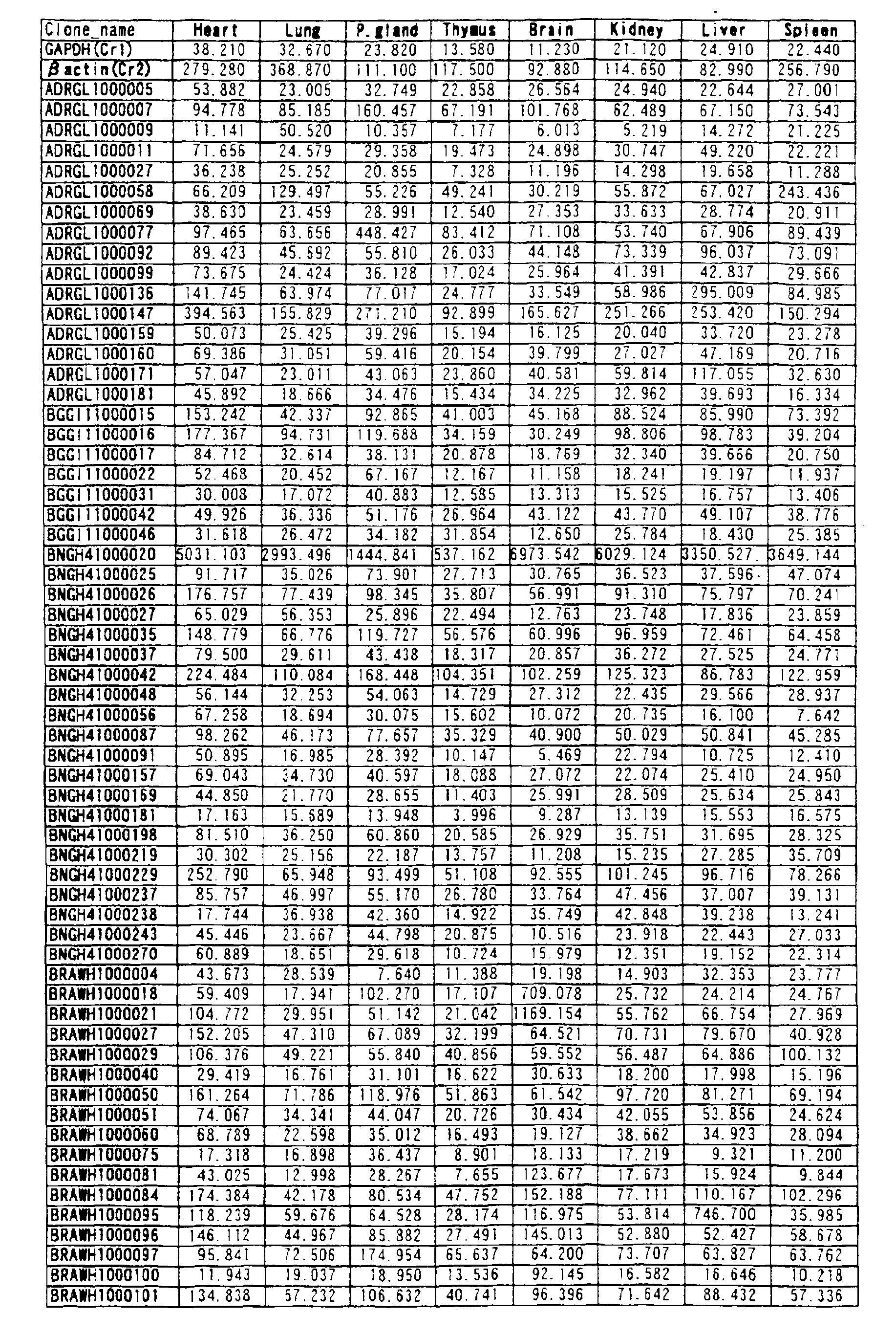

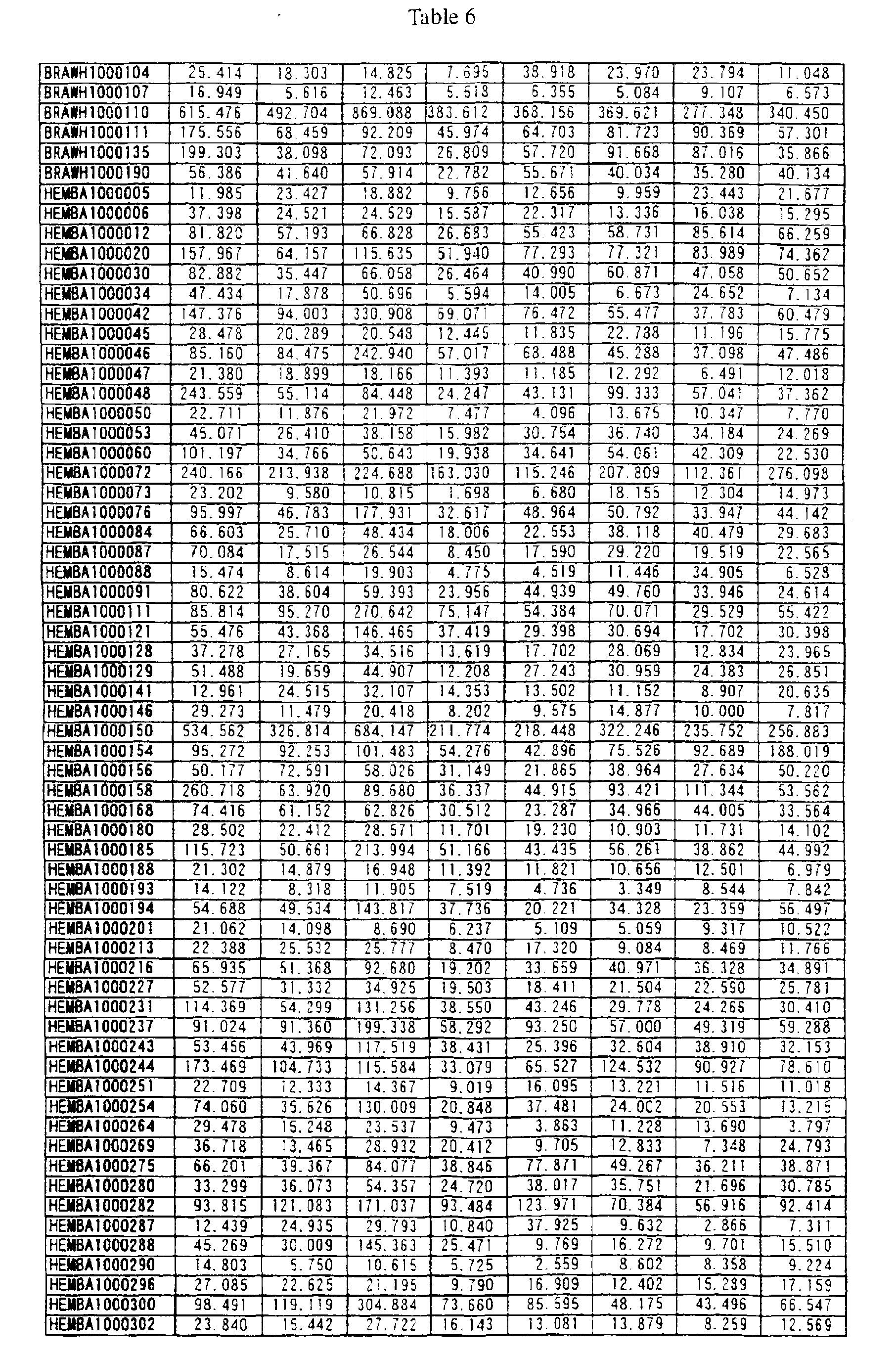

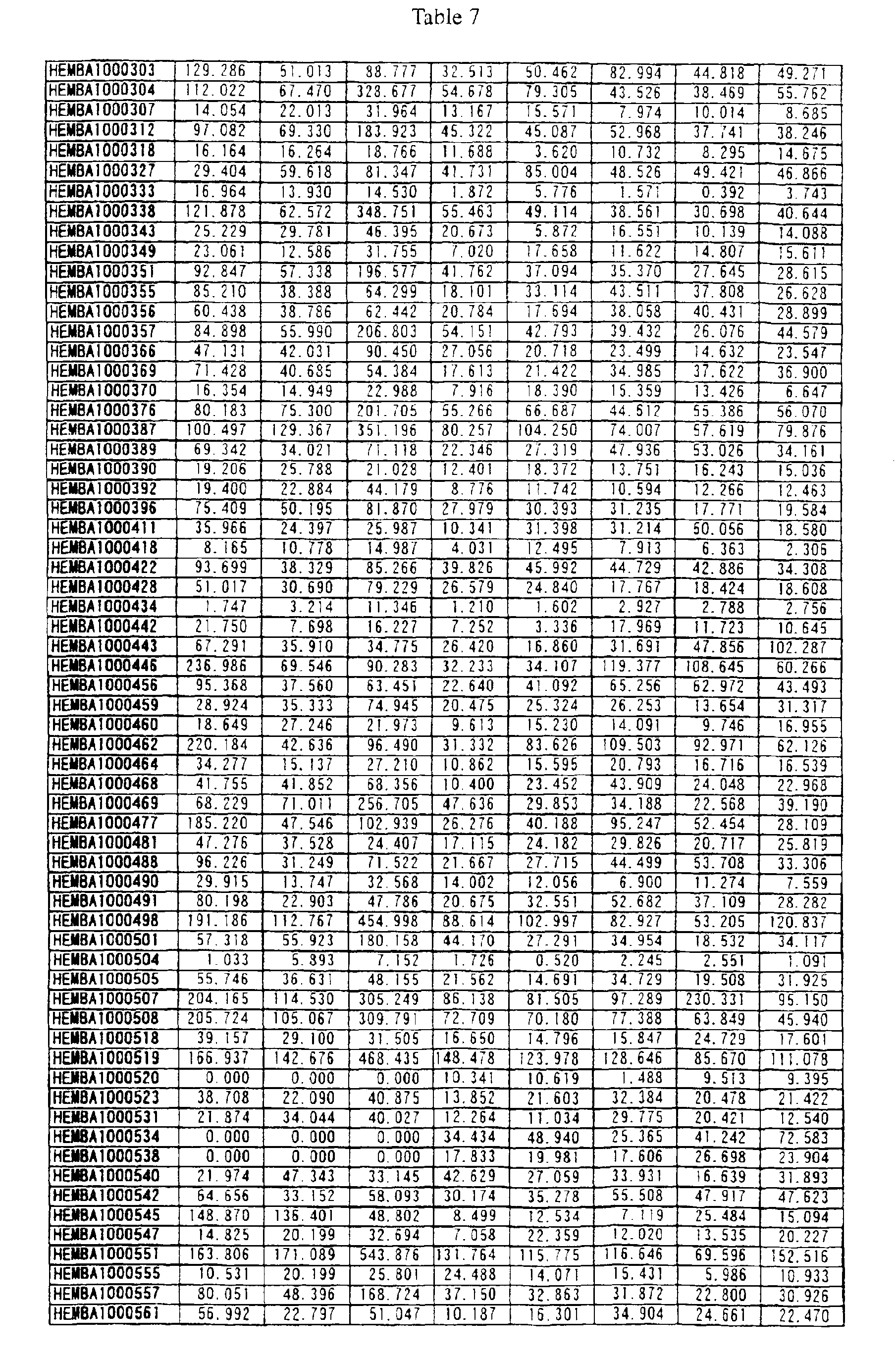

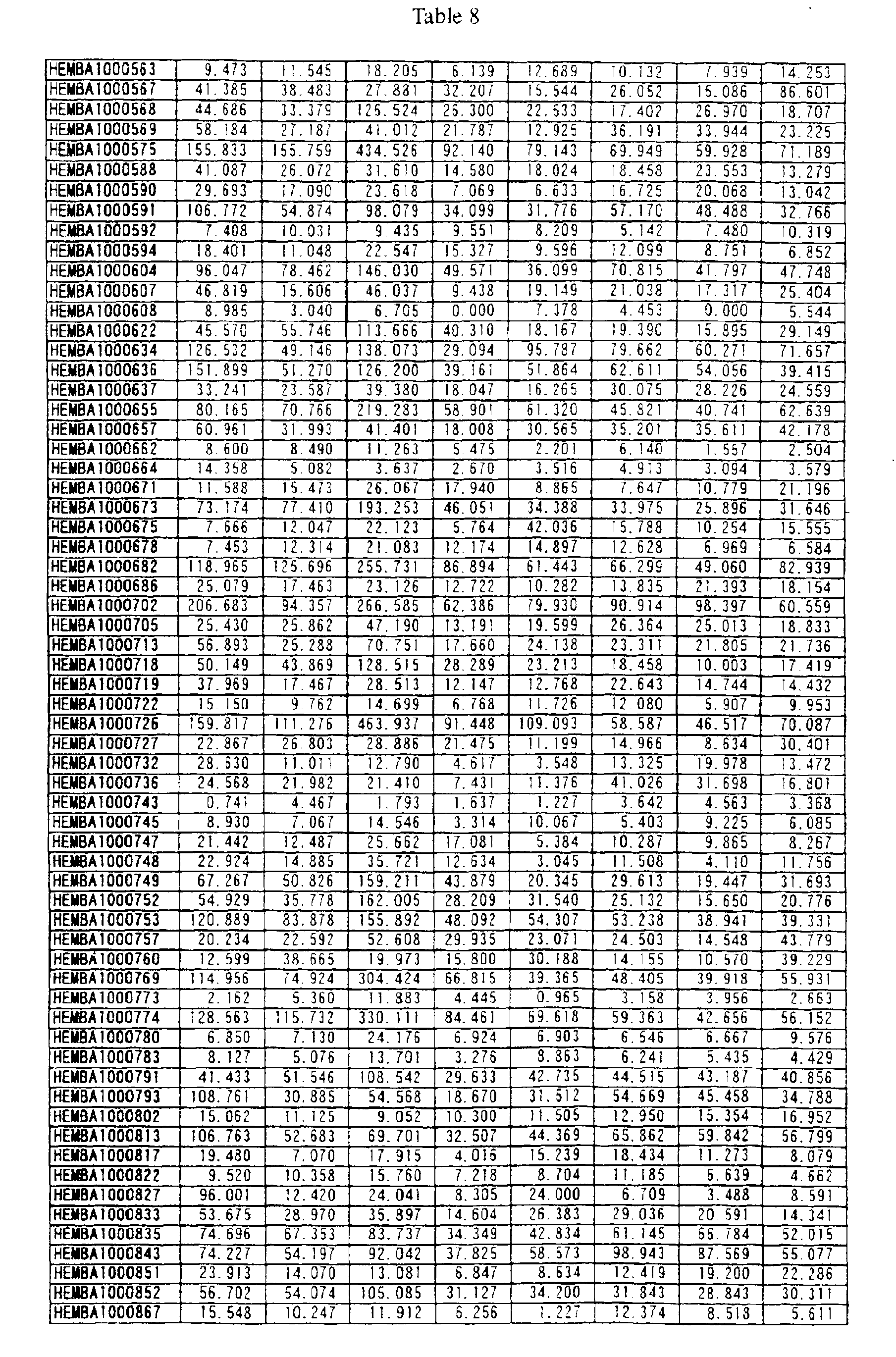

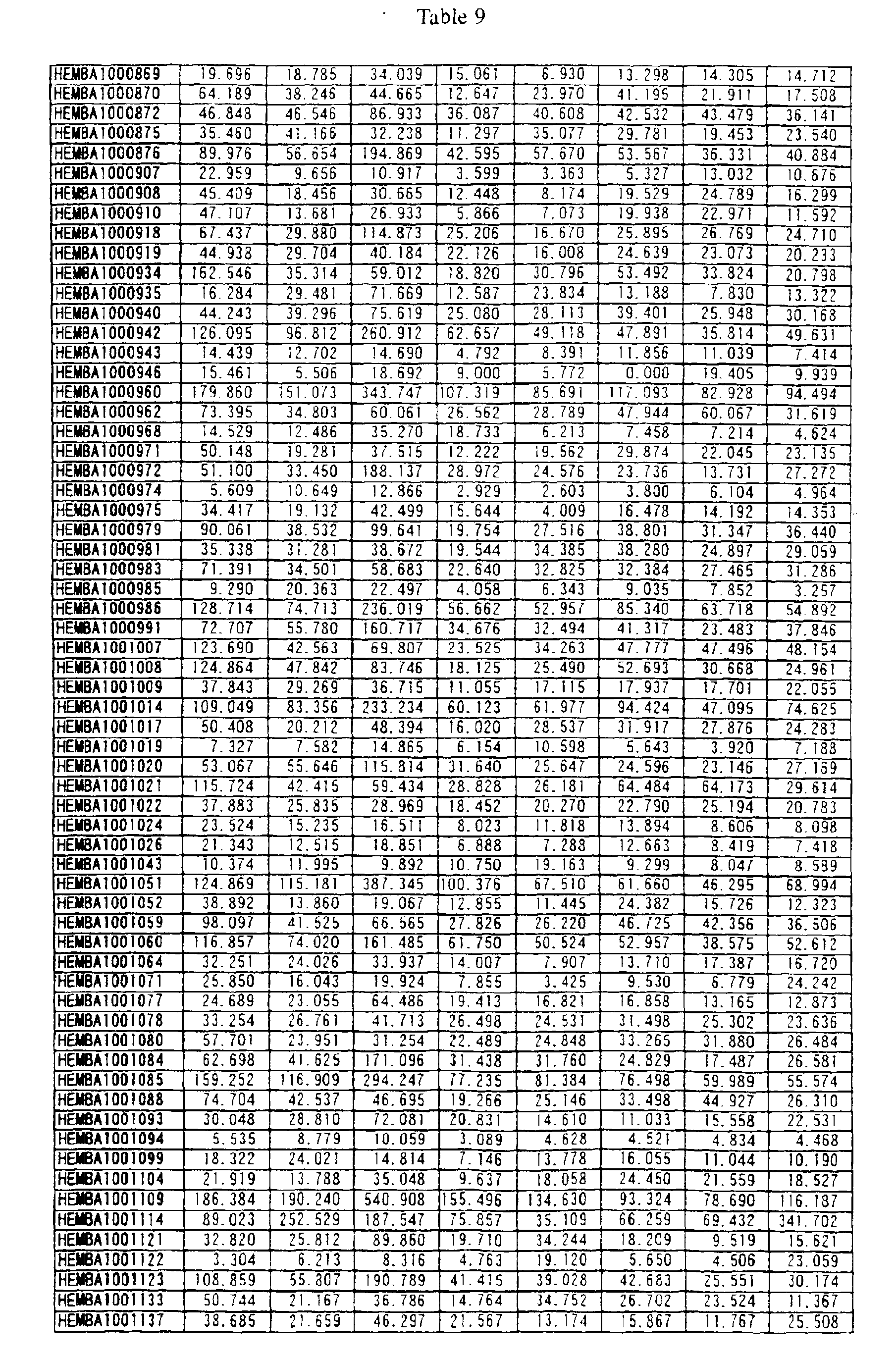

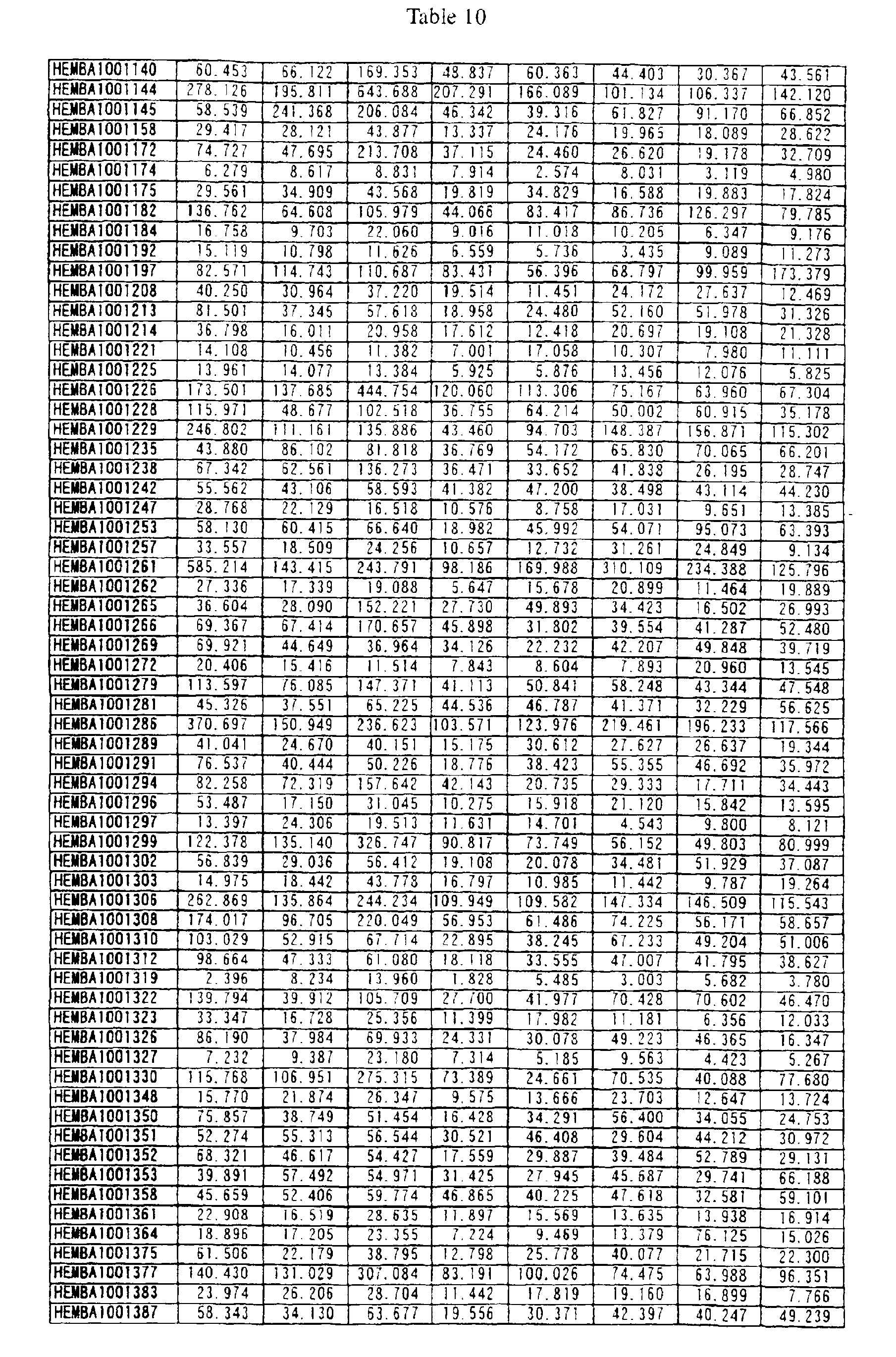

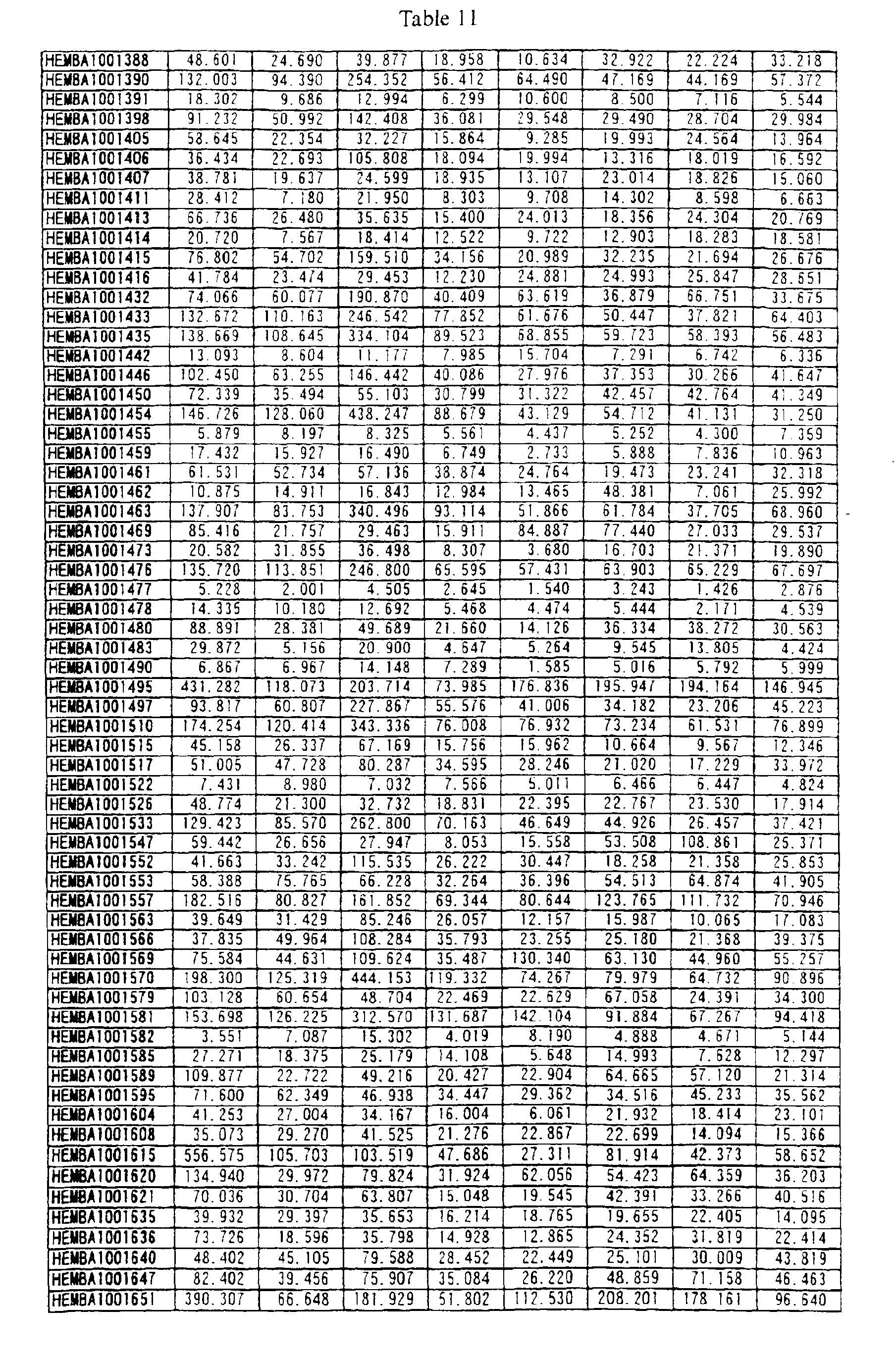

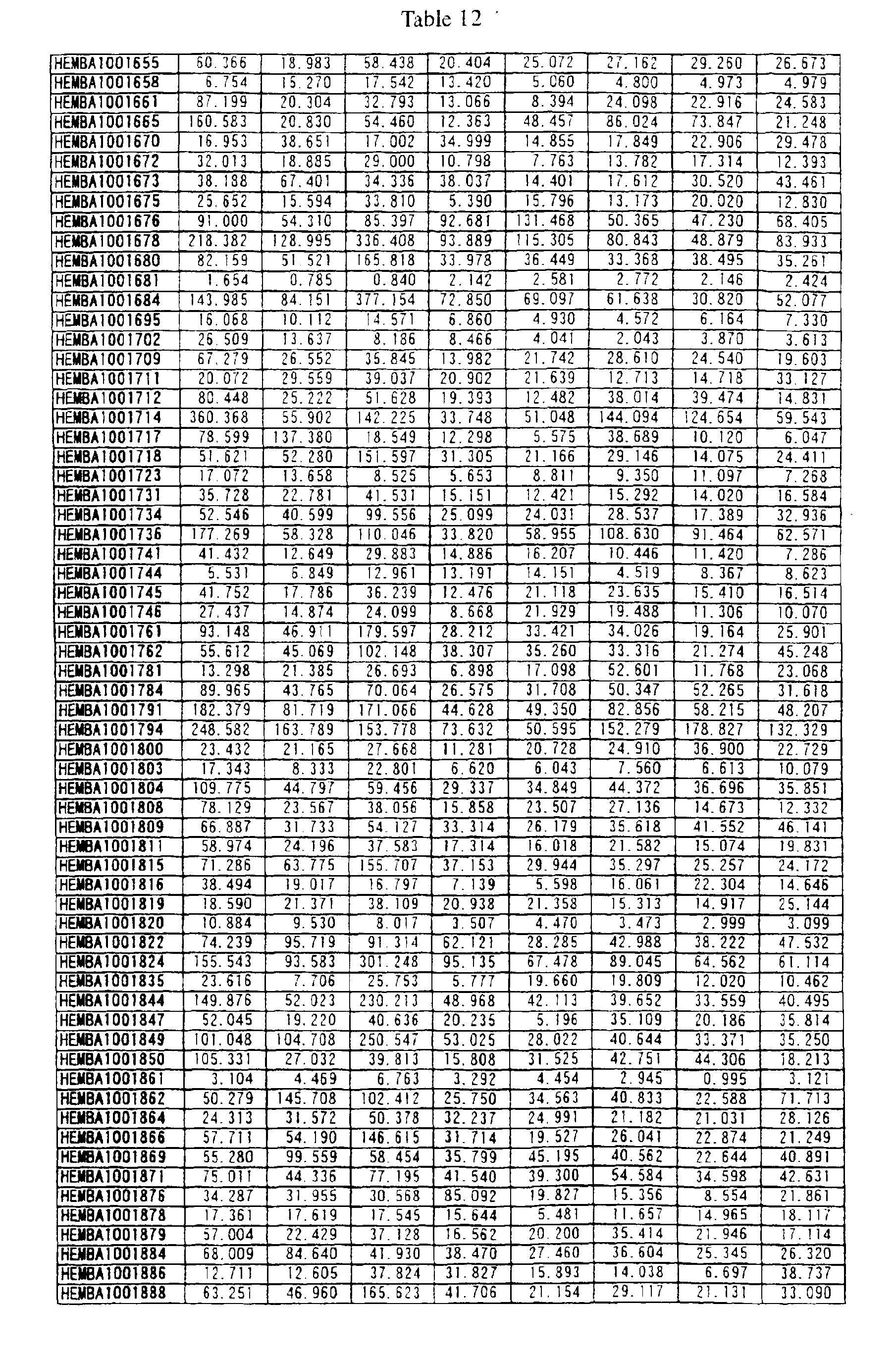

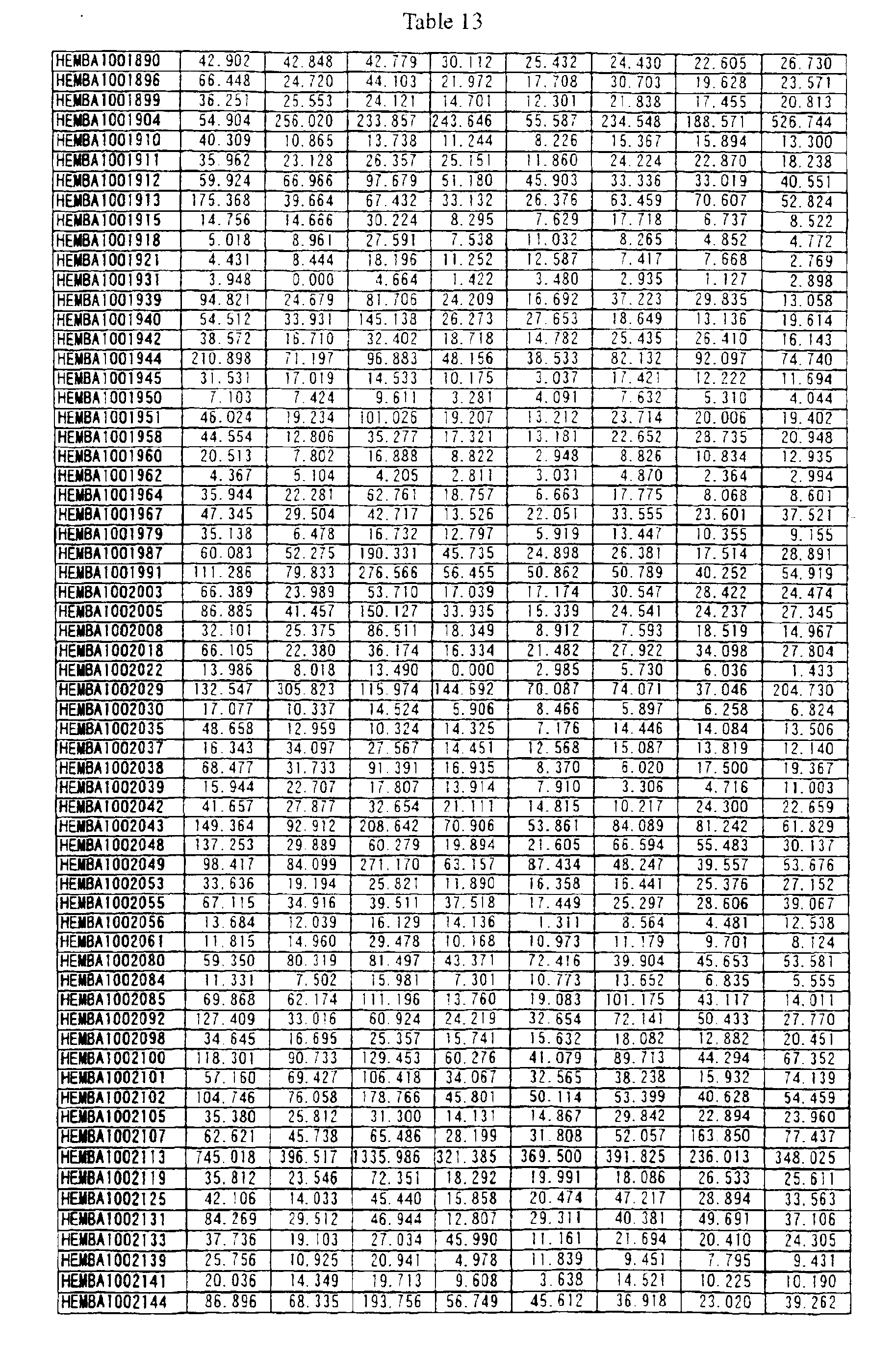

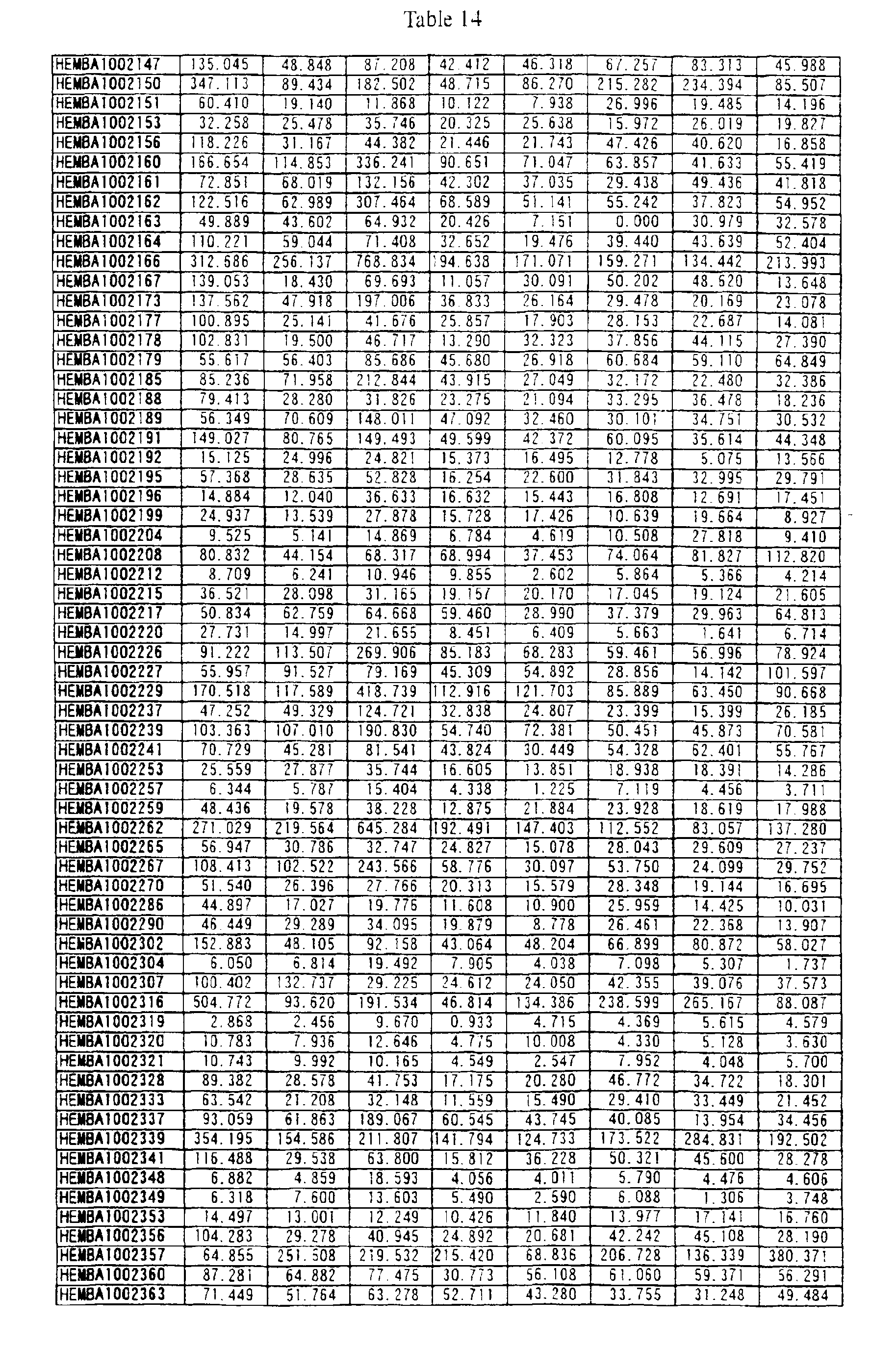

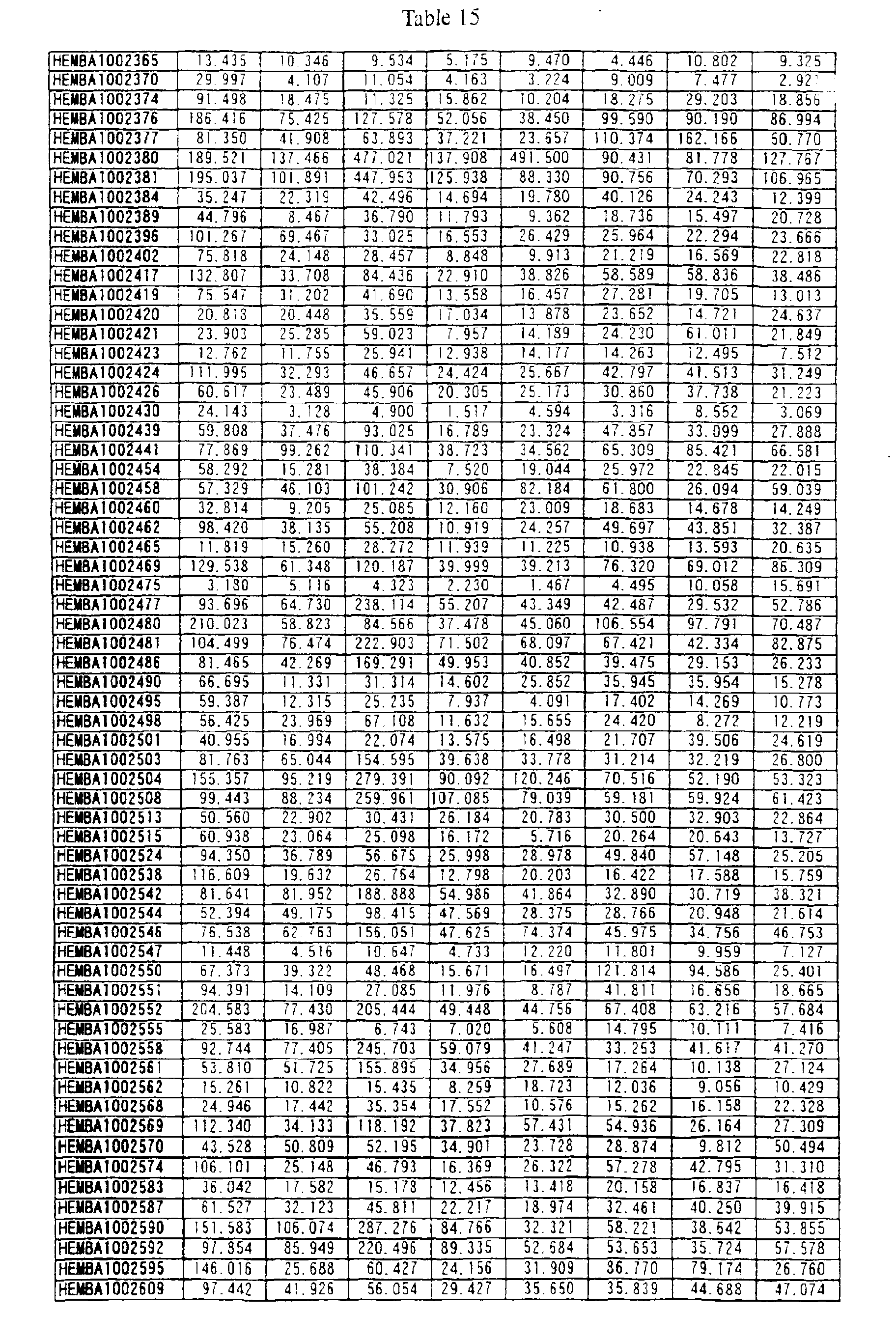

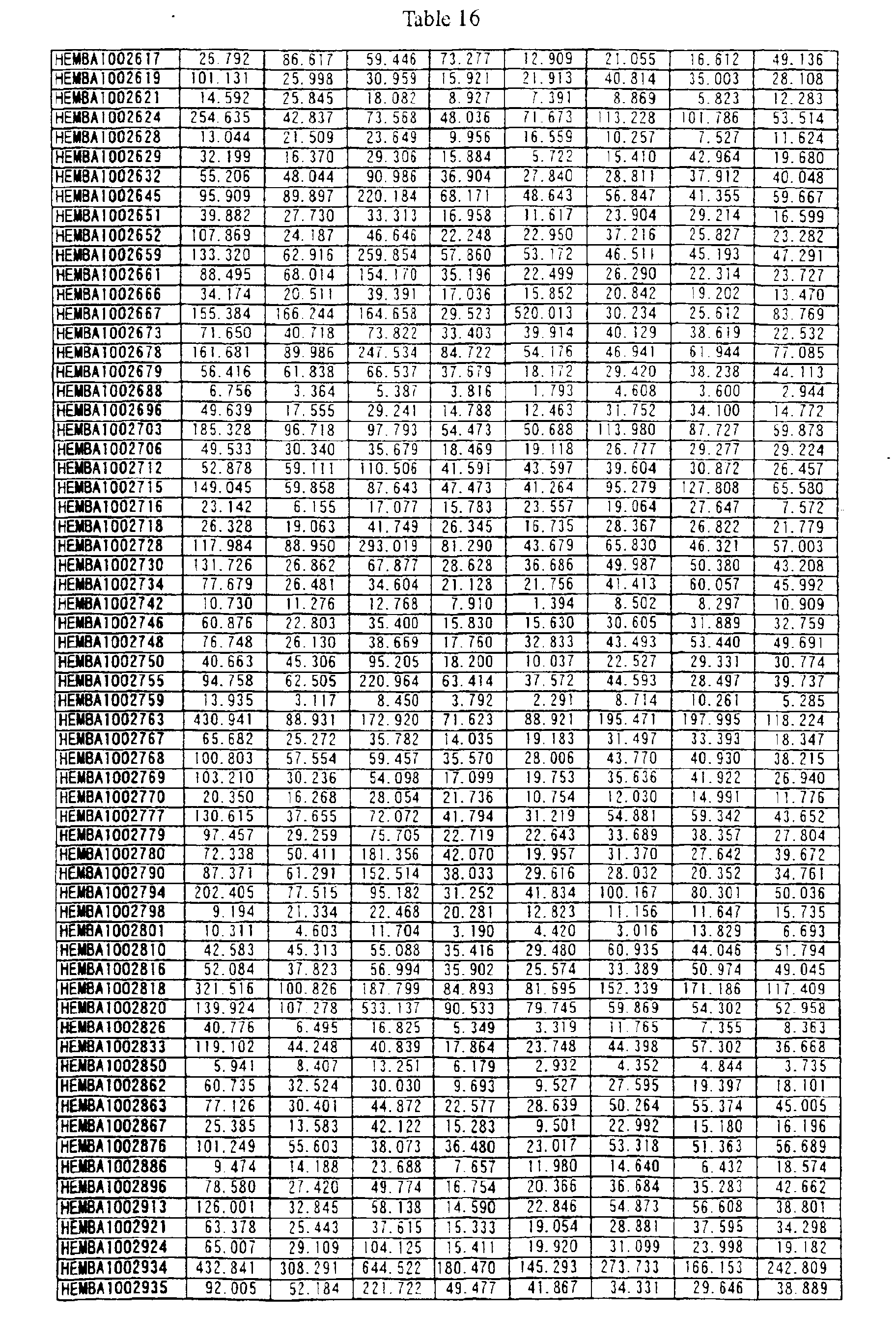

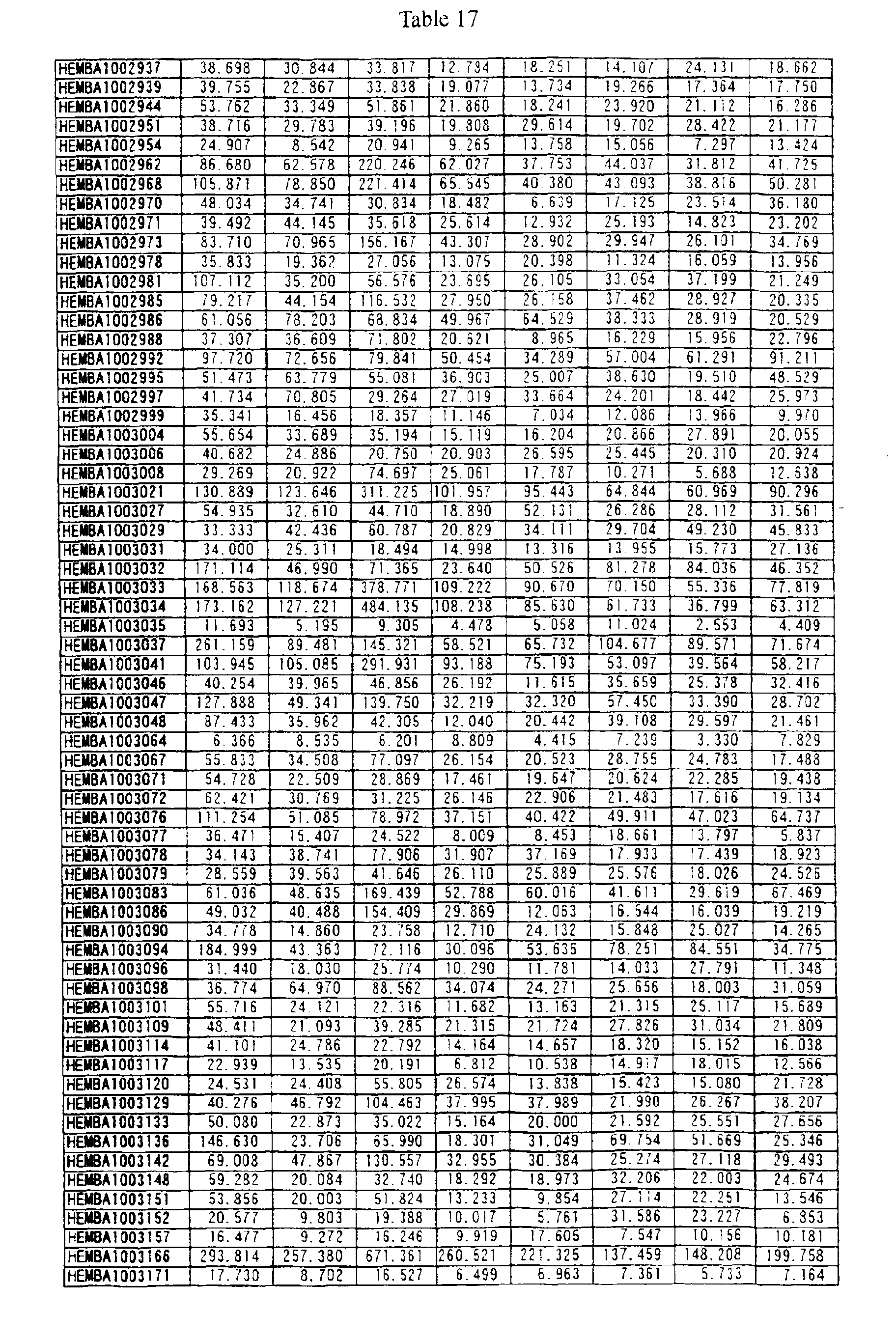

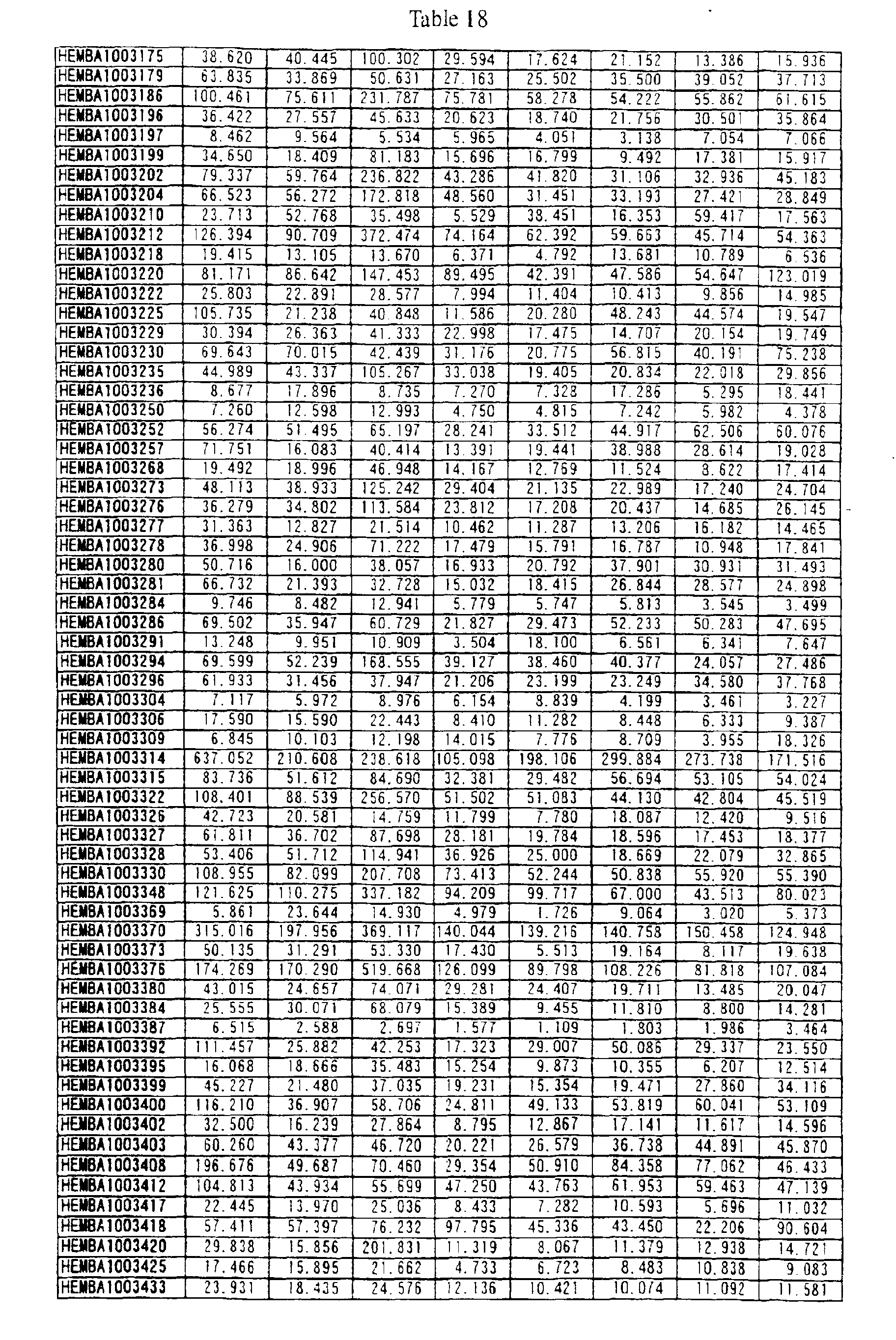

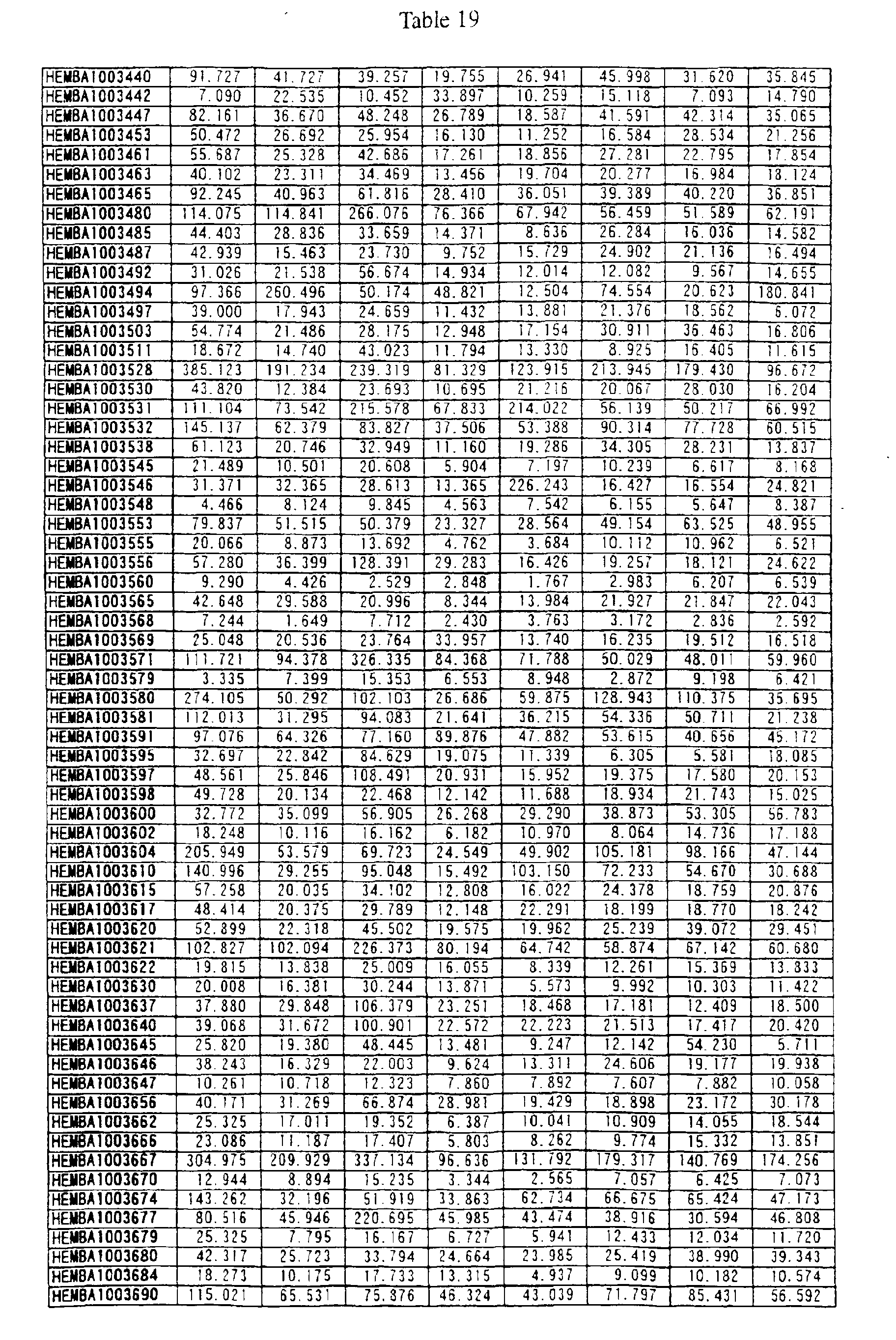

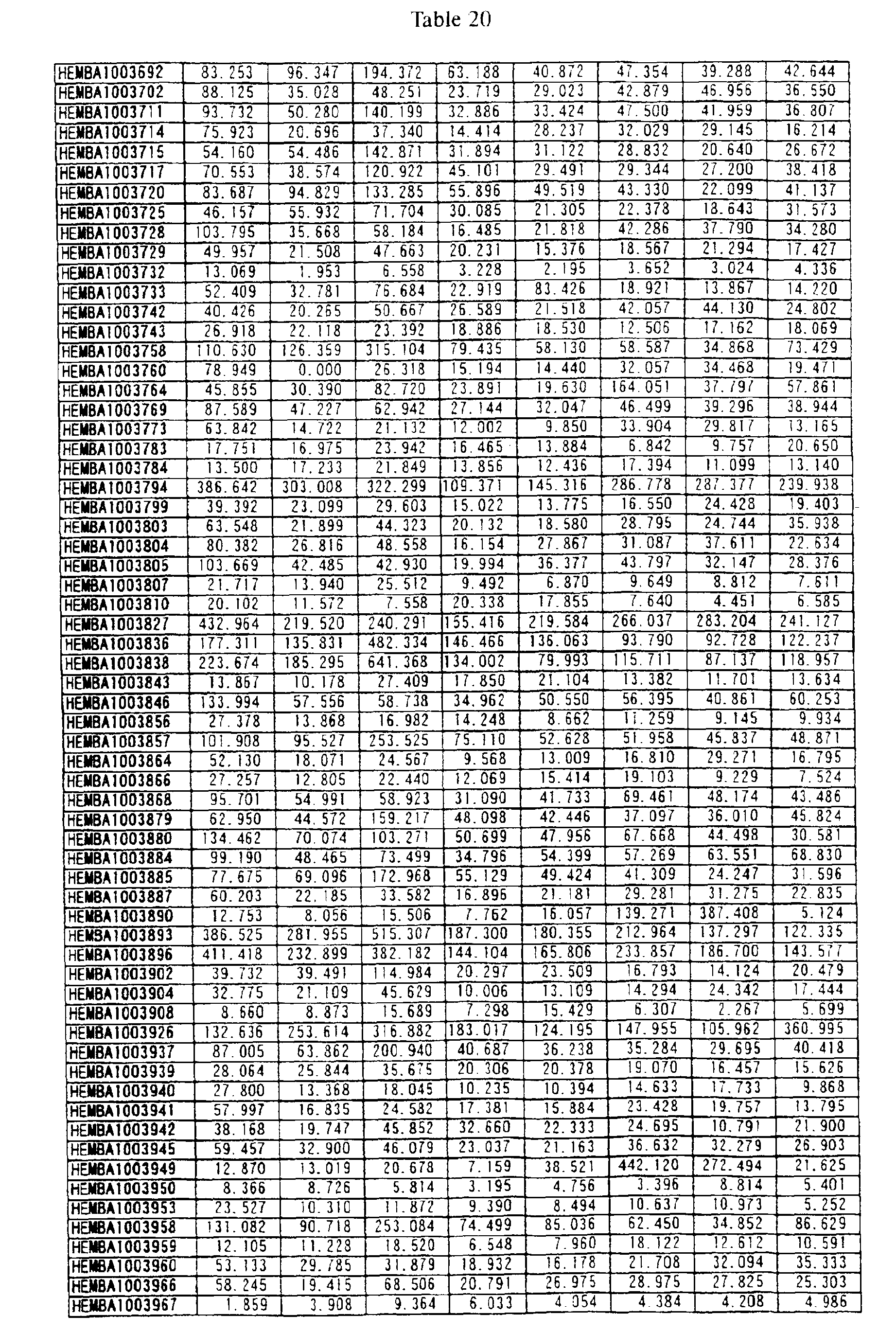

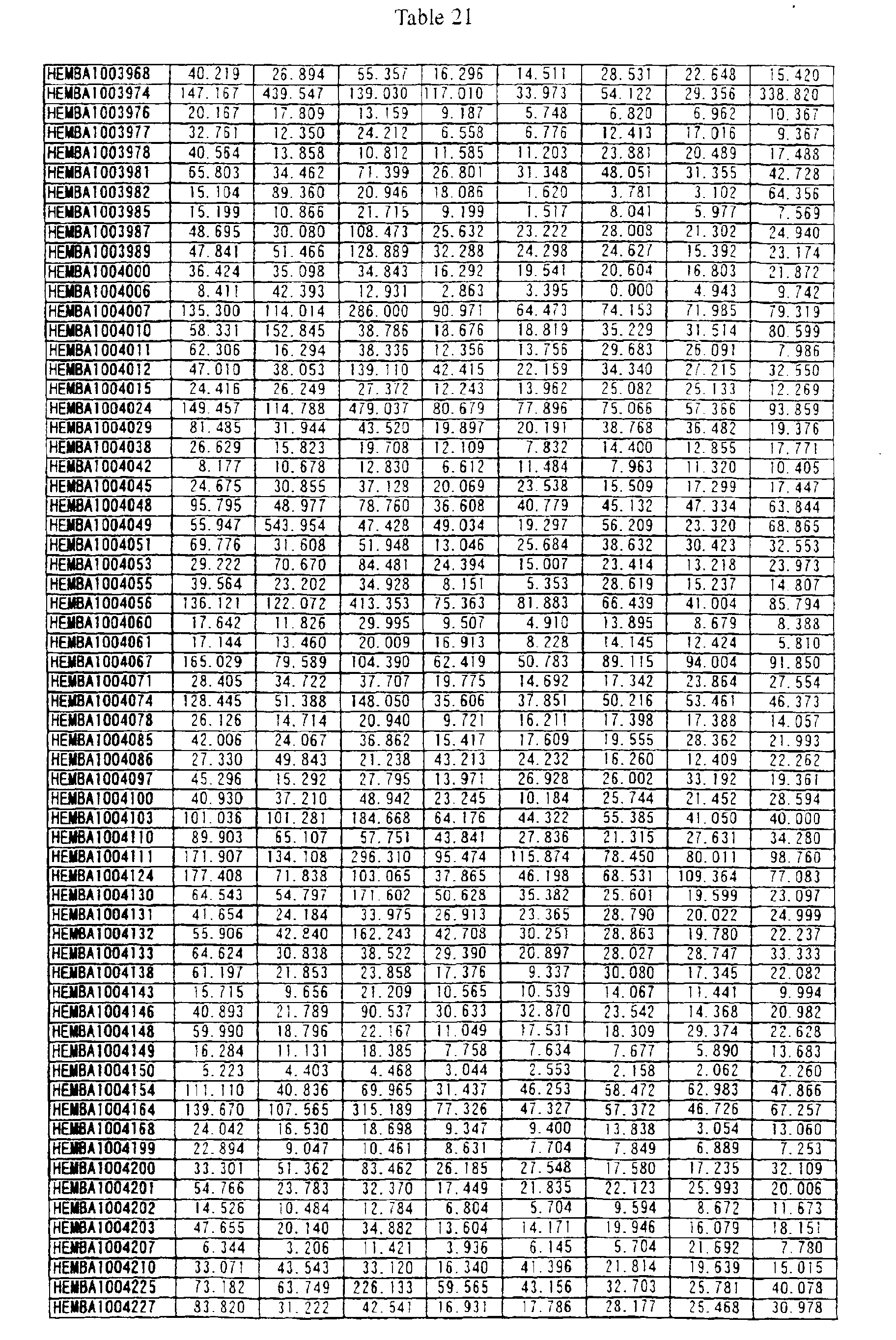

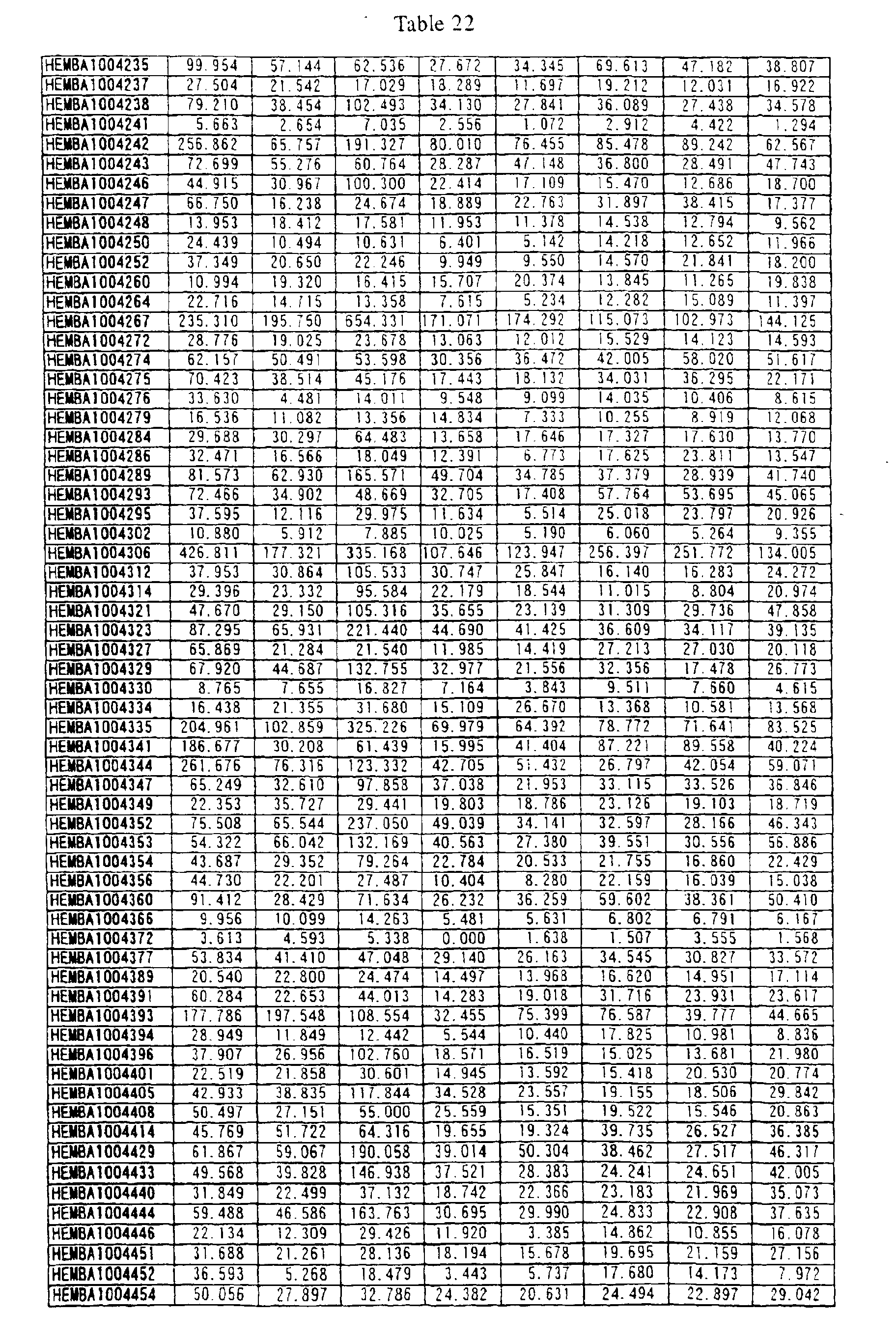

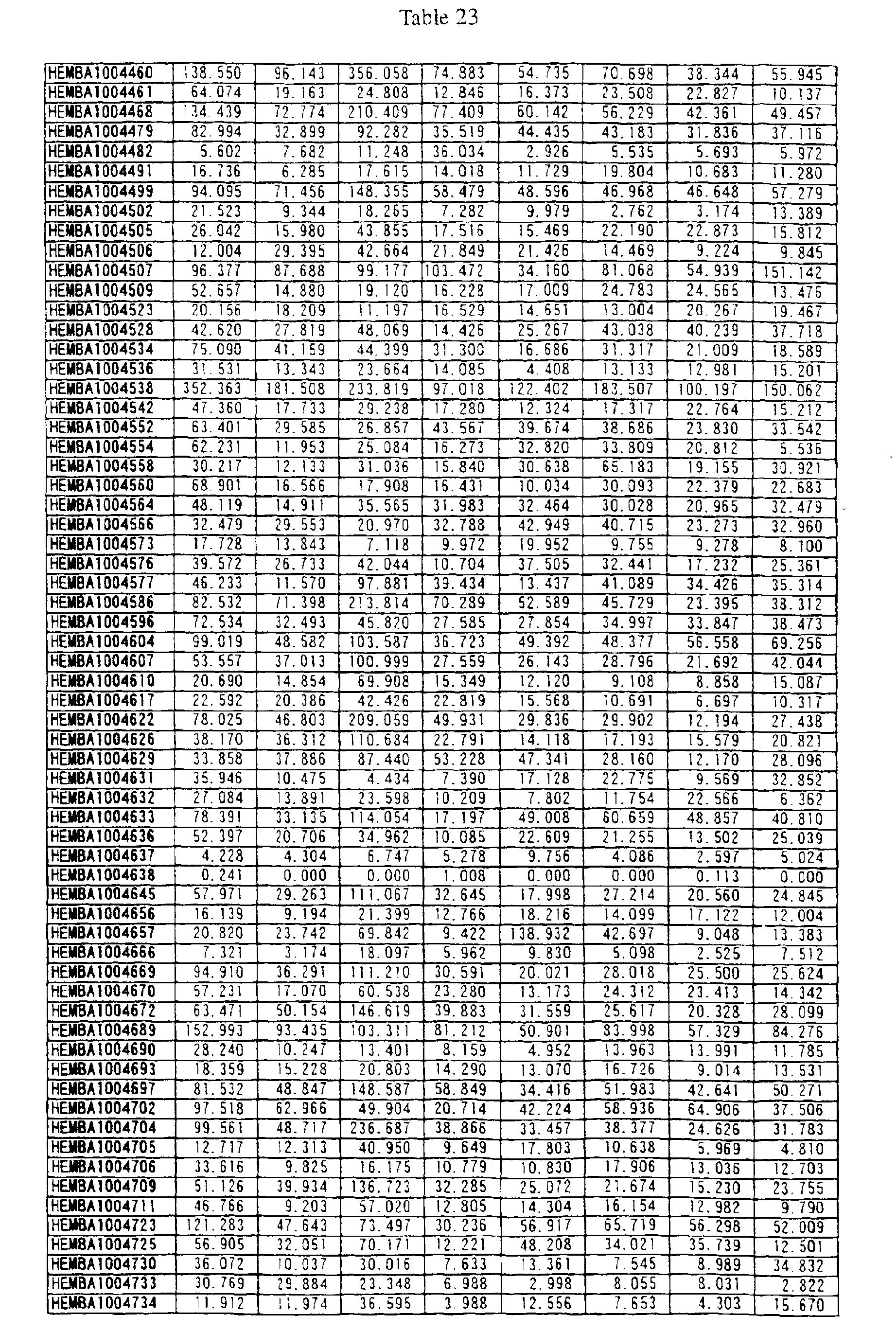

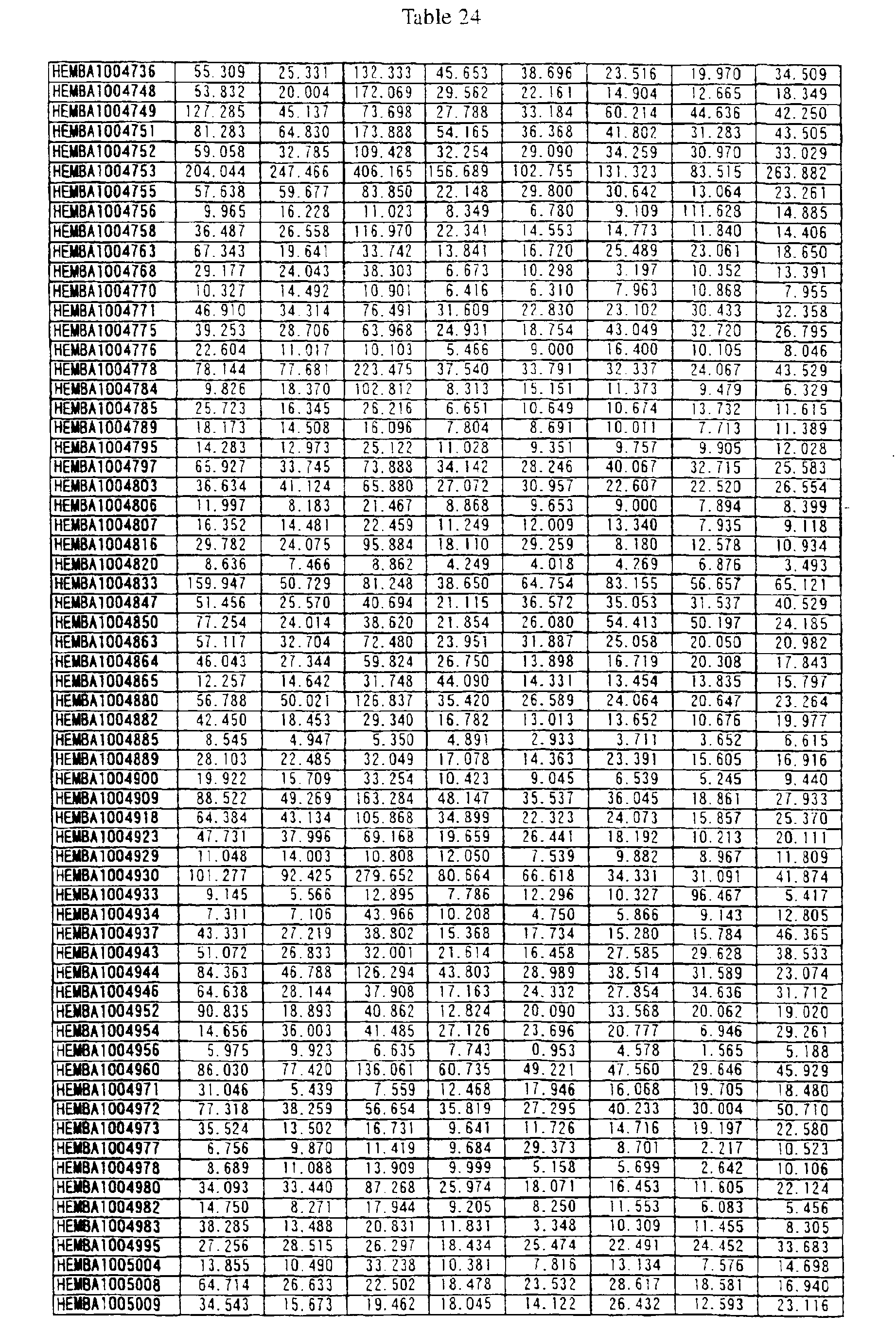

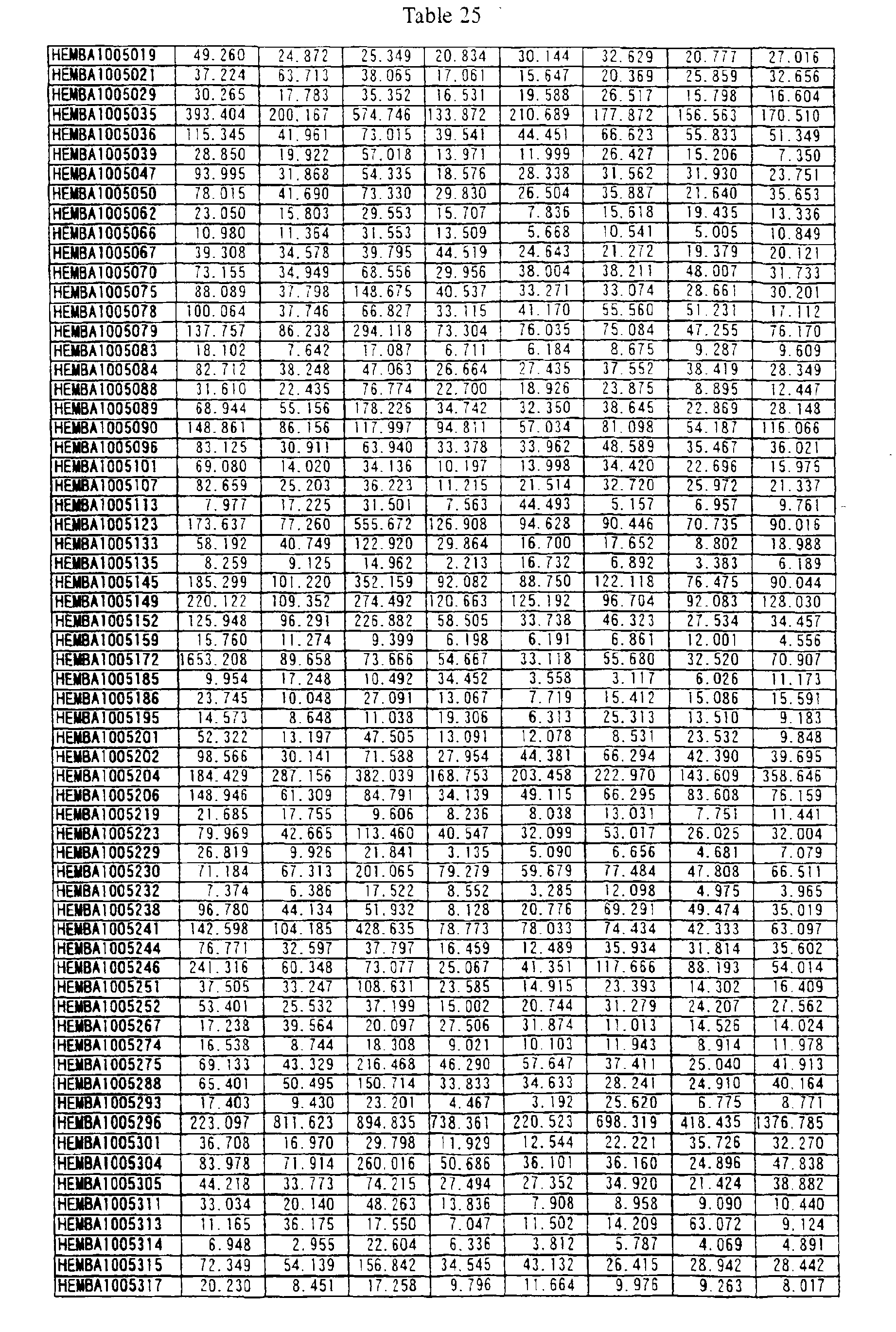

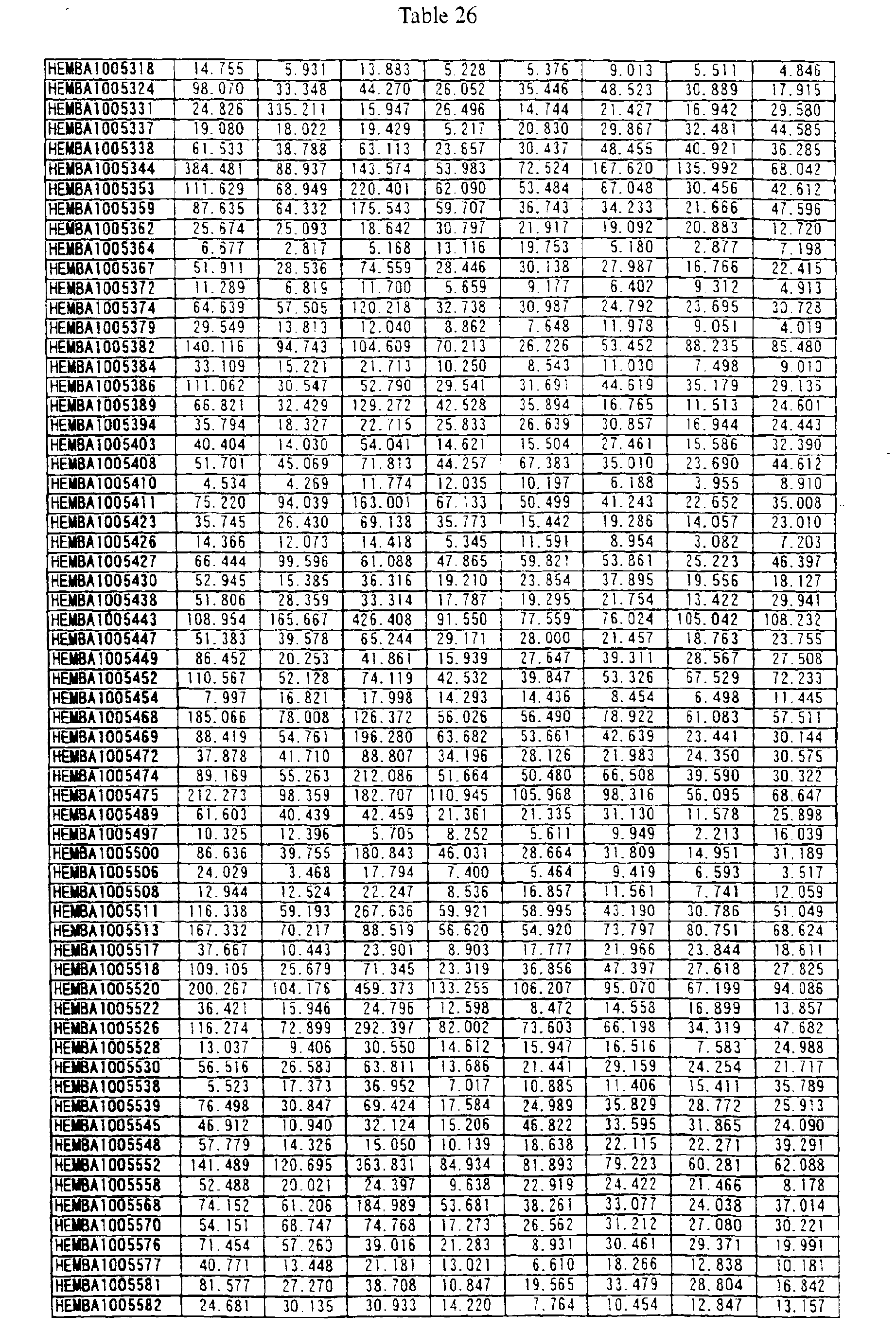

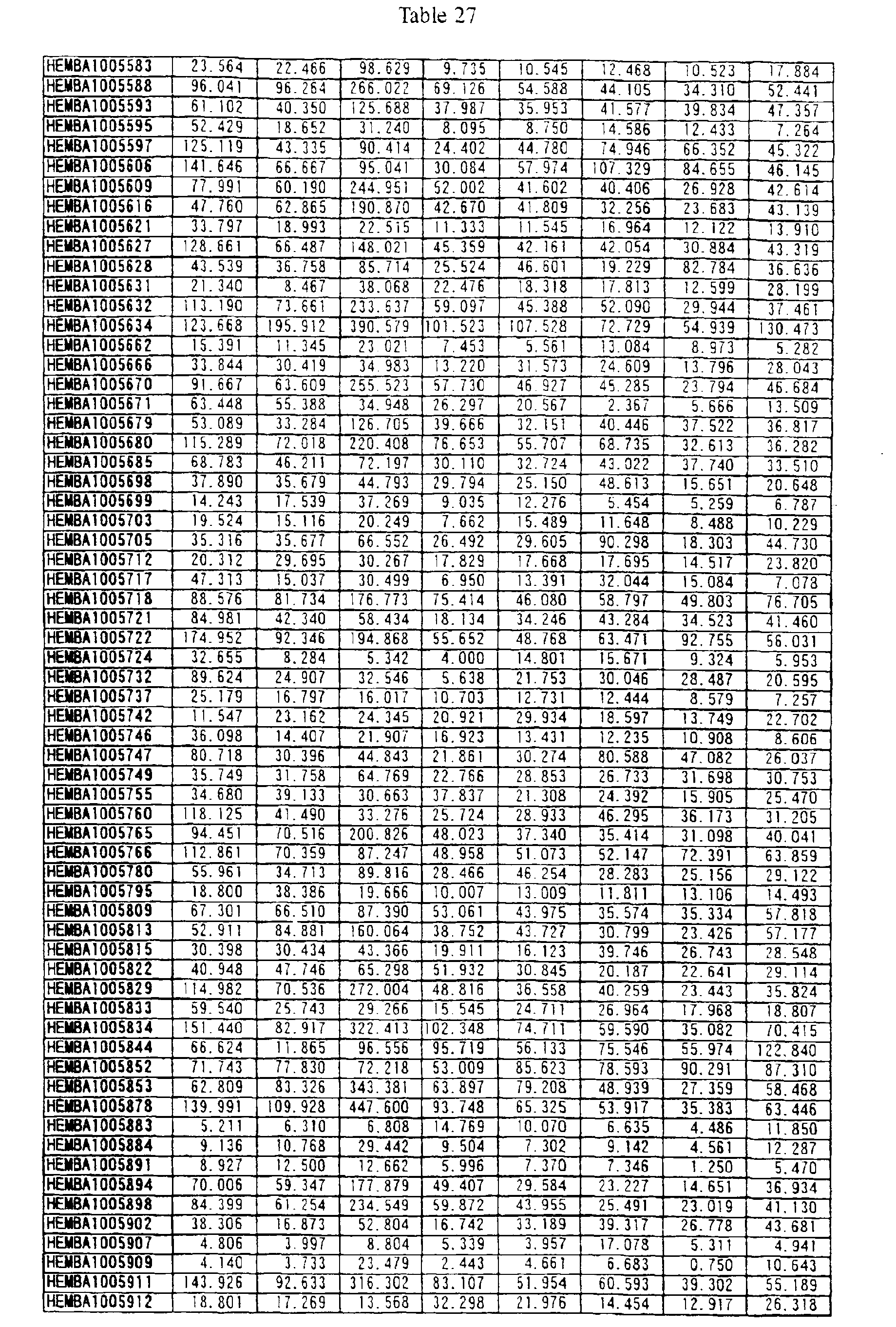

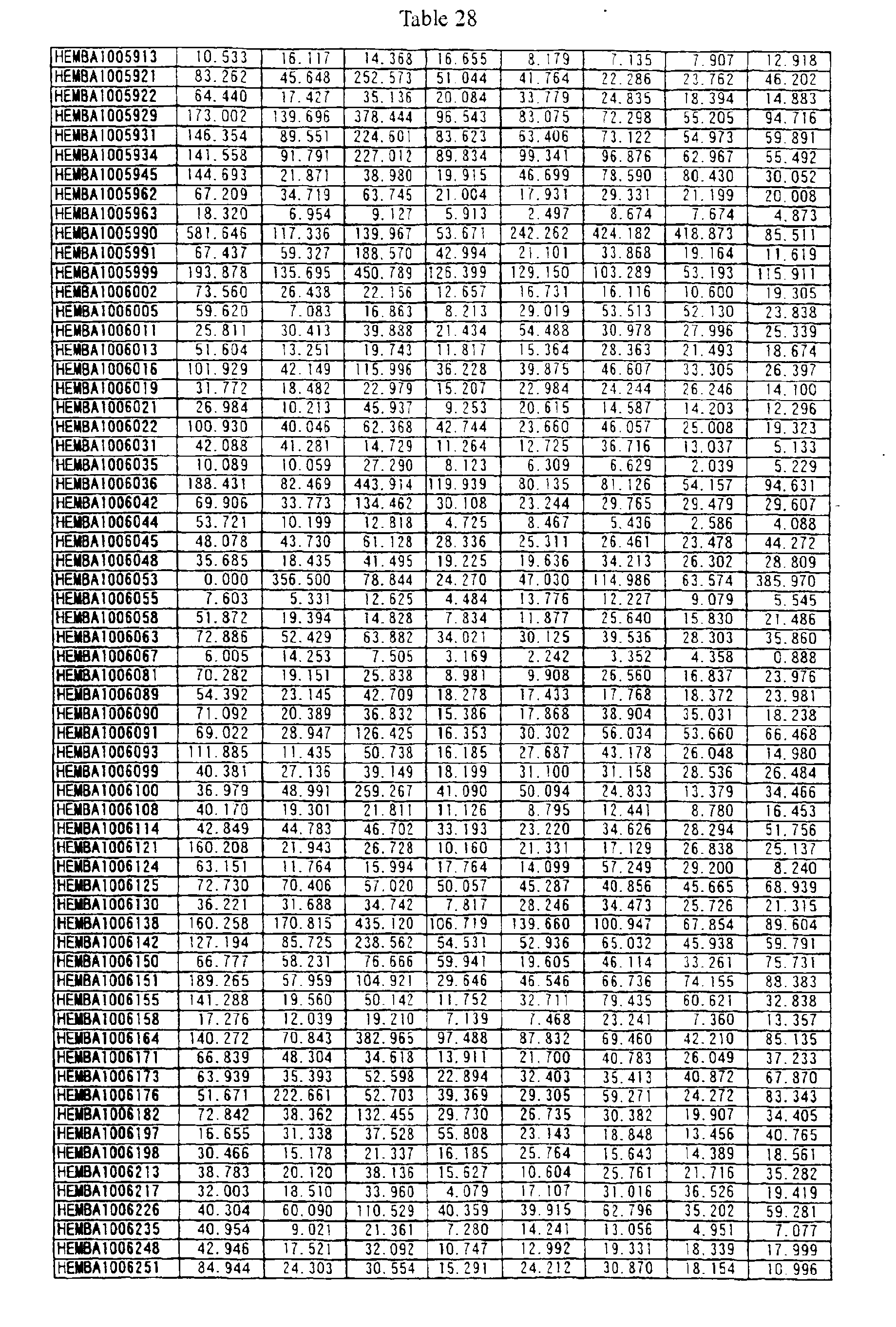

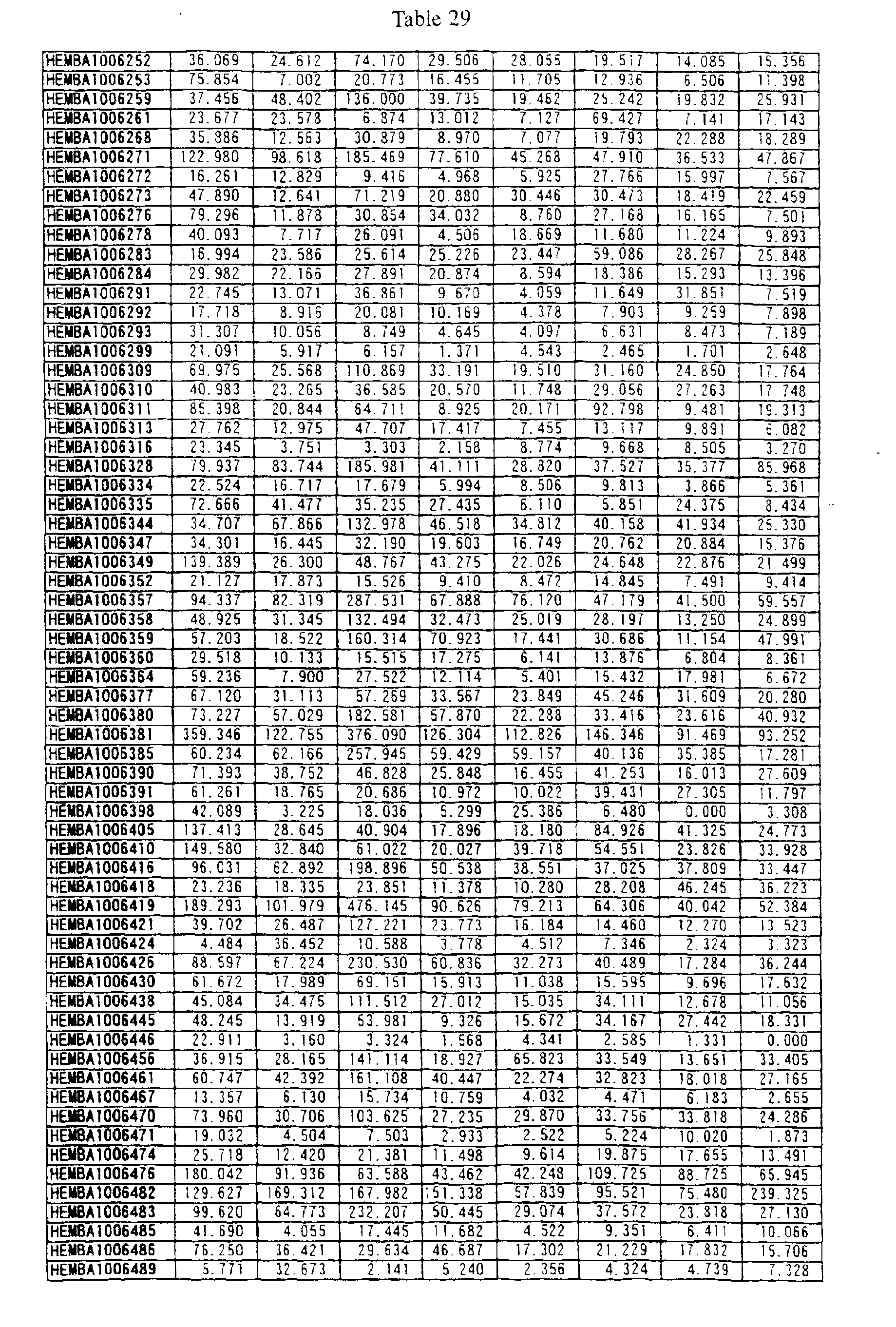

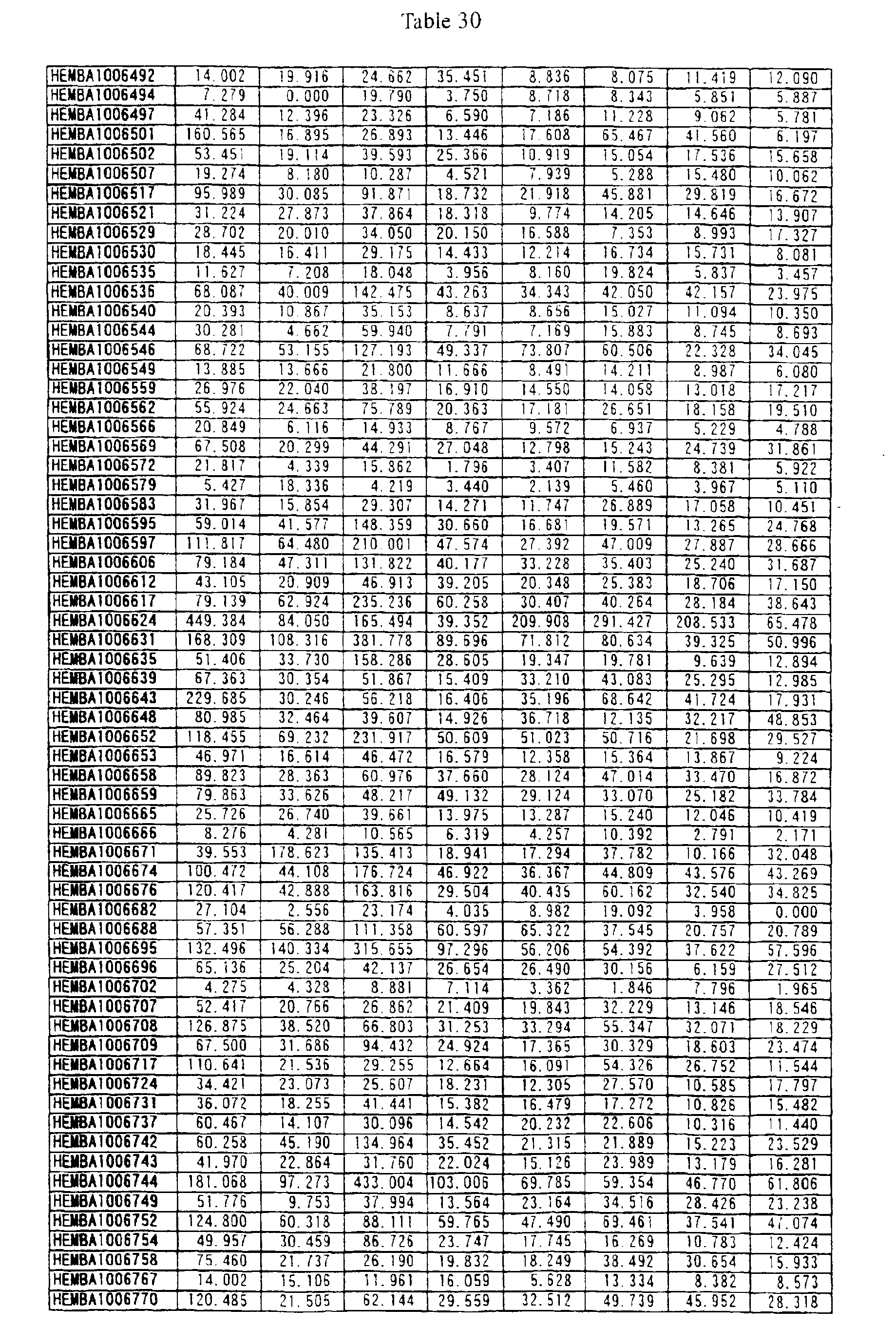

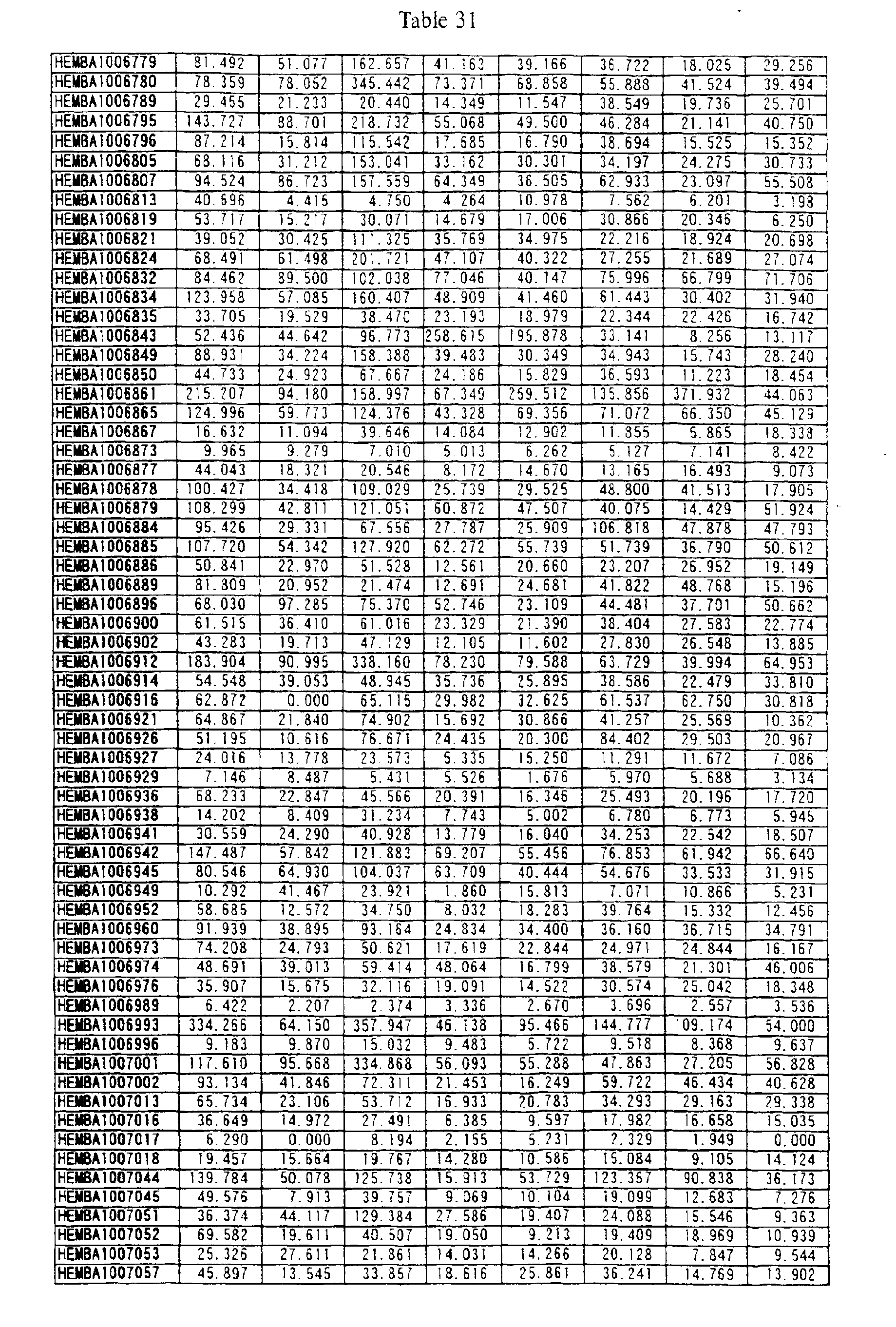

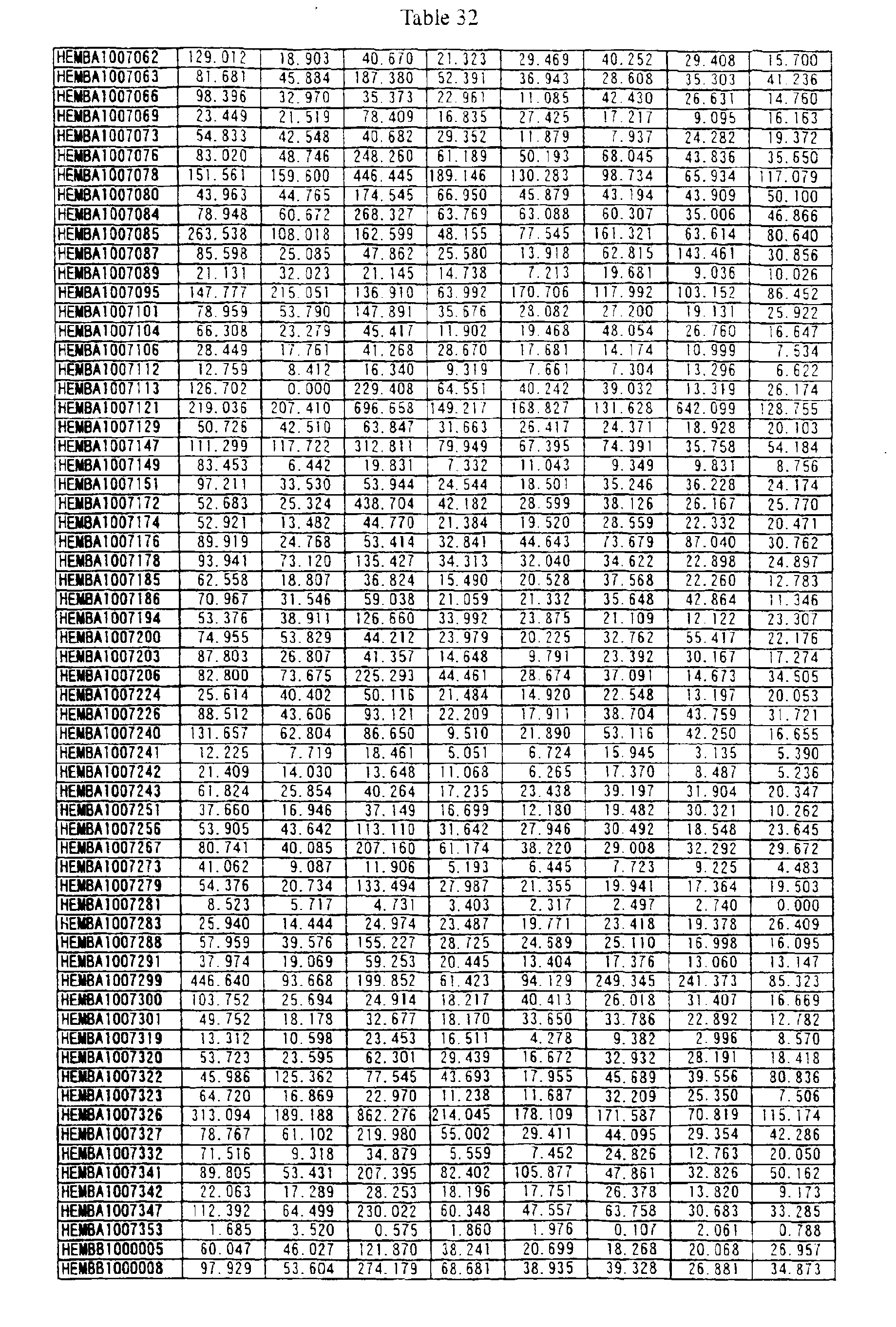

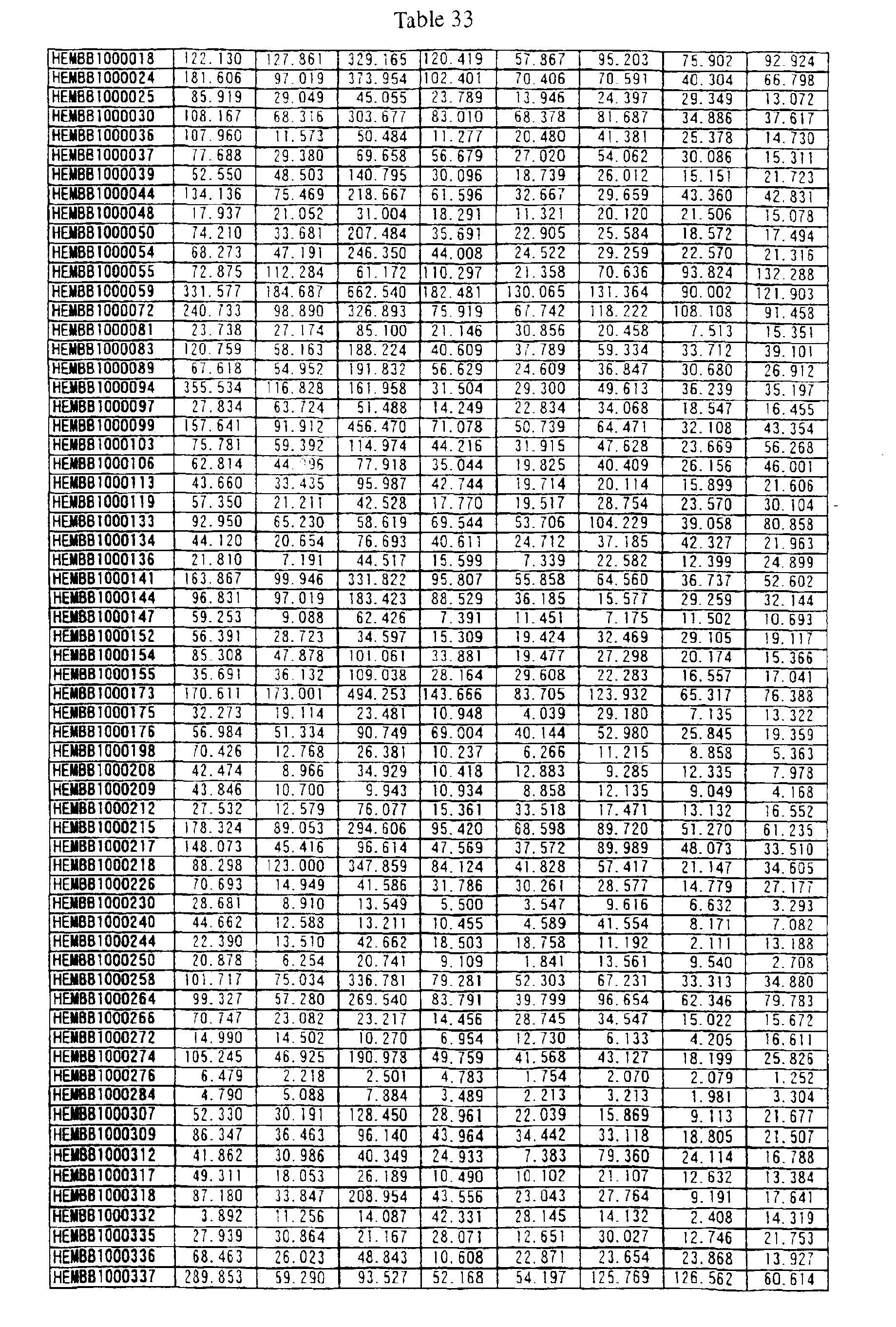

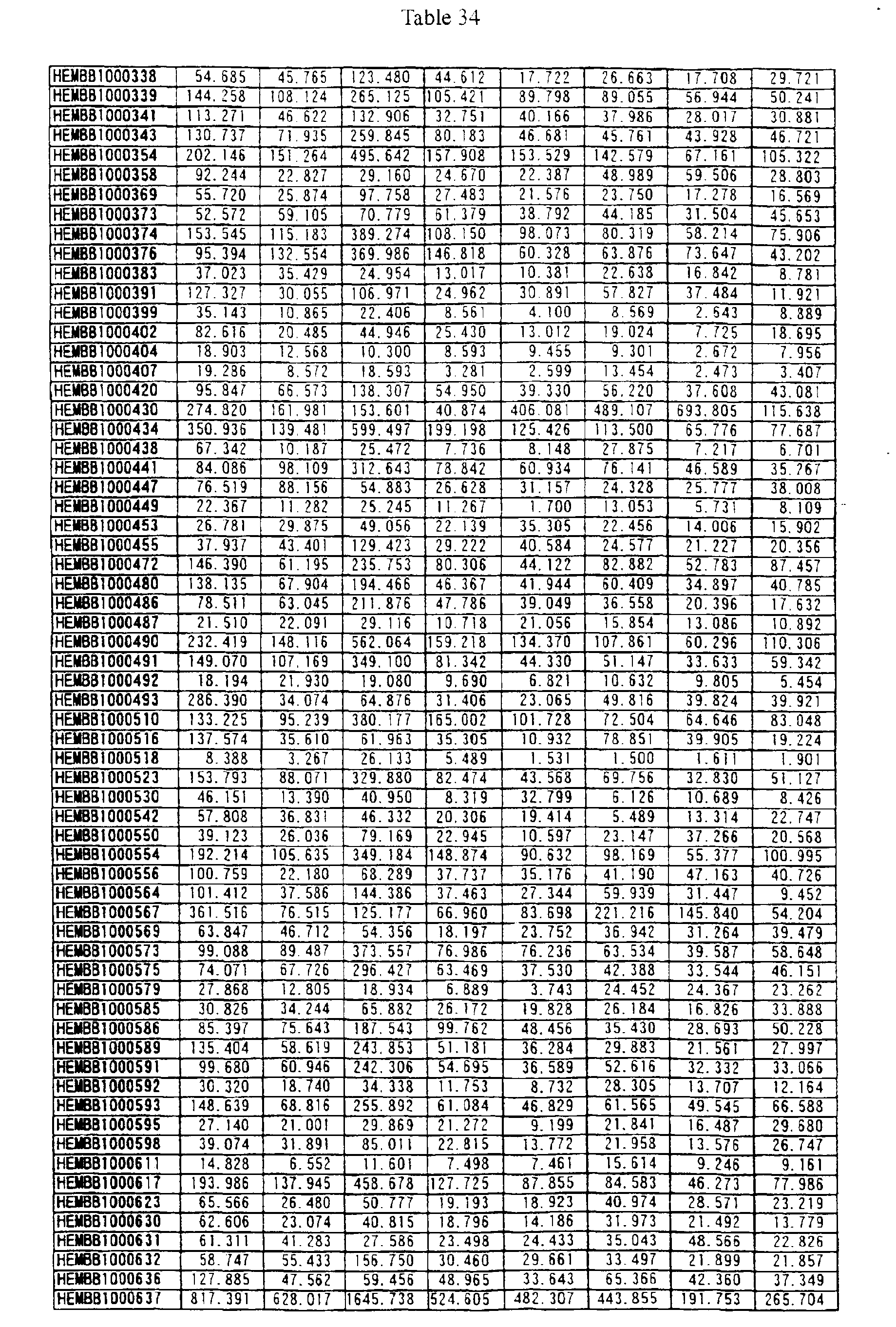

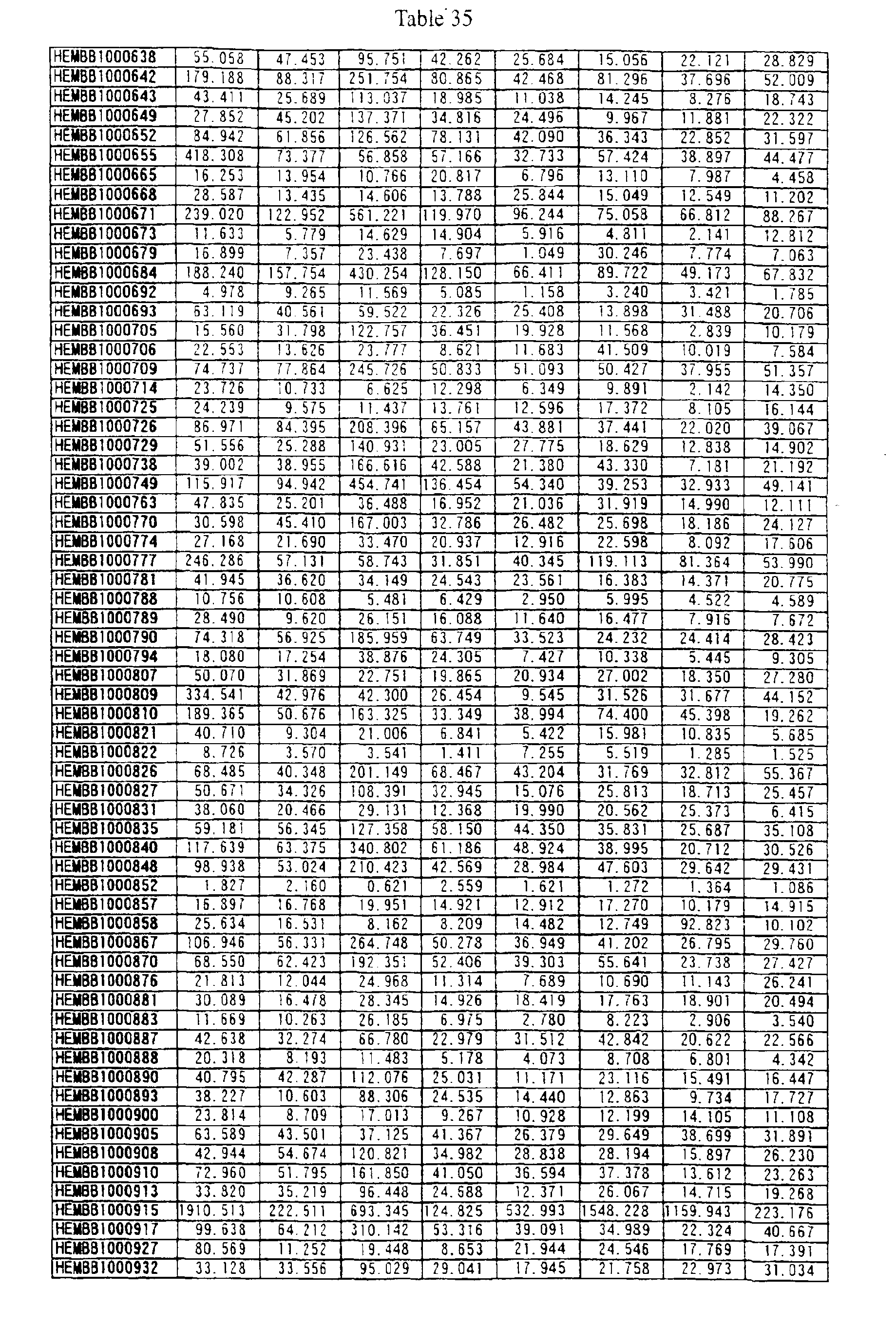

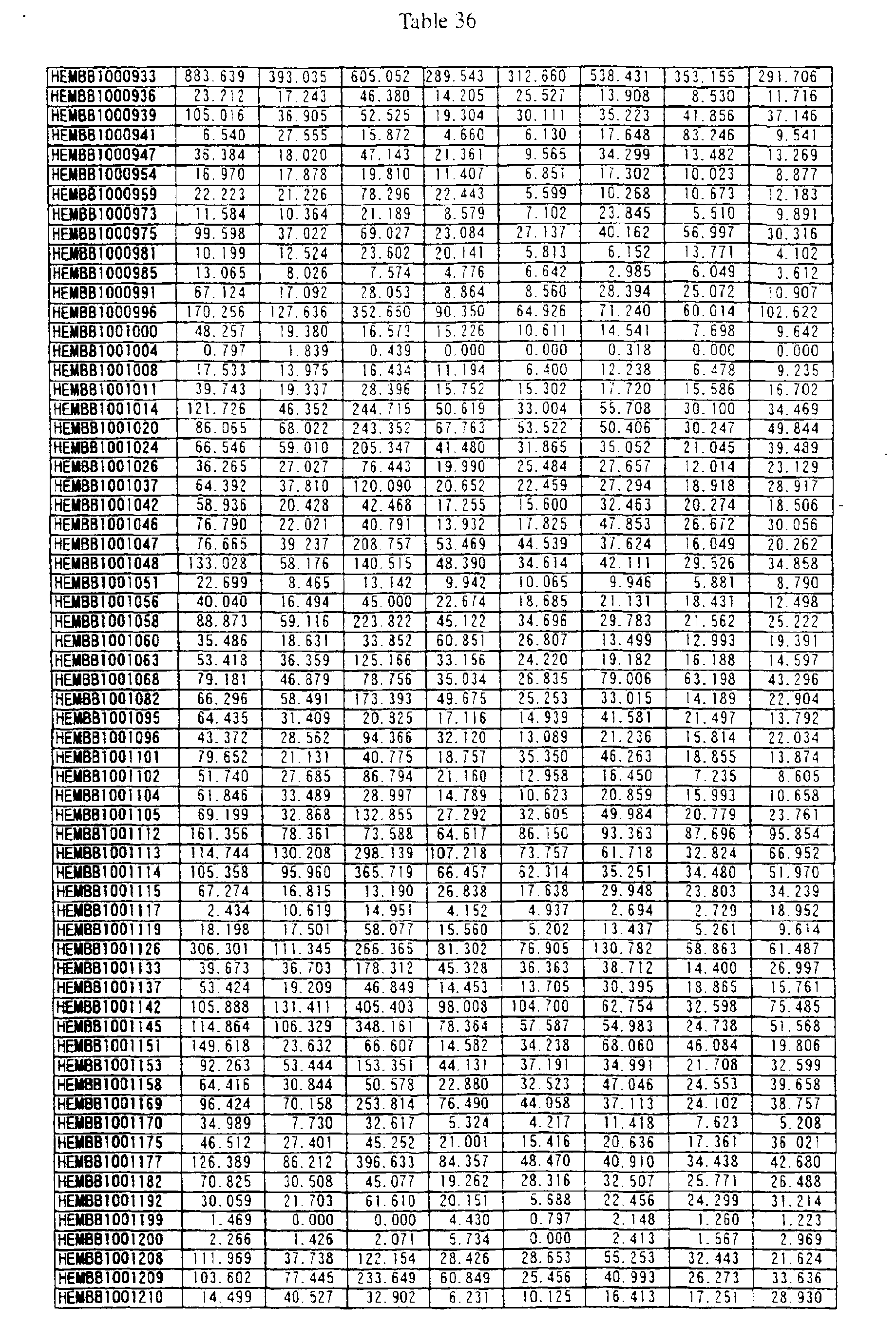

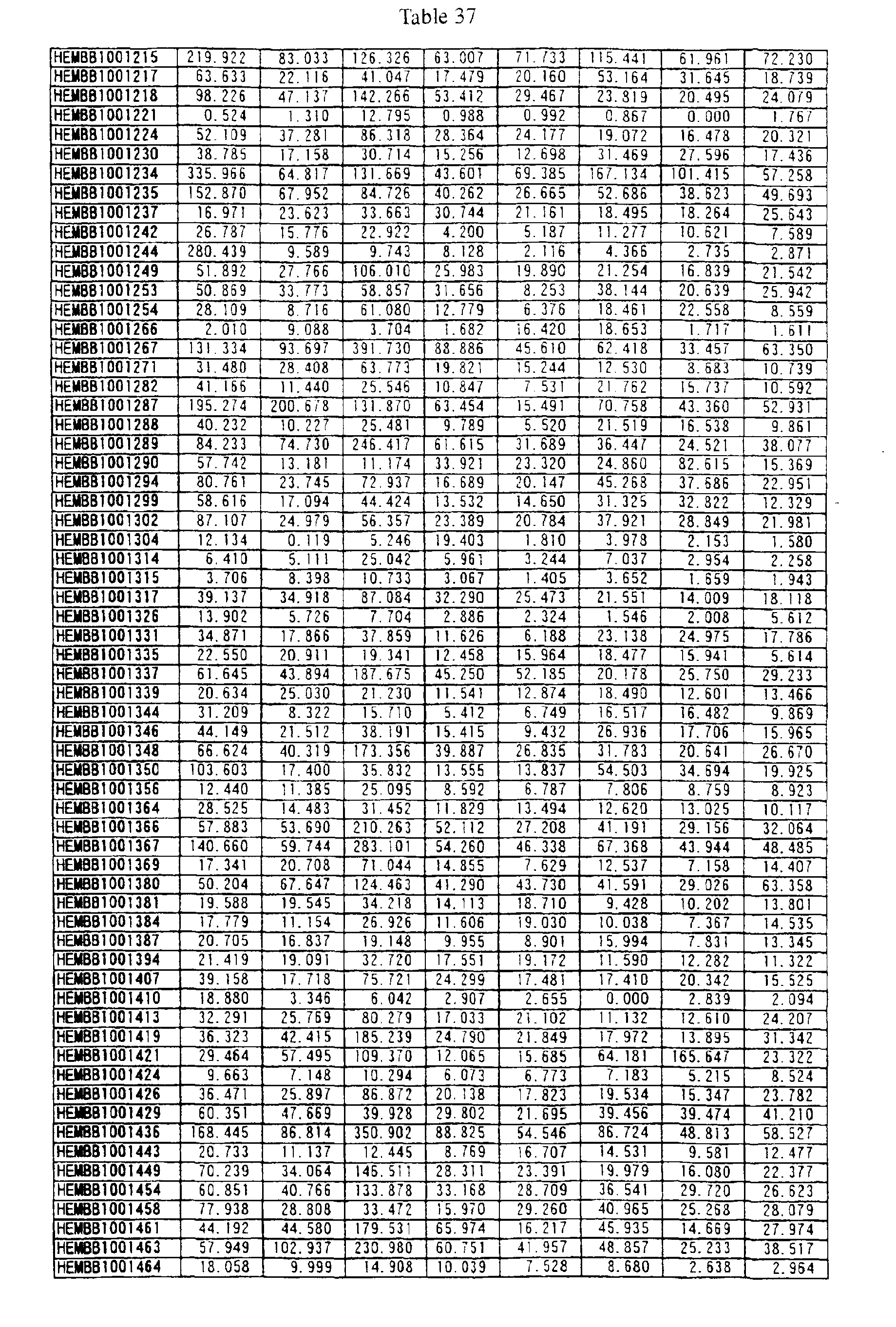

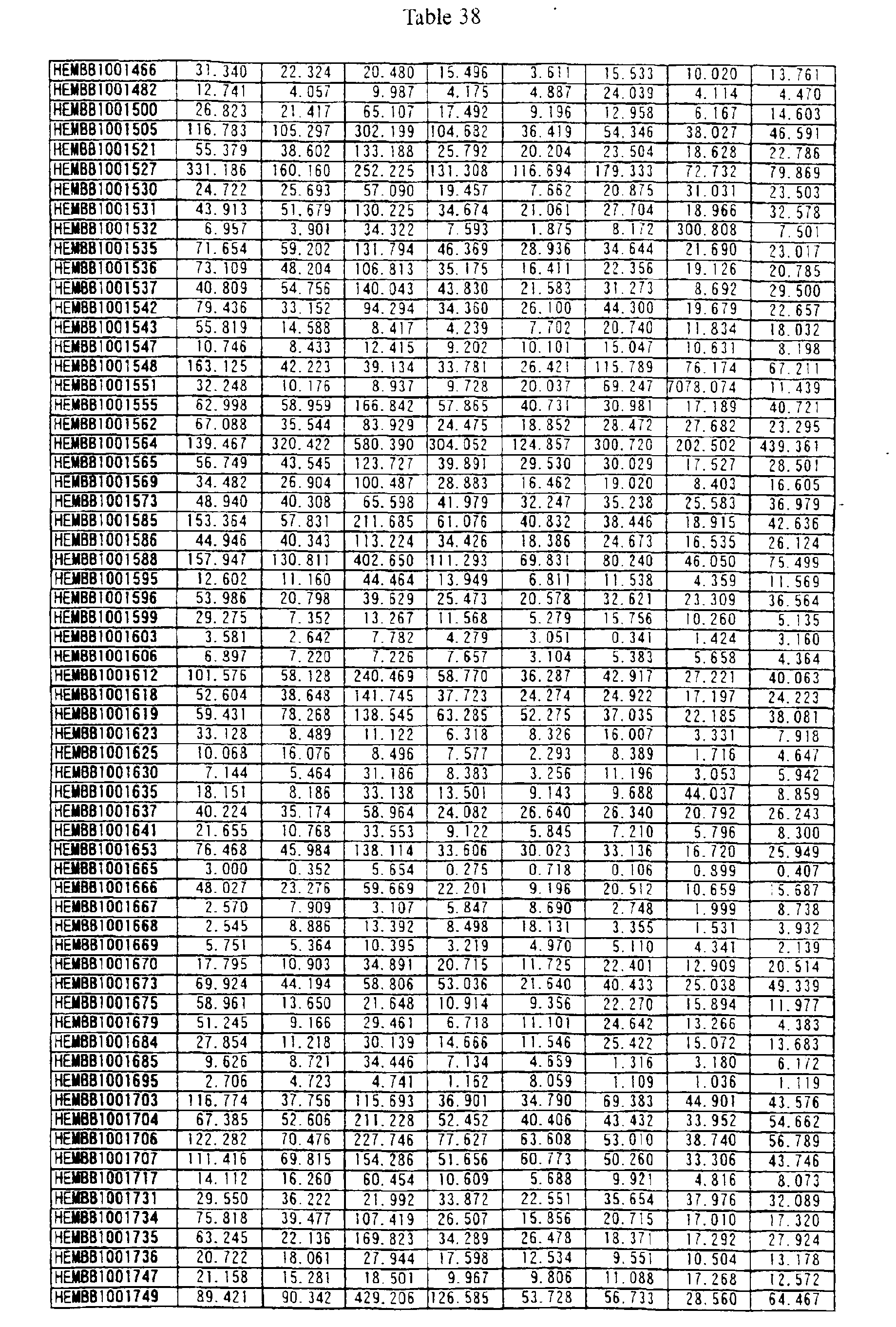

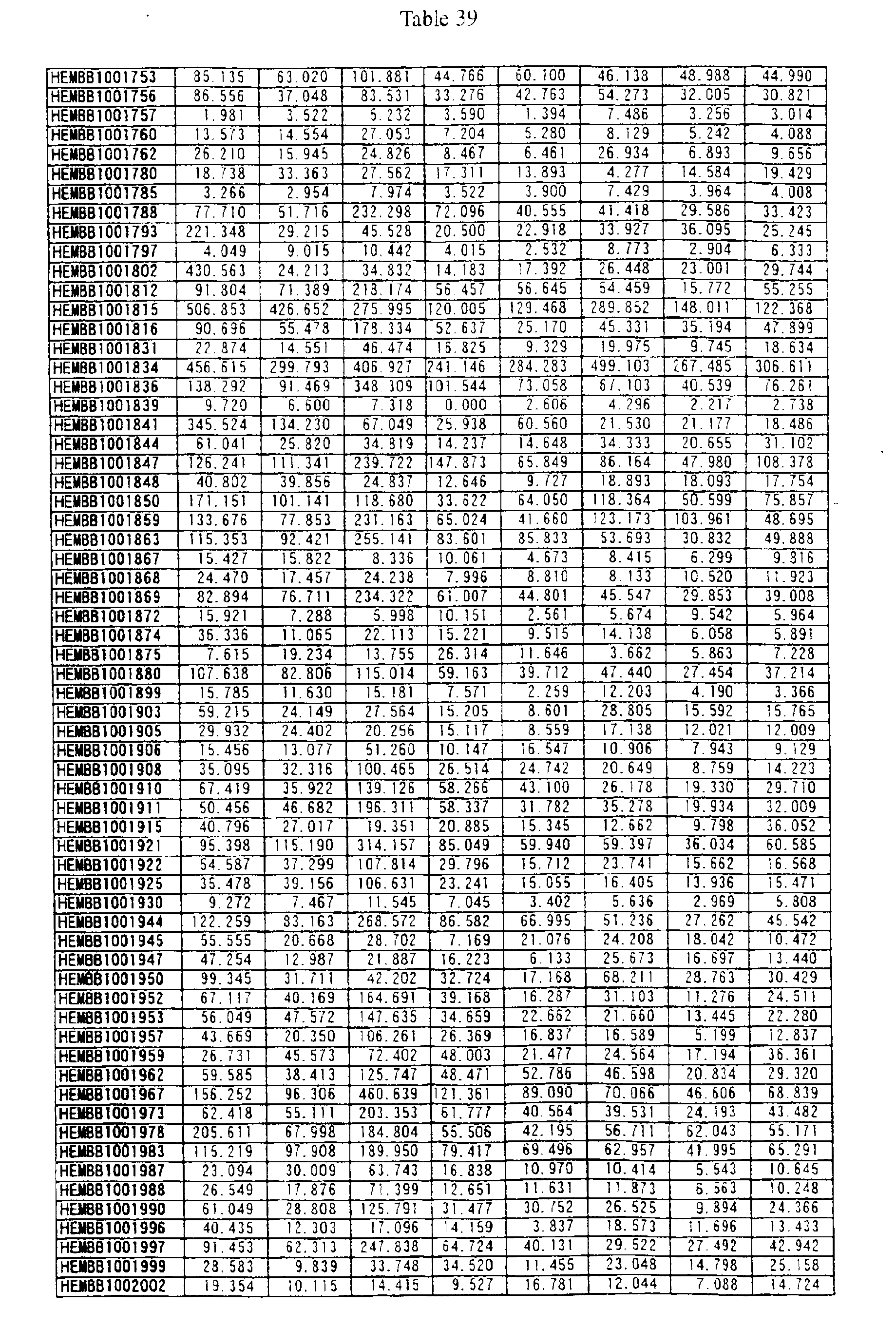

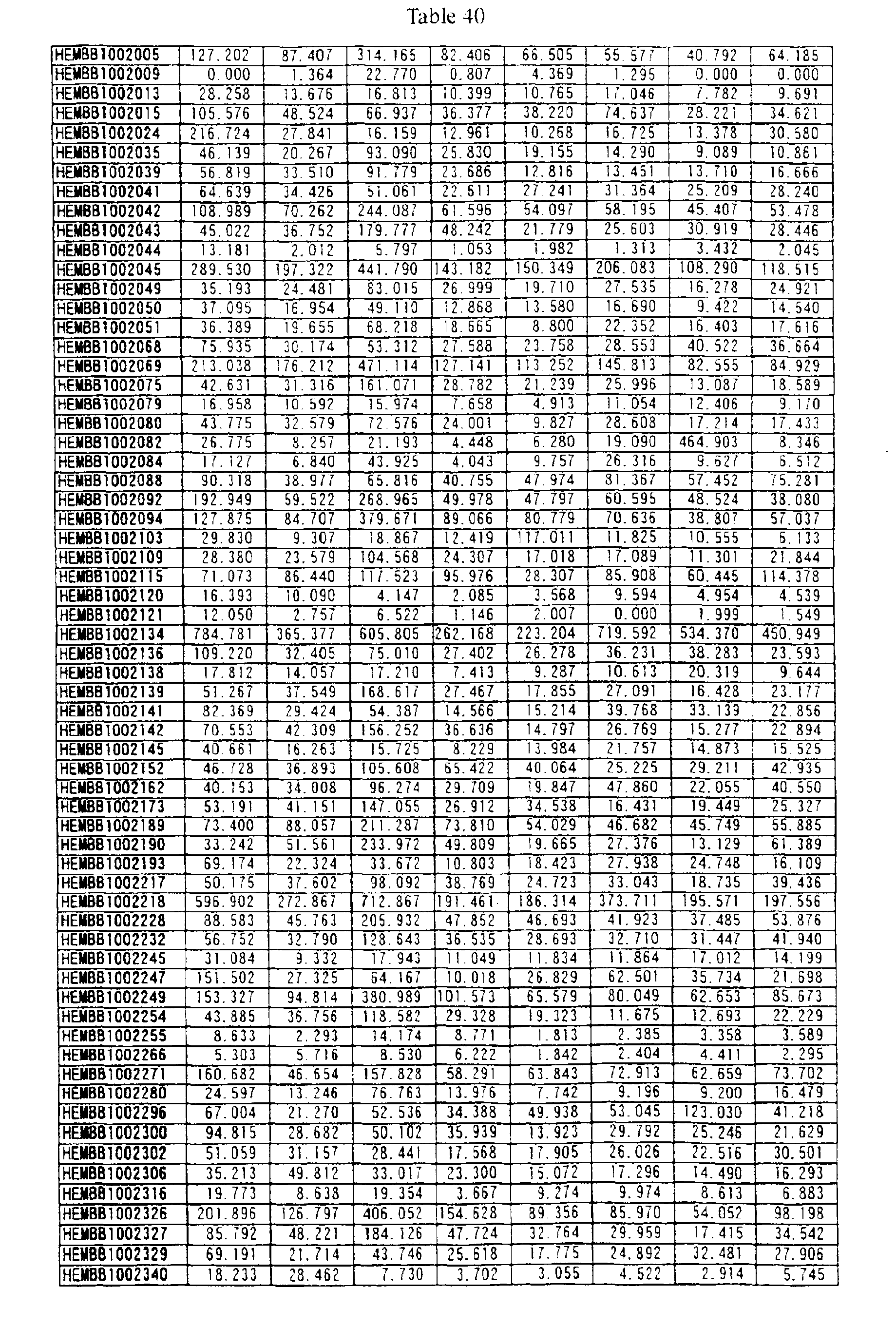

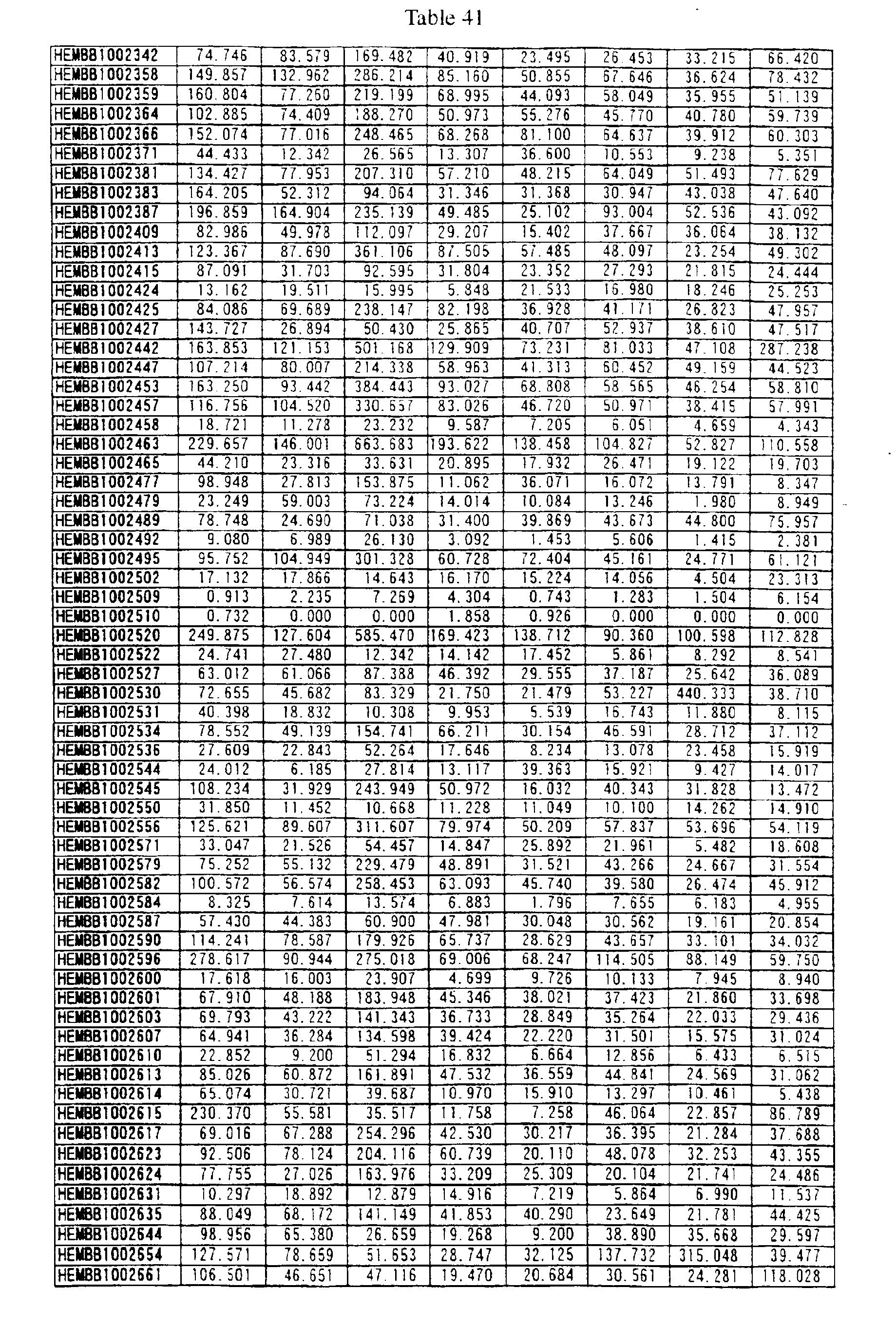

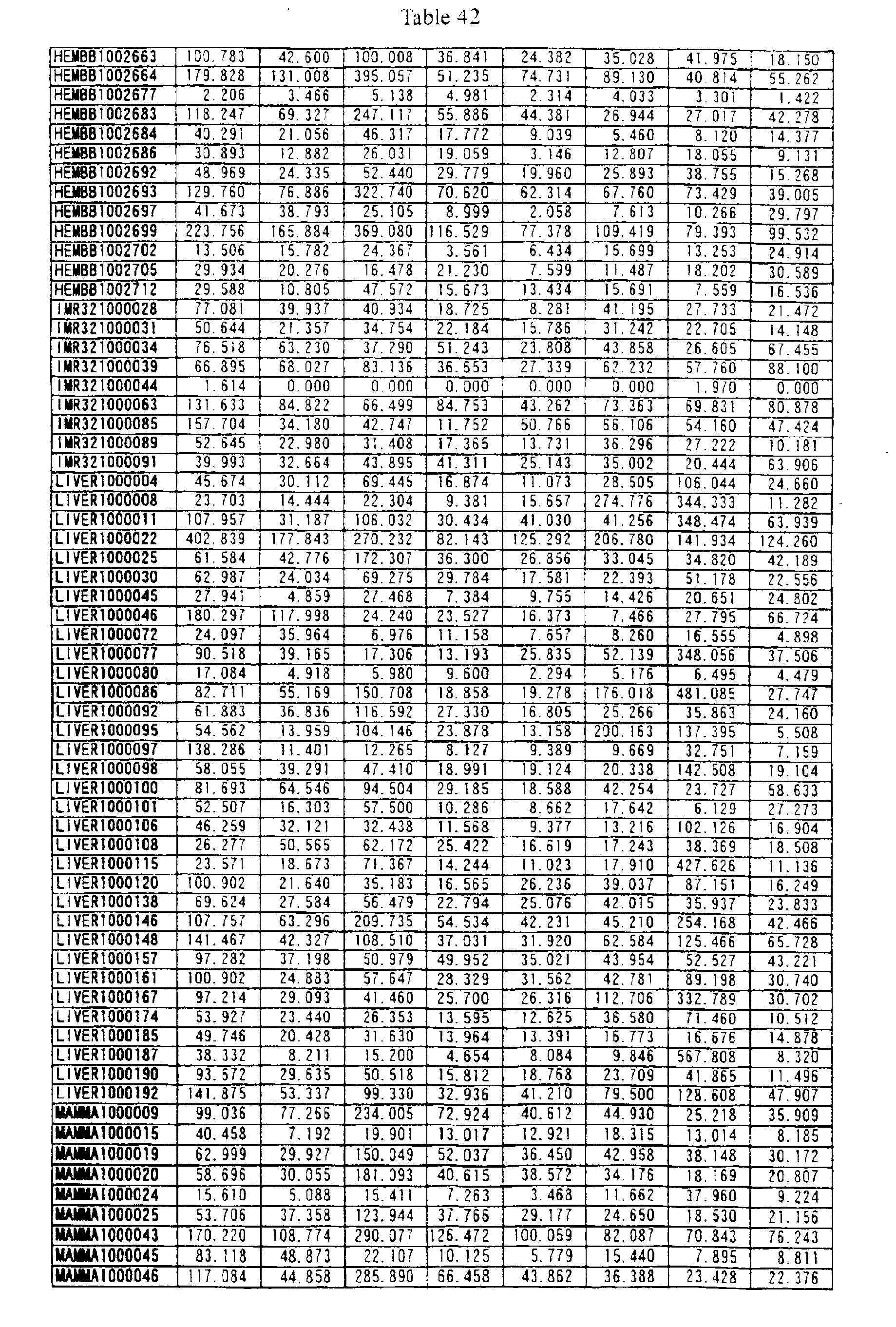

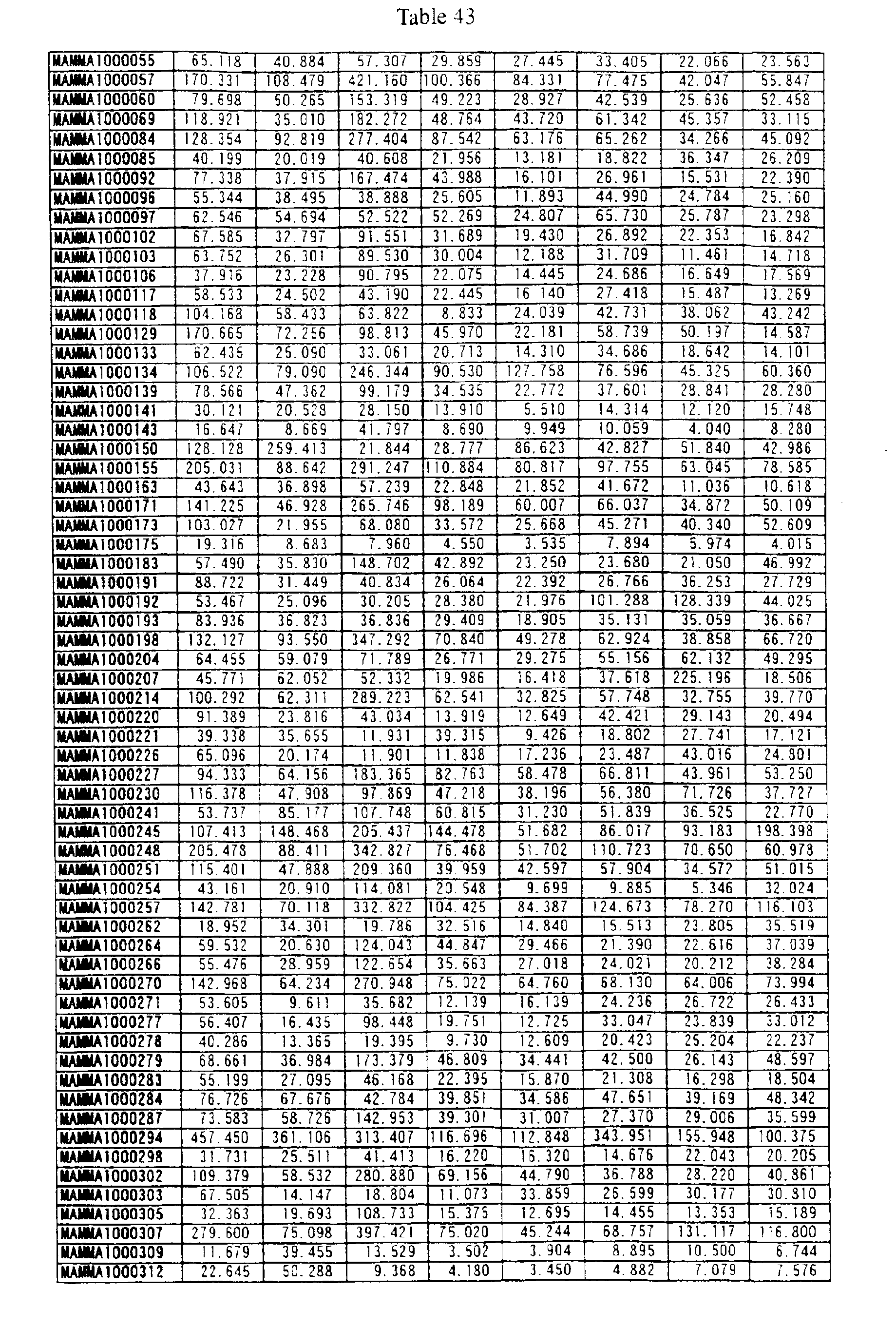

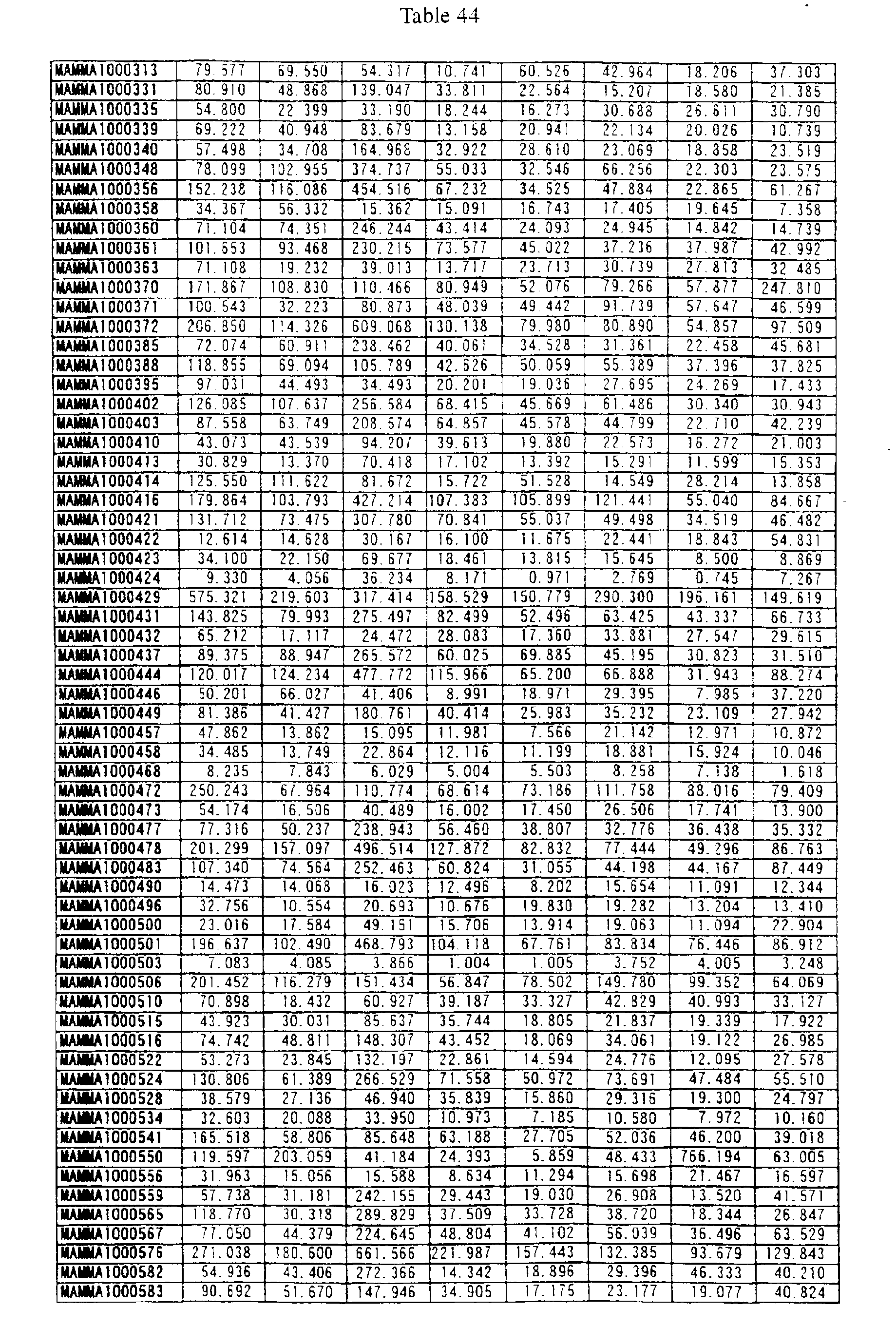

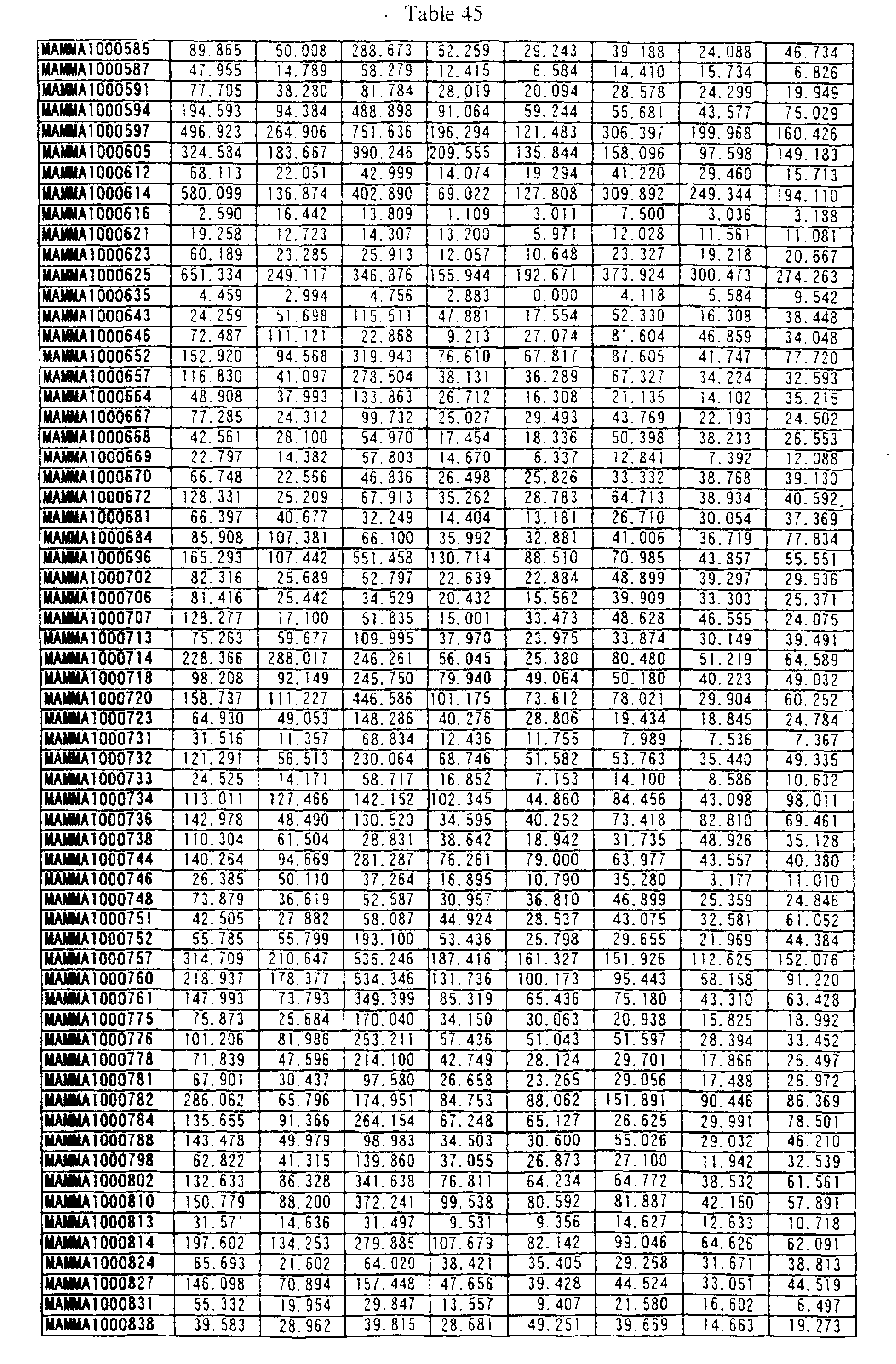

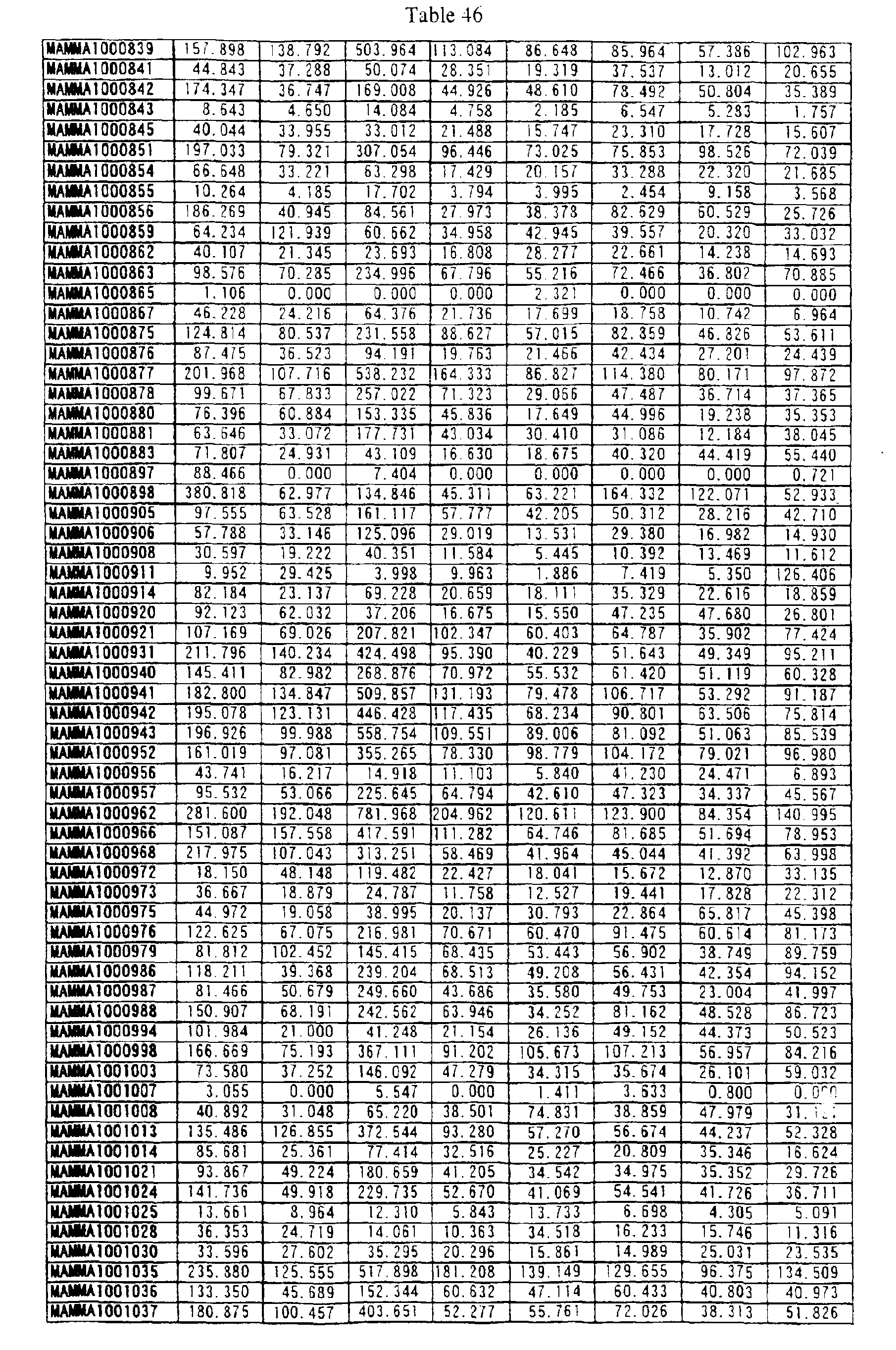

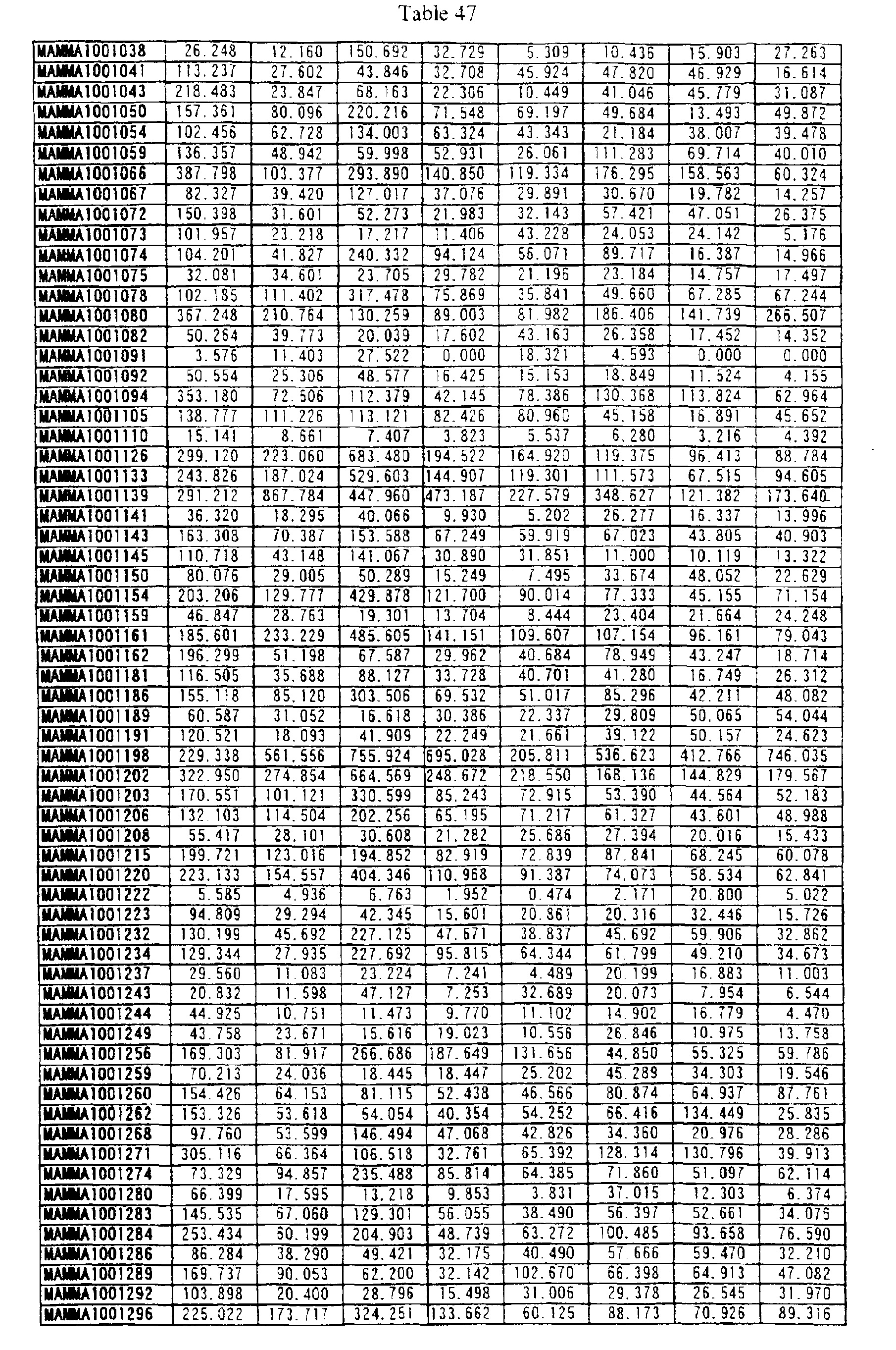

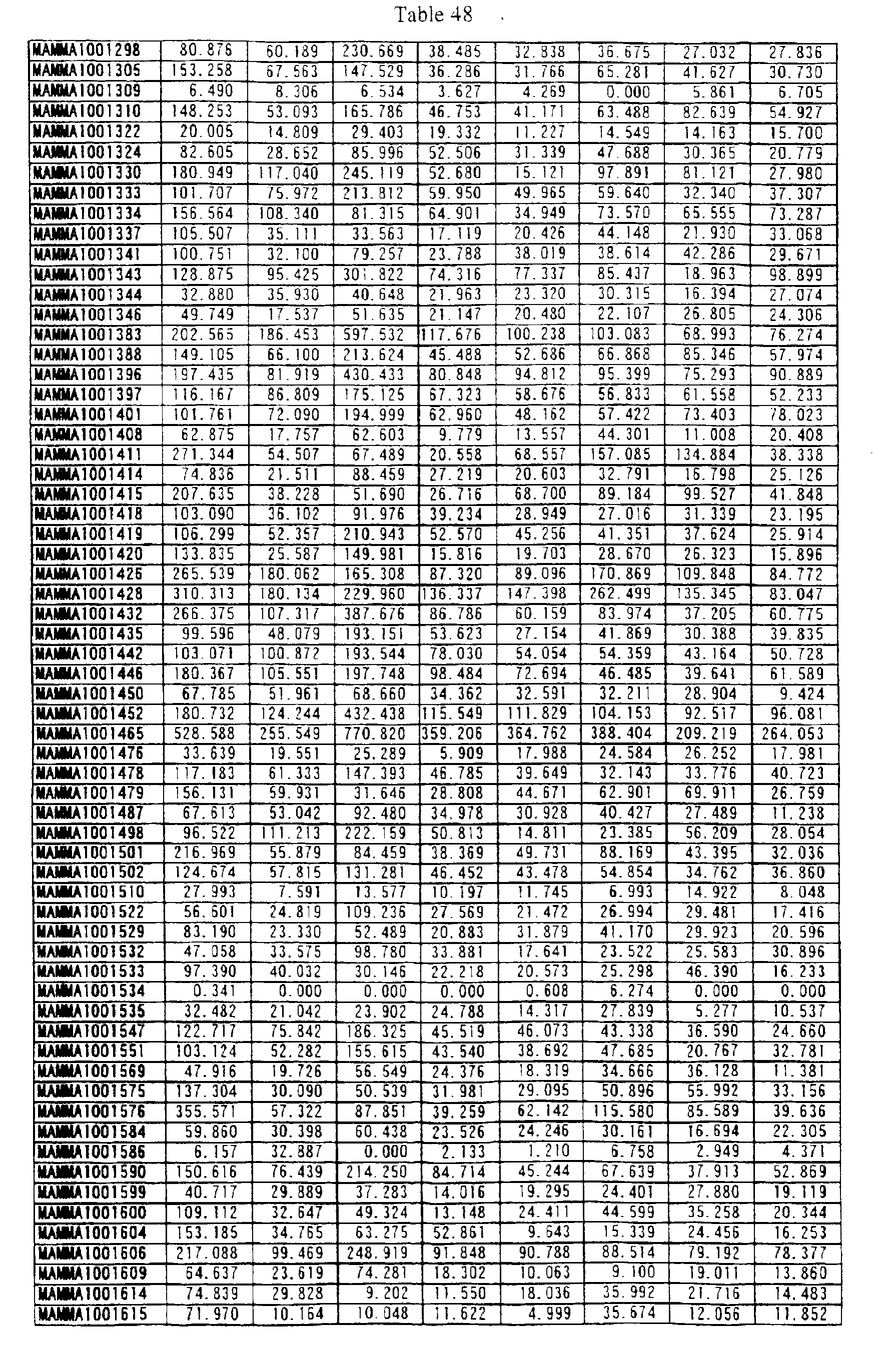

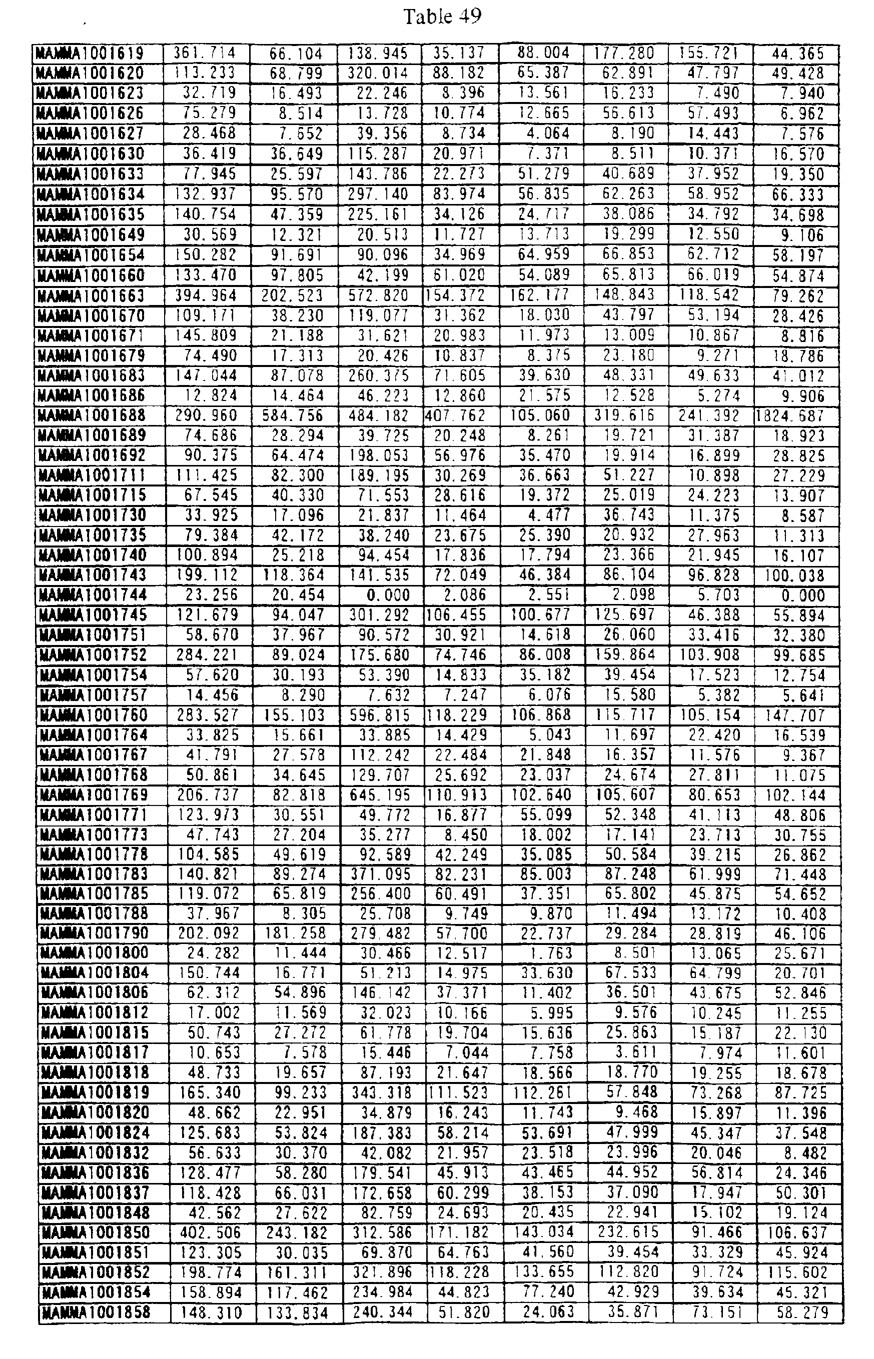

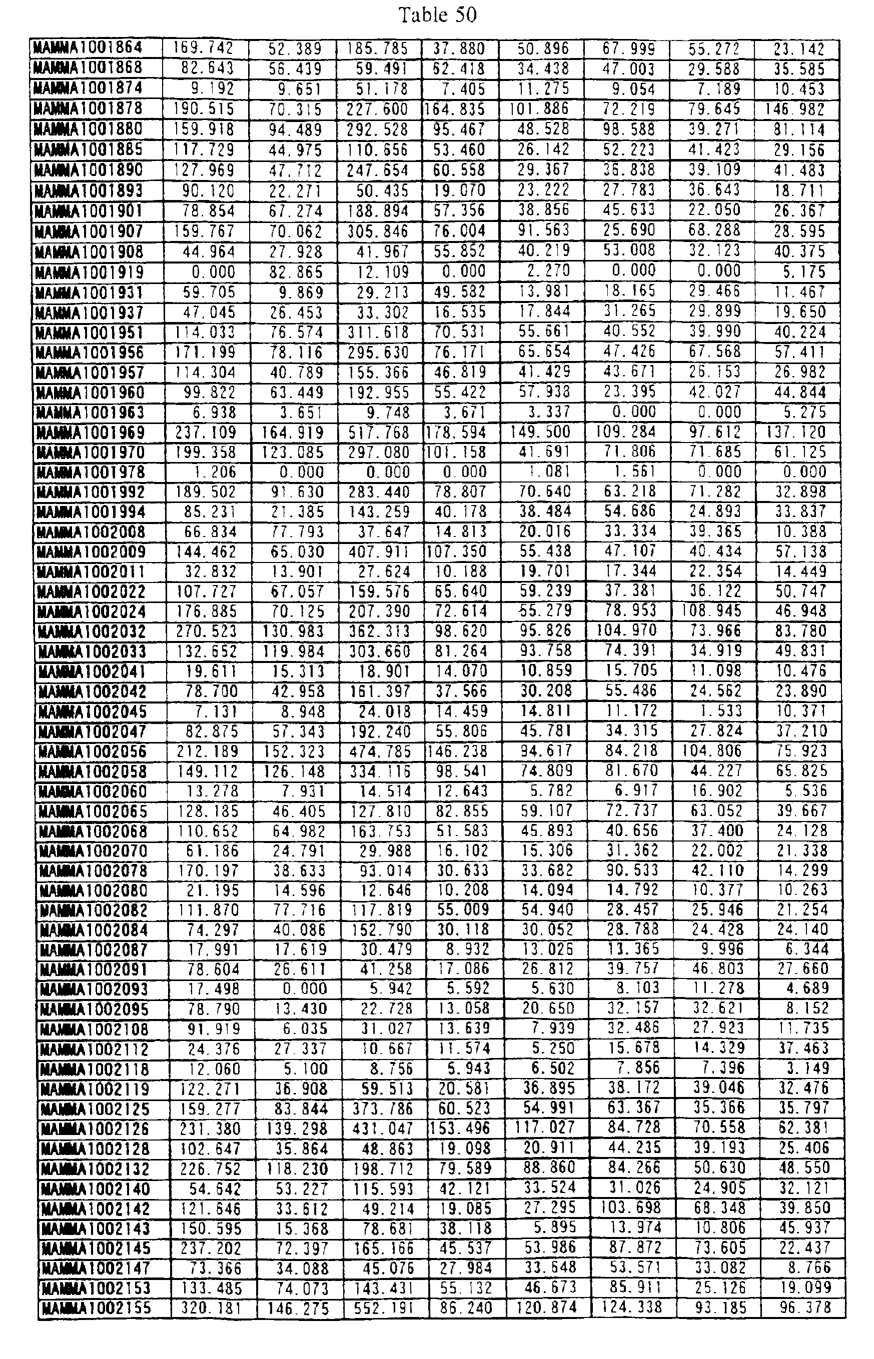

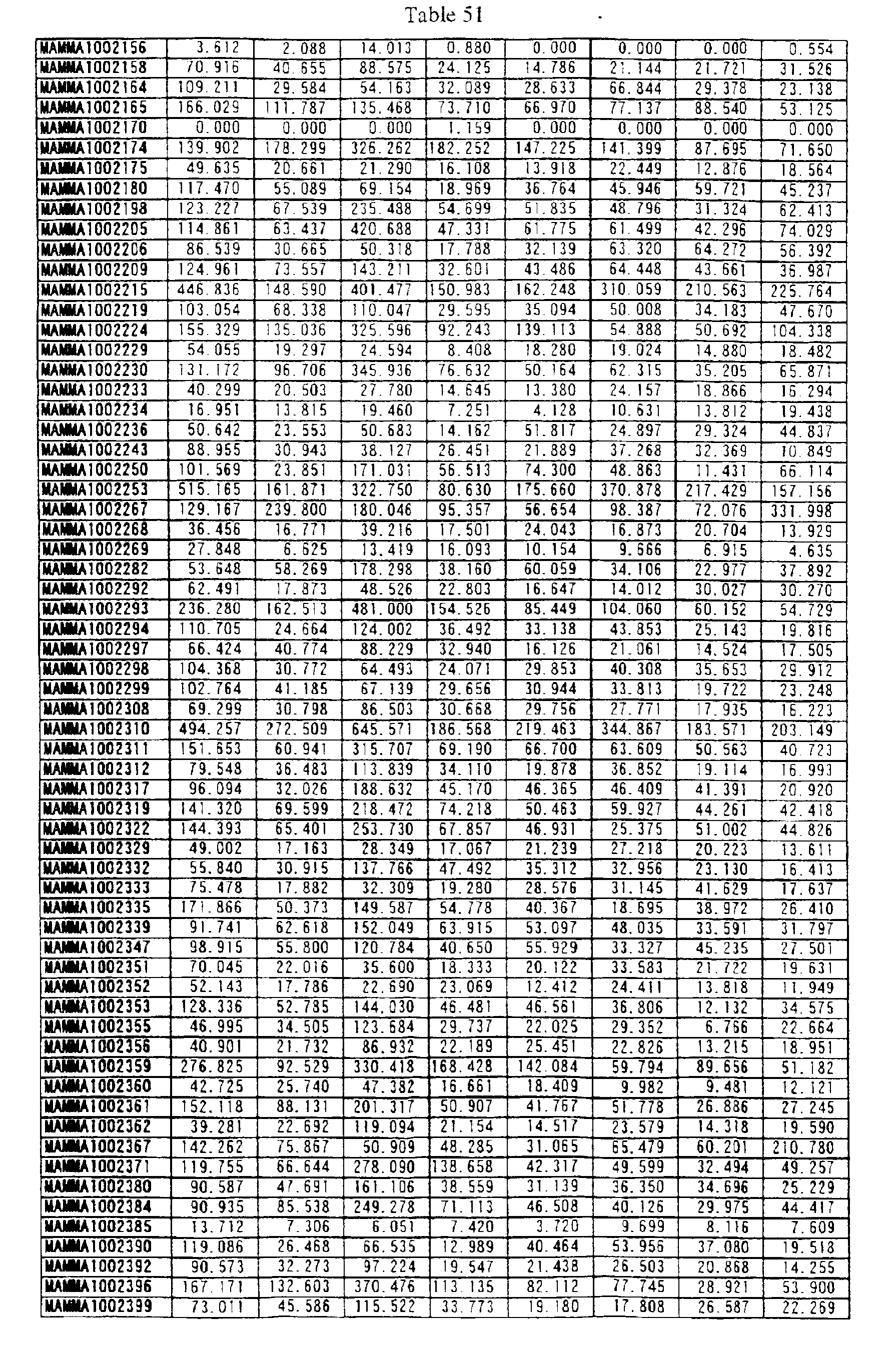

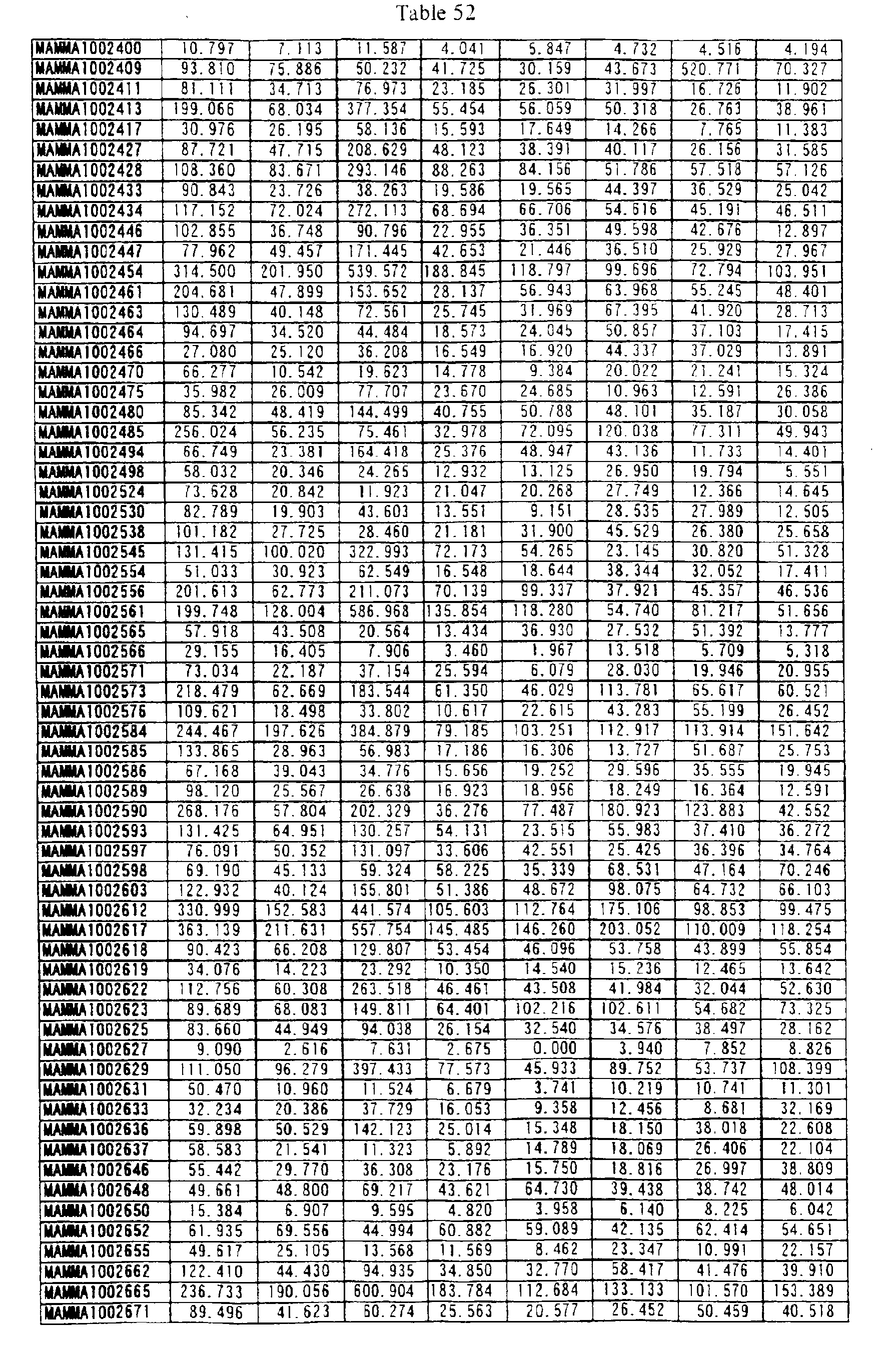

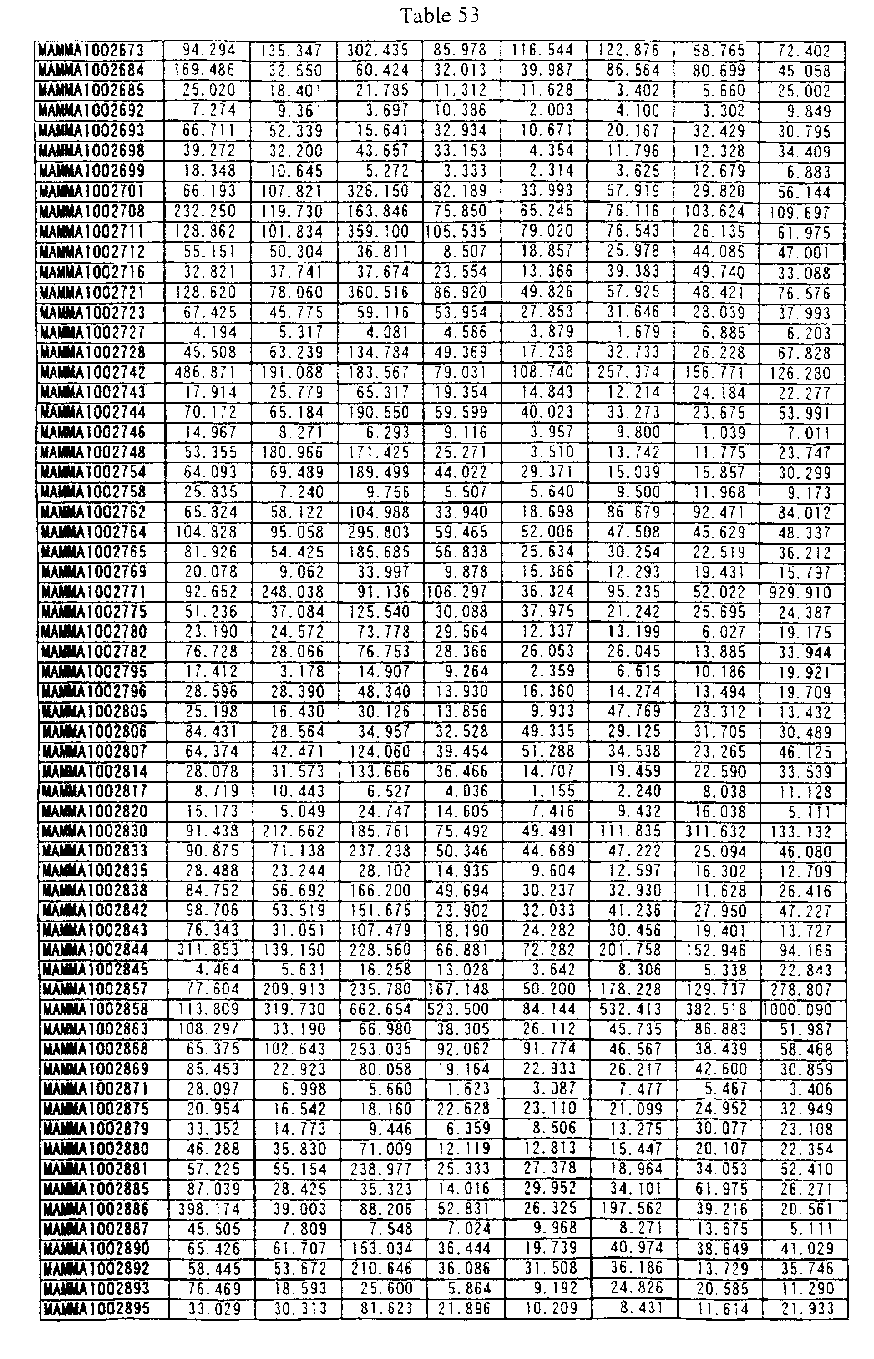

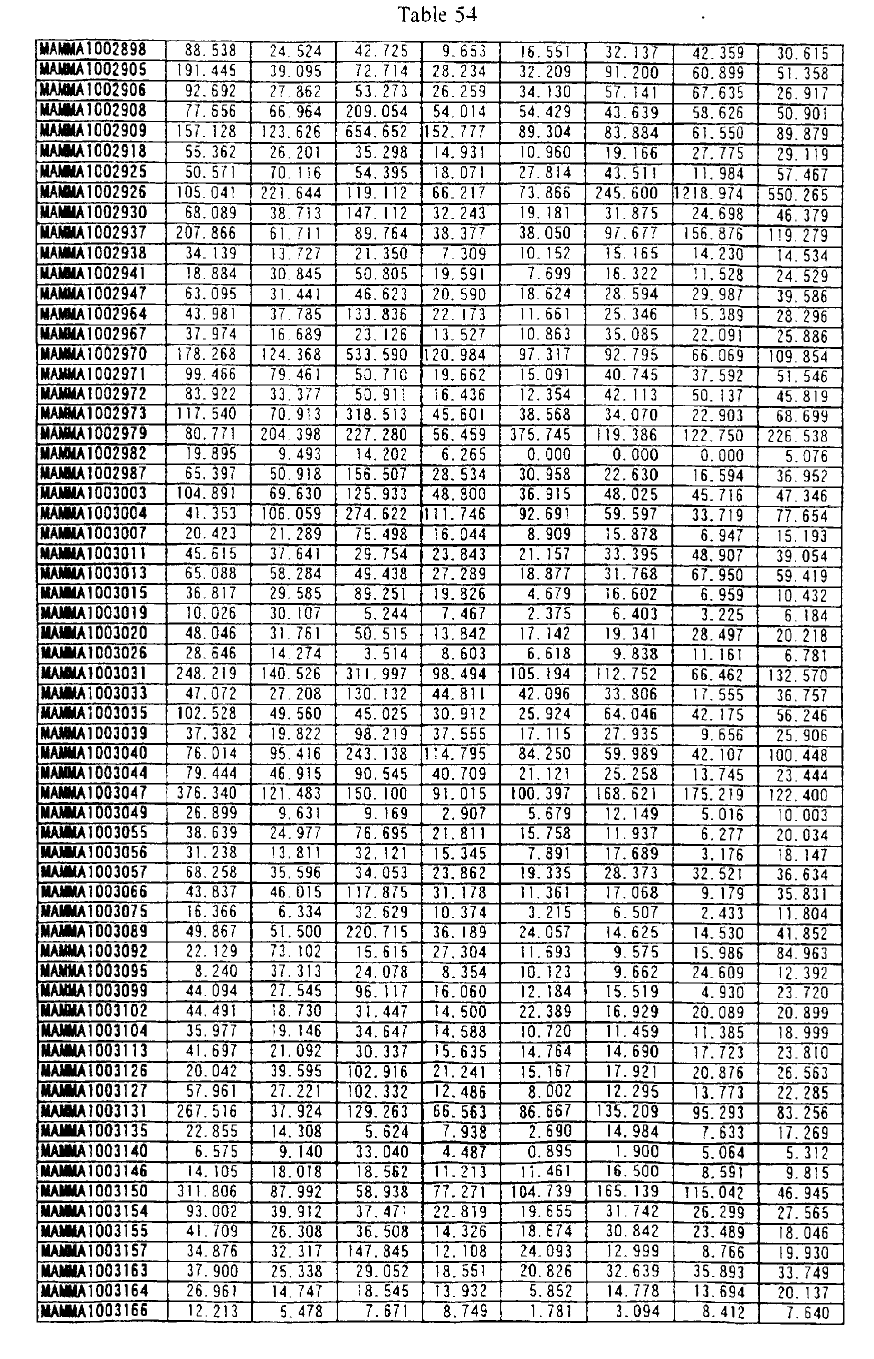

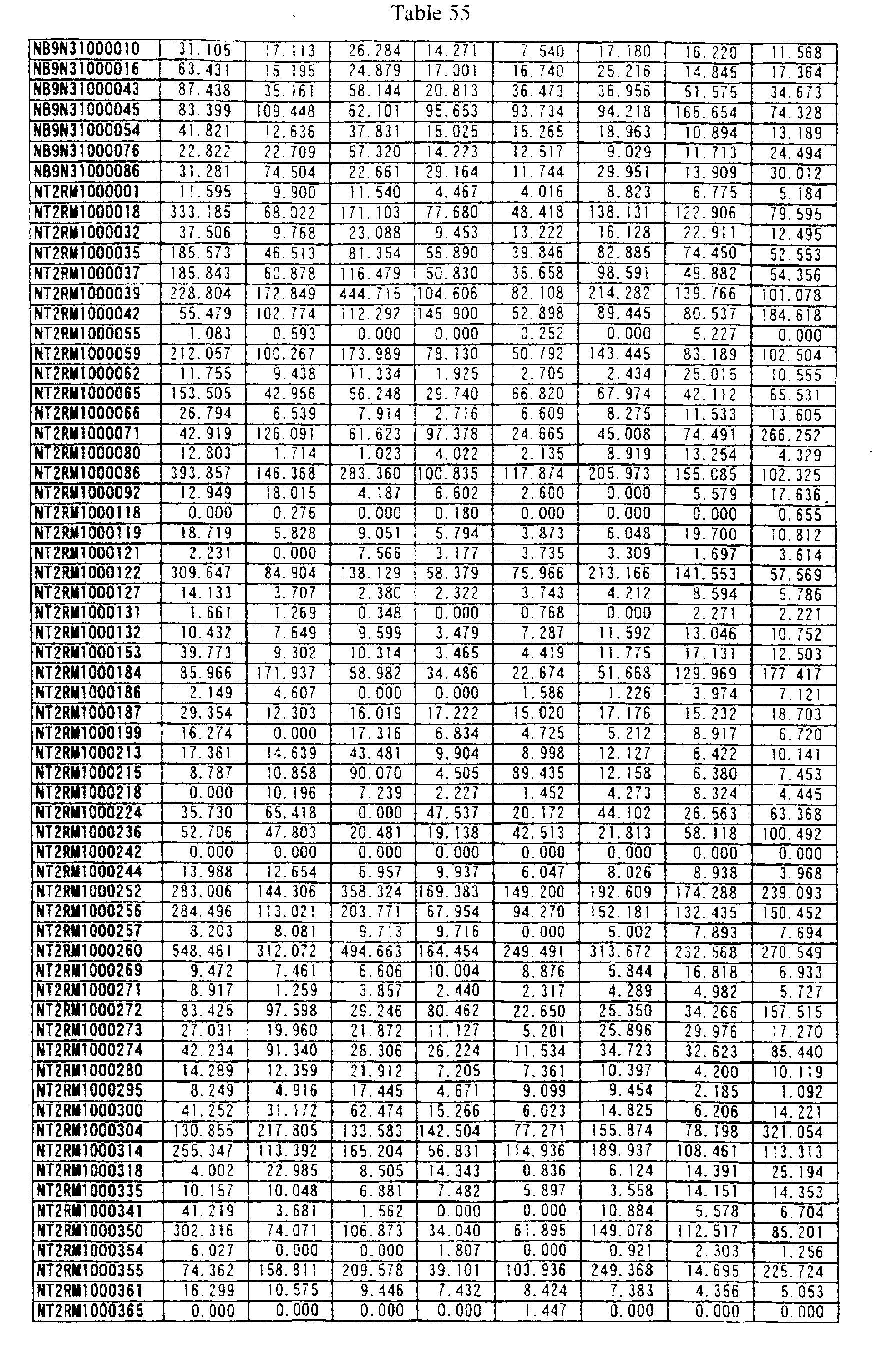

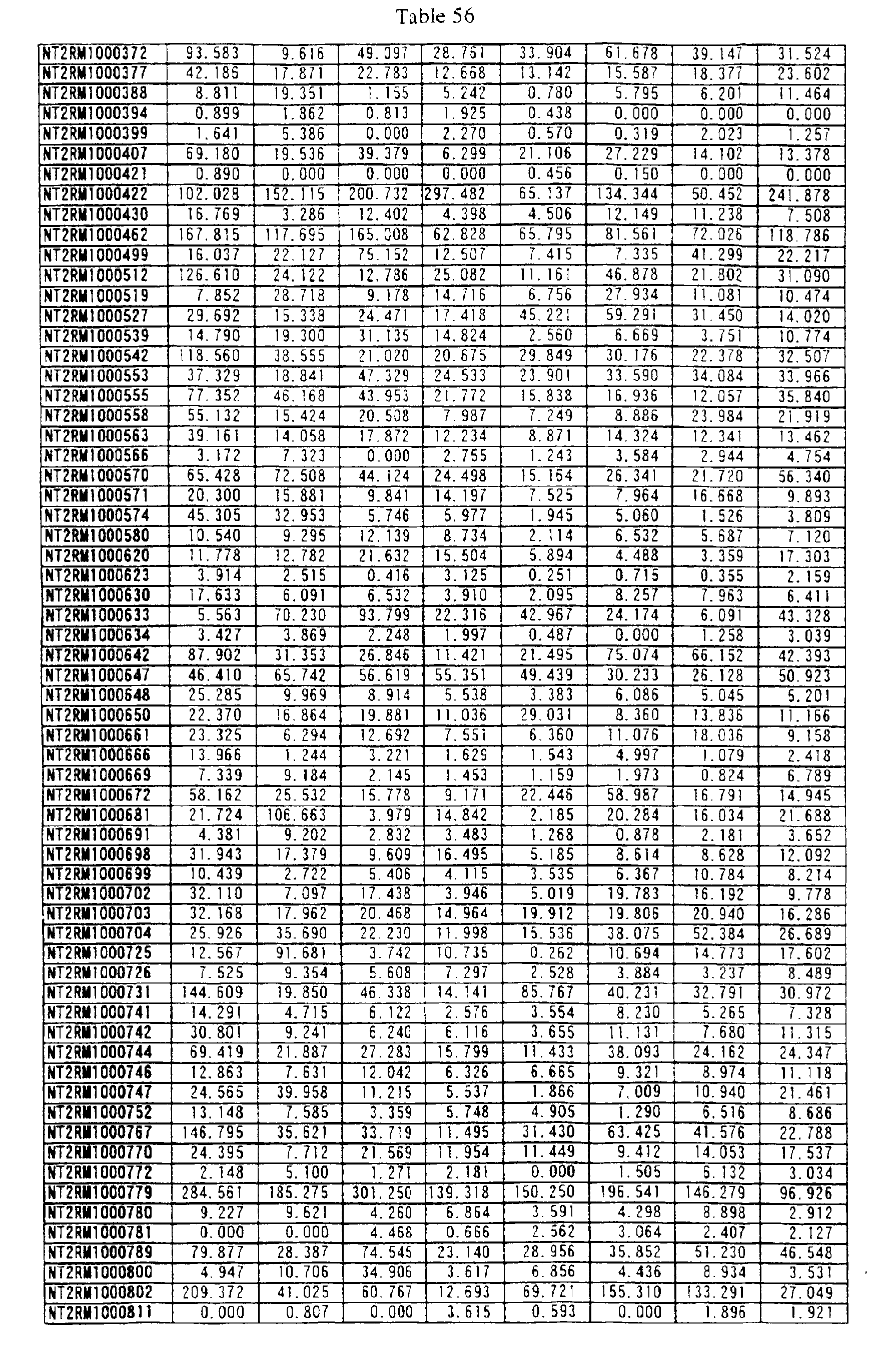

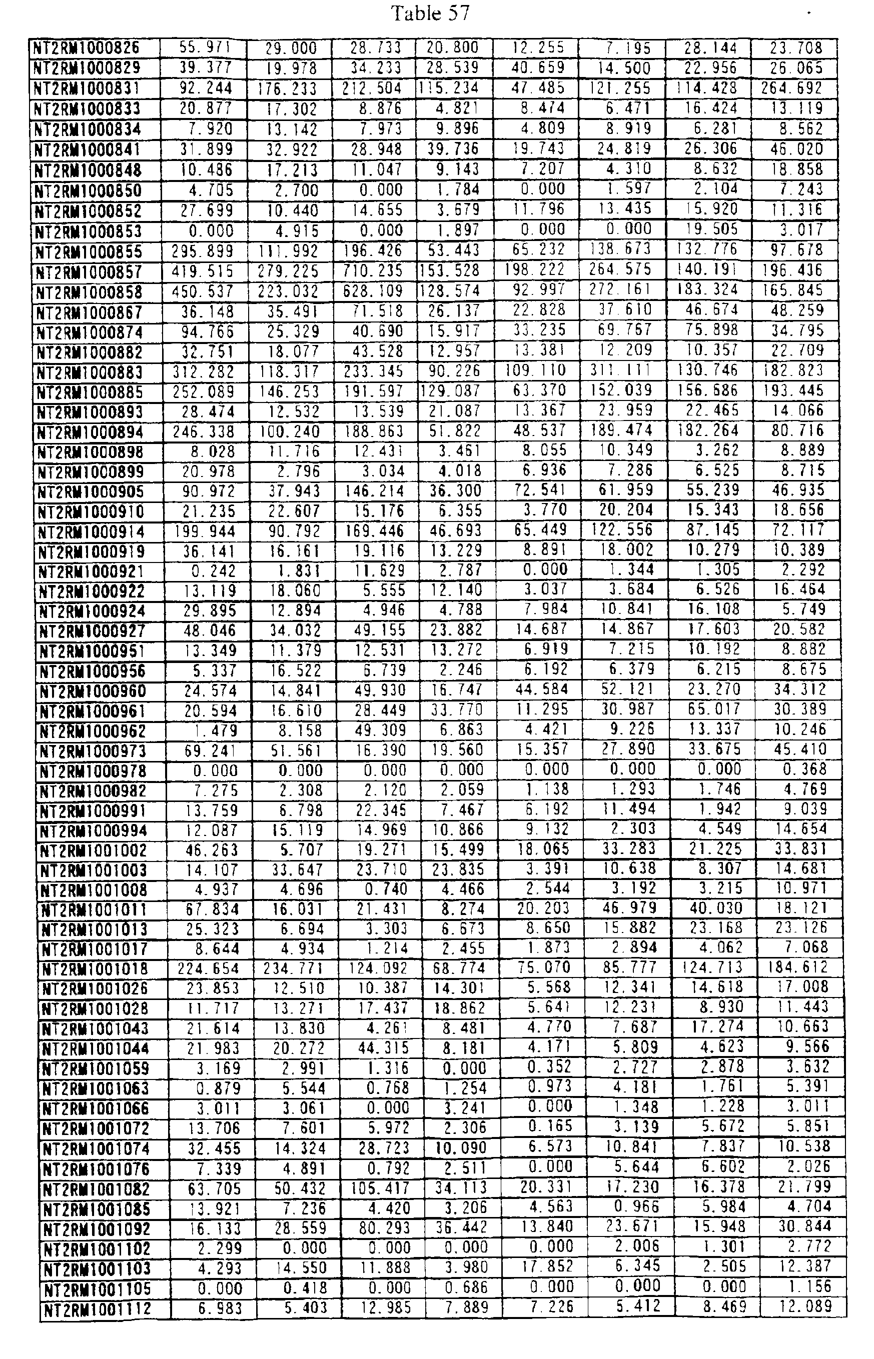

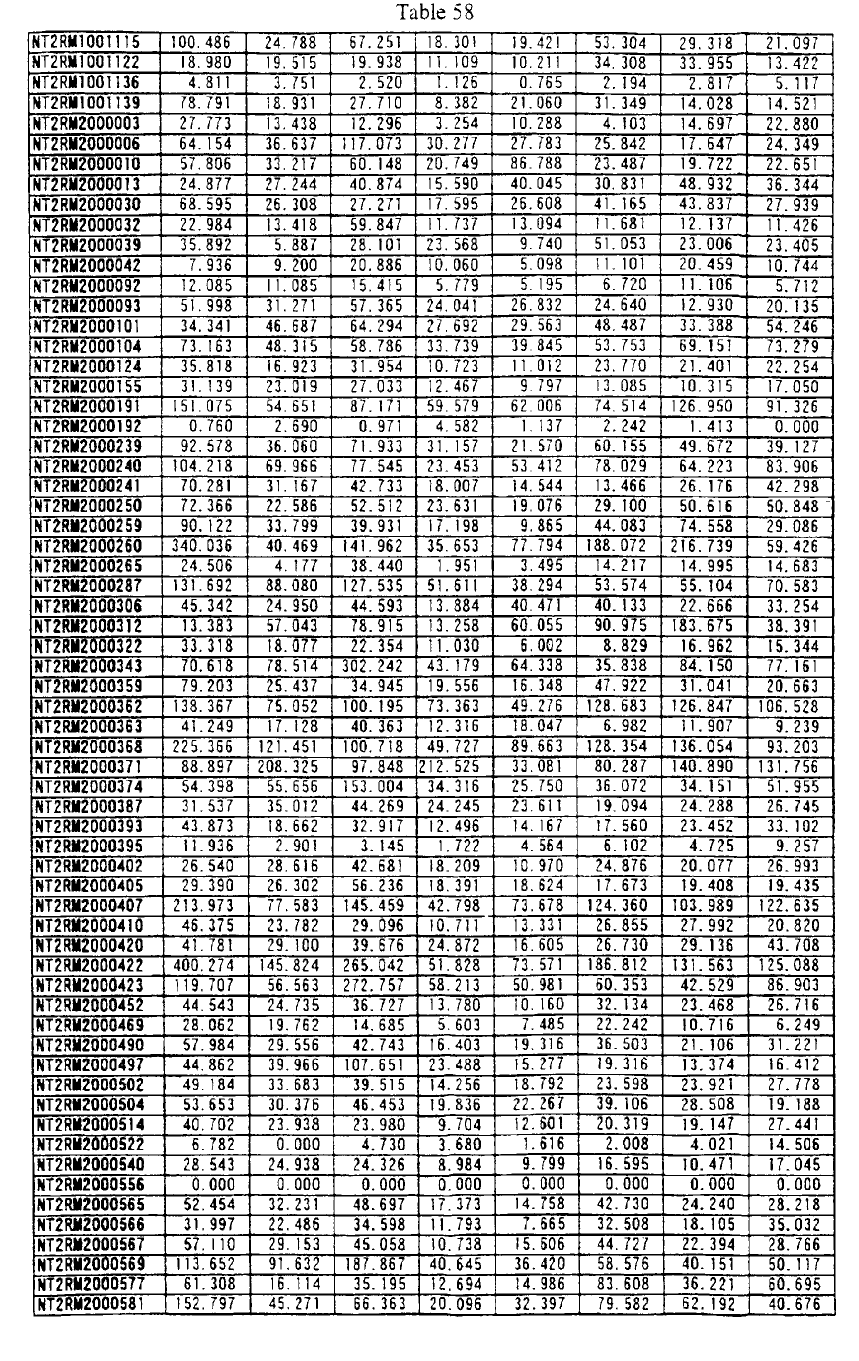

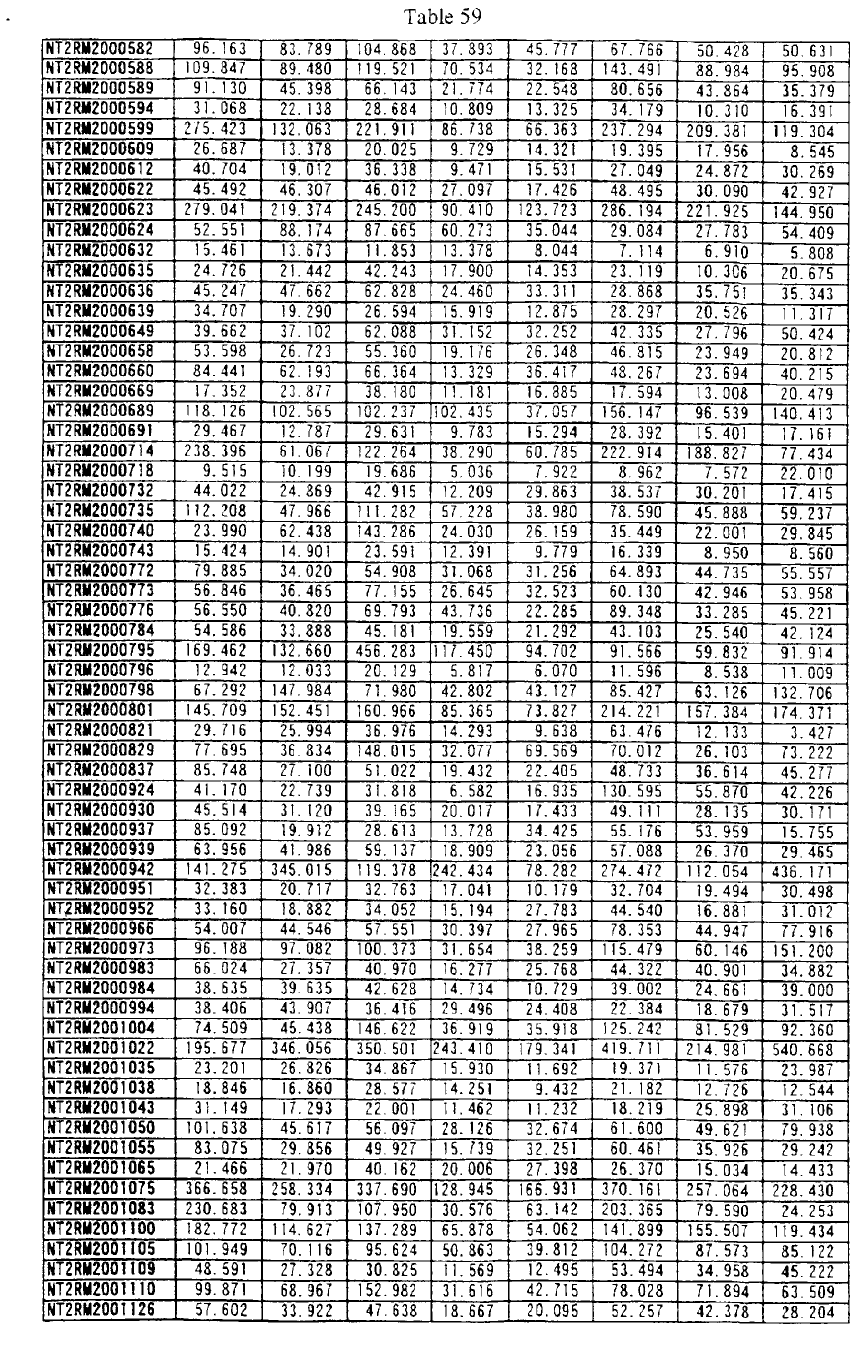

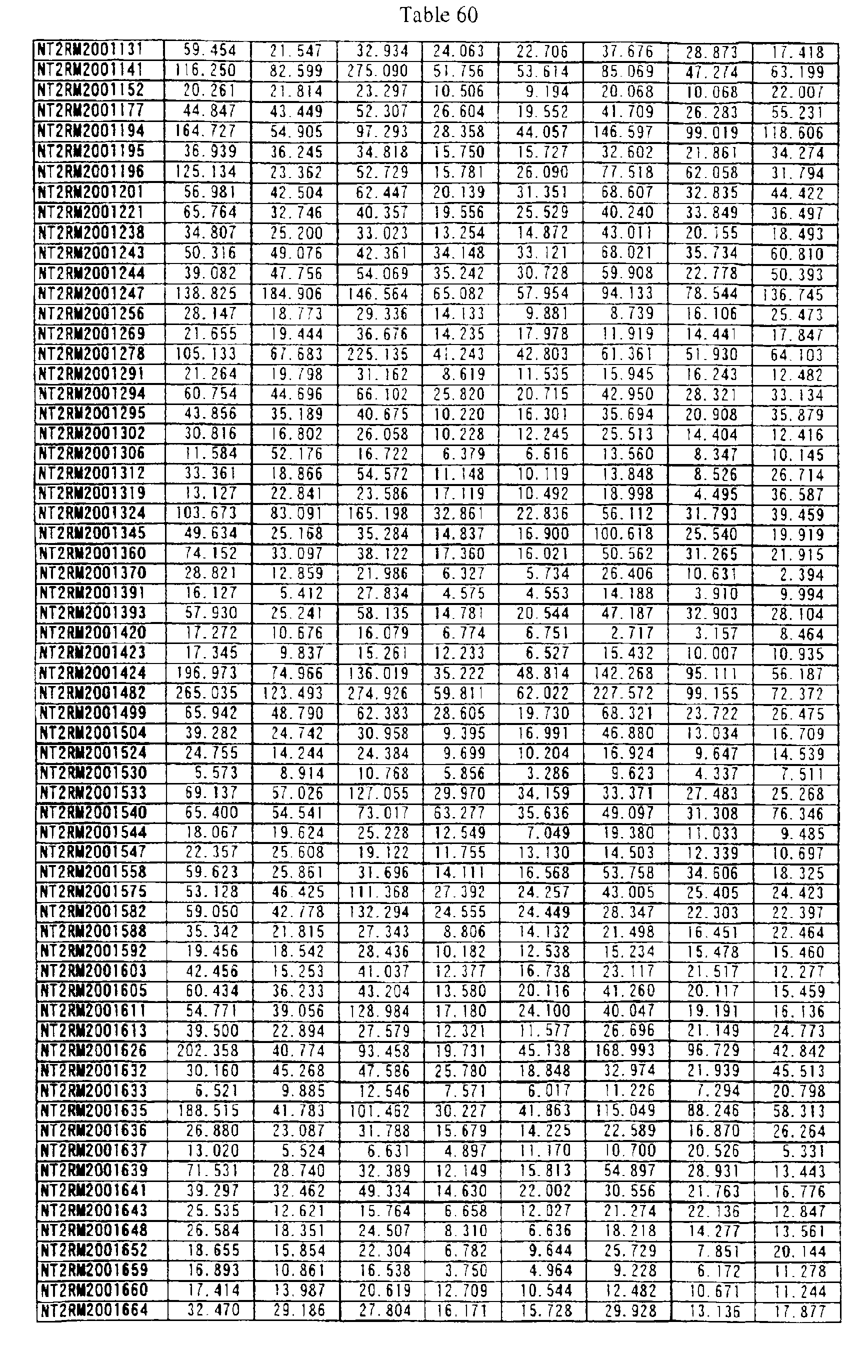

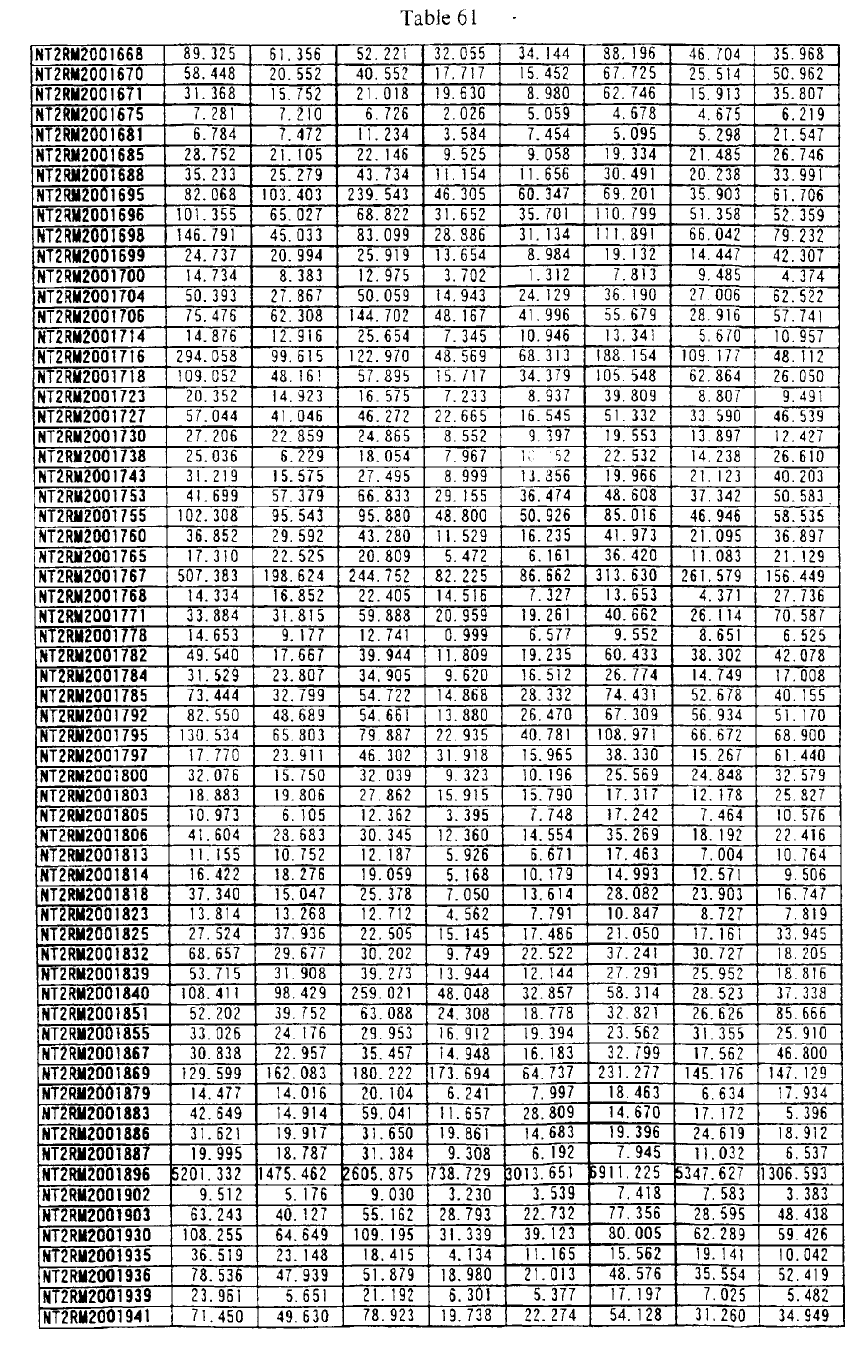

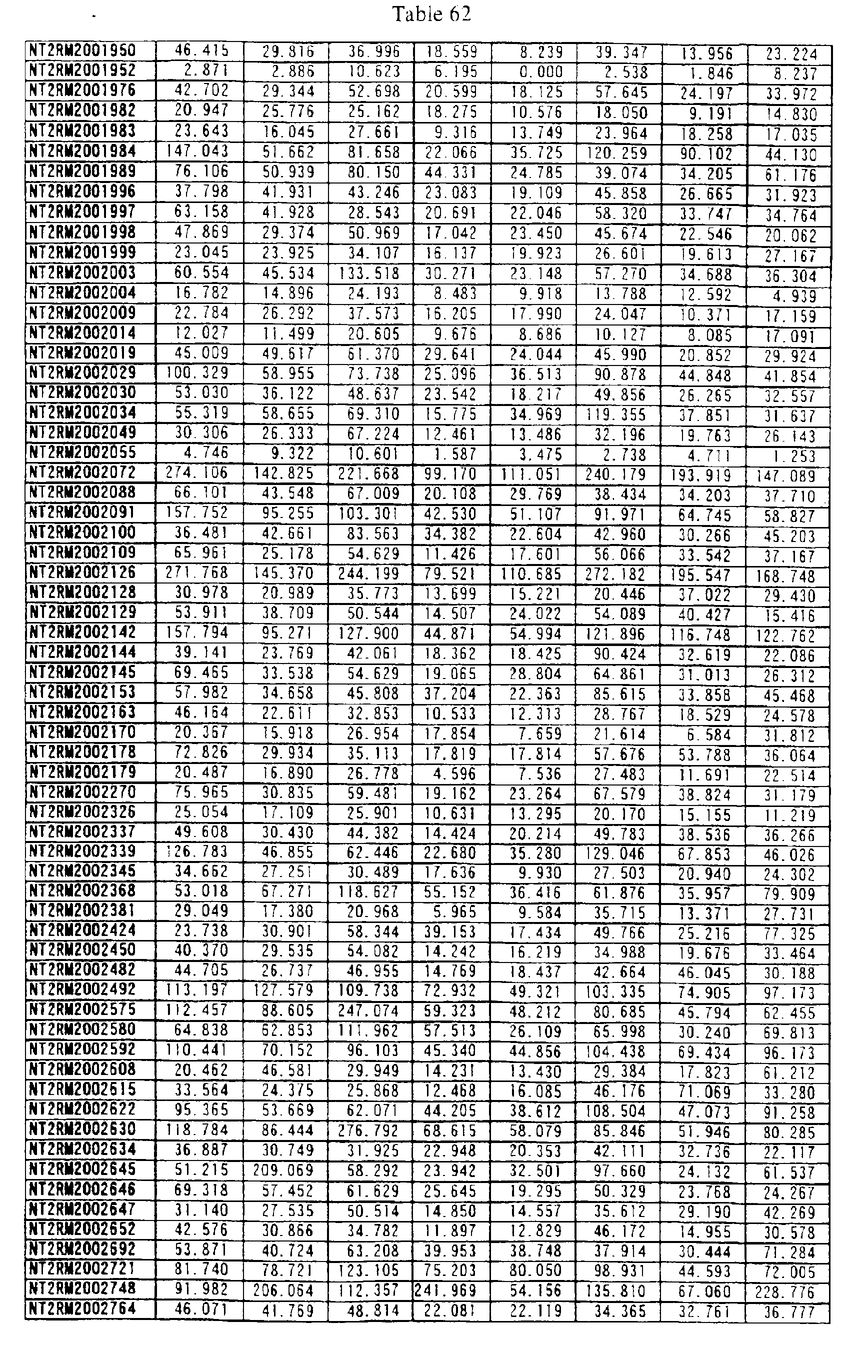

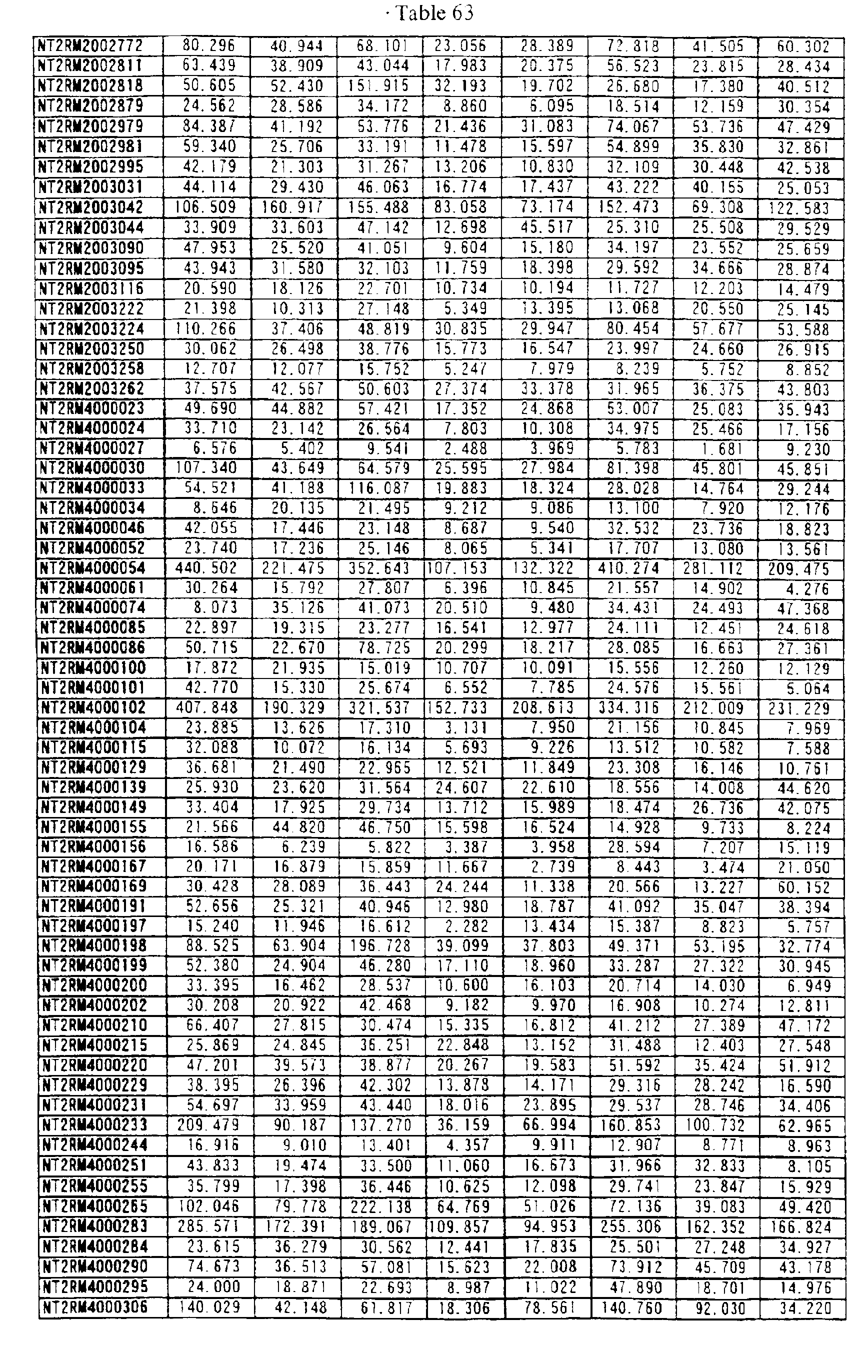

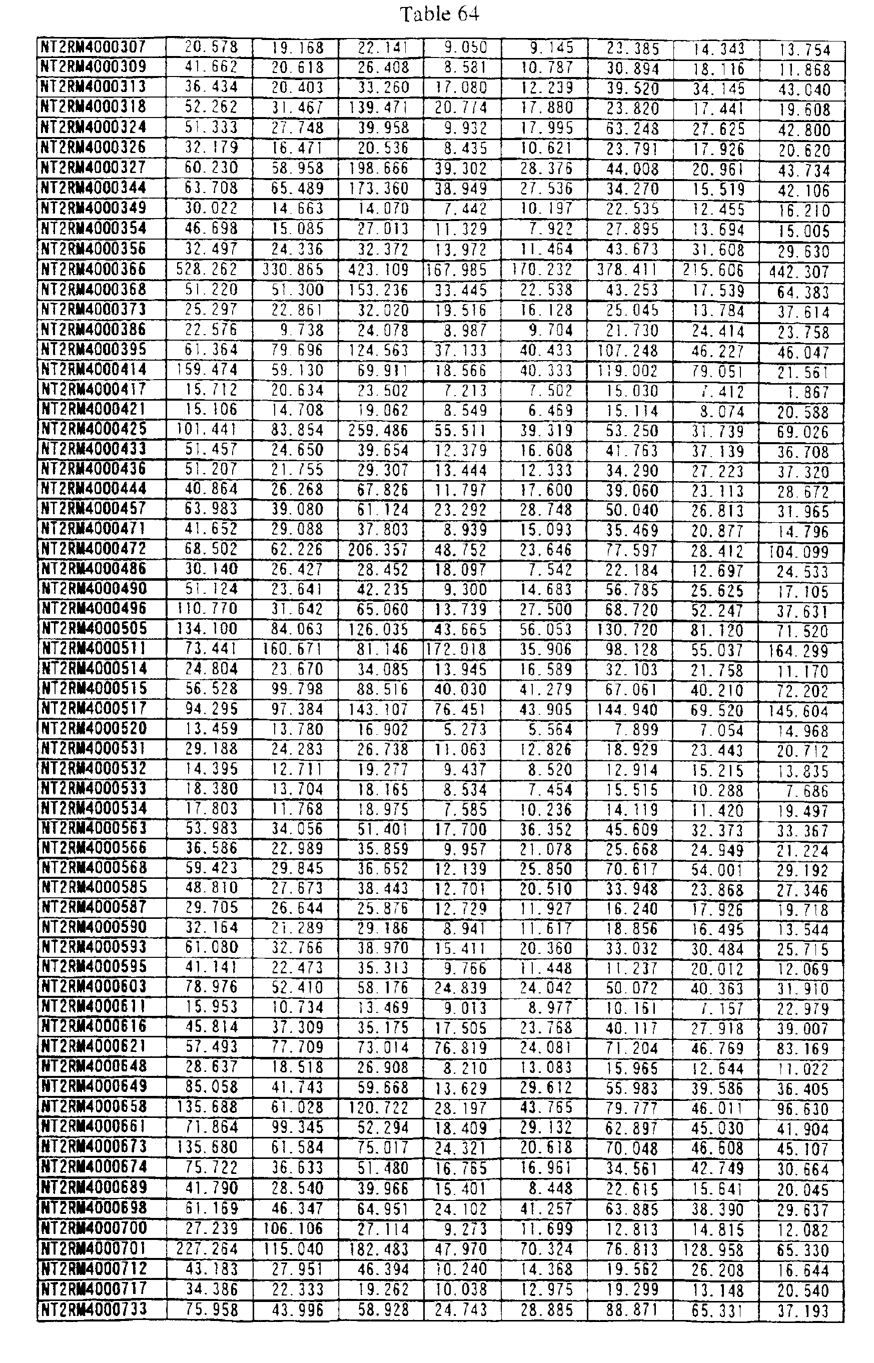

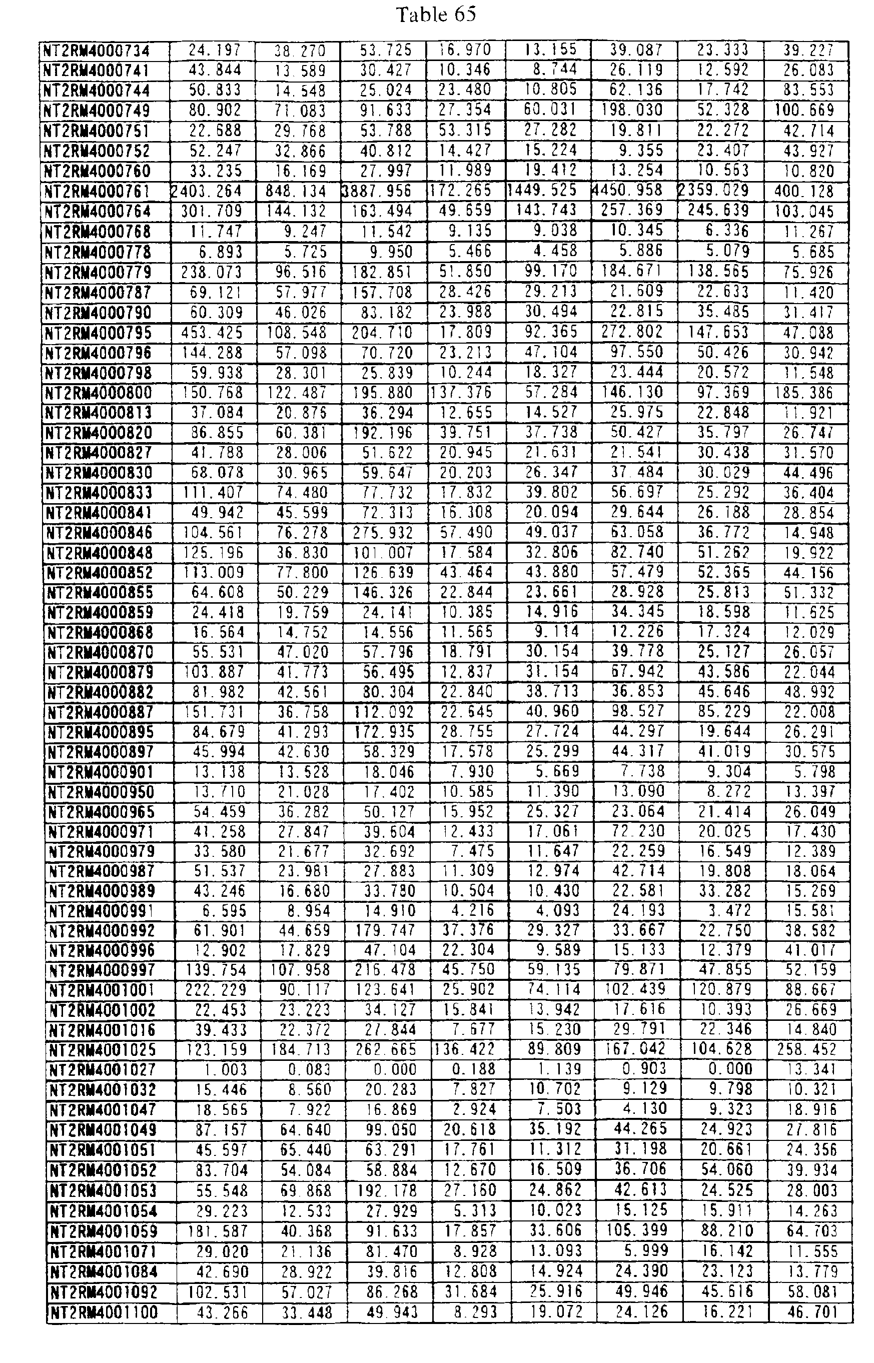

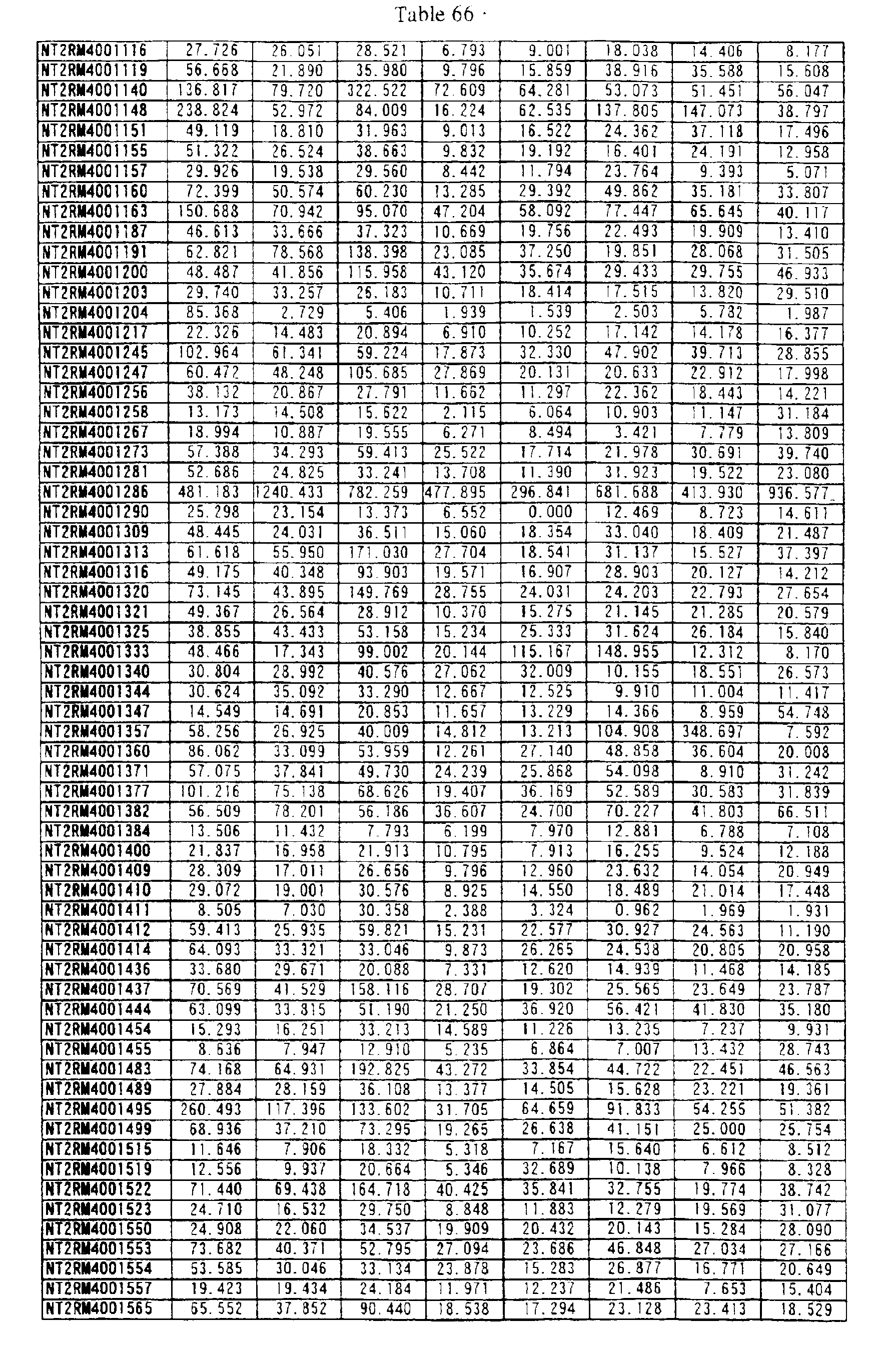

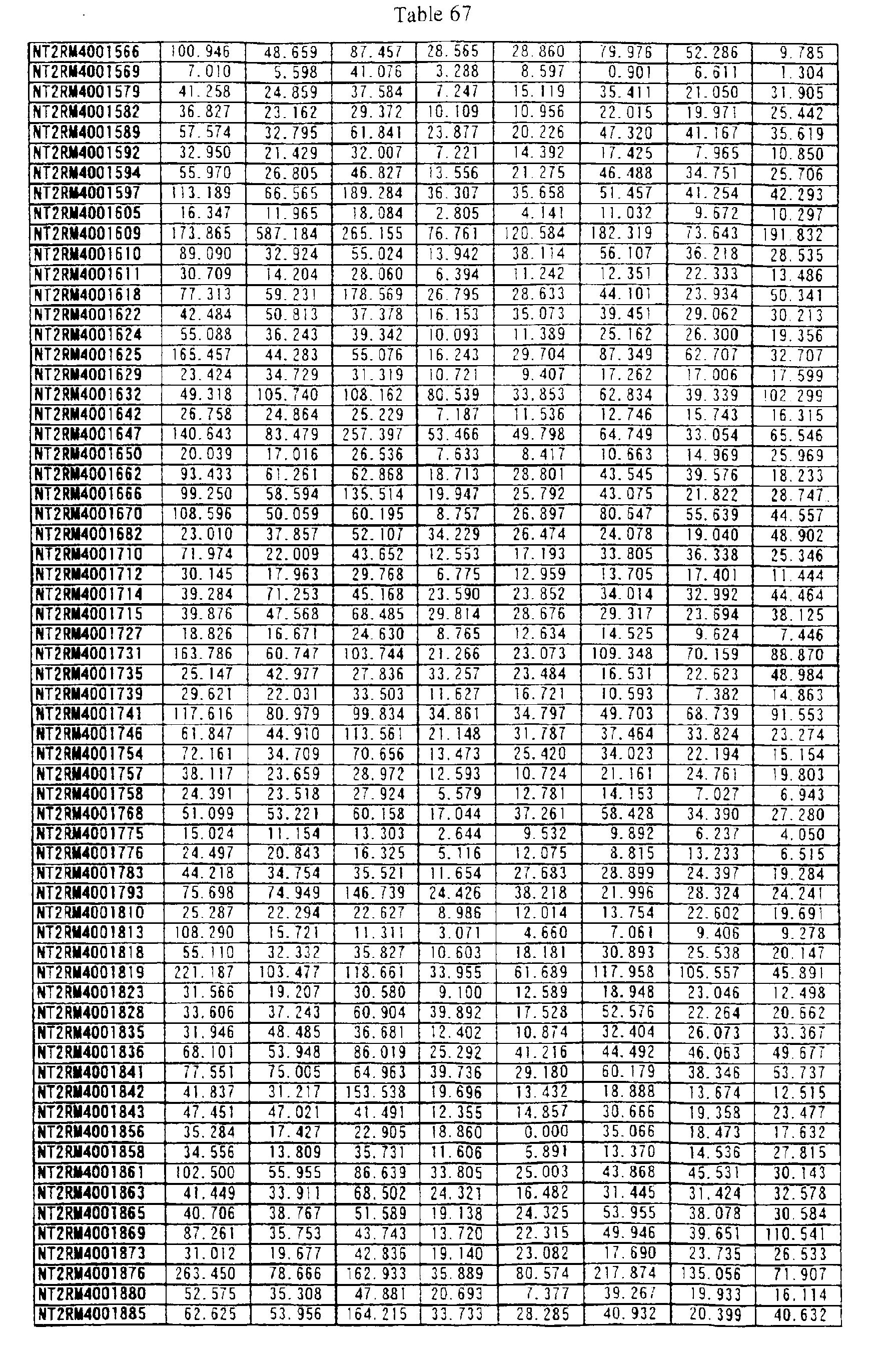

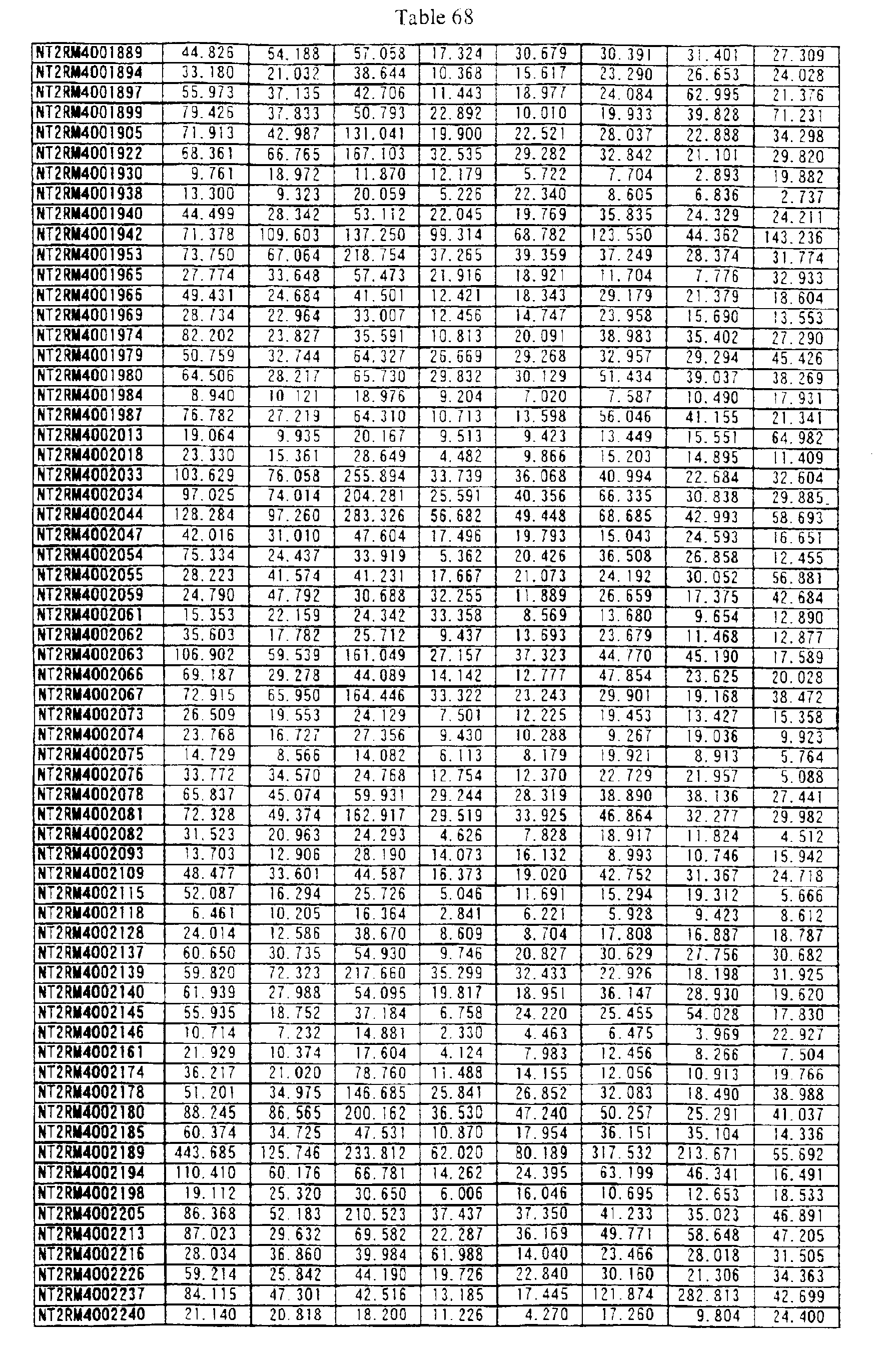

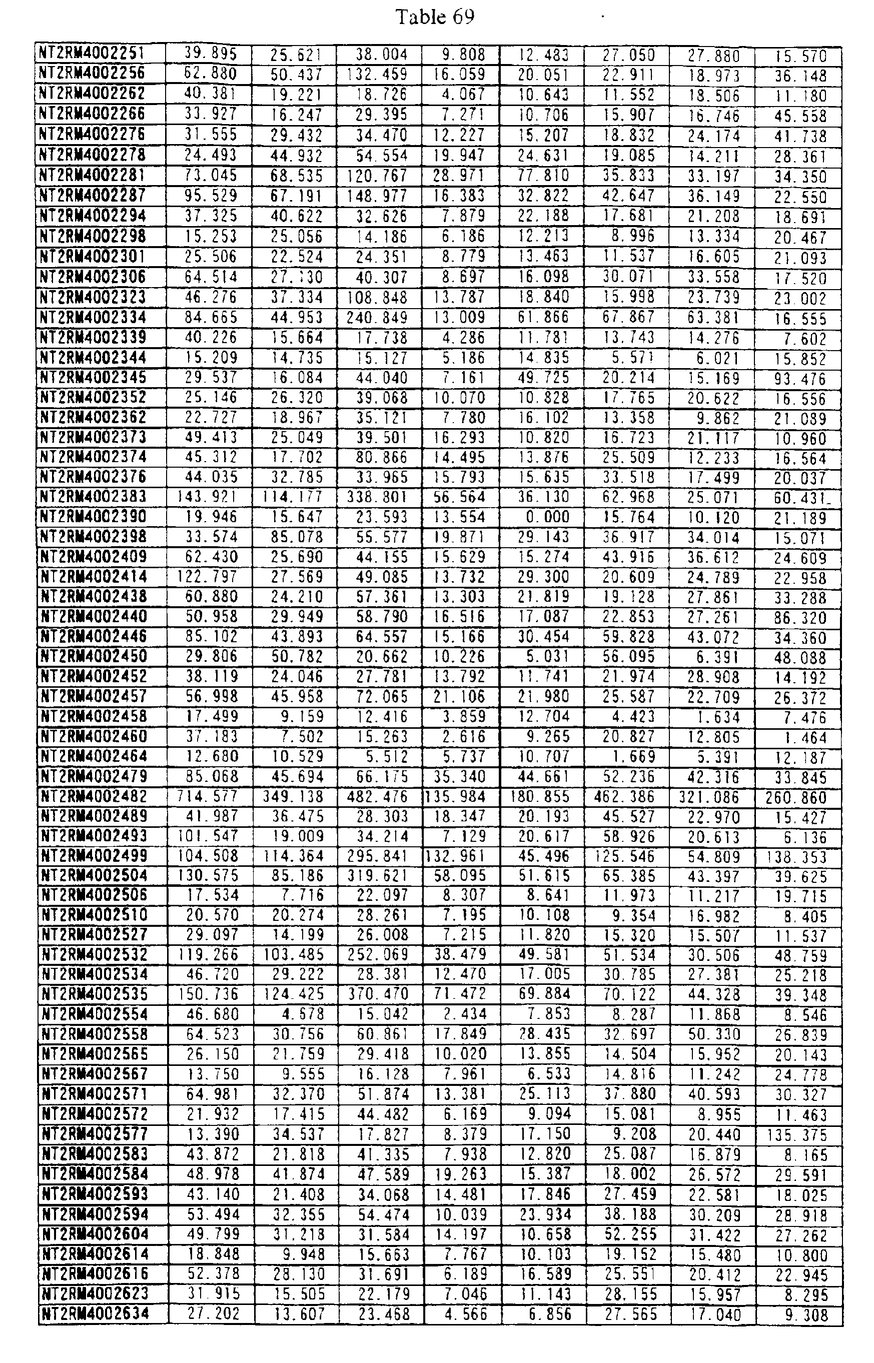

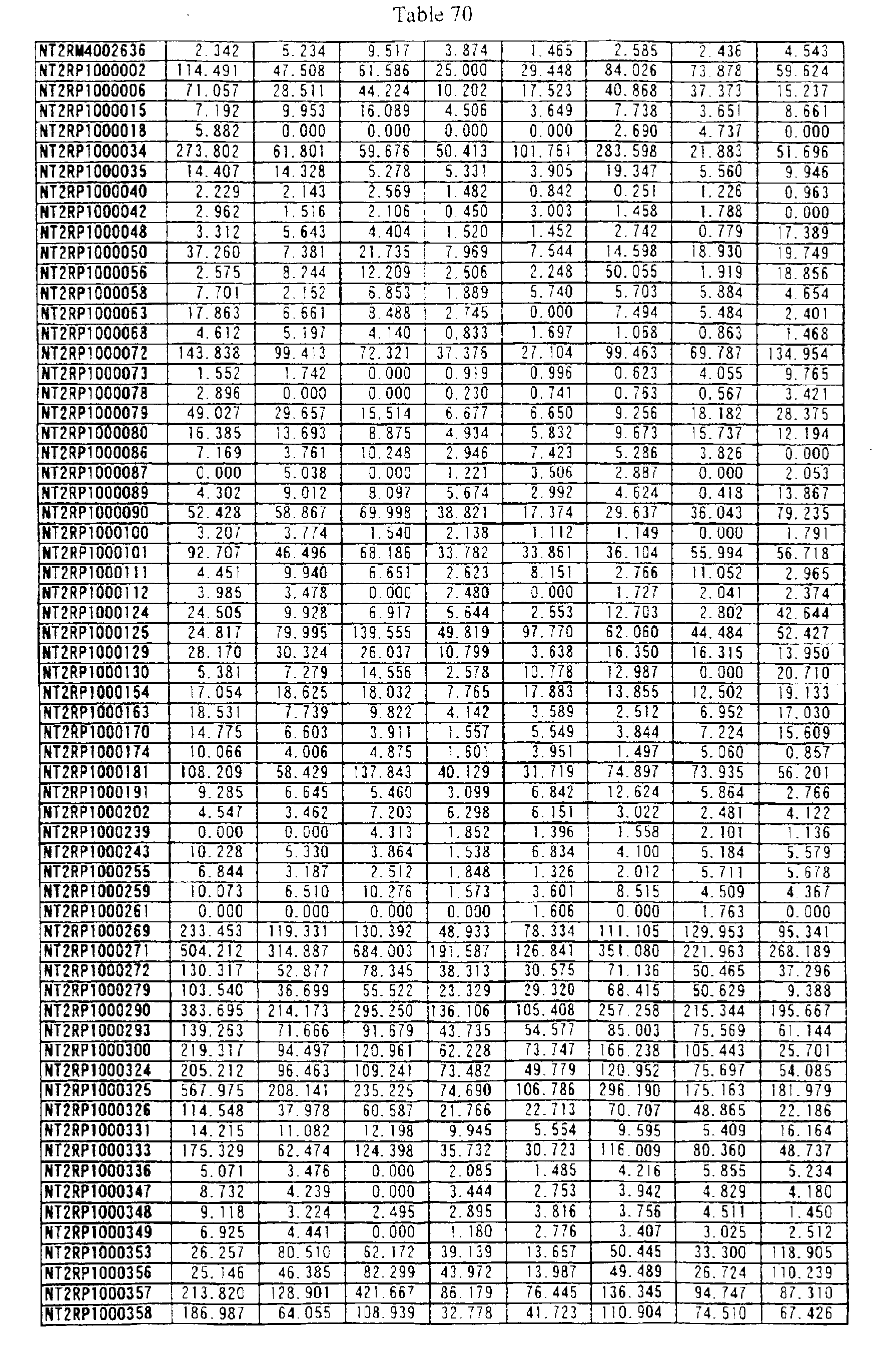

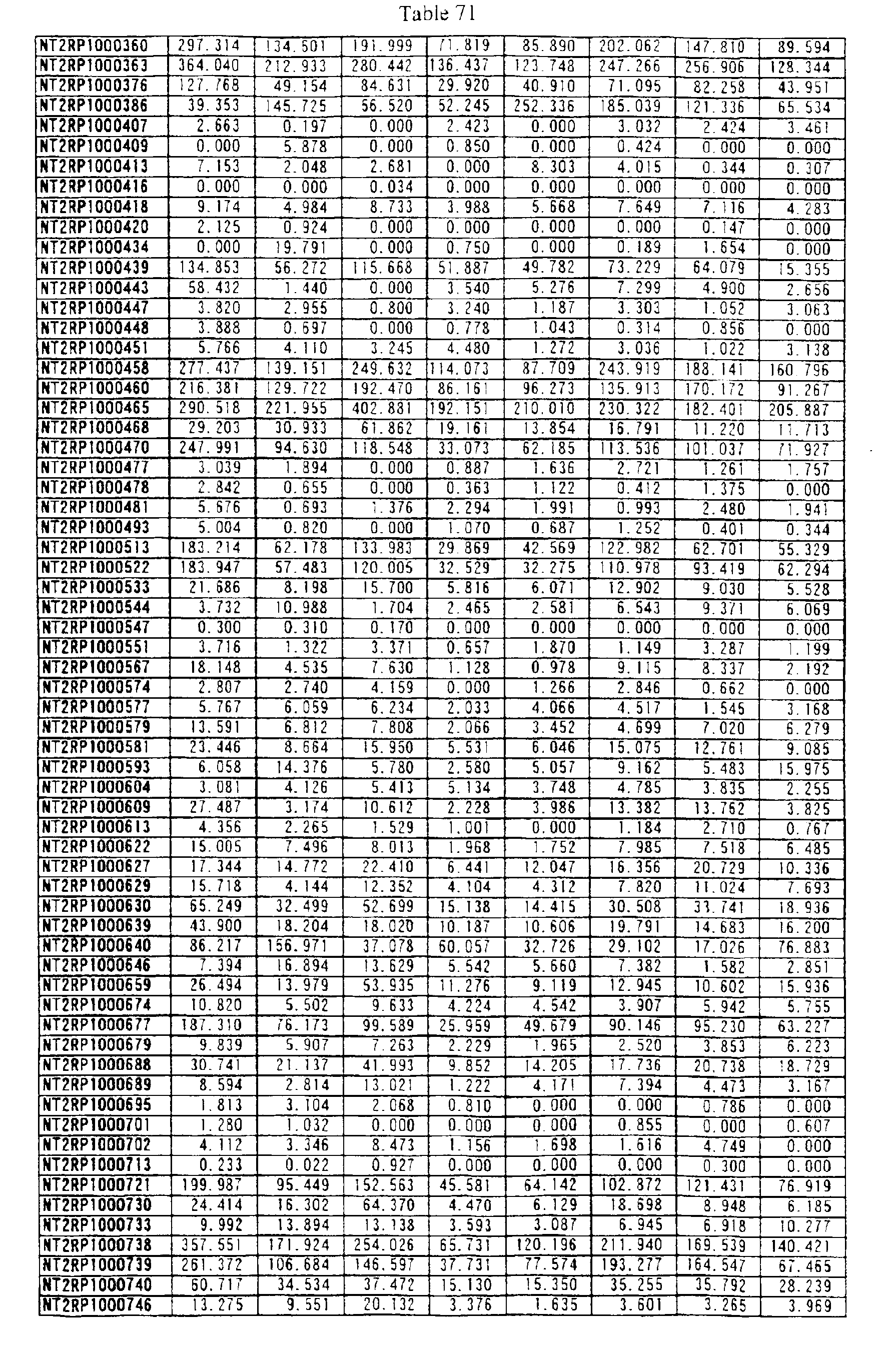

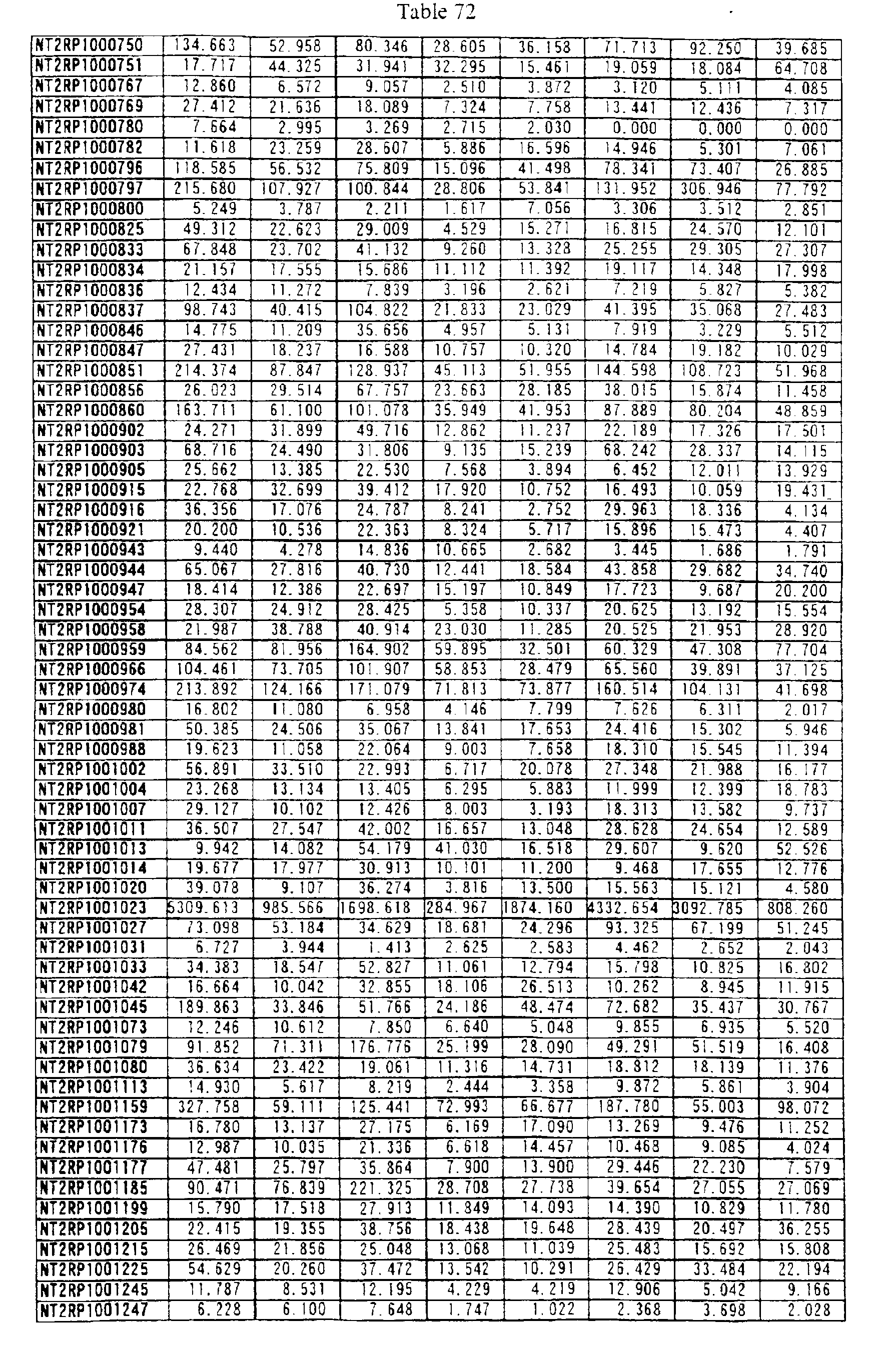

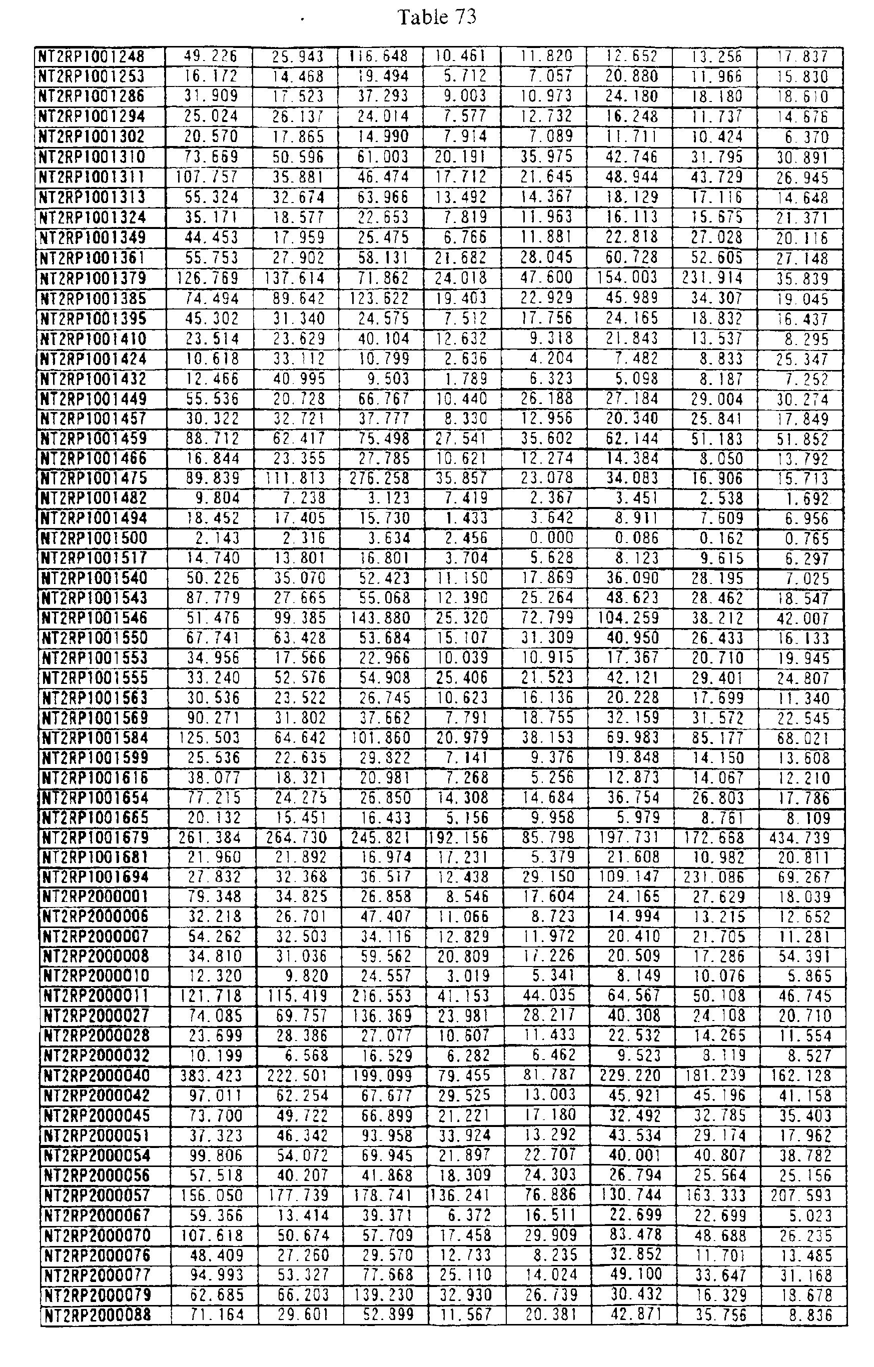

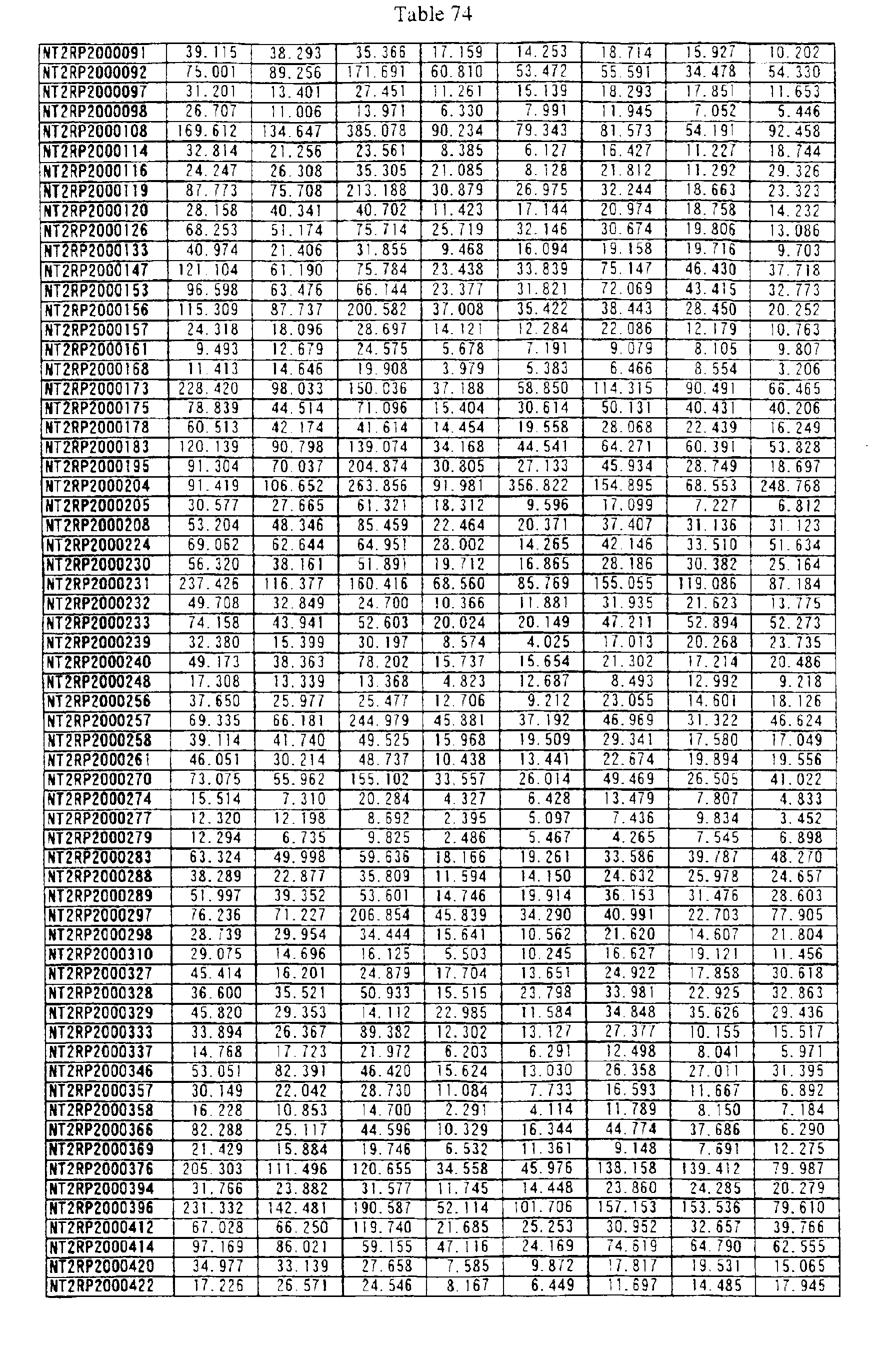

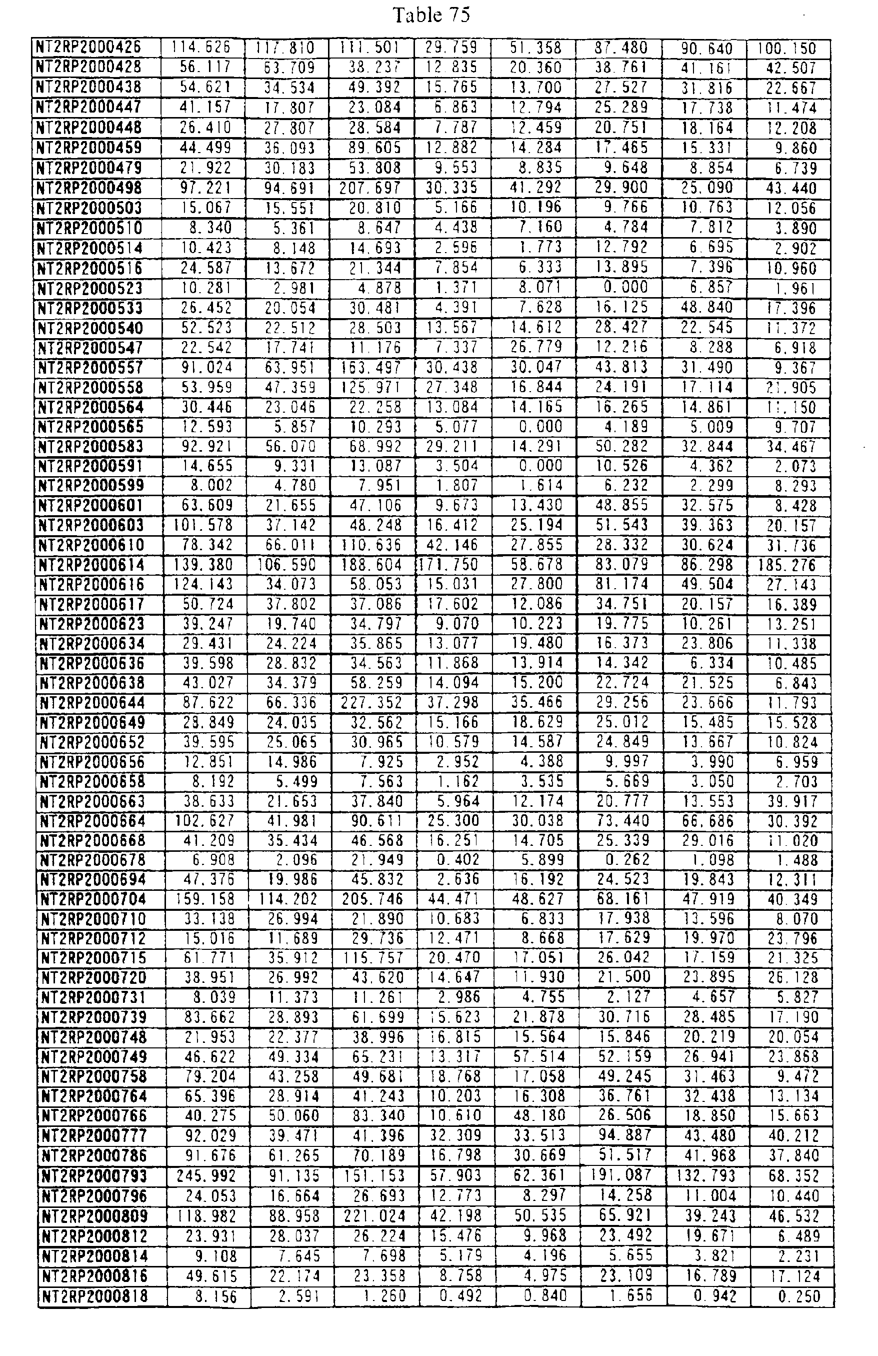

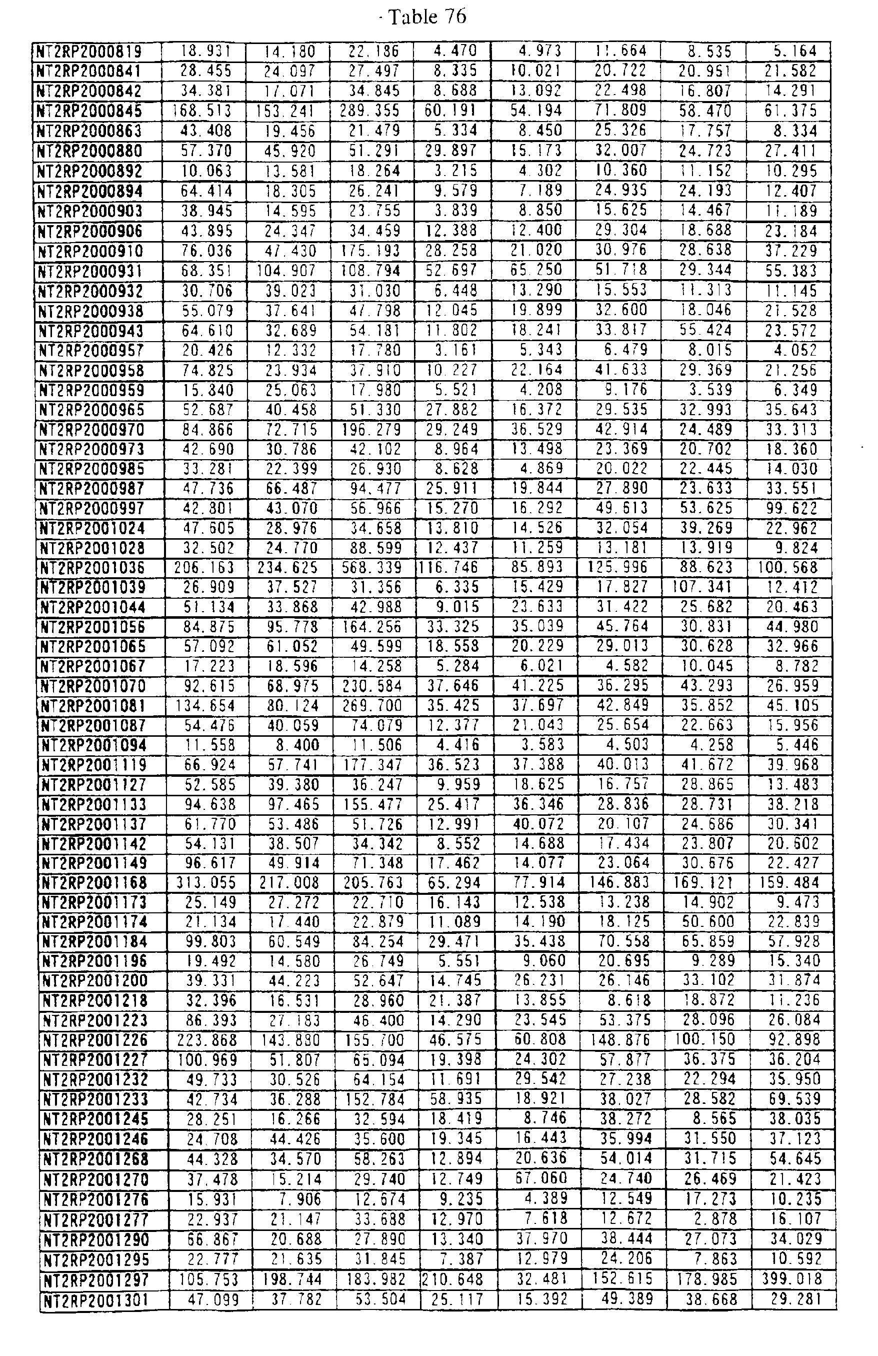

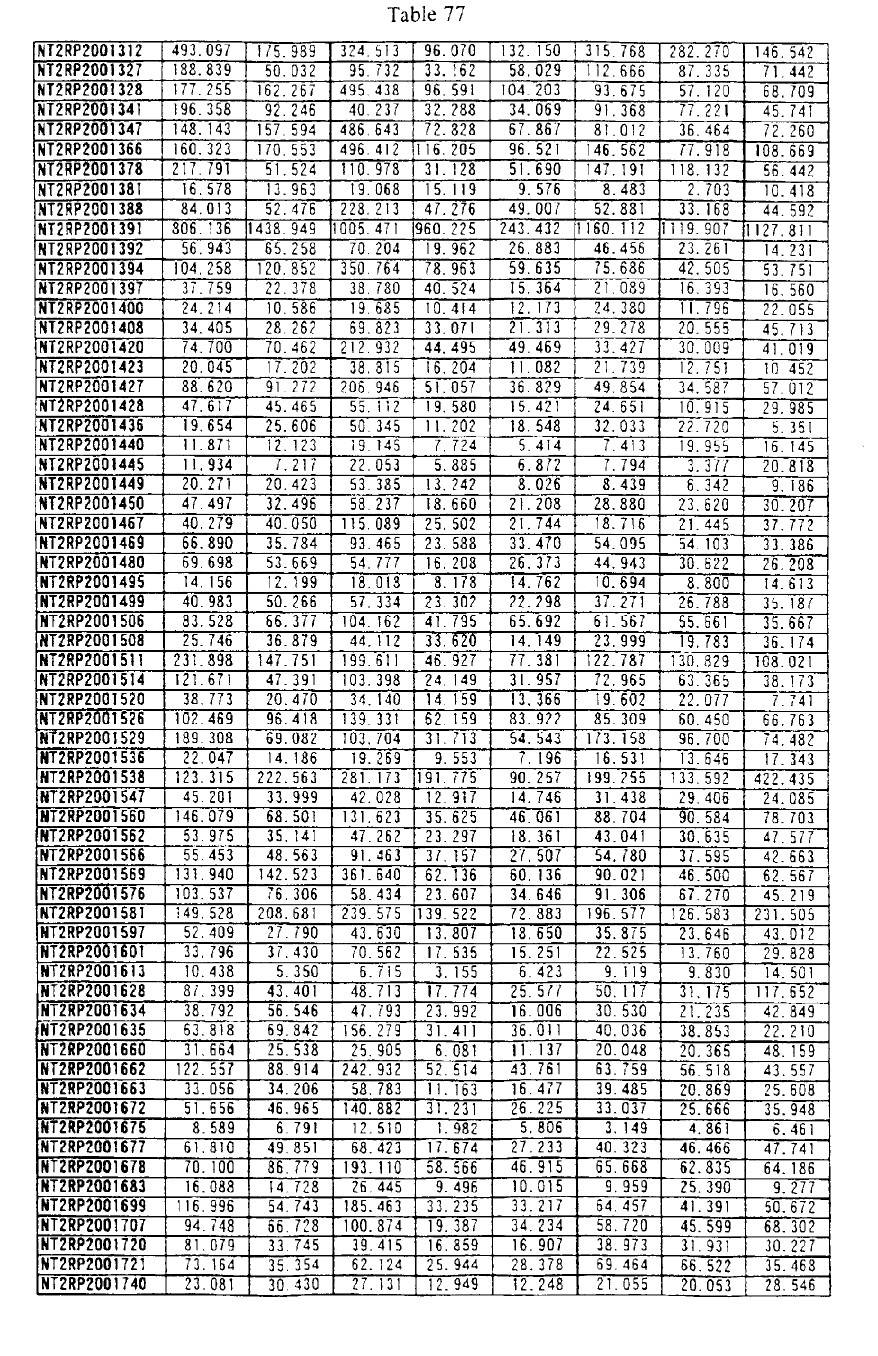

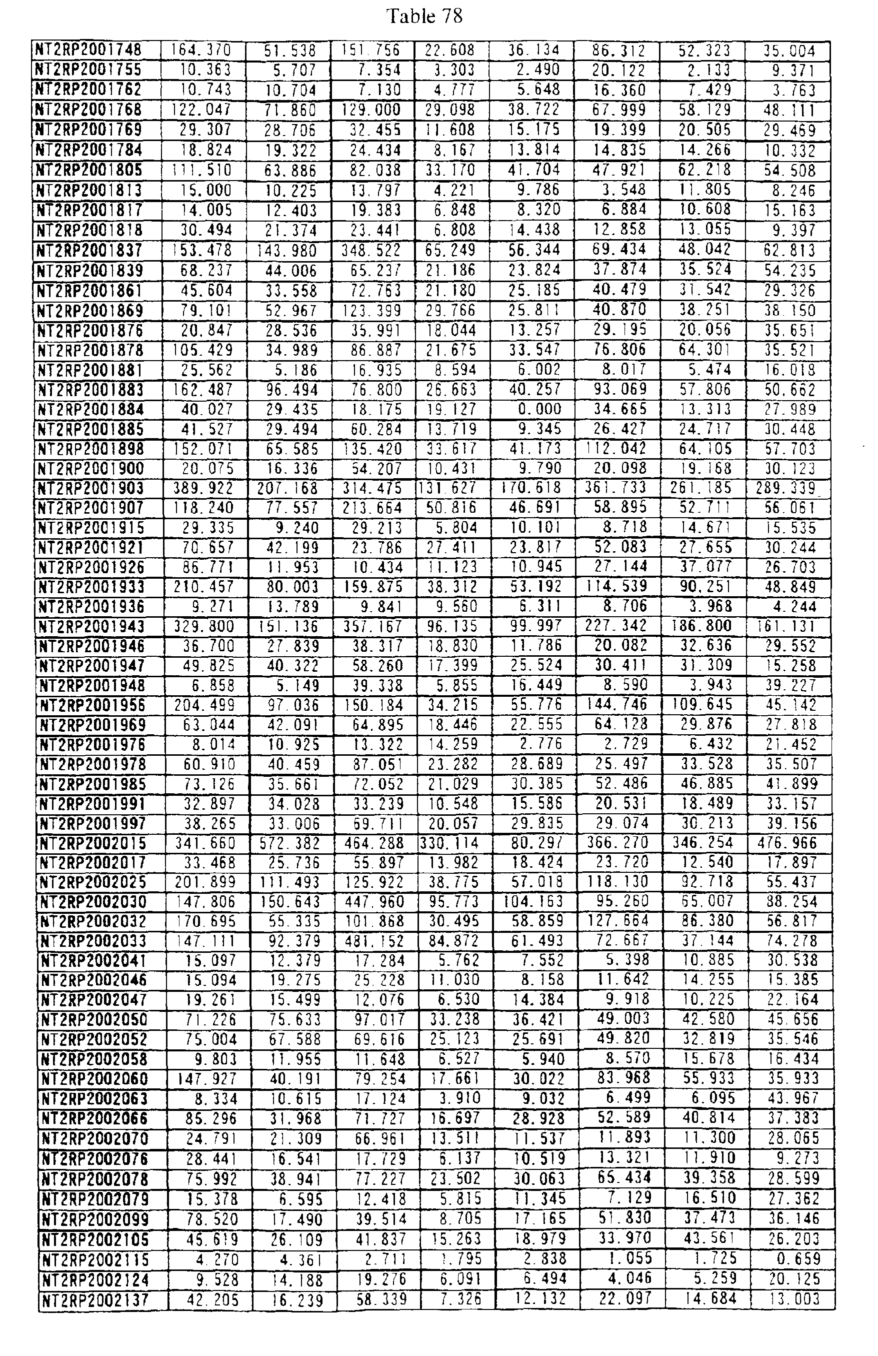

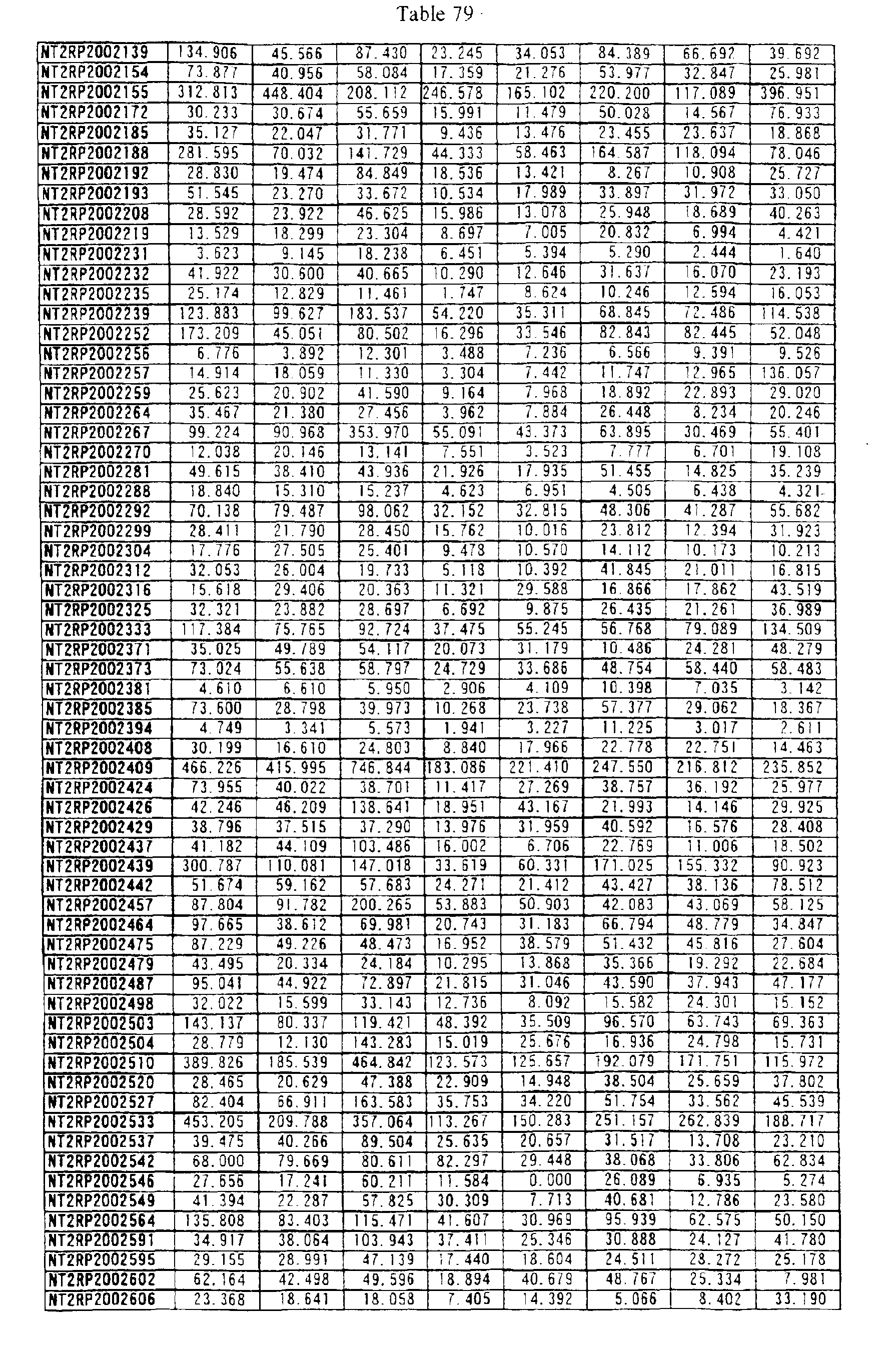

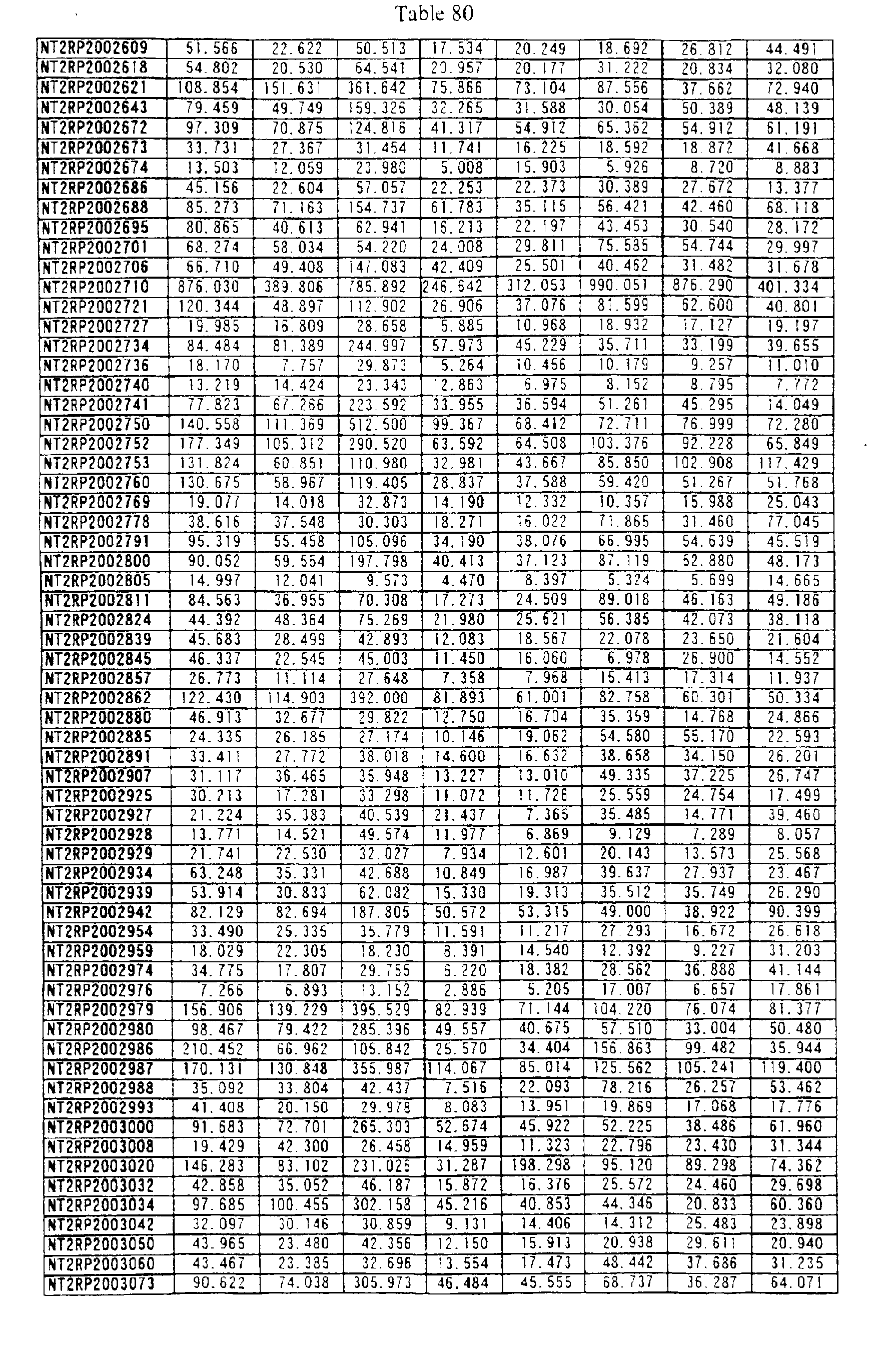

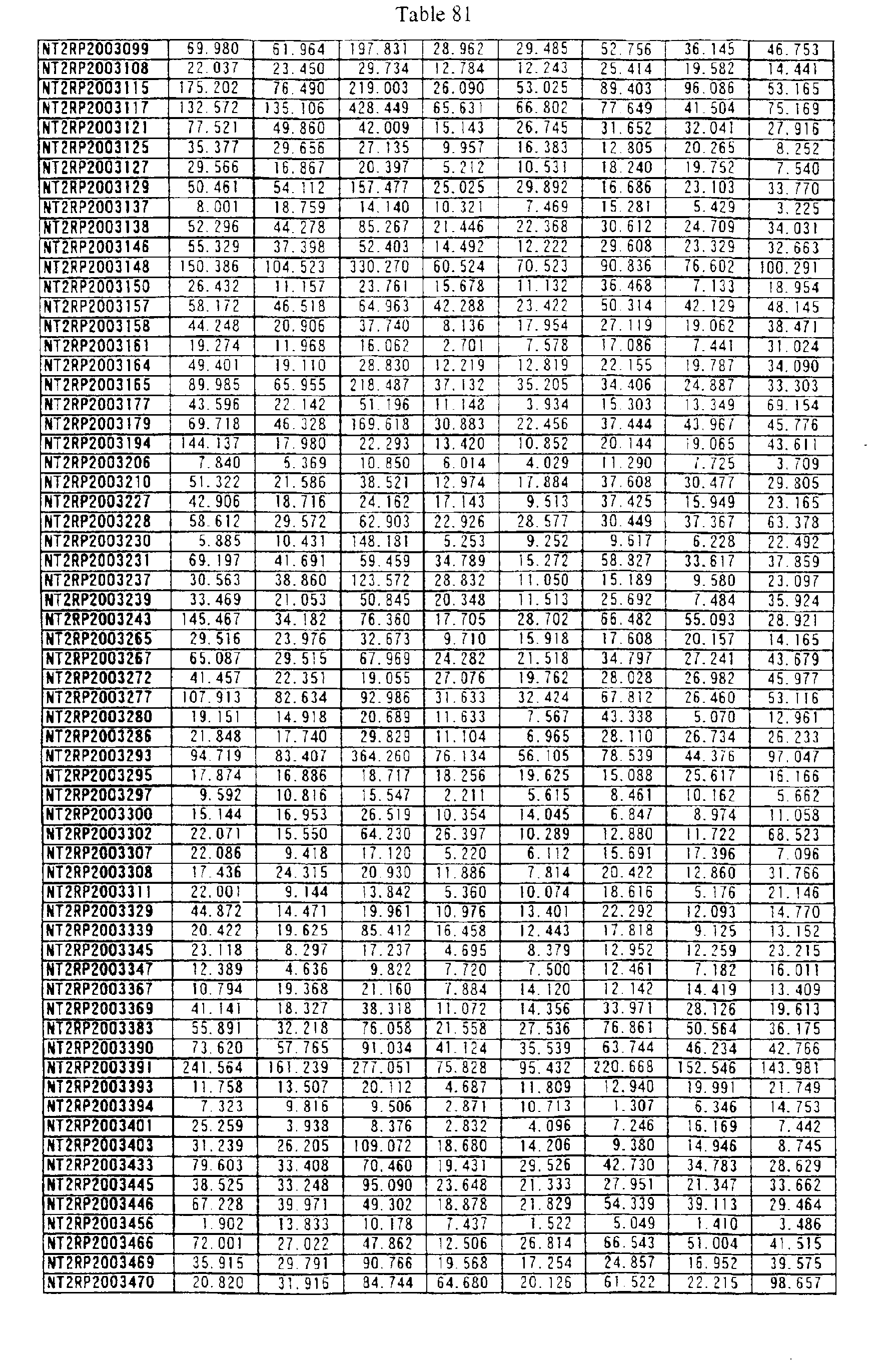

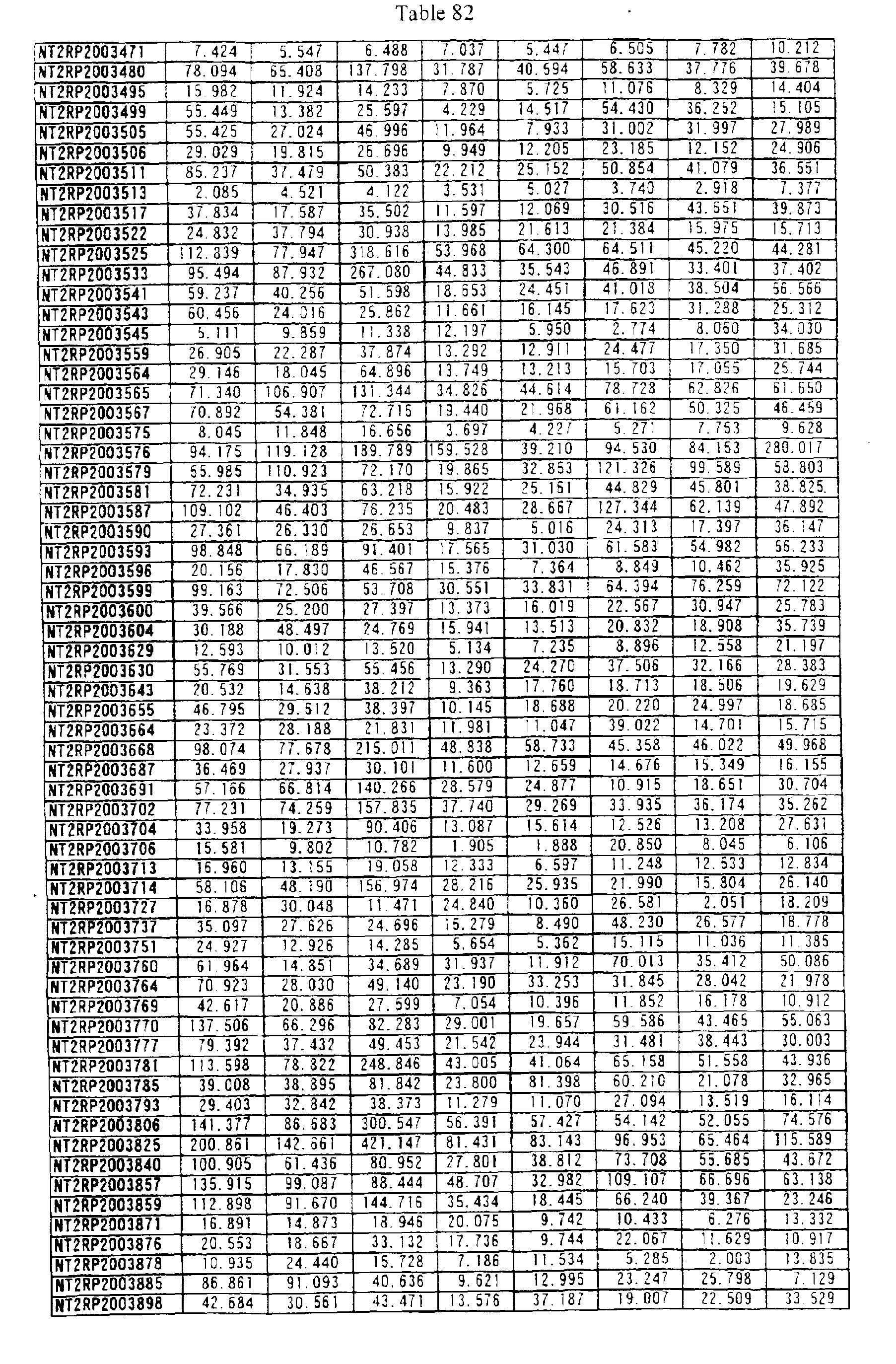

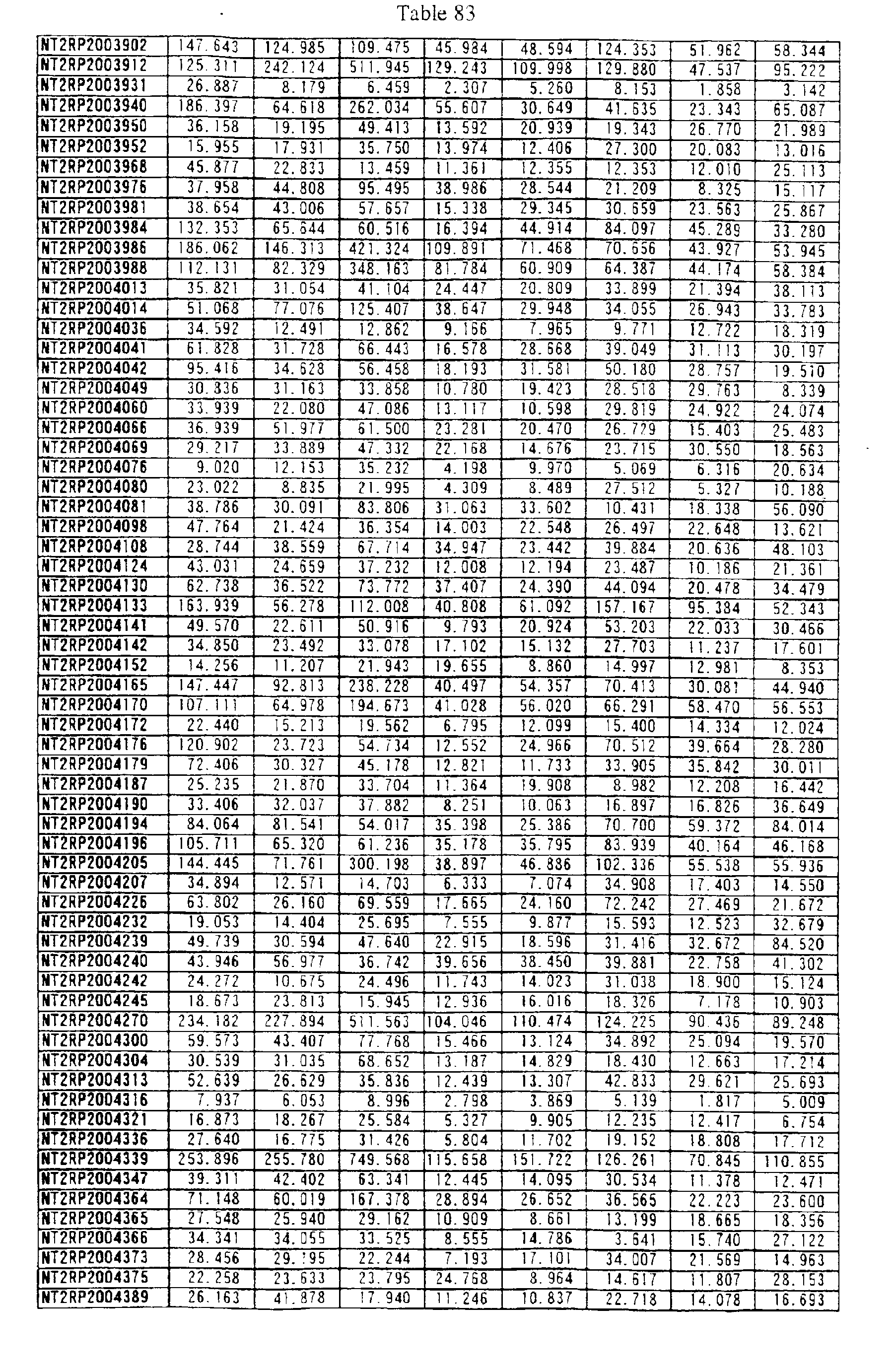

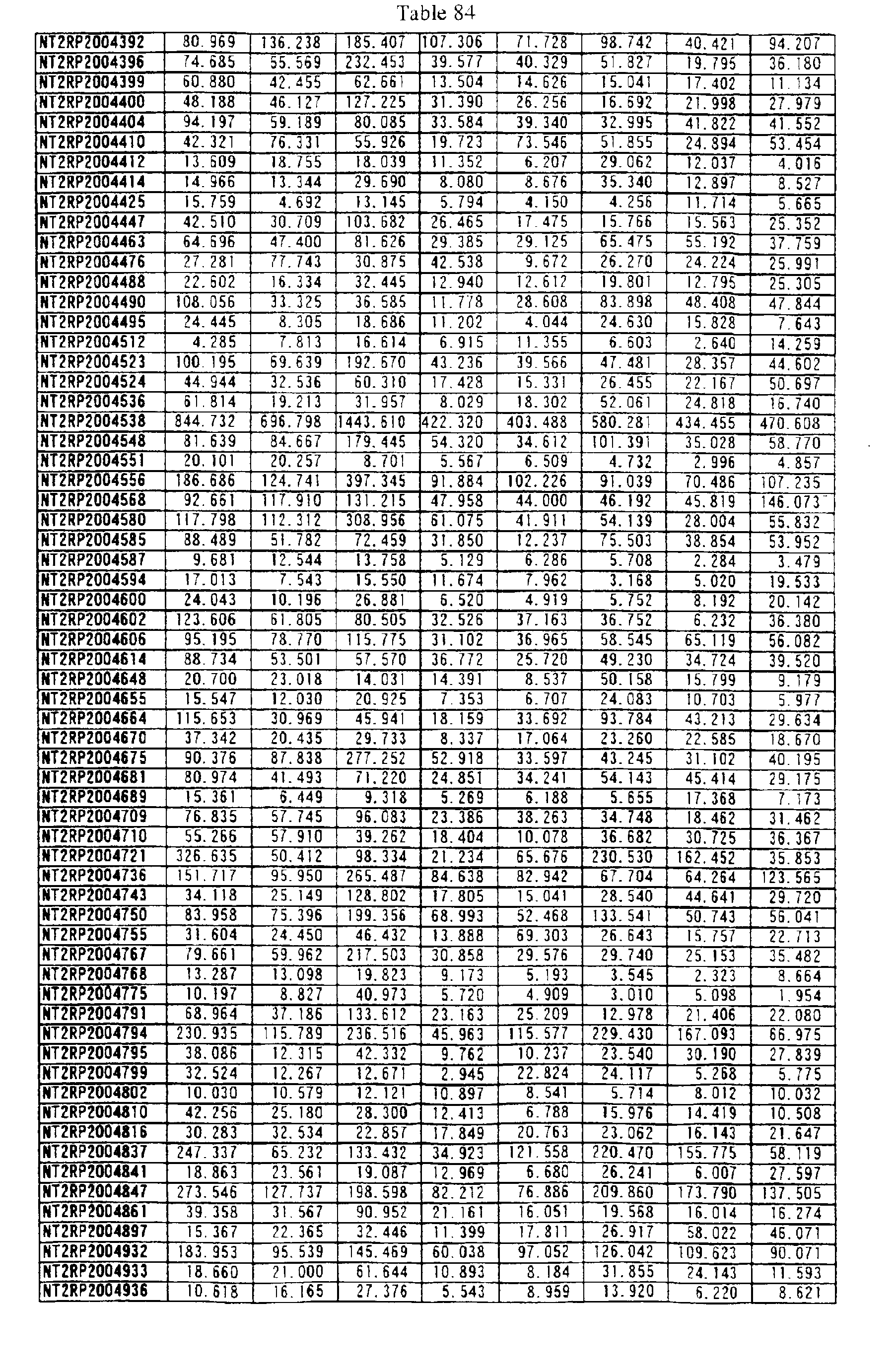

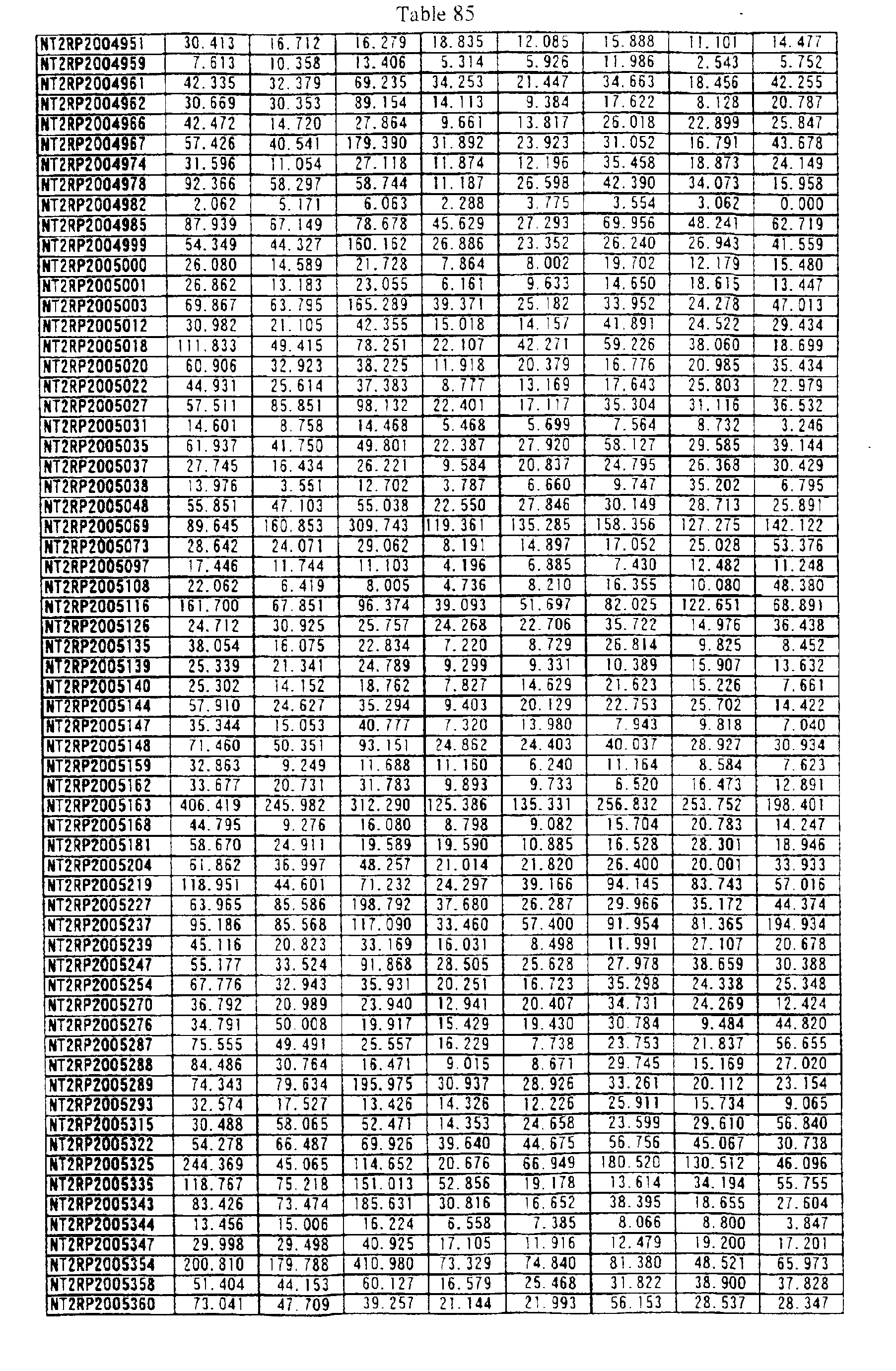

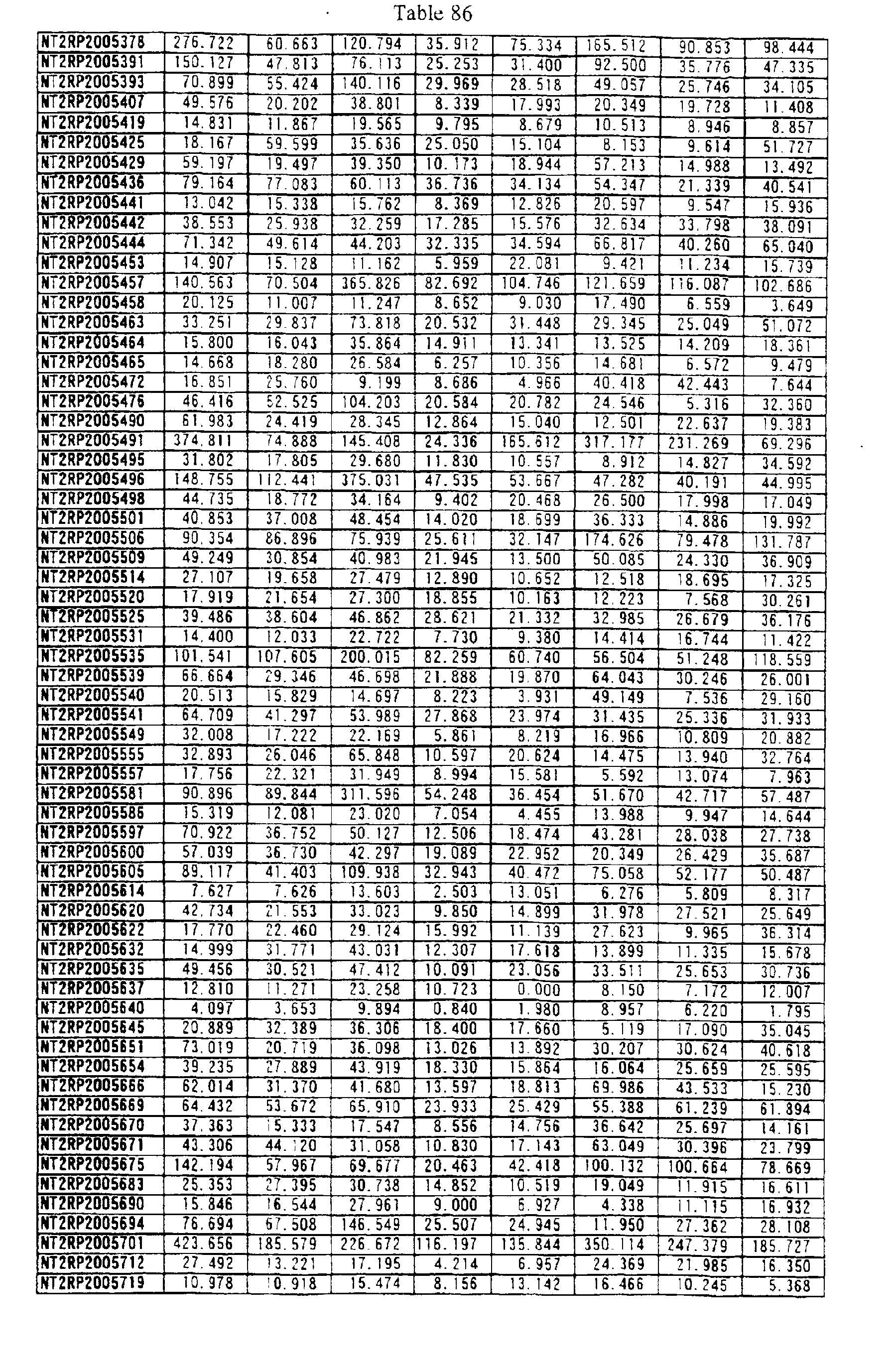

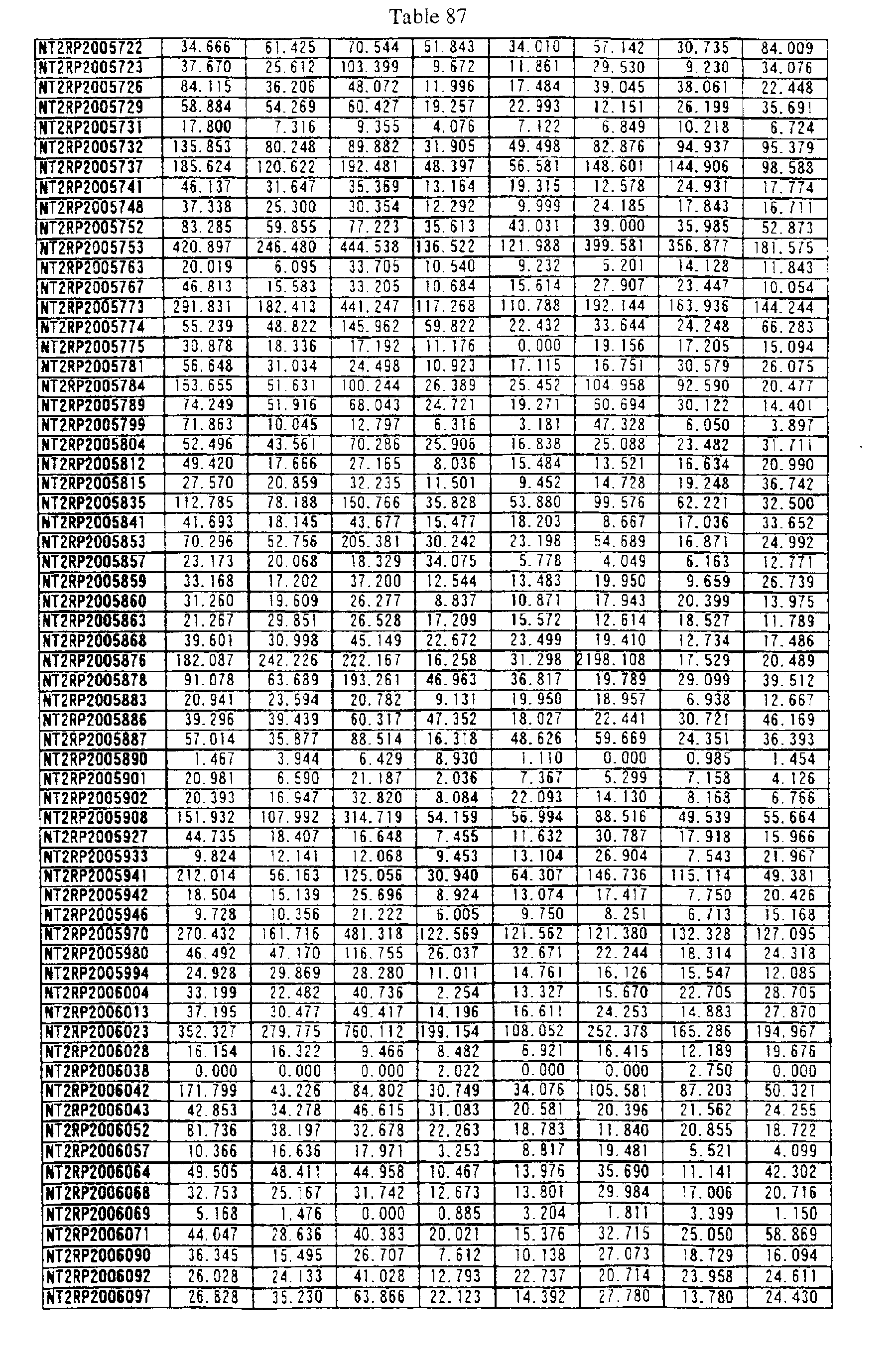

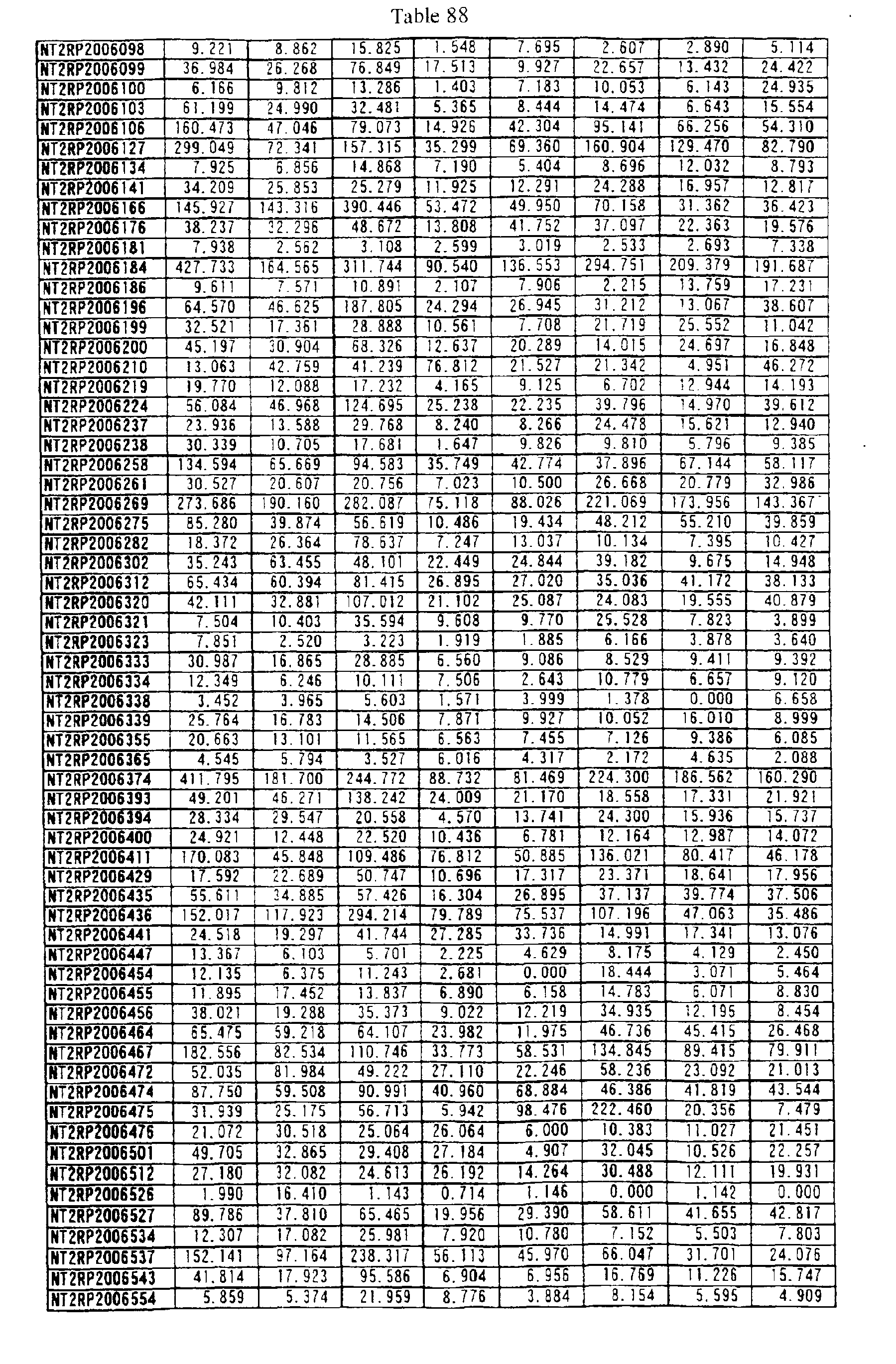

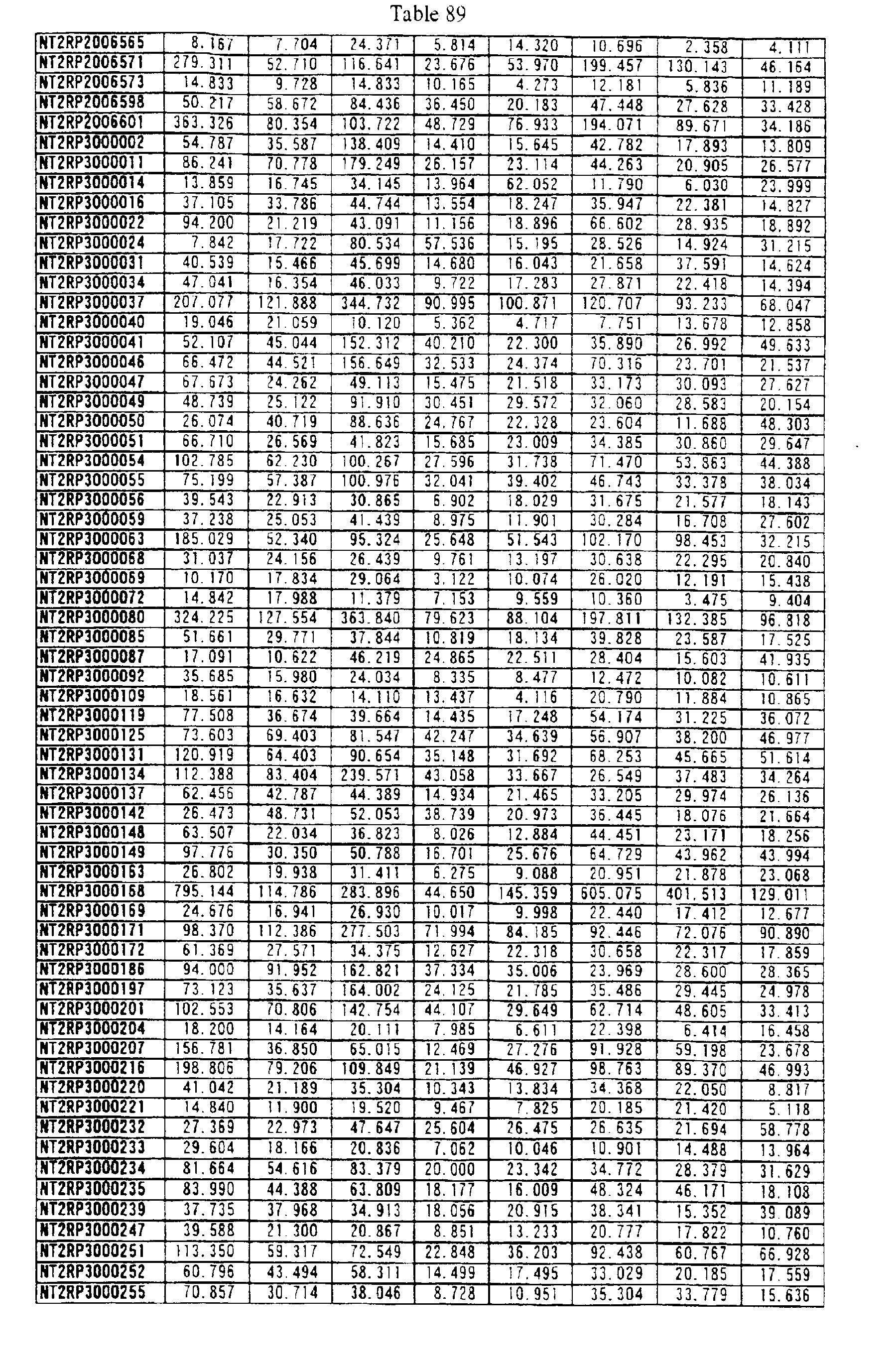

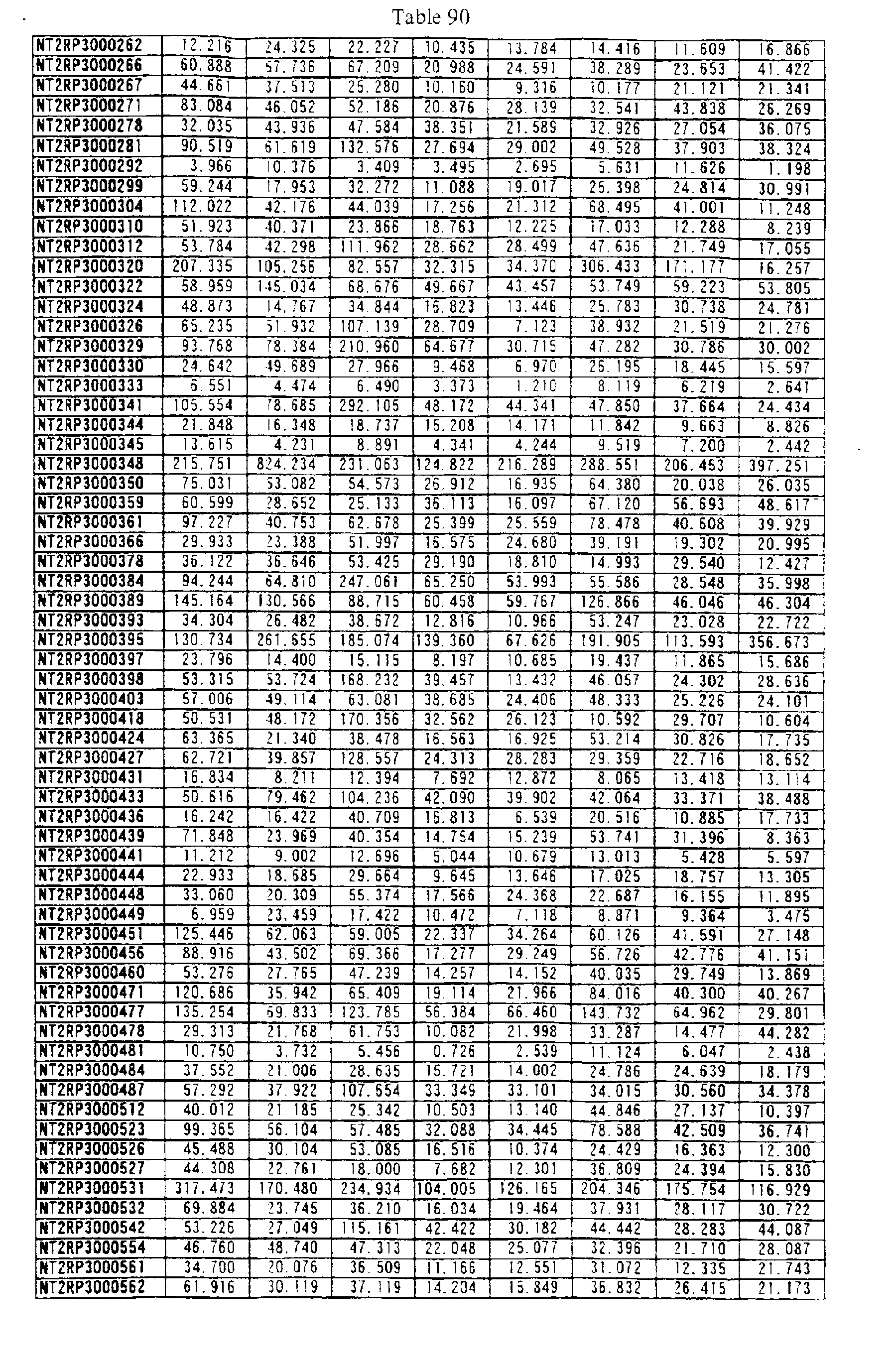

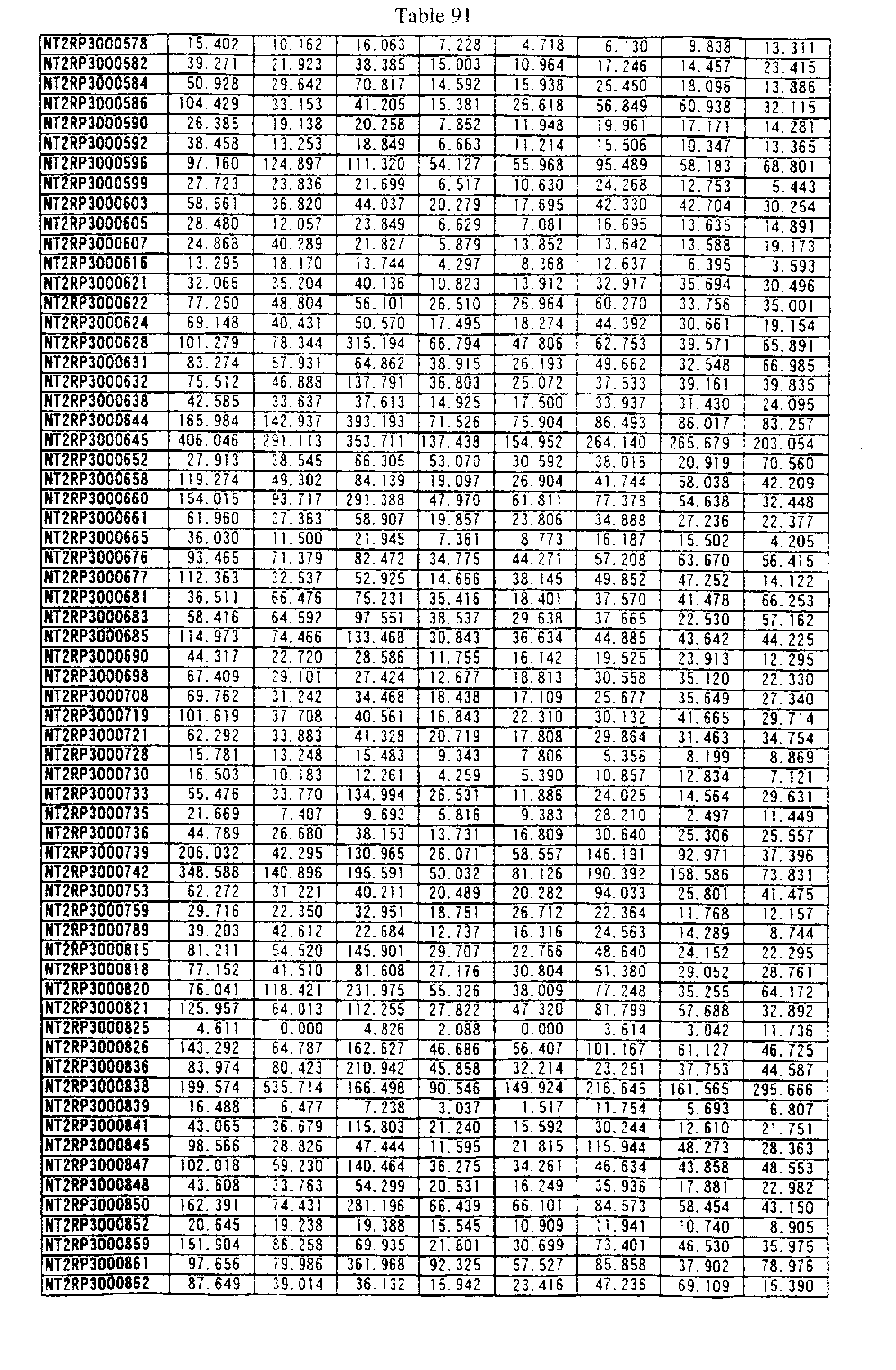

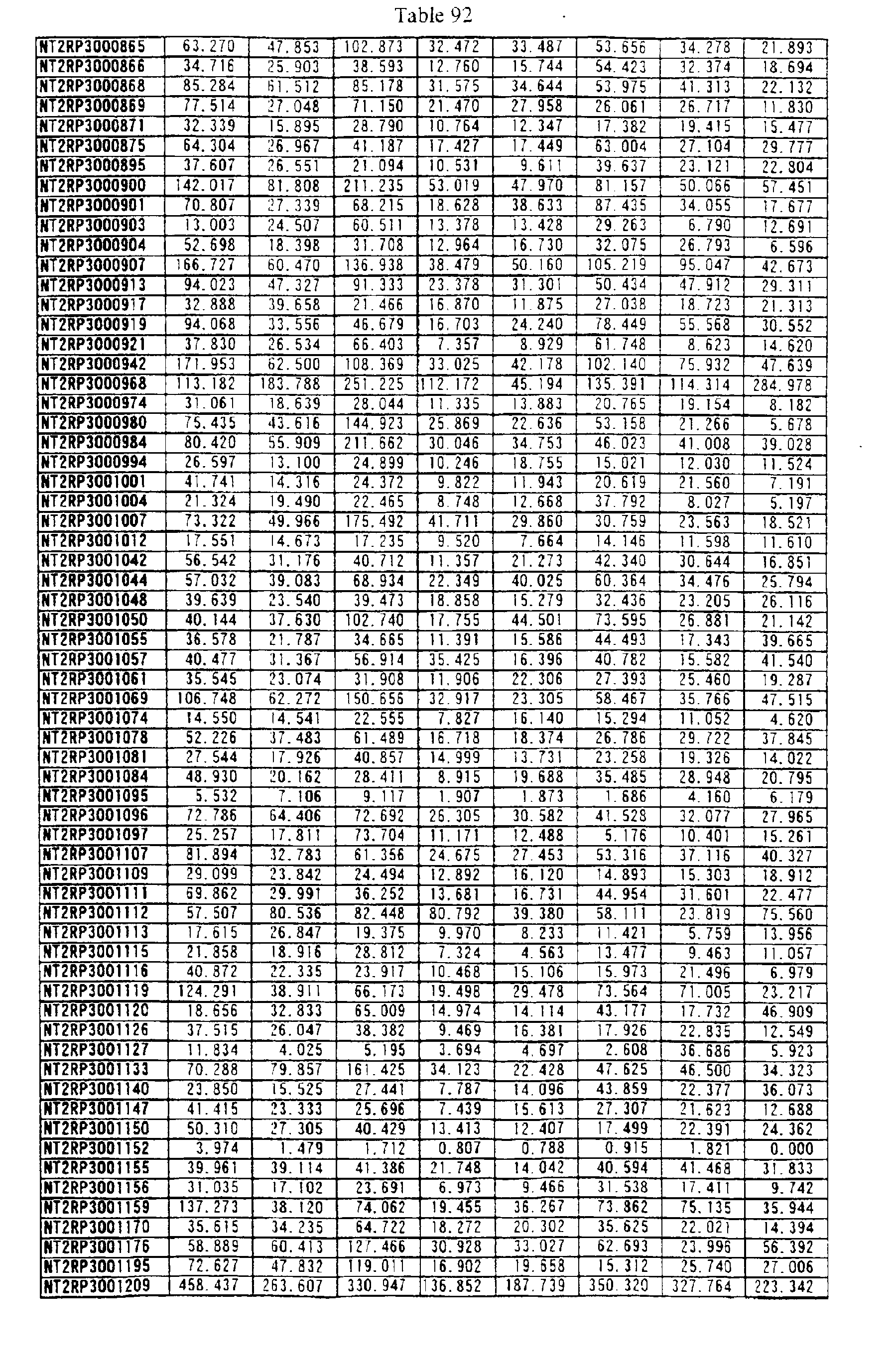

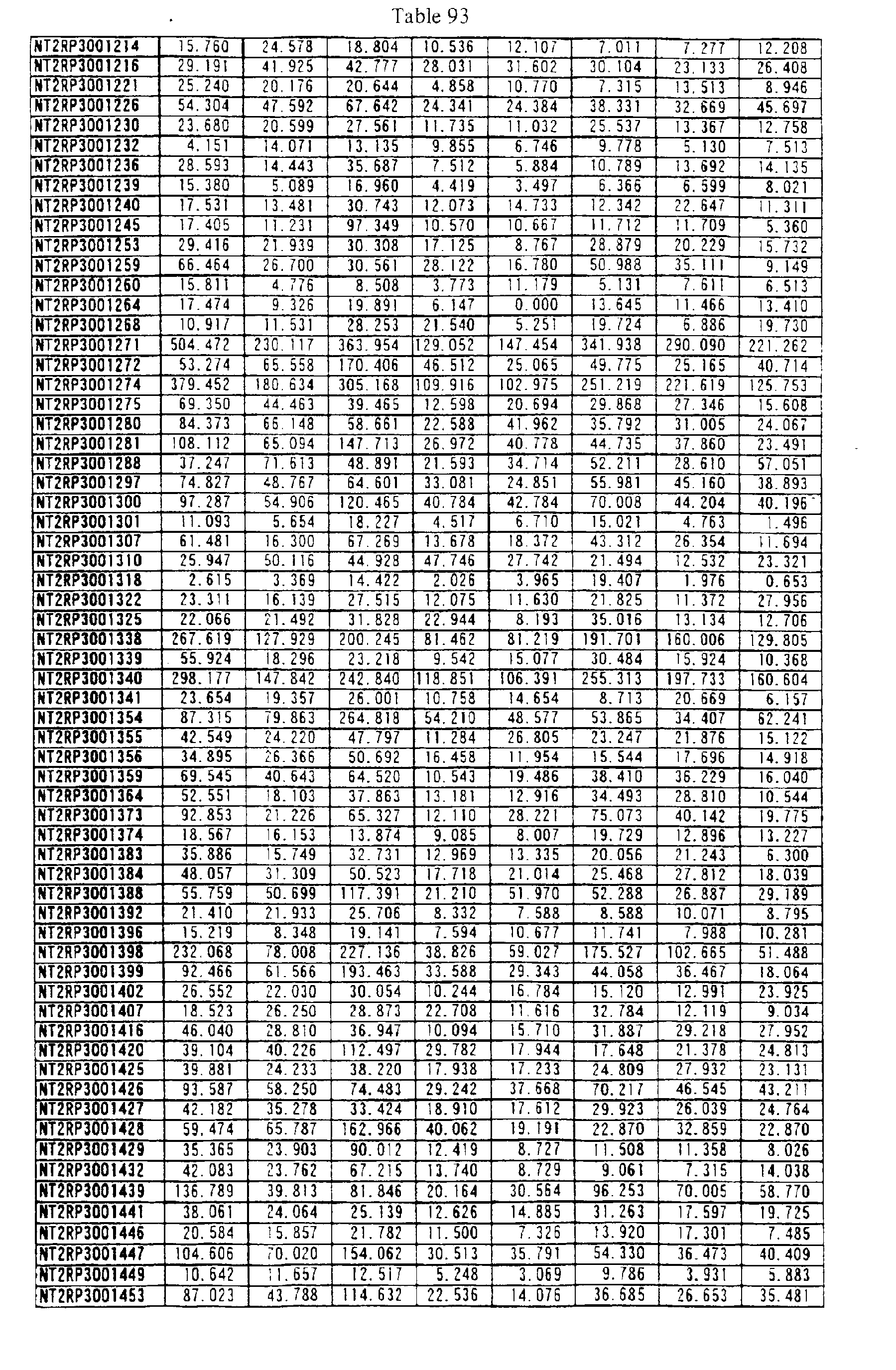

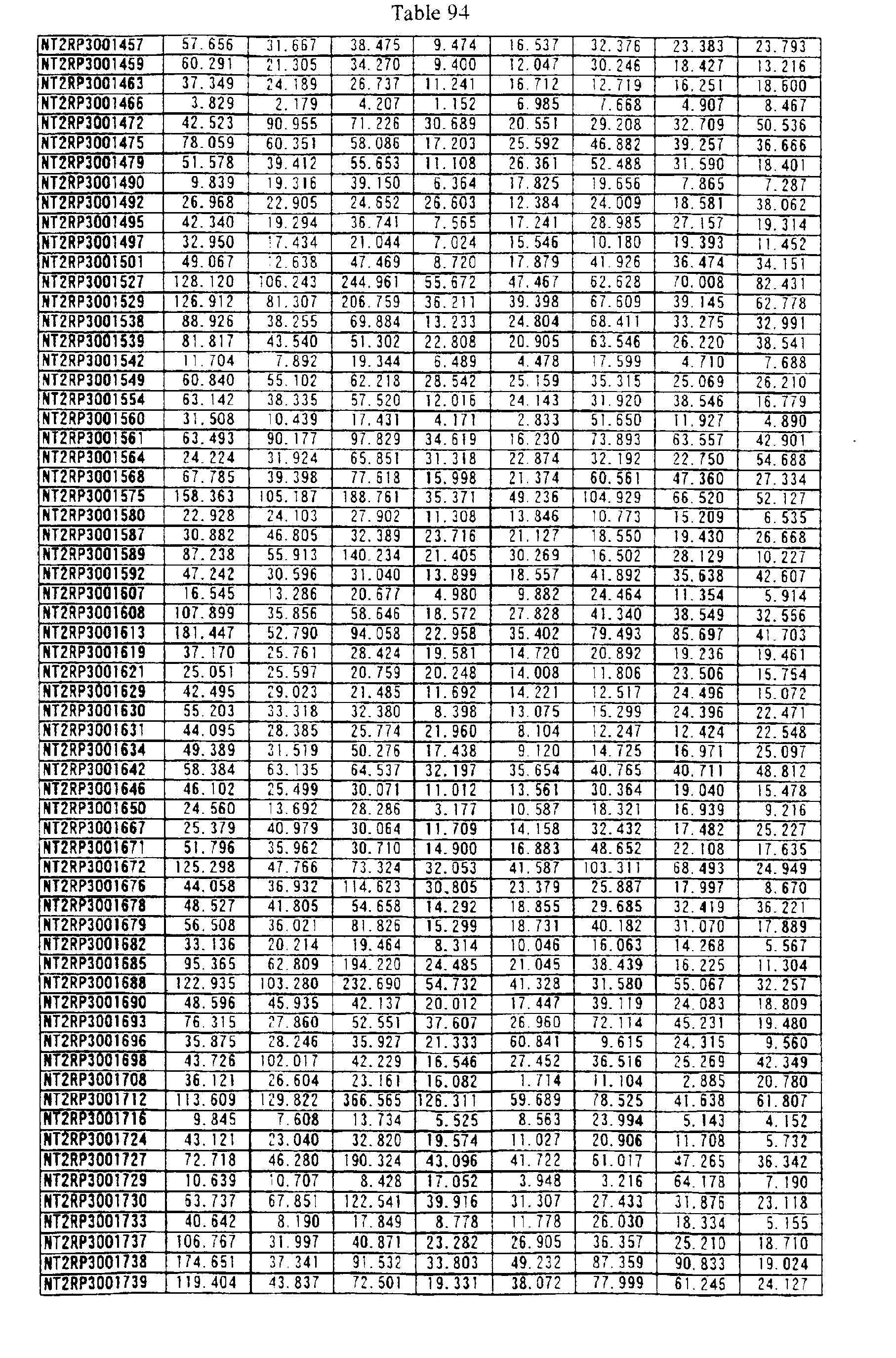

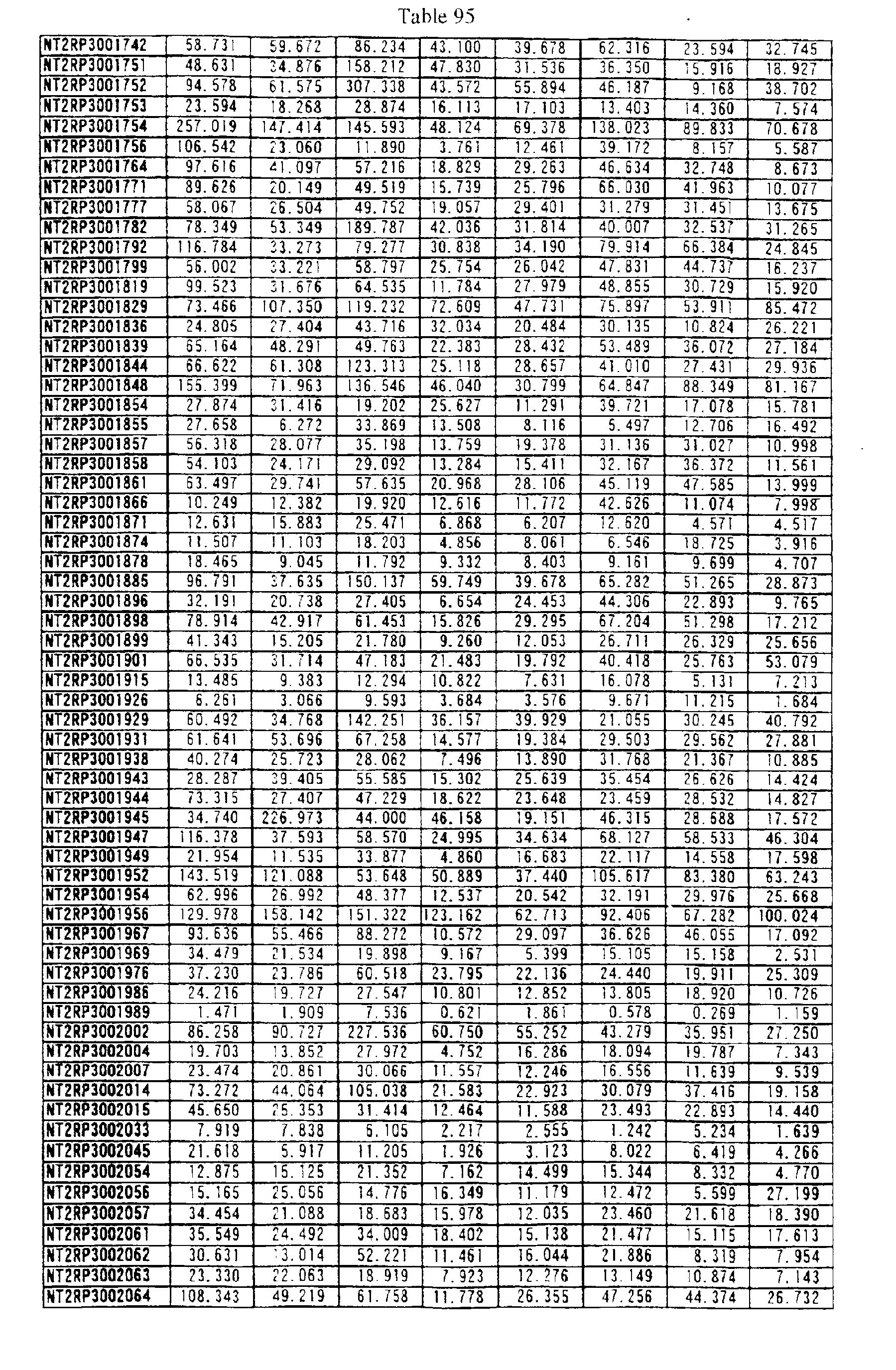

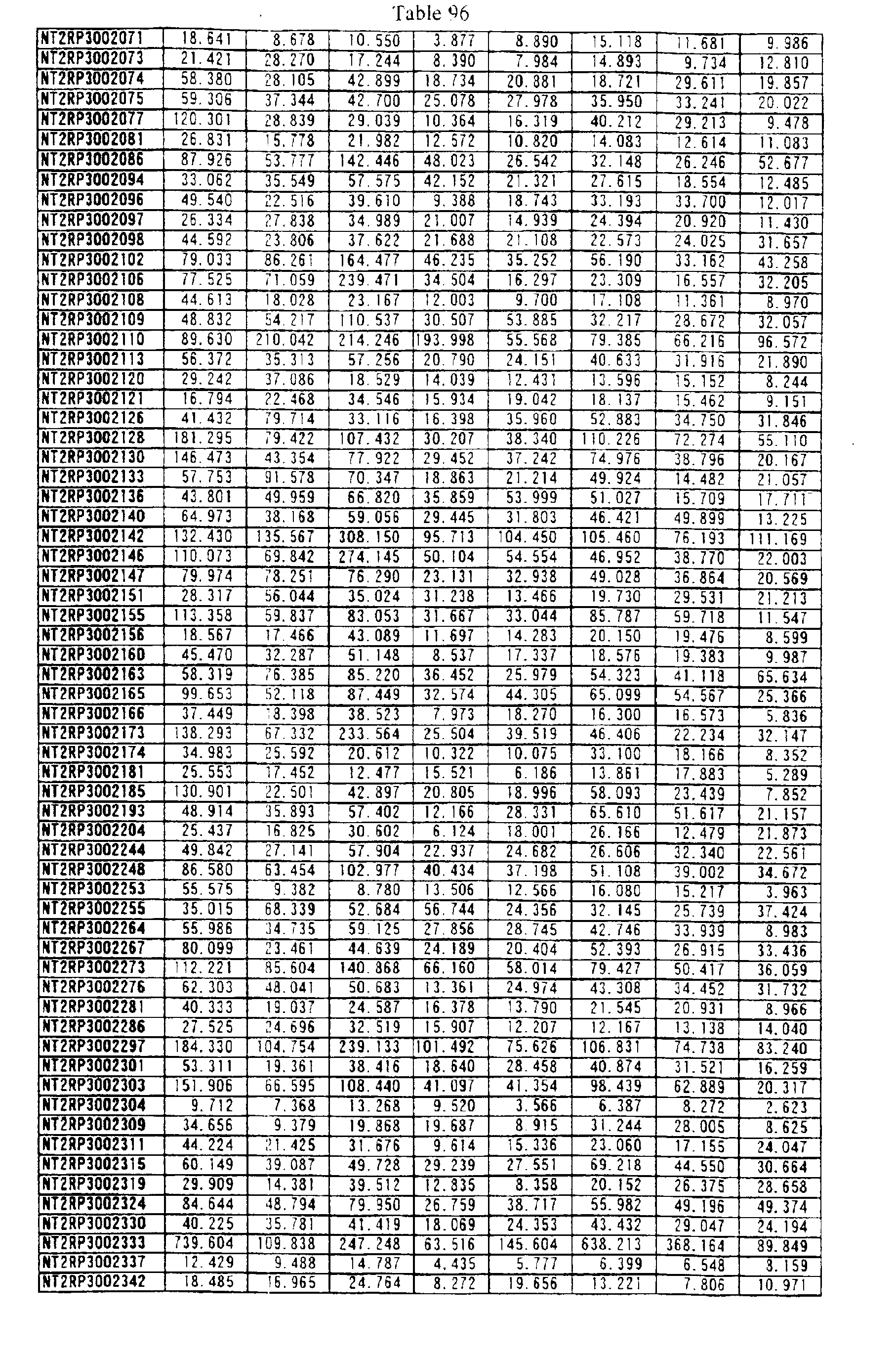

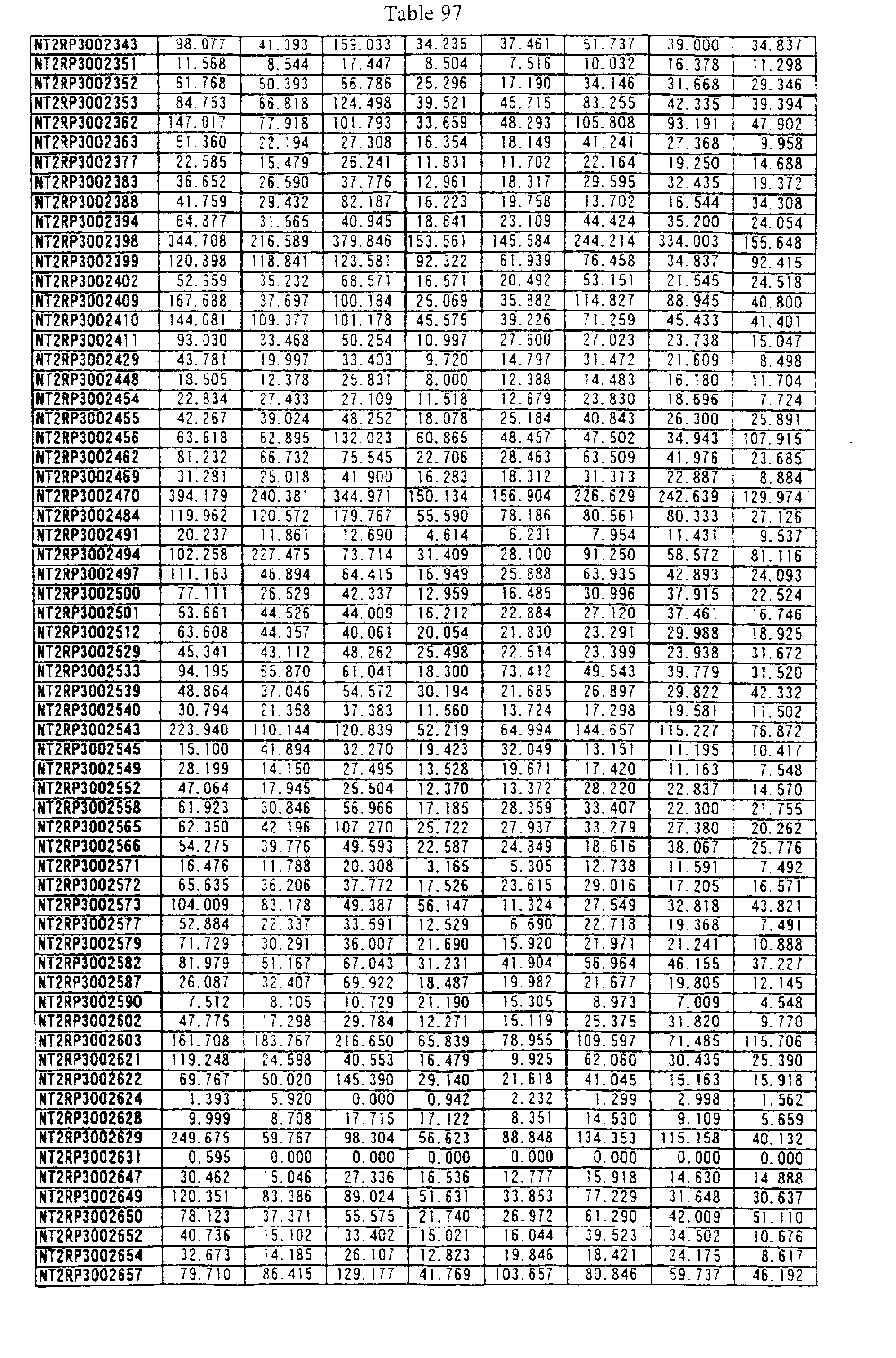

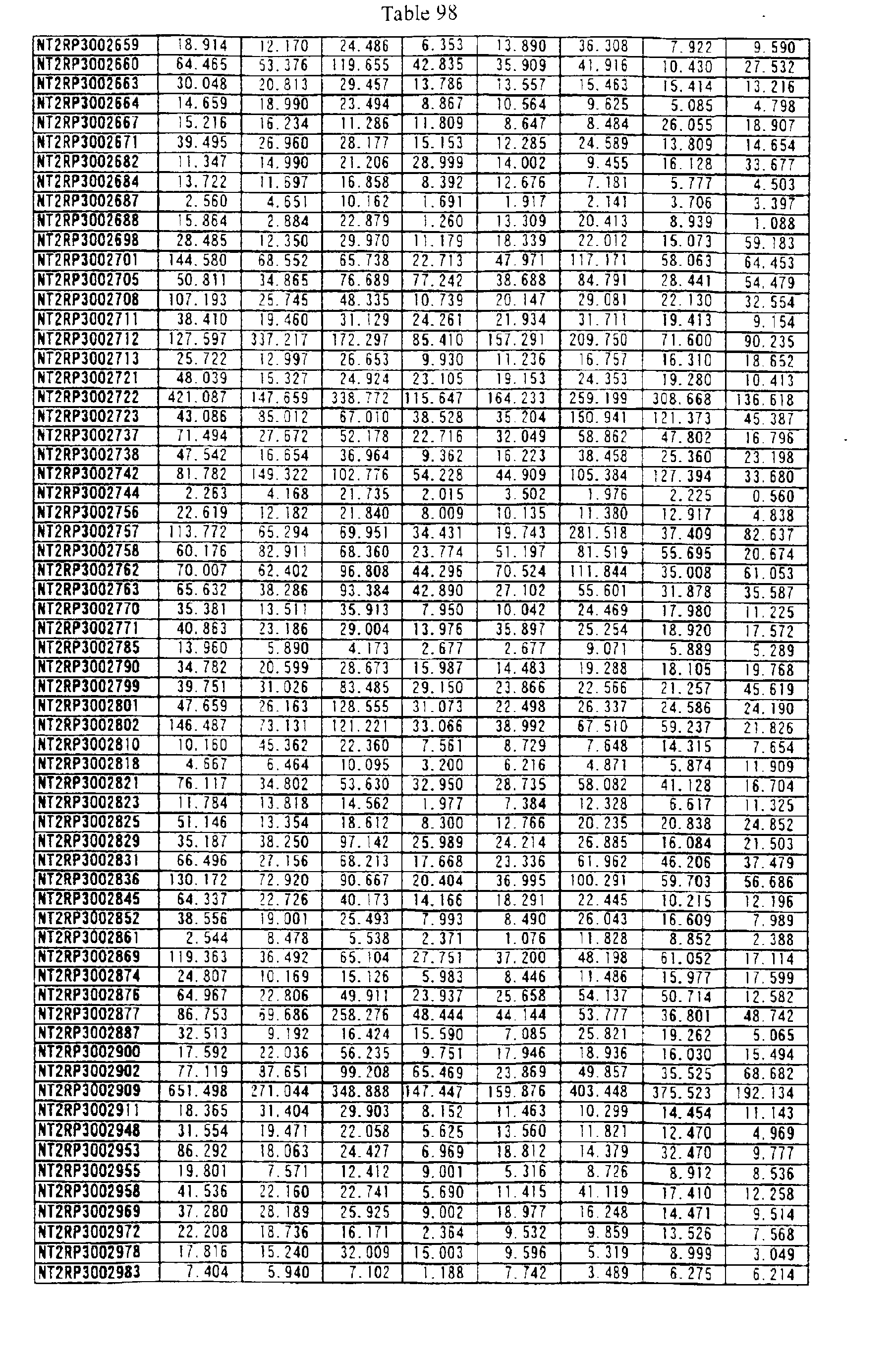

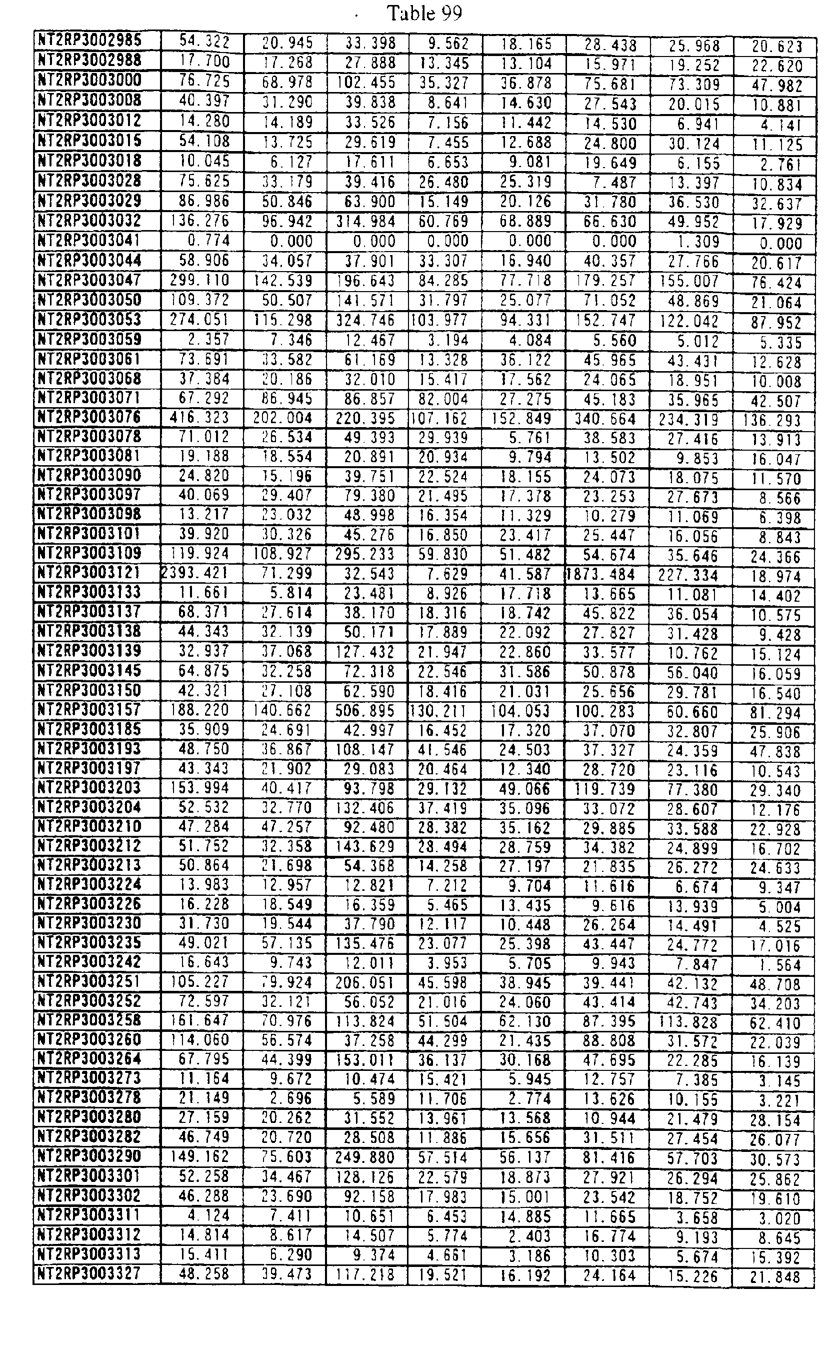

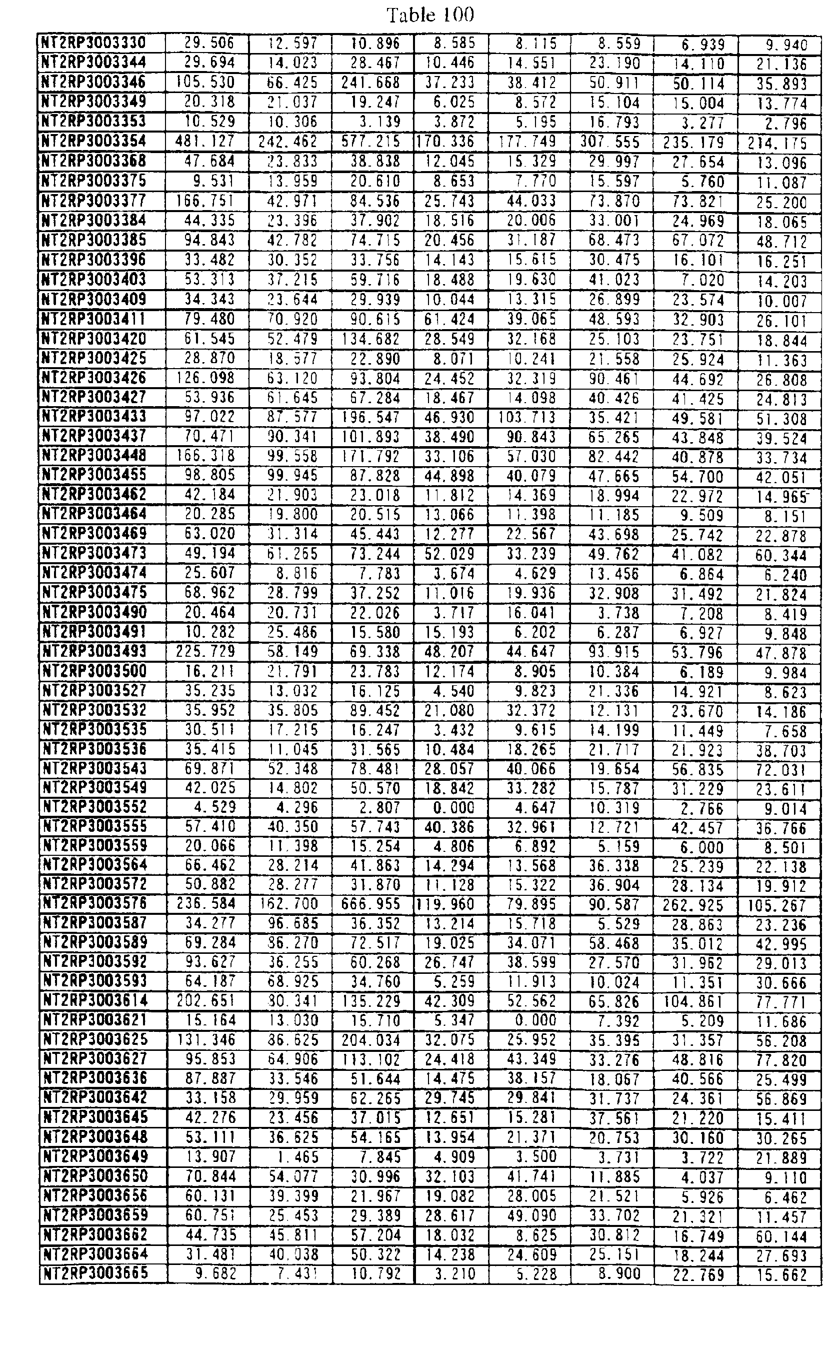

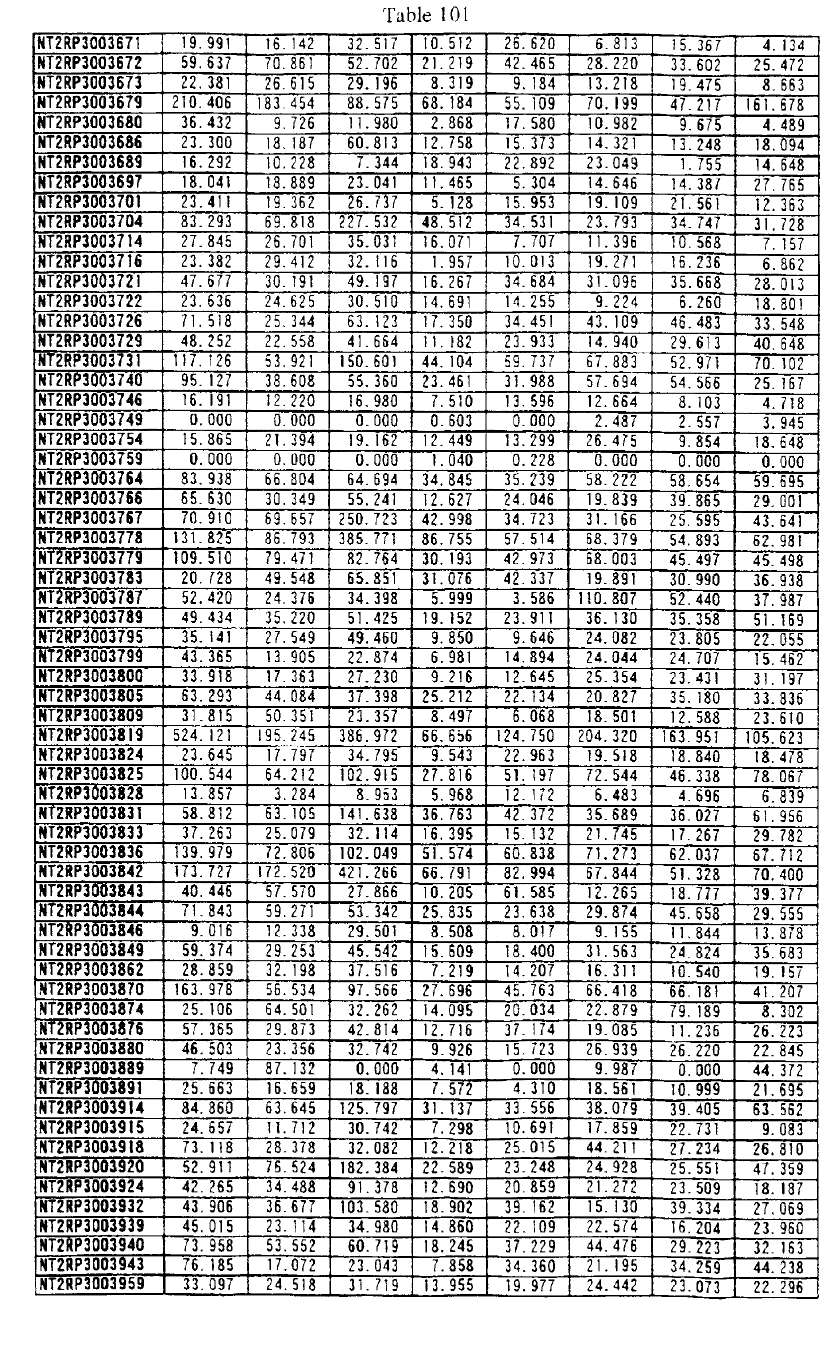

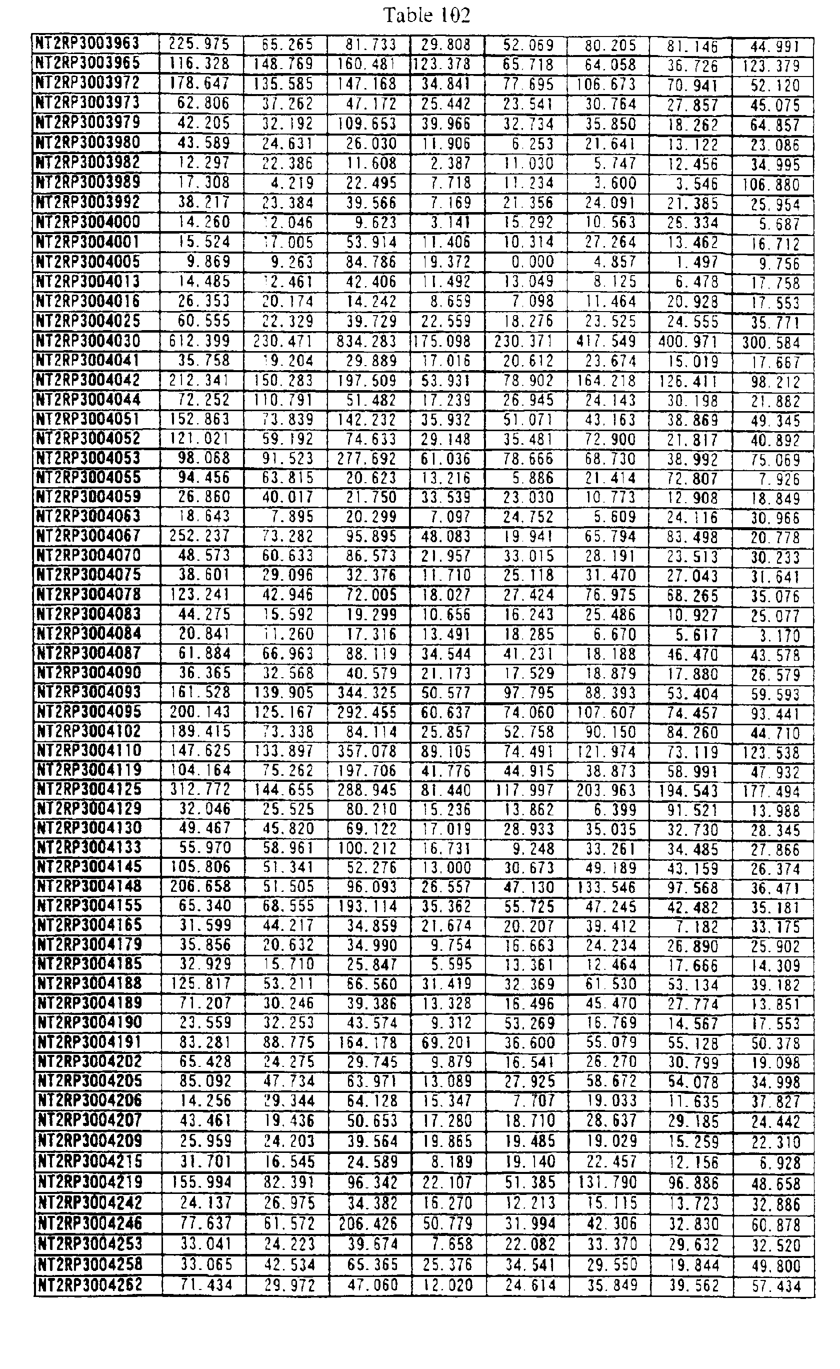

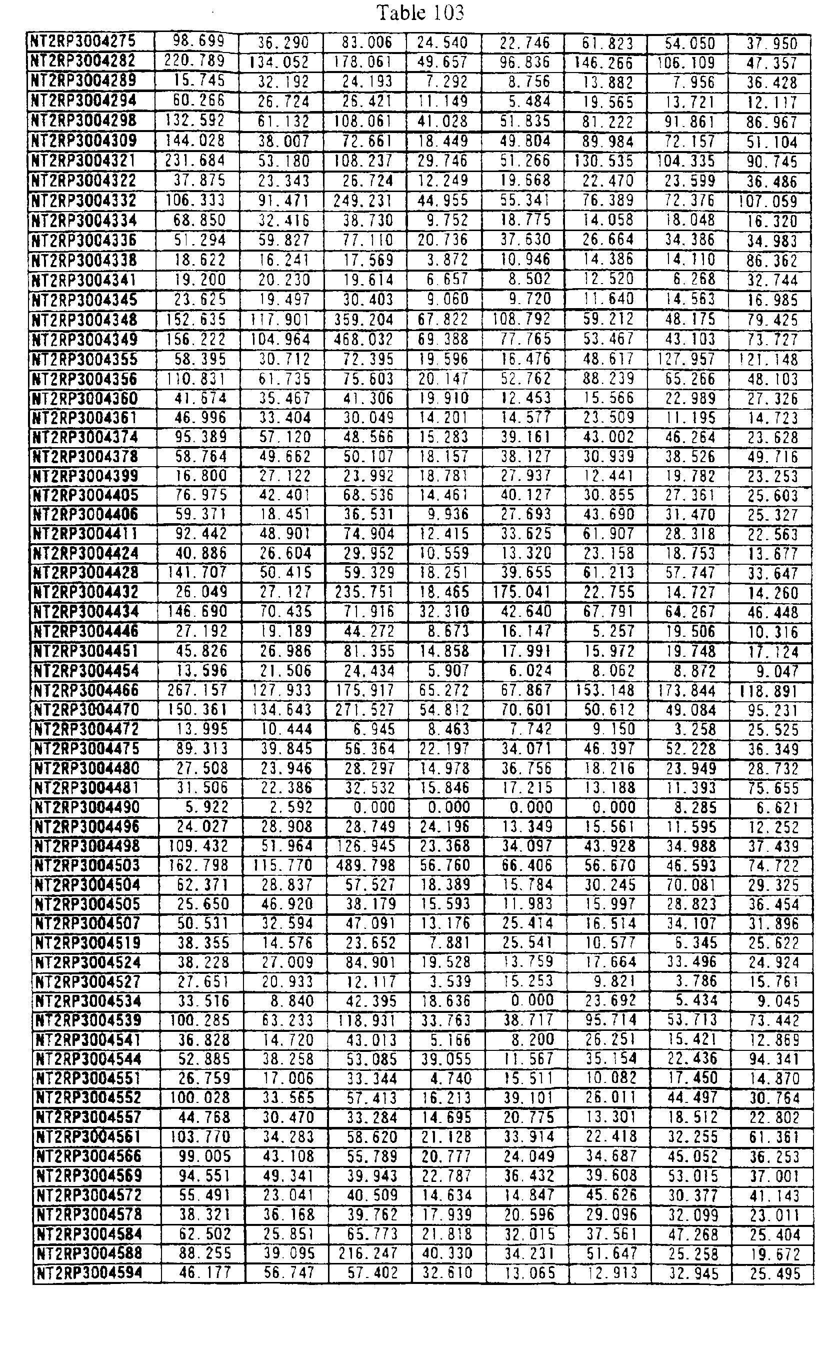

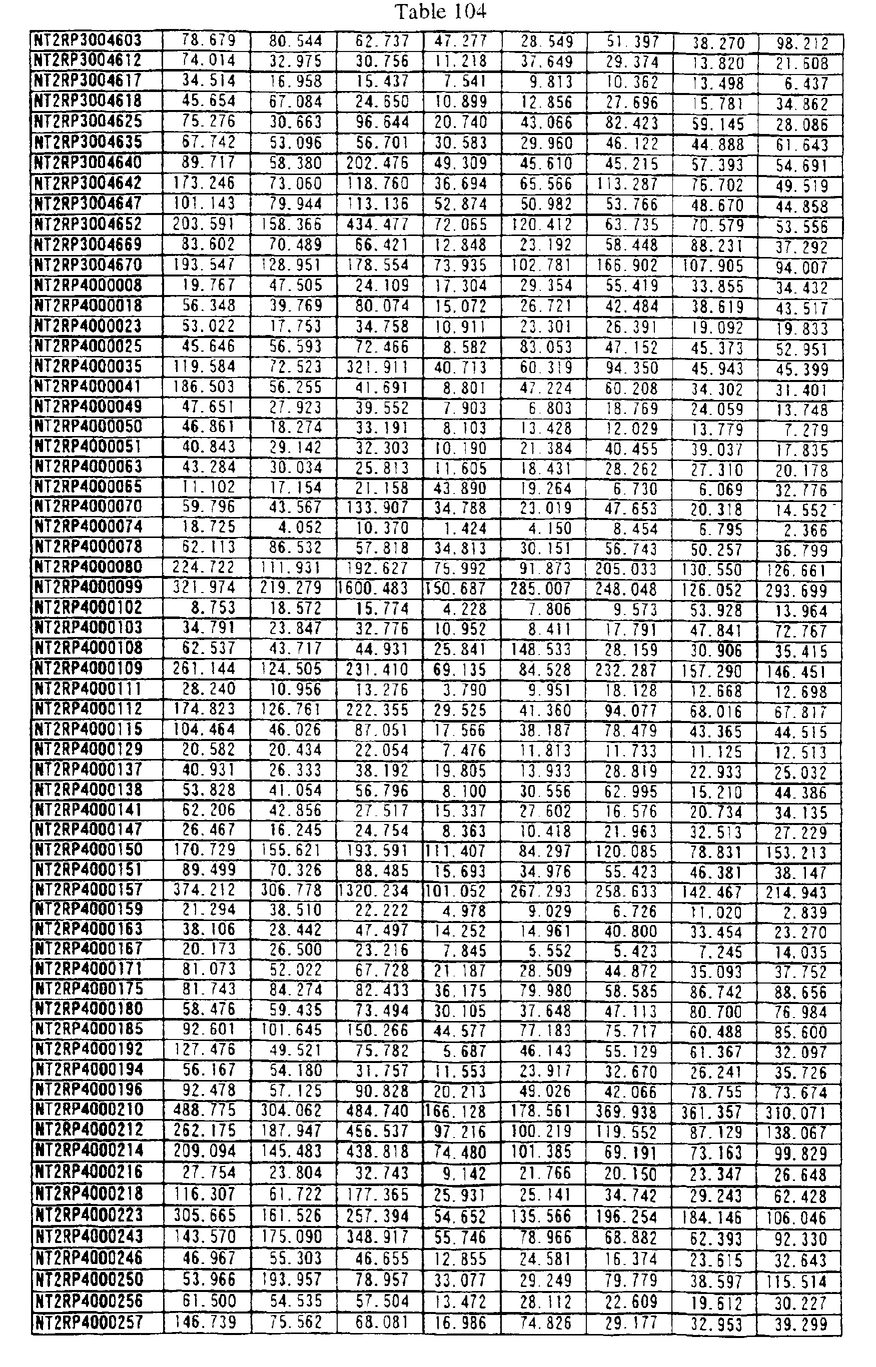

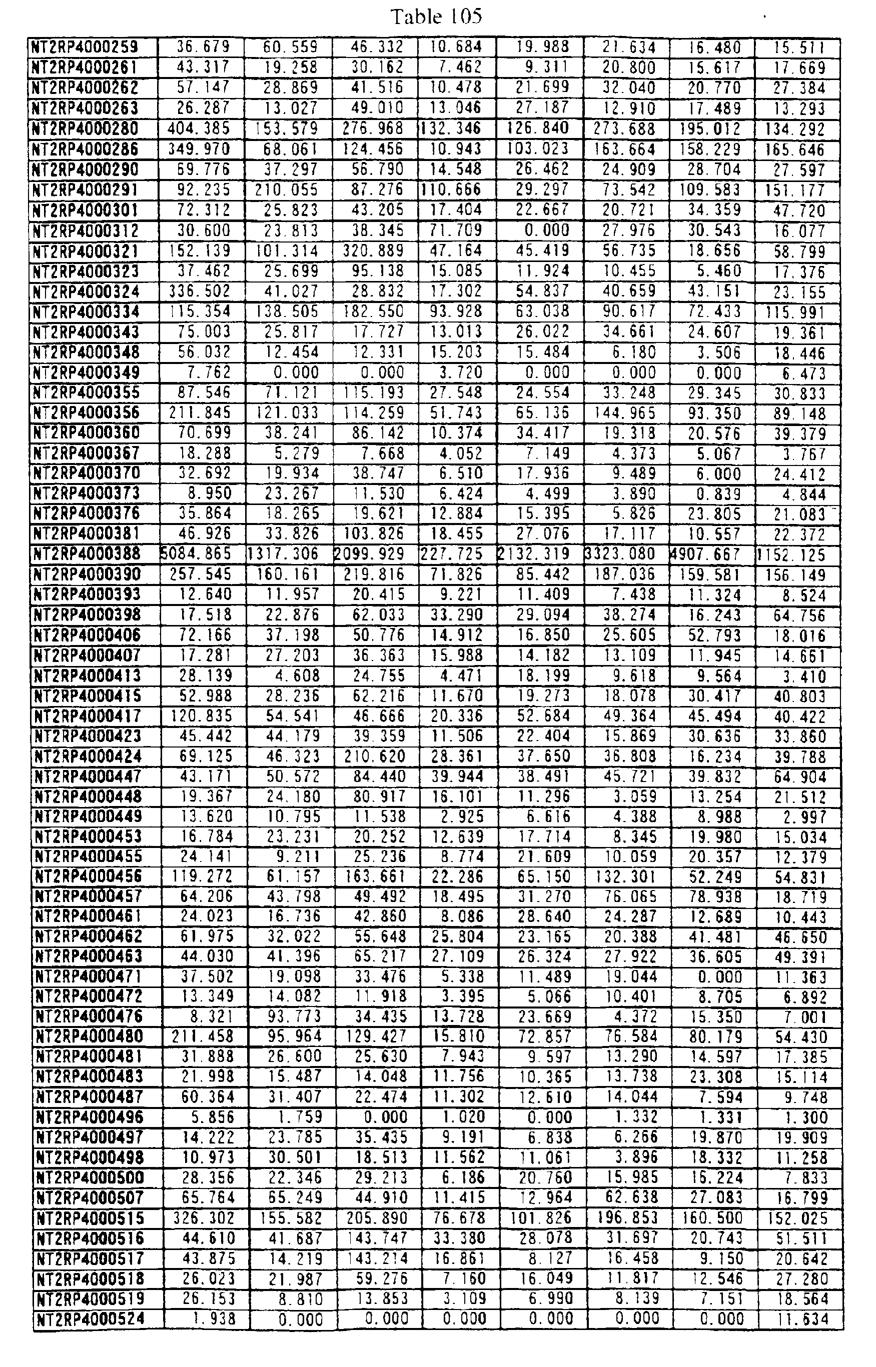

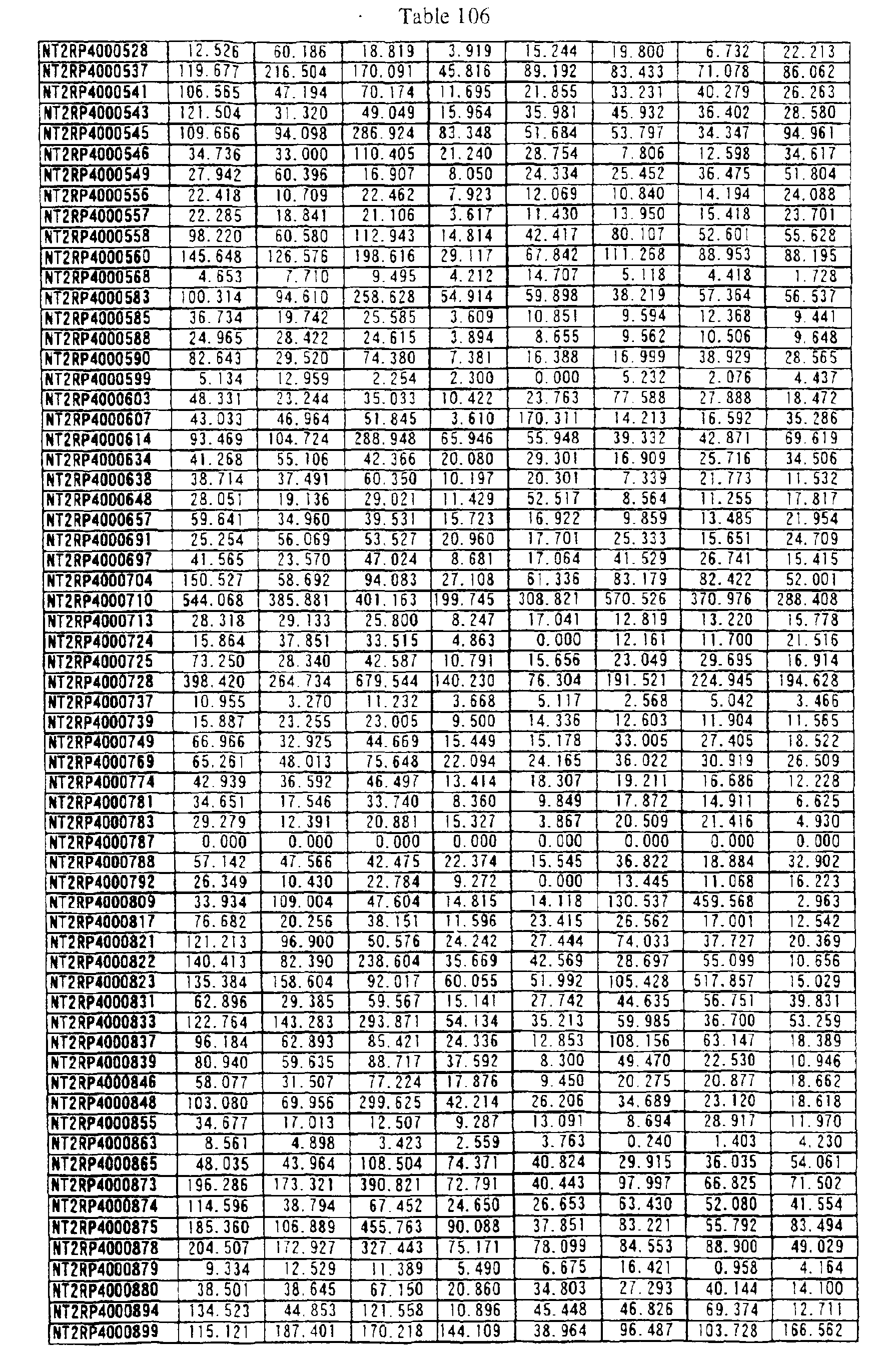

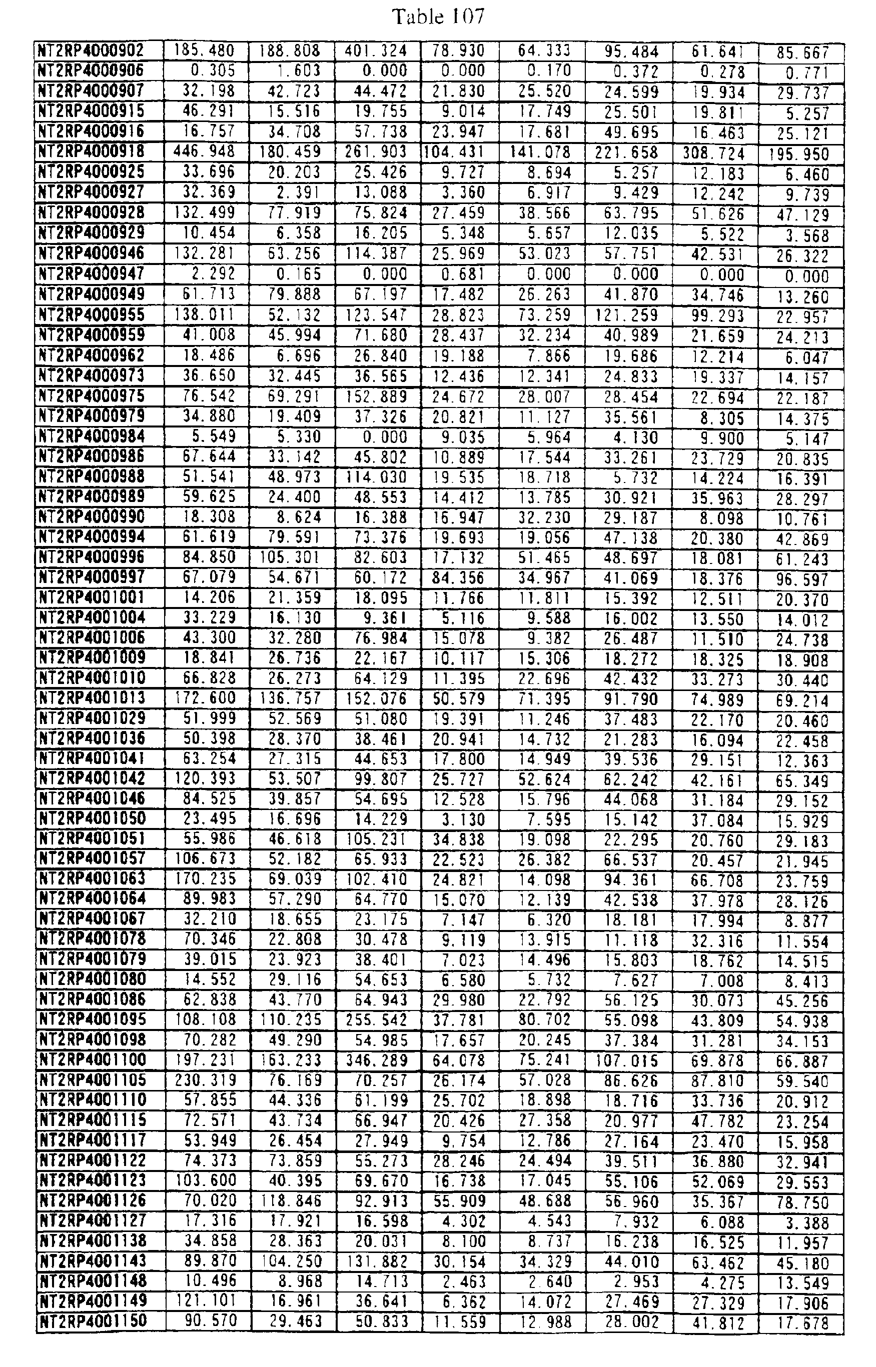

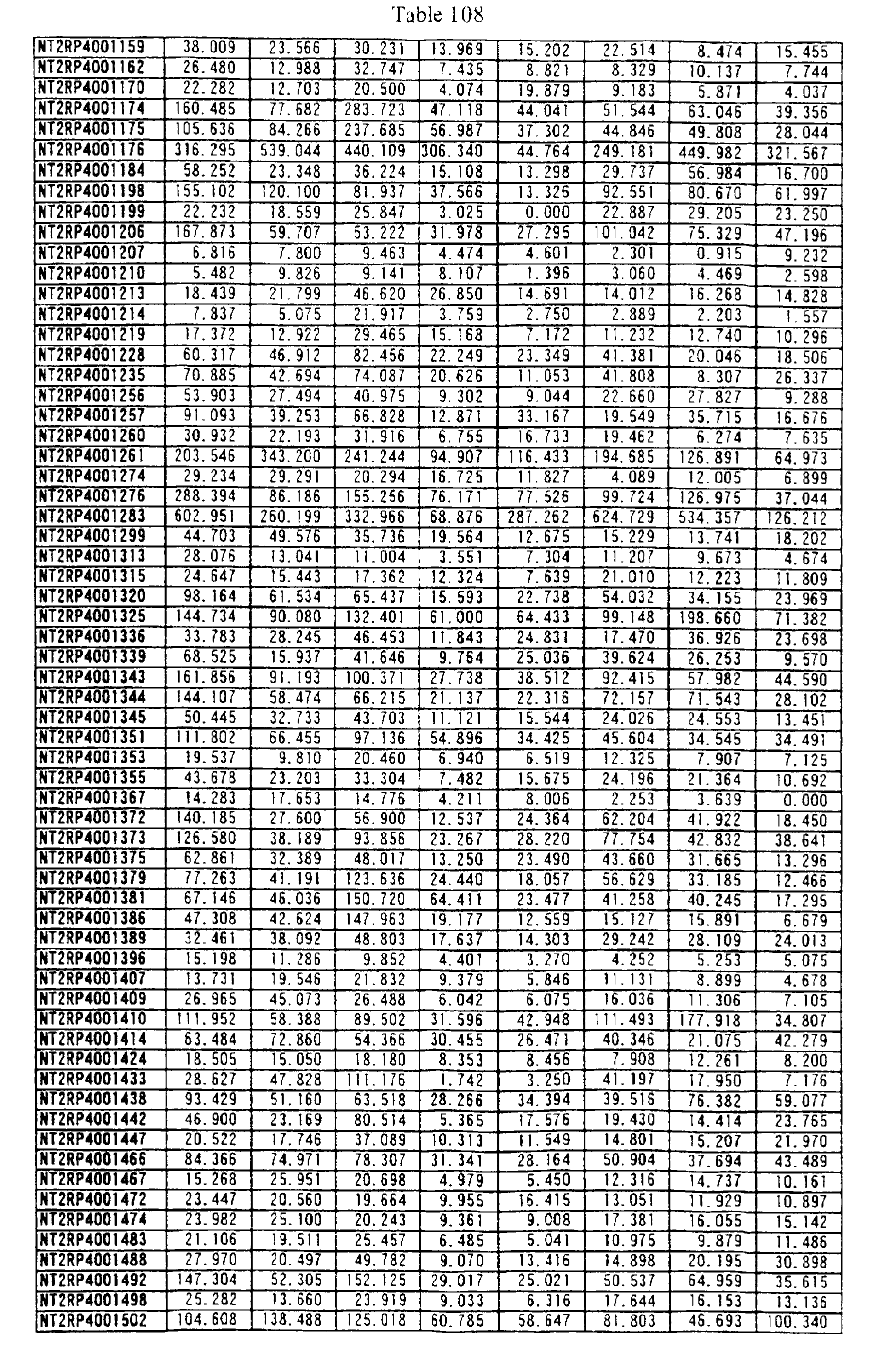

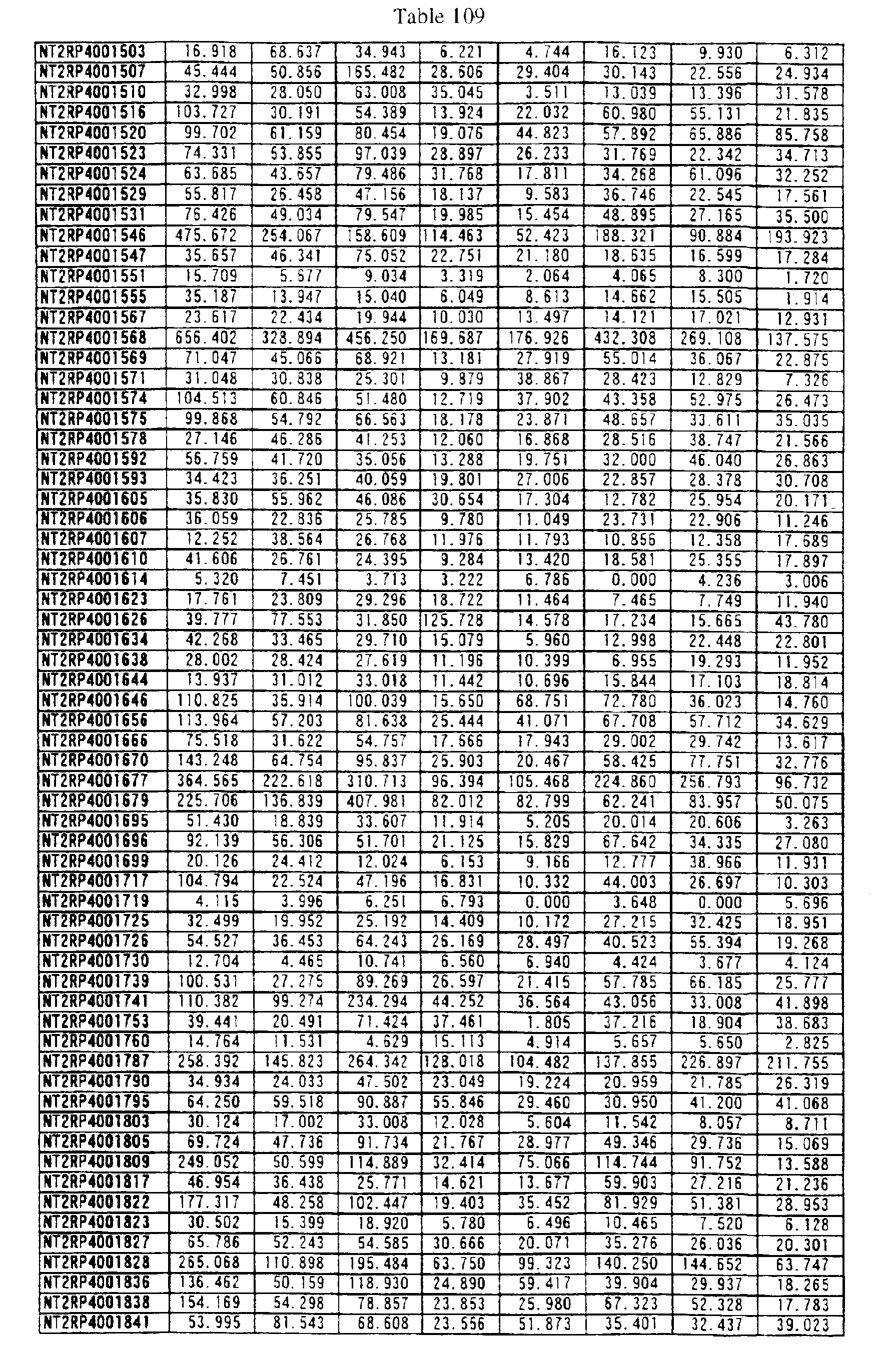

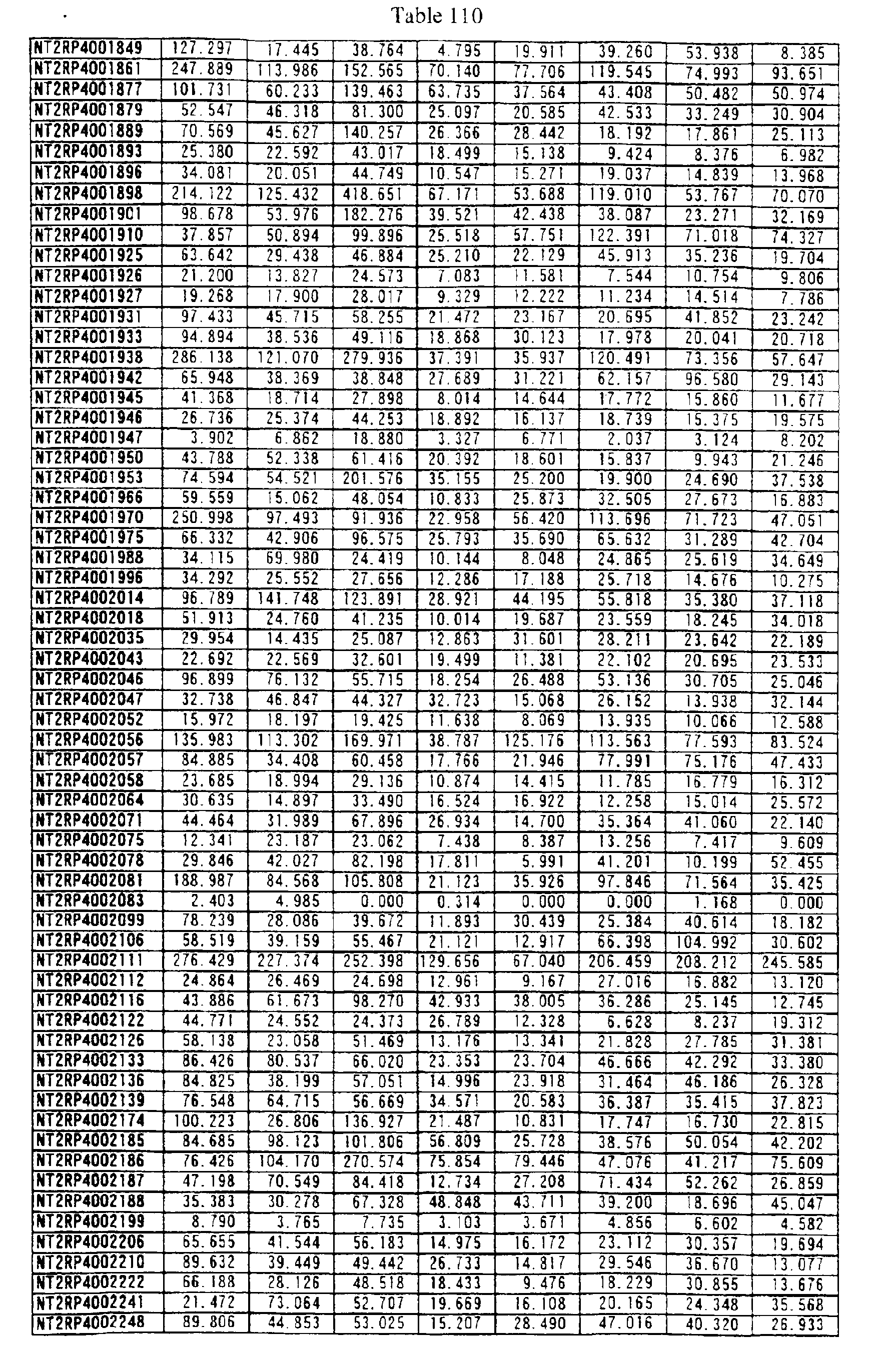

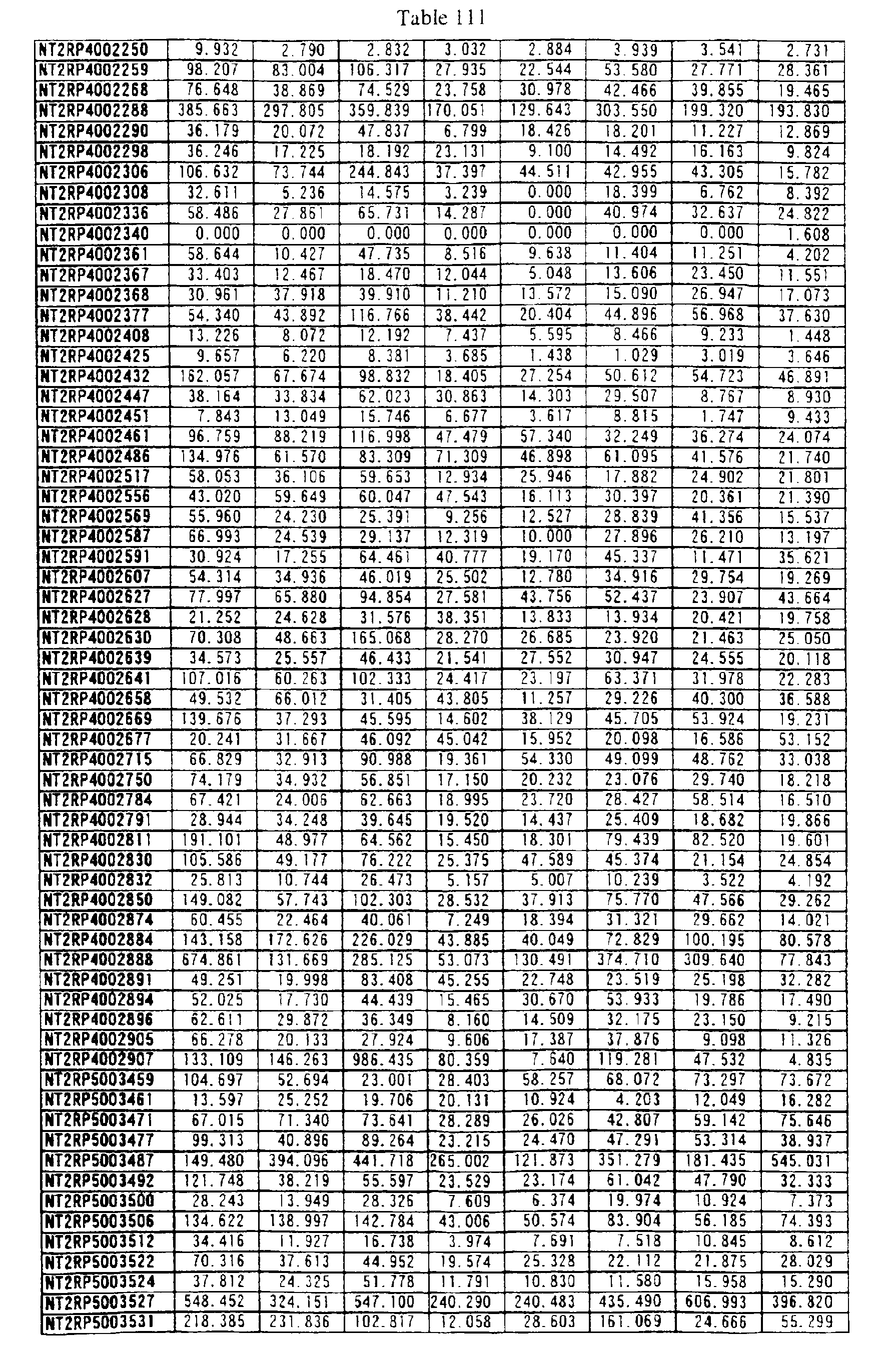

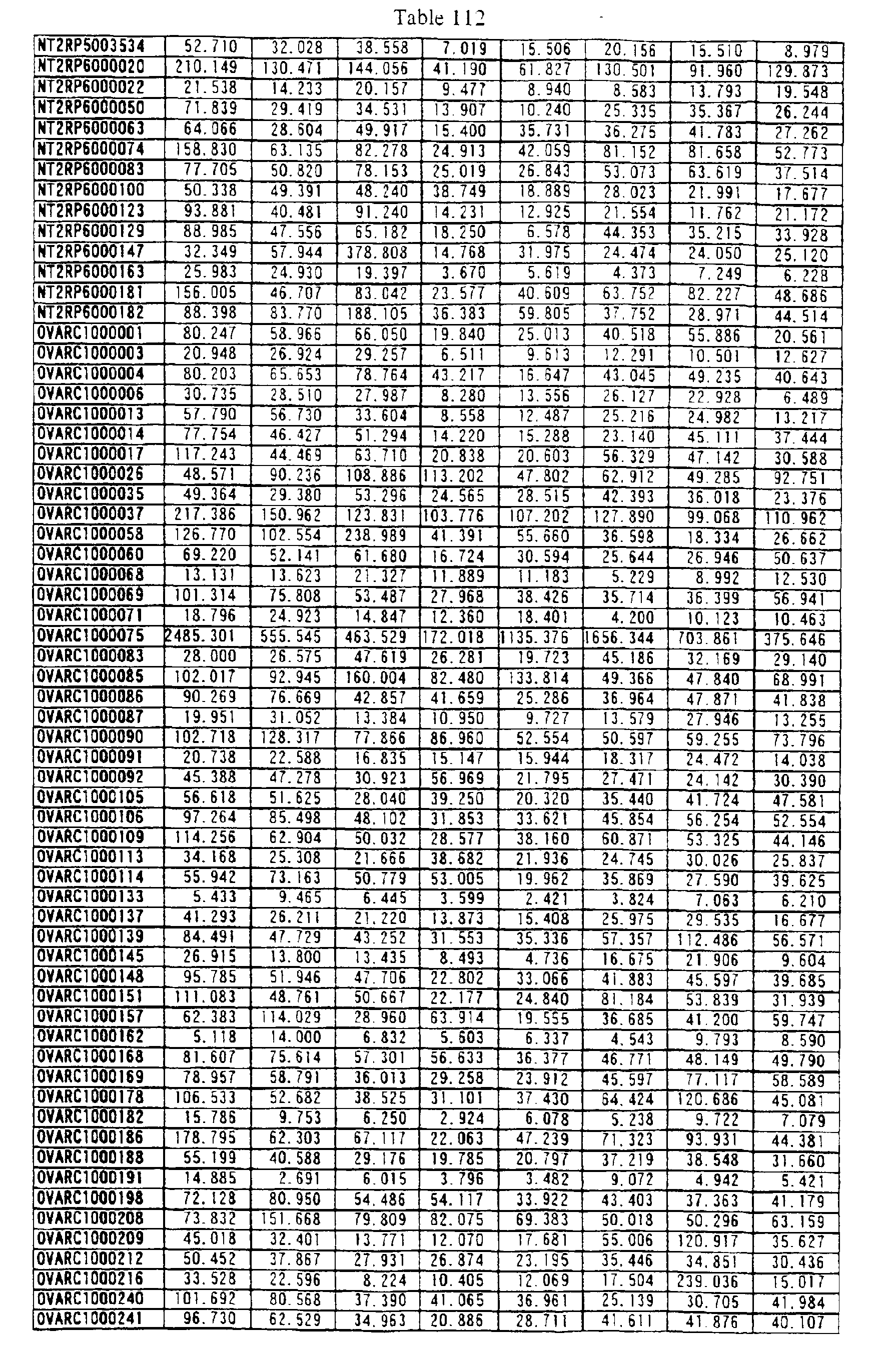

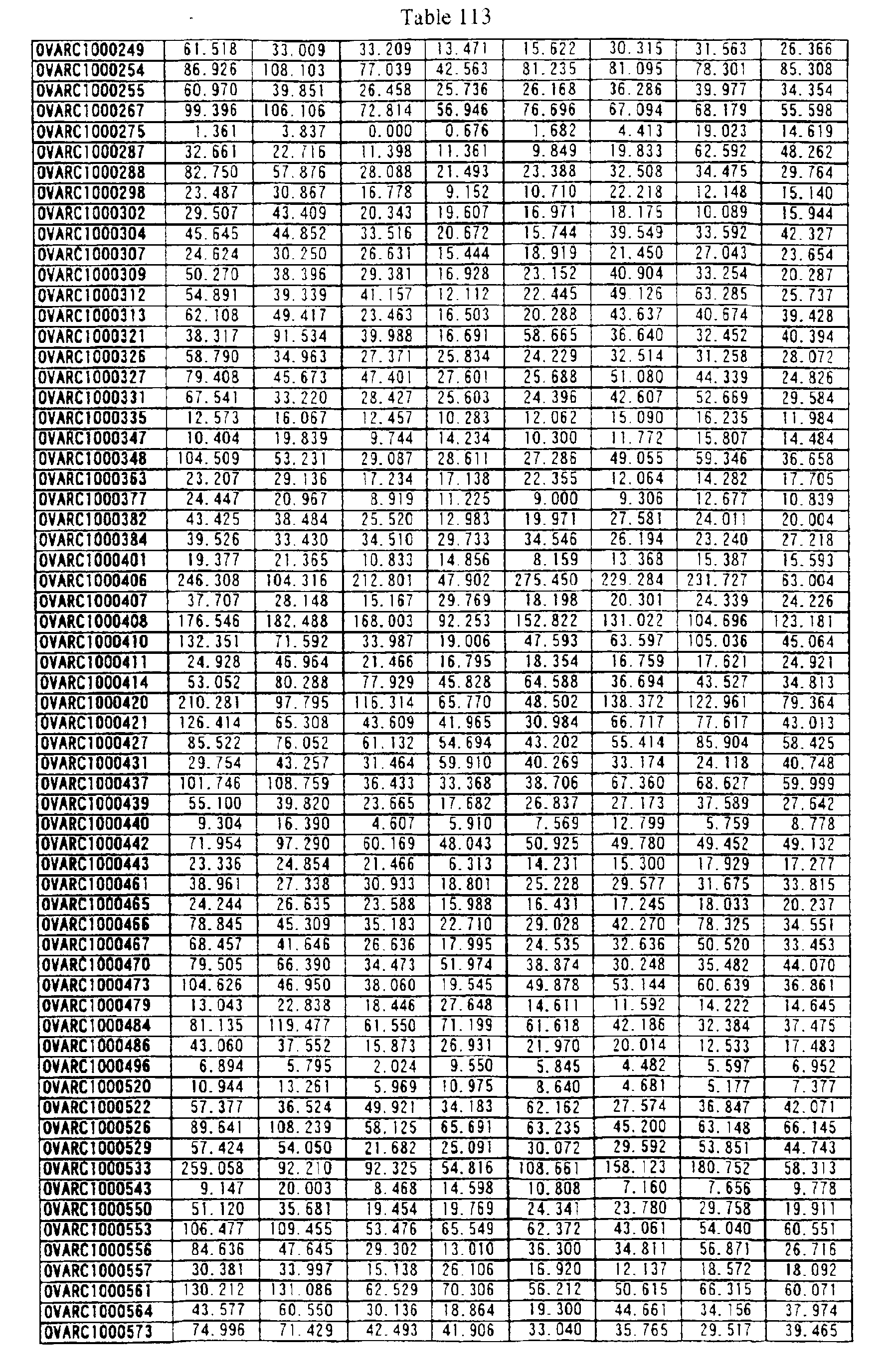

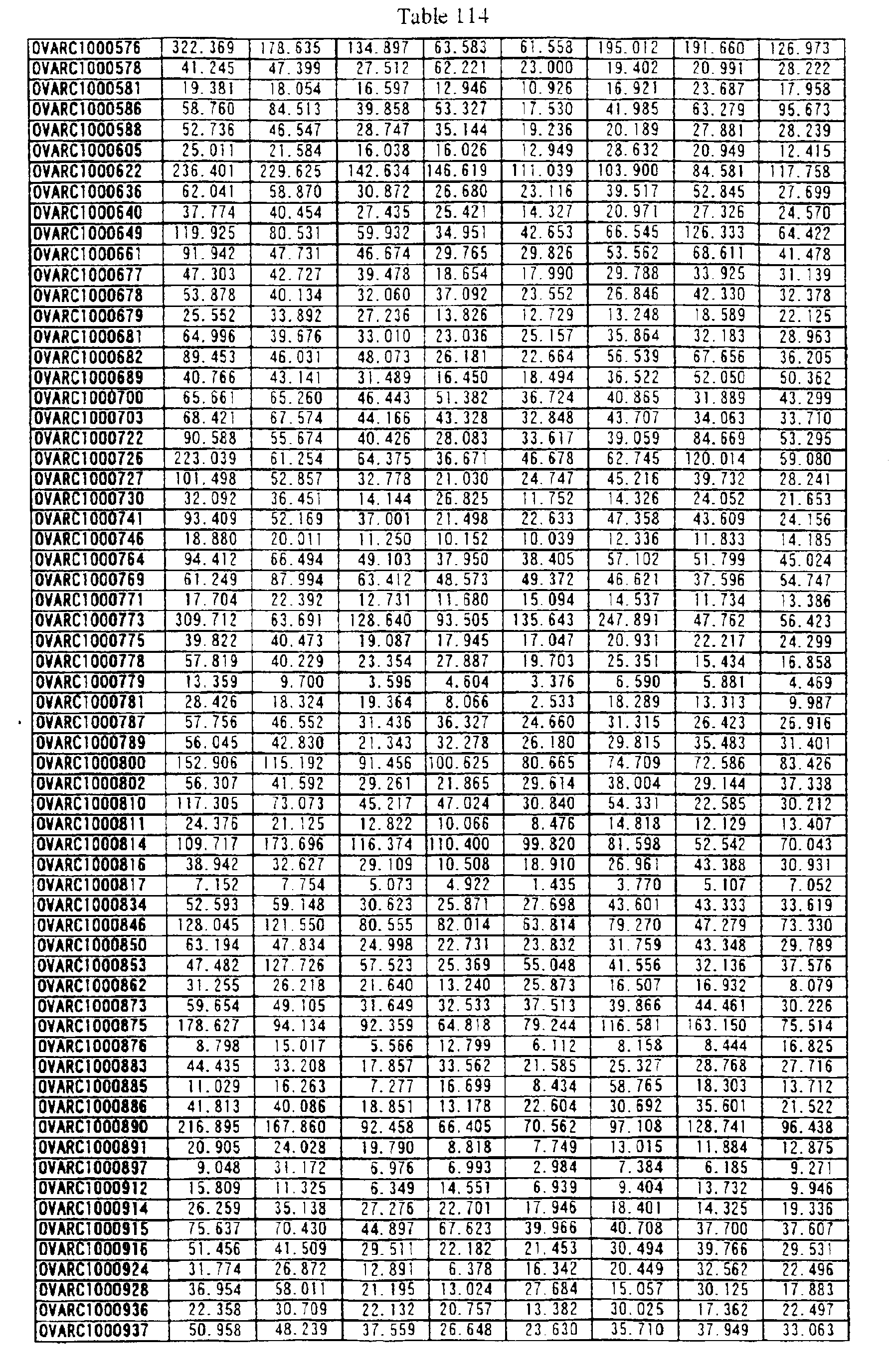

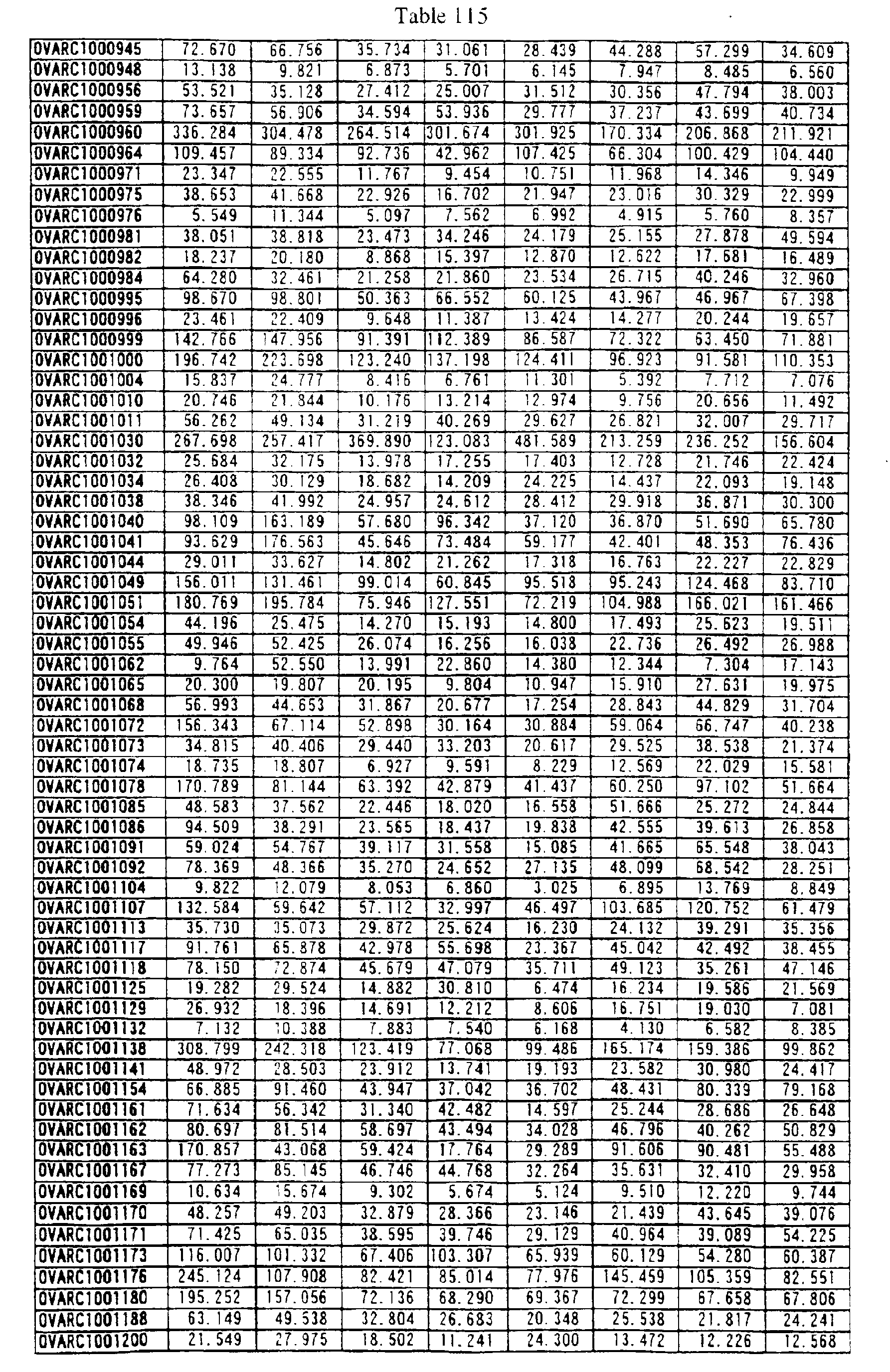

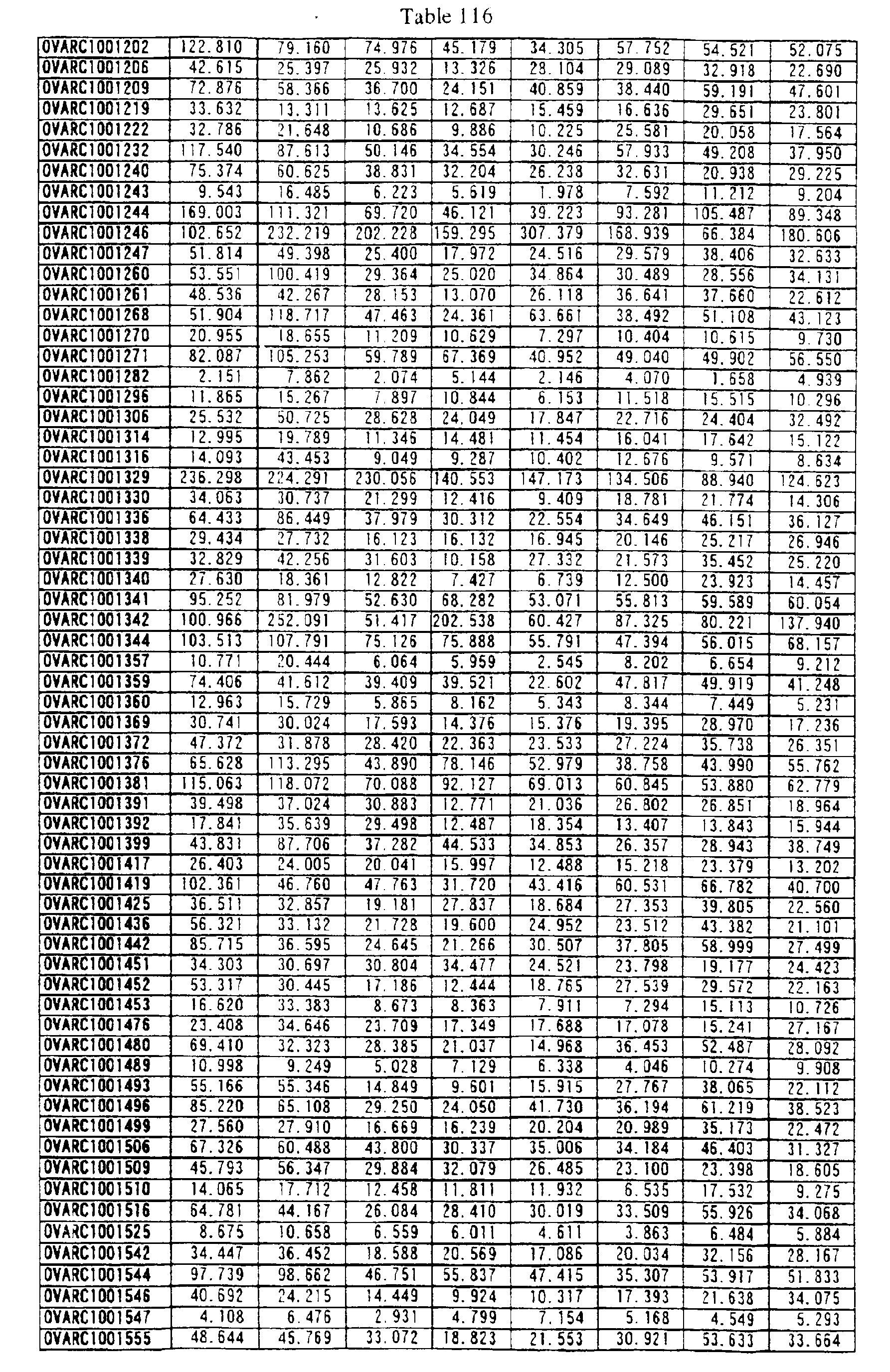

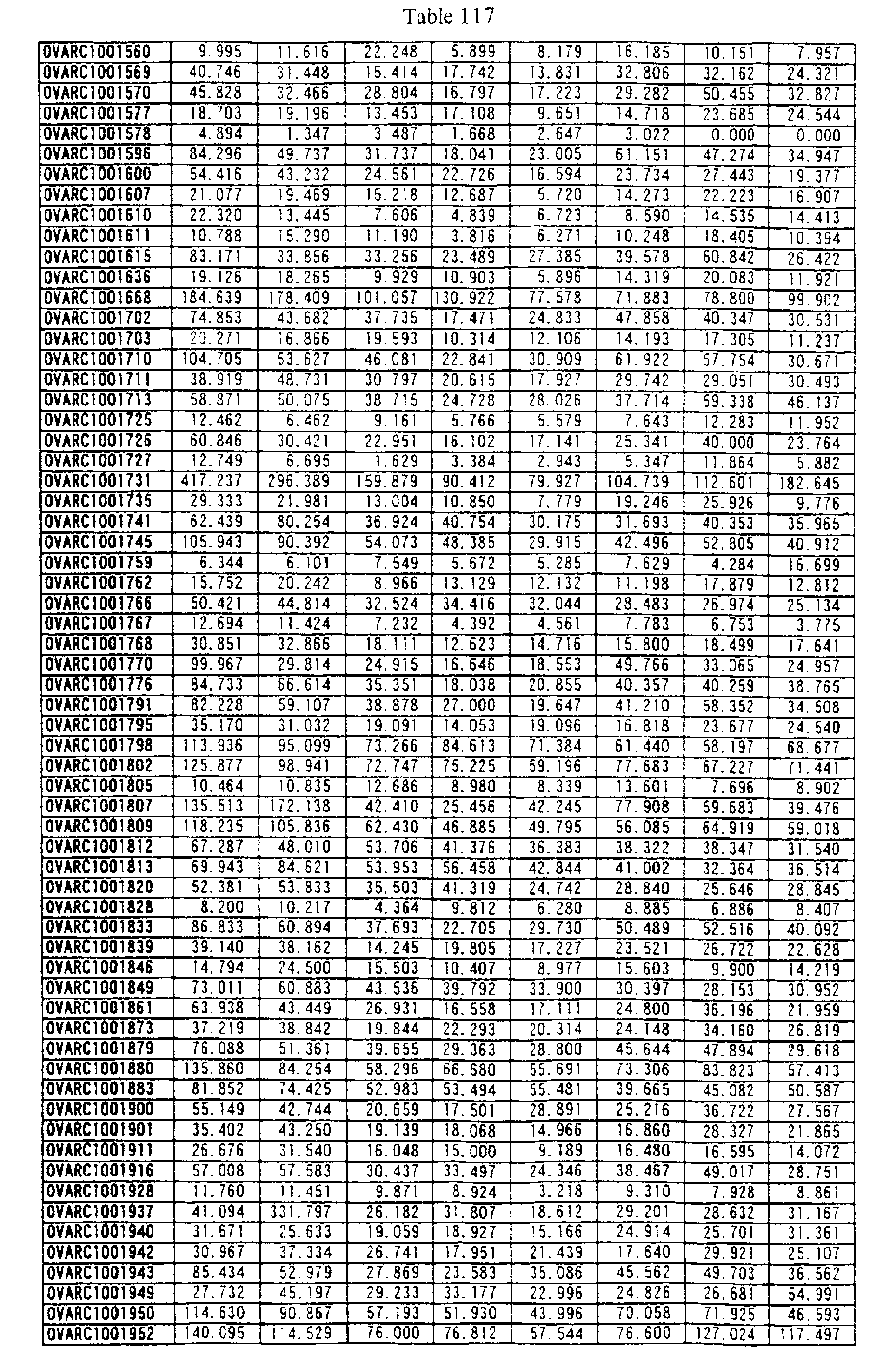

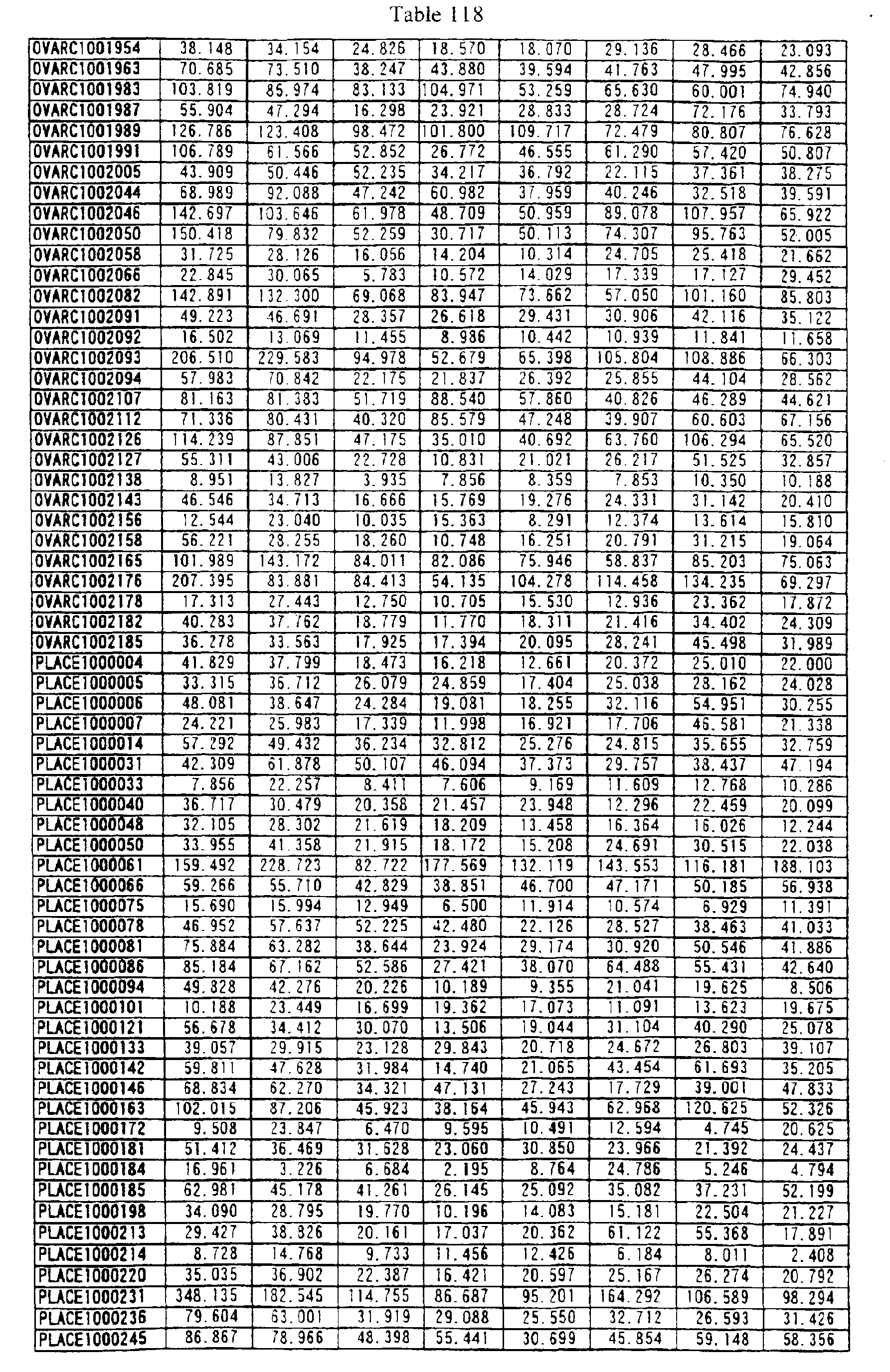

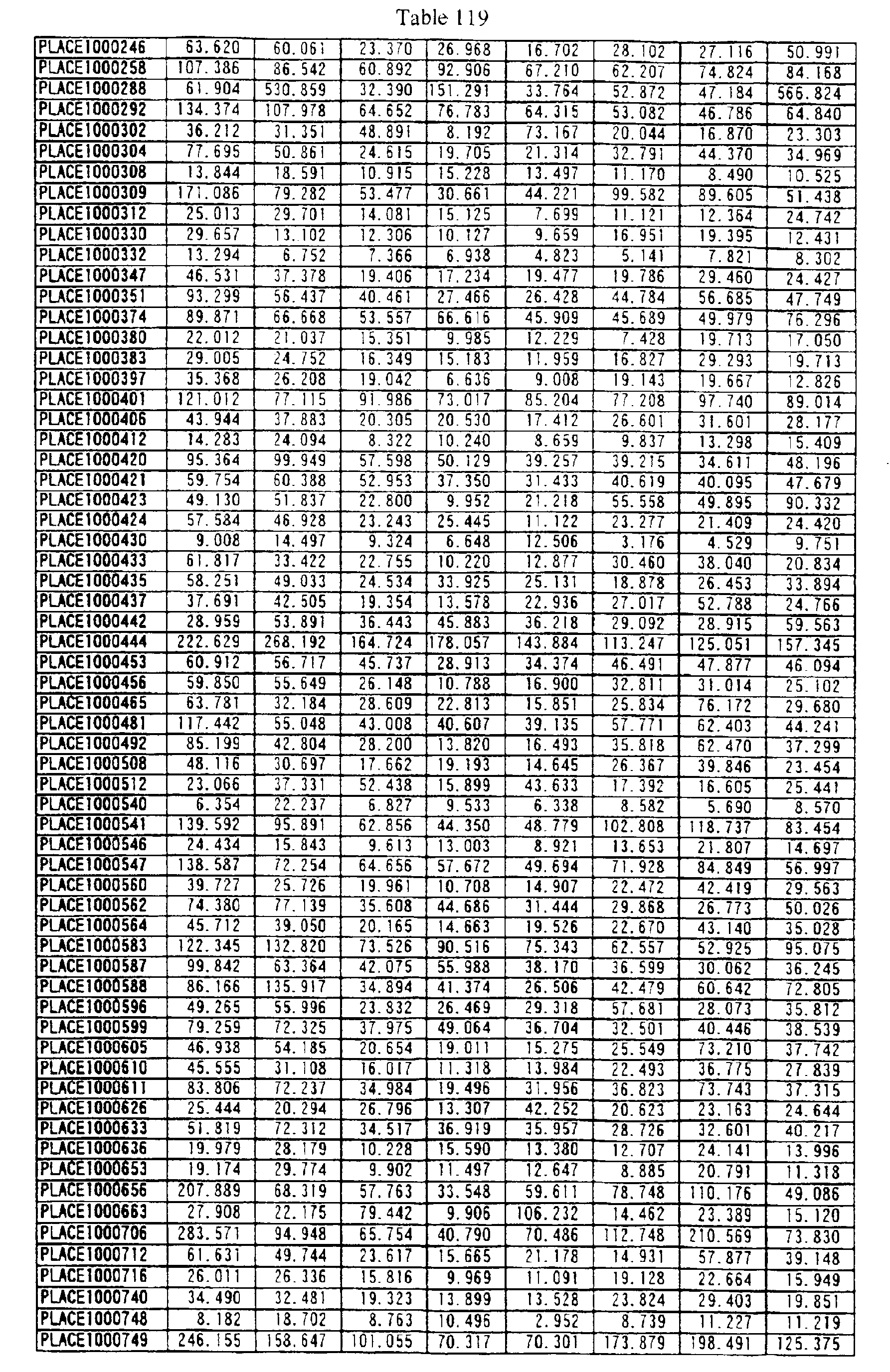

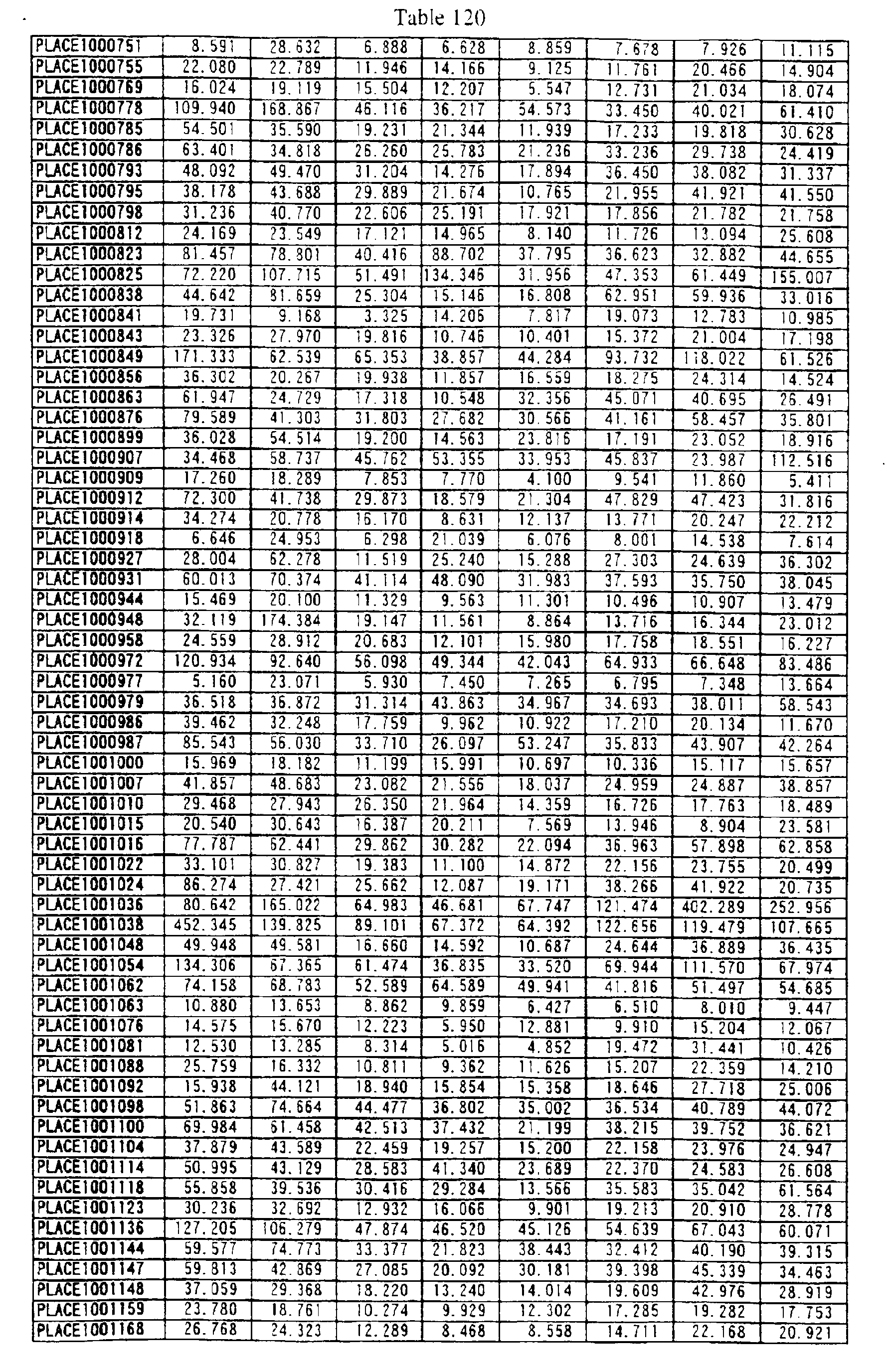

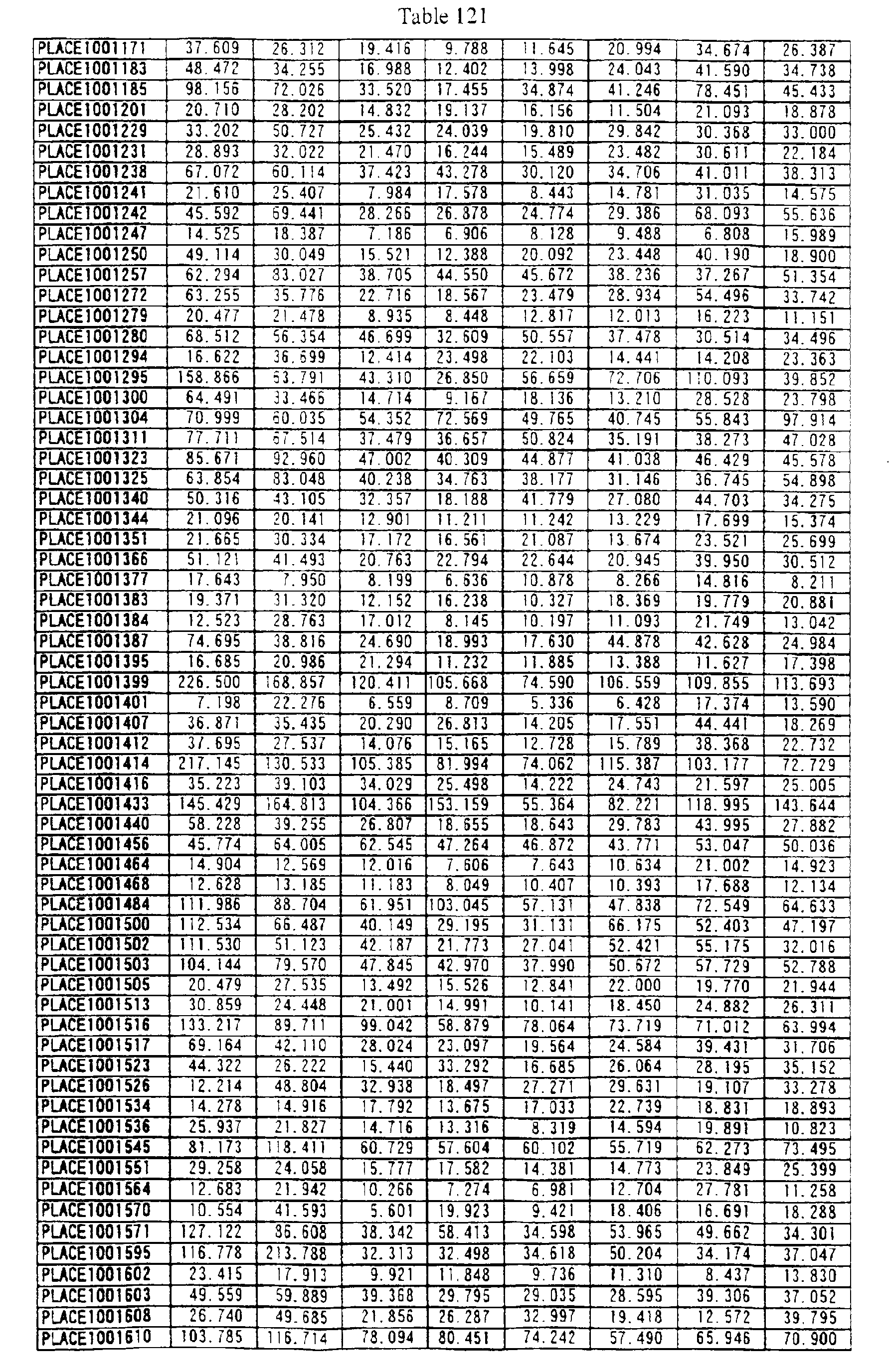

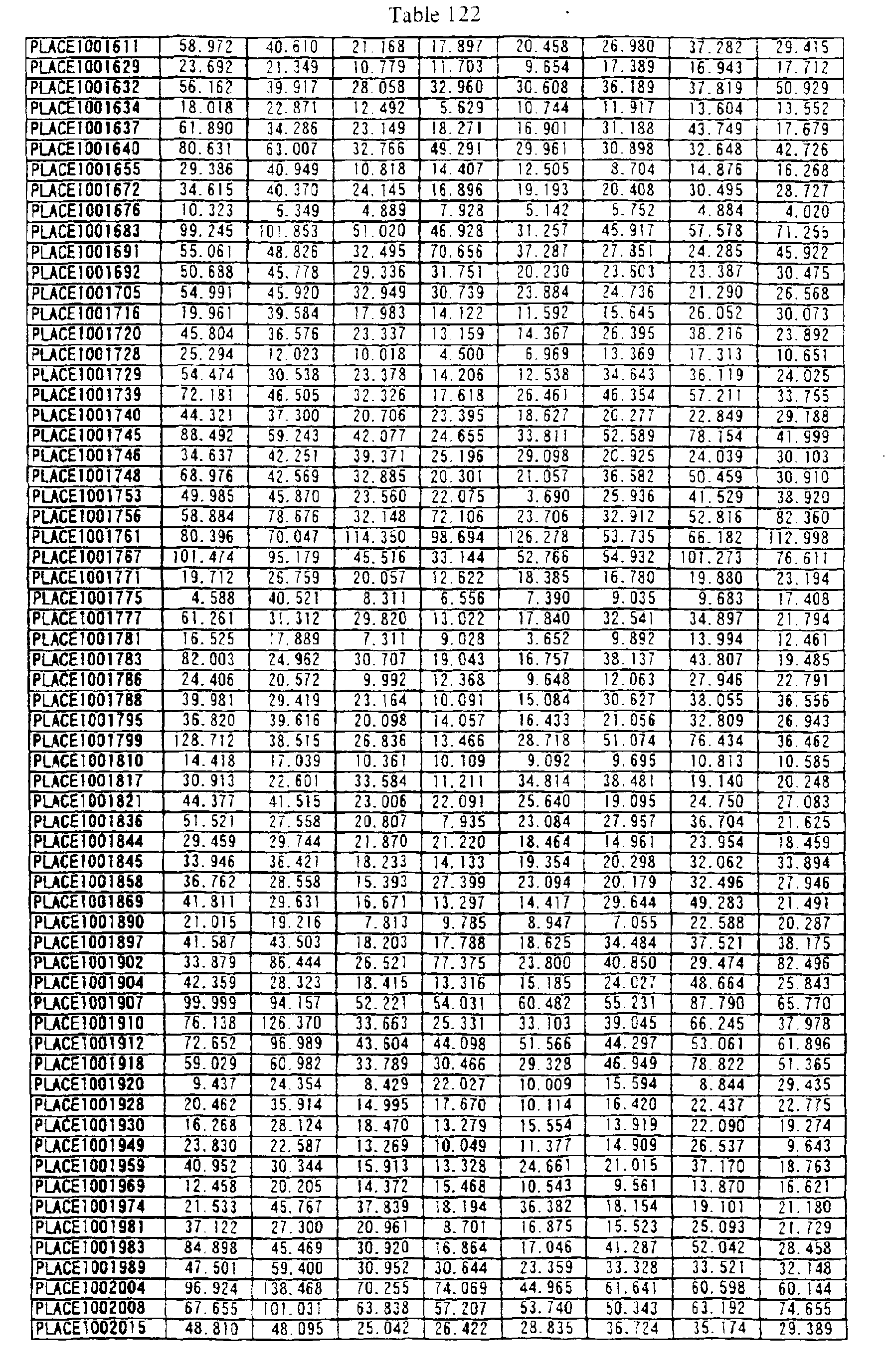

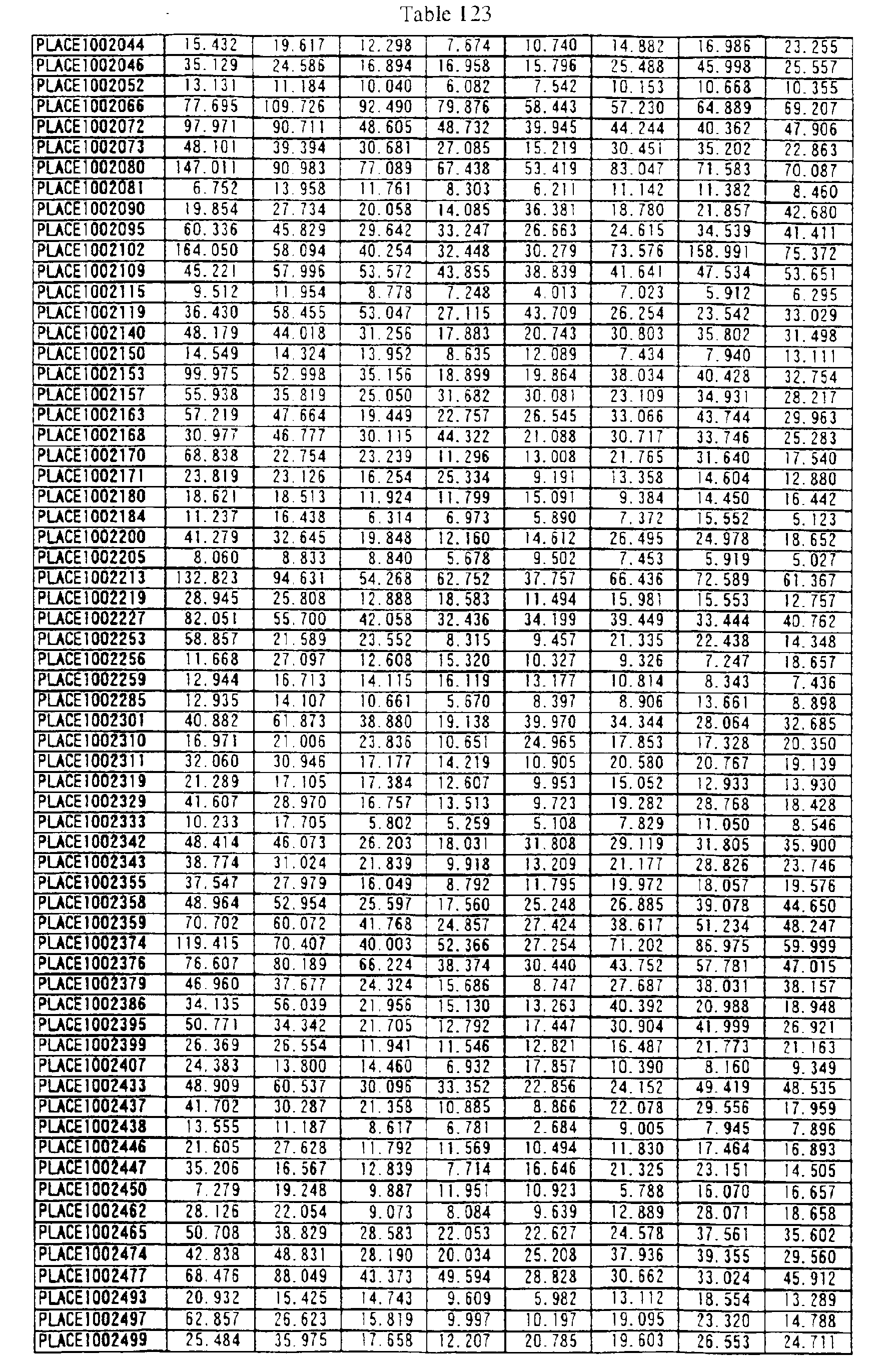

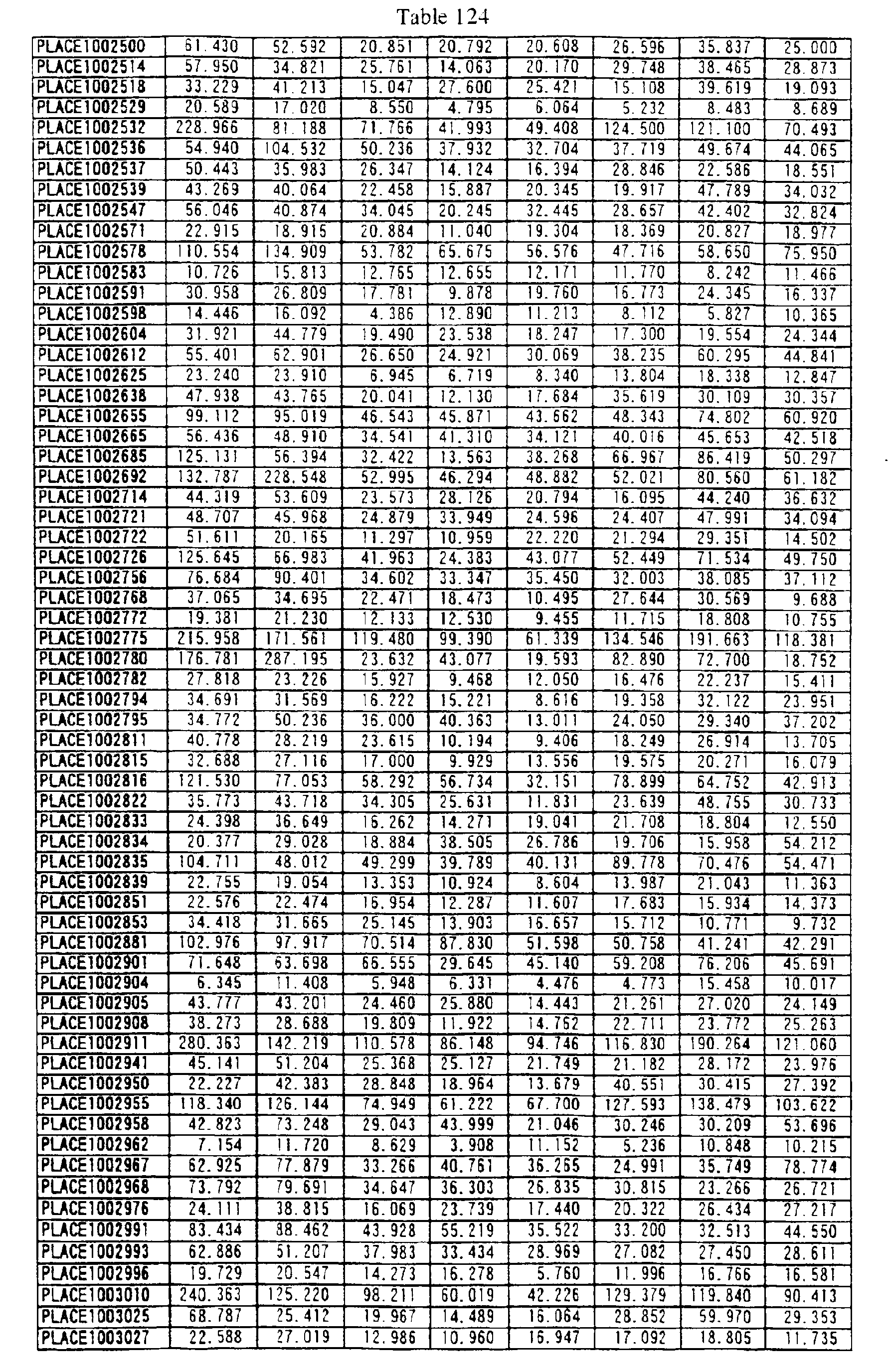

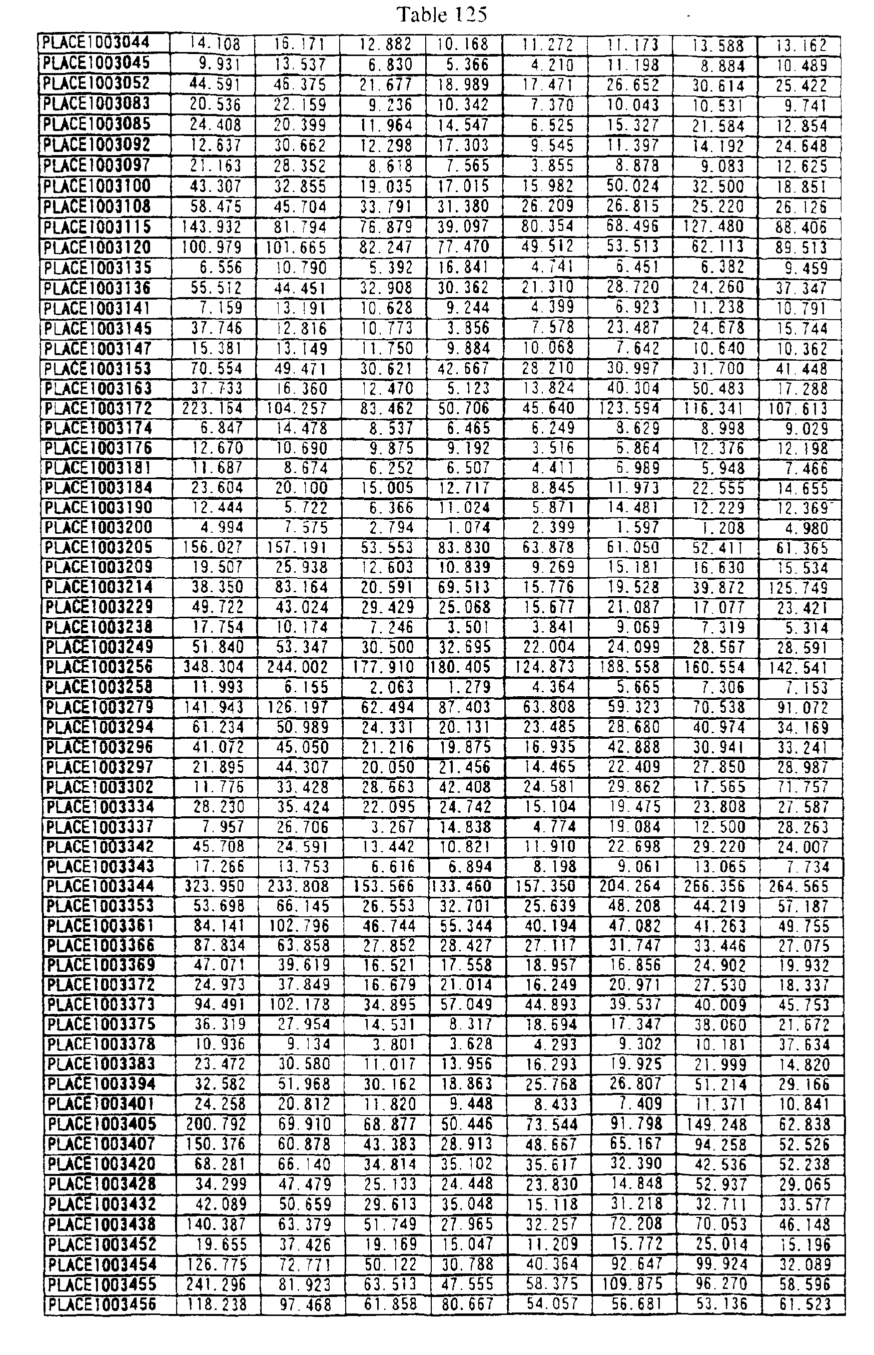

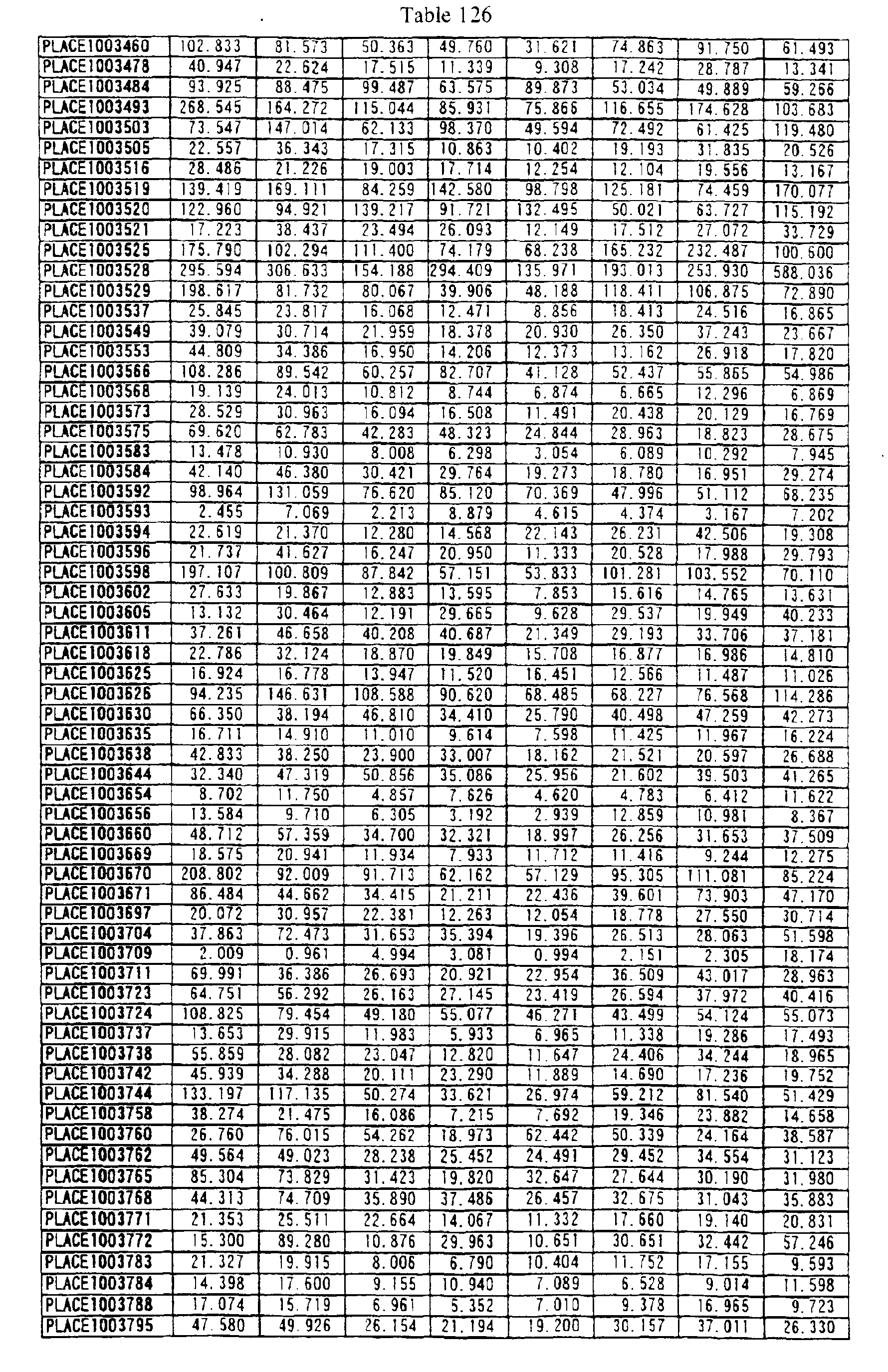

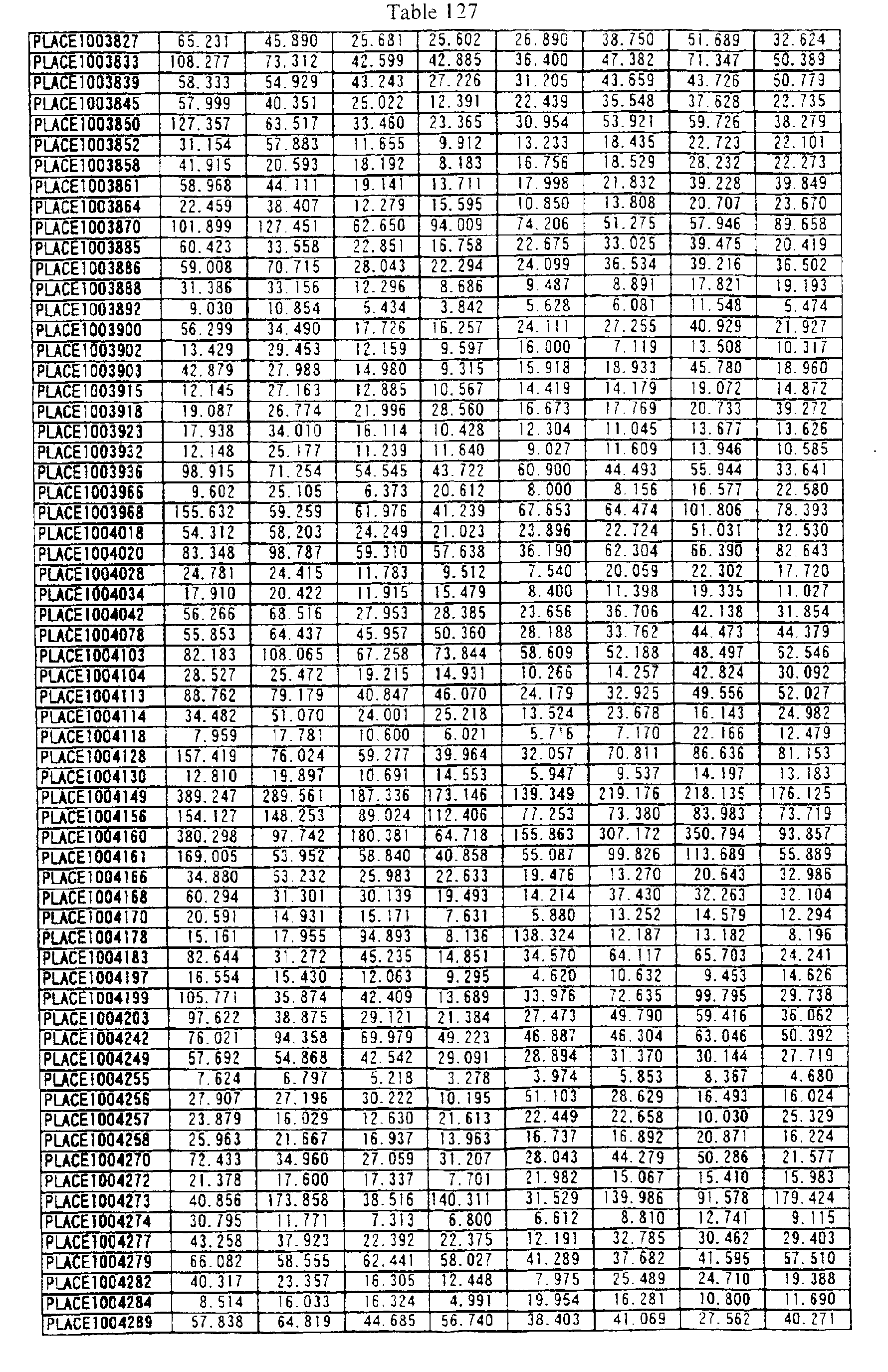

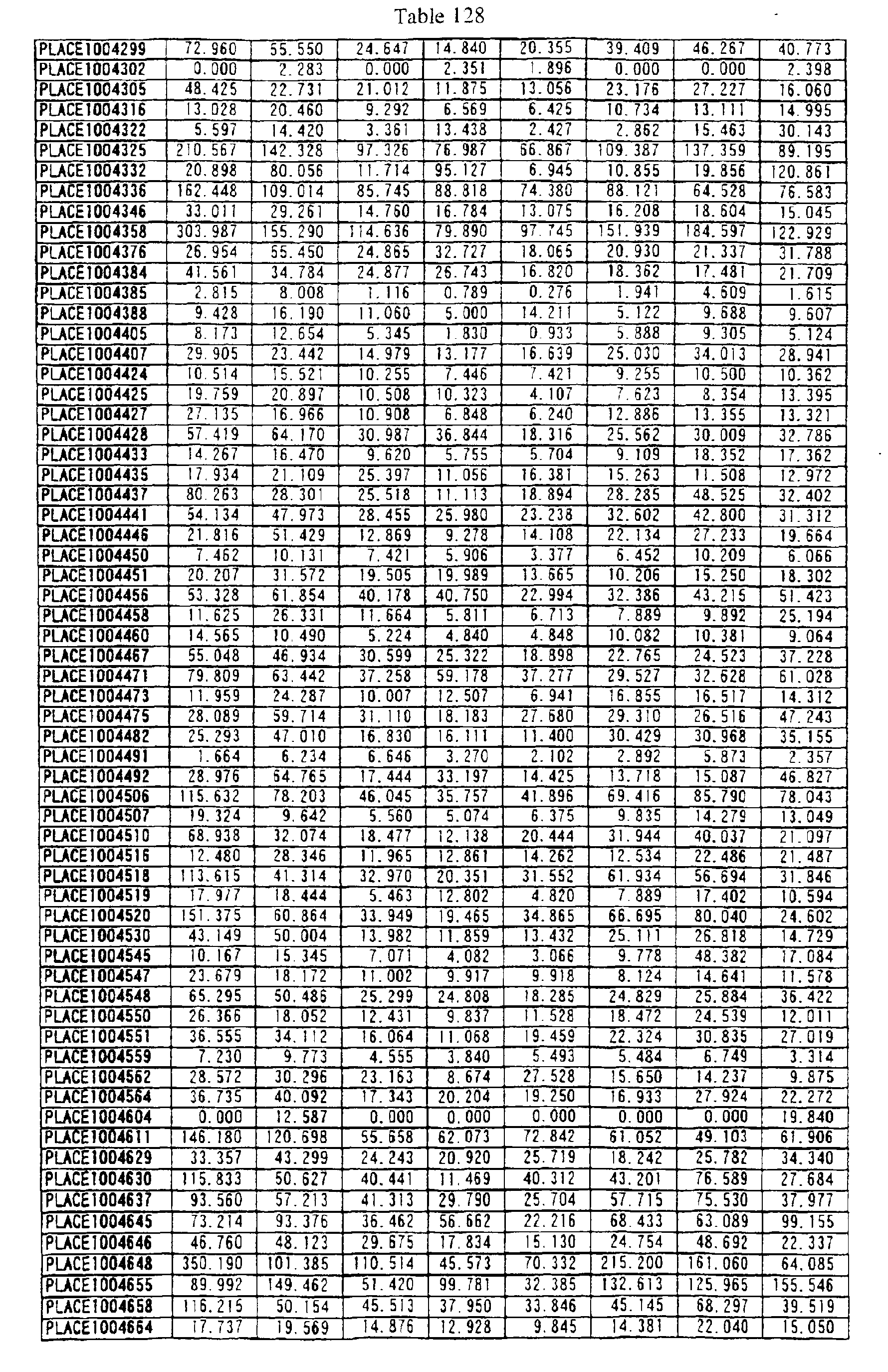

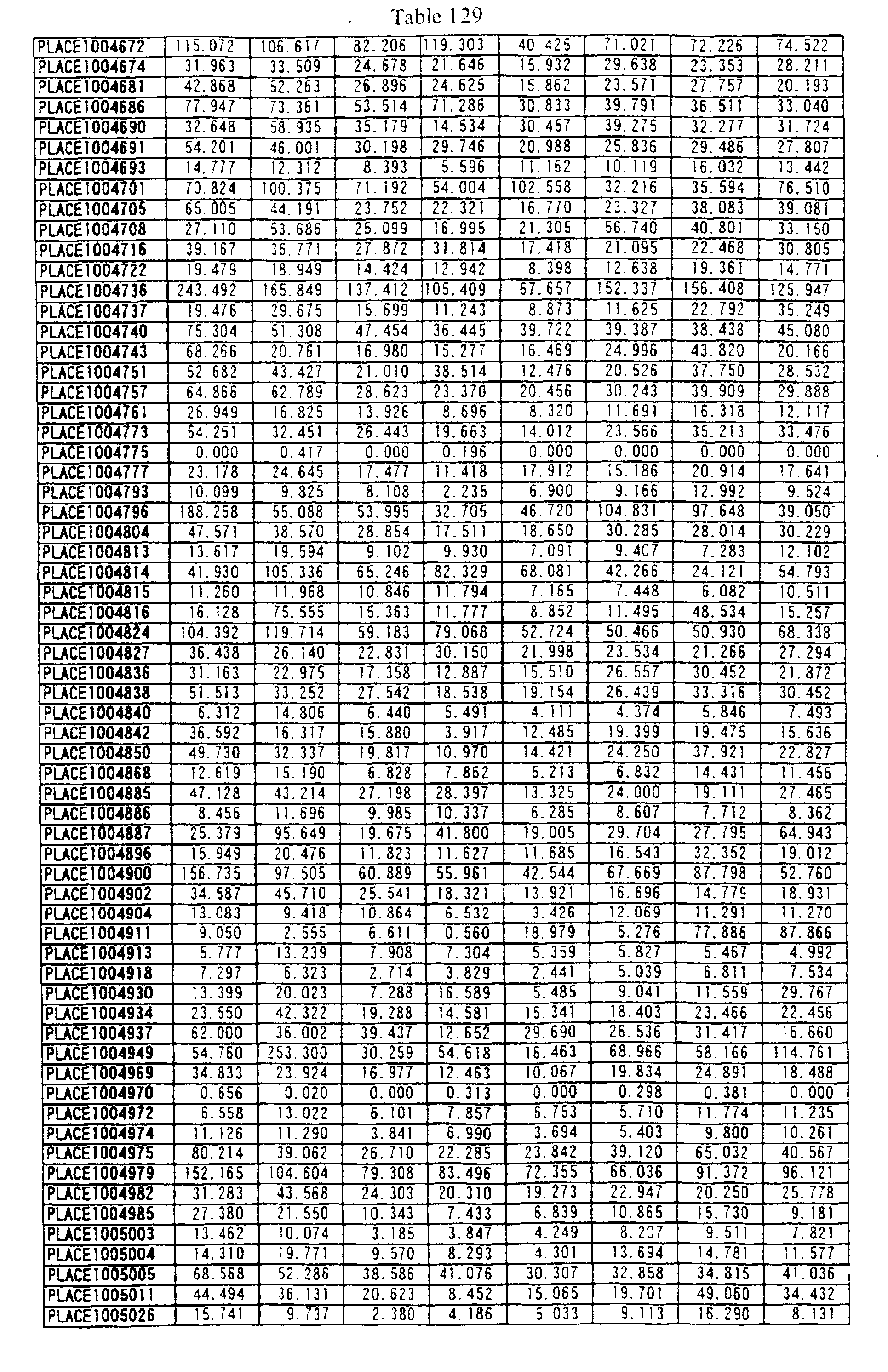

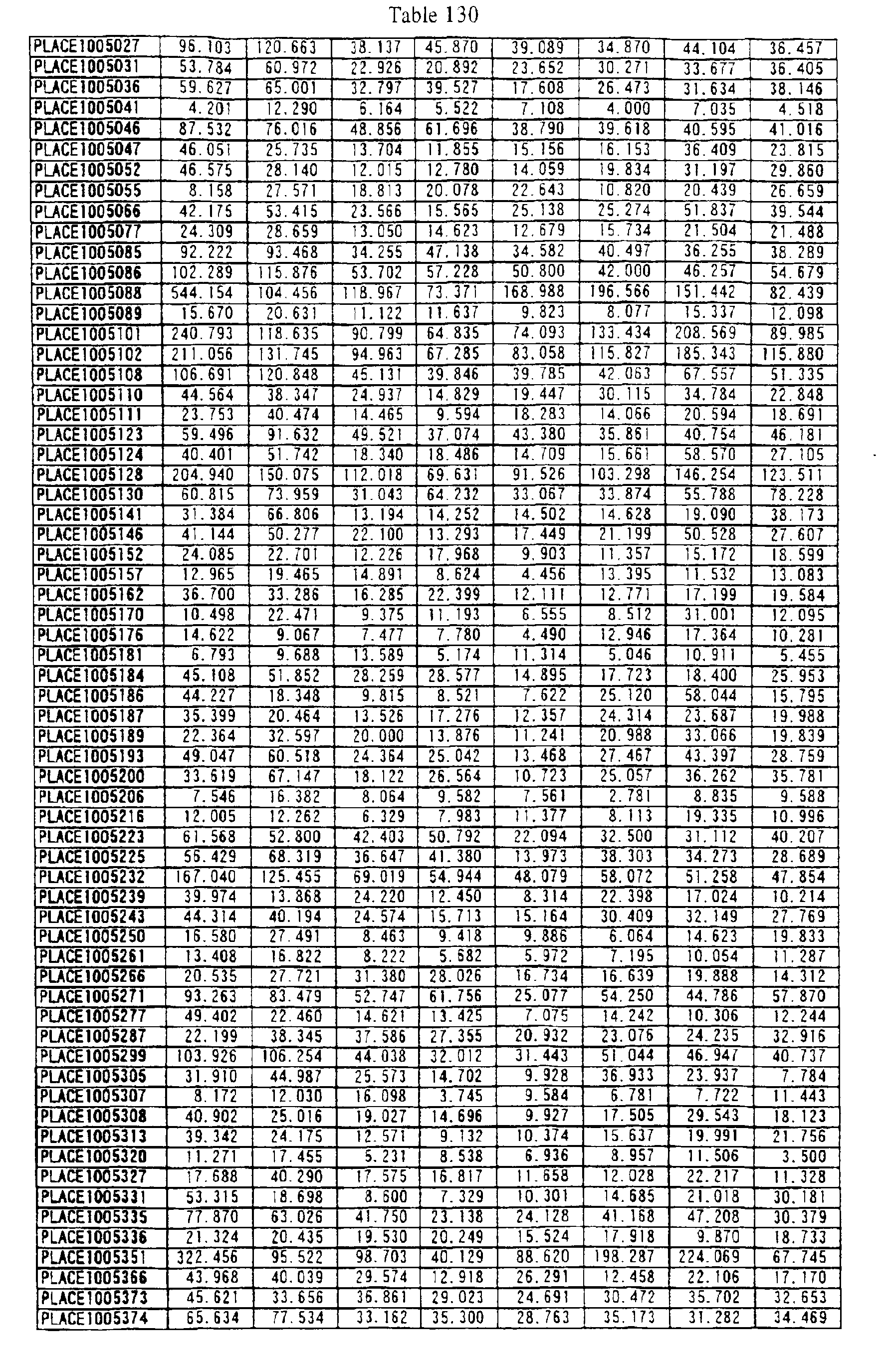

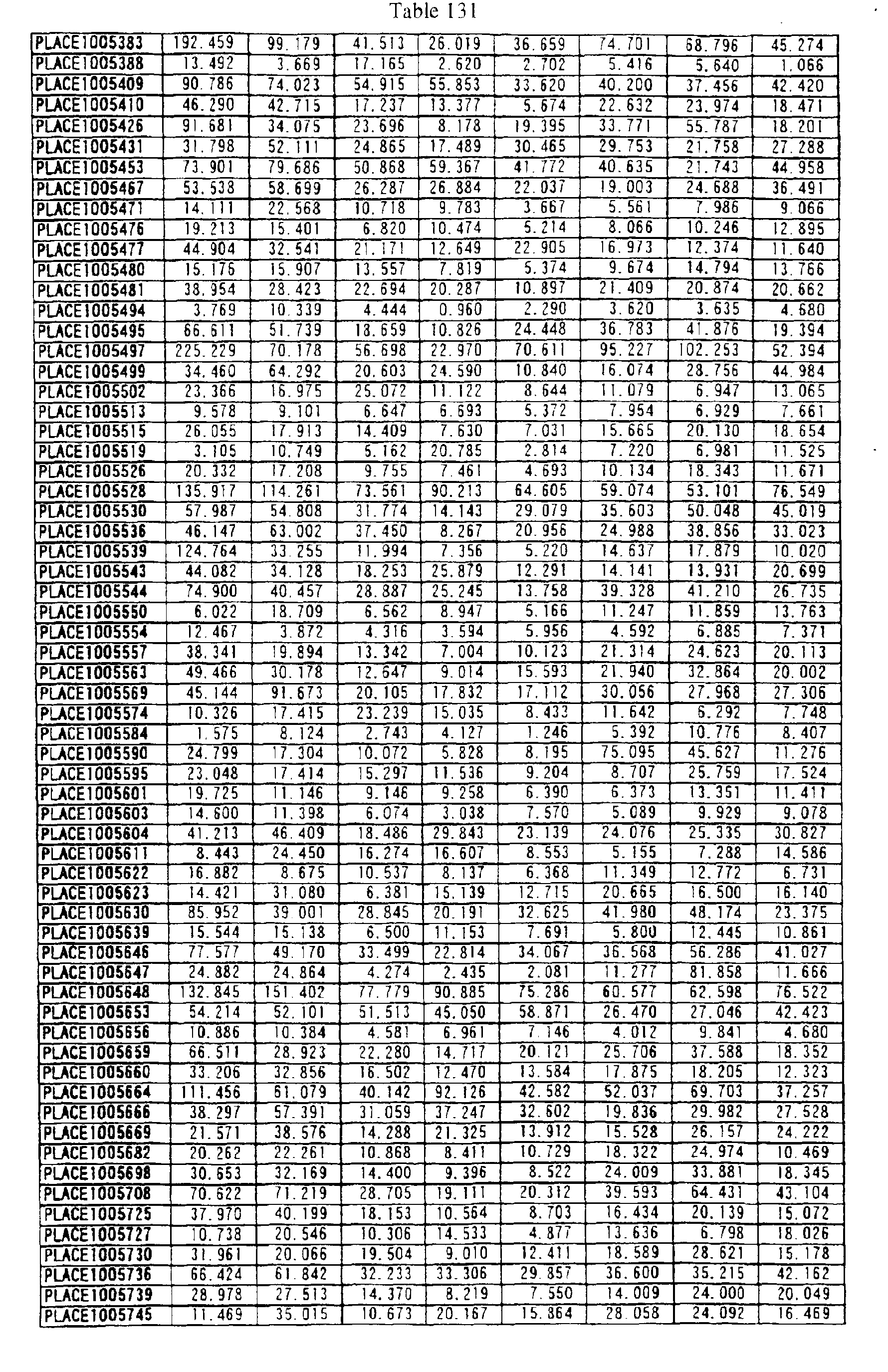

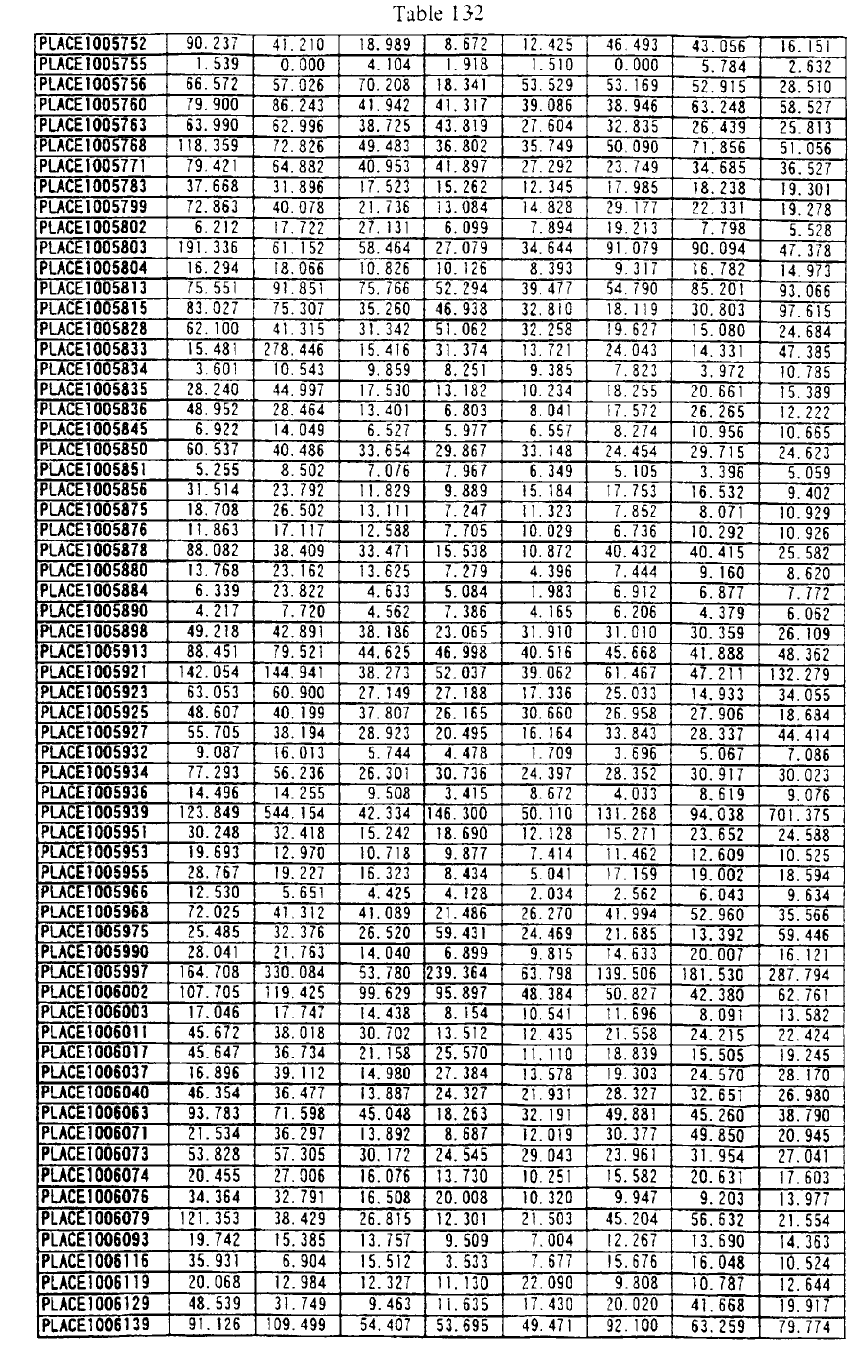

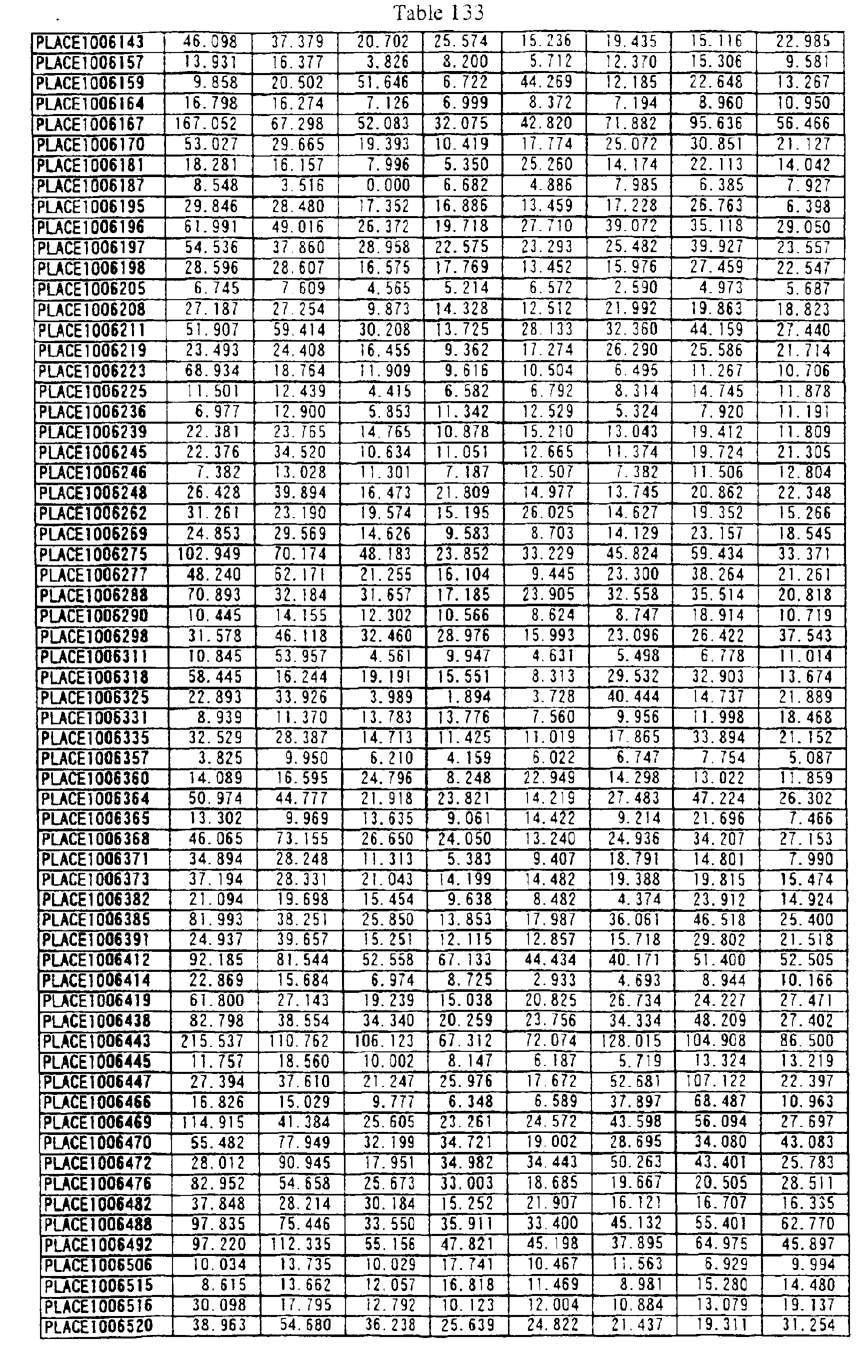

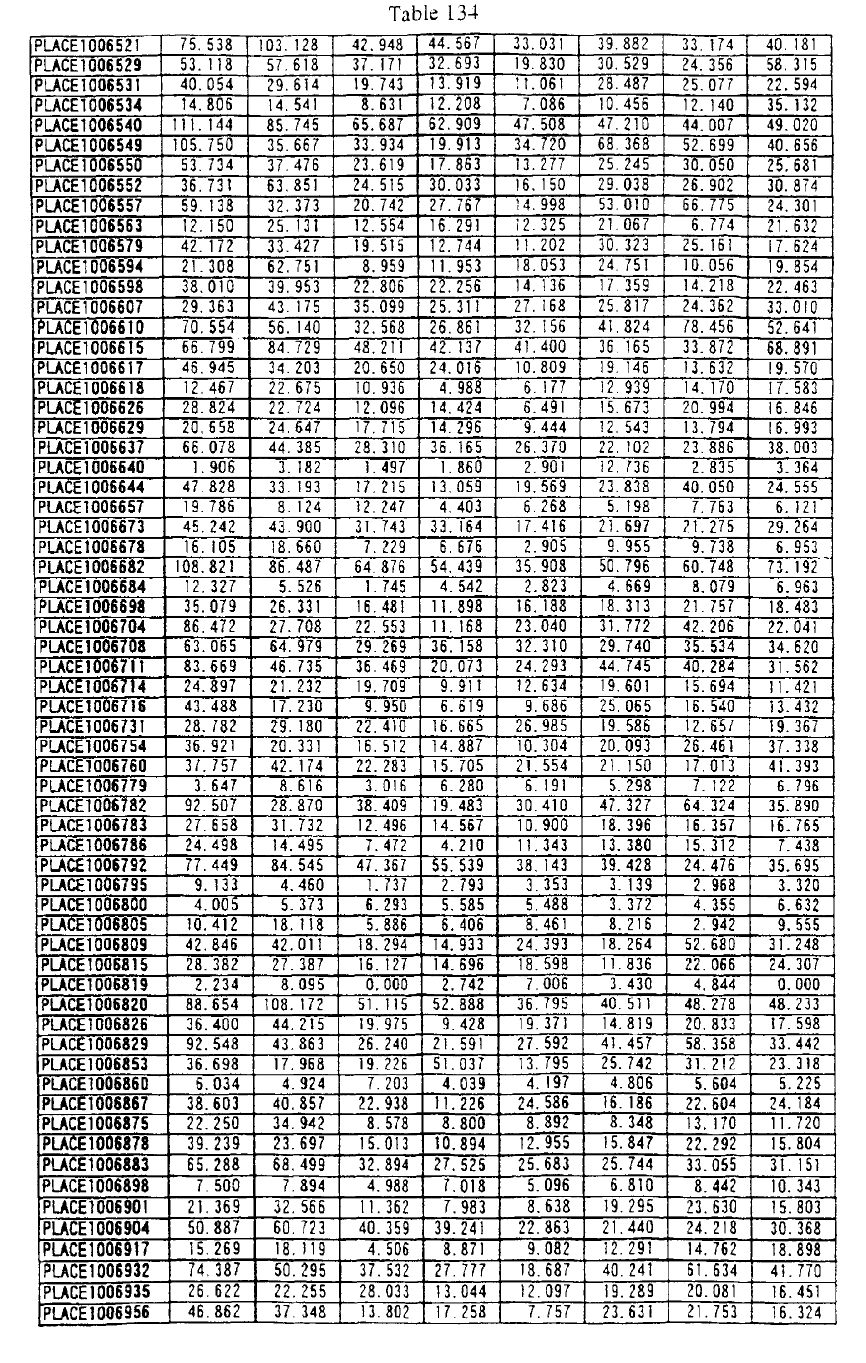

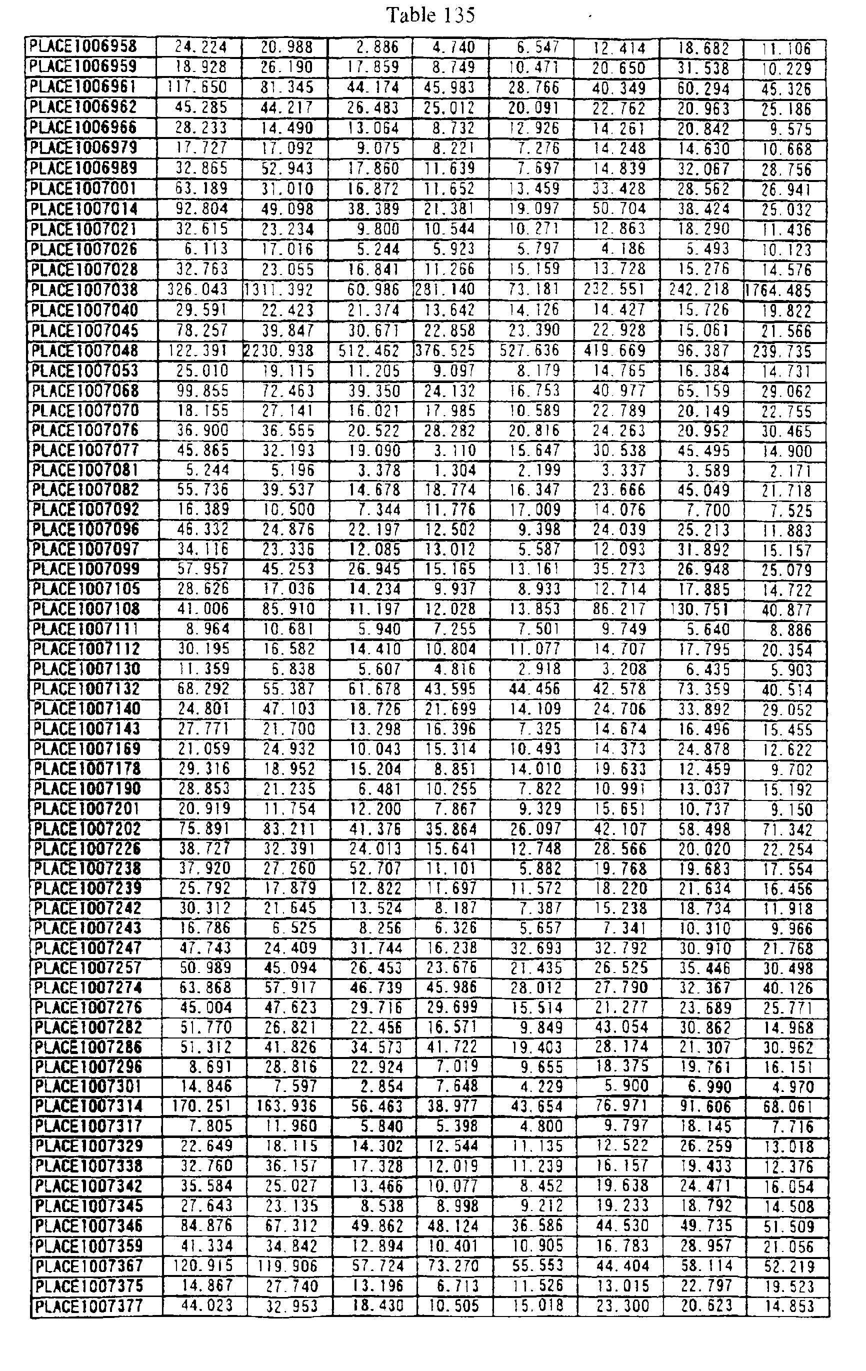

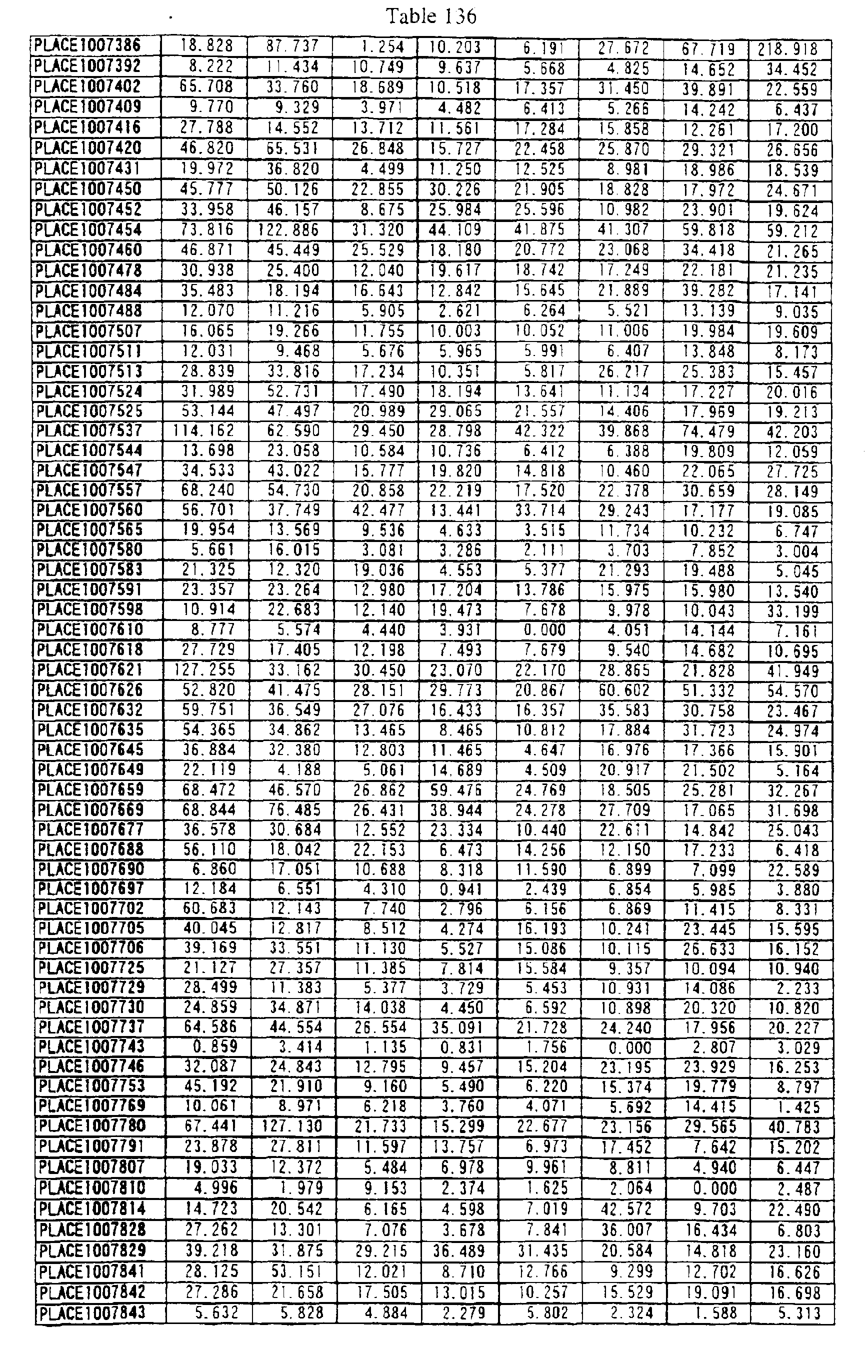

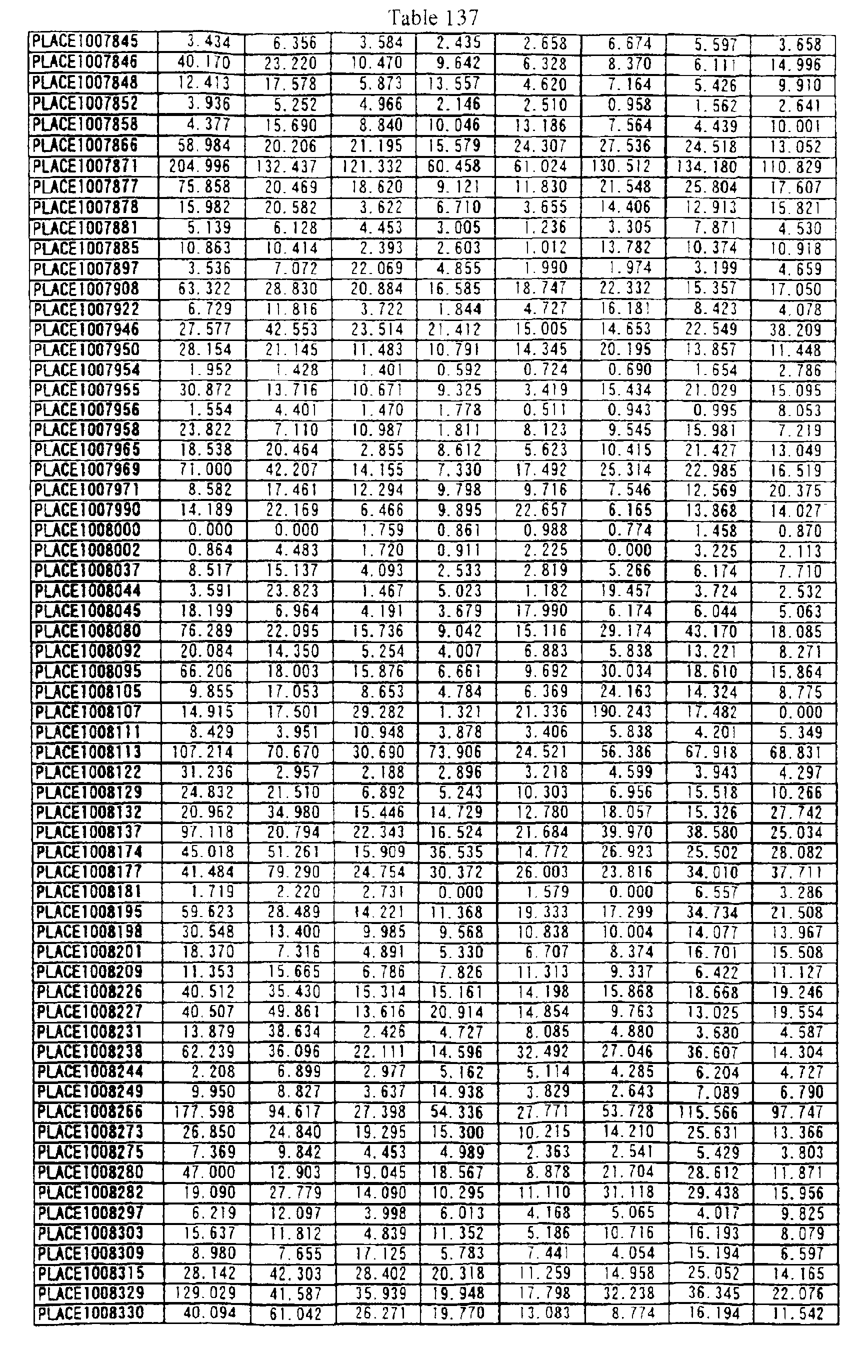

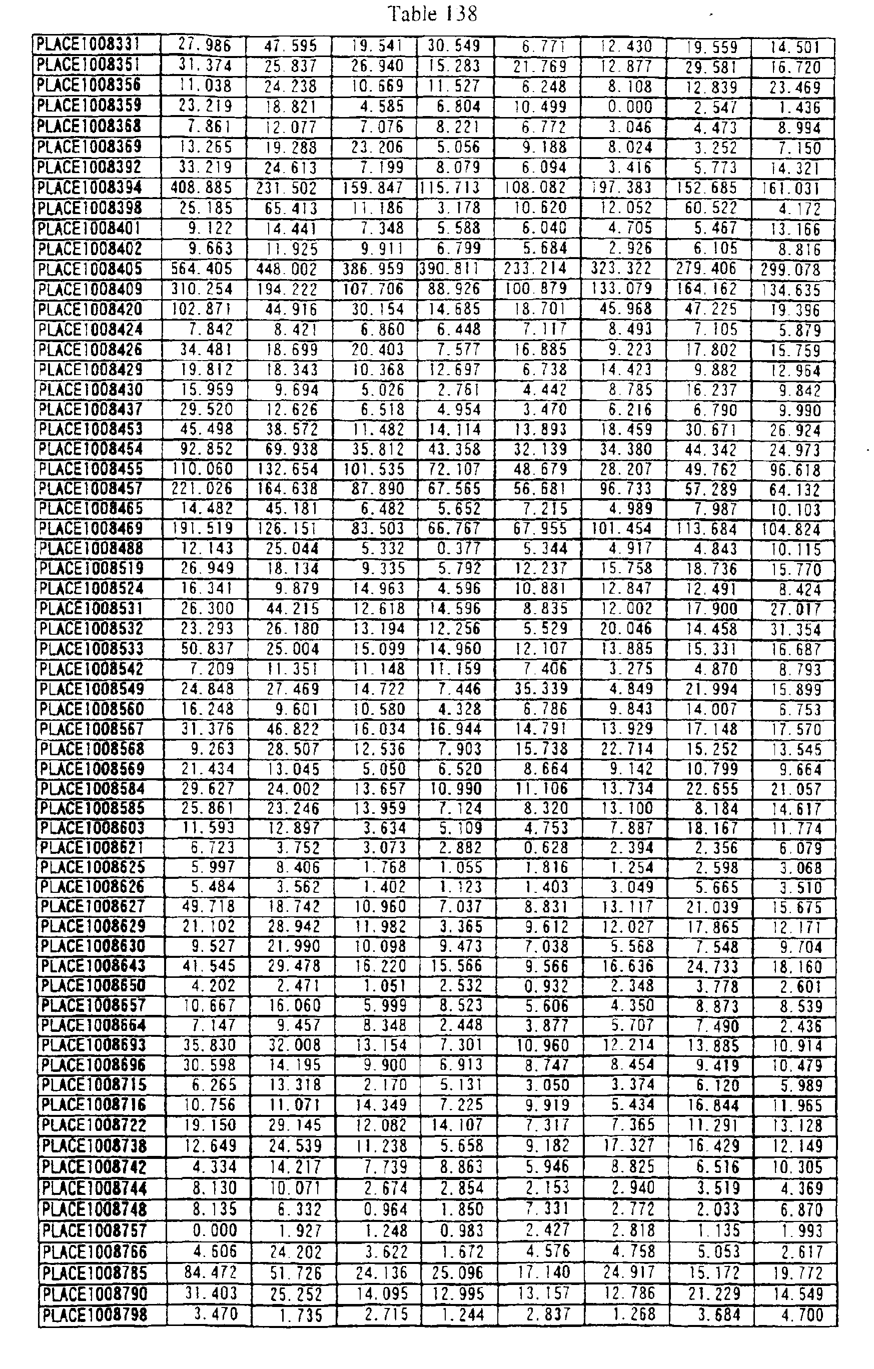

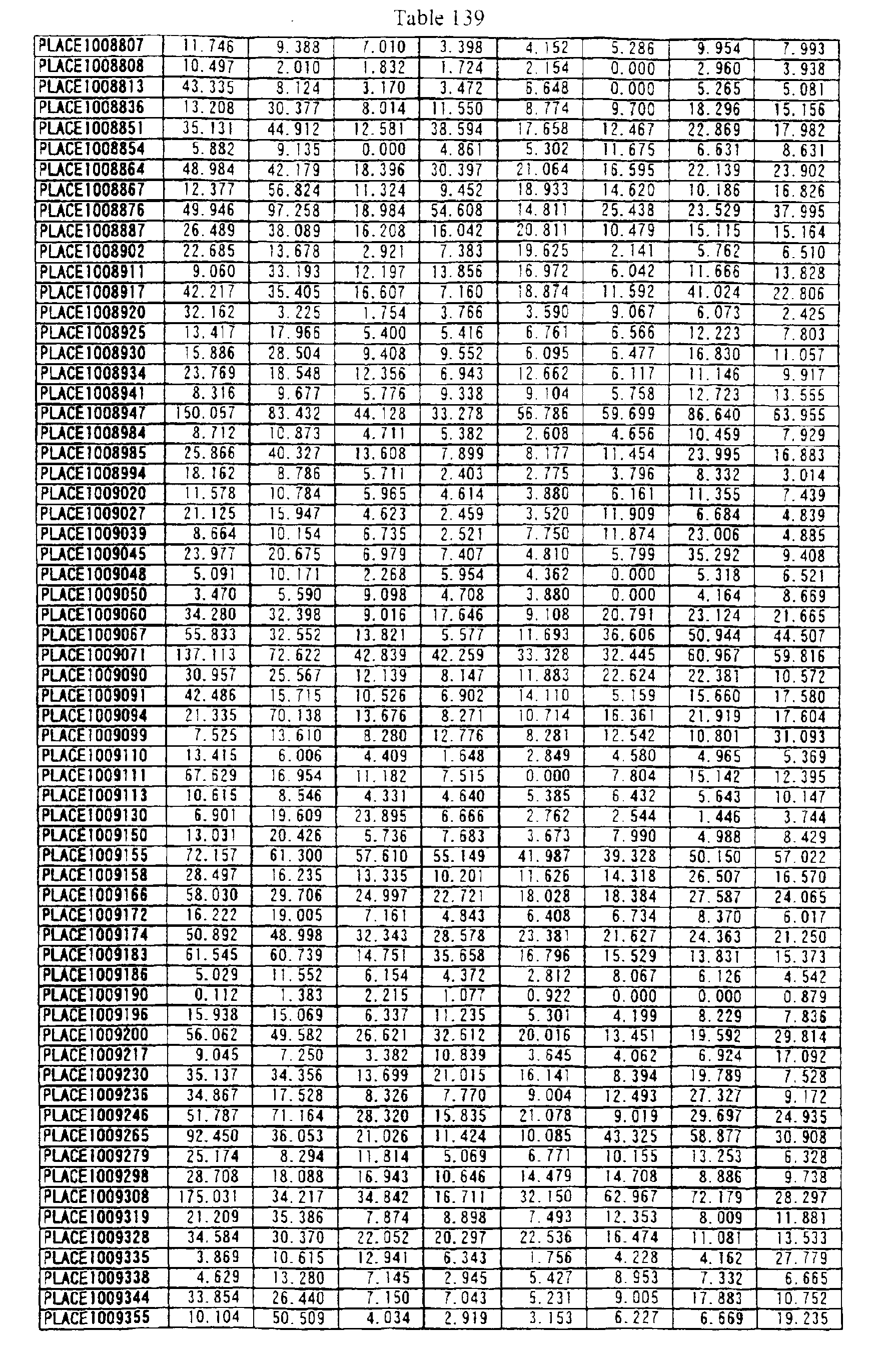

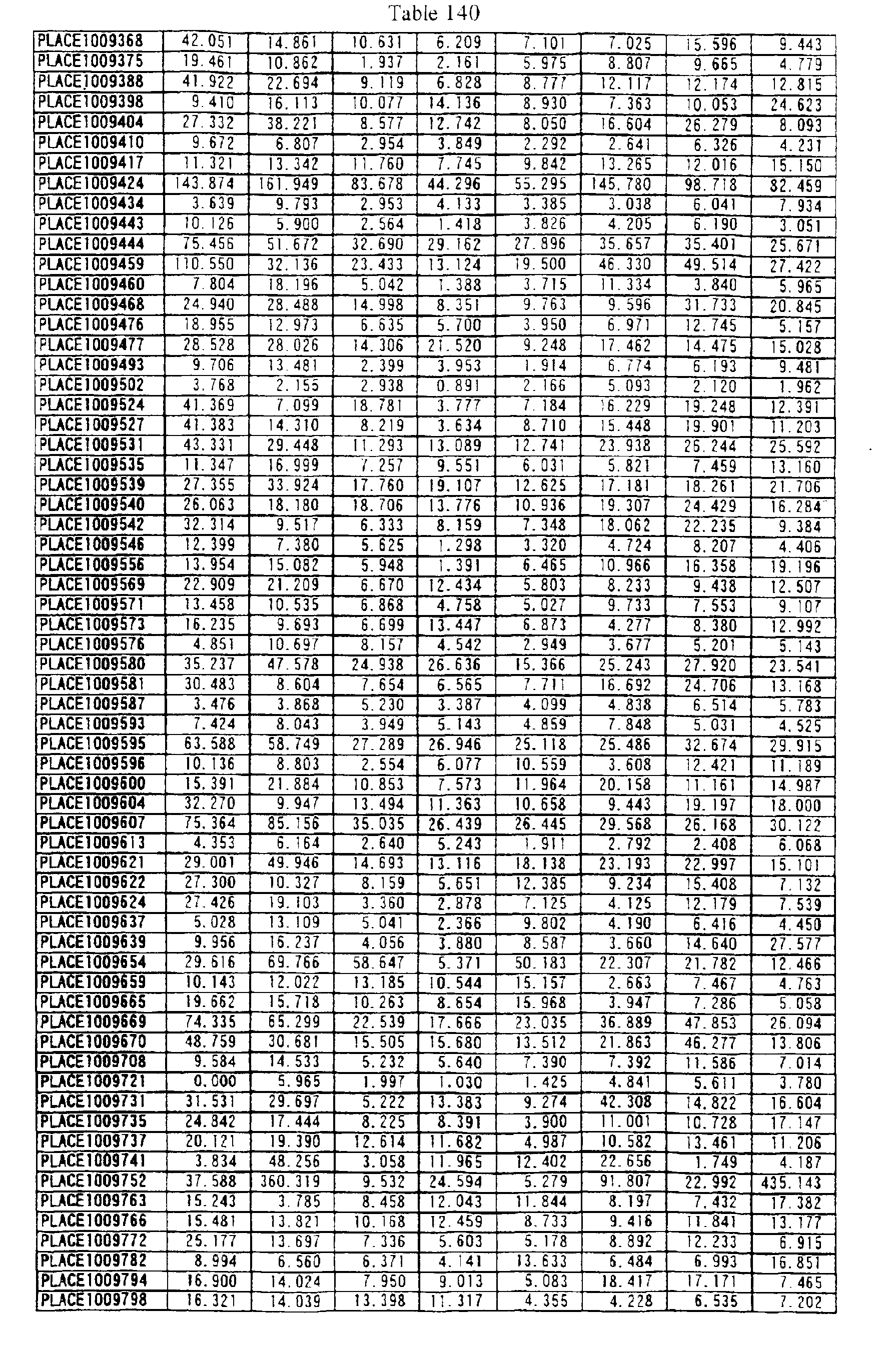

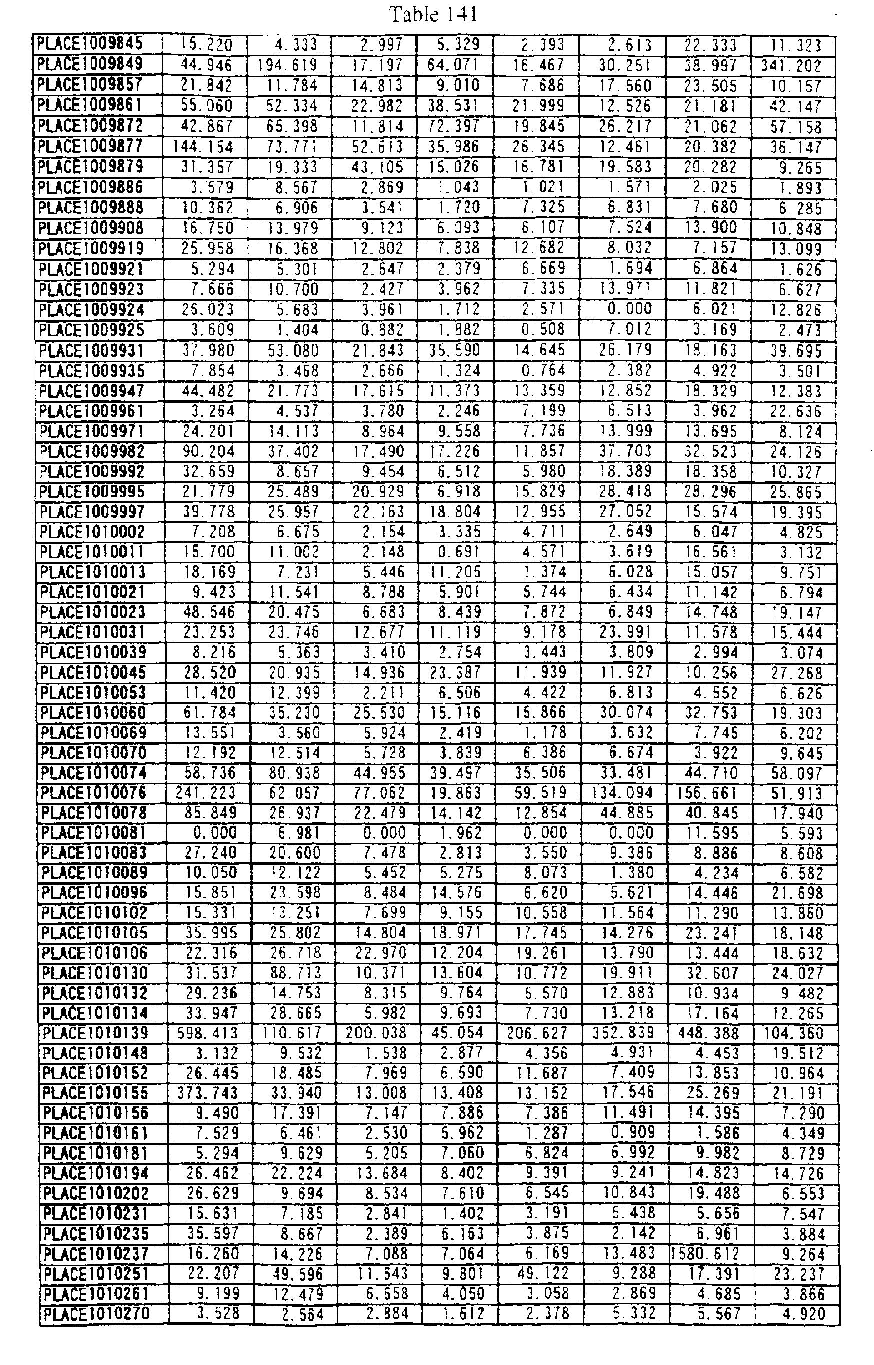

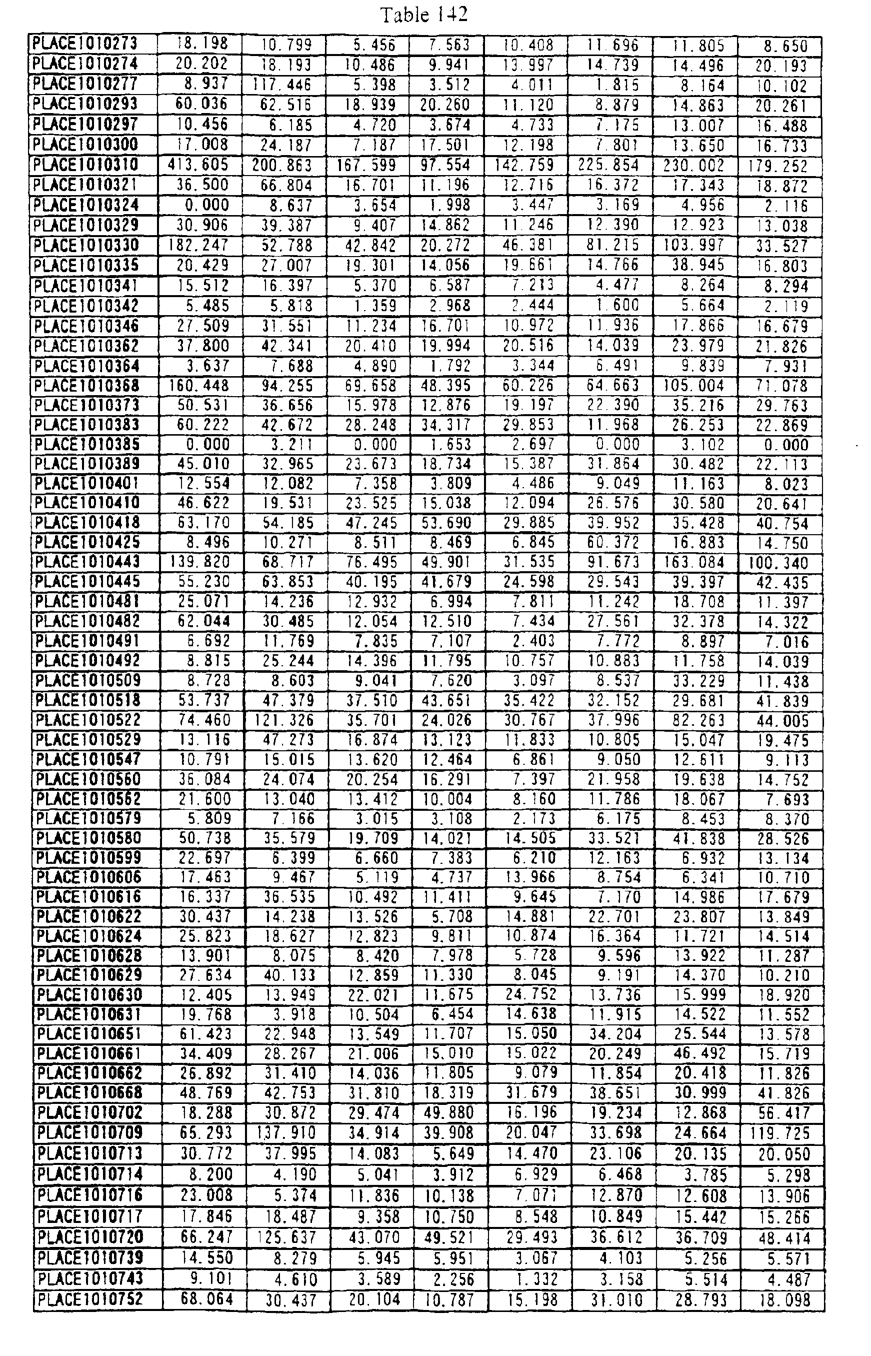

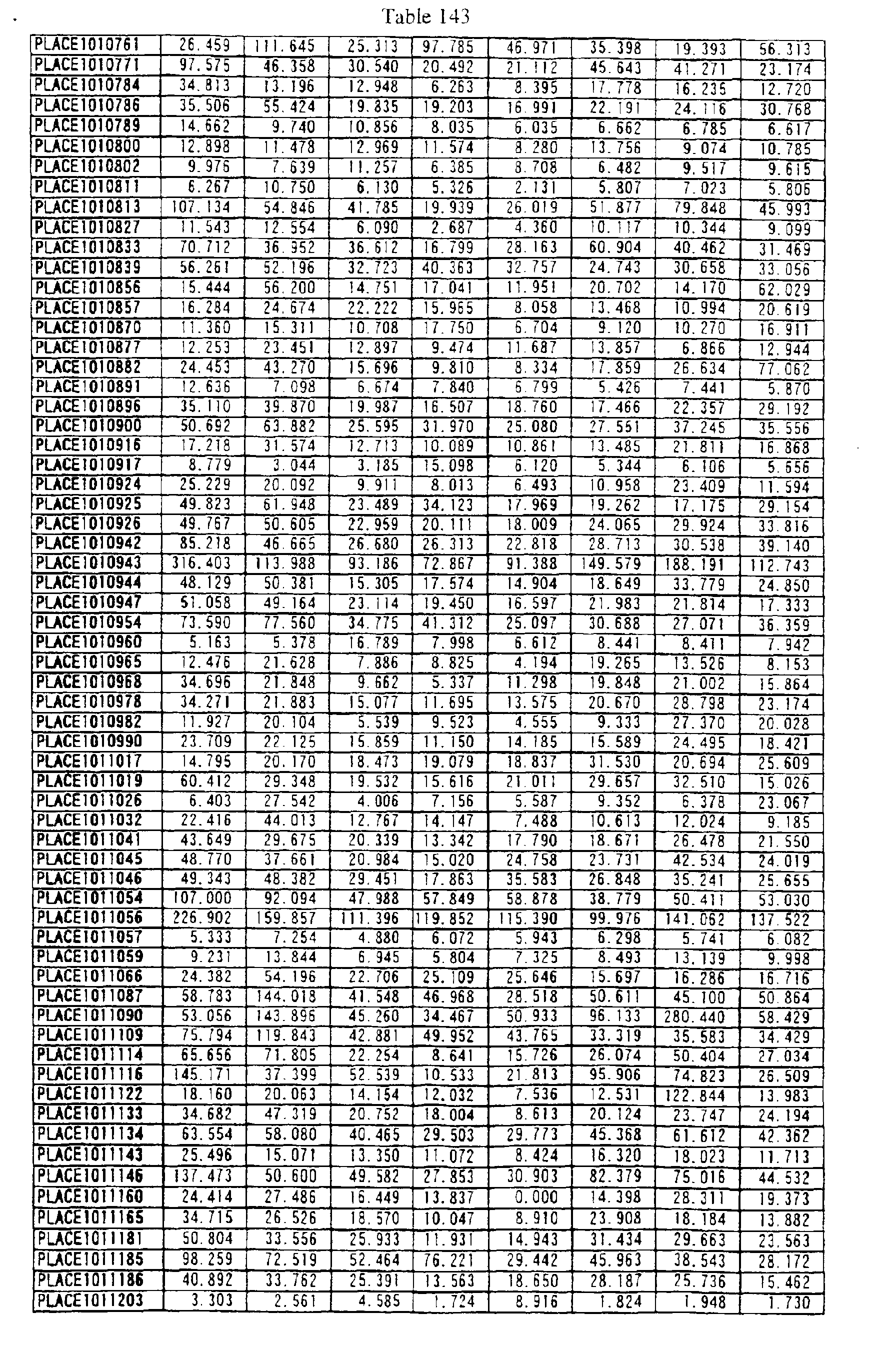

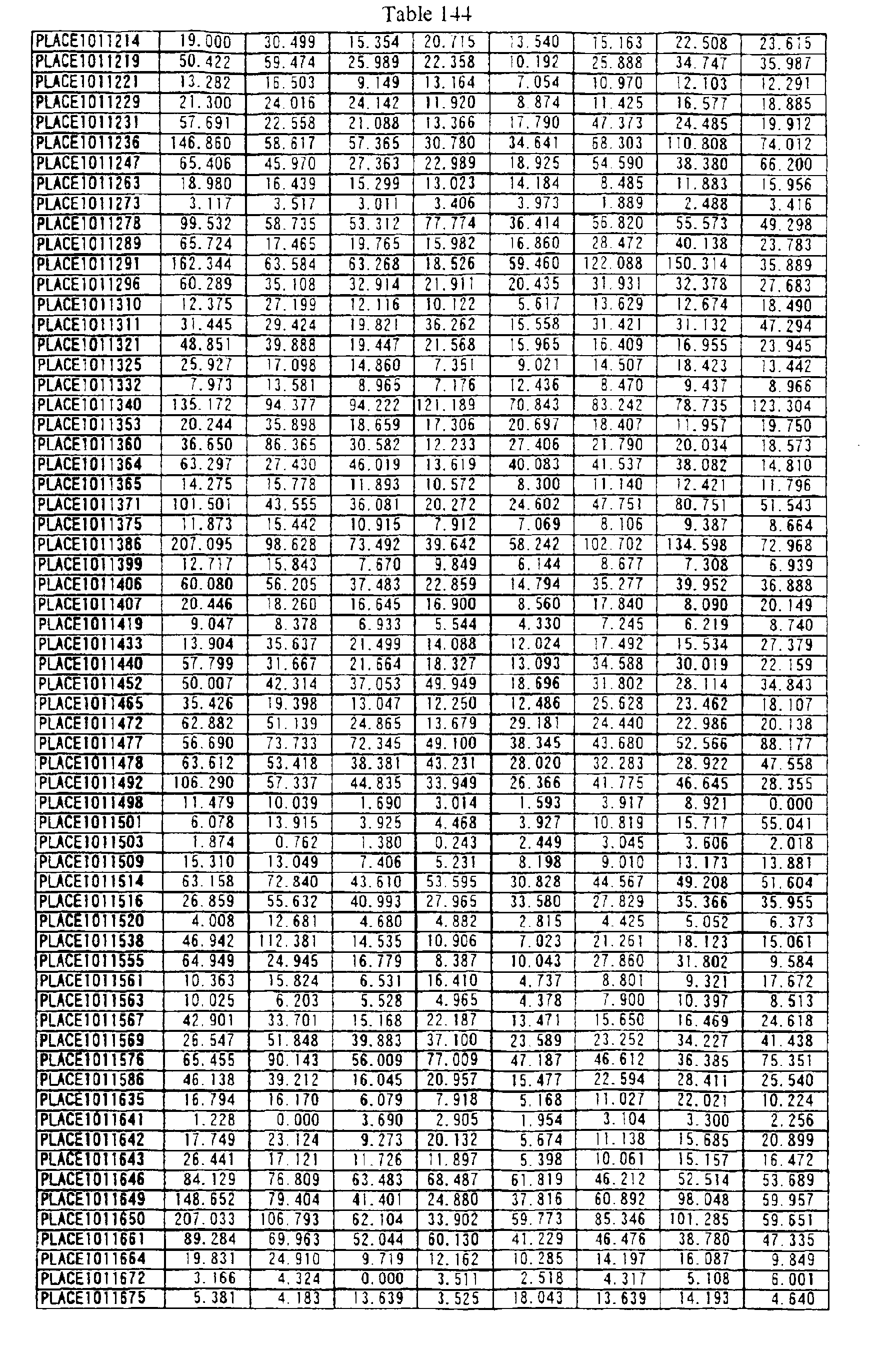

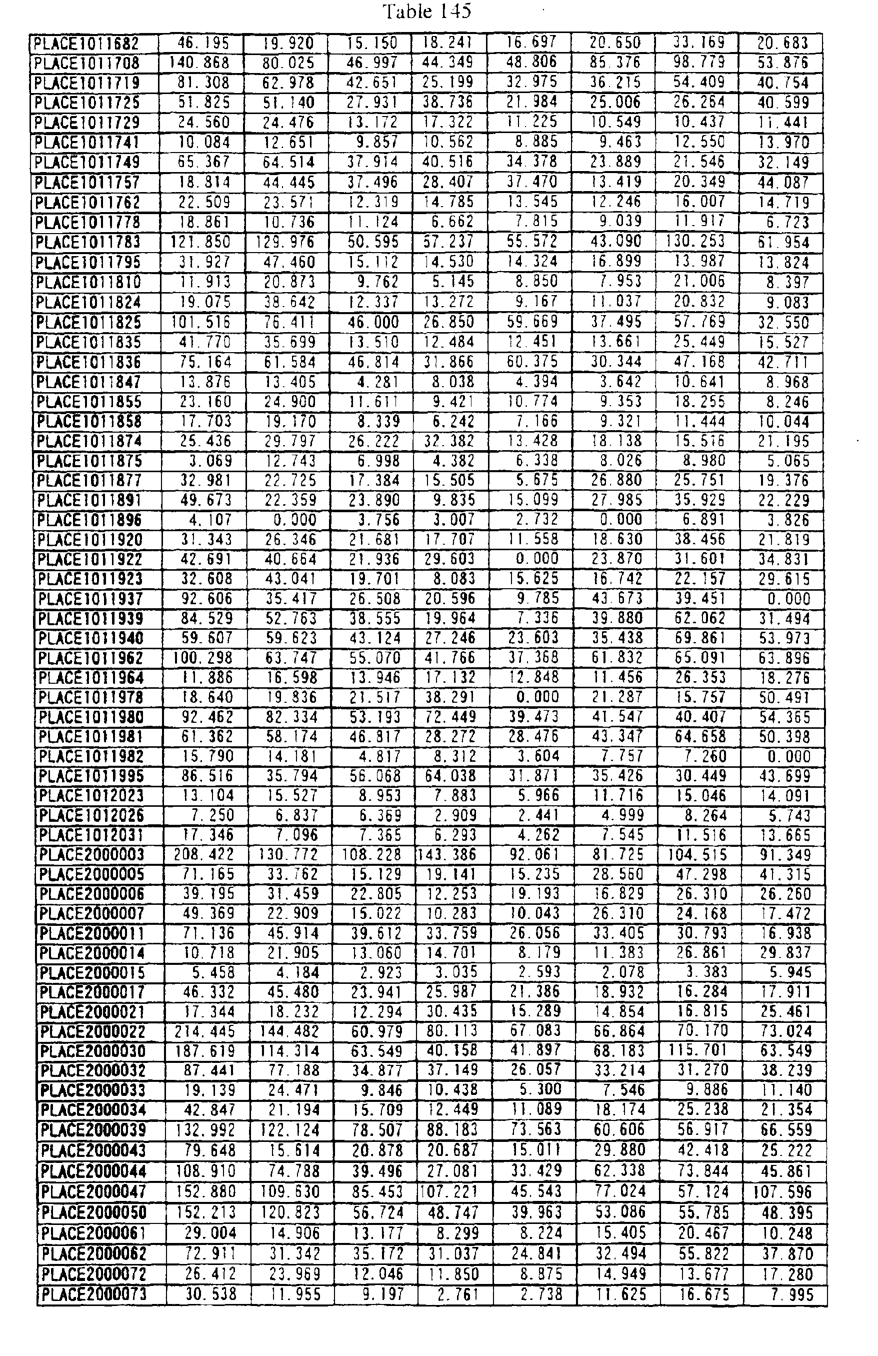

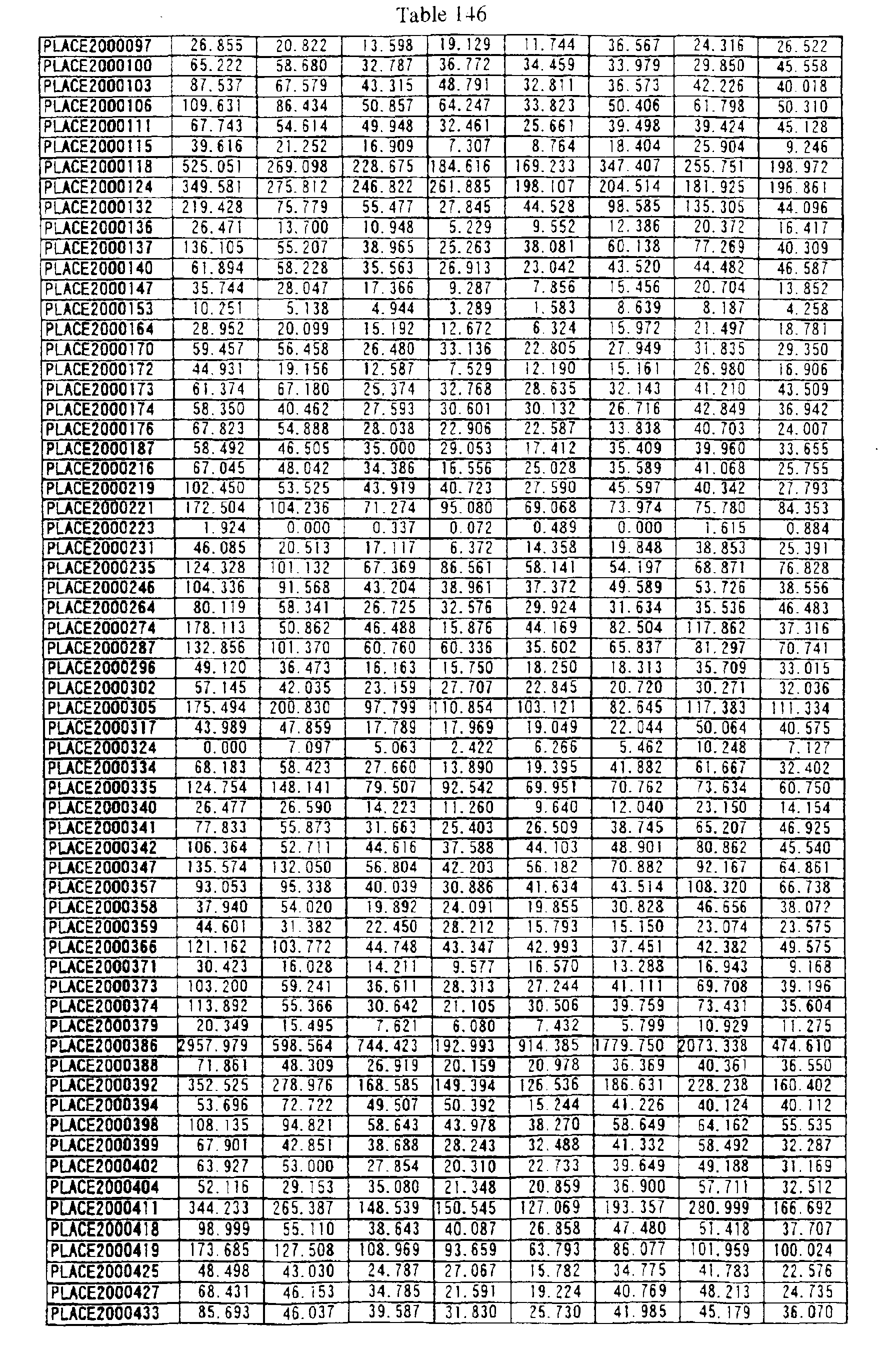

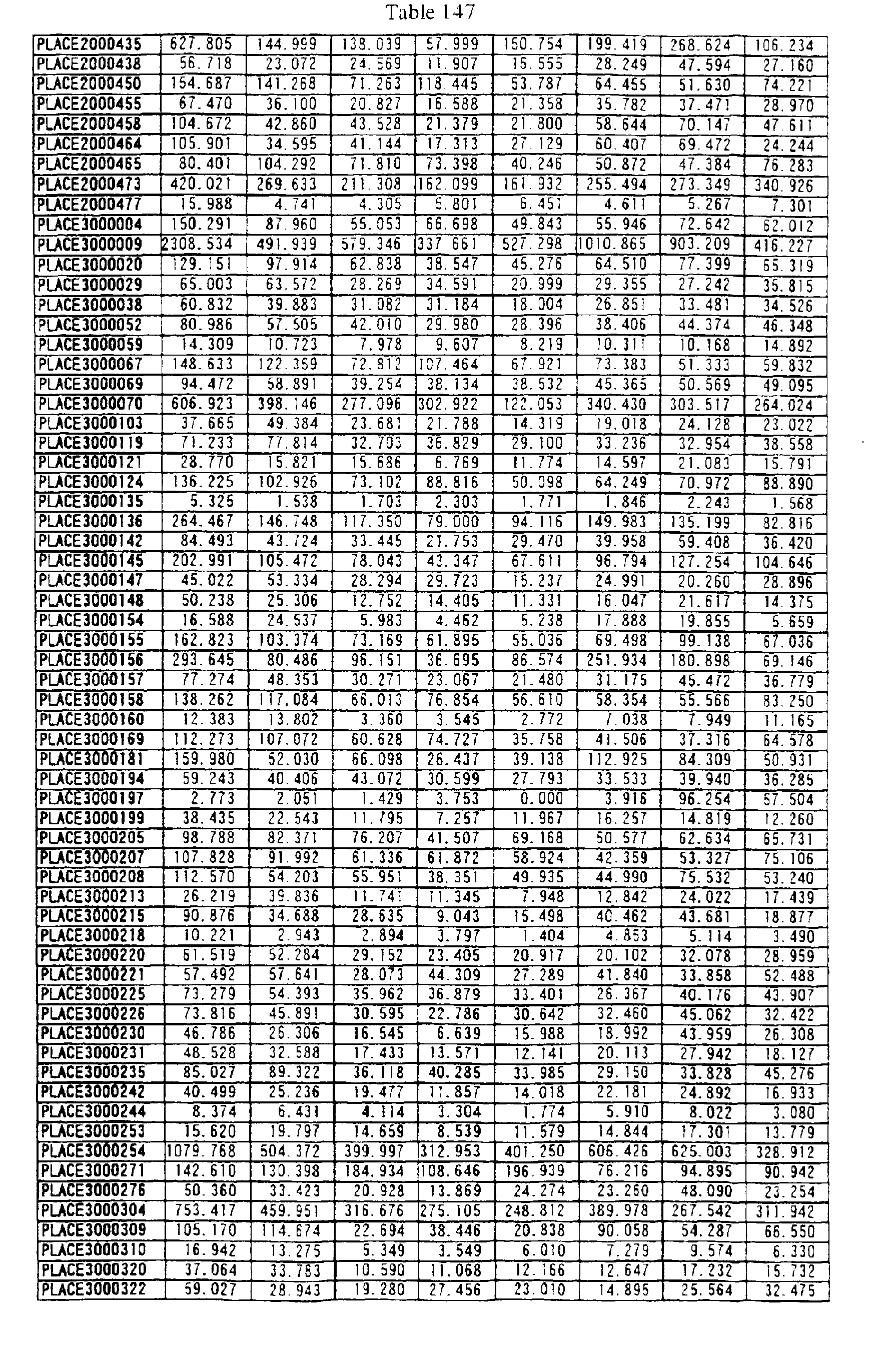

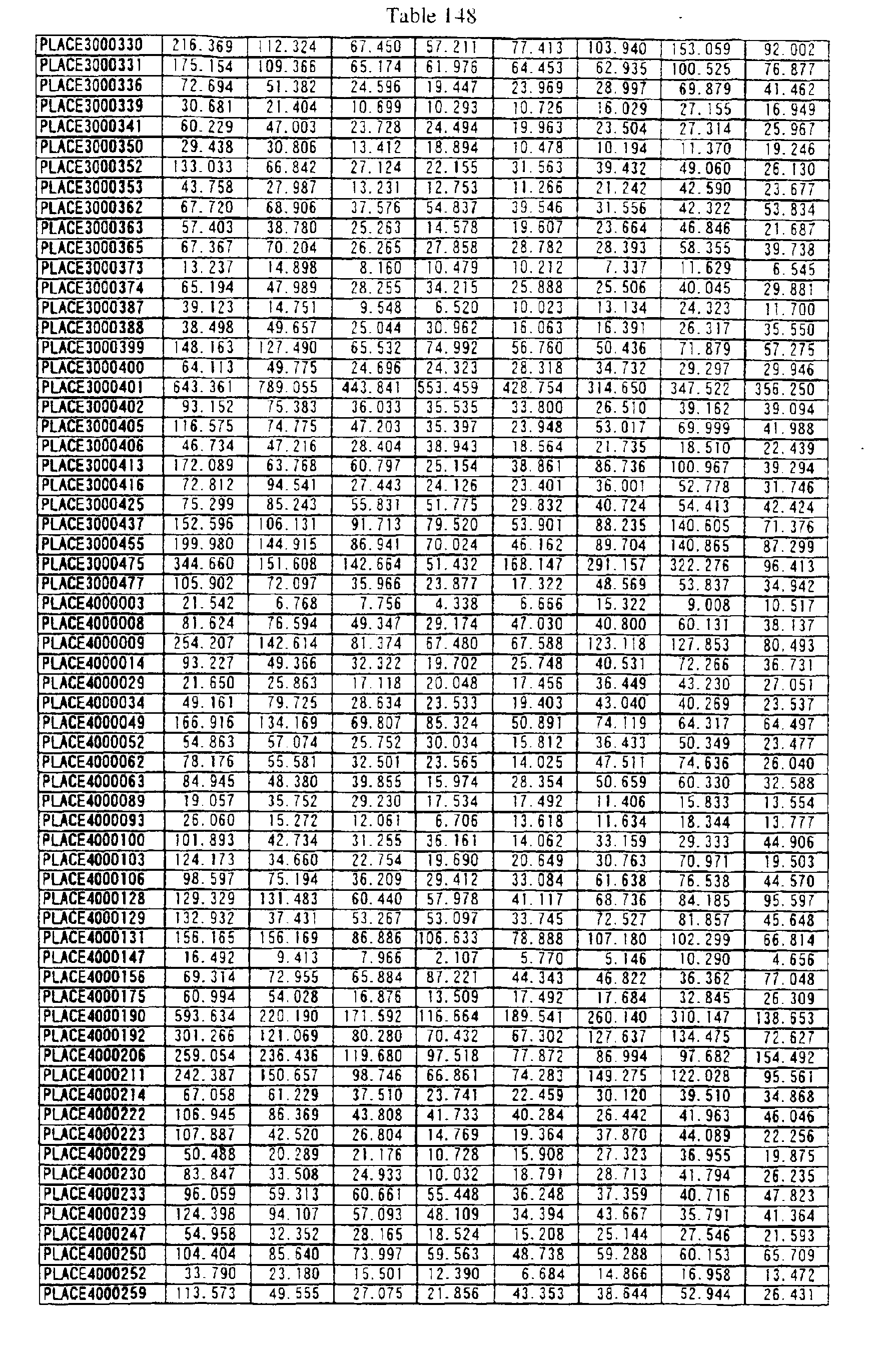

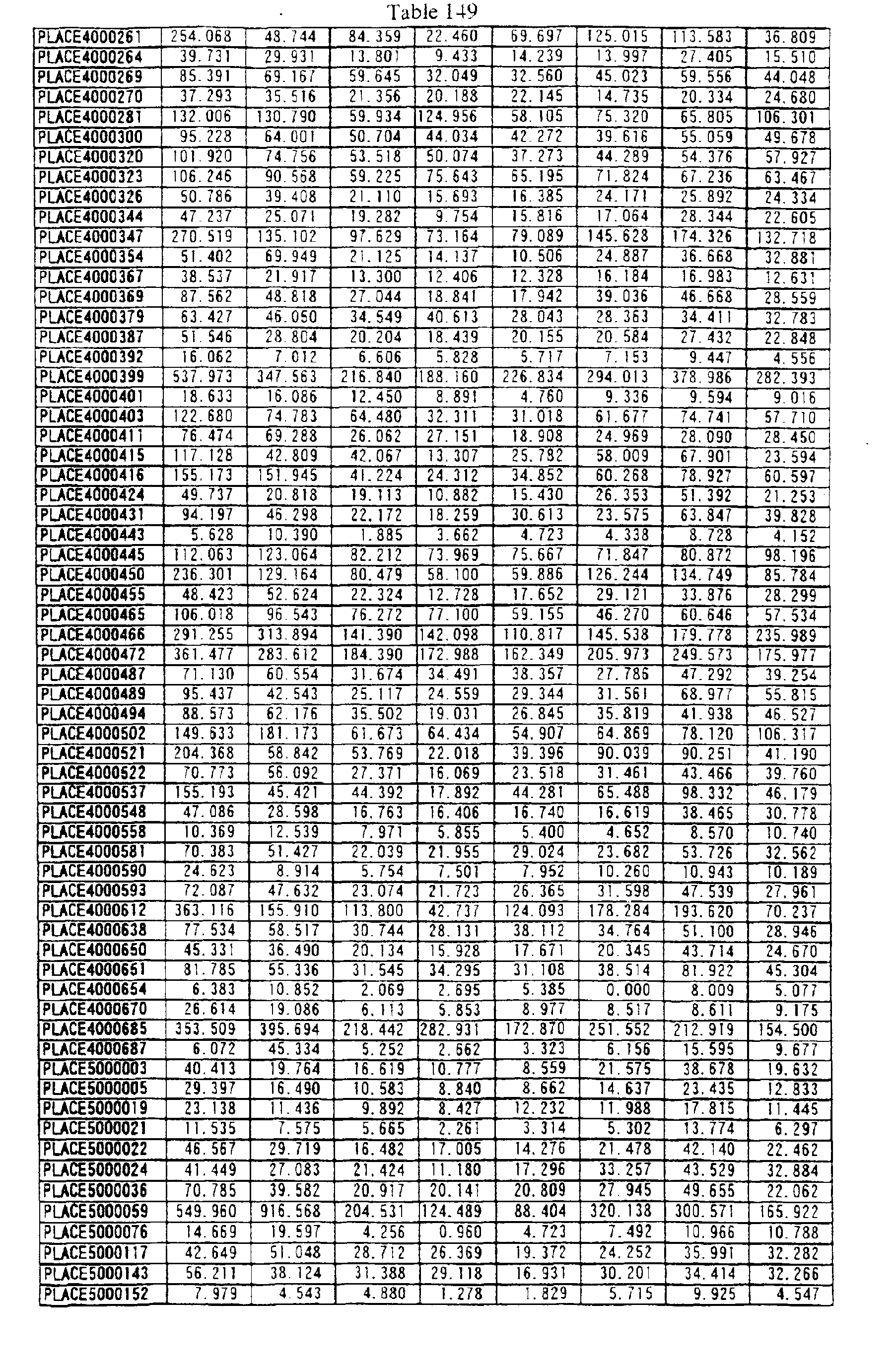

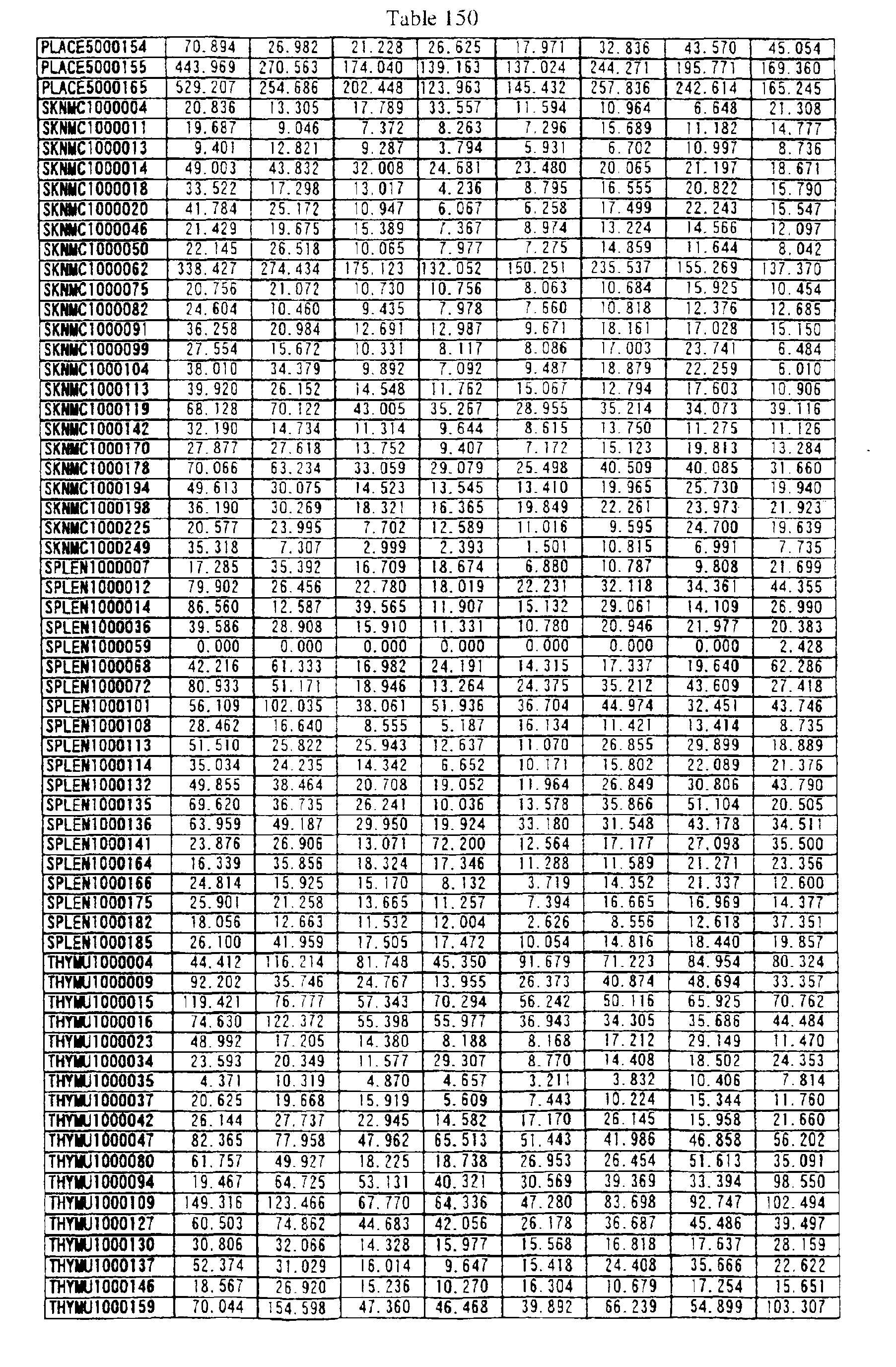

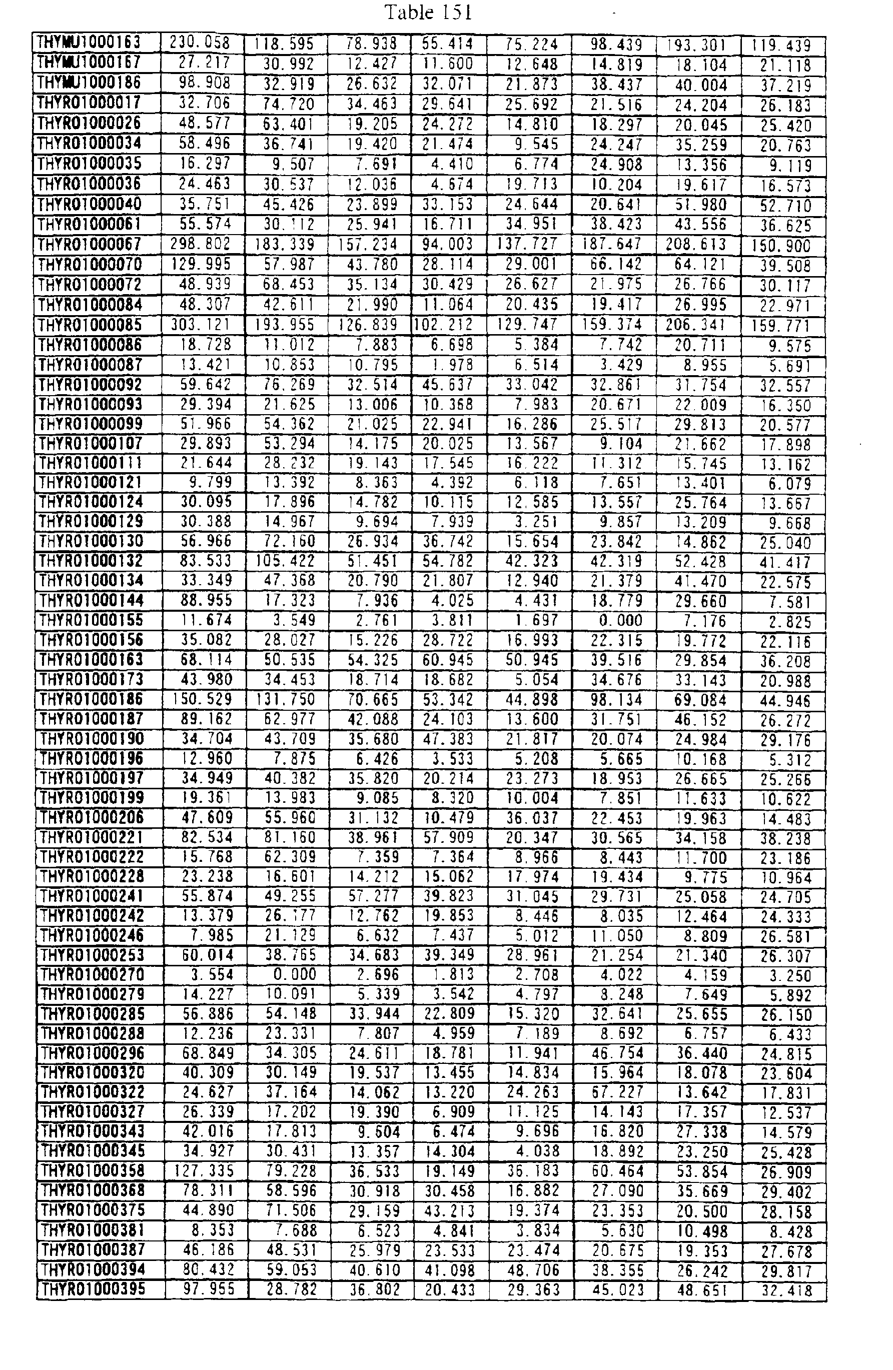

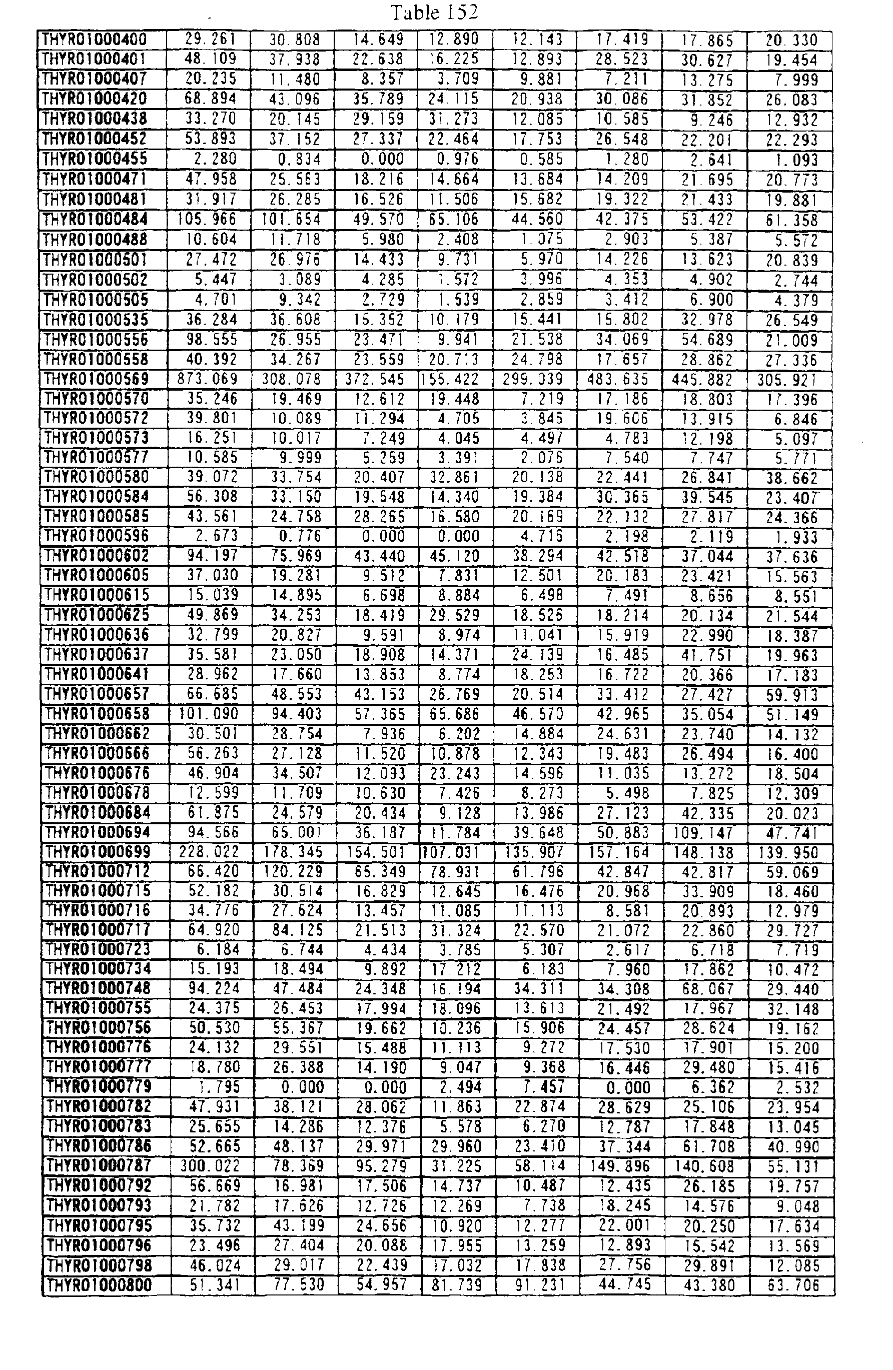

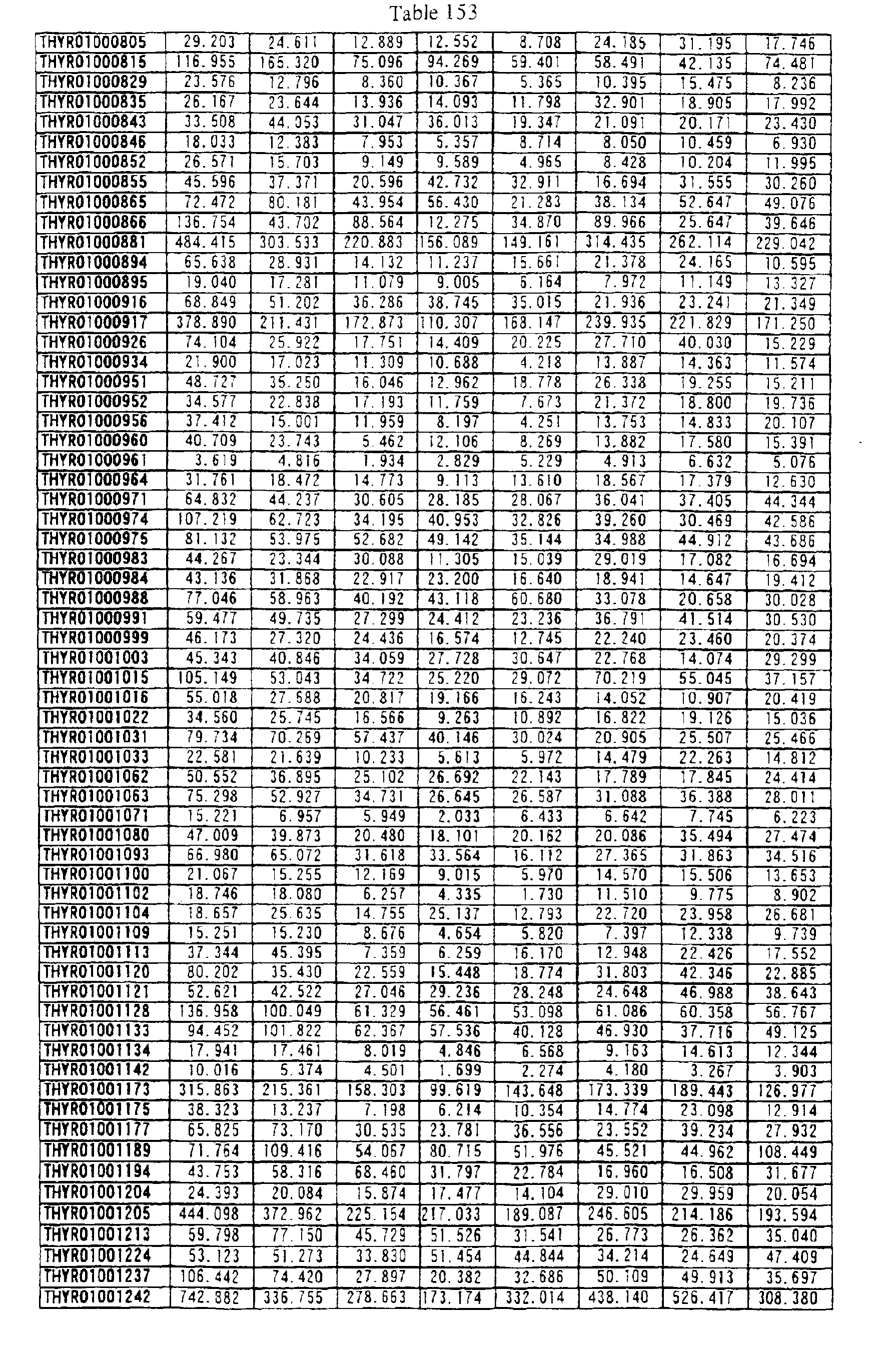

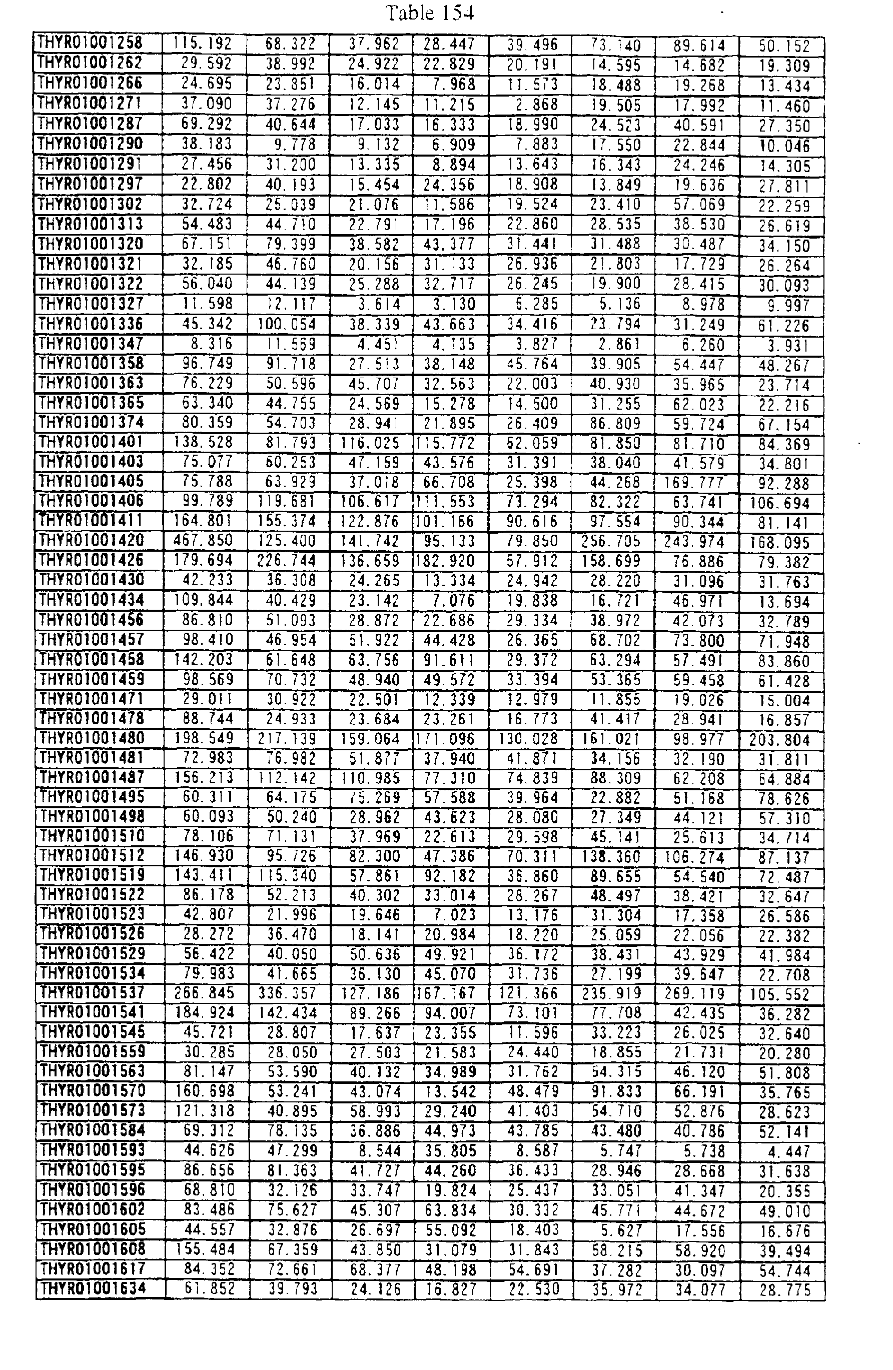

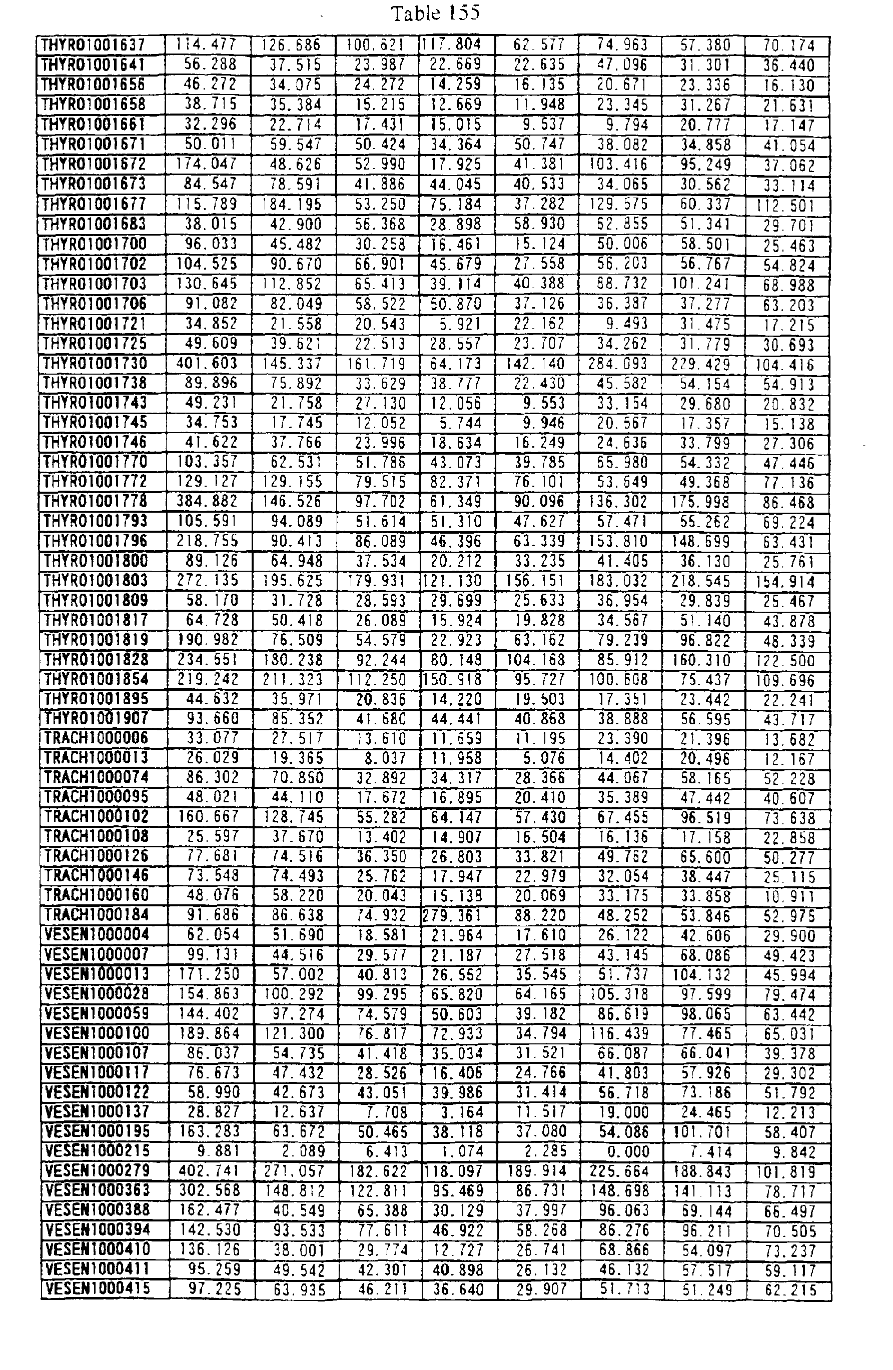

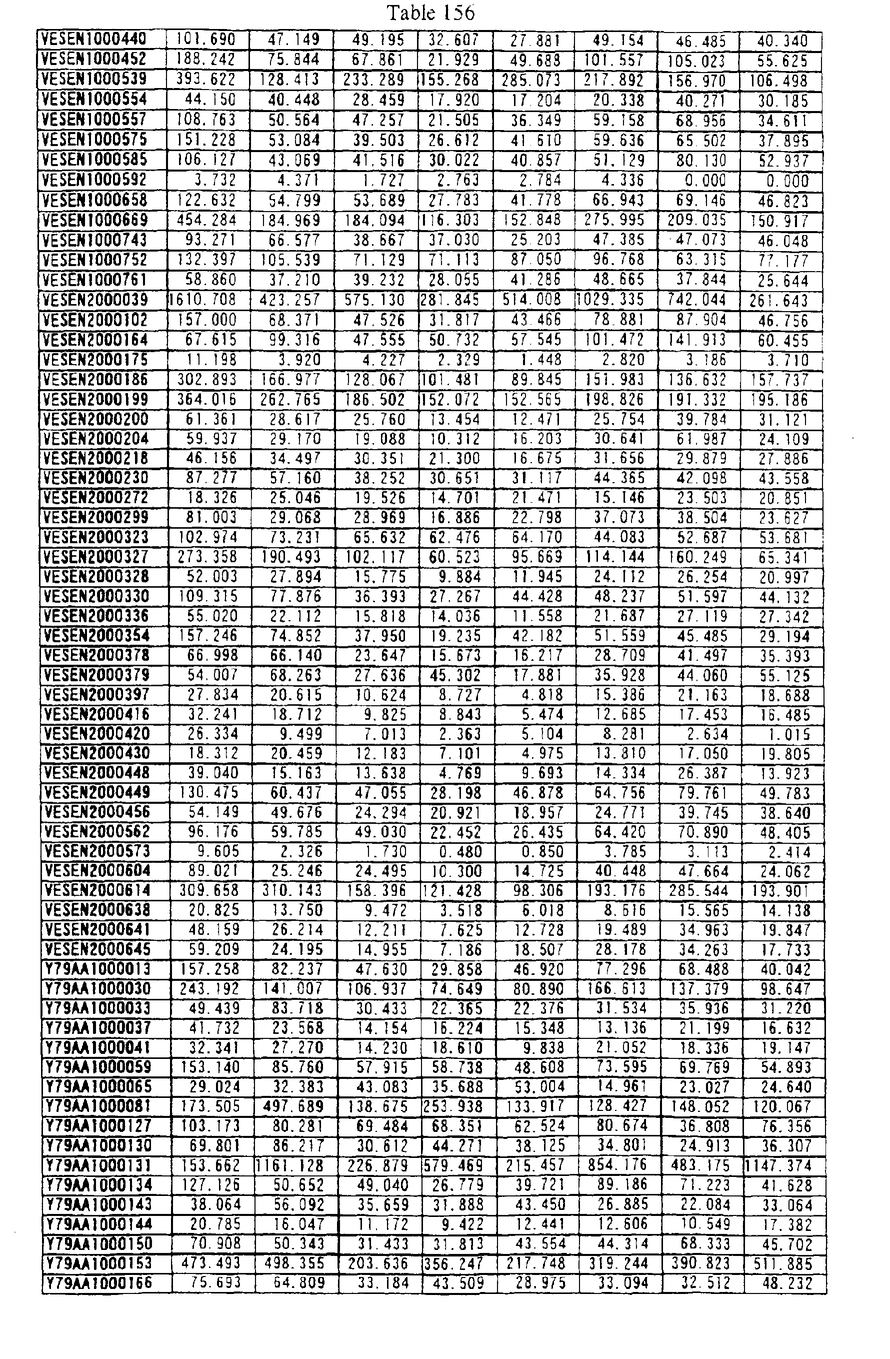

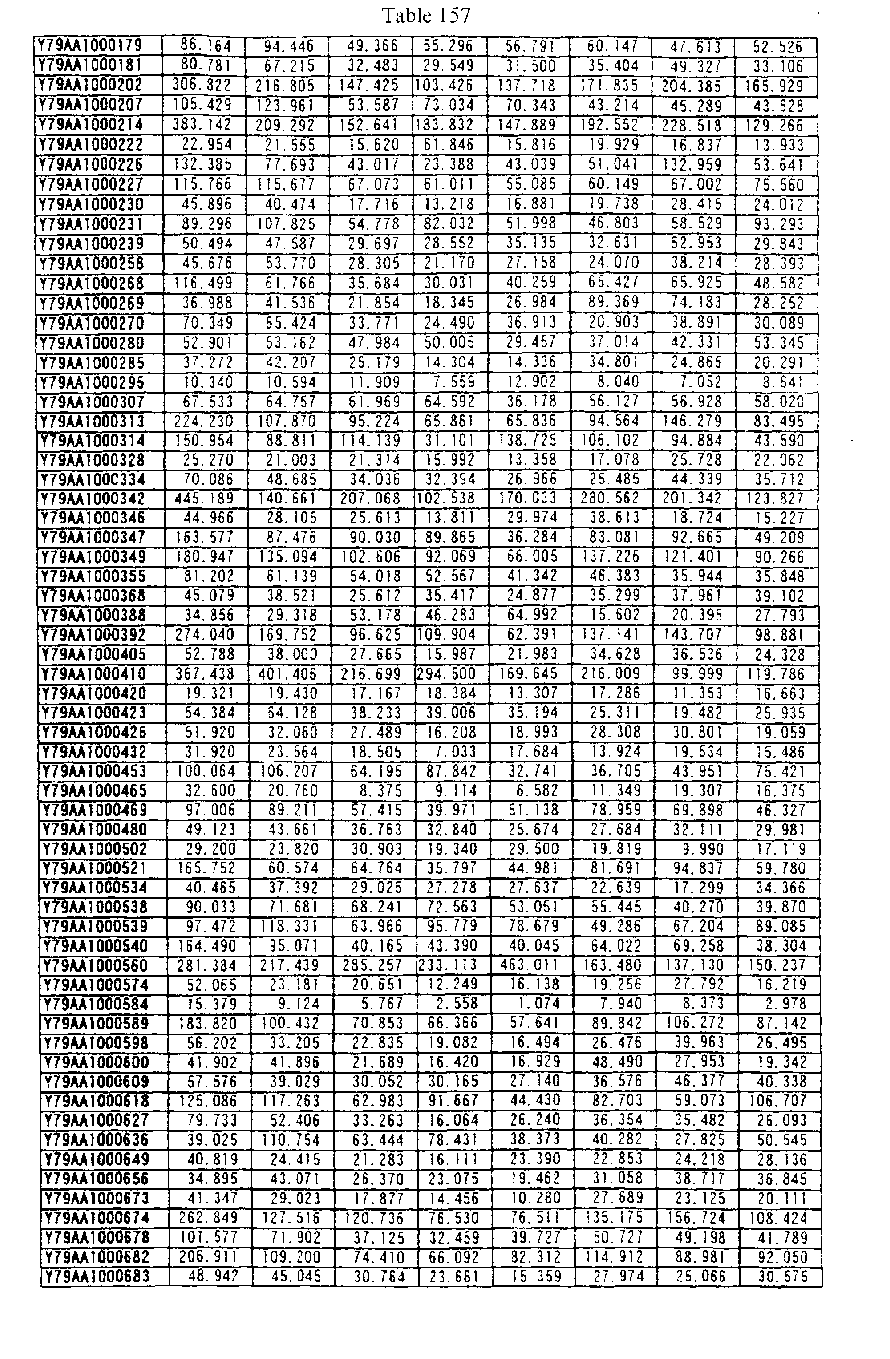

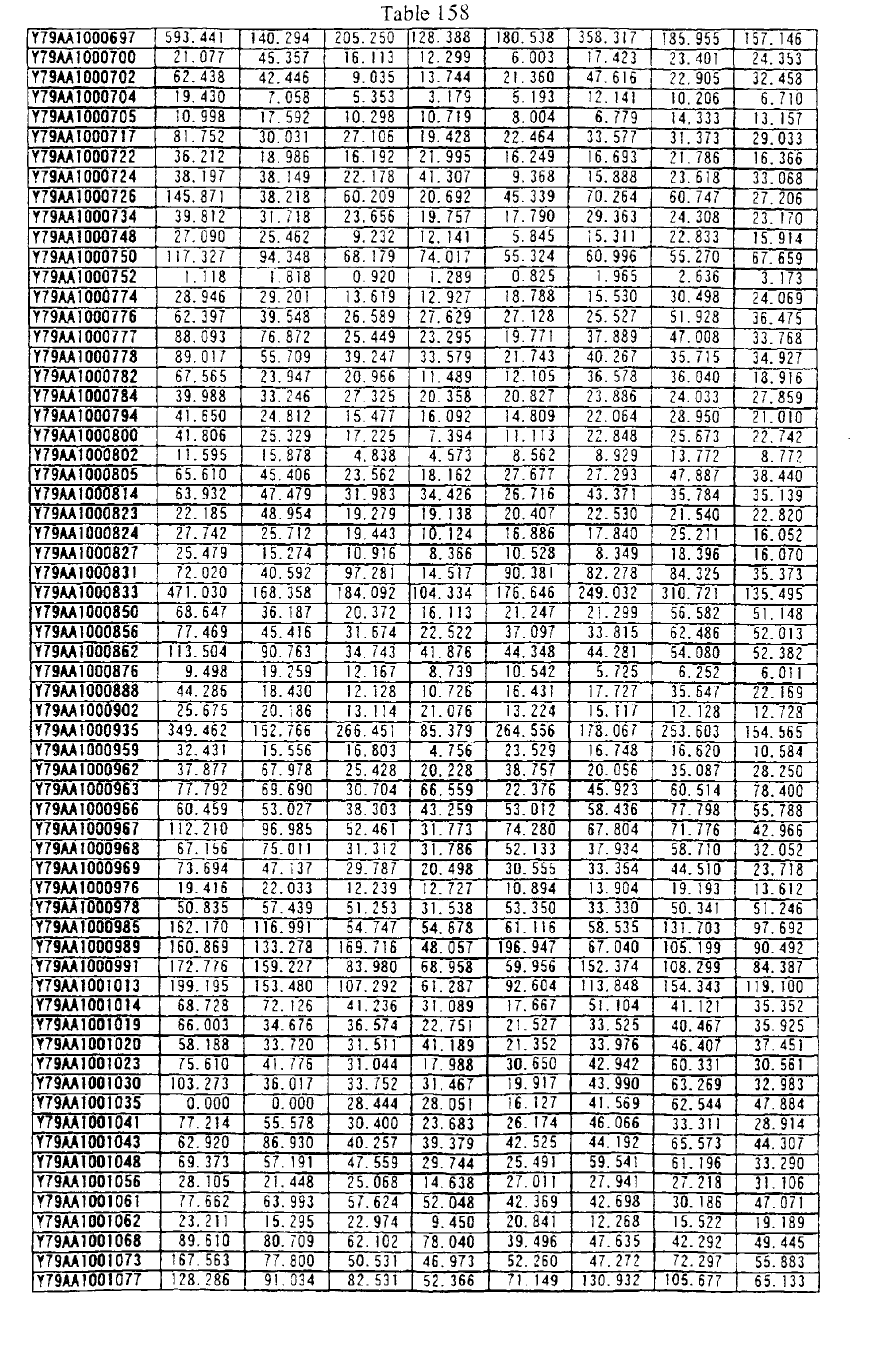

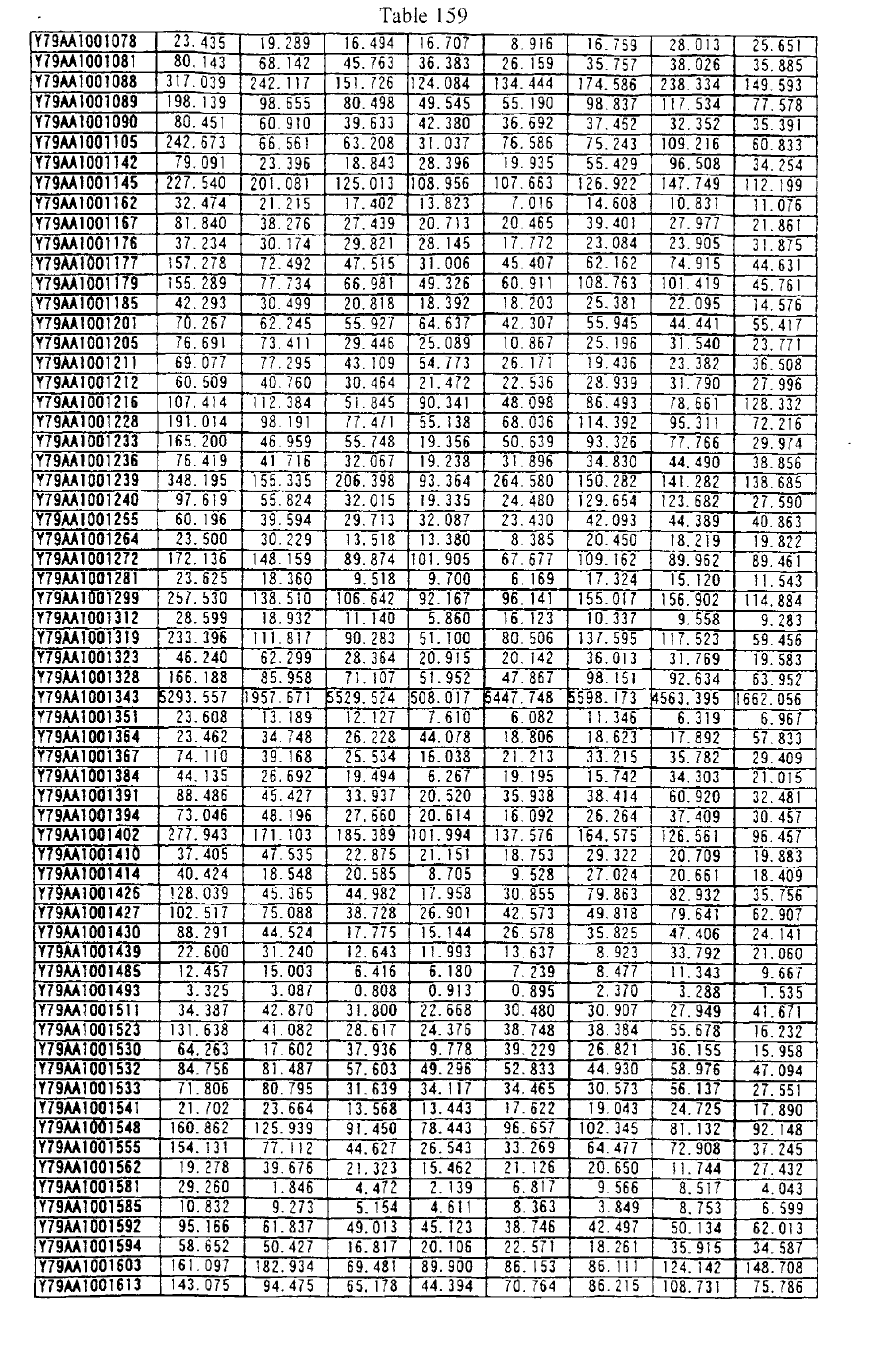

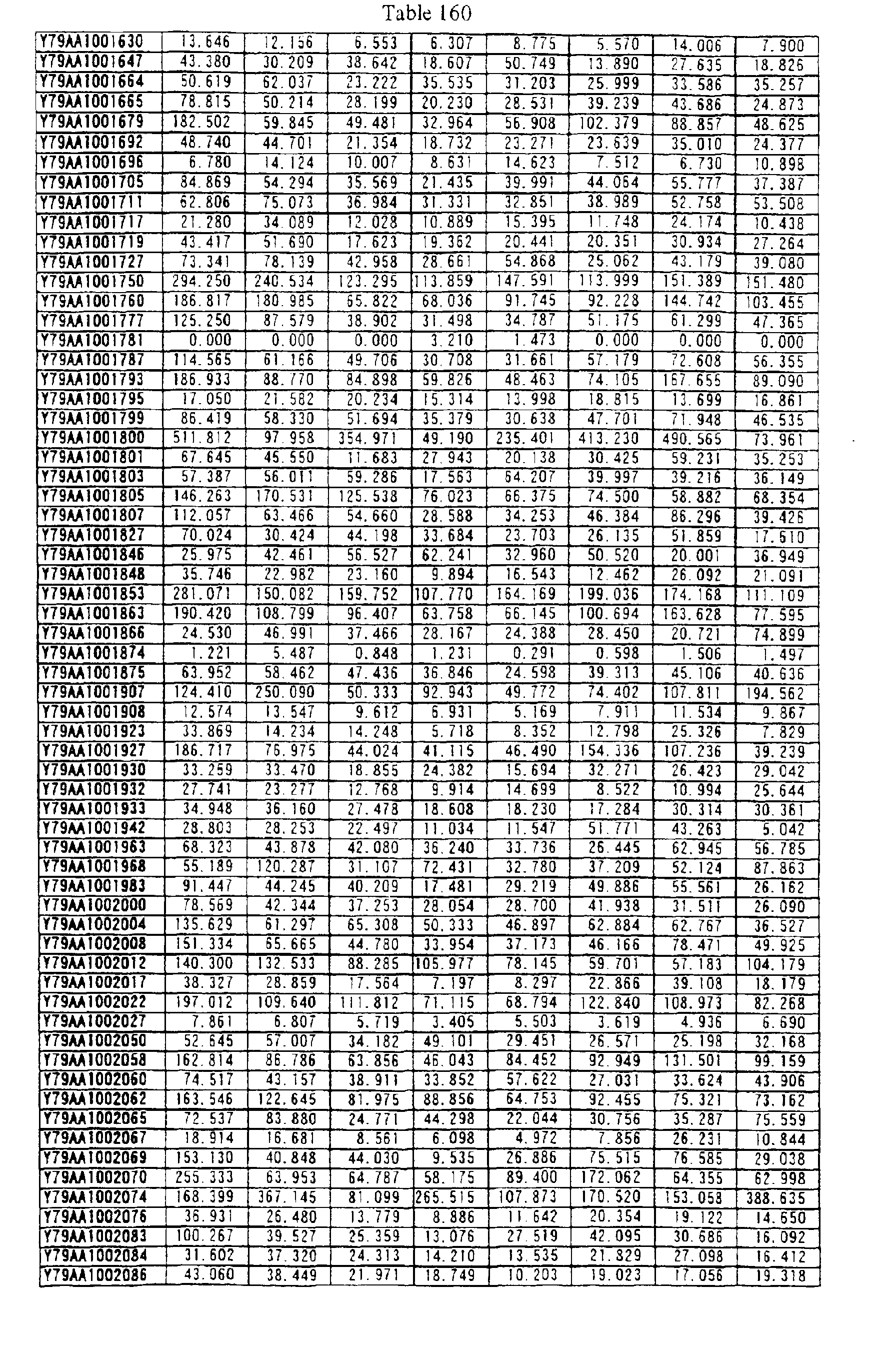

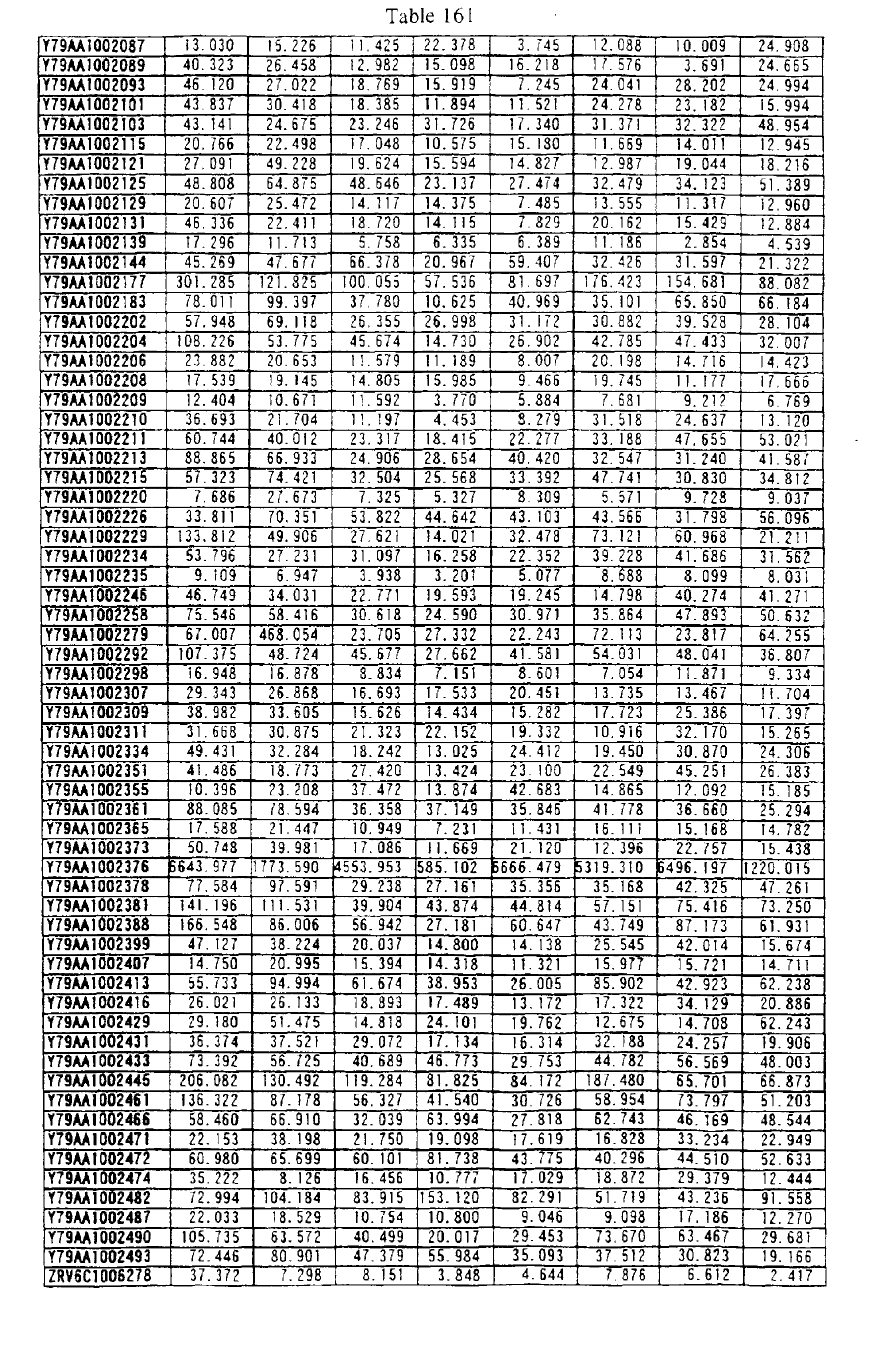

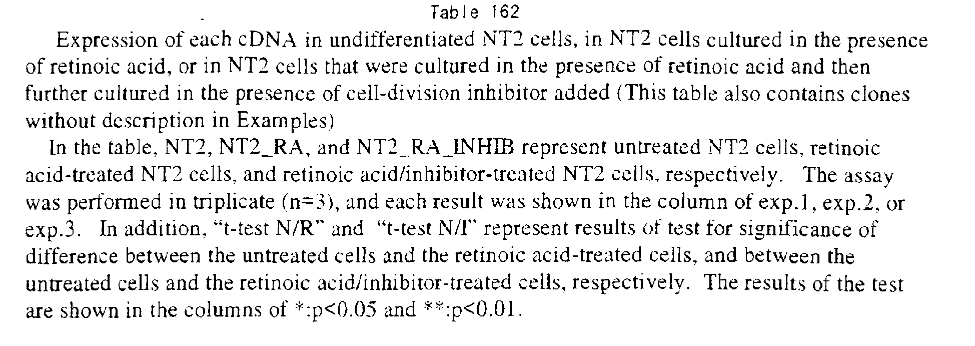

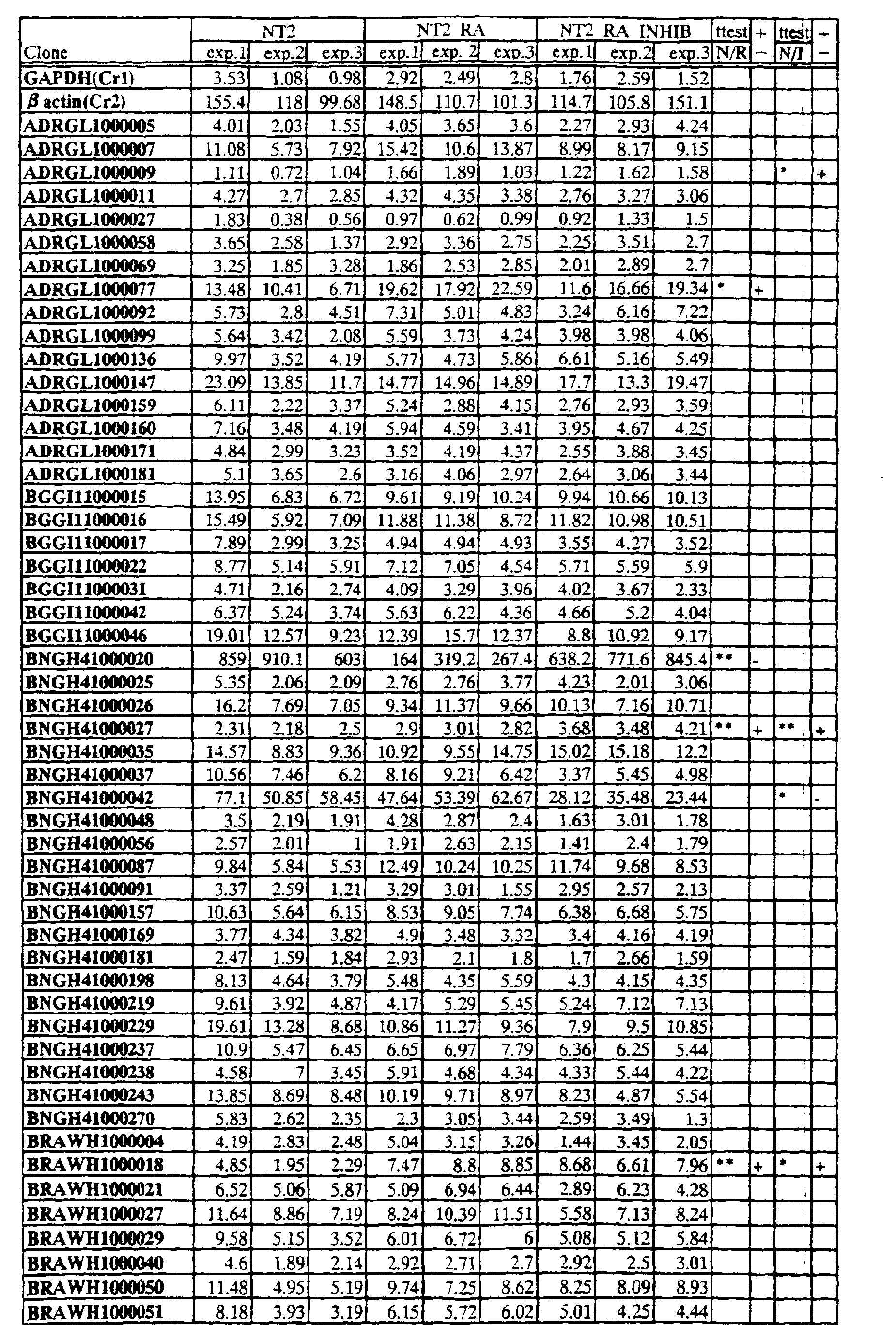

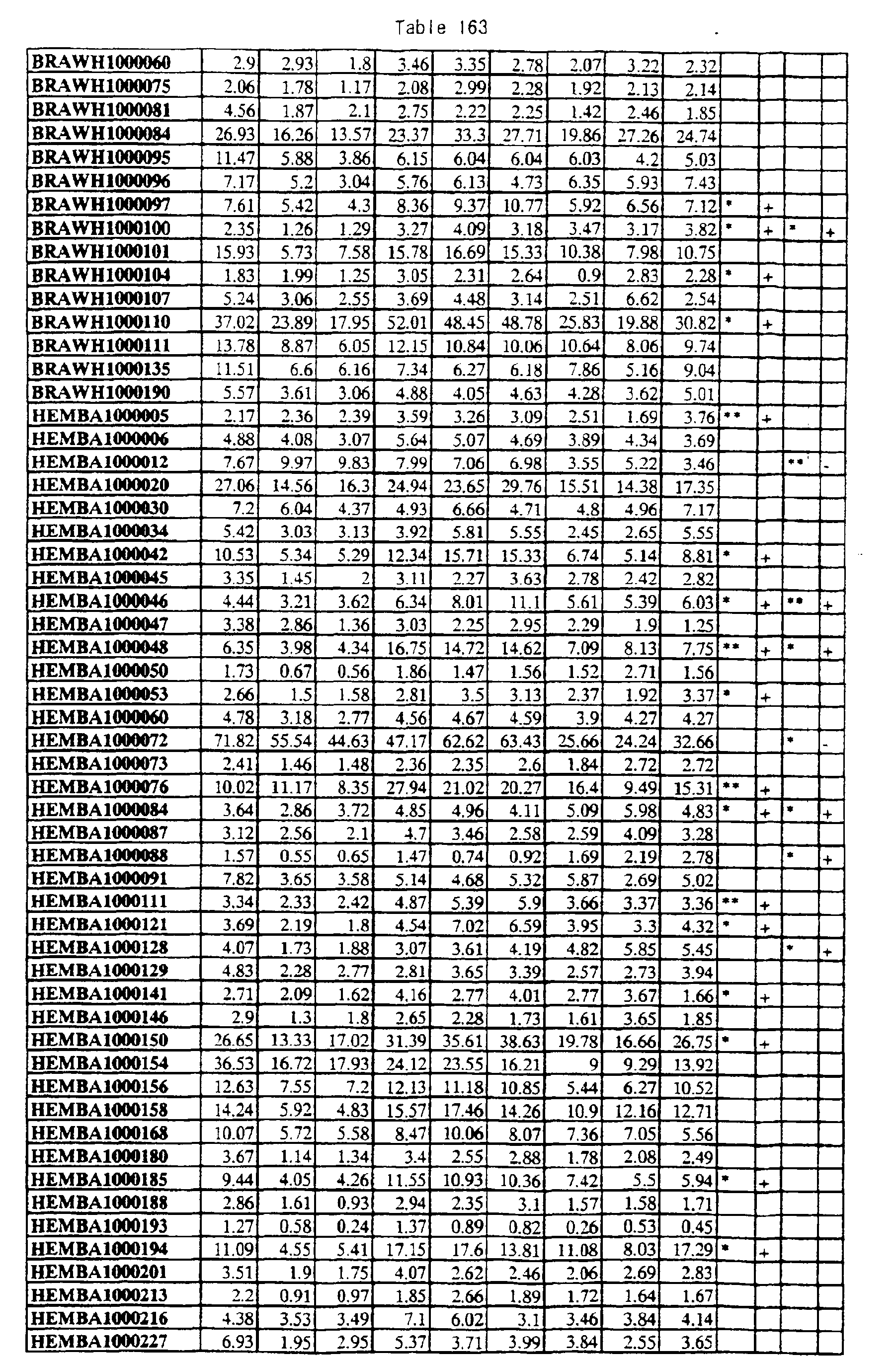

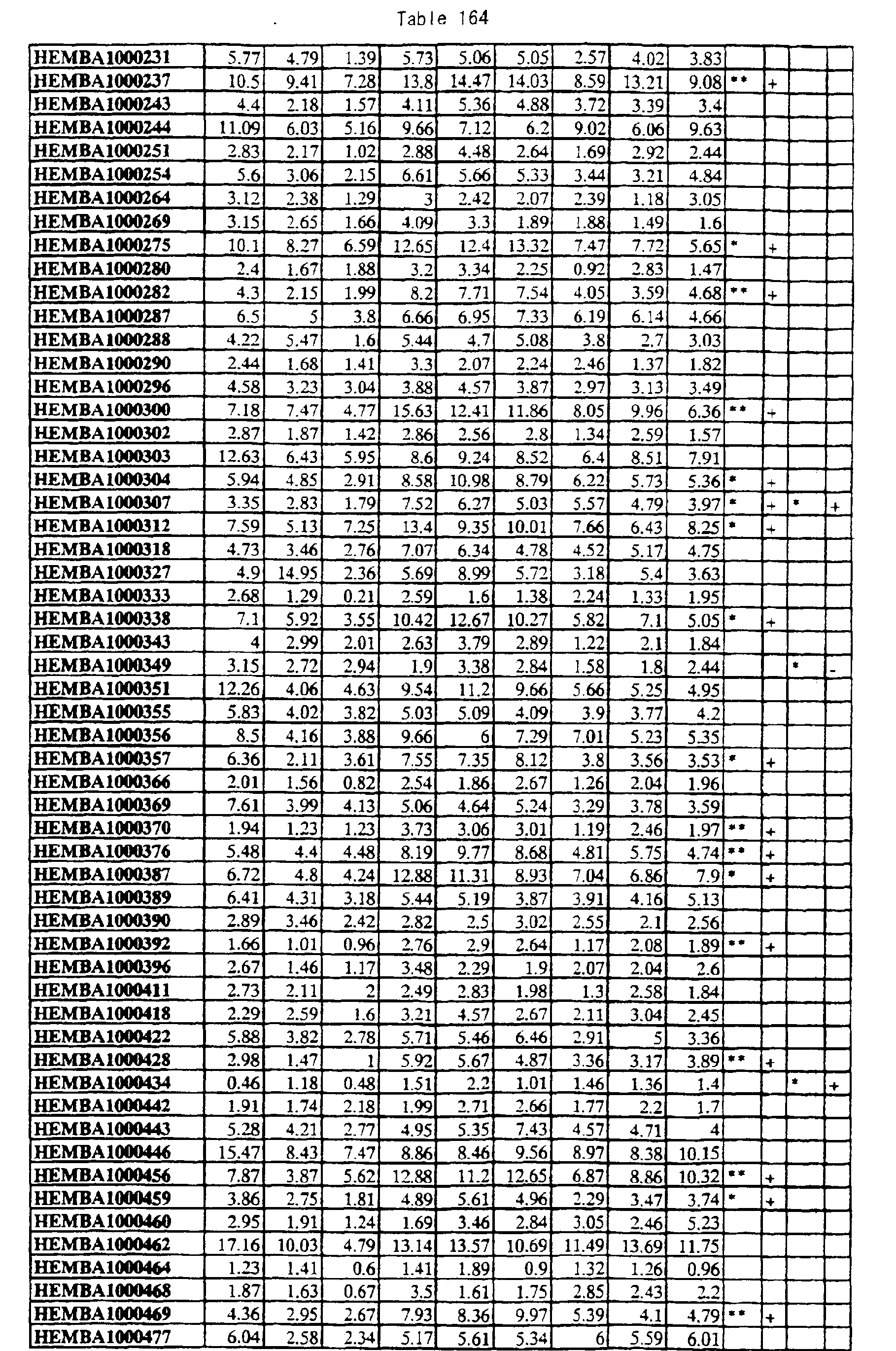

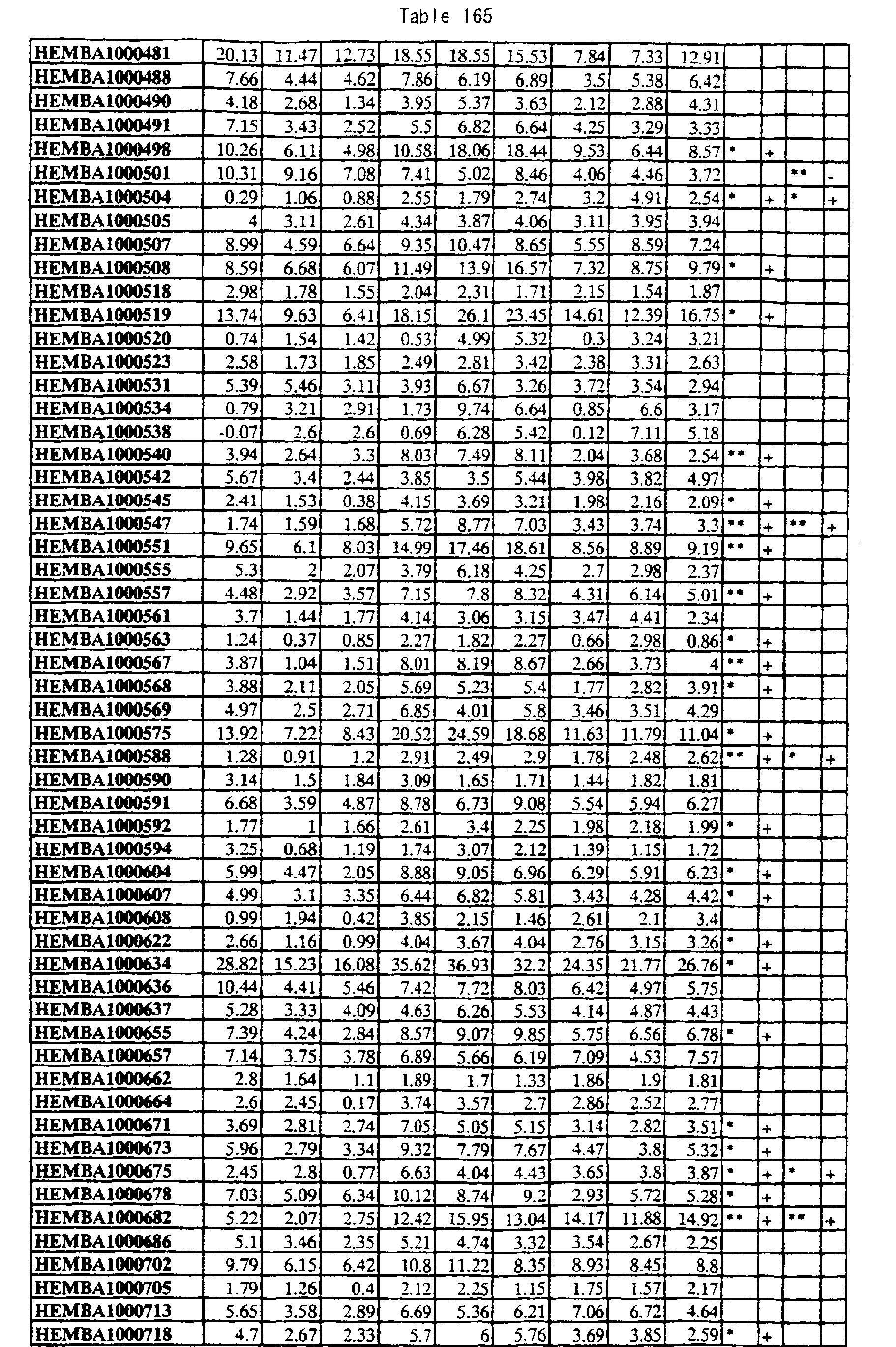

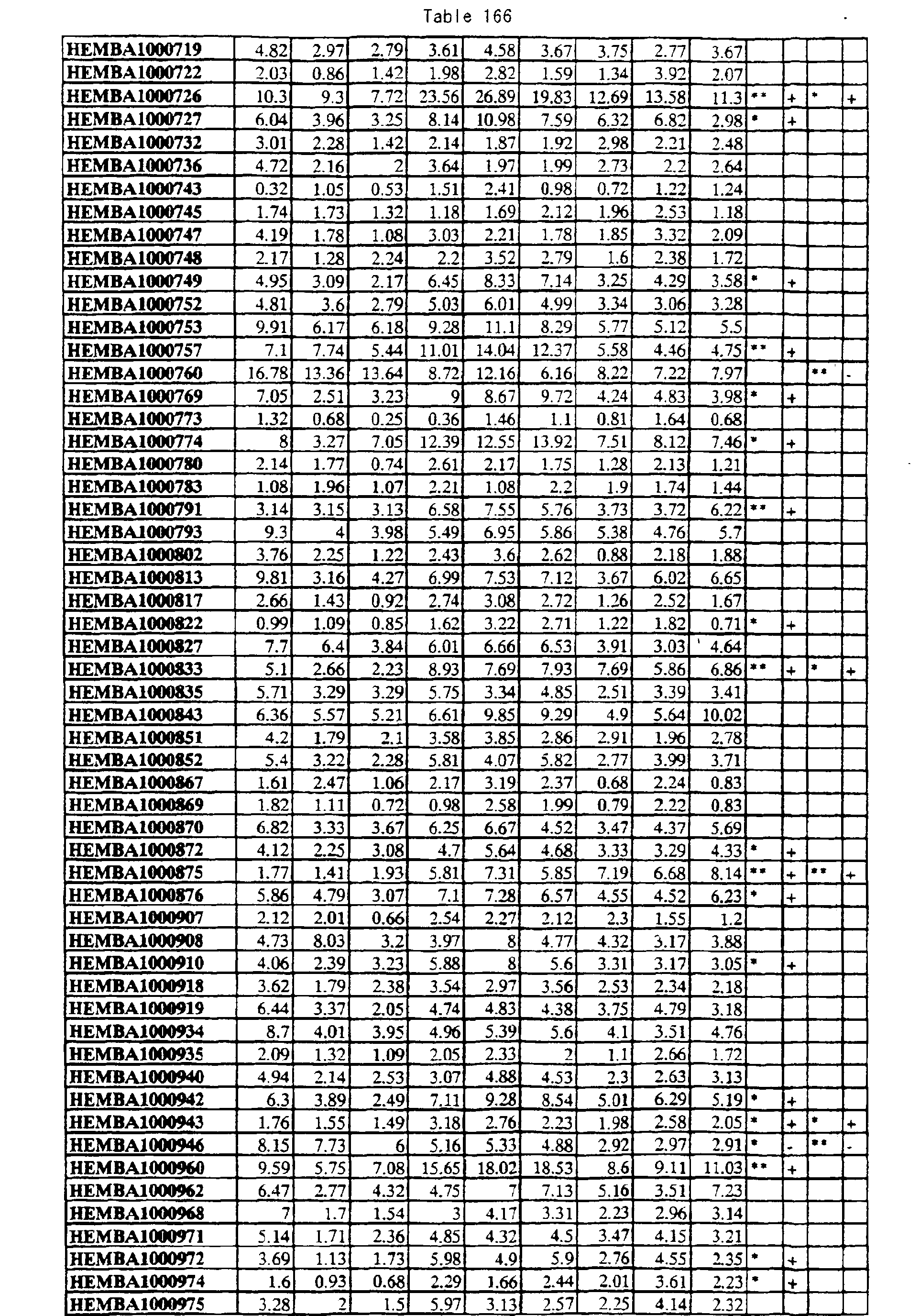

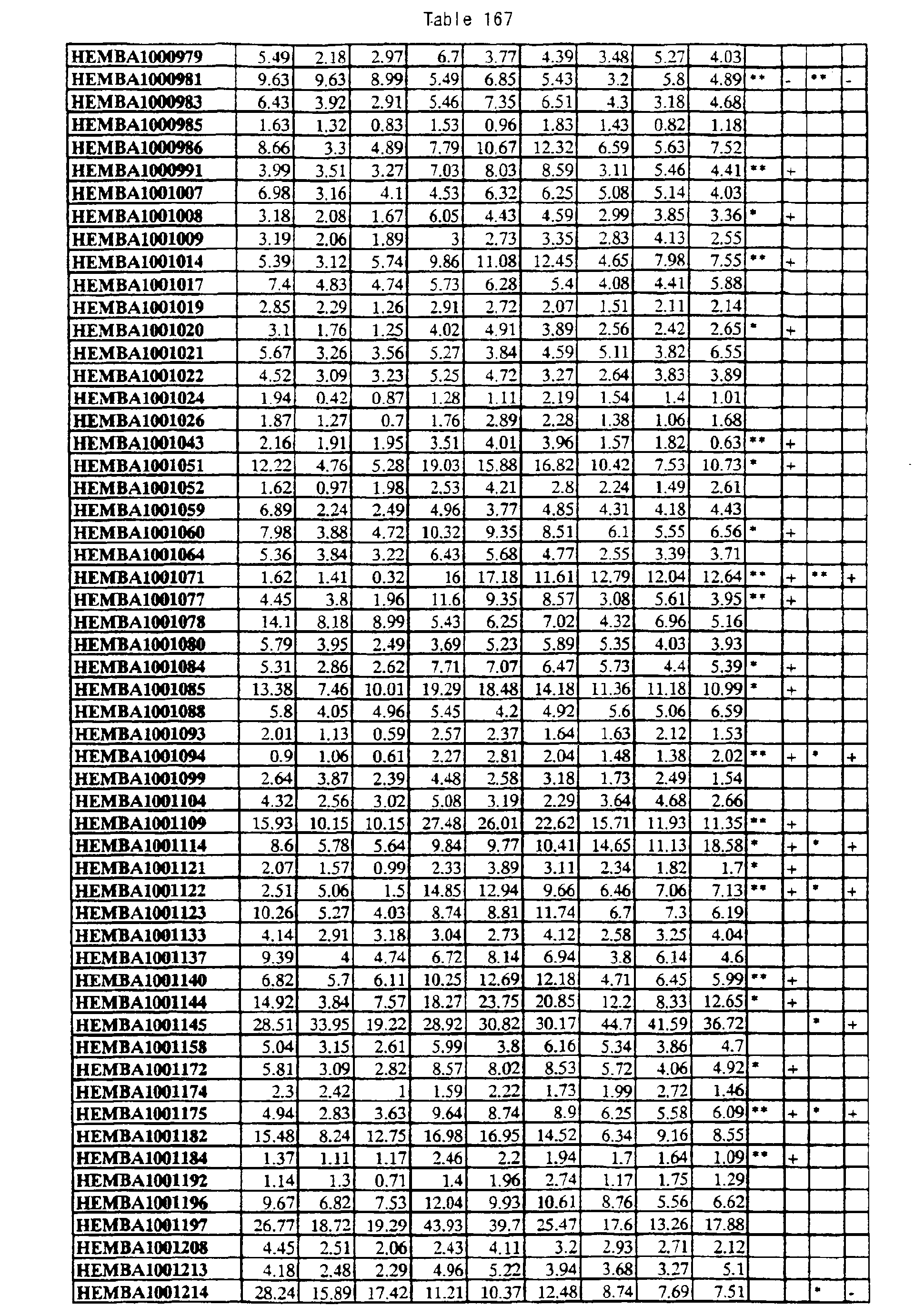

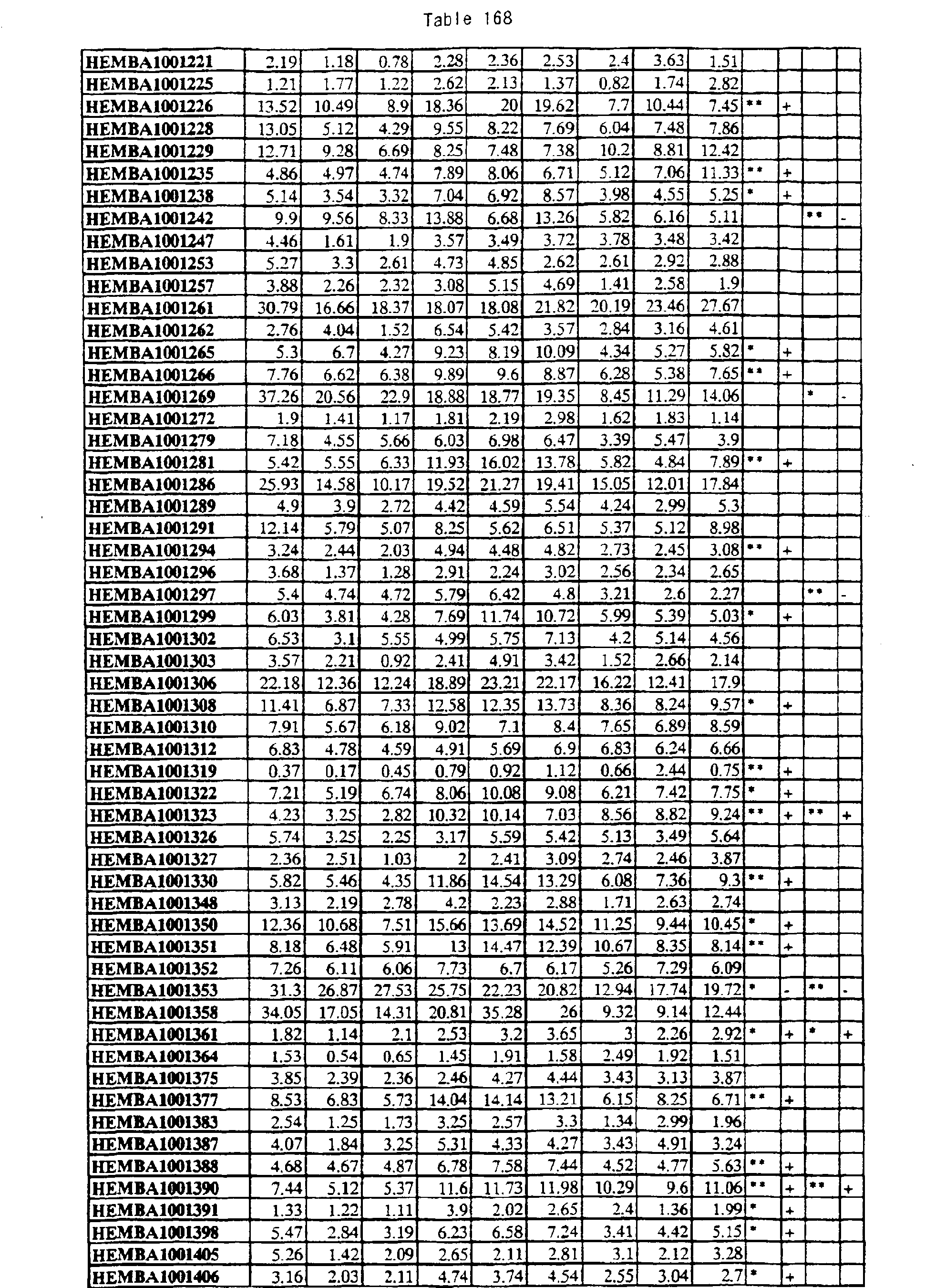

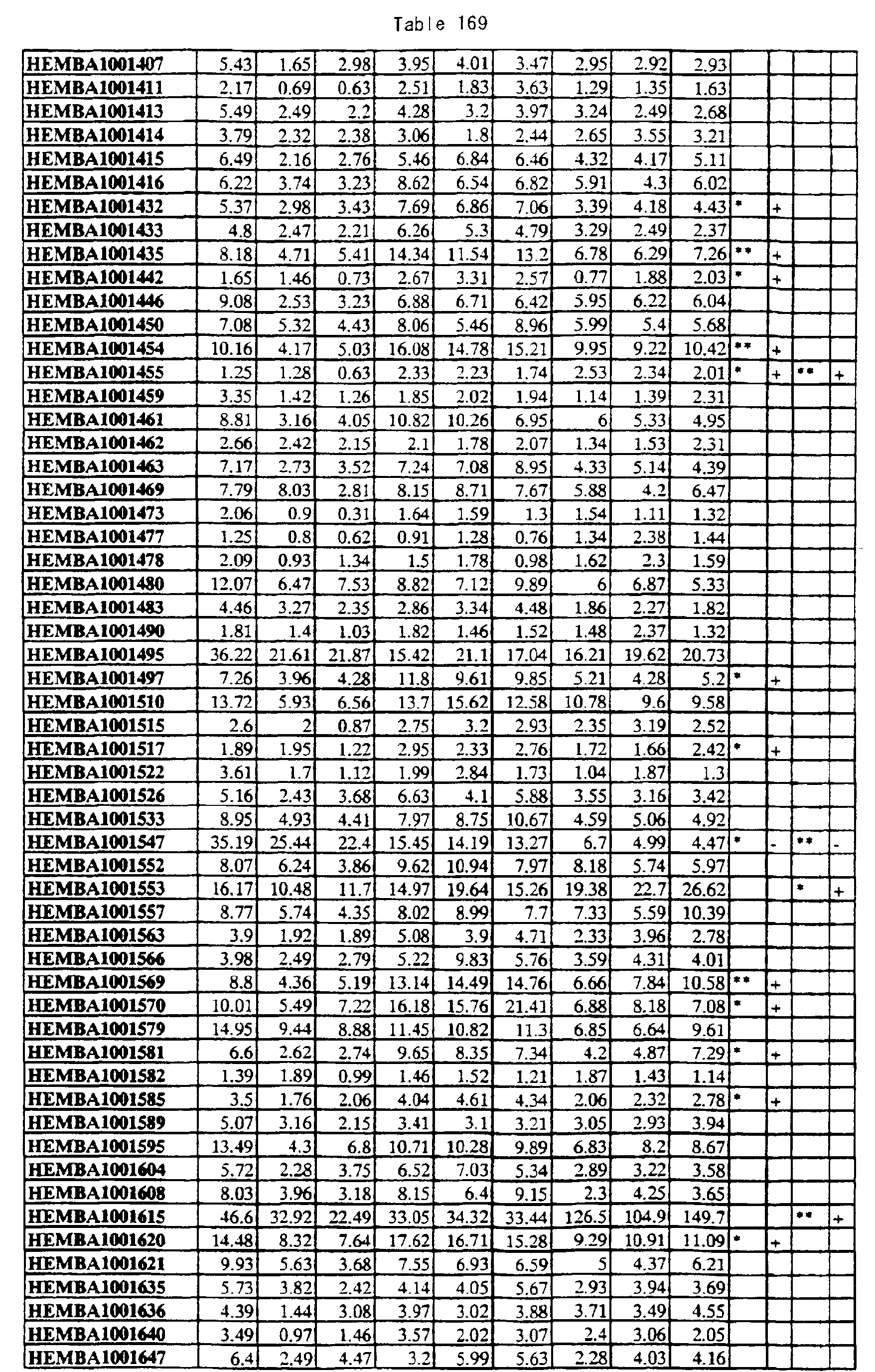

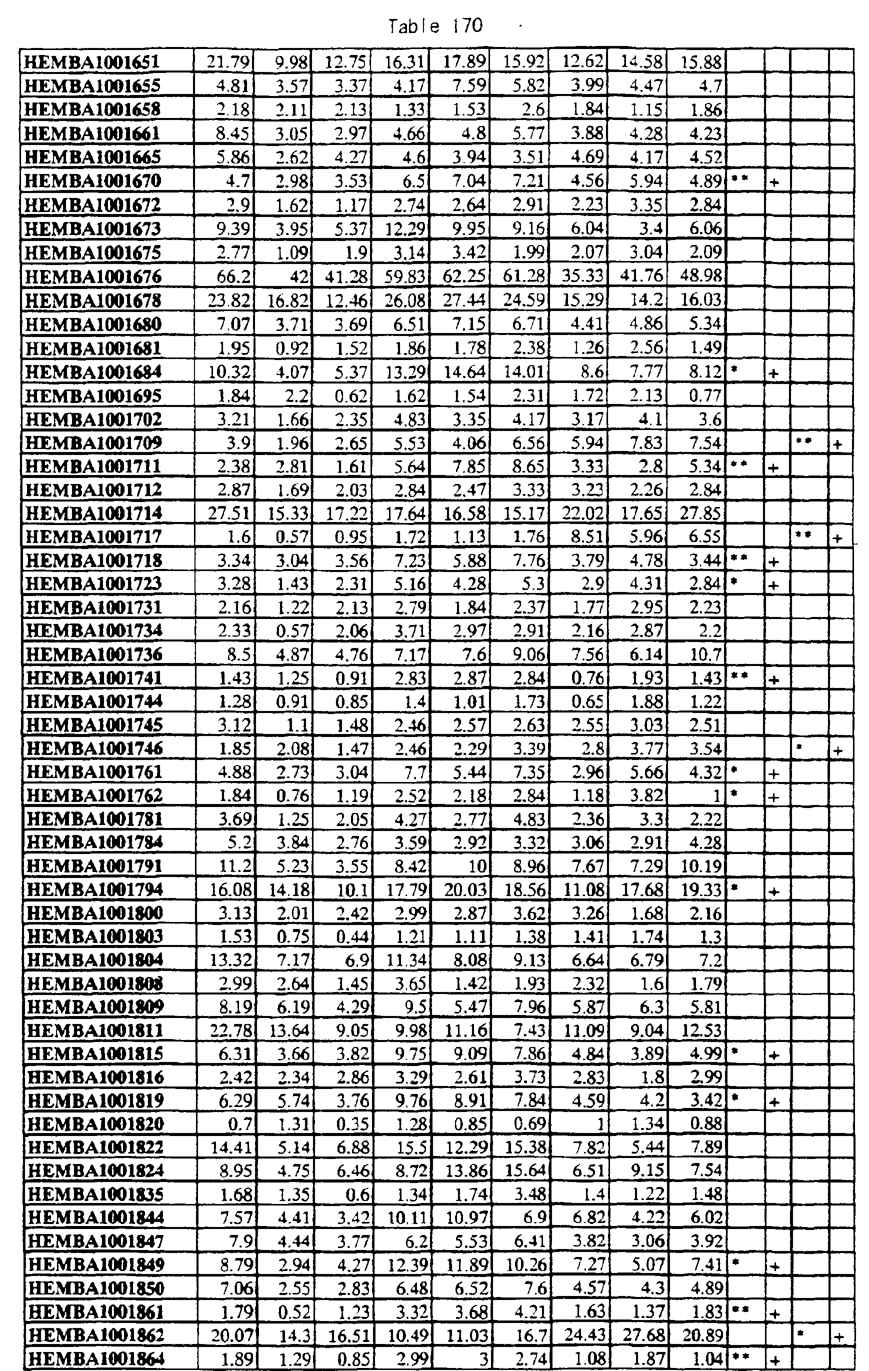

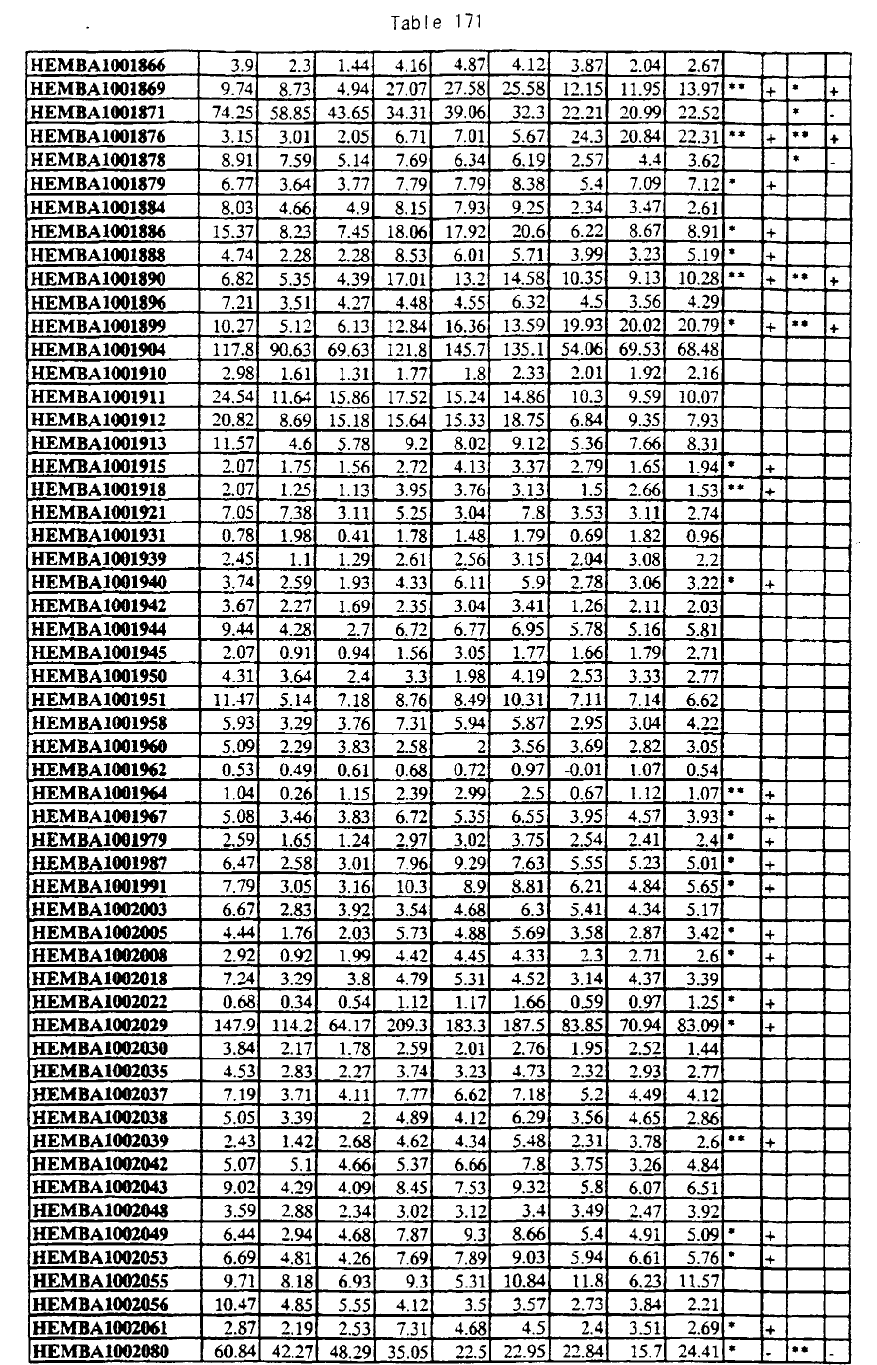

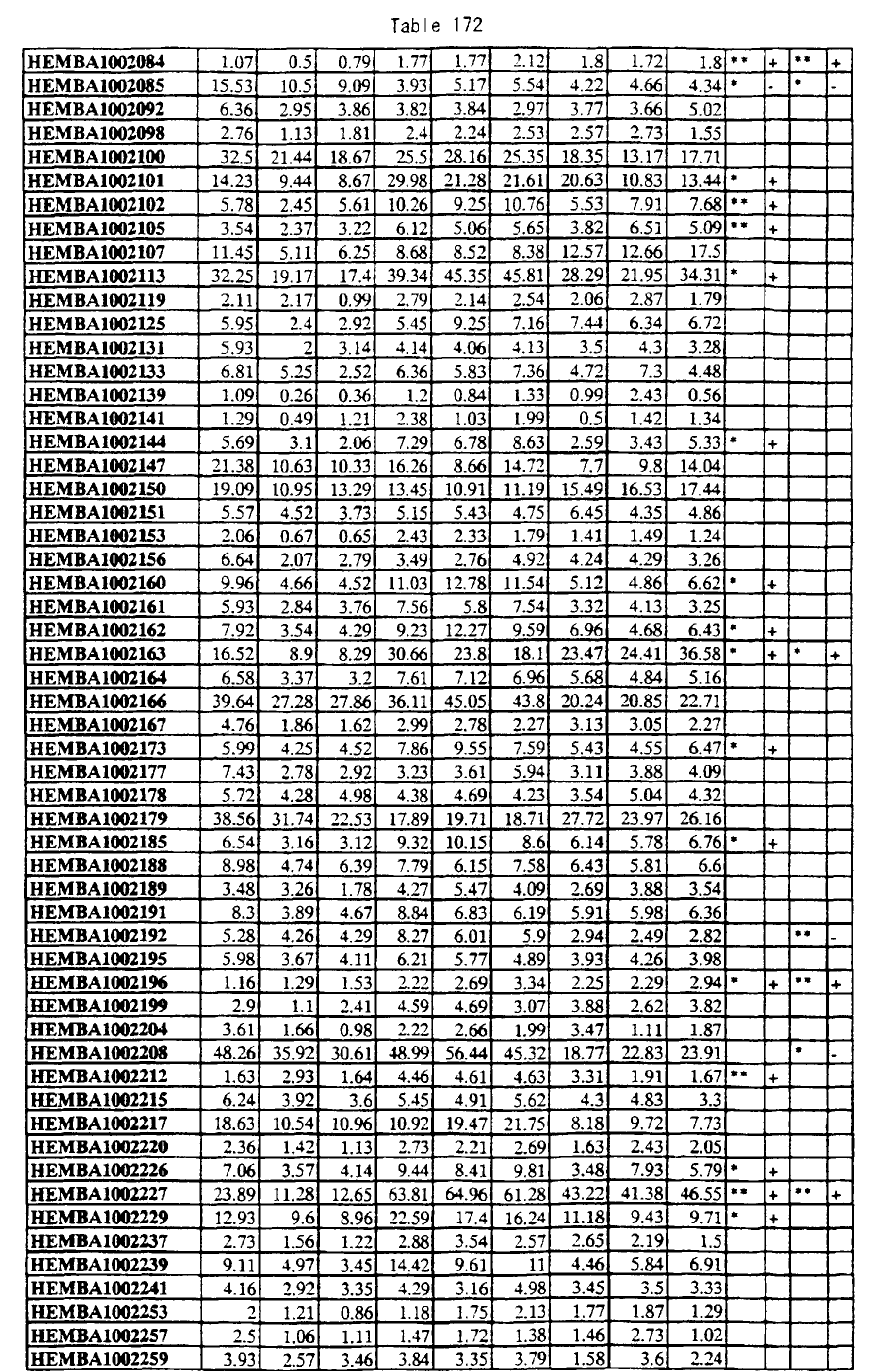

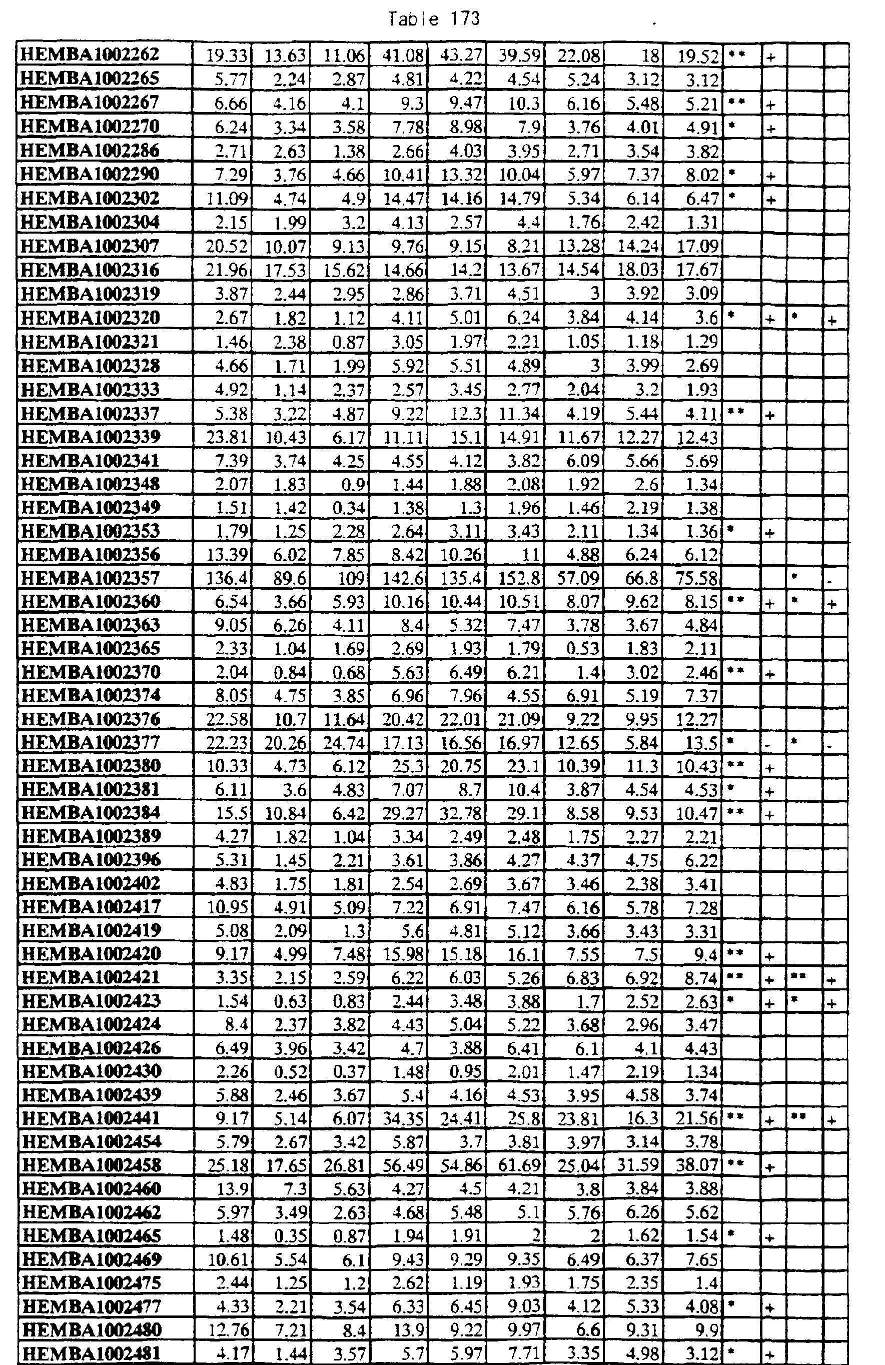

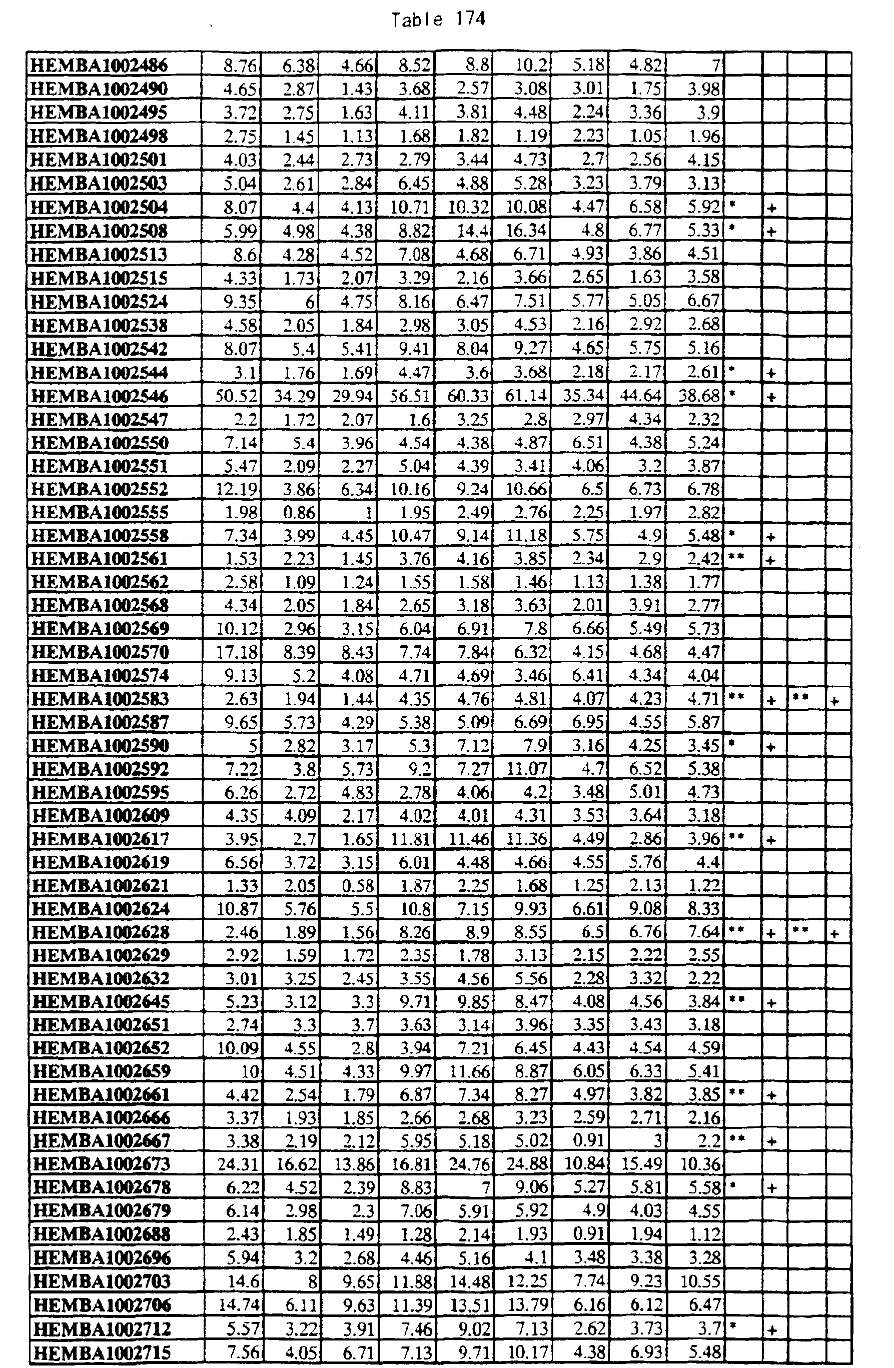

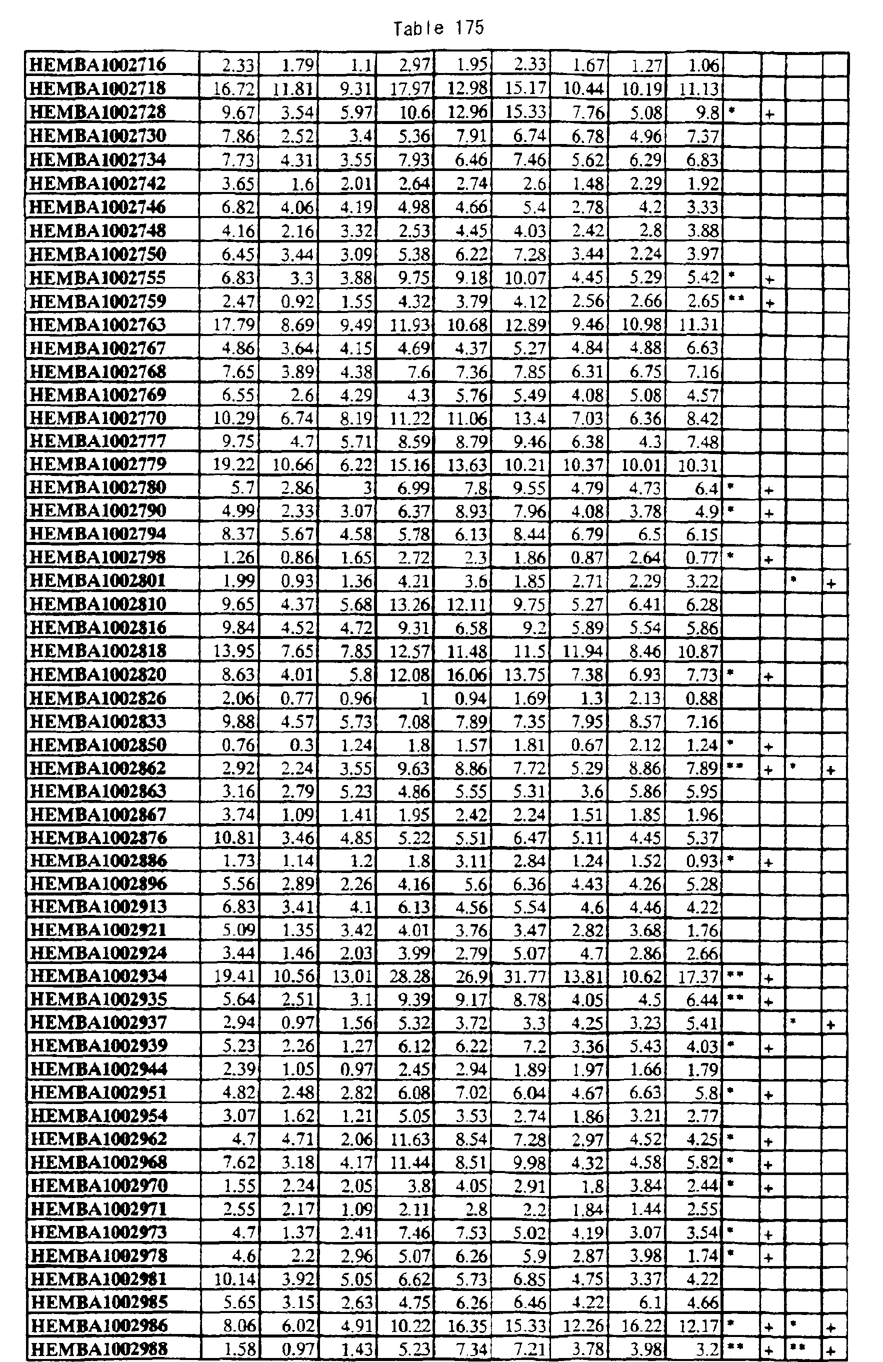

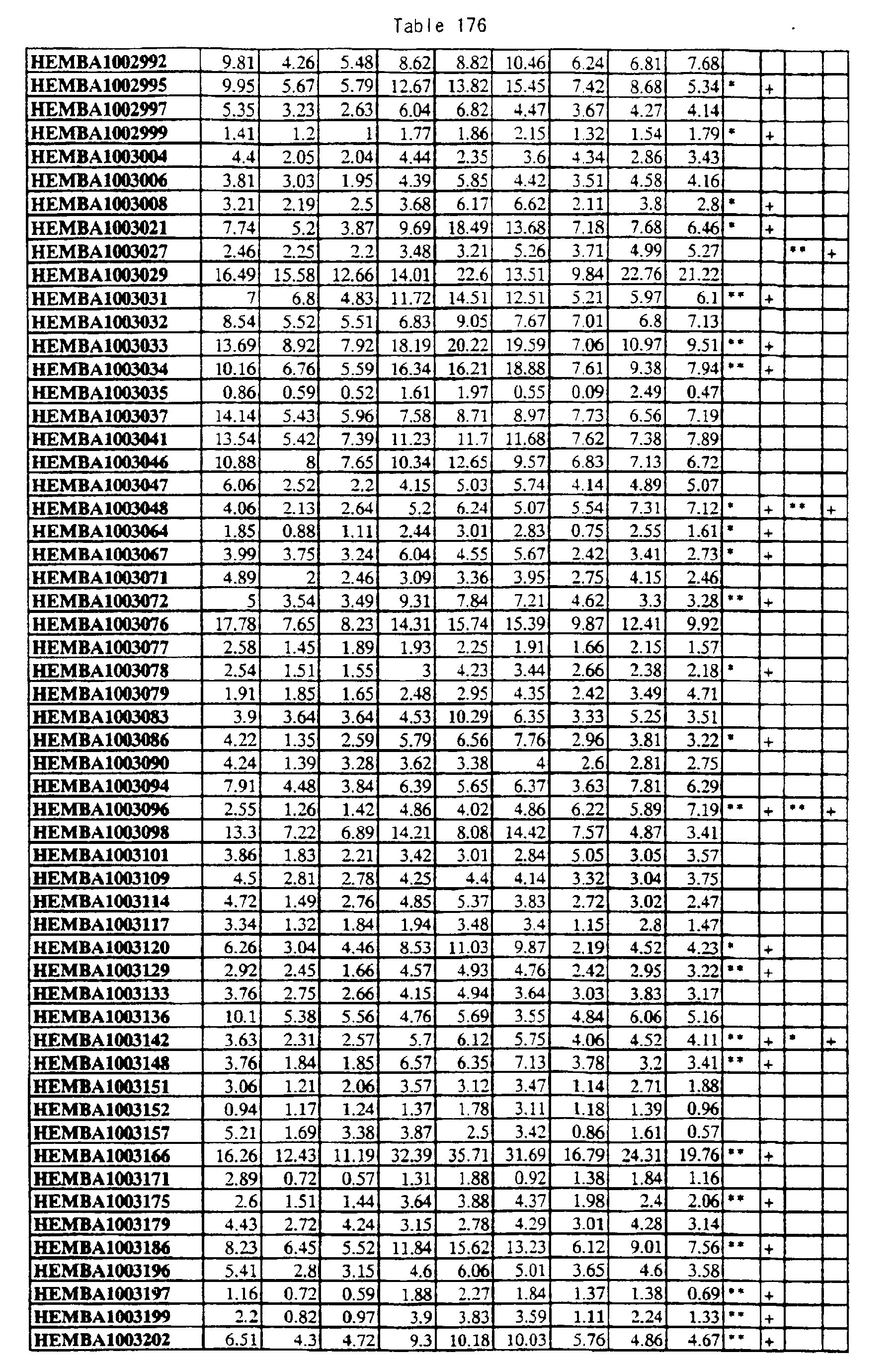

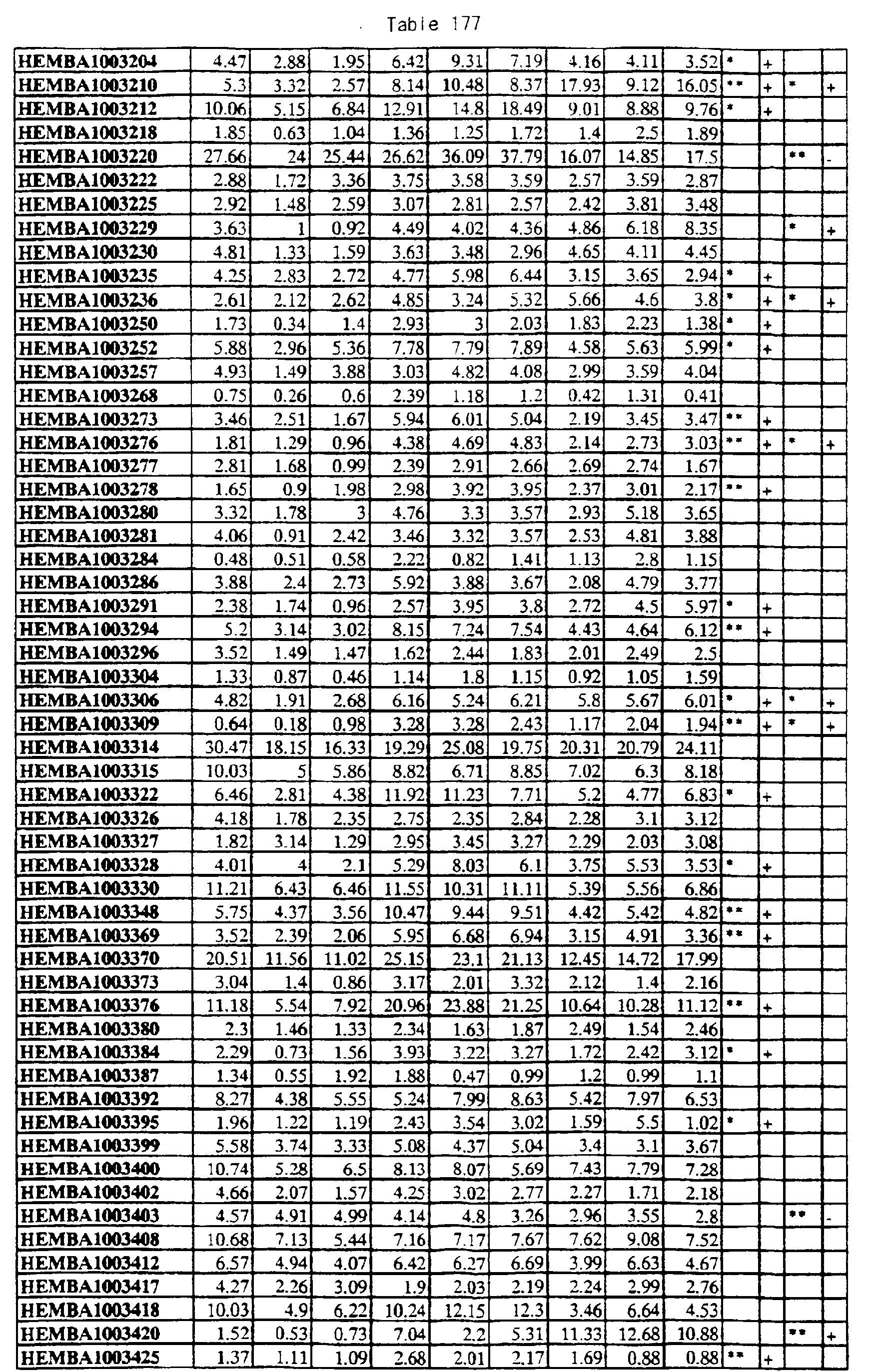

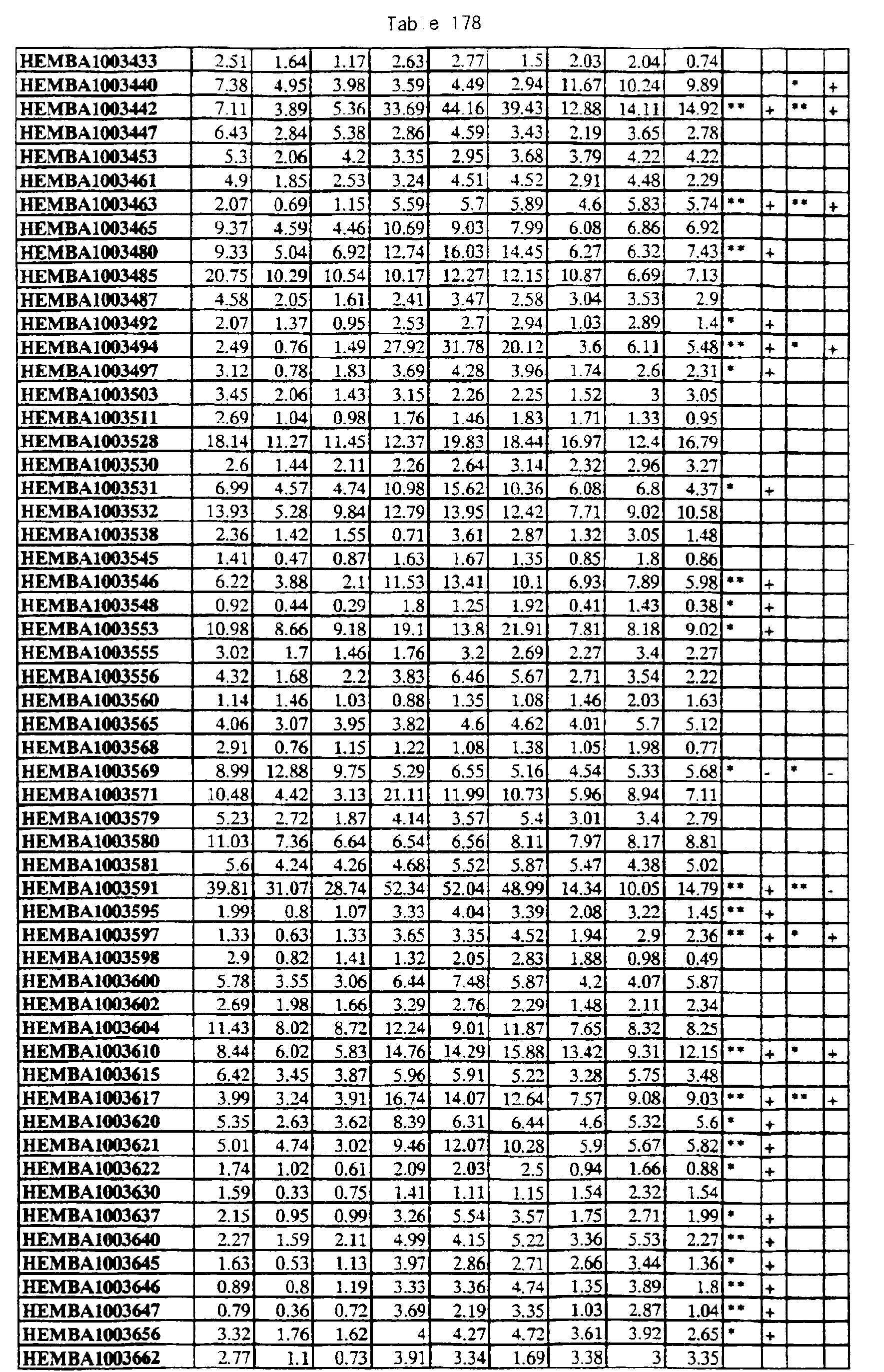

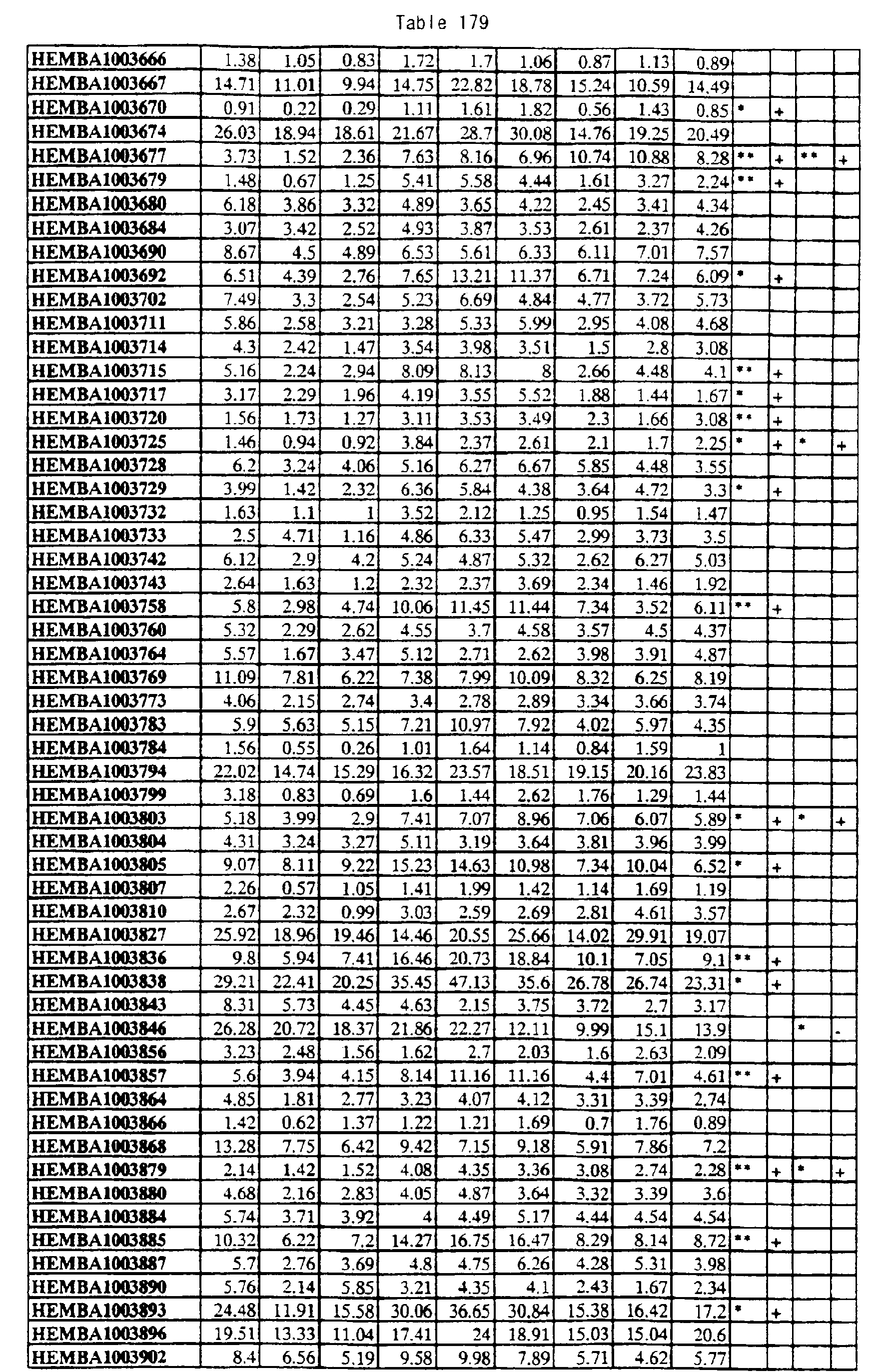

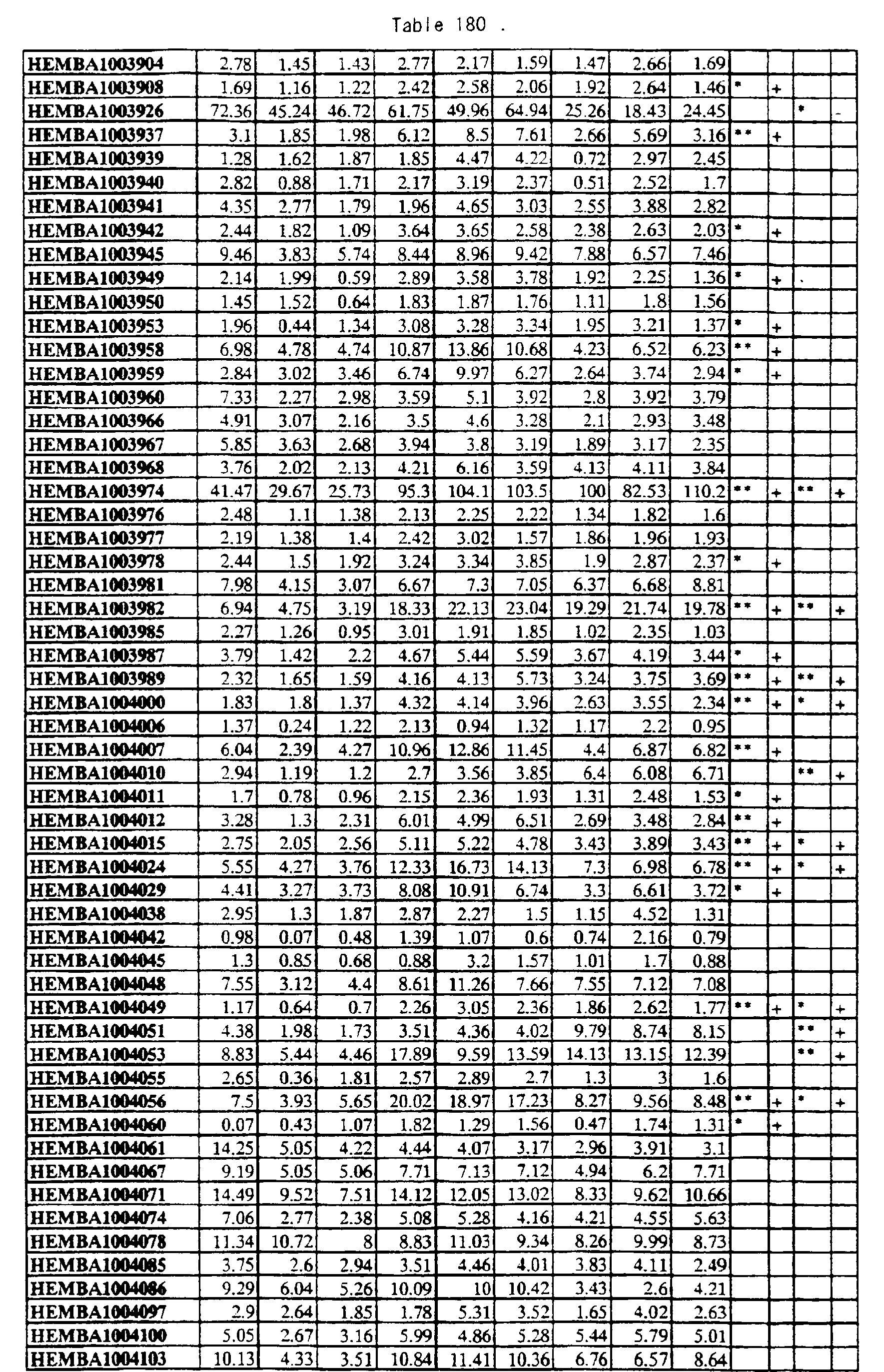

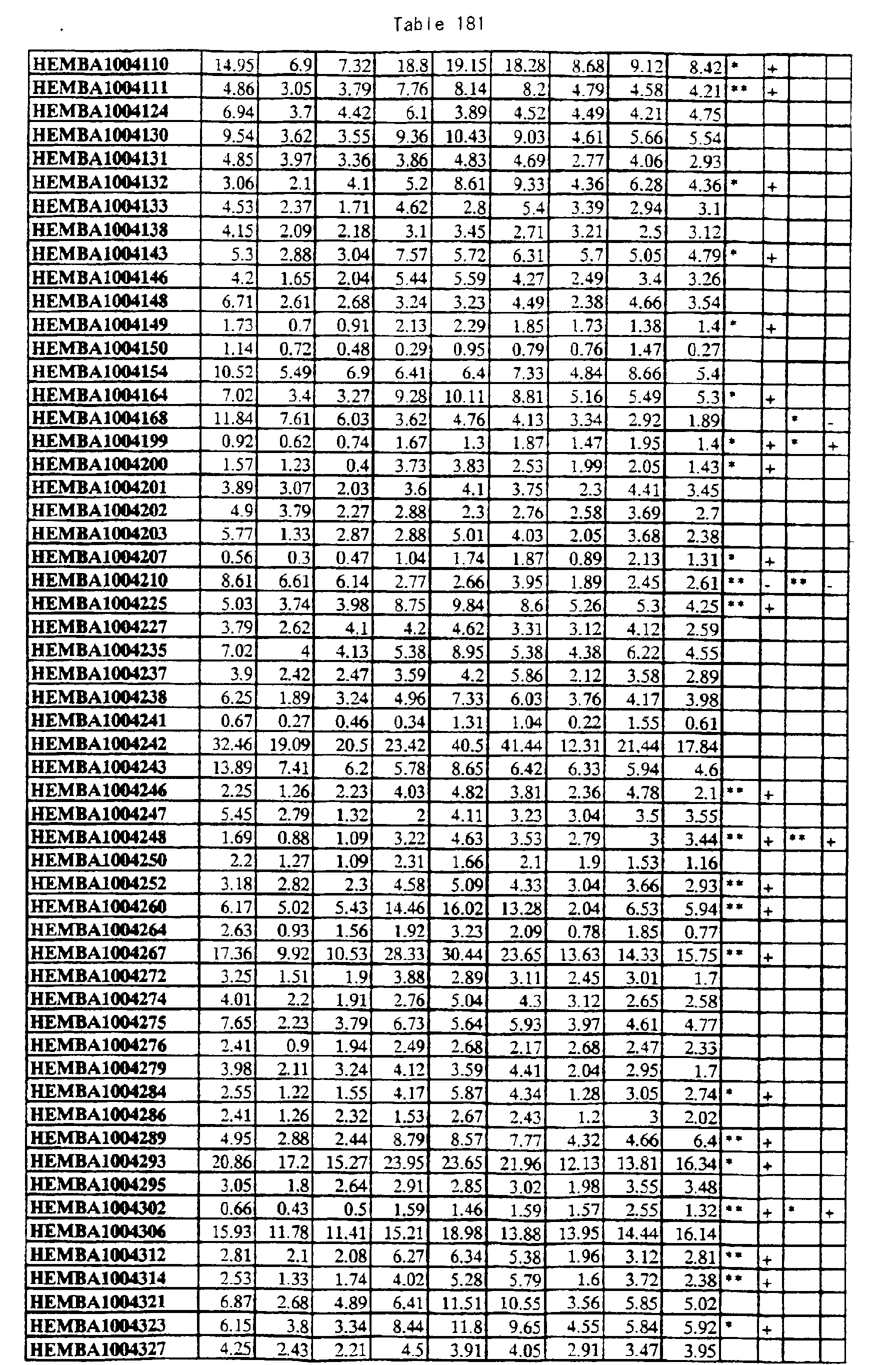

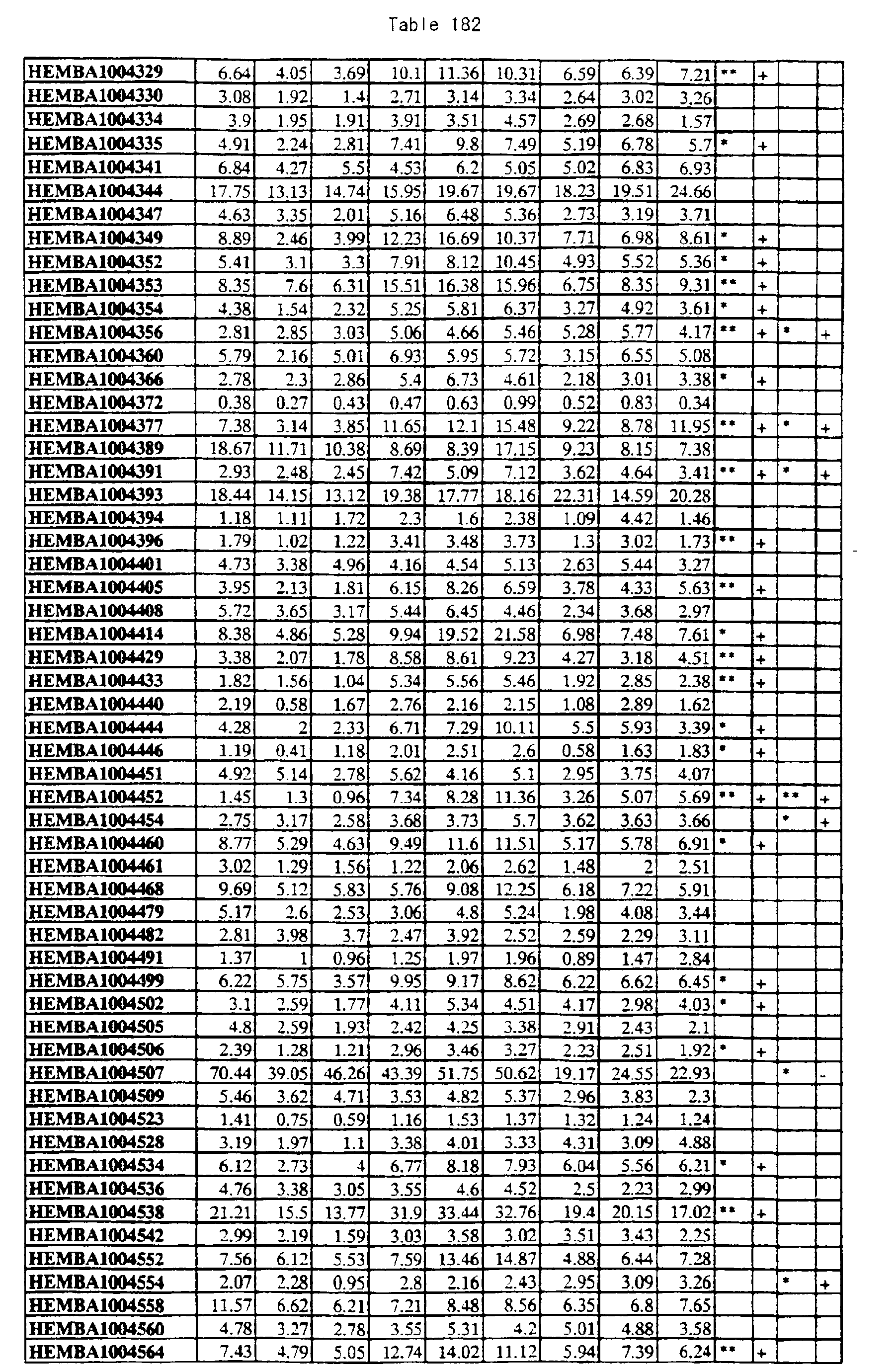

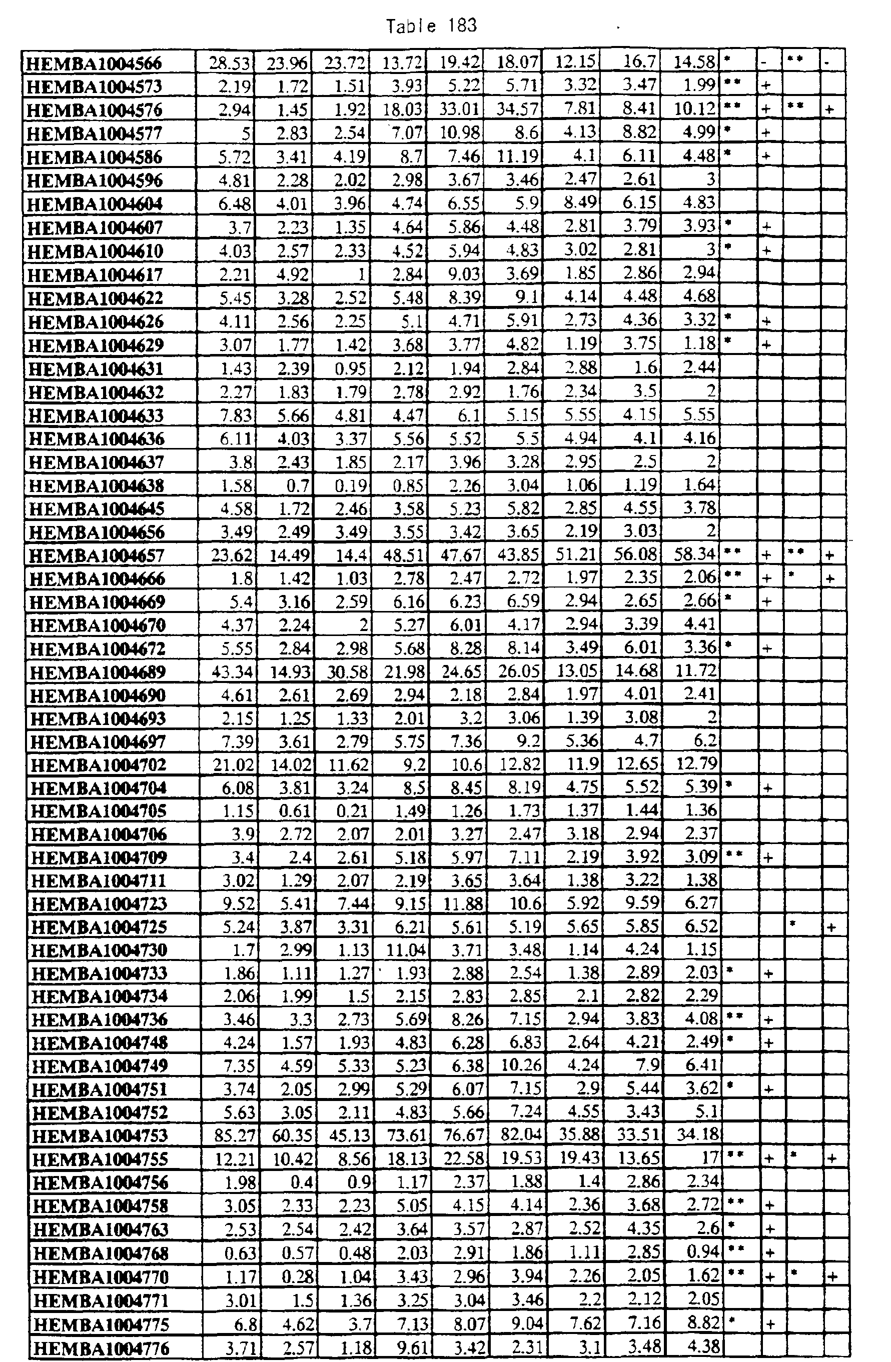

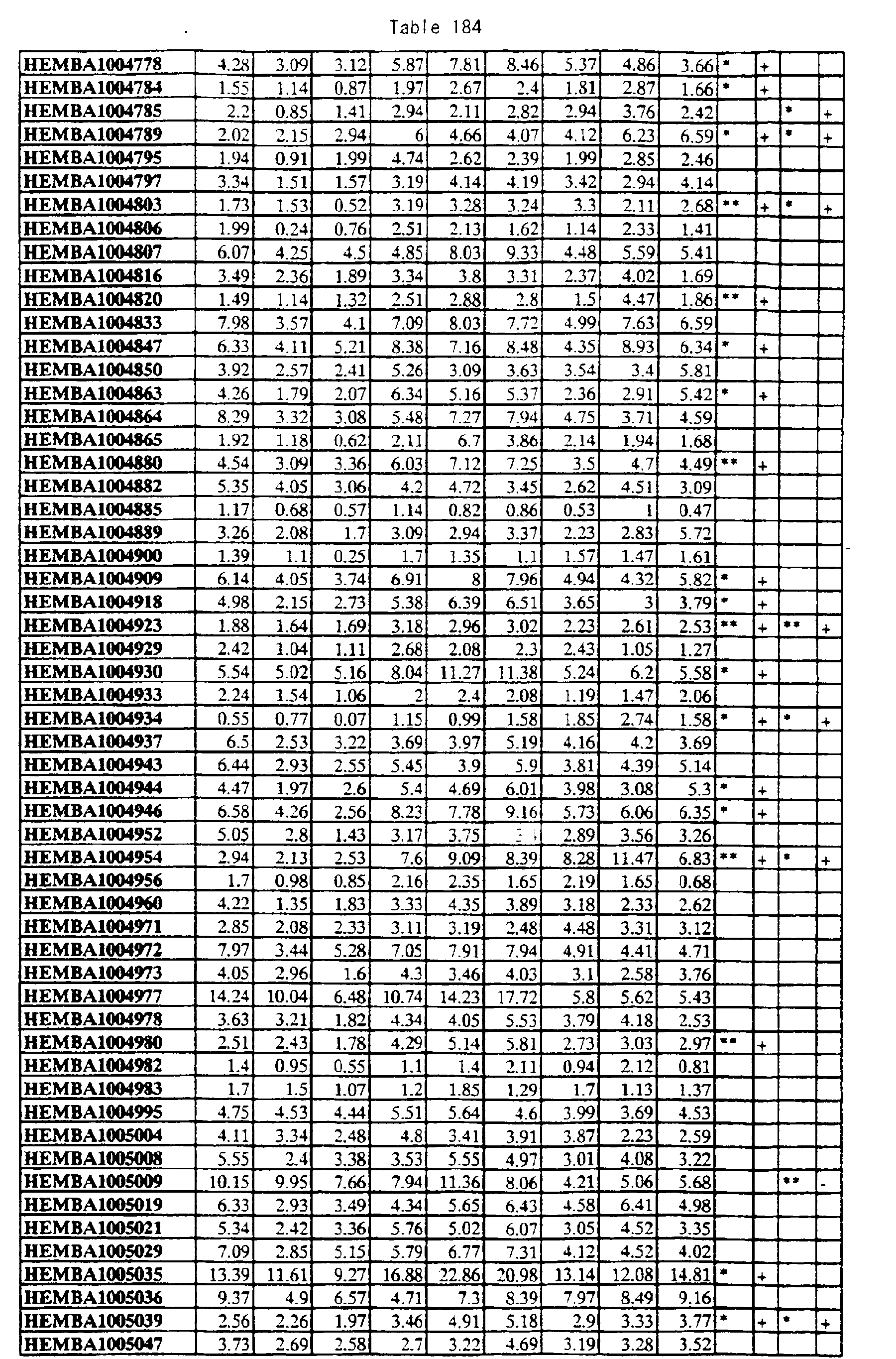

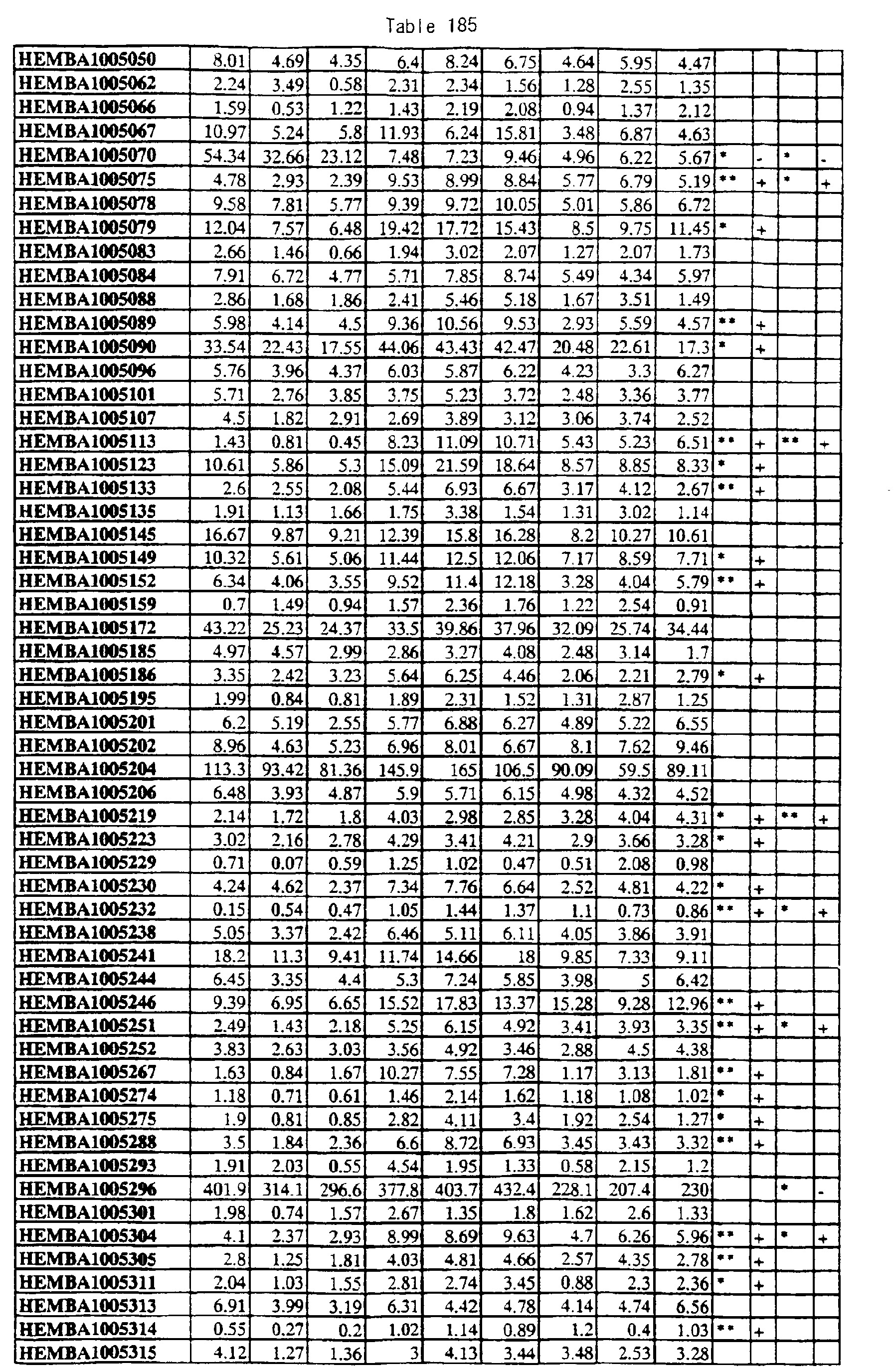

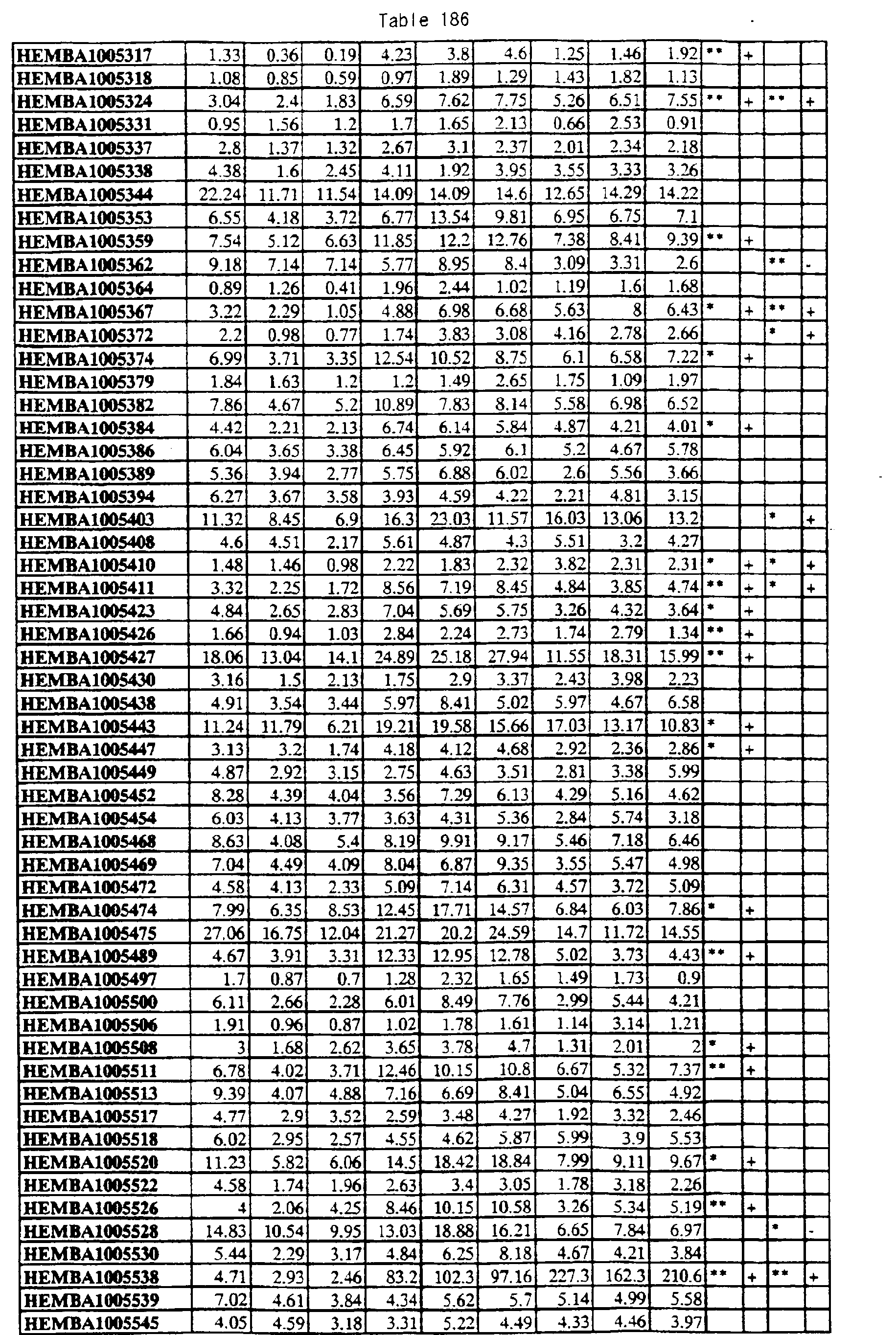

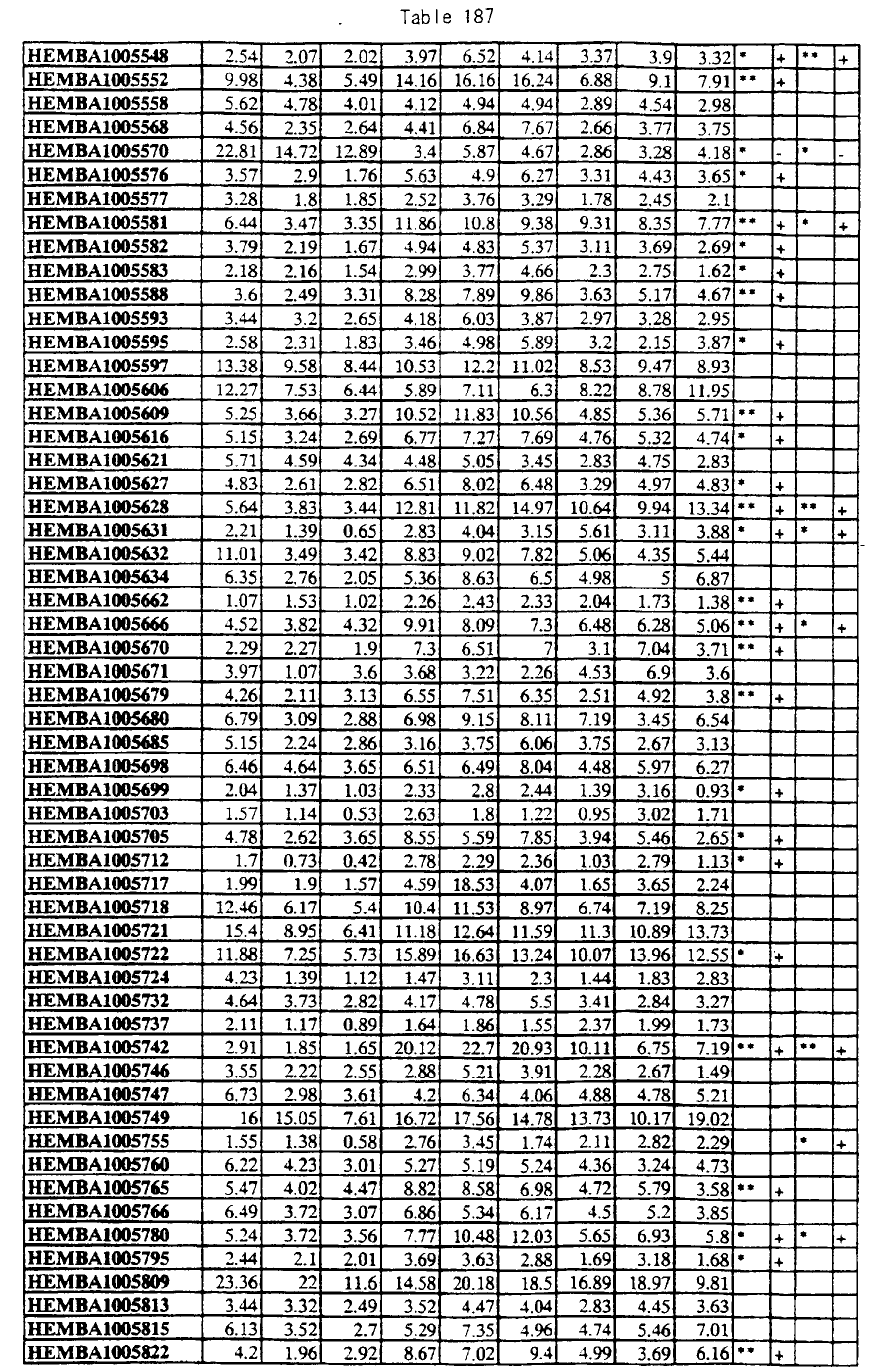

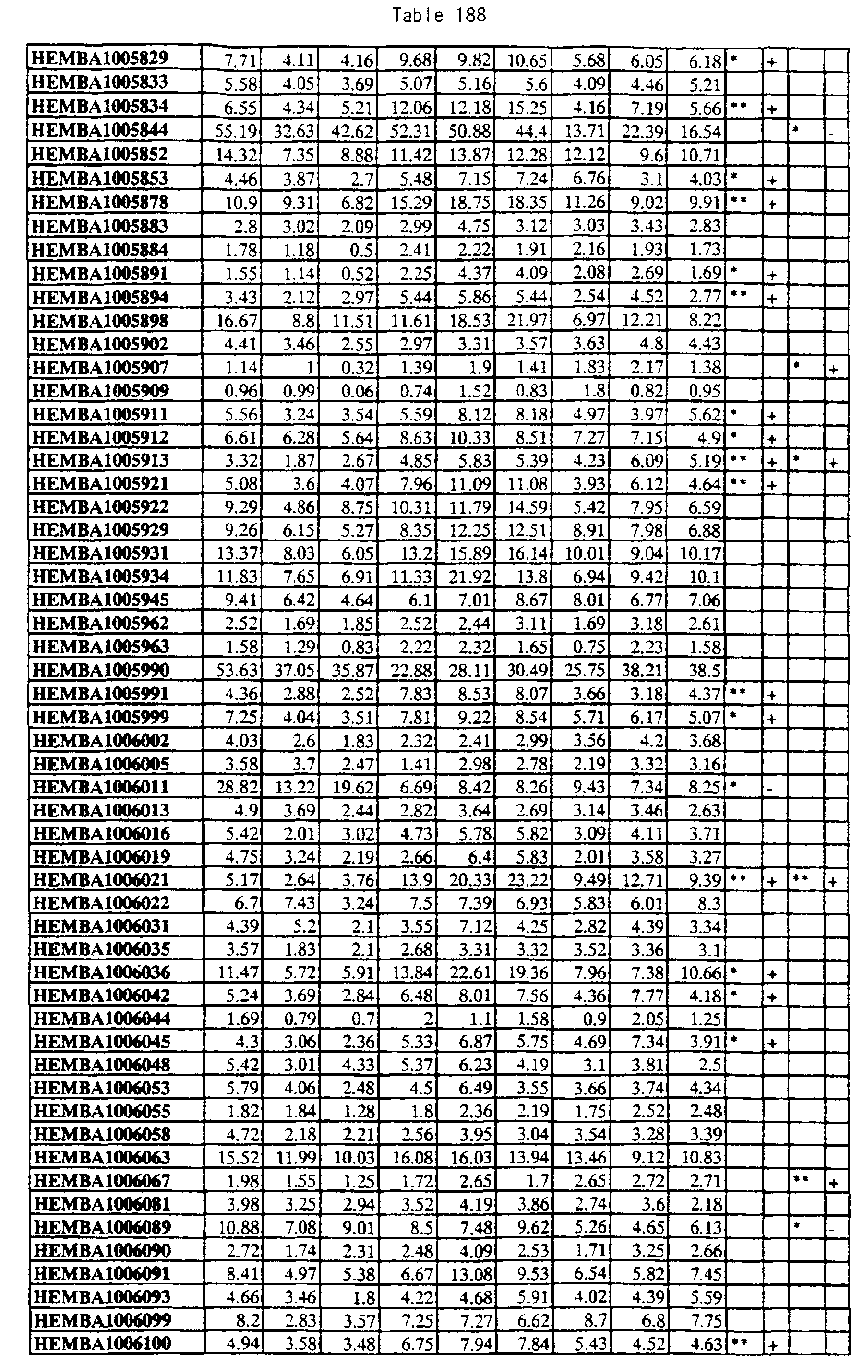

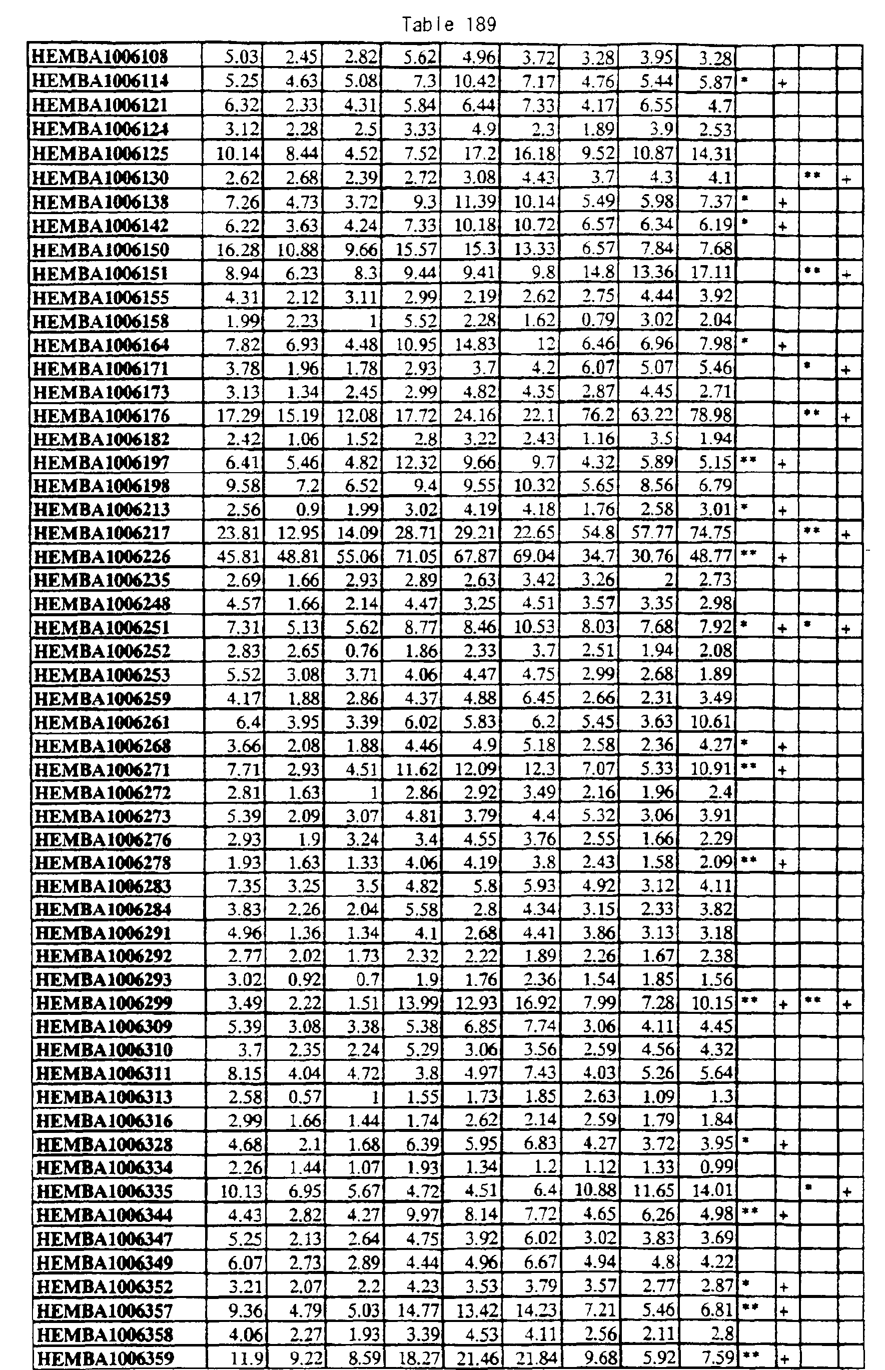

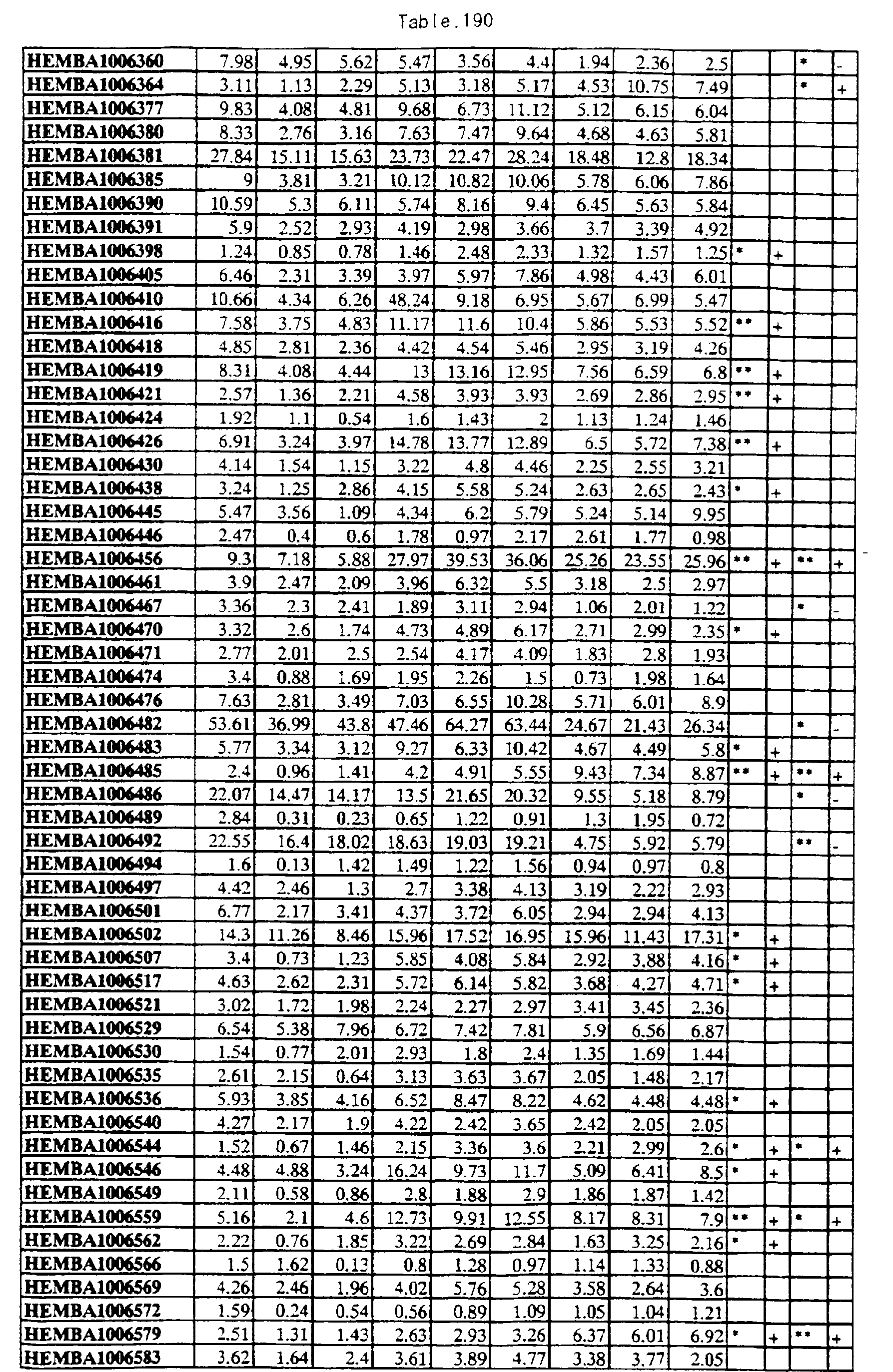

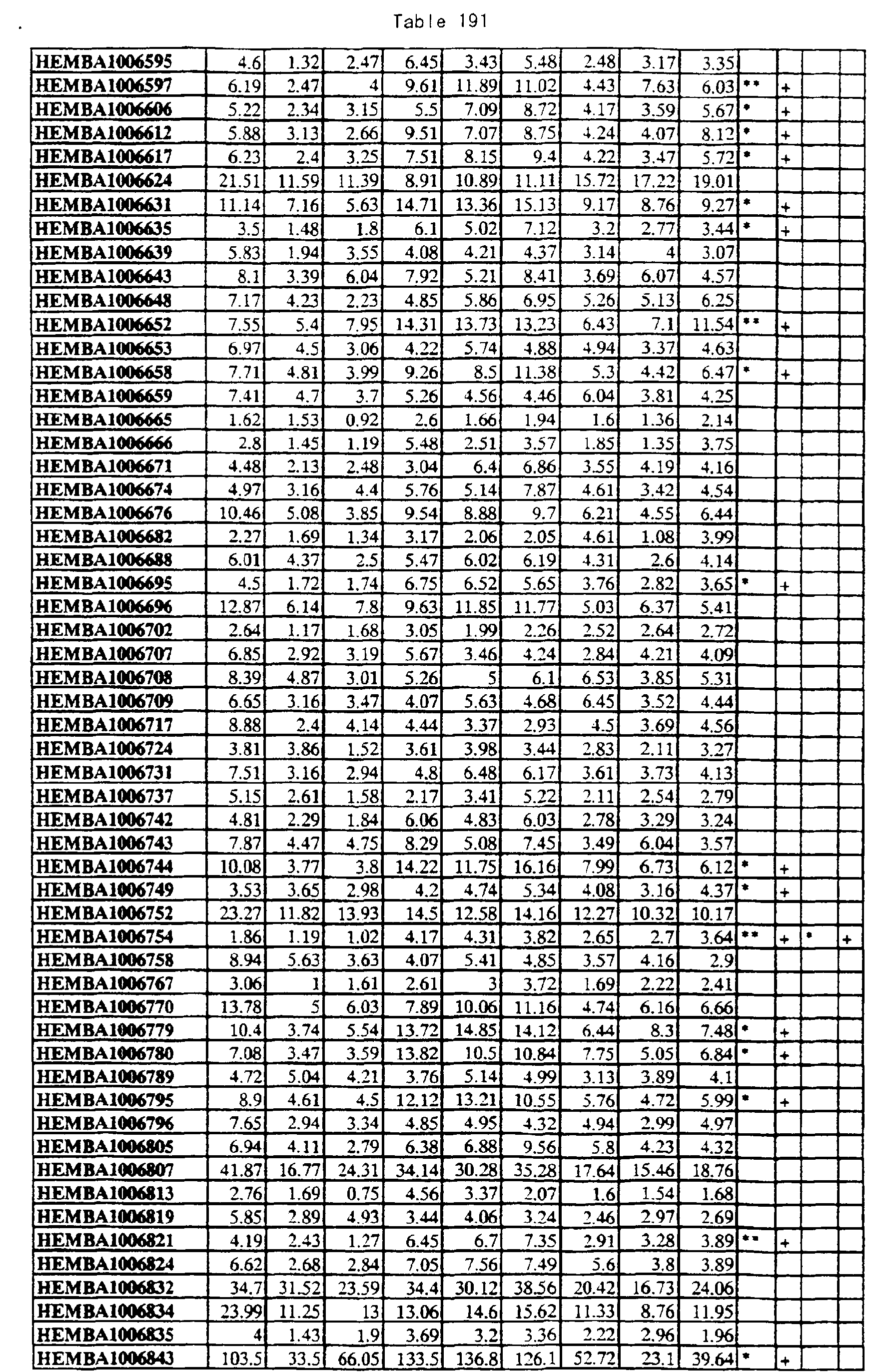

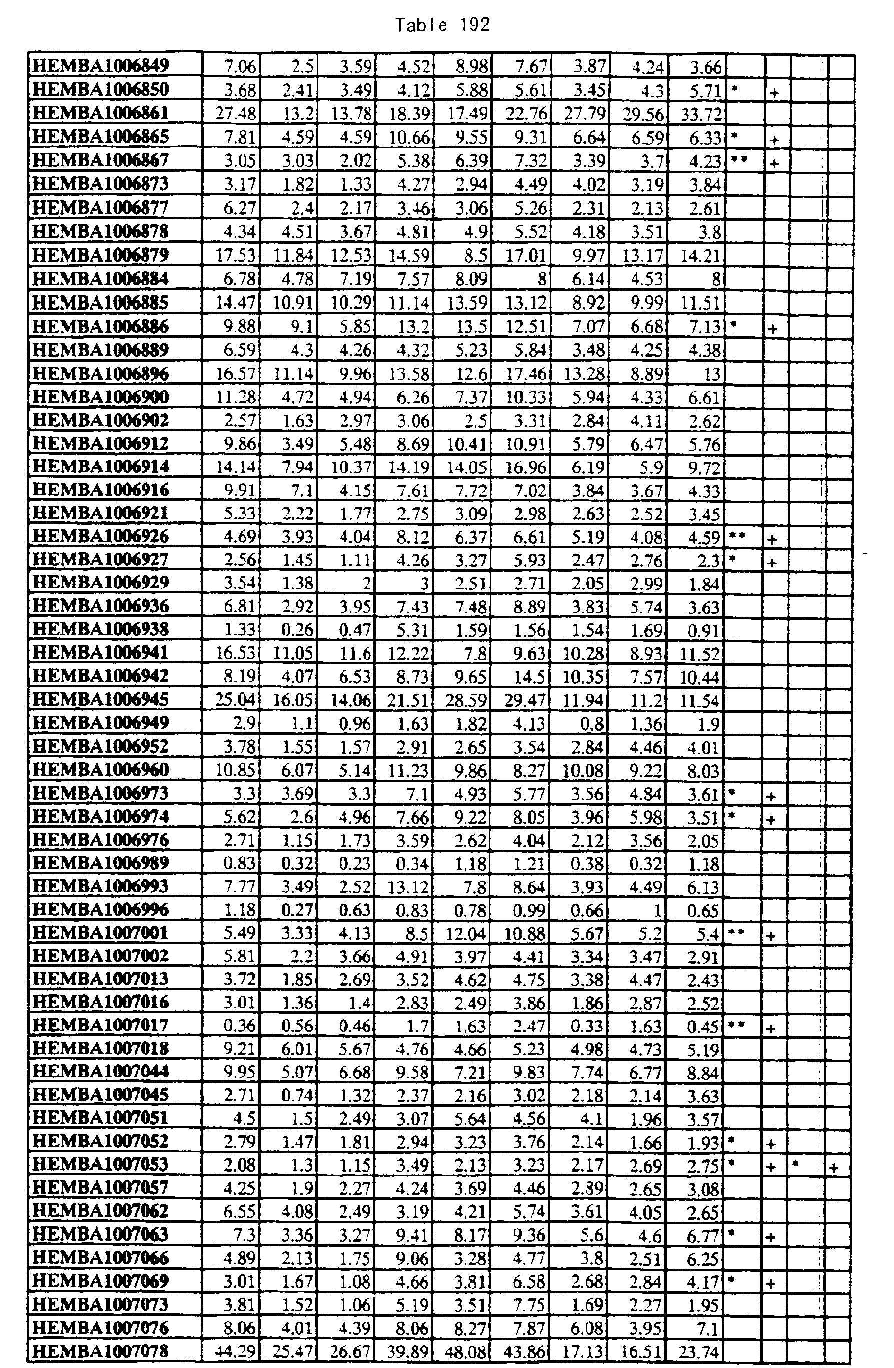

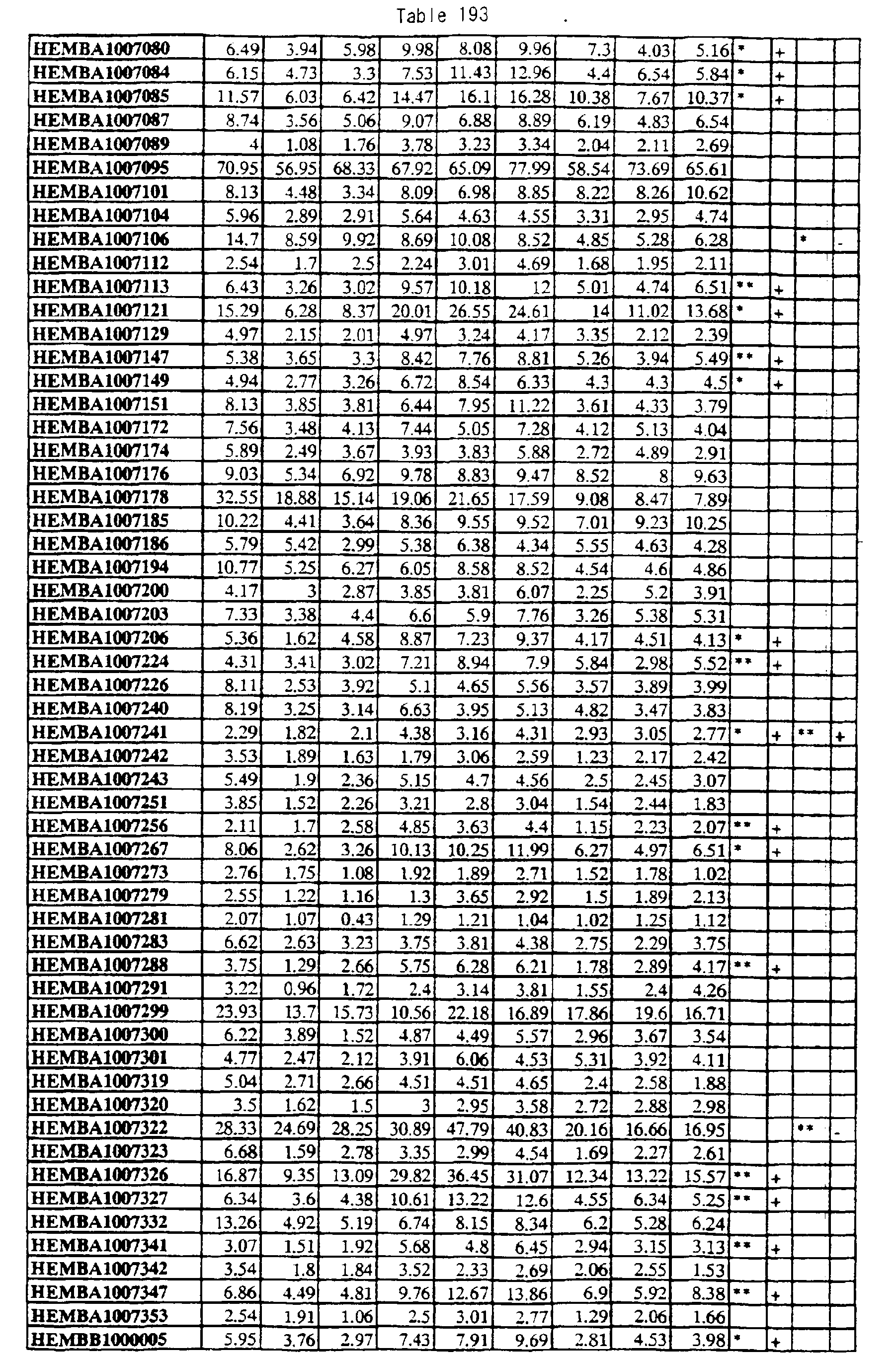

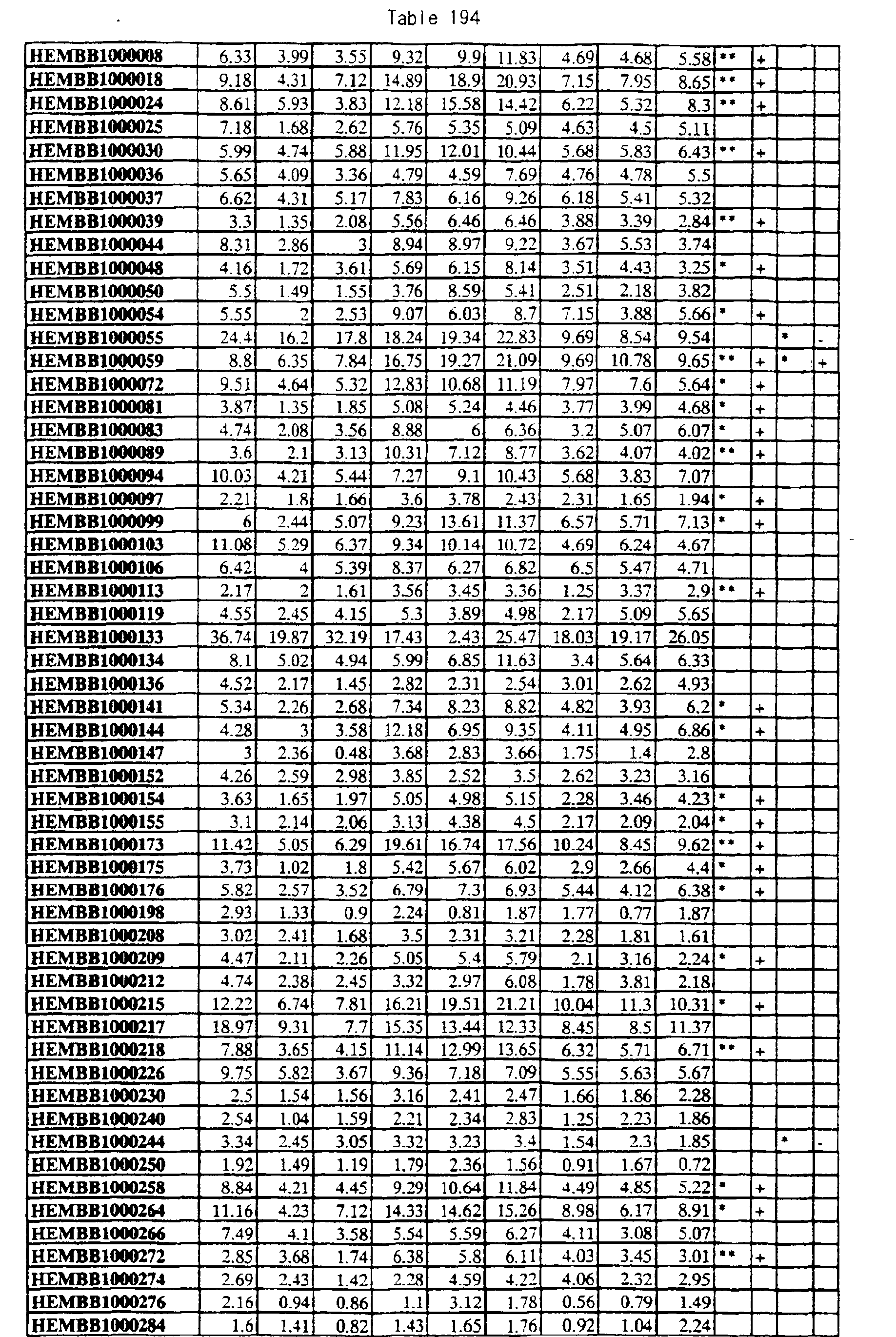

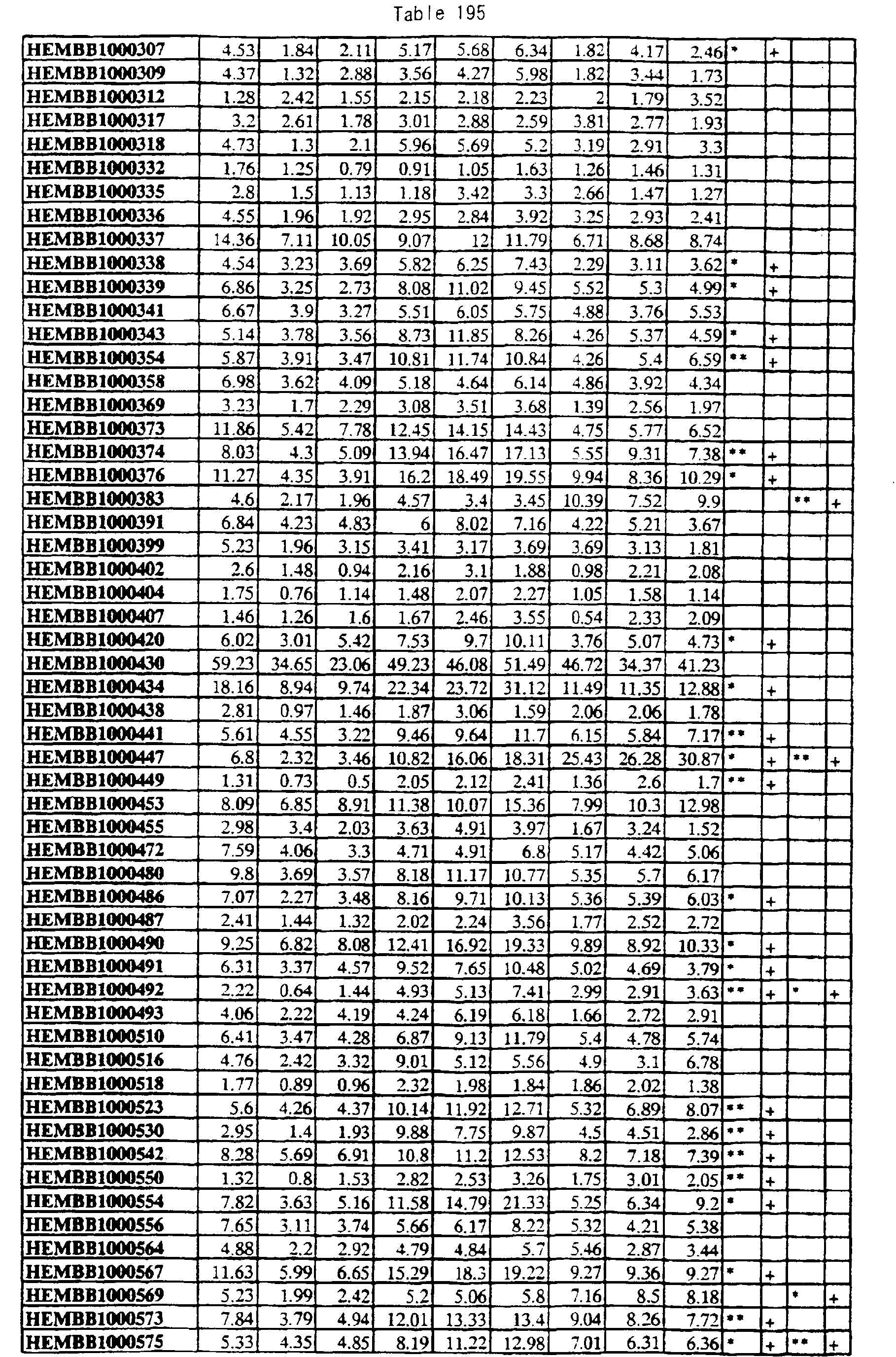

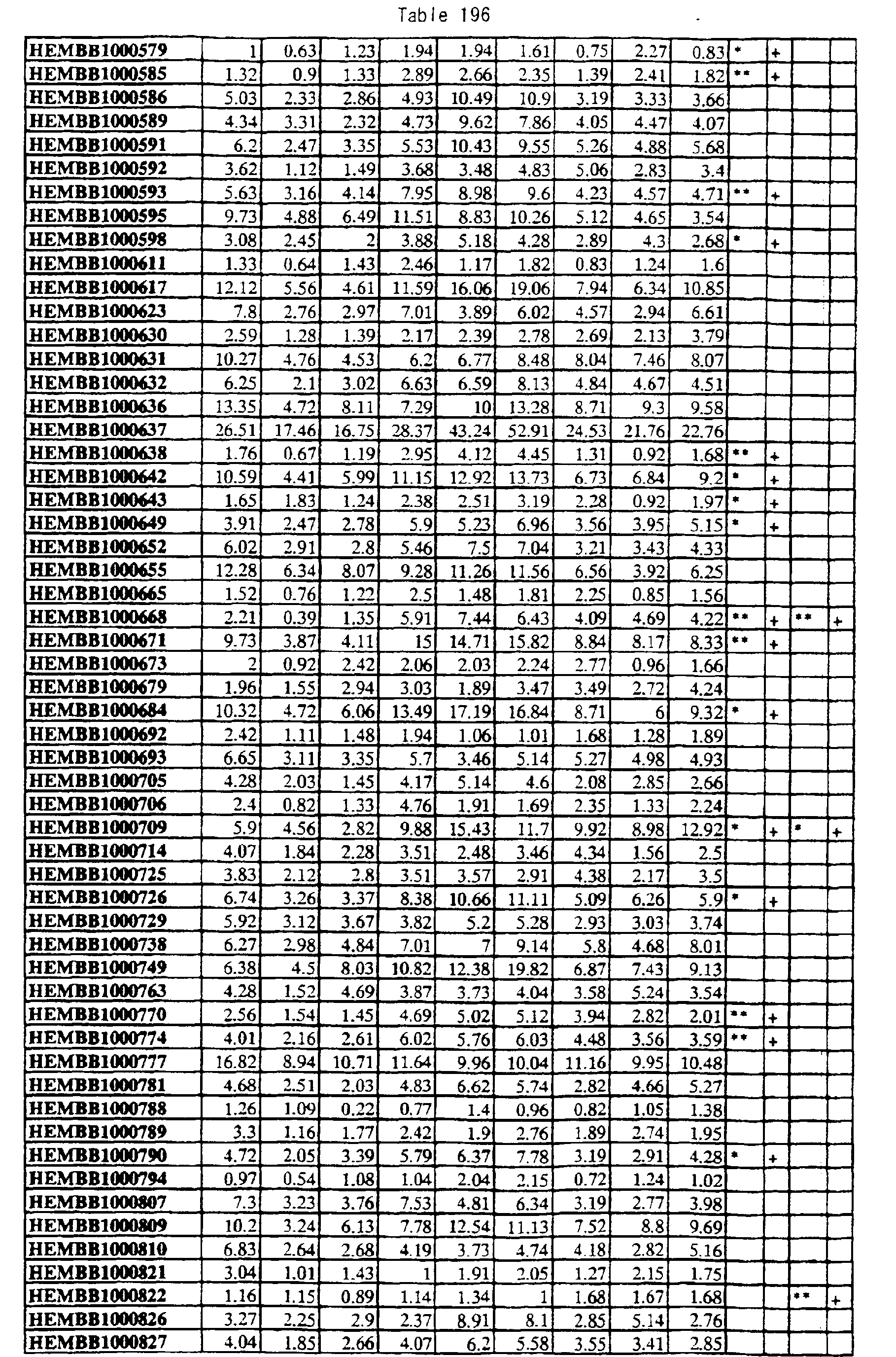

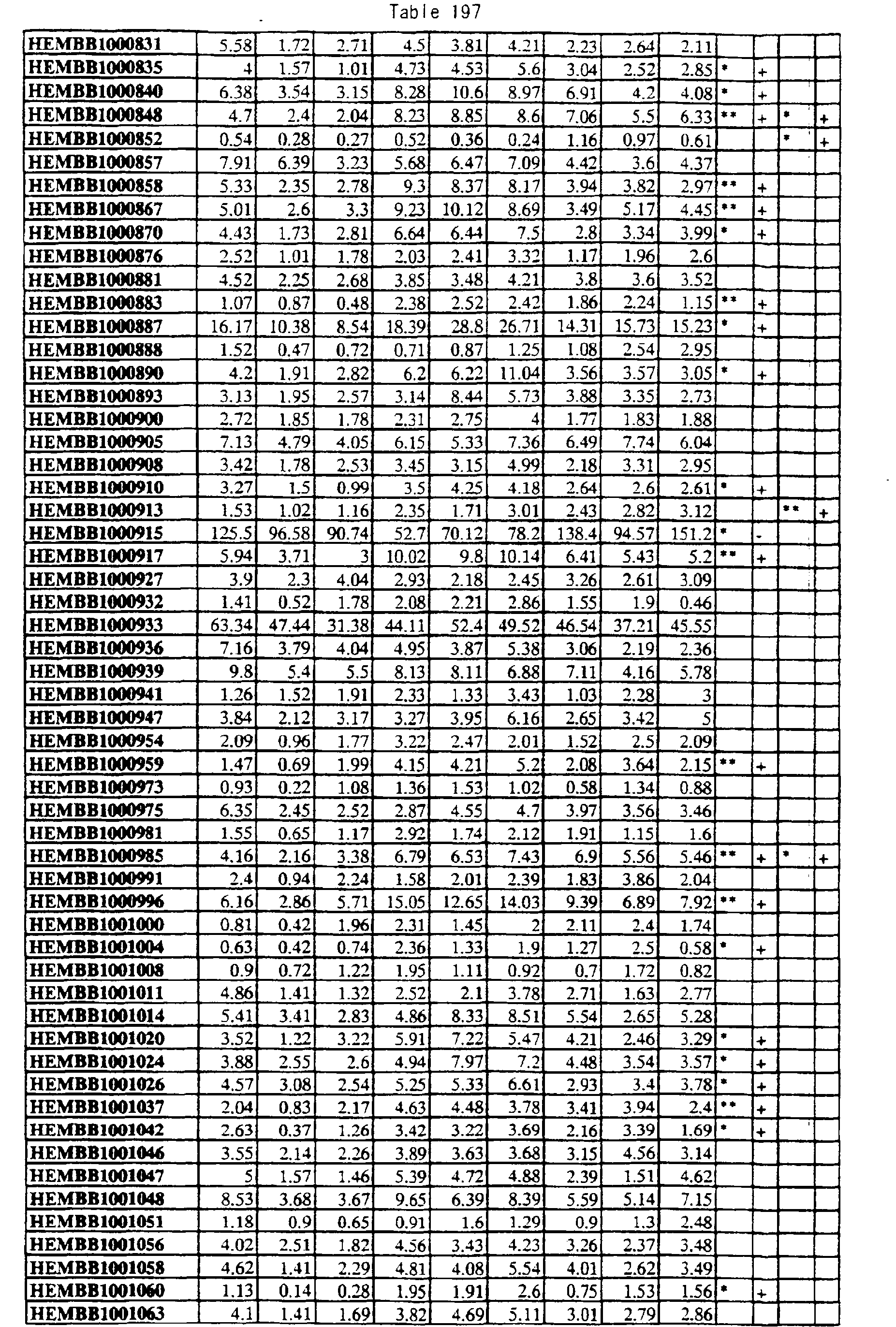

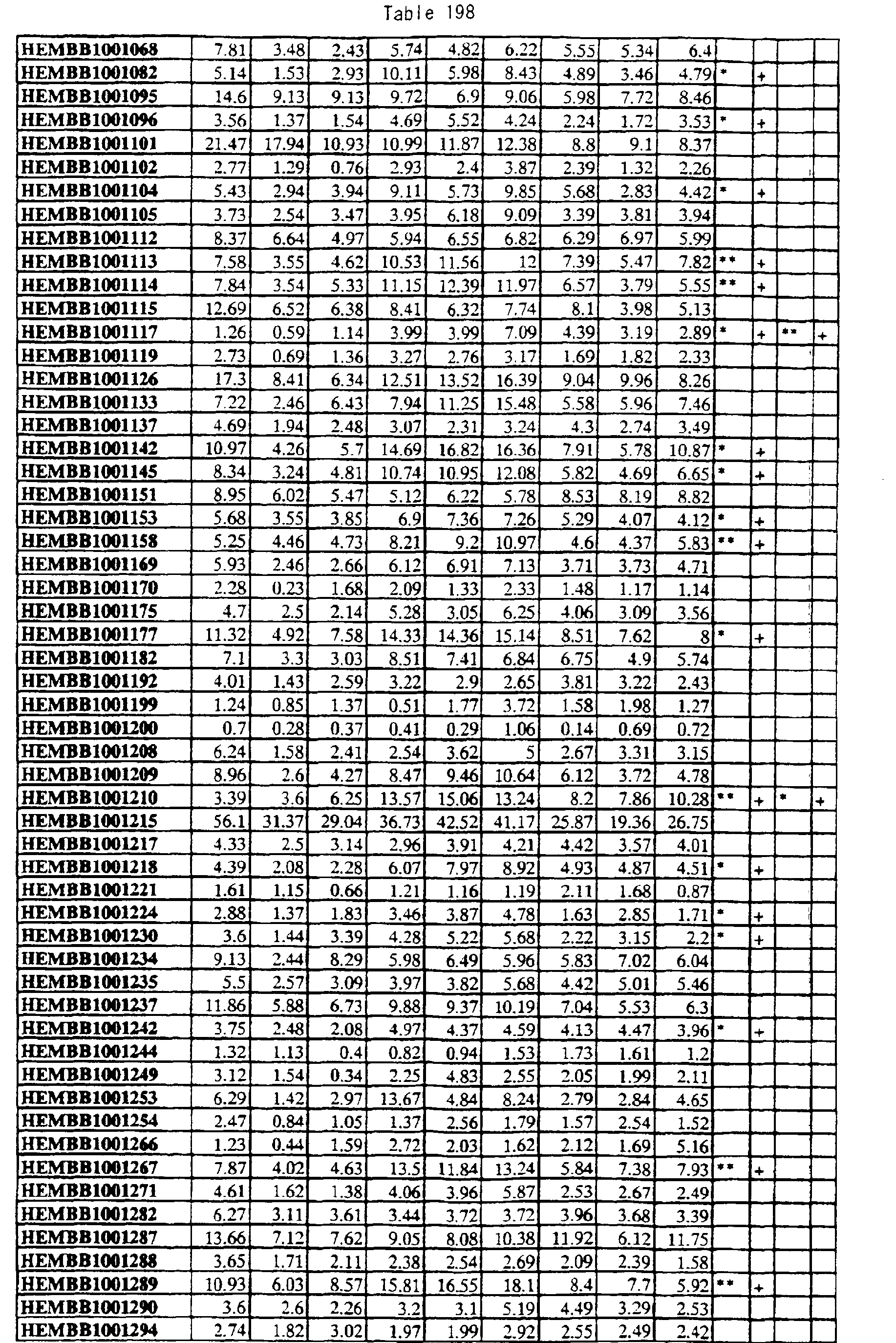

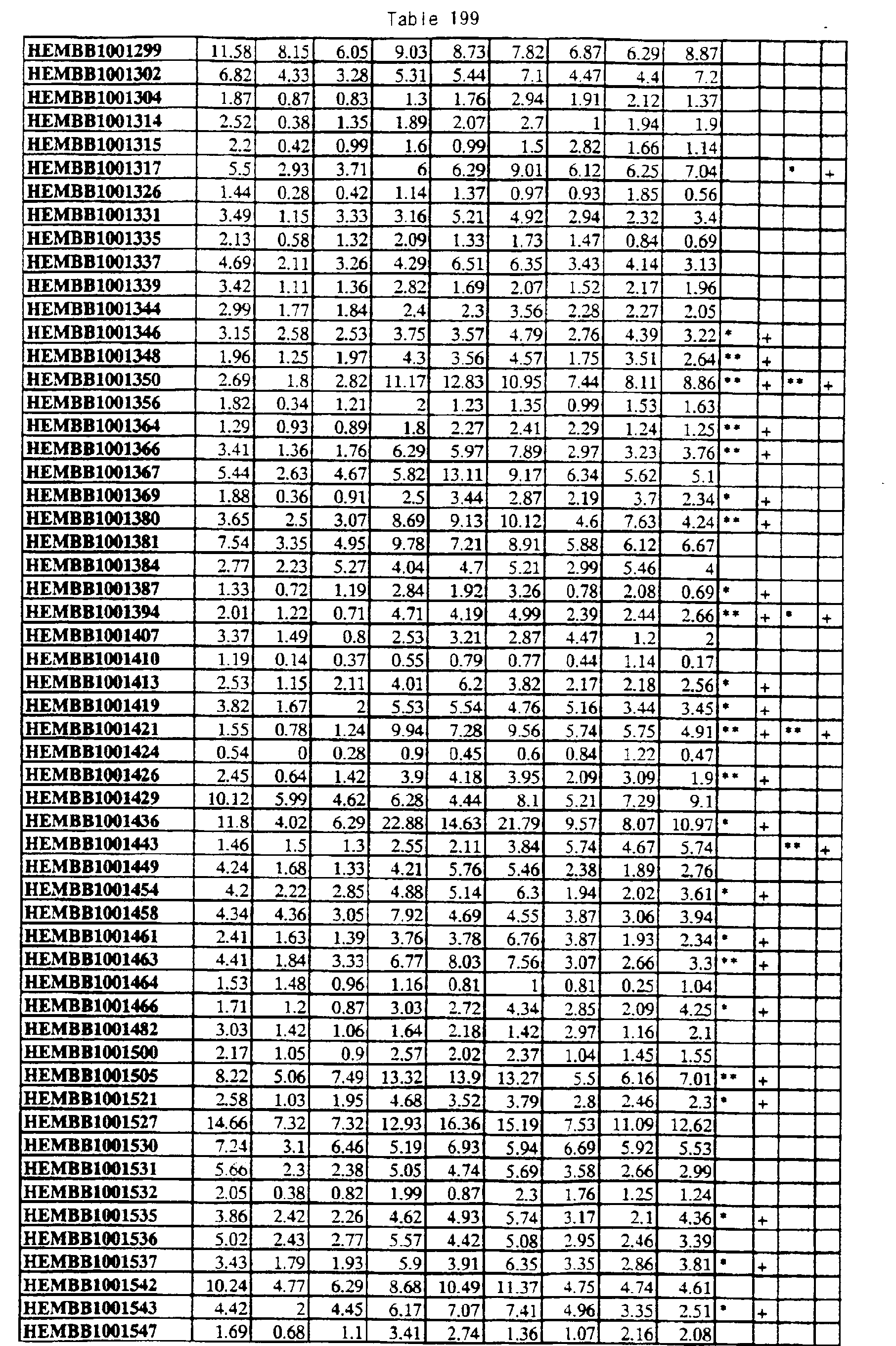

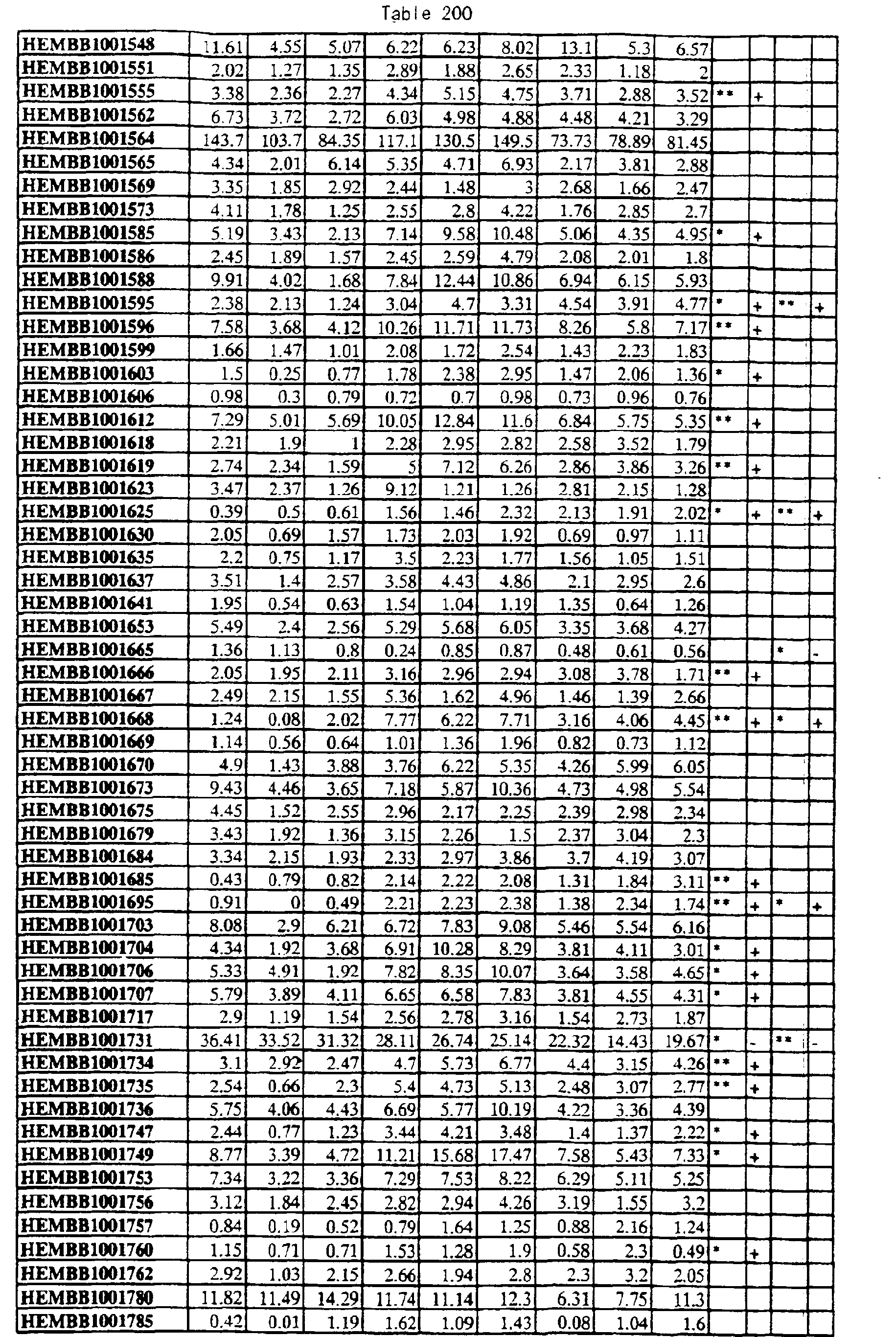

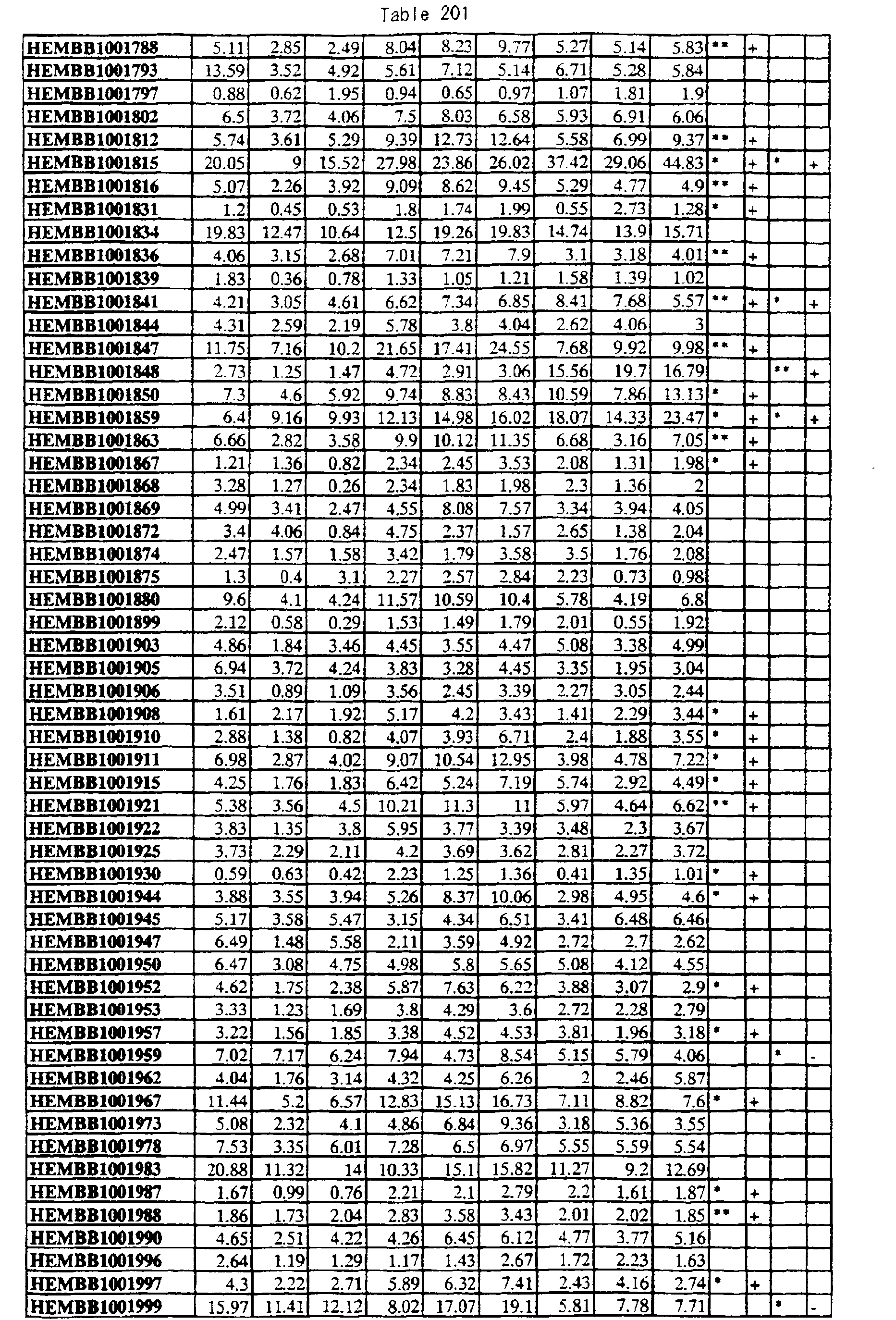

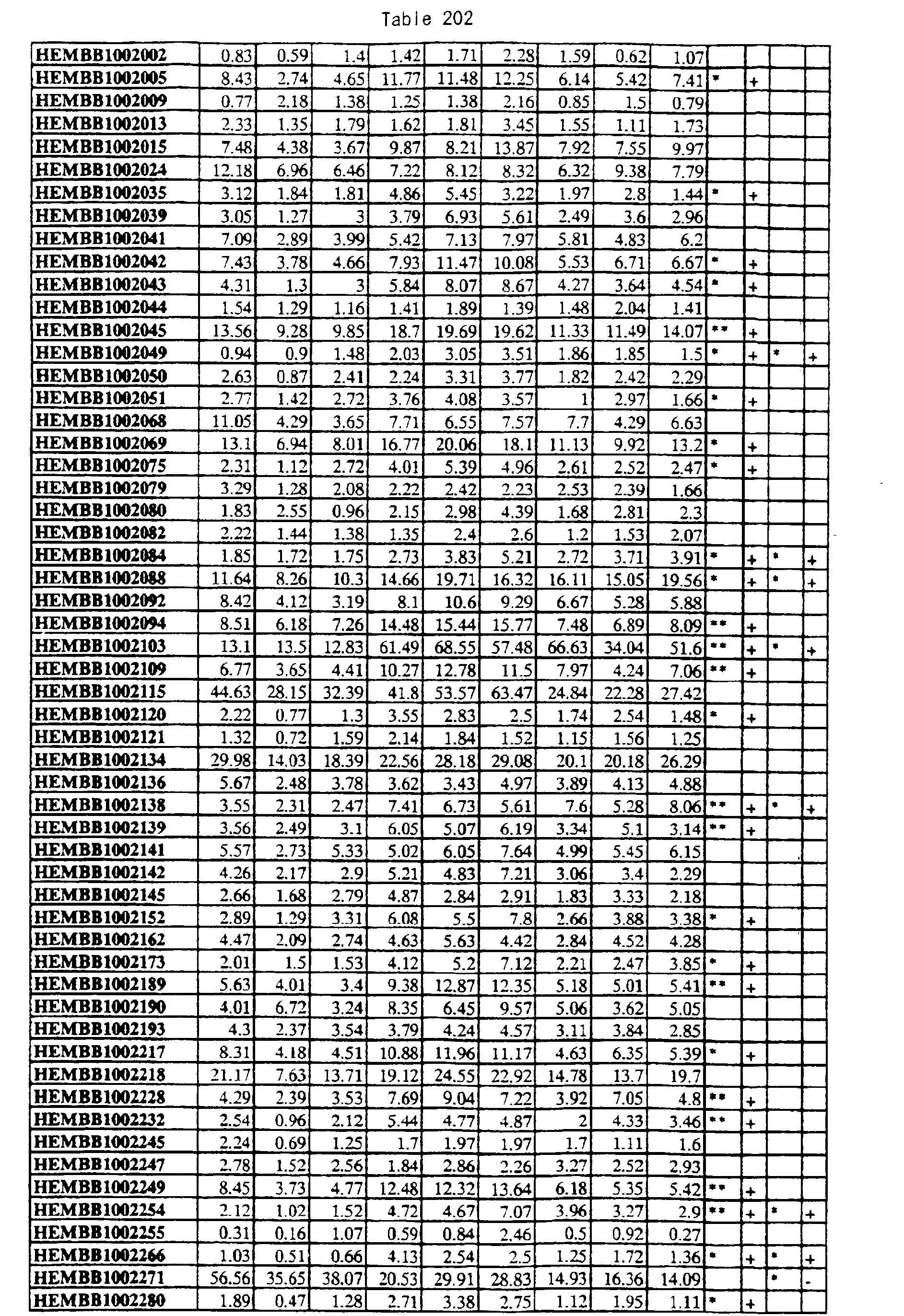

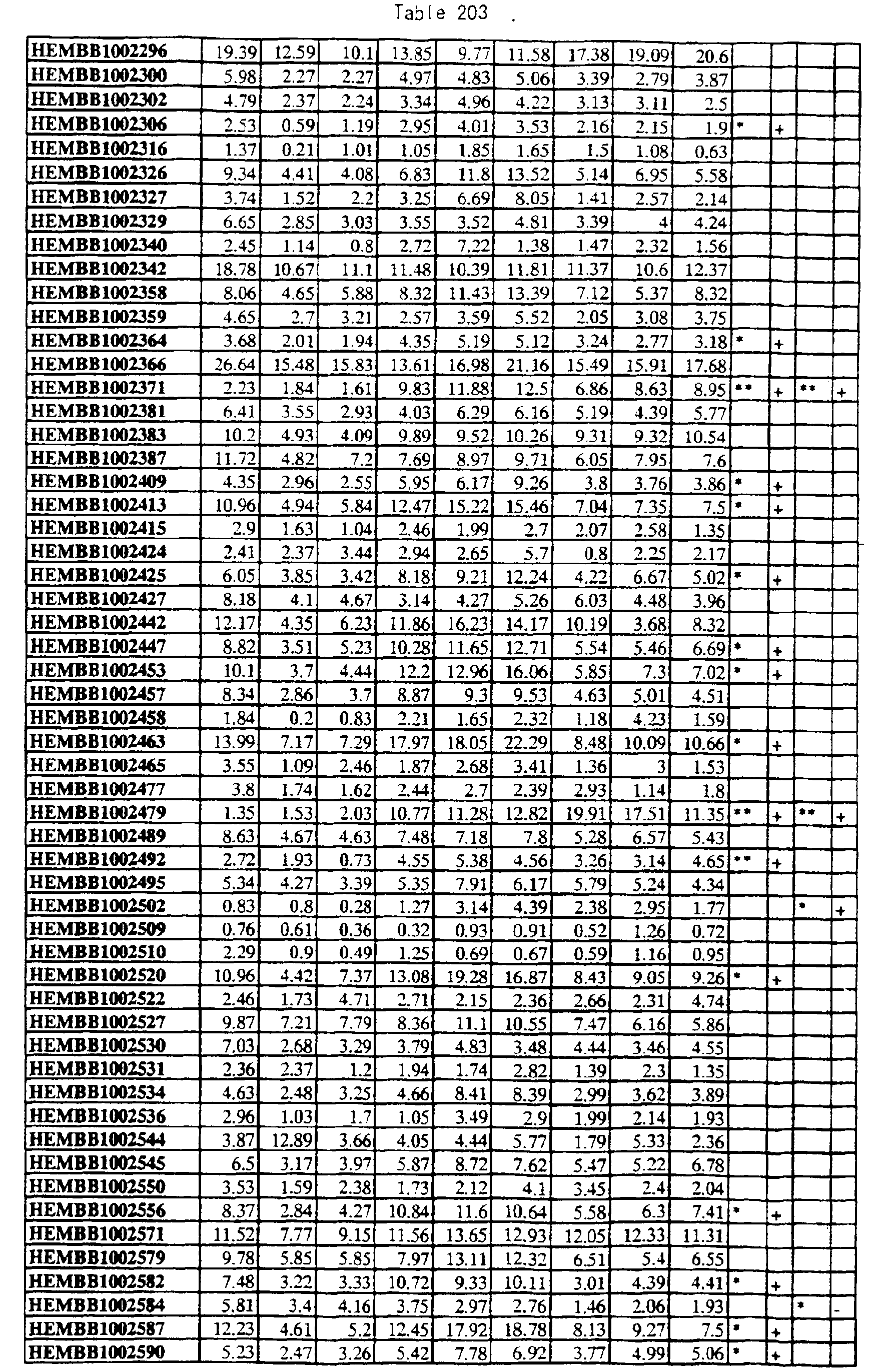

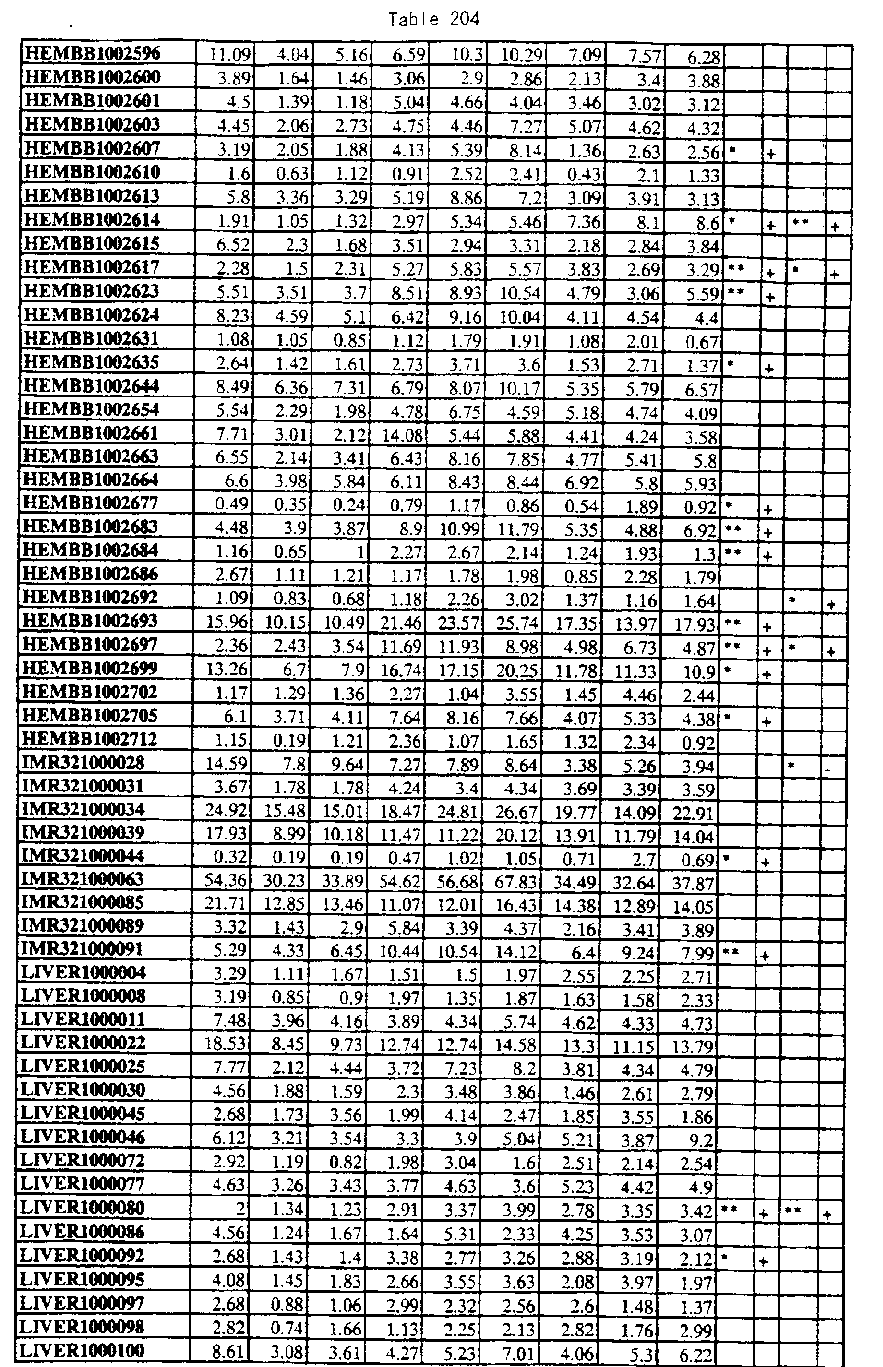

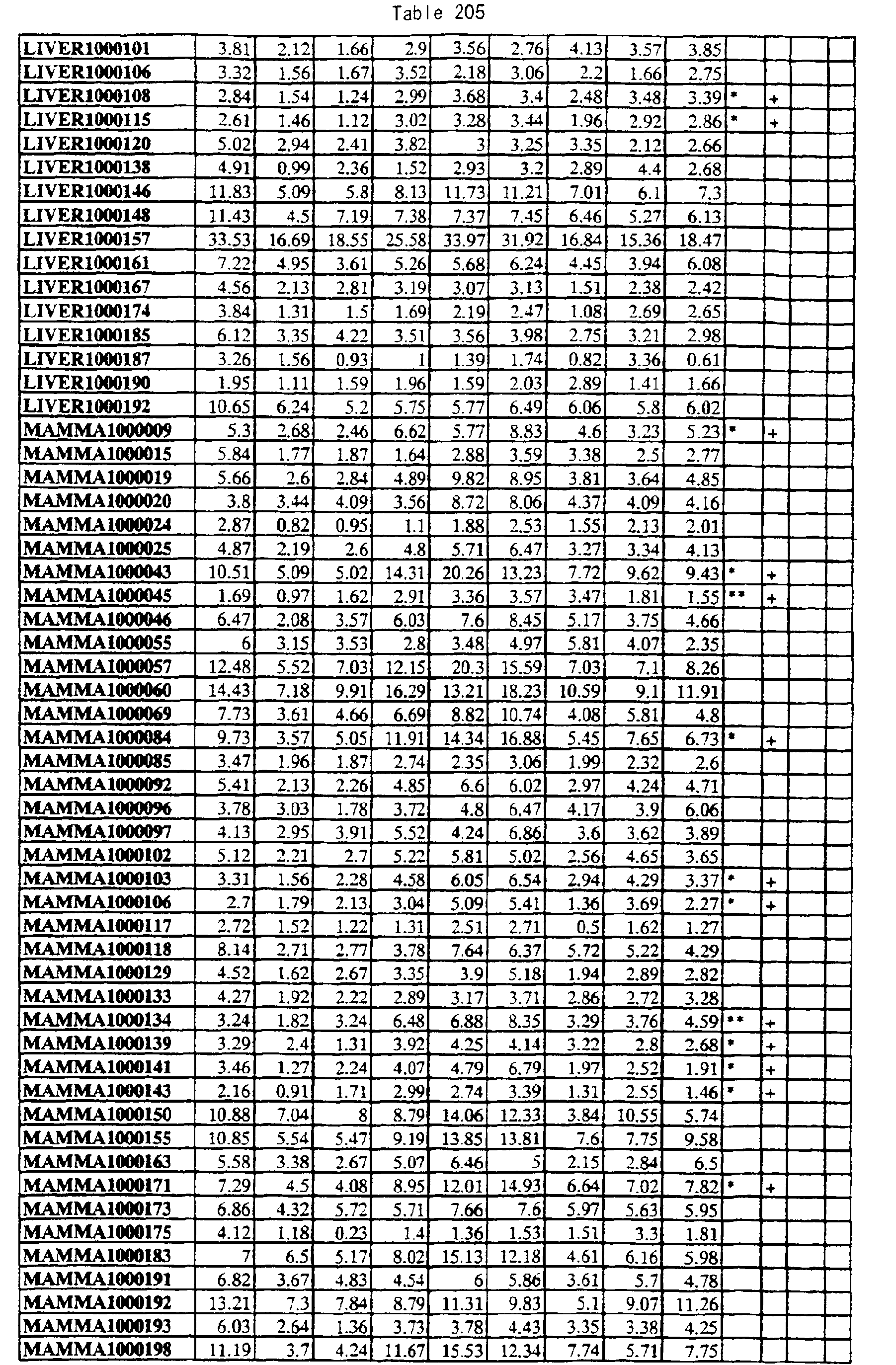

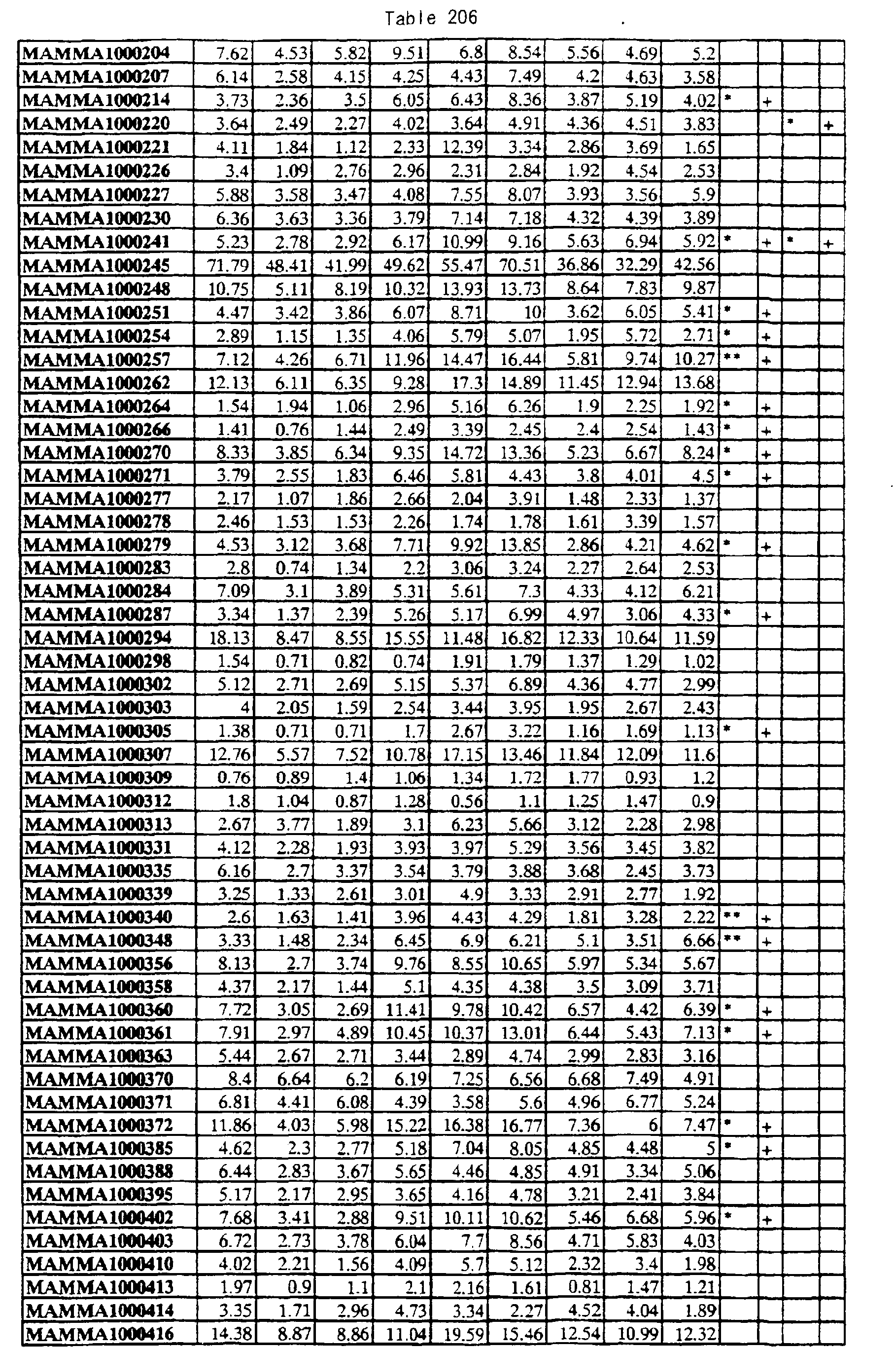

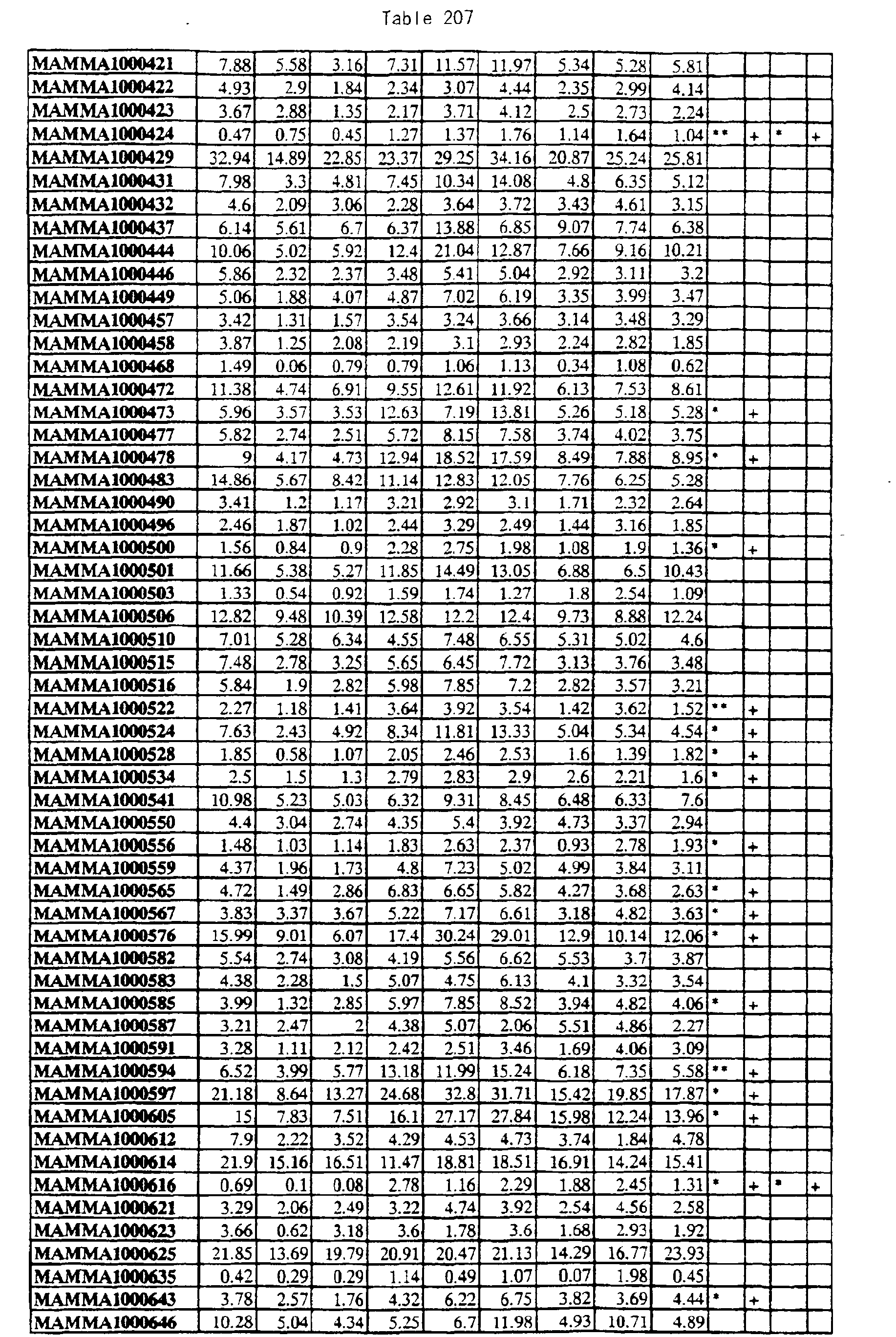

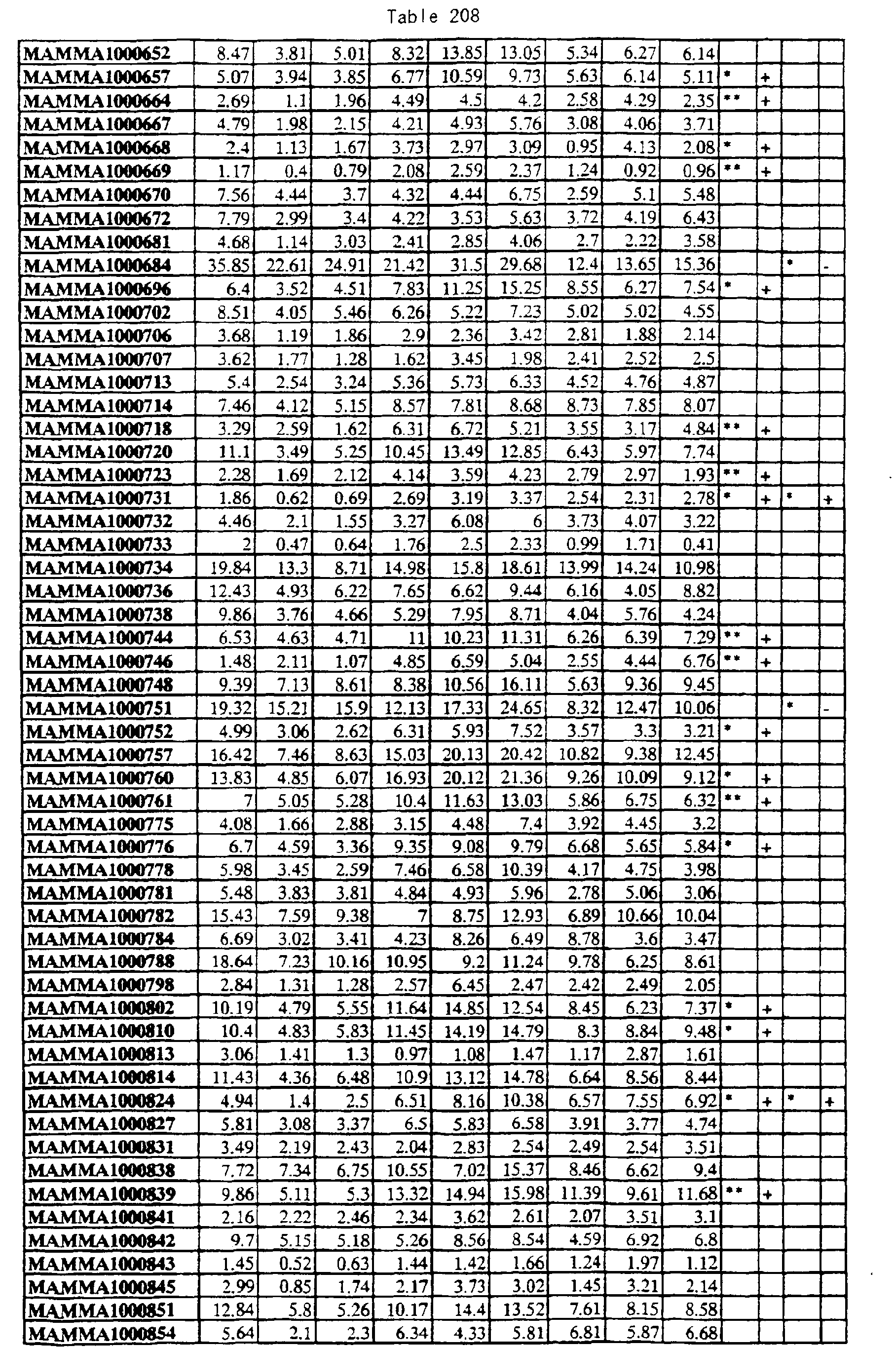

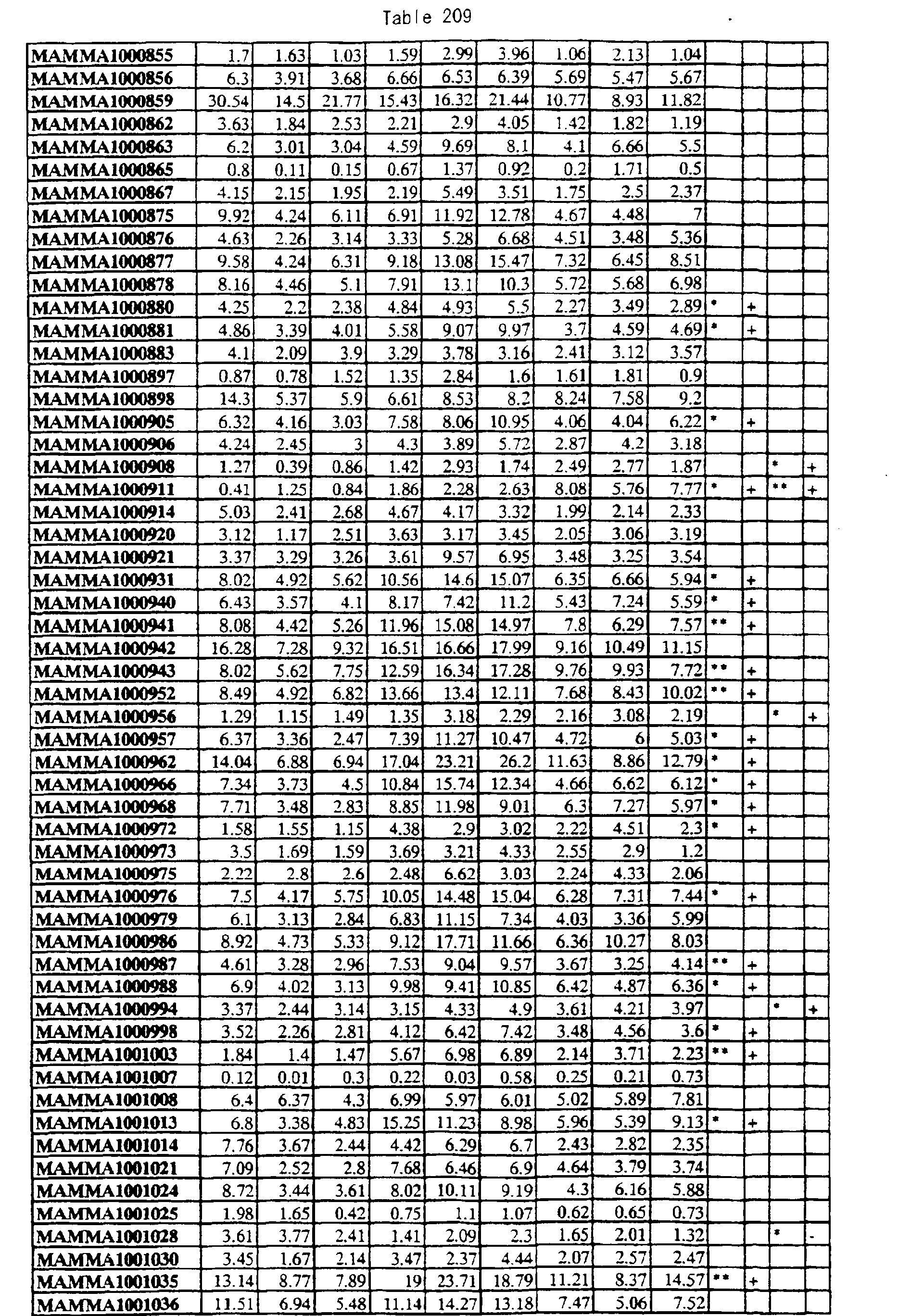

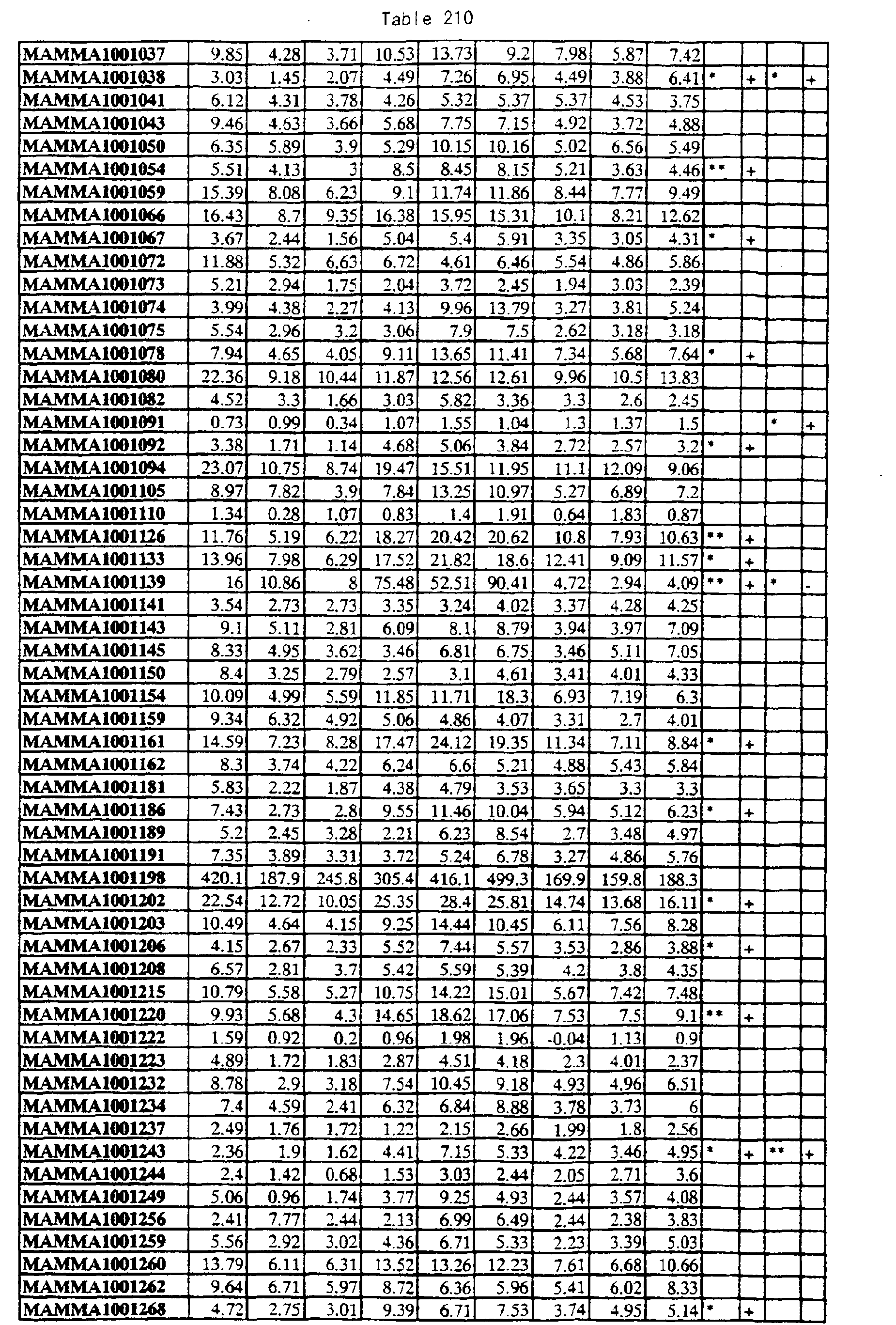

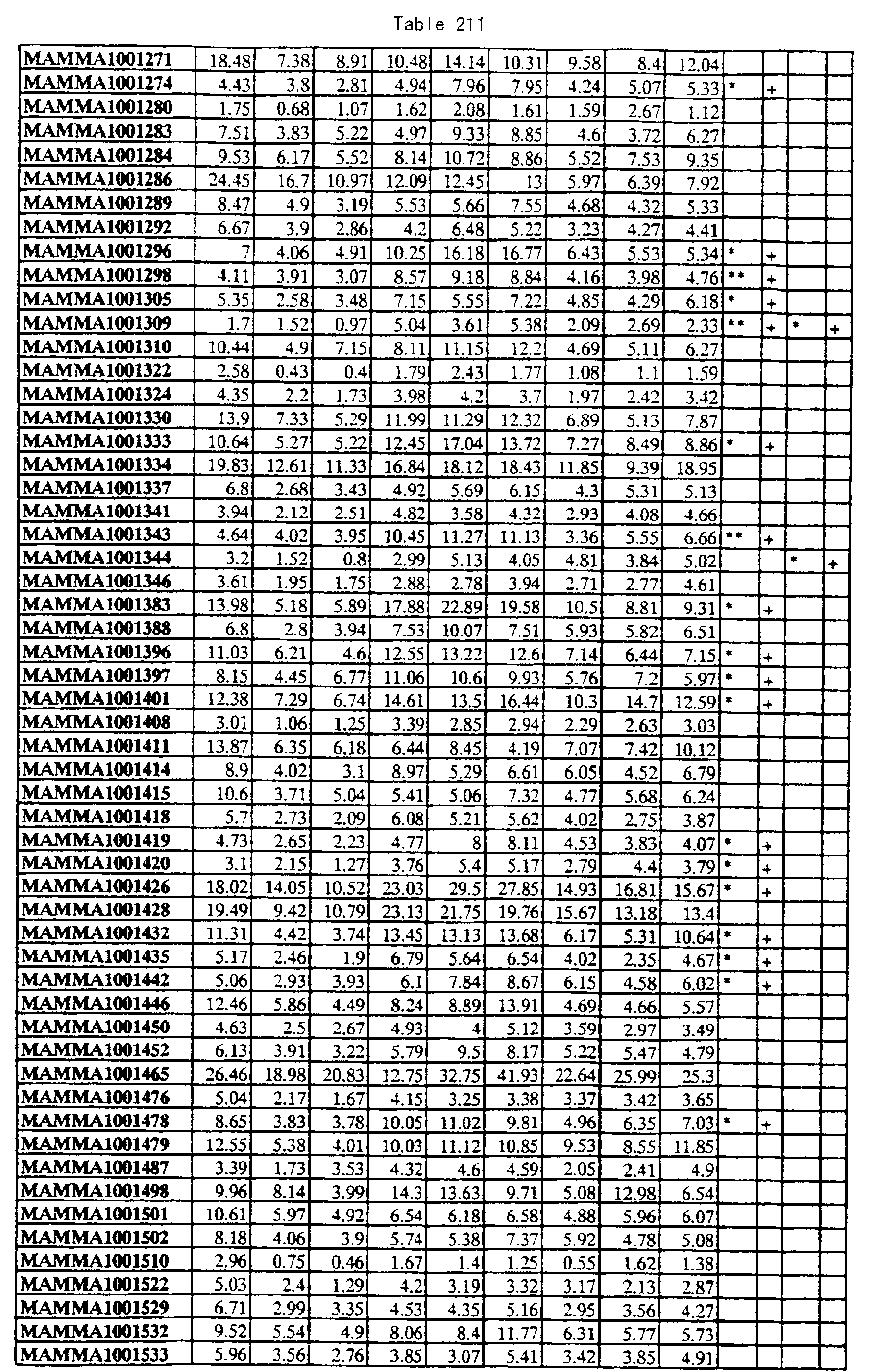

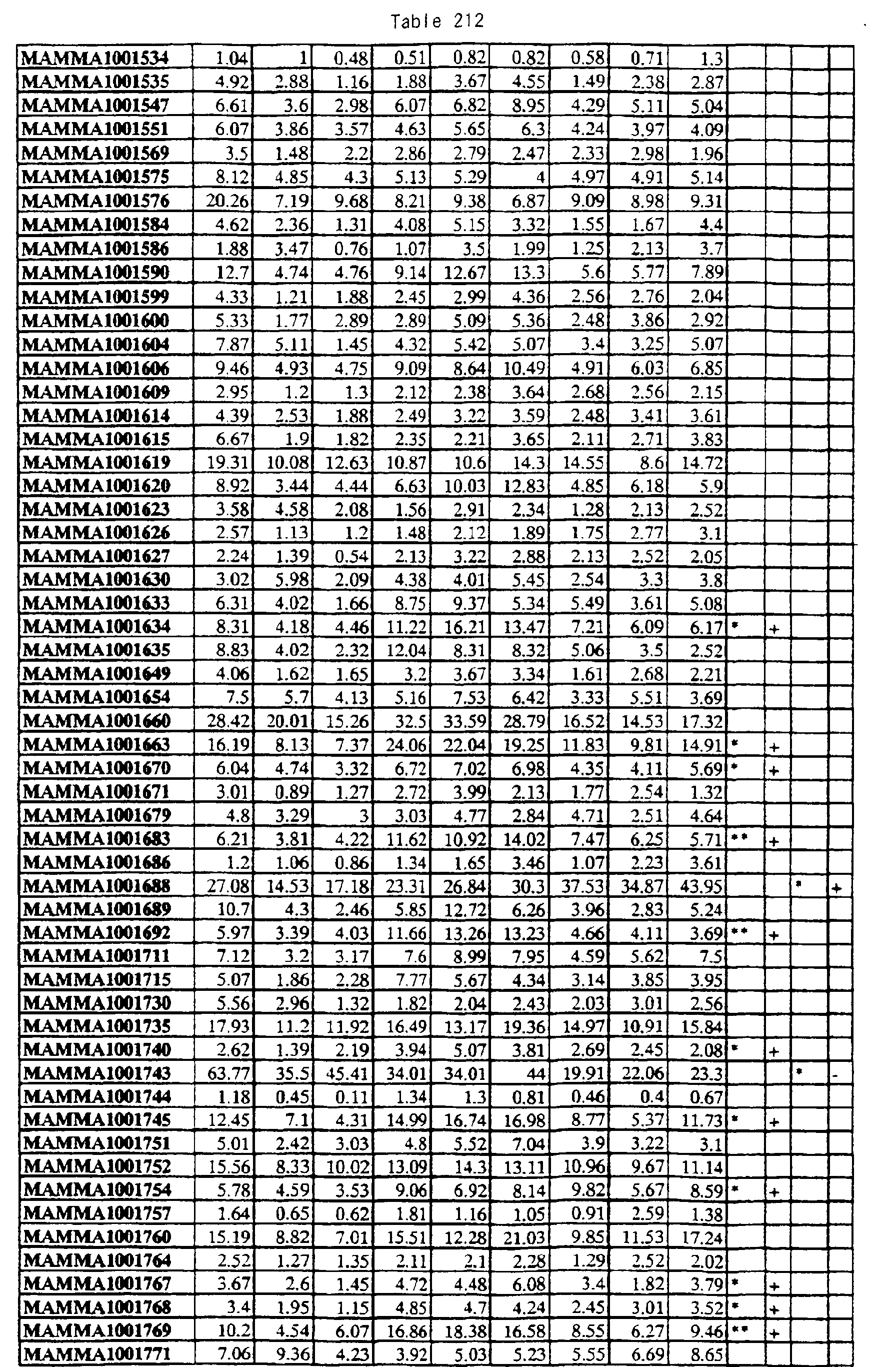

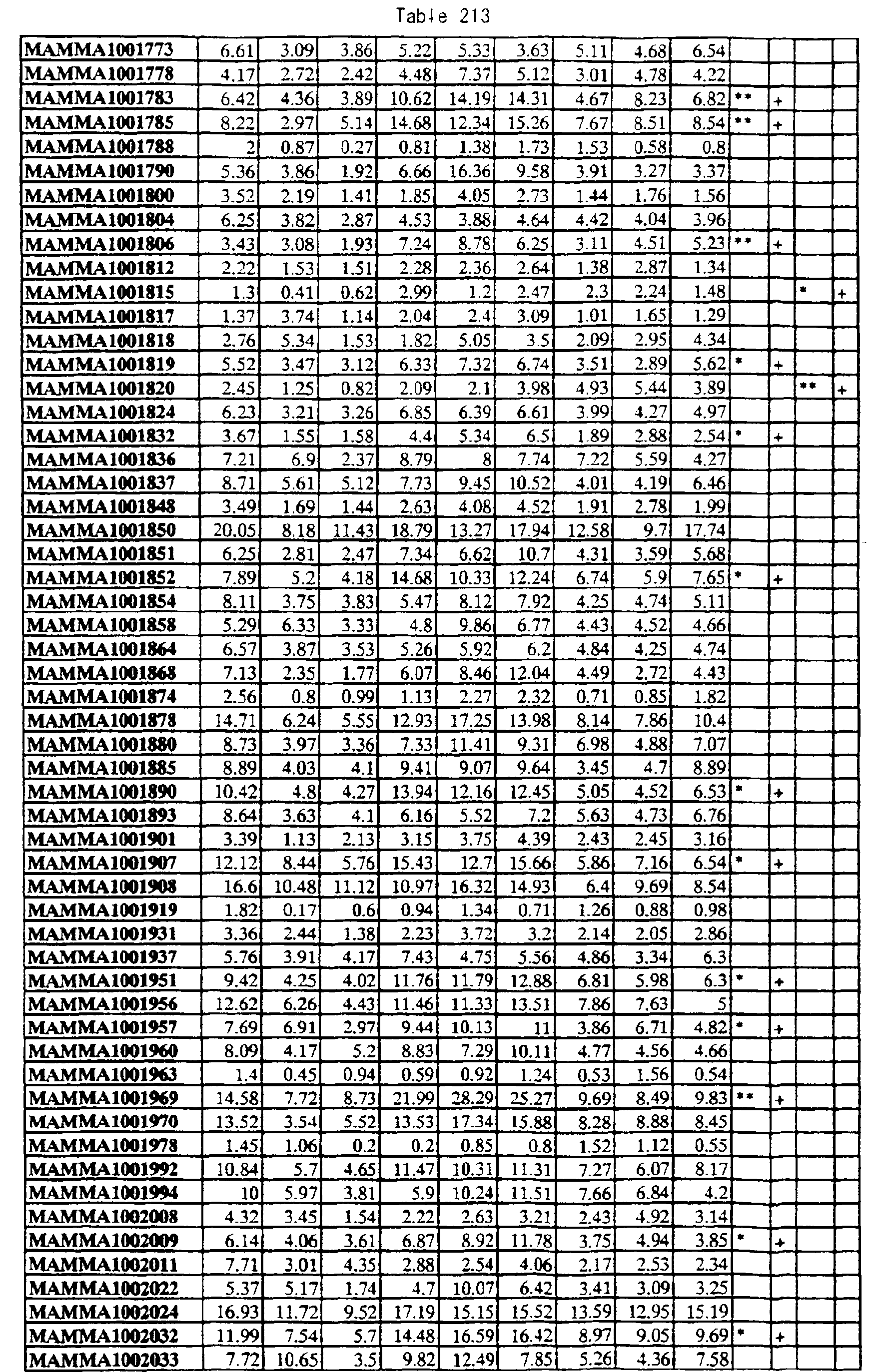

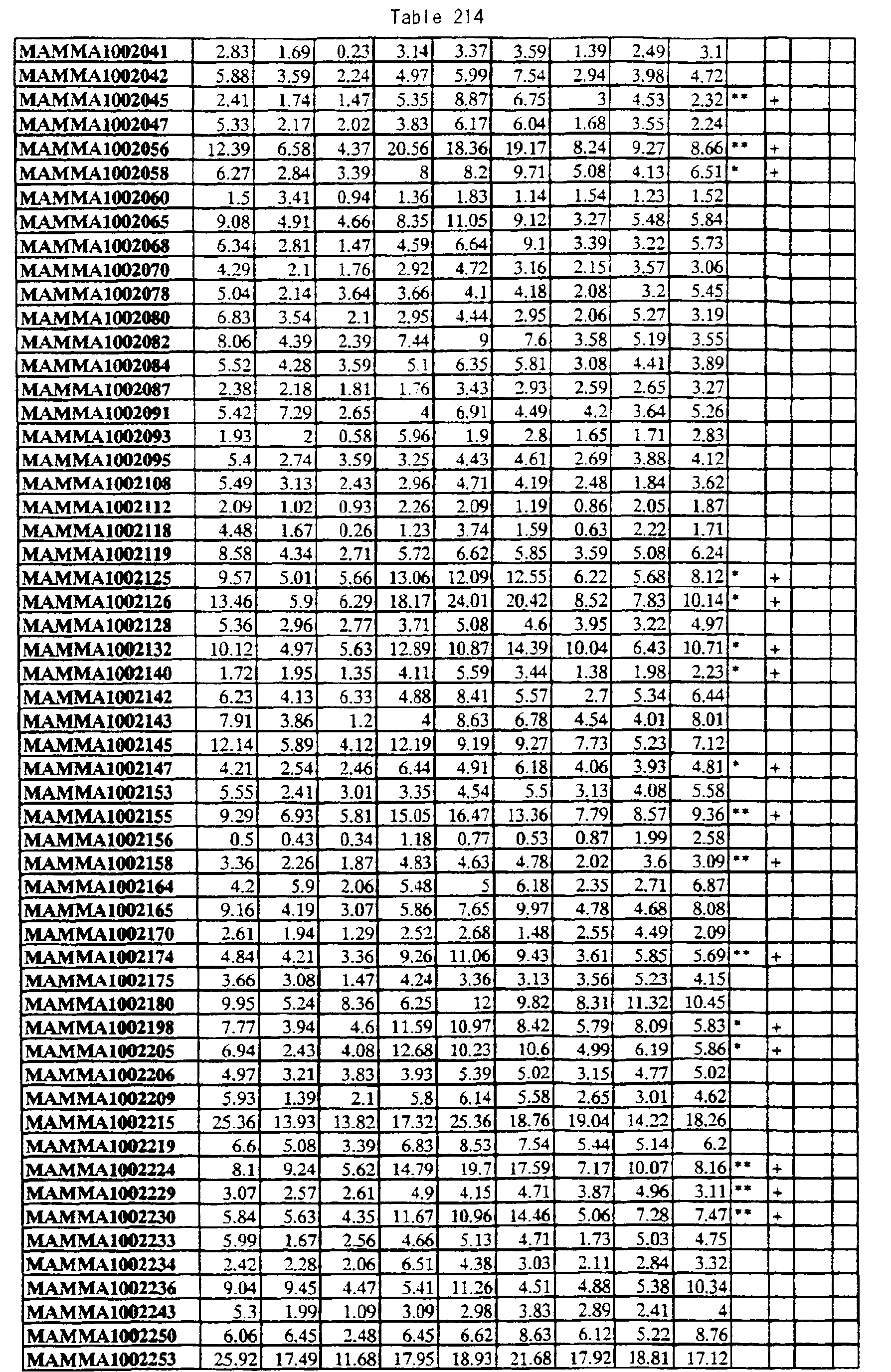

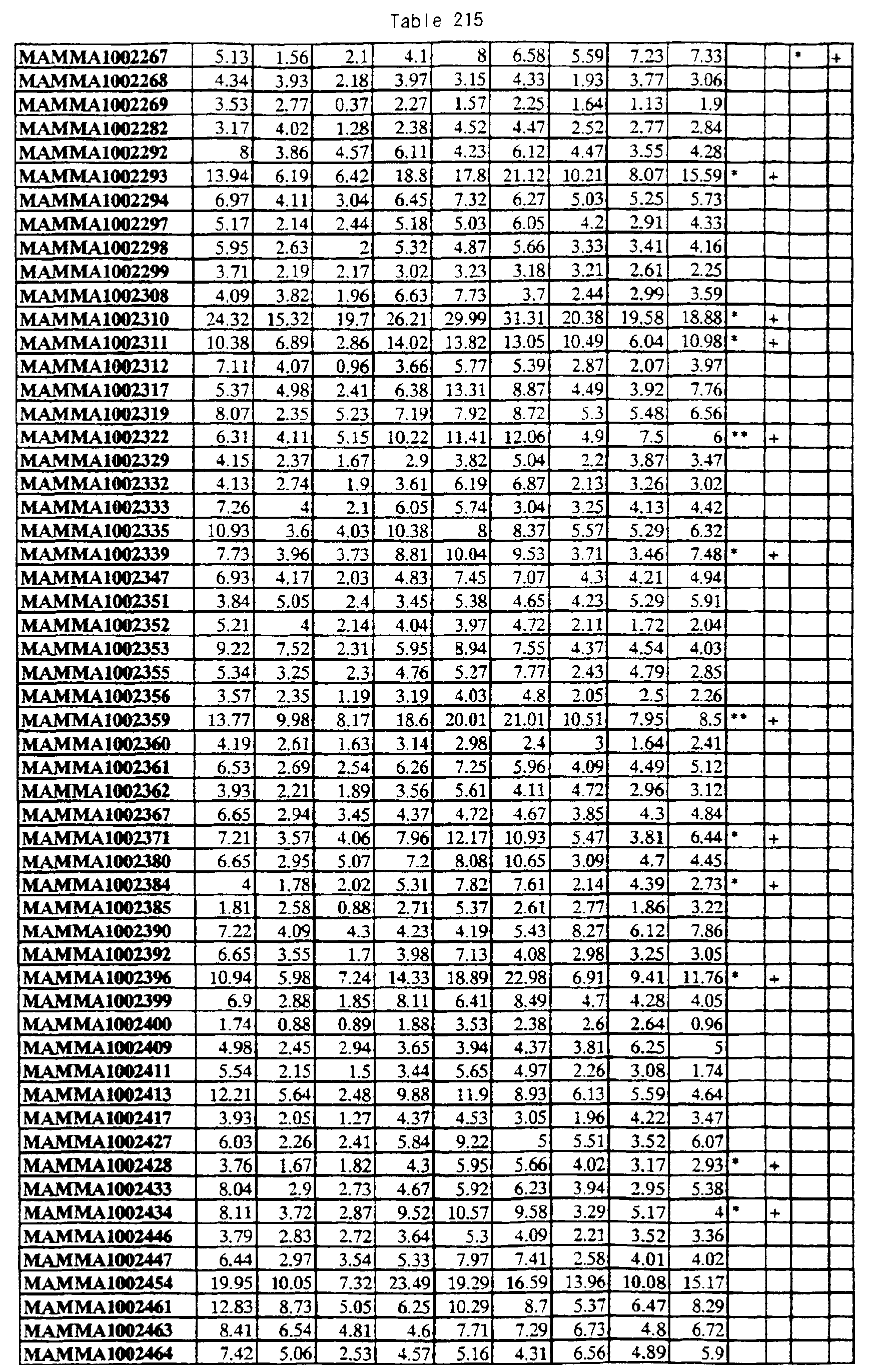

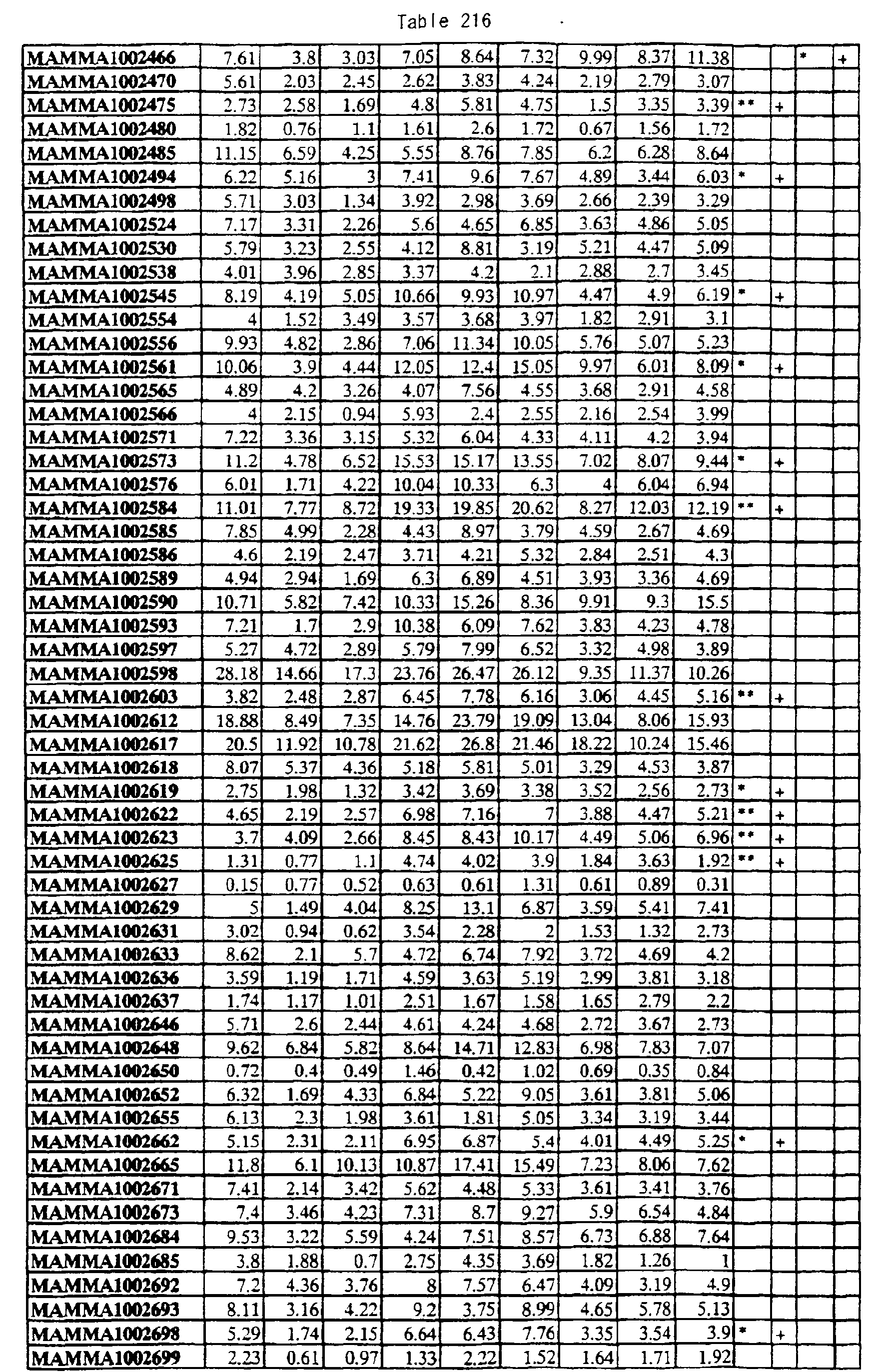

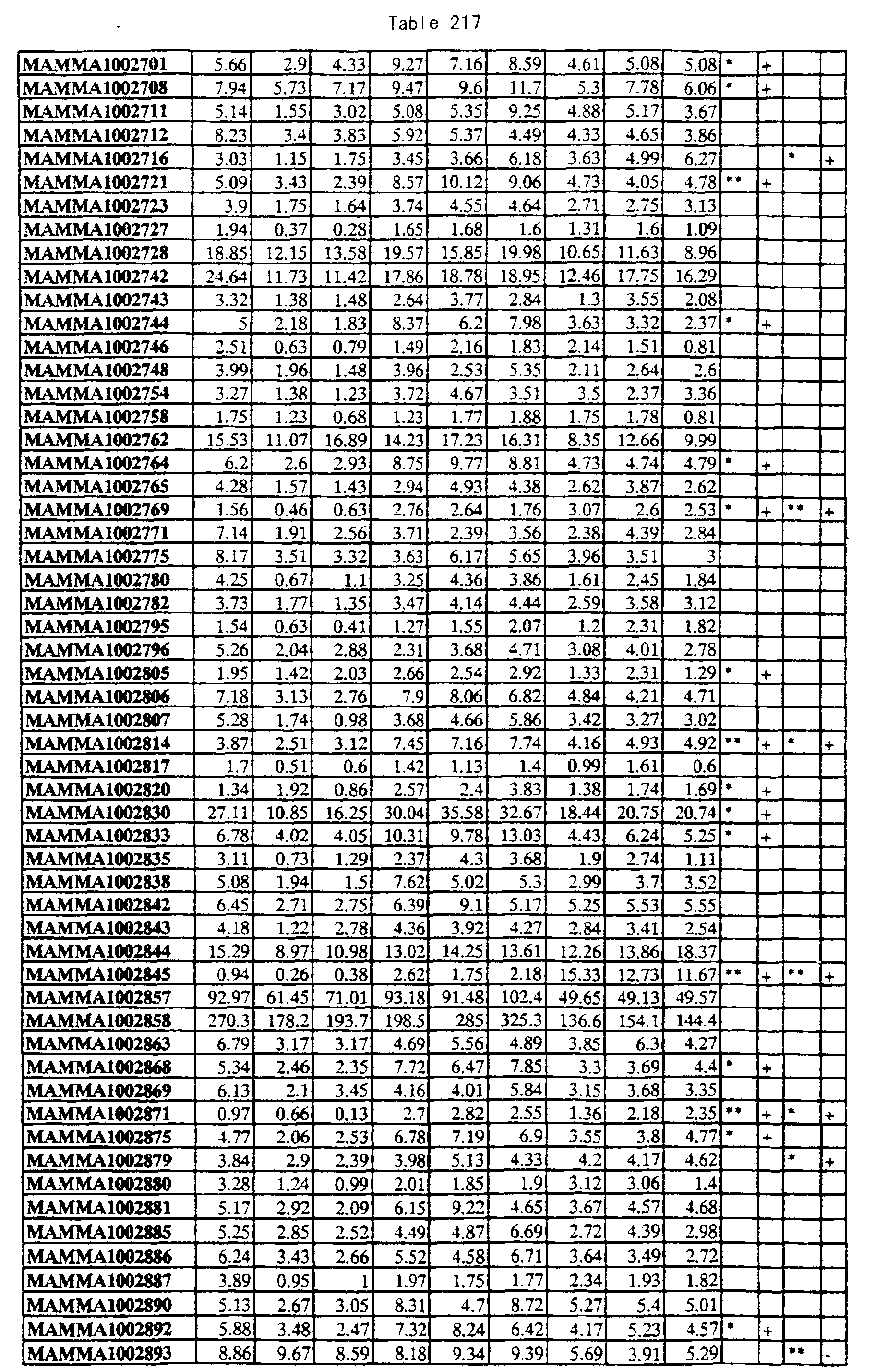

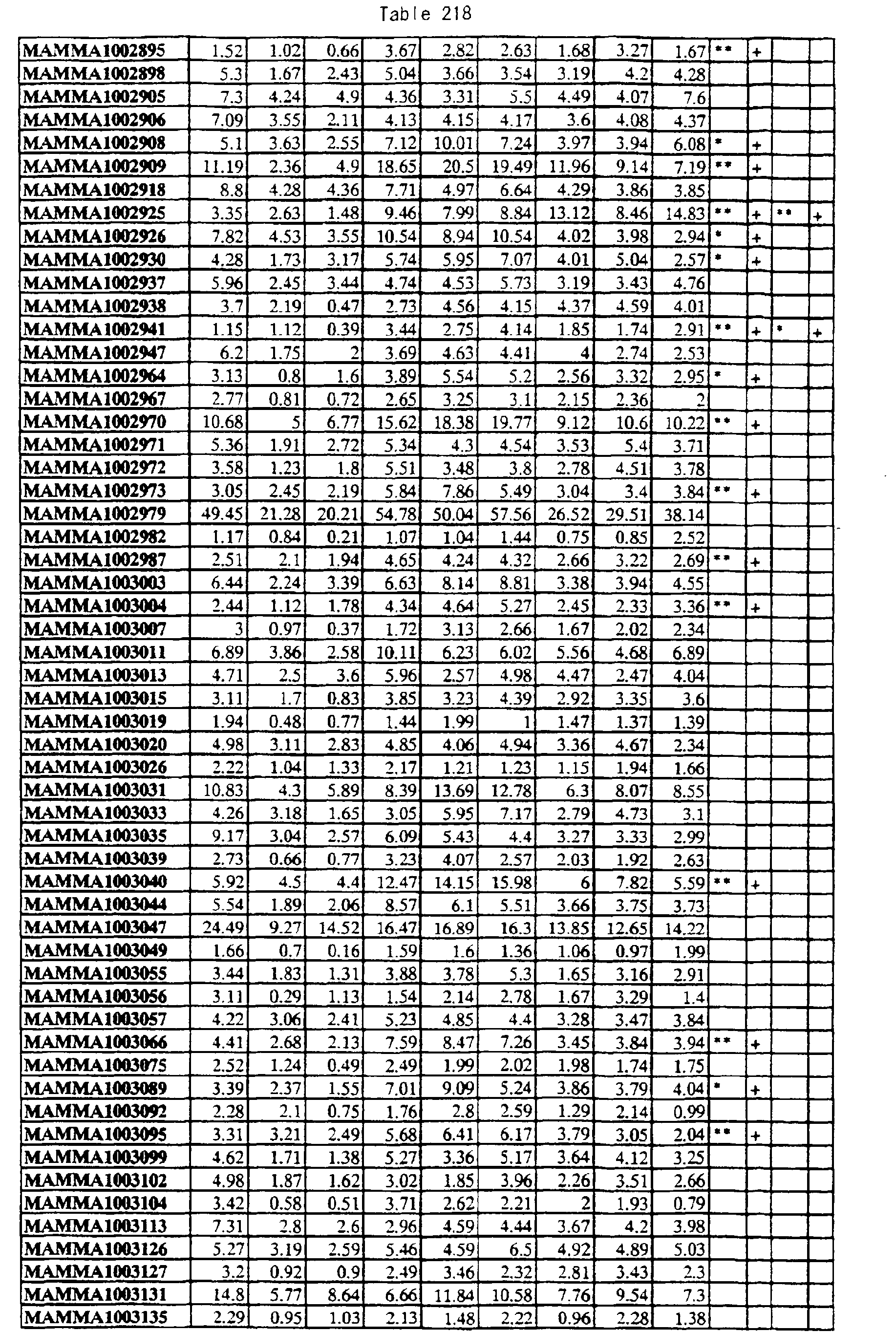

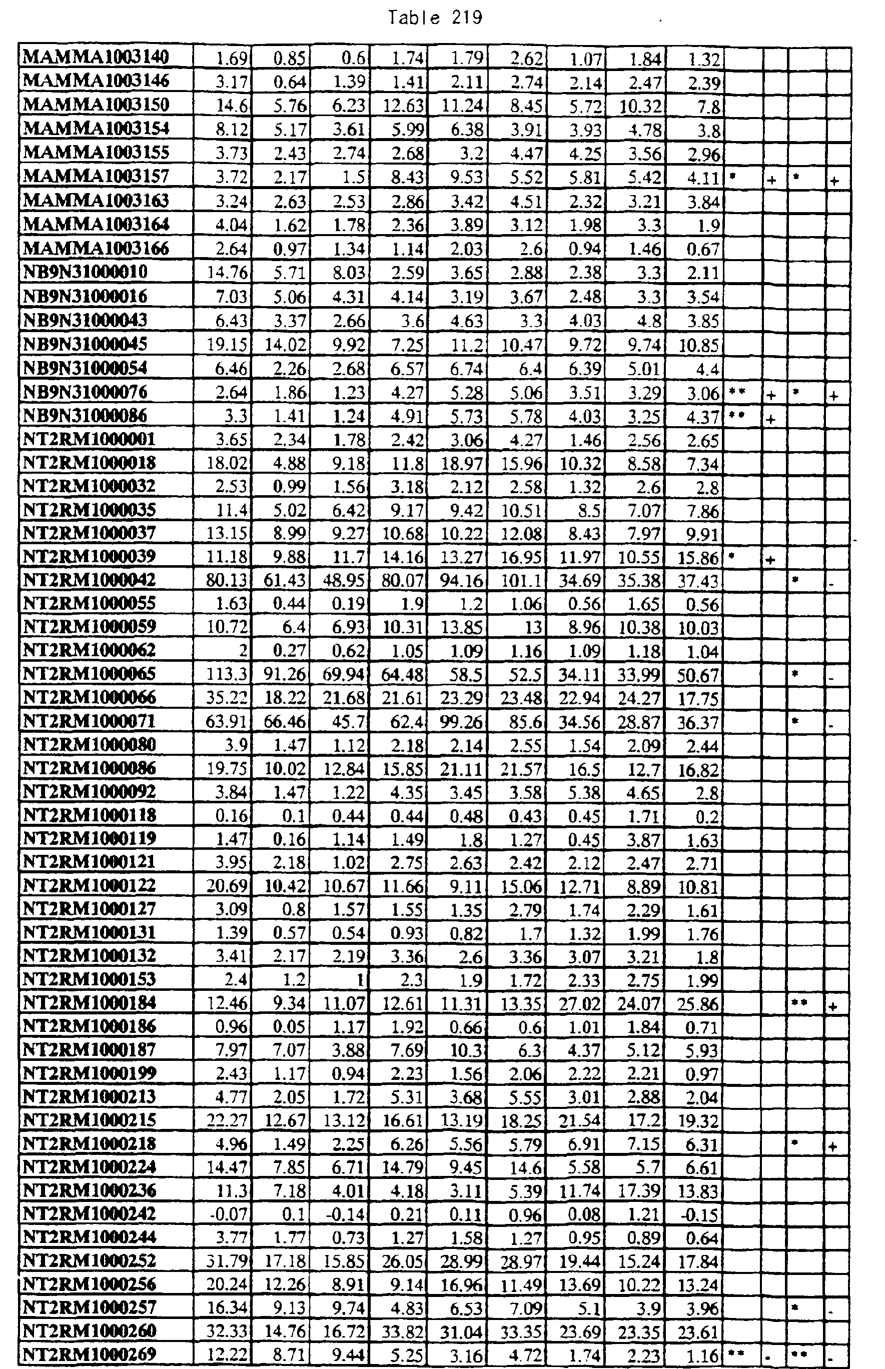

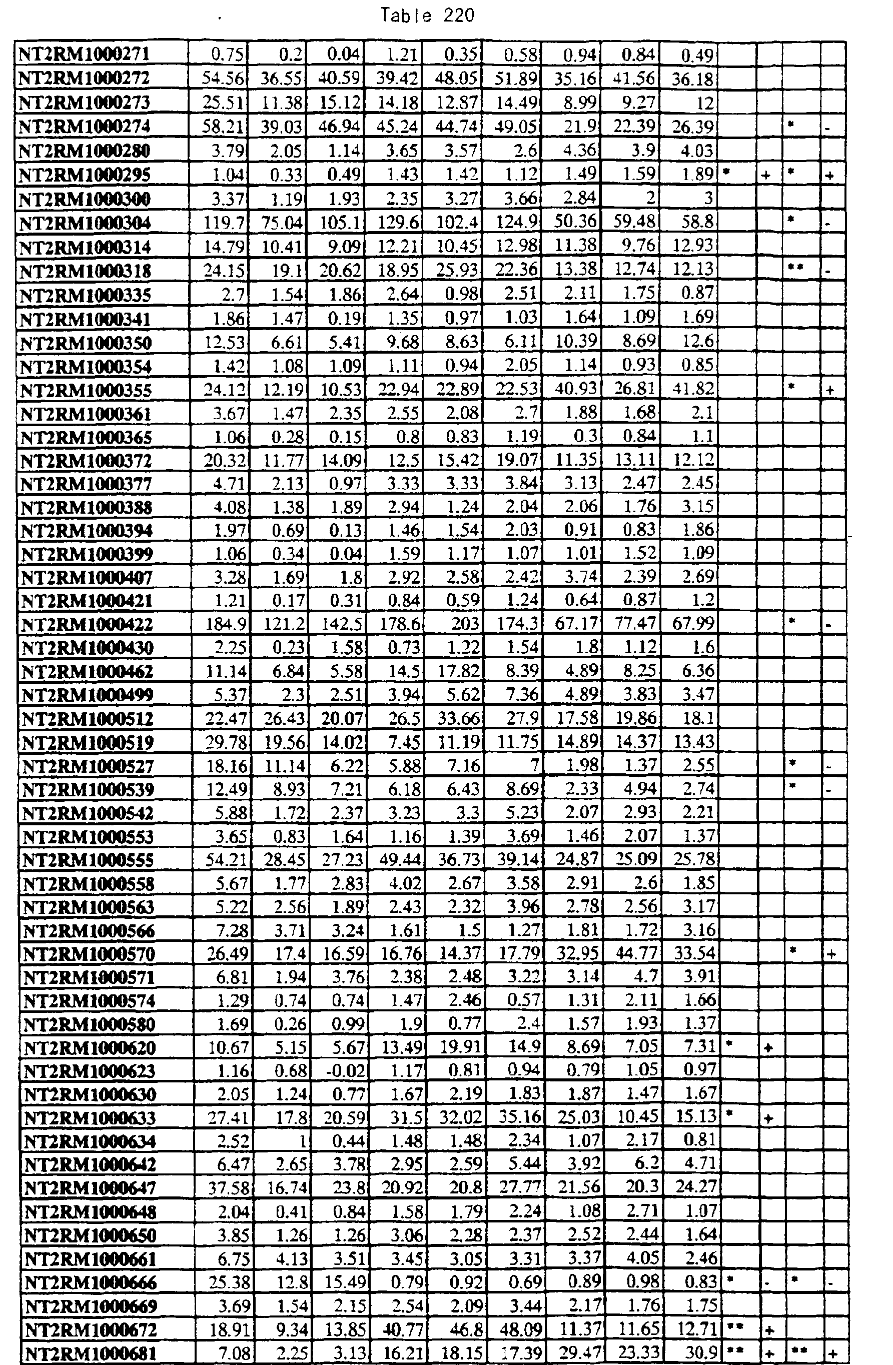

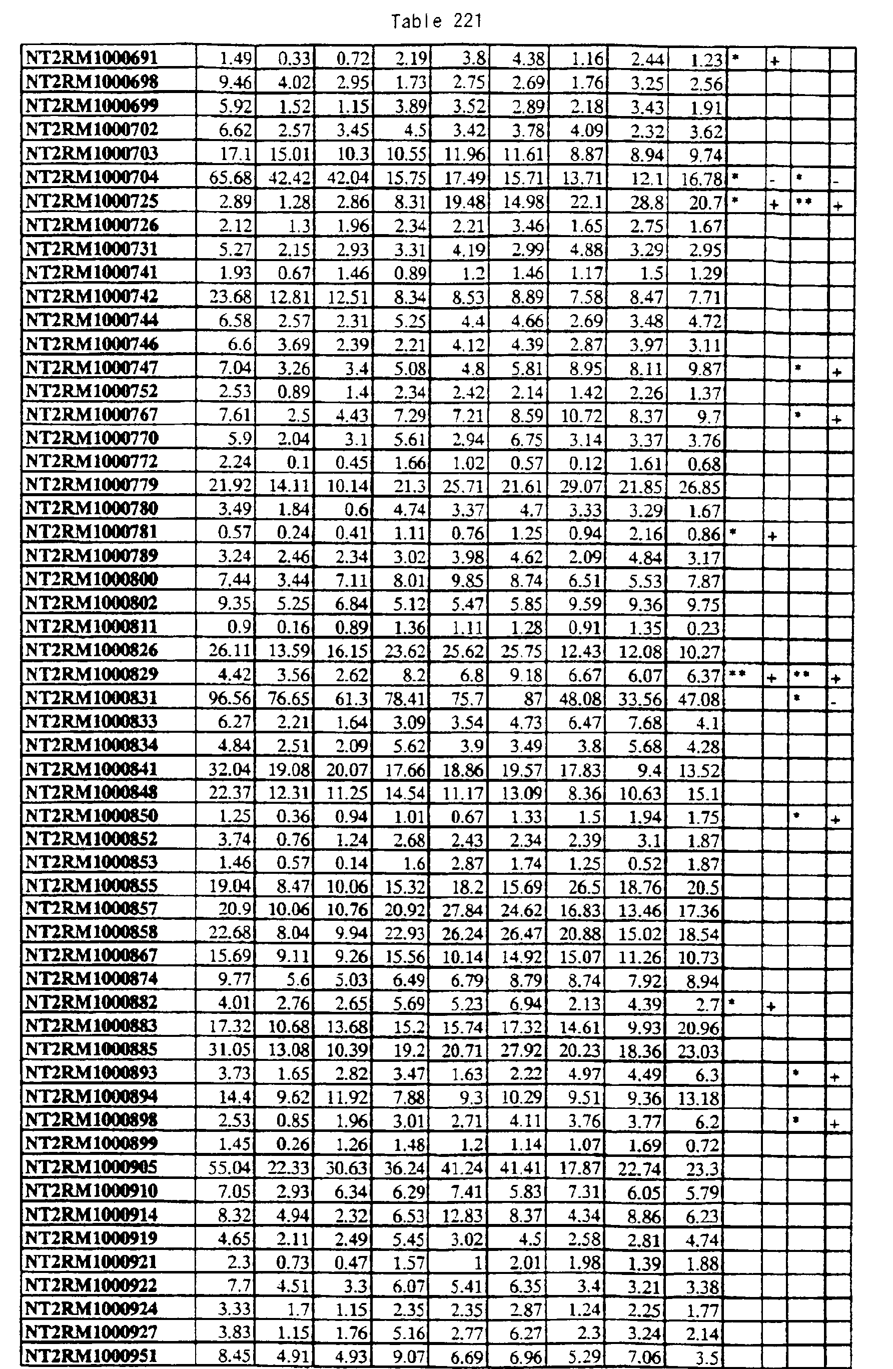

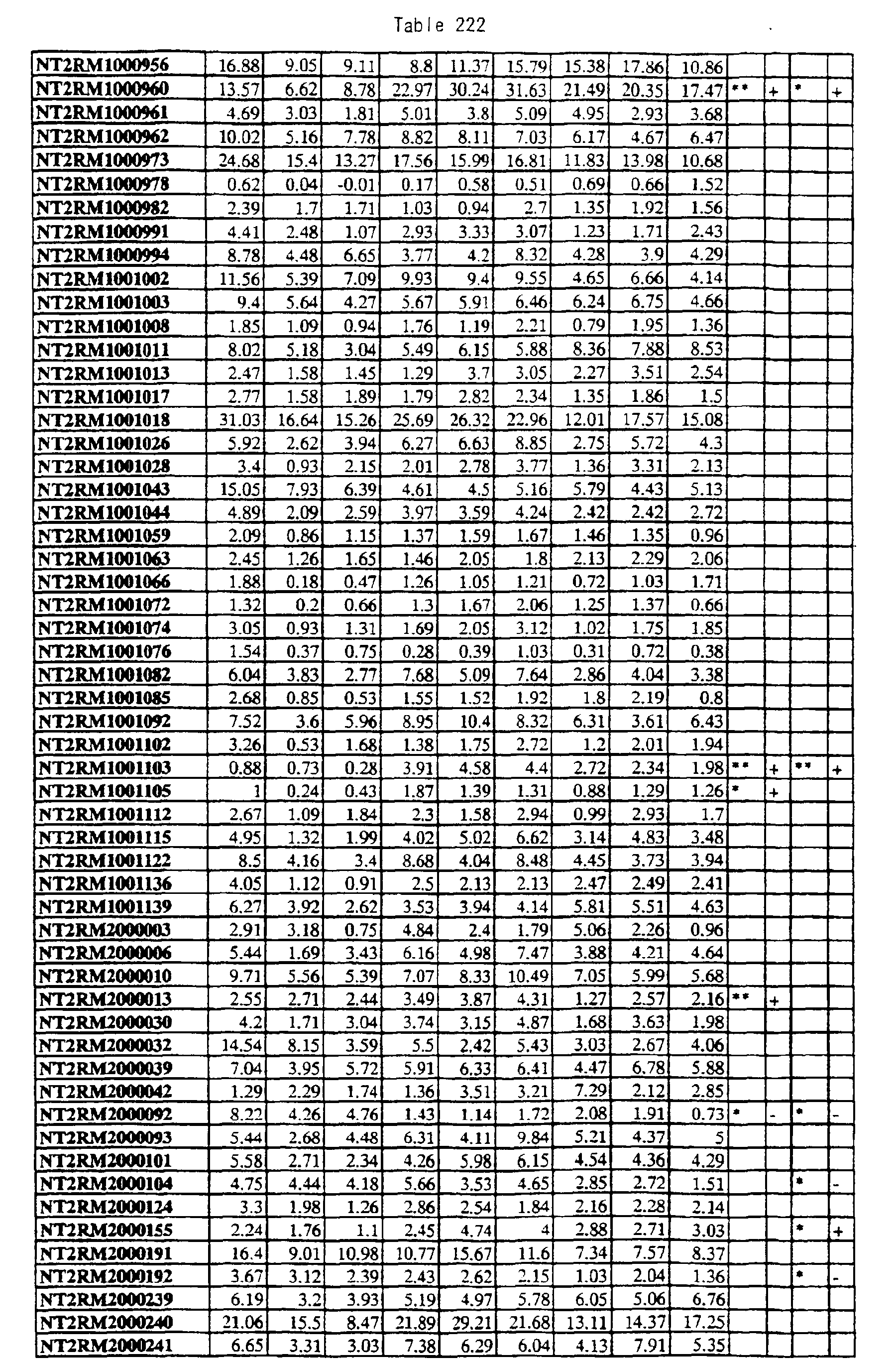

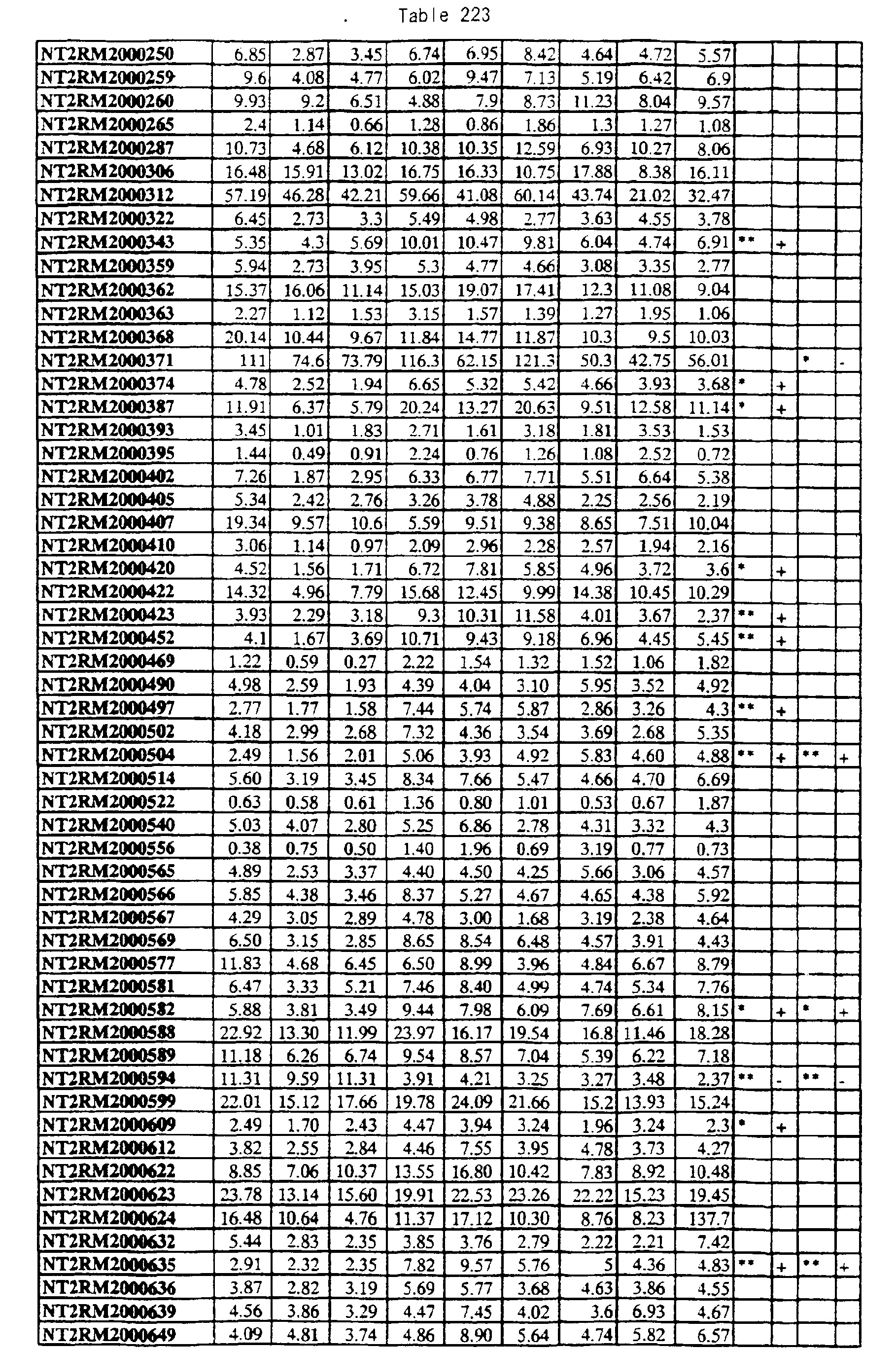

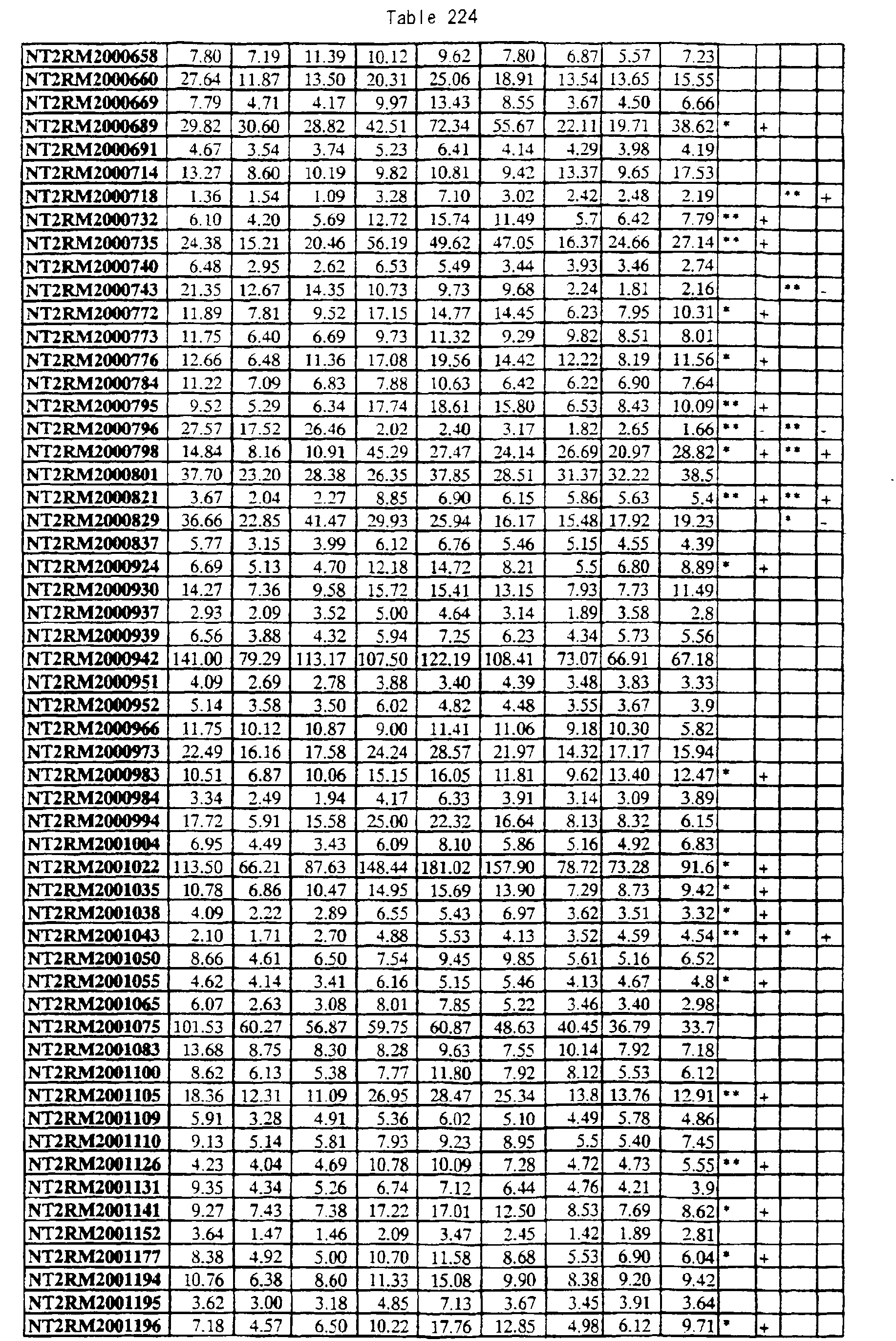

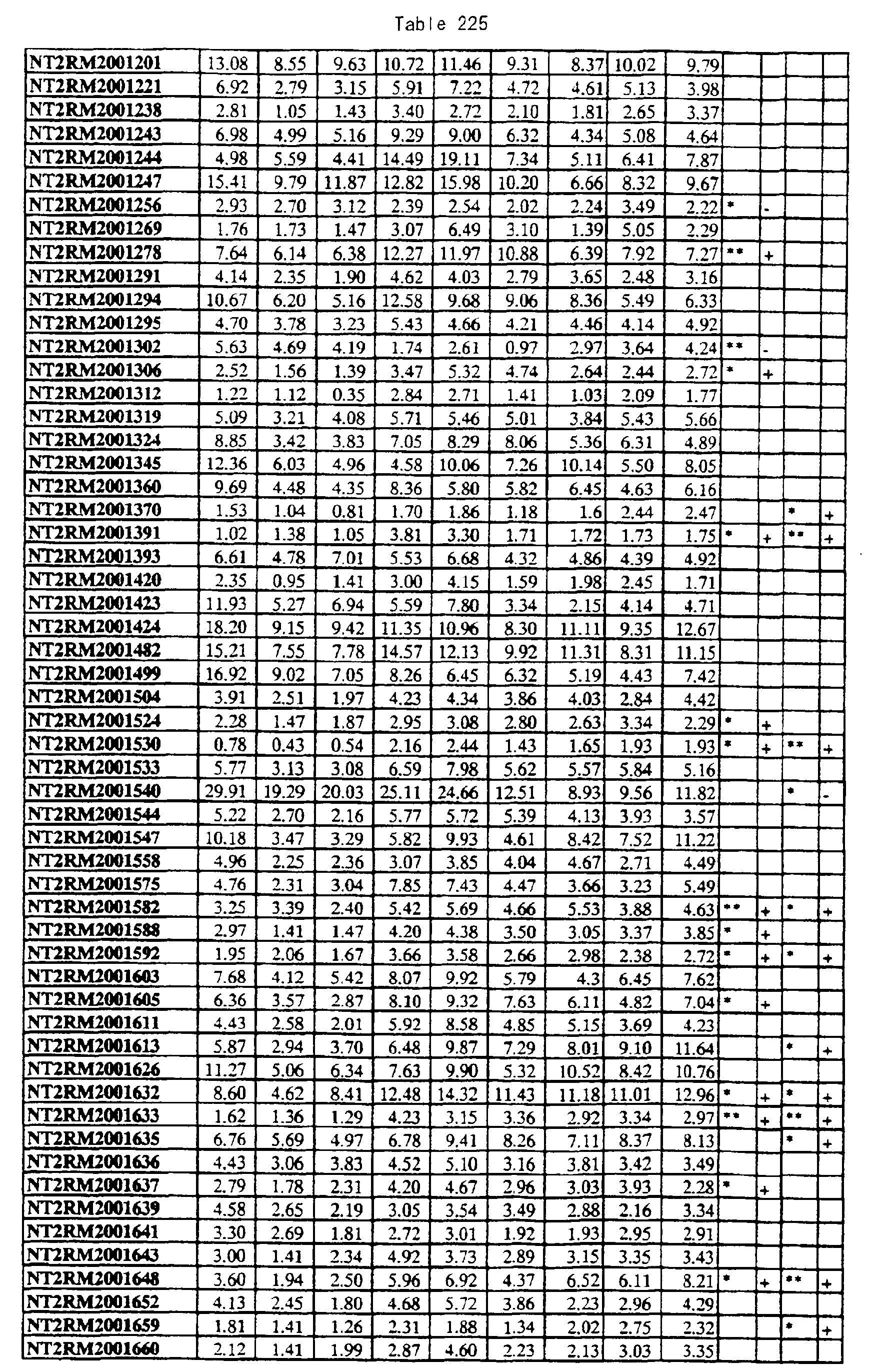

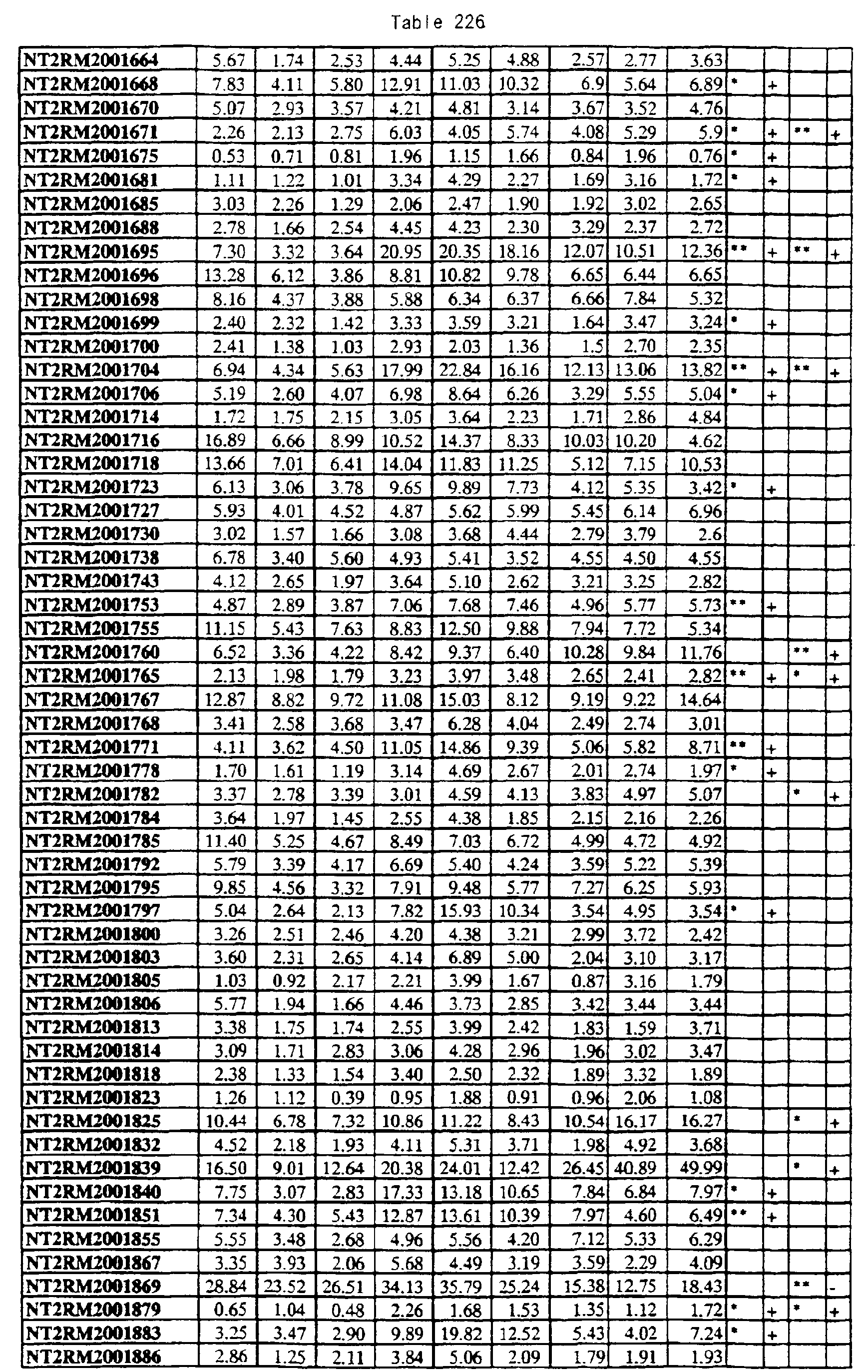

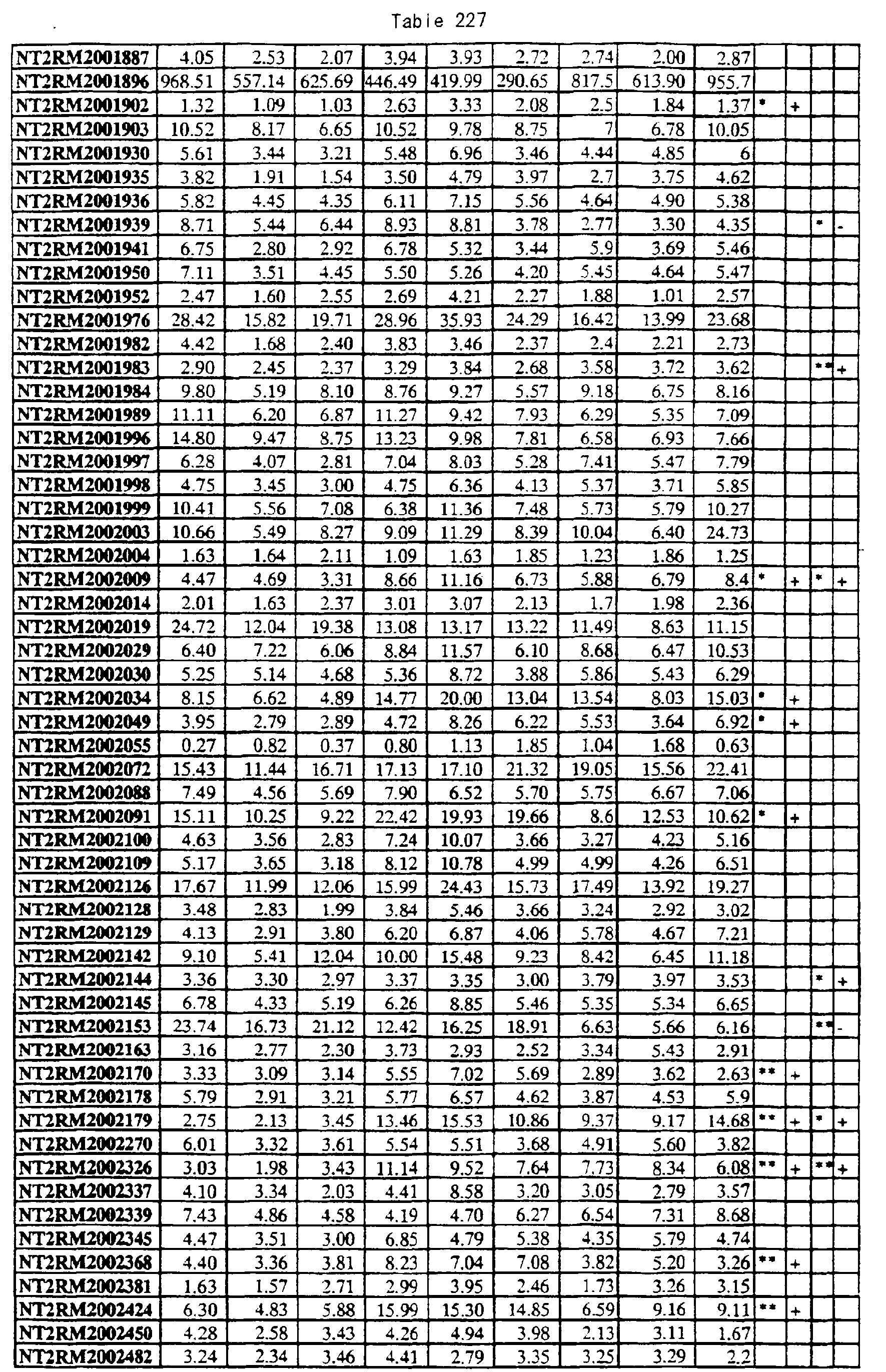

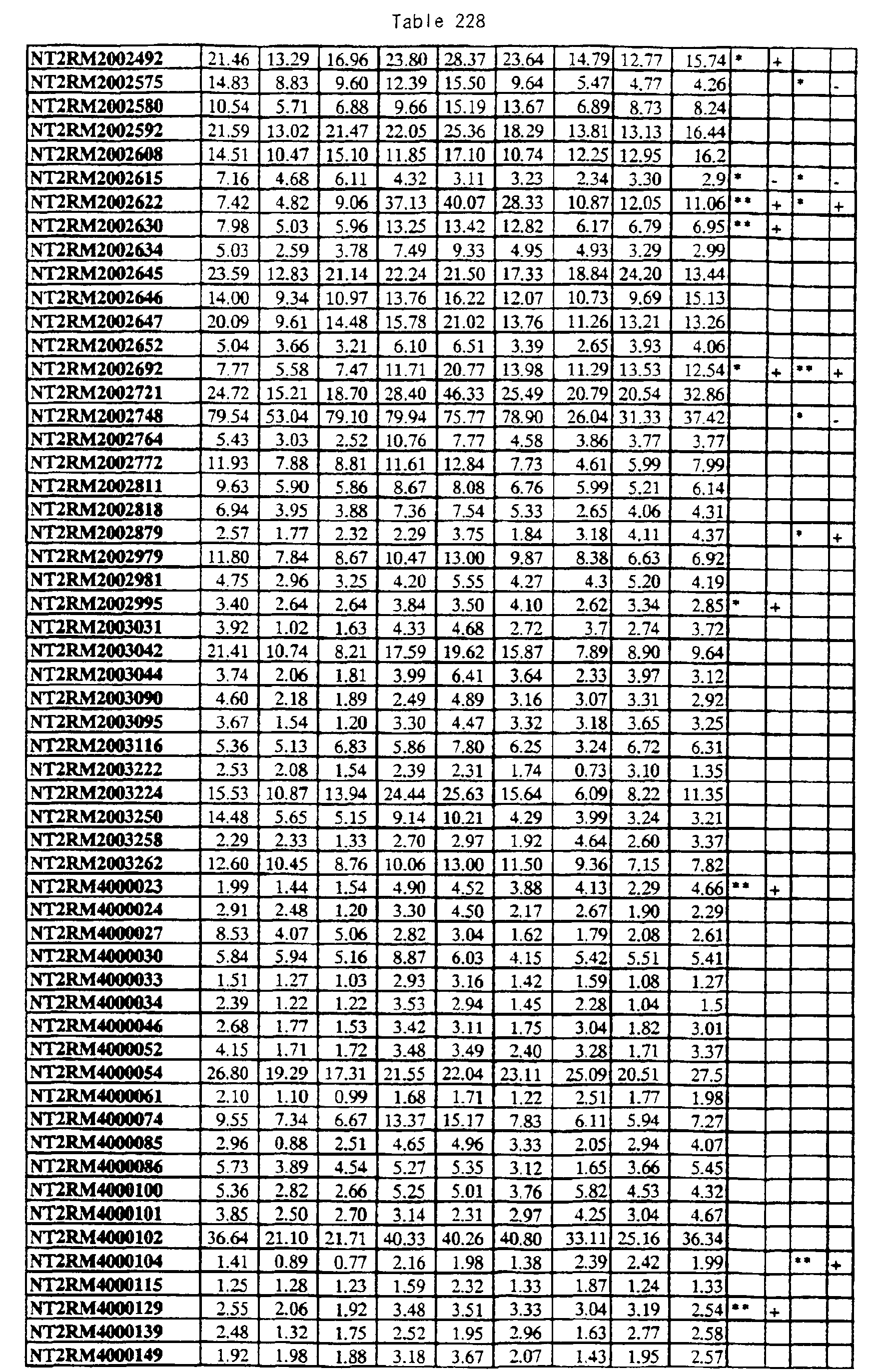

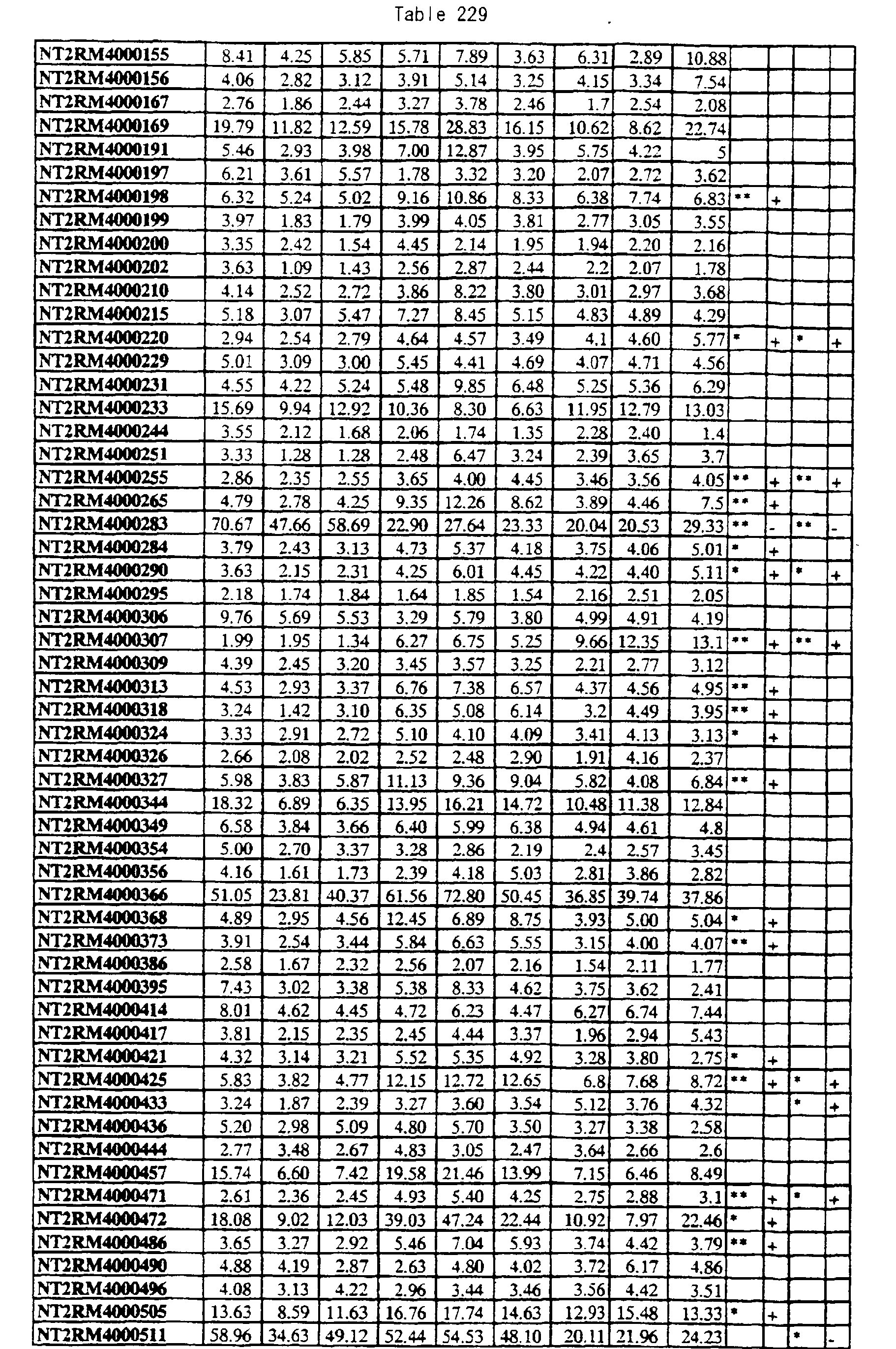

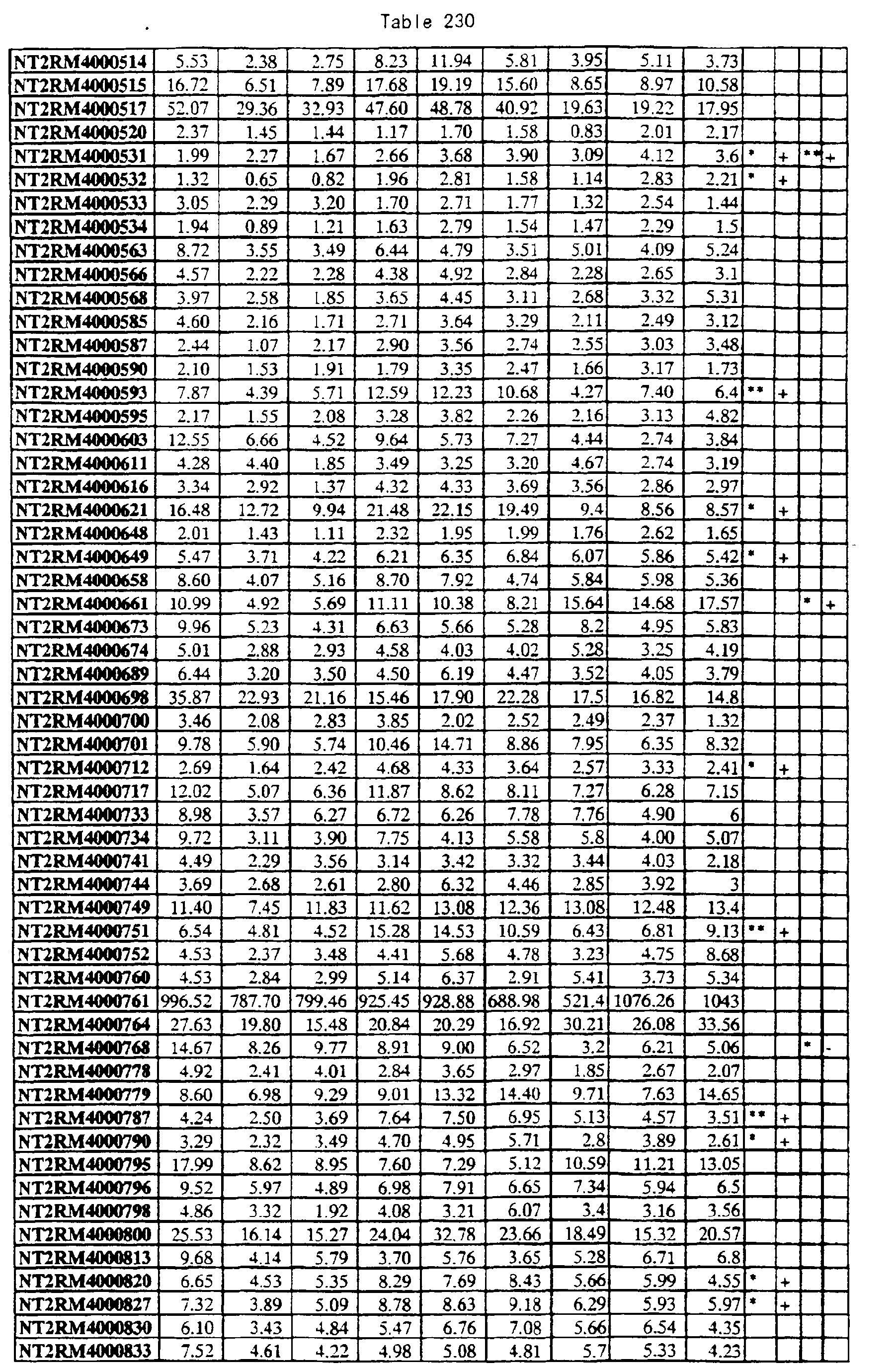

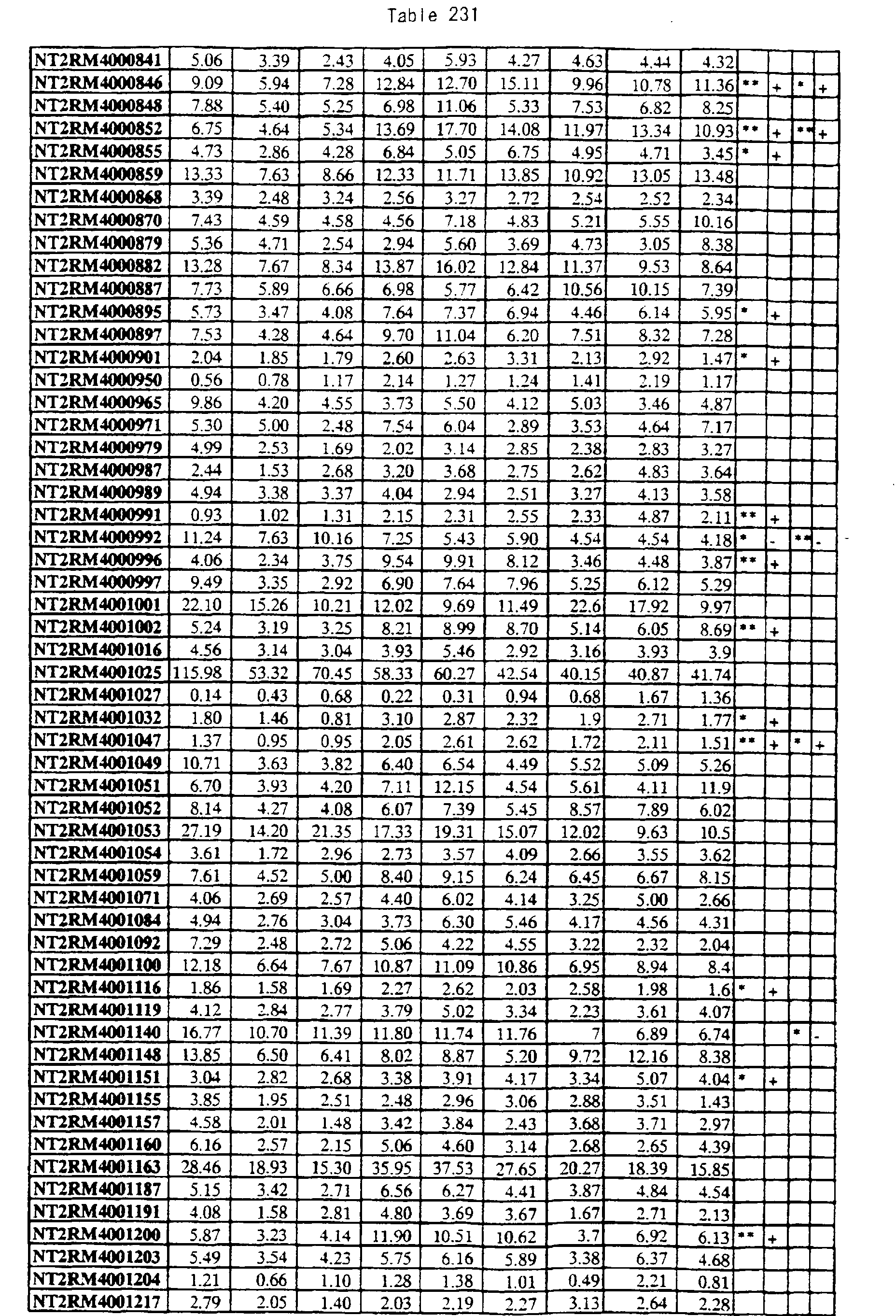

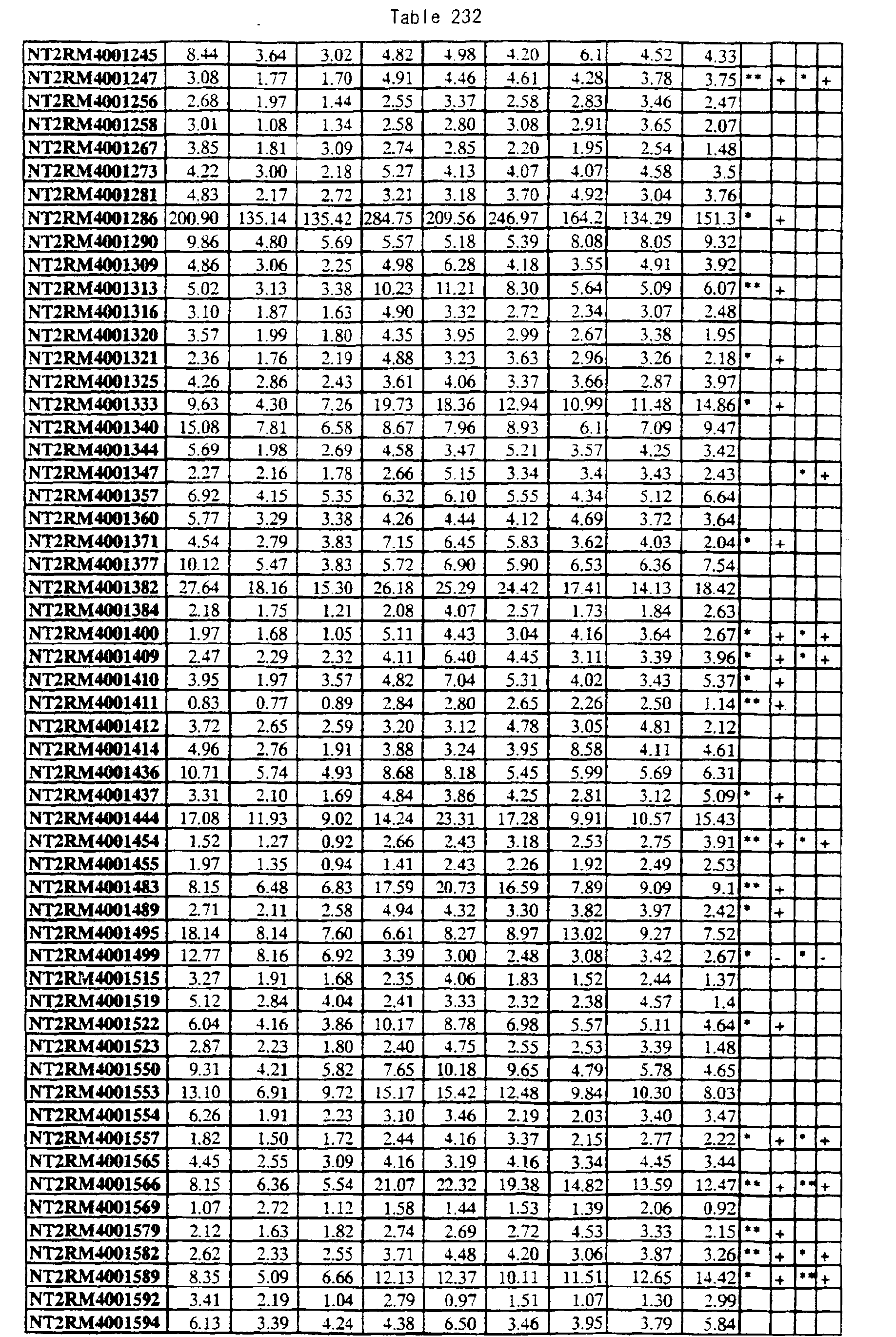

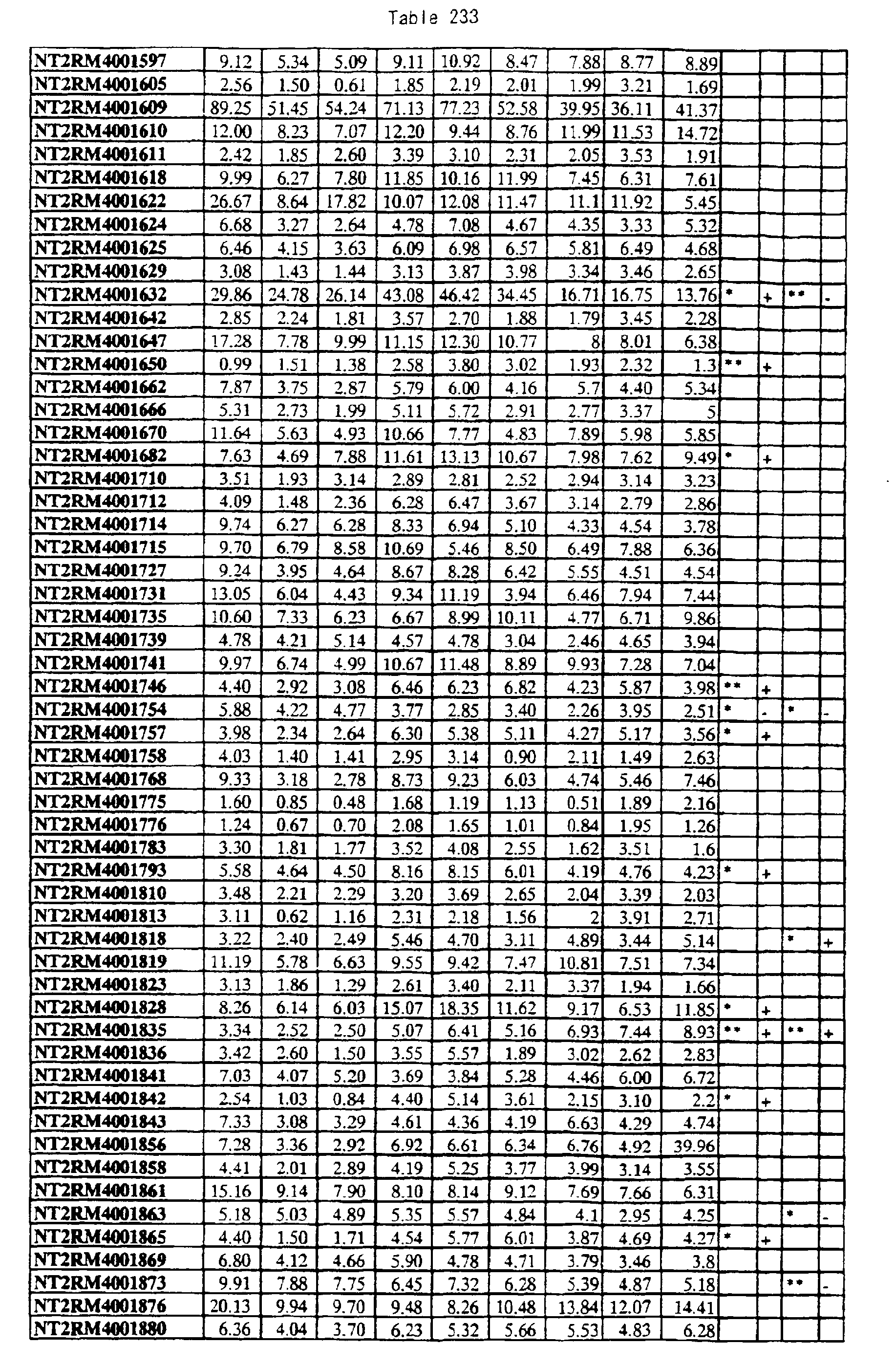

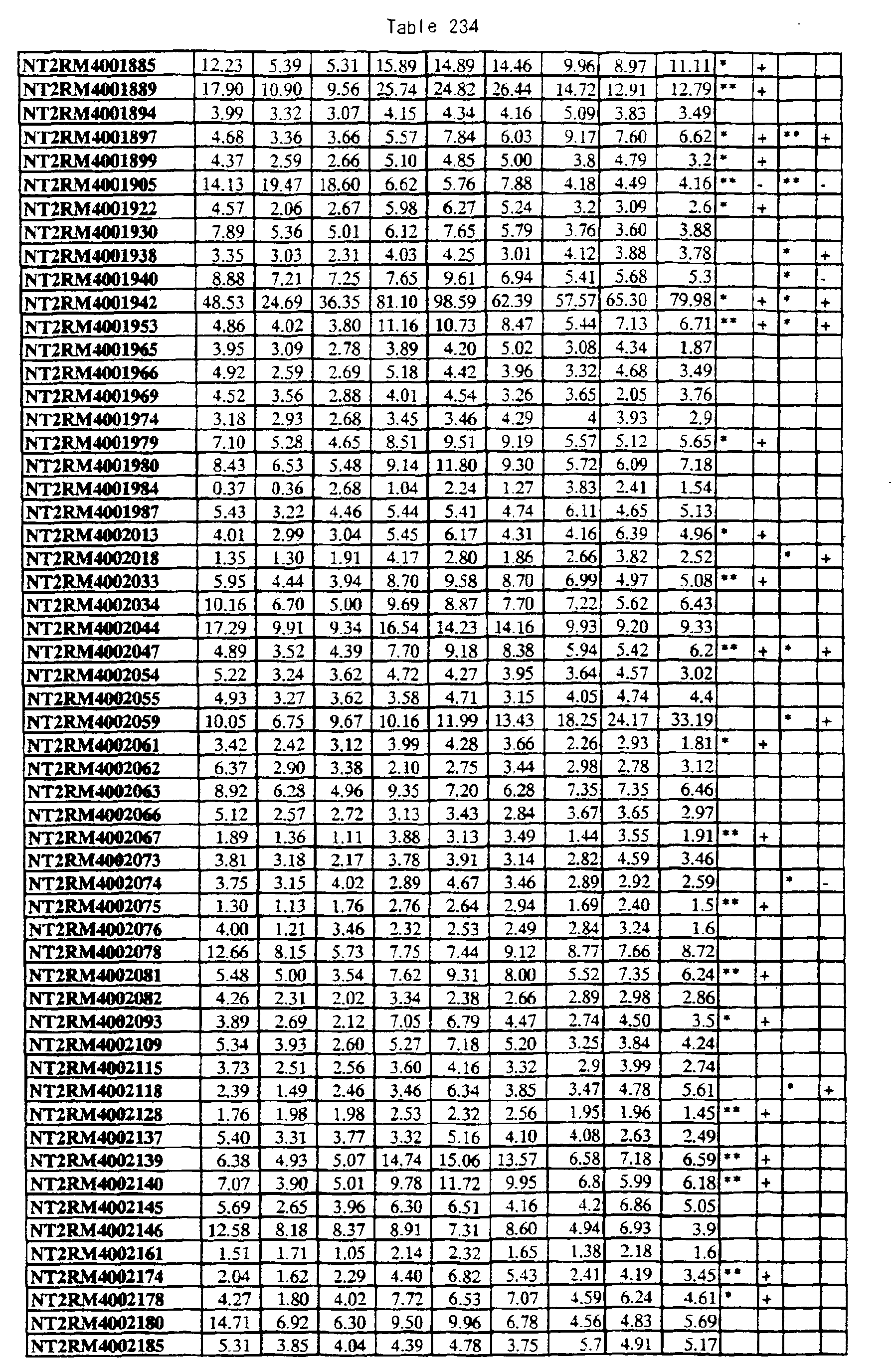

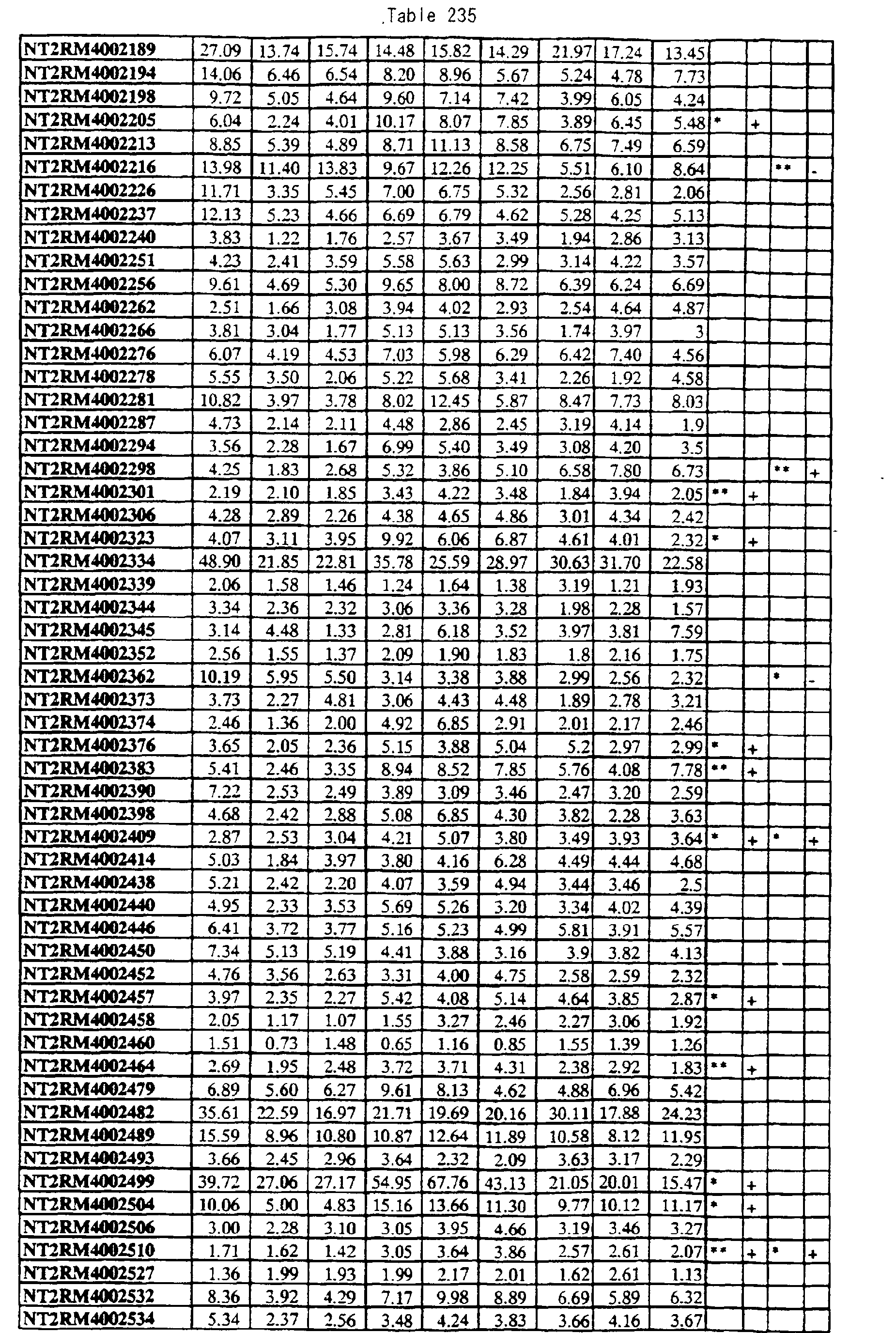

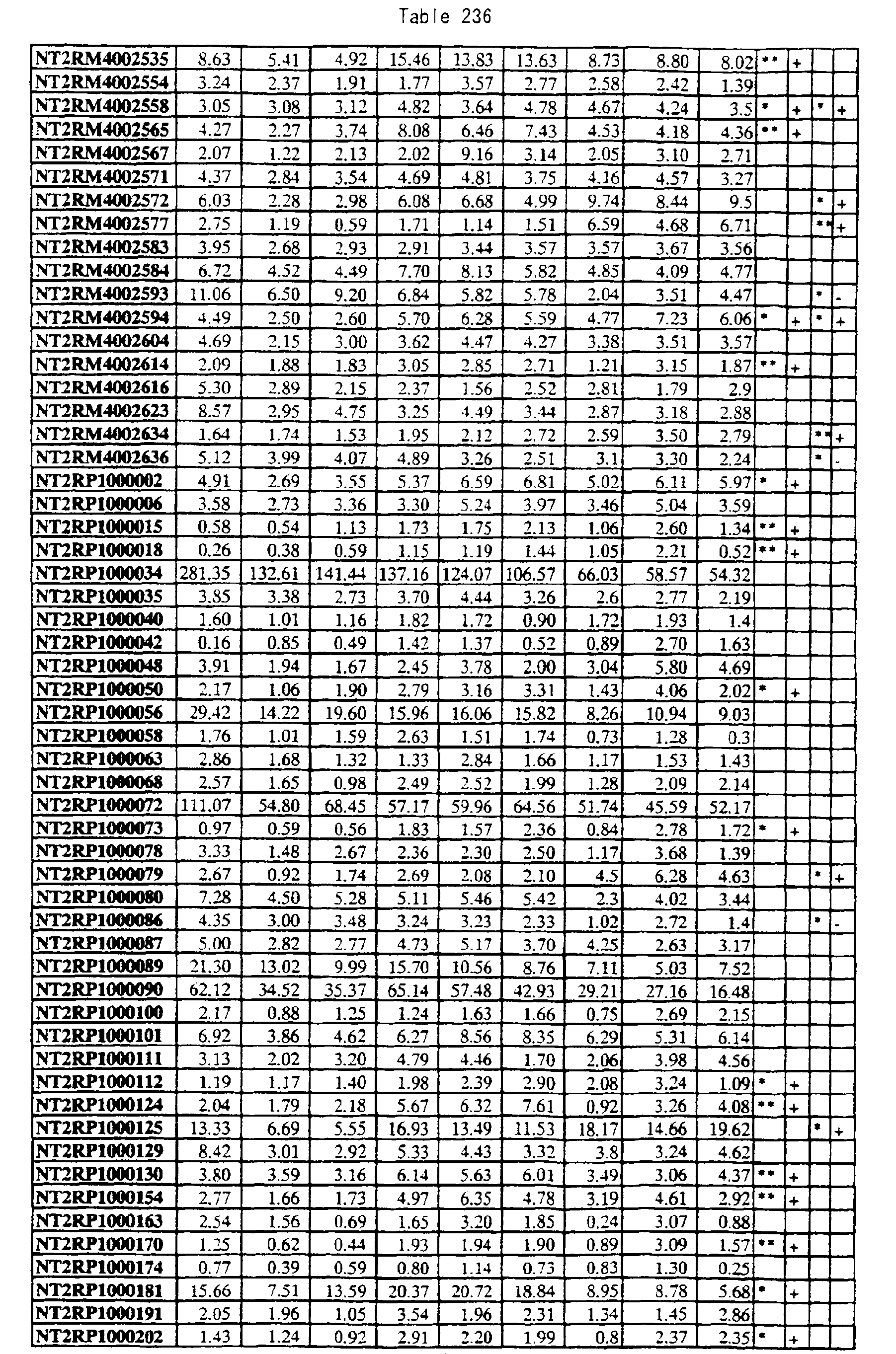

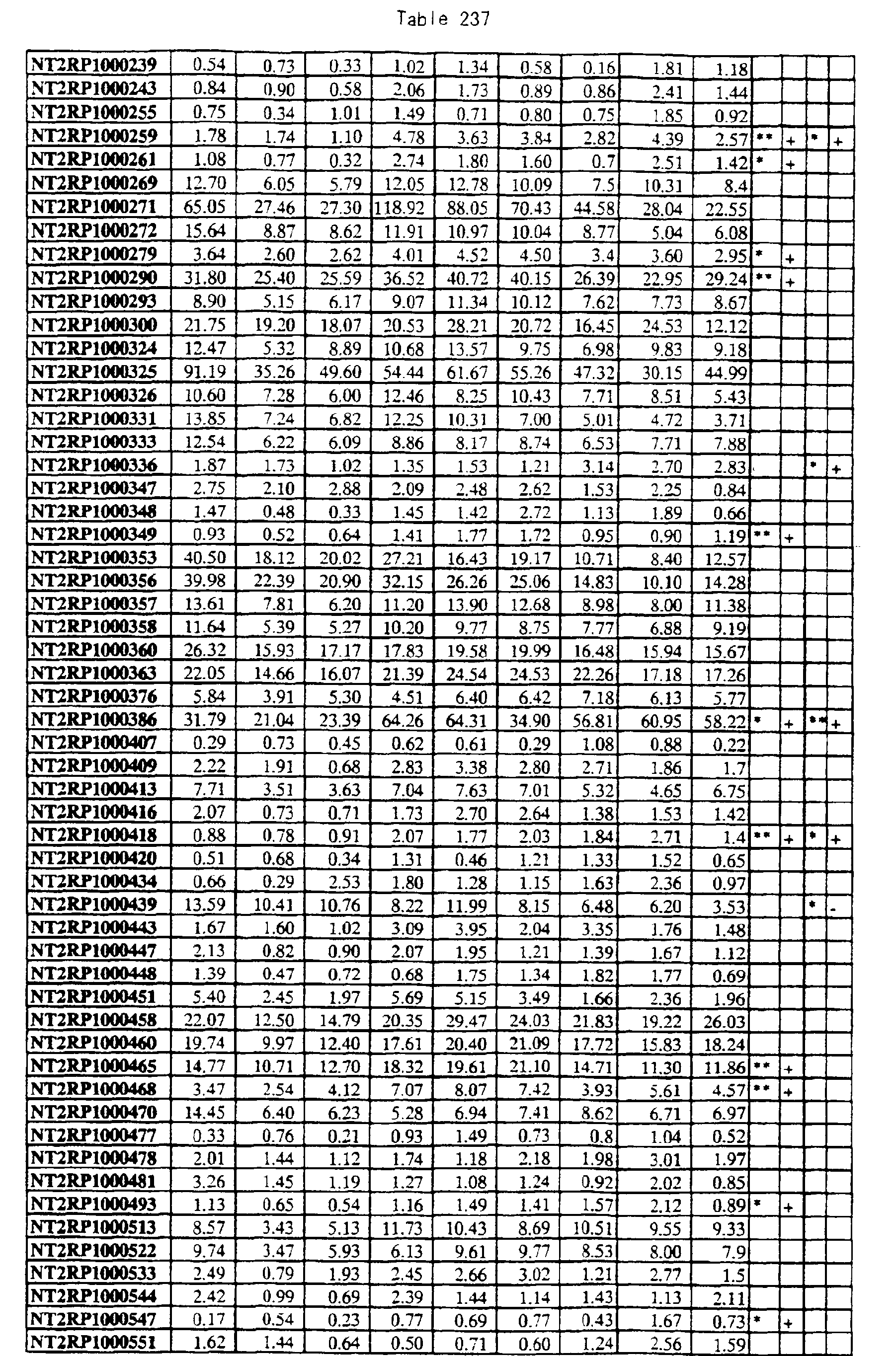

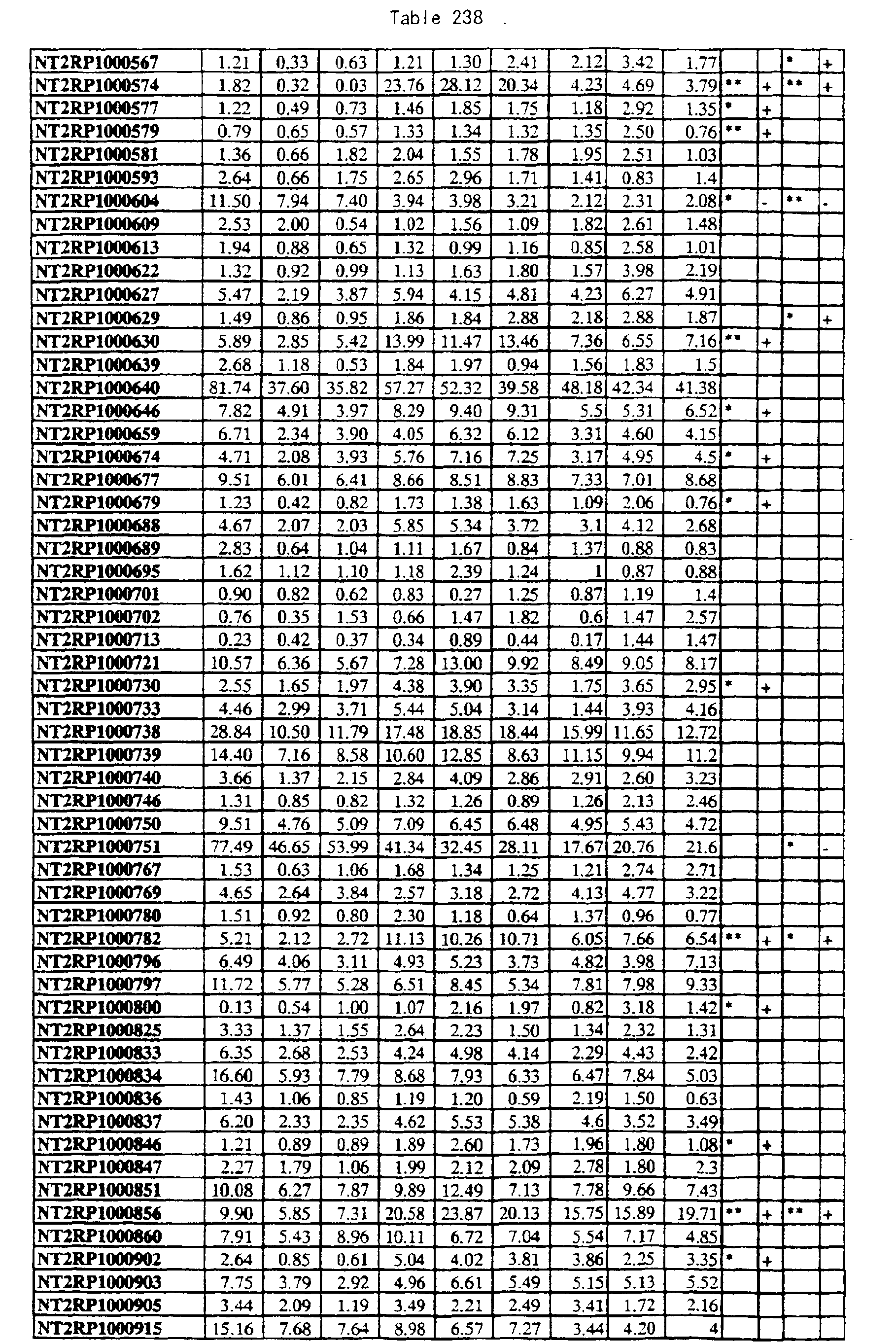

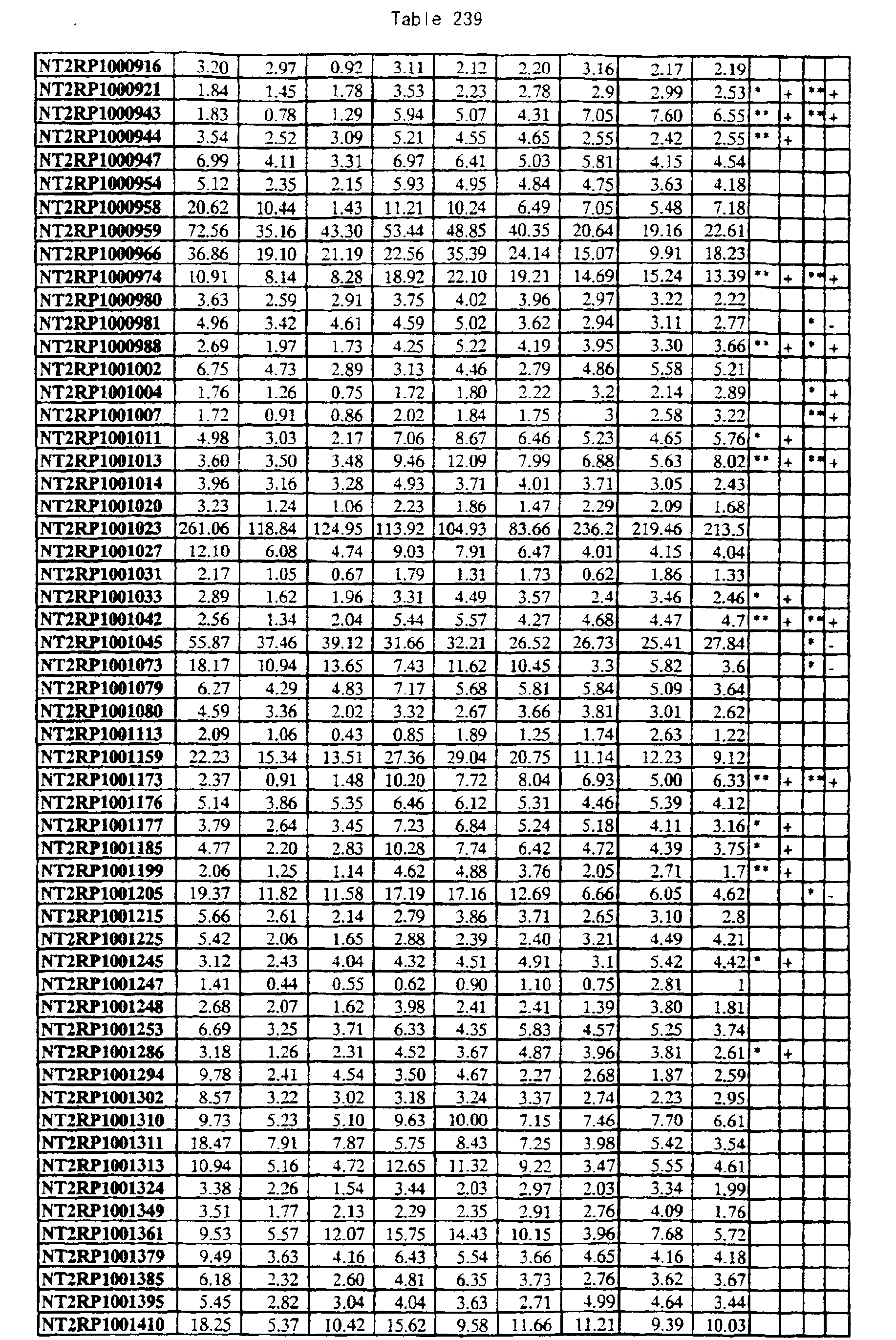

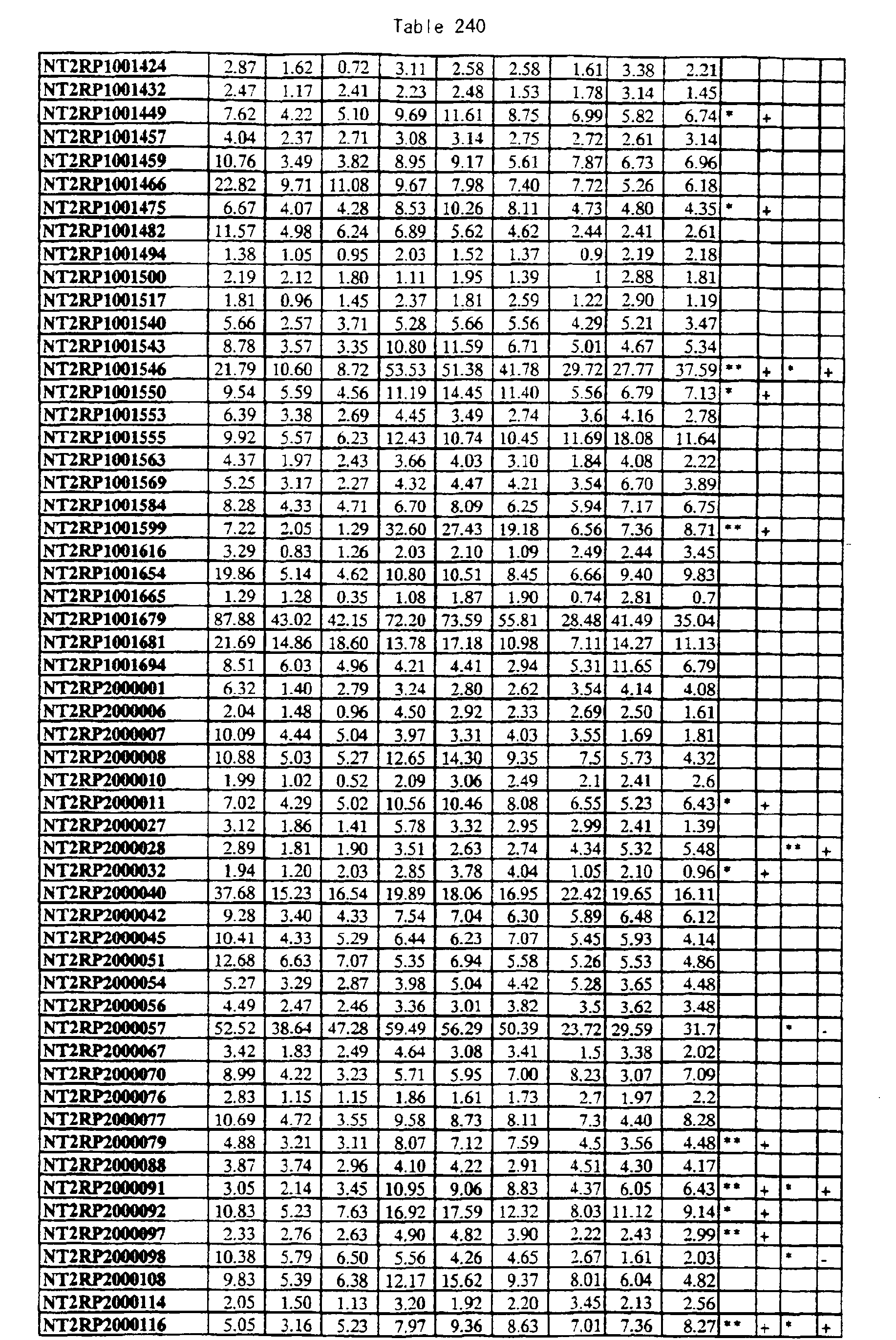

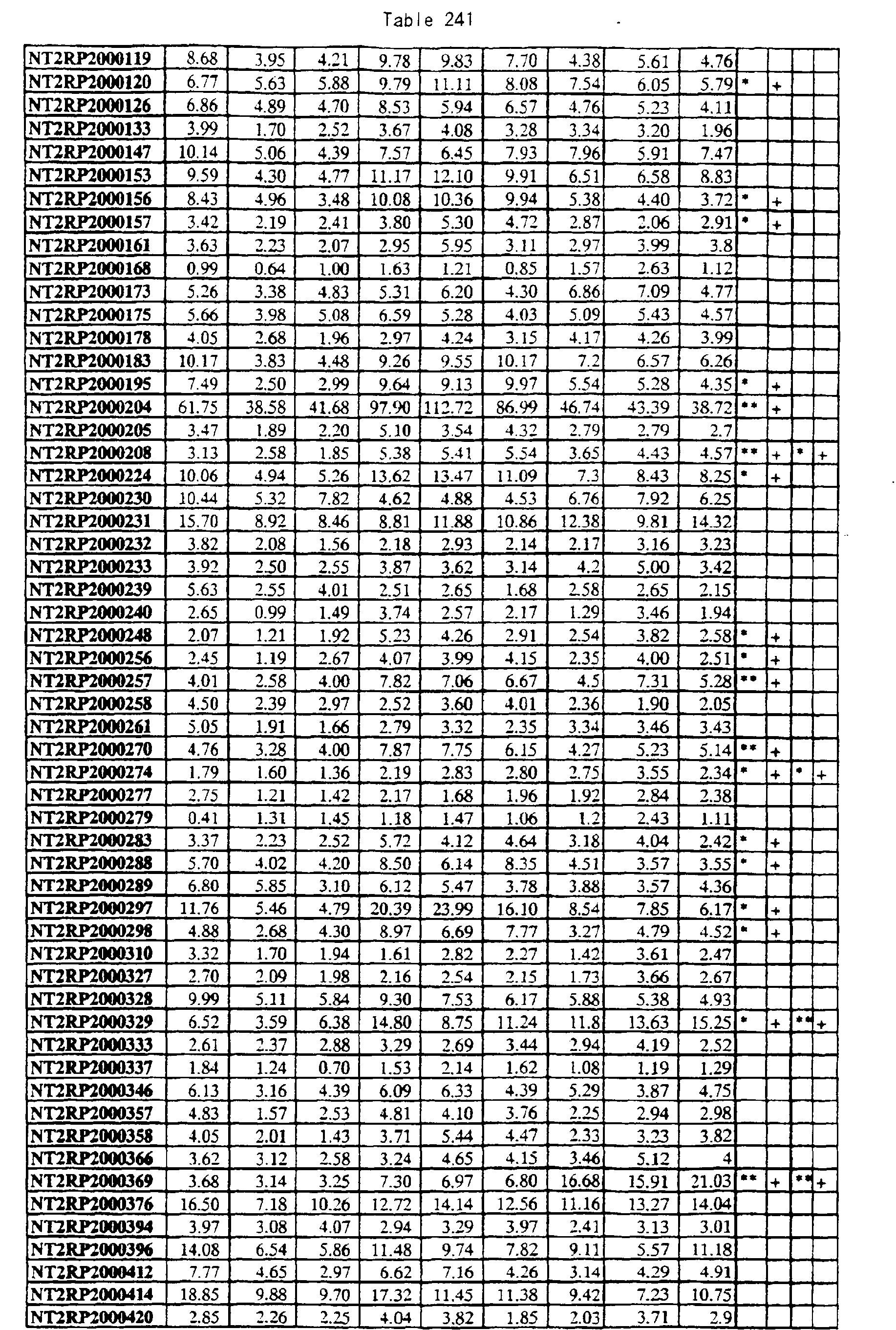

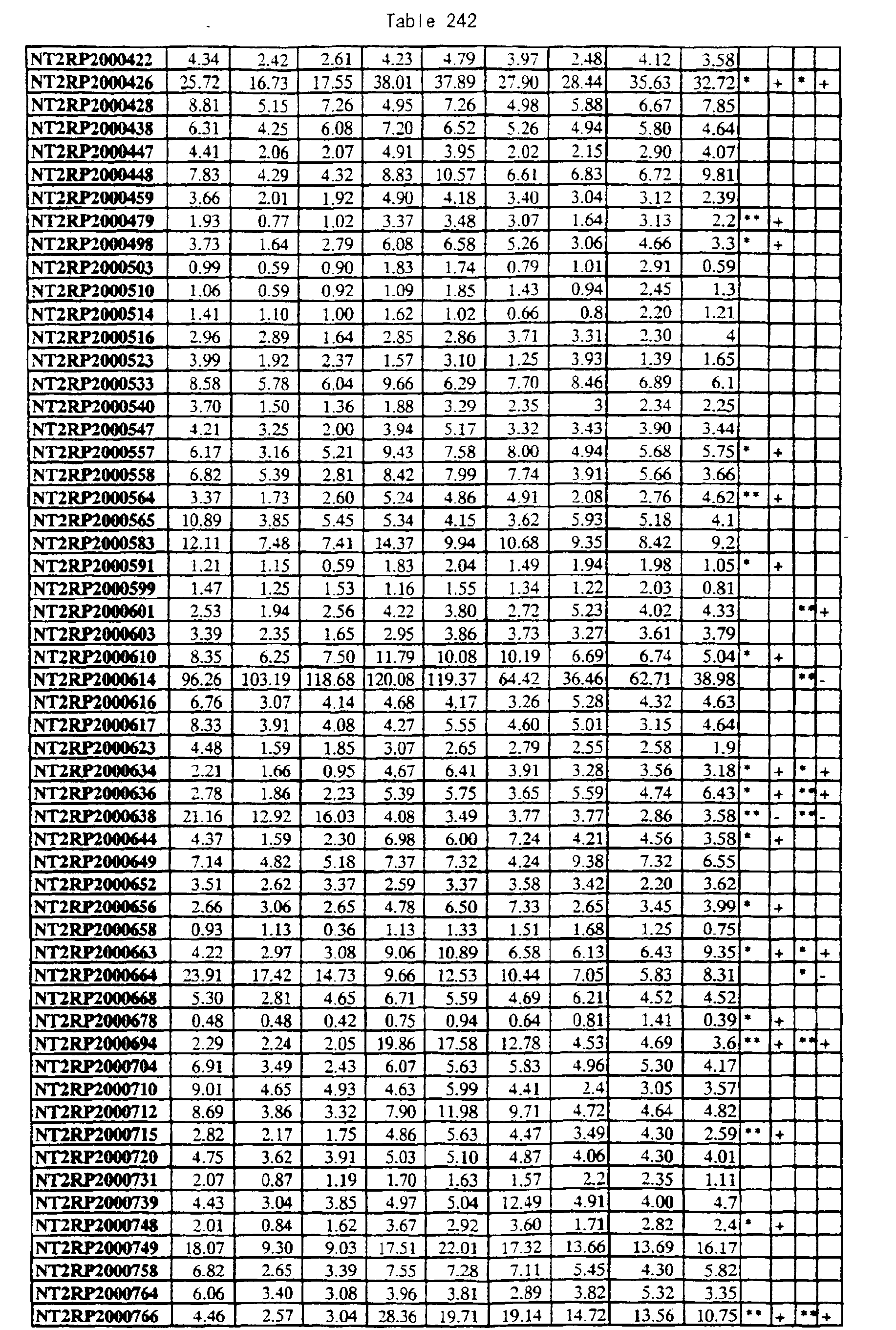

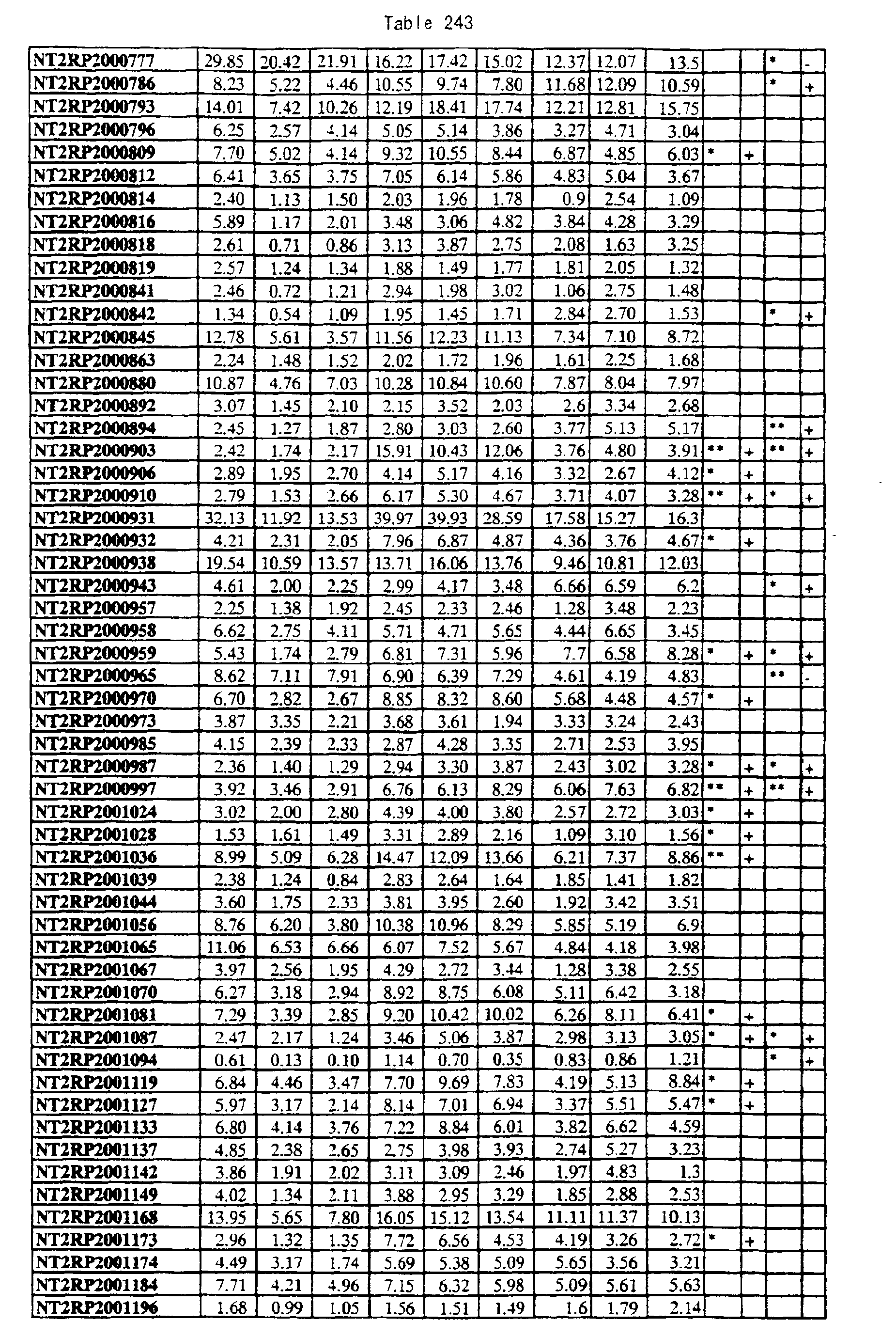

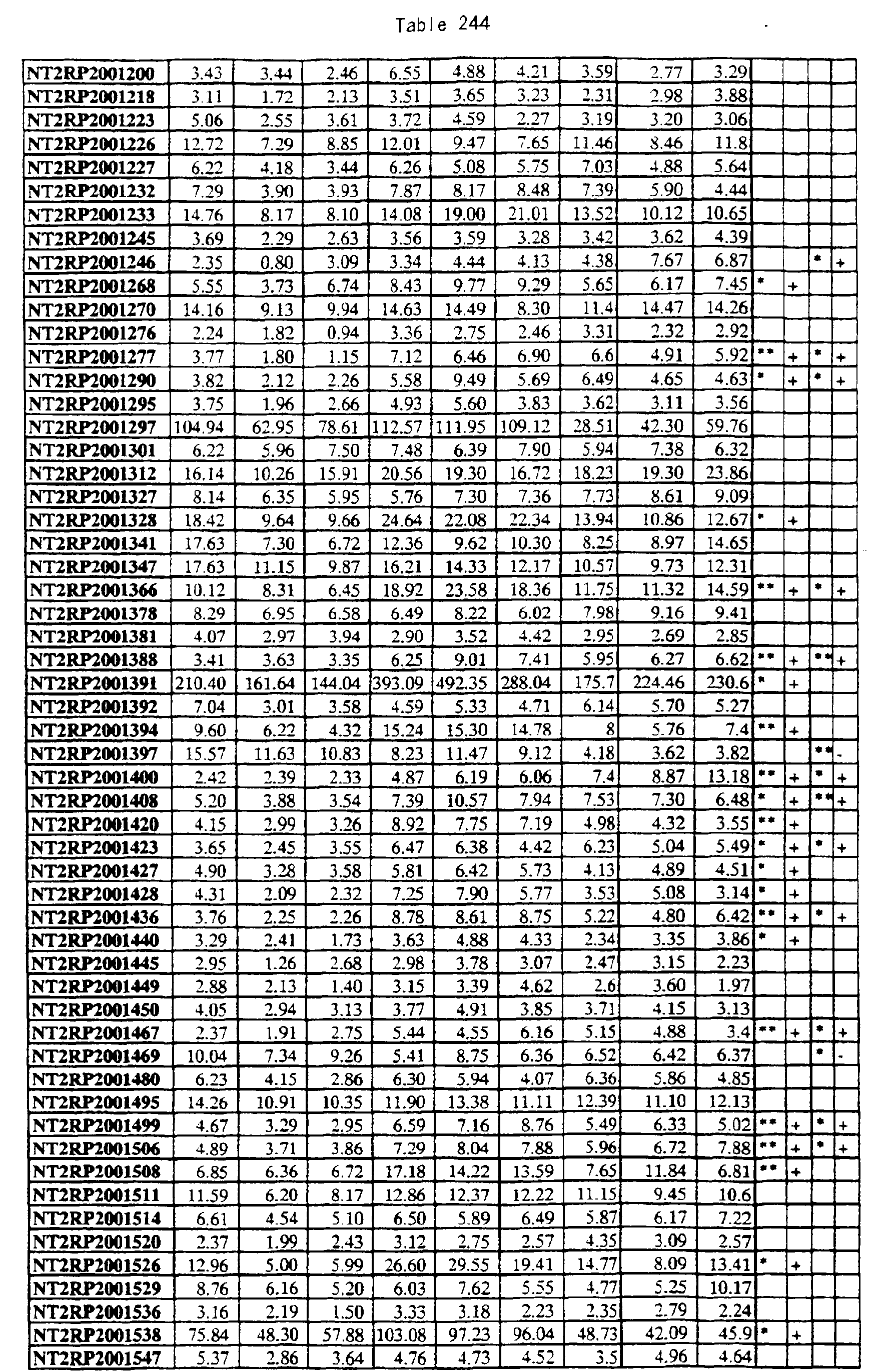

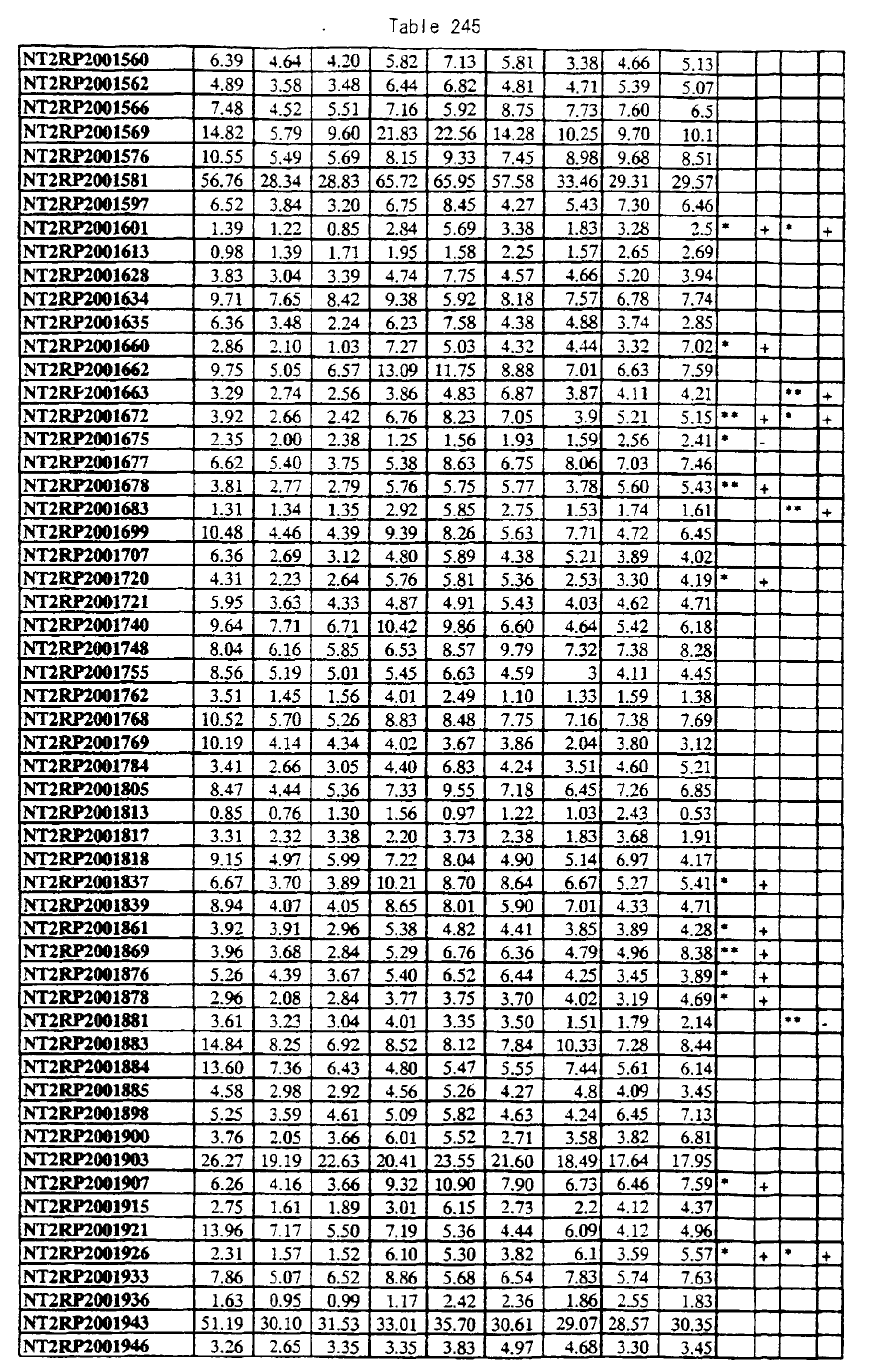

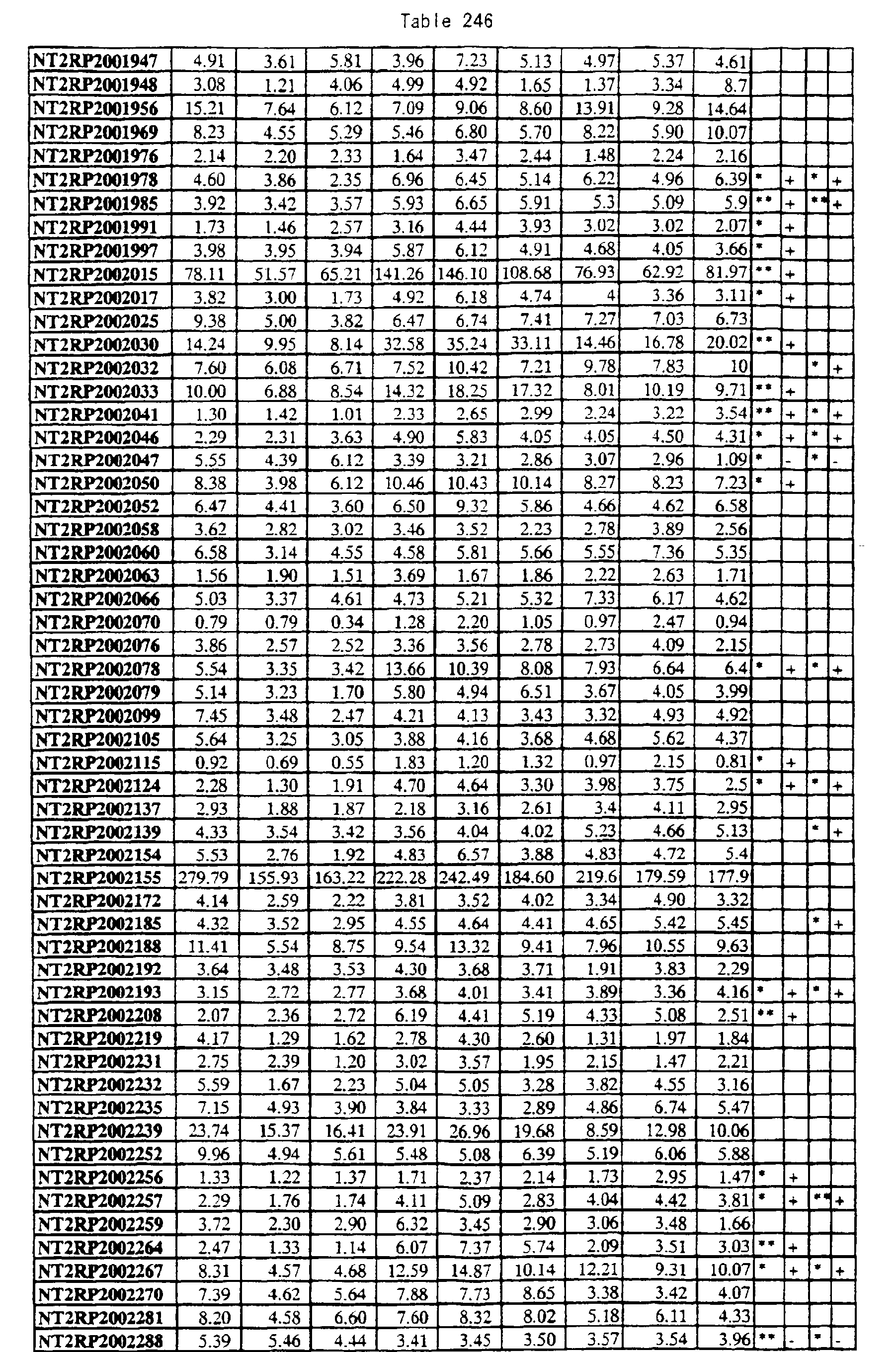

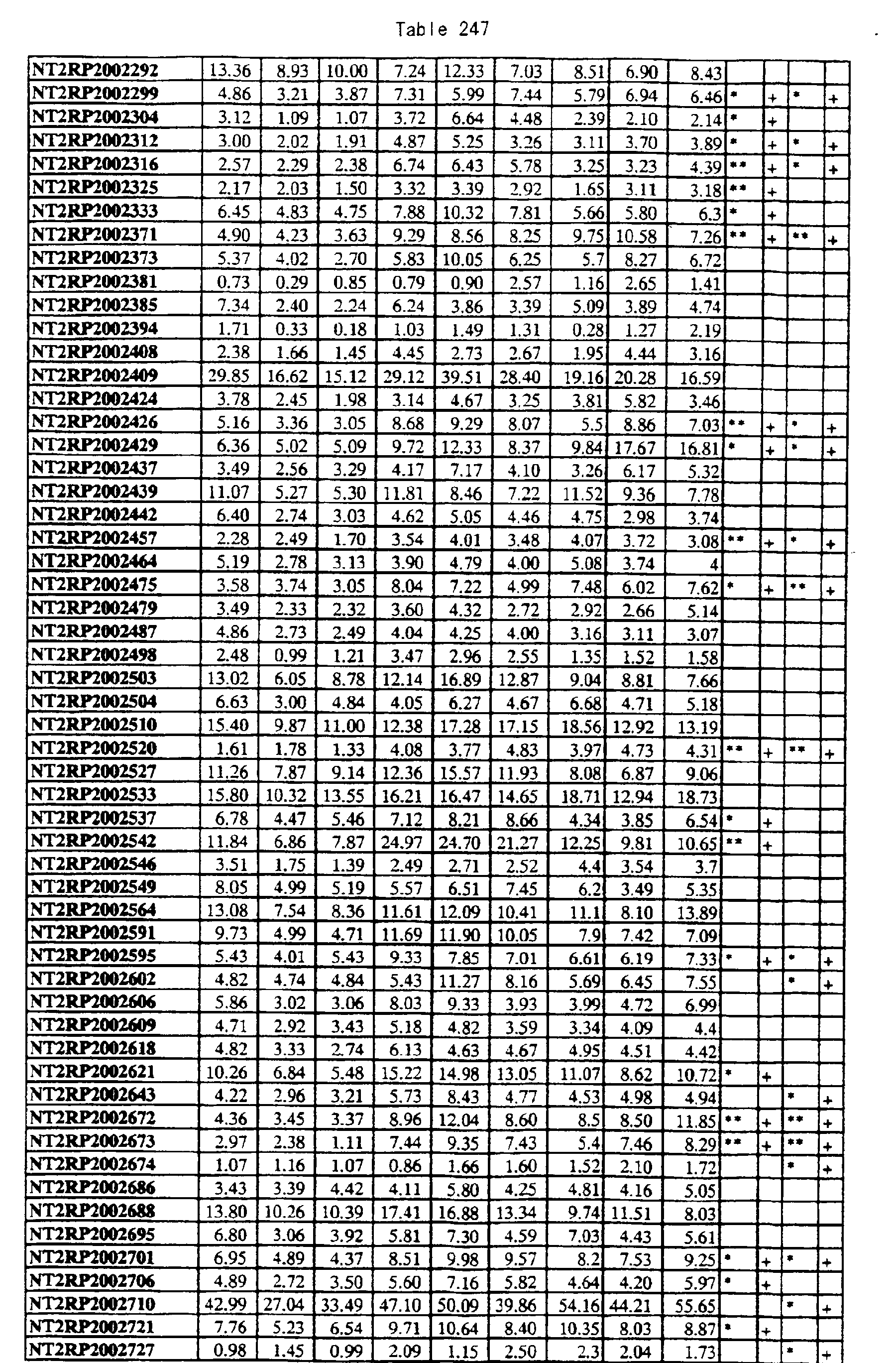

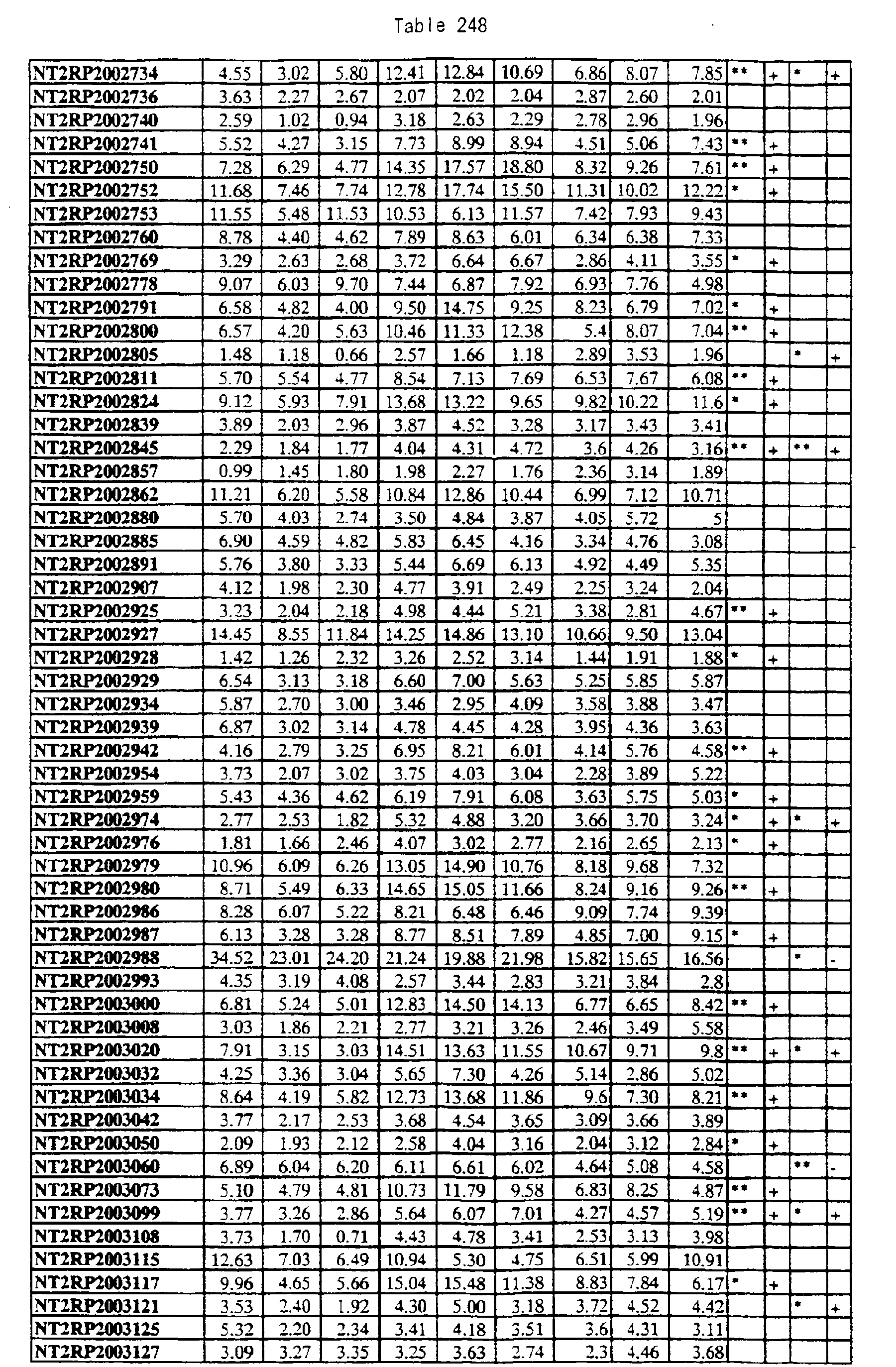

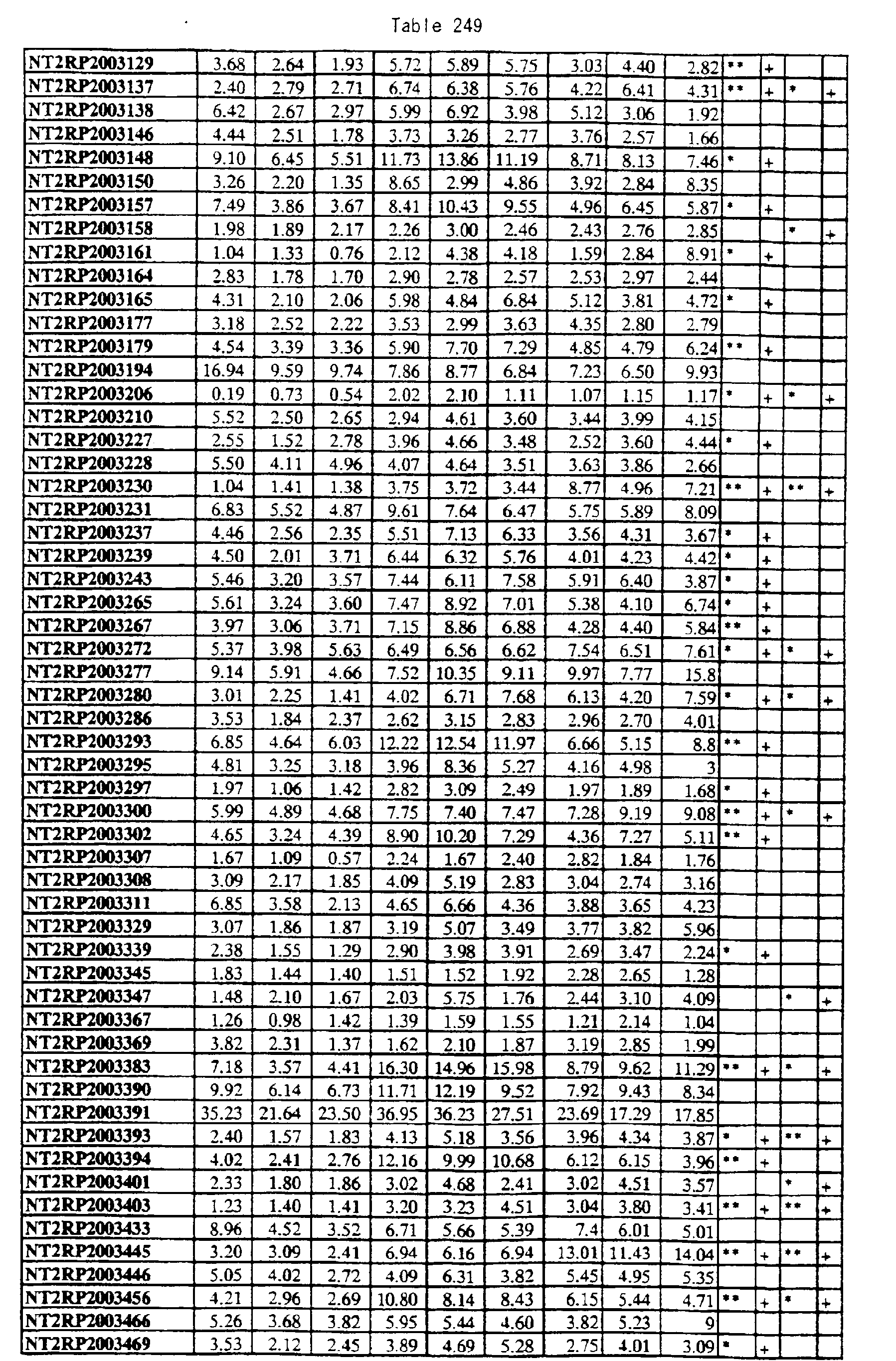

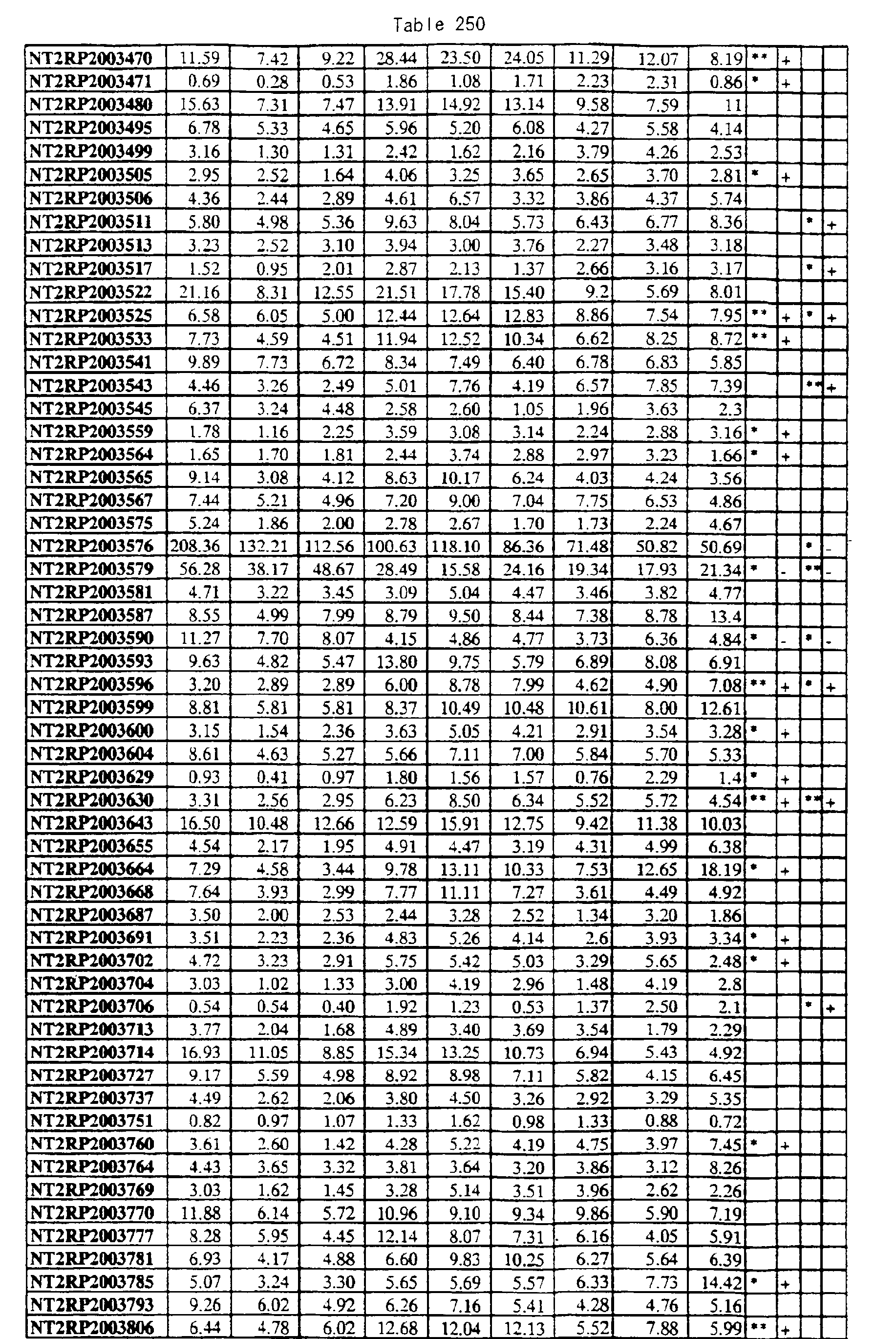

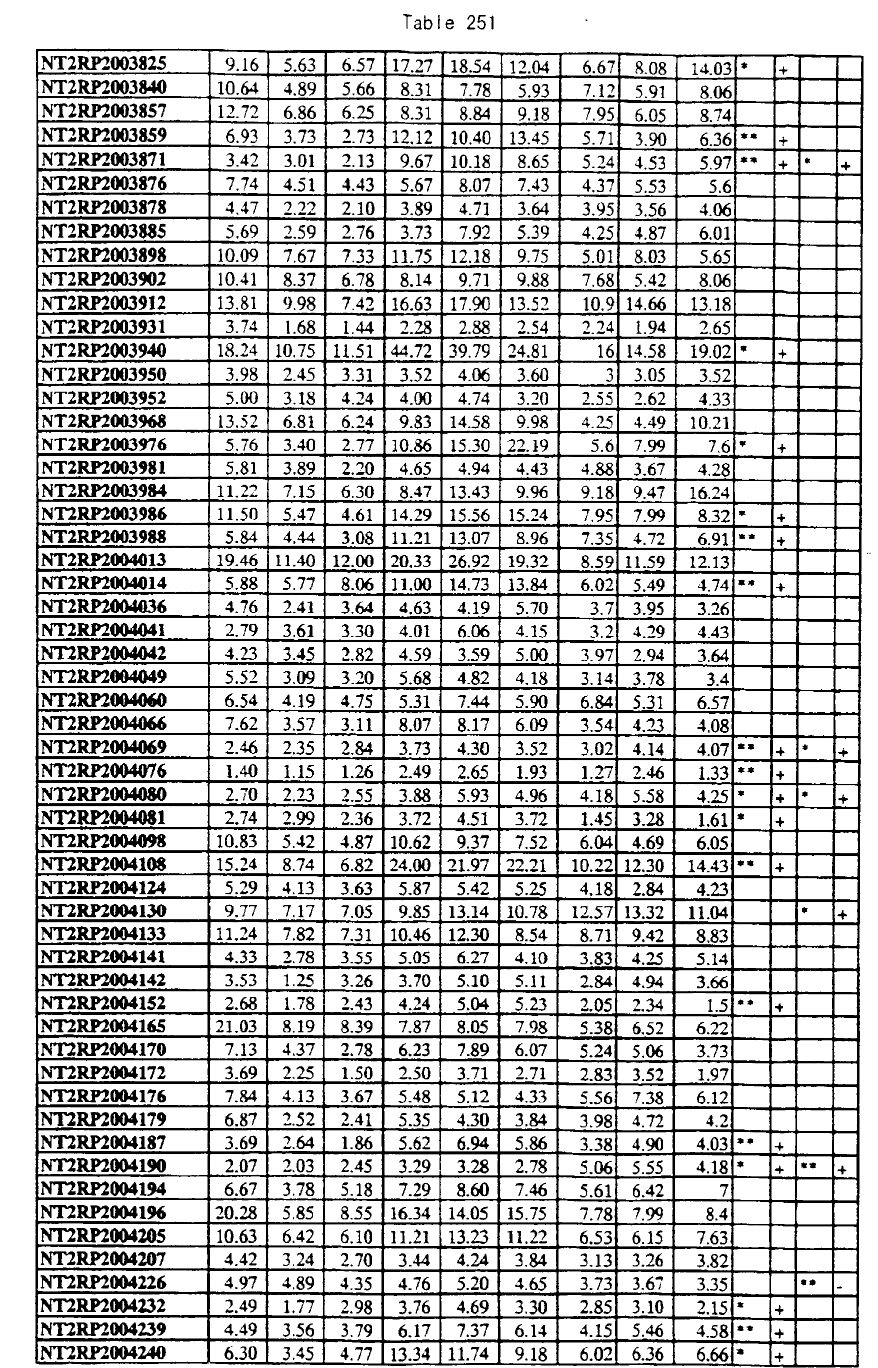

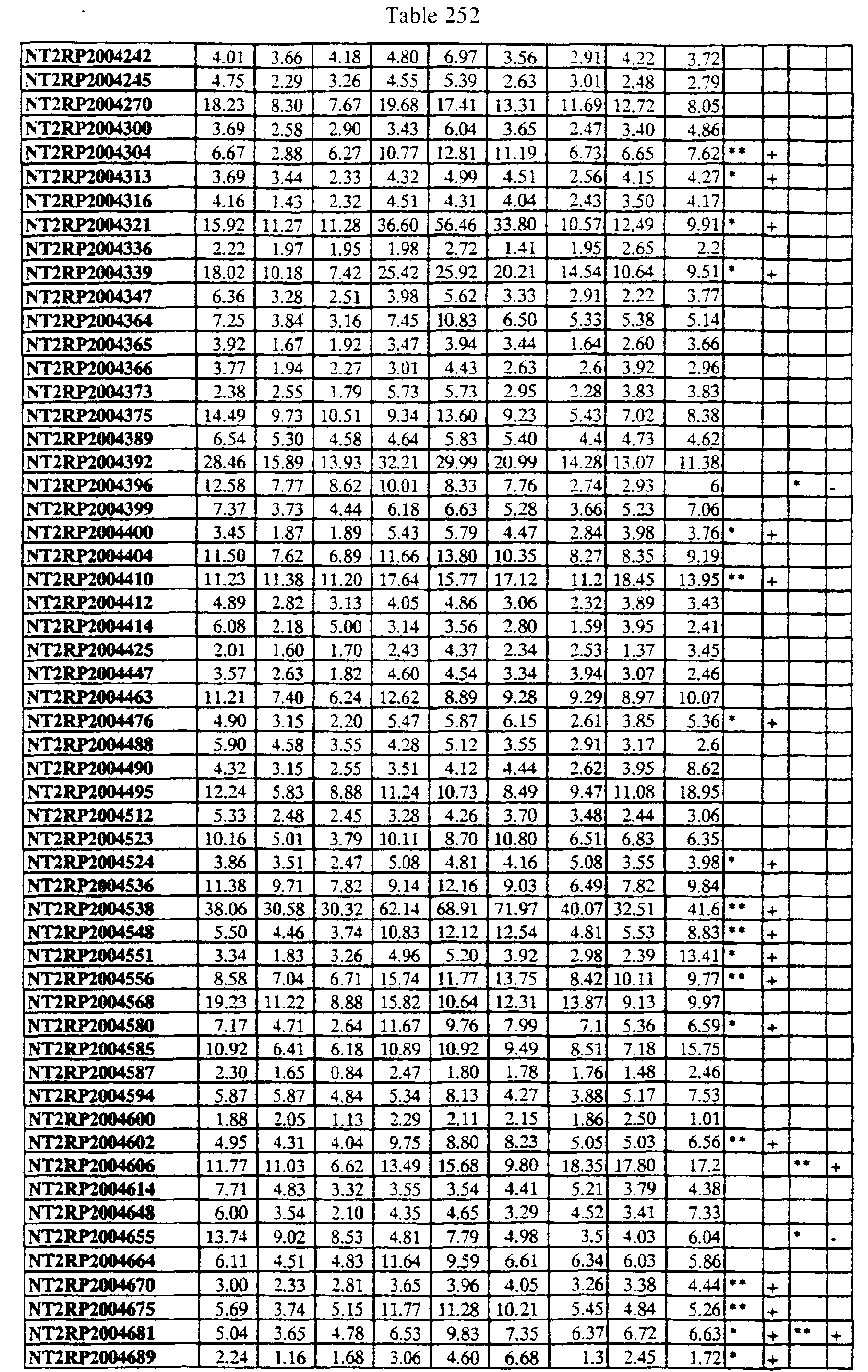

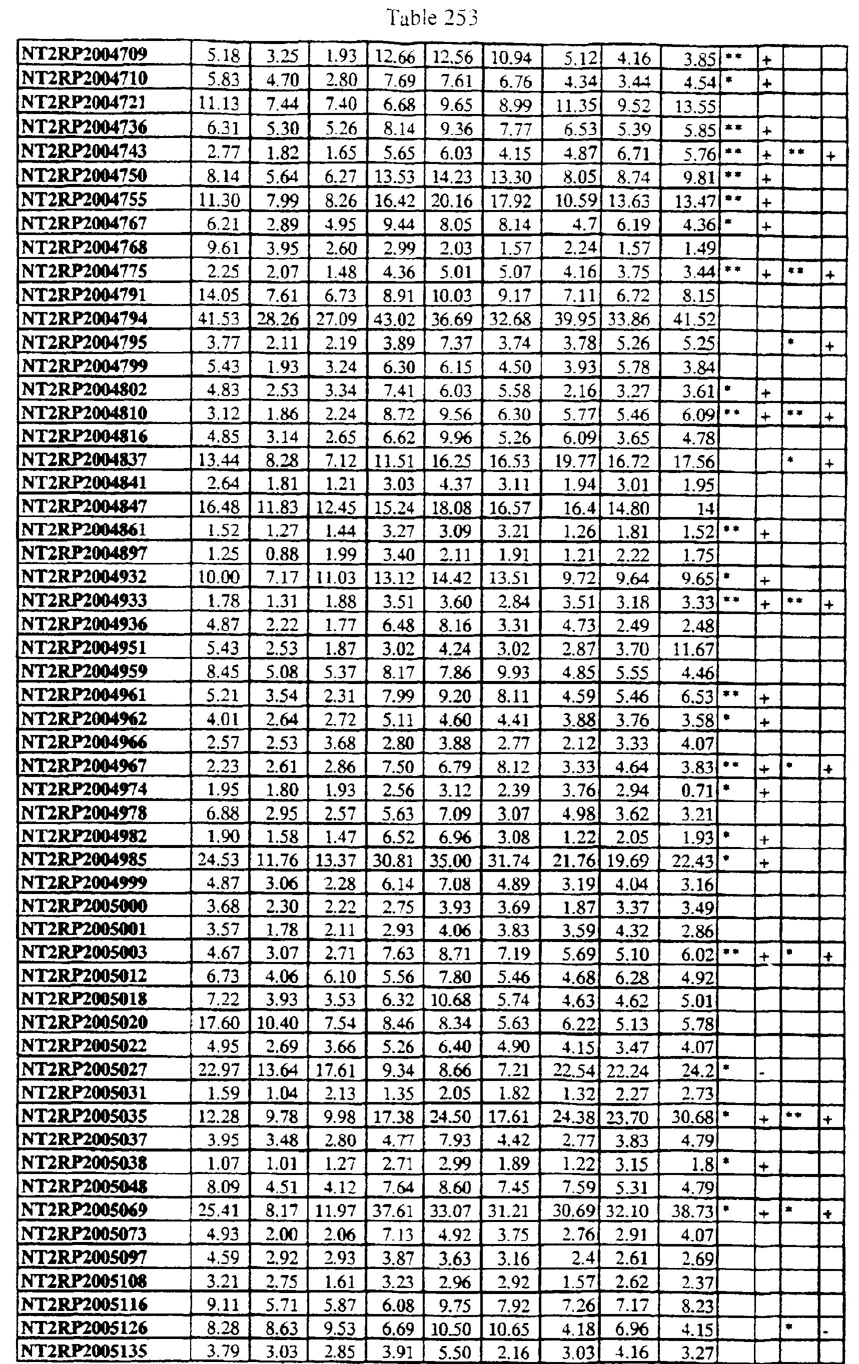

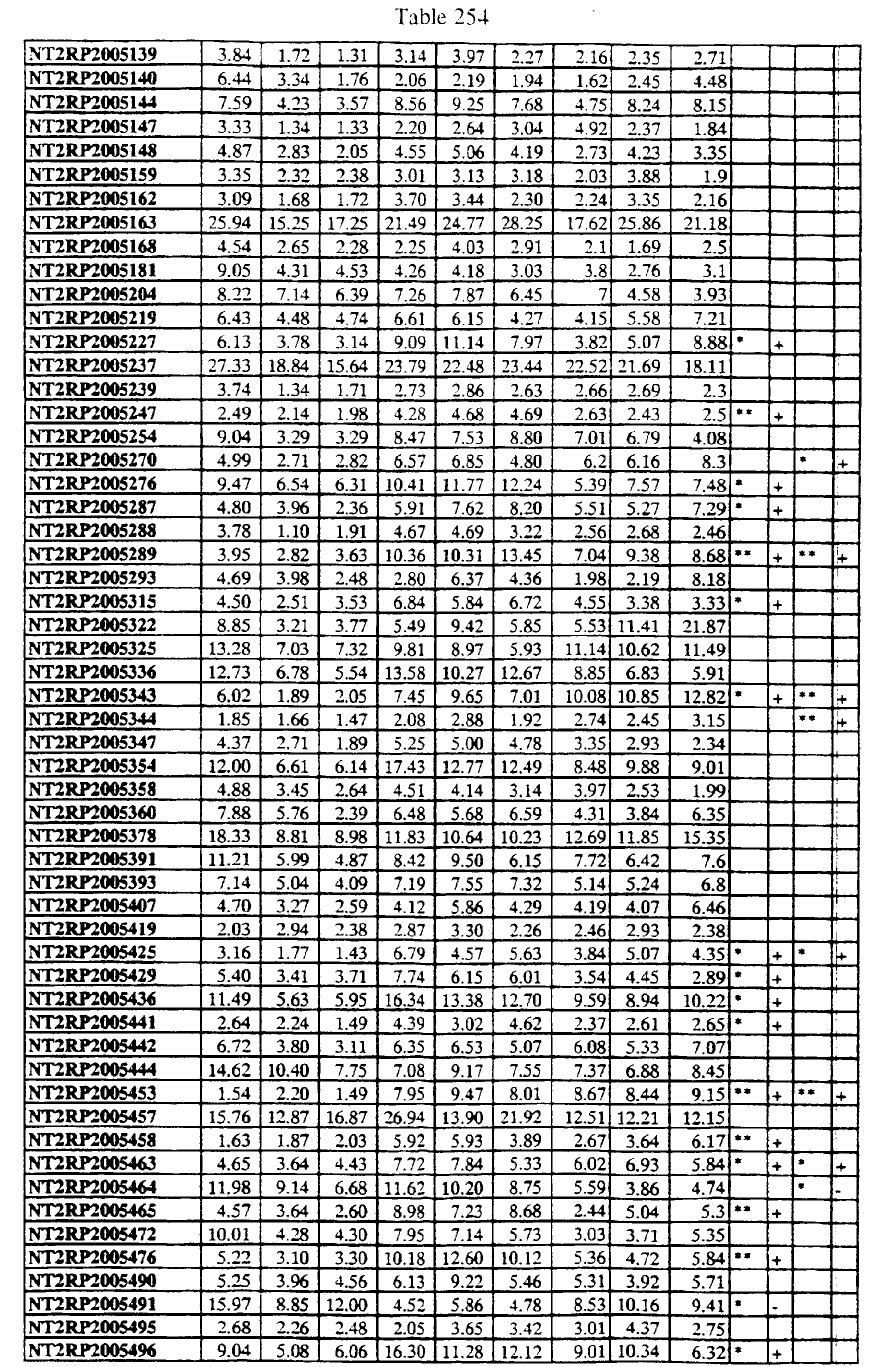

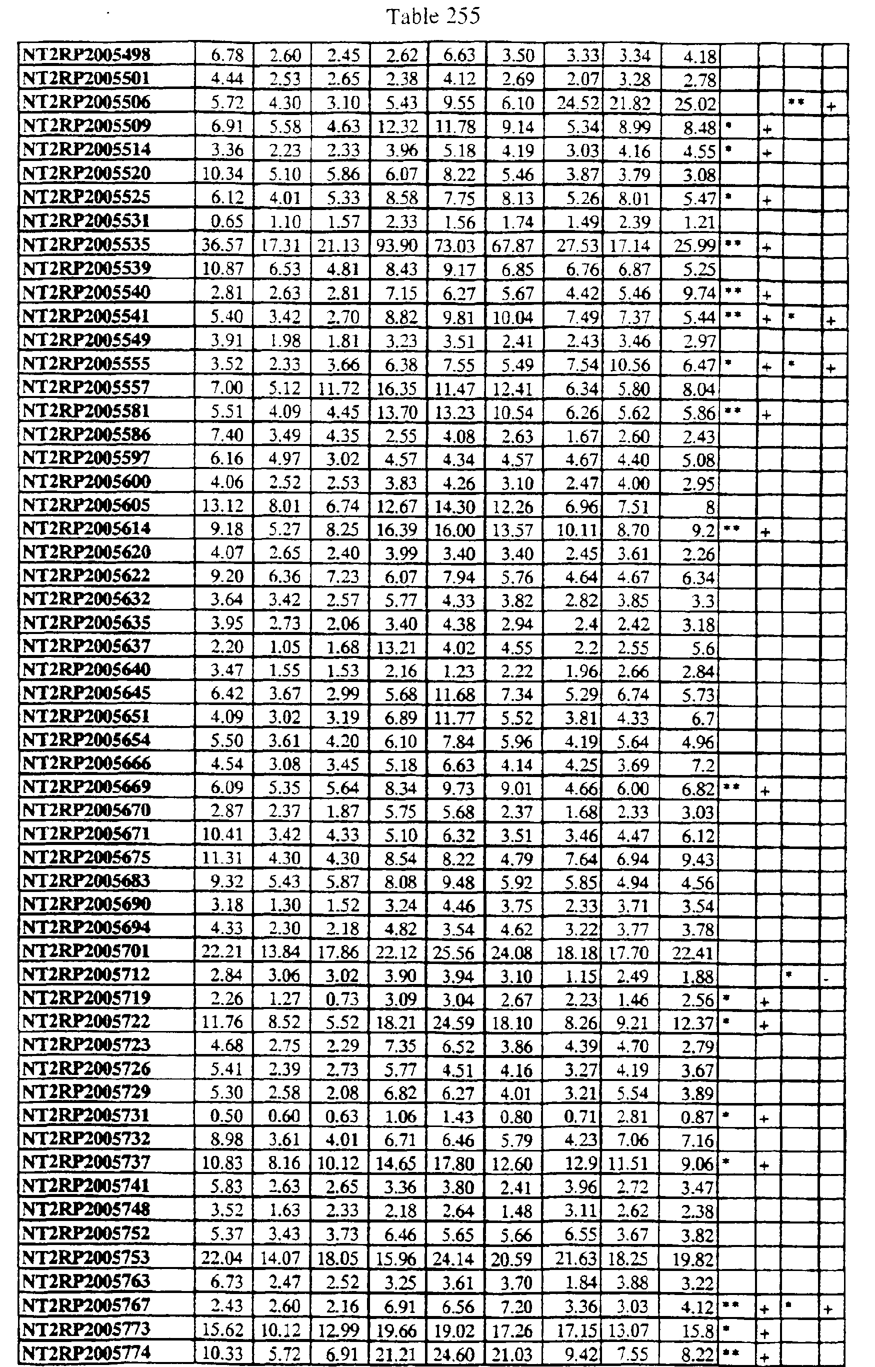

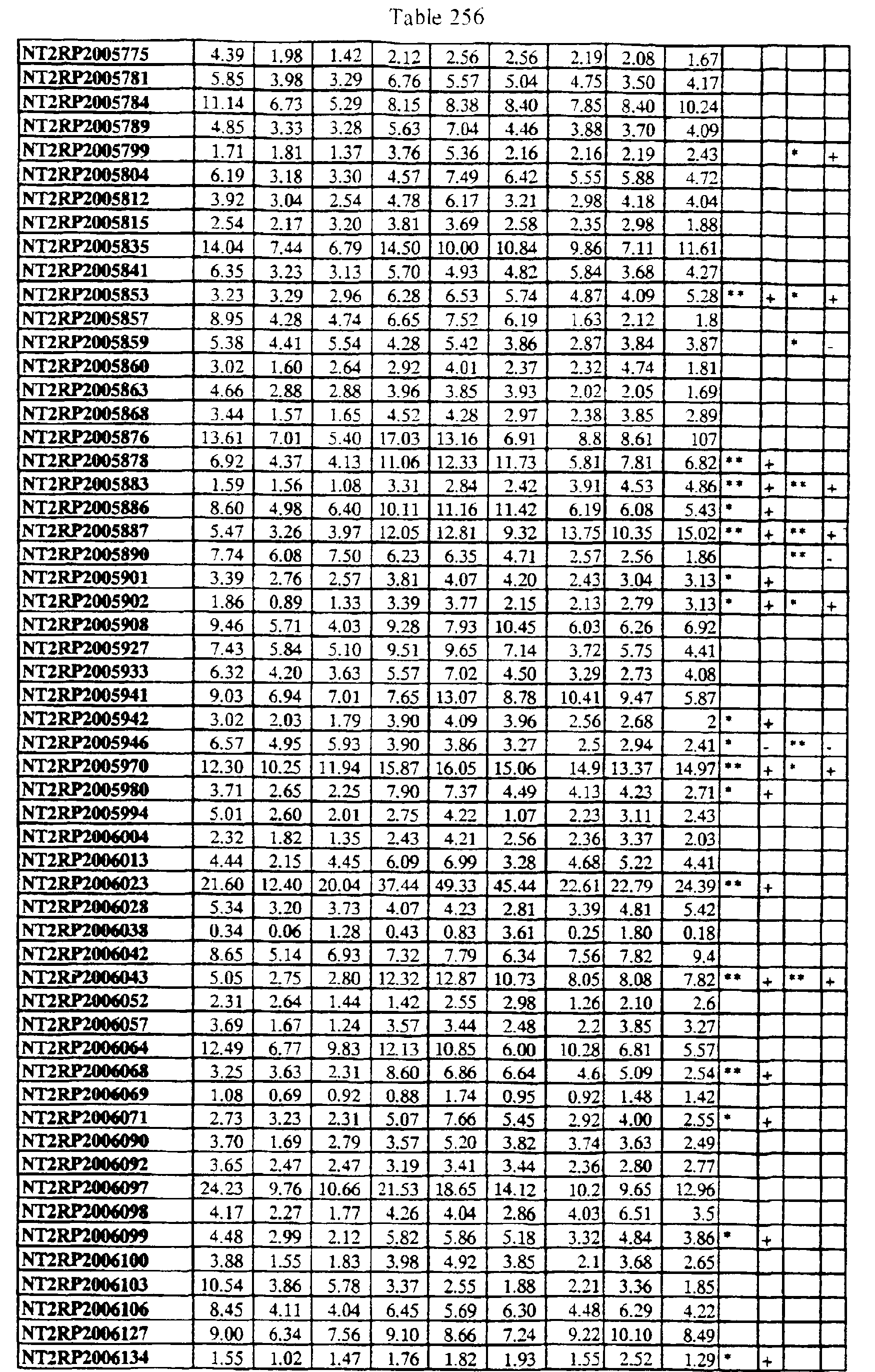

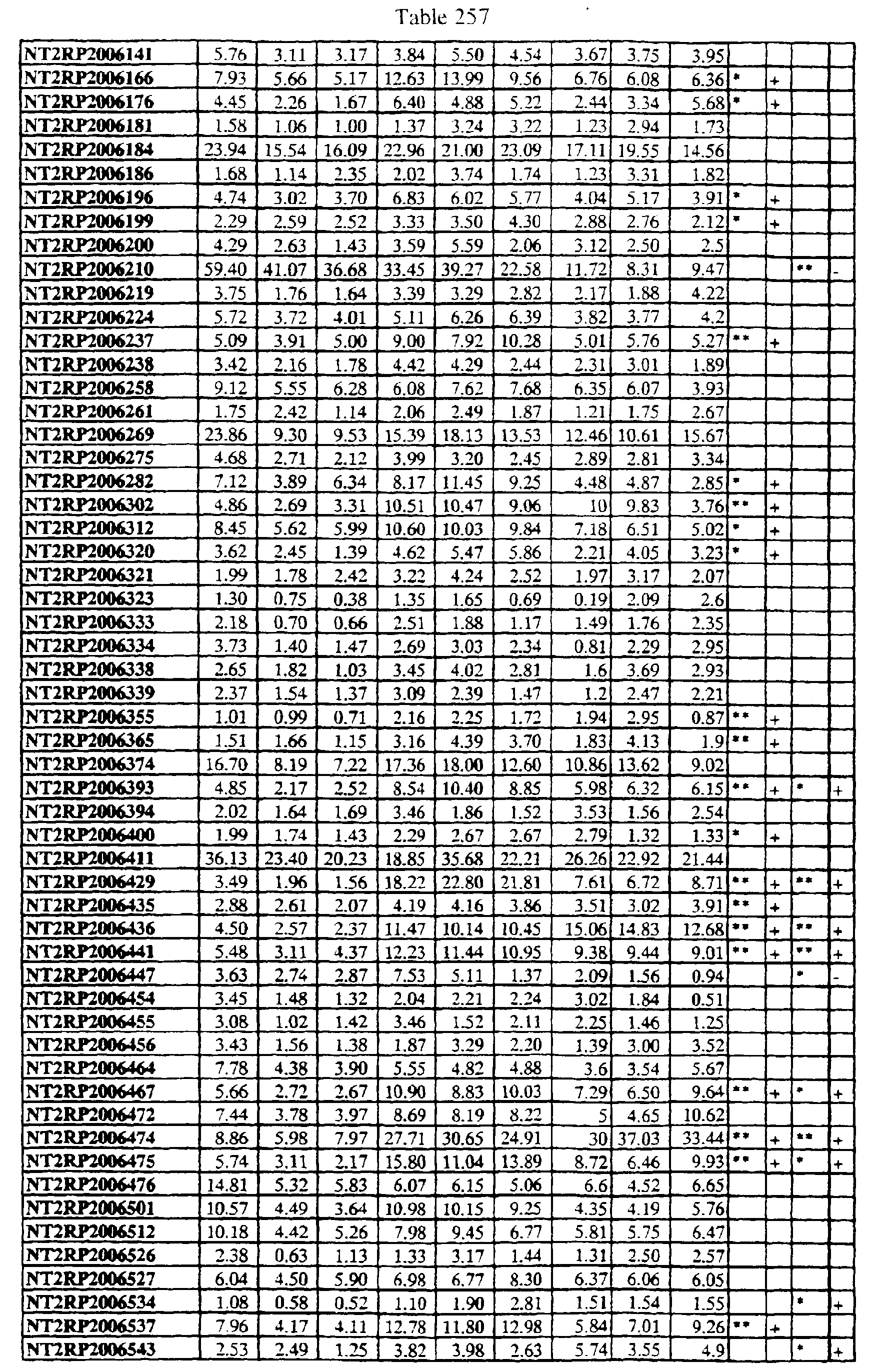

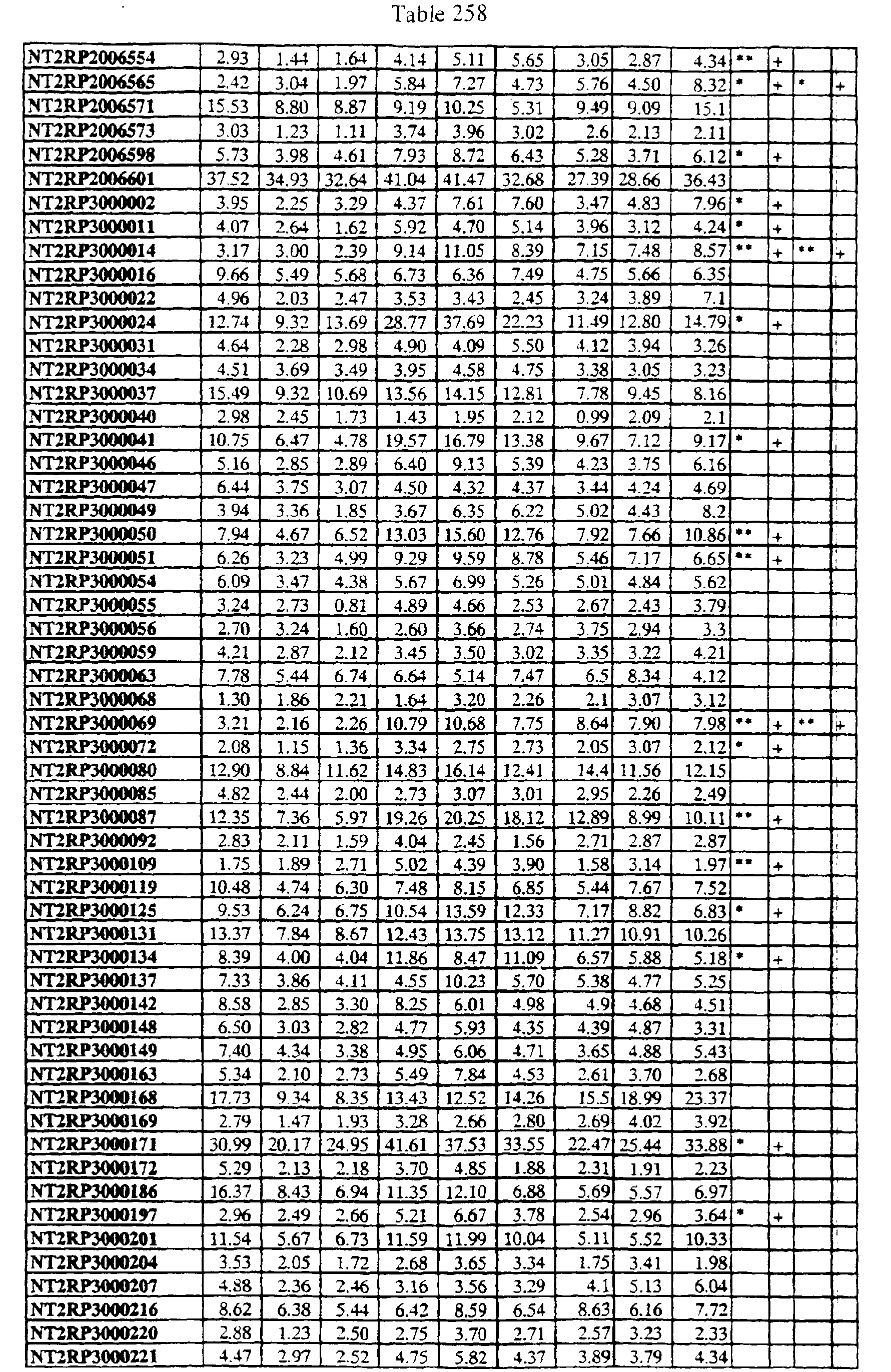

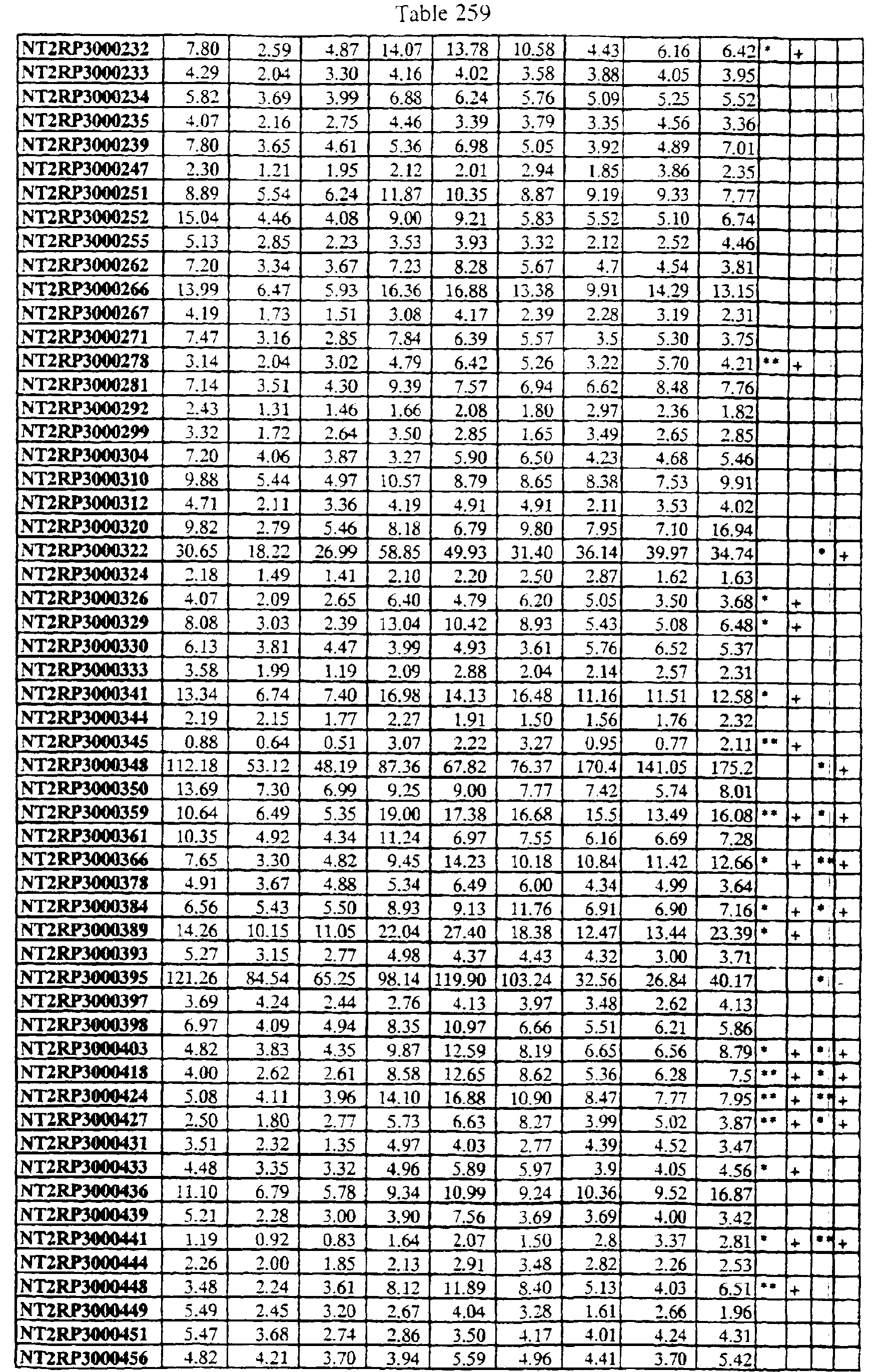

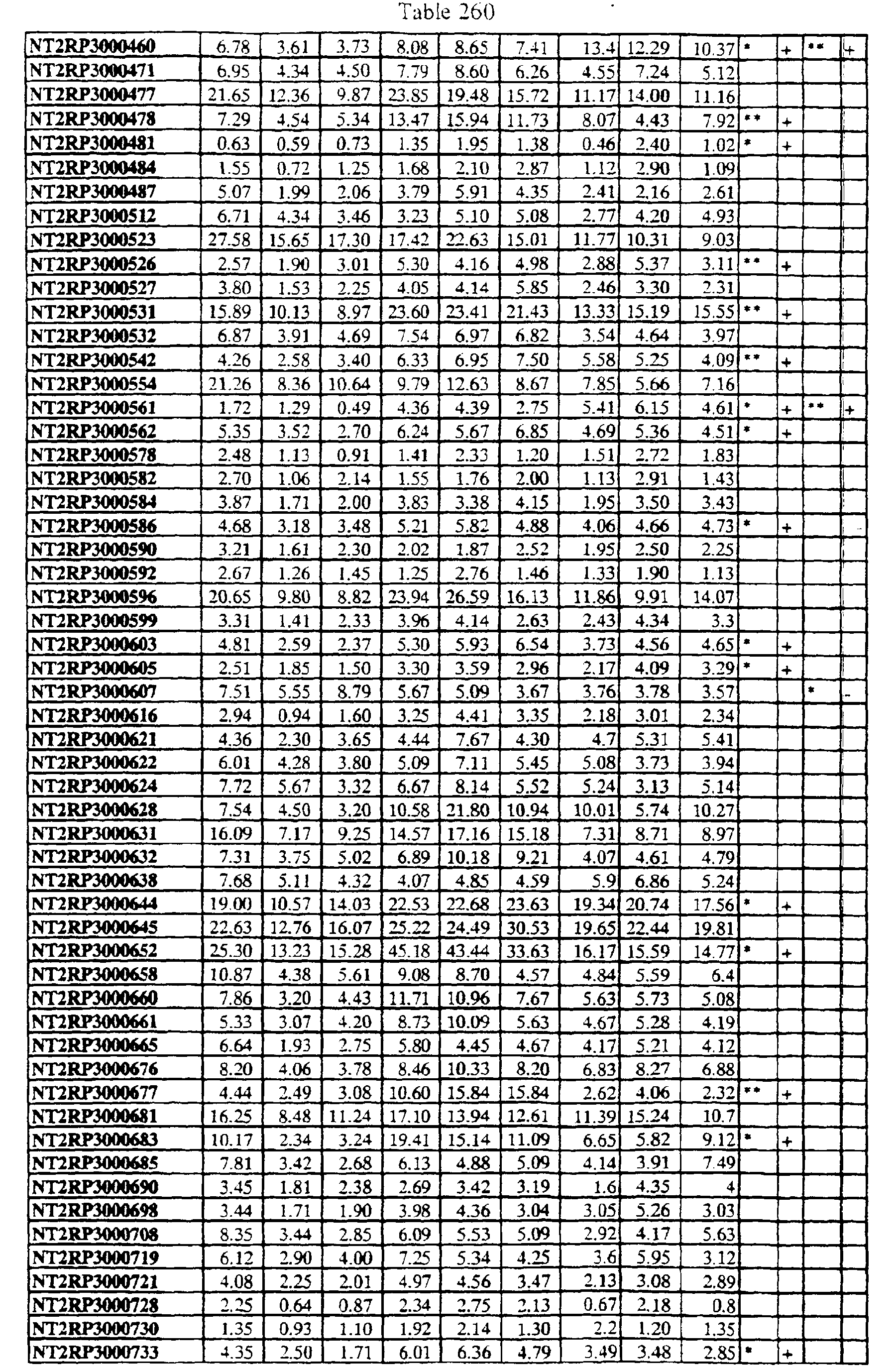

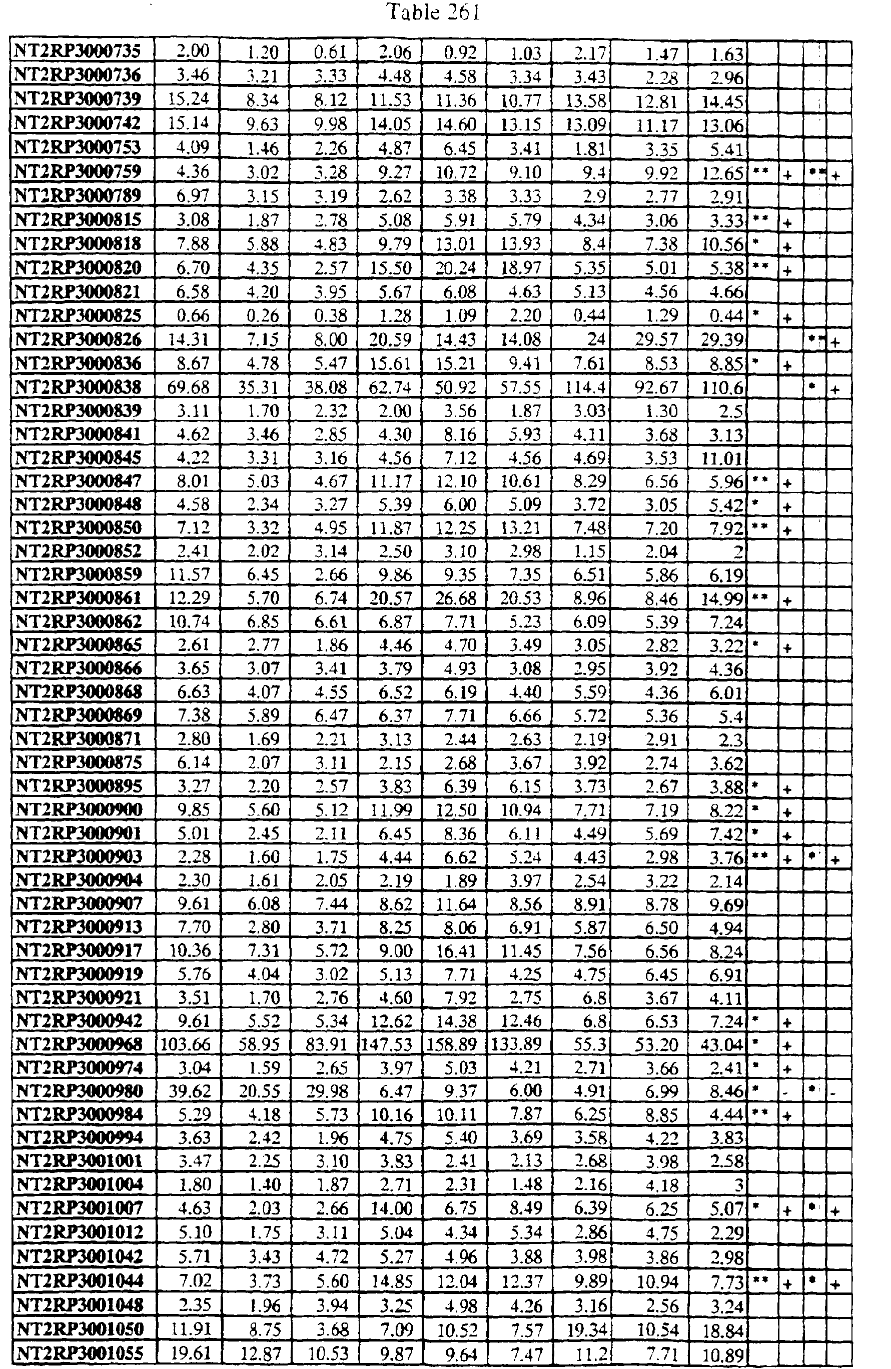

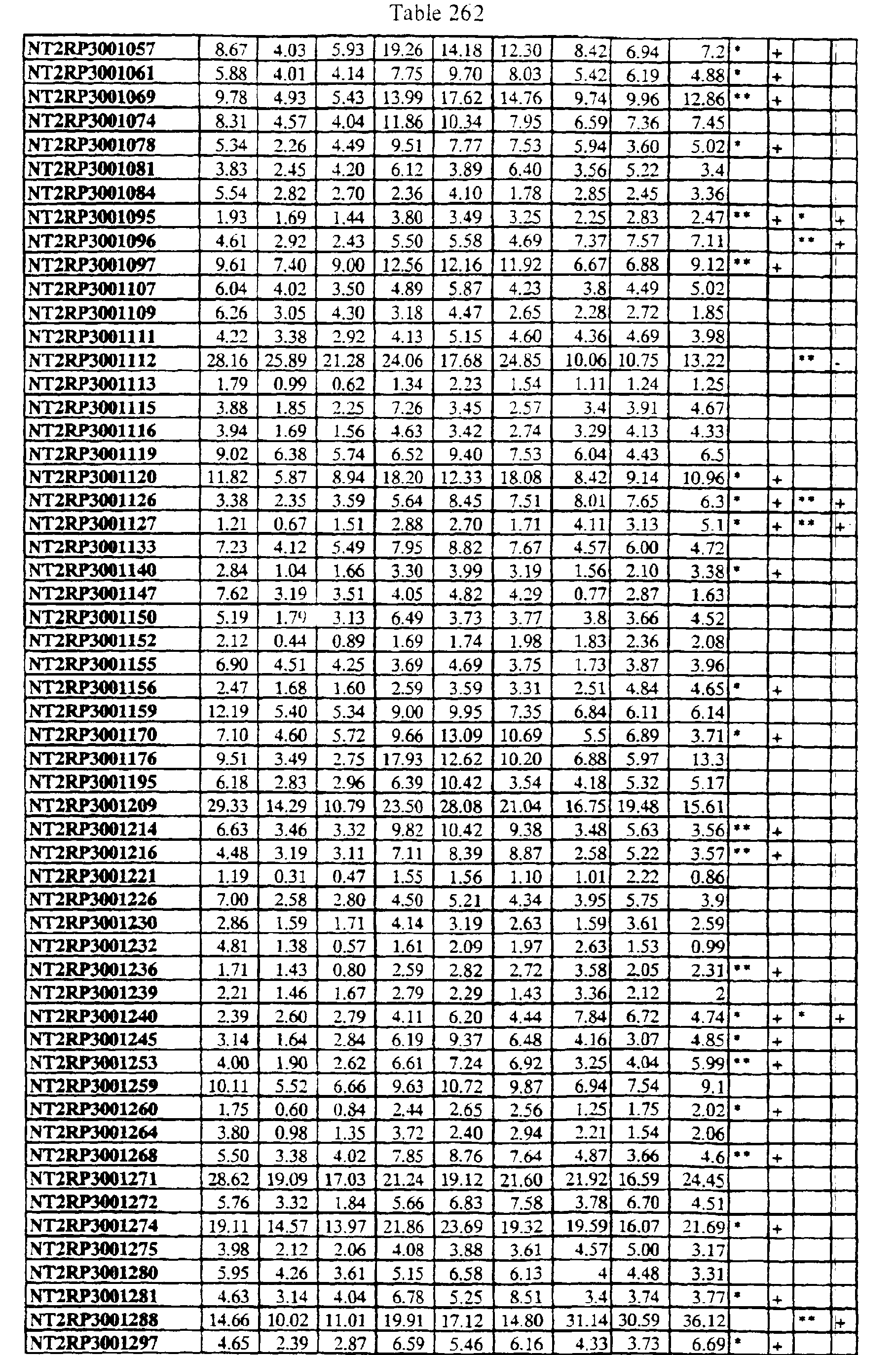

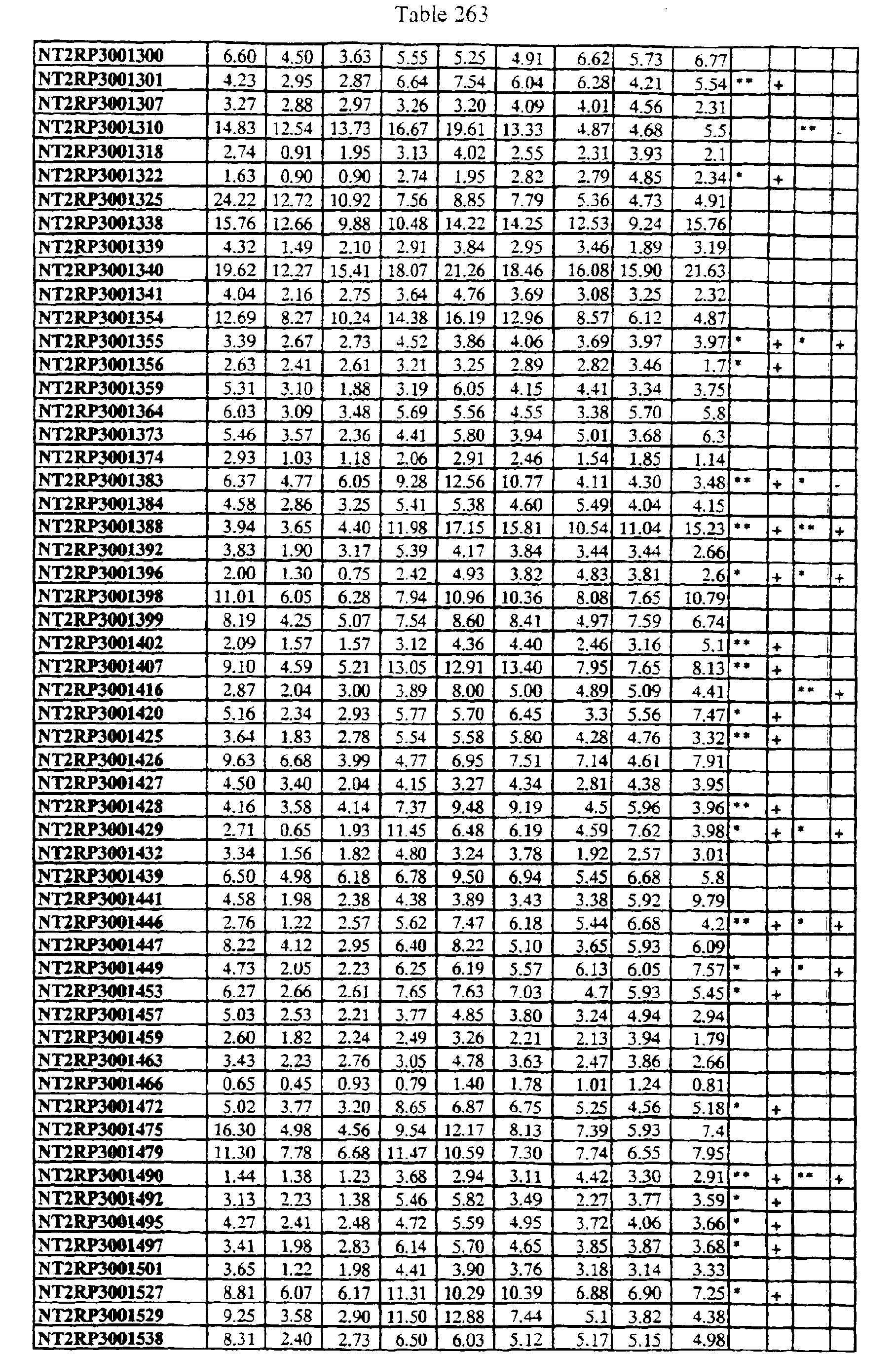

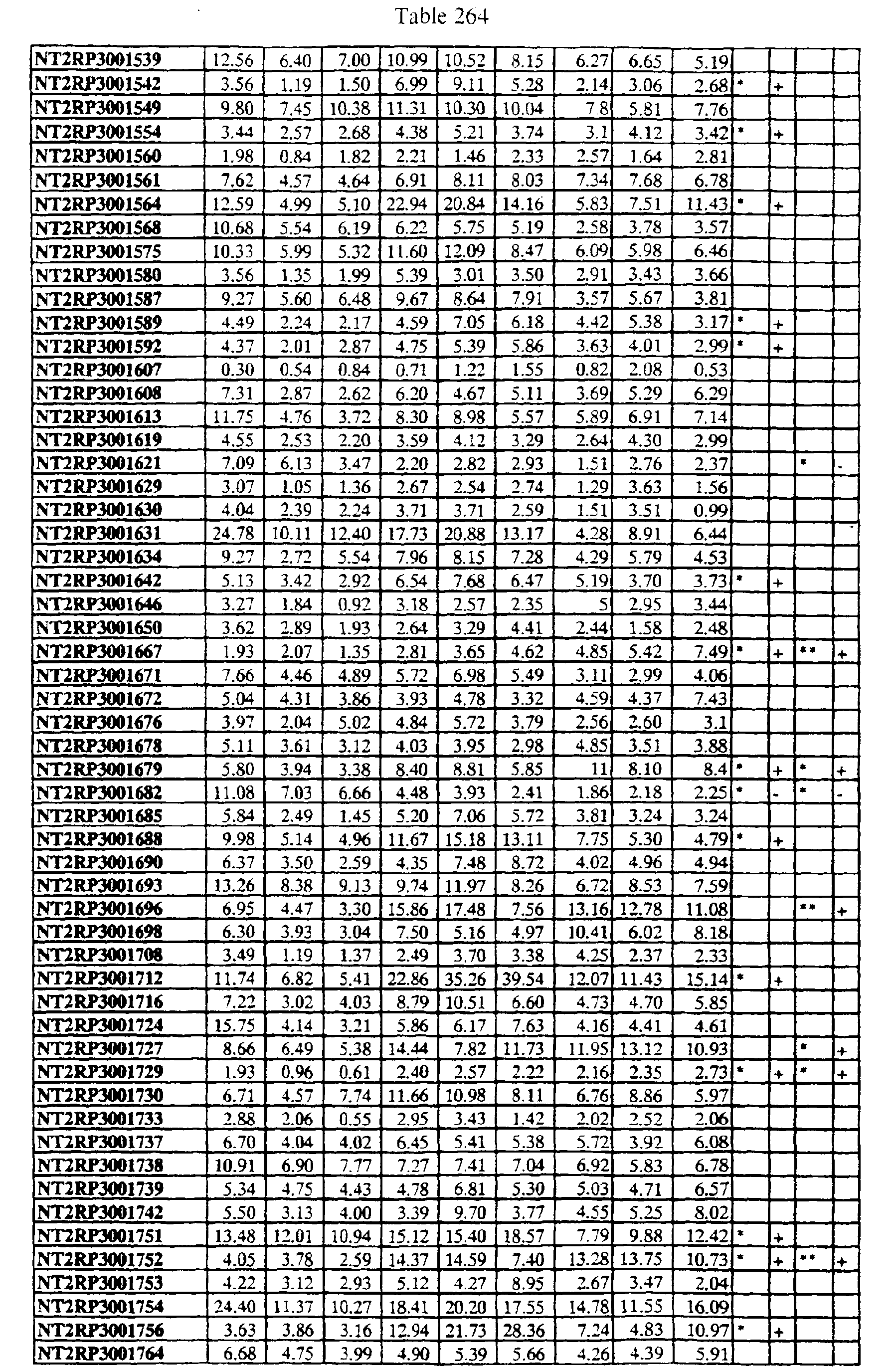

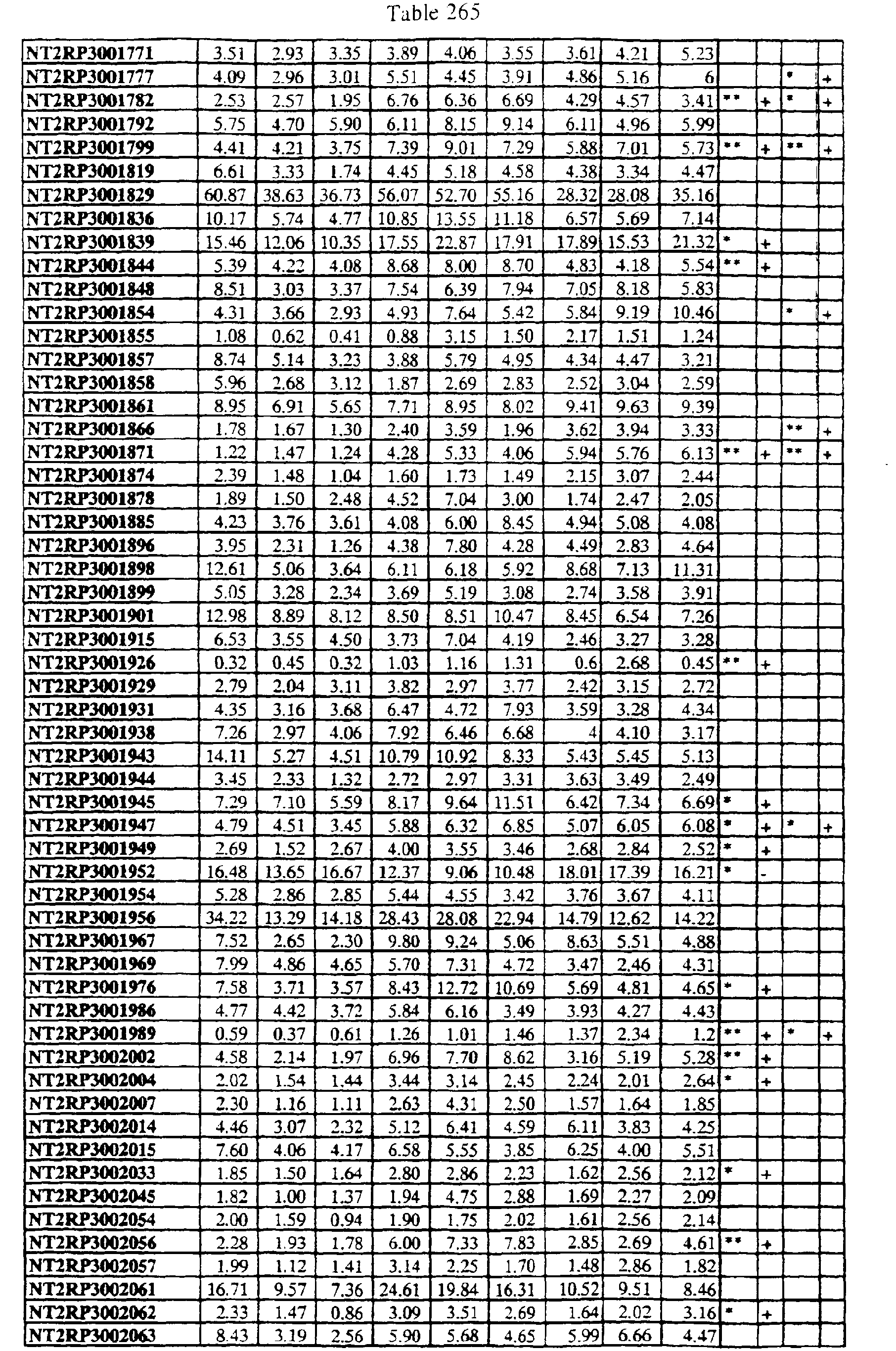

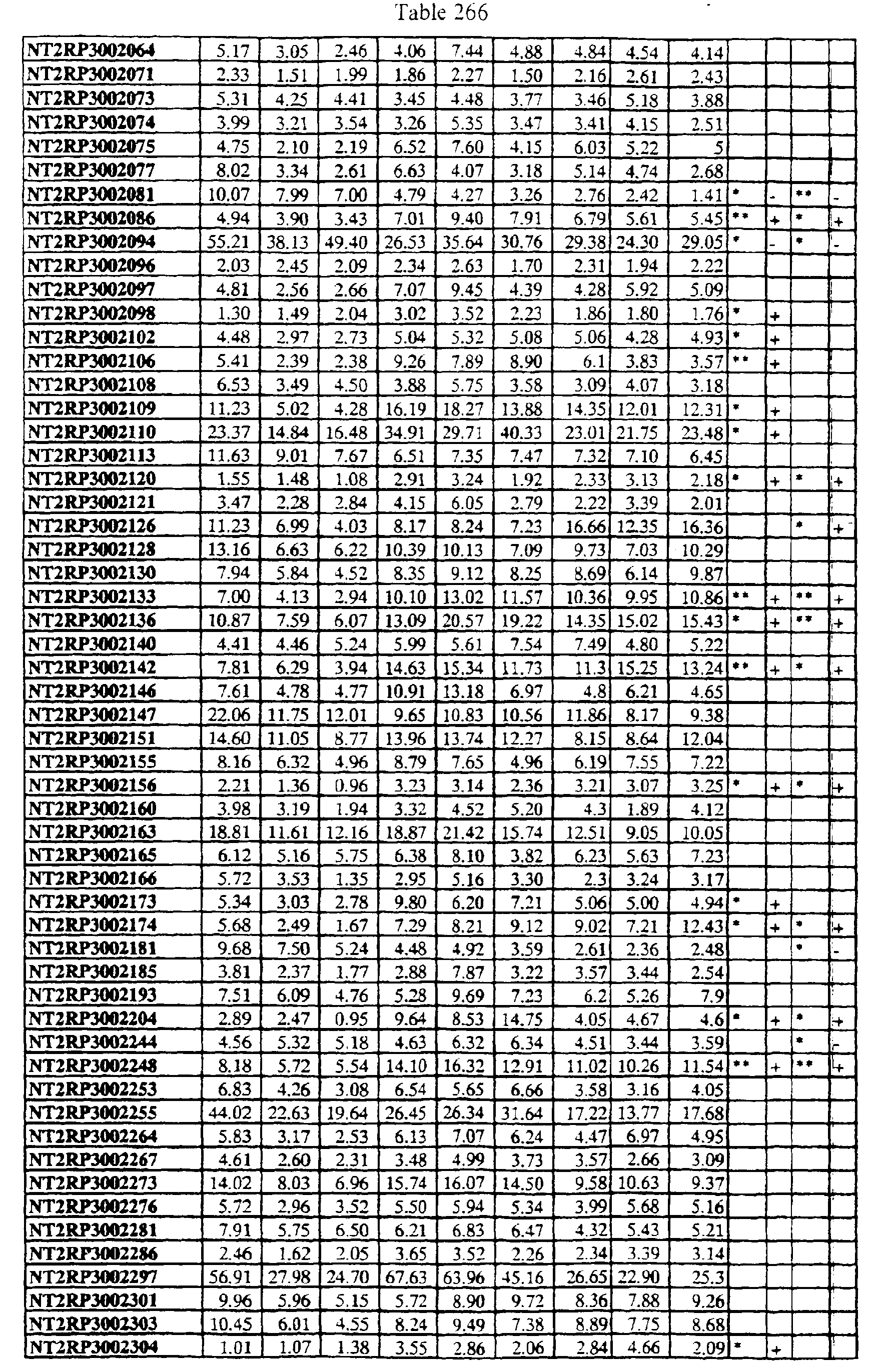

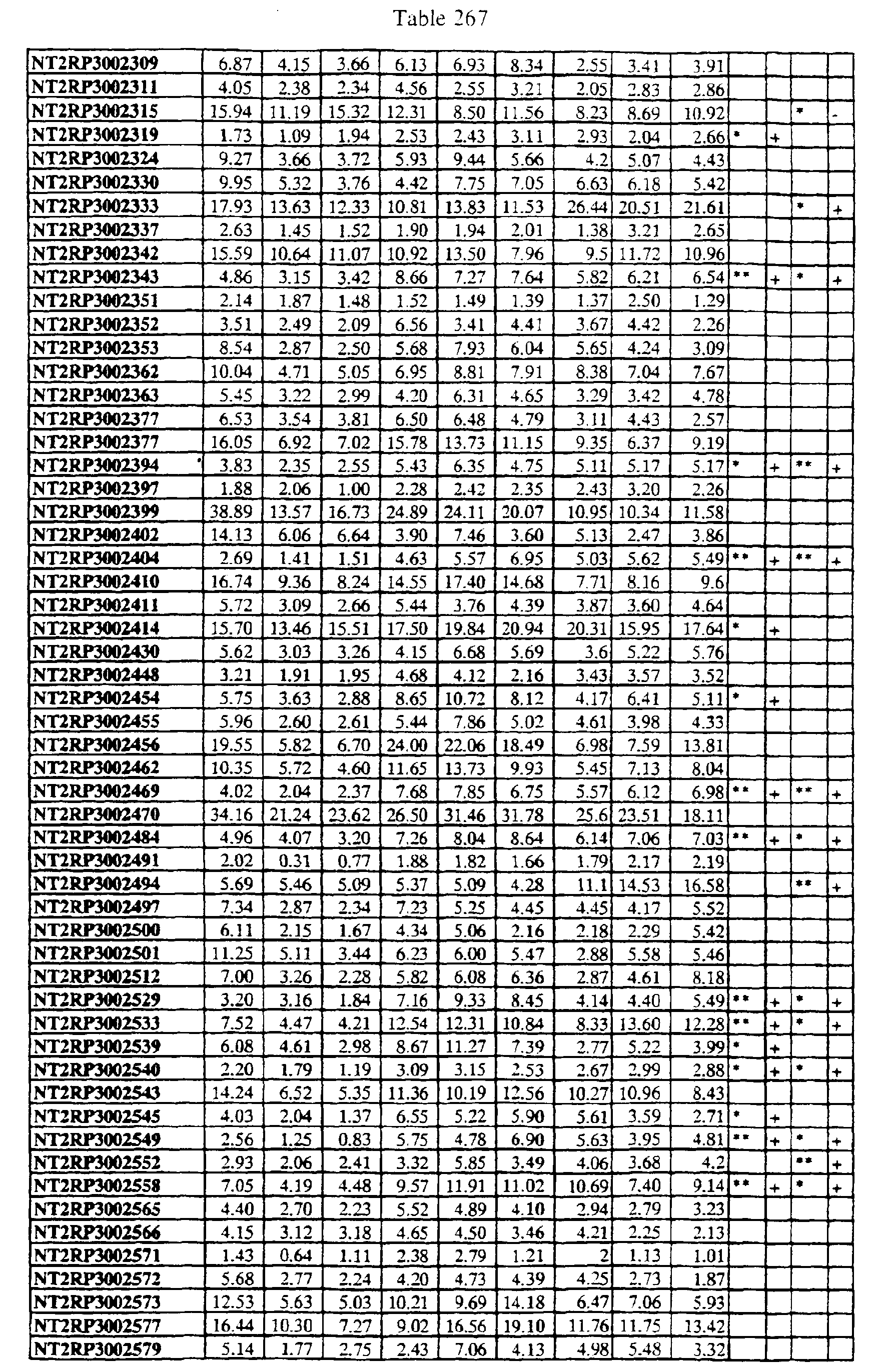

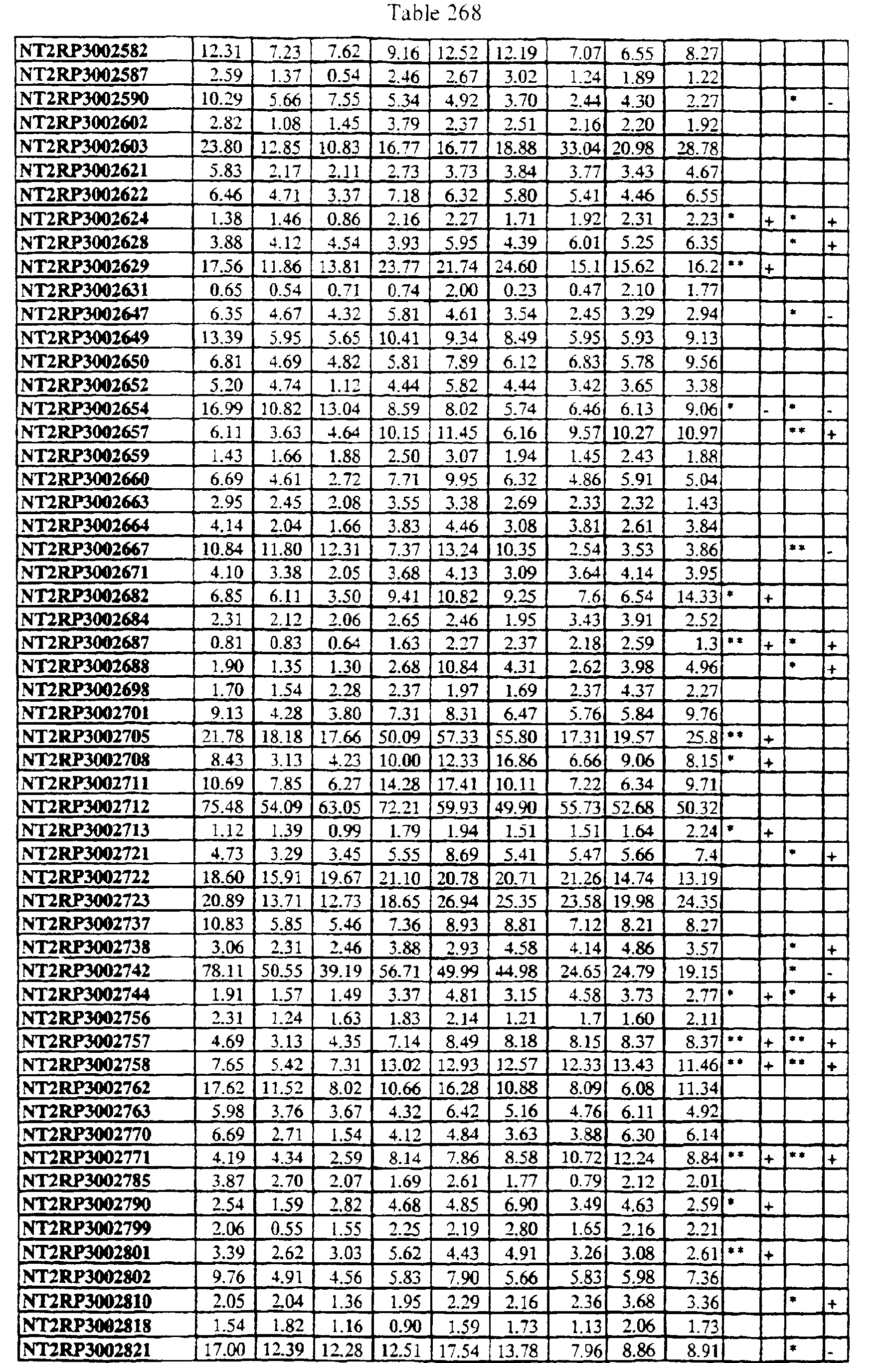

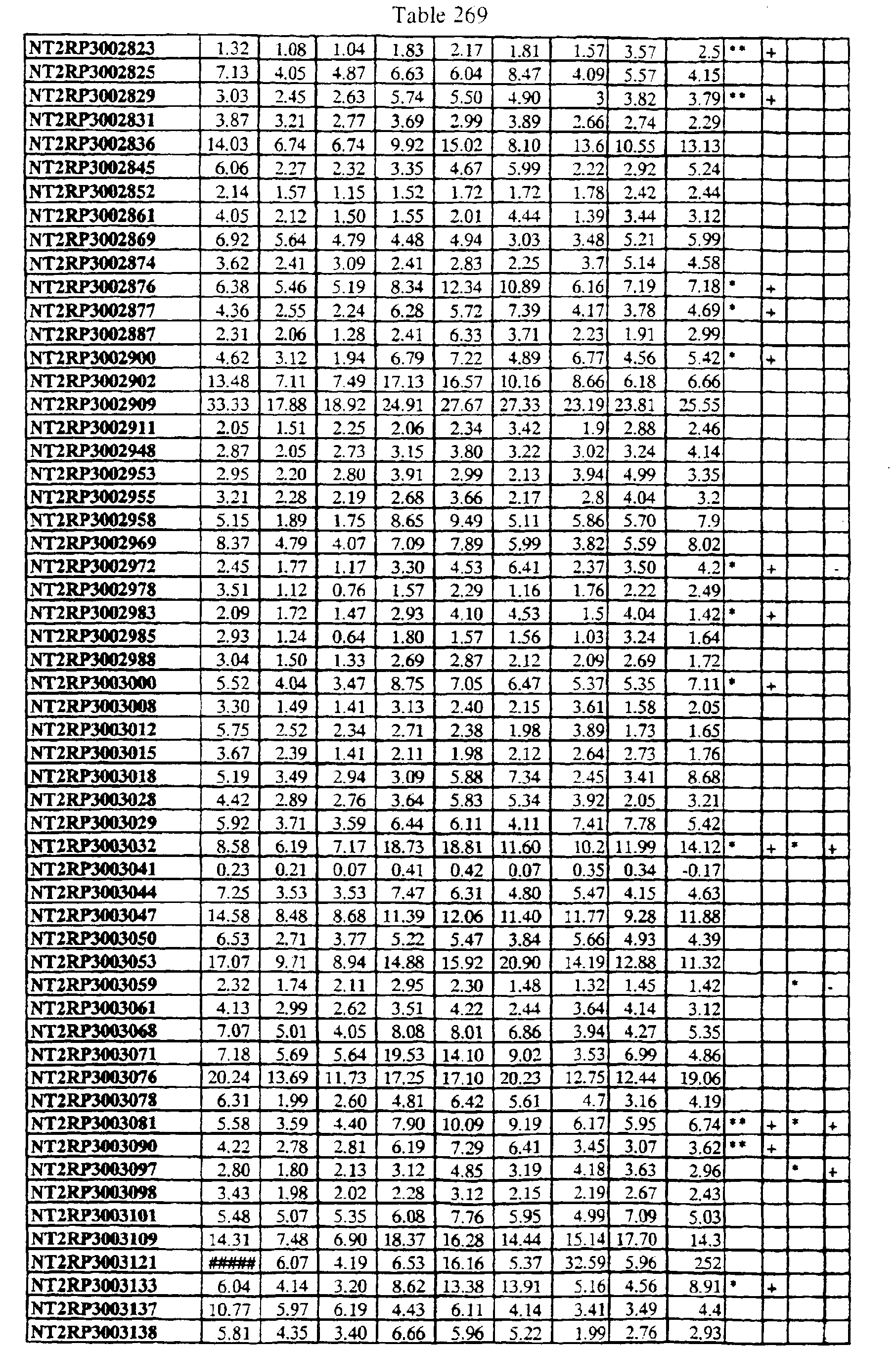

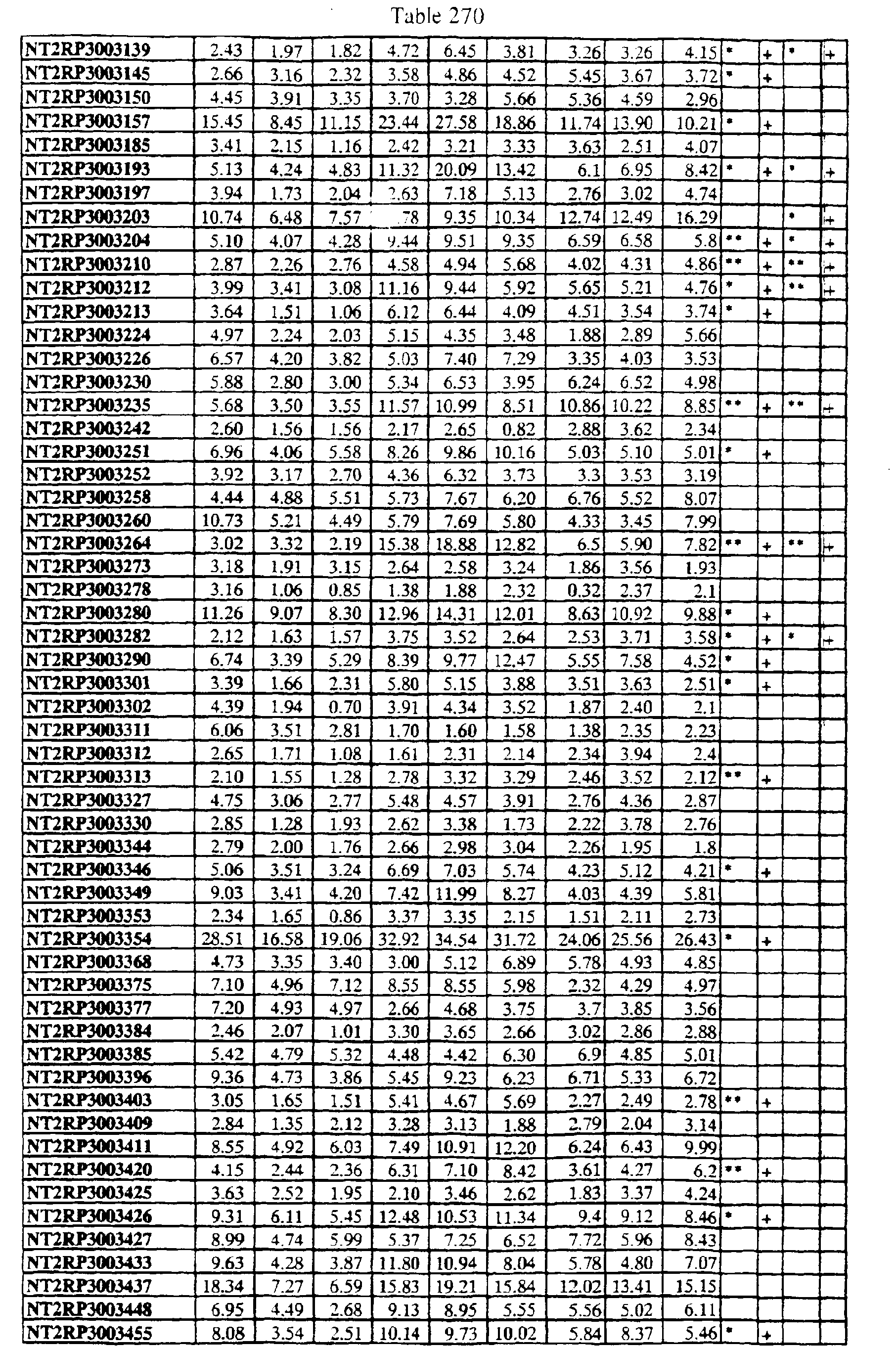

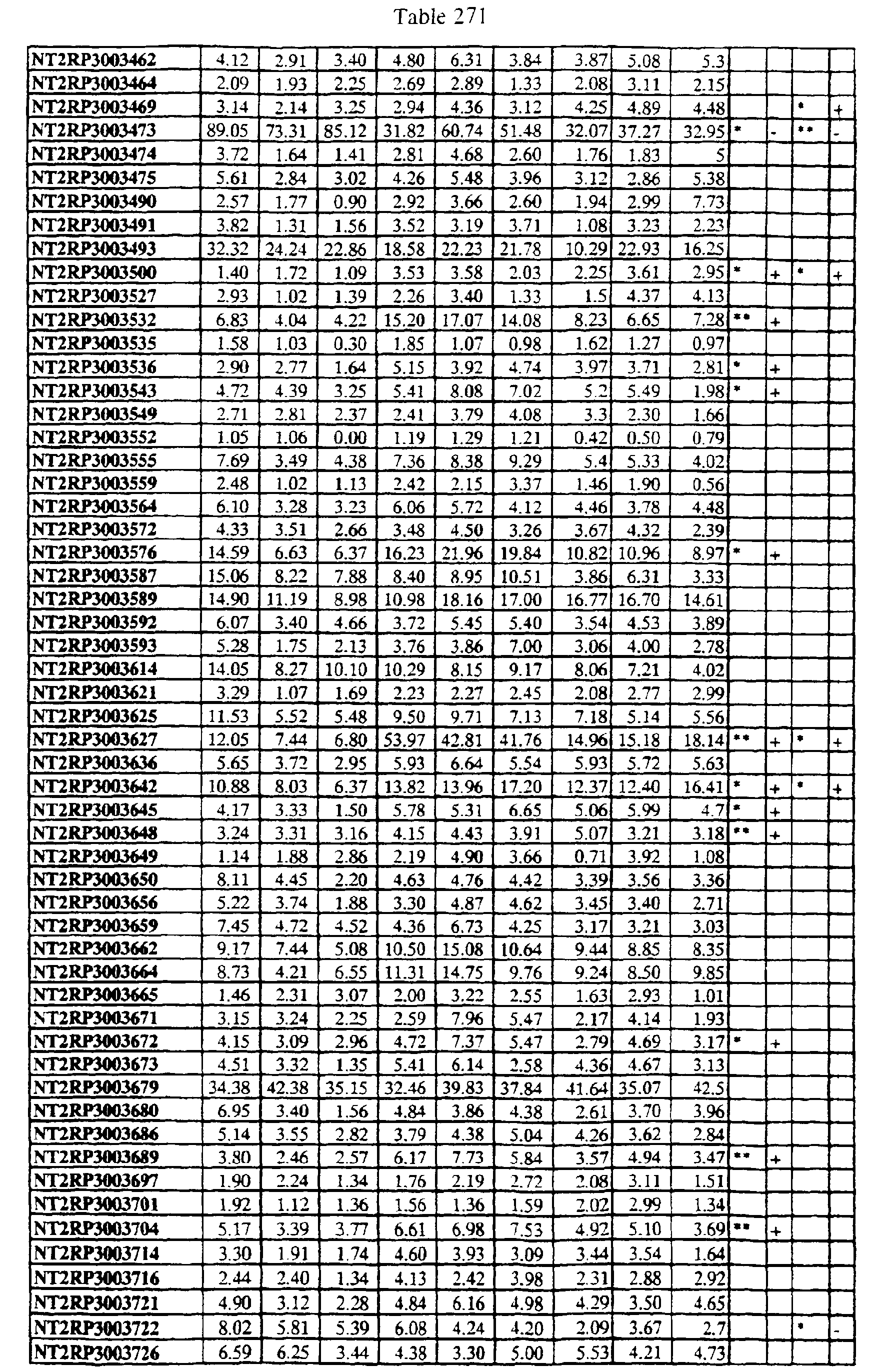

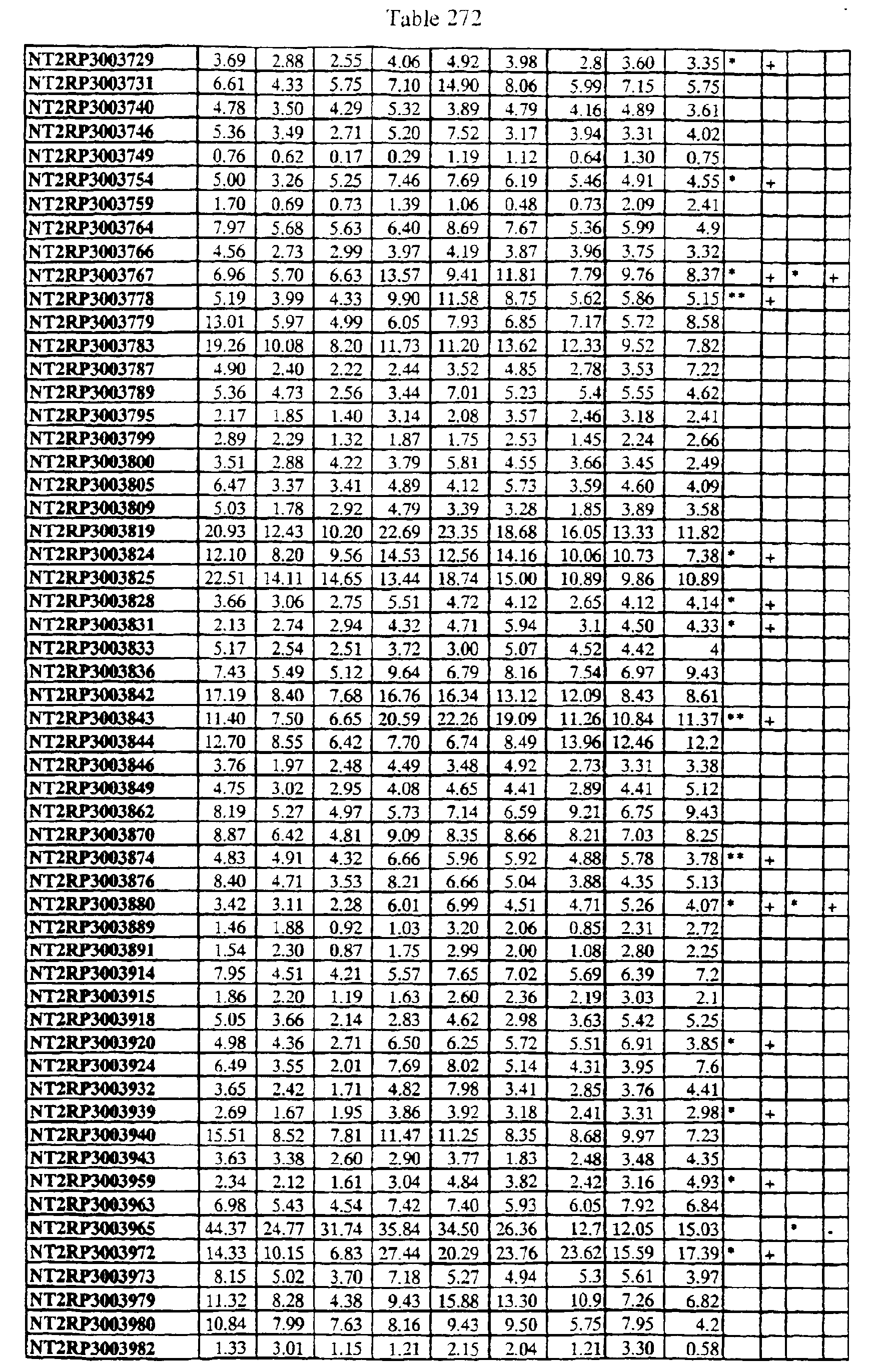

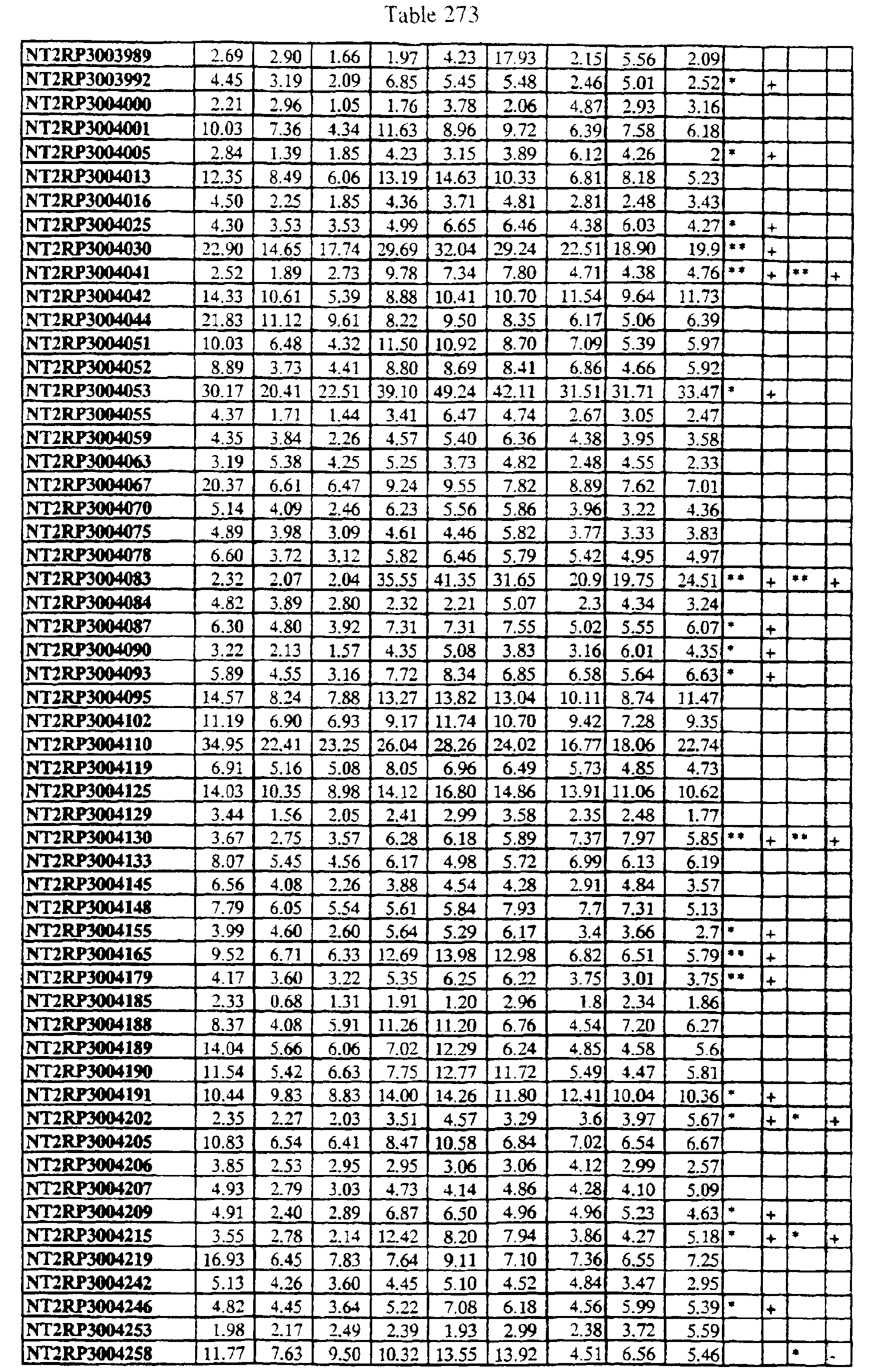

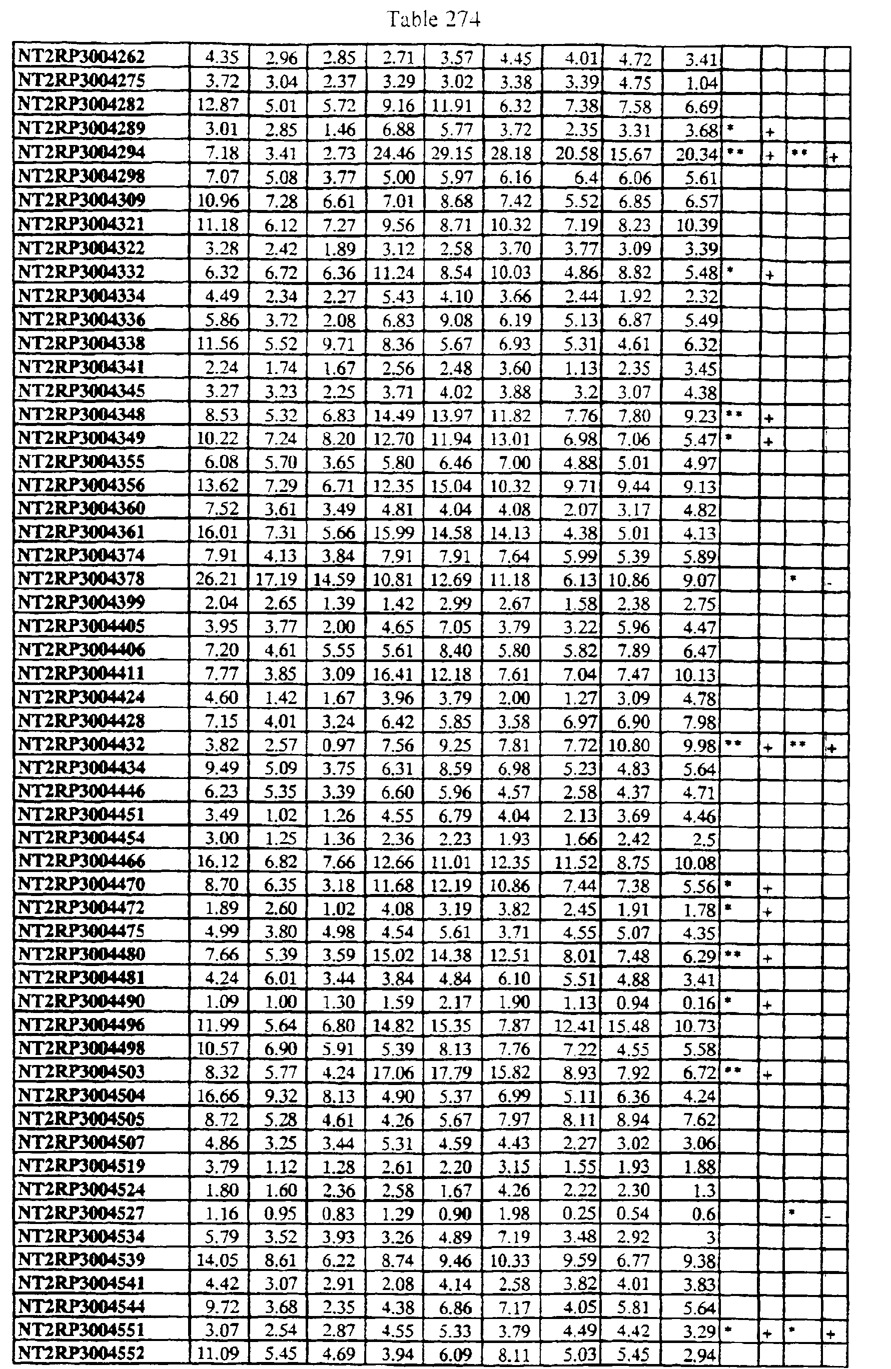

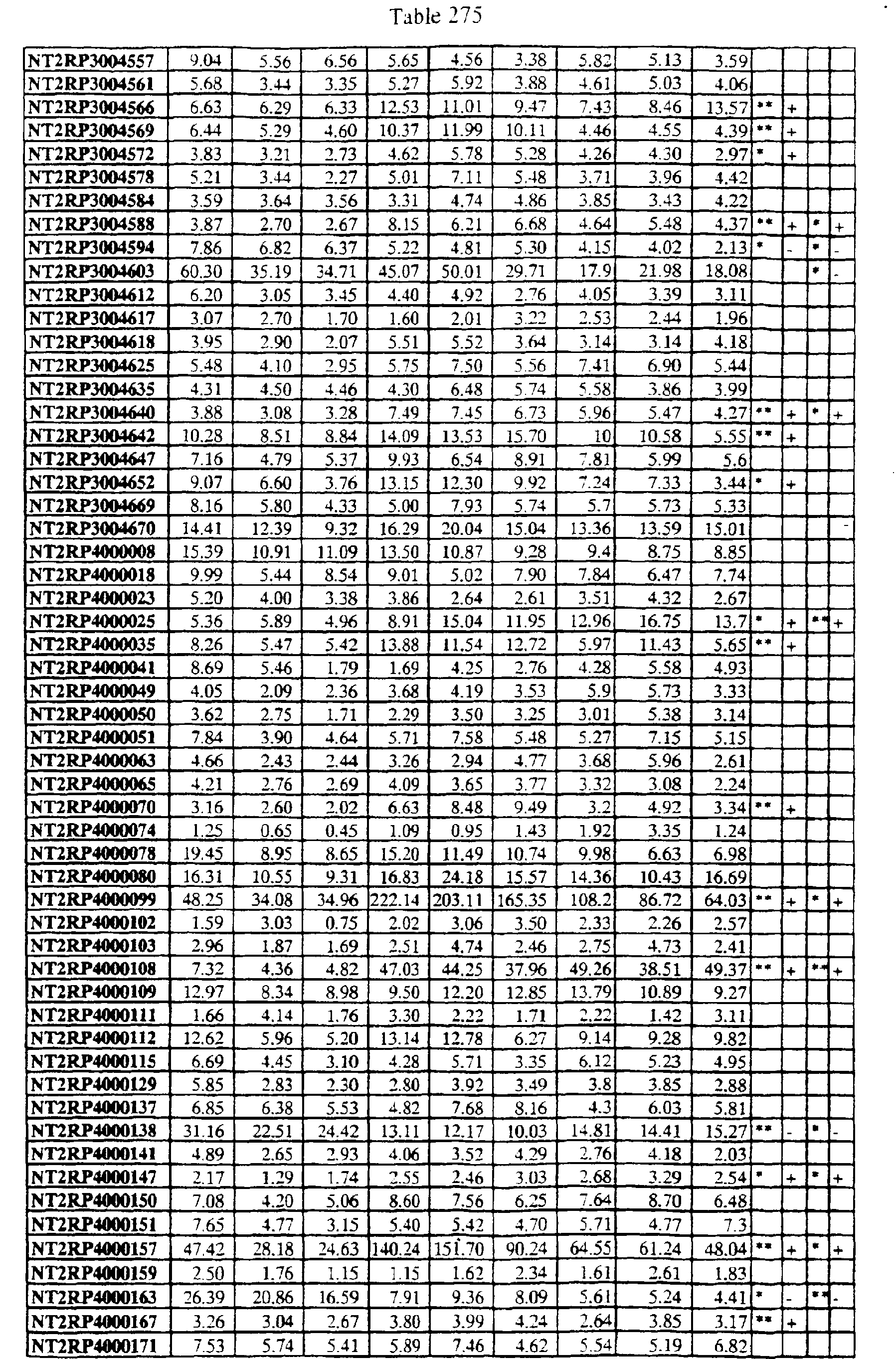

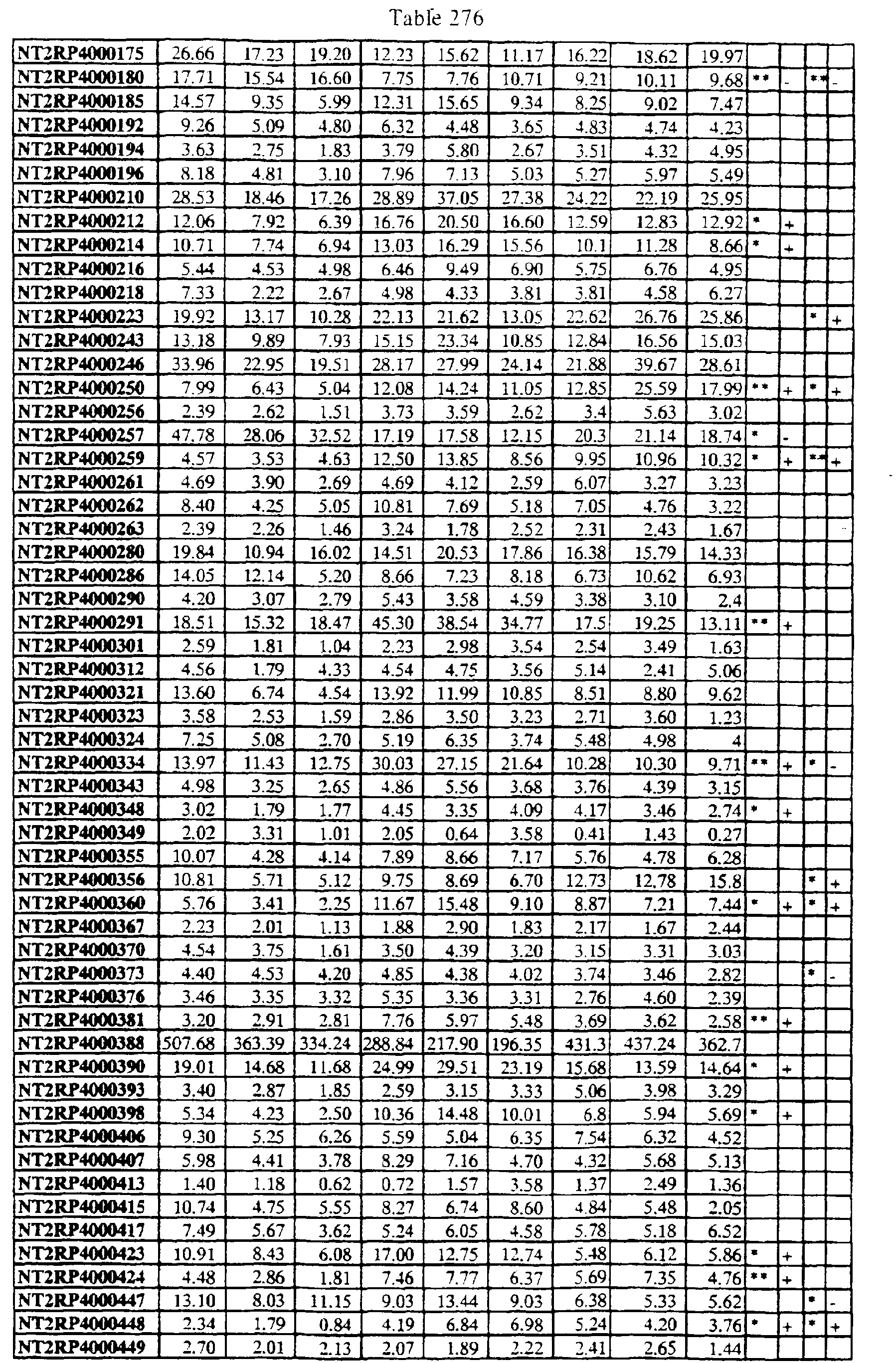

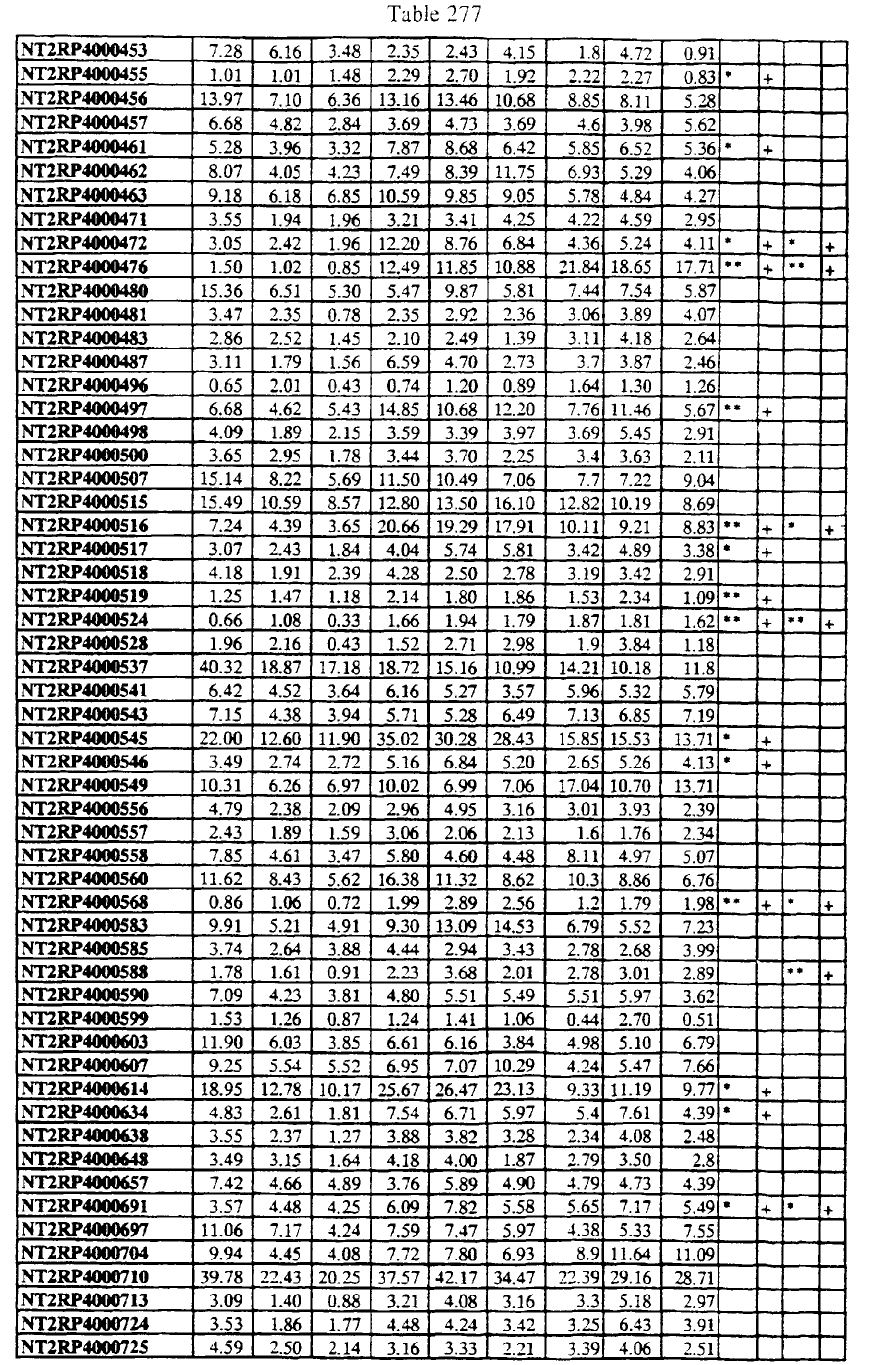

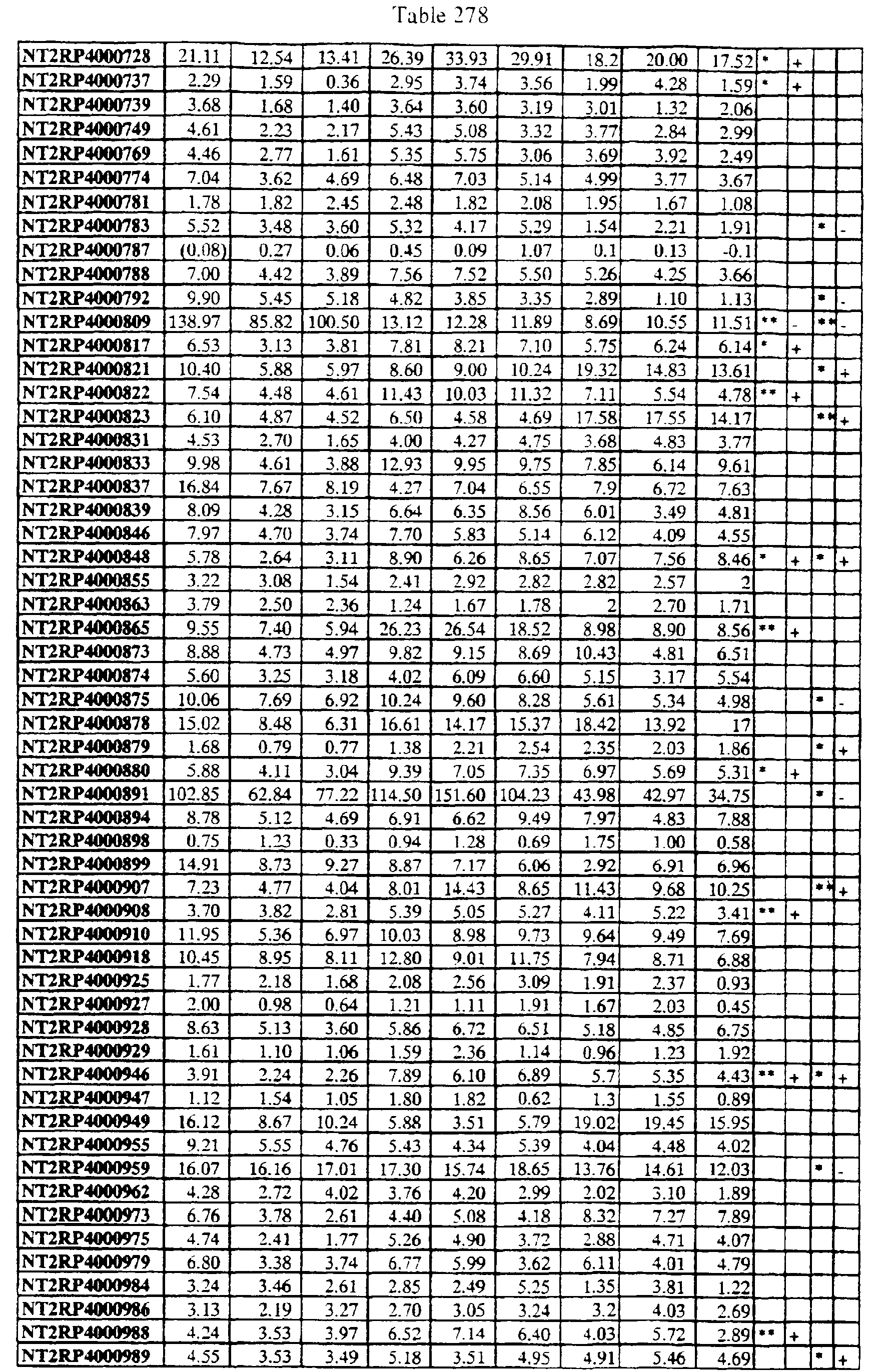

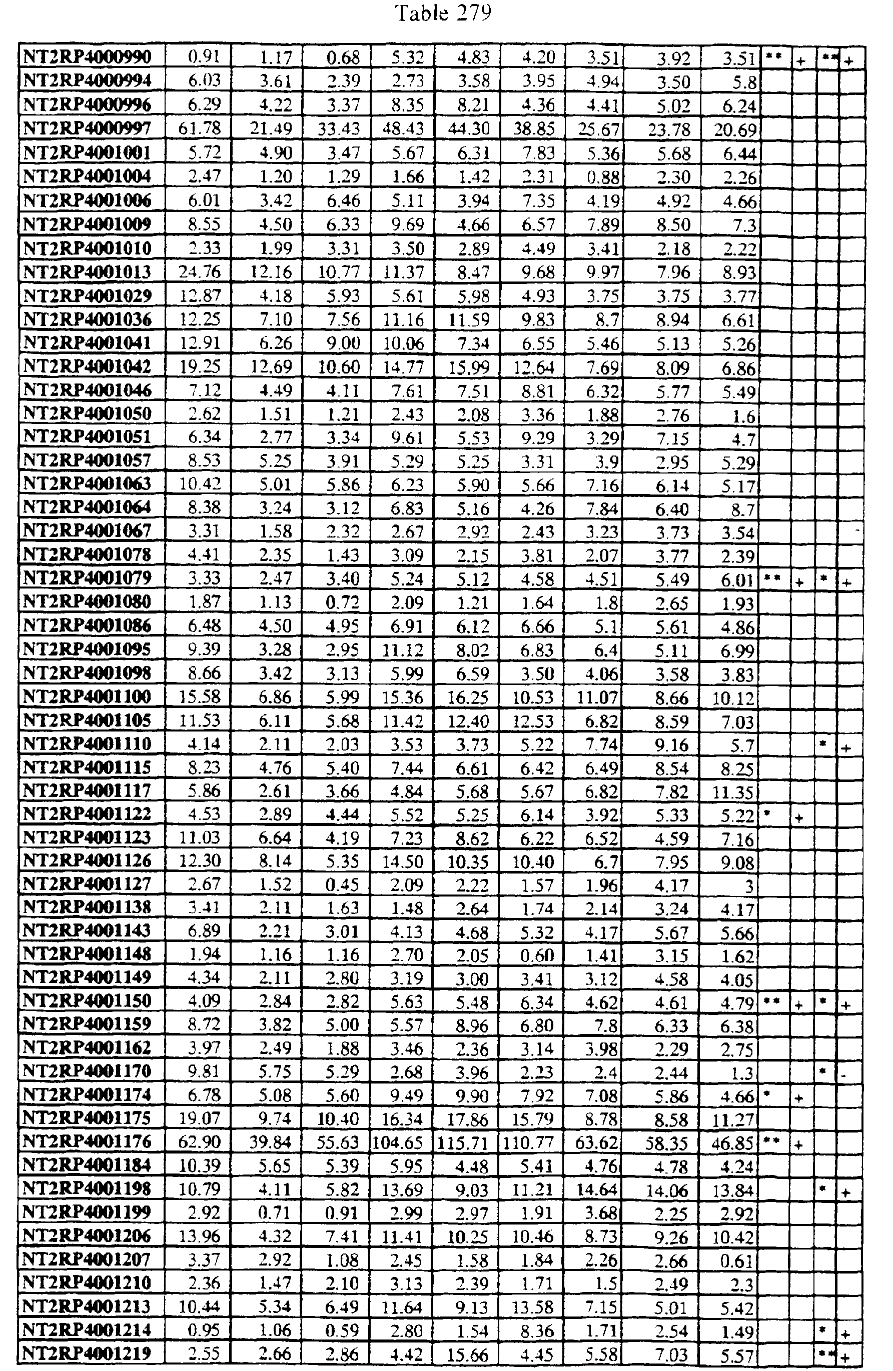

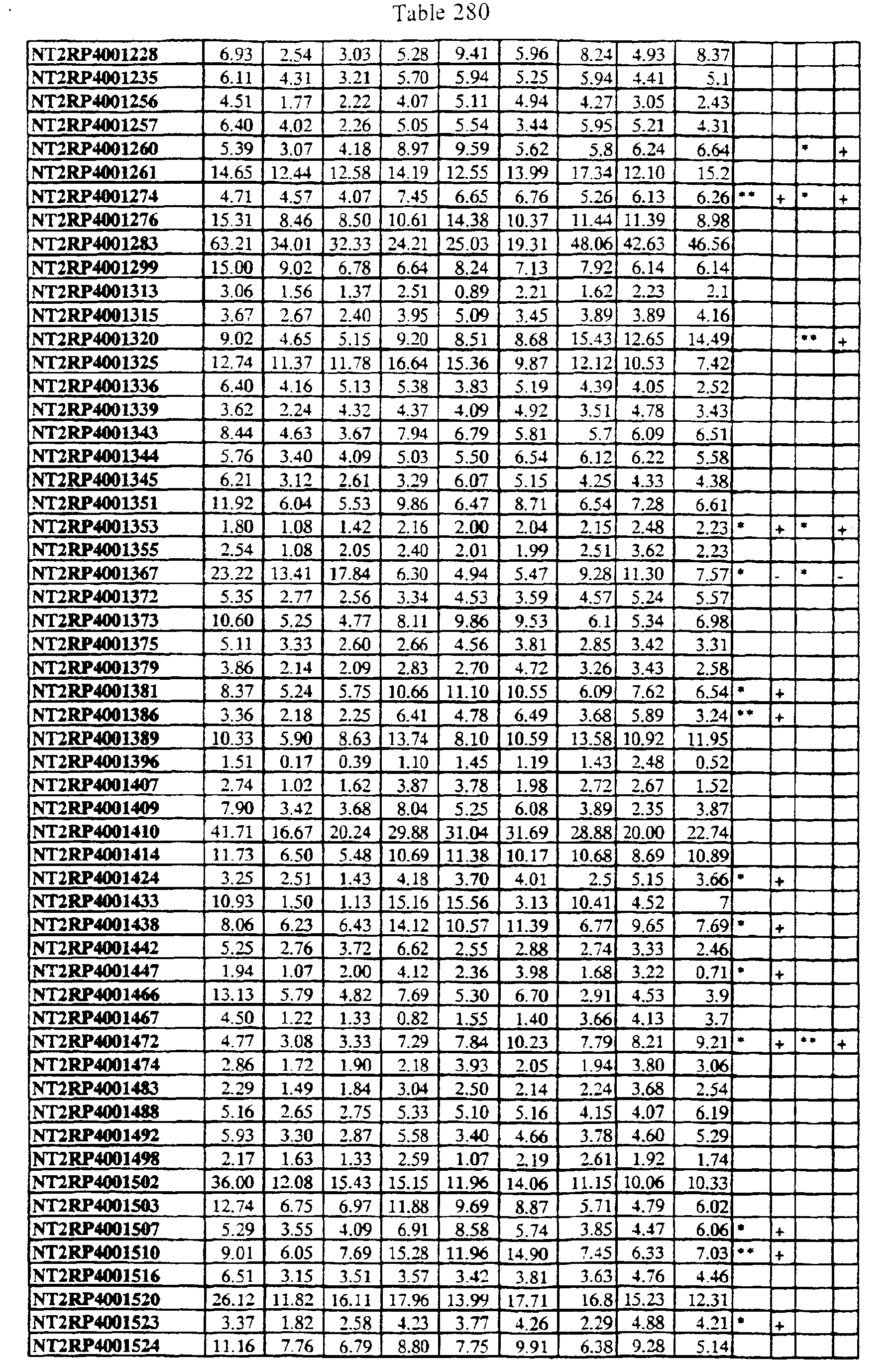

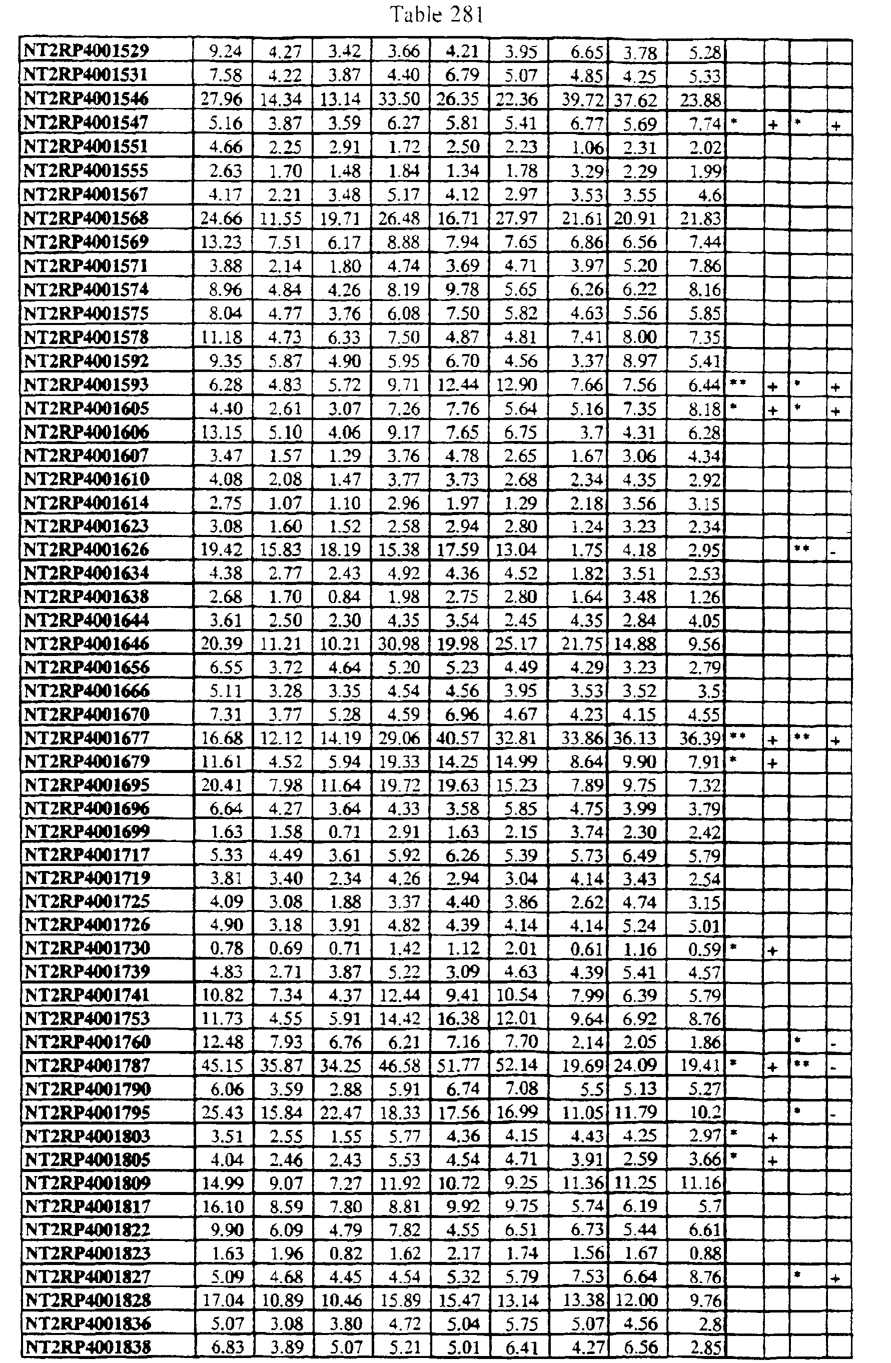

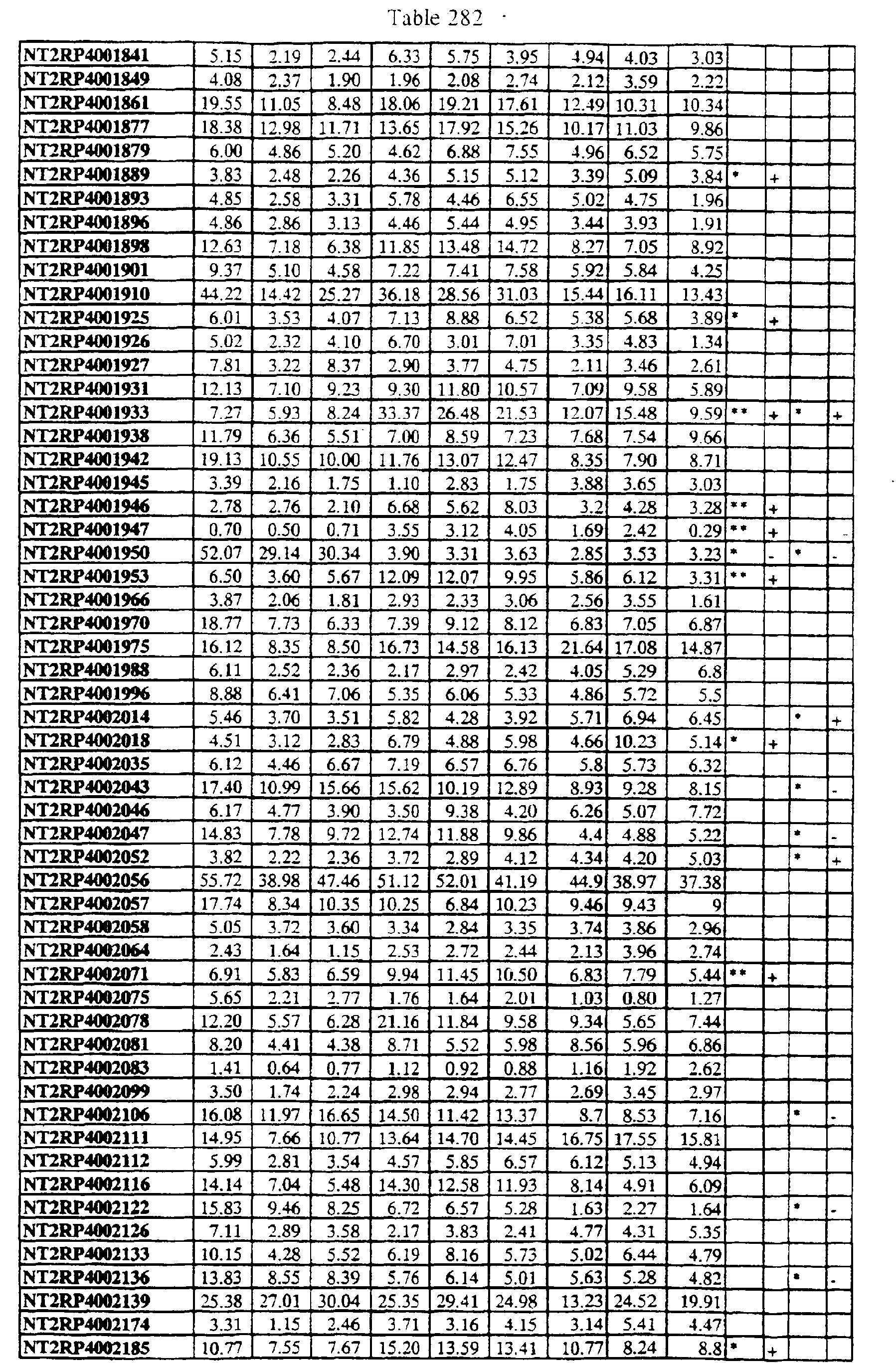

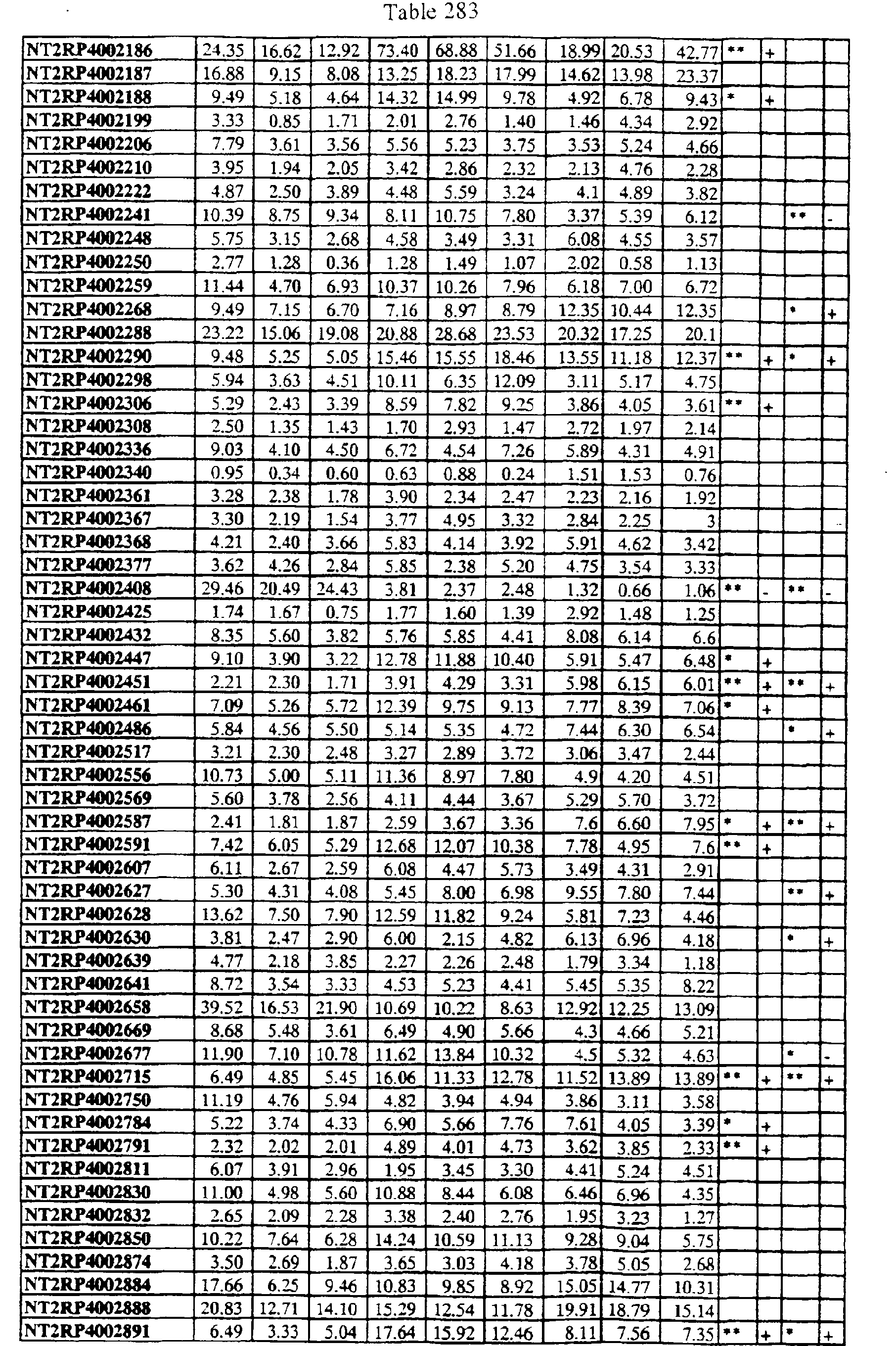

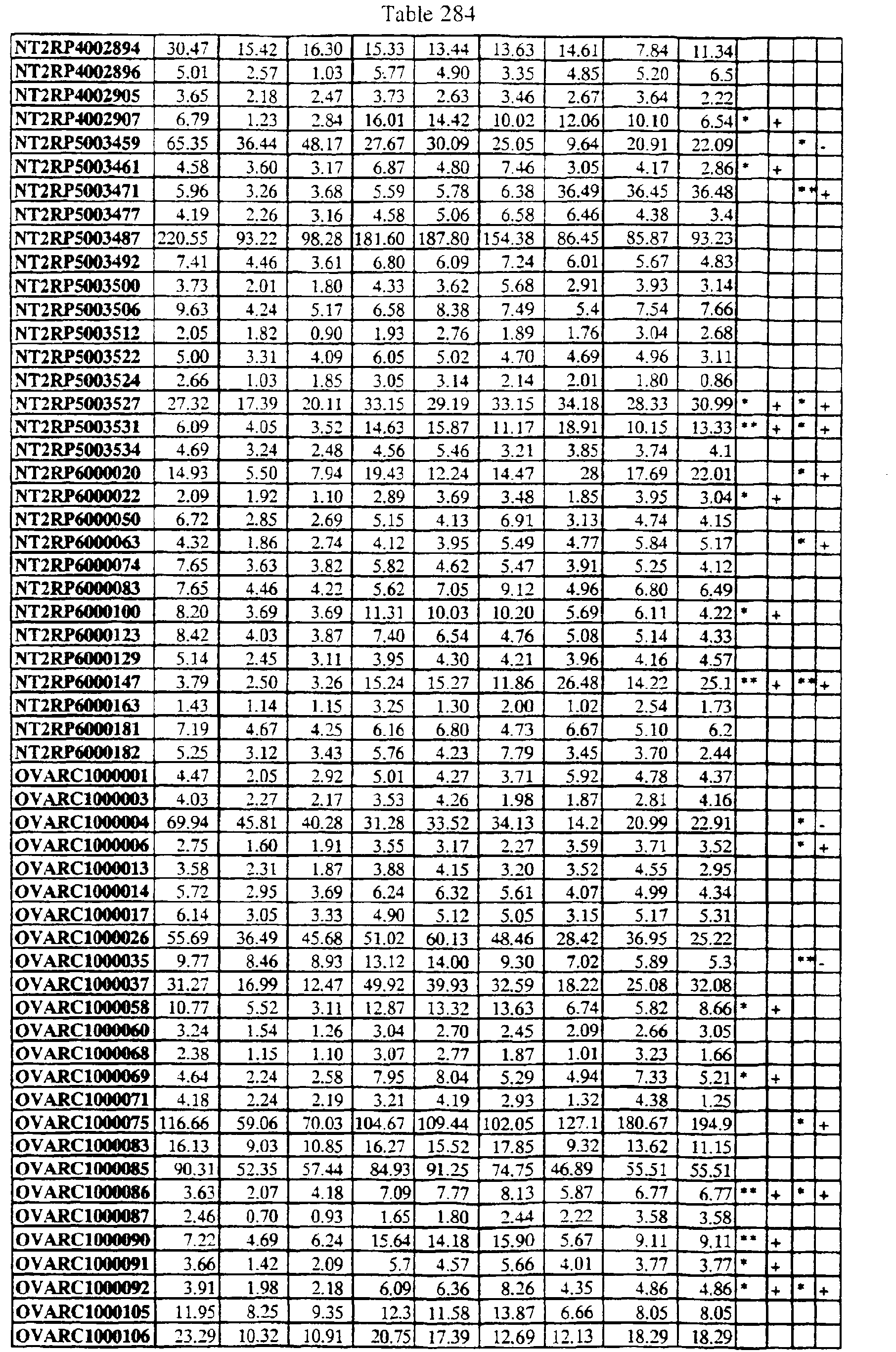

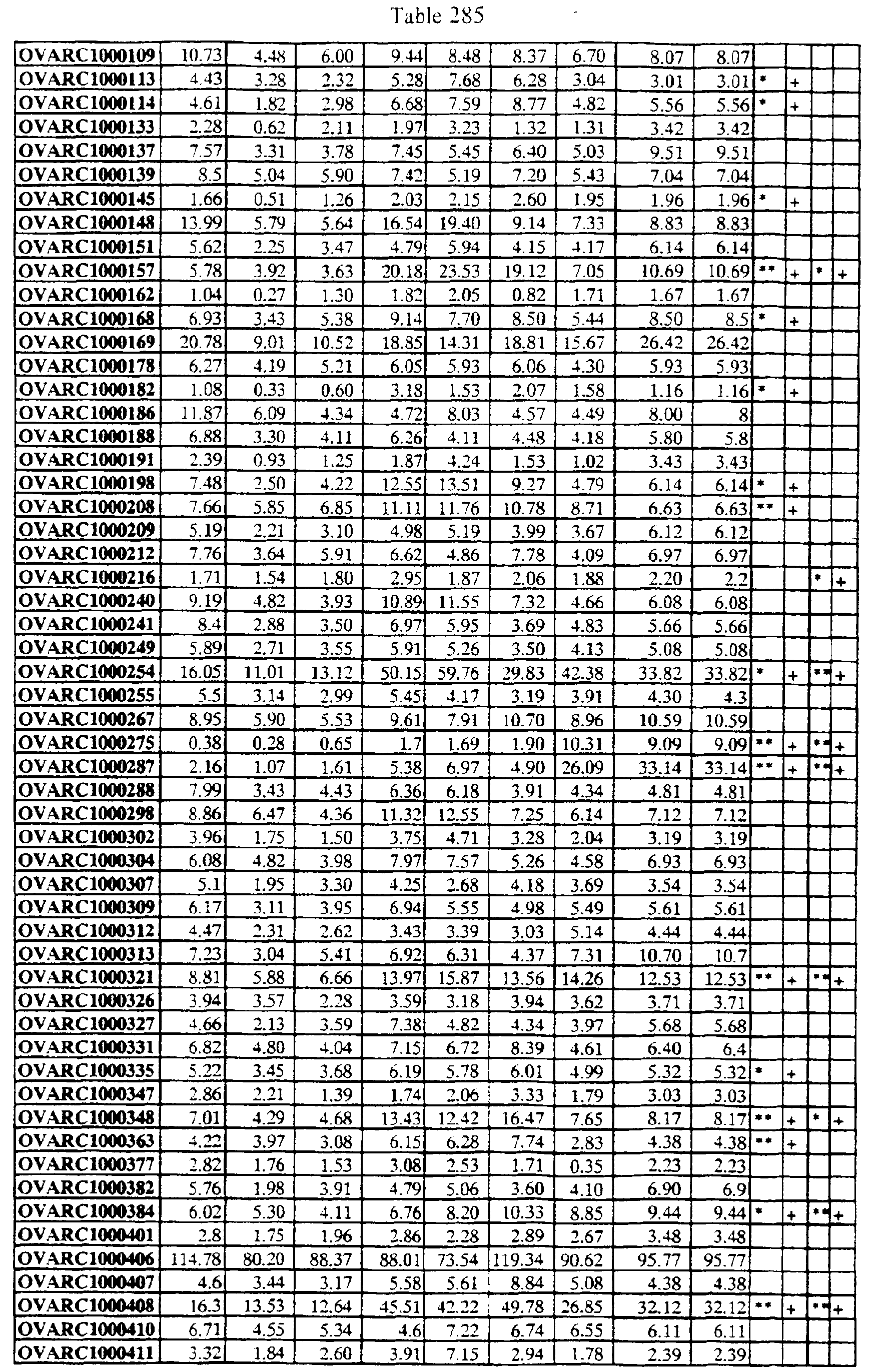

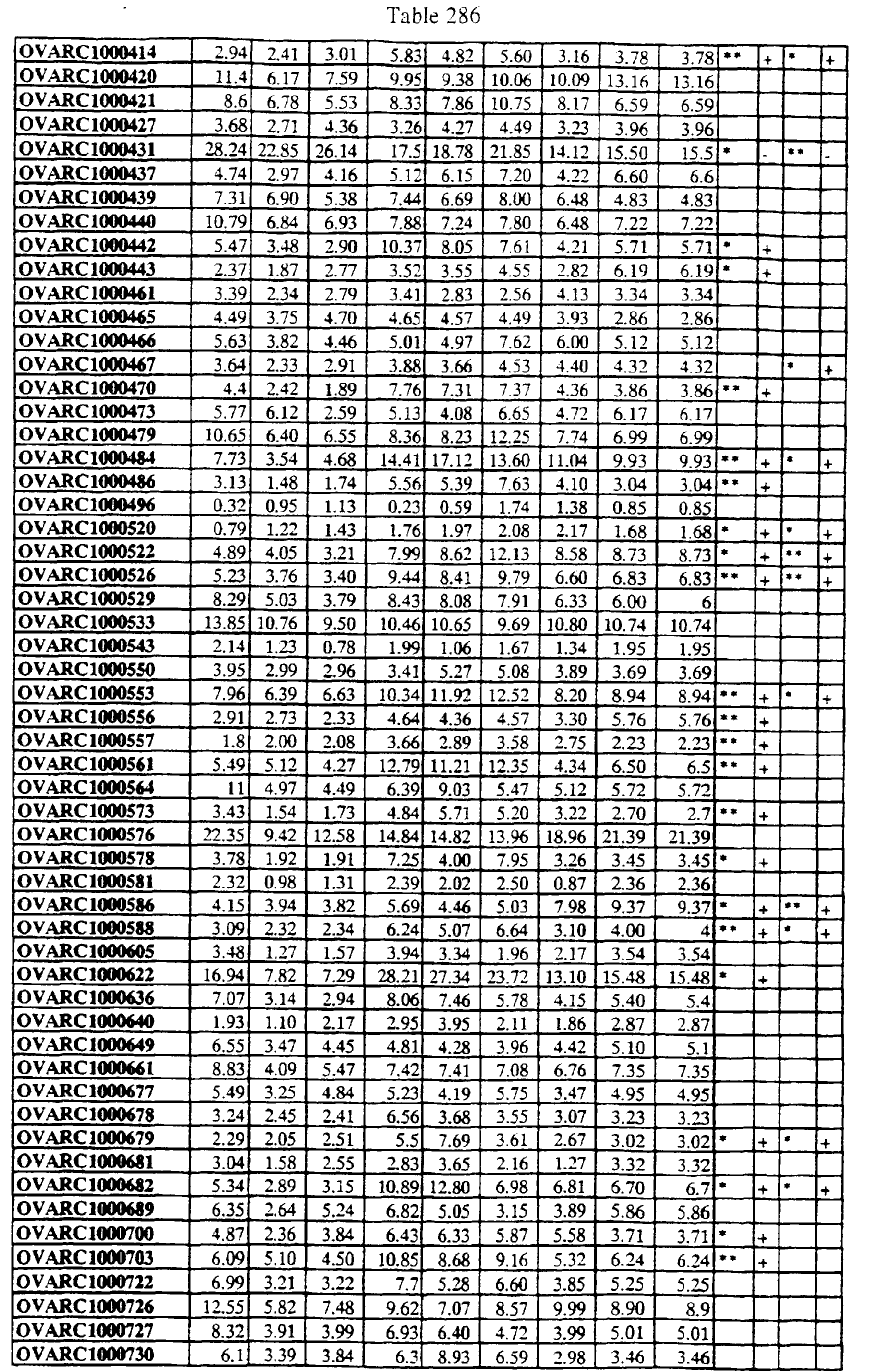

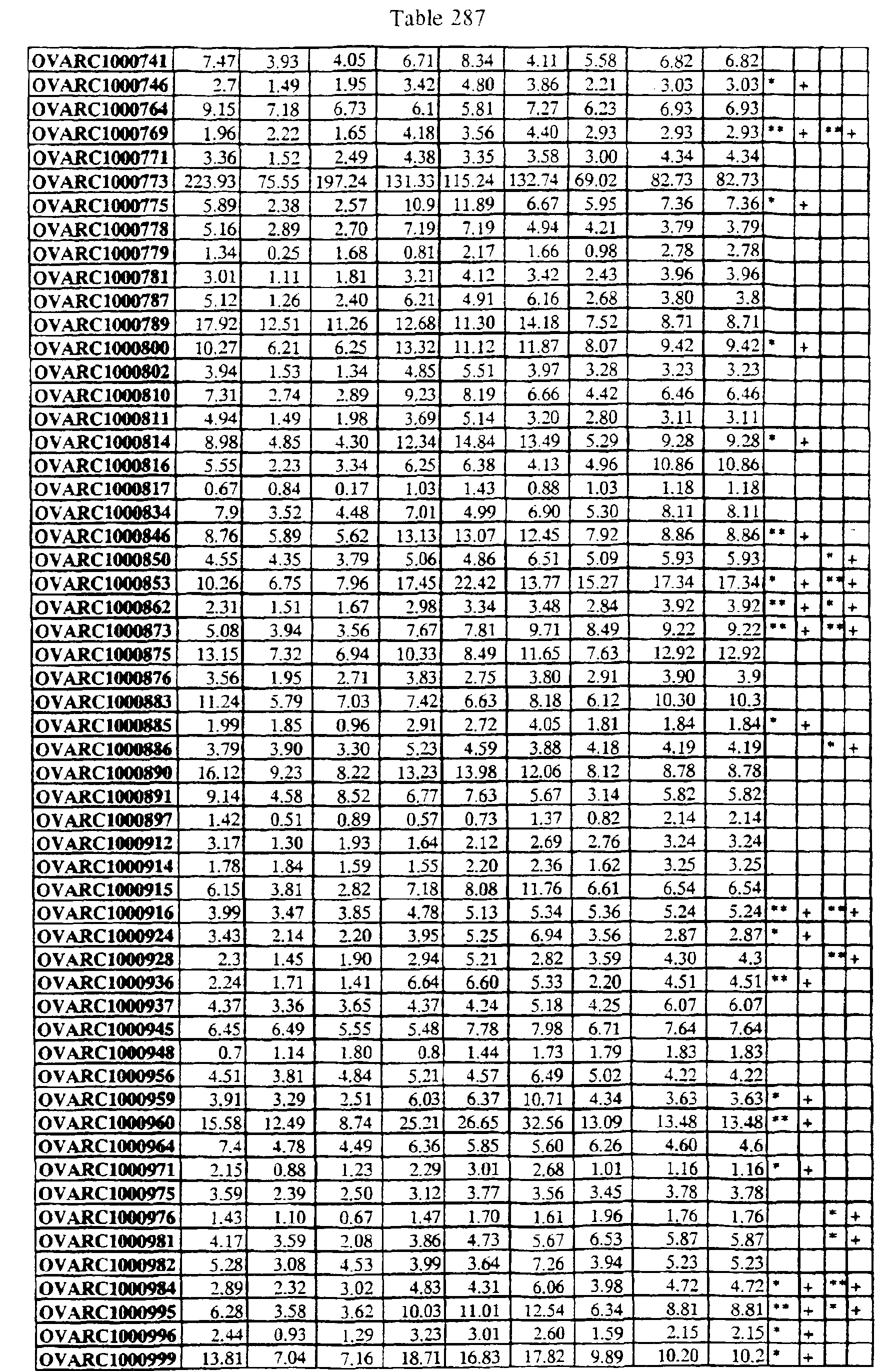

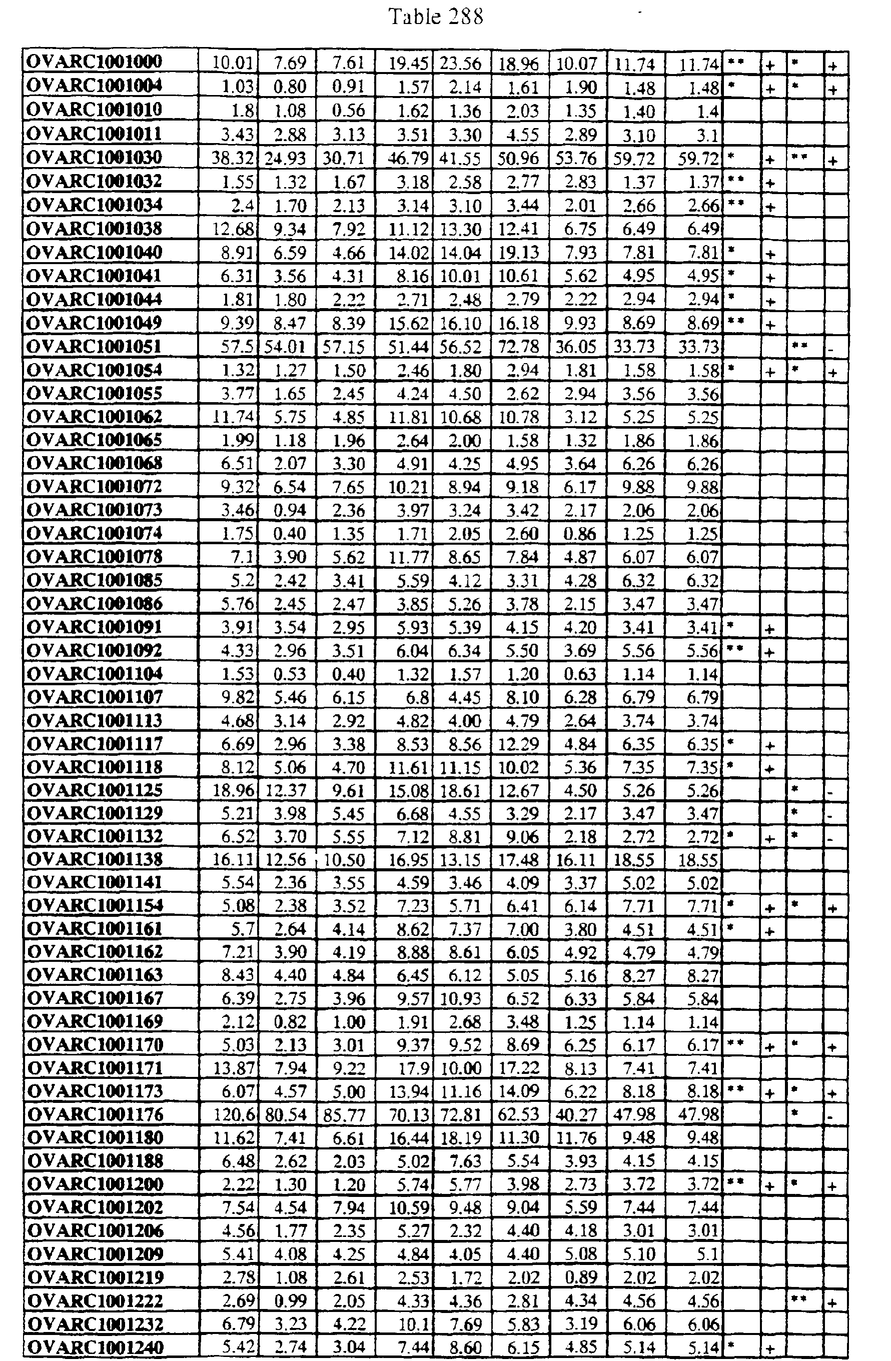

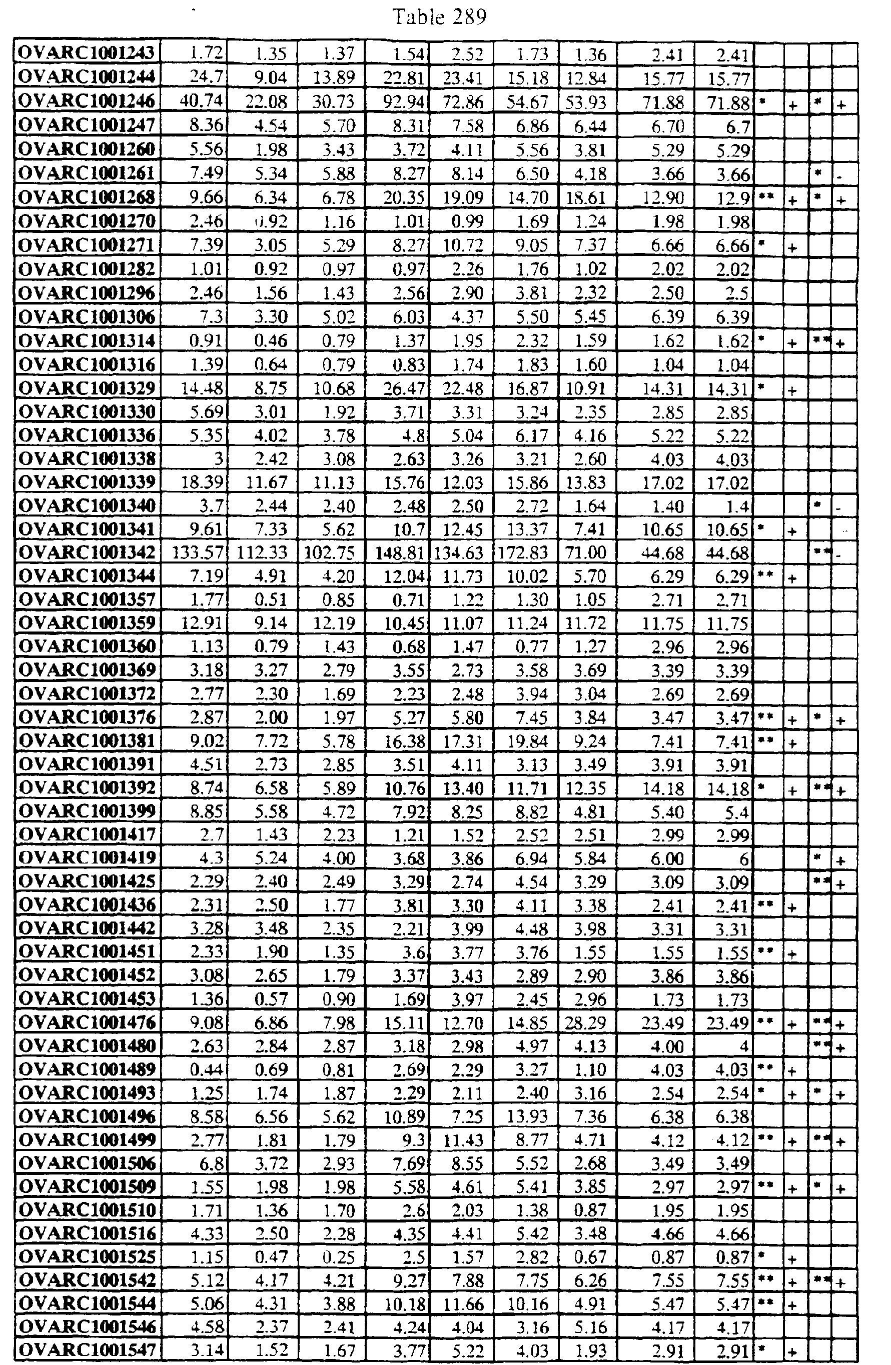

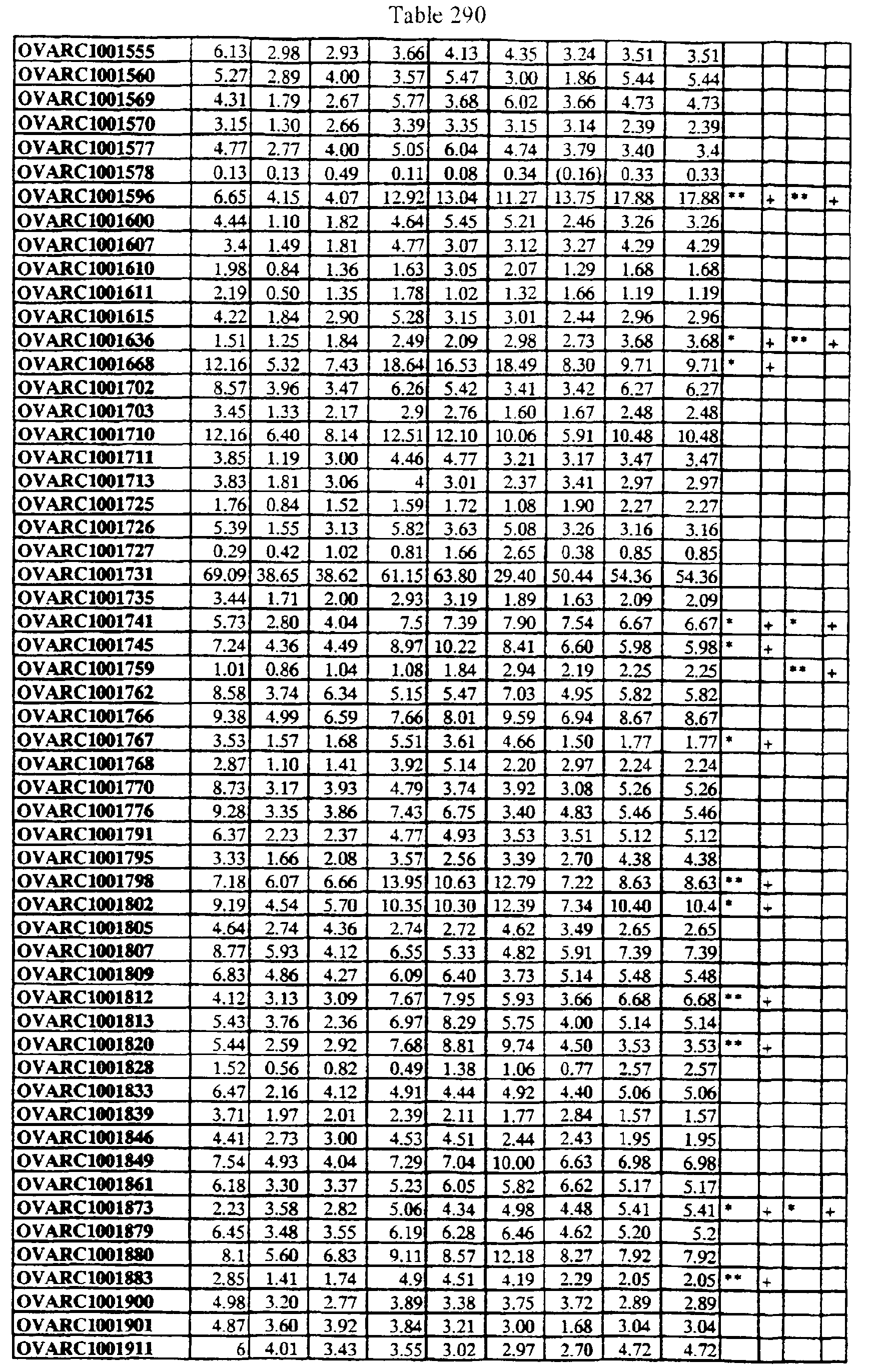

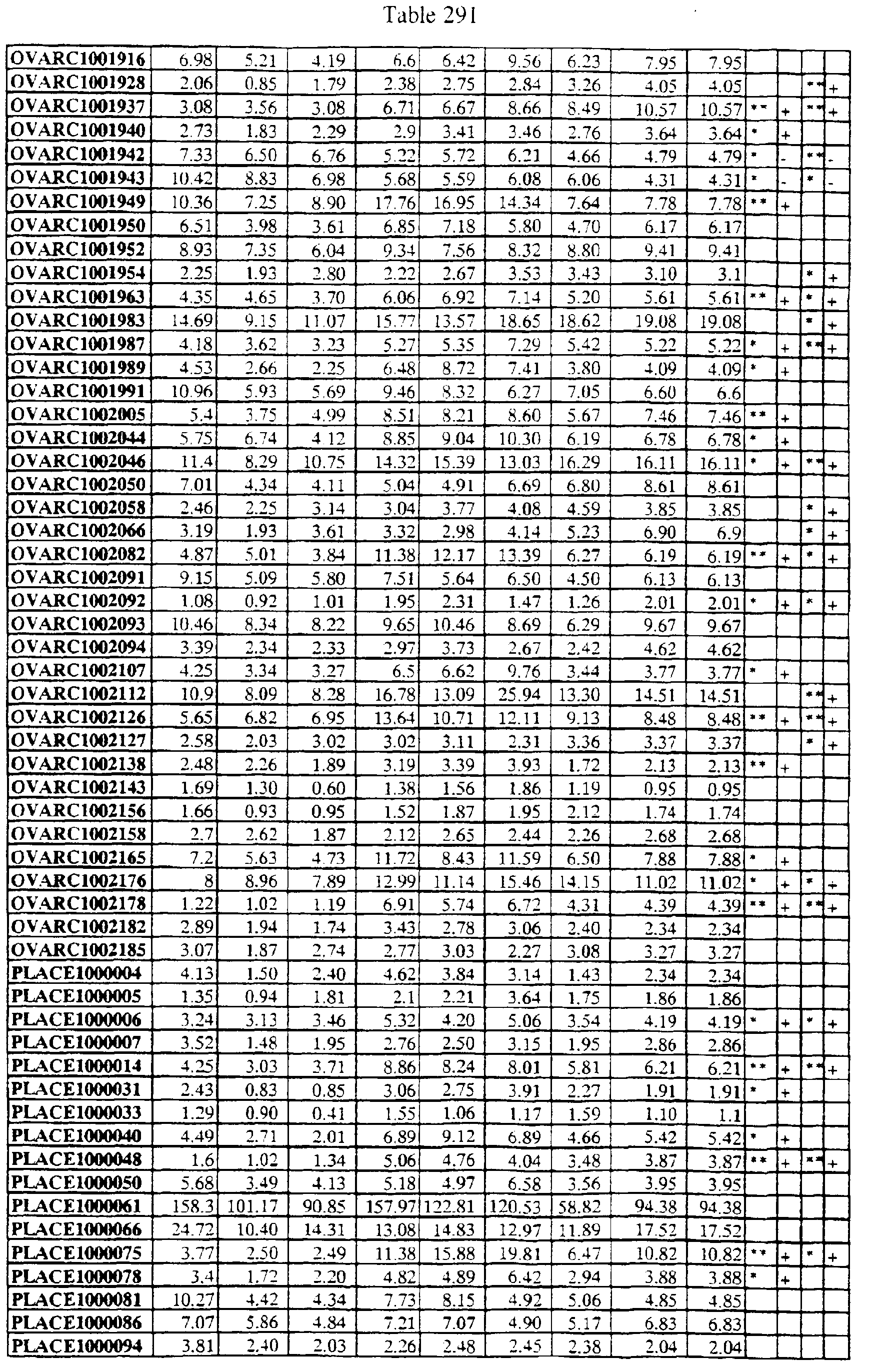

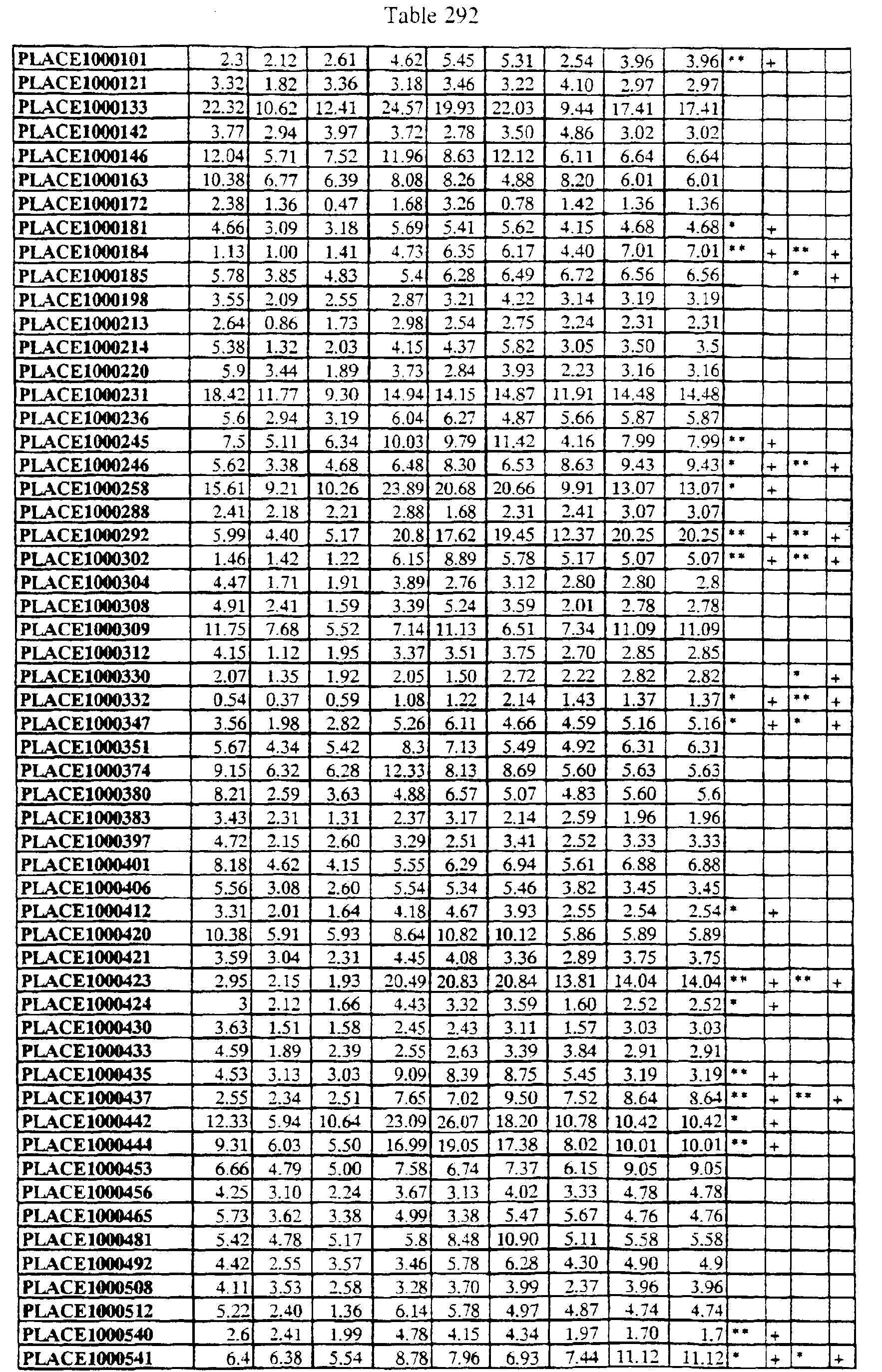

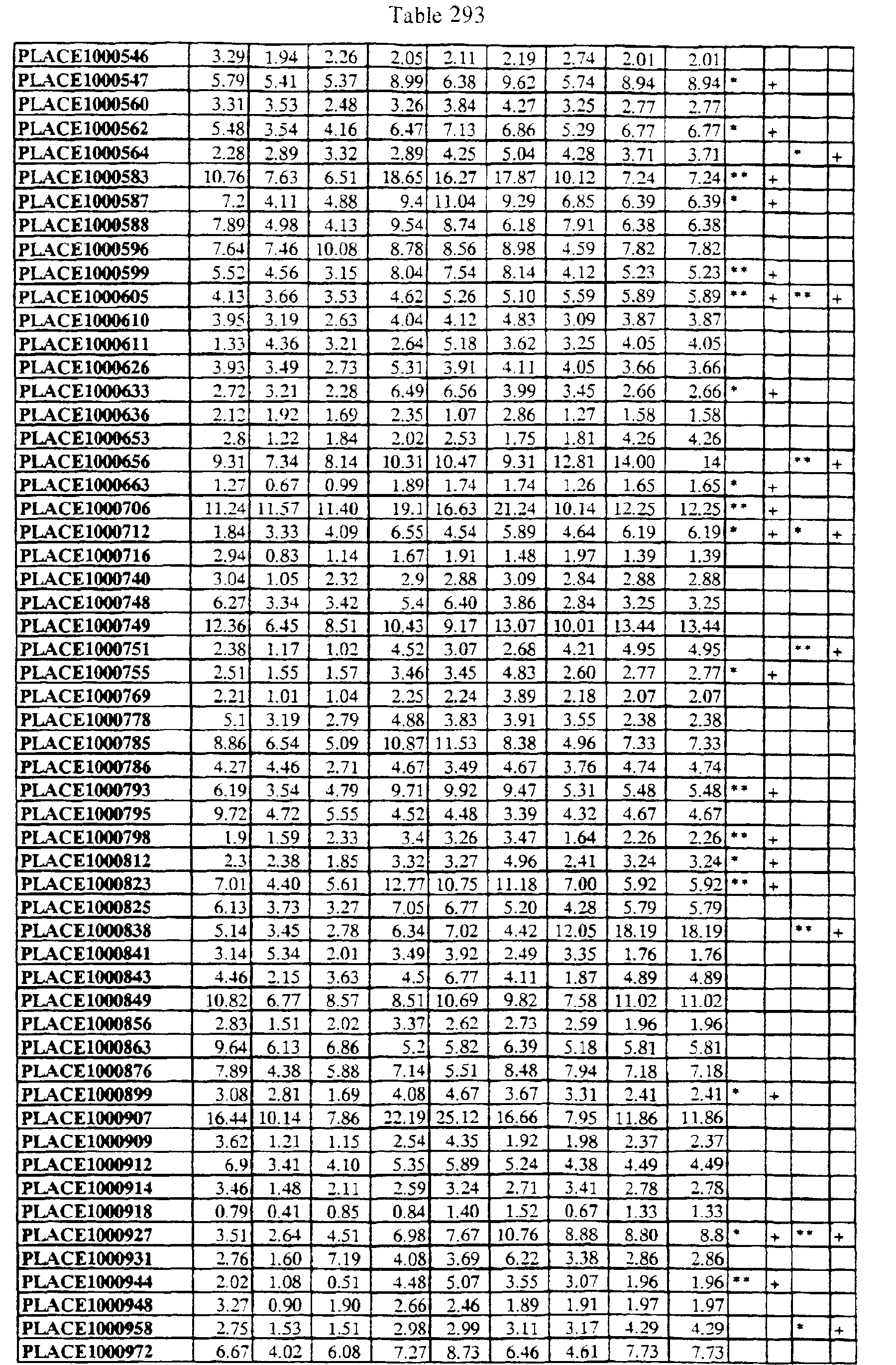

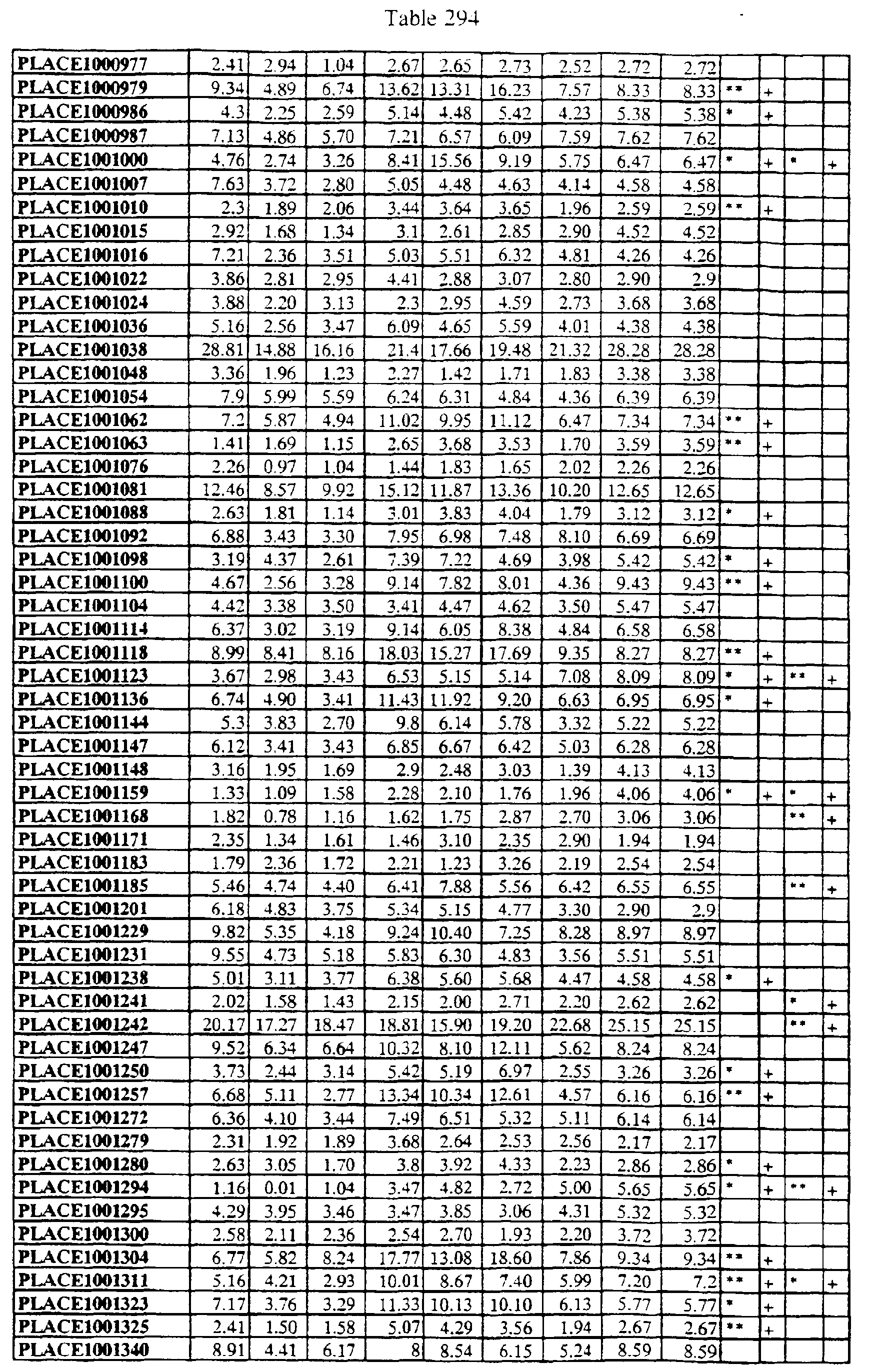

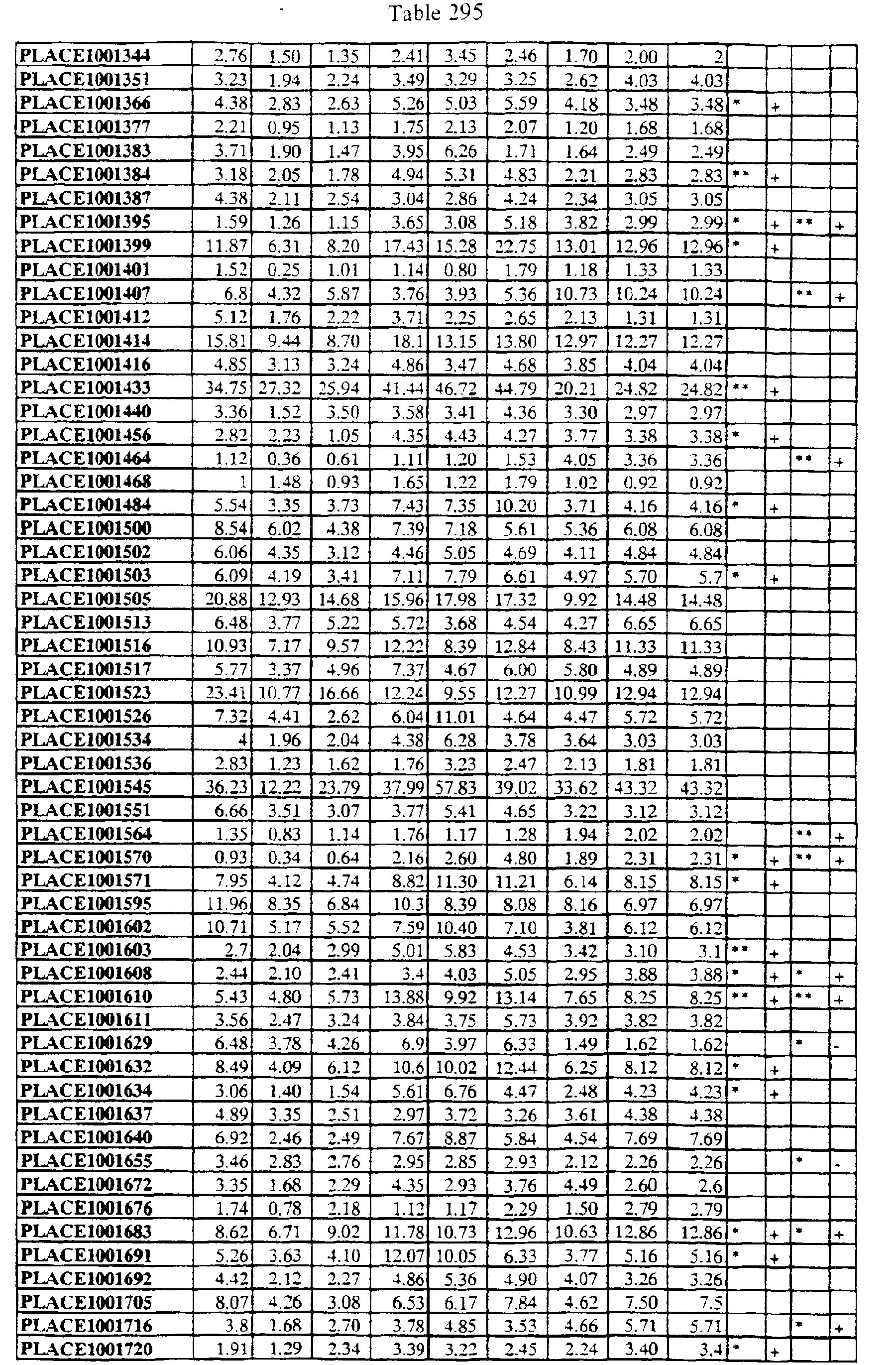

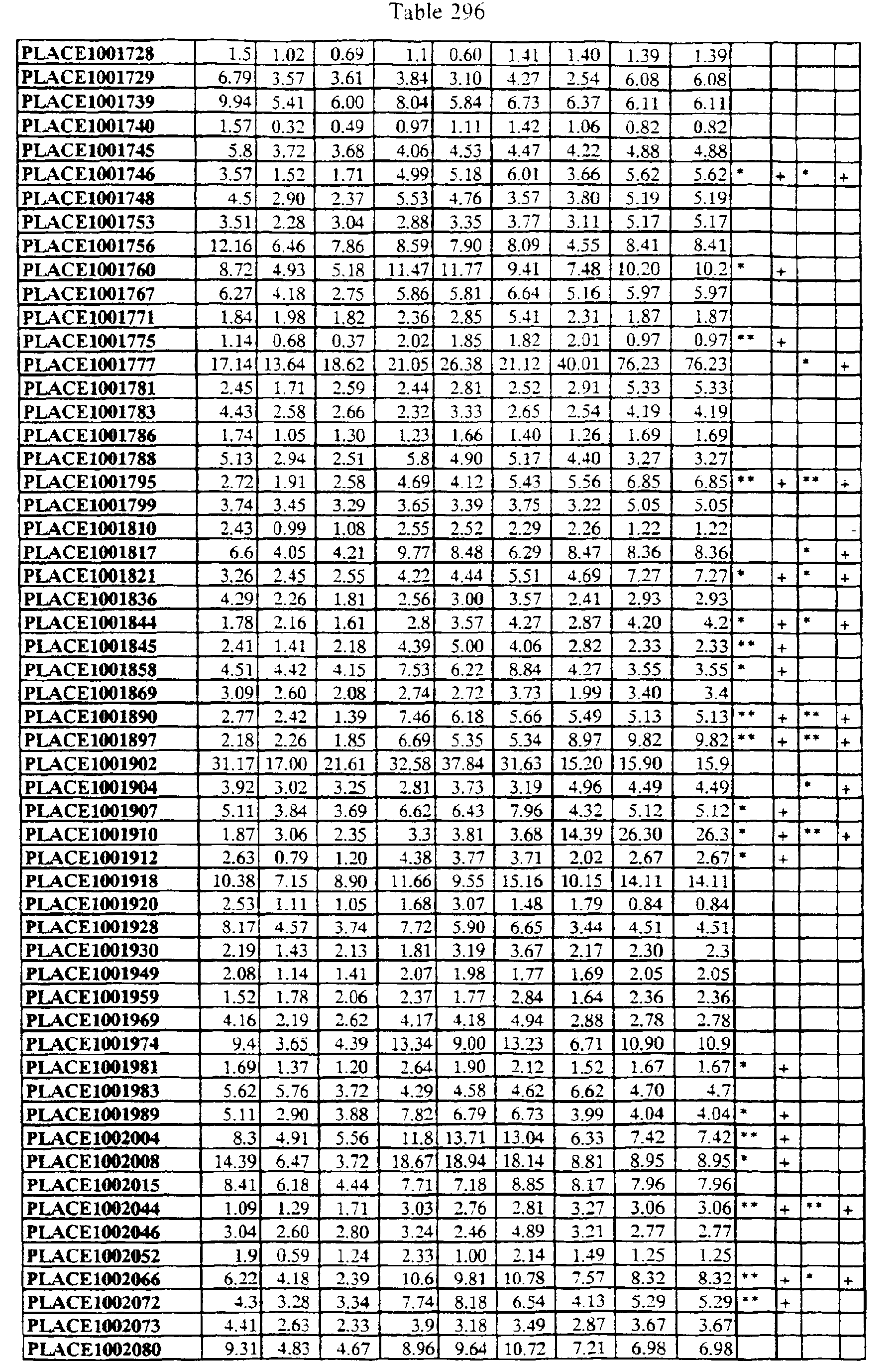

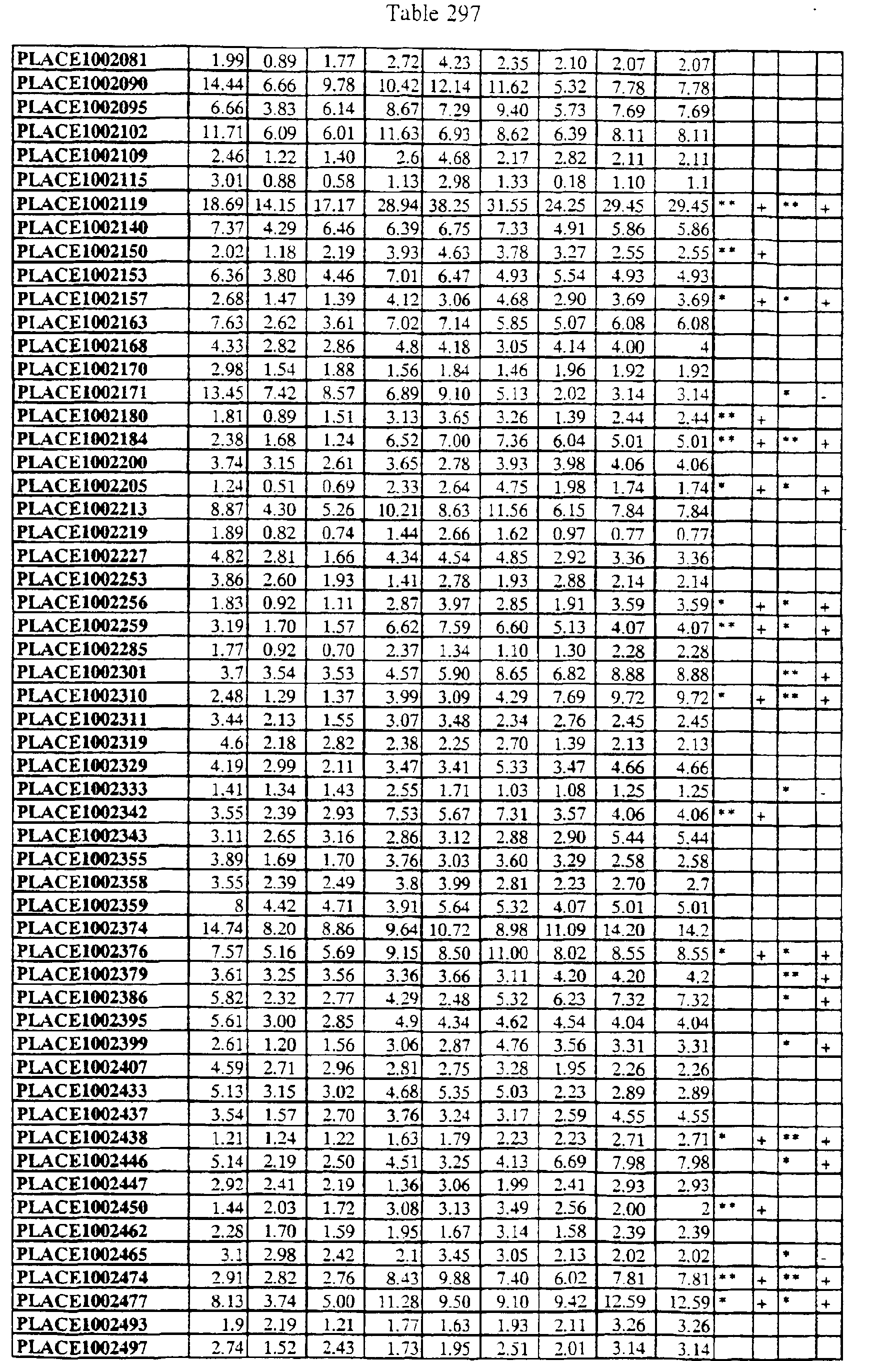

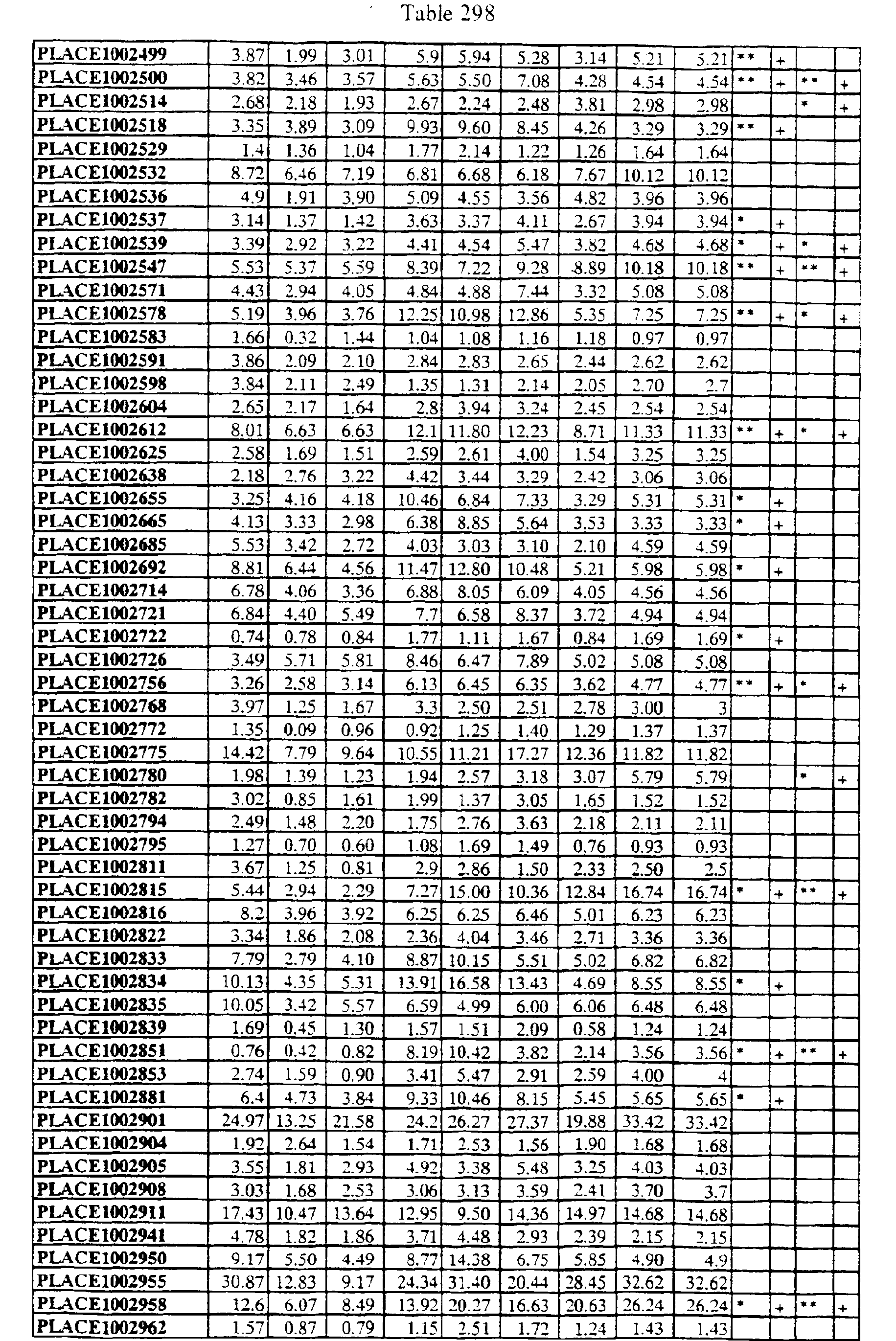

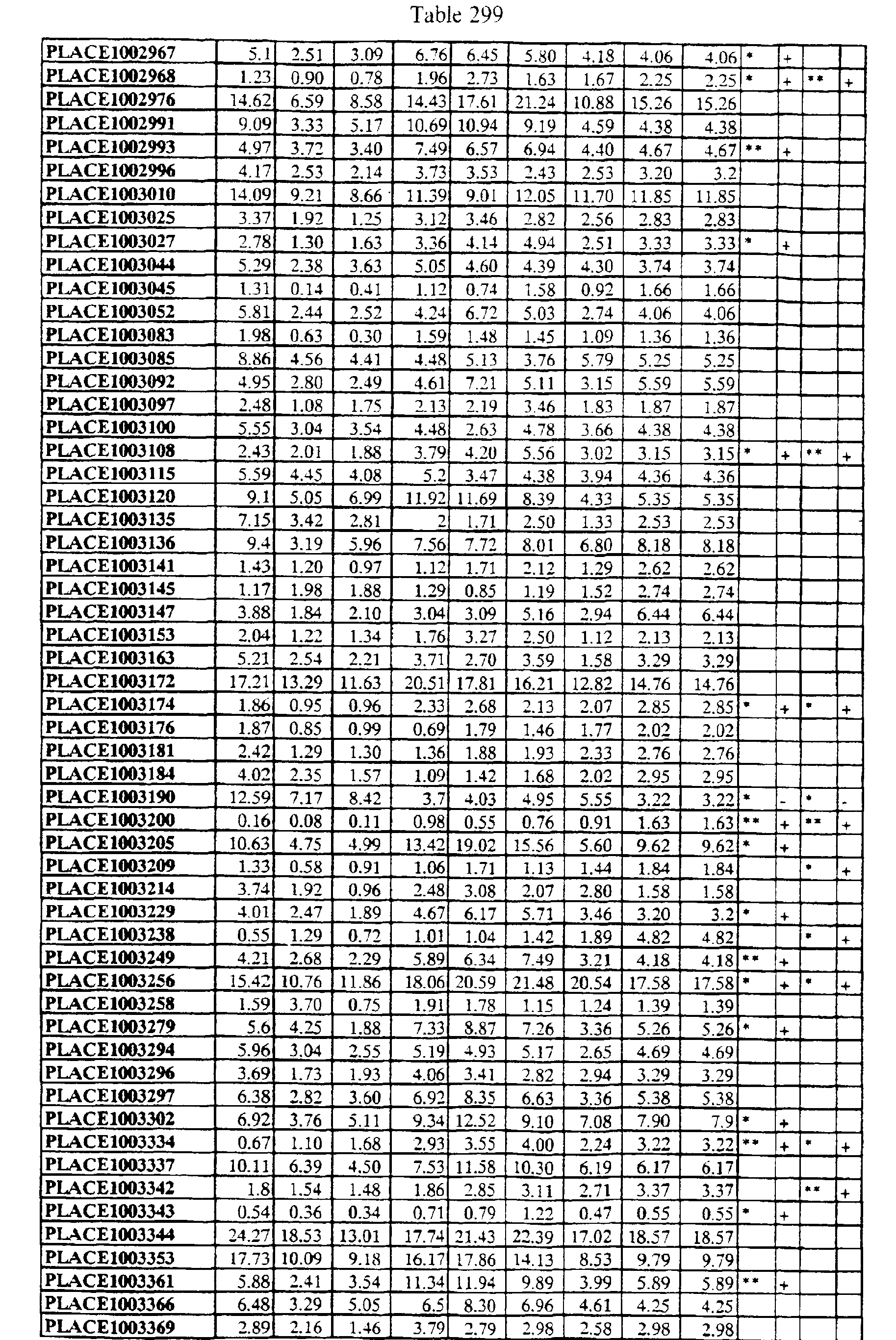

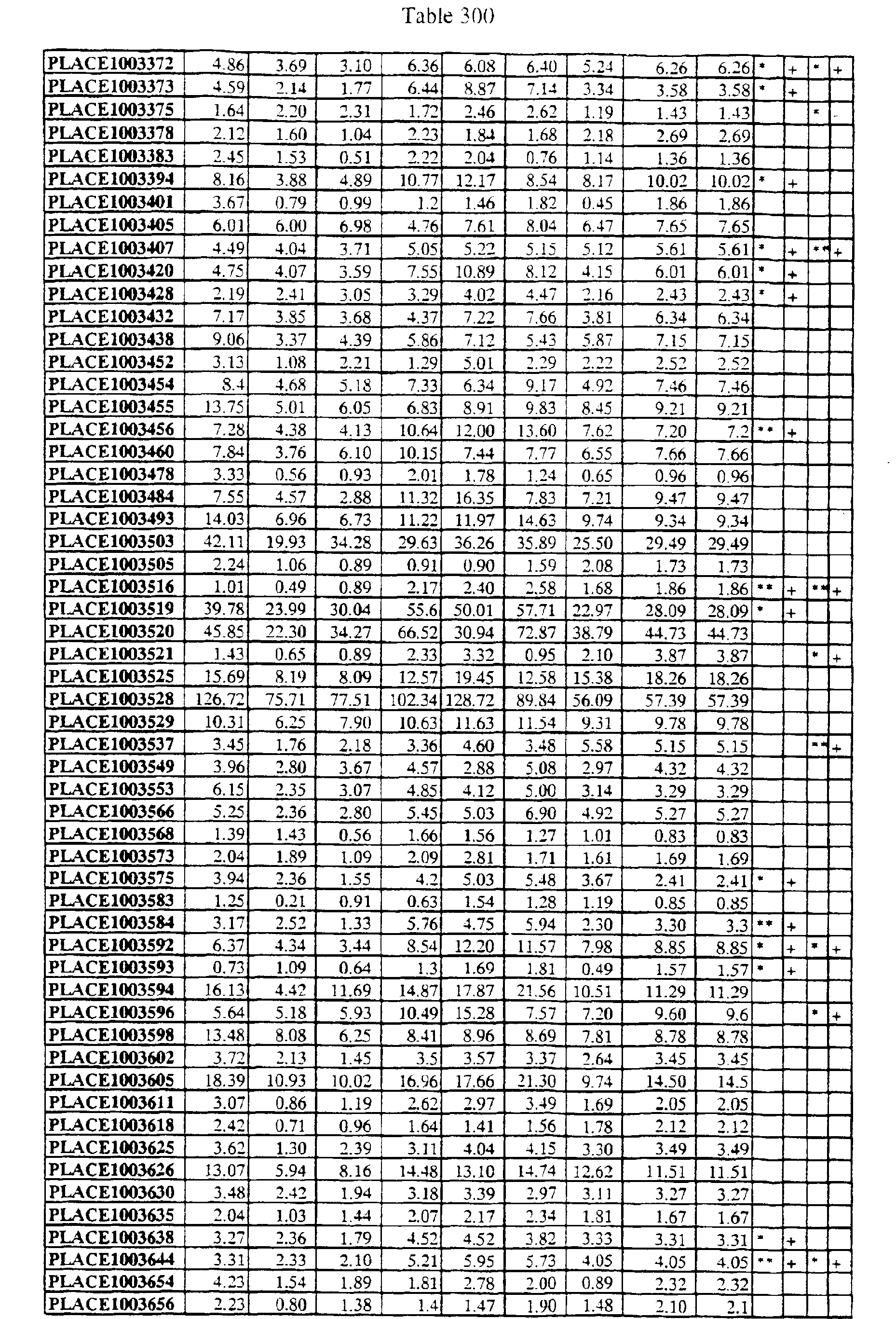

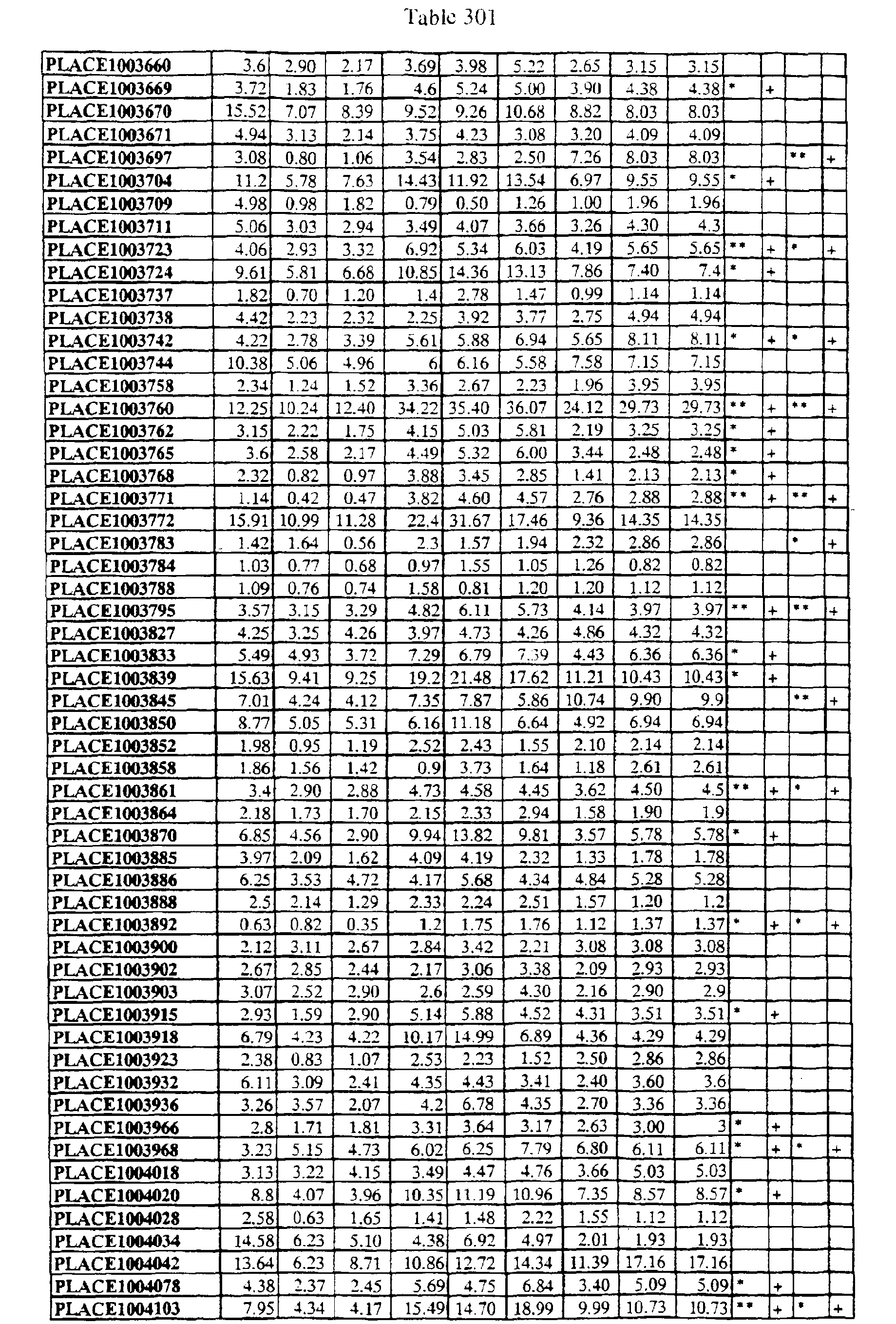

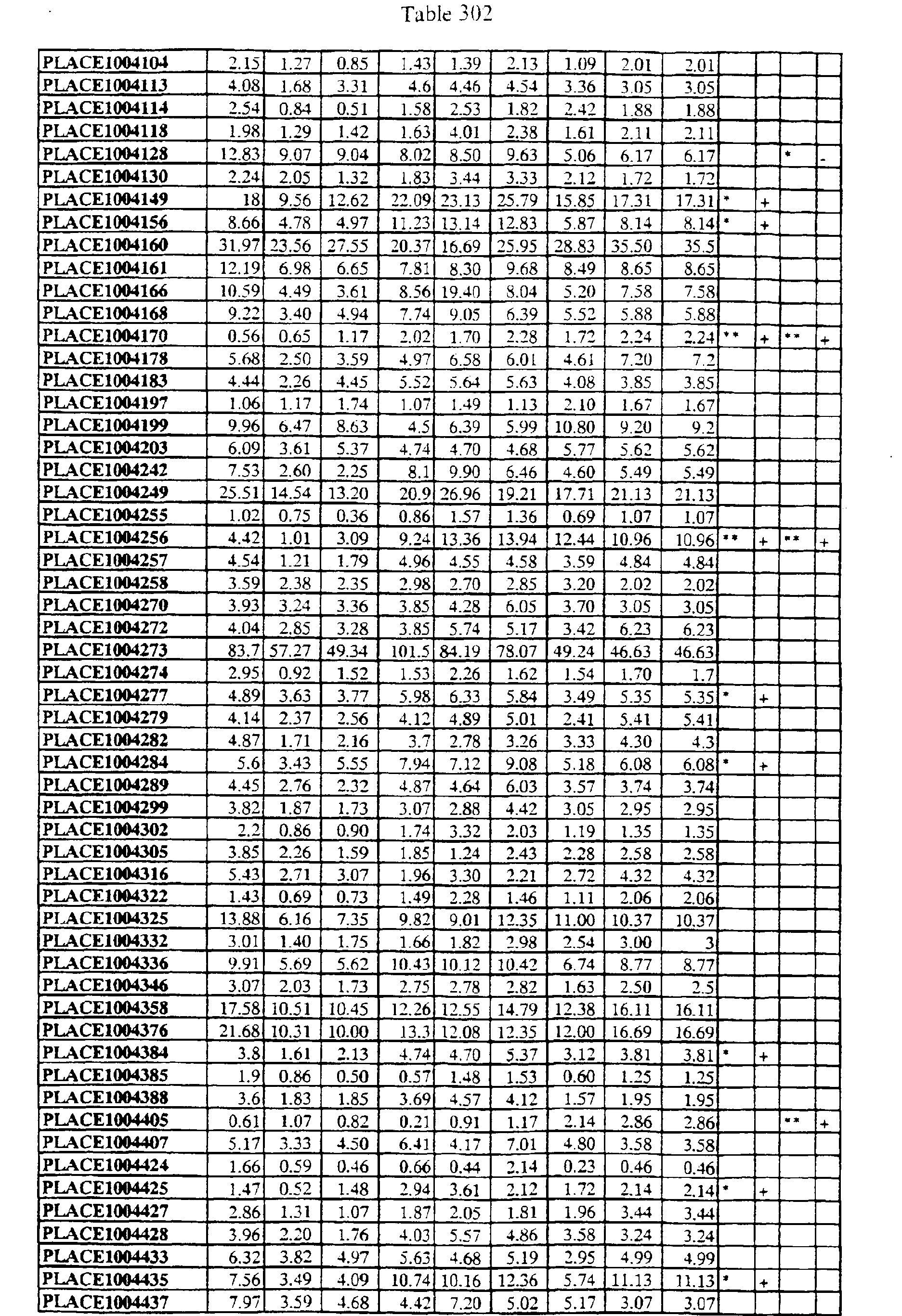

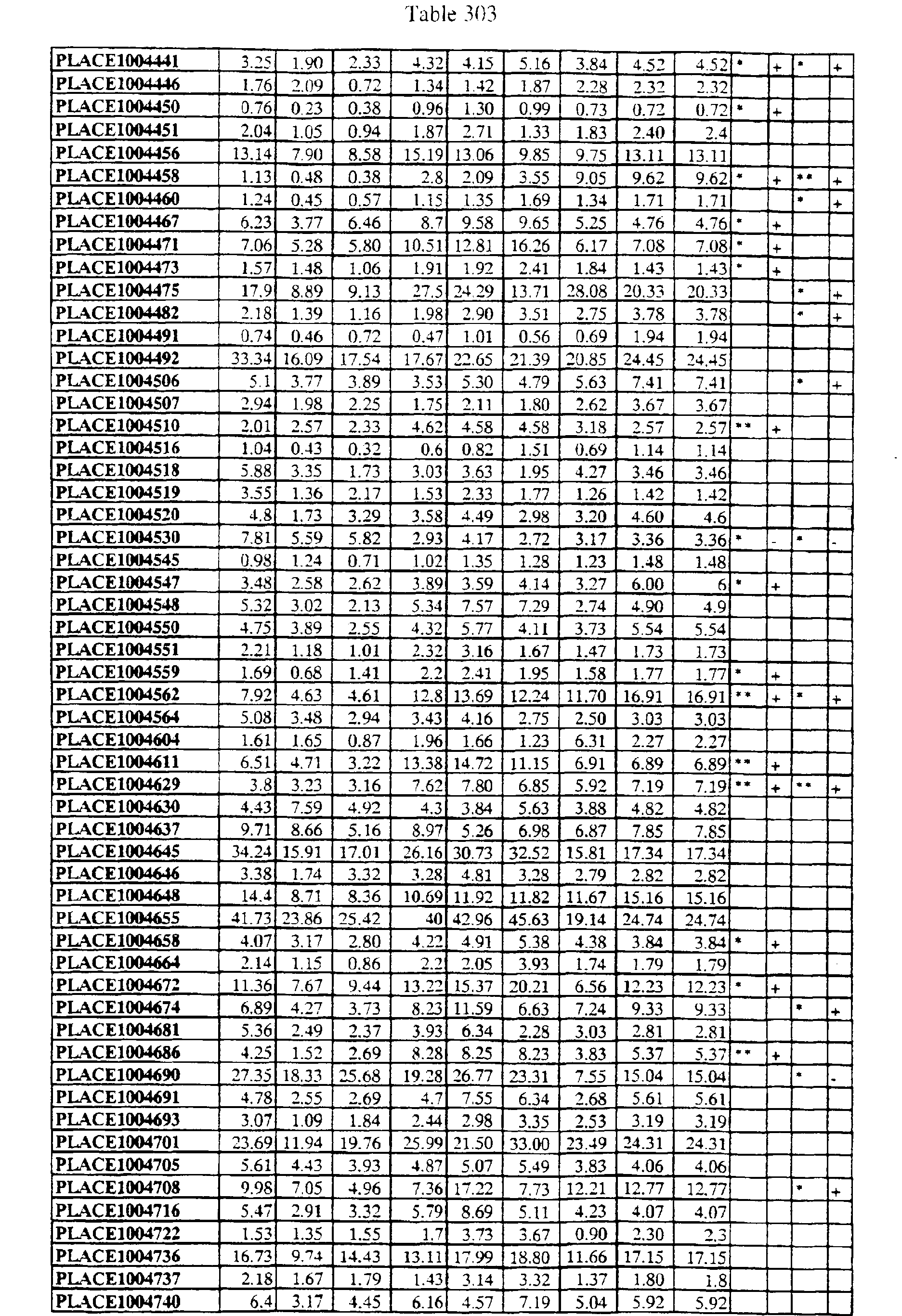

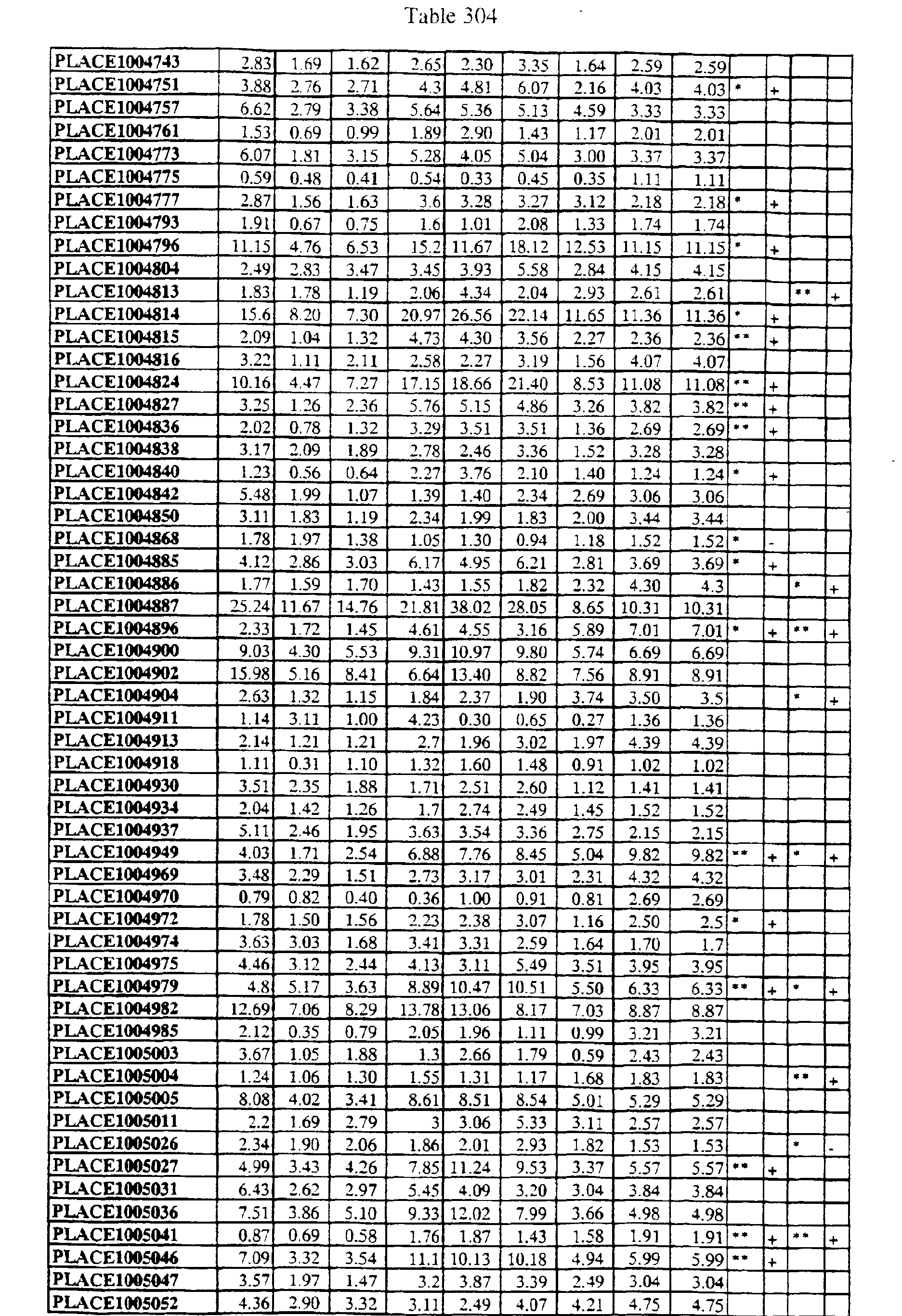

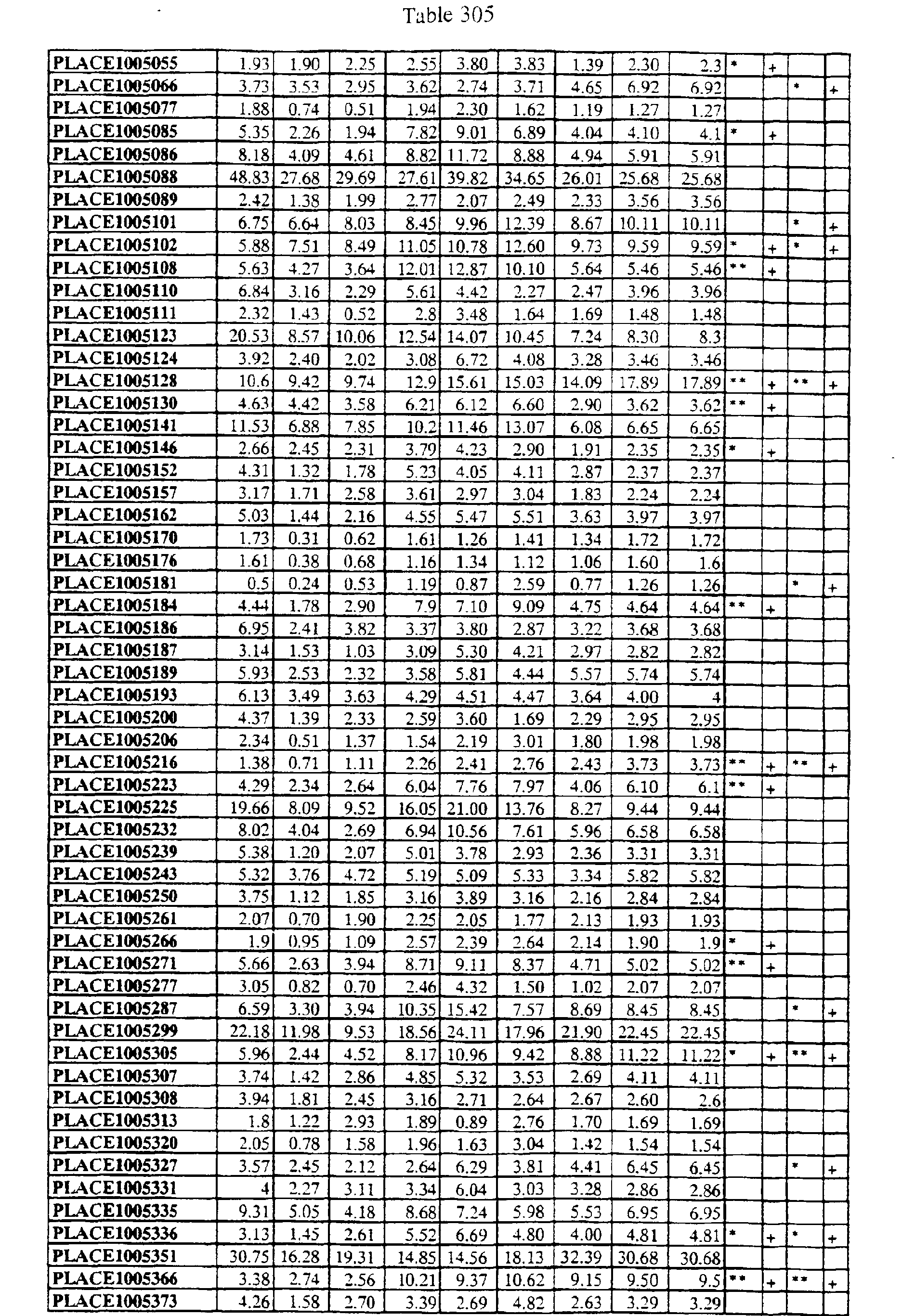

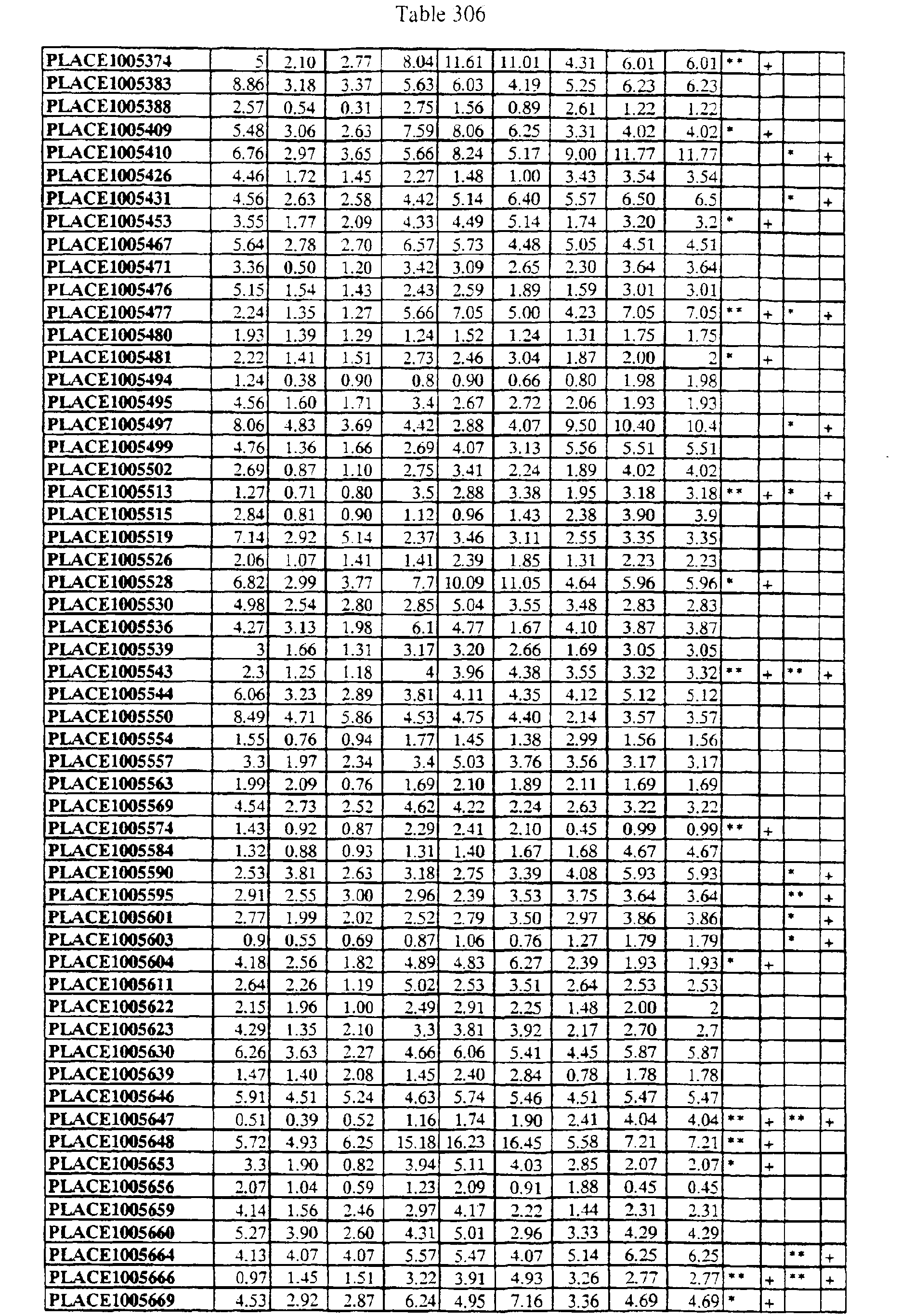

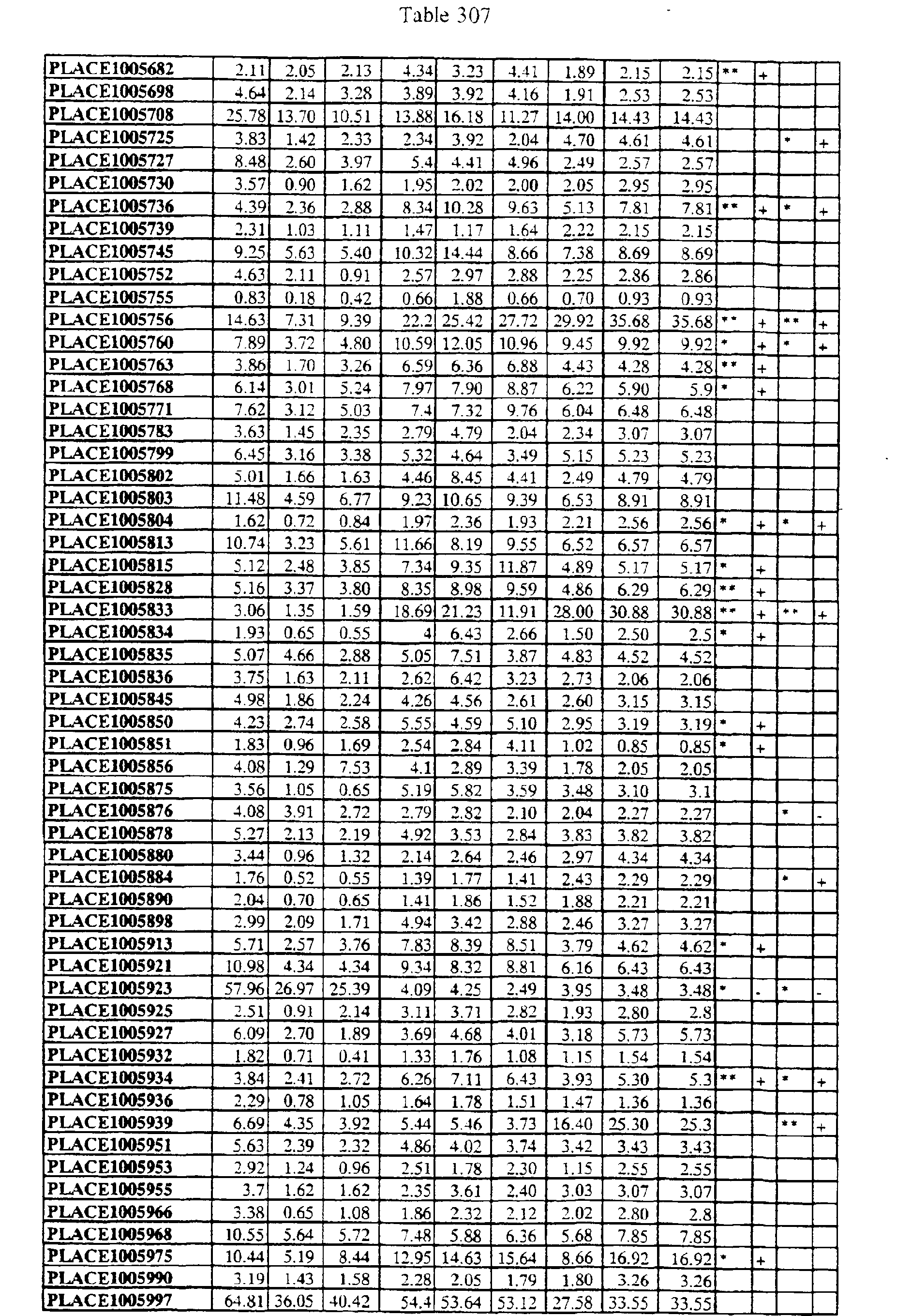

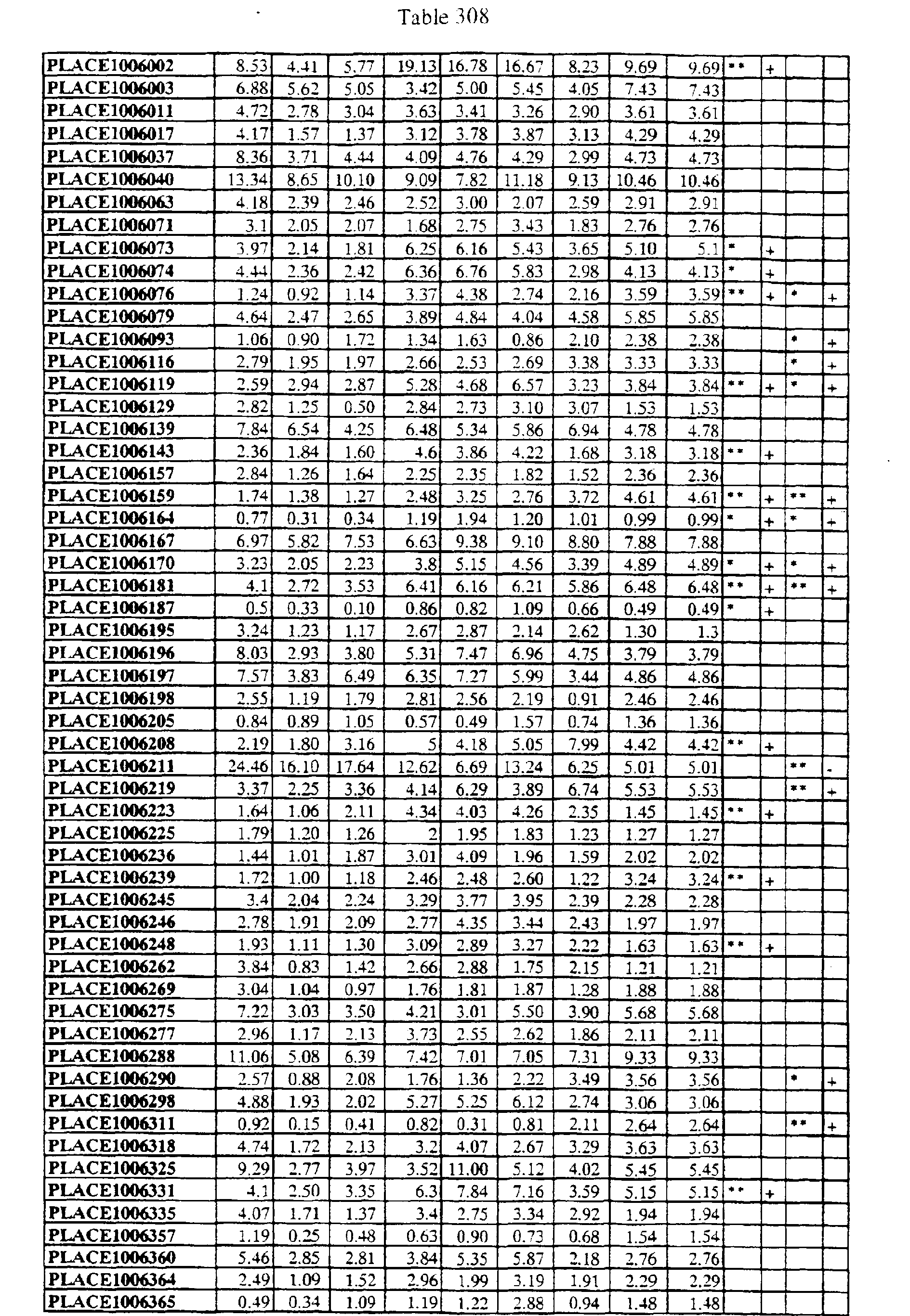

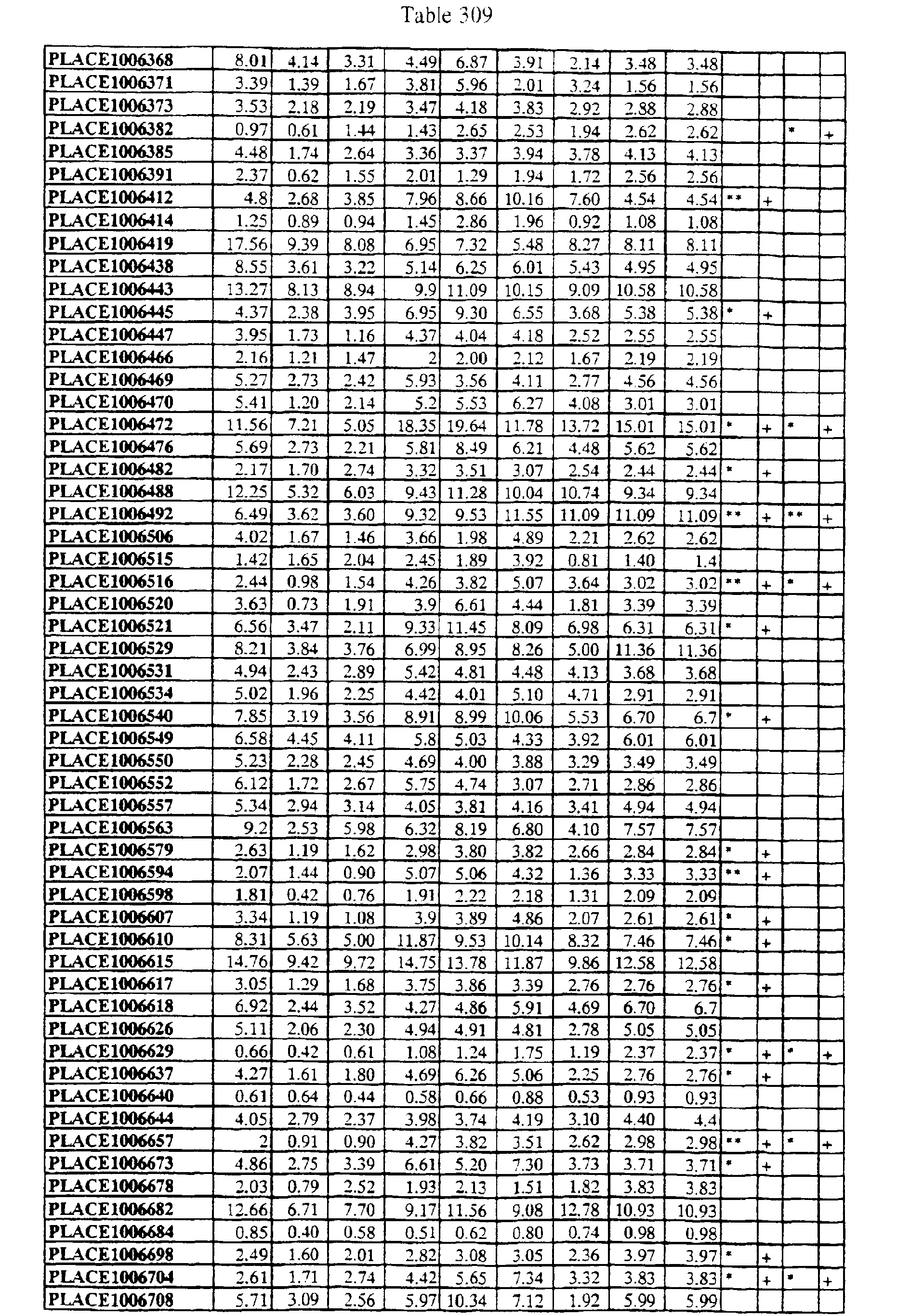

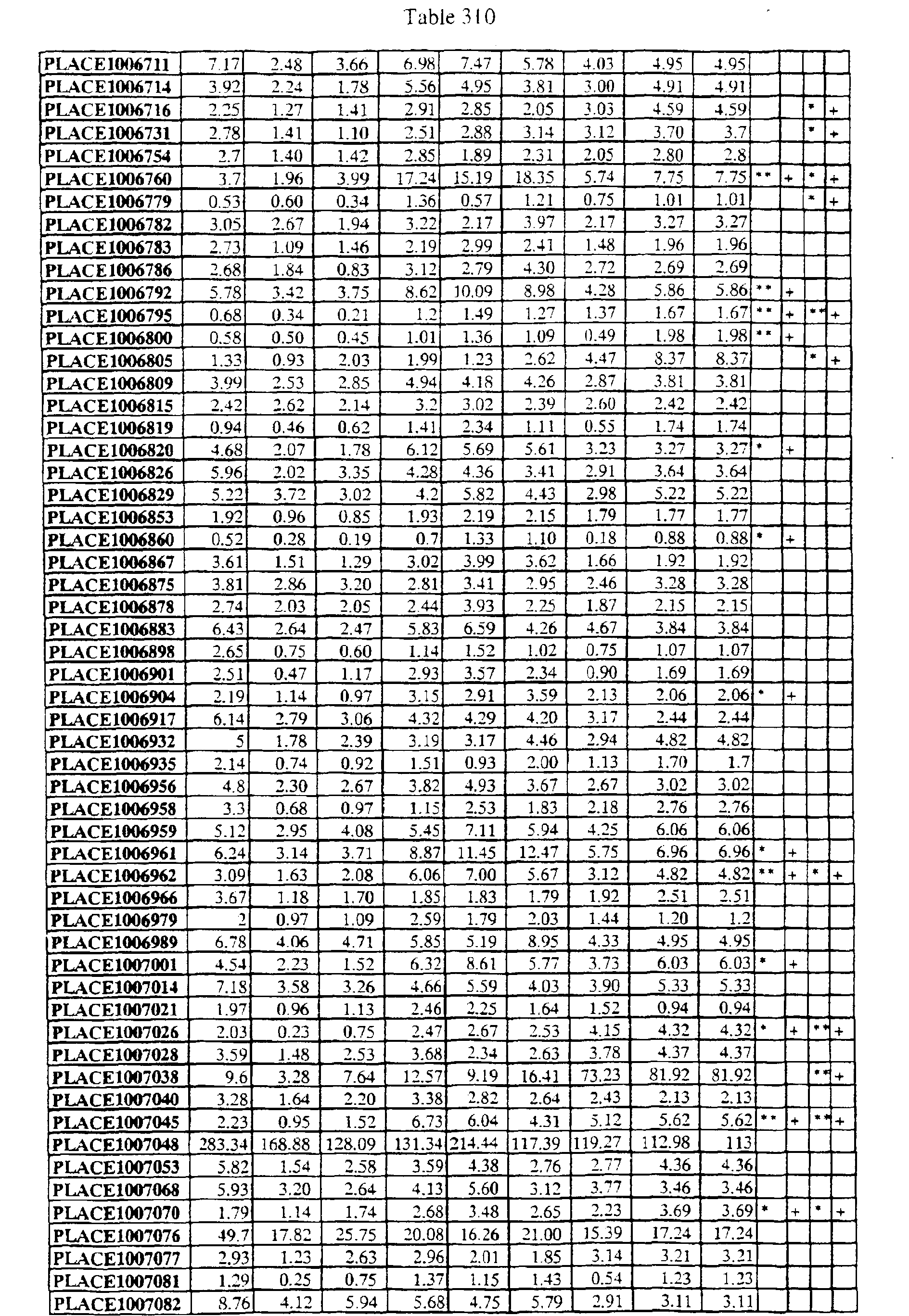

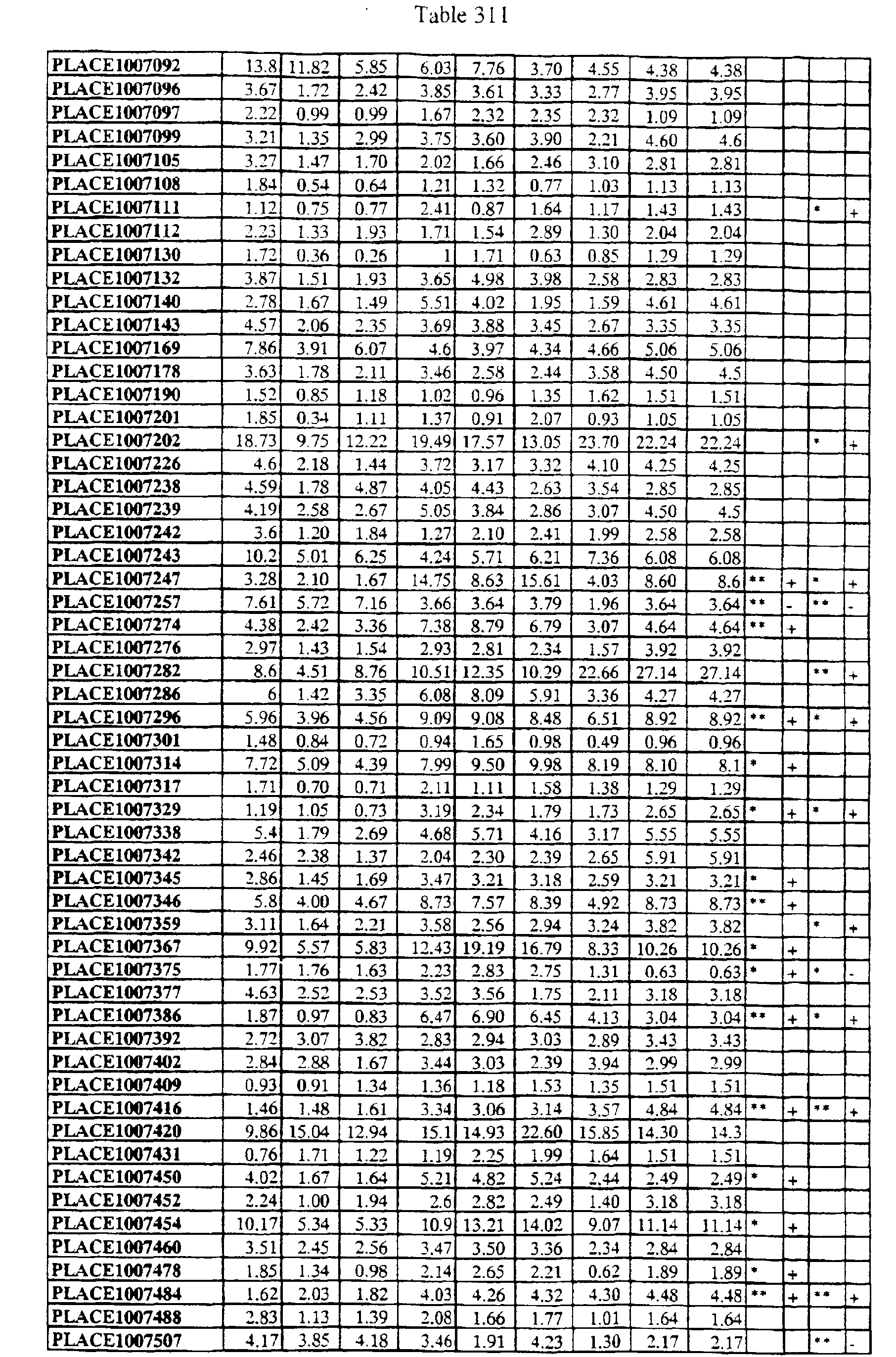

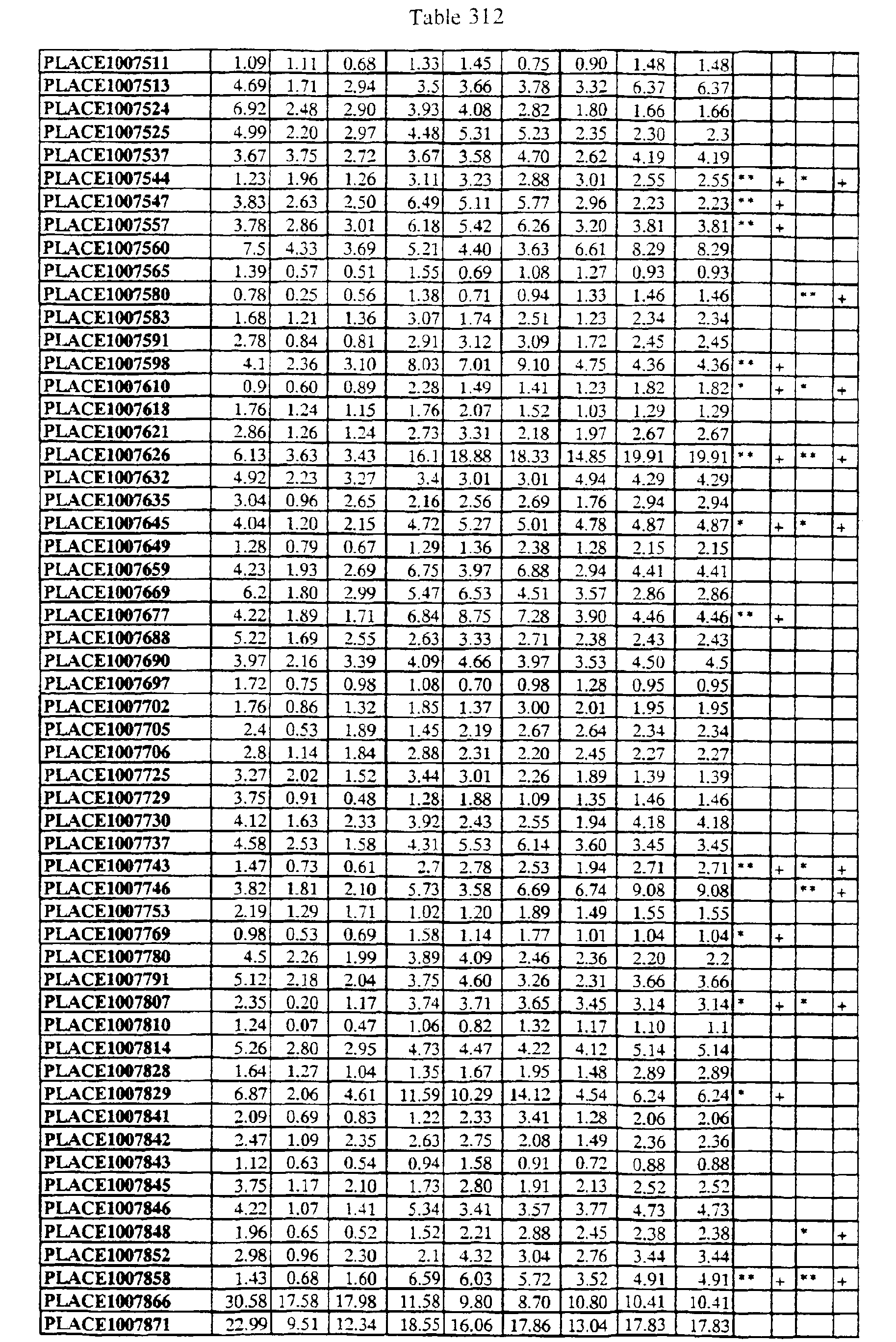

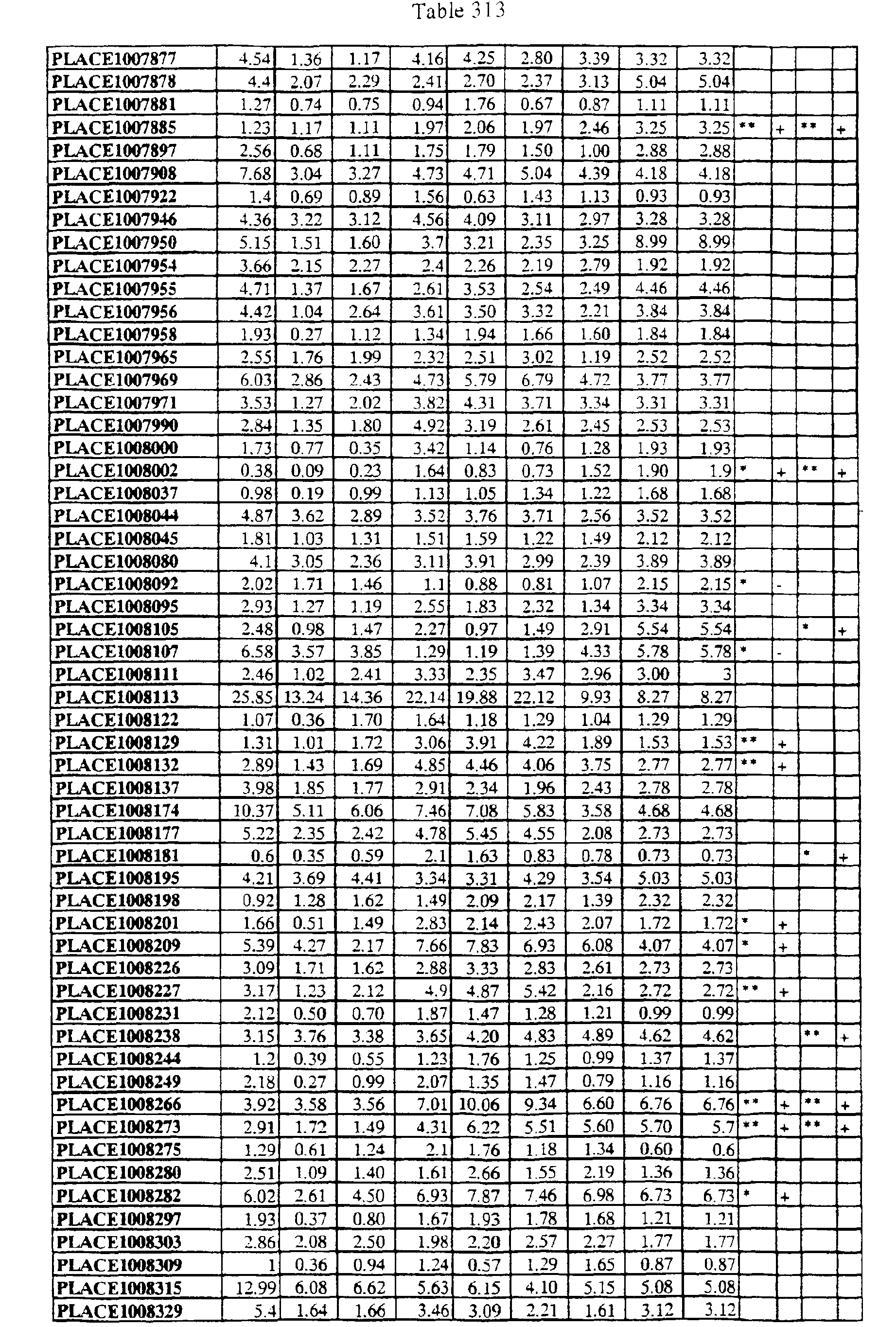

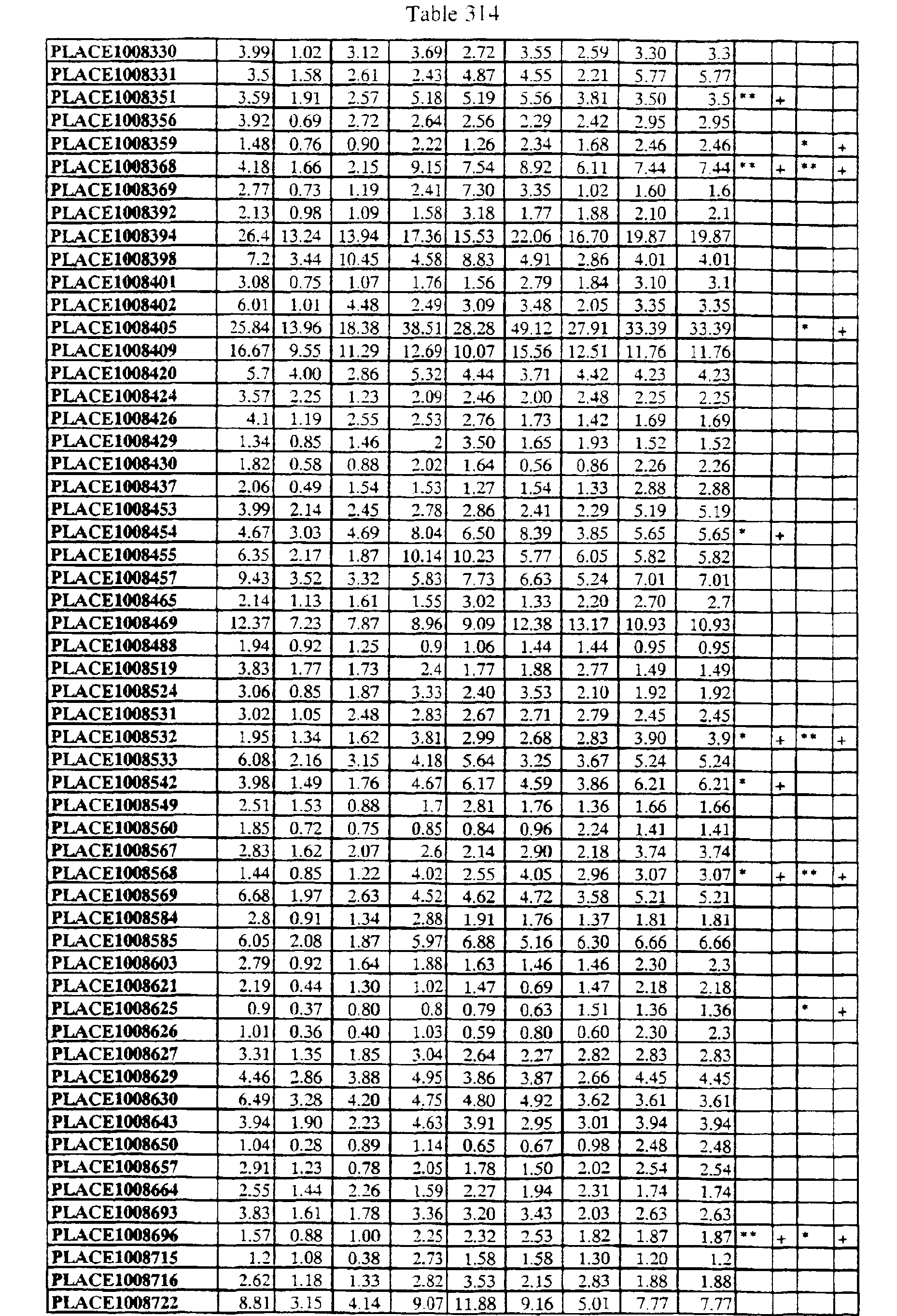

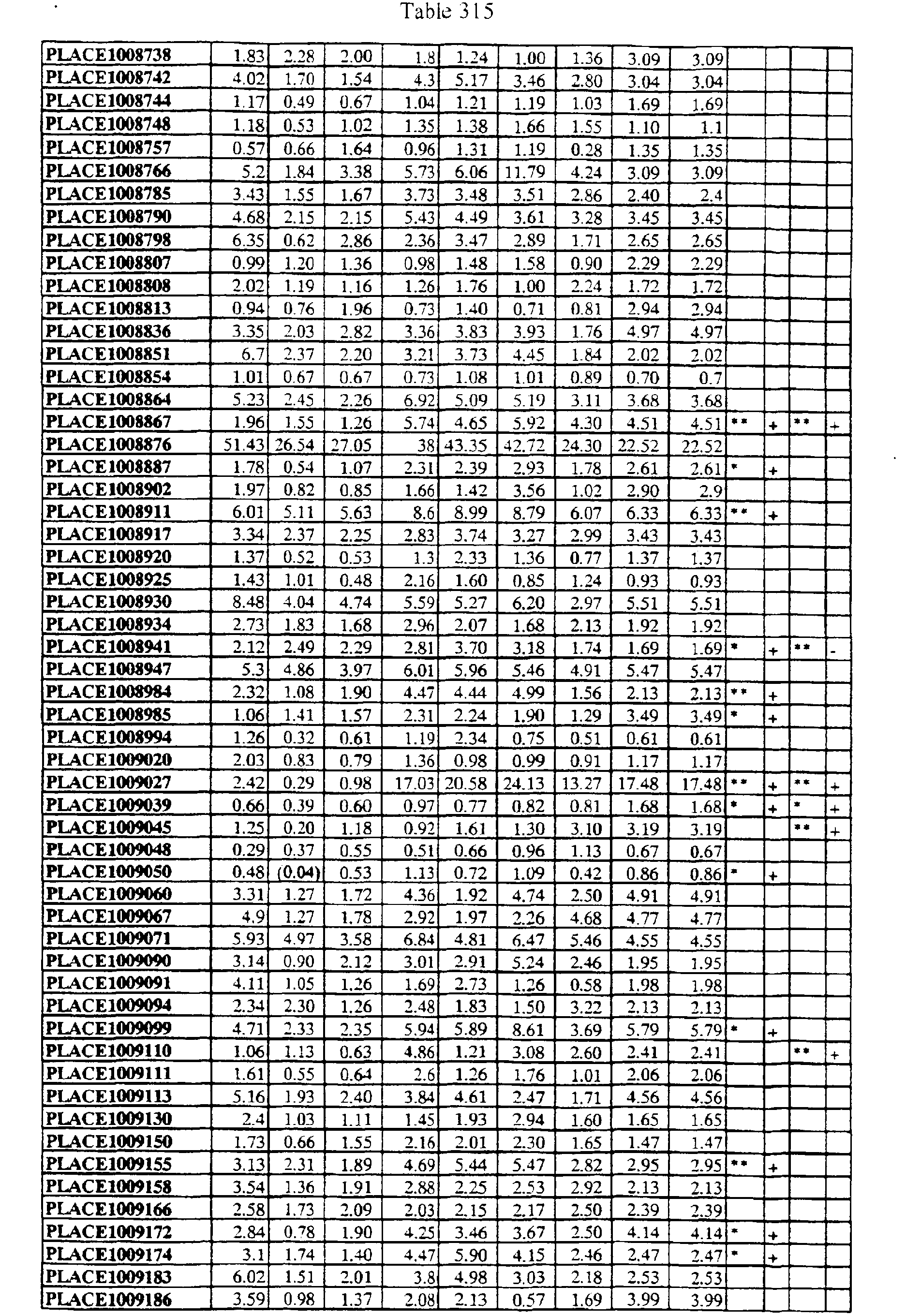

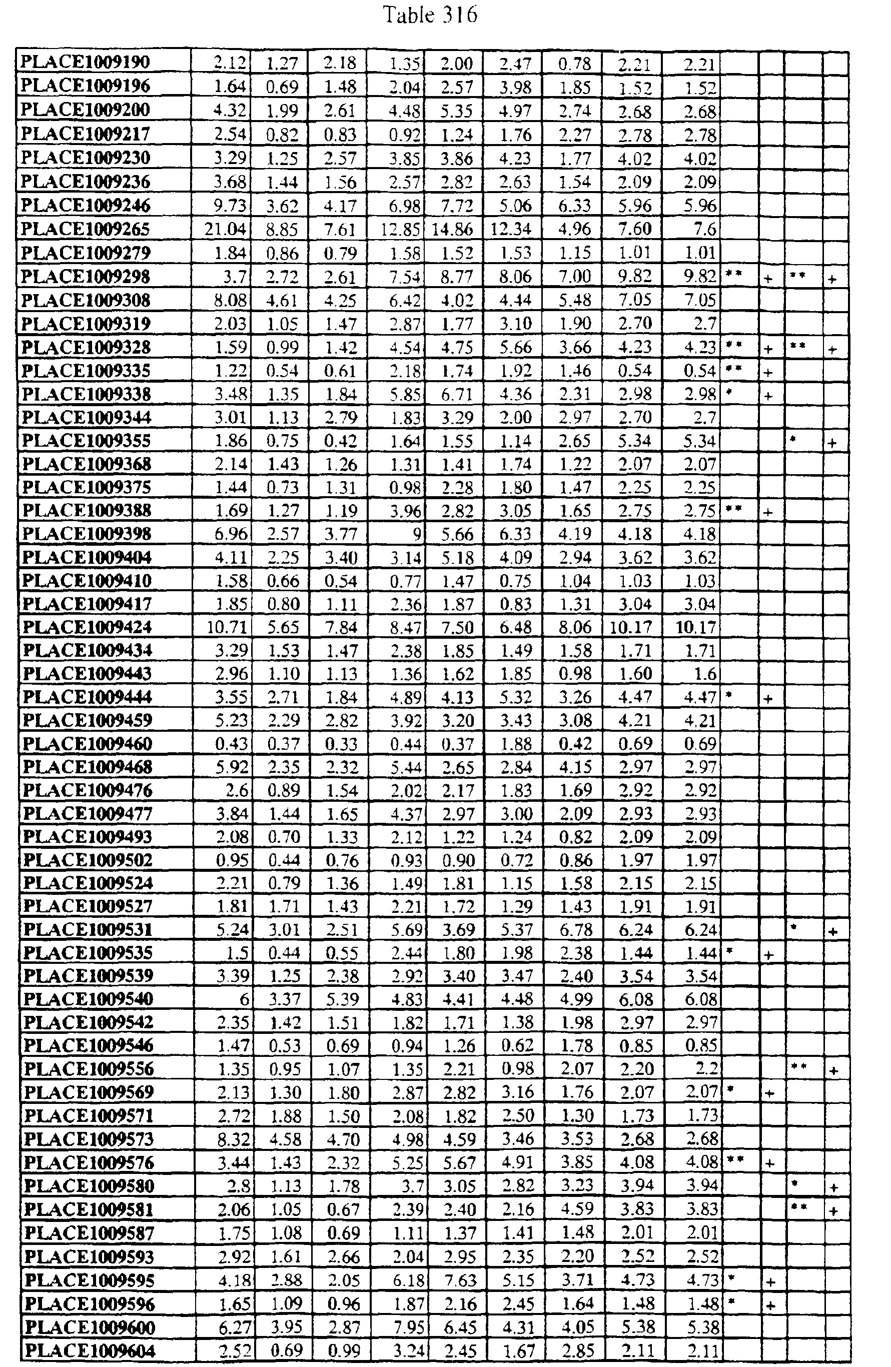

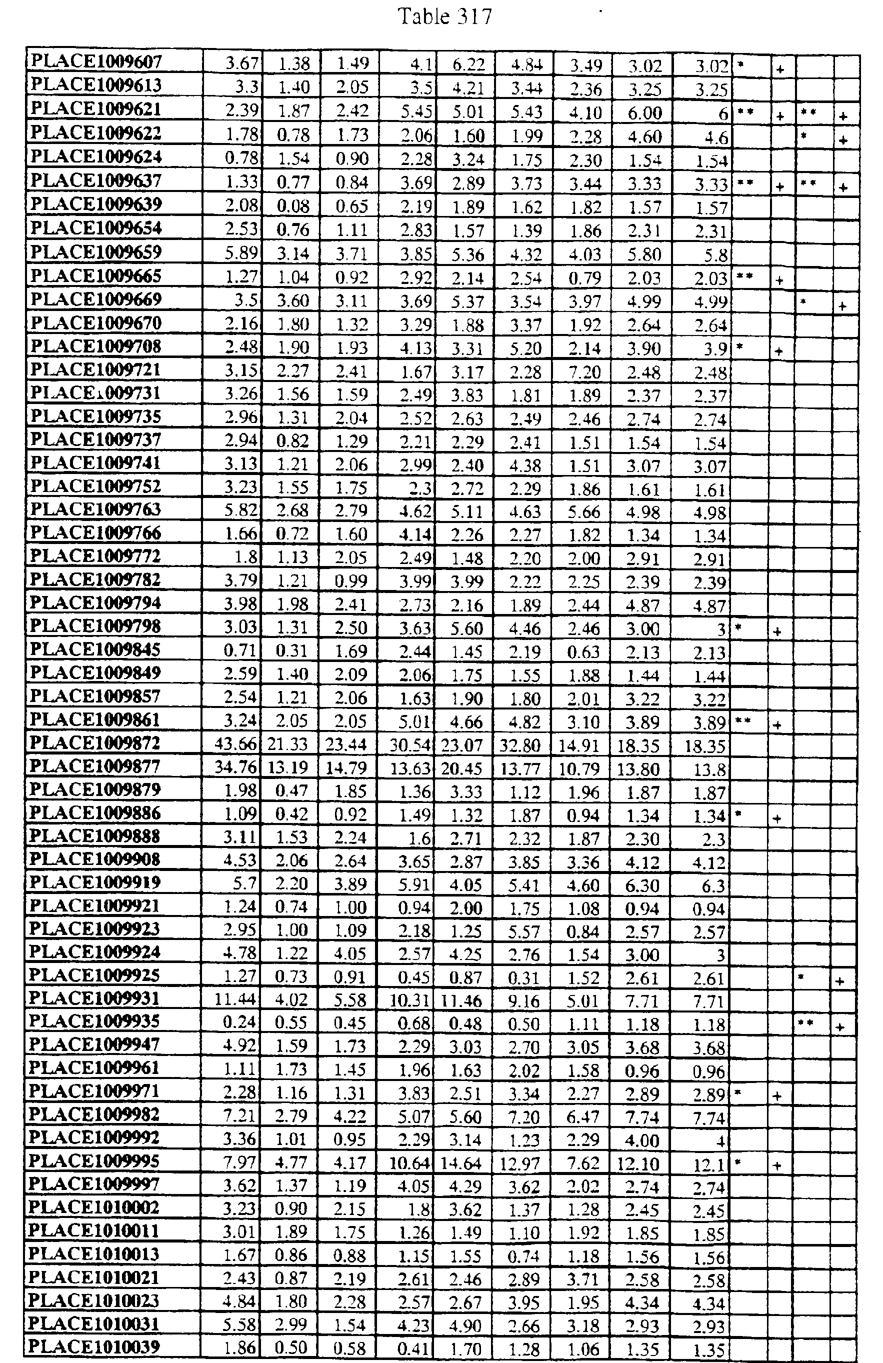

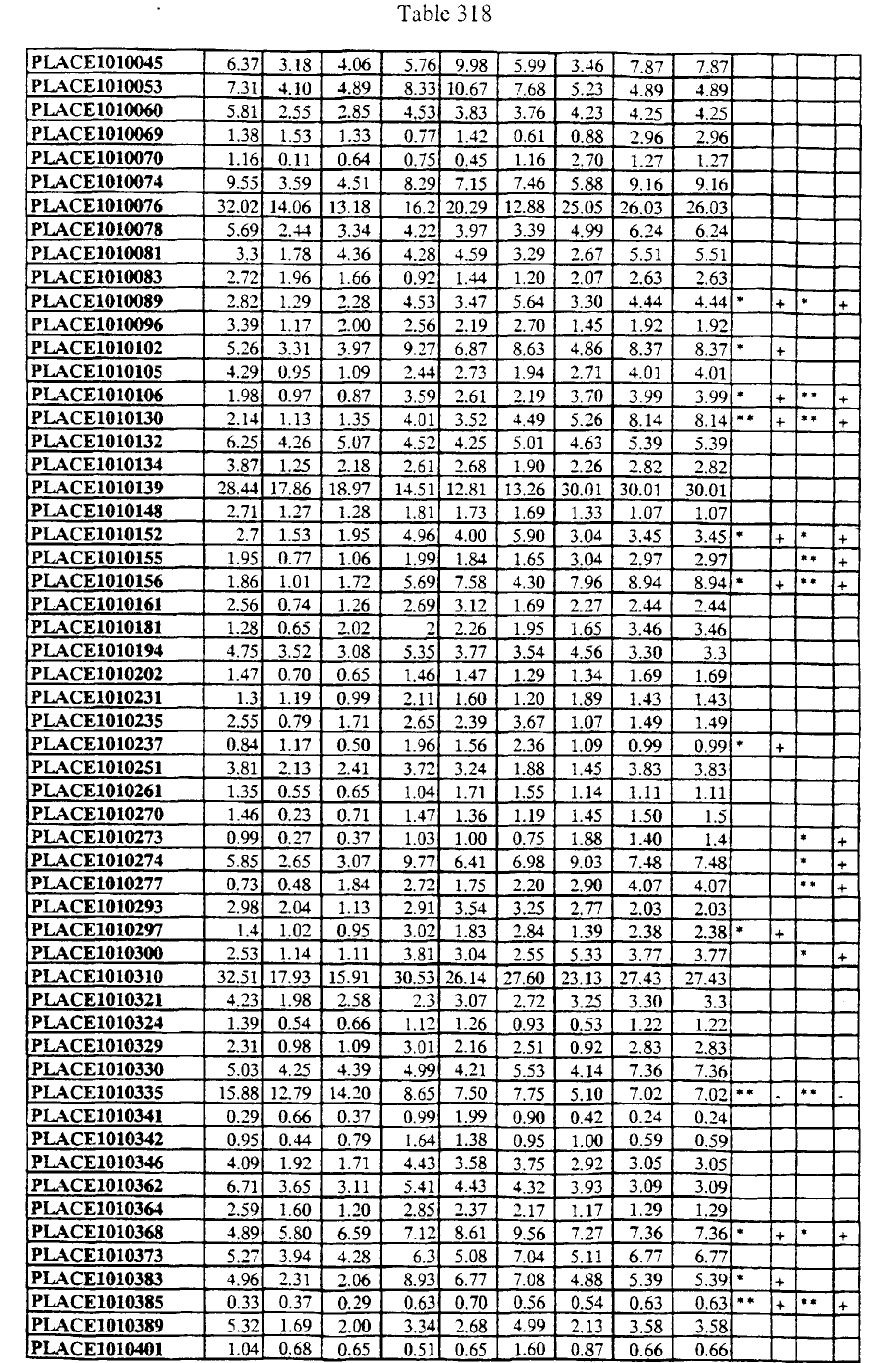

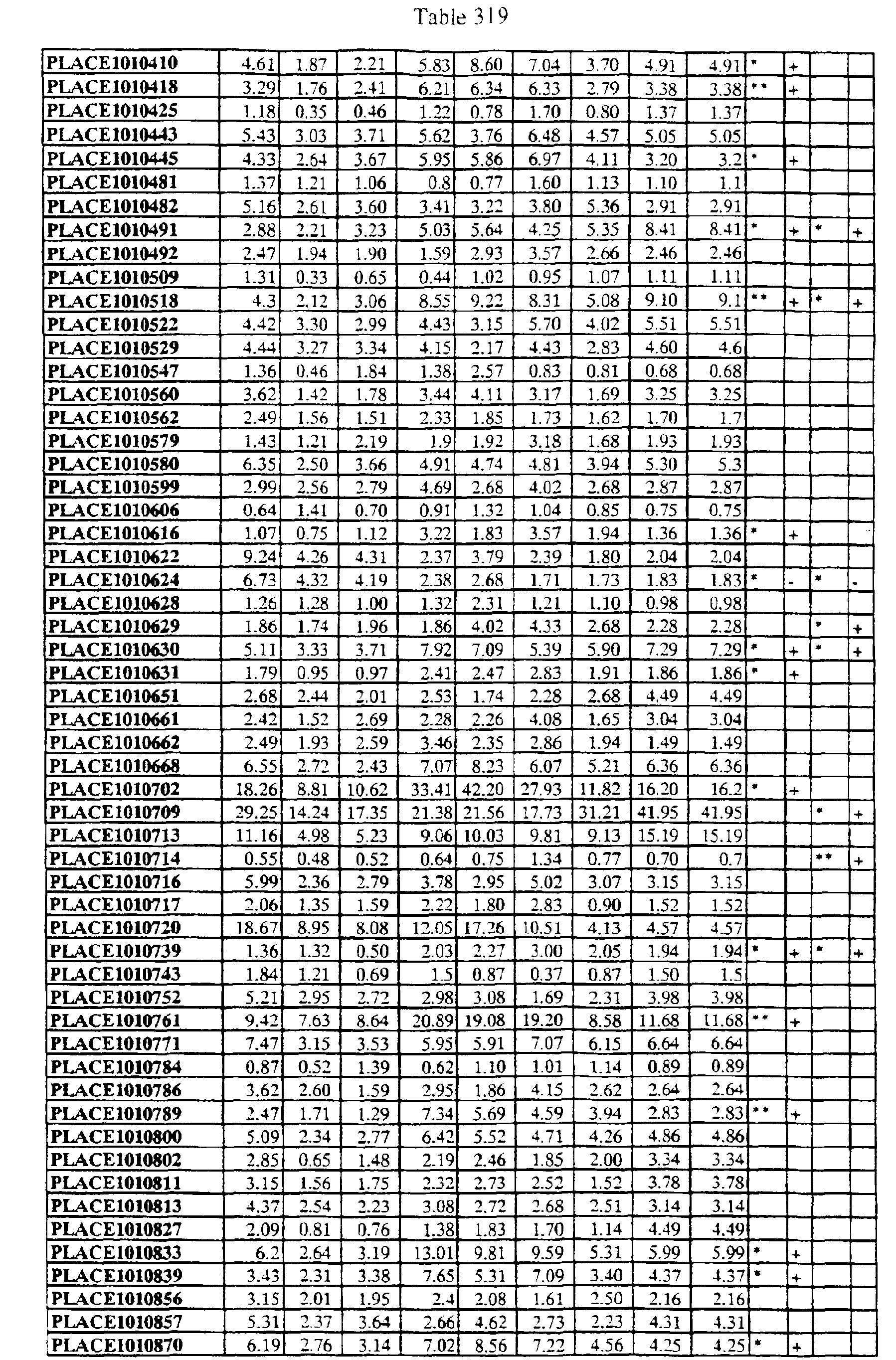

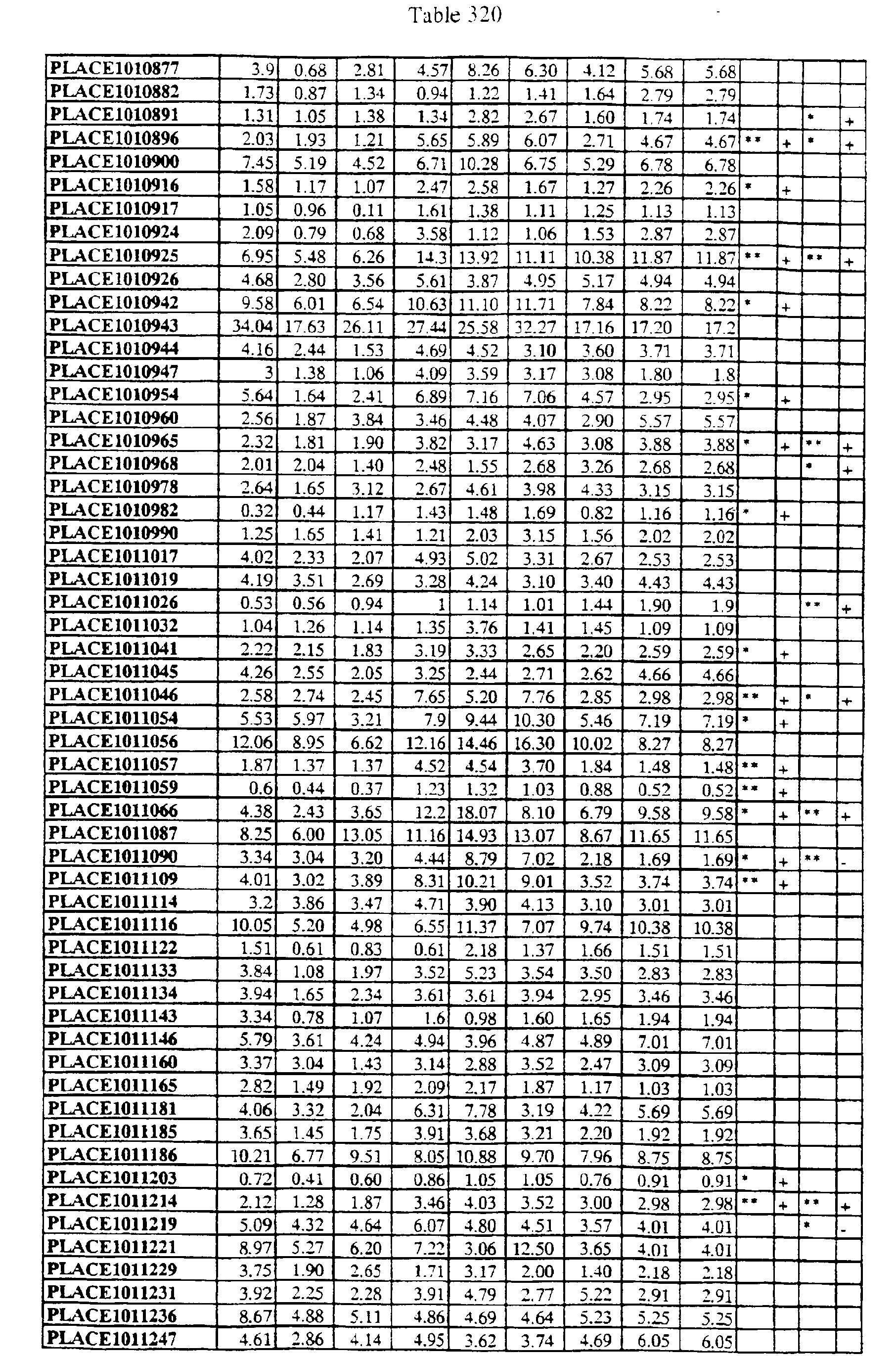

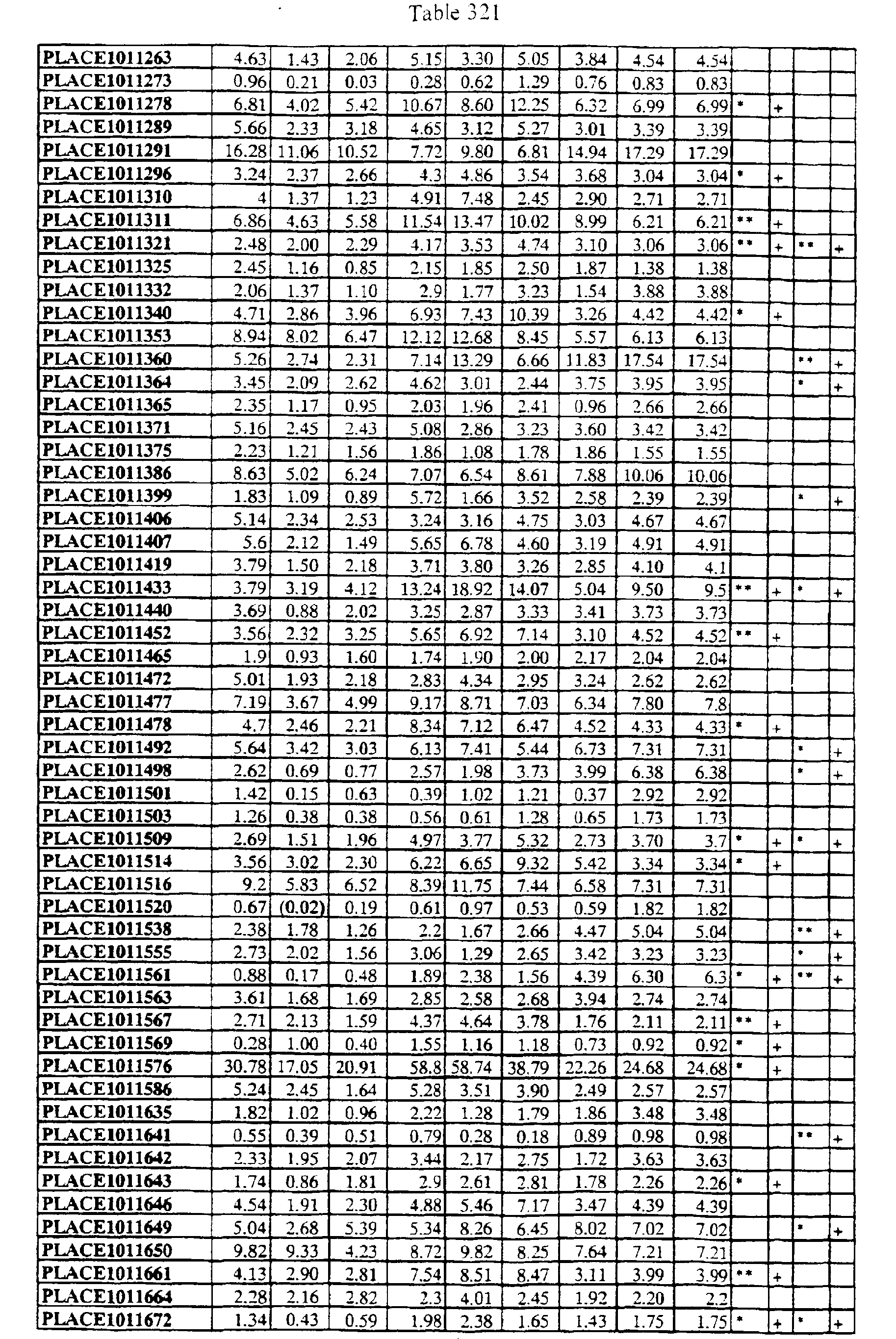

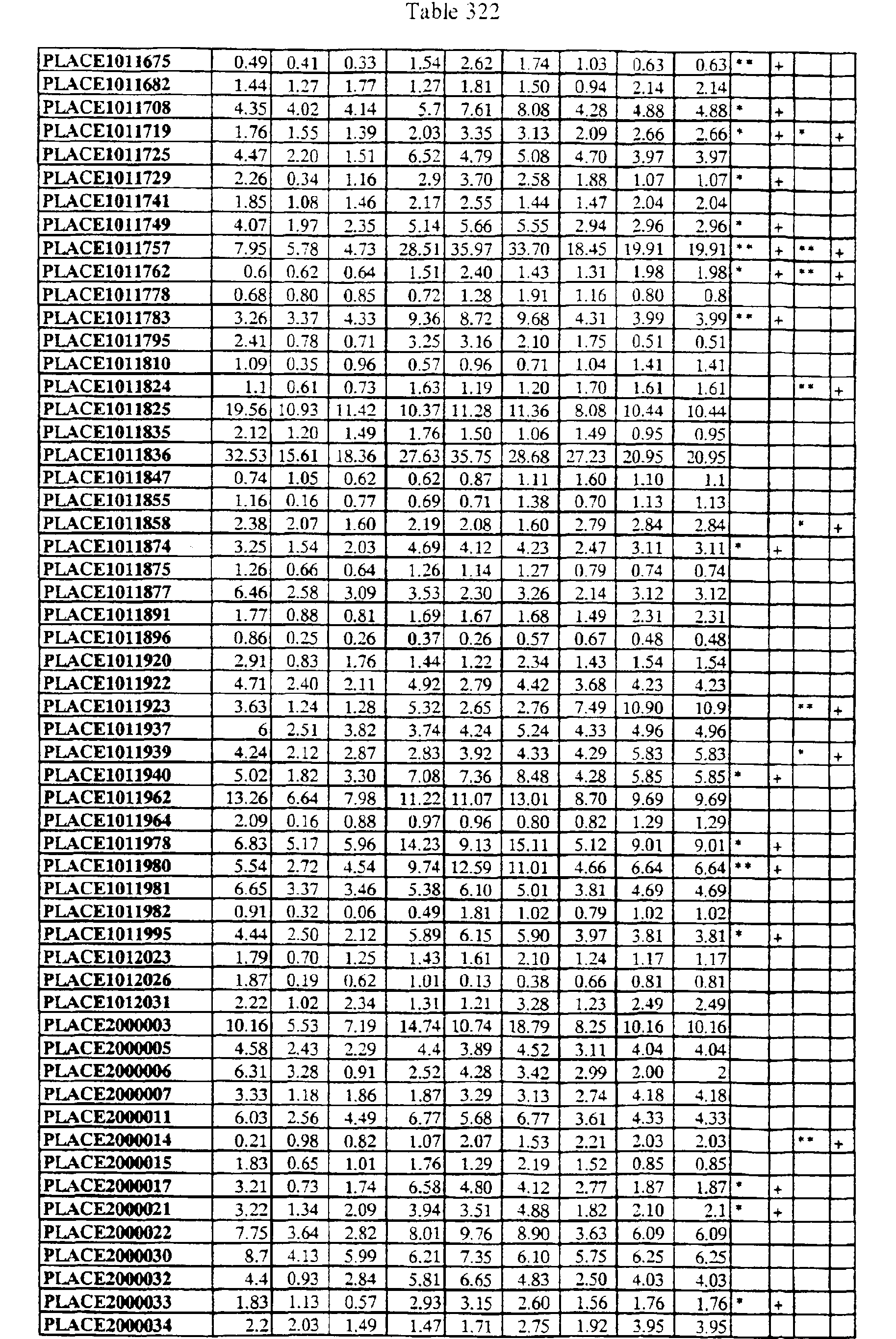

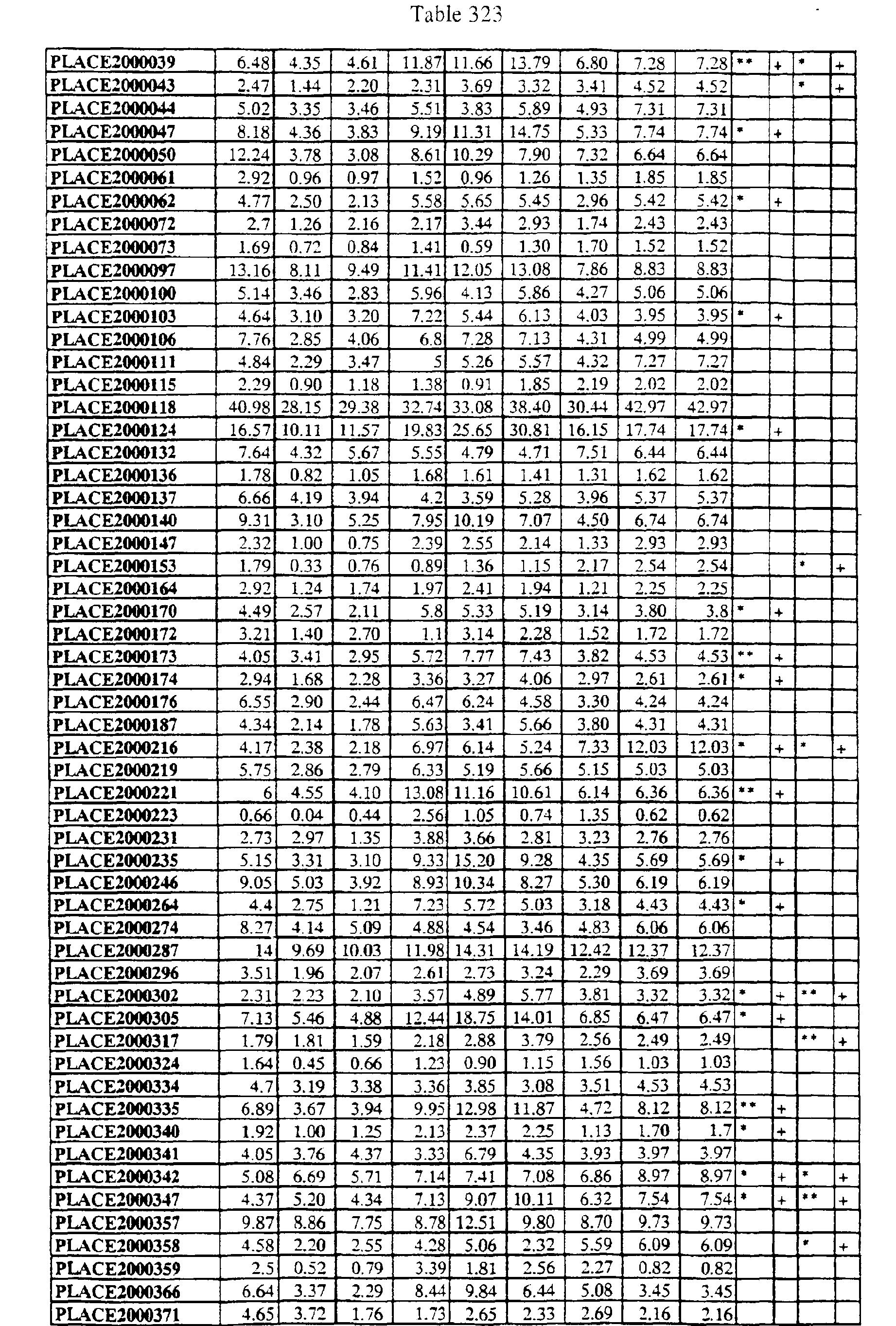

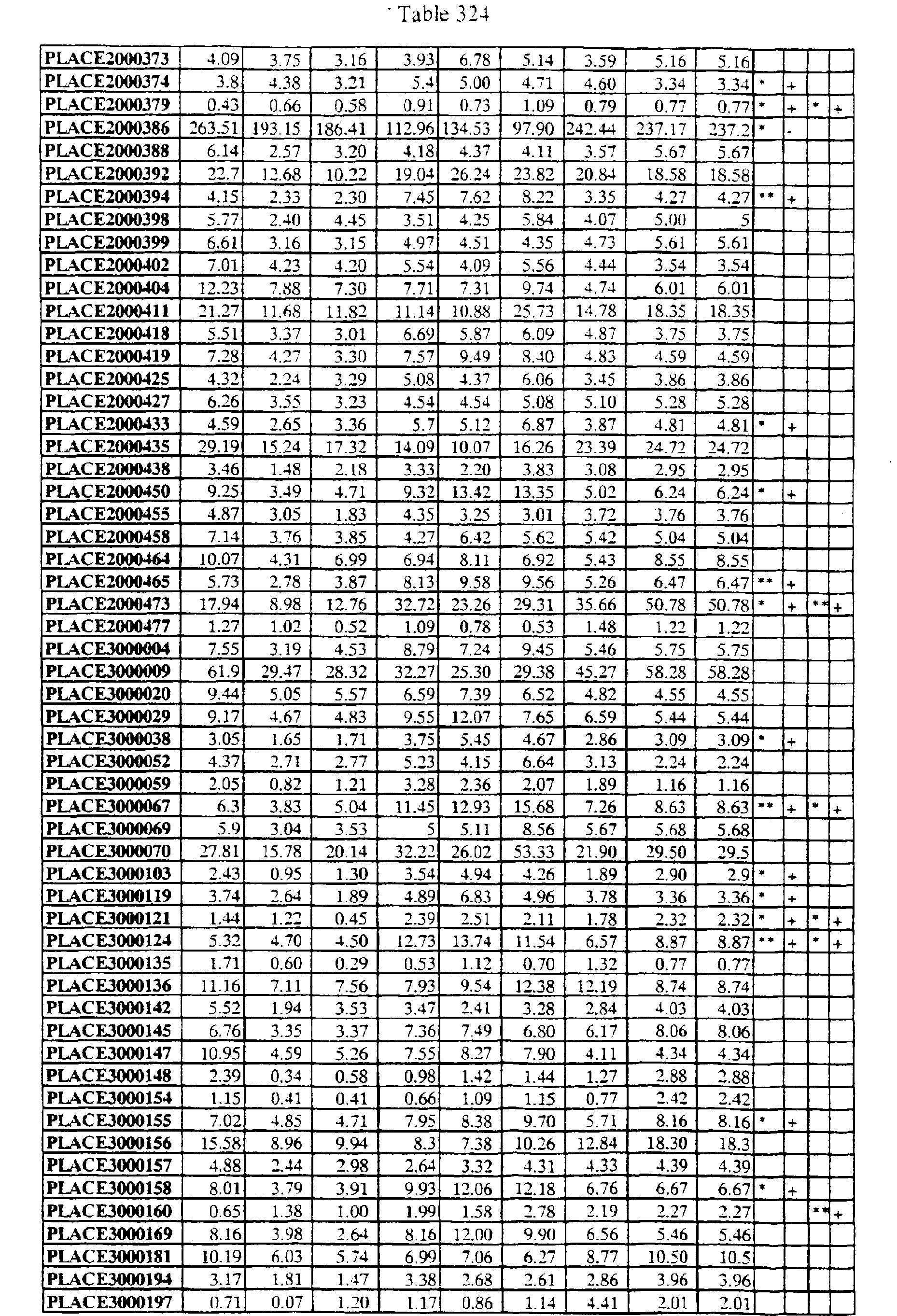

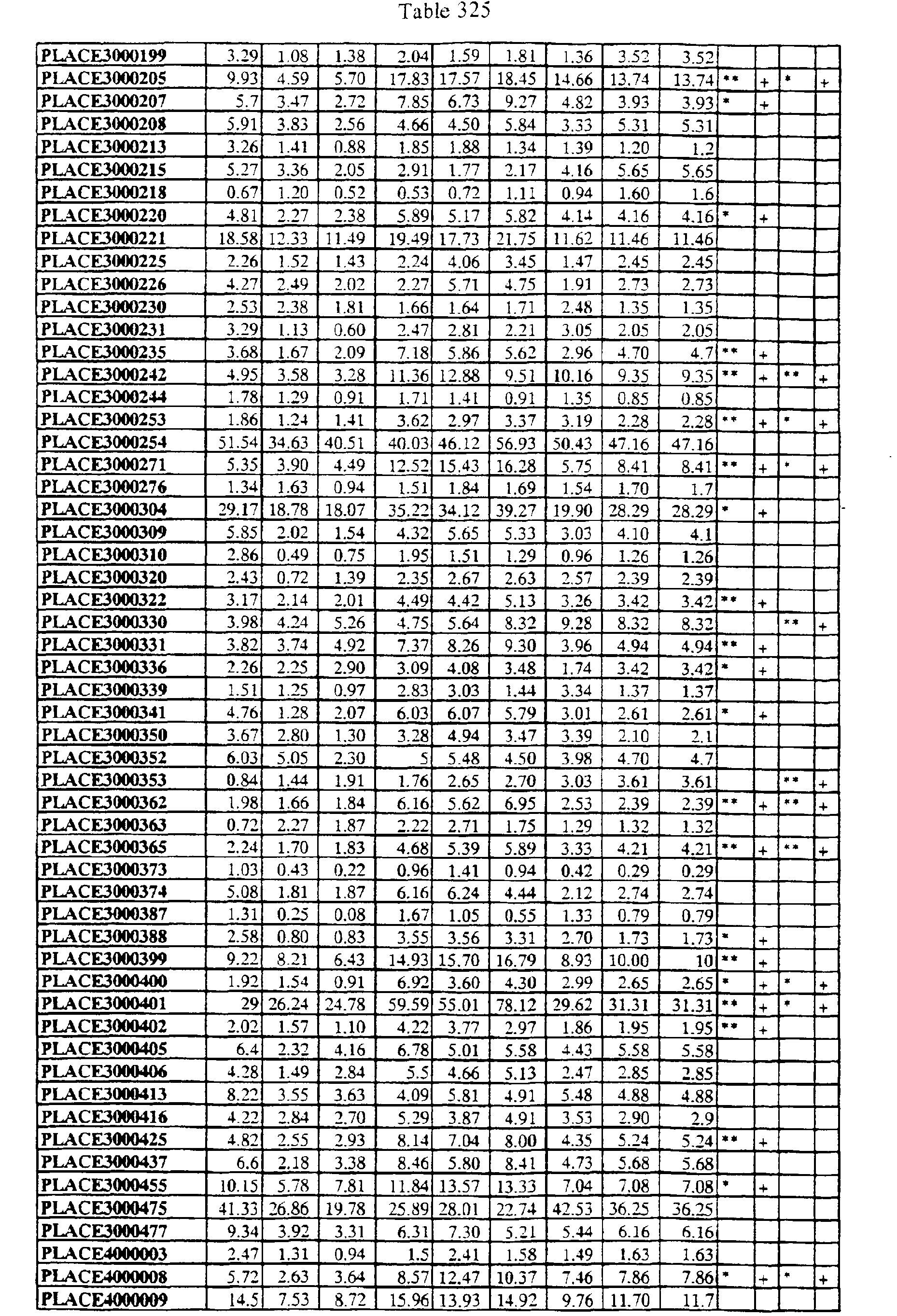

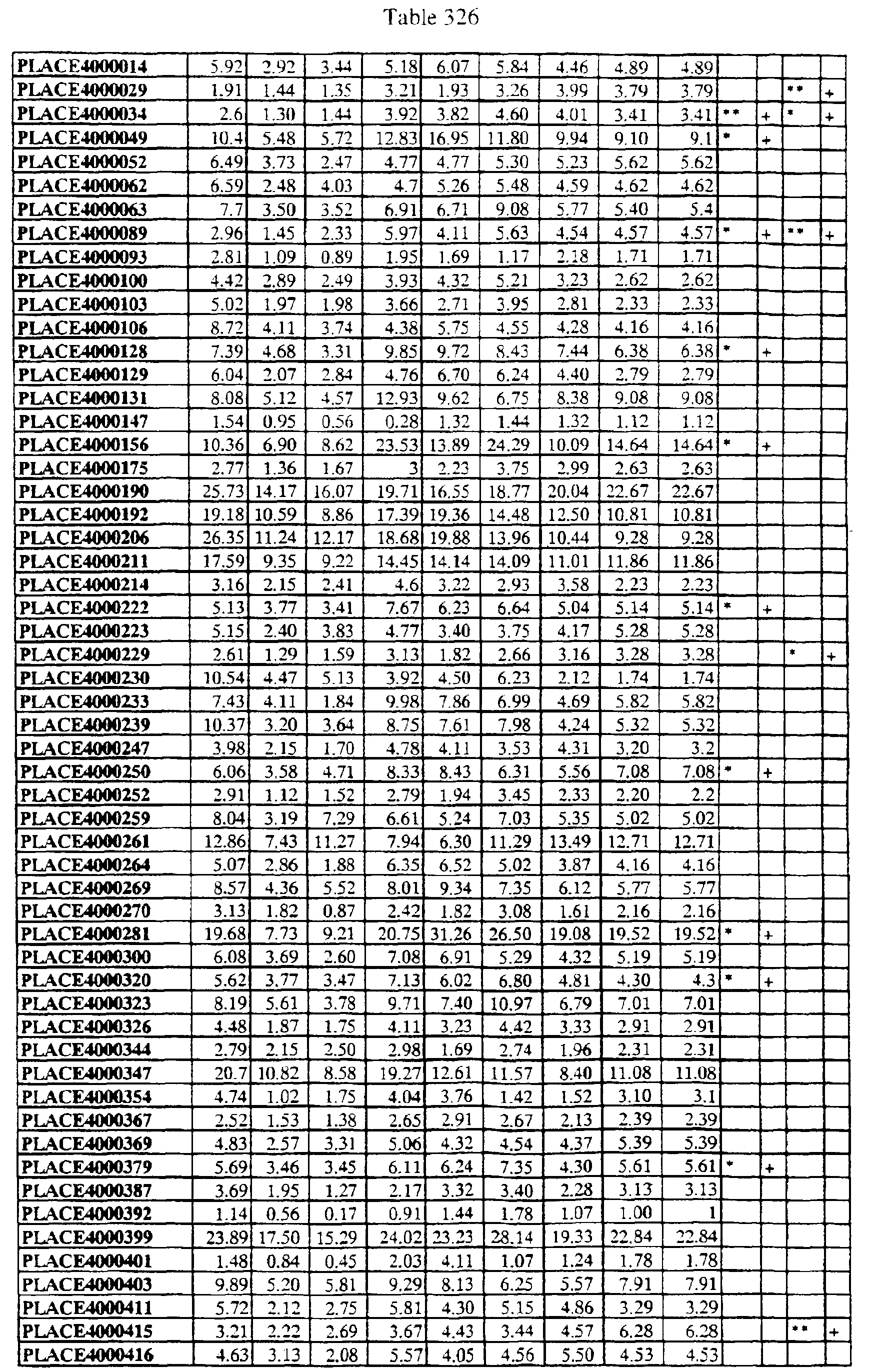

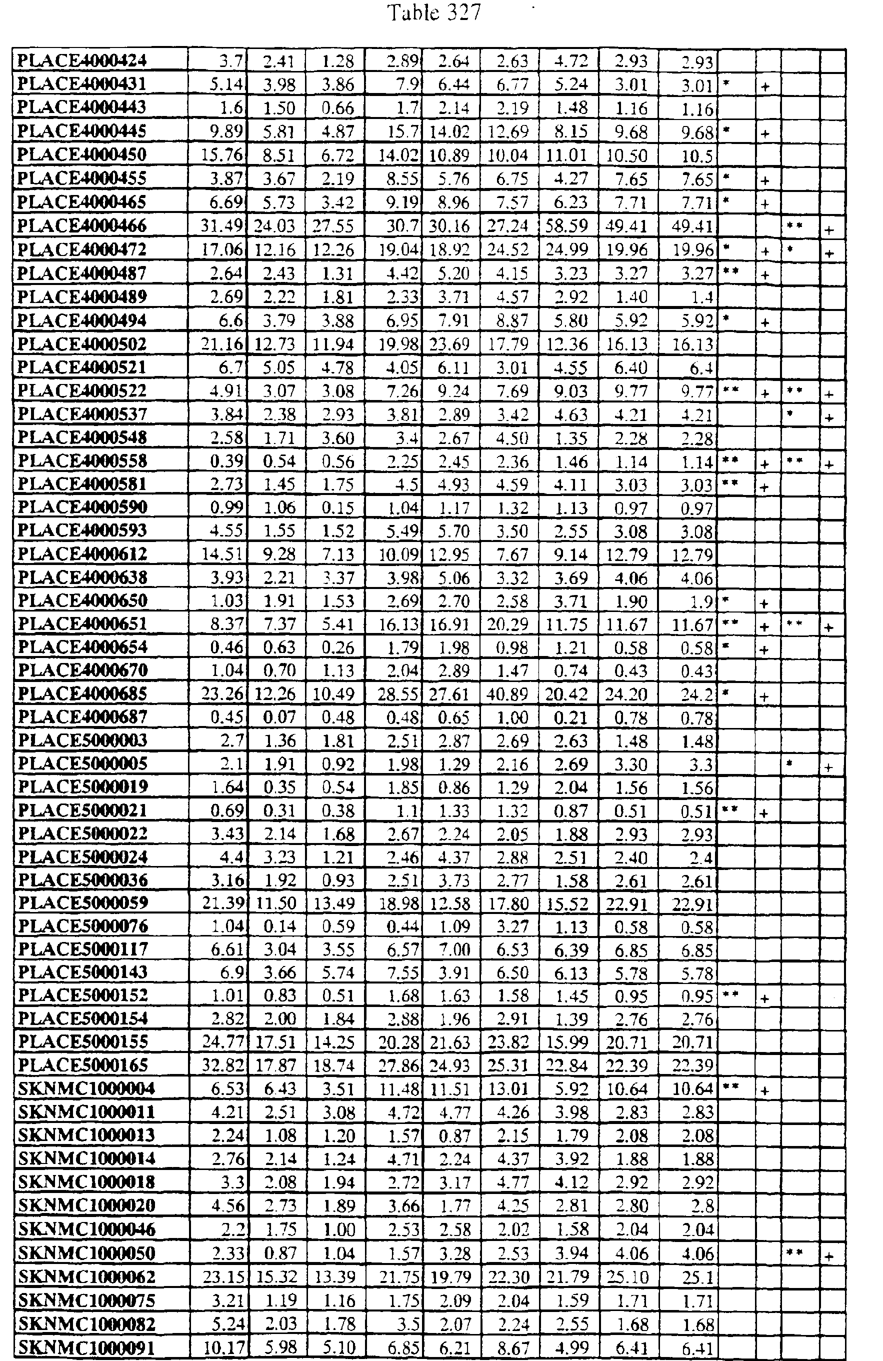

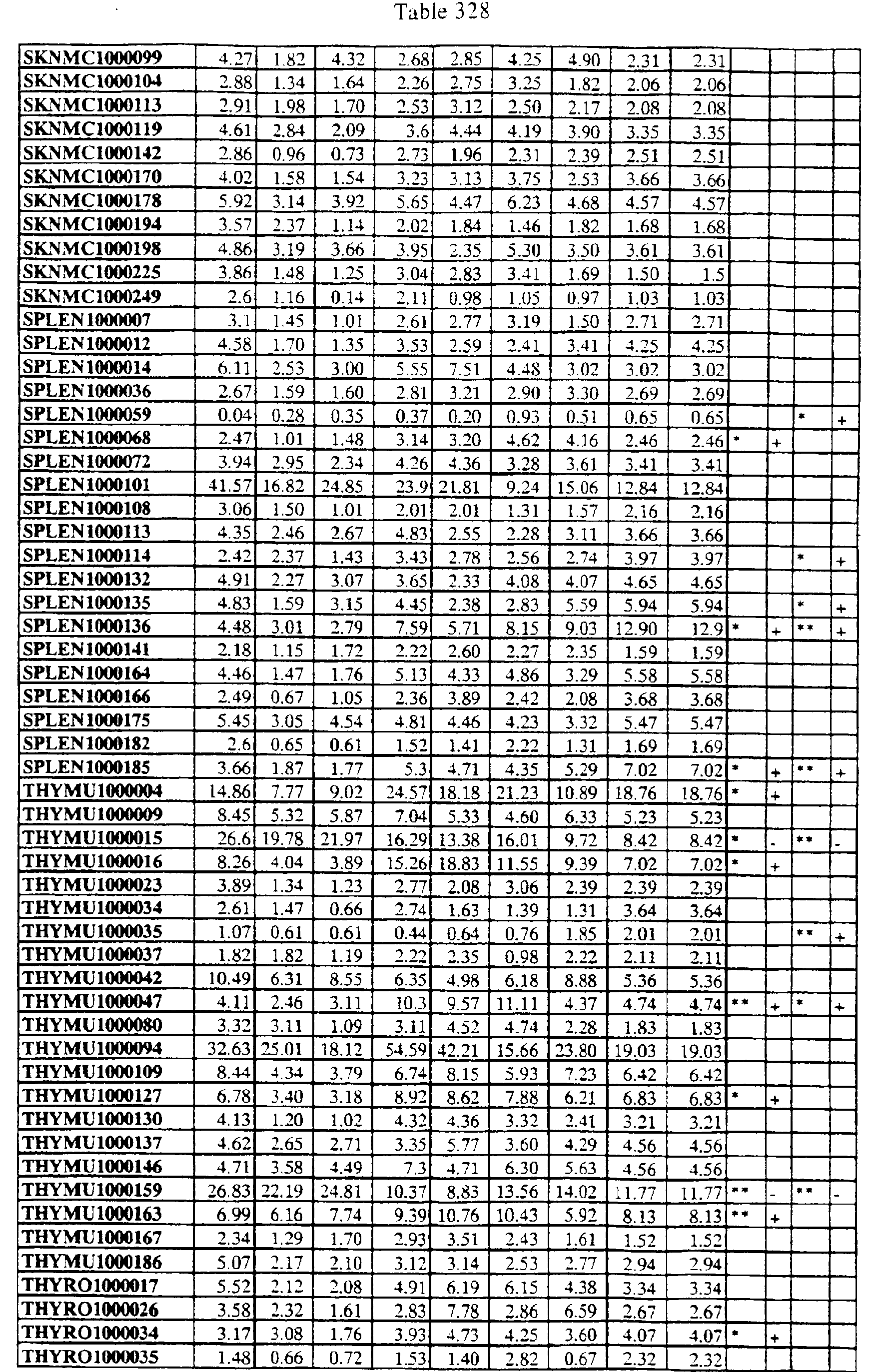

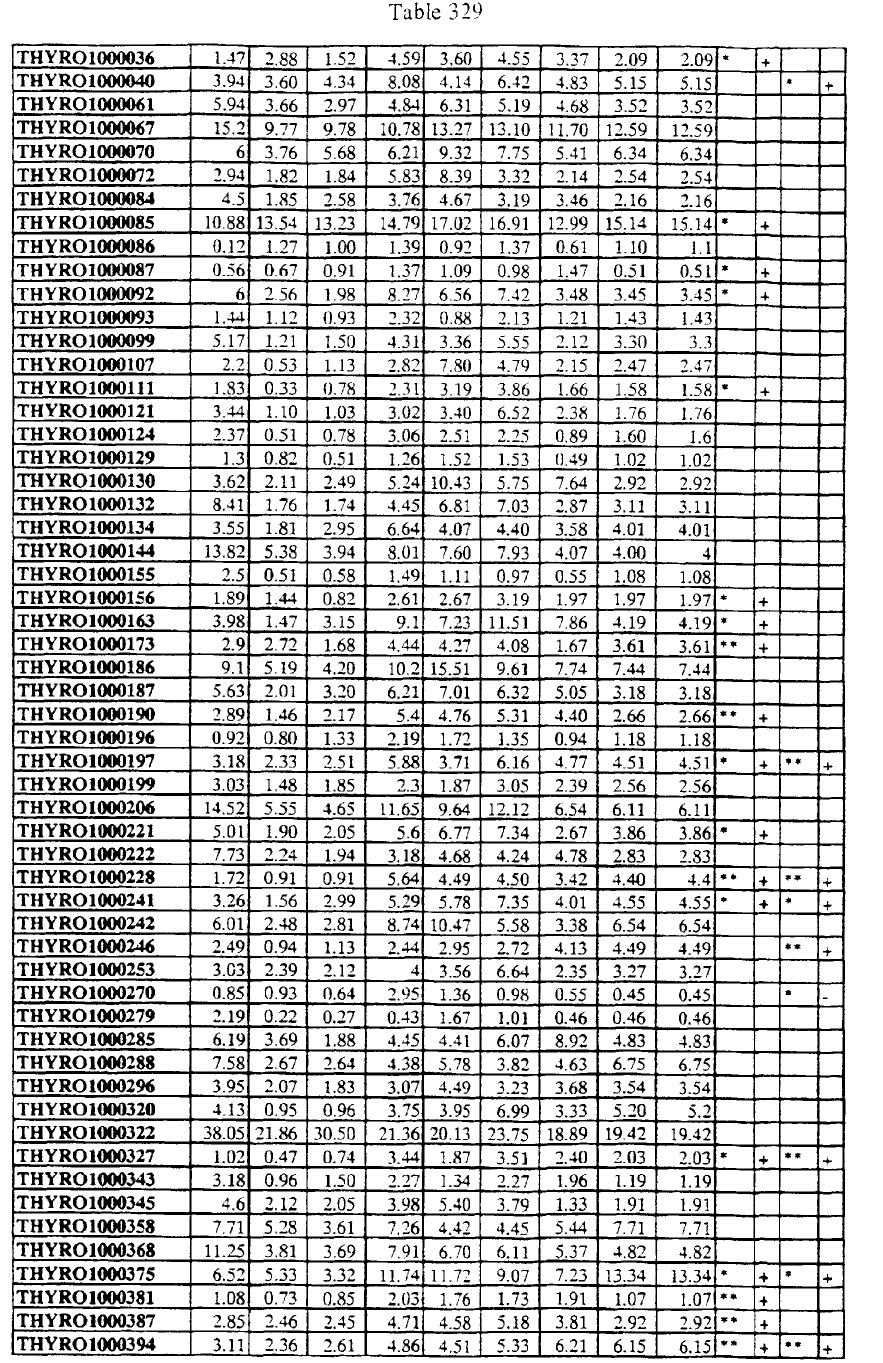

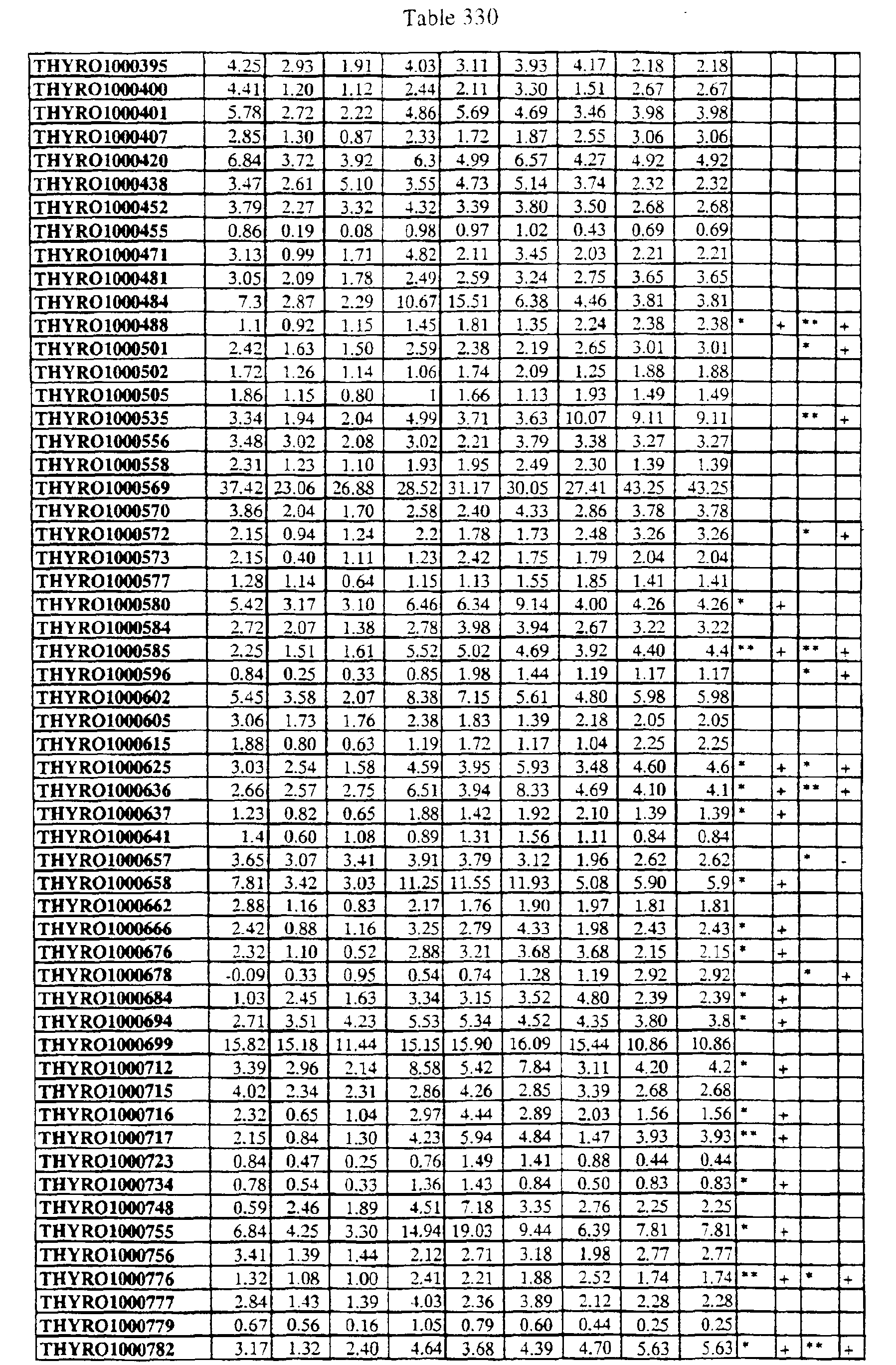

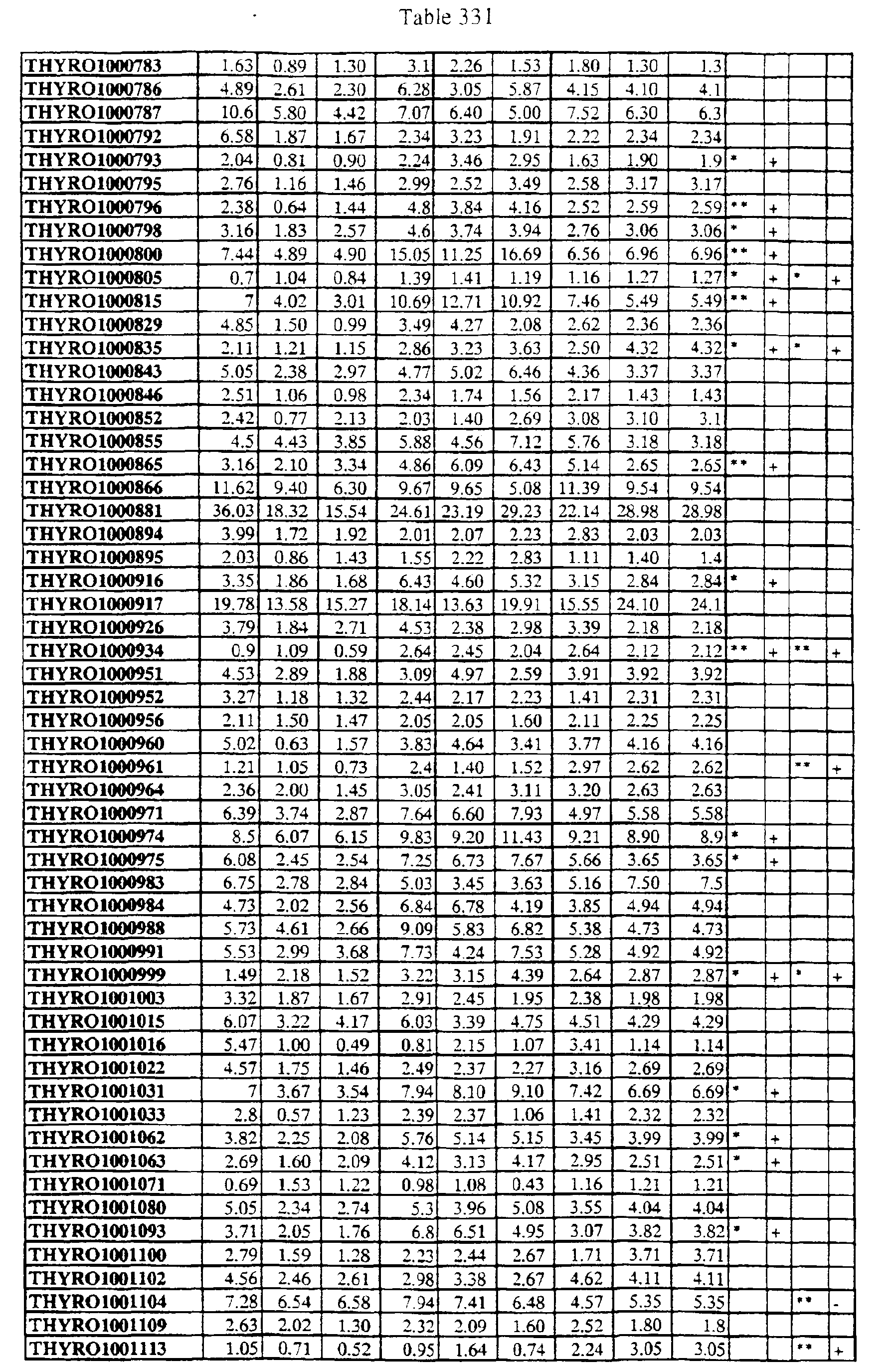

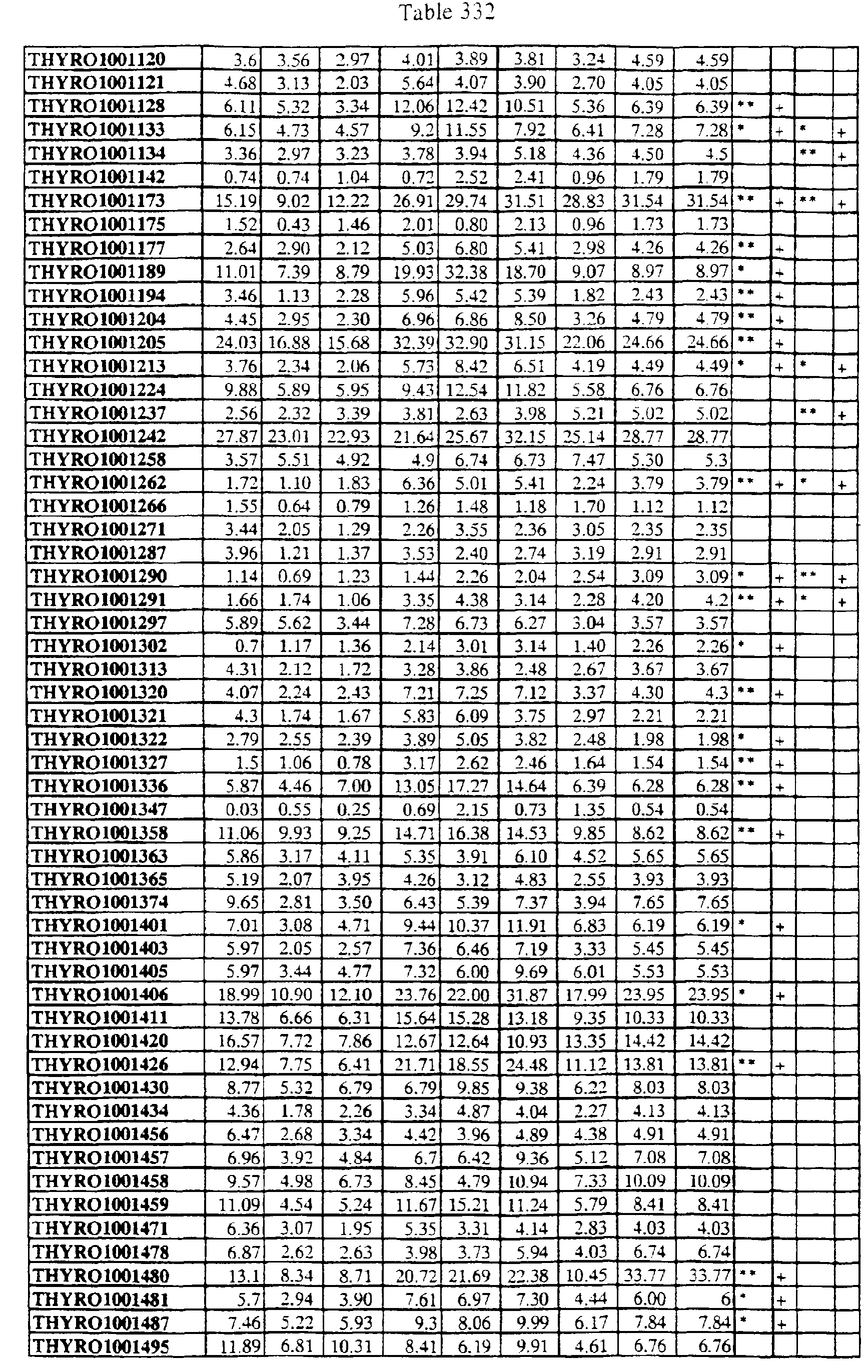

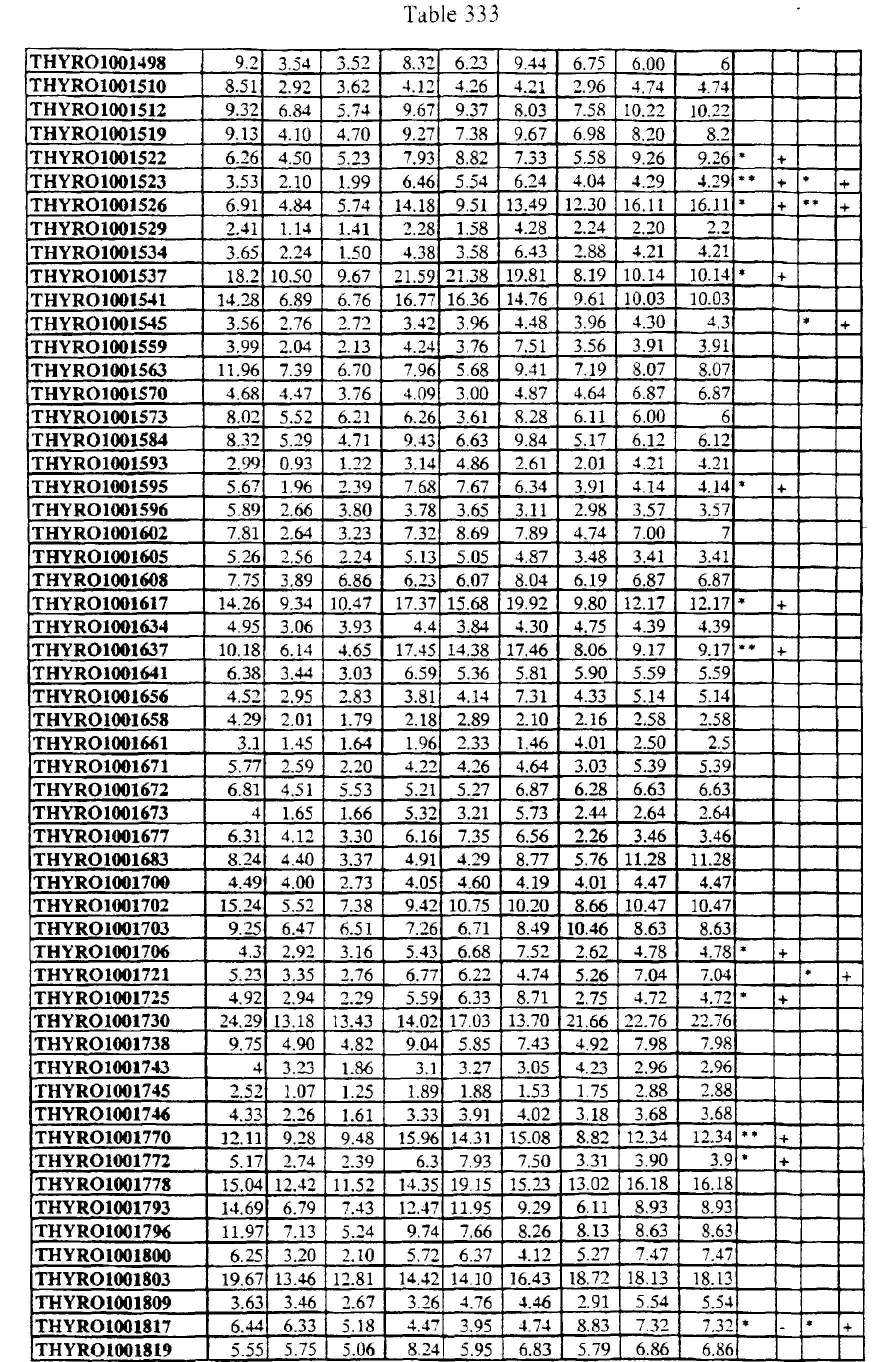

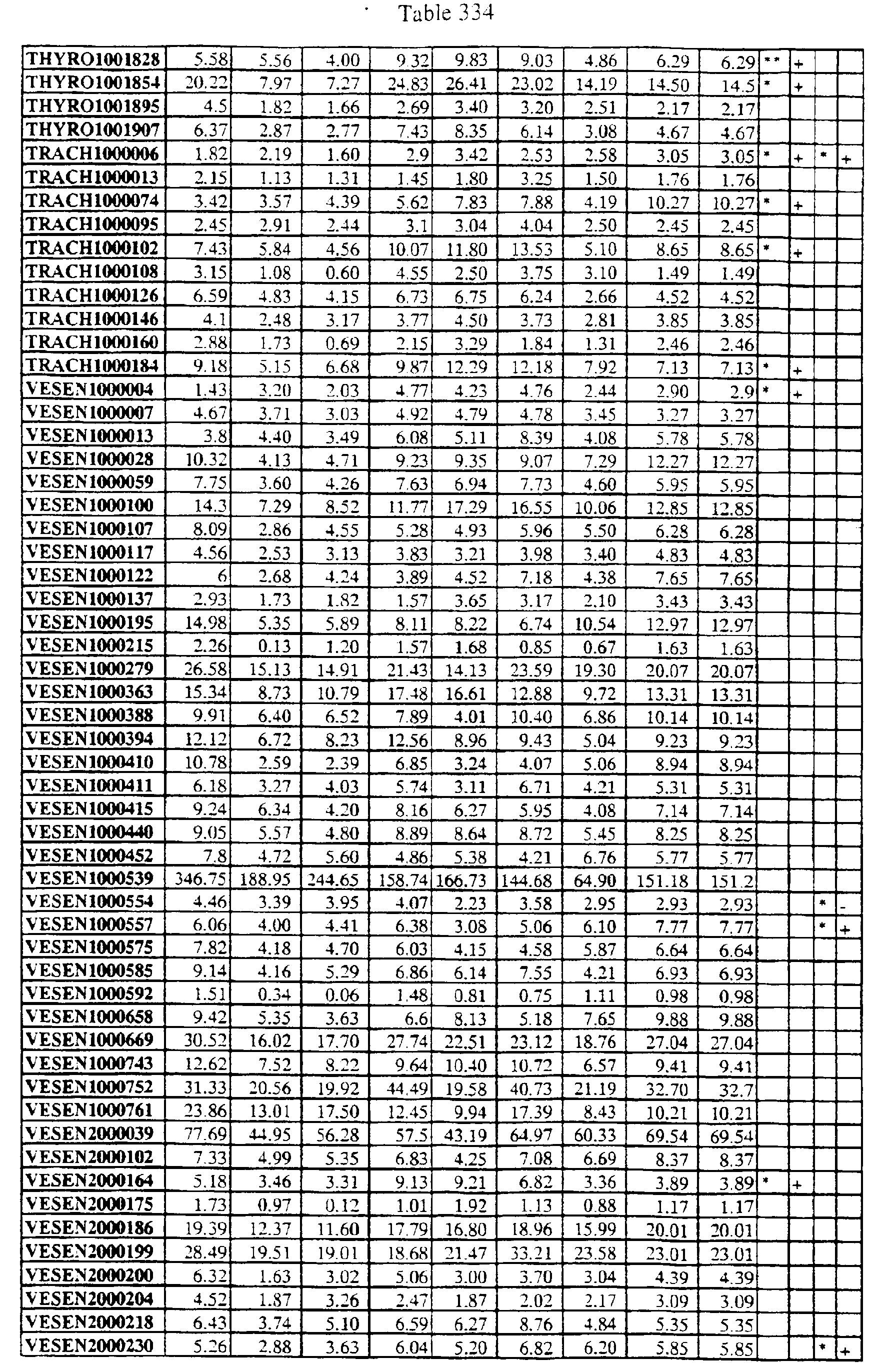

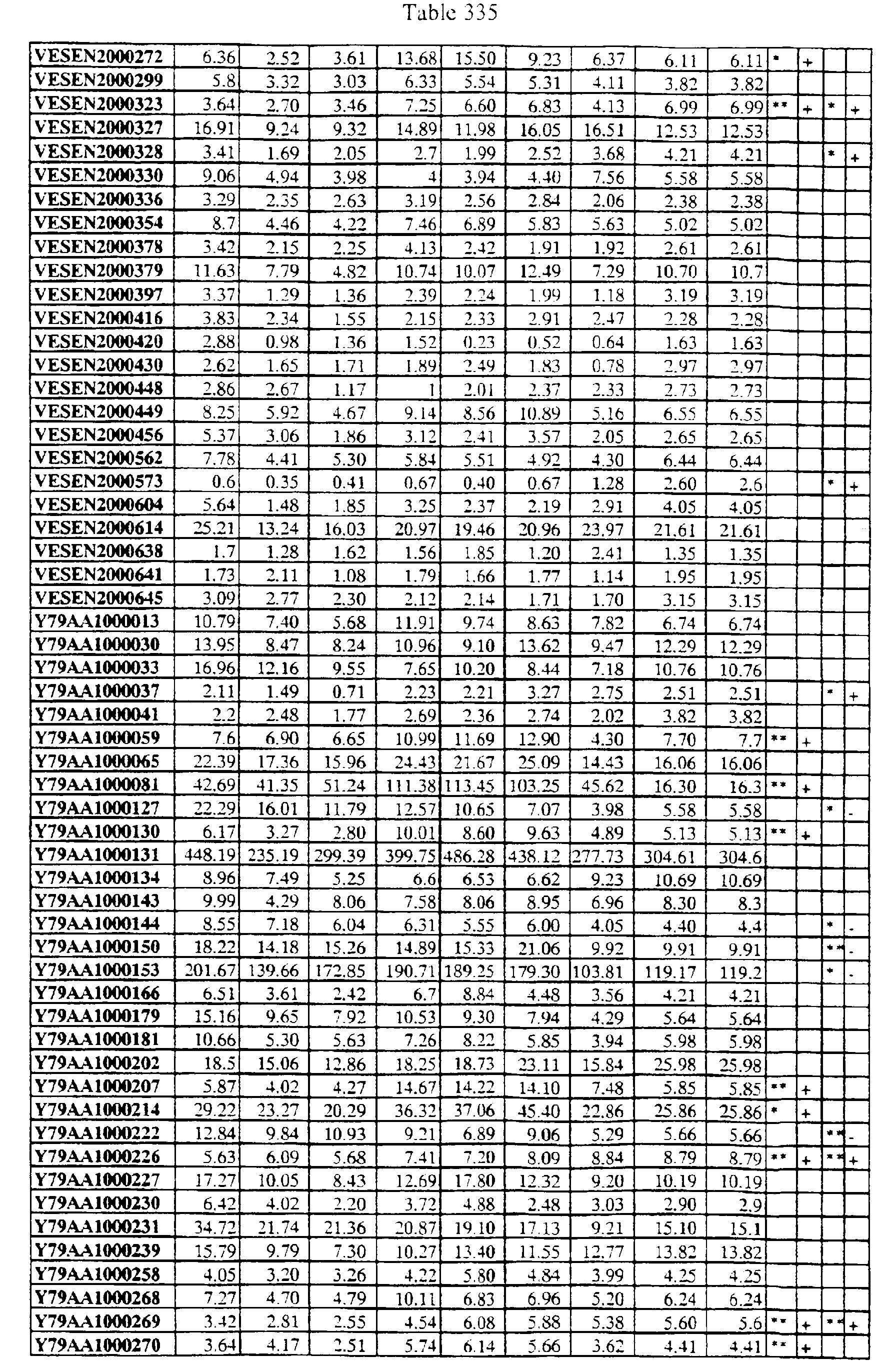

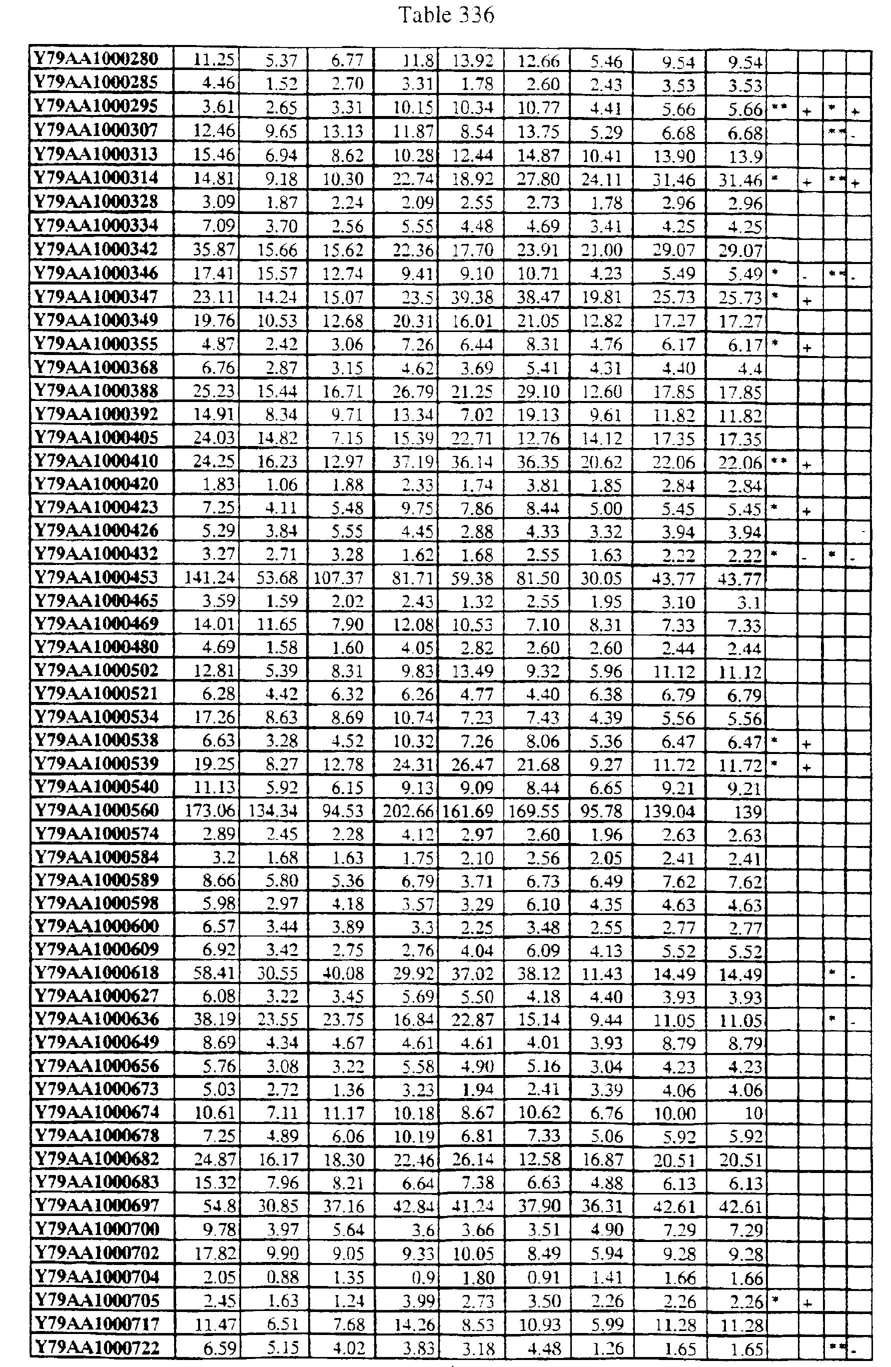

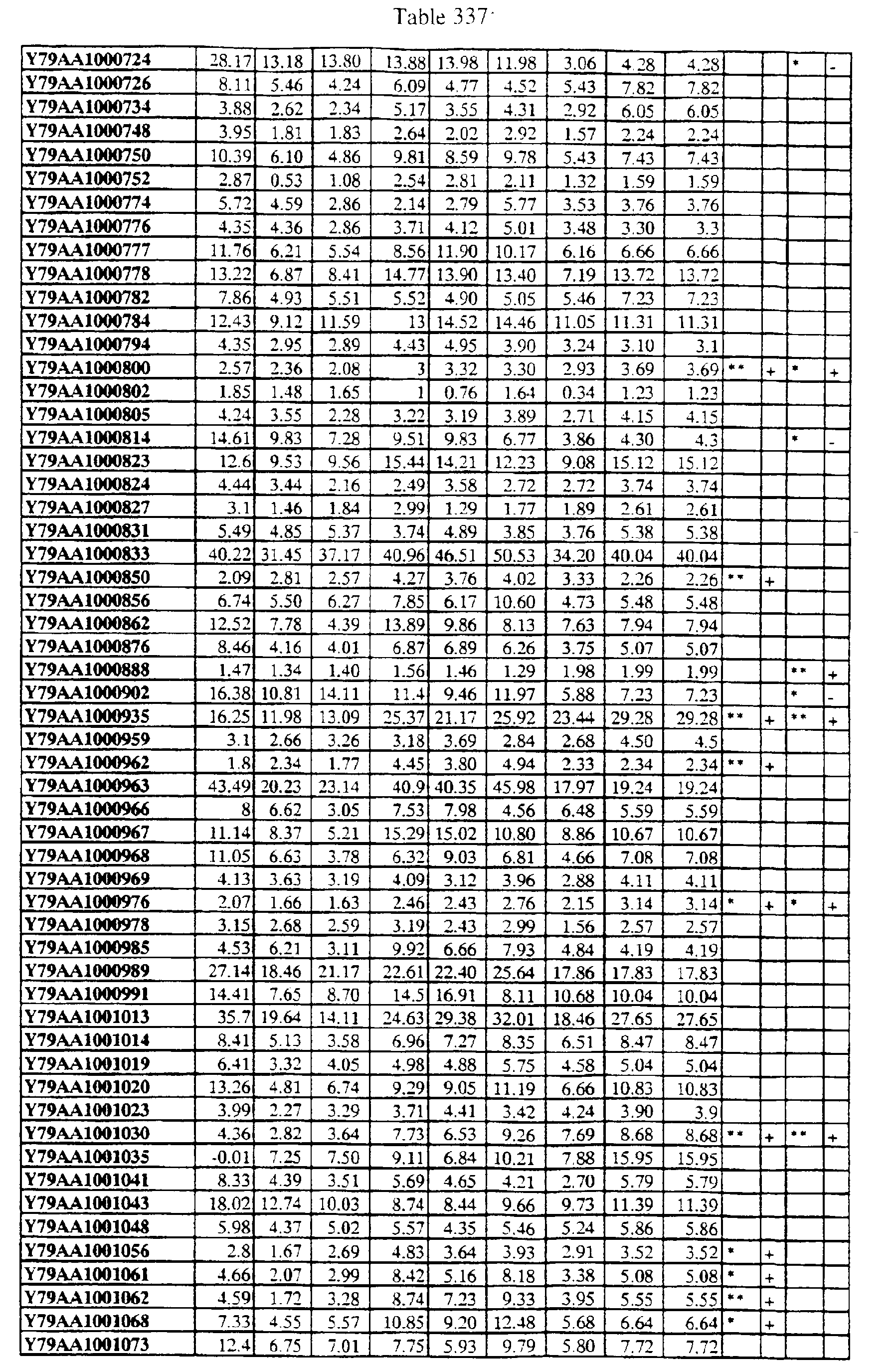

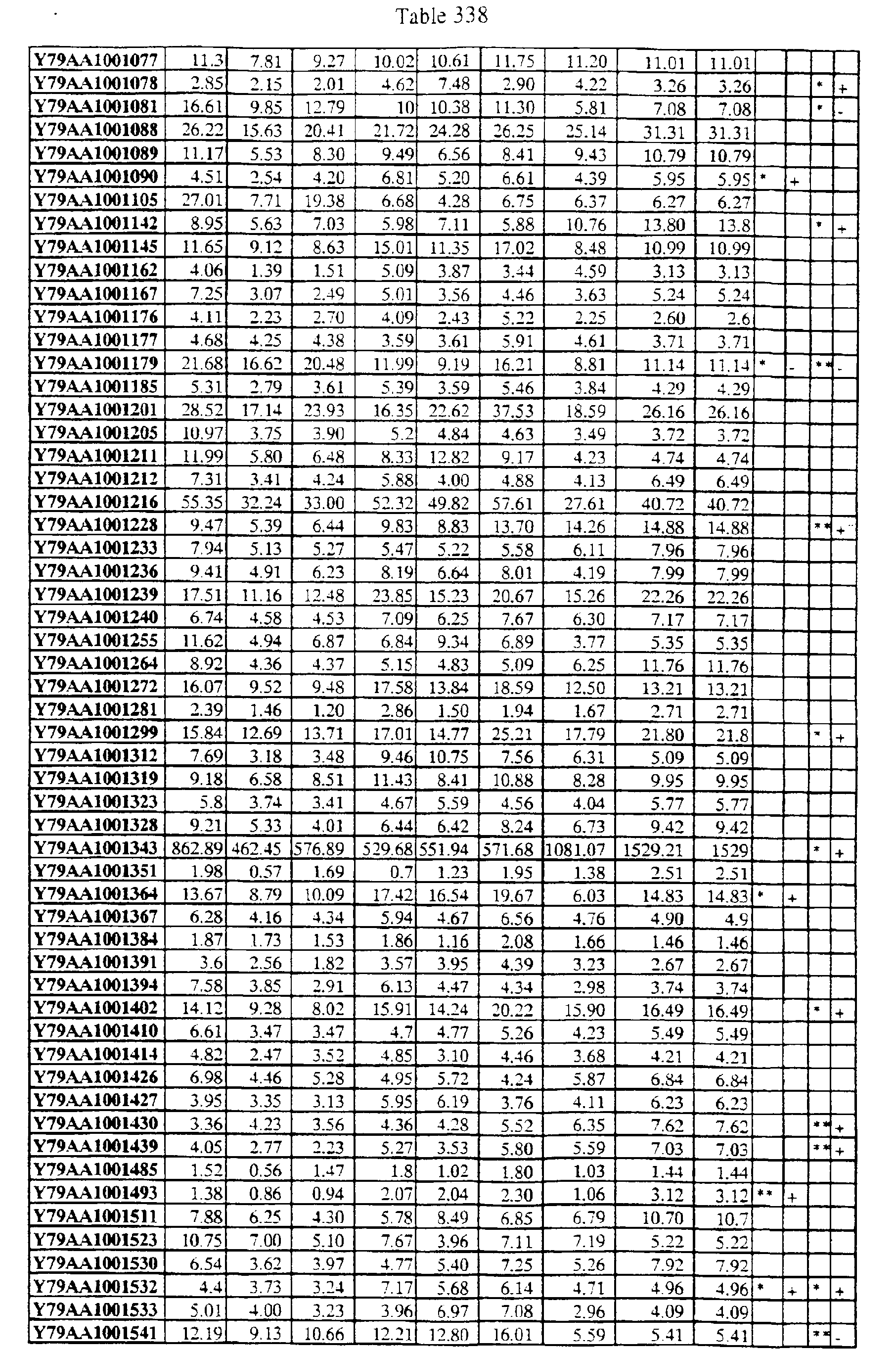

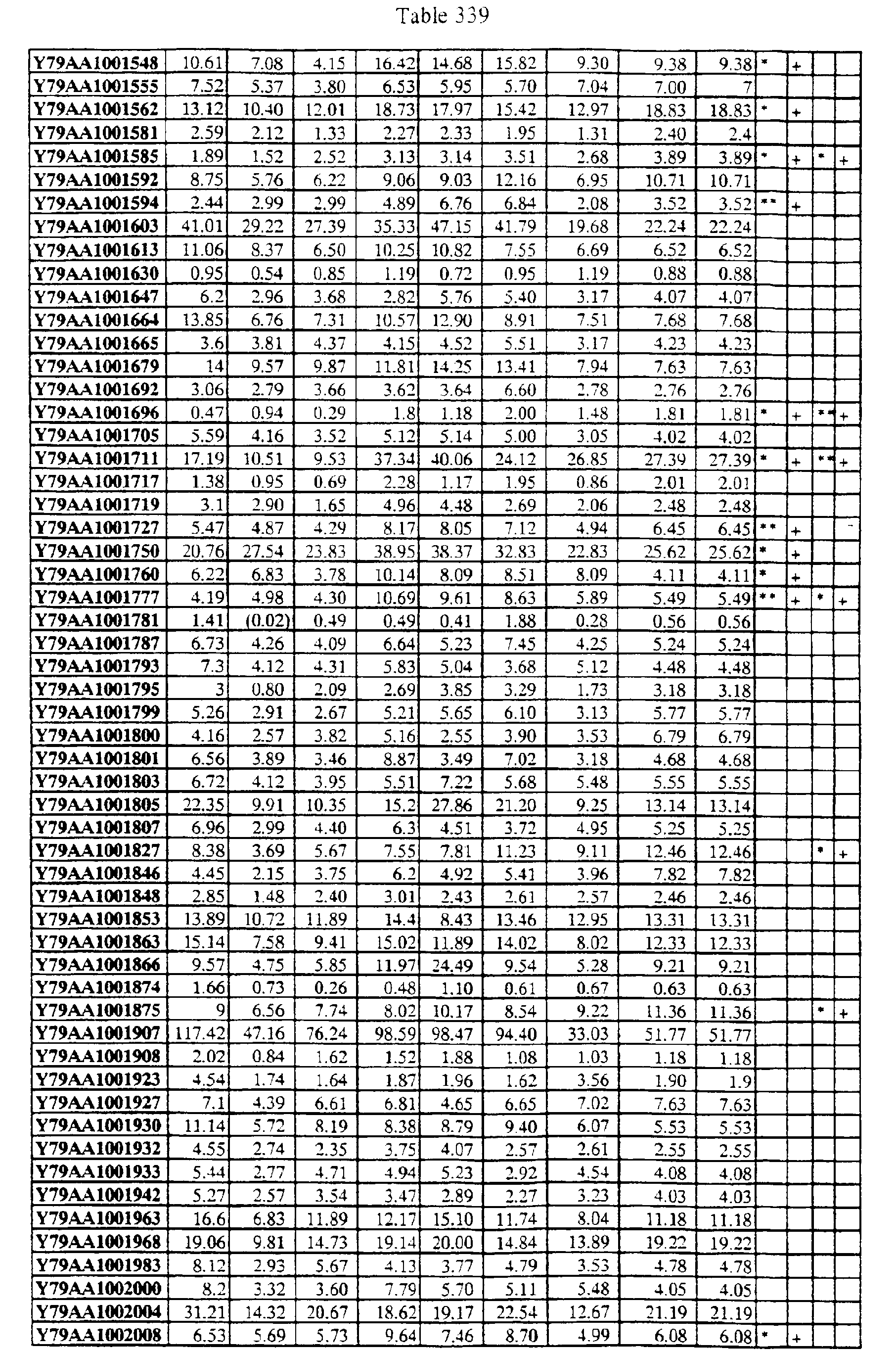

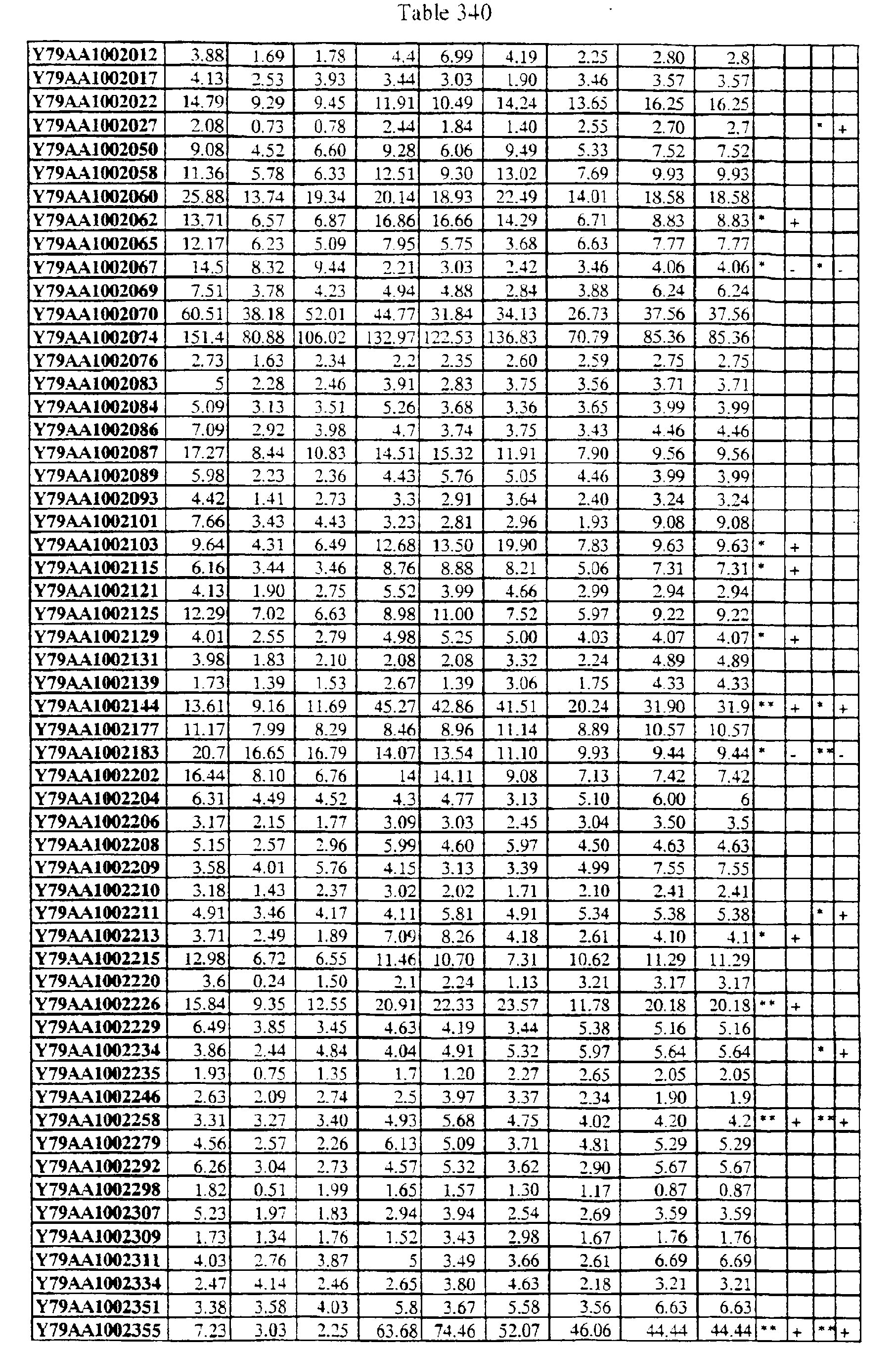

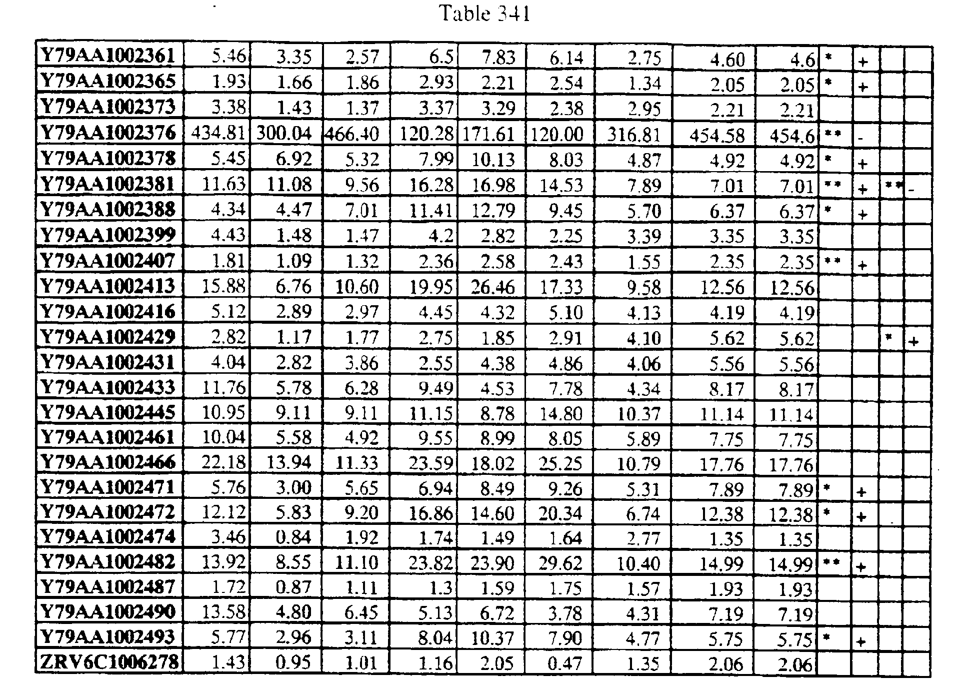

The clone numbers shown in Tables 5-341 correspond to the respective PSEC clone numbers as follows:

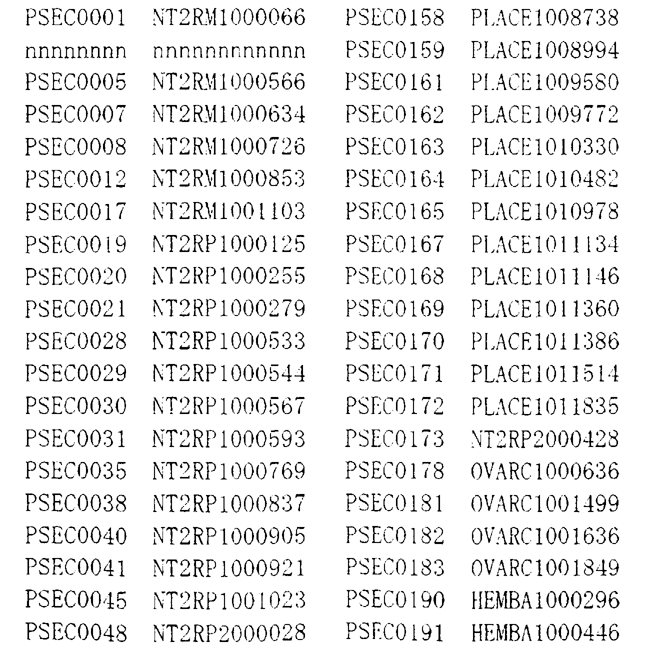

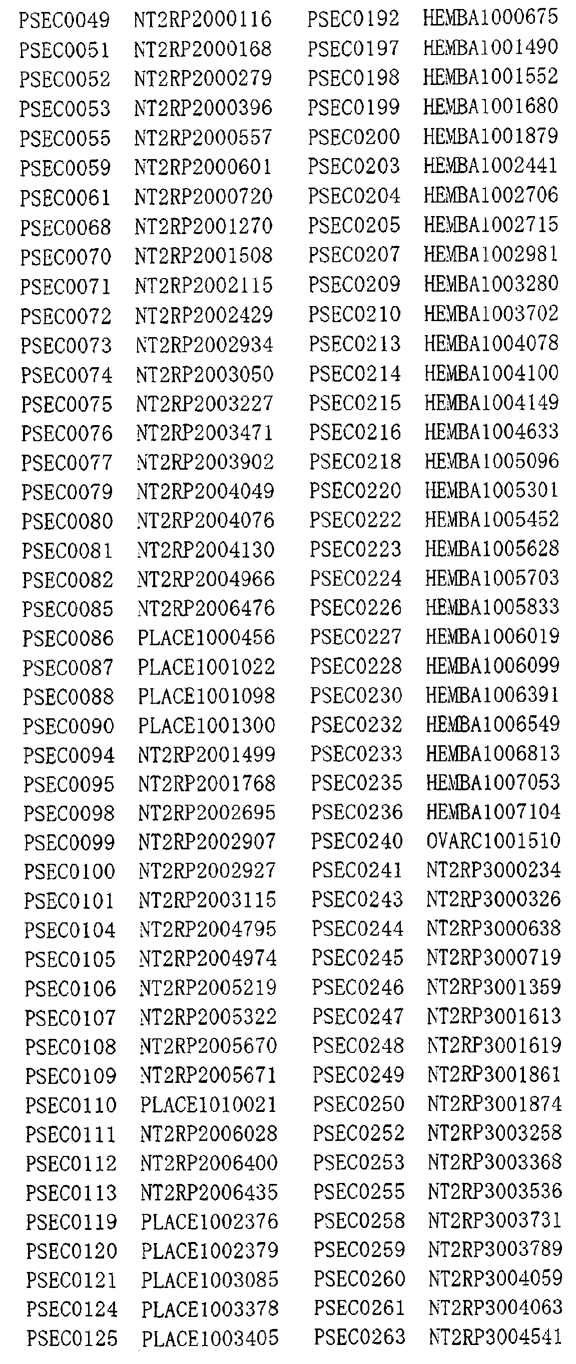

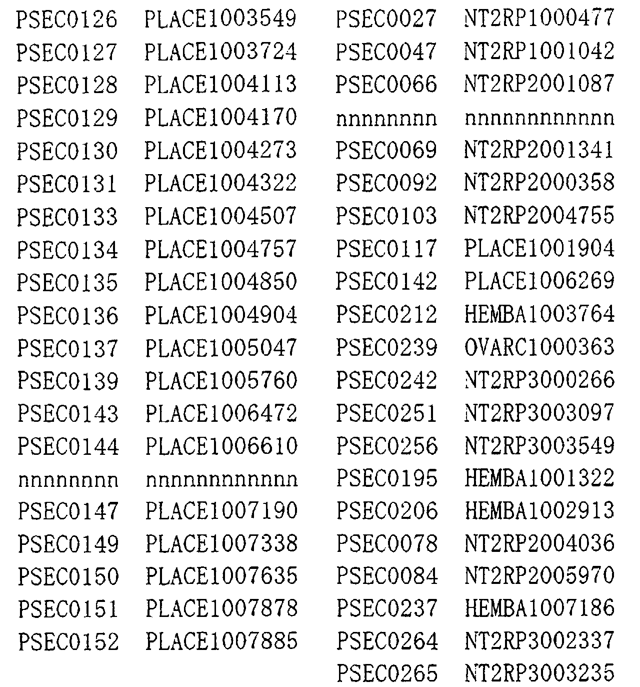

### EXAMPLE 8

### Expression frequency analysis for PSEC clones during the stages of neural differentiation of NT2 cells using RT-PCR

Total RNA was prepared from NT2 cells (NT2 Precursor Cells: Stratagene) at each stage of differentiation (at a pre-differentiation stage; at 1, 3, or 5 weeks after retinoic acid-treatment; after addition of cell-division inhibitor; or at a stage of NT2 neuron). Alterations in expression levels of PSEC clones were examined by RT-PCR. PSEC clones to be tested by RT-PCR were chosen among the clones obtained from cDNA libraries derived from NT2 cells (NT2RM1, NT2RP1, NT2RP2 and NT2RP3) or human embryo-derived tissues that were enriched with brain (HEMBA1).

The NT2 cells were treated basically according to supplier's instruction manual. "Undifferentiated NT2 cells" means NT2 cells successively cultured in an Opti-MEM I (GIBCO BRL; catalog No. 31985) containing 10%(v/v) fetal bovine serum and 1%(v/v) penicillin-streptomycin (GIBCO BRL). "NT2 cells cultured in the presence of retinoic acid for 1, 3, or 5 weeks after addition thereof" means the cells resulted from transferring undifferentiated NT2 cells into a retinoic acid-containing medium, which consists of D-MEM (GIBCO BRL; catalog No. 11965), 10%(v/v) fetal bovine serum, 1%(v/v) penicillin-streptomycin and 10 µM retinoic acid (GIBCO BRL), and the subsequent successive culture therein for 1, 3, or 5 weeks. "NT2 cells after addition of cell-division inhibitor" means NT2 cells resulted from transferring NT2 cells cultured in the presence of retinoic acid for 5 weeks into a cell-division inhibitor-containing medium, which consisted of D-MEM (GIBCO BRL; catalog No. 11965), 10%(v/v) fetal bovine serum, 1 %(v/v) penicillin-streptomycin, 10 µM retinoic acid, 10 µM FudR (5-fluoro-2'-deoxyuridine: GIBCO BRL), 10 µM Urd (Uridine: GIBCO BRL) and 1 µM araC (Cytosine β-D-Arabinofuranoside: GIBCO BRL), and the subsequence successive culture for 2 weeks. "NT2 neuron" means NT2 cells resulted from successively culturing NT2 cells in the presence of cell-division inhibitor for about 10 days. The NT2 neurons were harvested by treating mildly with trypsin. Total RNA was prepared from each of the cells harvested by treating with trypsin. The preparation was performed by using an Rneasy Mini kit (QIAGEN) according to the attached protocol.

RT-PCR was performed by using 50 ng total RNA in a reaction and SUPERSCRIPT™ ONE-STEP™ RT-PCR System (GIBCO BRL). Although the reaction condition used were substantially the same as described in the protocol attached to SUPERSCRIPT™ ONE-STEP™ RT-PCR System, the annealing temperature and the number of cycles were altered in this experiment.

To analyze the PCR products obtained by the amplification, samples of each reaction solution were subjected to agarose gel electrophoresis. The bands derived from the PCR products were detected using FMBIO II Multi-View (Hitachi Ltd.). First, 90 PSEC clones obtained from cDNA libraries derived from NT2 cell (NT2RM1, NT2RP1, NT2RP2 and NT2RP3) or human embryo-derived tissues enriched with brain (HEMBA1) were analyzed for the change in the expression levels thereof between undifferentiated NT2 cells and NT2 cells cultured in the presence of cell-division inhibitor added. Many clones showed no marked change in the expression levels thereof or no specific bands in PCR assay, and therefore such clones were not analyzed further.

As for the PSEC clones whose expression levels were expected to change in the above analysis, the temporal expression at a pre-differentiation stage, 1, 3, or 5 weeks after retinoic acid-treatment and, further, the expression in NT2 neurons were examined. The result showed that the clones, PSEC0005, PSEC0048, PSEC0059, PSEC0200 and PSEC0232, exhibited the differences in the amount of the PCR products (Figures 4 and 5). On the other hand, no marked difference in the expression level was observed in each of the clones, PSEC0001, PSEC0029, PSEC0031, PSEC0078, PSEC0099, PSEC0173, PSEC0197, PSEC0198, PSEC0213, PSEC0124 and PSEC0260.

Figure 6 shows changes in intensities of the bands generated by RT-PCR under particular reaction conditions (the conditions are indicated in the figure). RT-PCR was carried out by using a pair of primers shown in SEQ ID NOs: 355 and 356 for clone PSEC0005; primers shown in SEQ ID NOs: 357 and 358 for clone PSEC0048; primers shown in SEQ ID NOs: 359 and 360 for clone PSEC0059; primers shown in SEQ ID NOs: 361 and 362 for clone PSEC0200; primers shown in SEQ ID NOs: 363 and 364 for clone PSEC0232; (the annealing temperature and the number of cycles used in PCR are as indicated in Figures 4 and 5). A pair of primers shown in SEQ ID NOs: 365 and 366 were used for the amplification of the β-actin gene as a control. A pair of primers shown in SEQ ID NOs: 368 and 369 were used to perform RT-PCR for the gene encoding prostaglandin D2 synthase (Neuroscience, 69, 967-975 (1995); Eur. J. Neurosci. 9, 1566-1573 (1997)), which has been known to be expressed strongly (the annealing temperature and the number of cycles used in PCR are as indicated in Figures 4 and 5). The primers were designed based on a cDNA sequence (SEQ ID NO: 367) that was isolated from a cDNA library derived from NT2 cells and shared 94% or more residues both at the nucleotide level and at the amino acid level with the prostaglandin D2 synthase clone registered under an accession number M61900 in GenBank database.

The expression level of PSEC0232 was highly elevated depending on the degree of neural differentiation of NT2 cell. Therefore, it is clear that the gene is closely associated with neural differentiation. Although PSEC0048 and PSEC0200 exhibited only weak expression in NT2 neurons, the expression levels thereof were observed to be elevated during the course of differentiation. These genes were also considered to be associated with neural differentiation. Similarly, PSEC0059 exhibited no expression in NT2 neurons but the expression level thereof was observed to be markedly elevated during the course of differentiation. This gene was also judged to be associated with neural differentiation. The expression level of PSEC0005 was markedly decreased during the course of differentiation. Although opposite to those of other genes, the pattern of expression showed that this gene was also involved in neural differentiation.

In order to find genes associated with neural differentiation, a similar experiment was performed by using hybridization with high-density DNA filter in the same manner as described in Example 7. In this experiment, a similar result to that shown above was obtained for 3 clones (PSEC0048: NT2RP2000028, PSEC0059: NT2RP2000601 and PSEC0200: HEMBA1001879). However, the results obtained by RT-PCR method were not necessarily consistent with those obtained by the hybridization method. The possible reason for the inconsistency is that specific bands were not generated in the RT-PCR experiments or that the signal intensity detected in the hybridization experiments was too low to assess the change in the expression level of the gene.

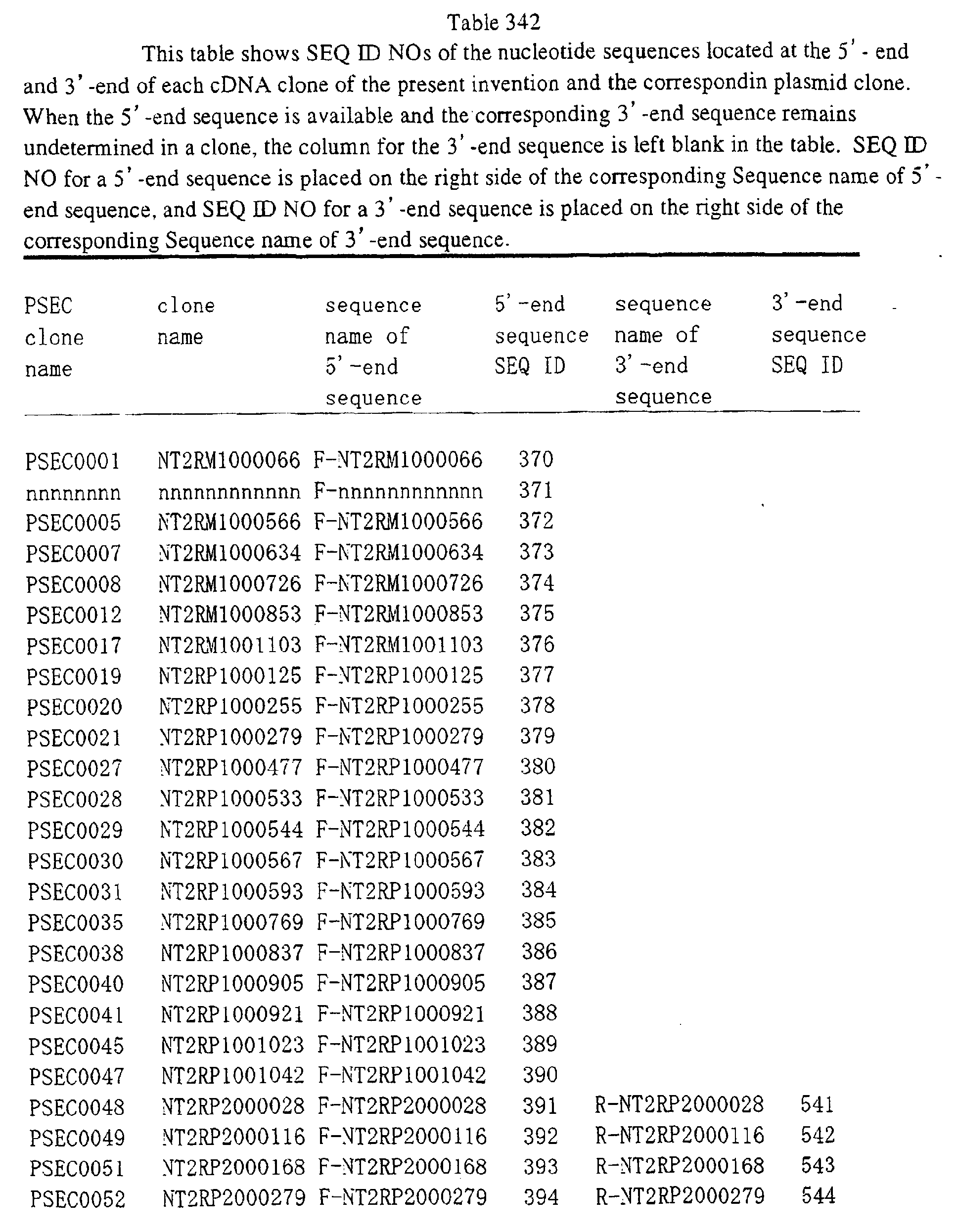

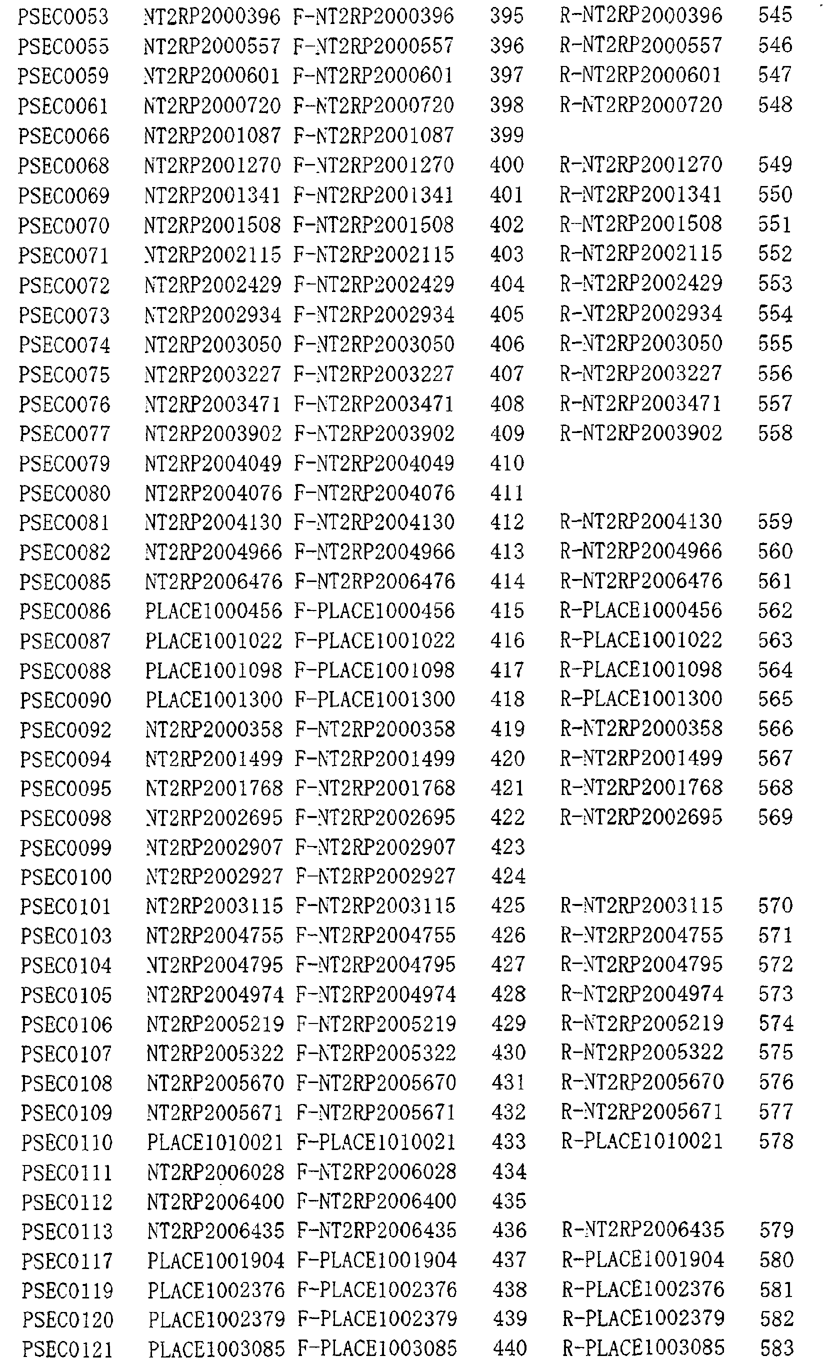

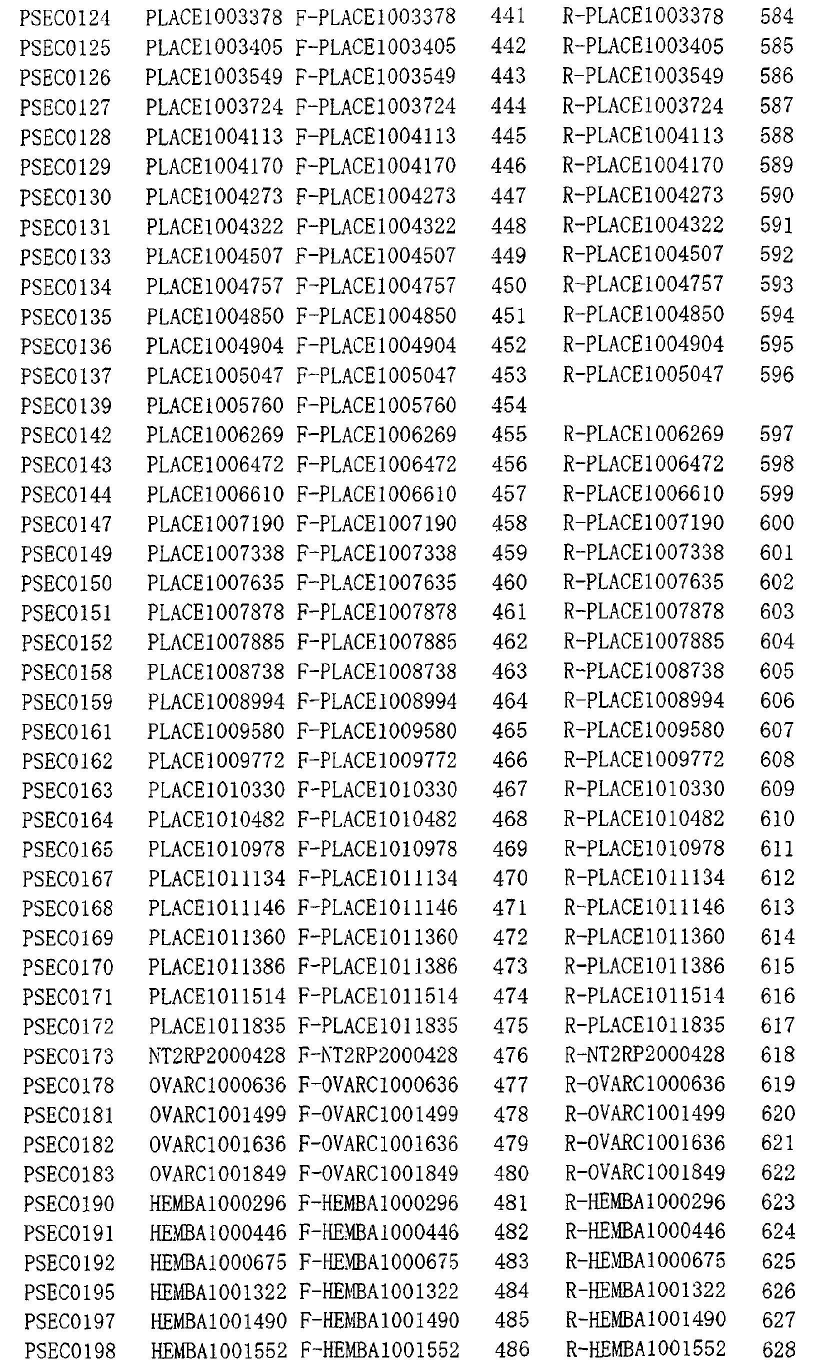

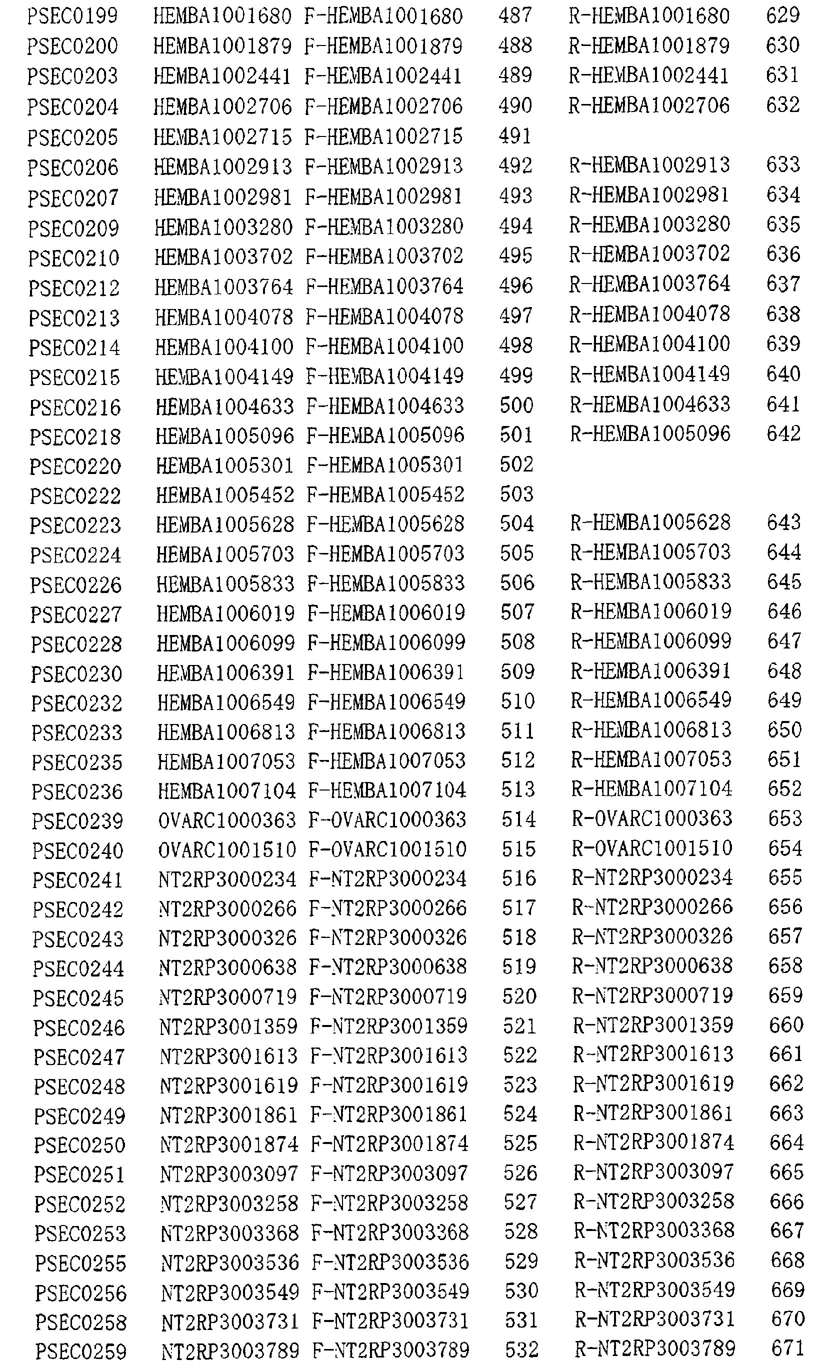

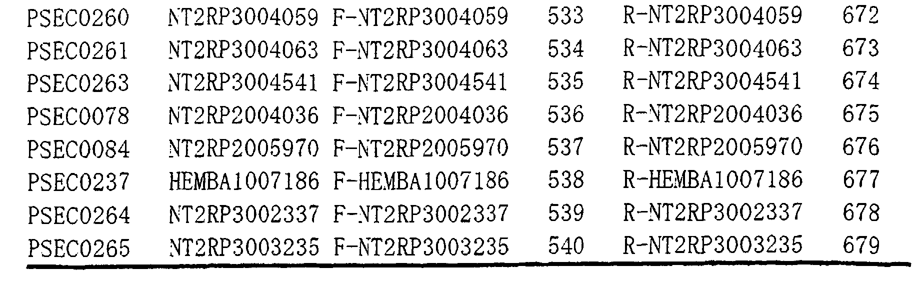

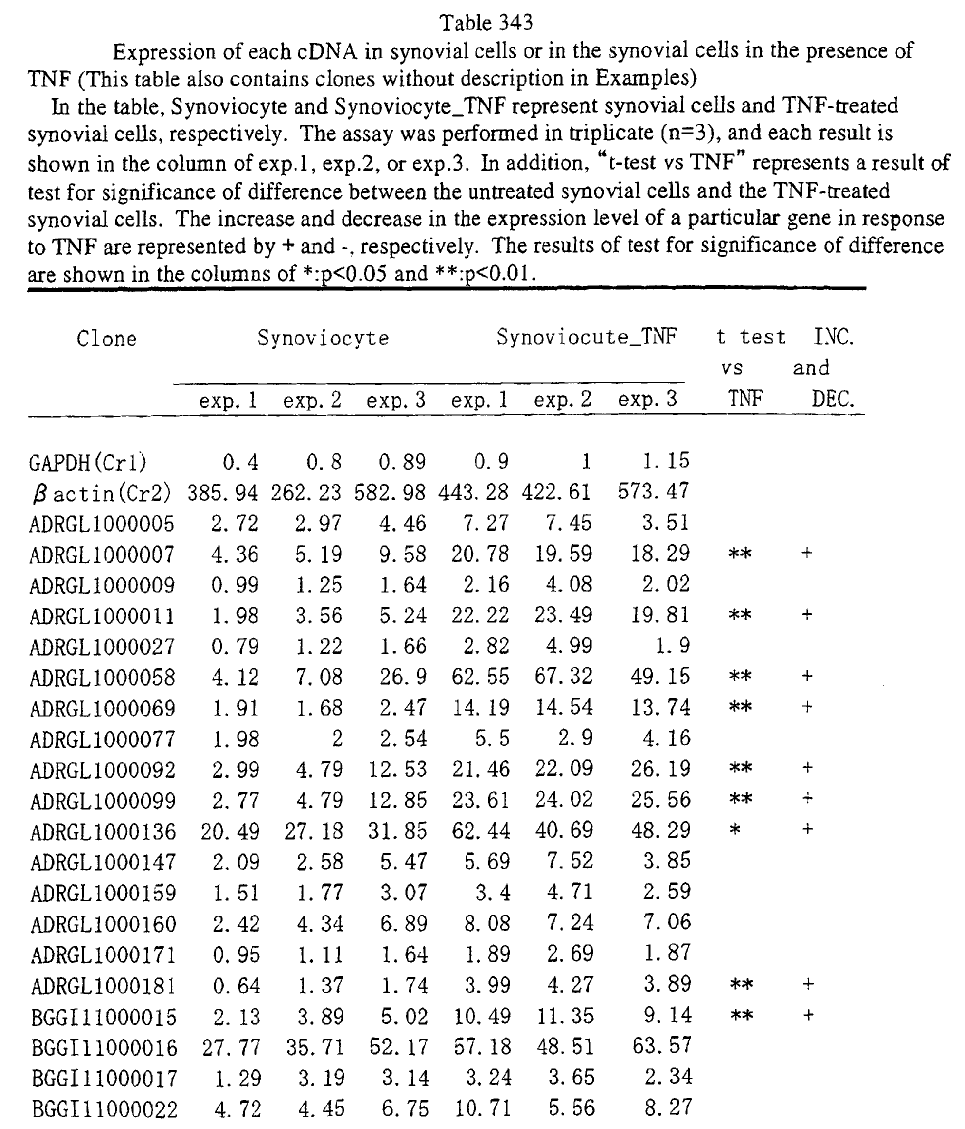

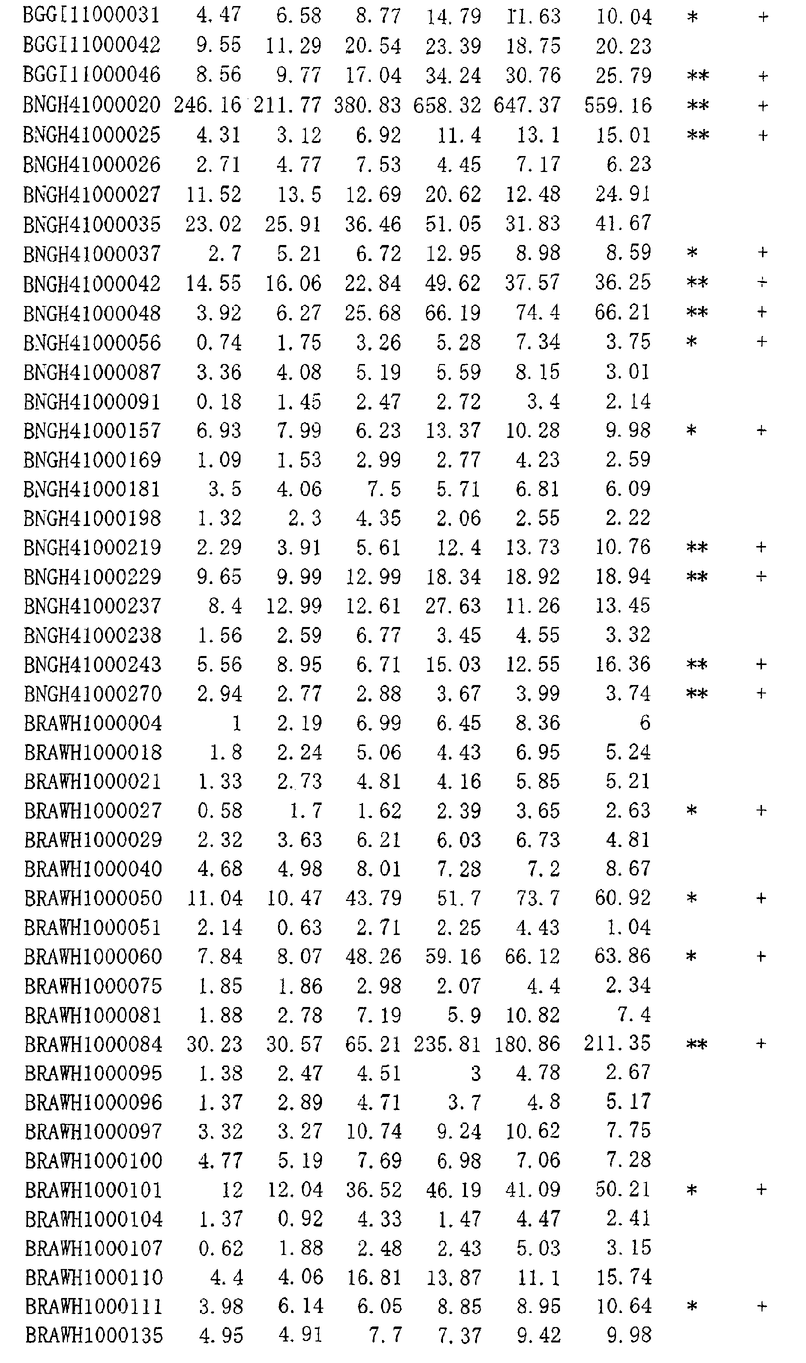

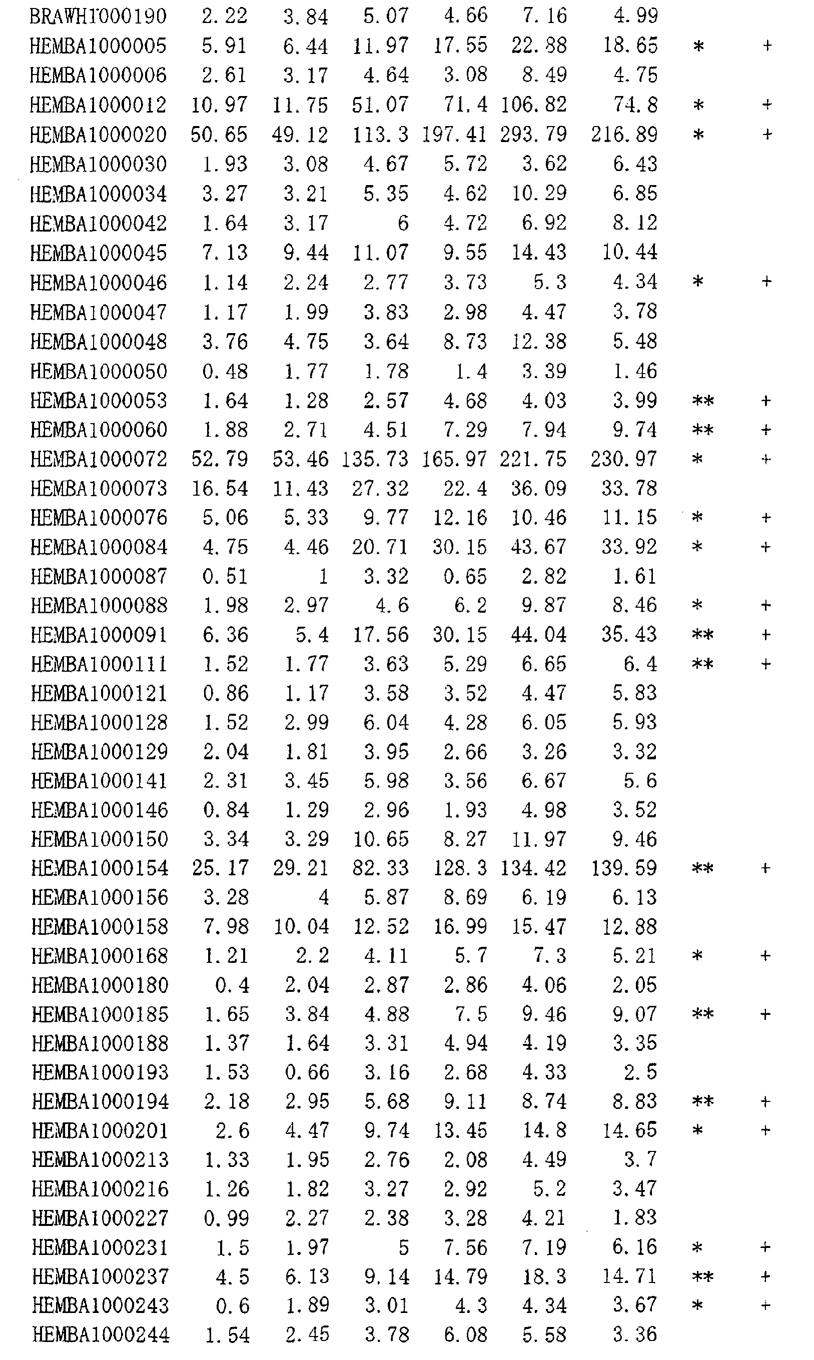

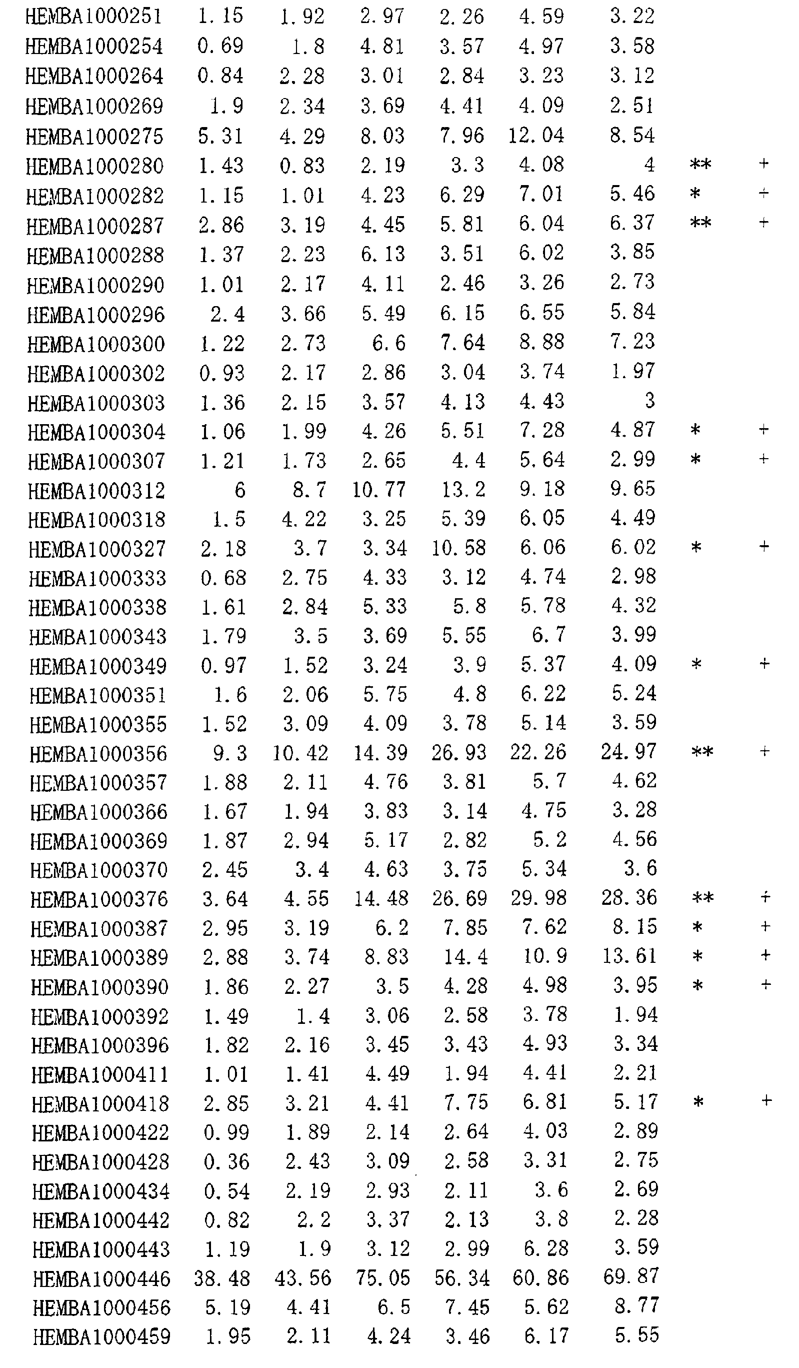

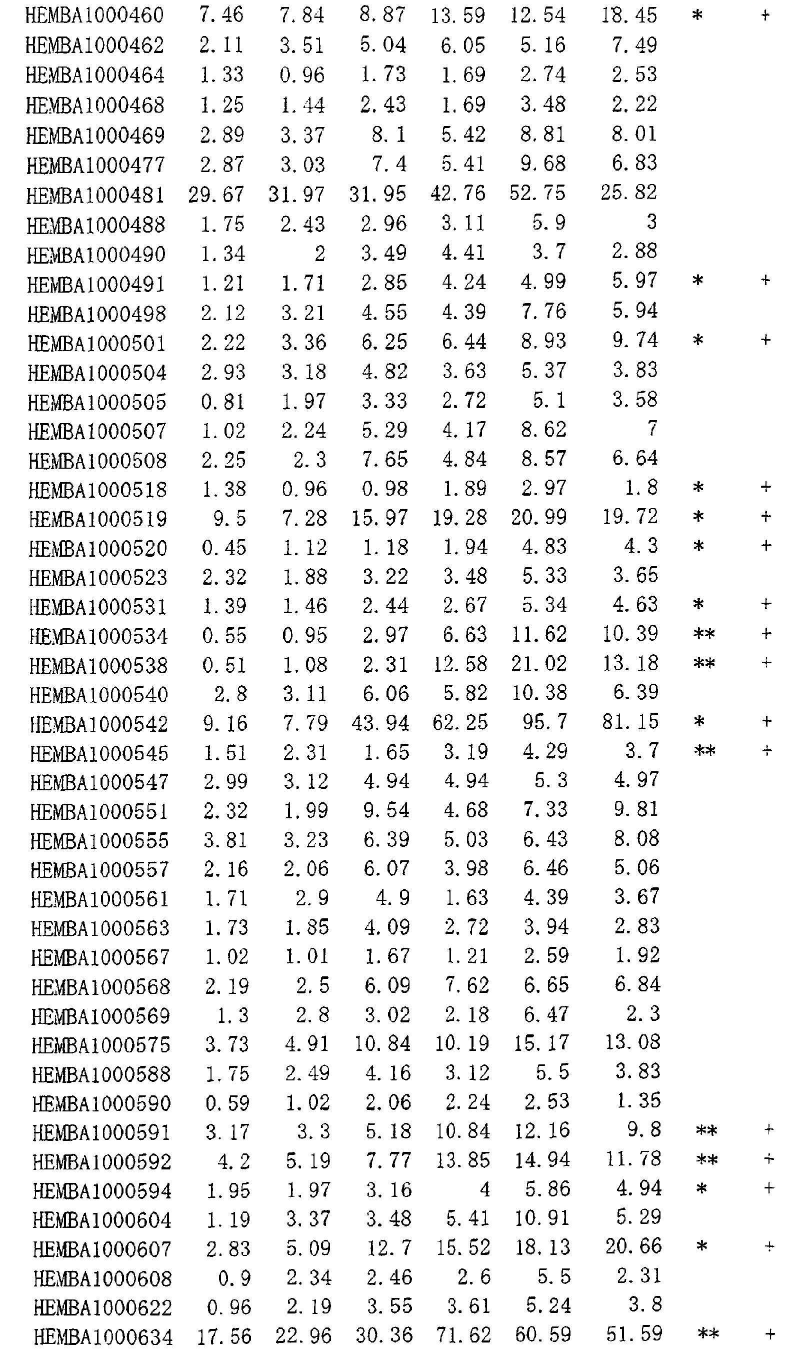

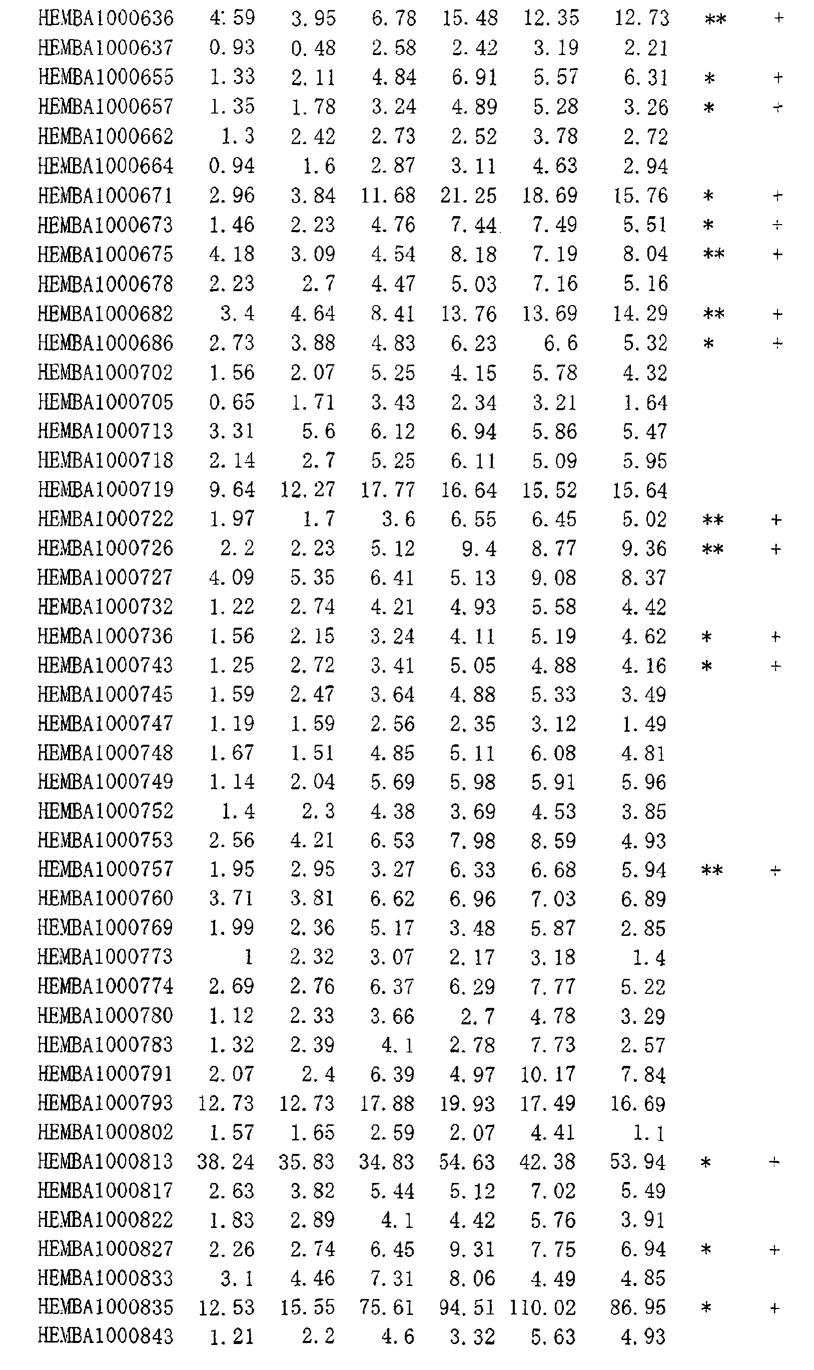

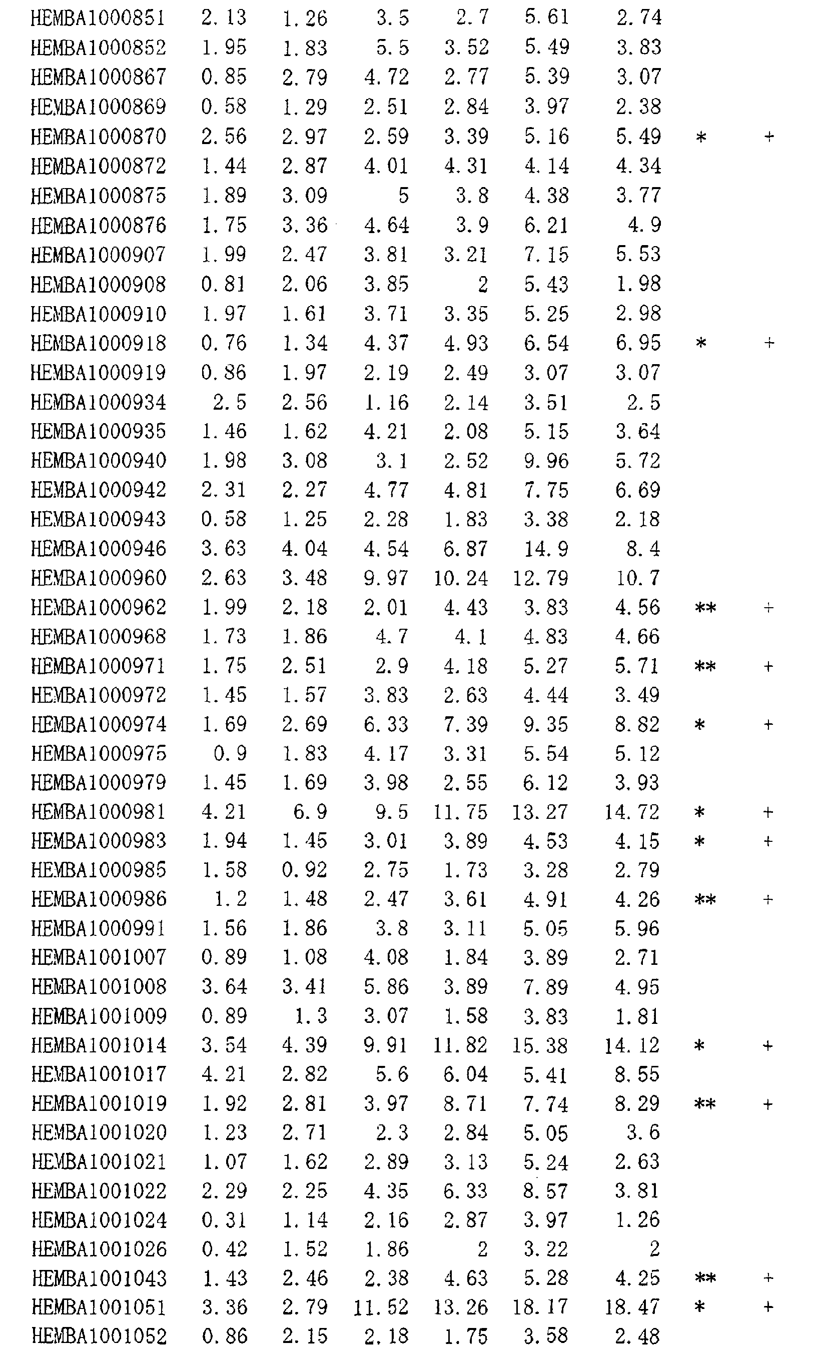

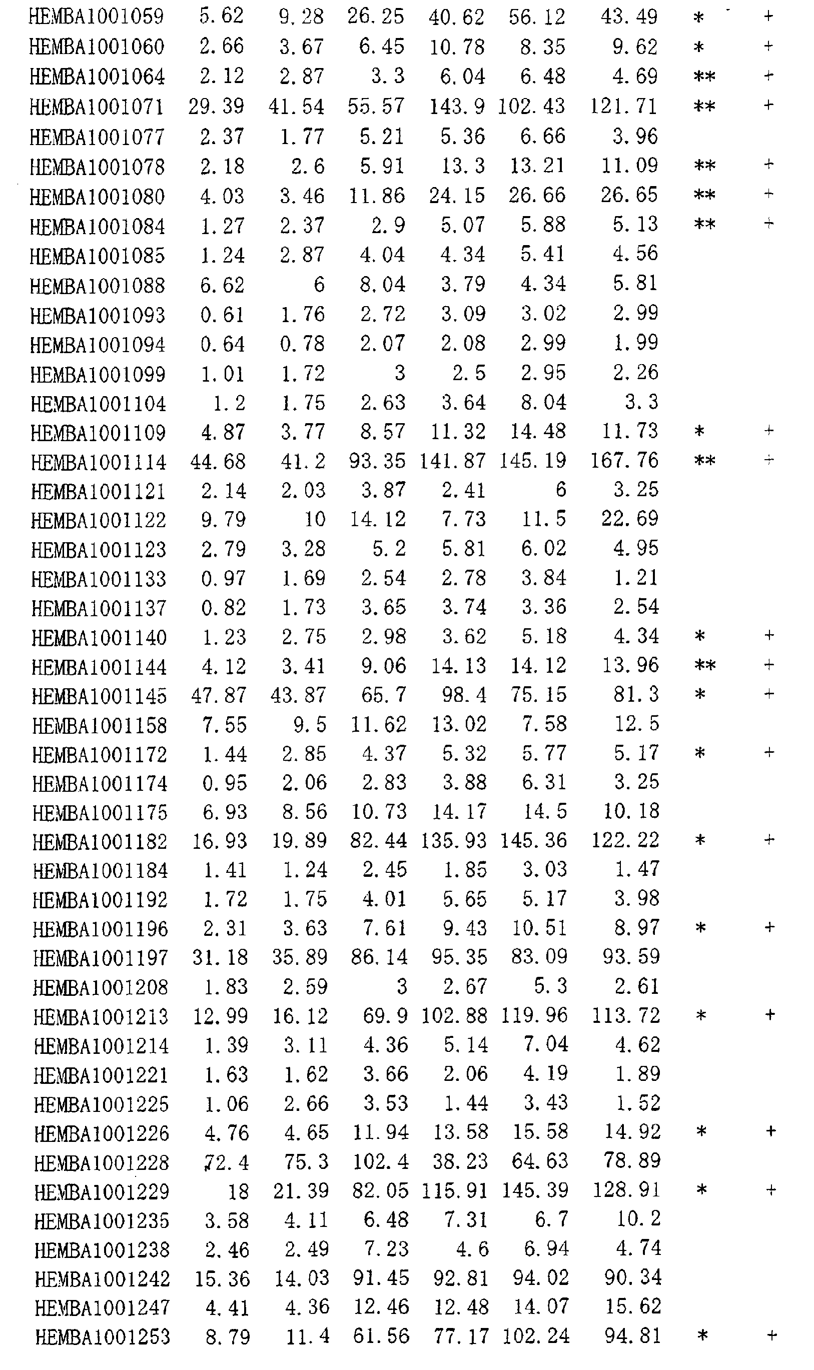

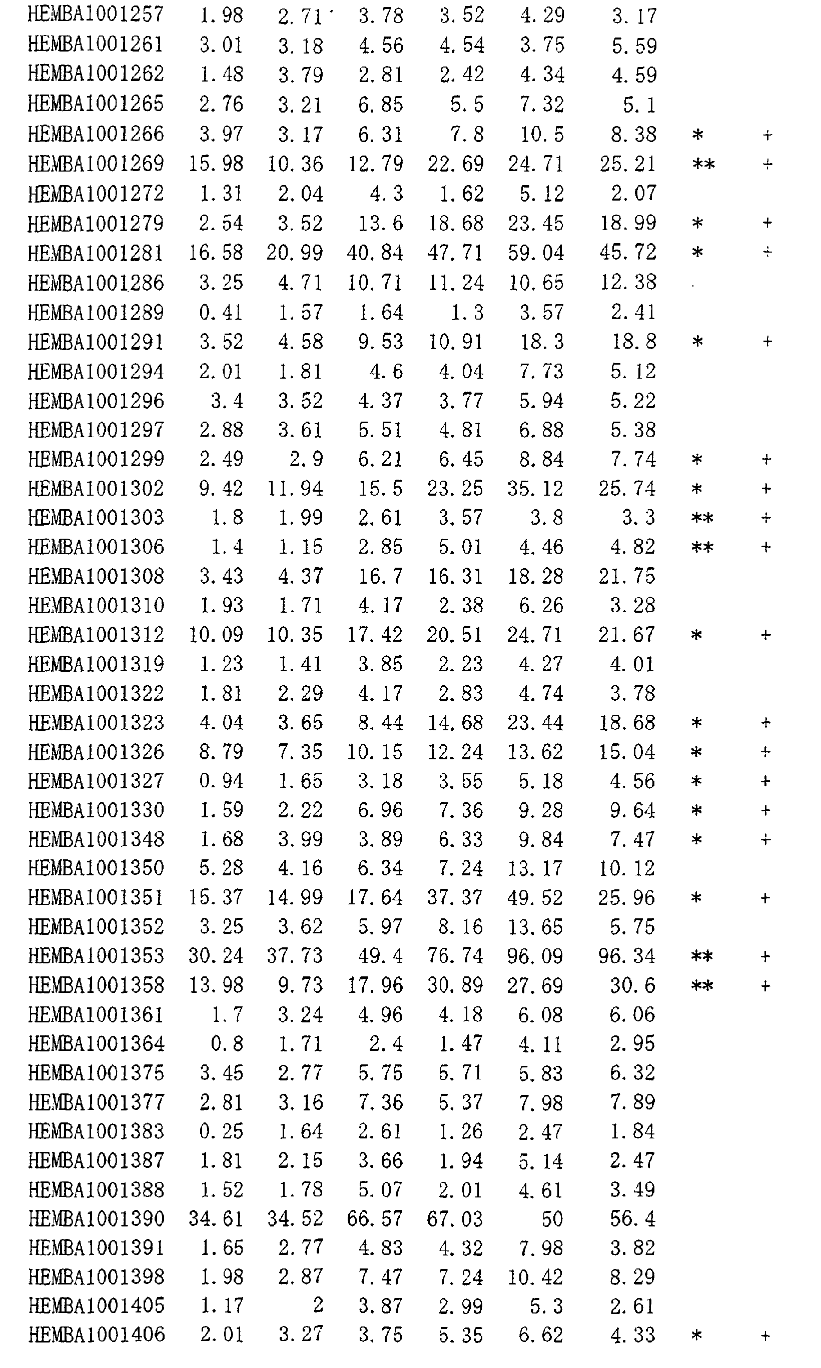

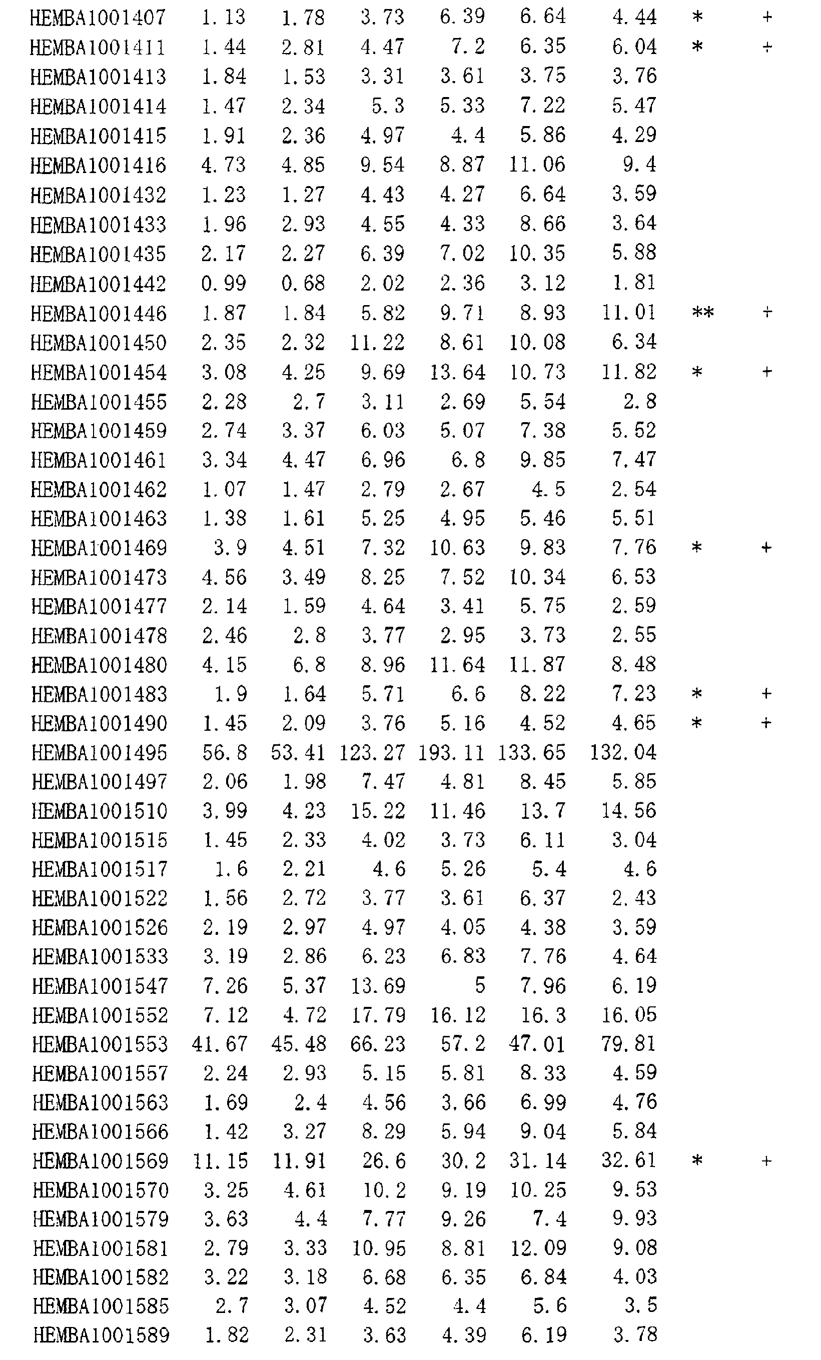

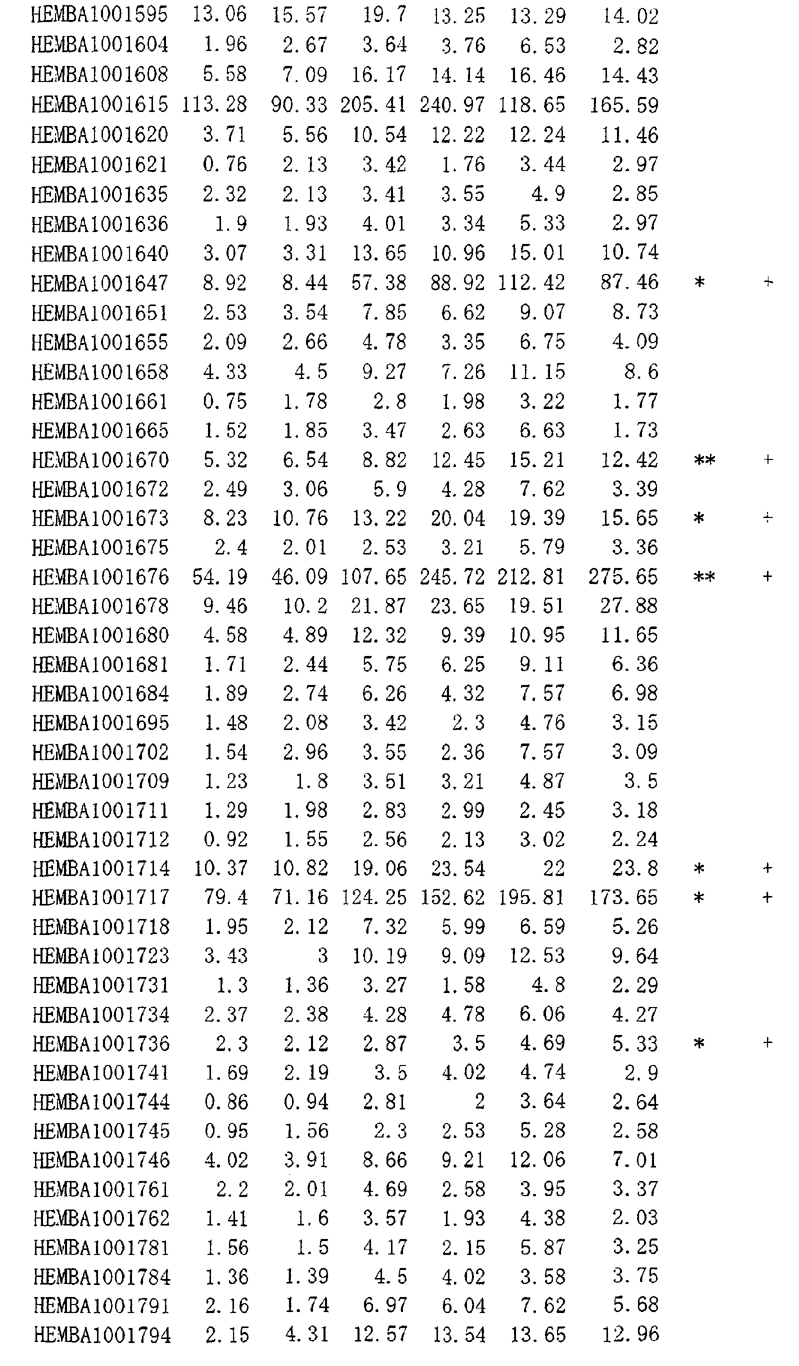

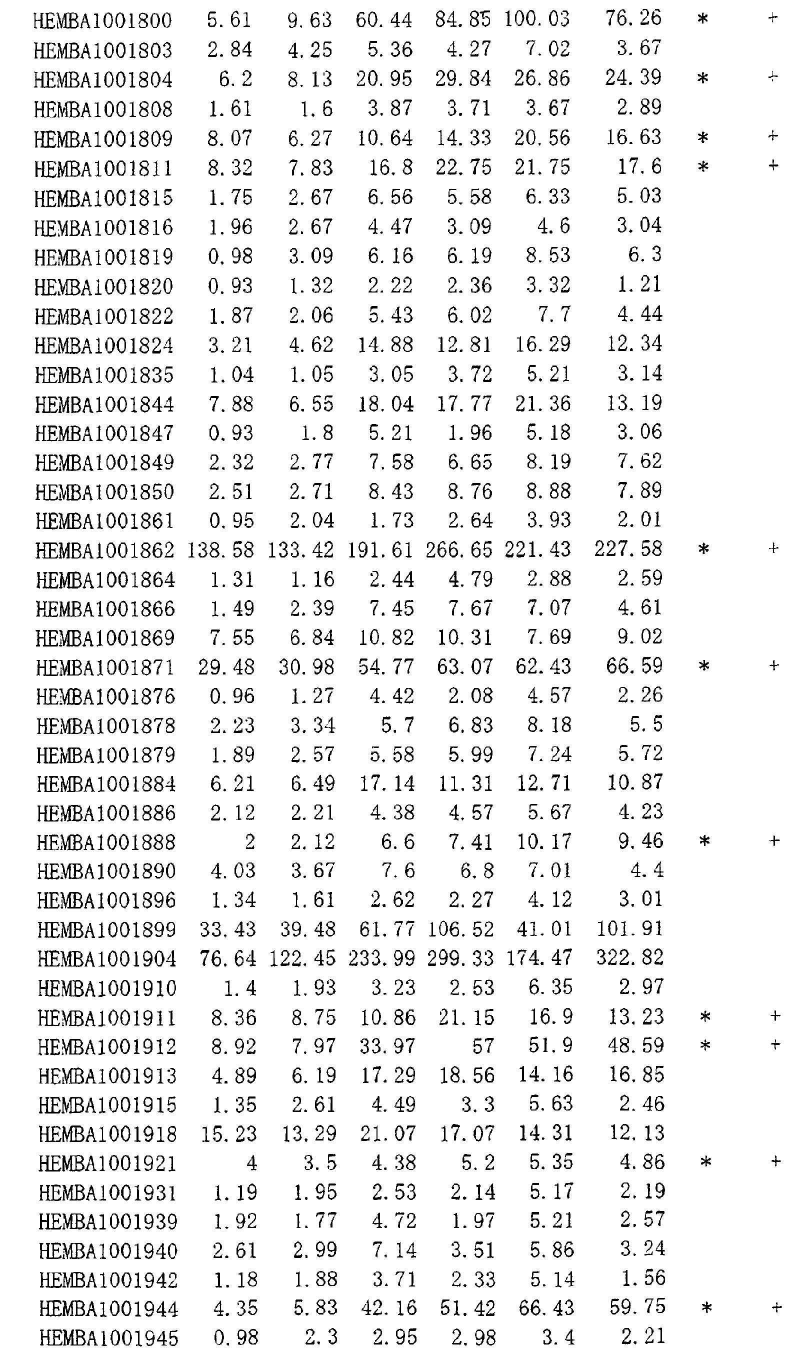

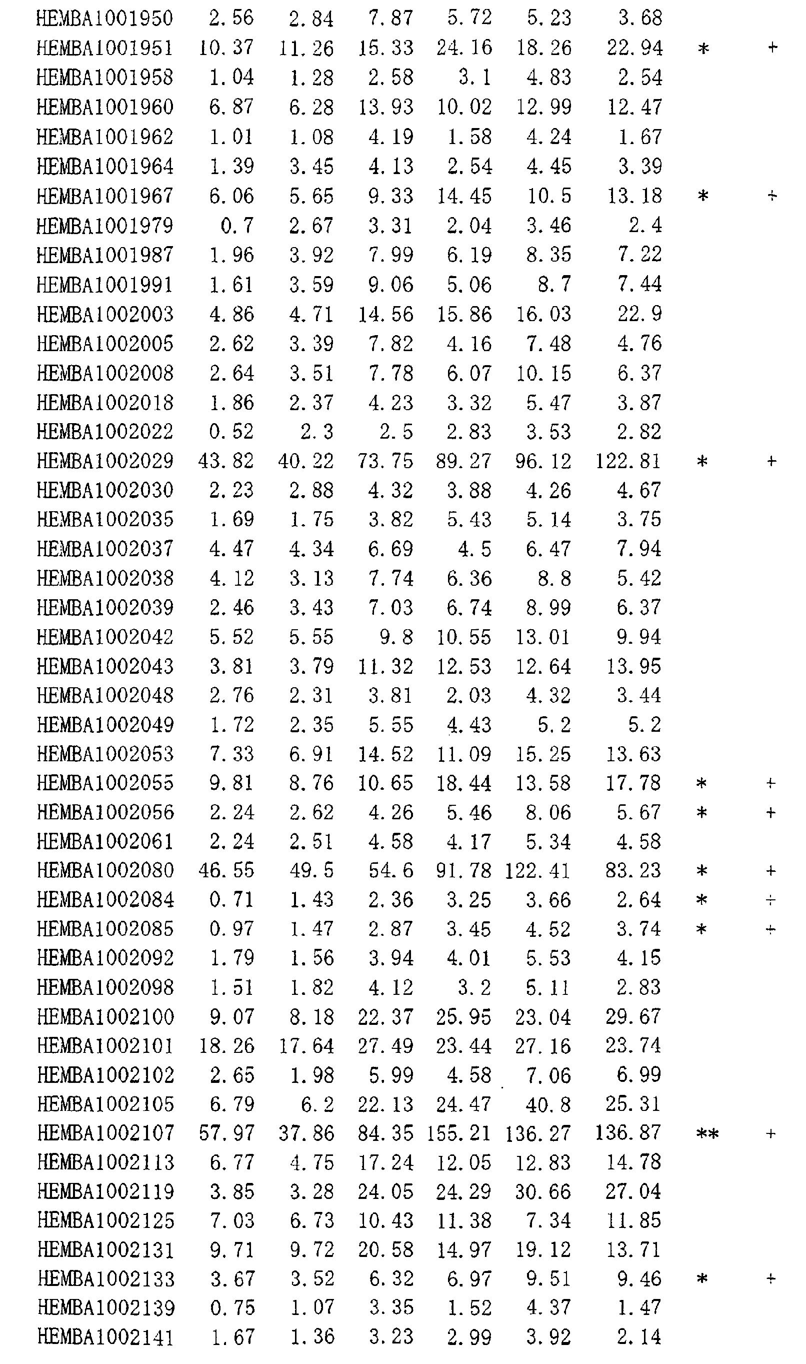

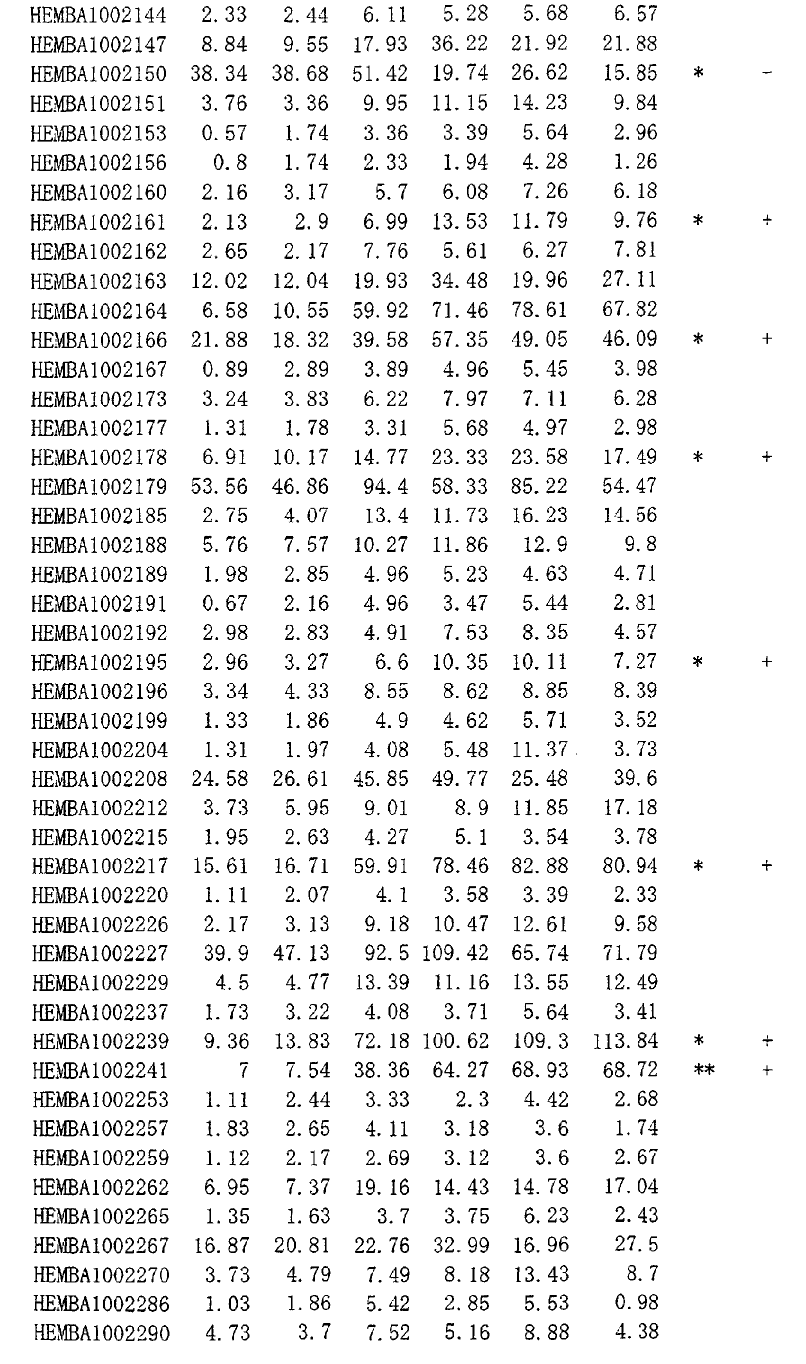

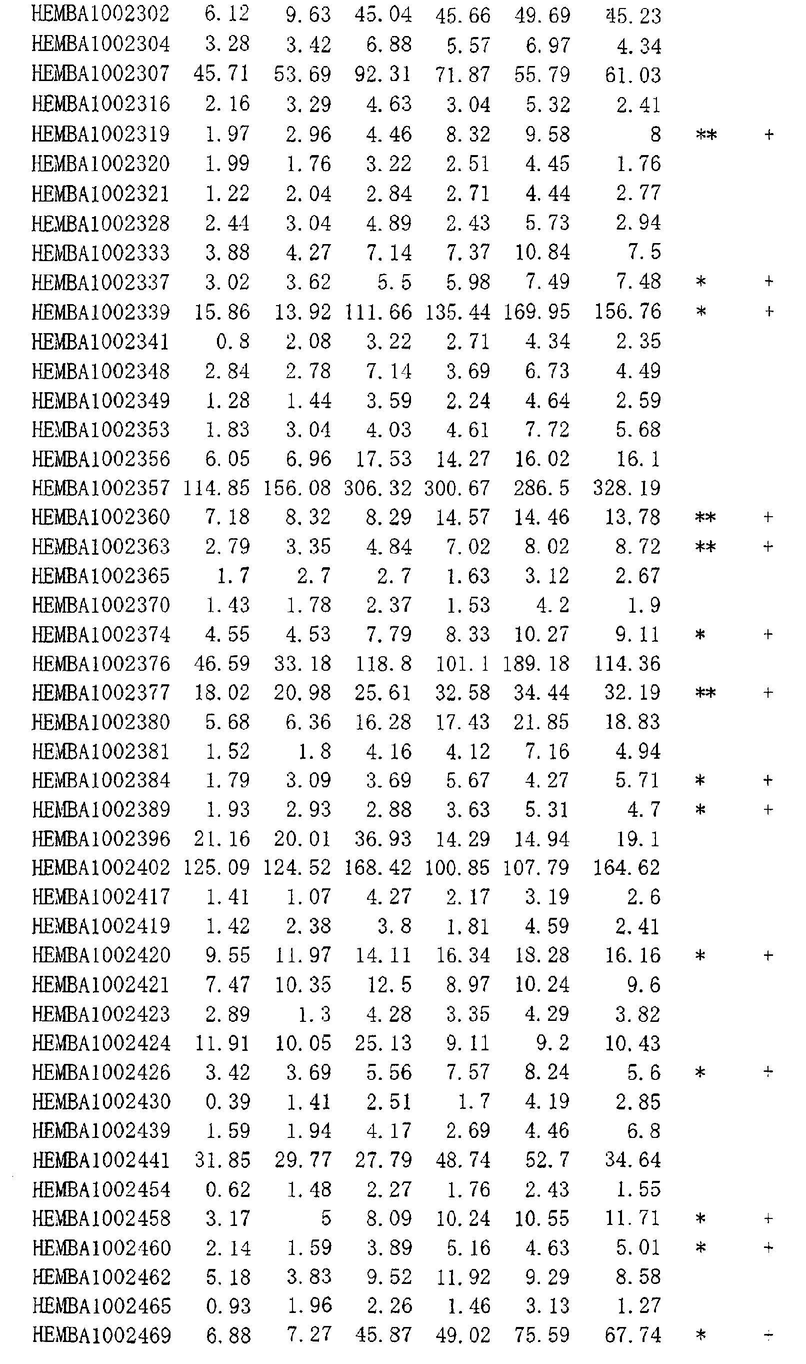

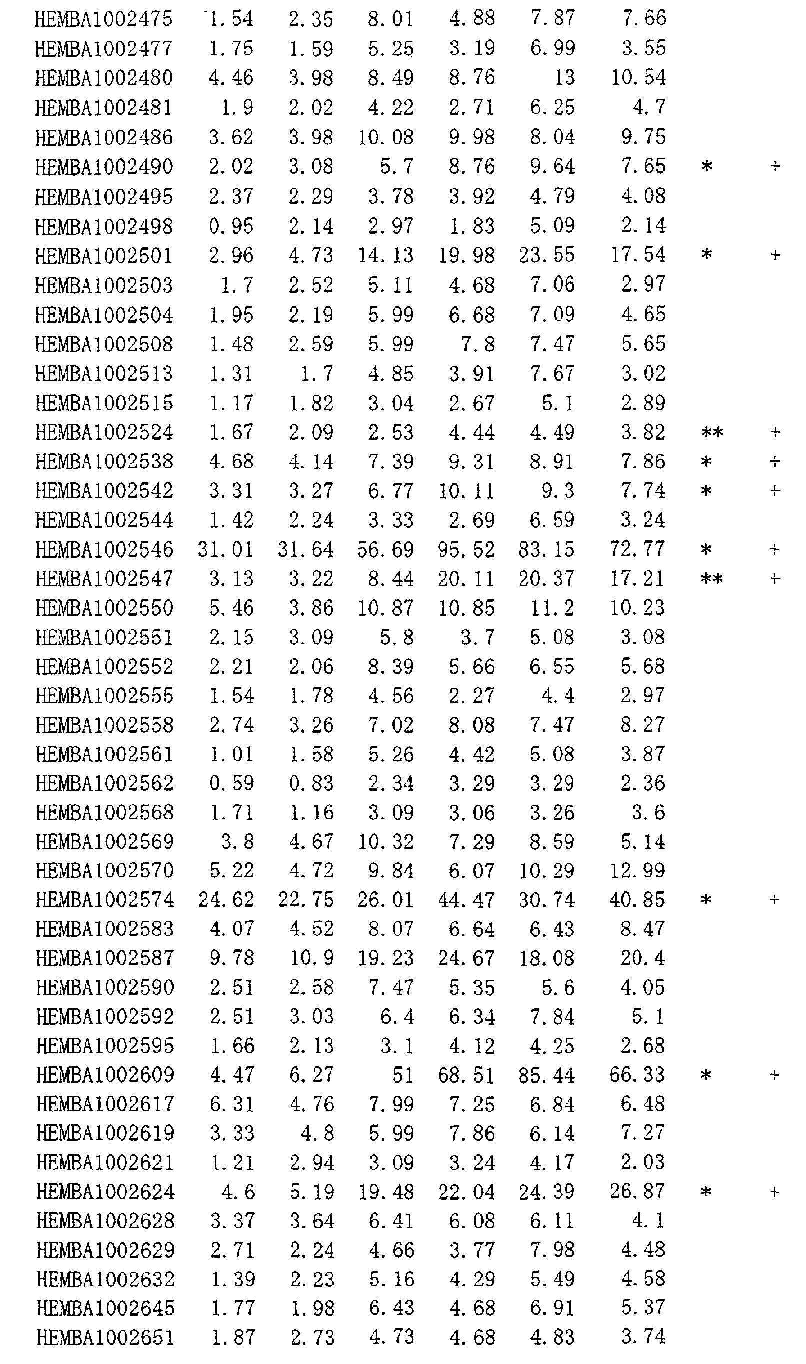

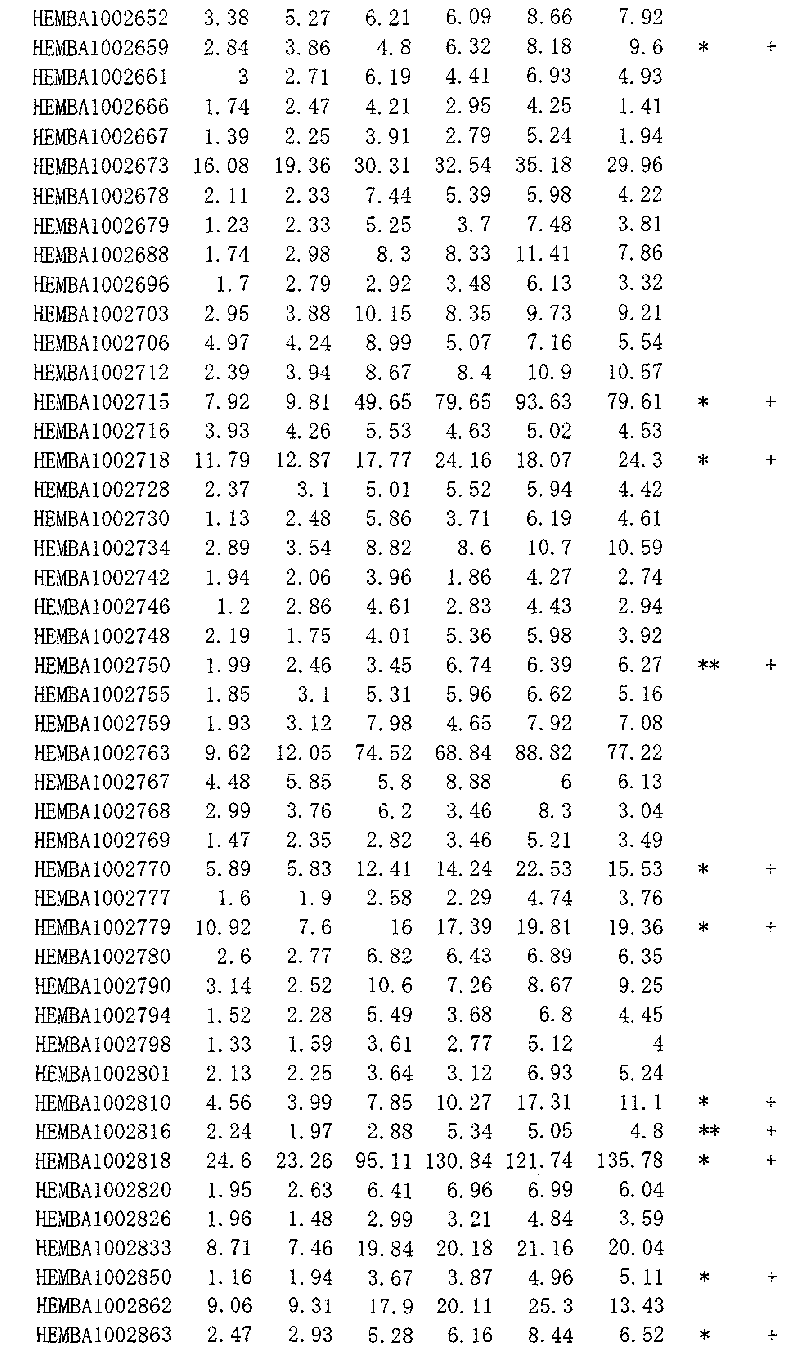

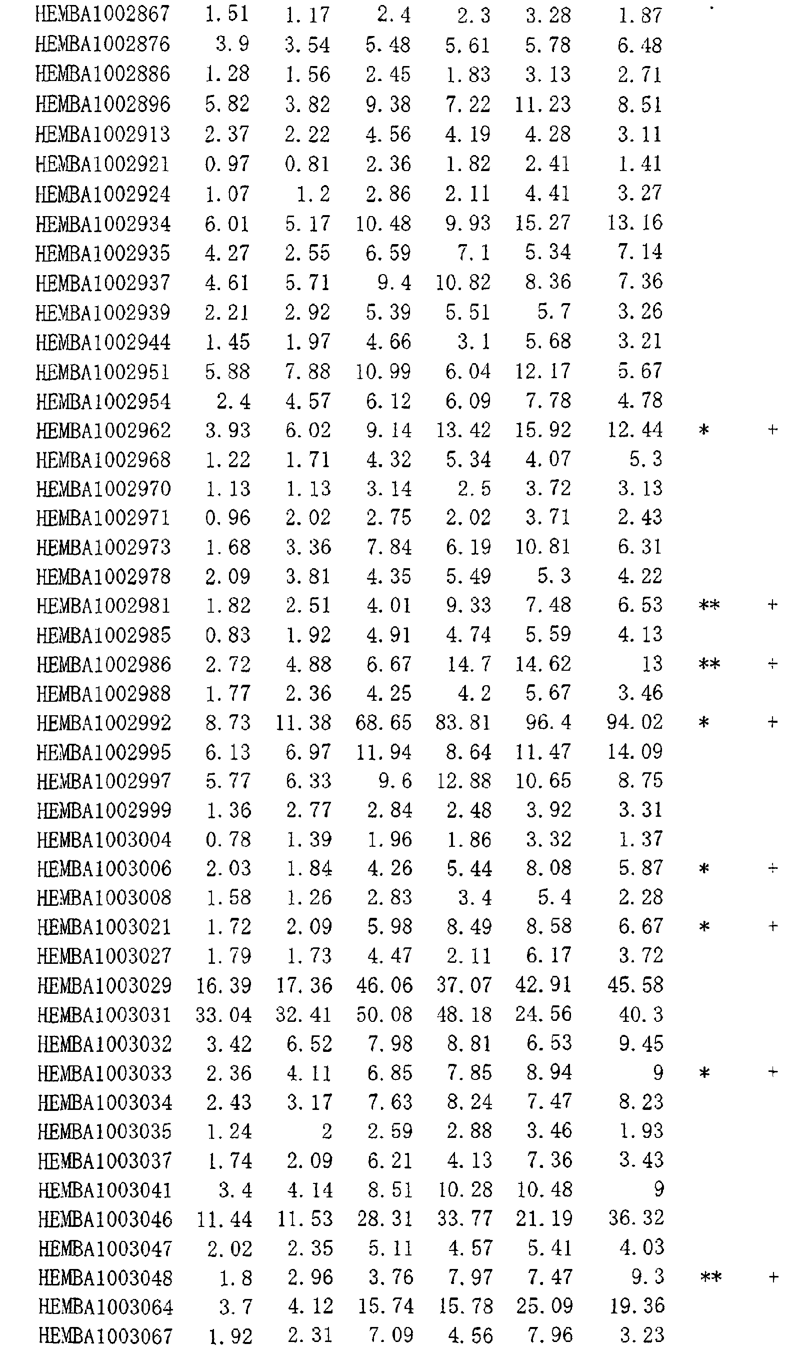

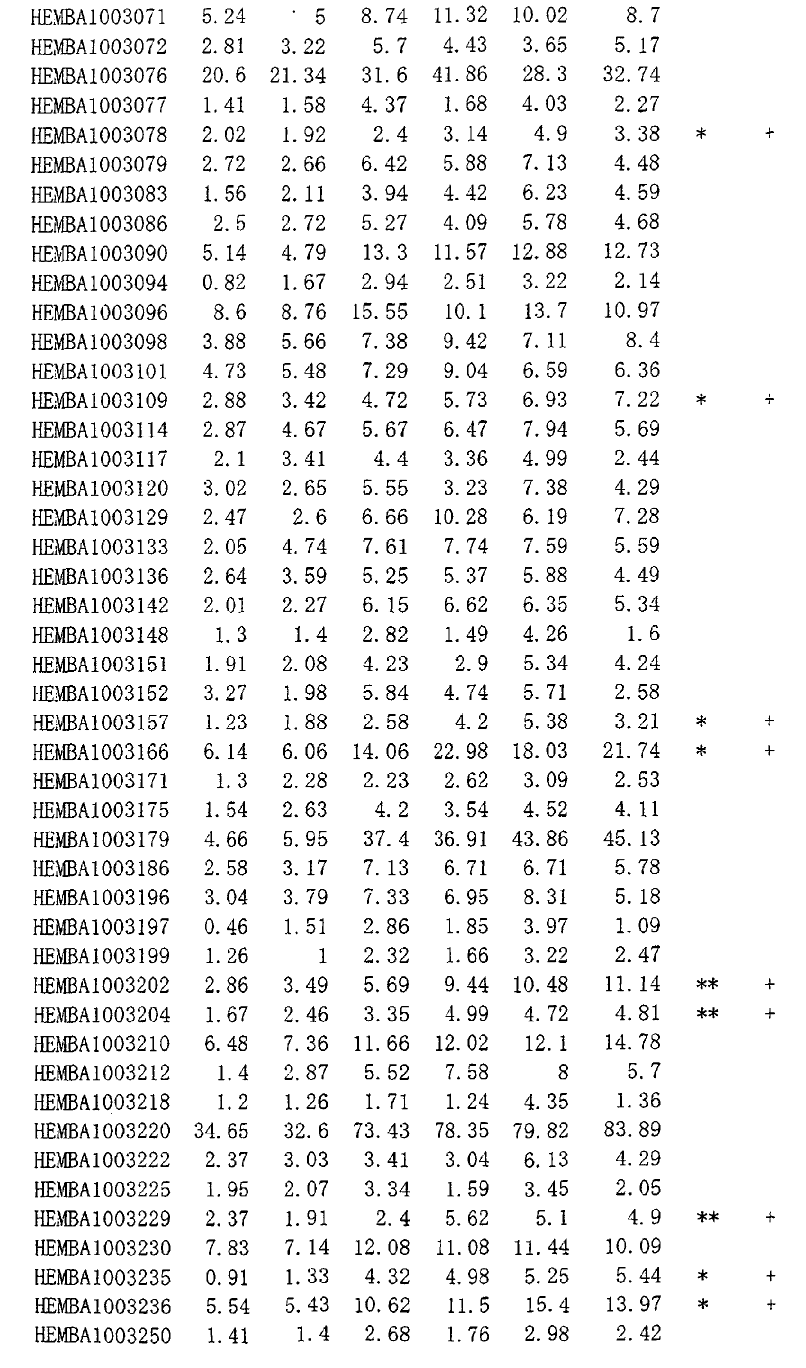

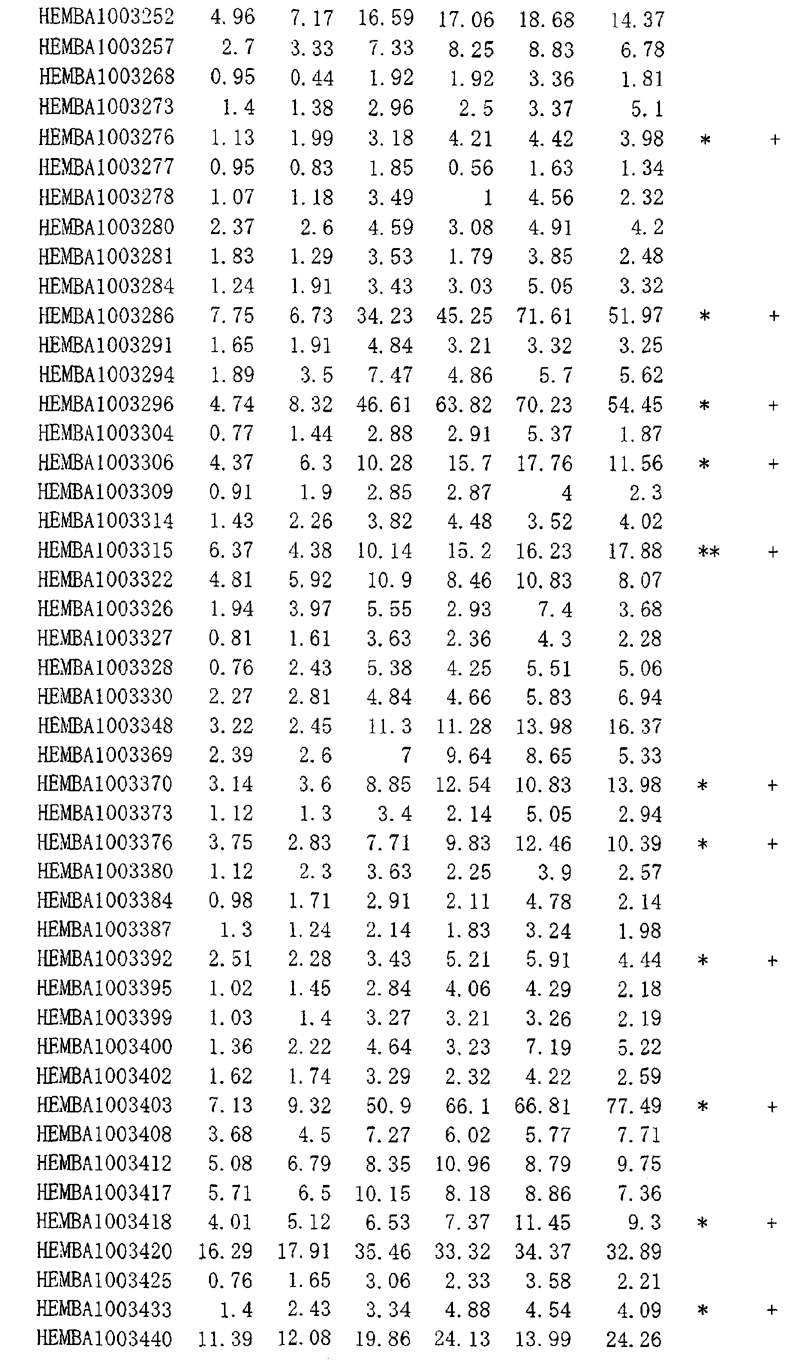

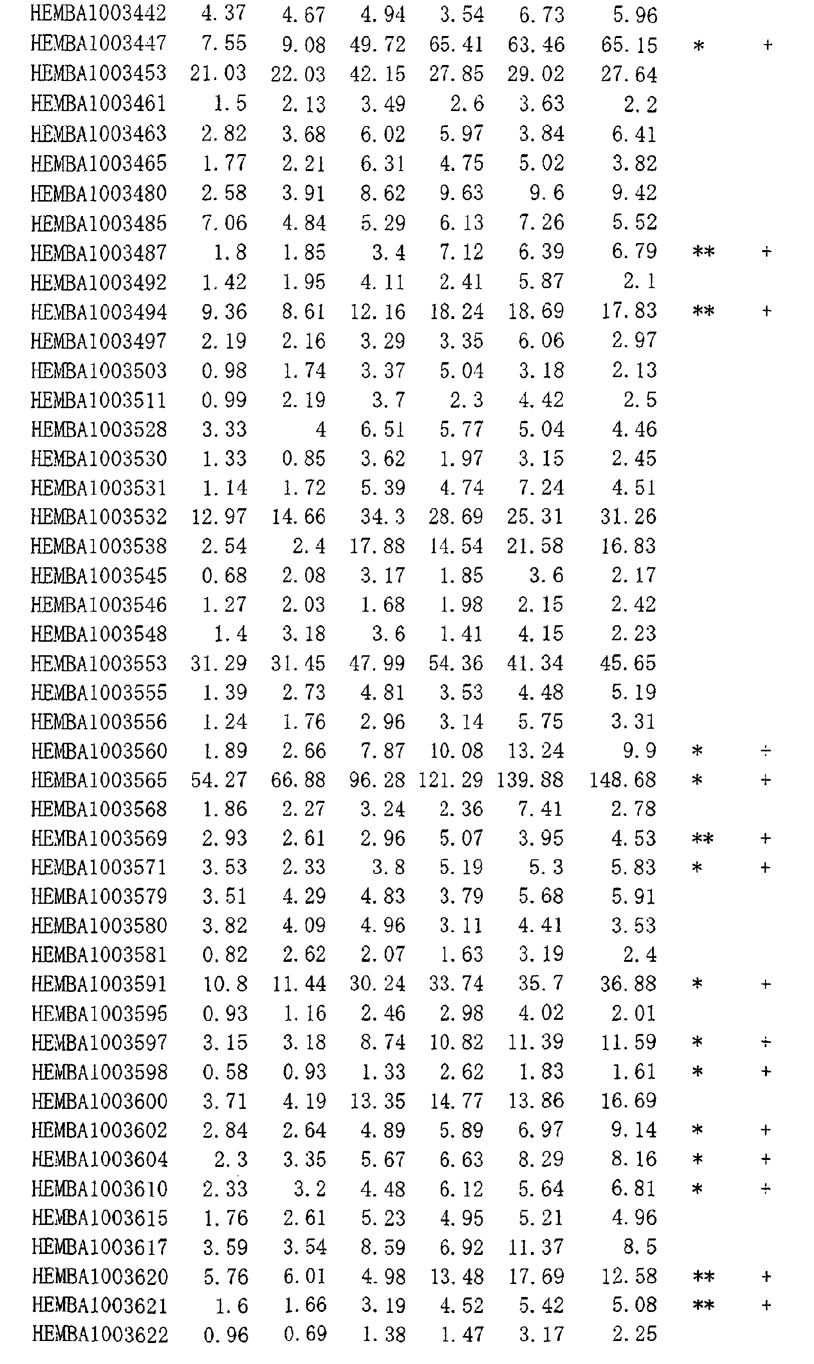

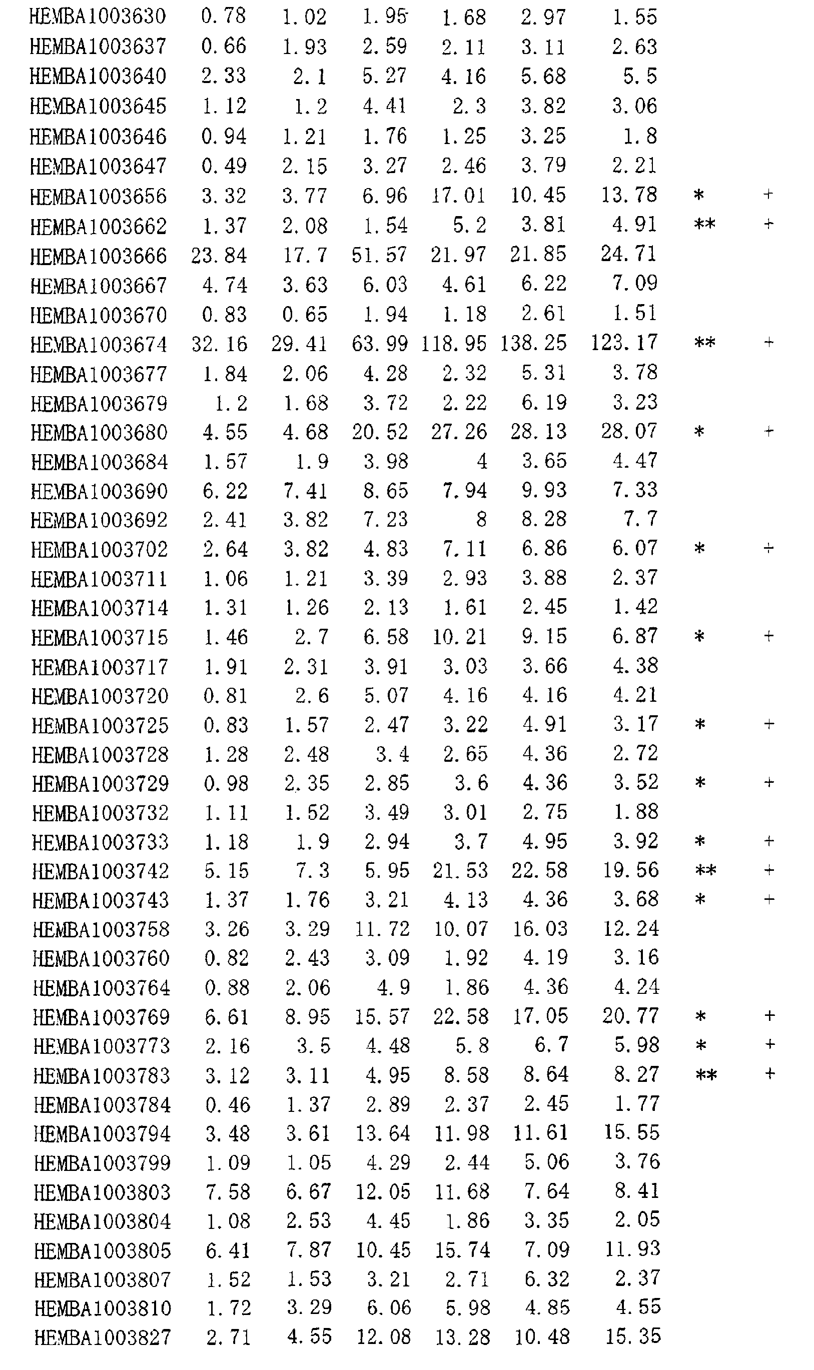

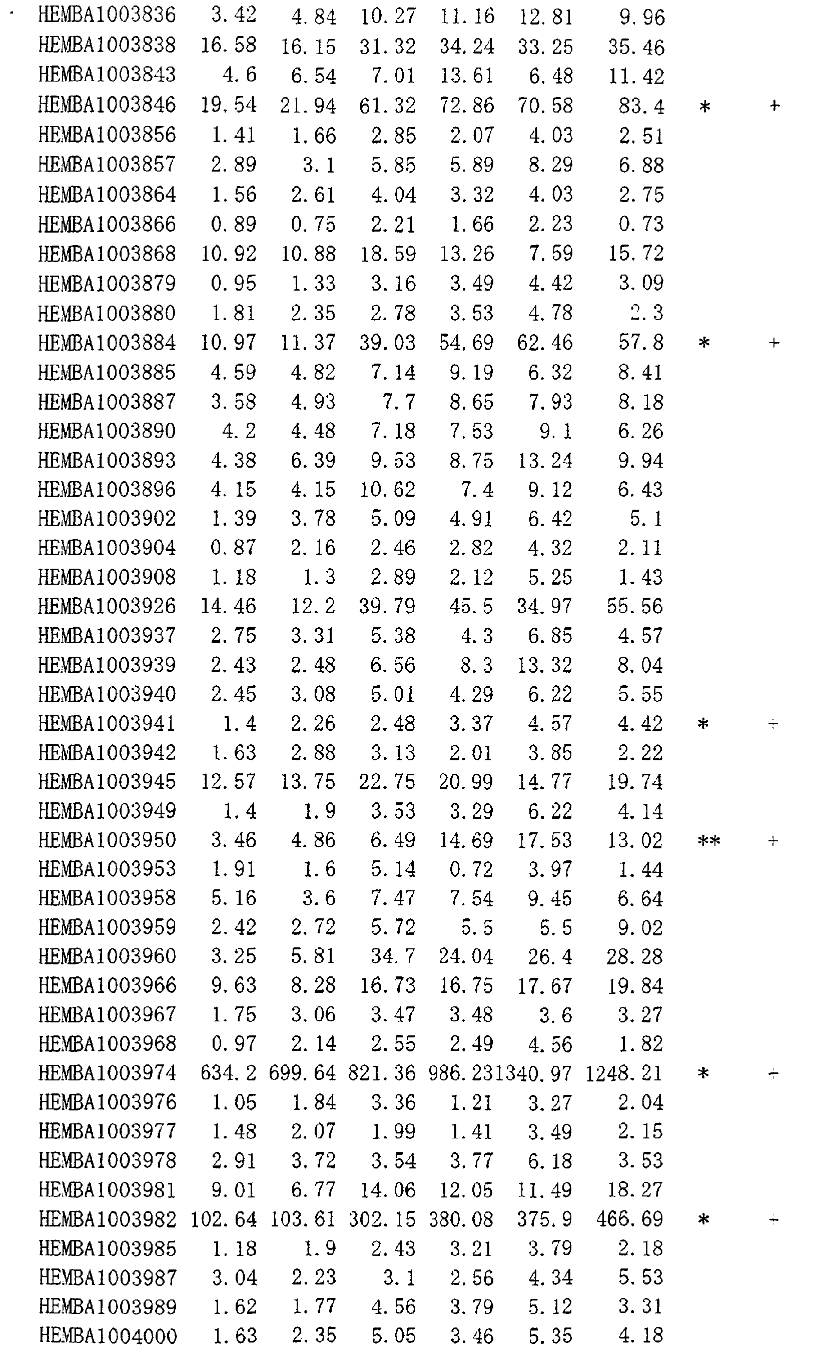

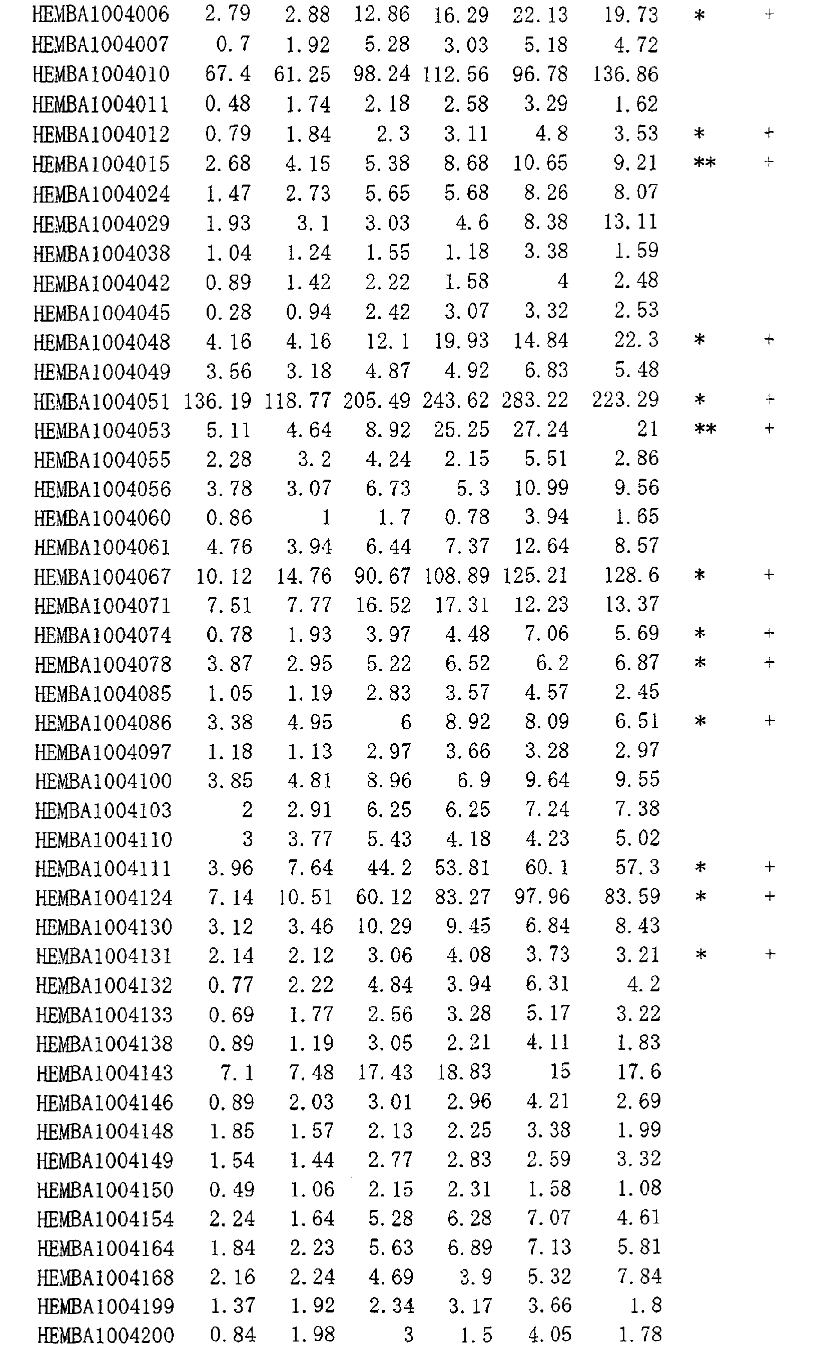

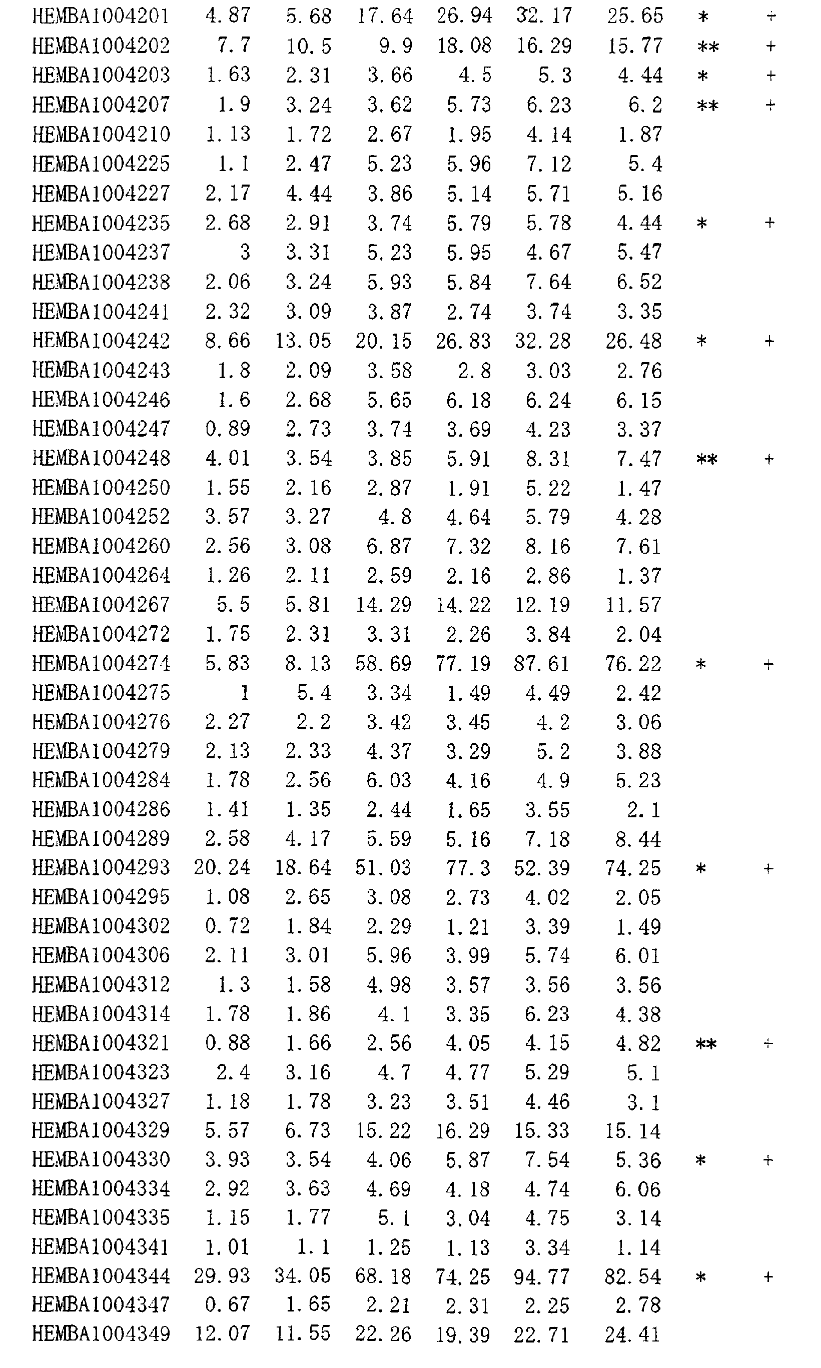

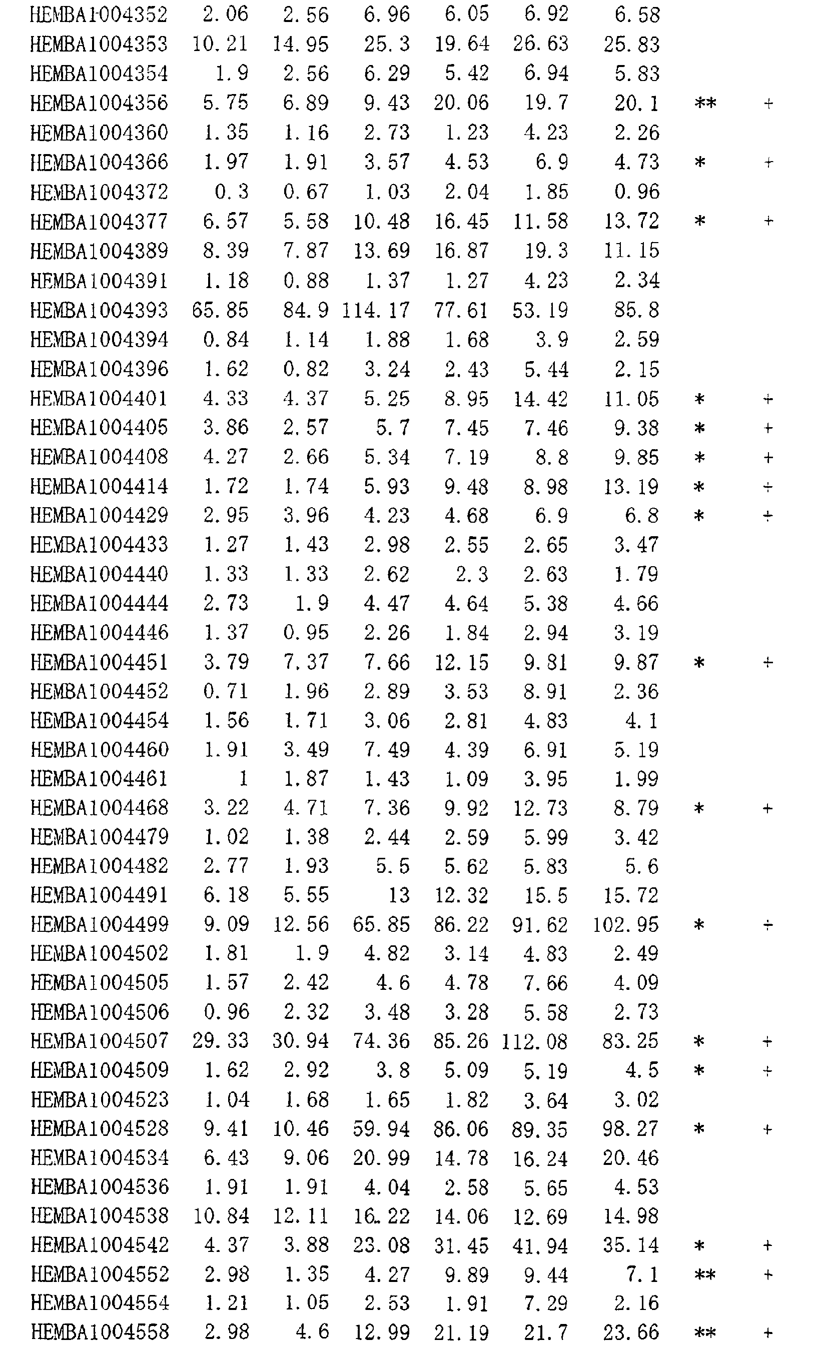

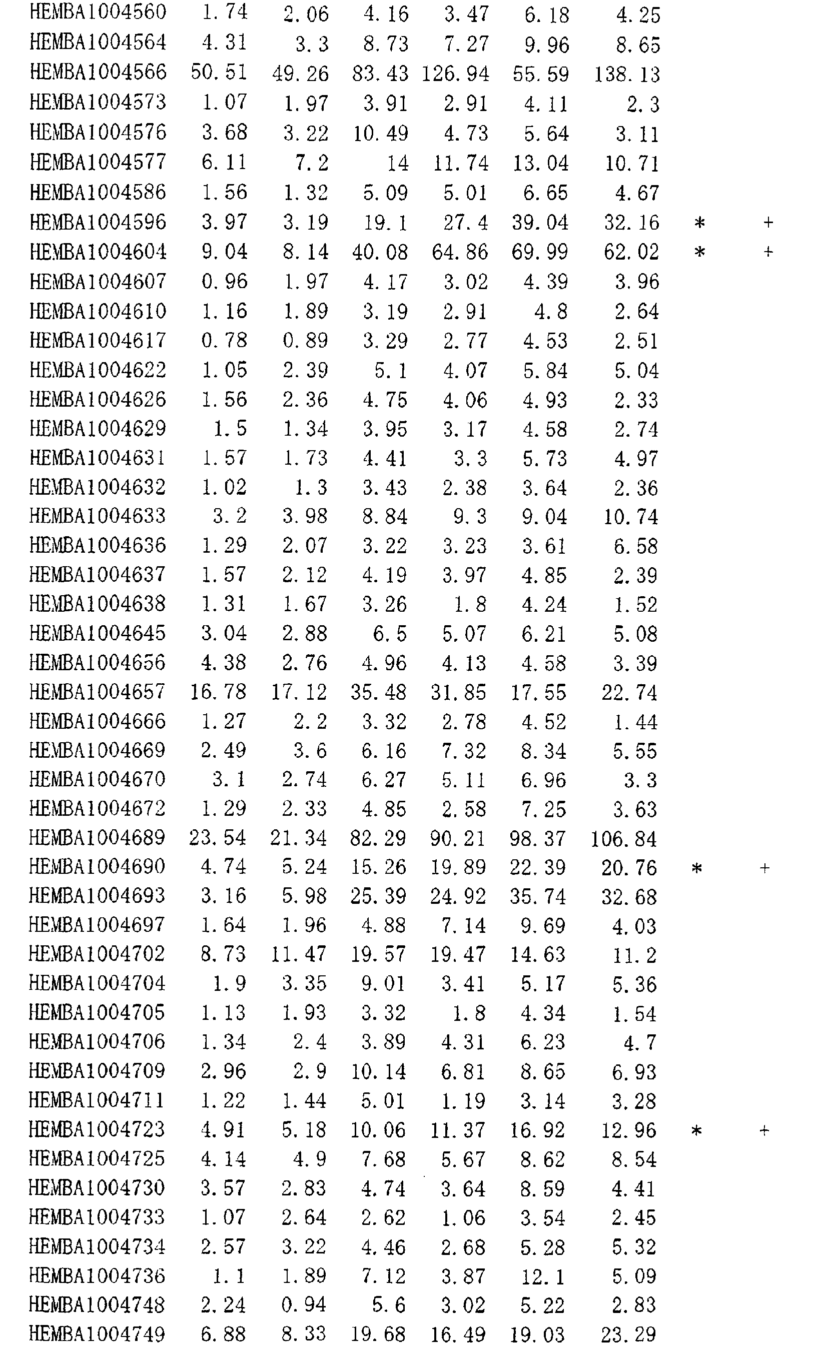

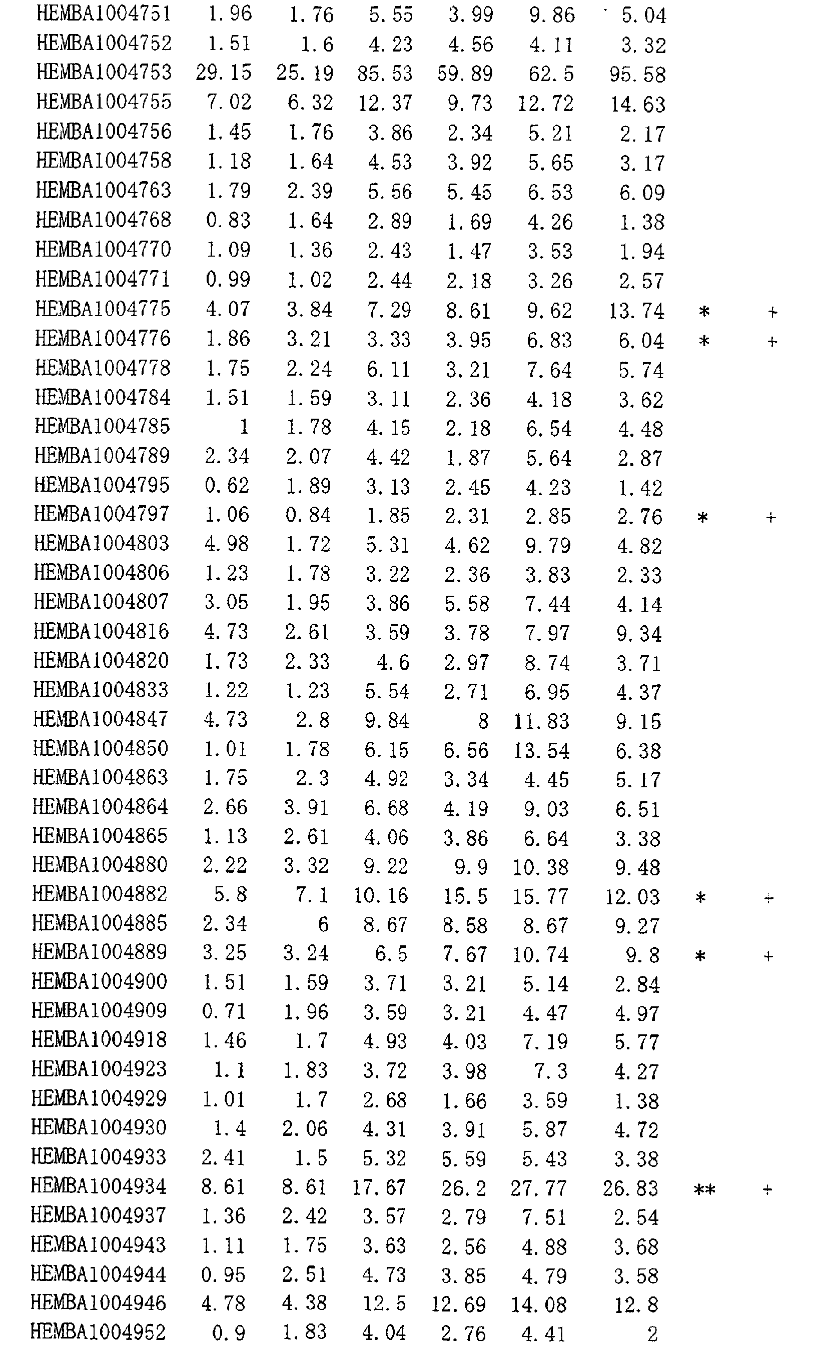

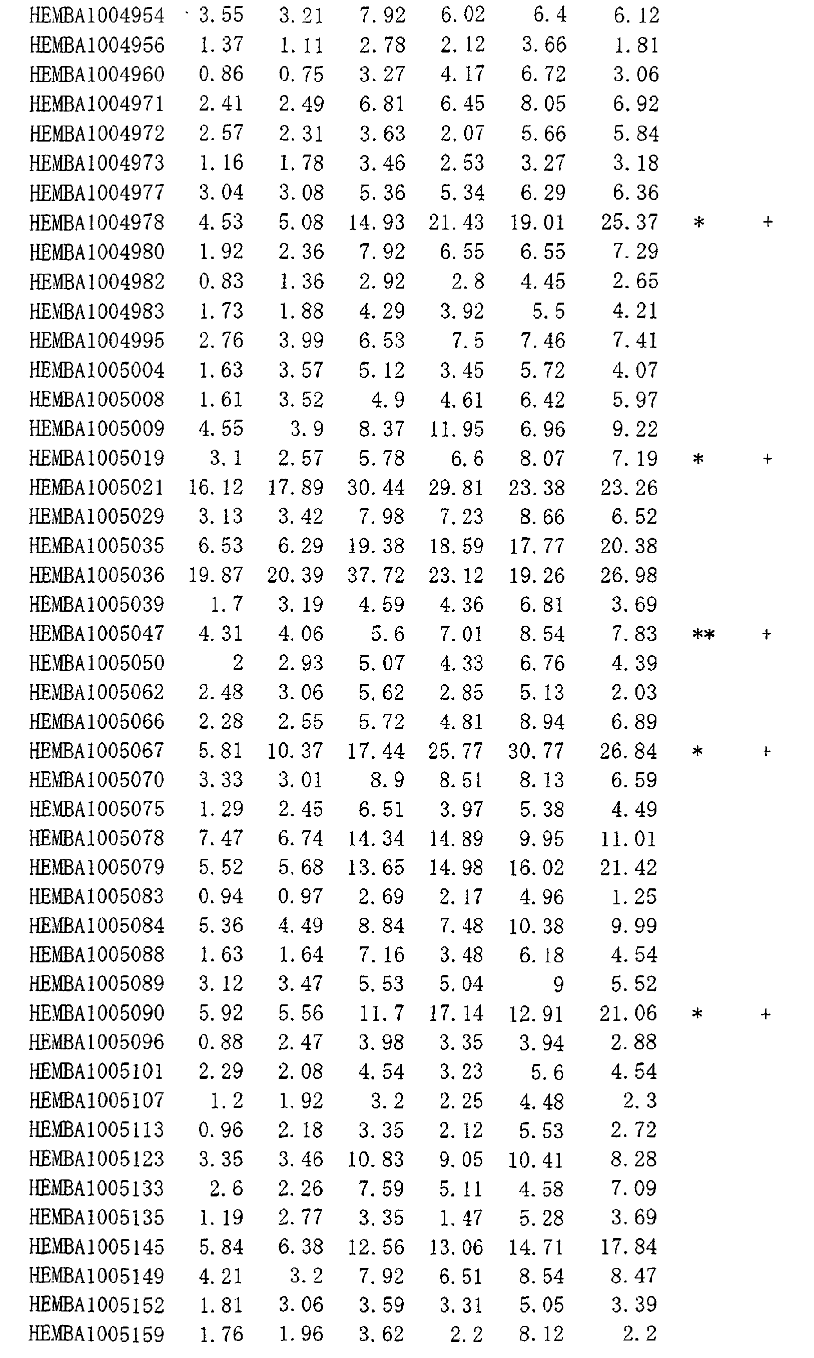

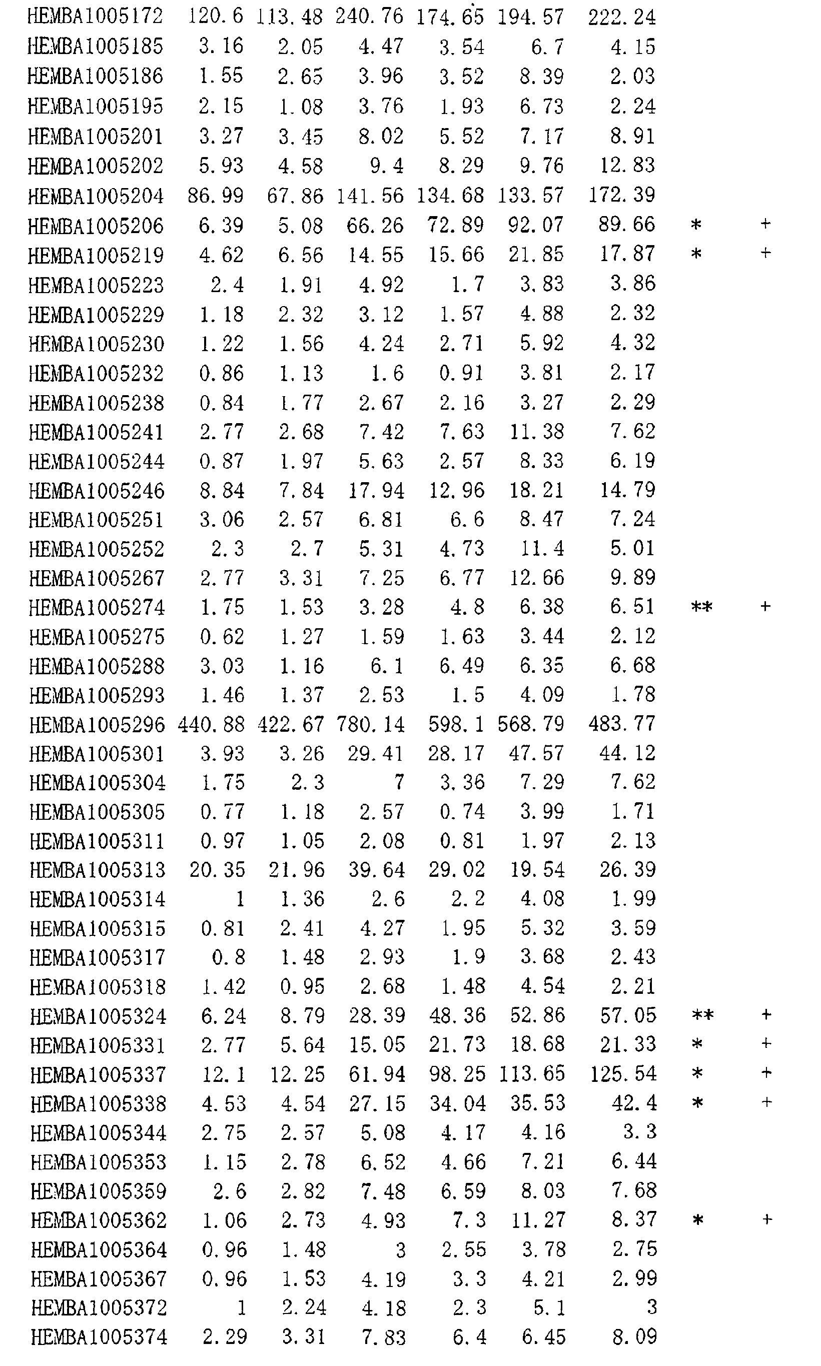

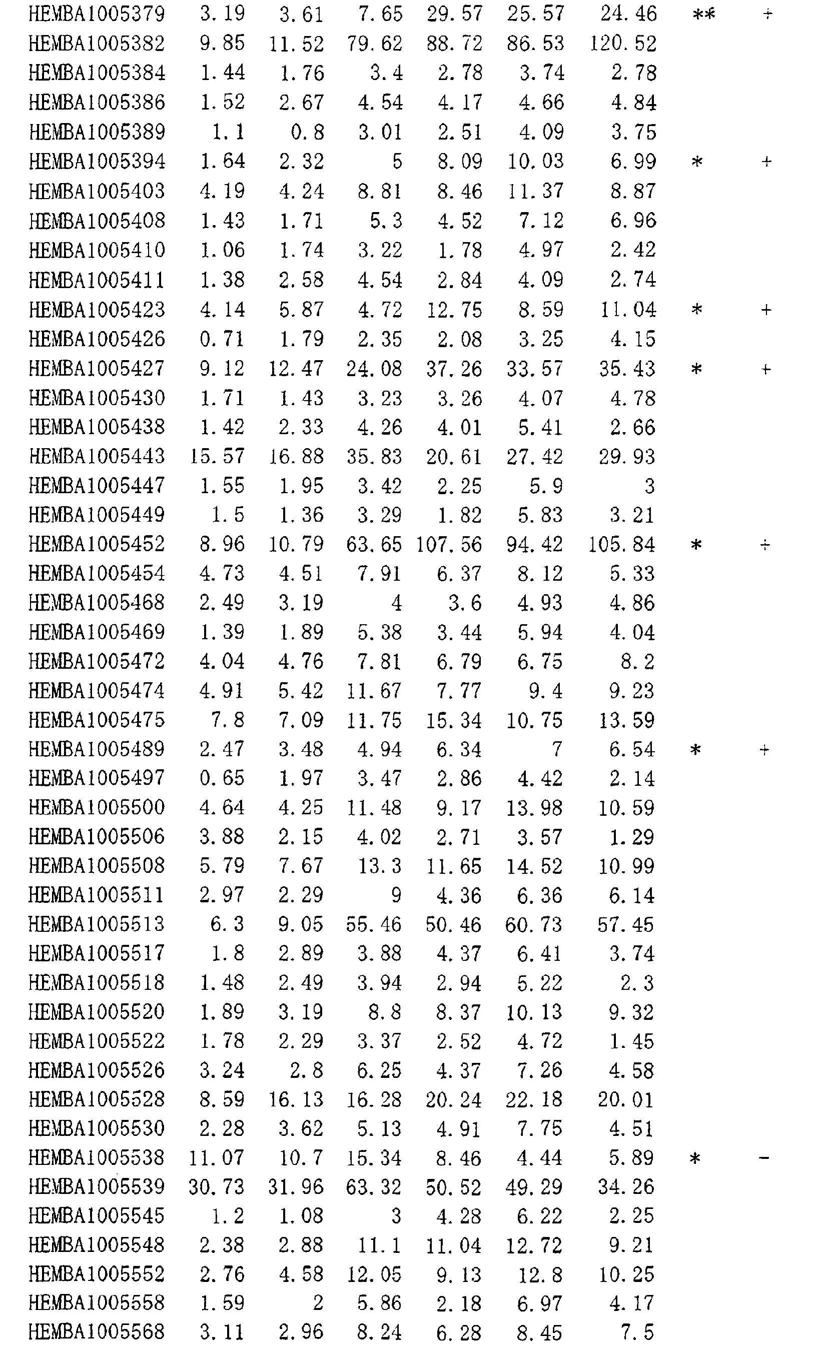

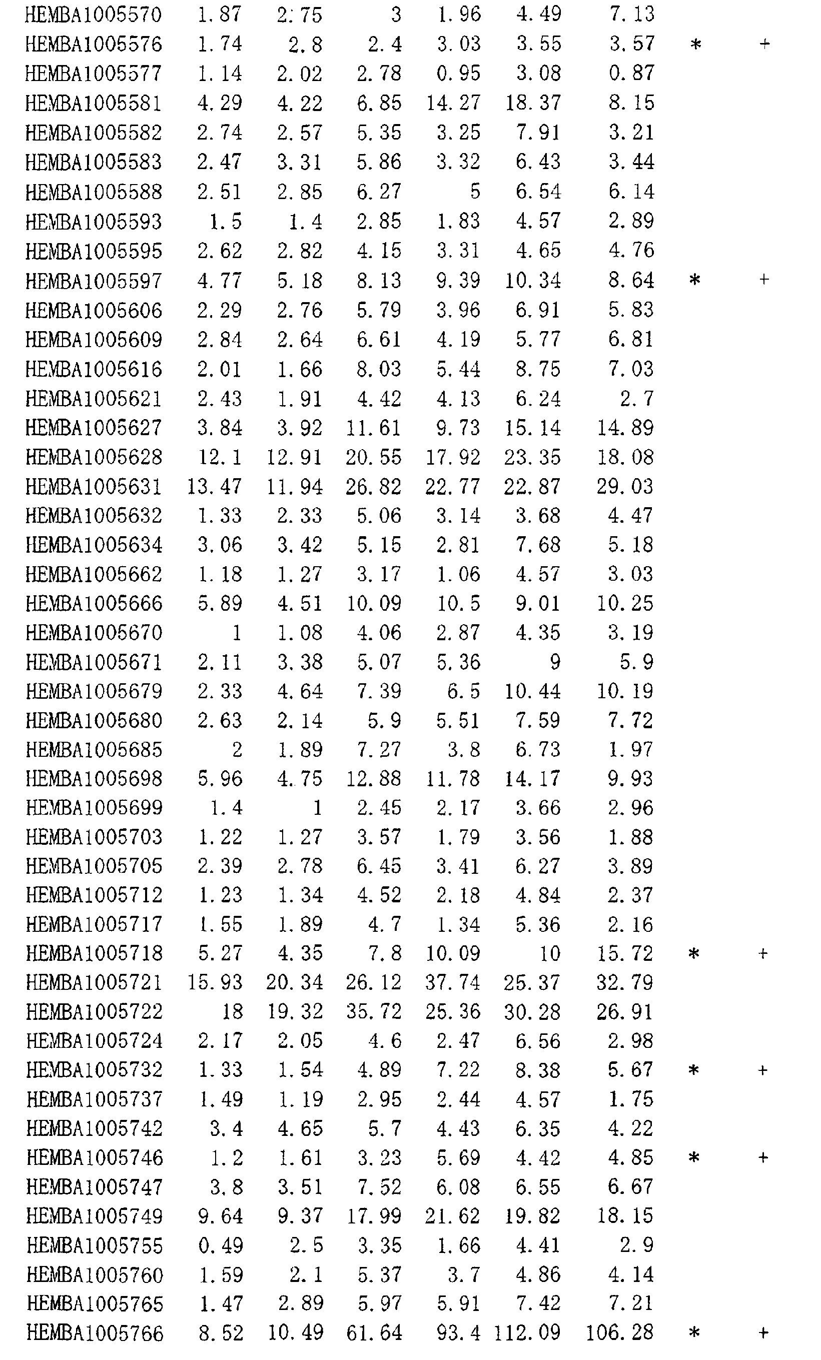

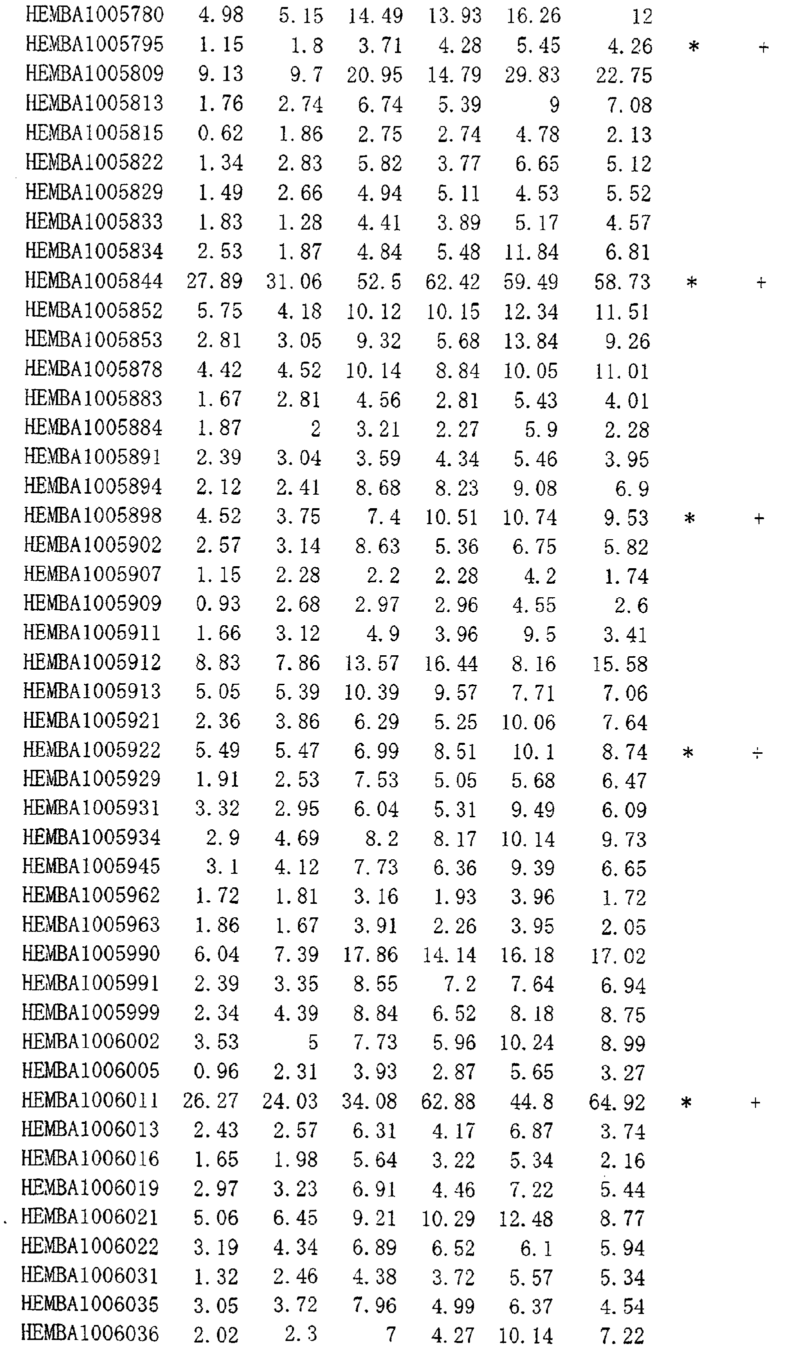

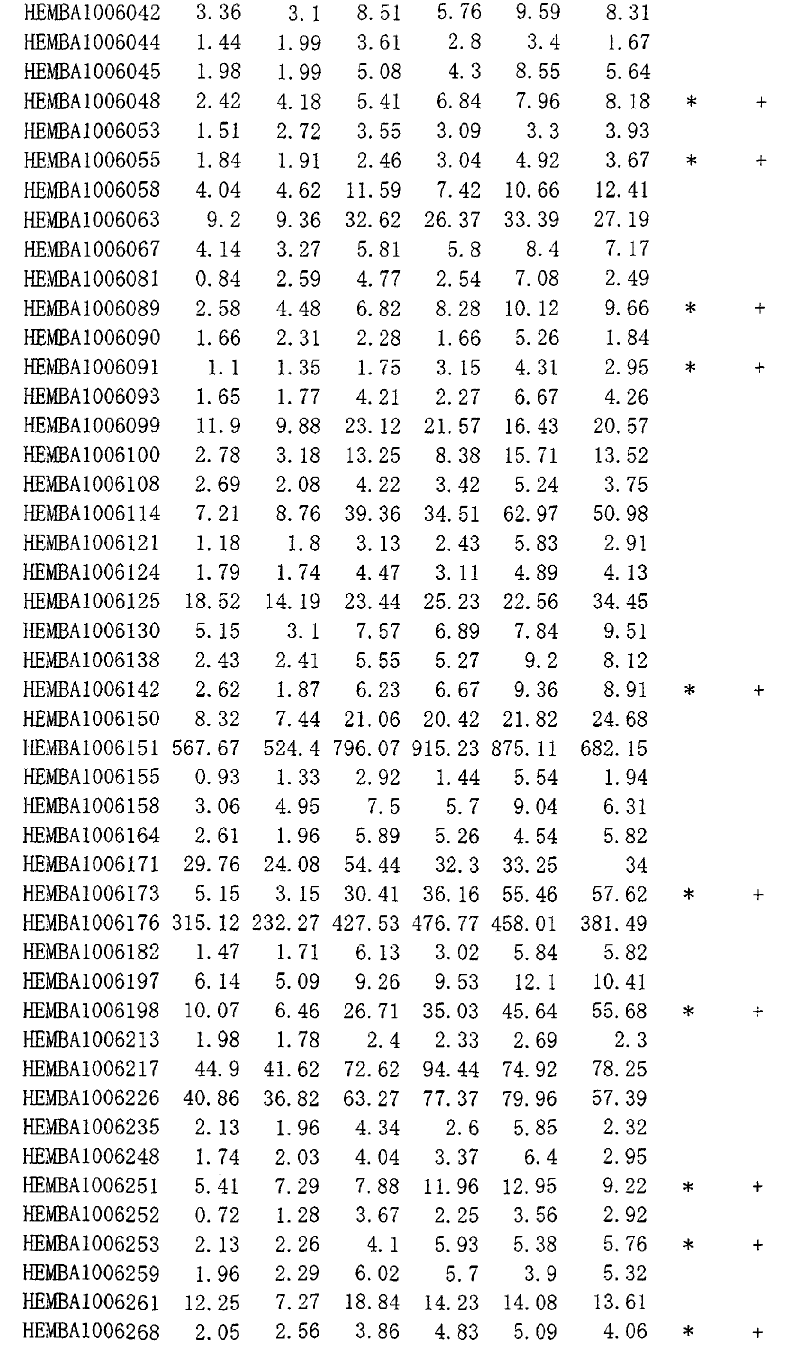

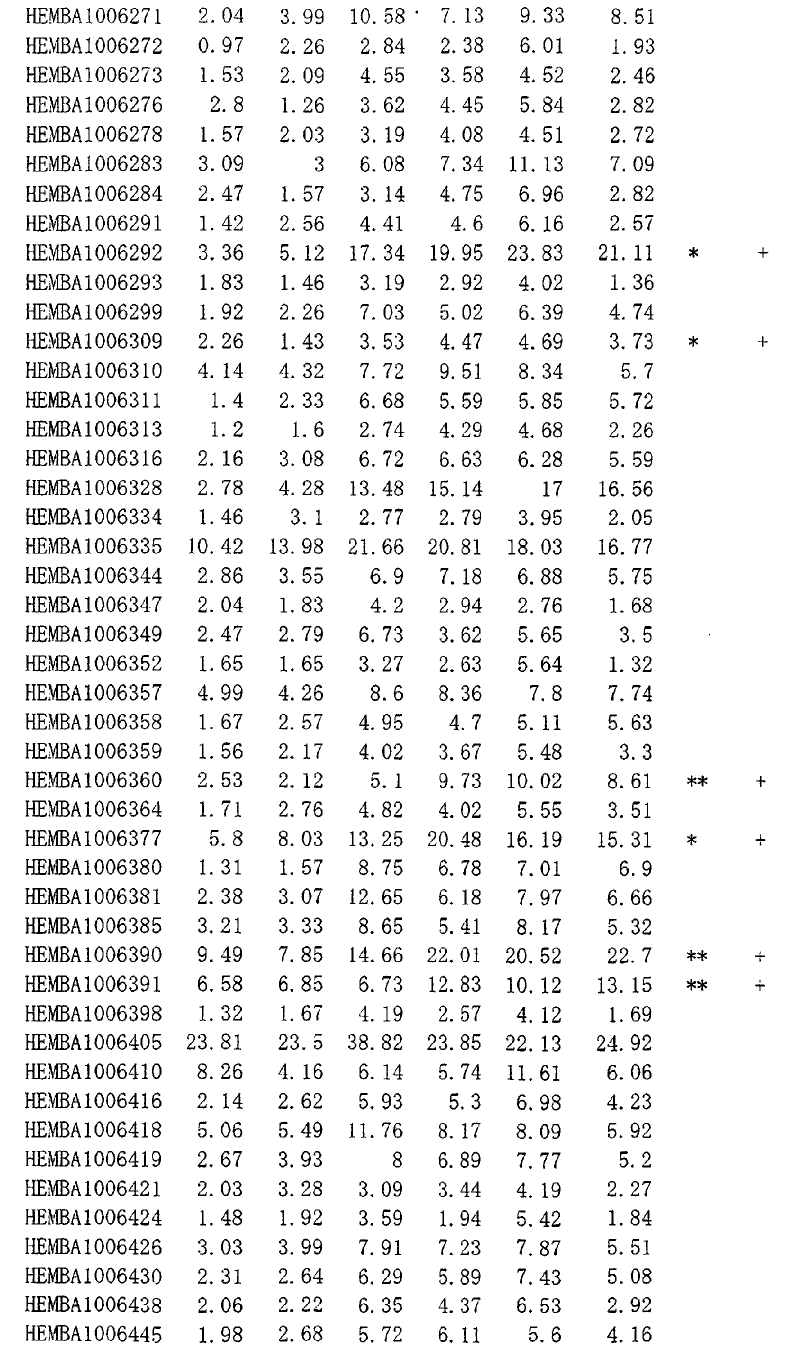

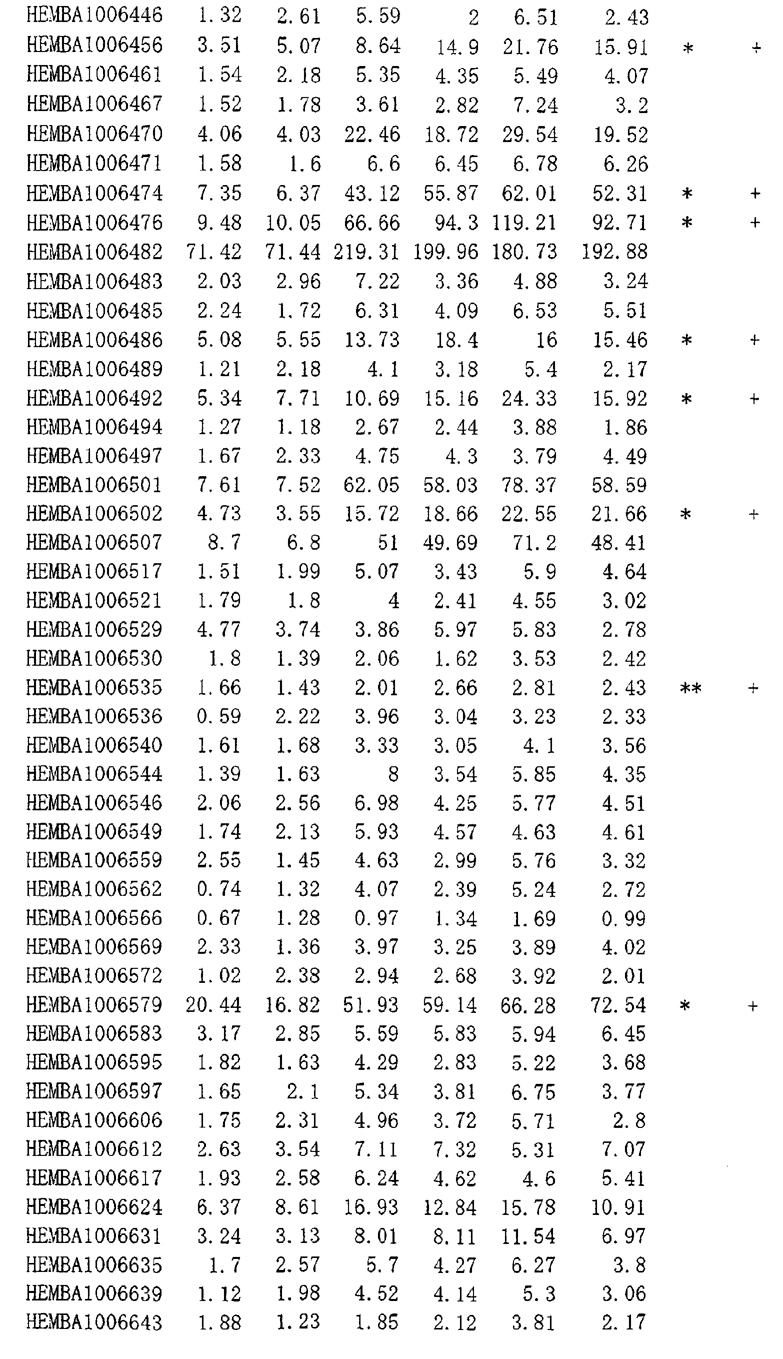

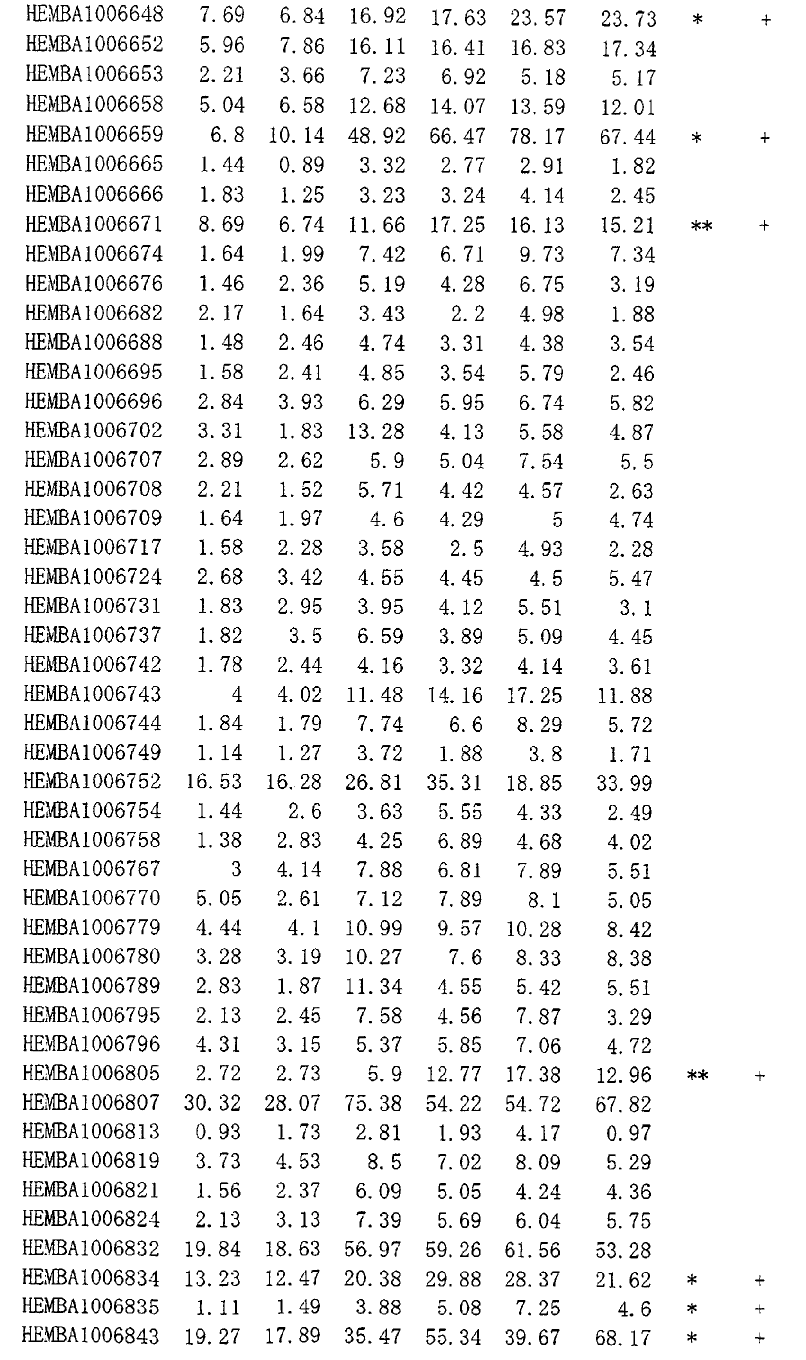

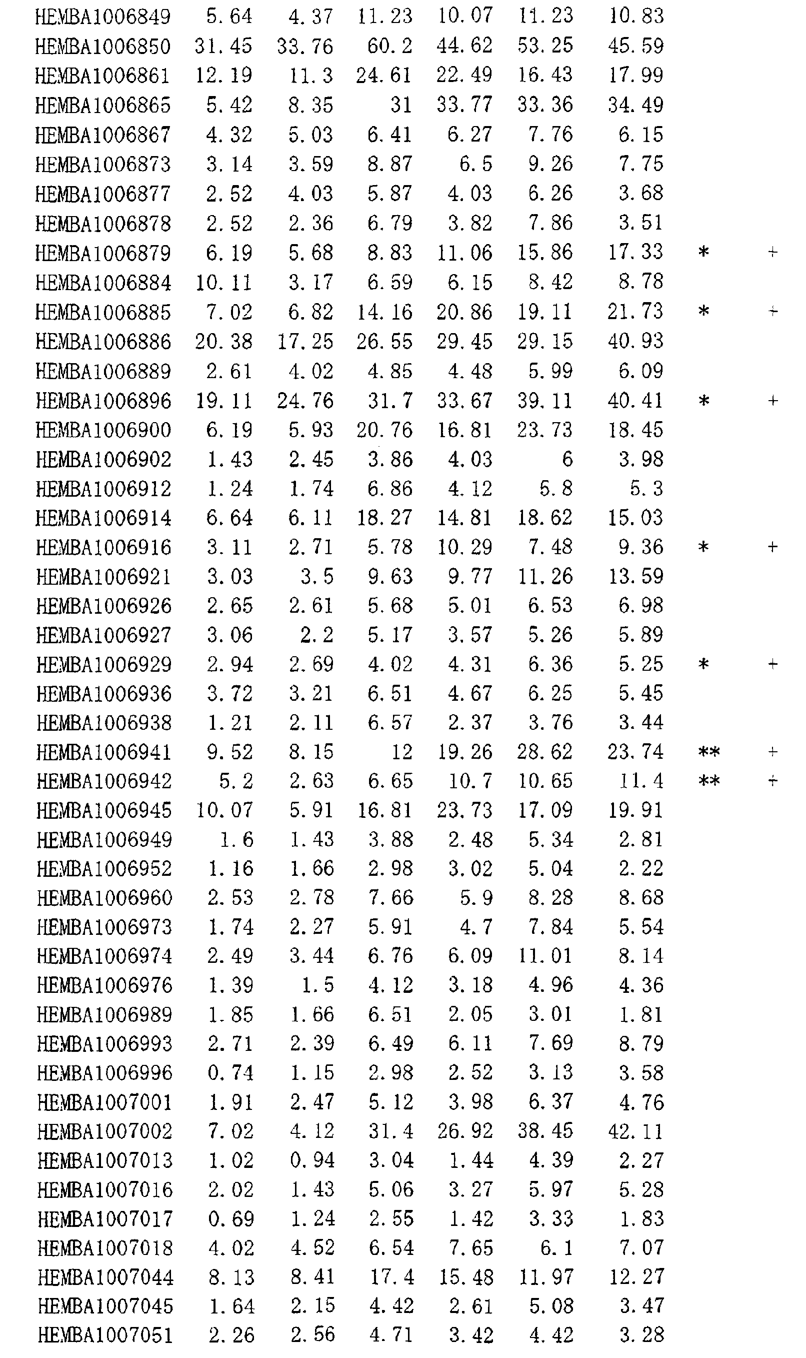

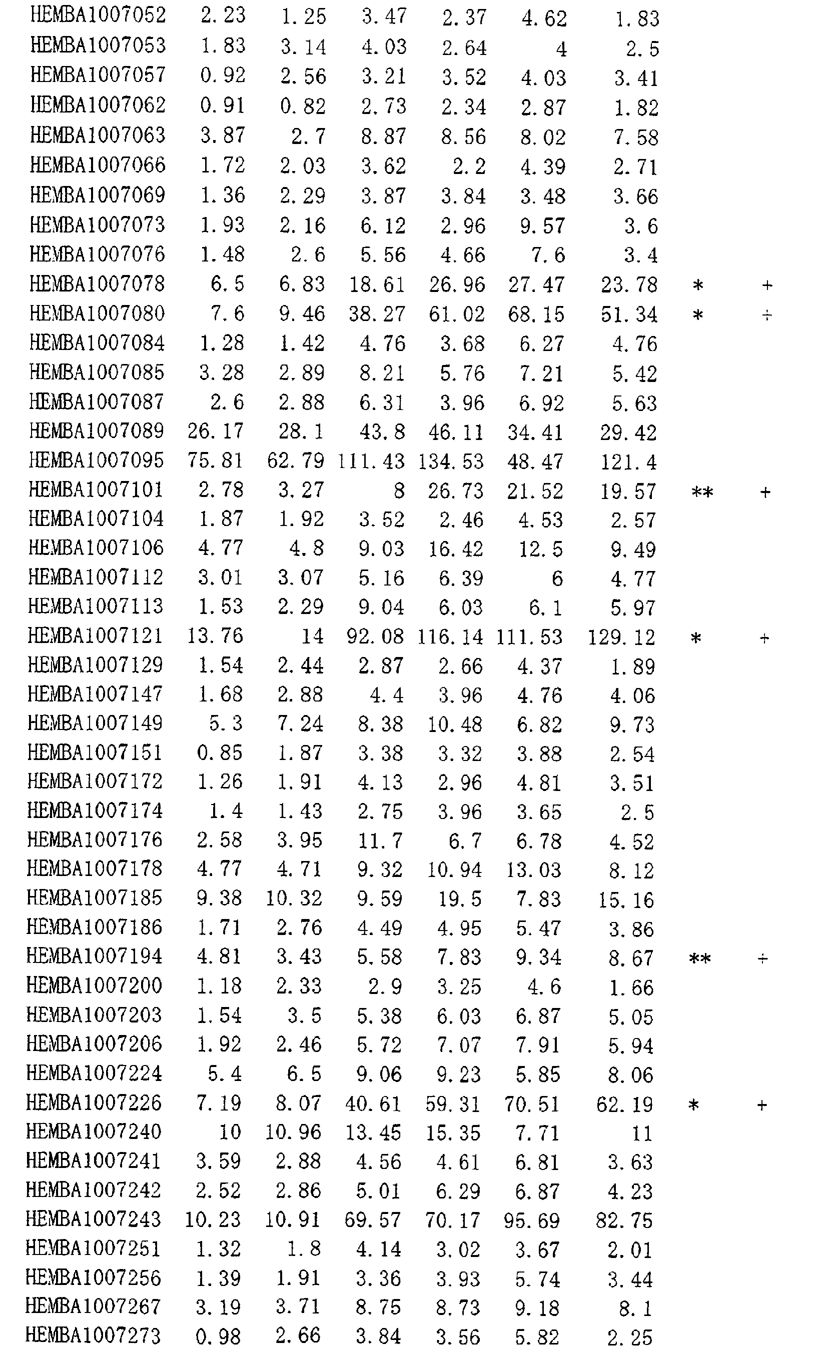

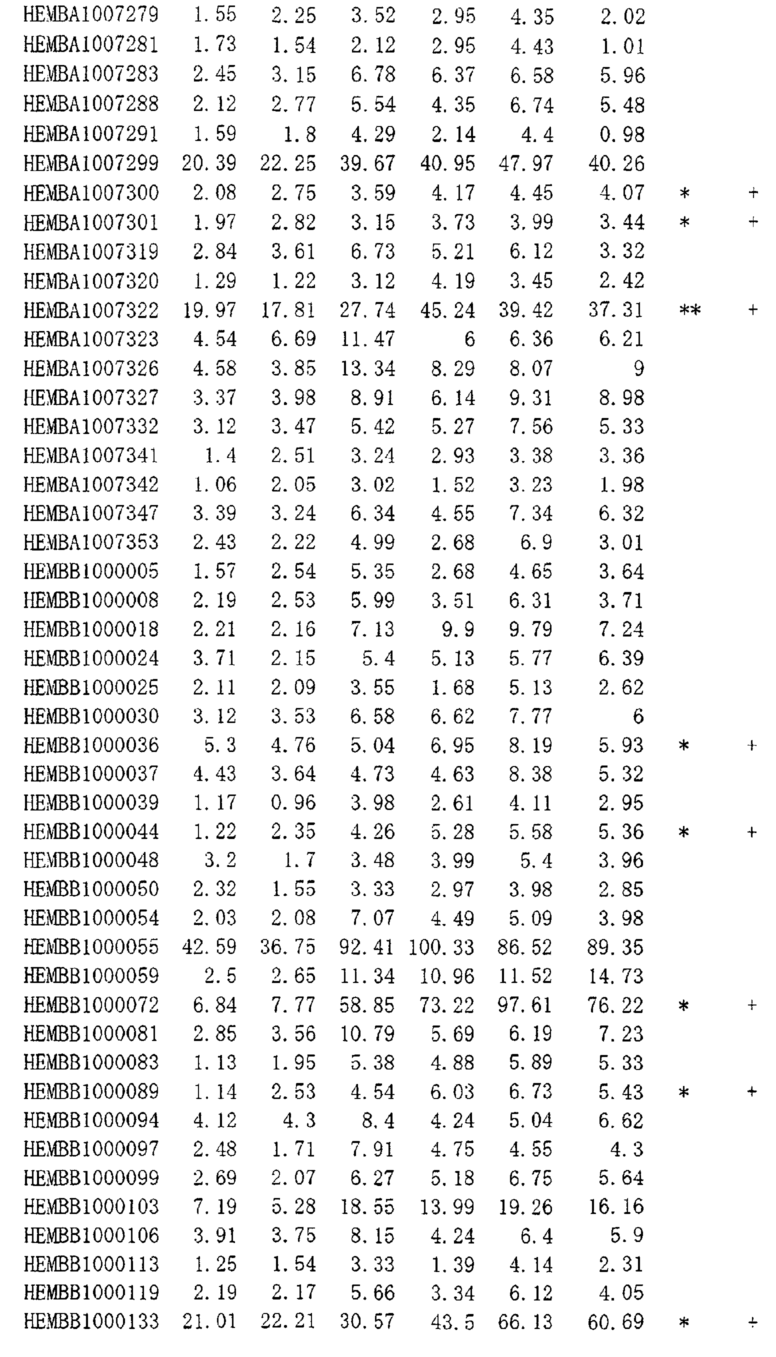

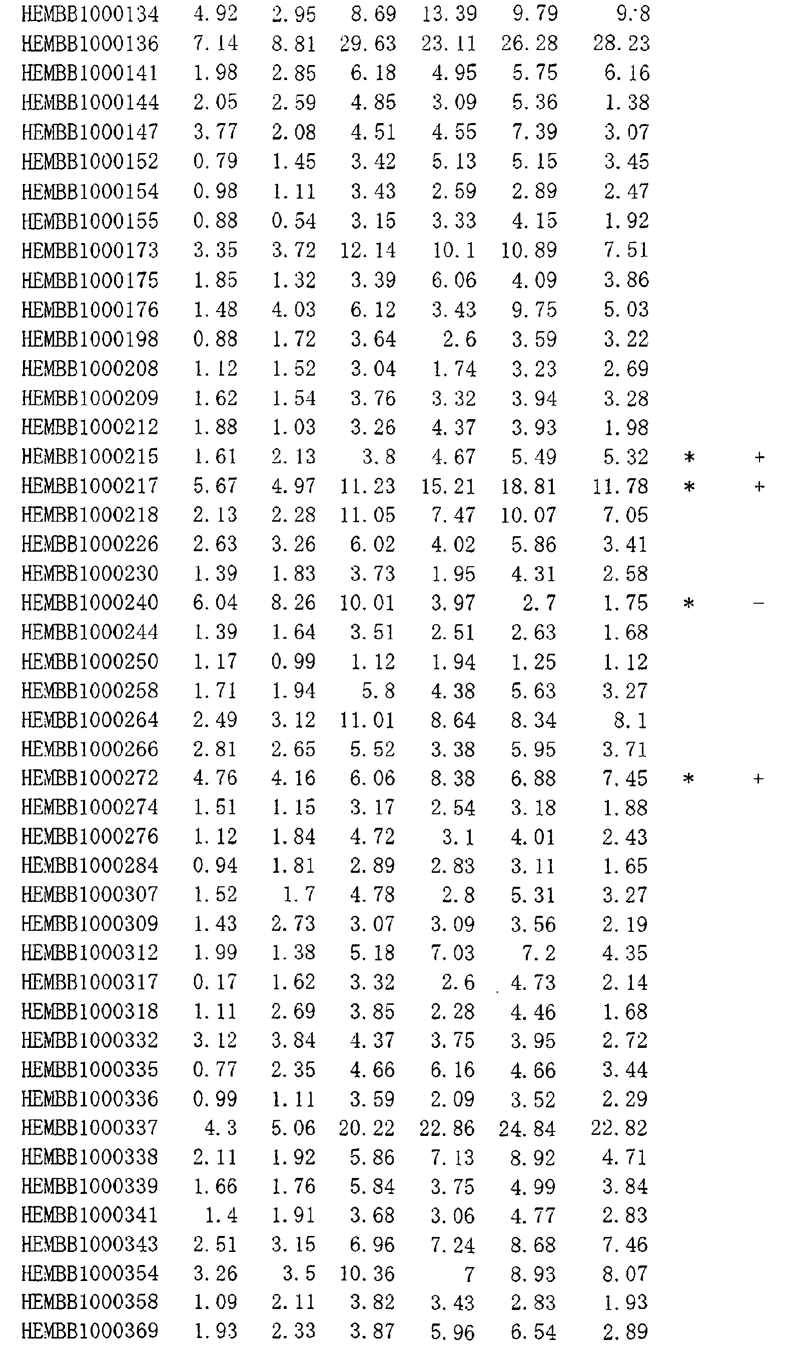

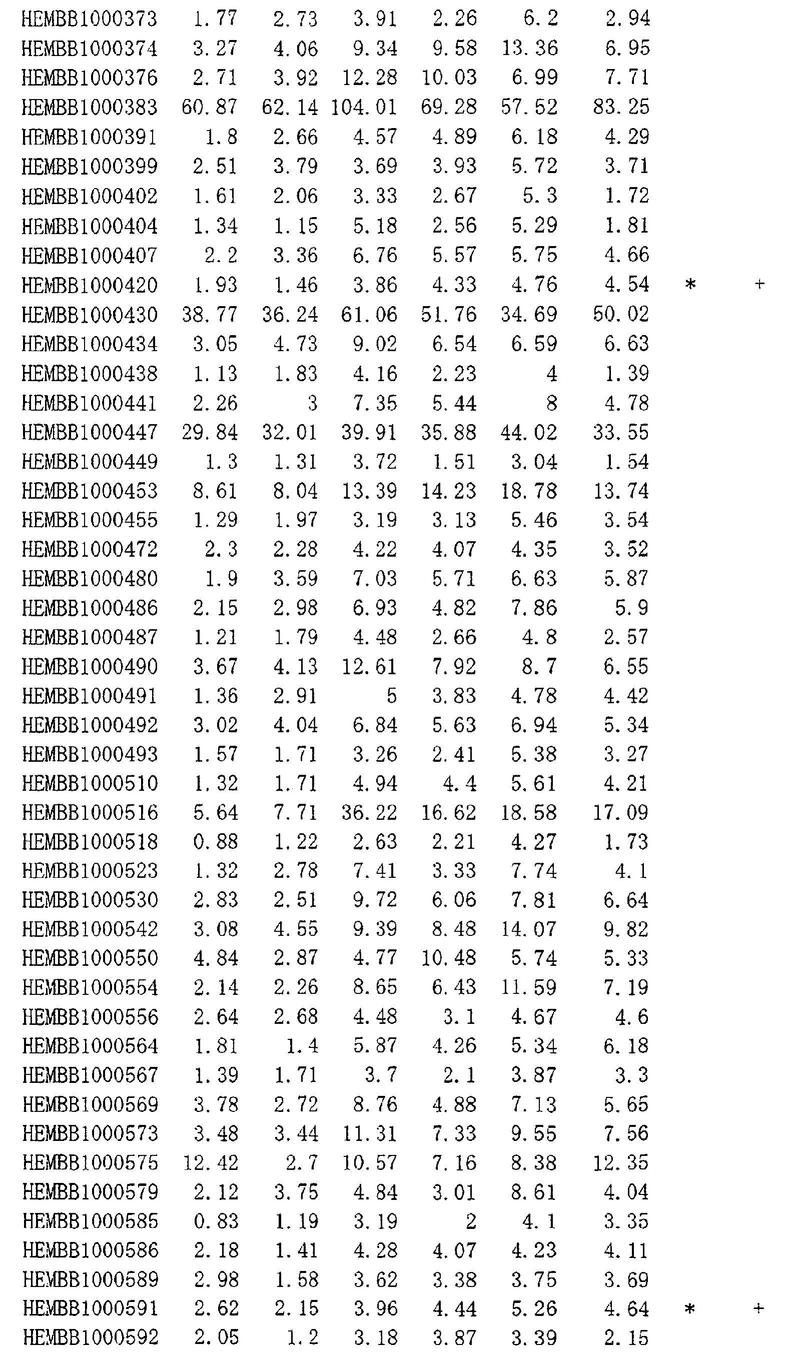

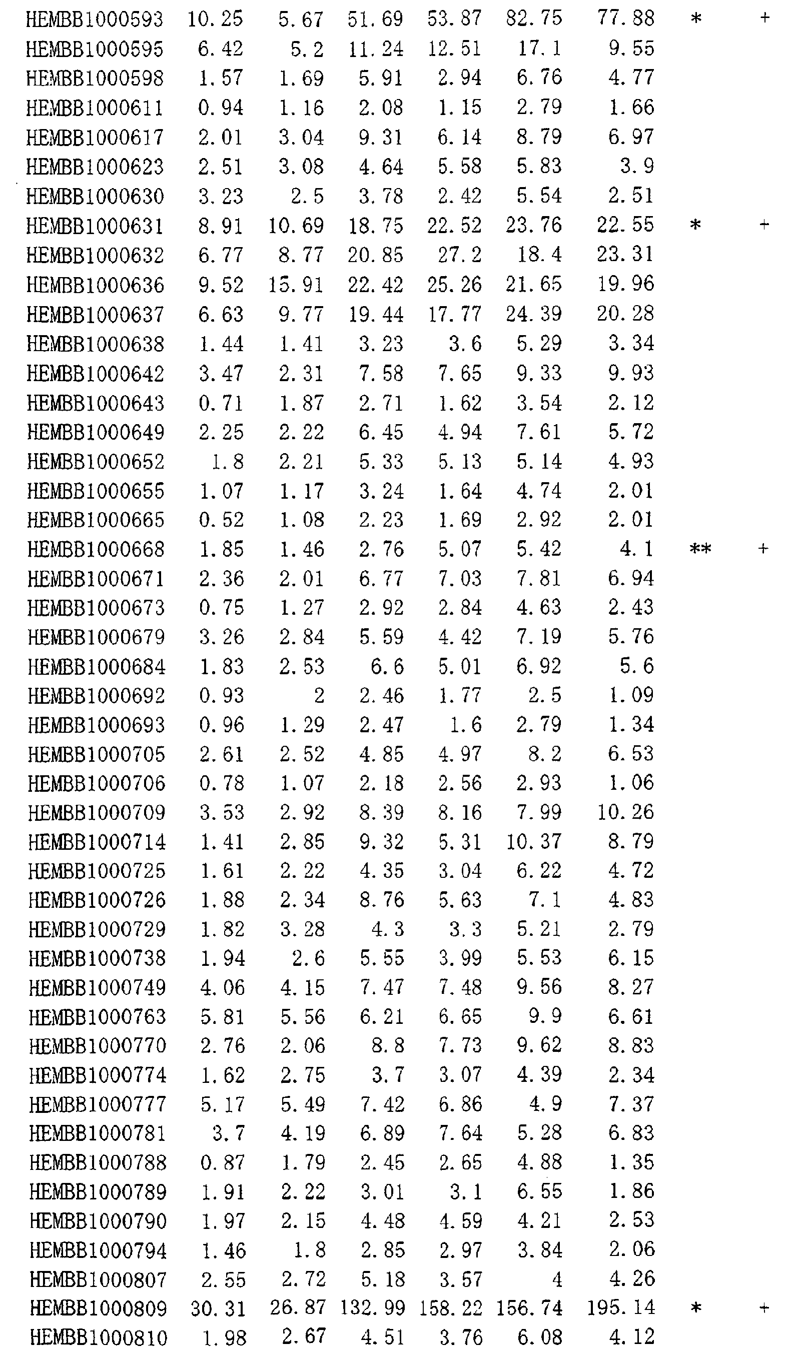

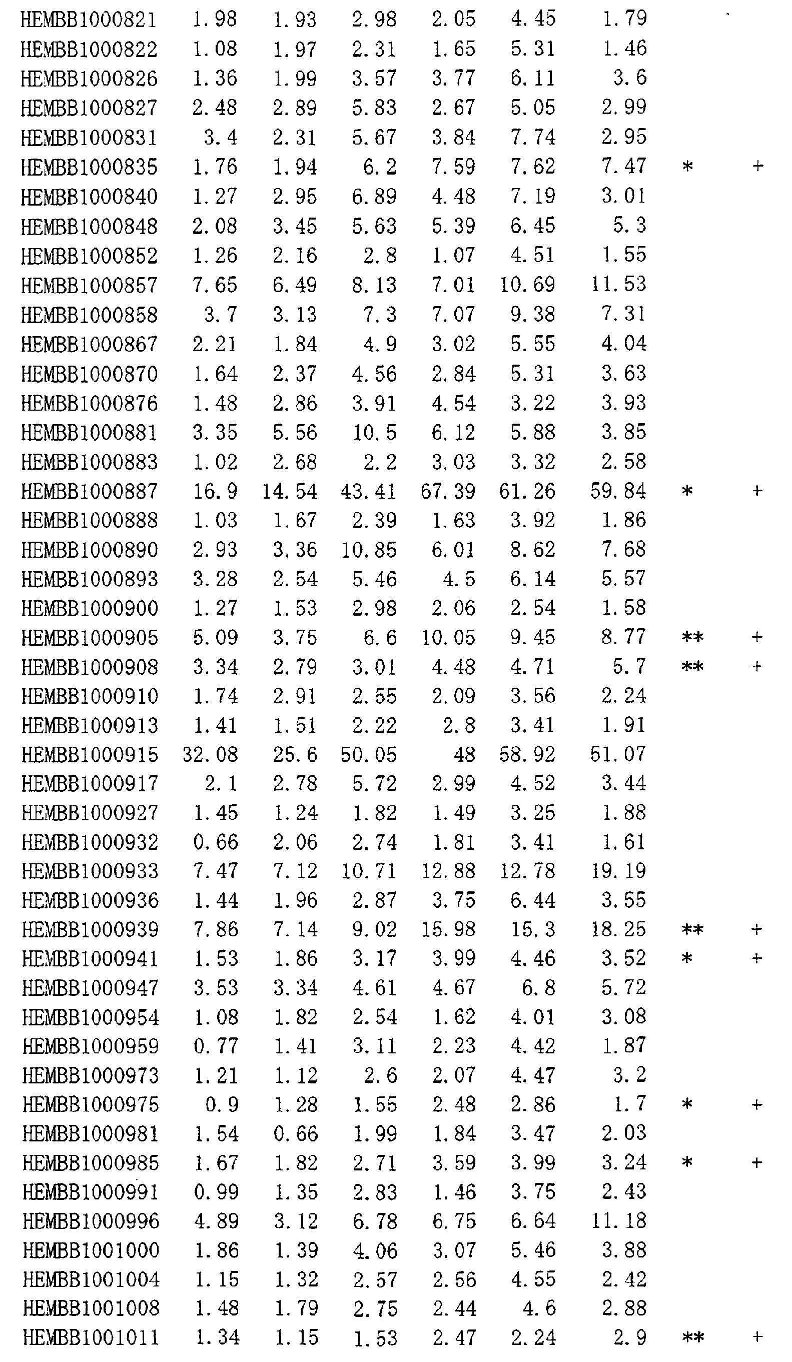

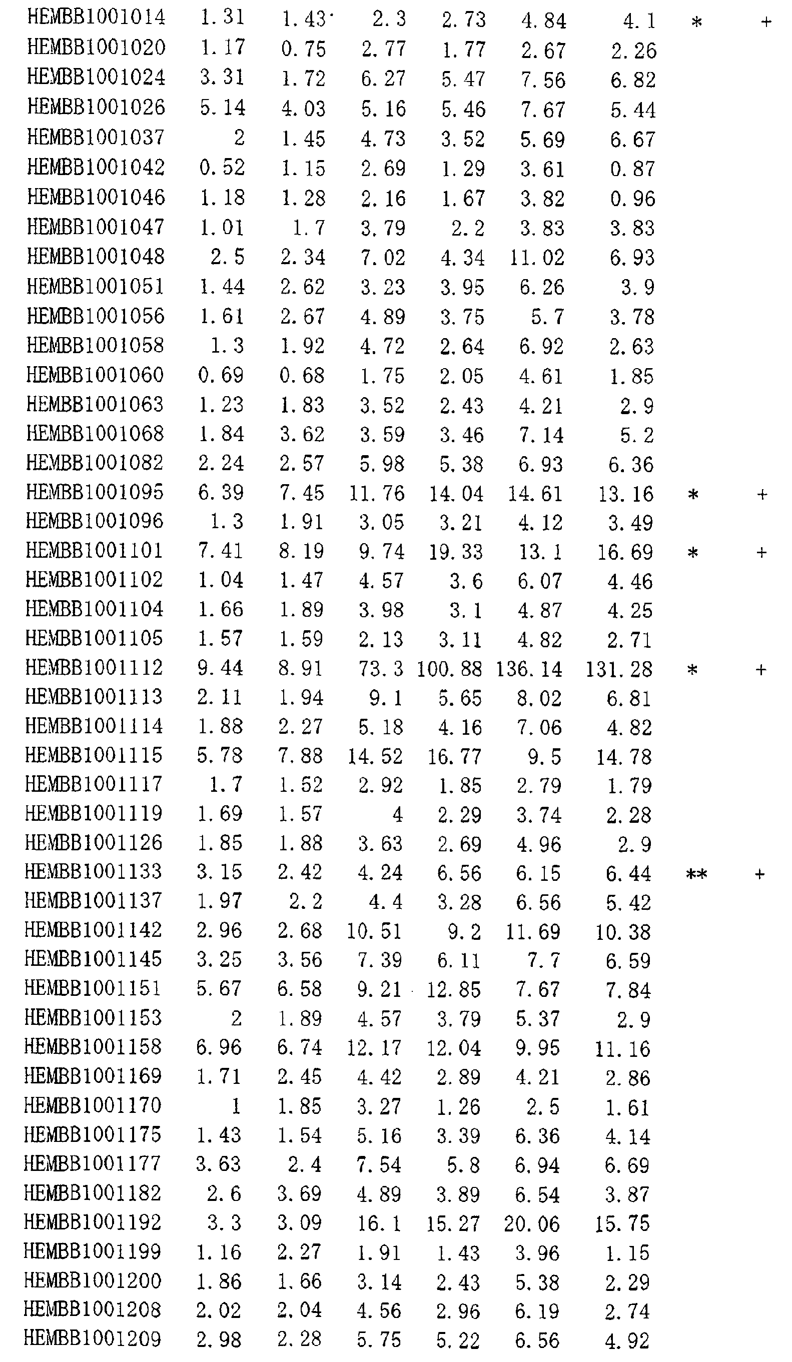

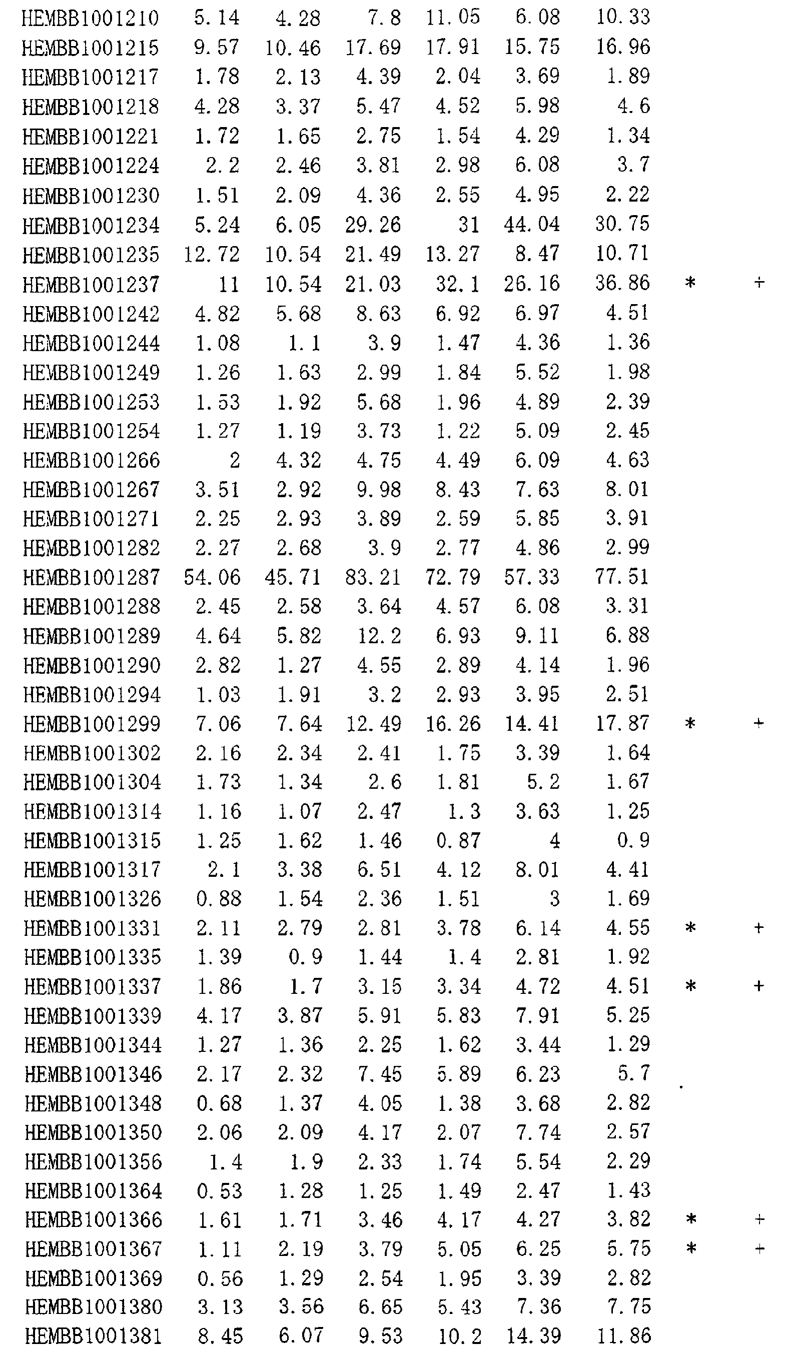

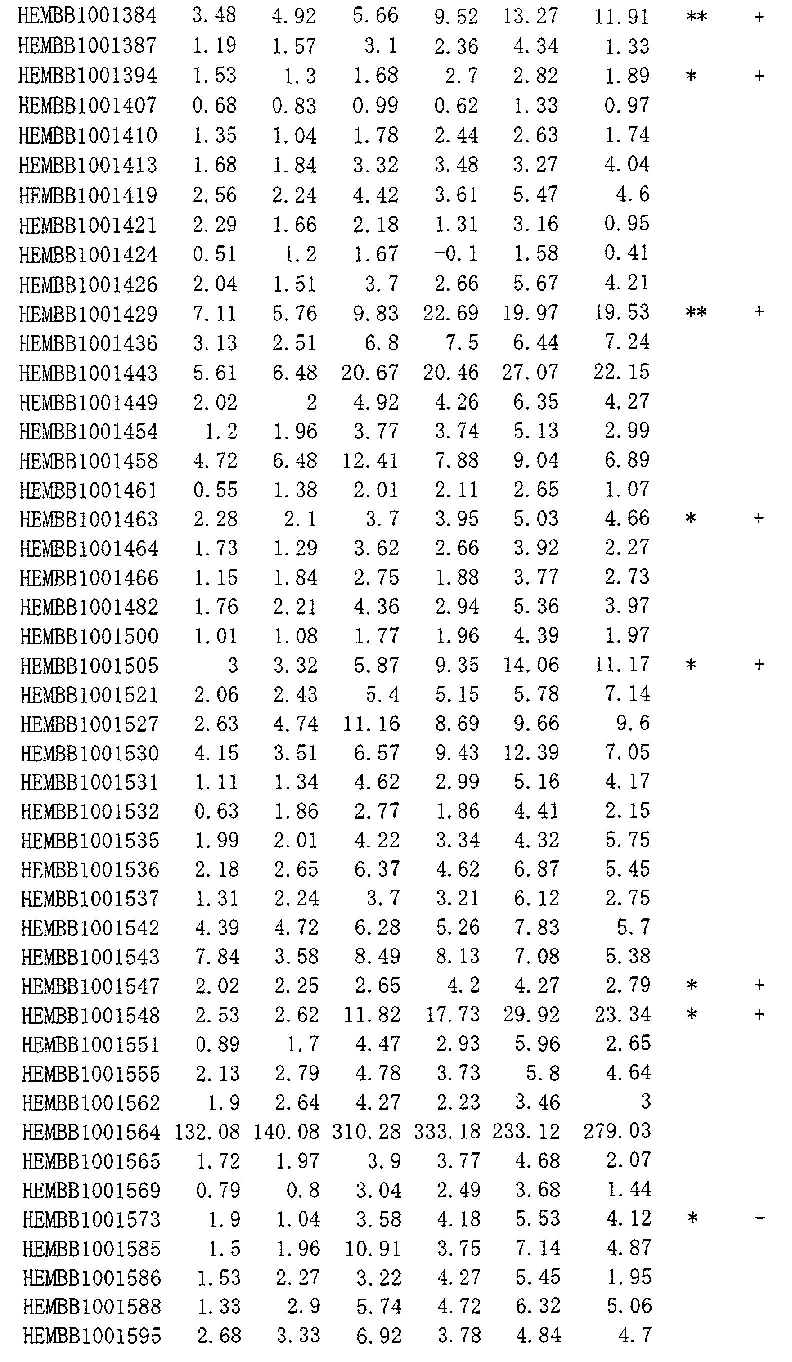

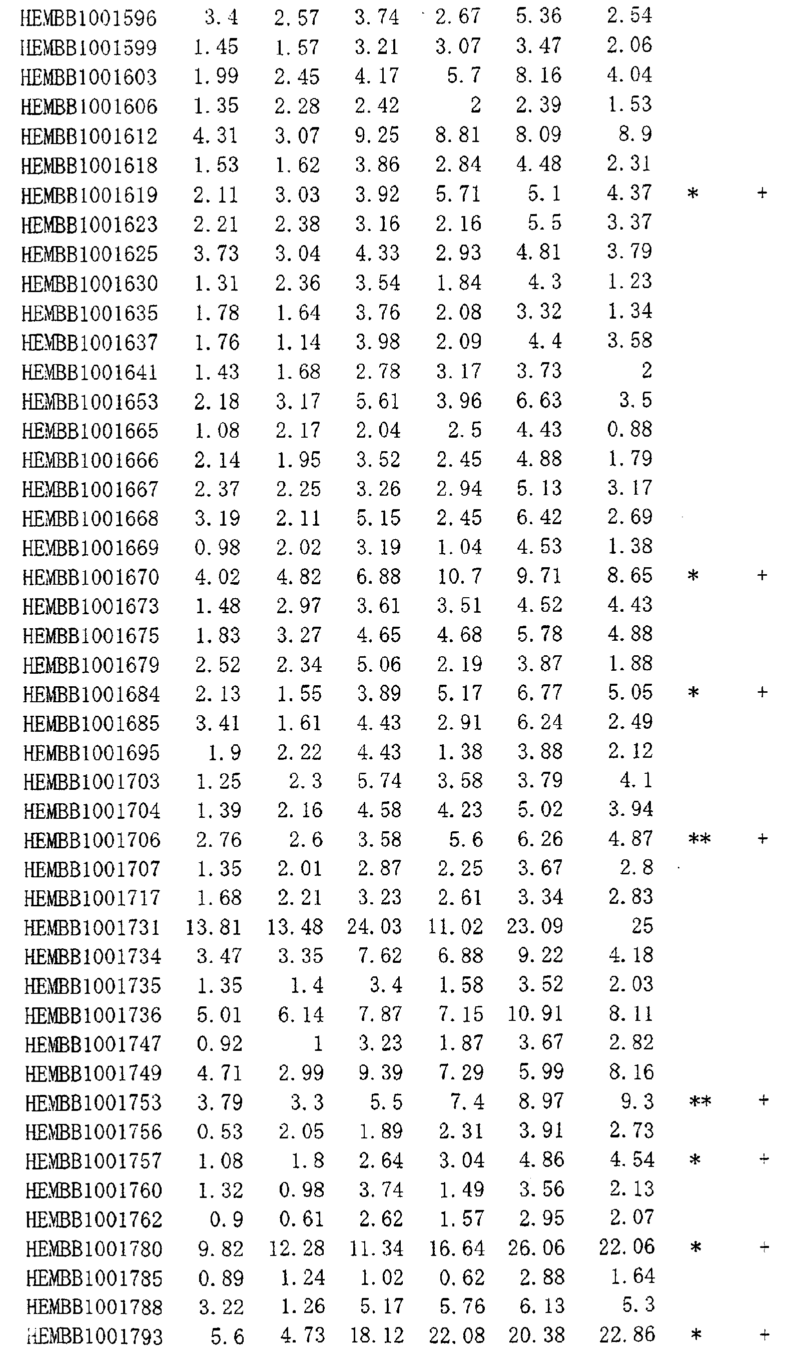

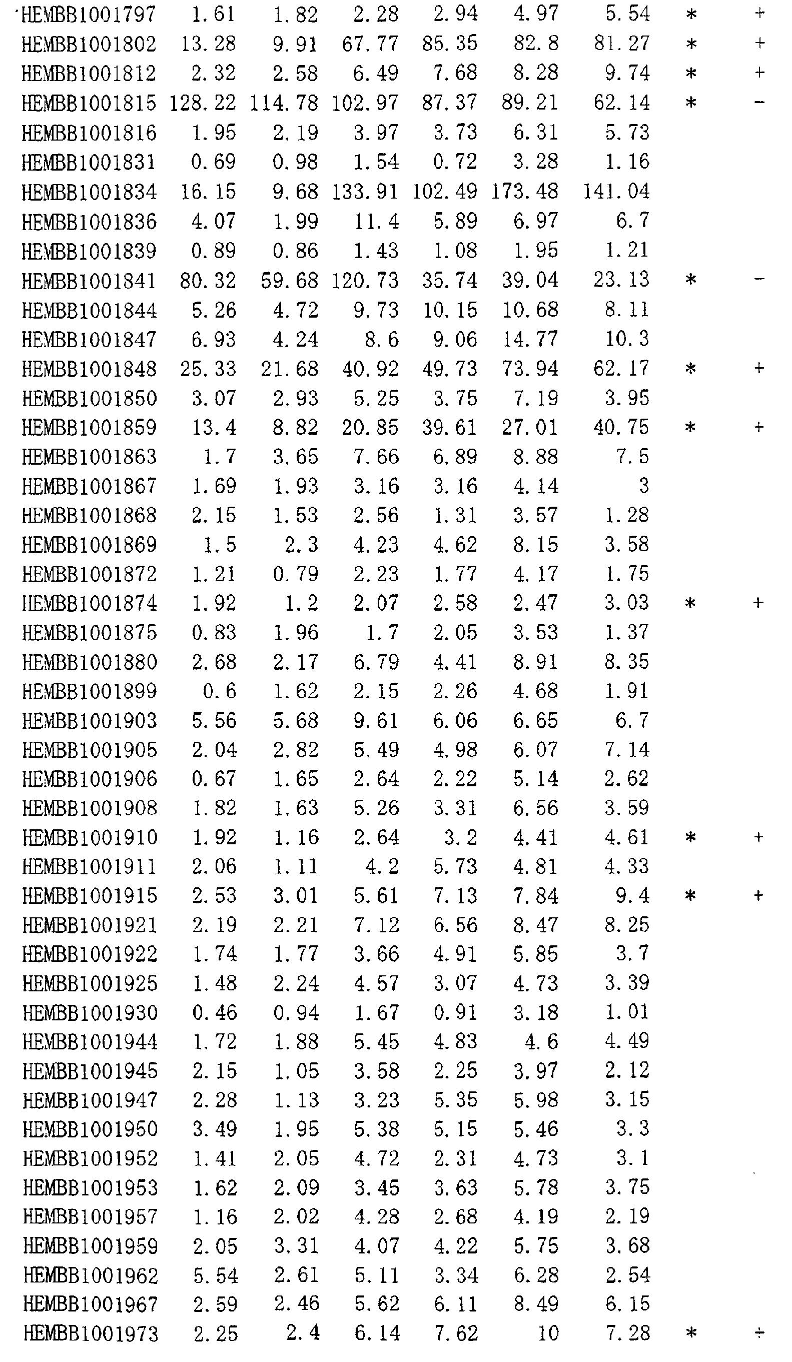

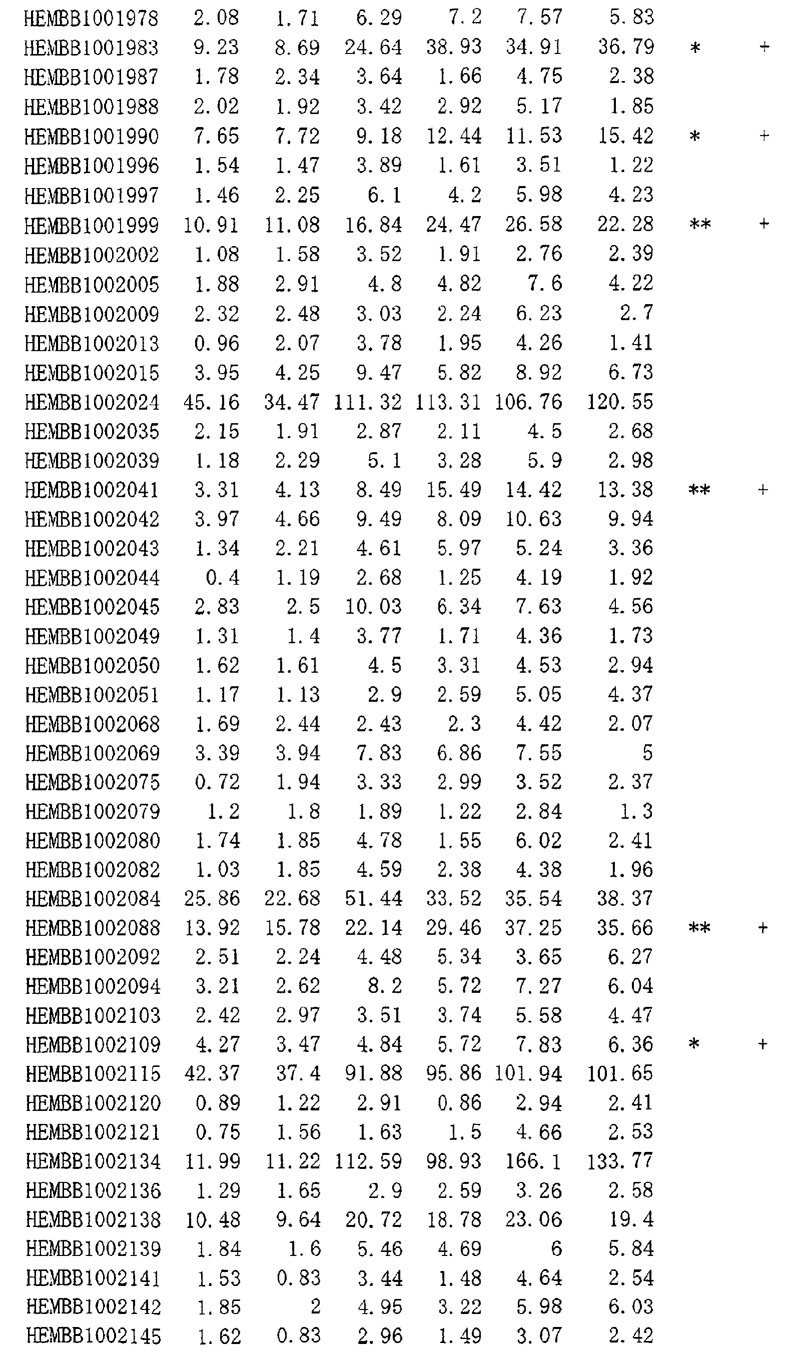

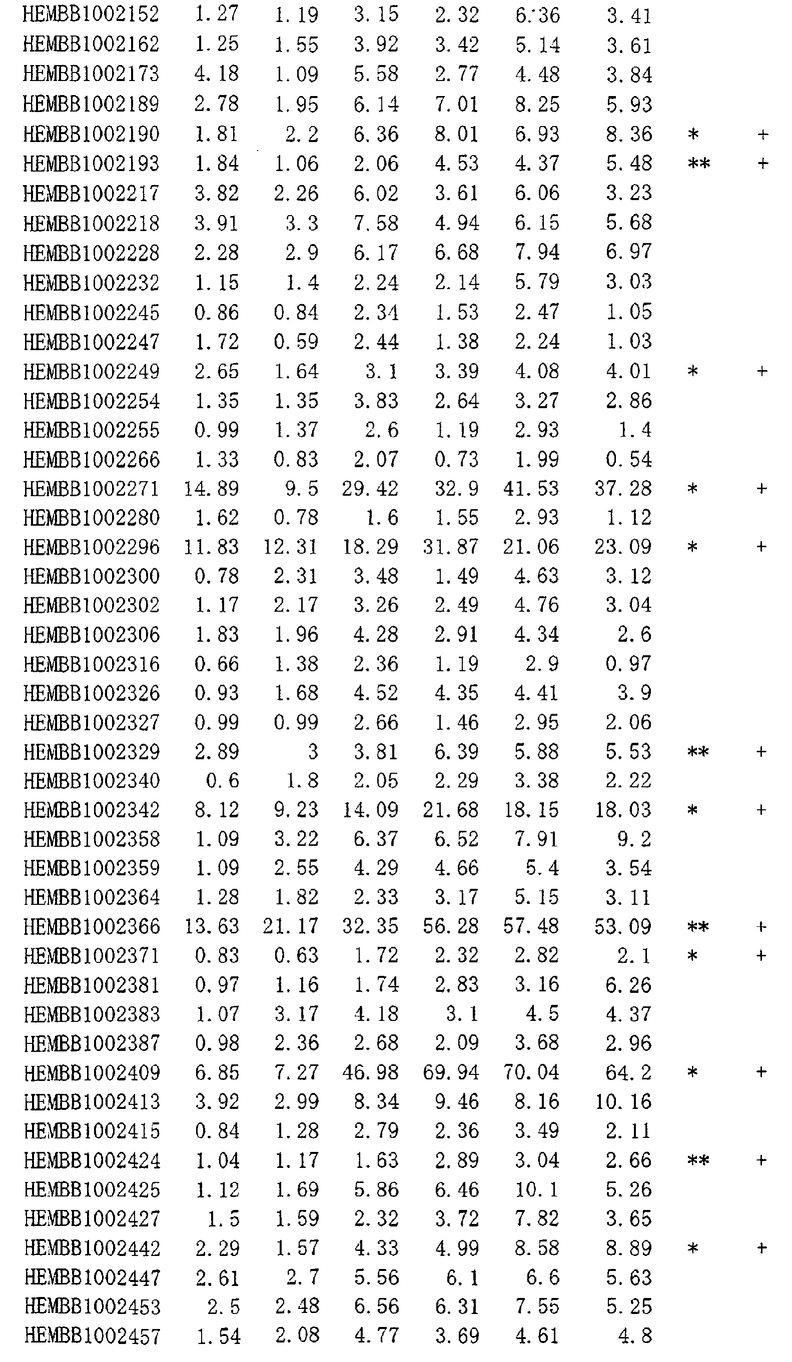

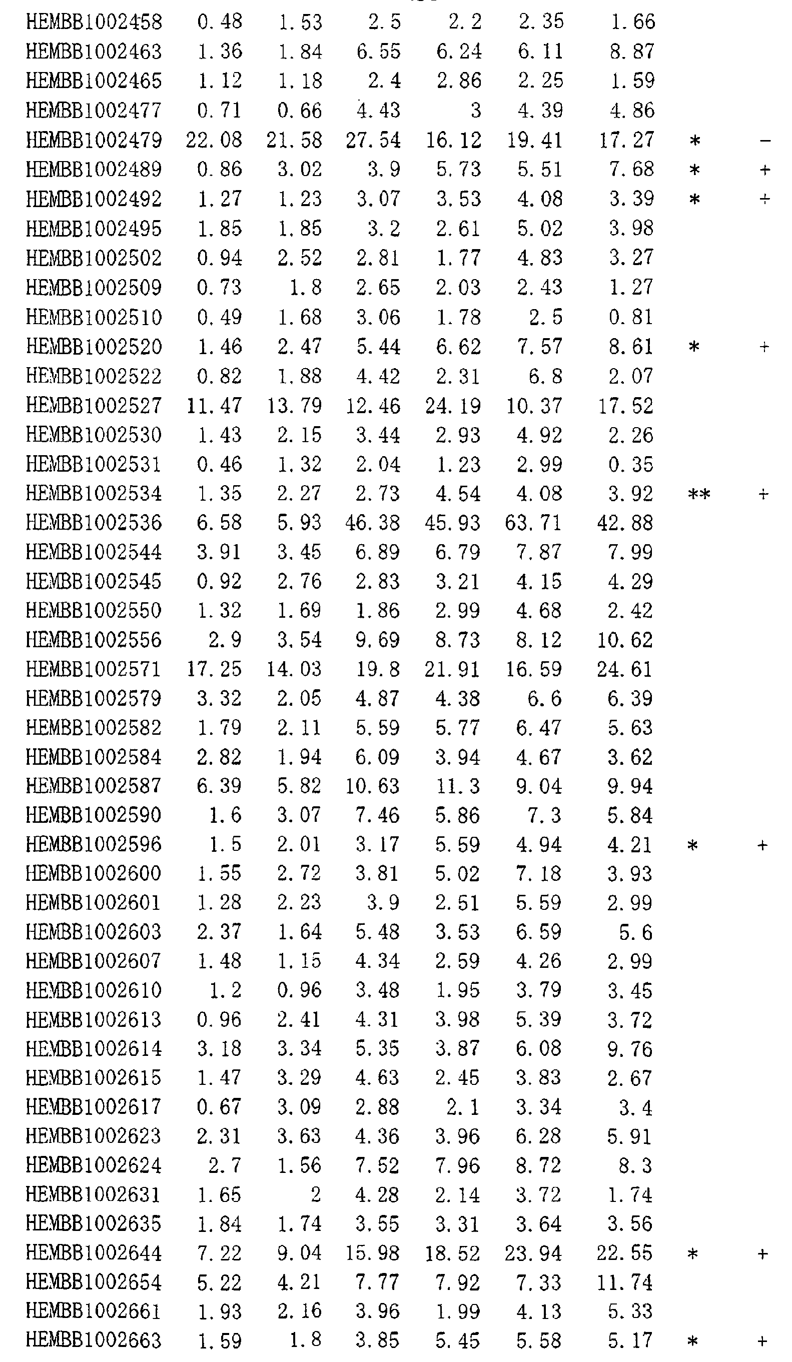

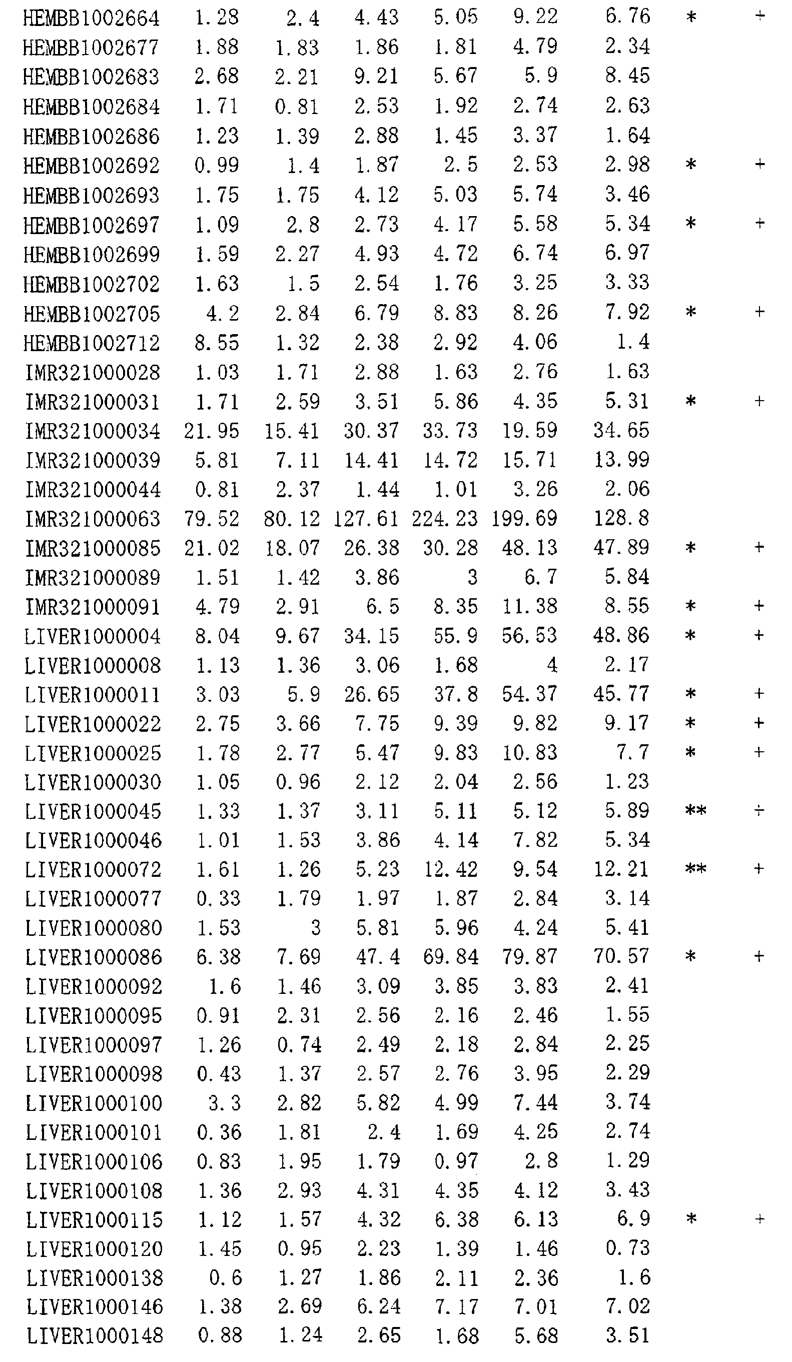

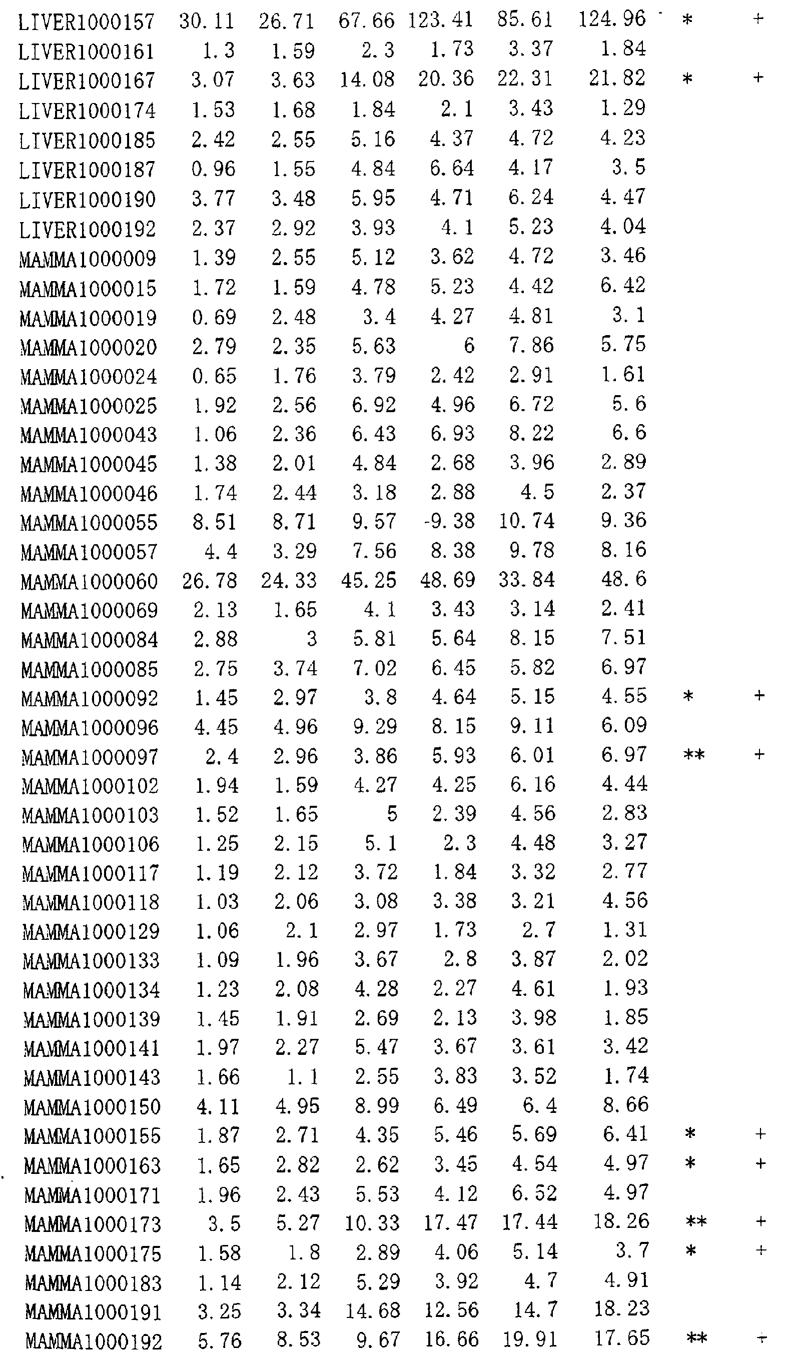

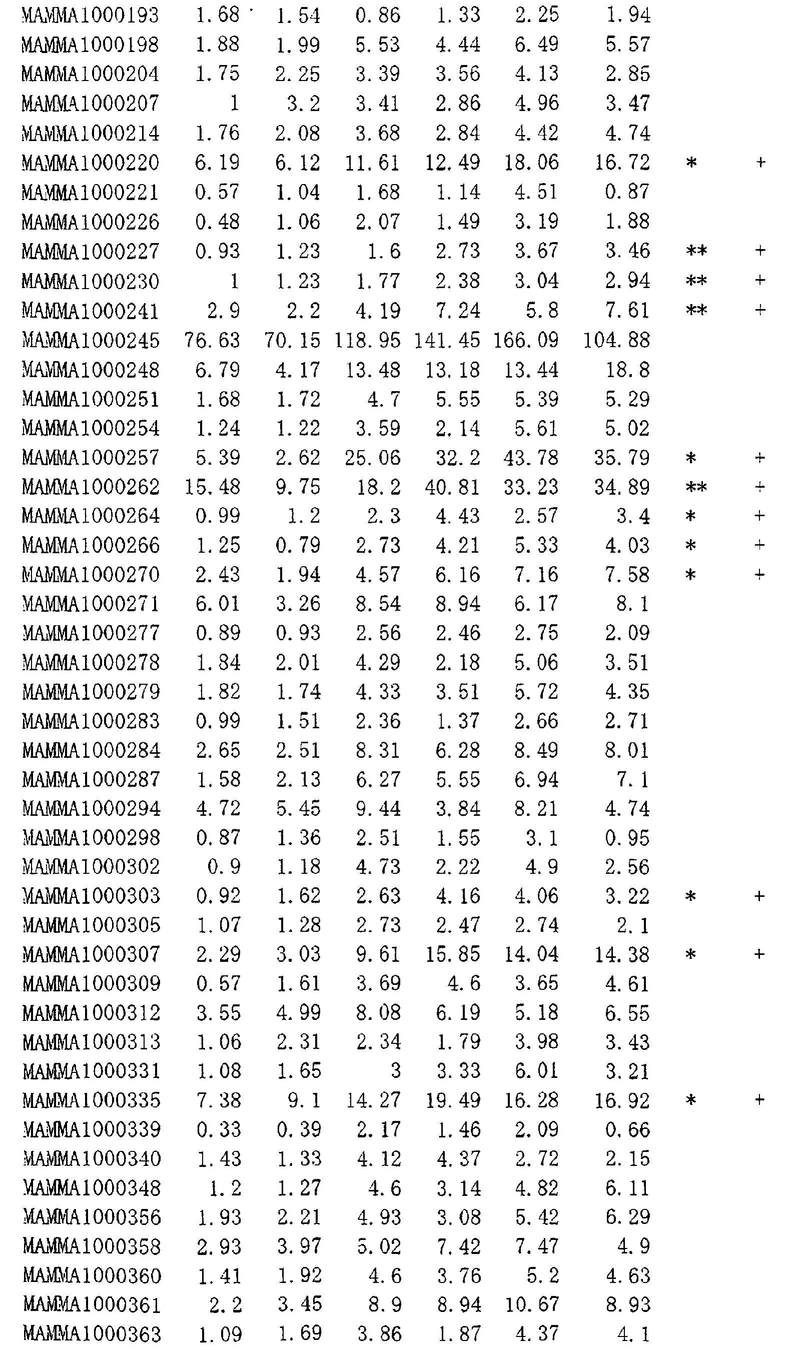

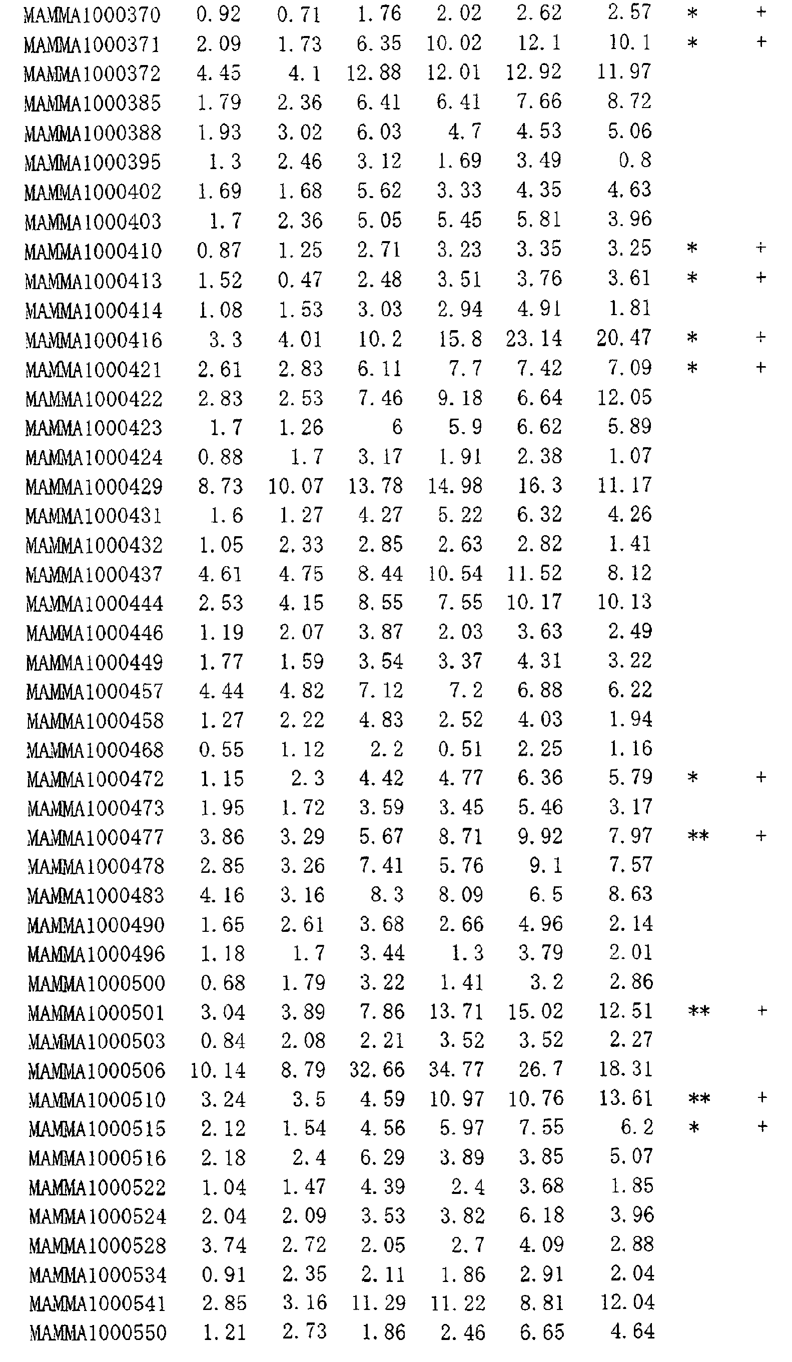

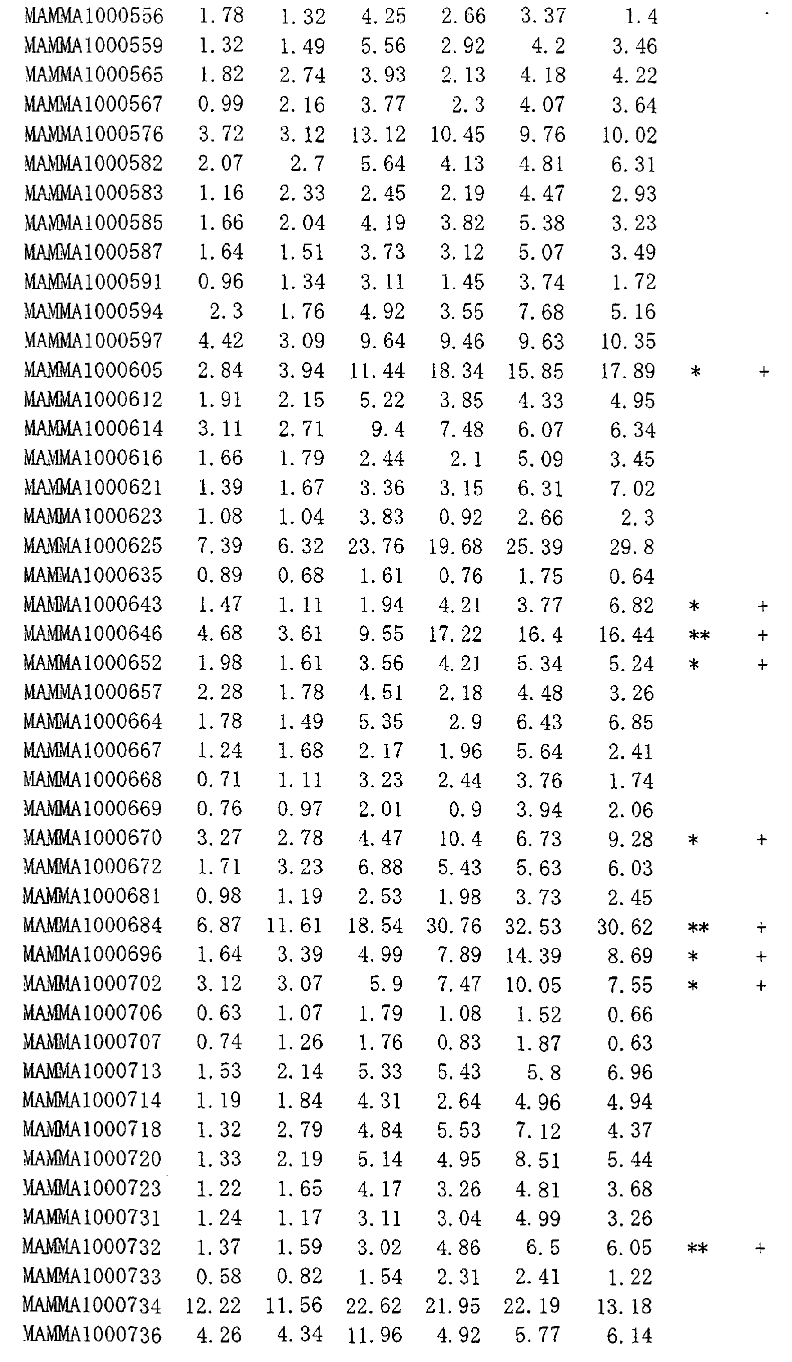

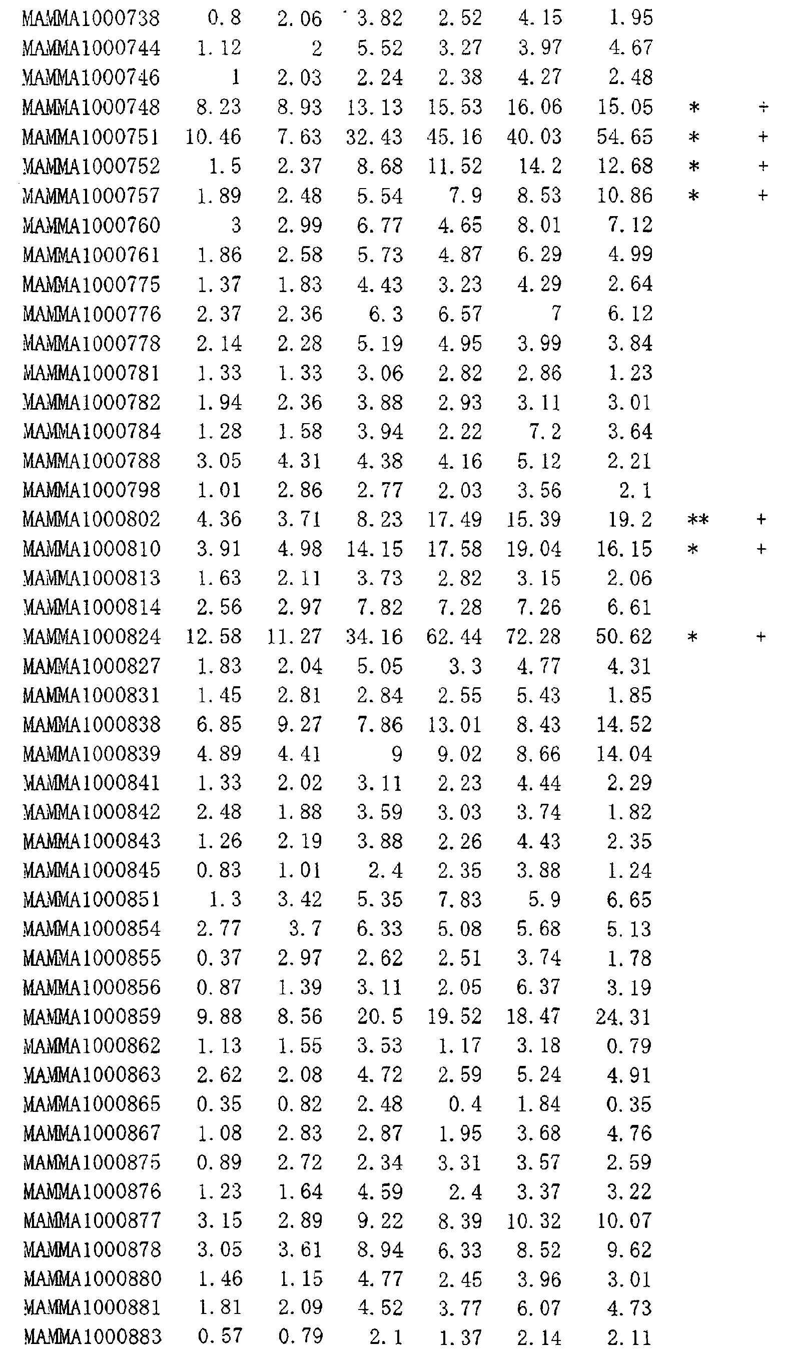

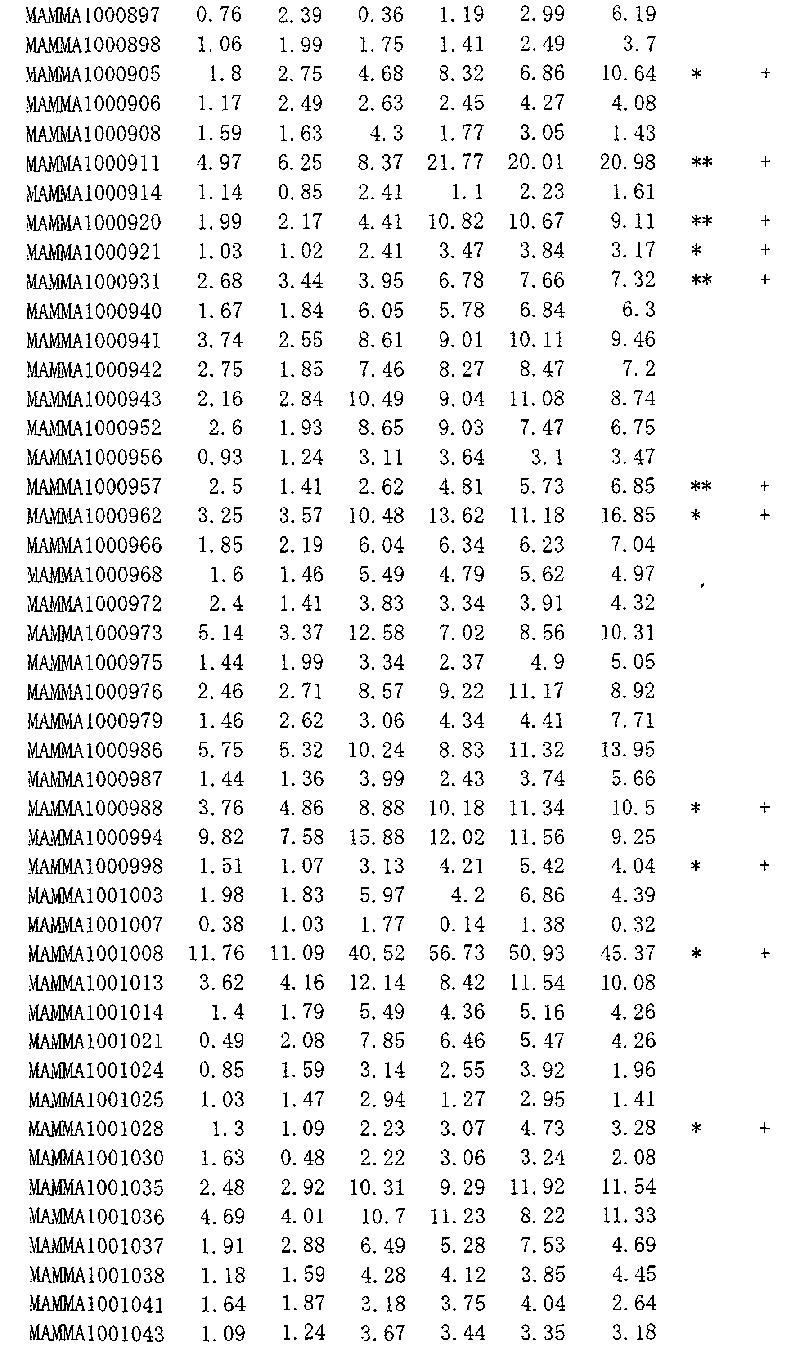

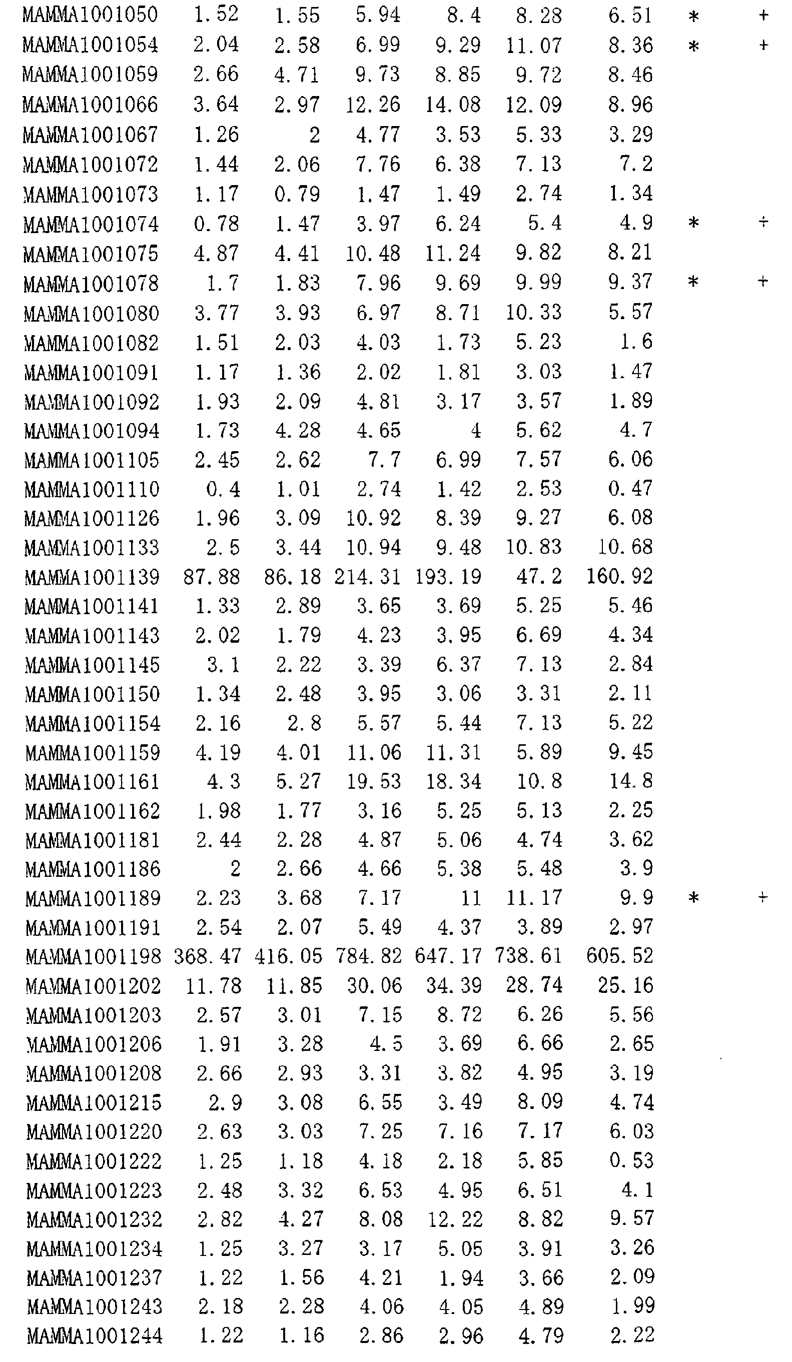

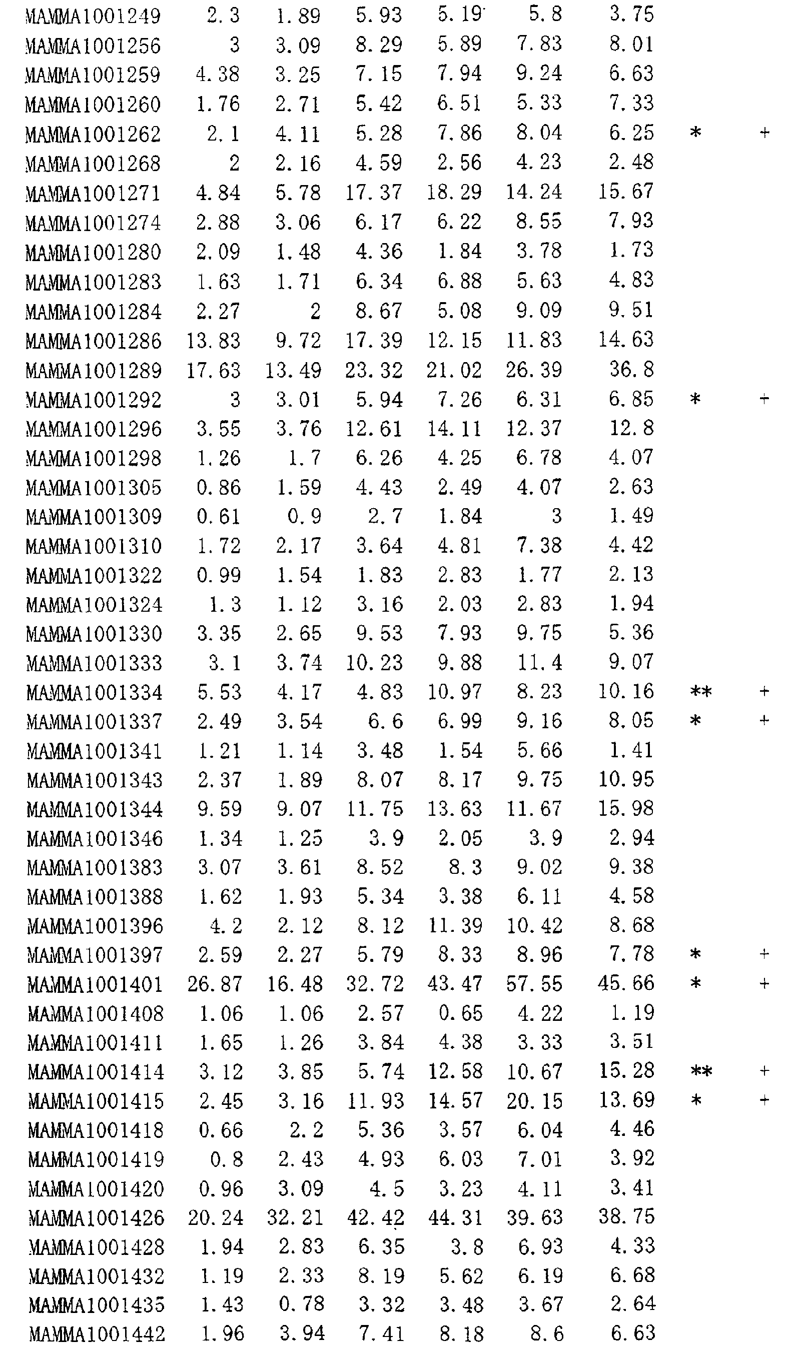

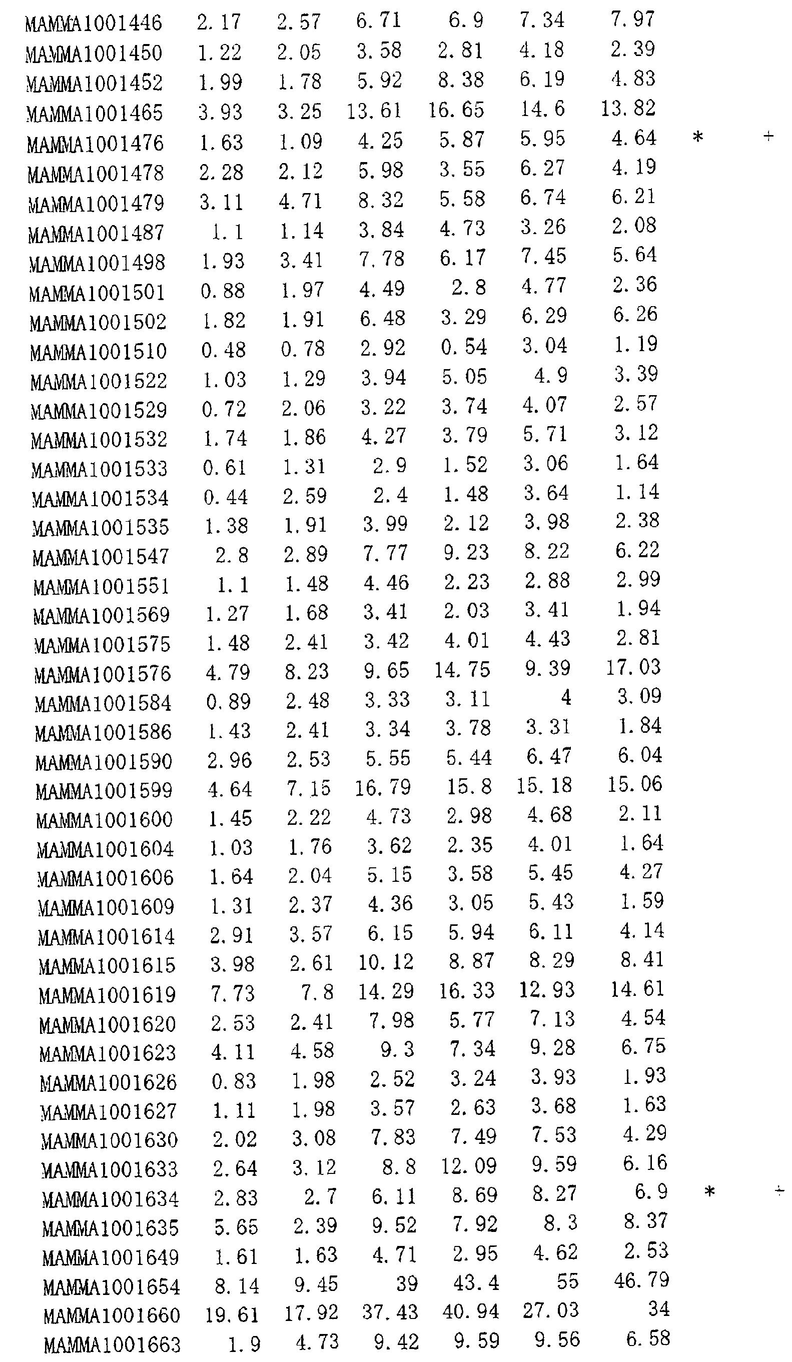

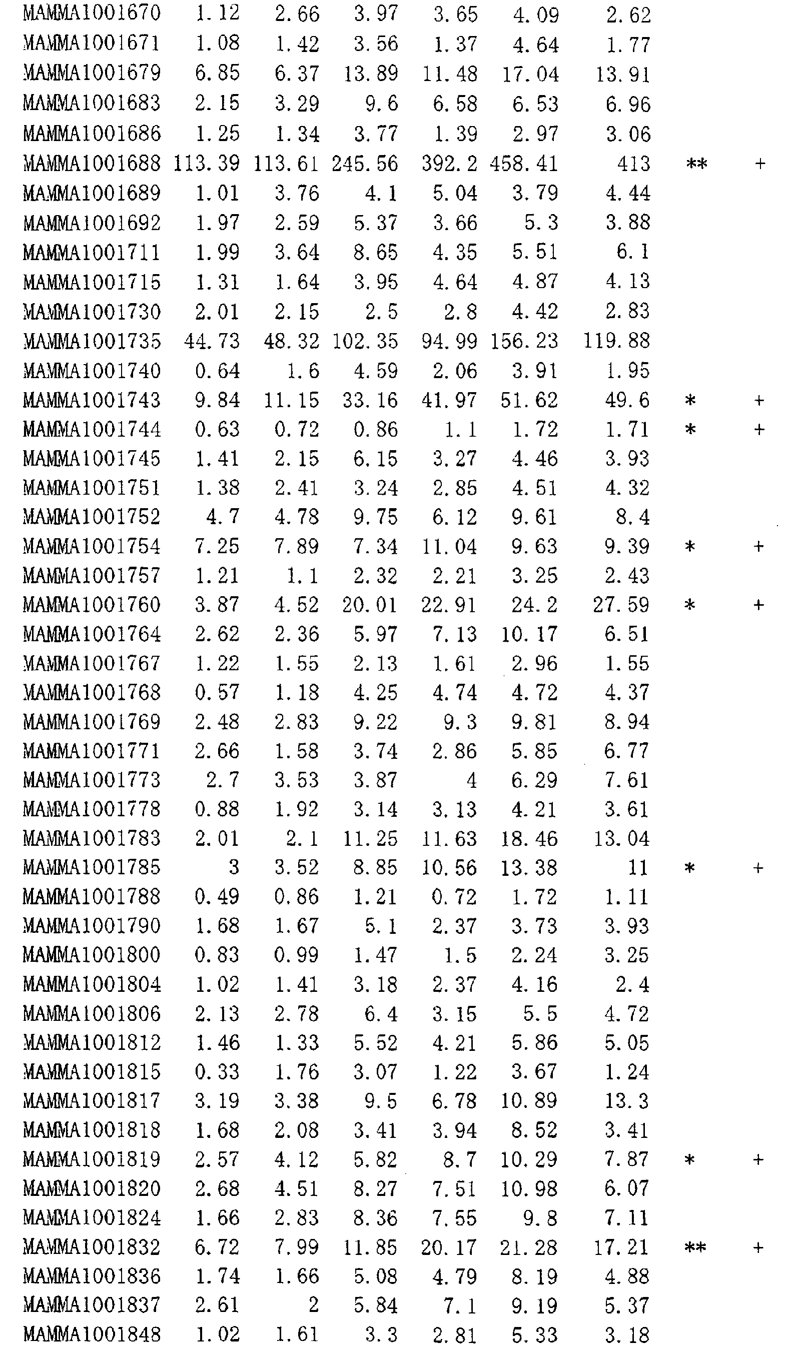

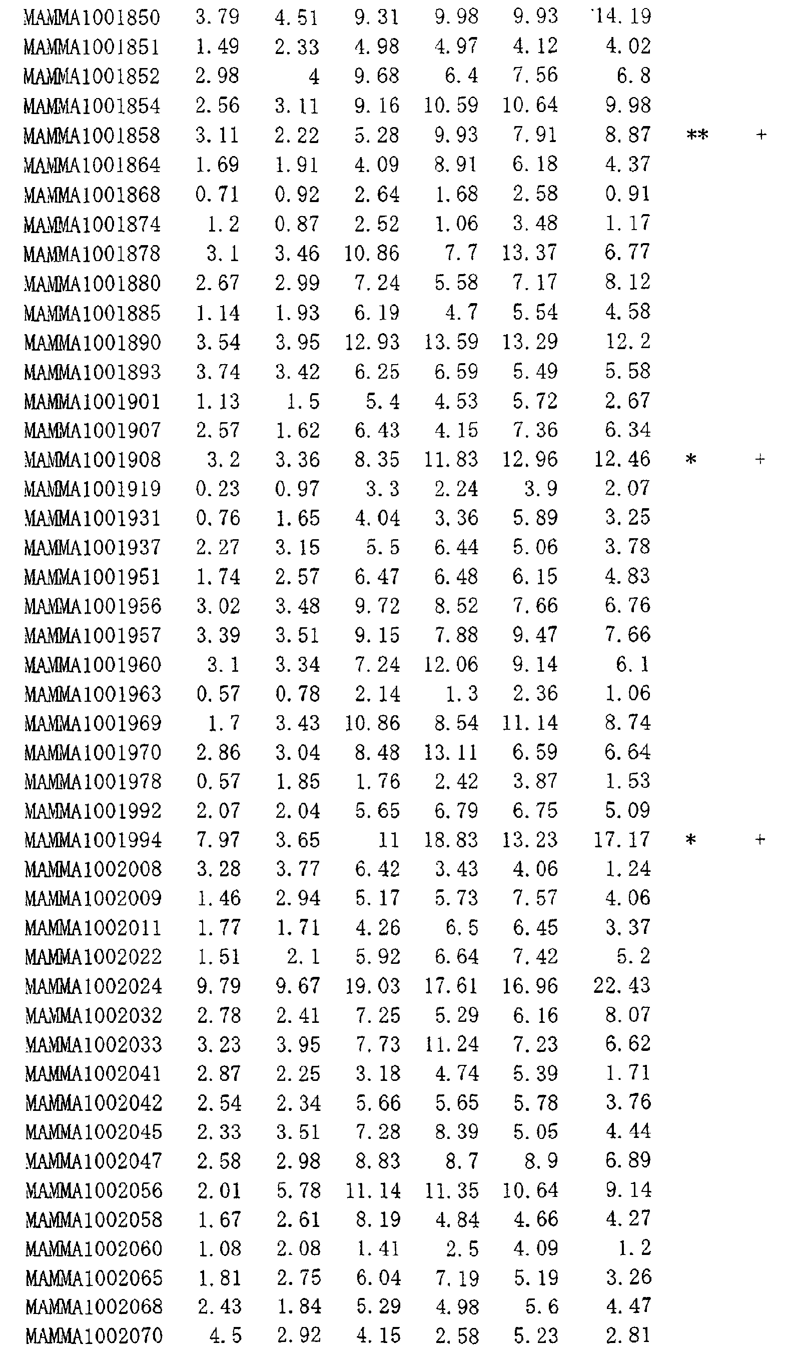

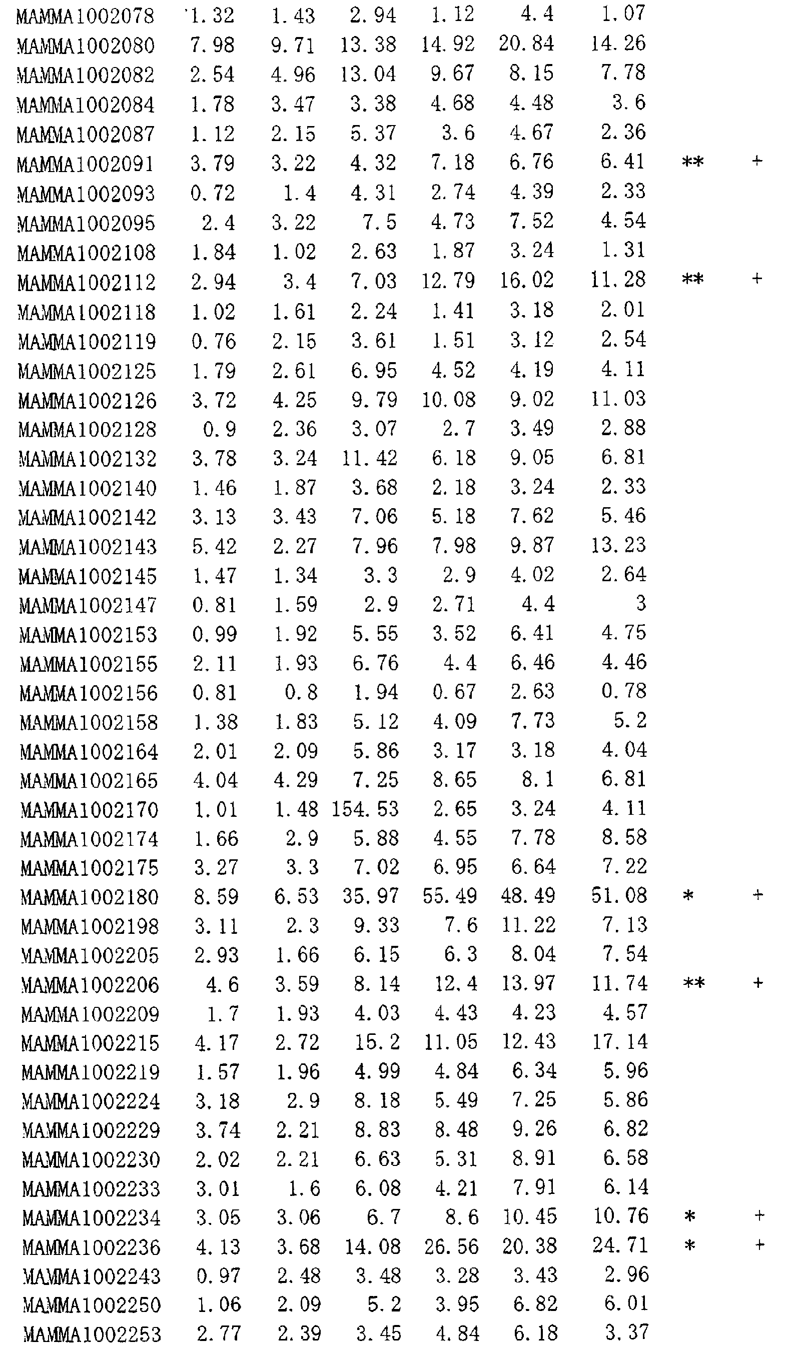

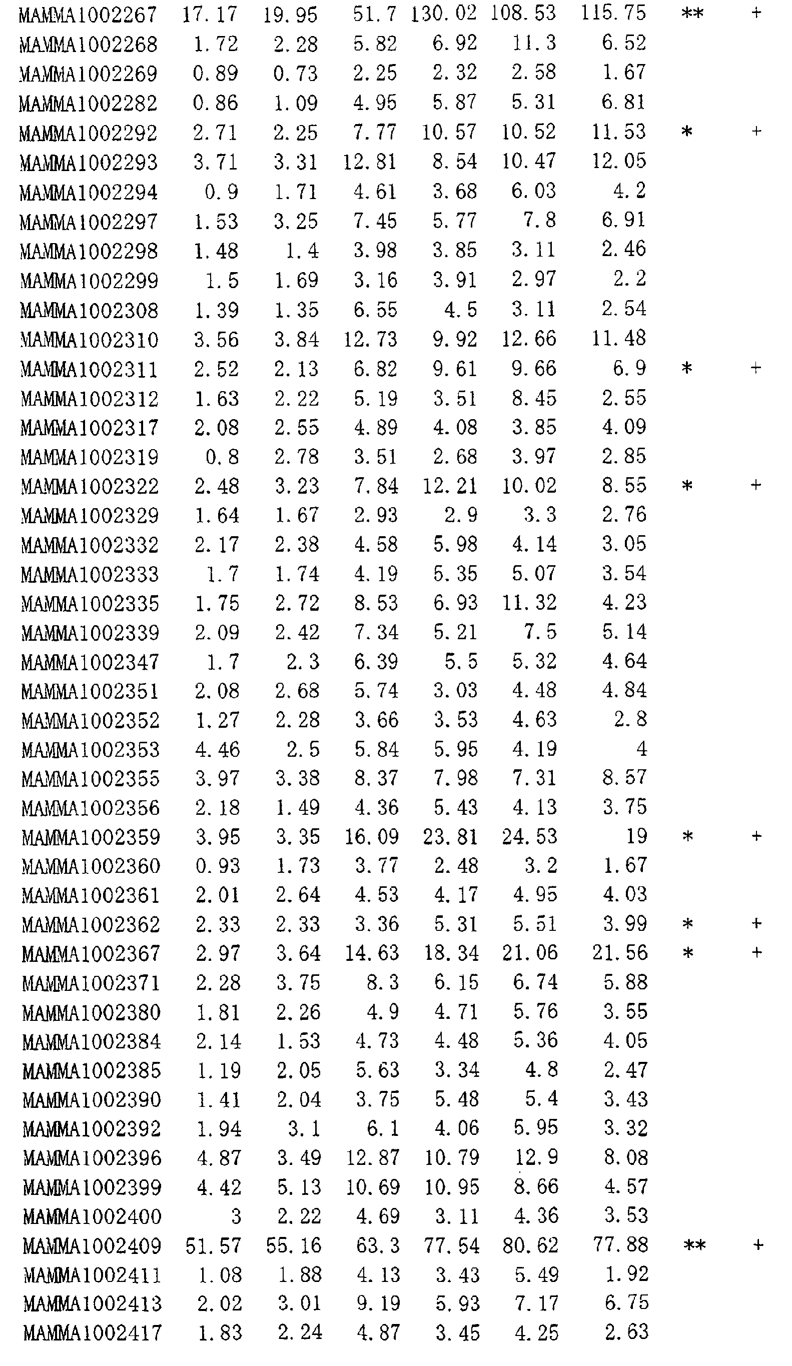

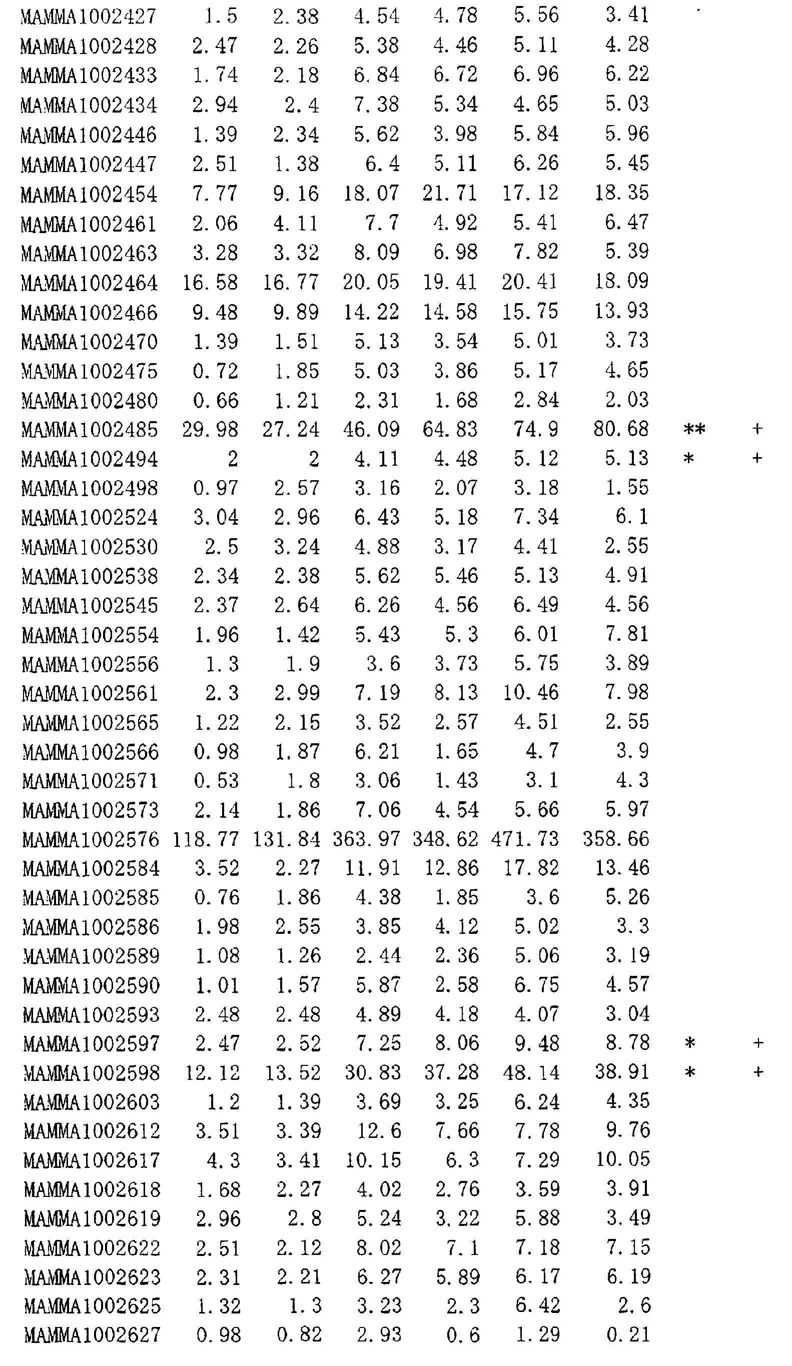

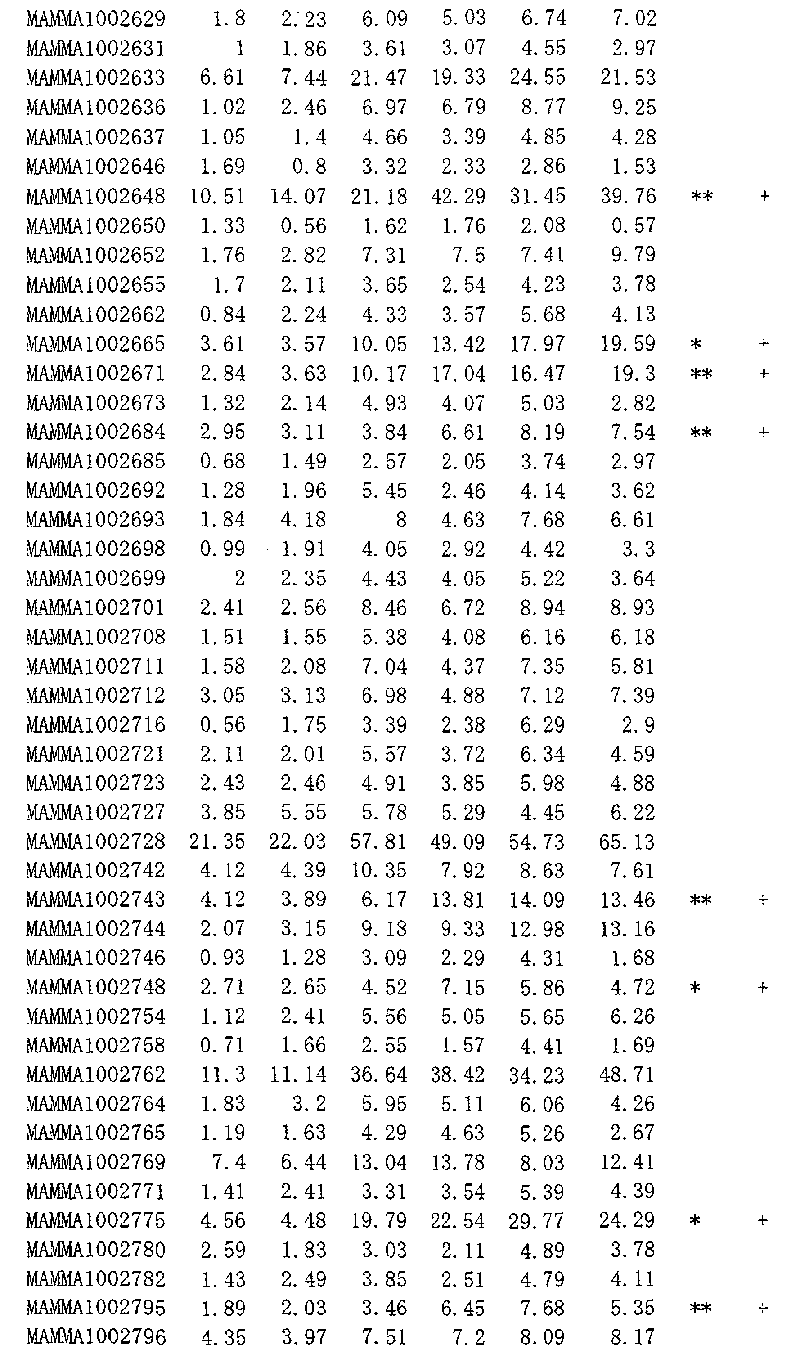

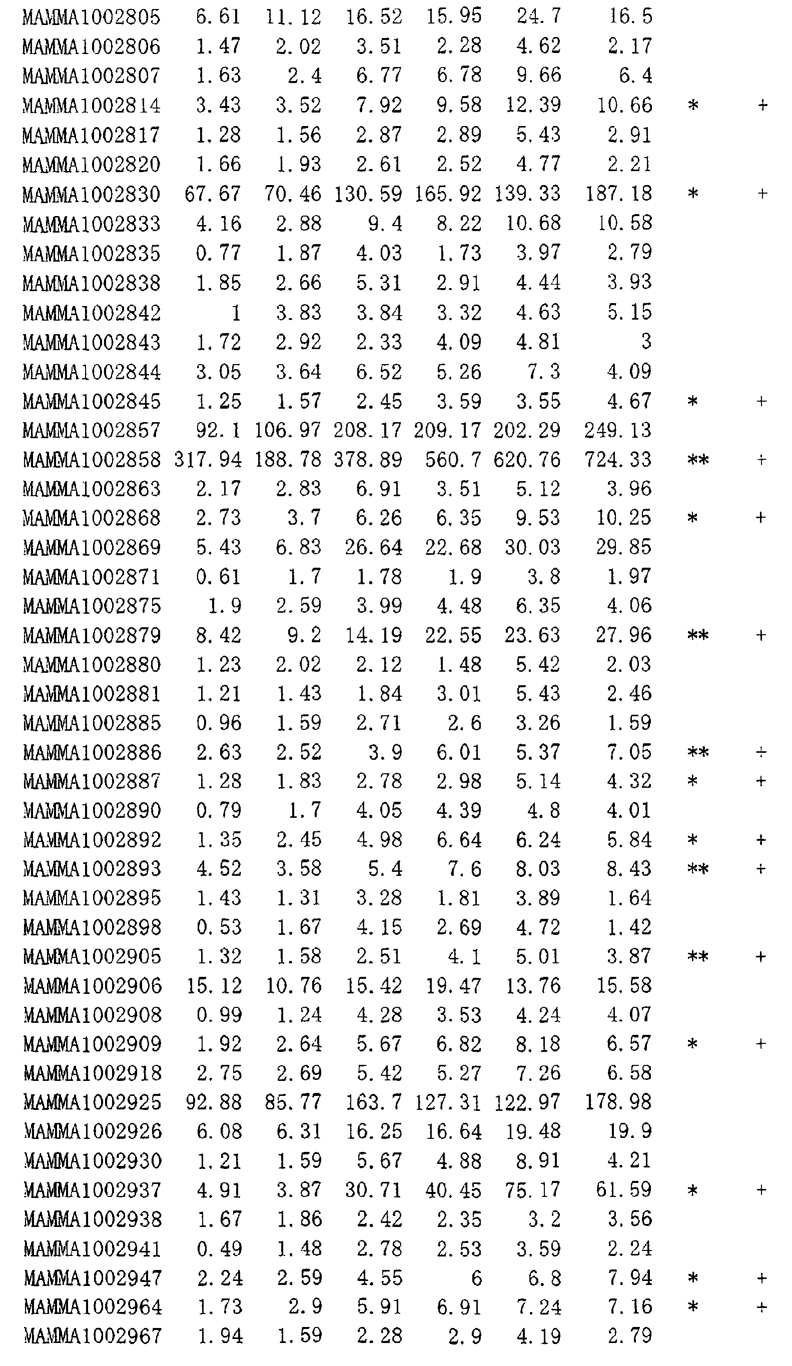

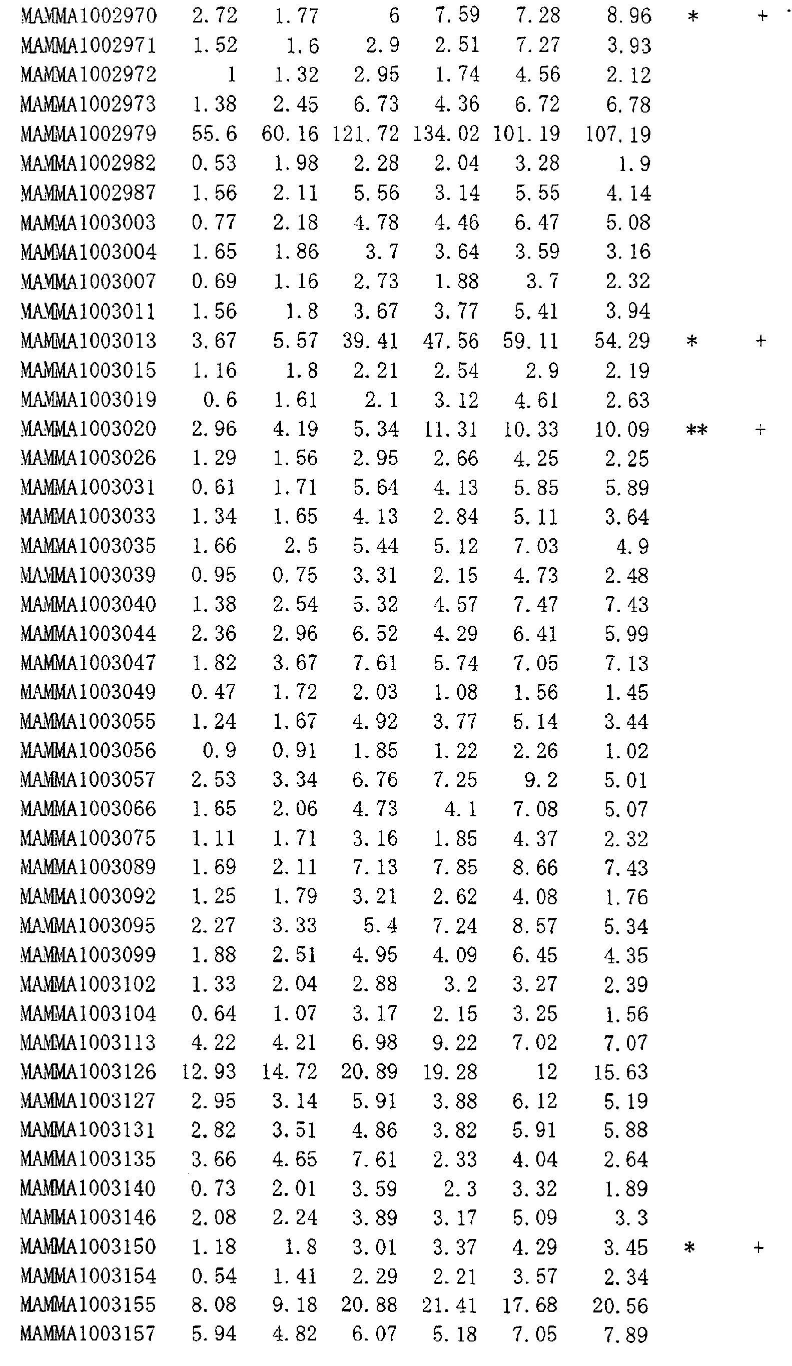

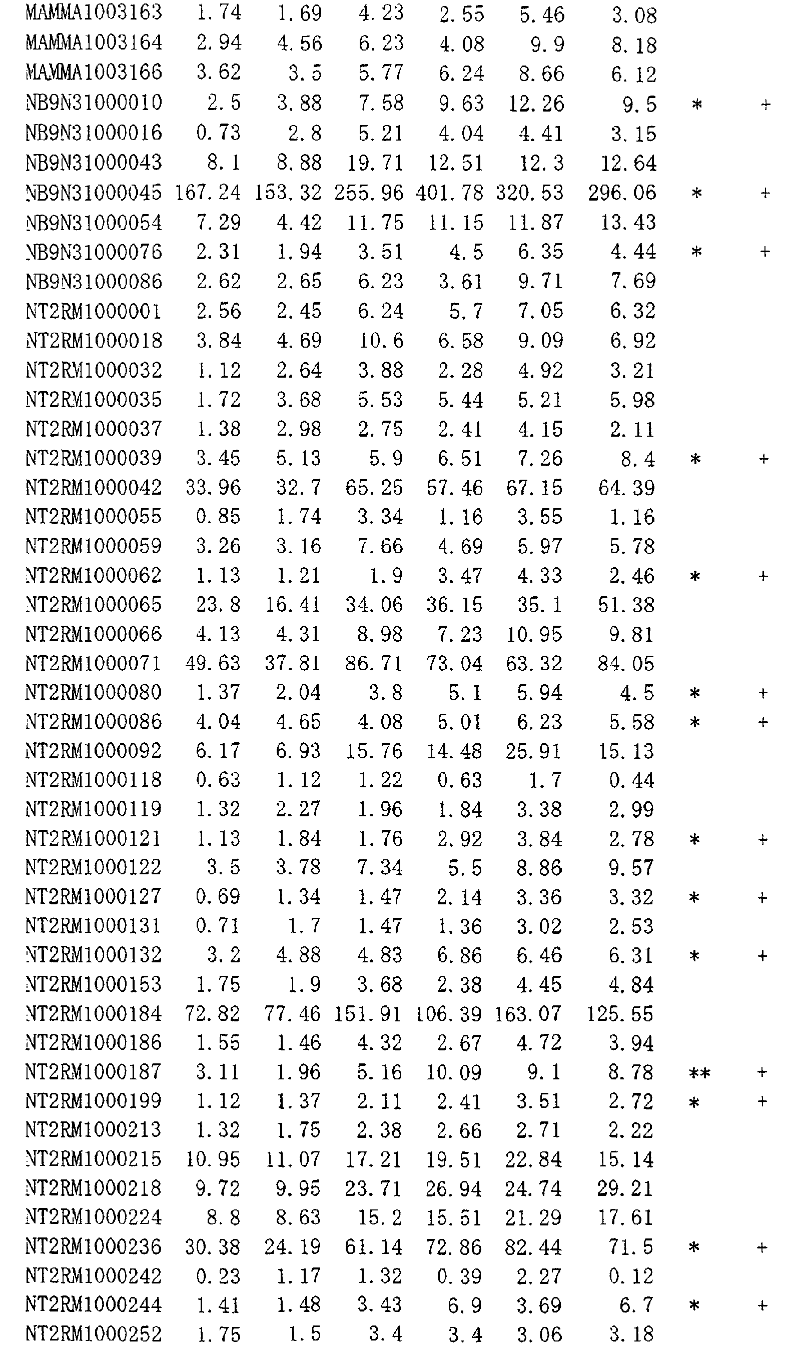

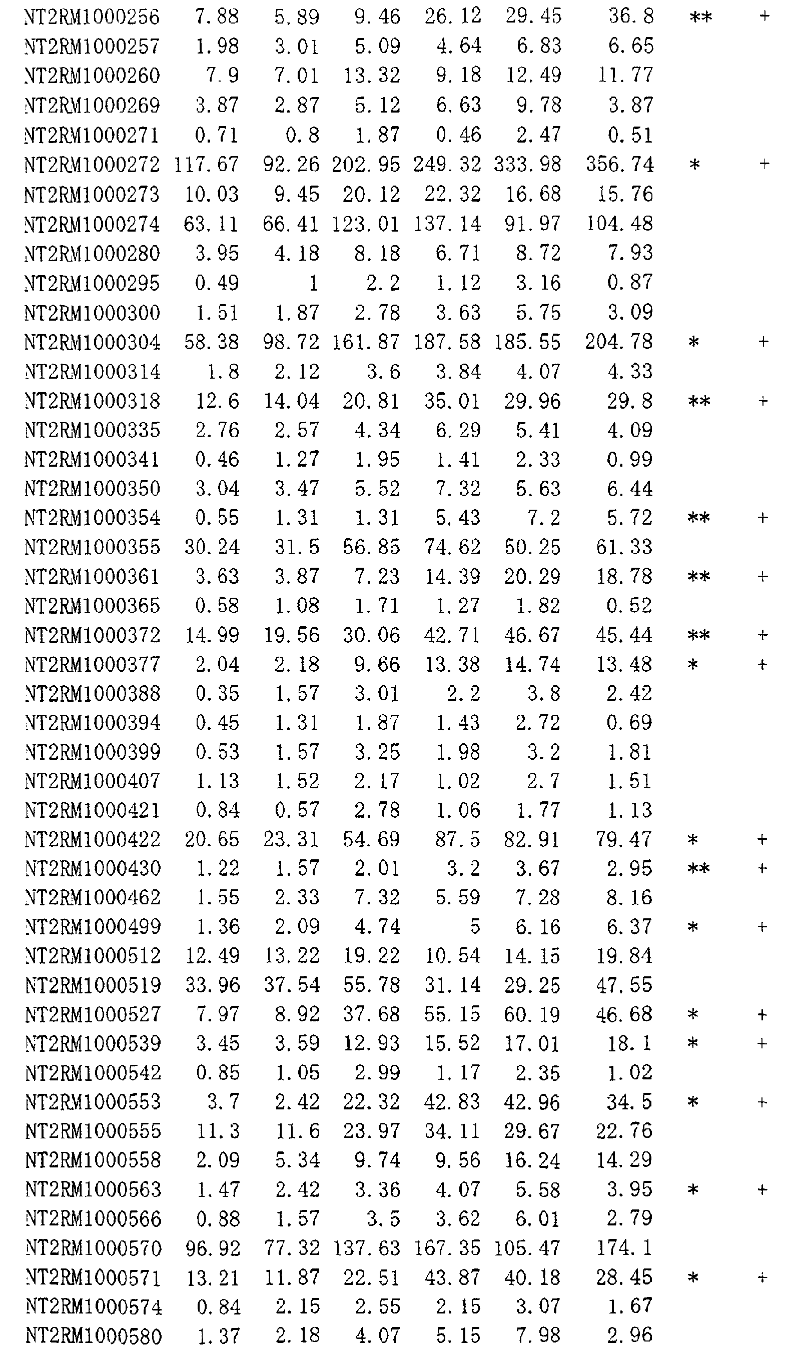

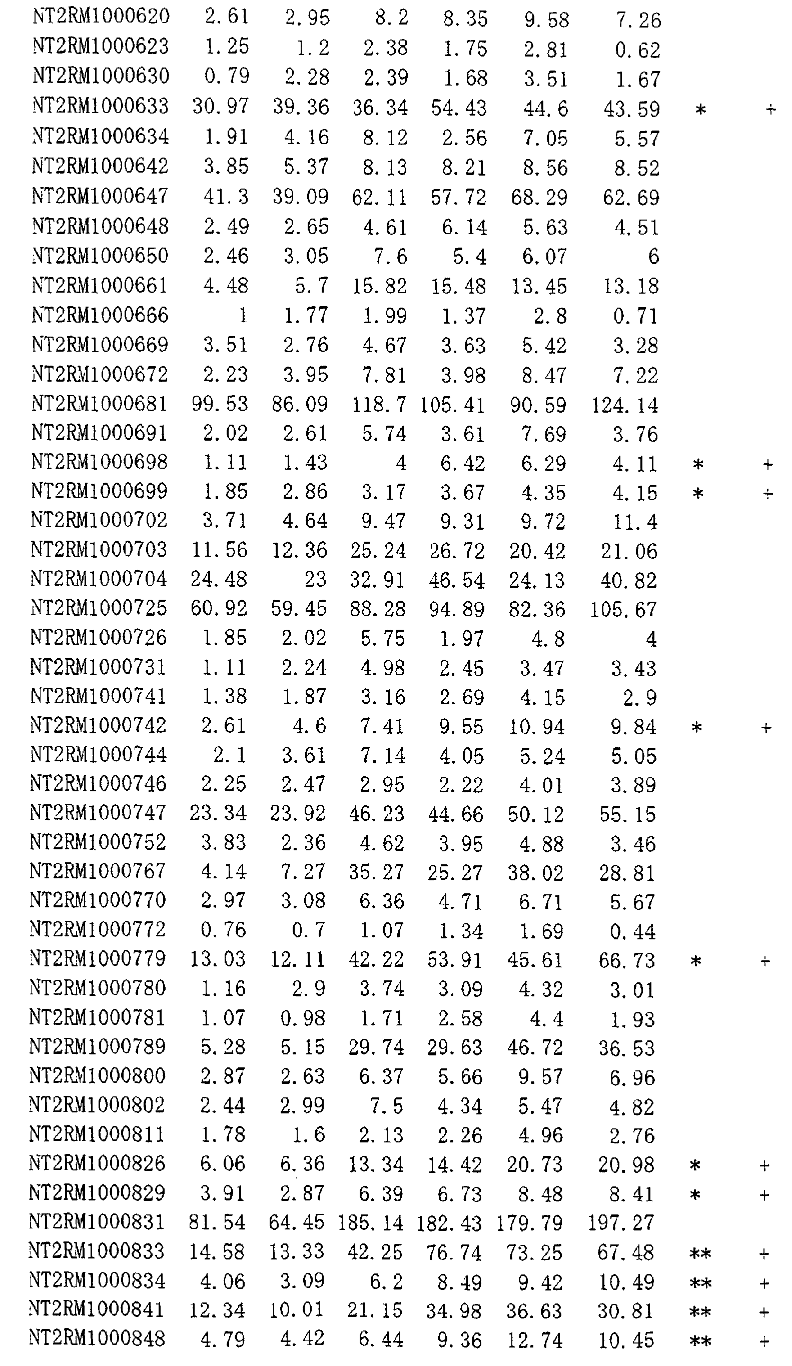

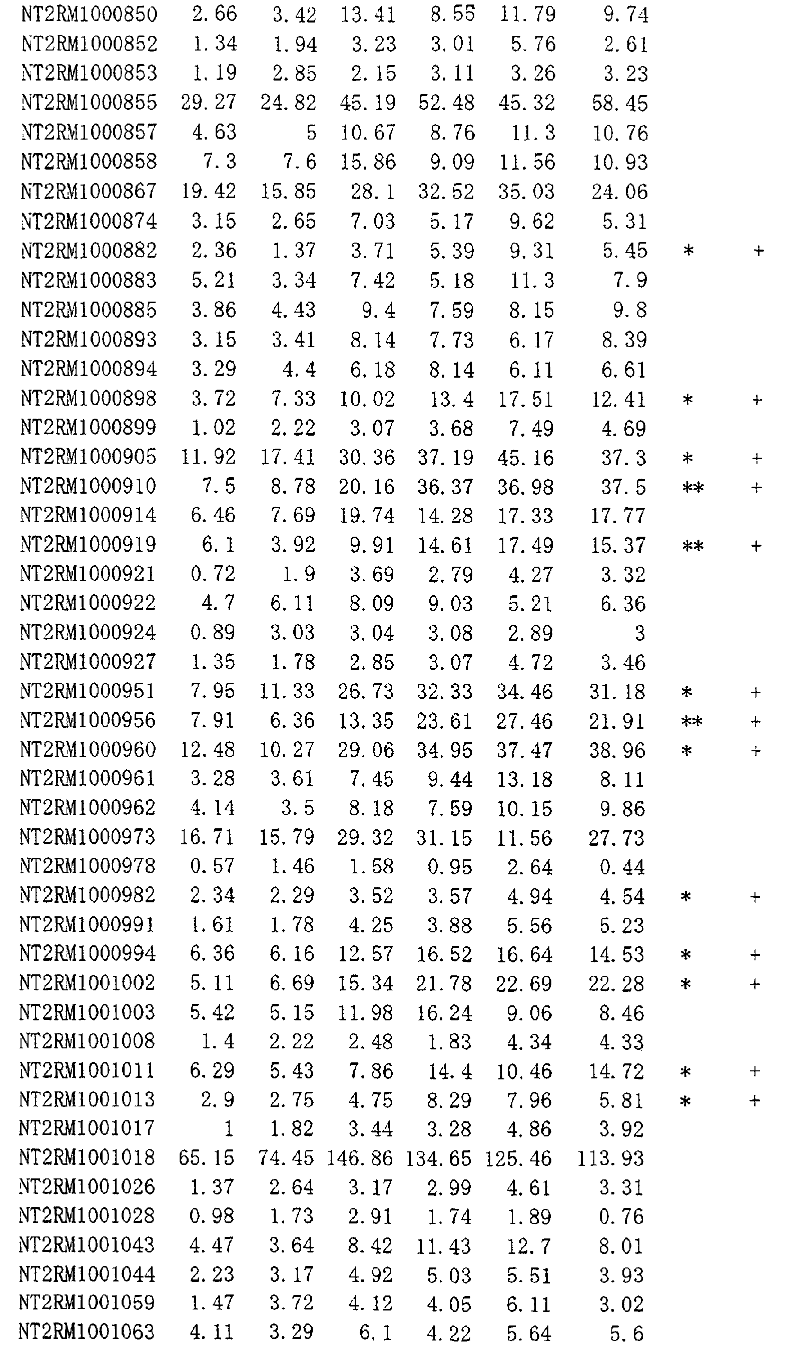

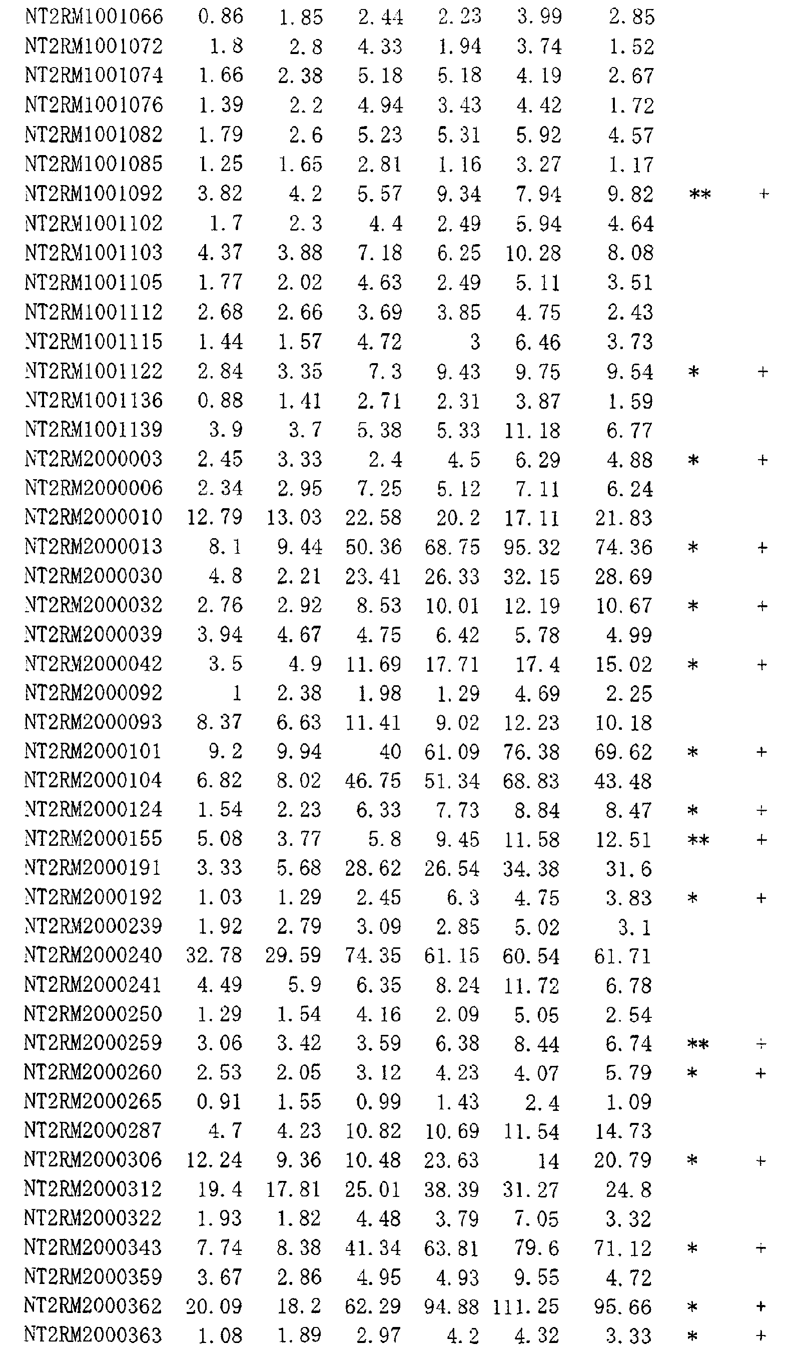

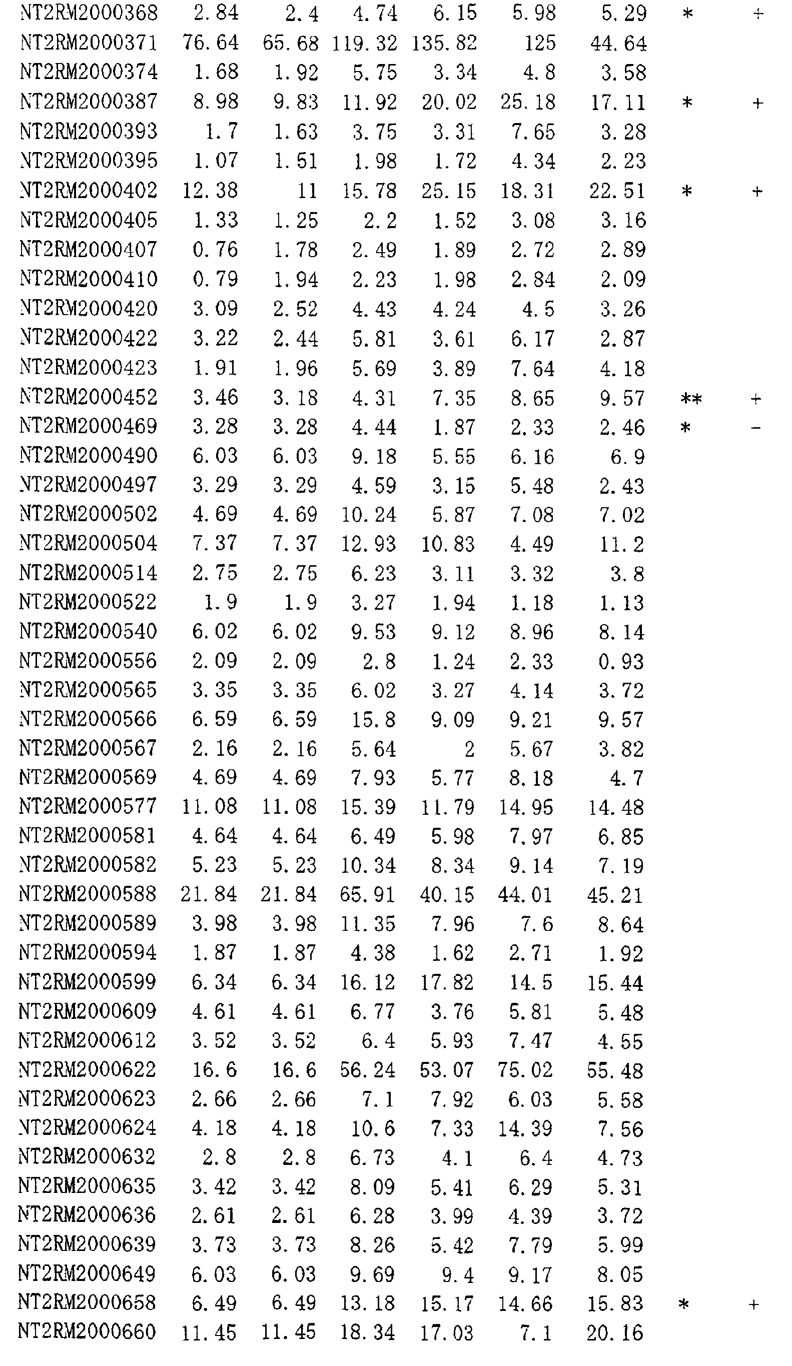

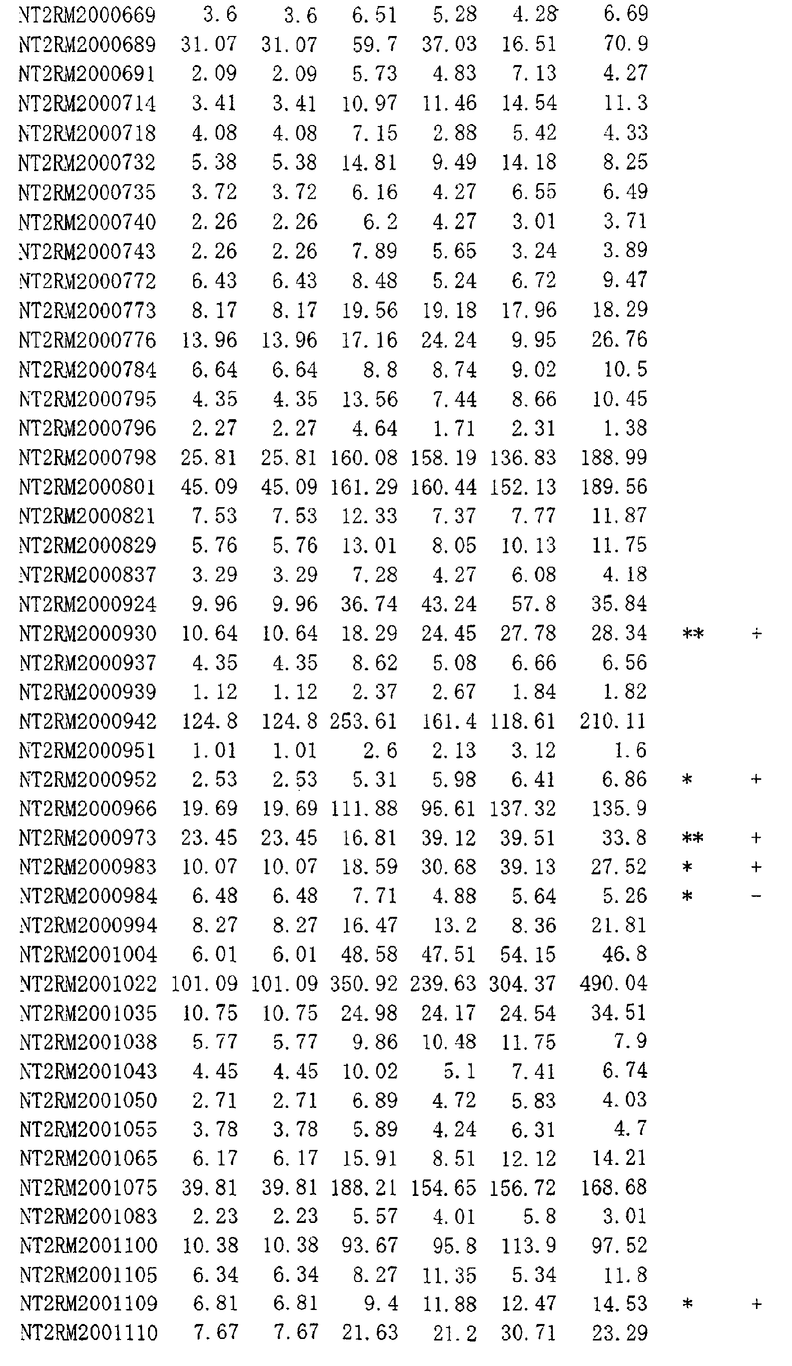

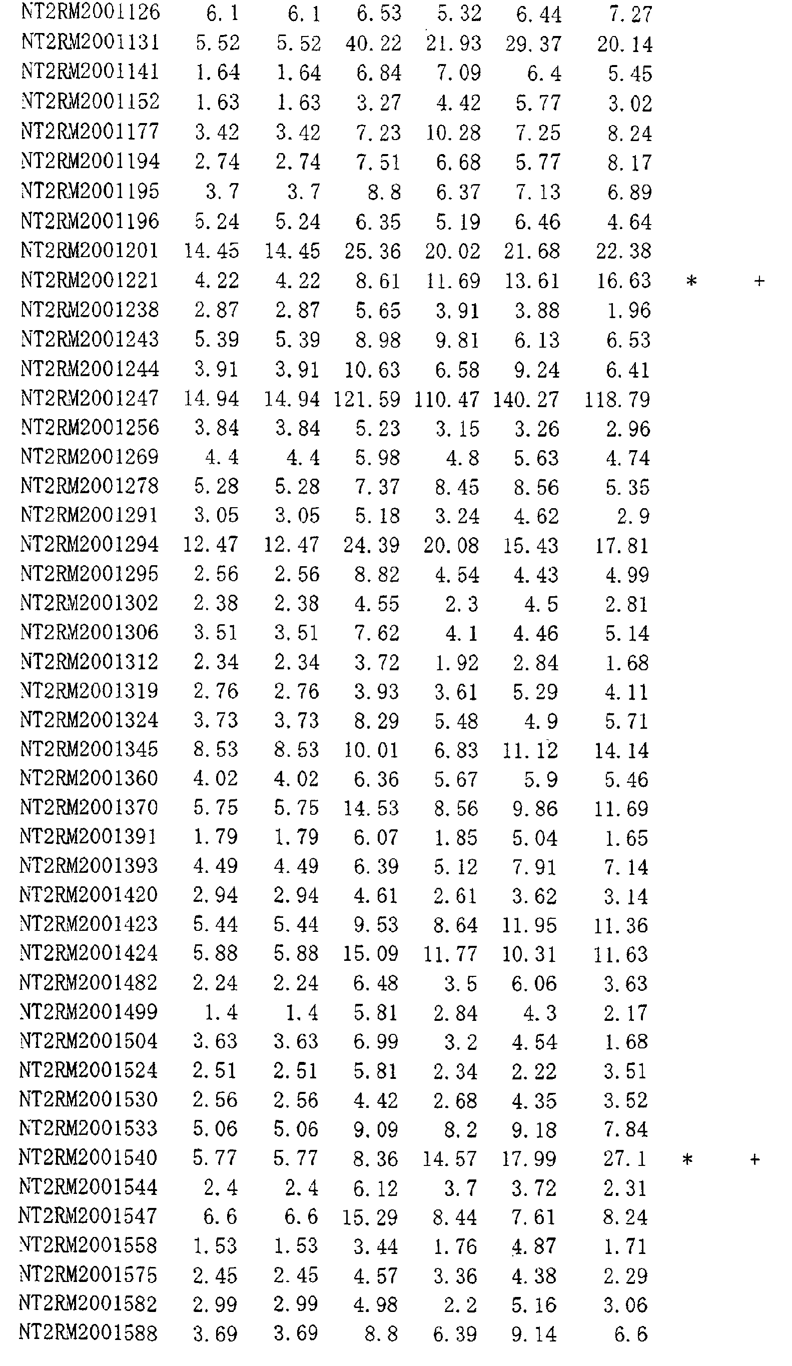

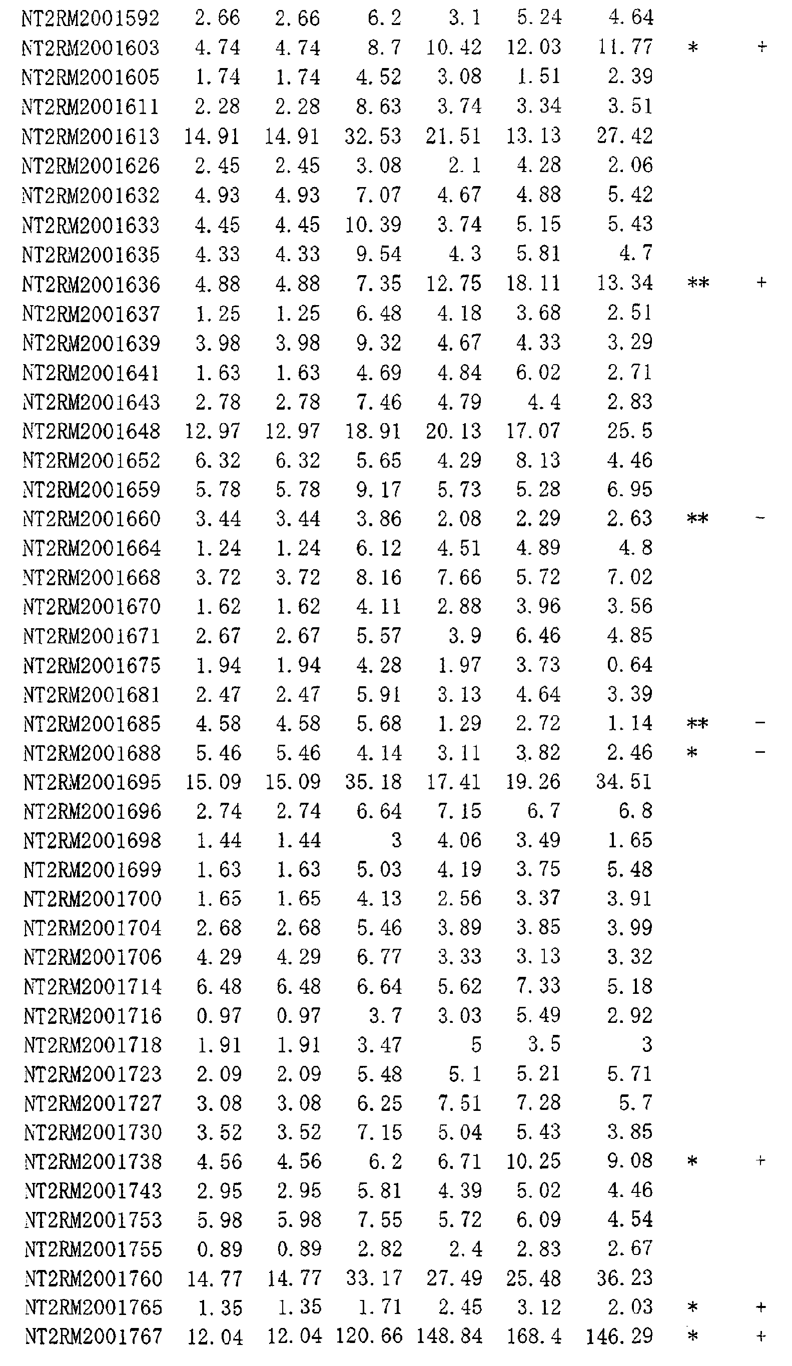

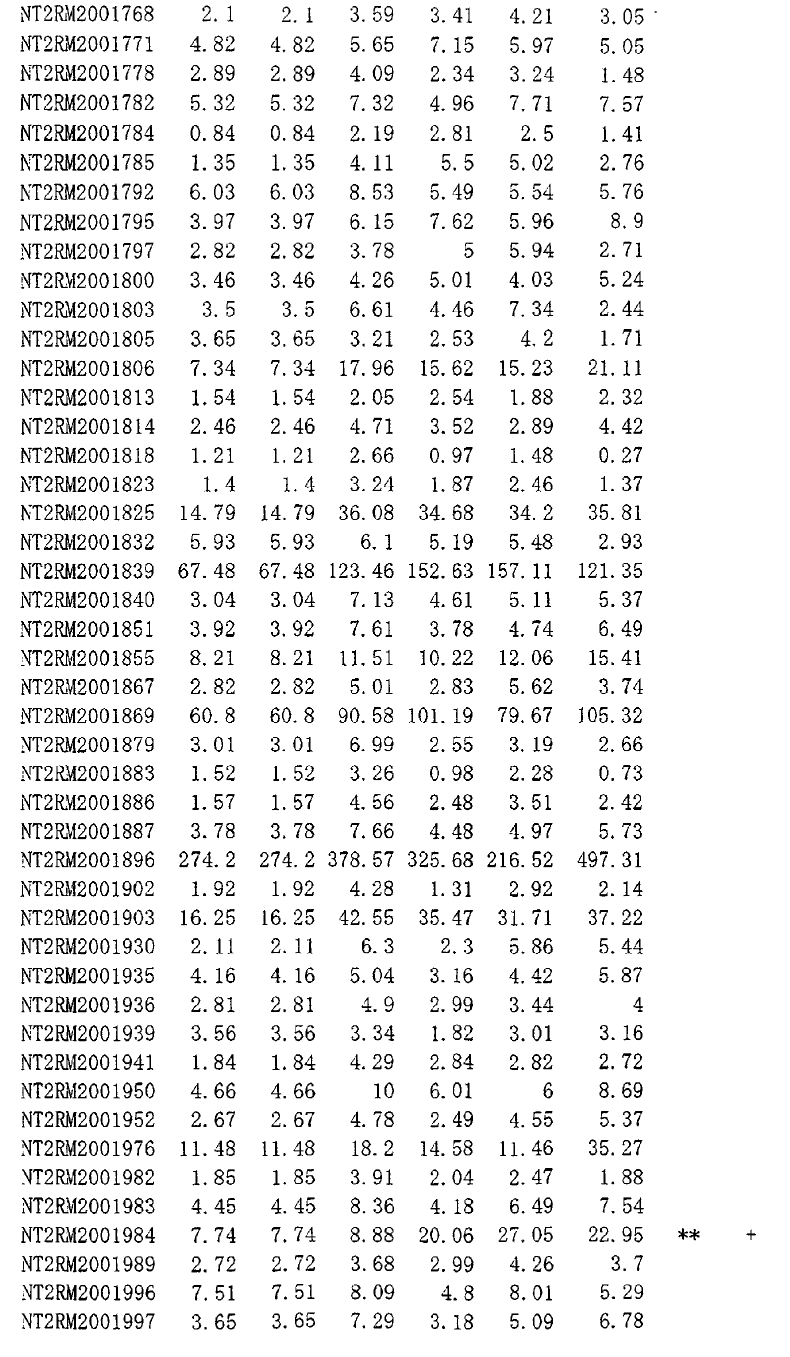

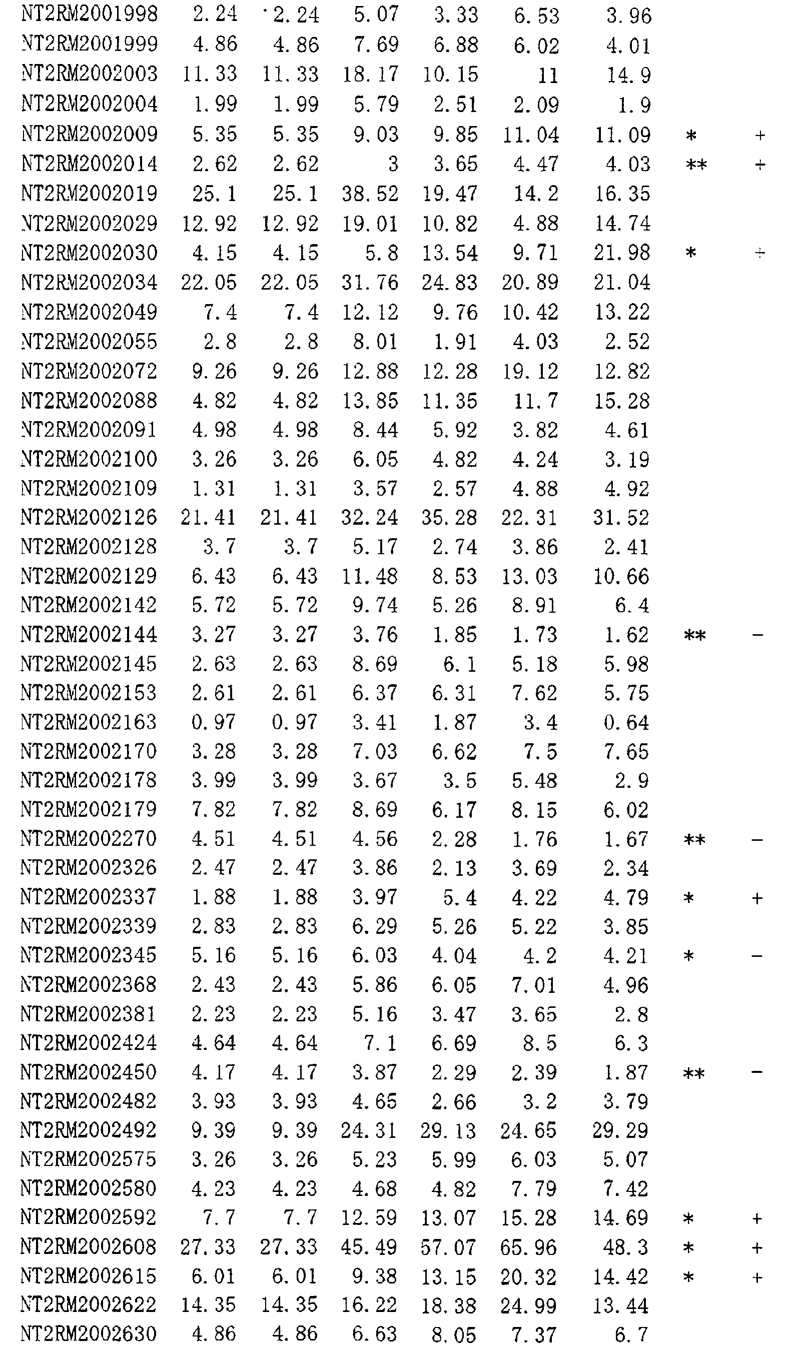

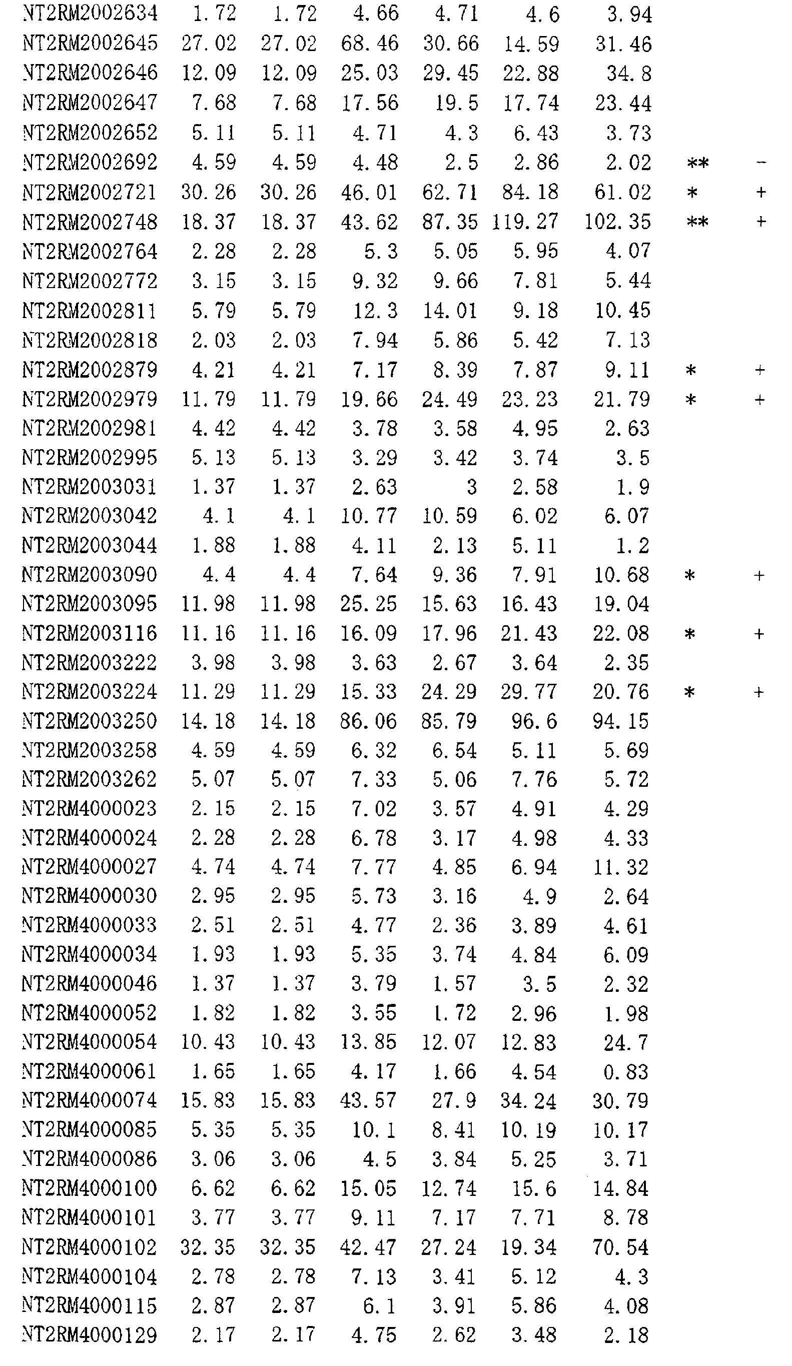

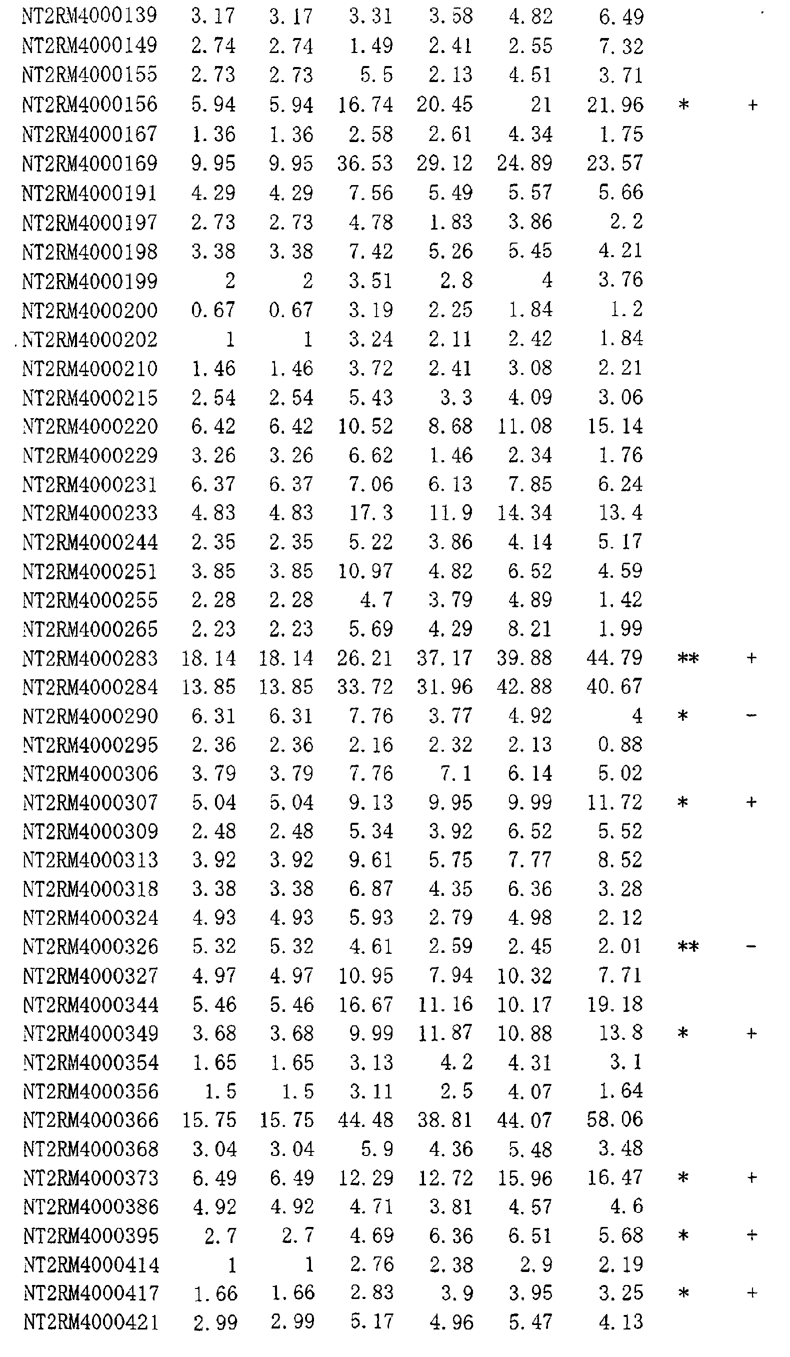

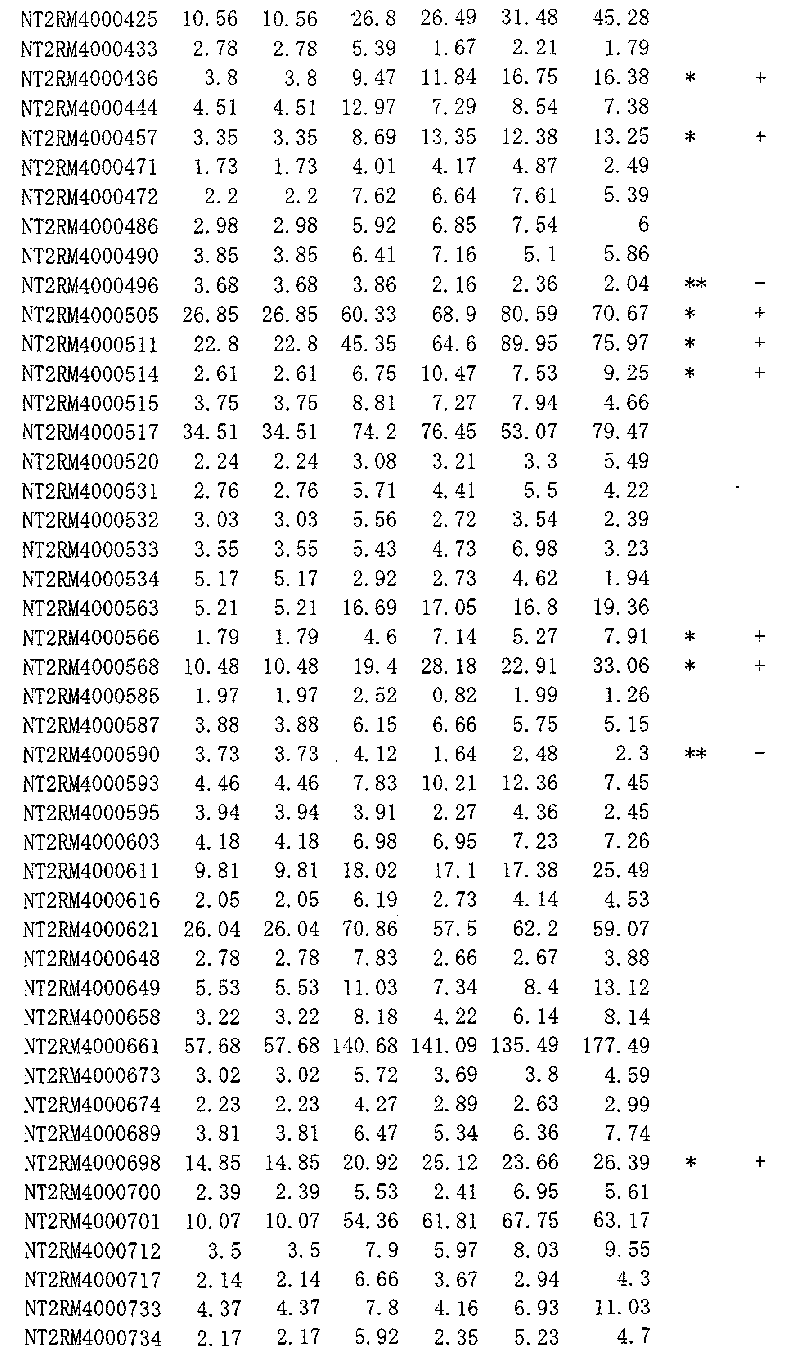

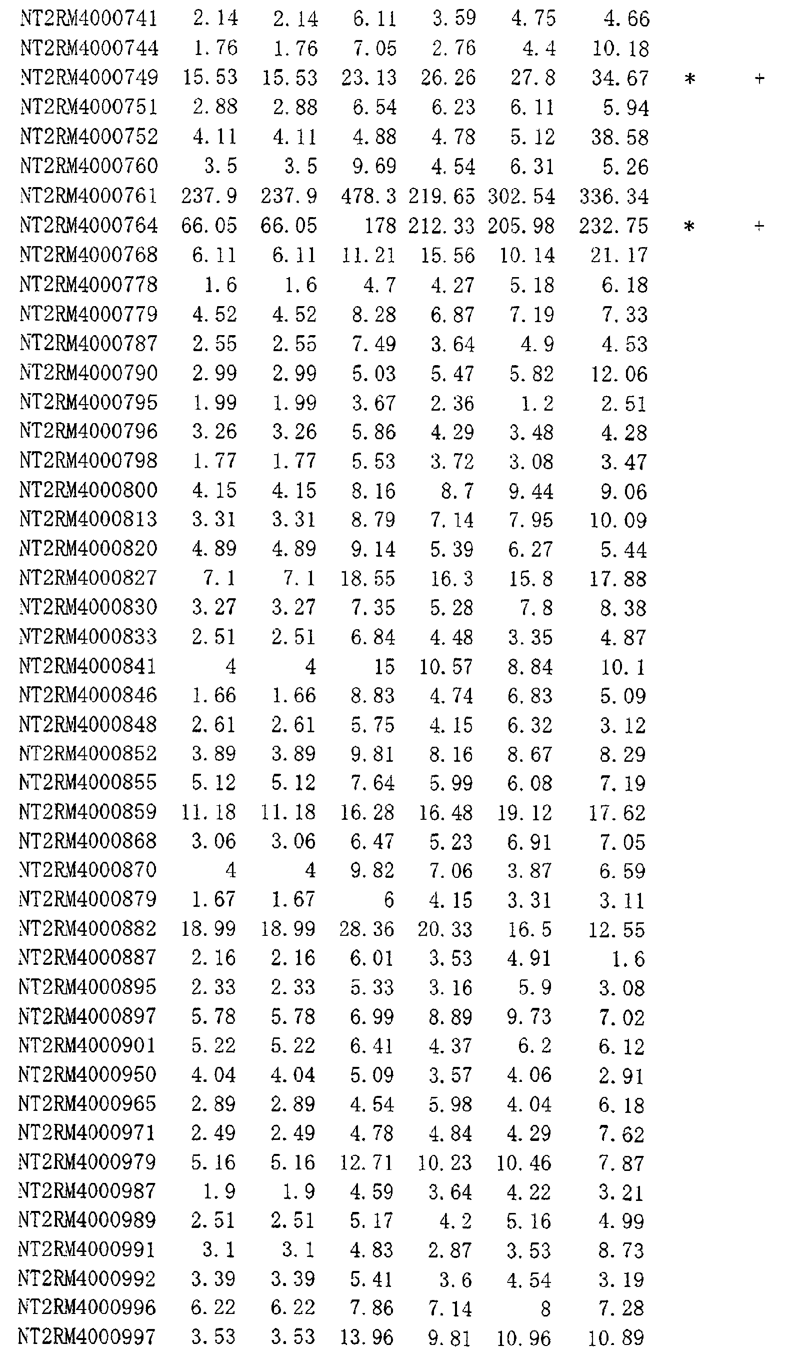

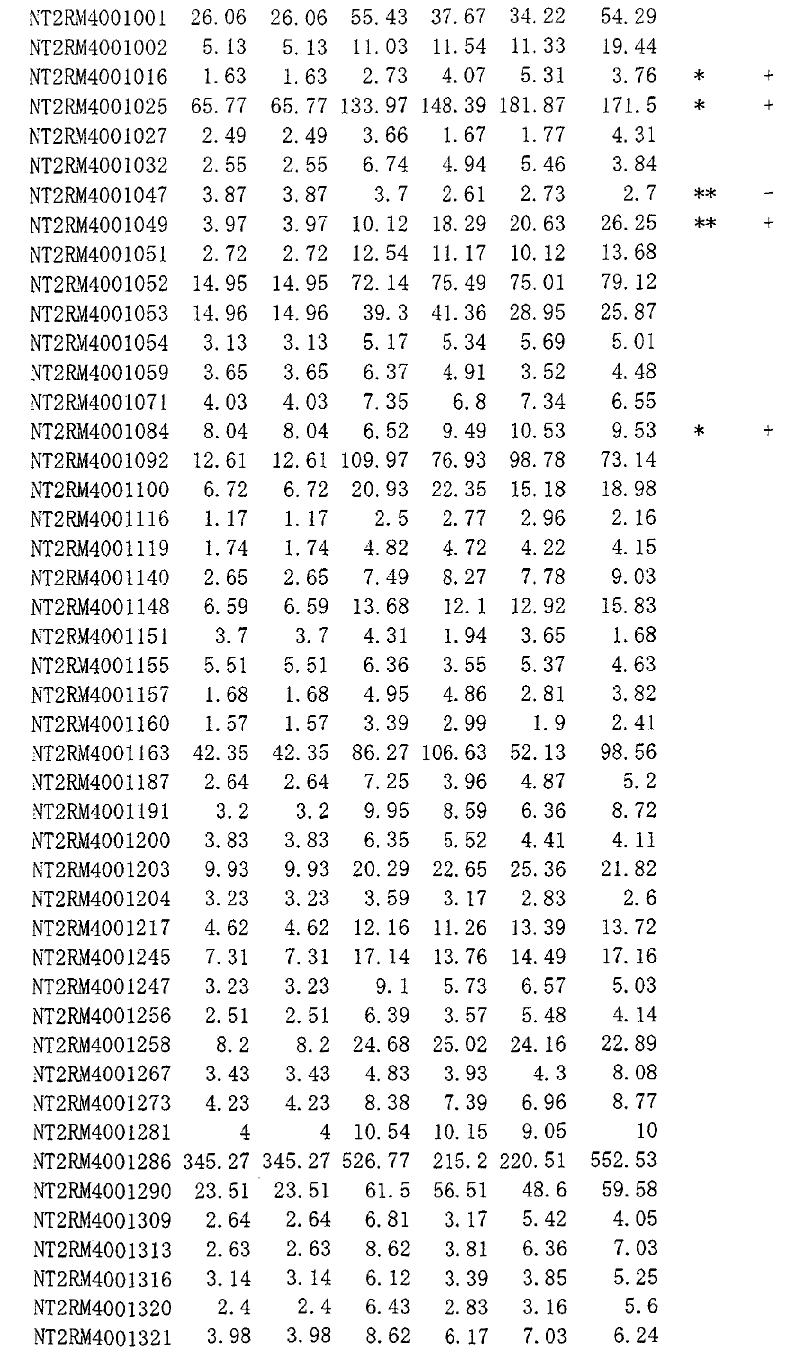

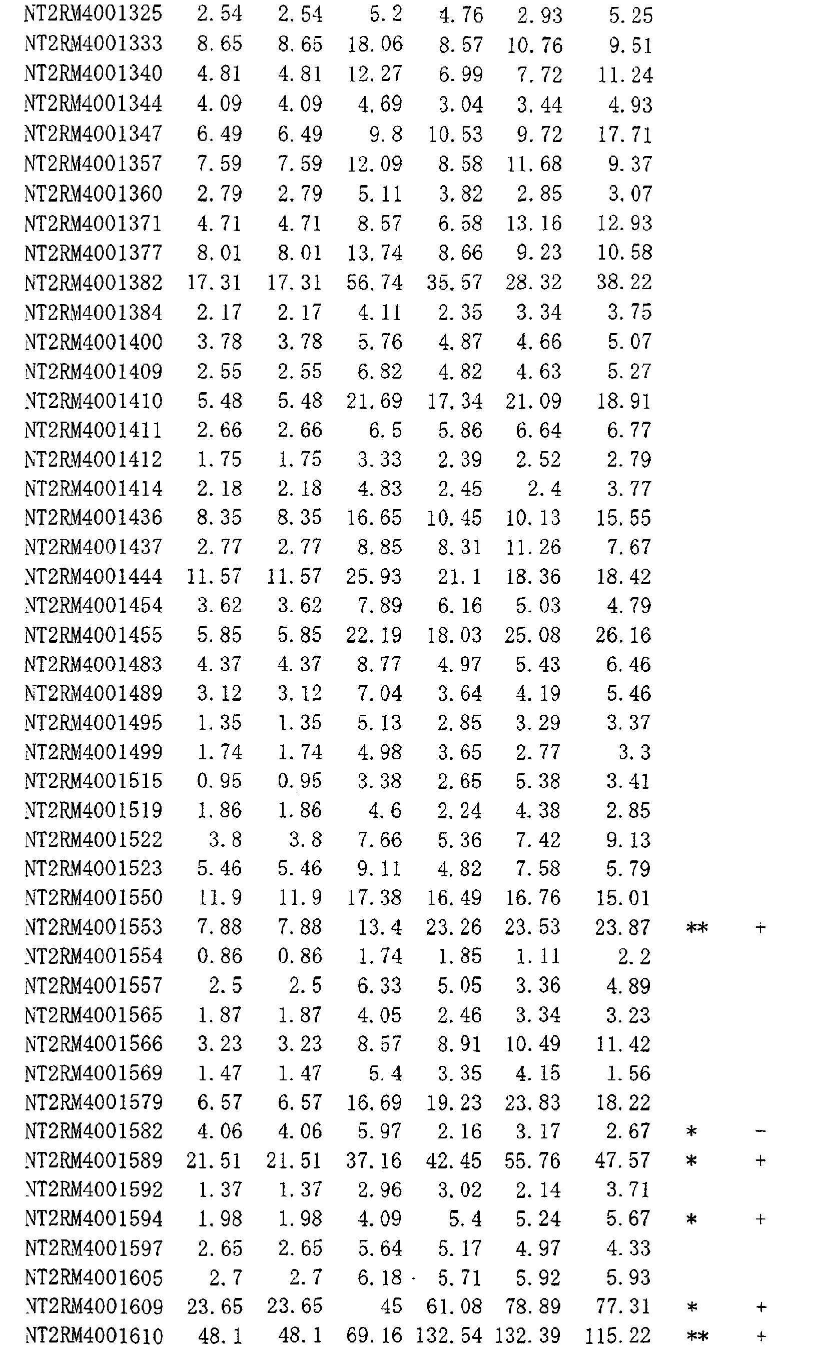

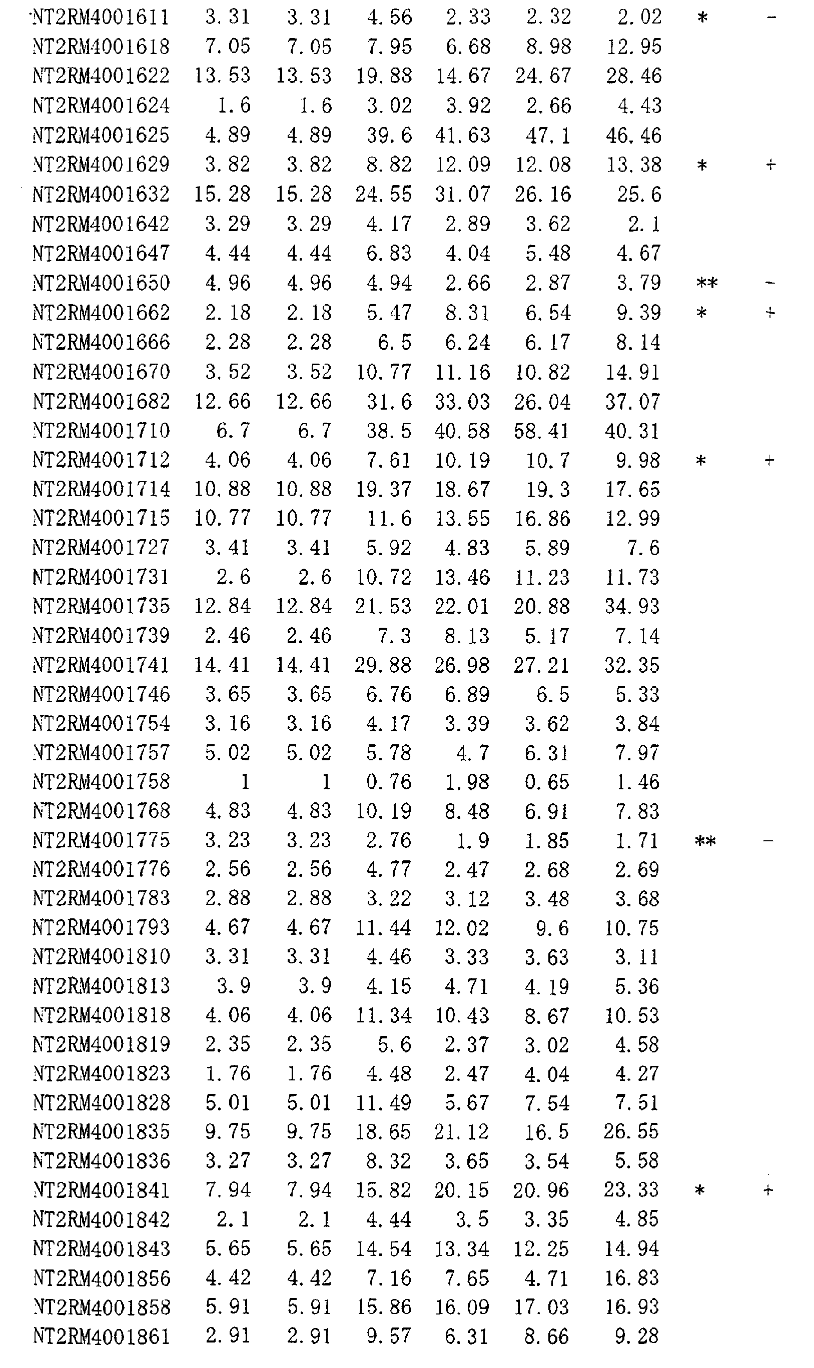

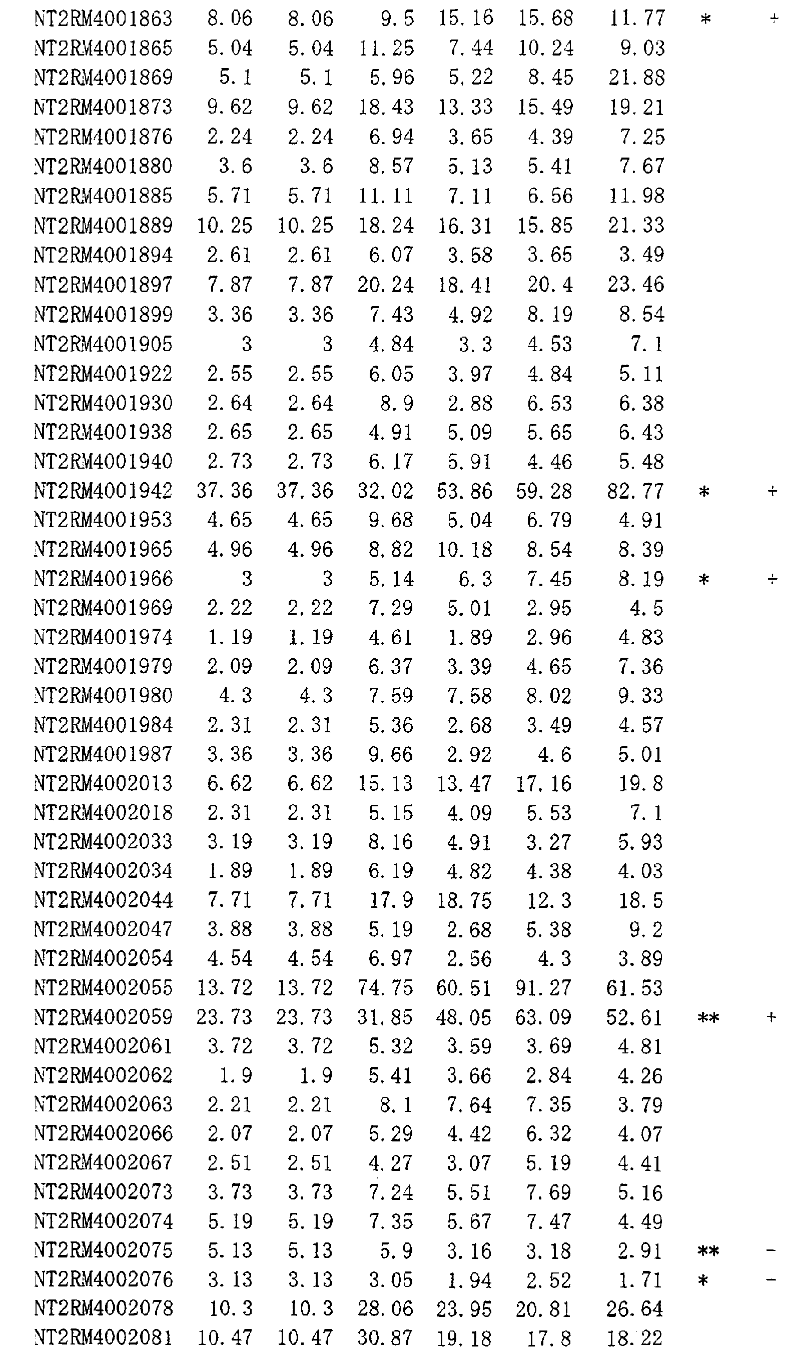

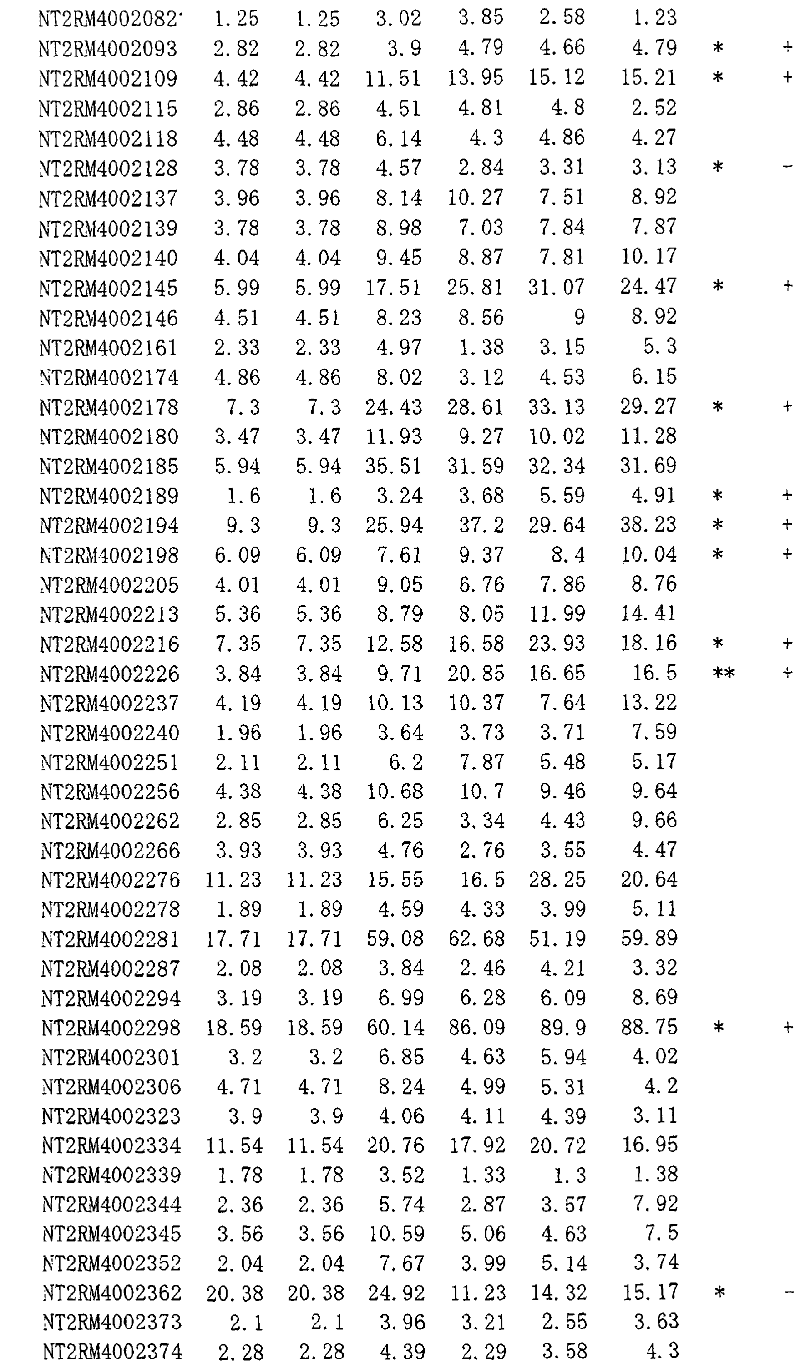

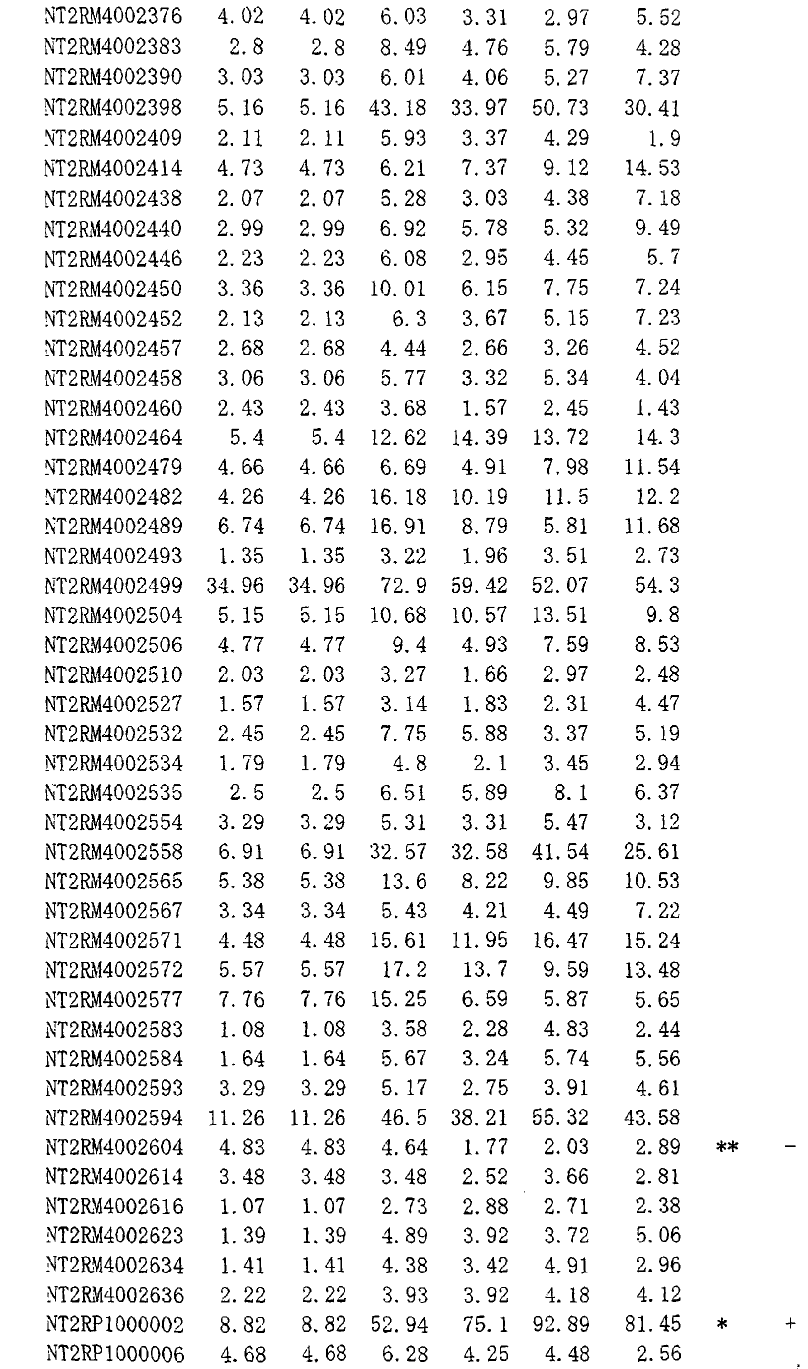

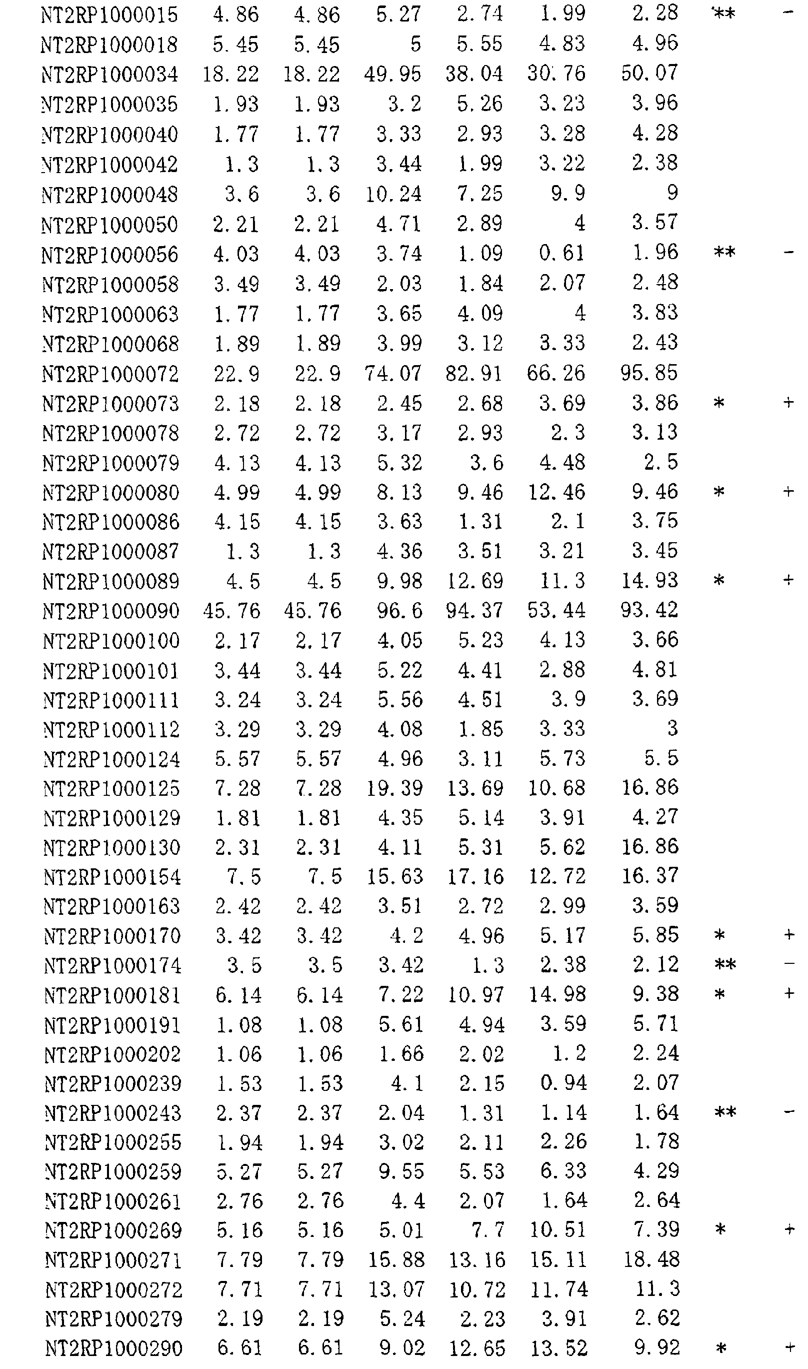

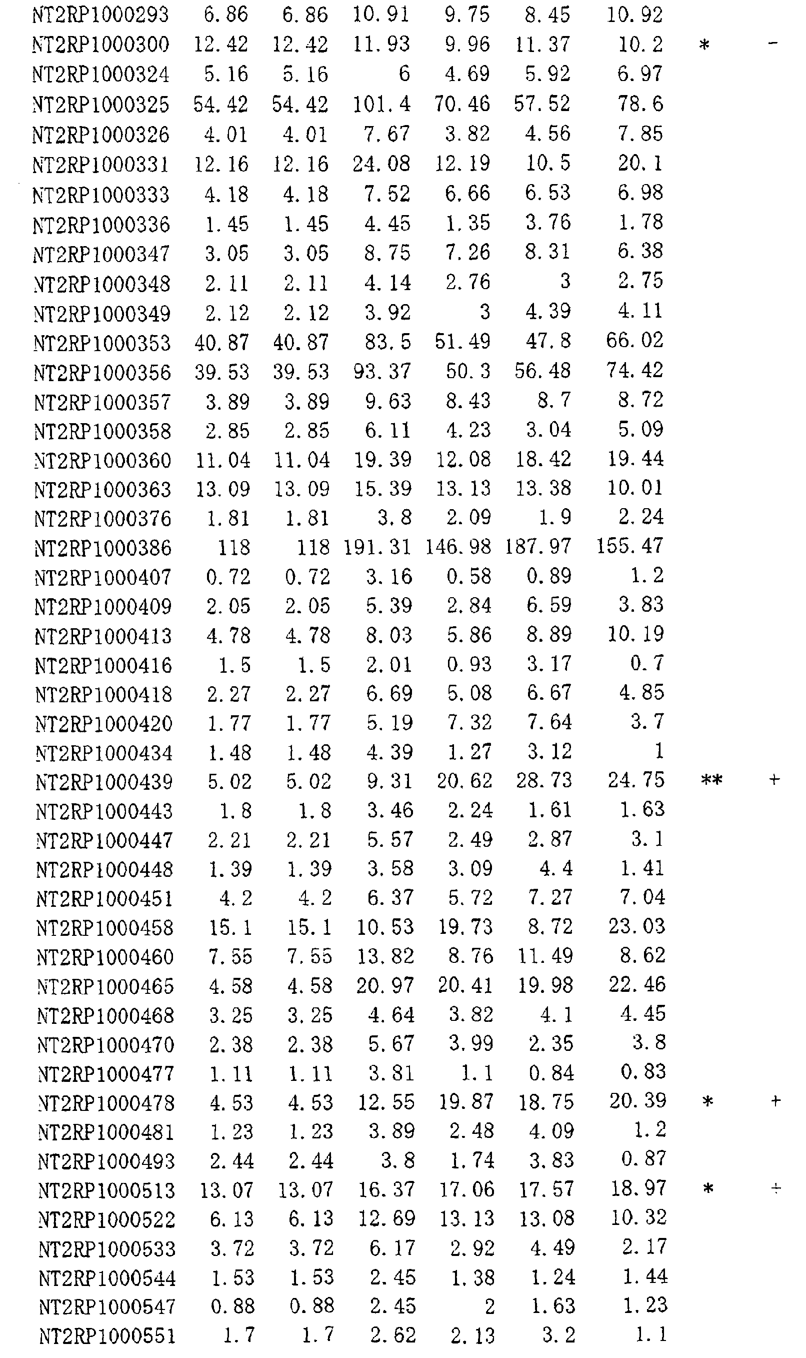

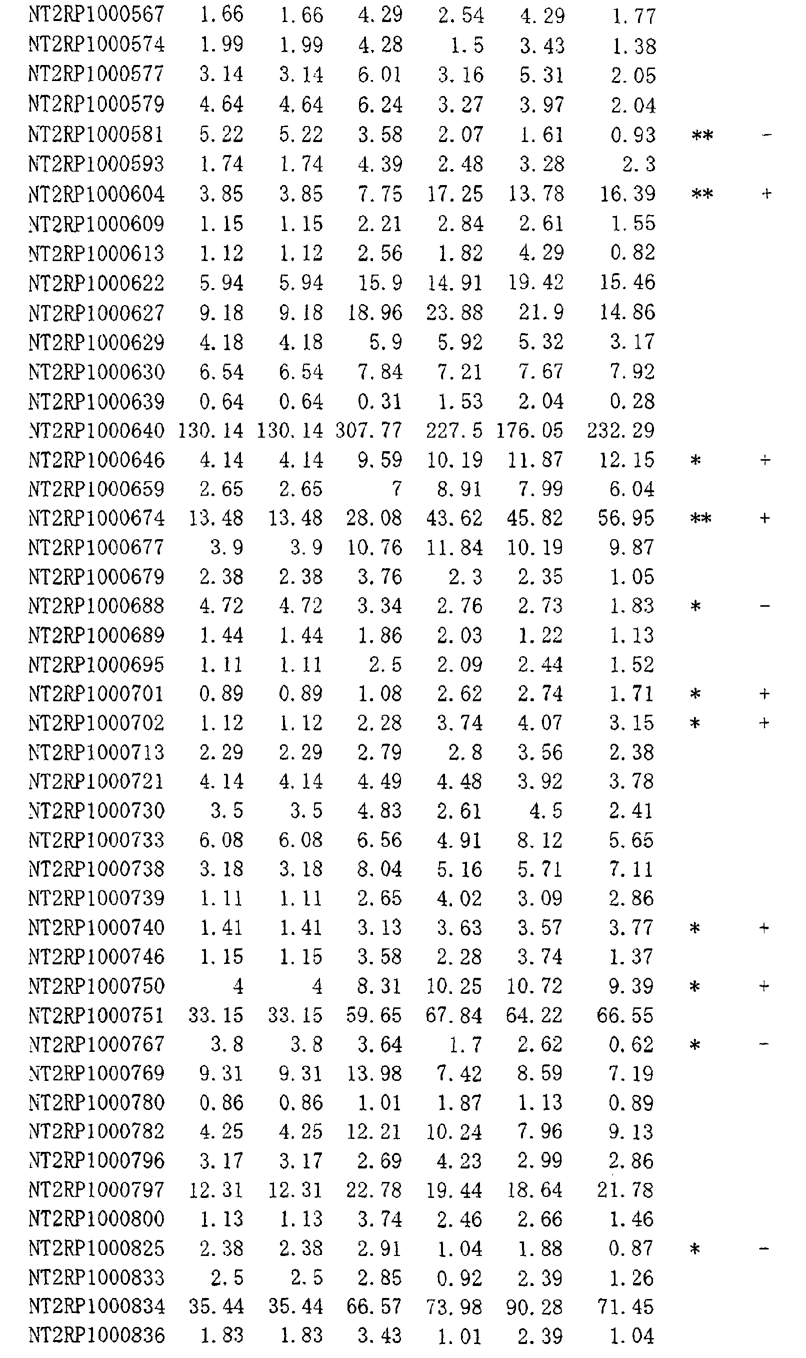

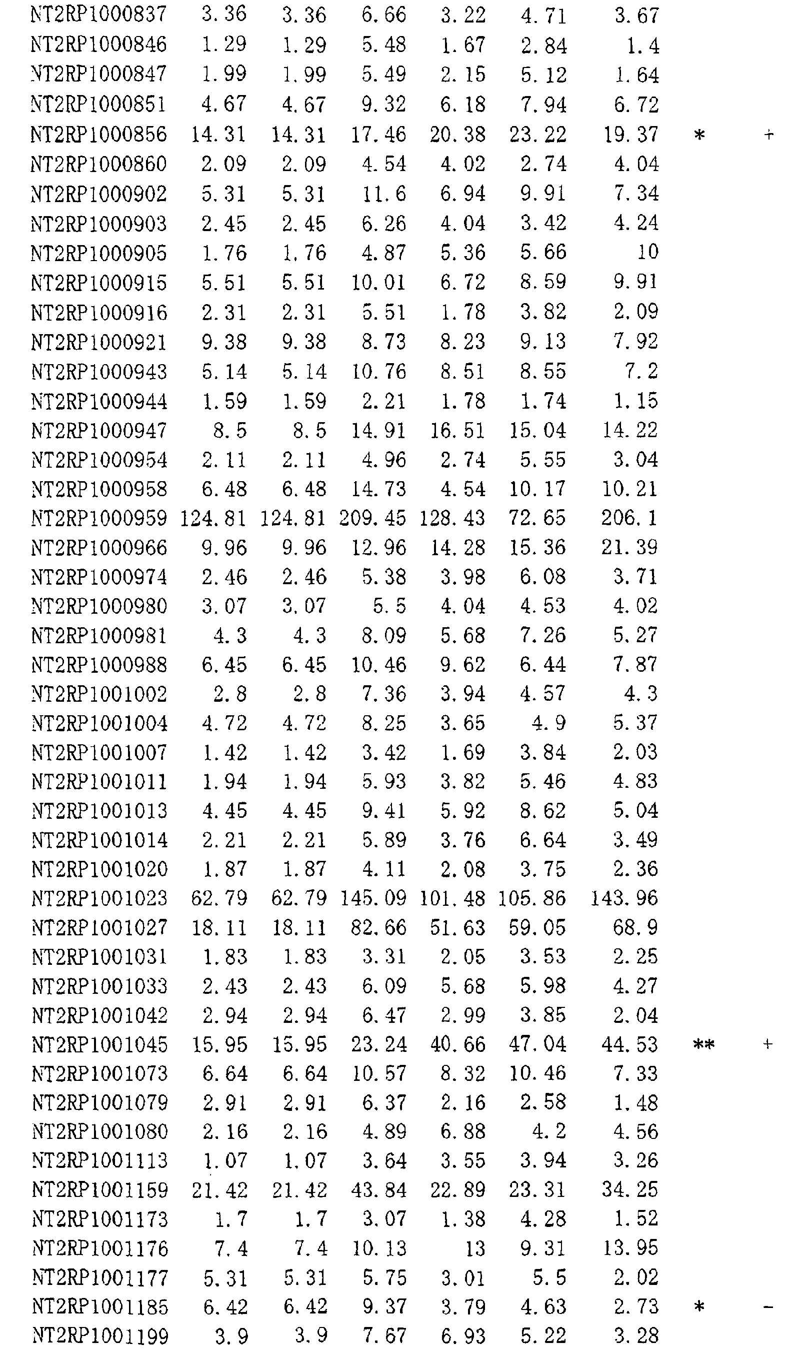

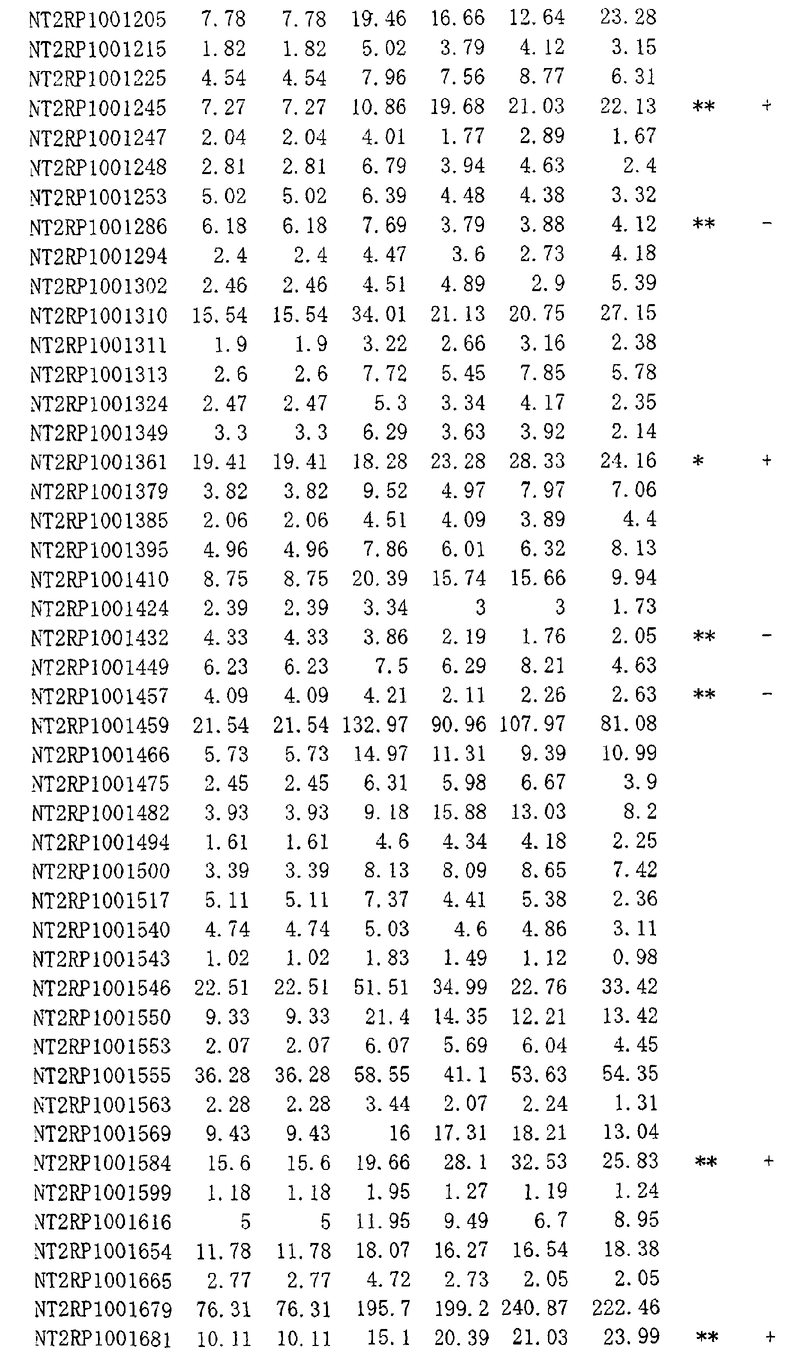

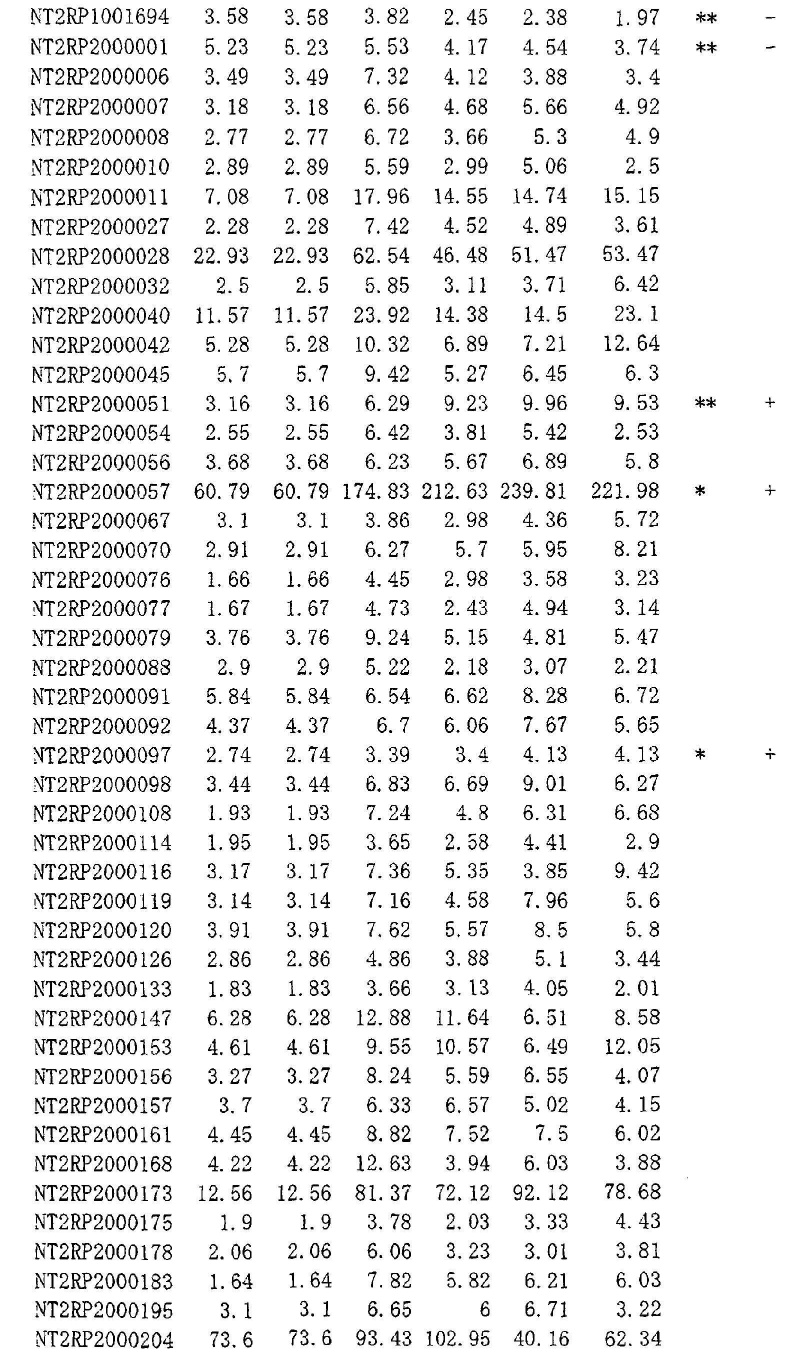

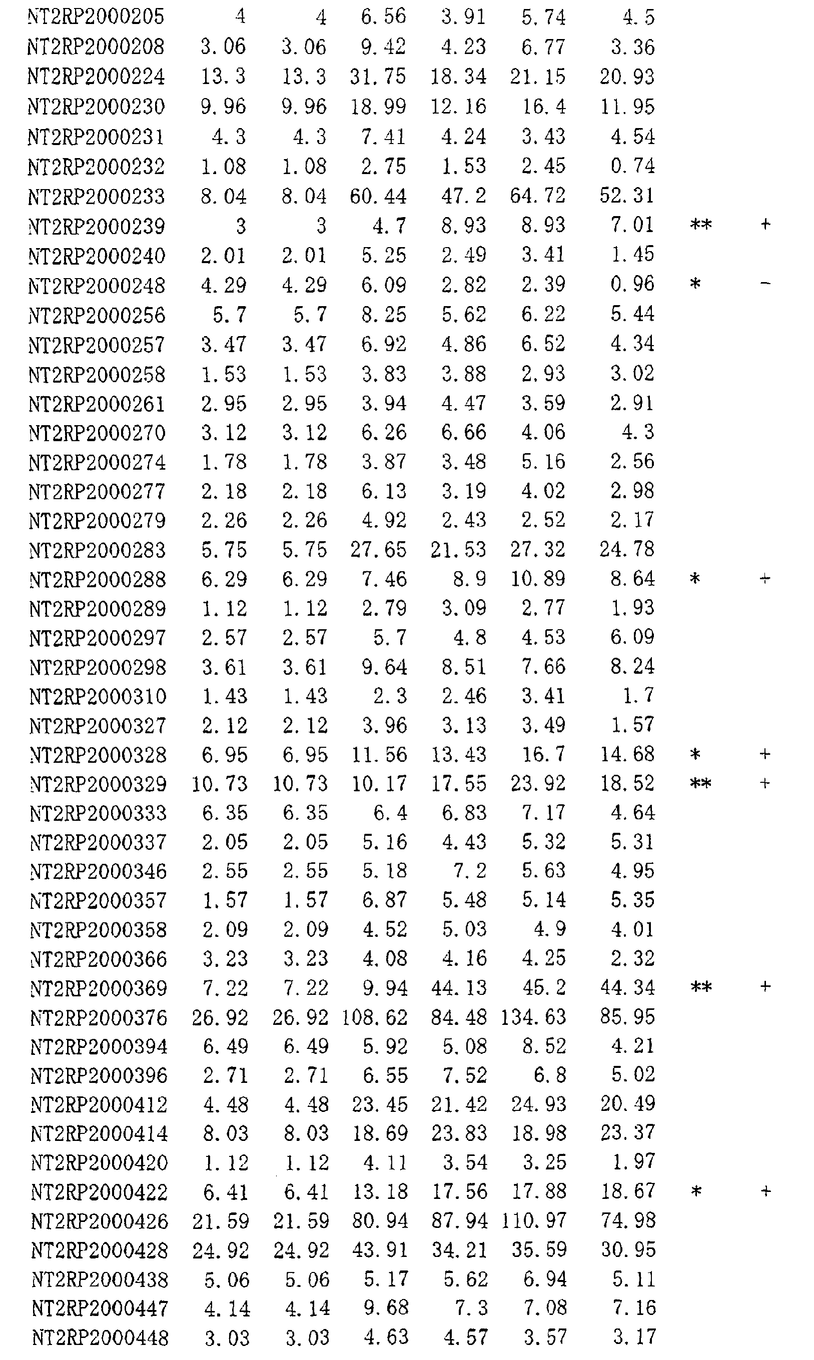

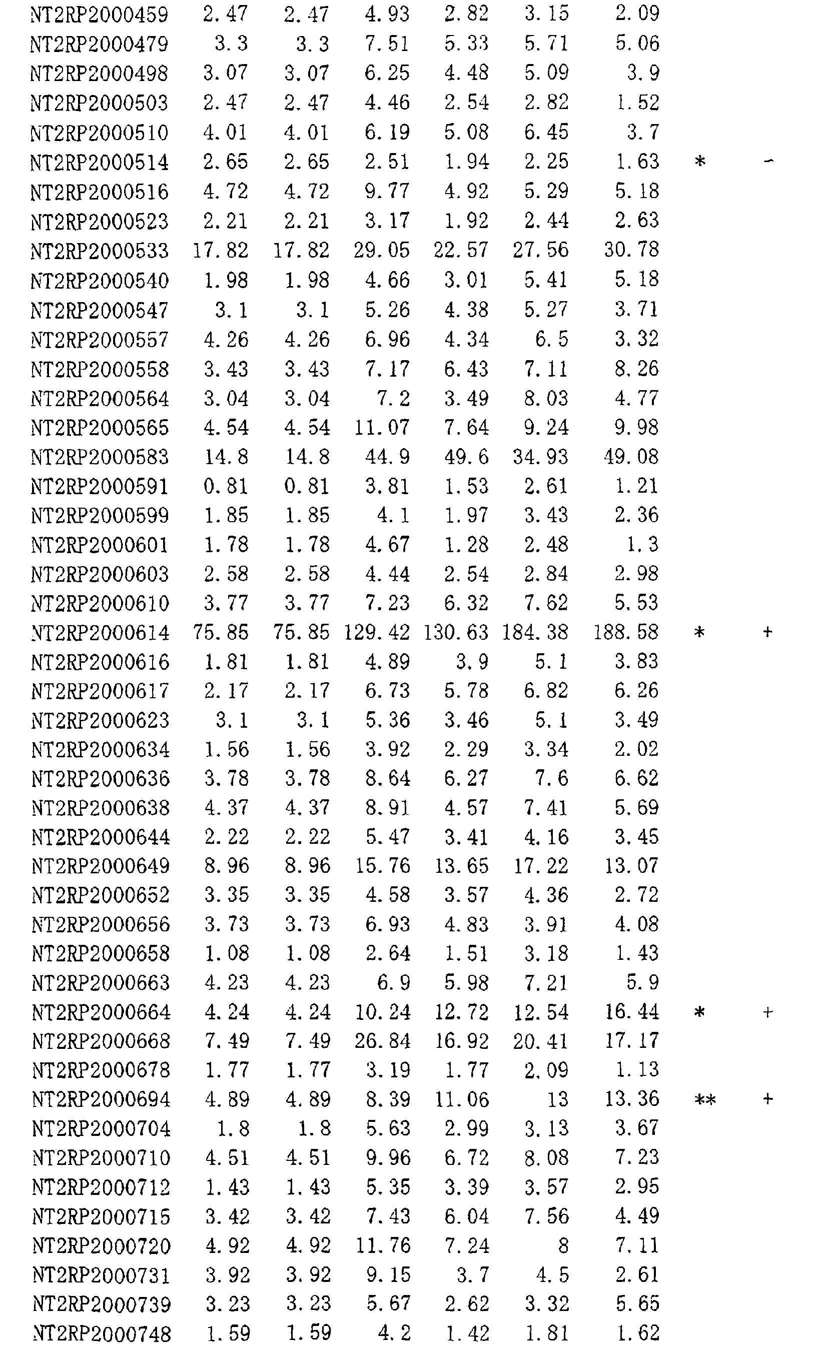

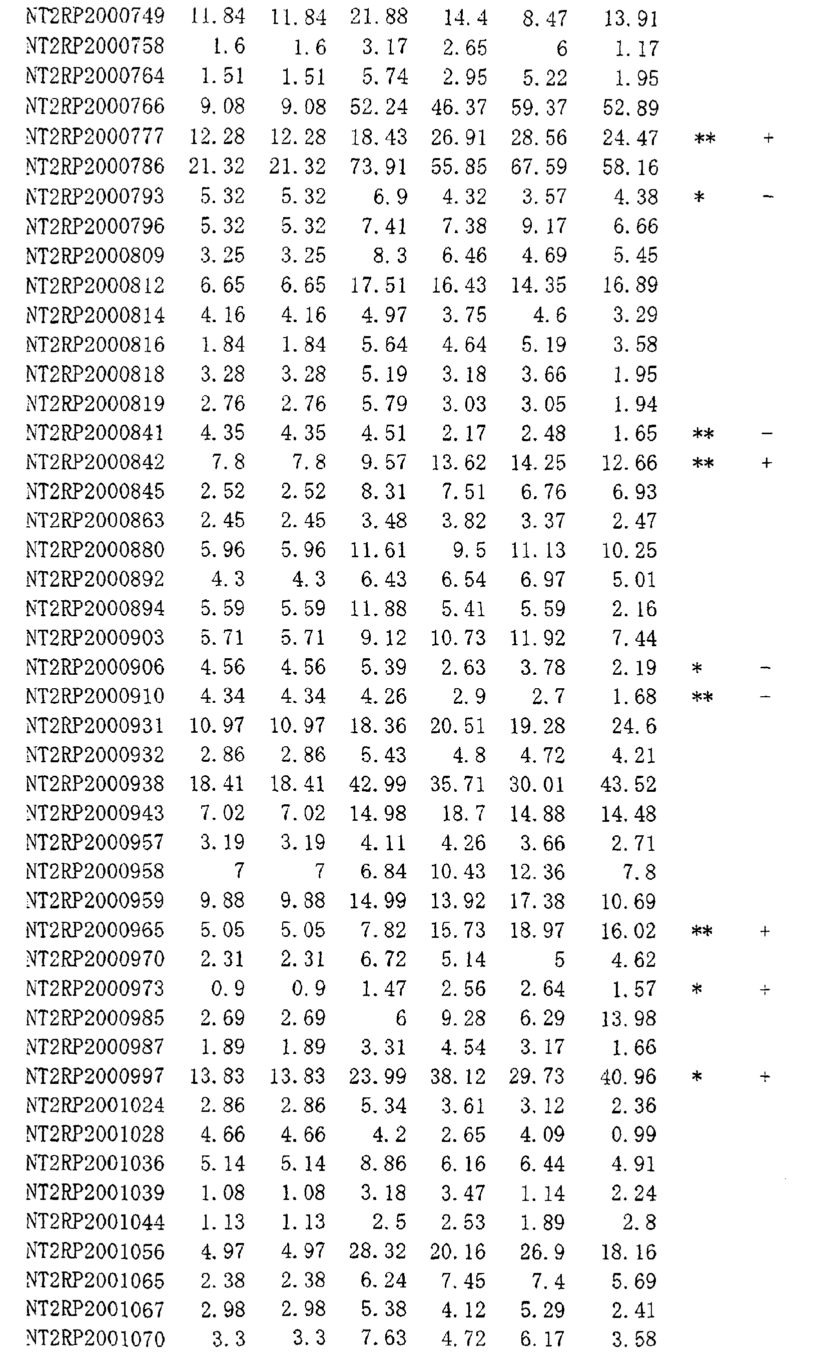

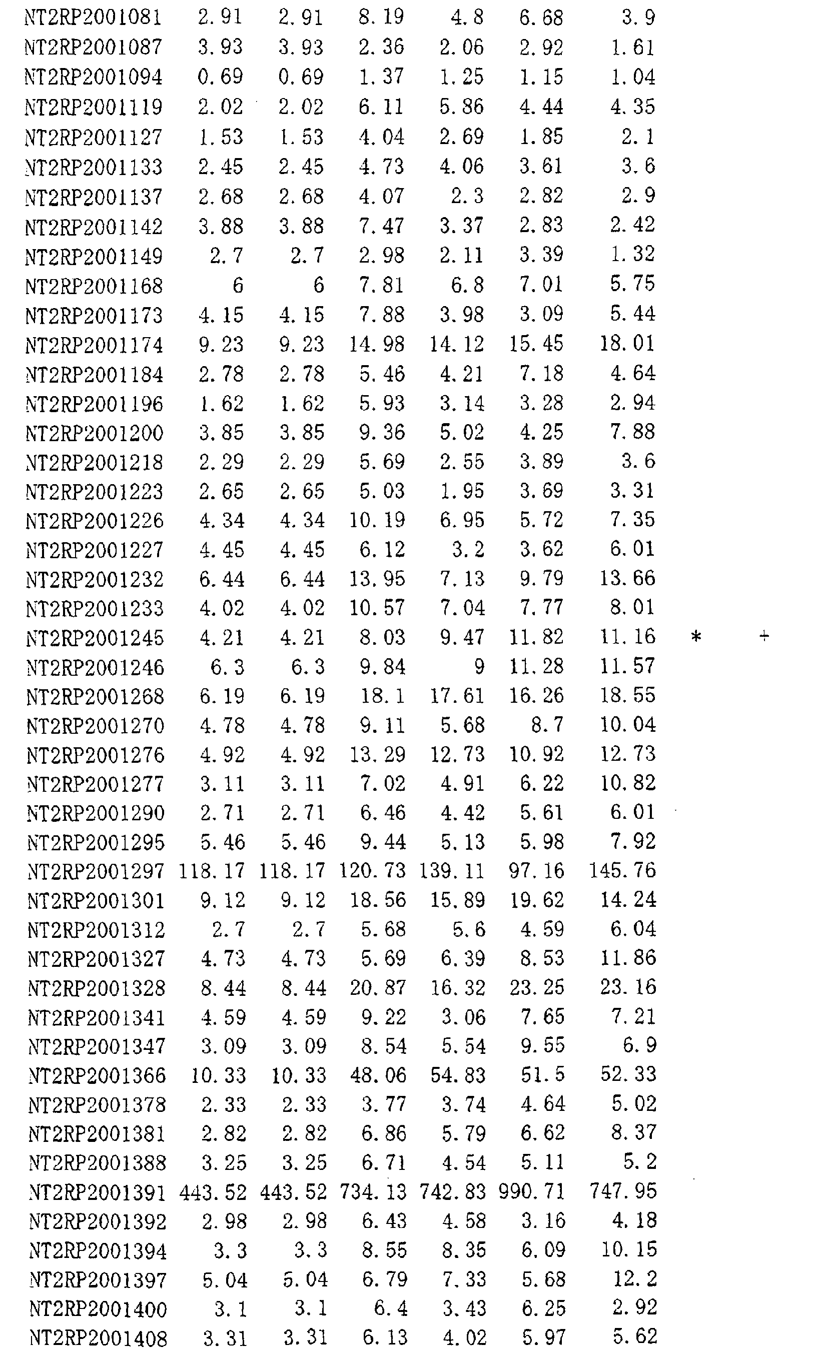

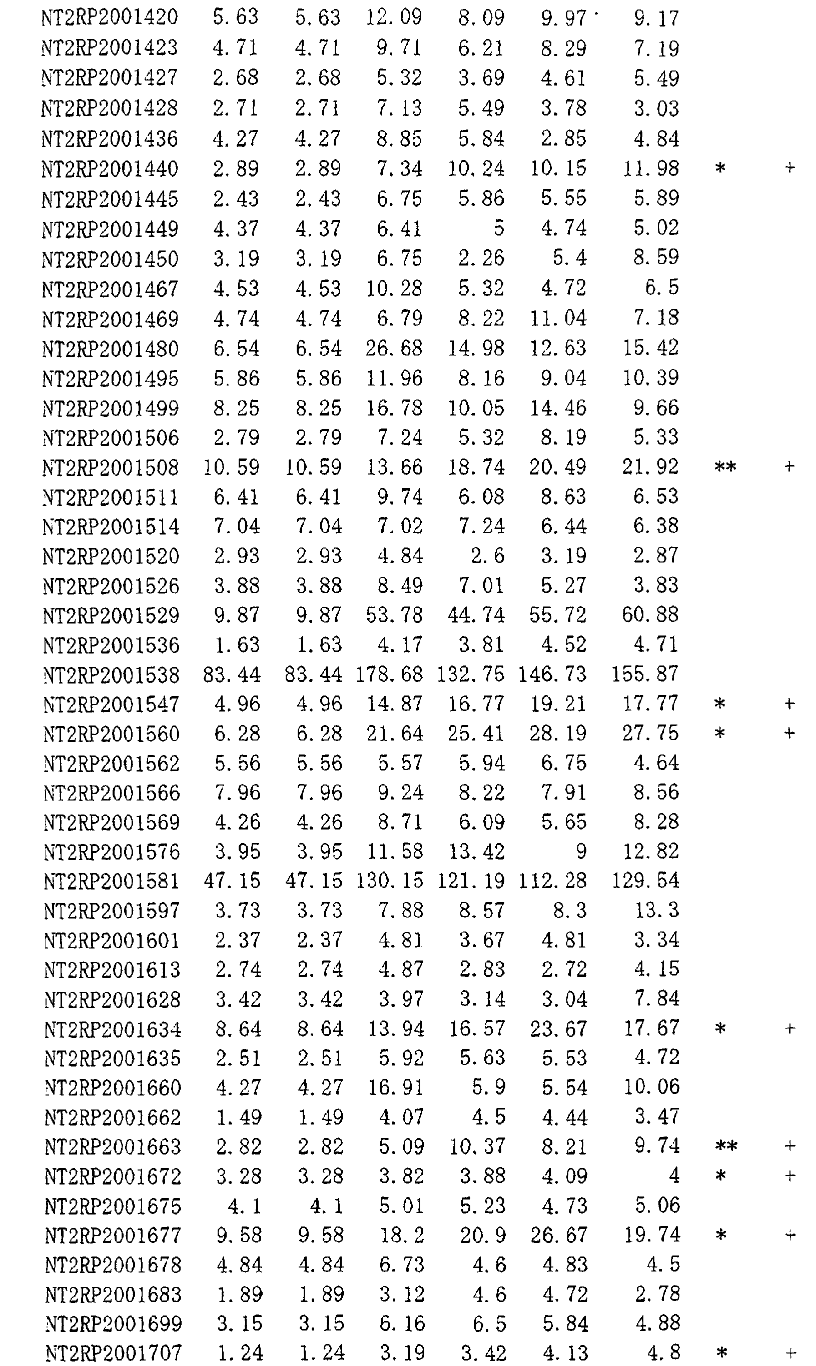

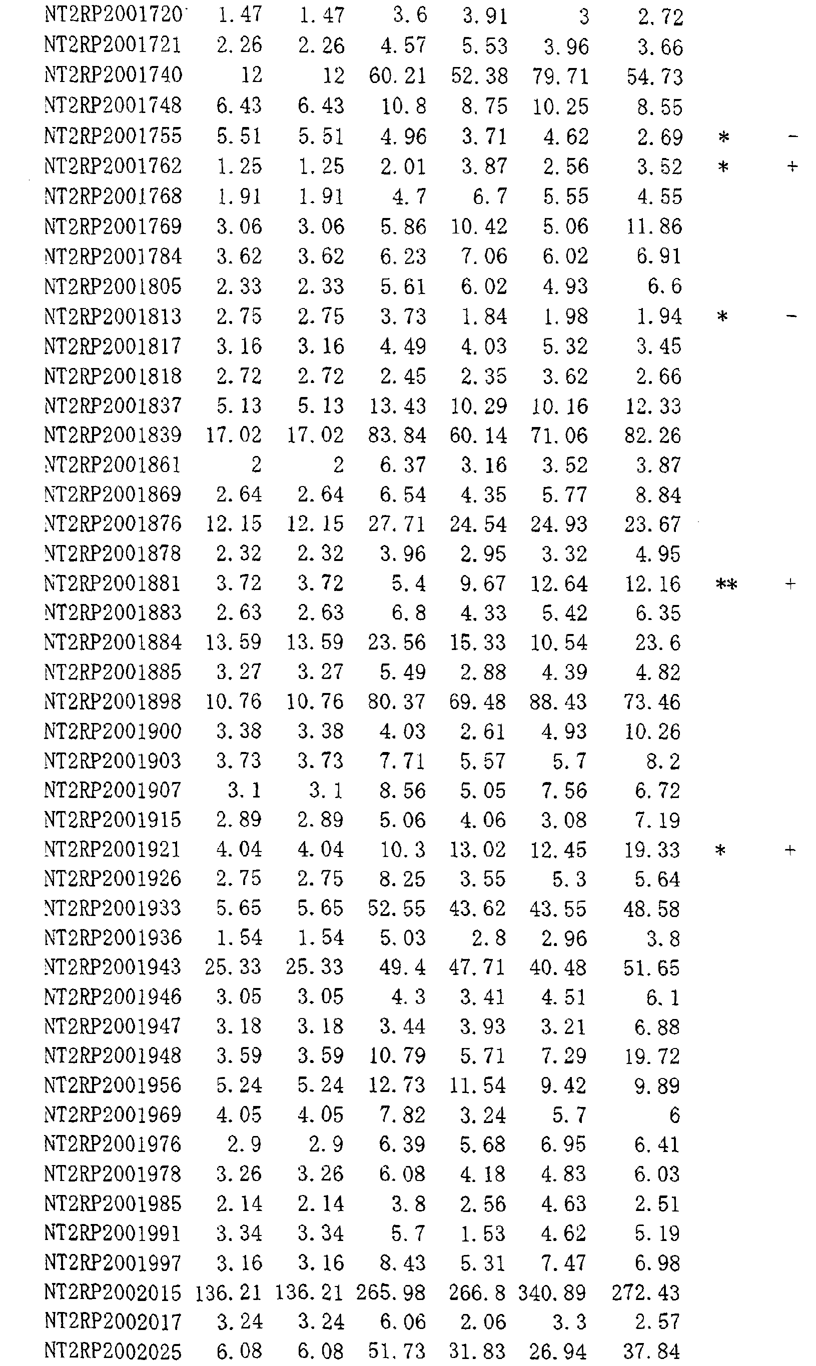

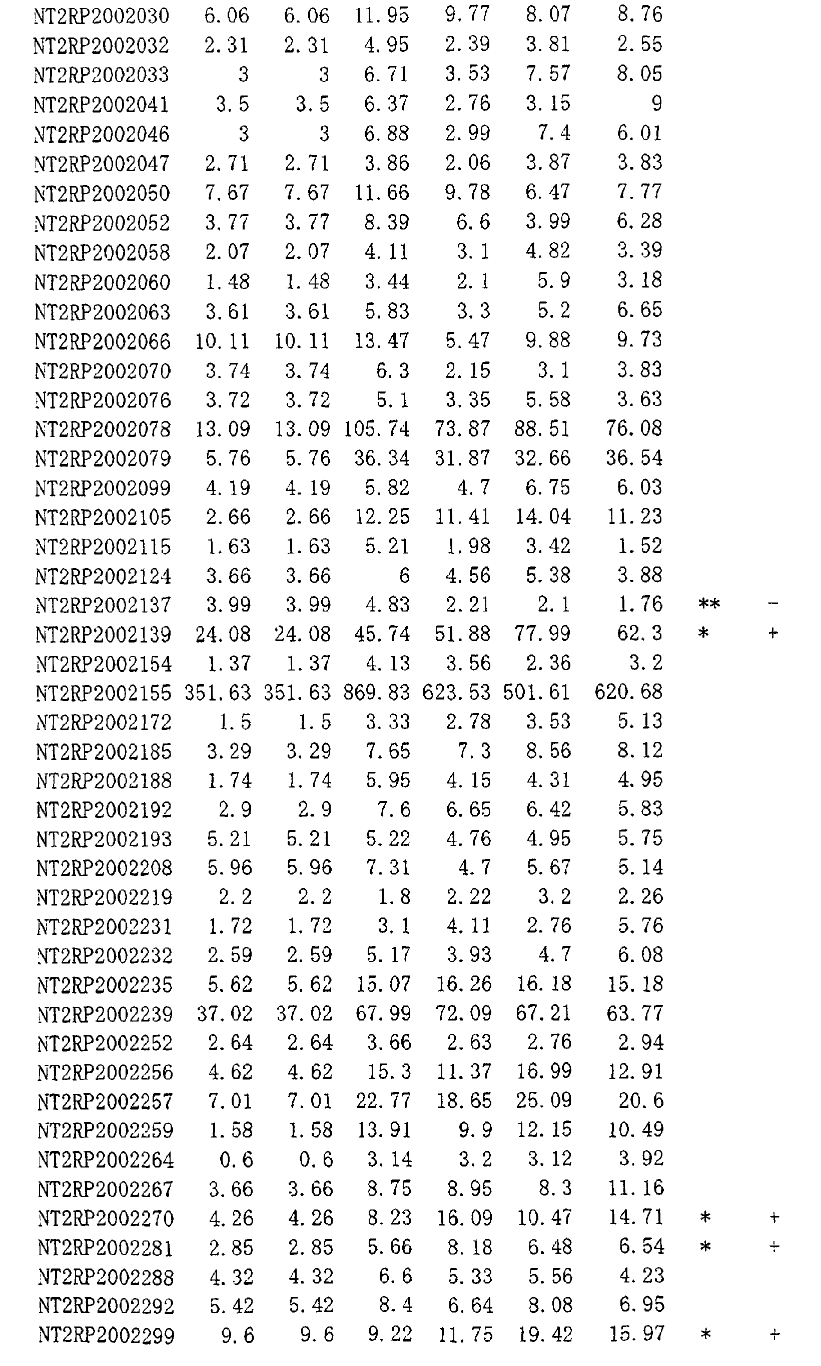

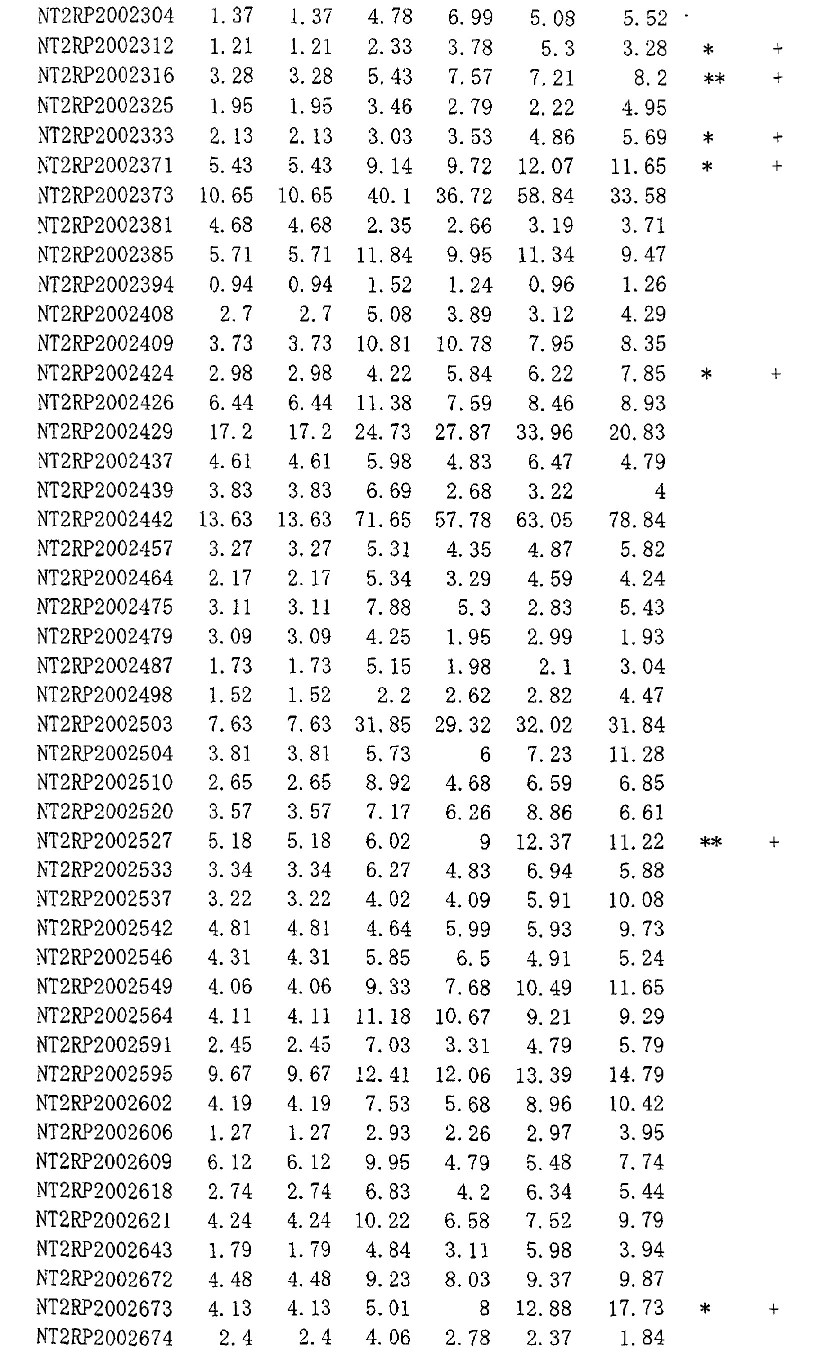

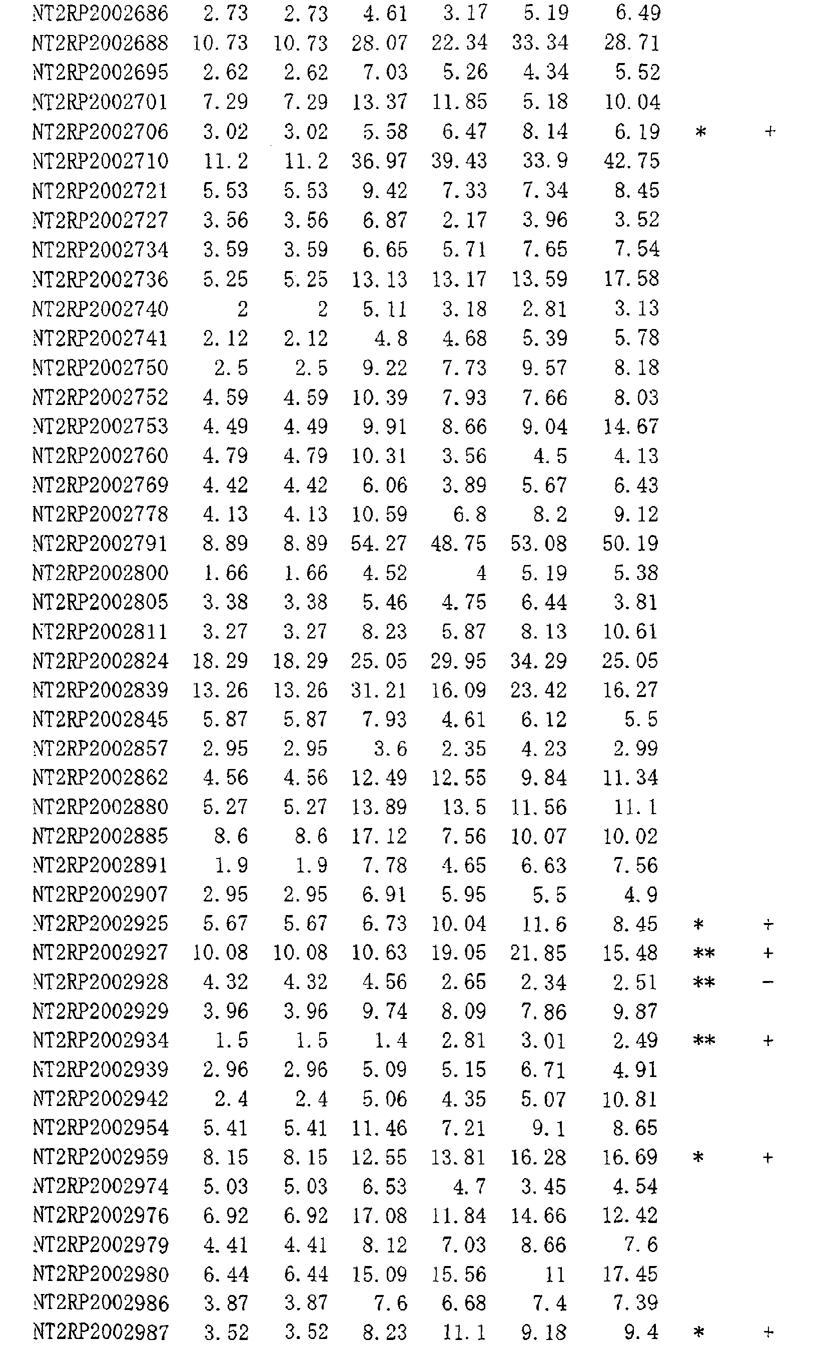

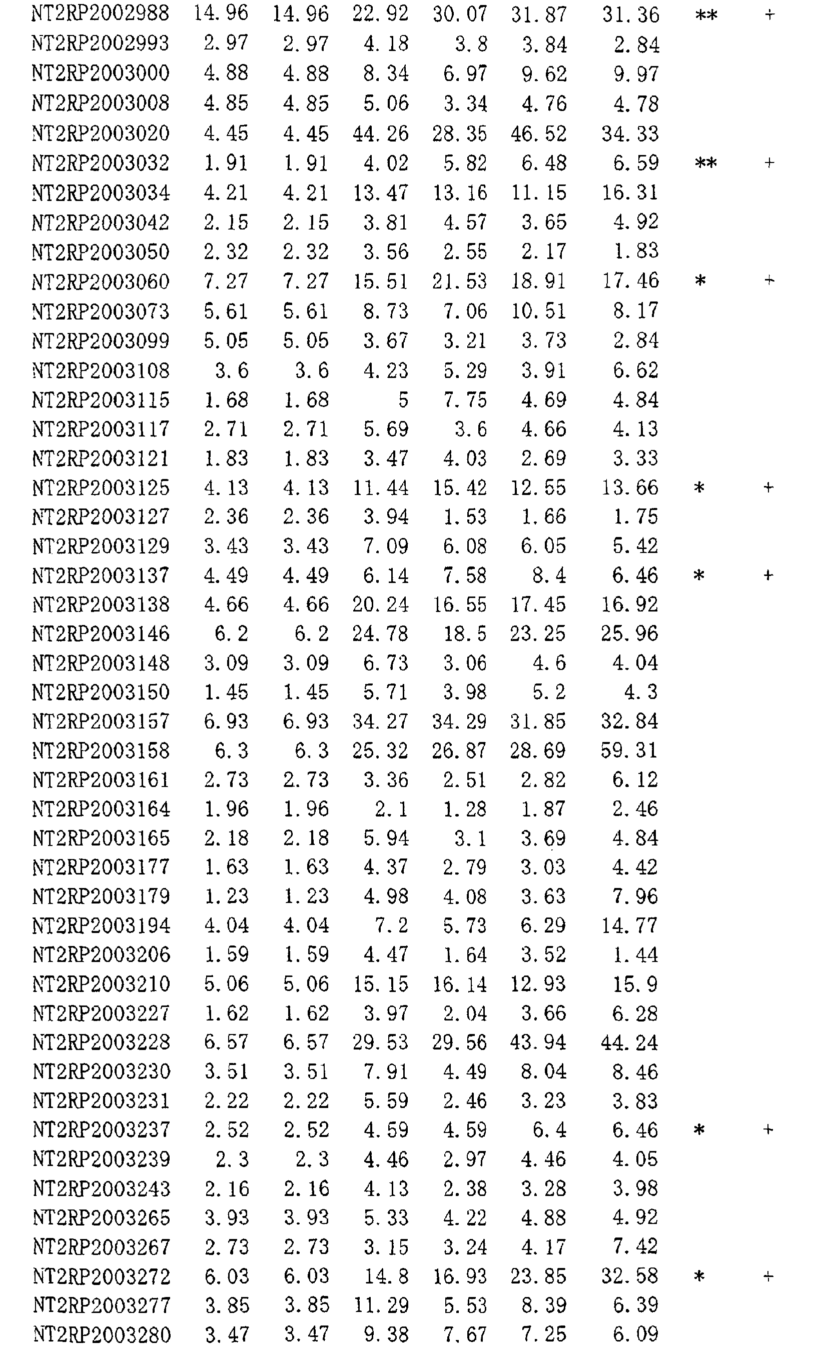

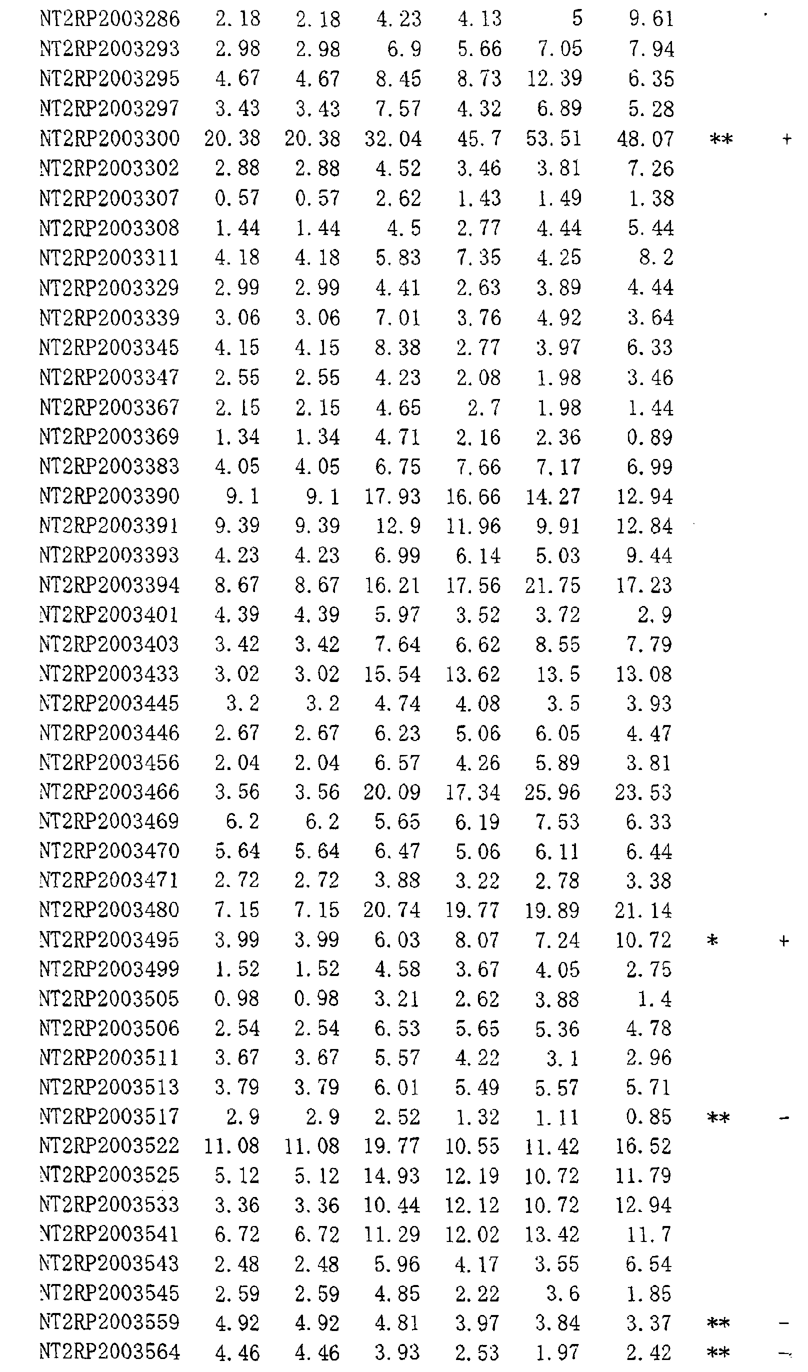

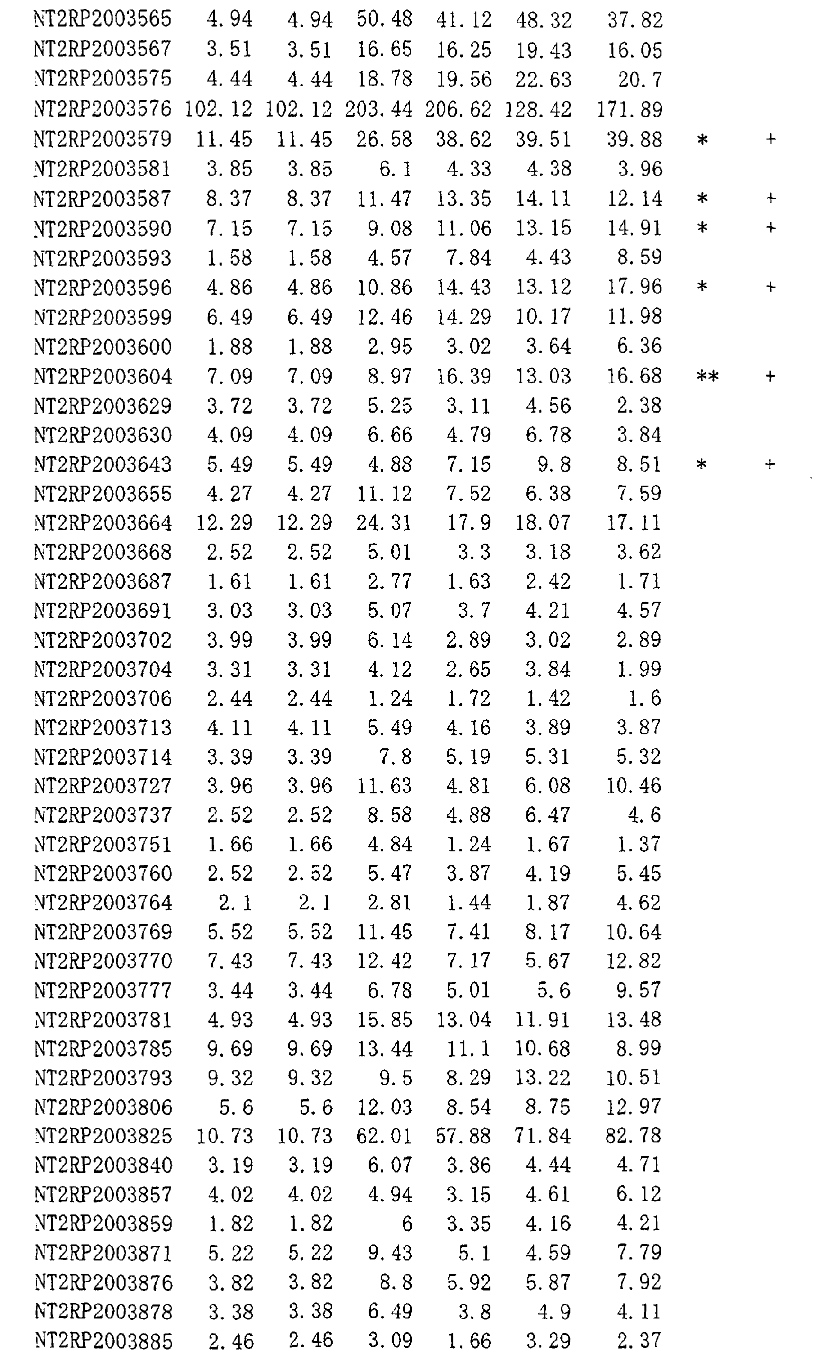

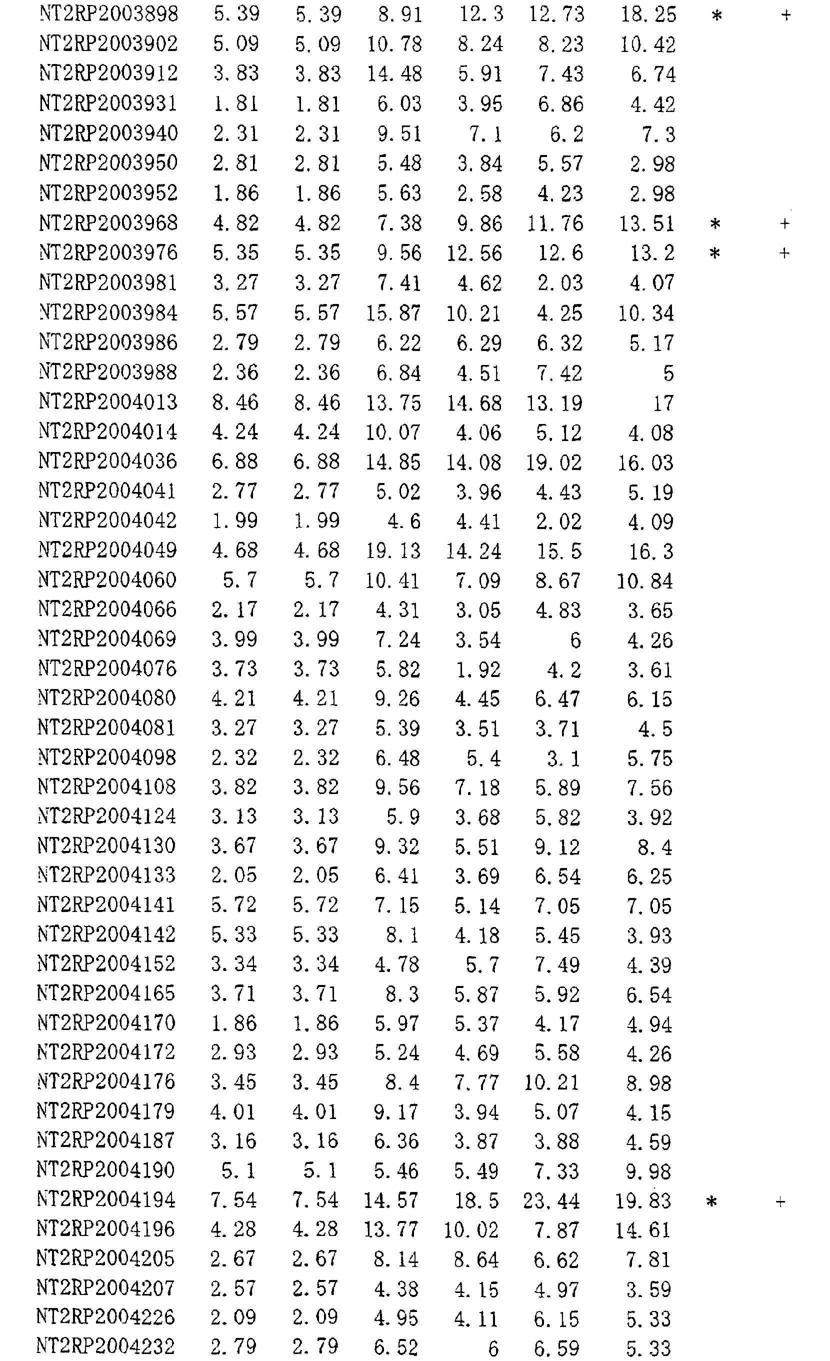

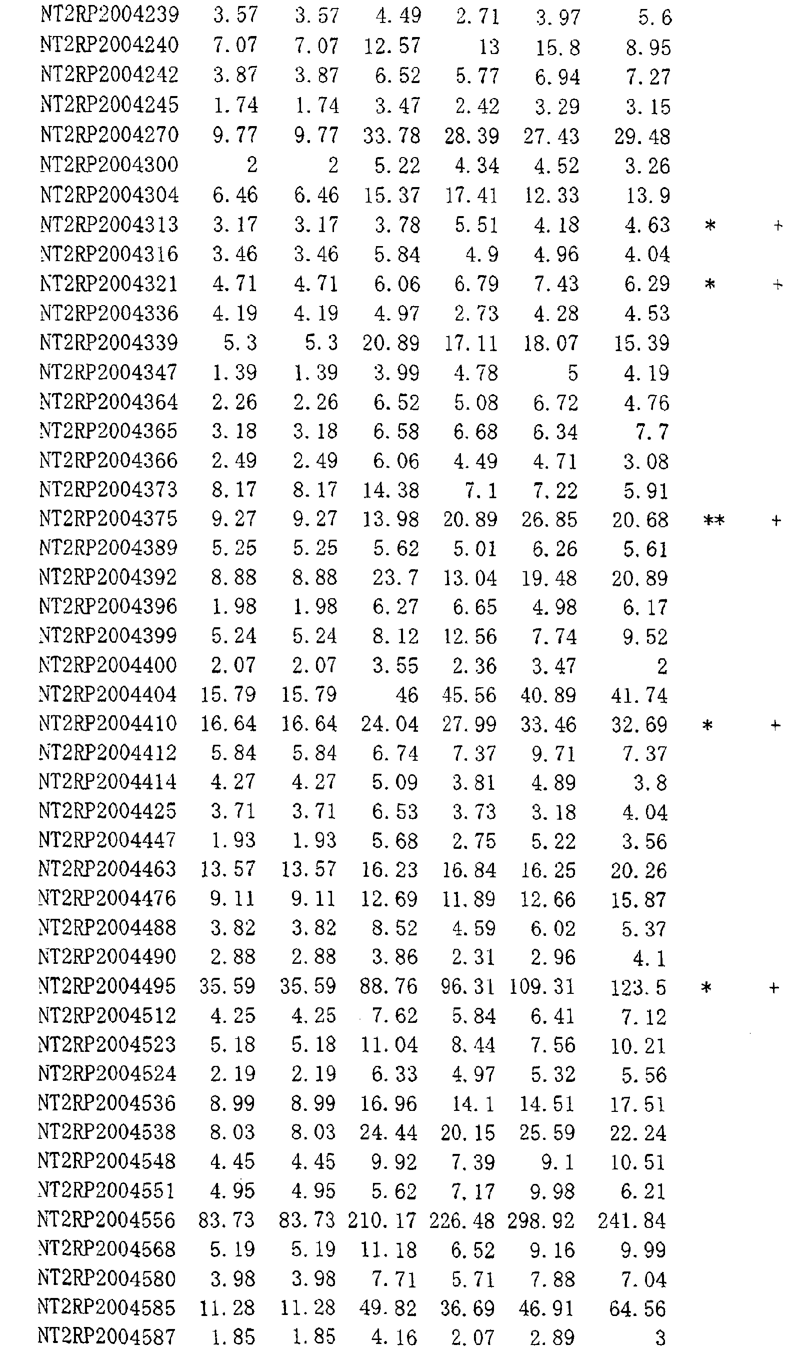

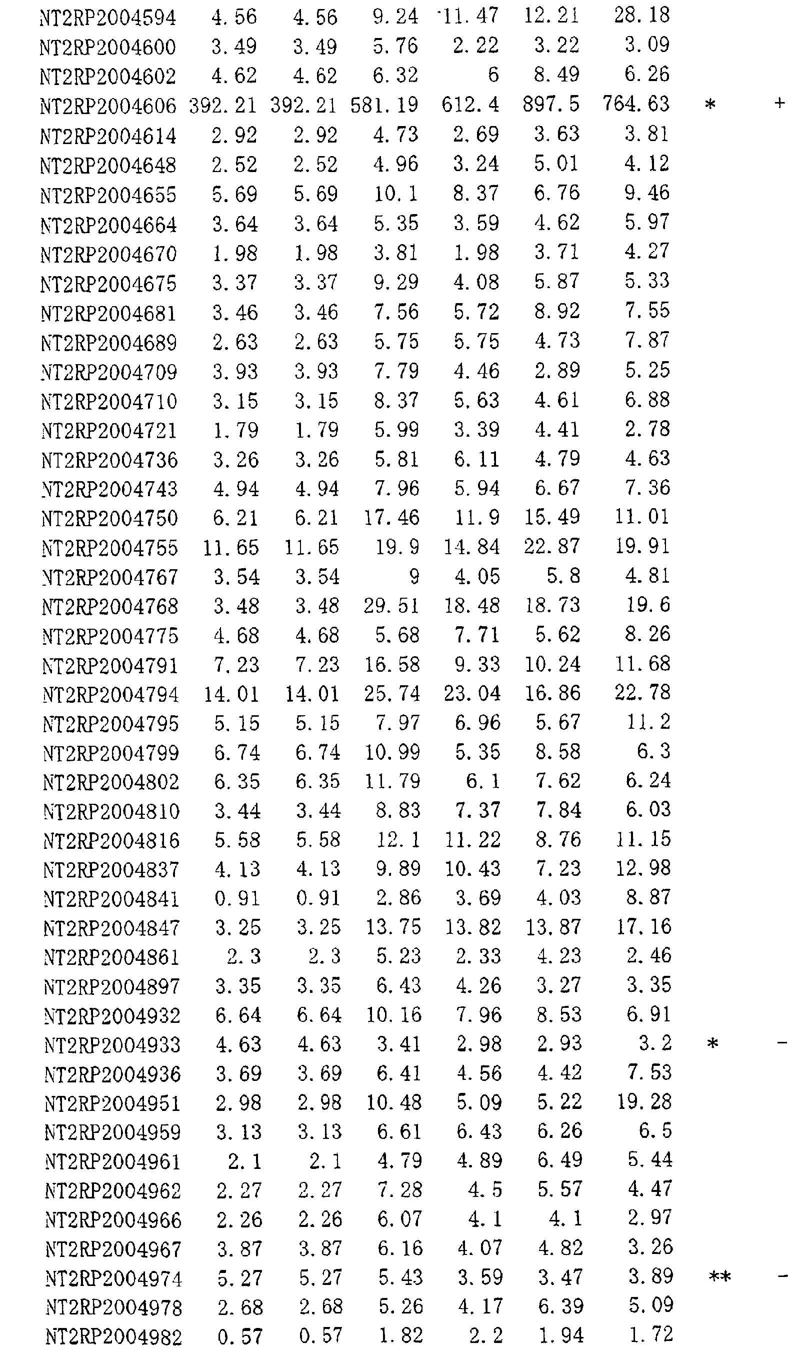

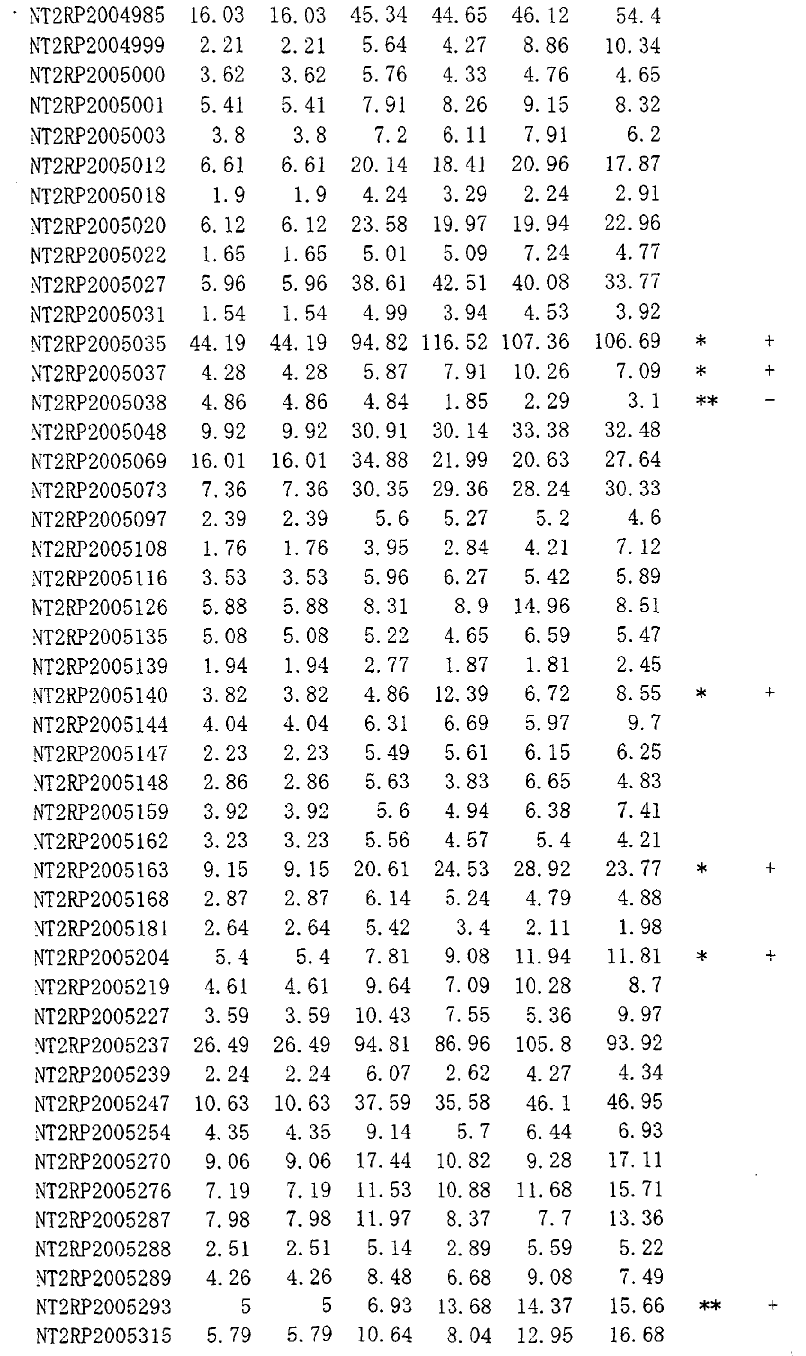

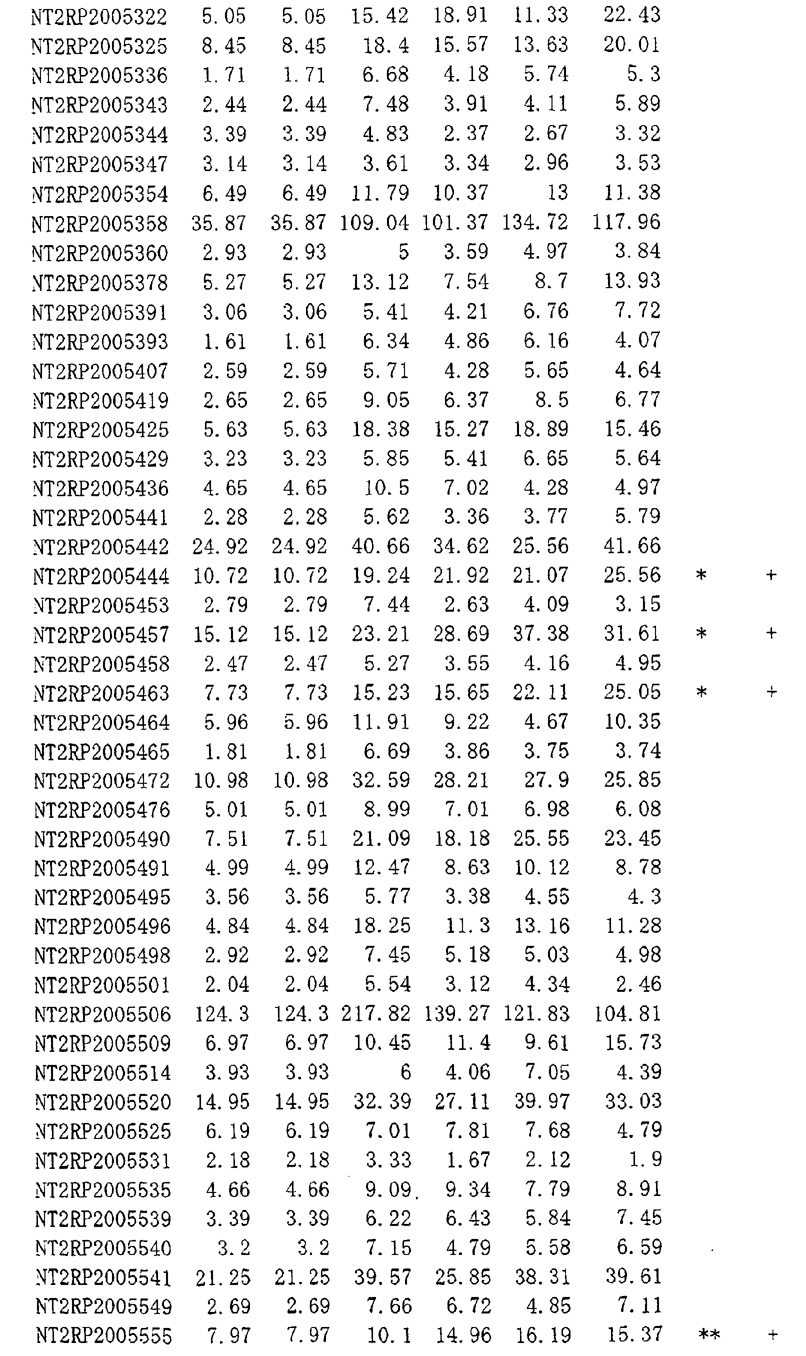

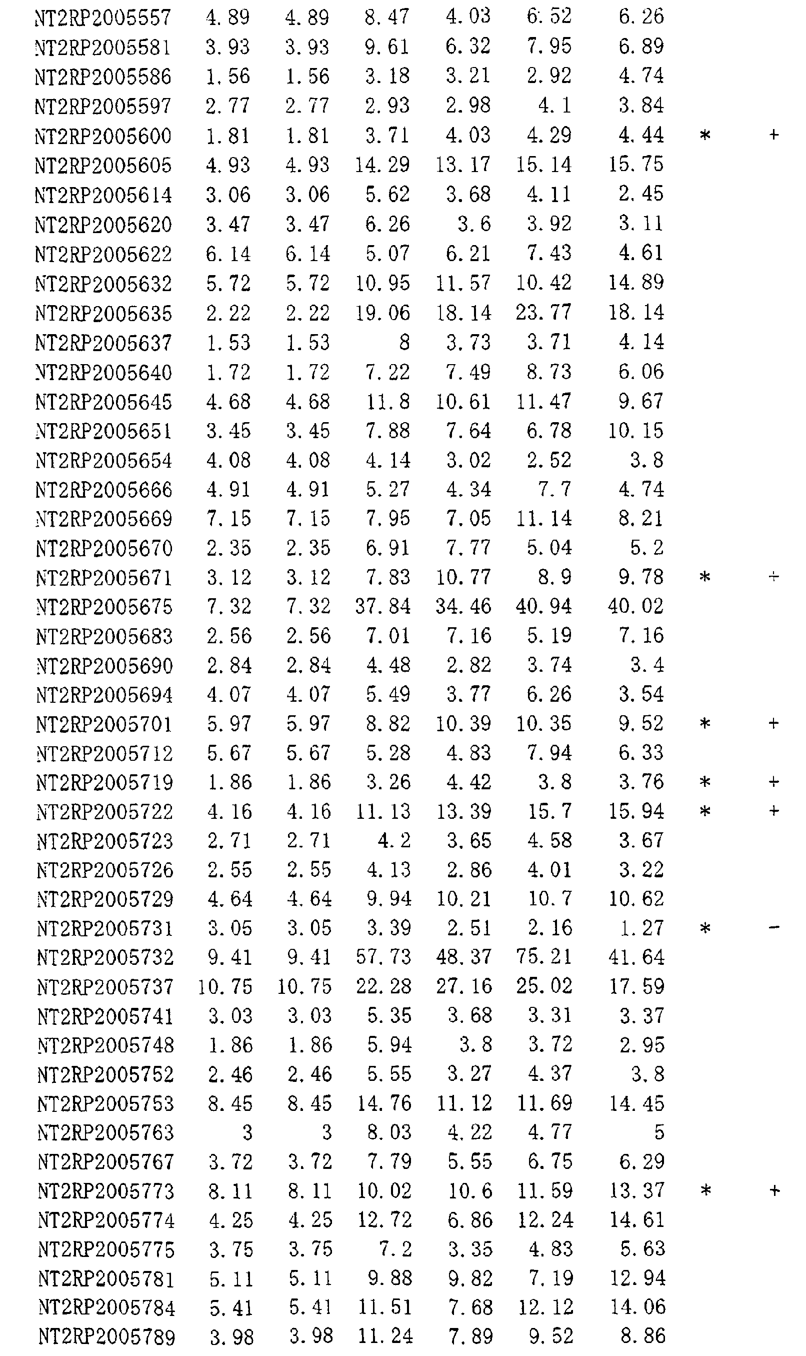

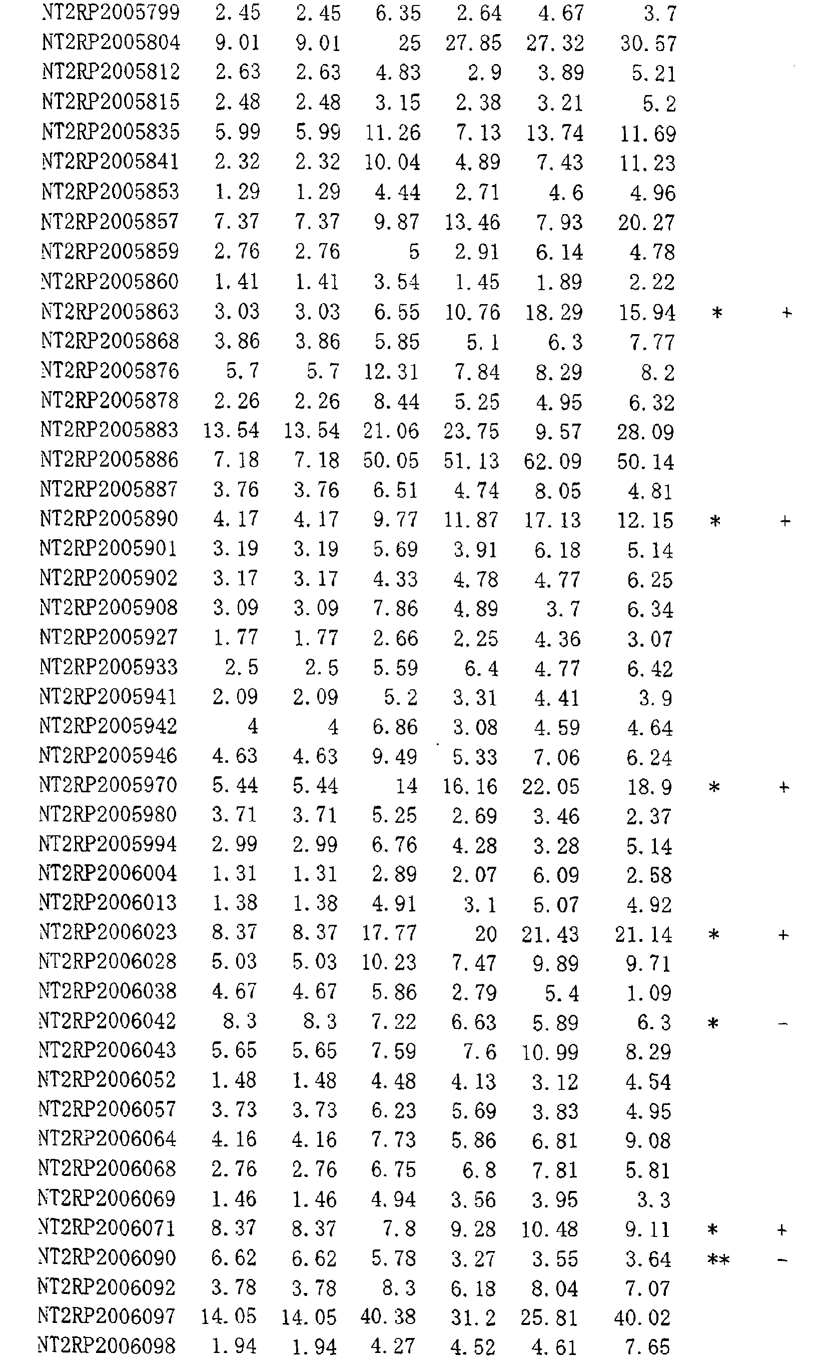

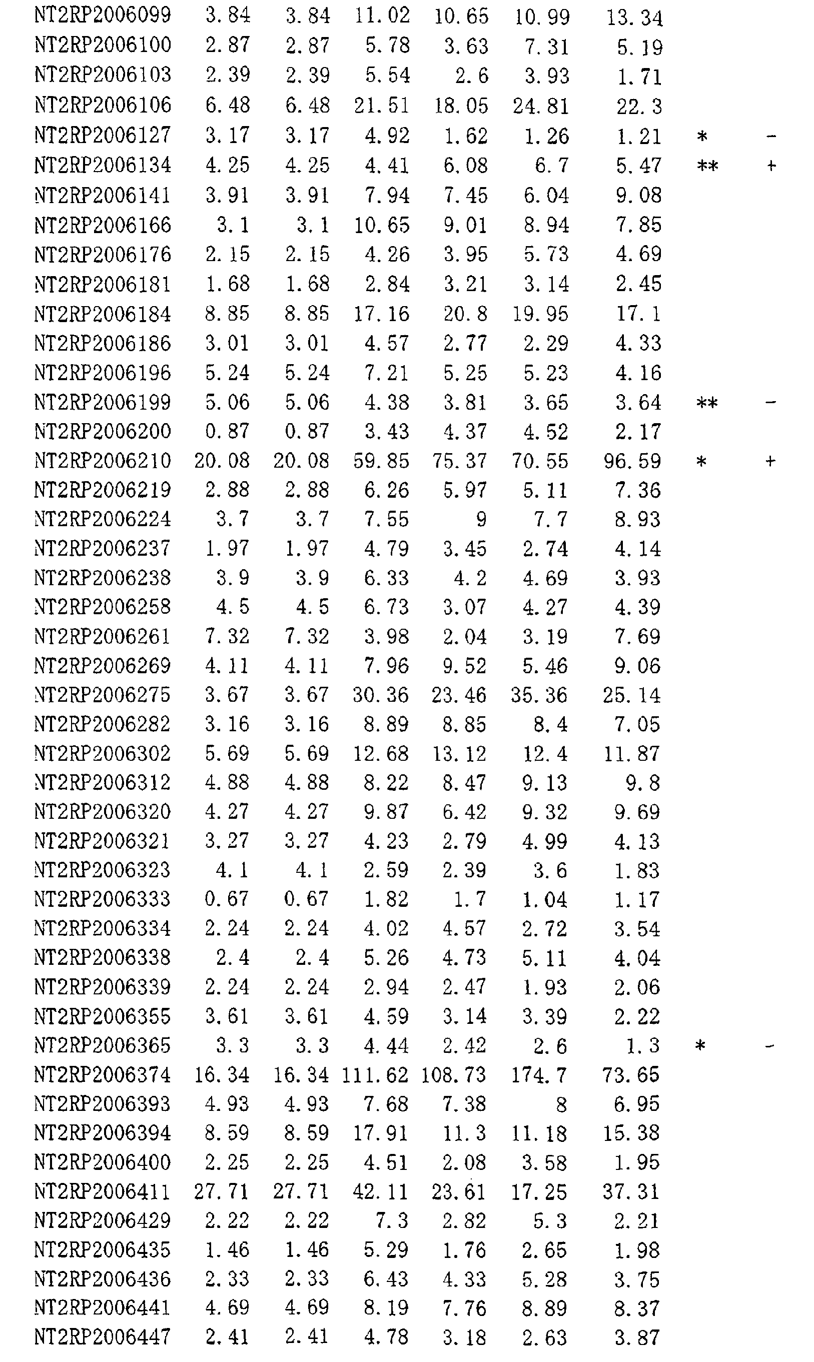

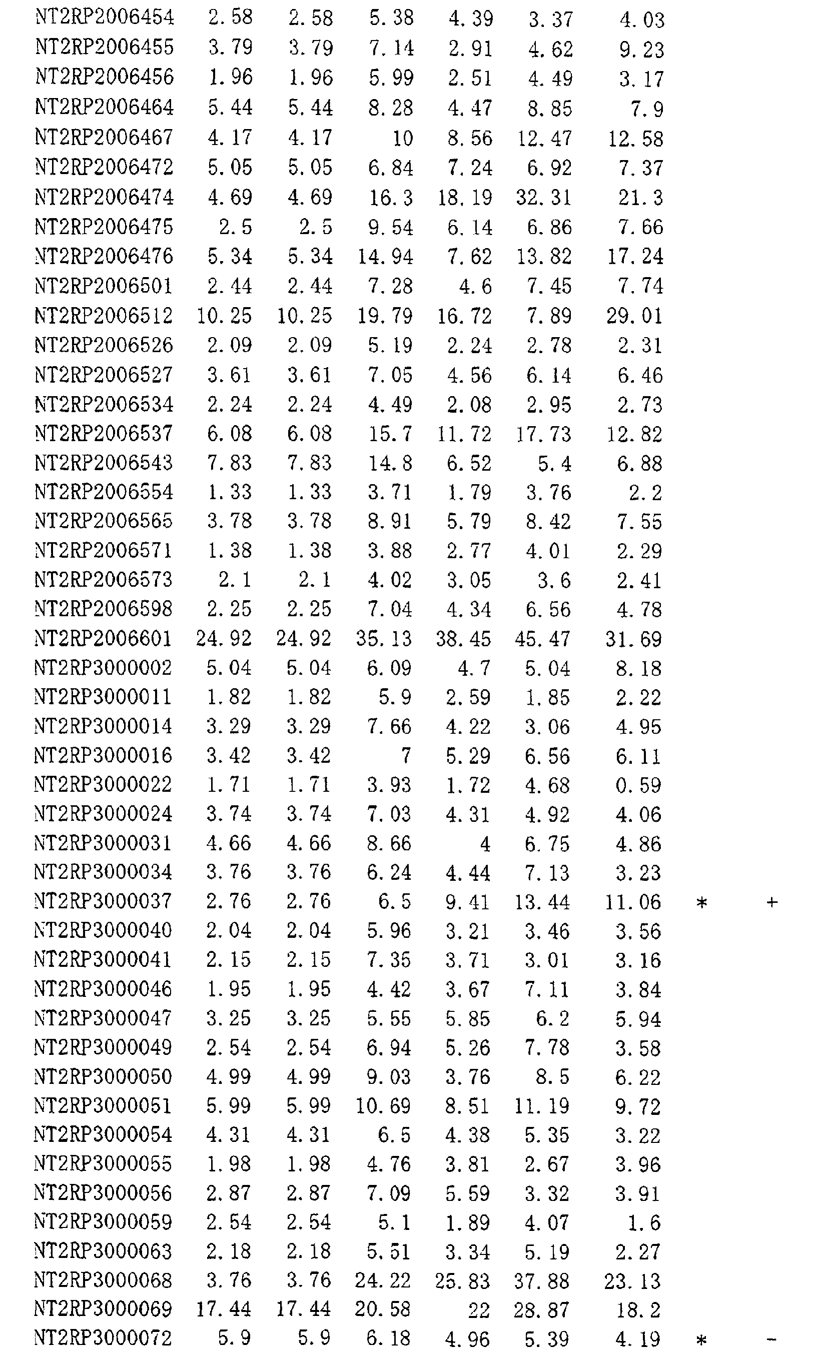

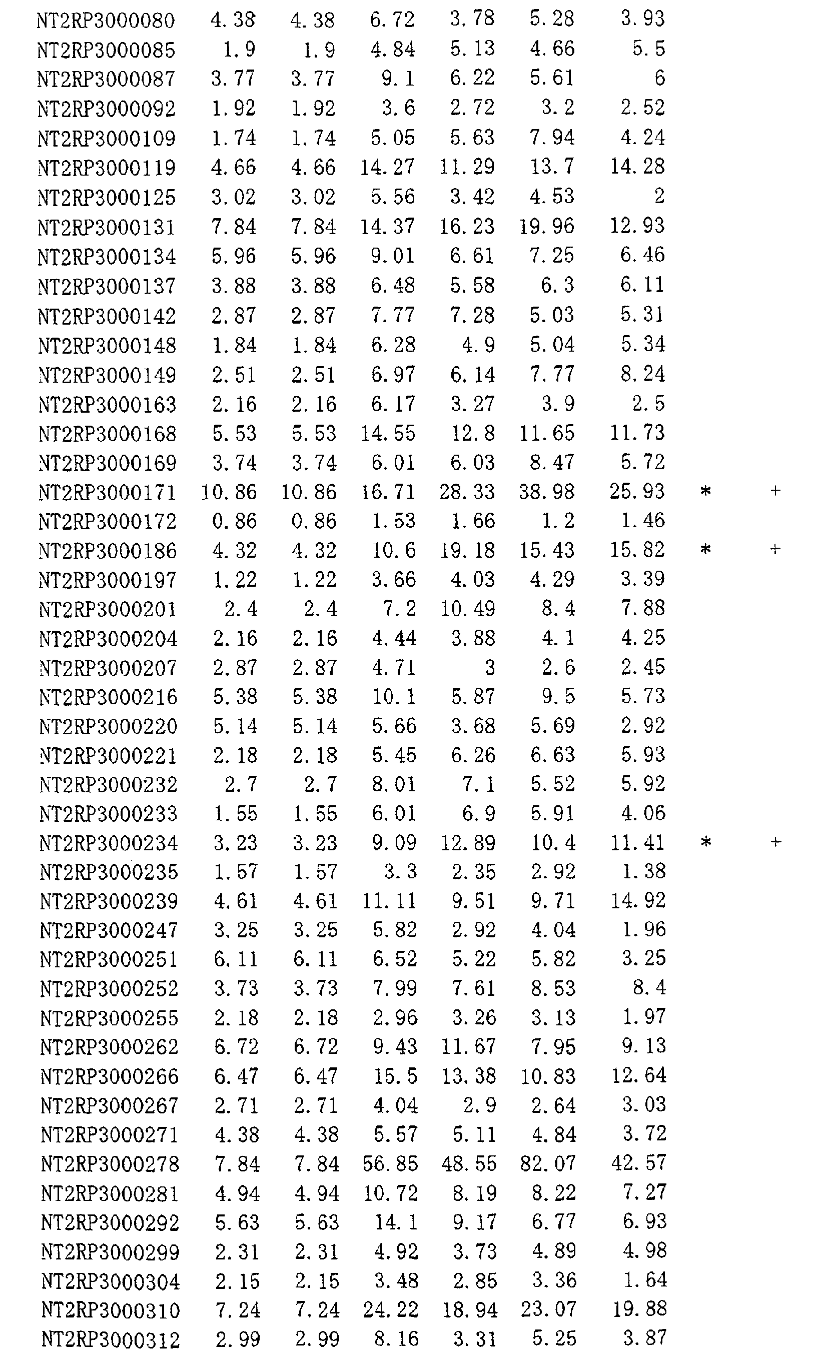

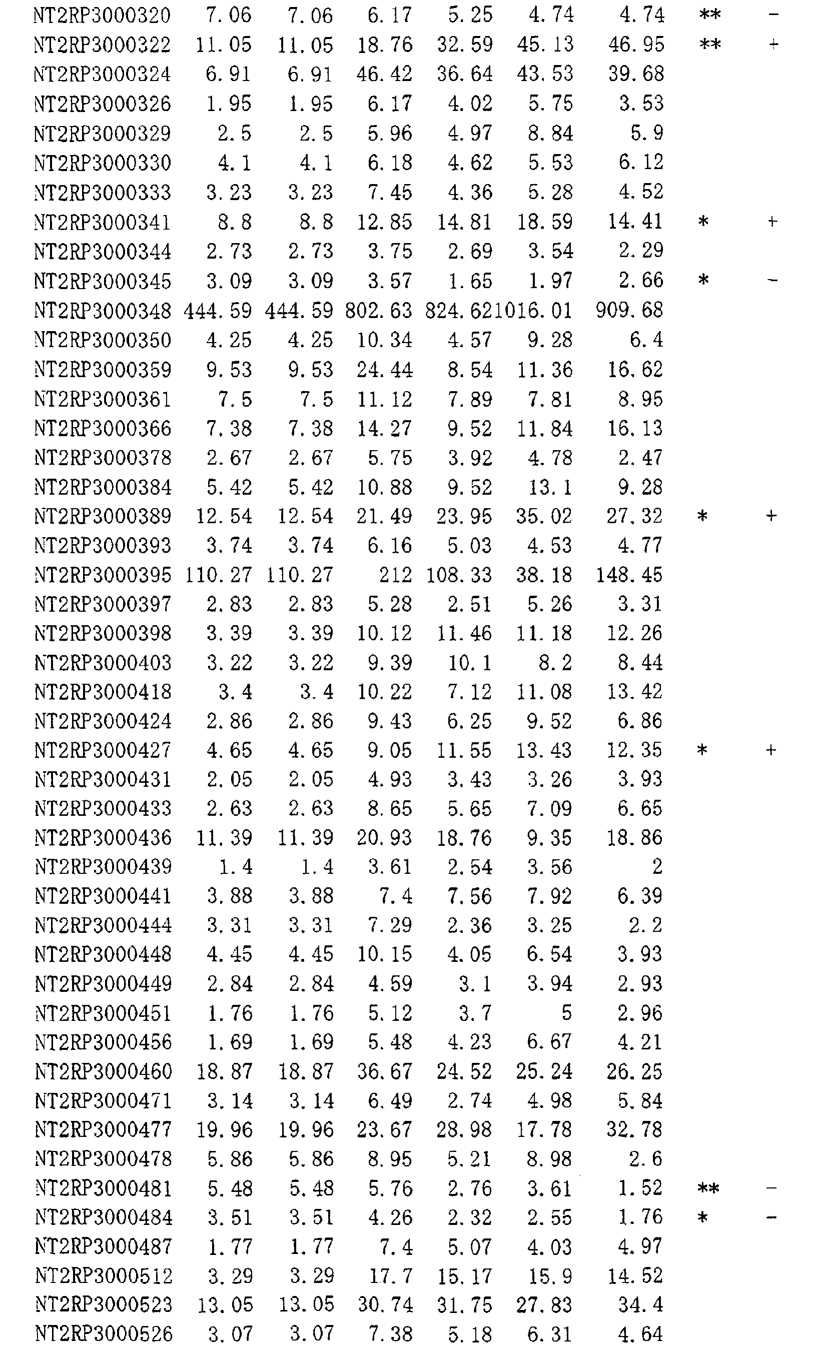

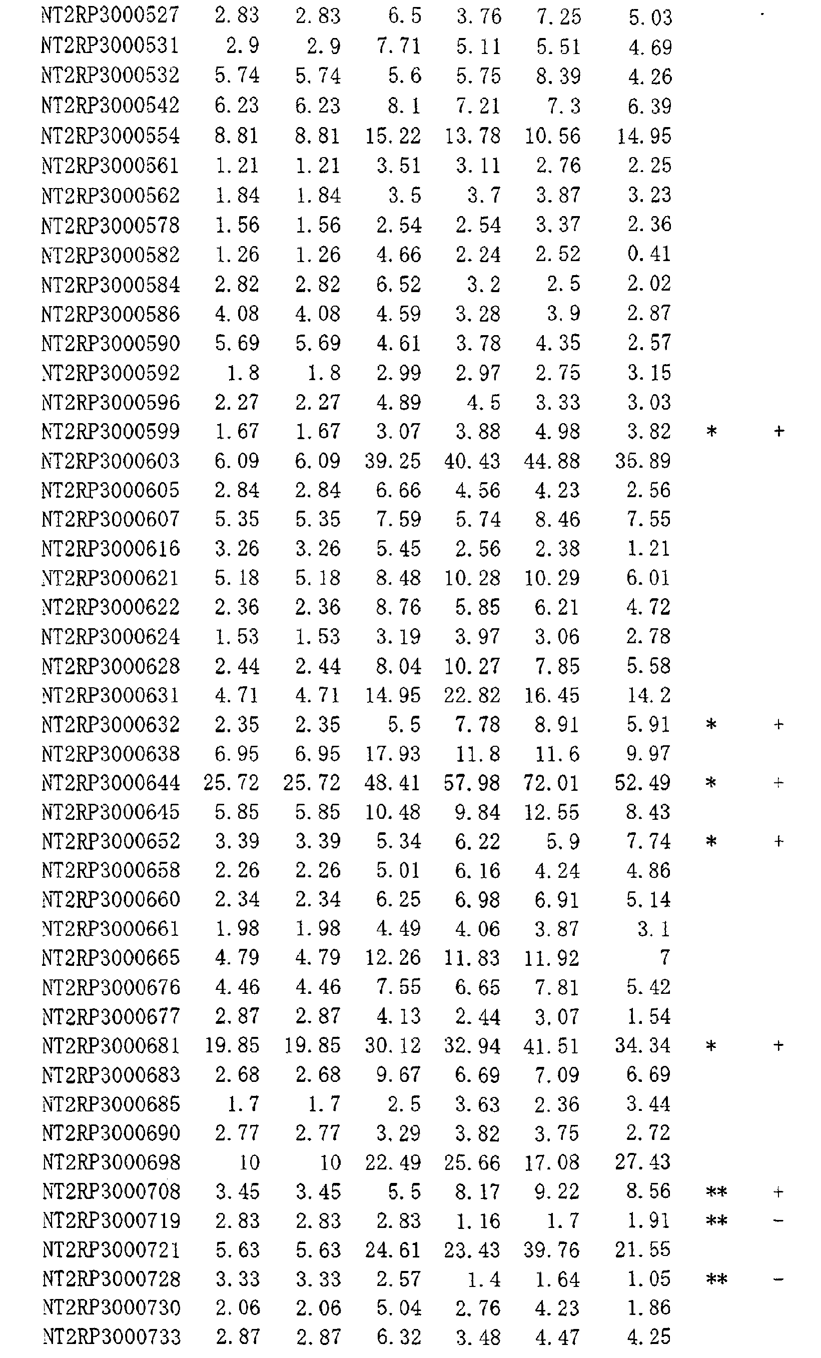

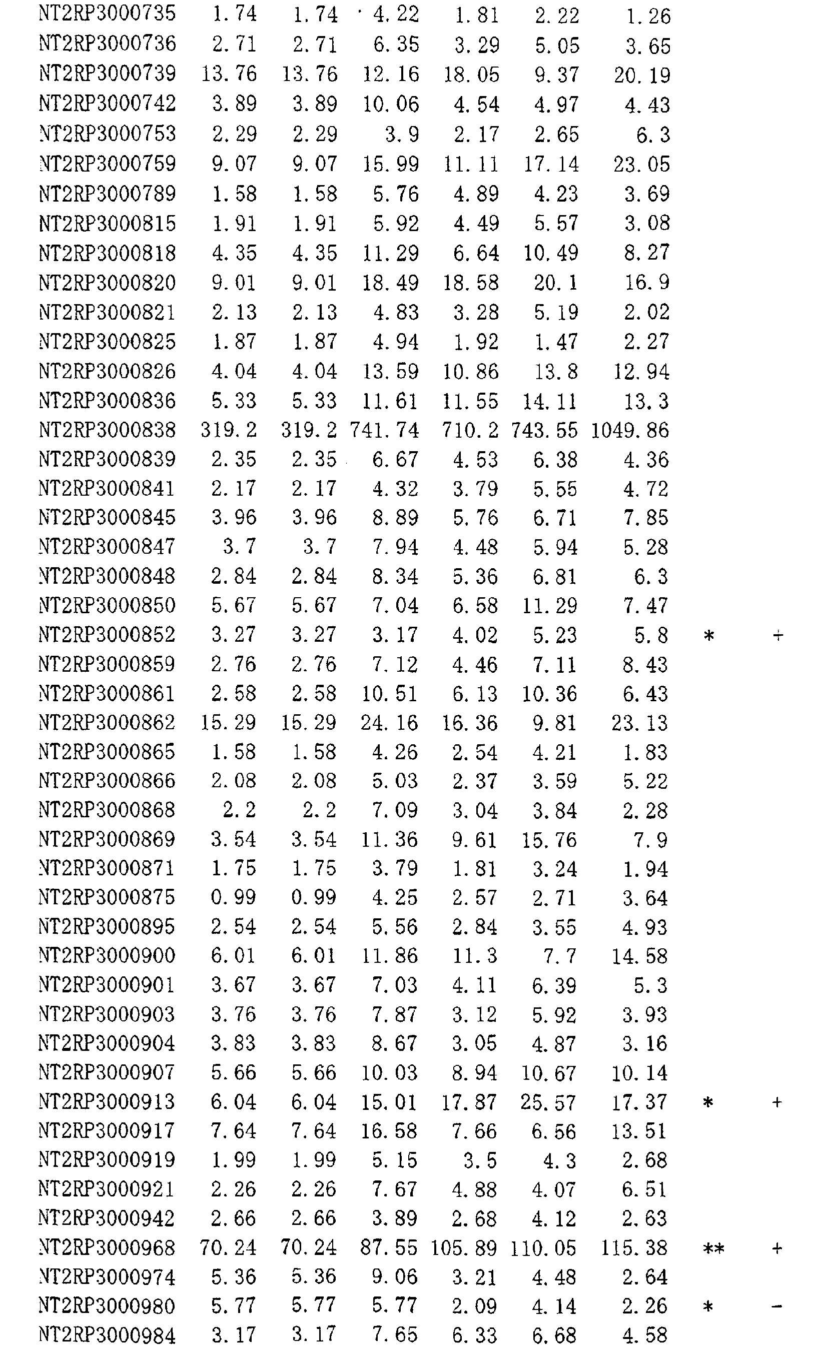

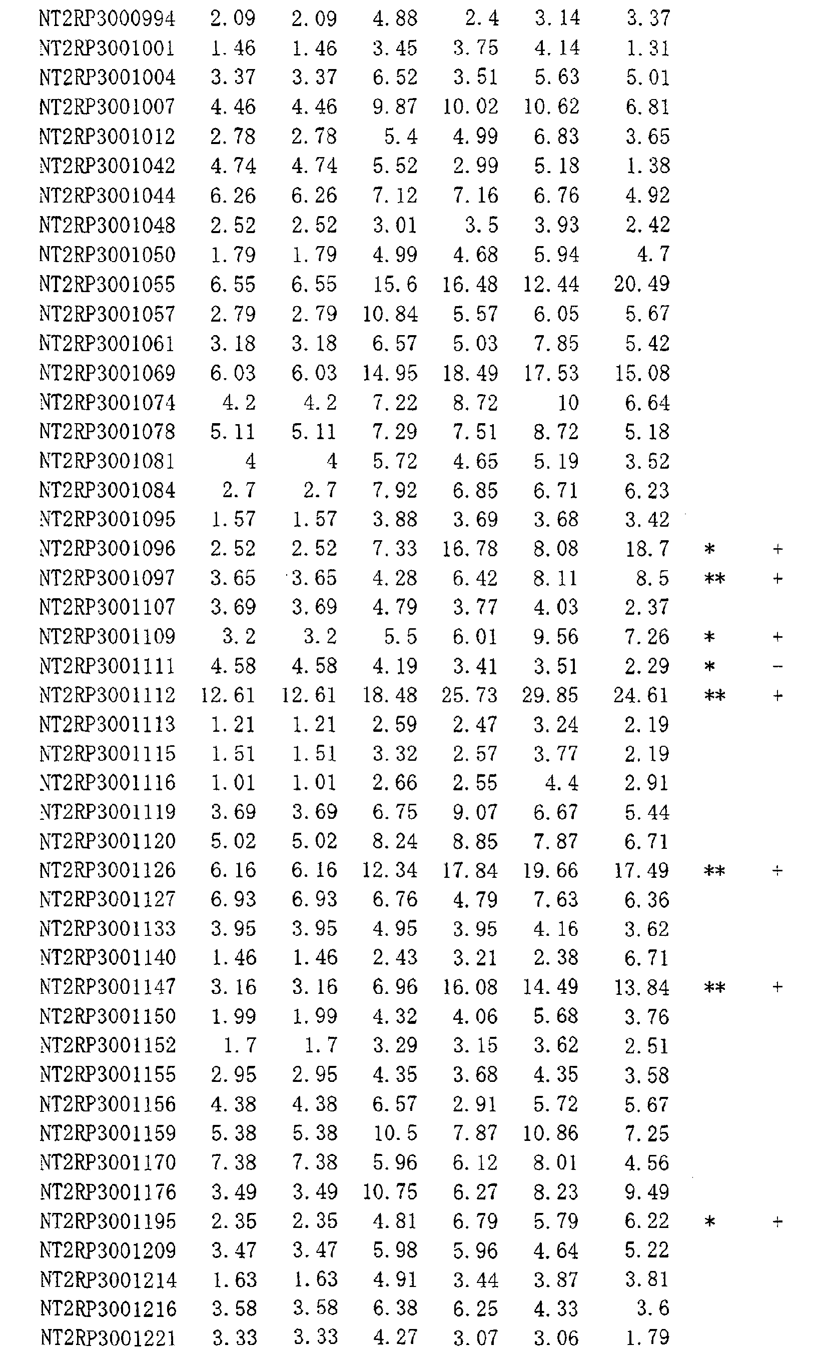

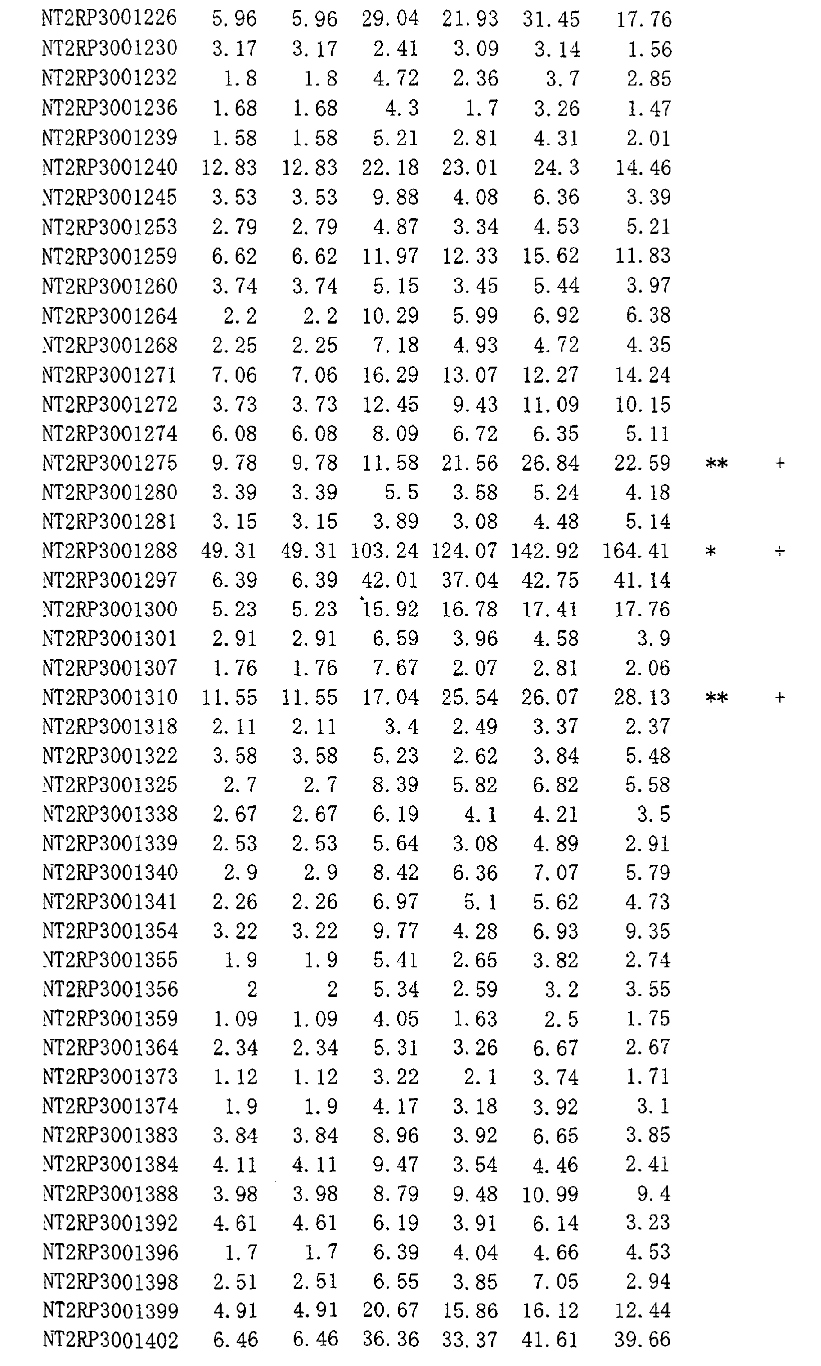

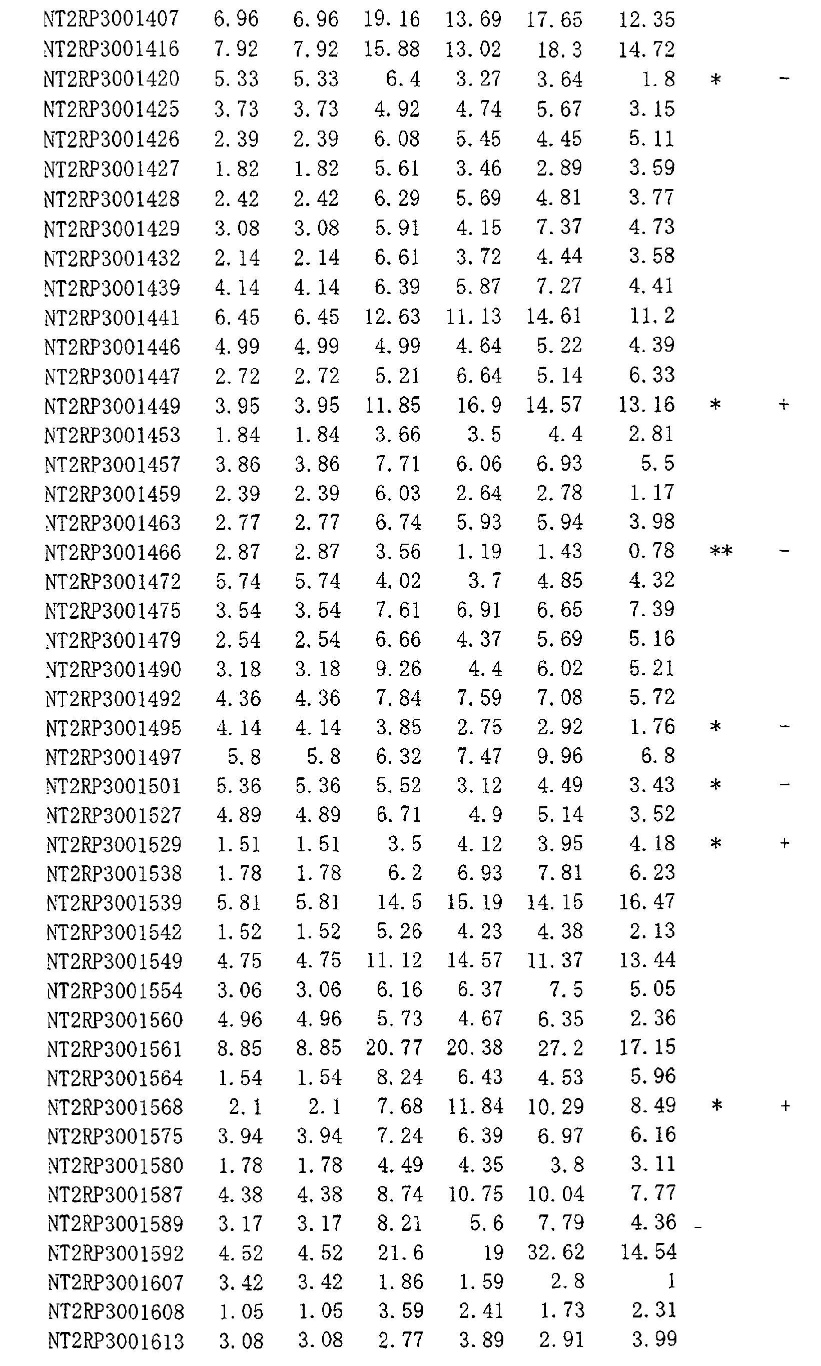

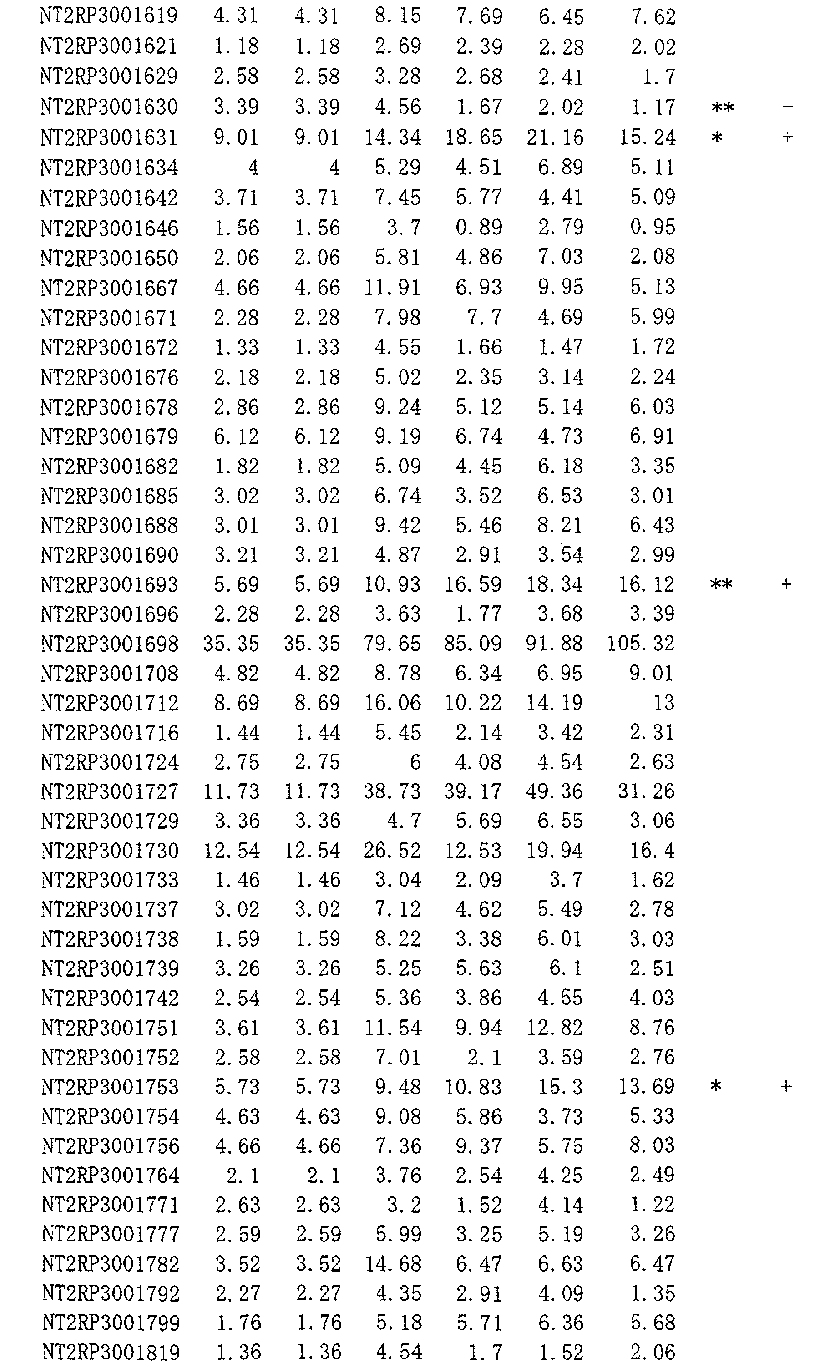

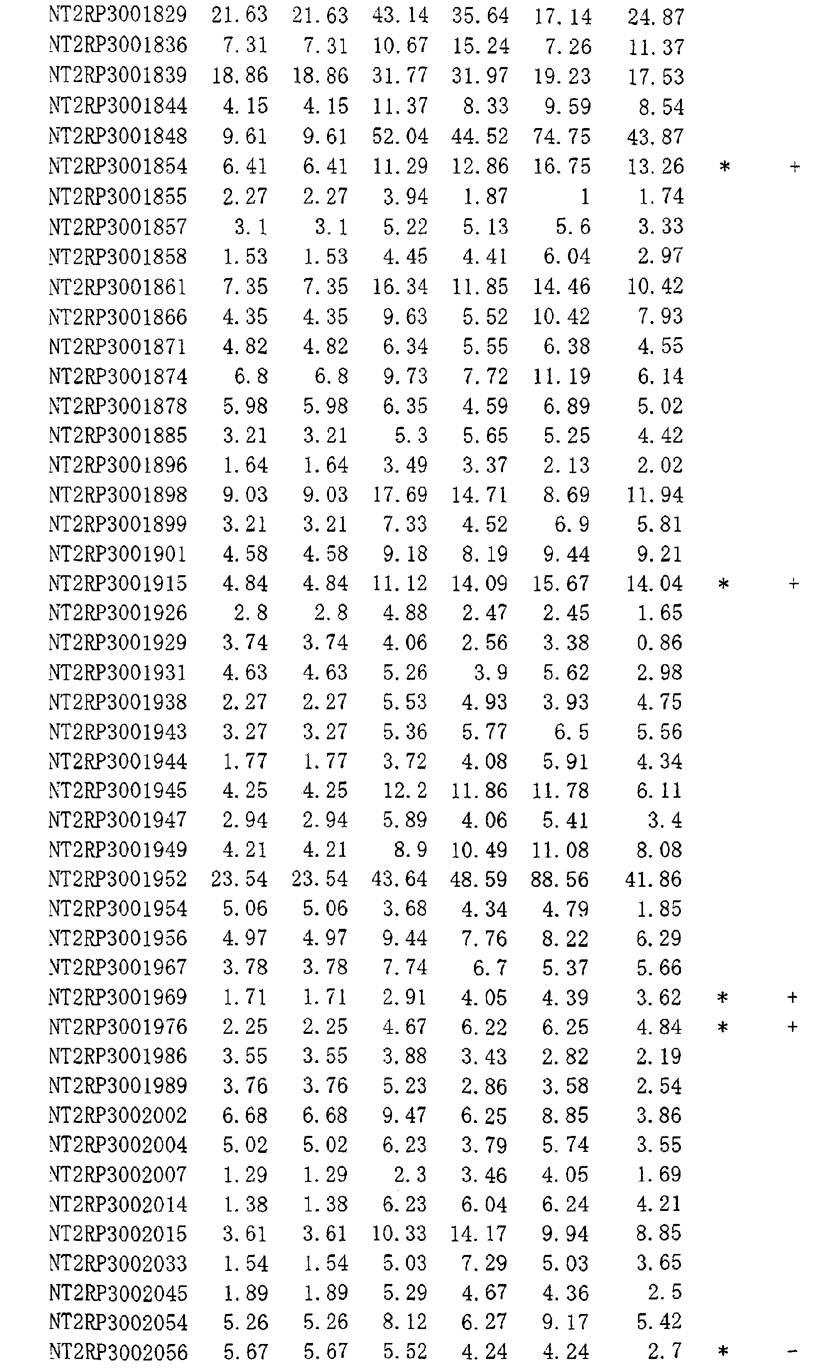

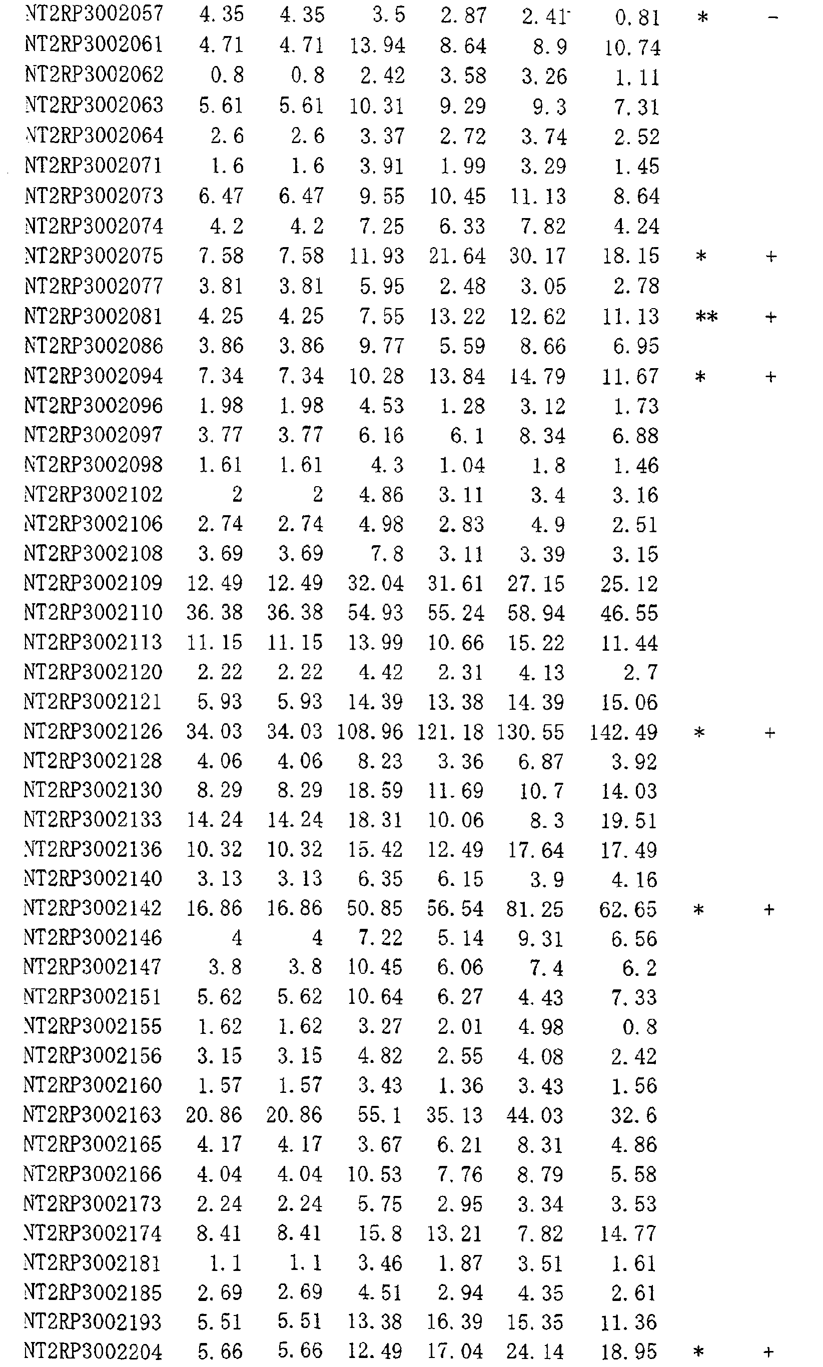

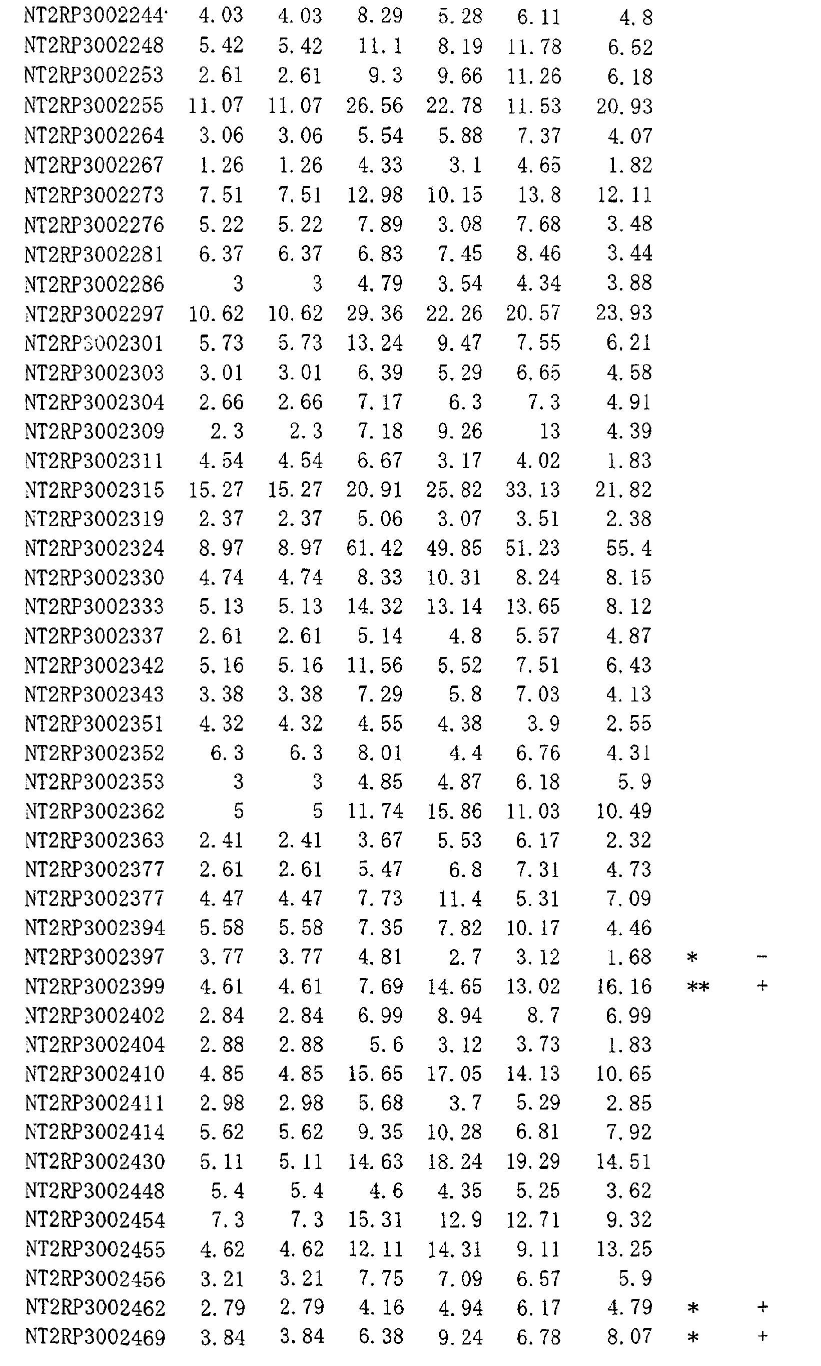

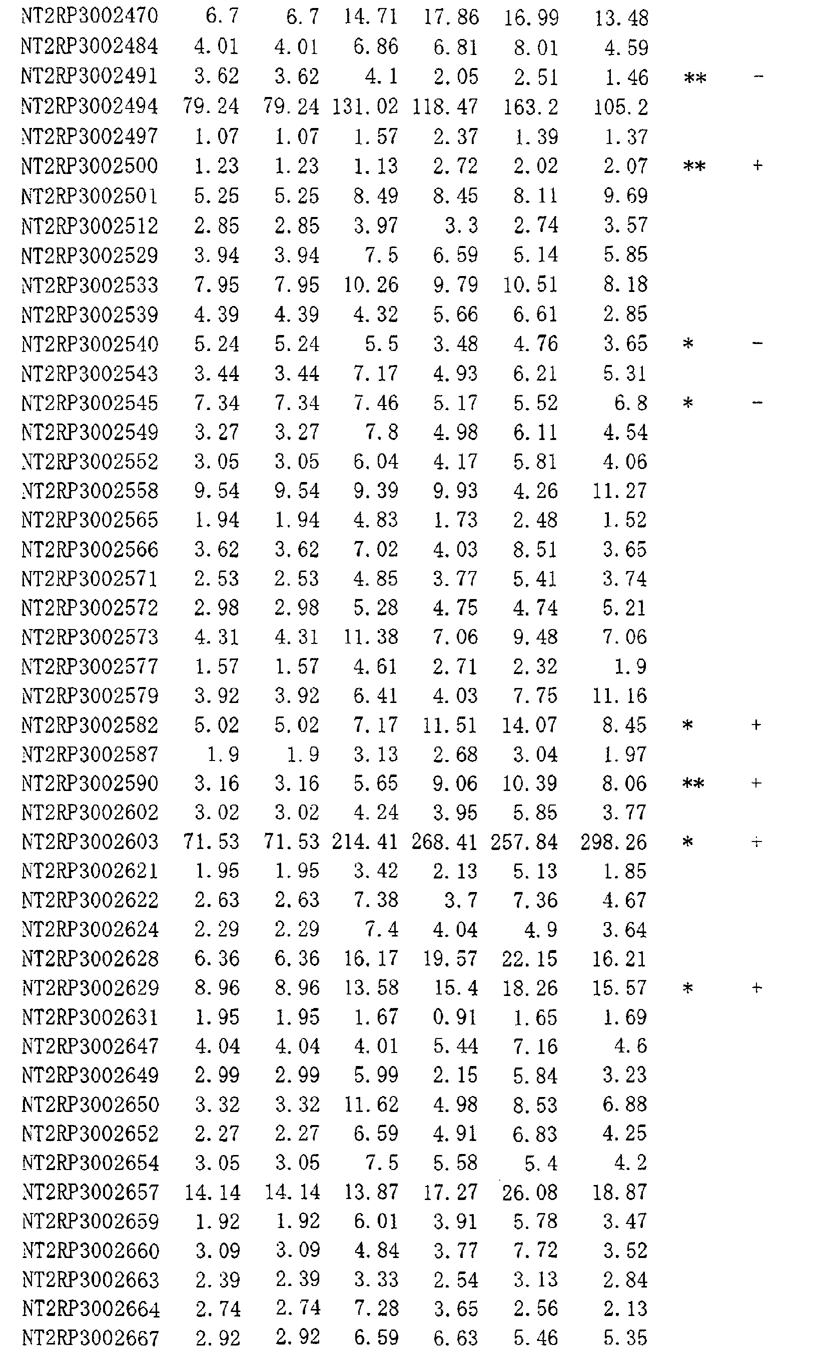

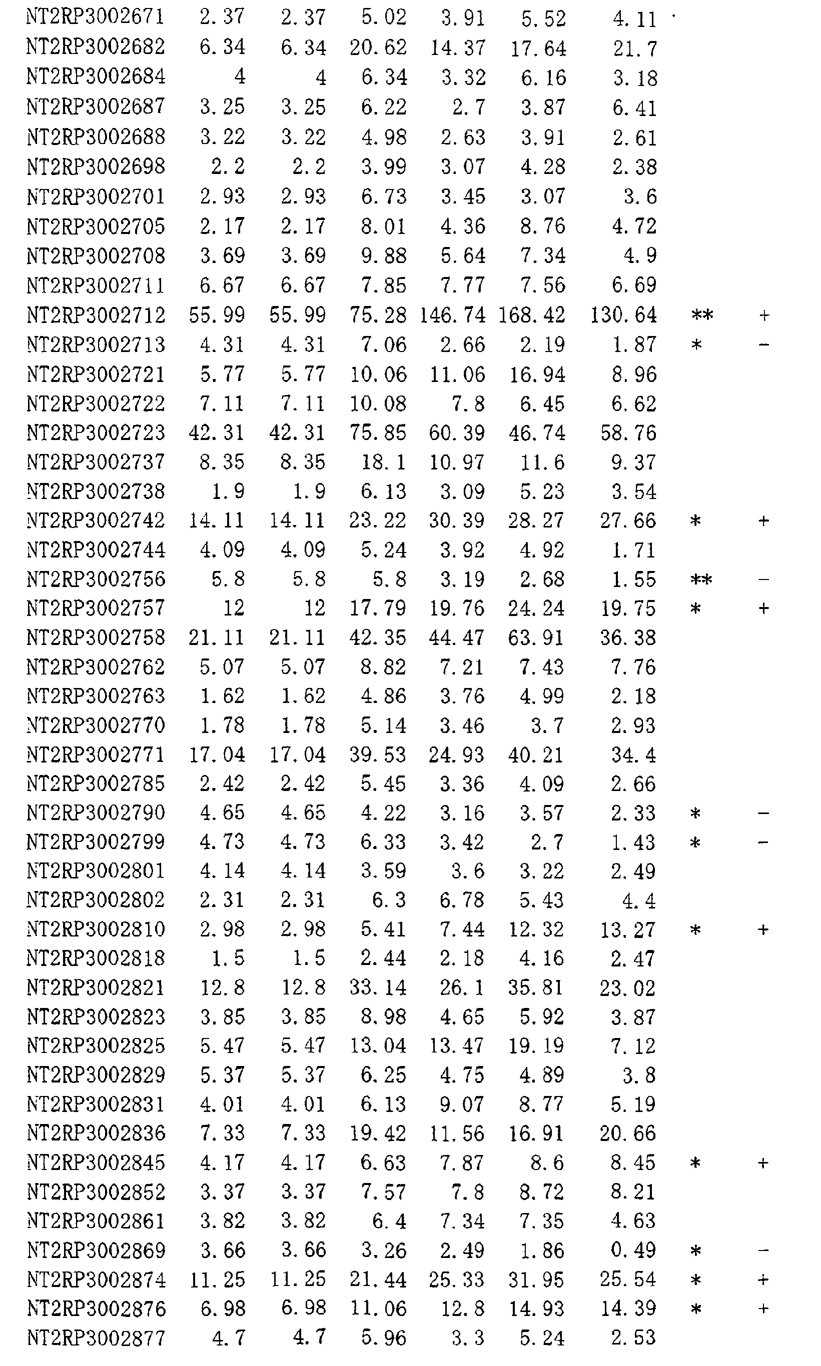

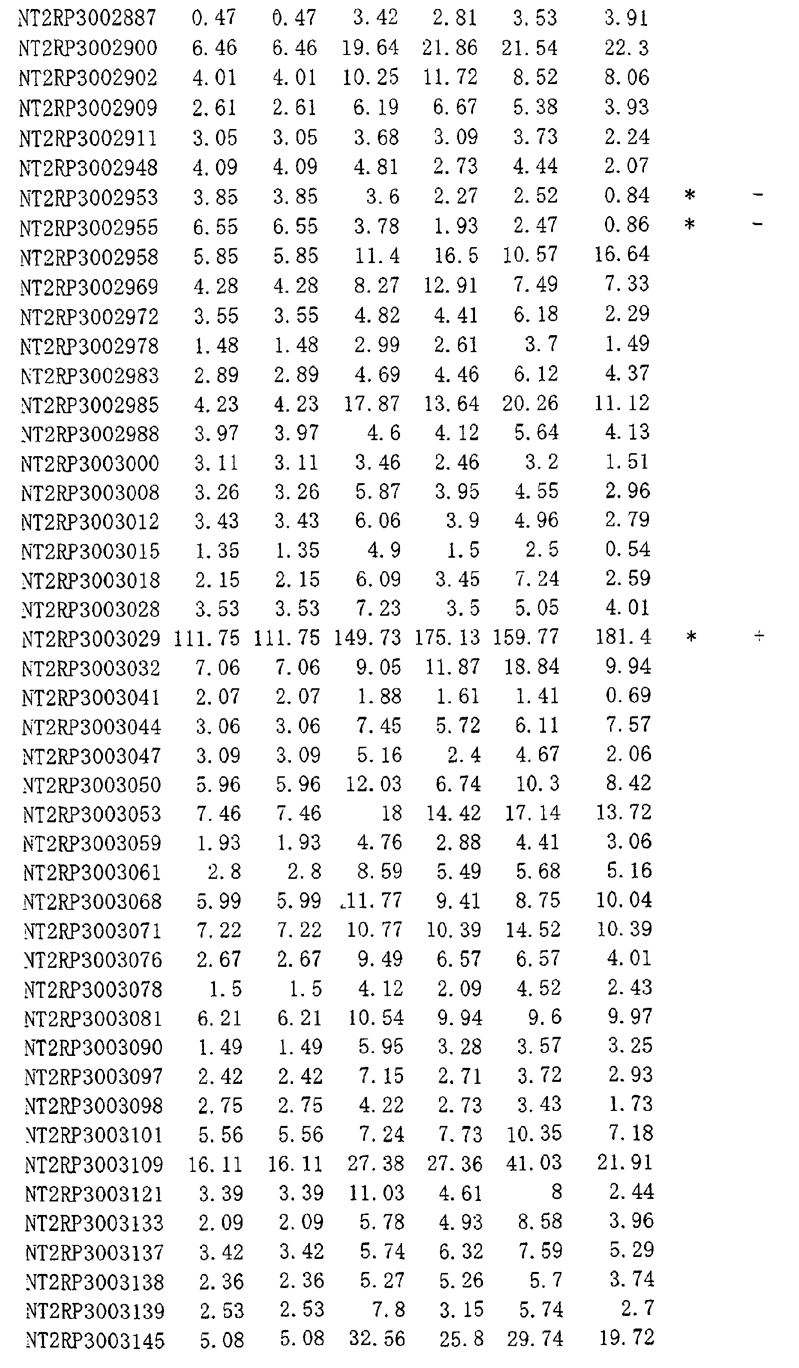

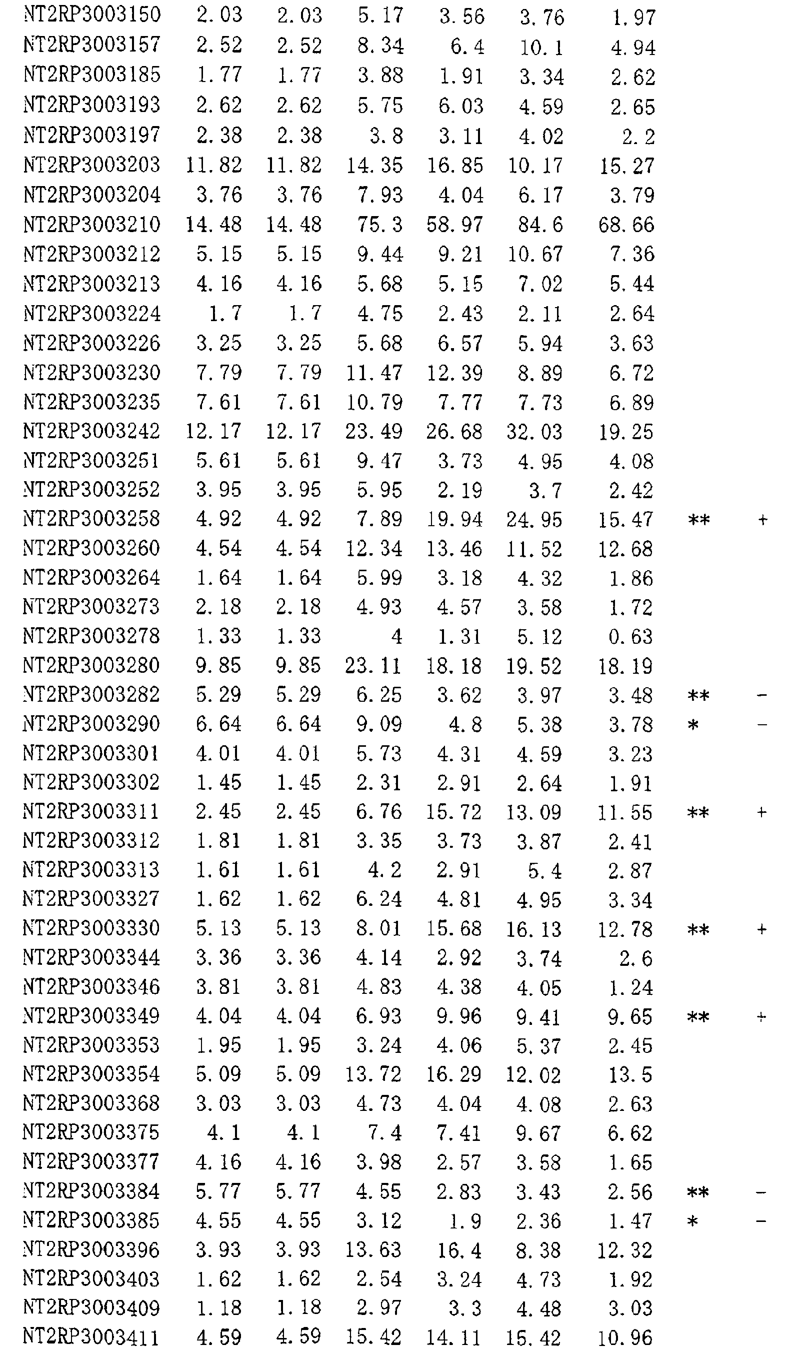

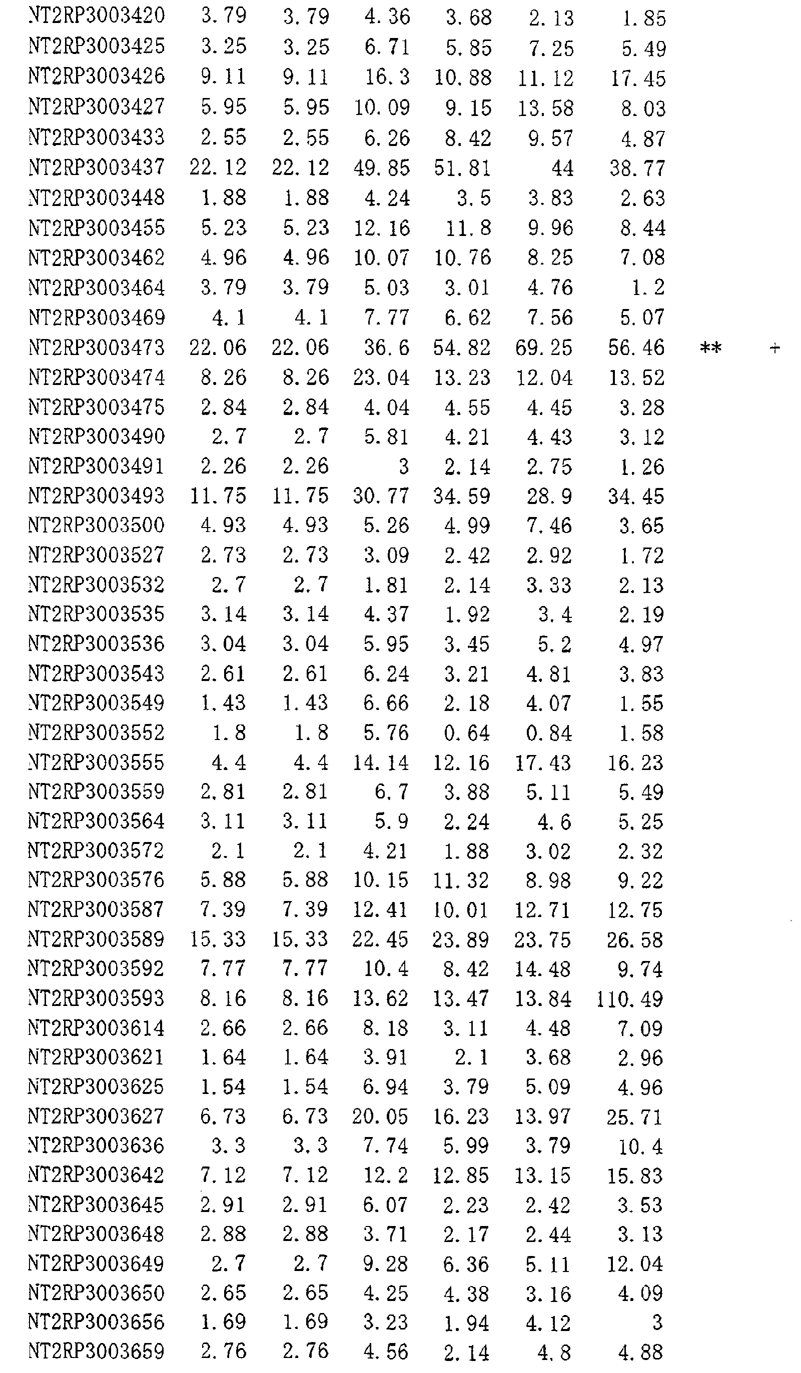

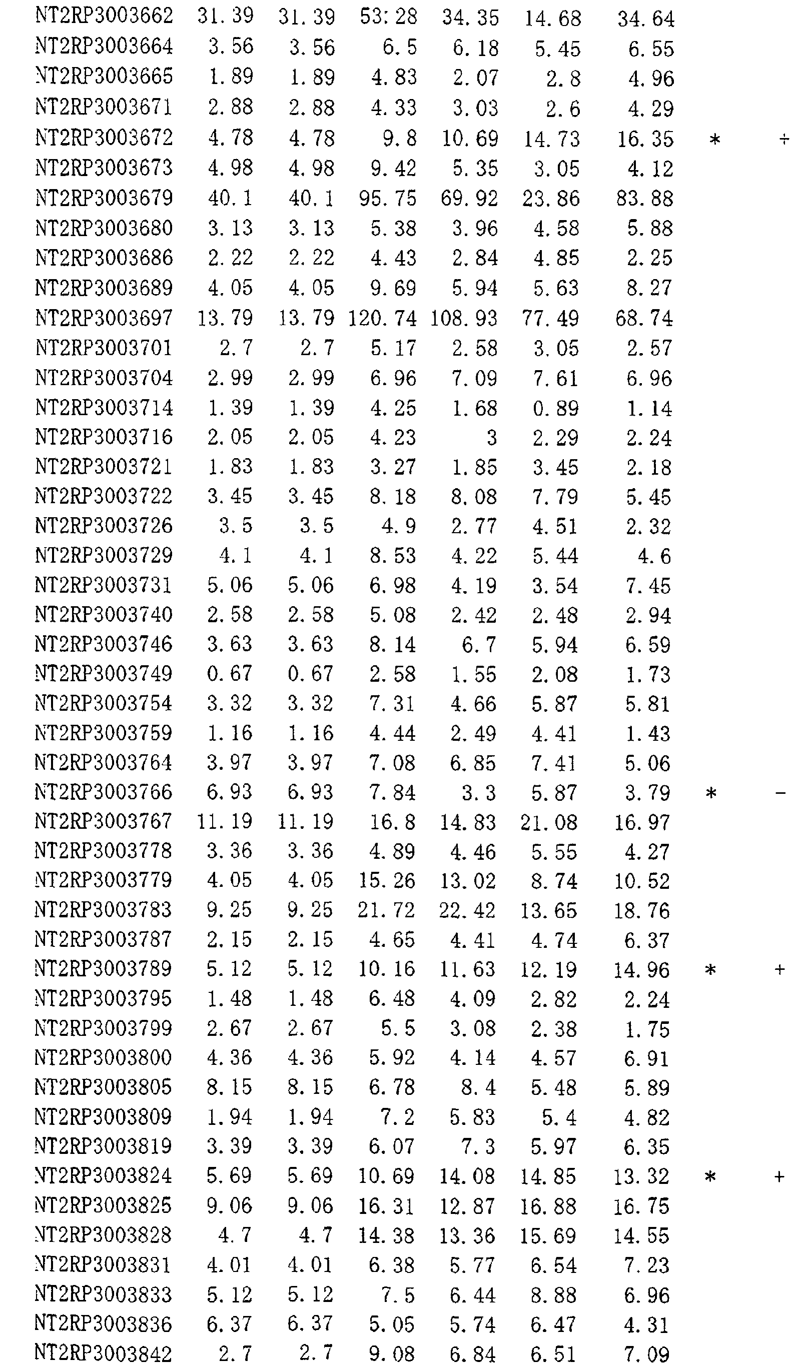

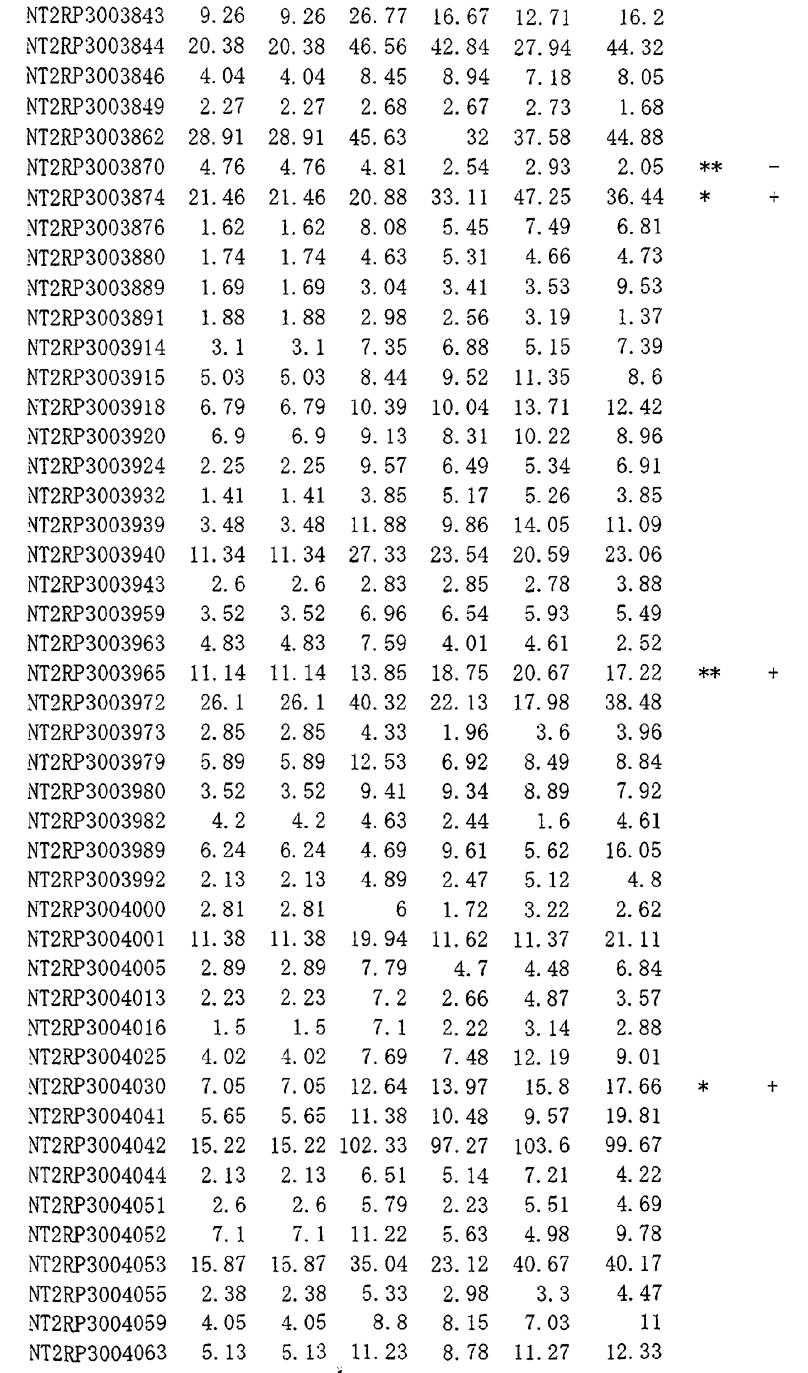

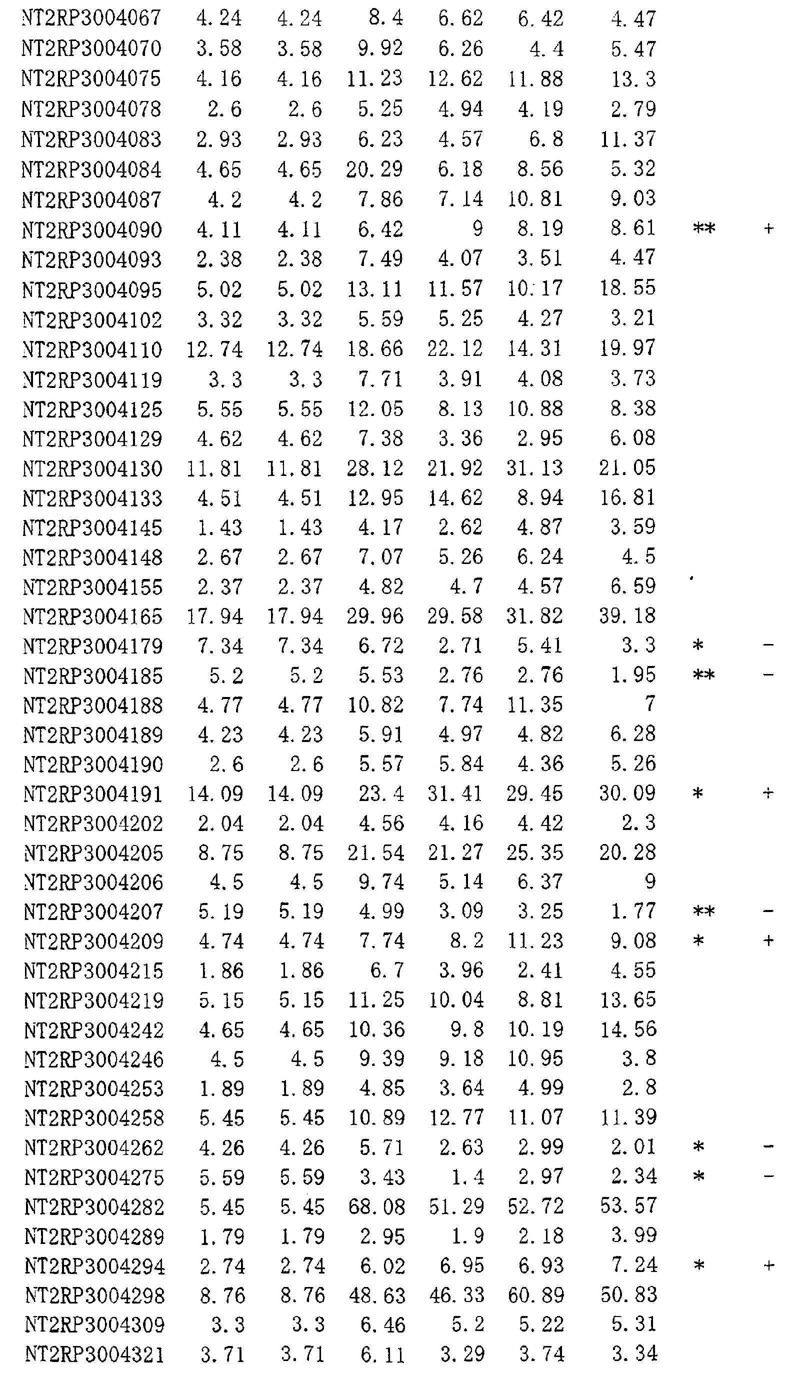

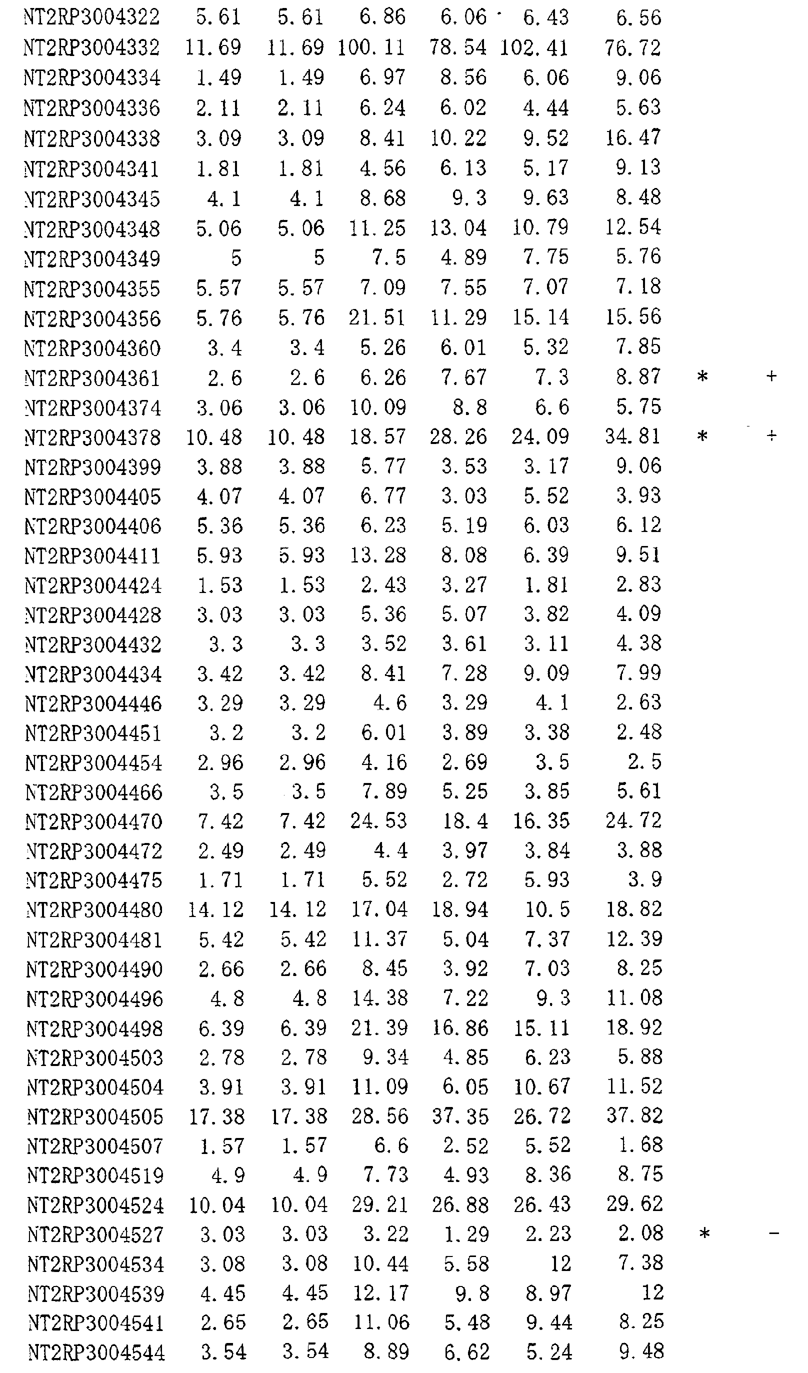

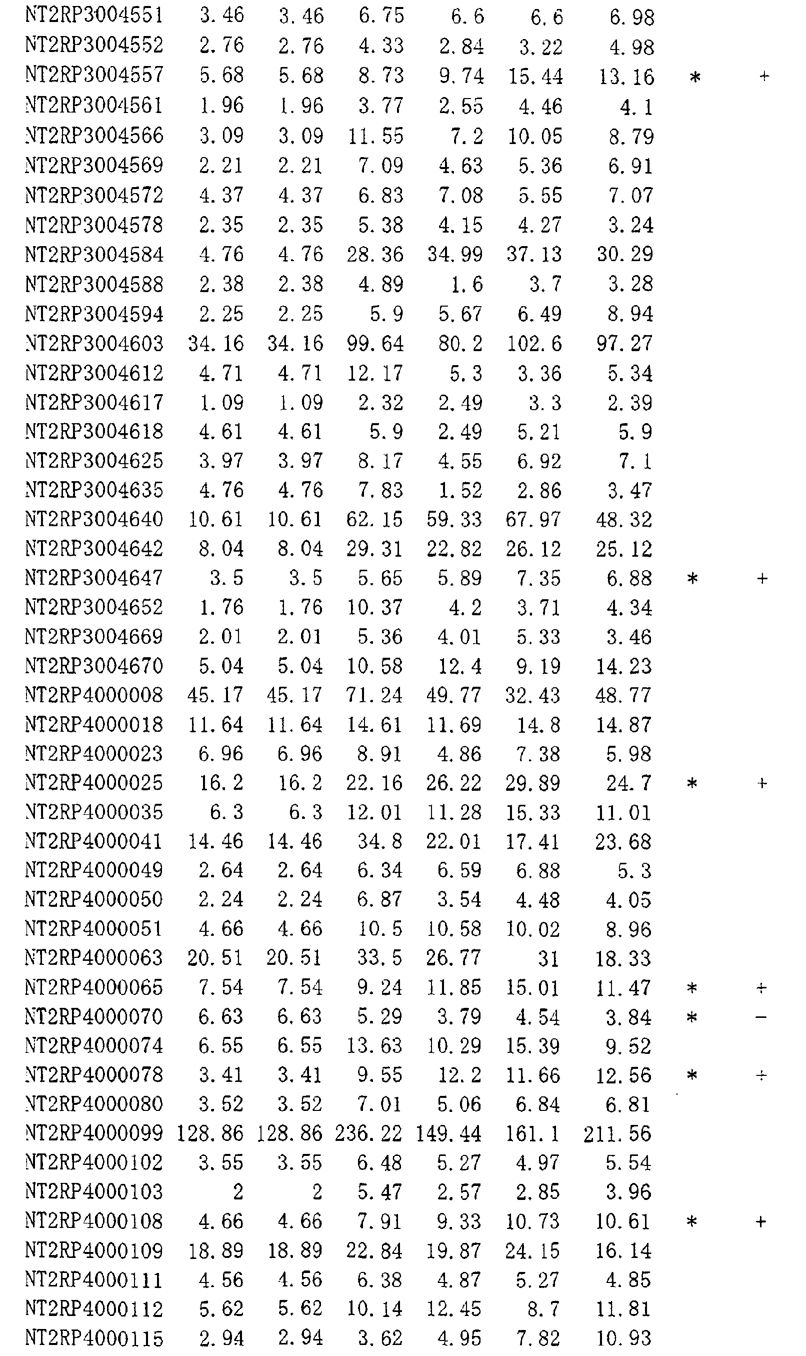

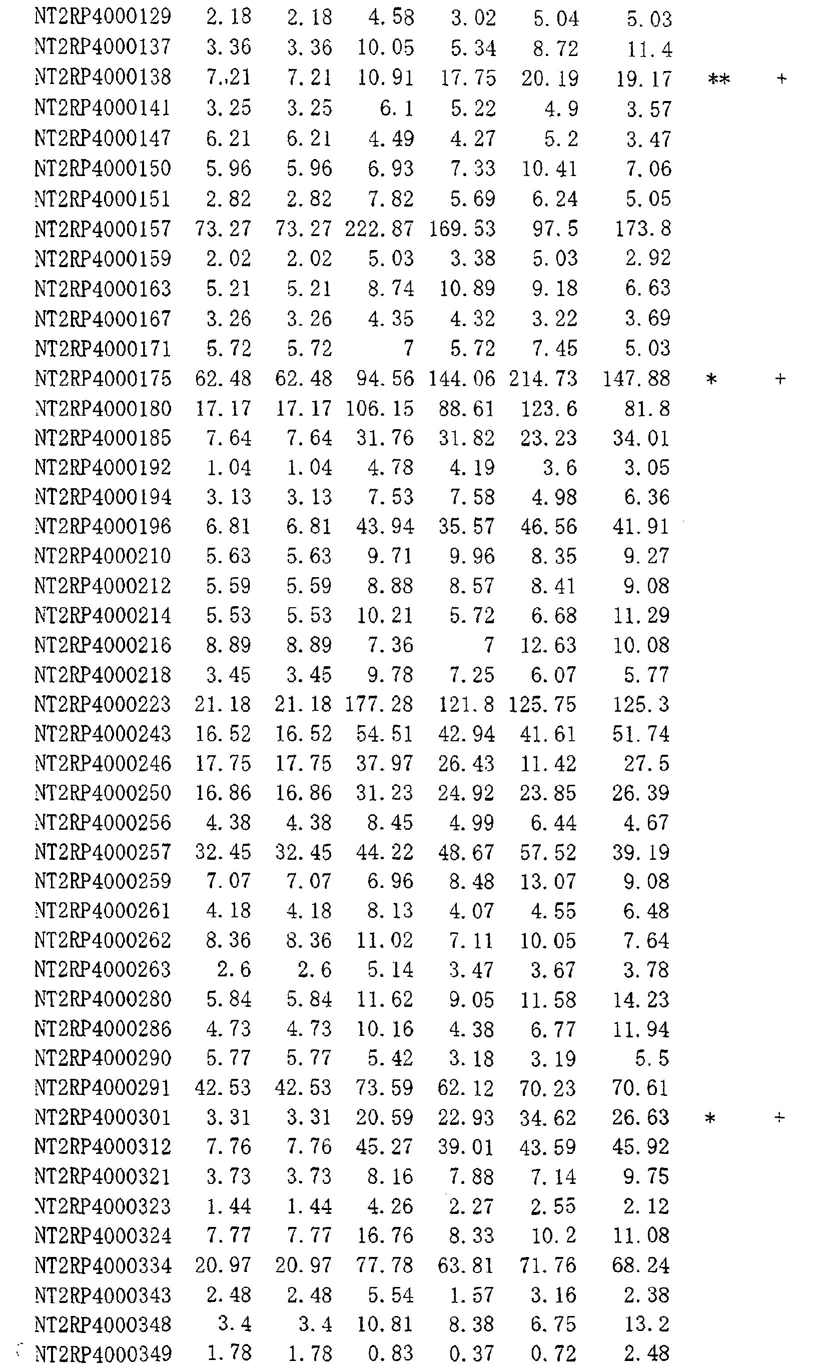

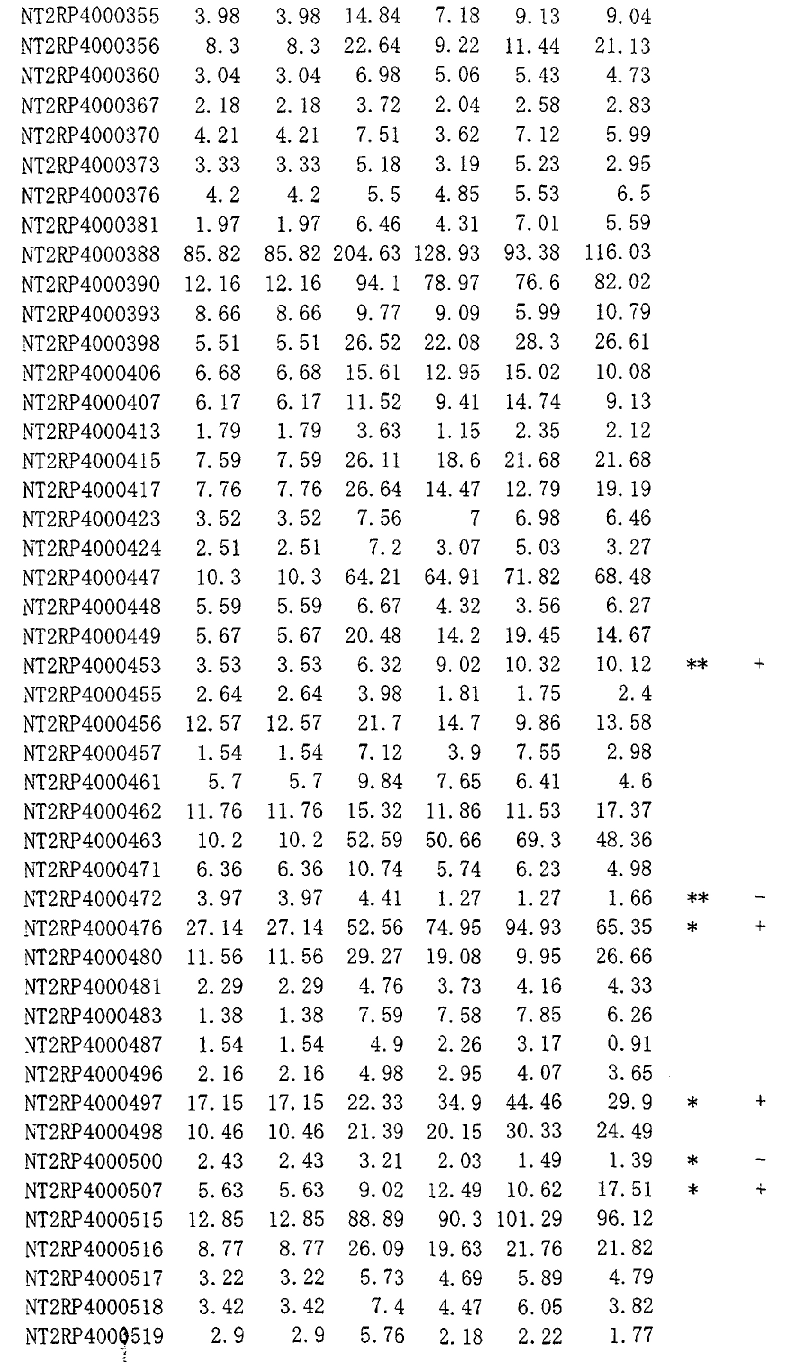

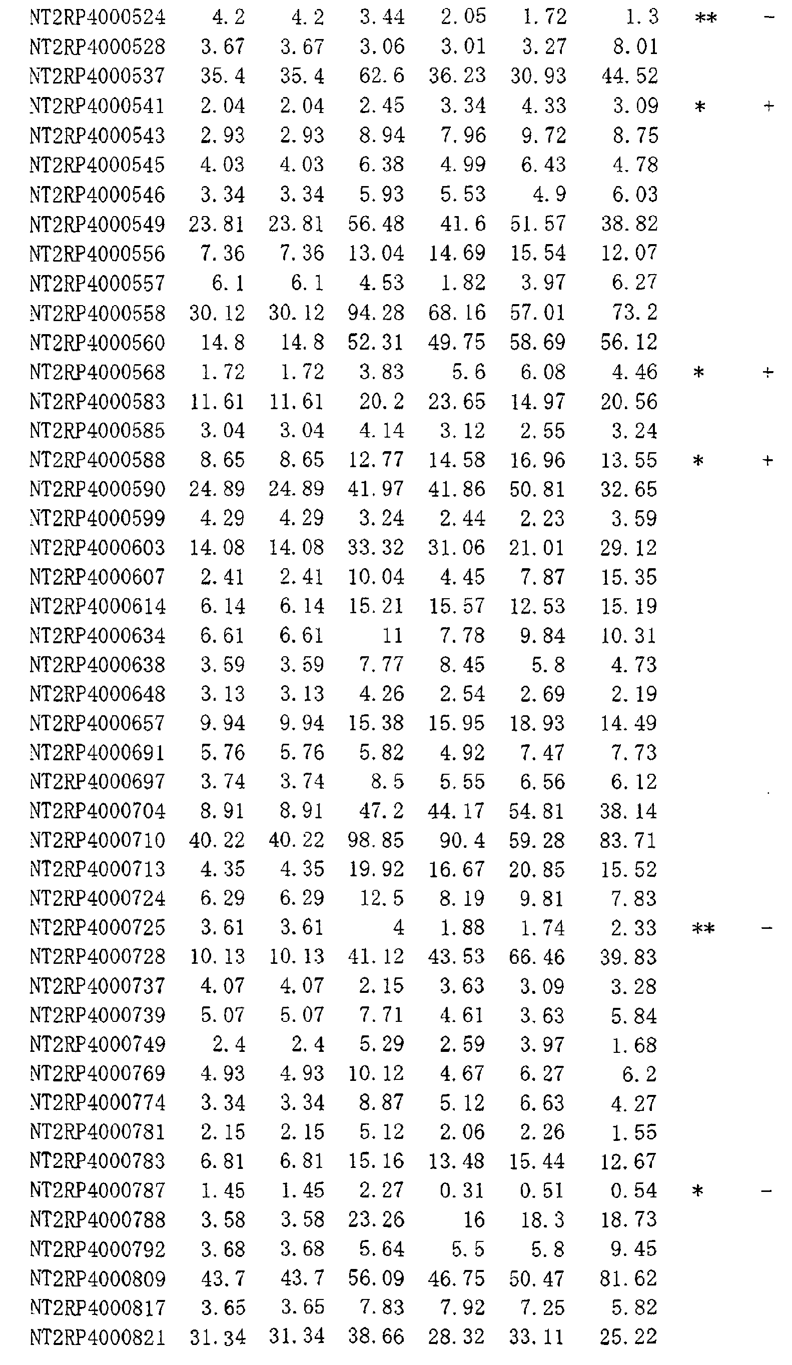

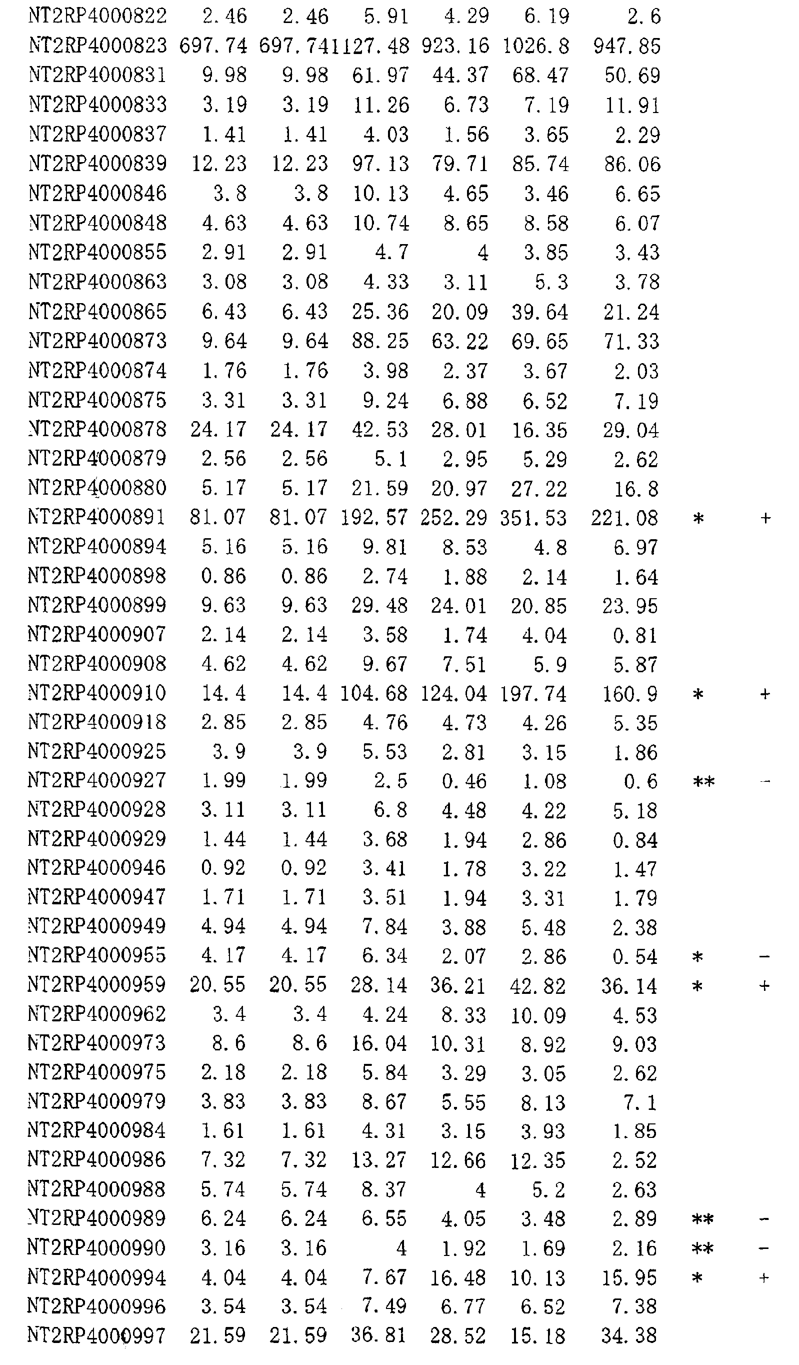

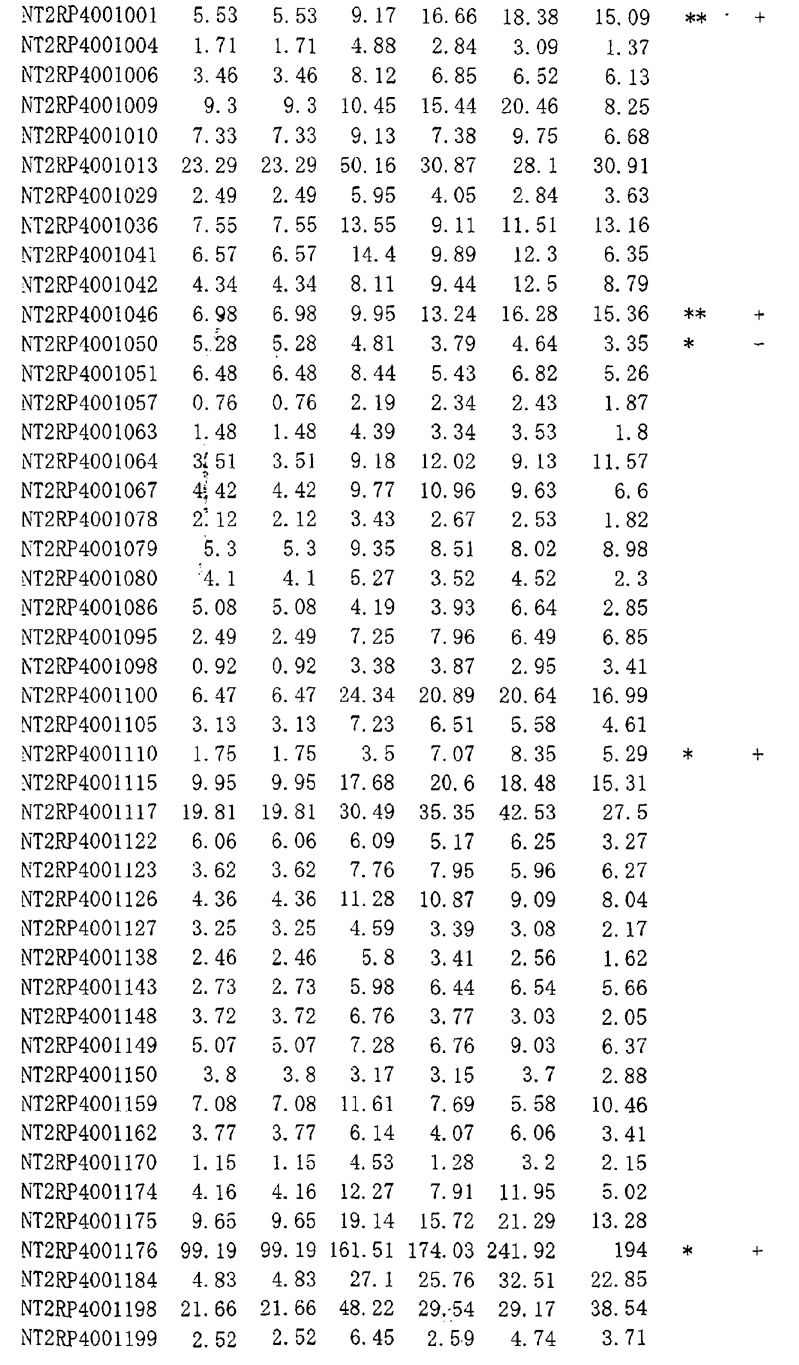

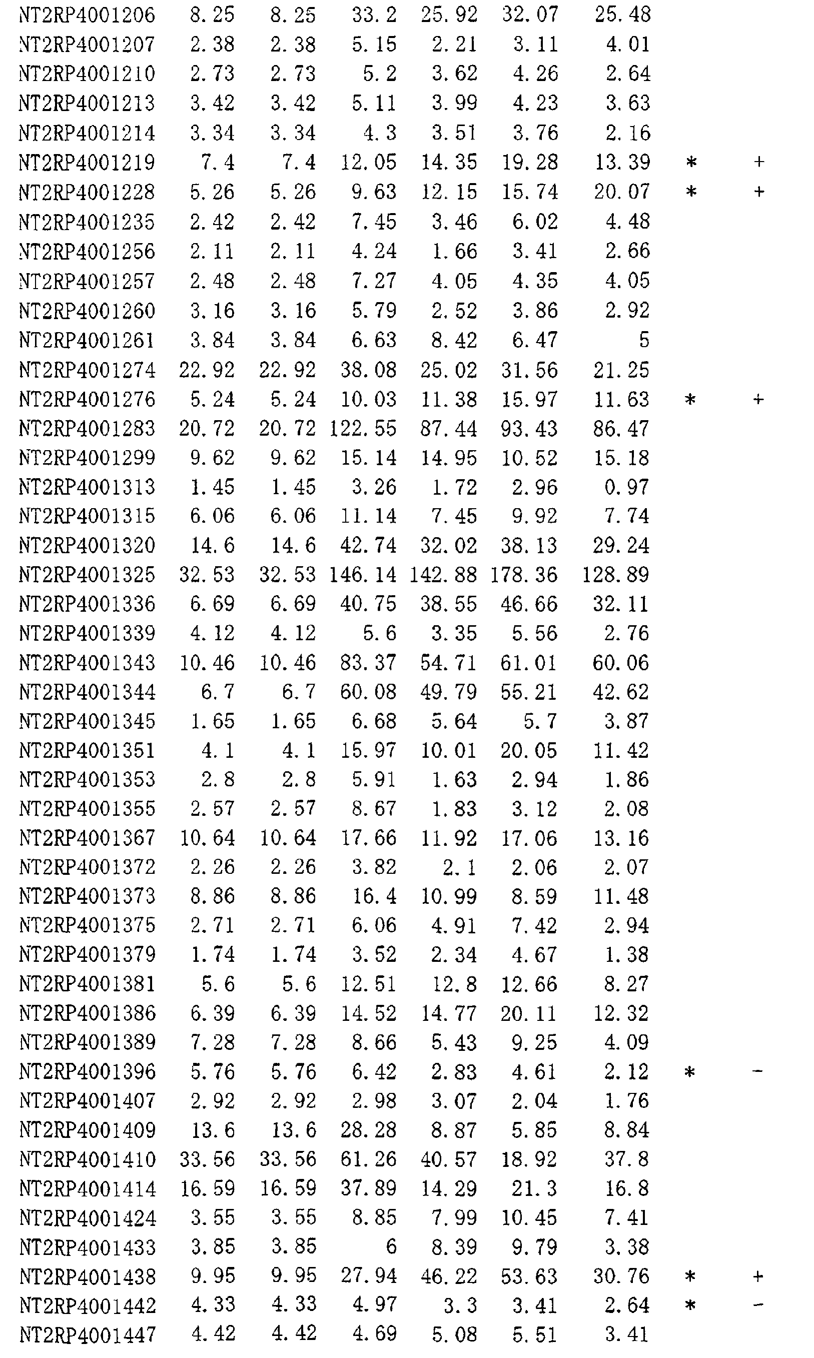

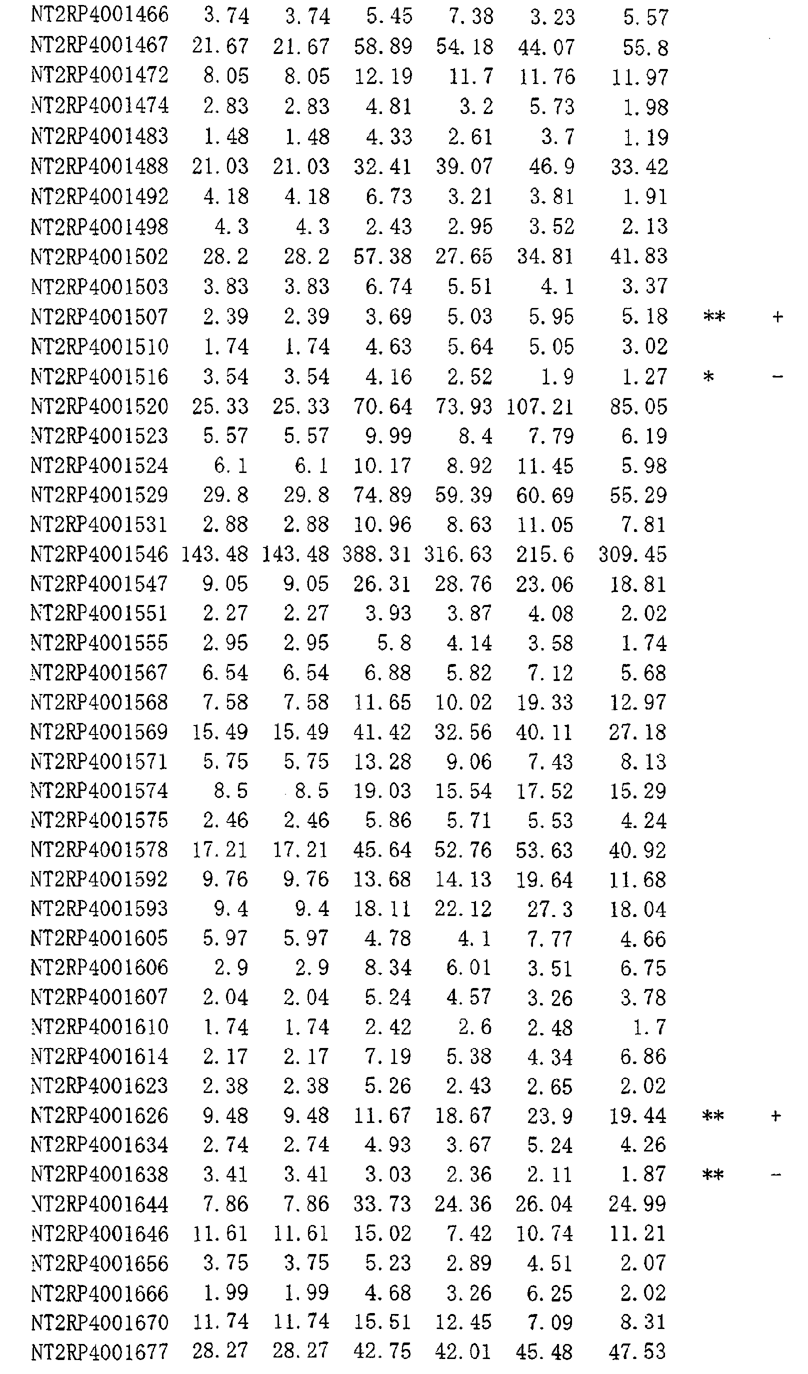

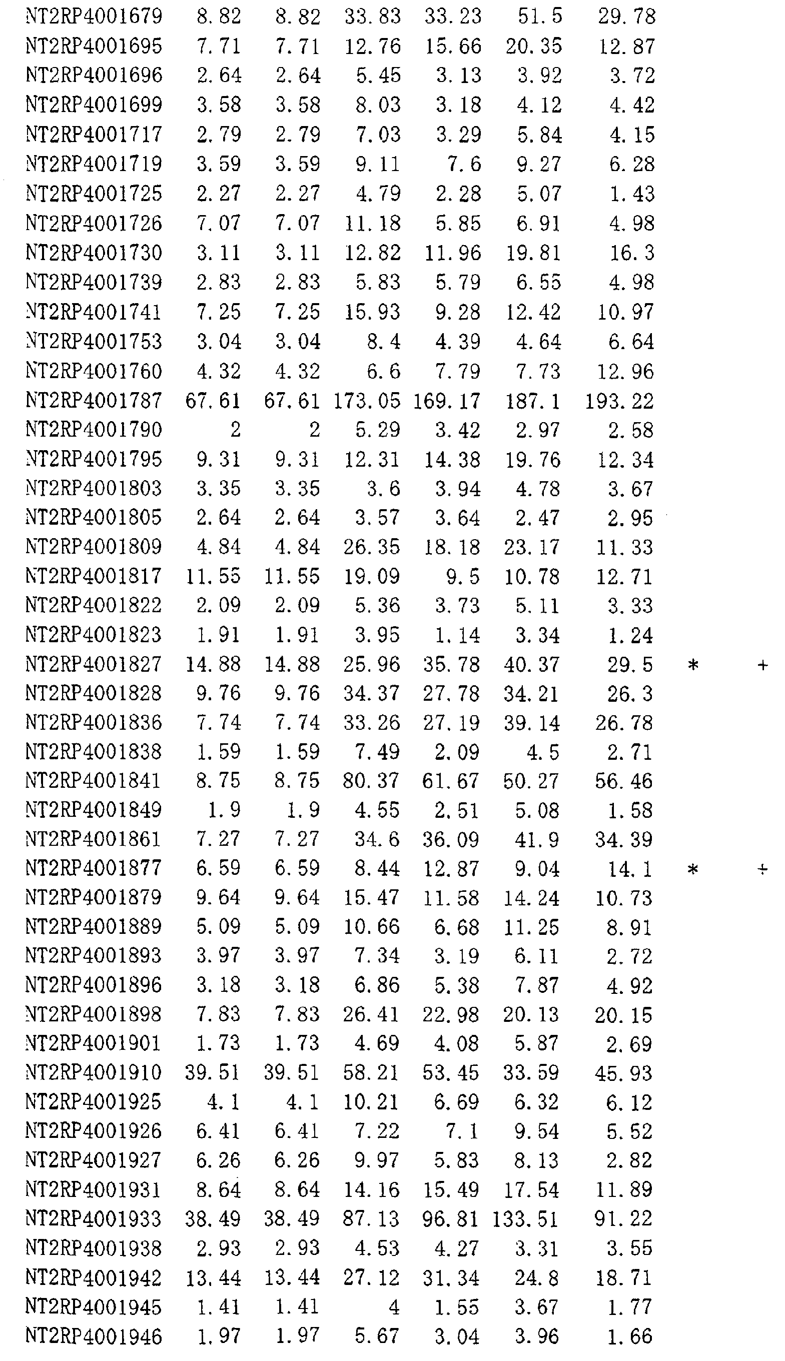

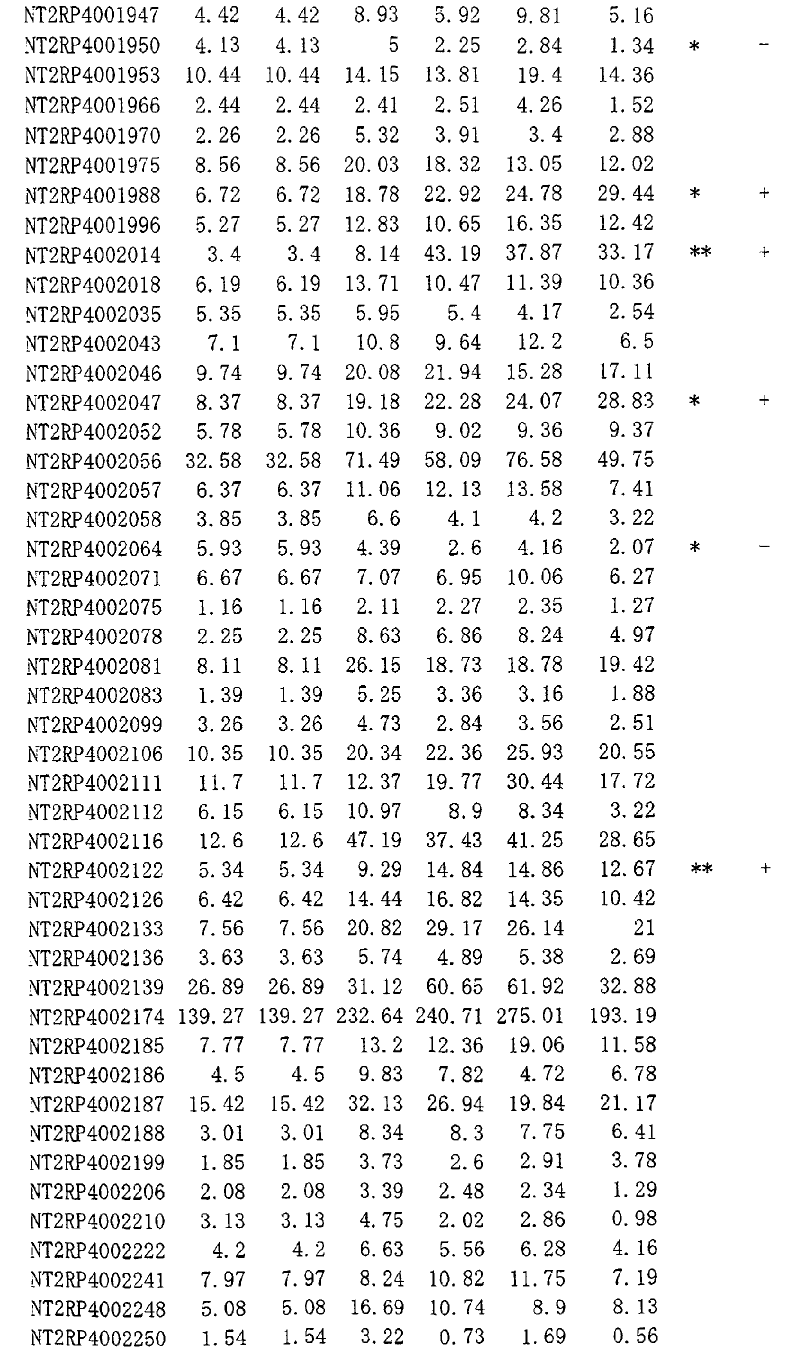

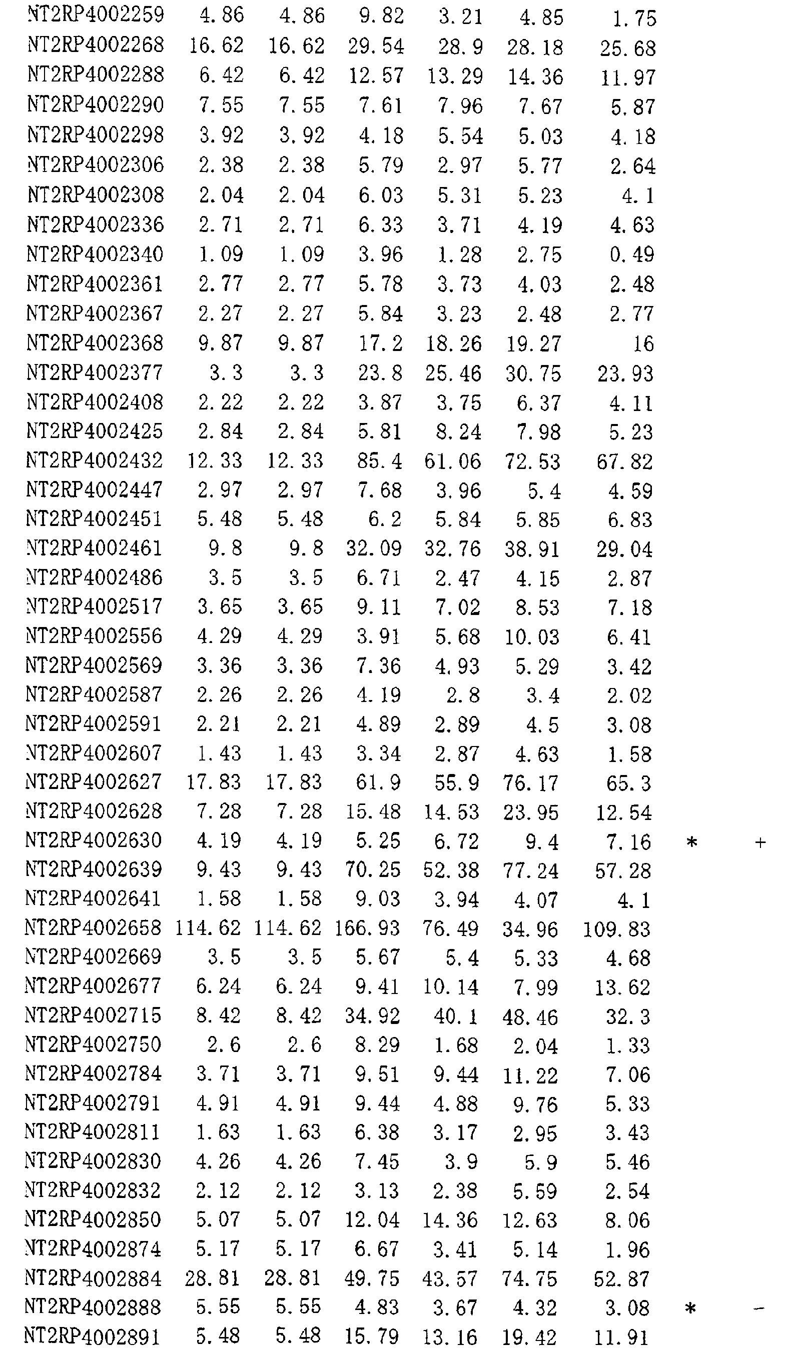

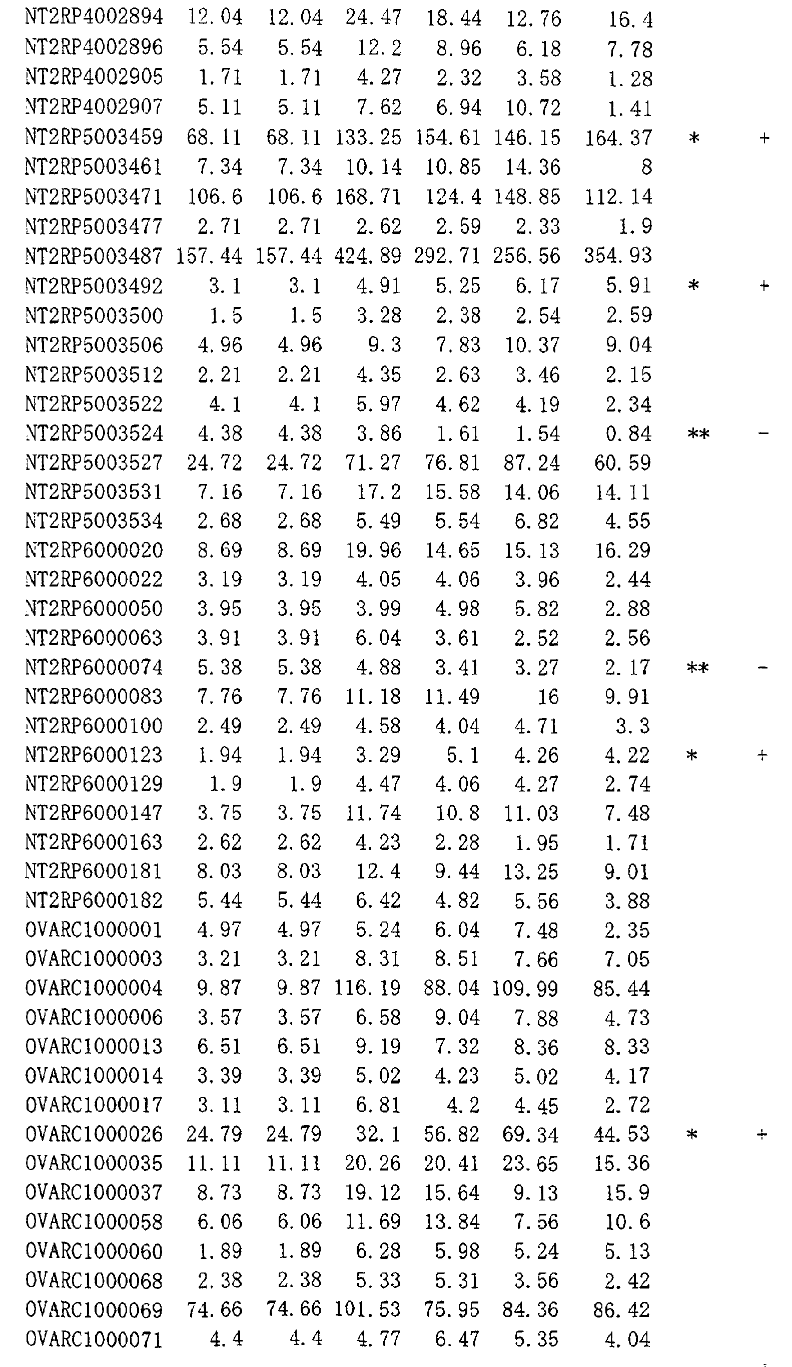

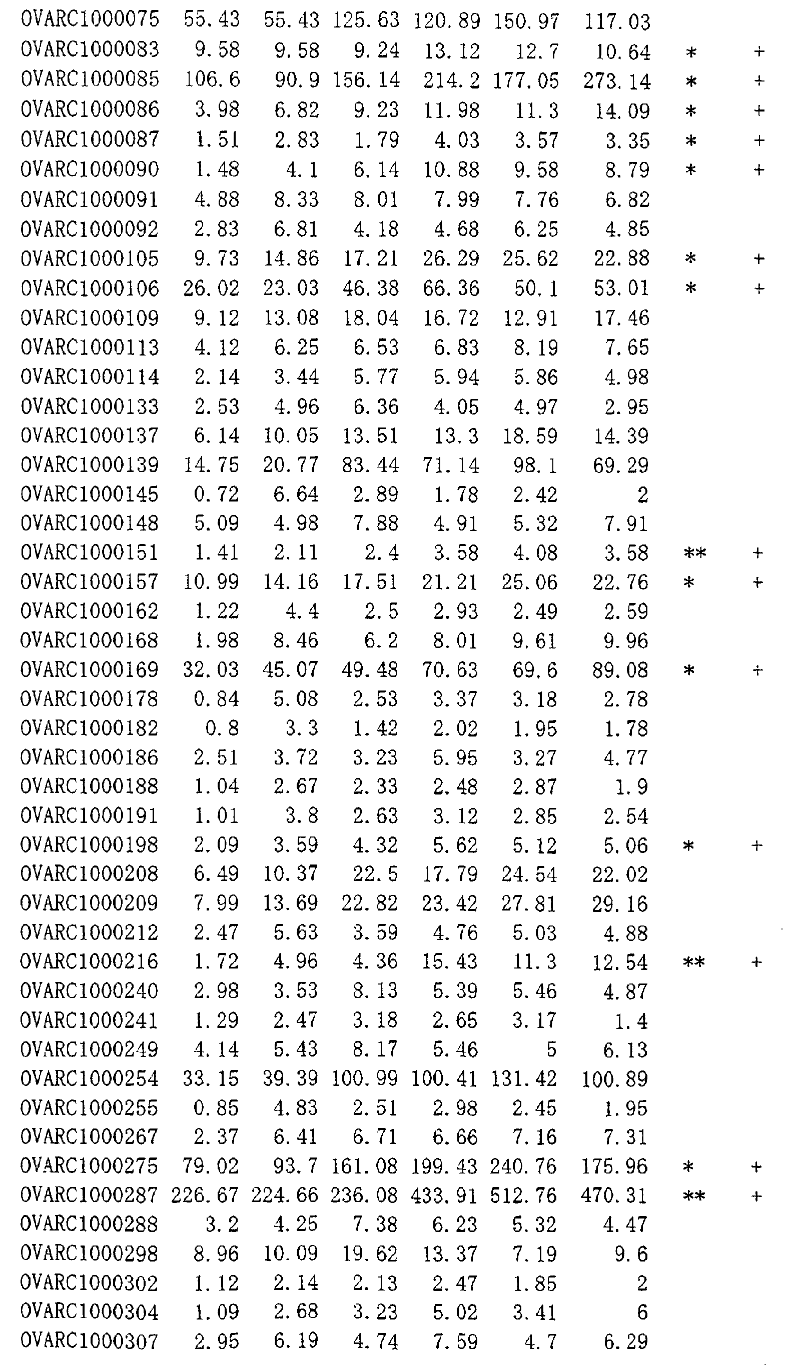

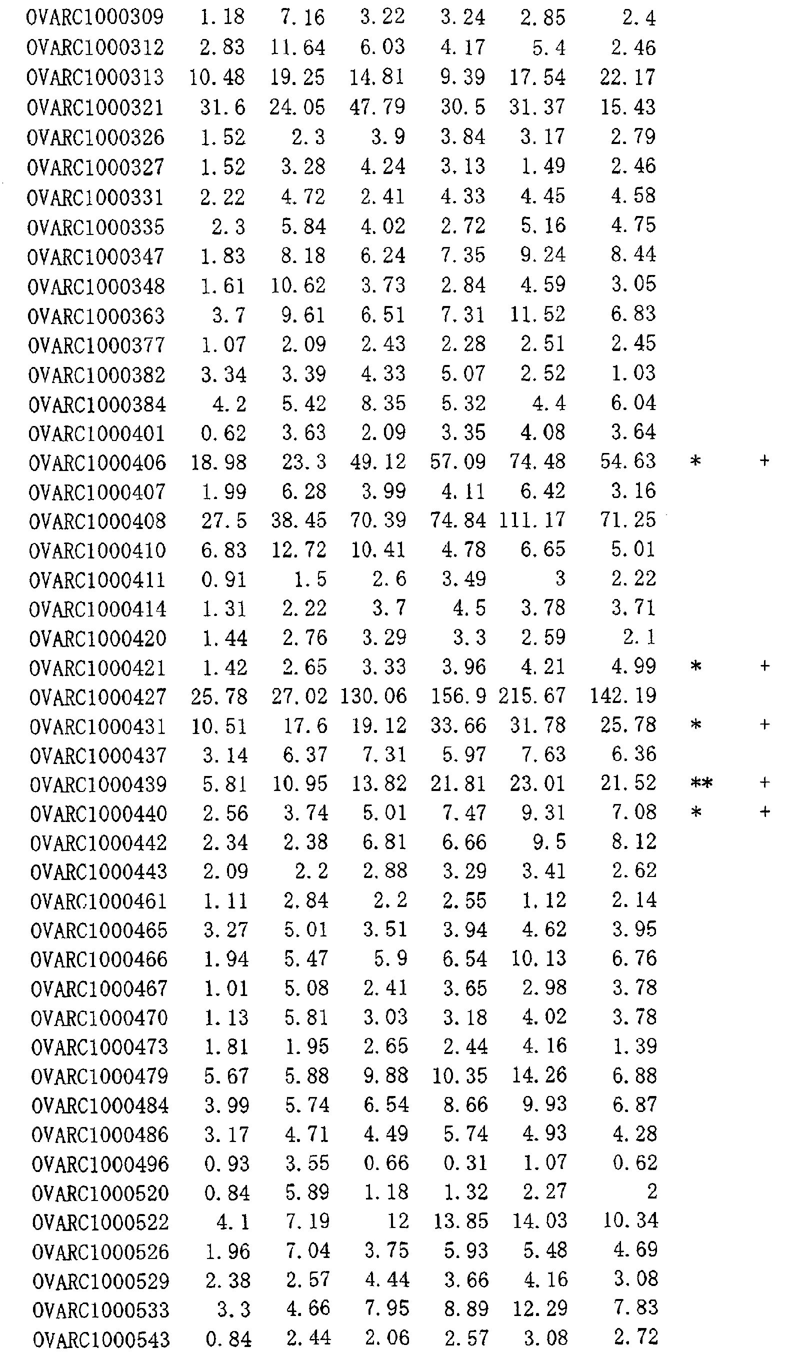

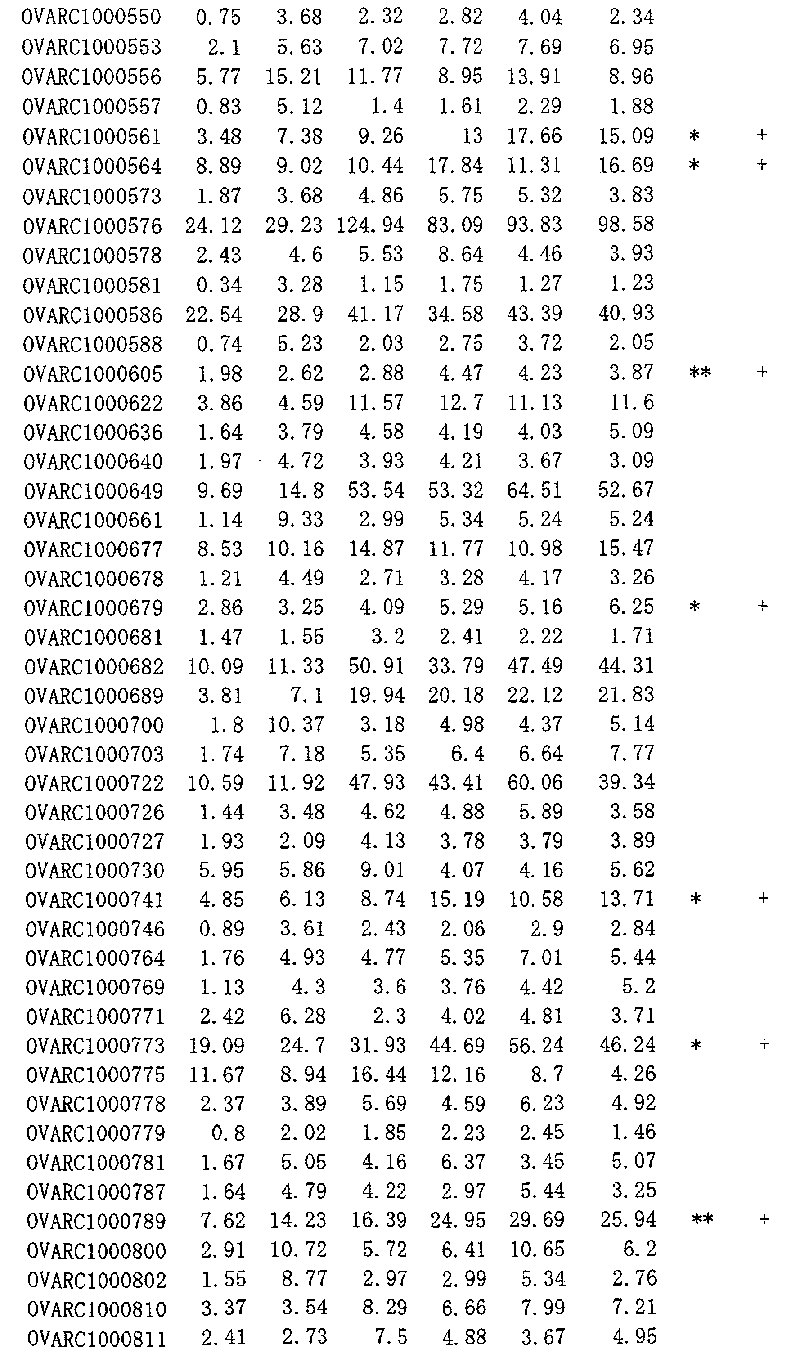

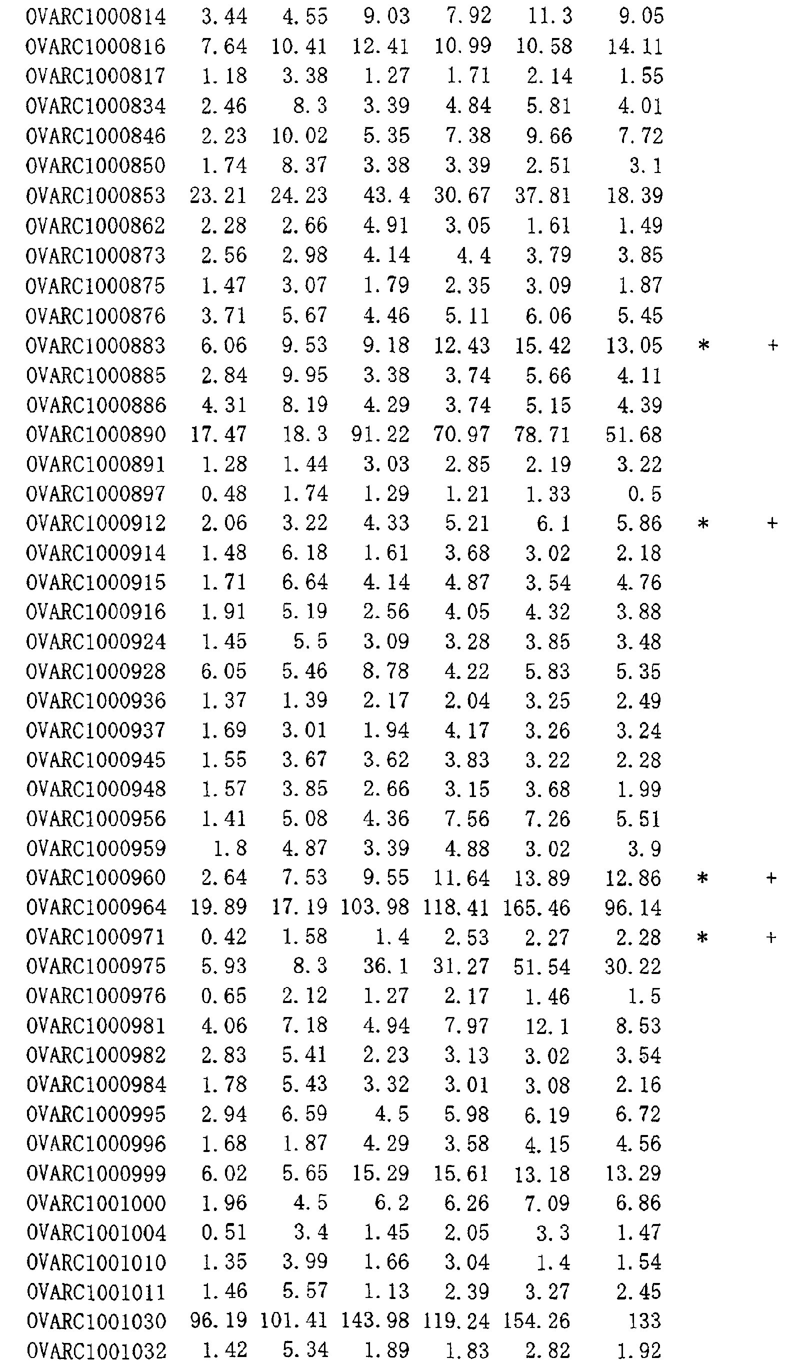

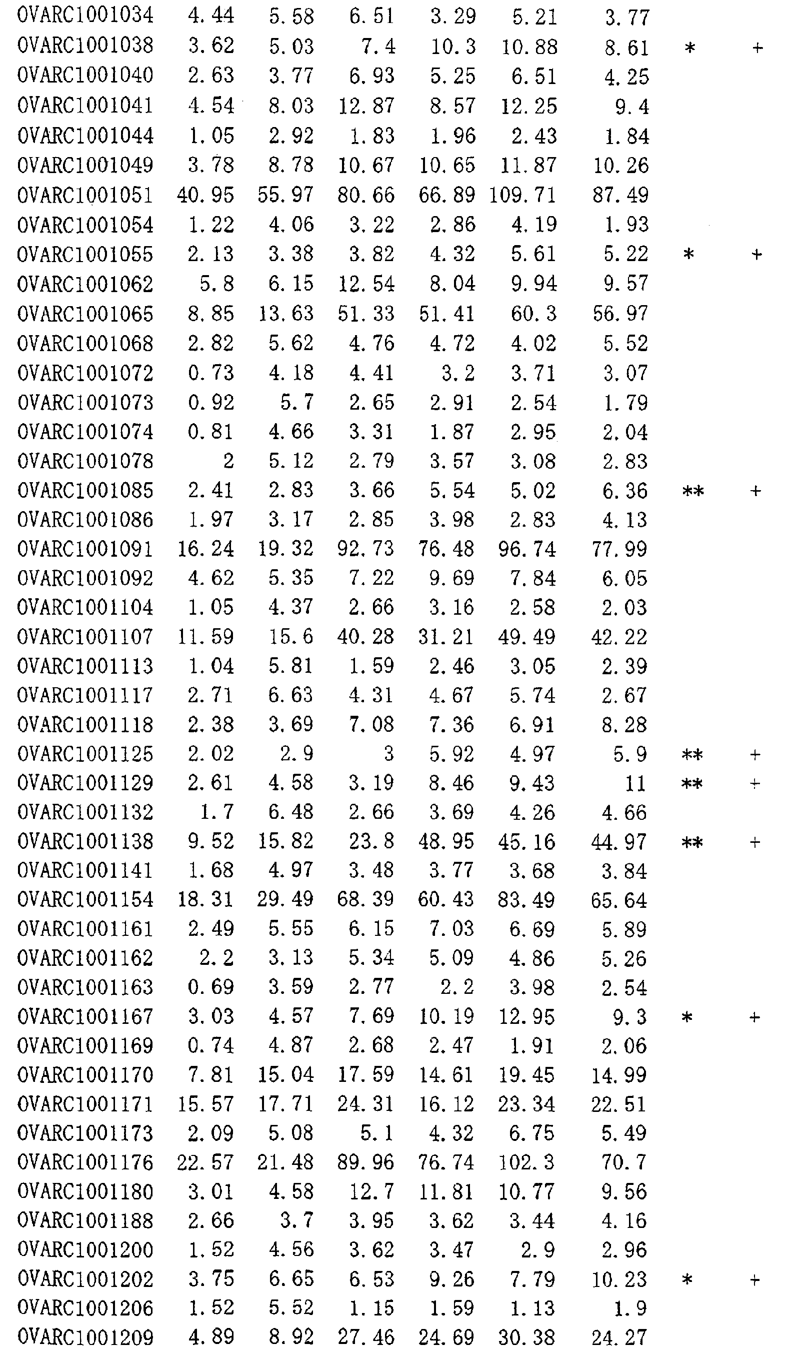

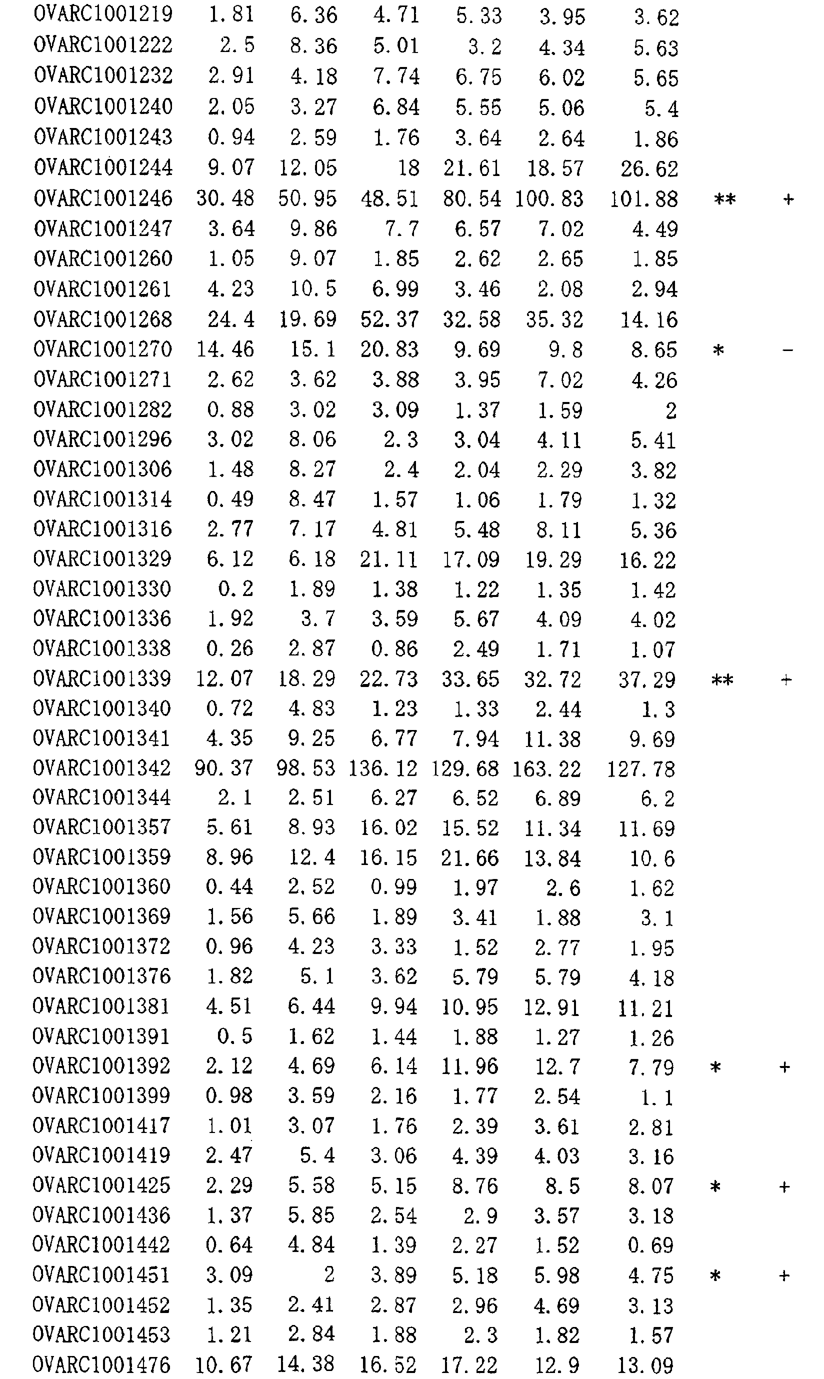

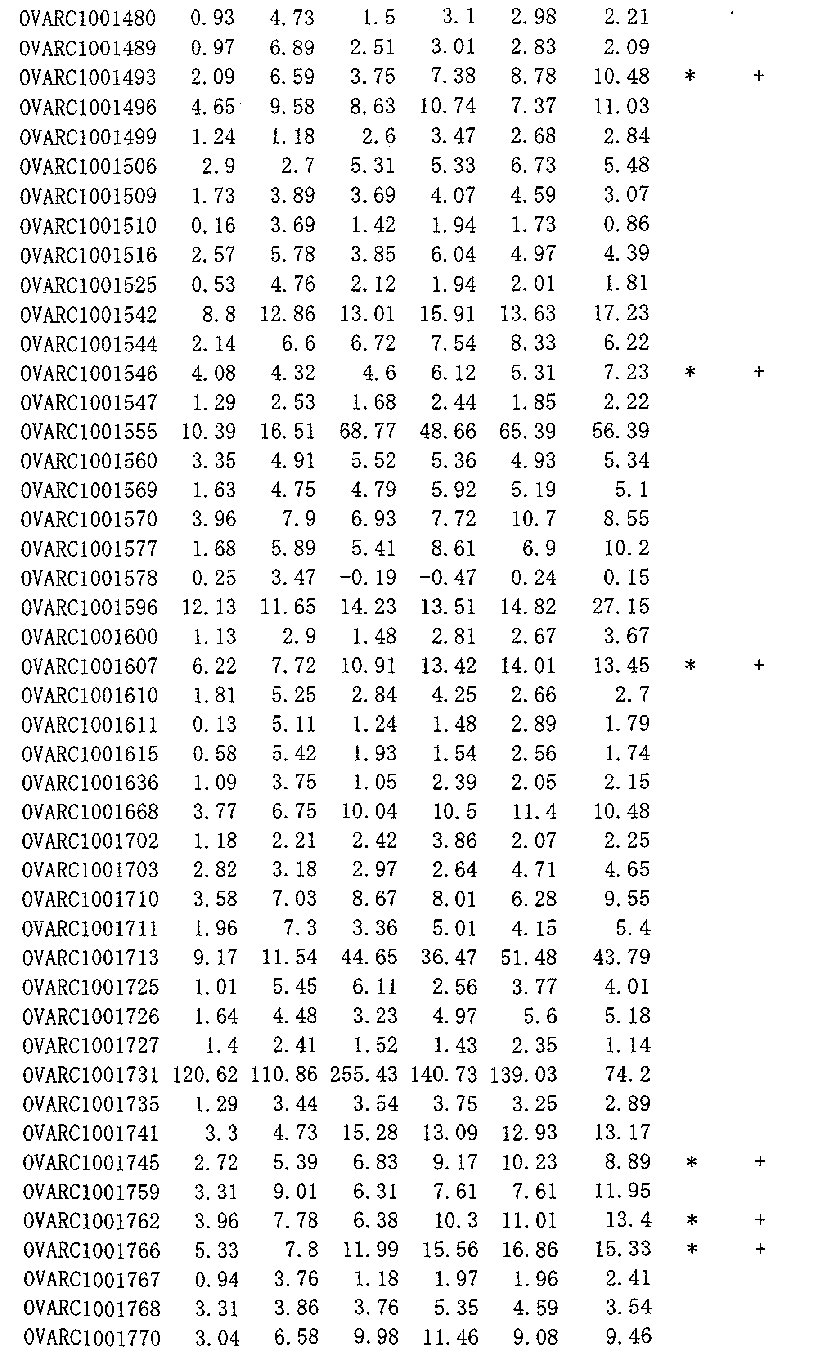

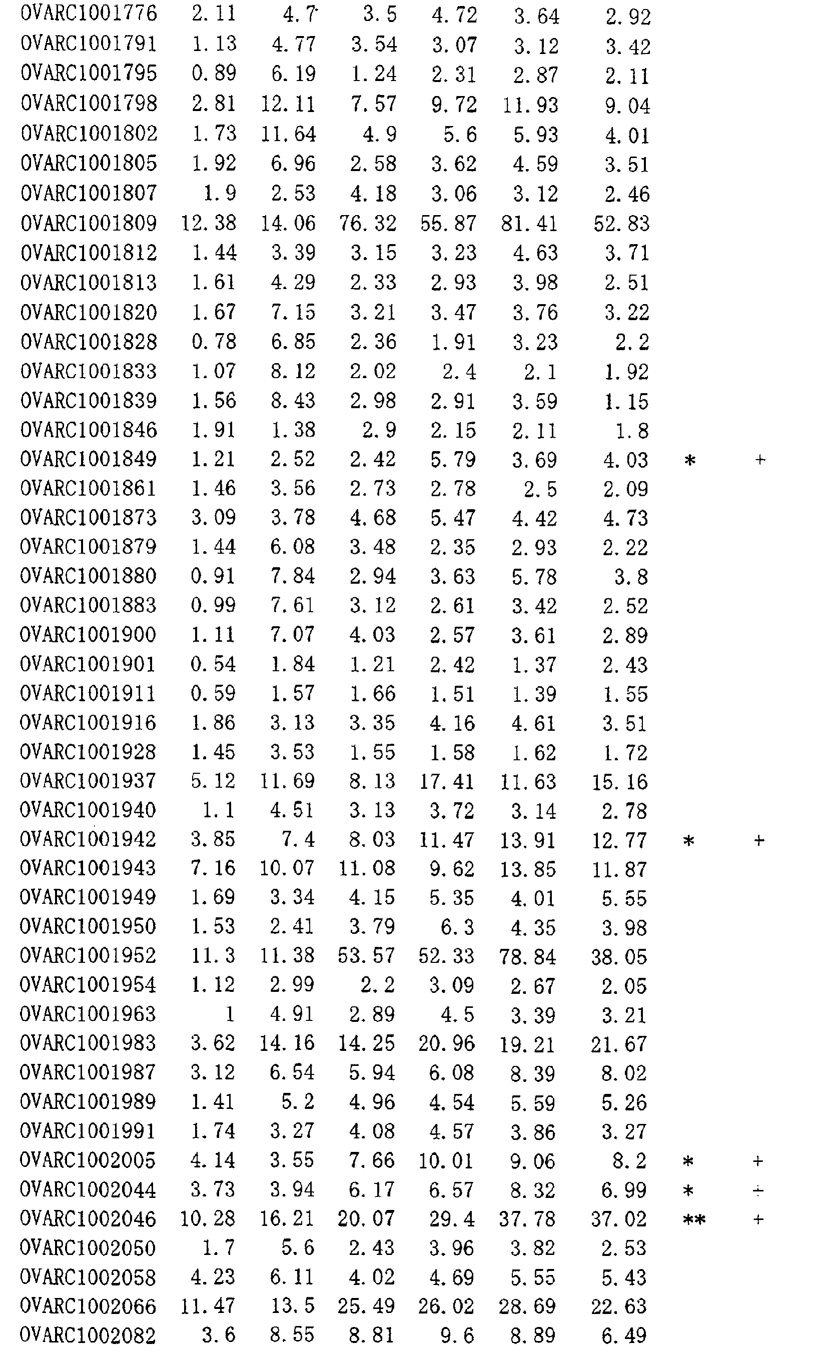

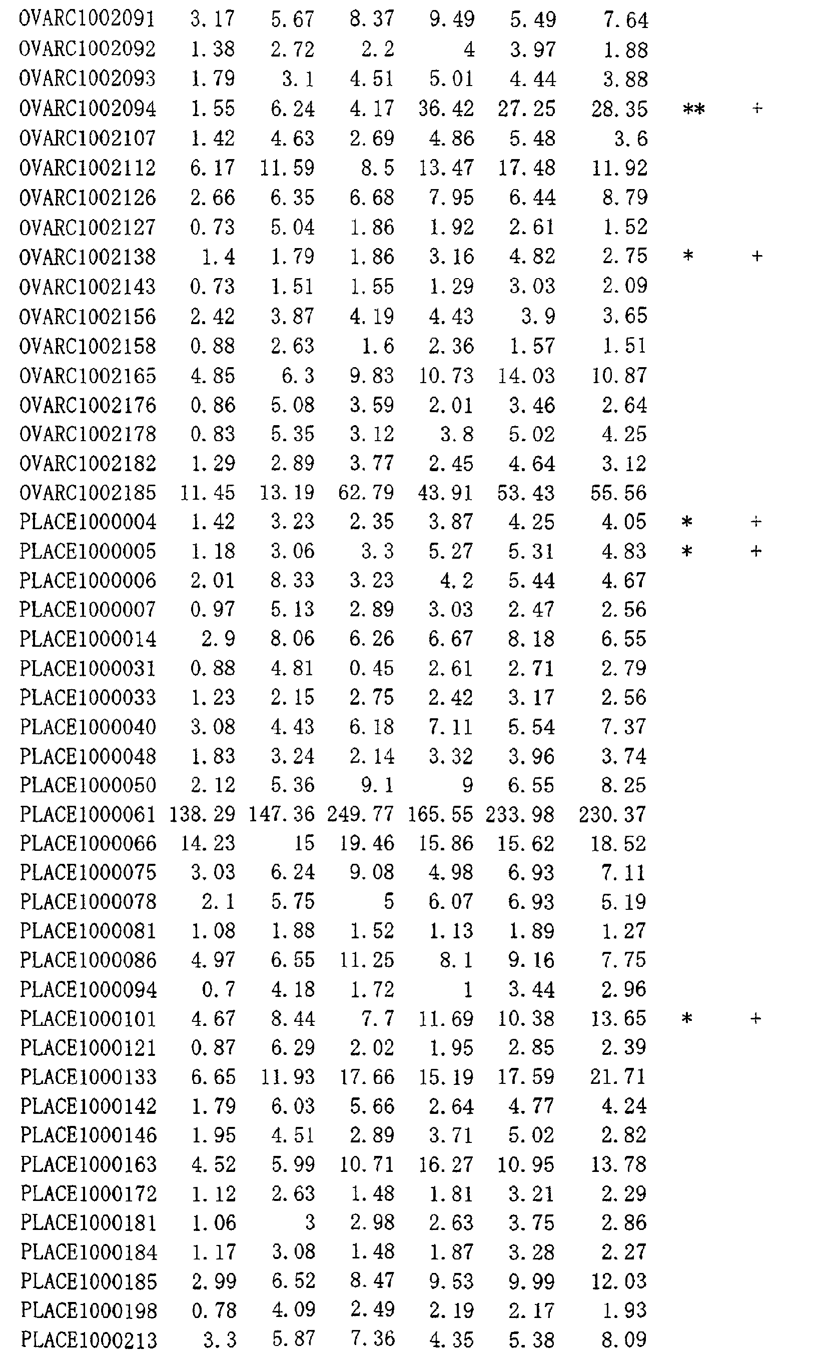

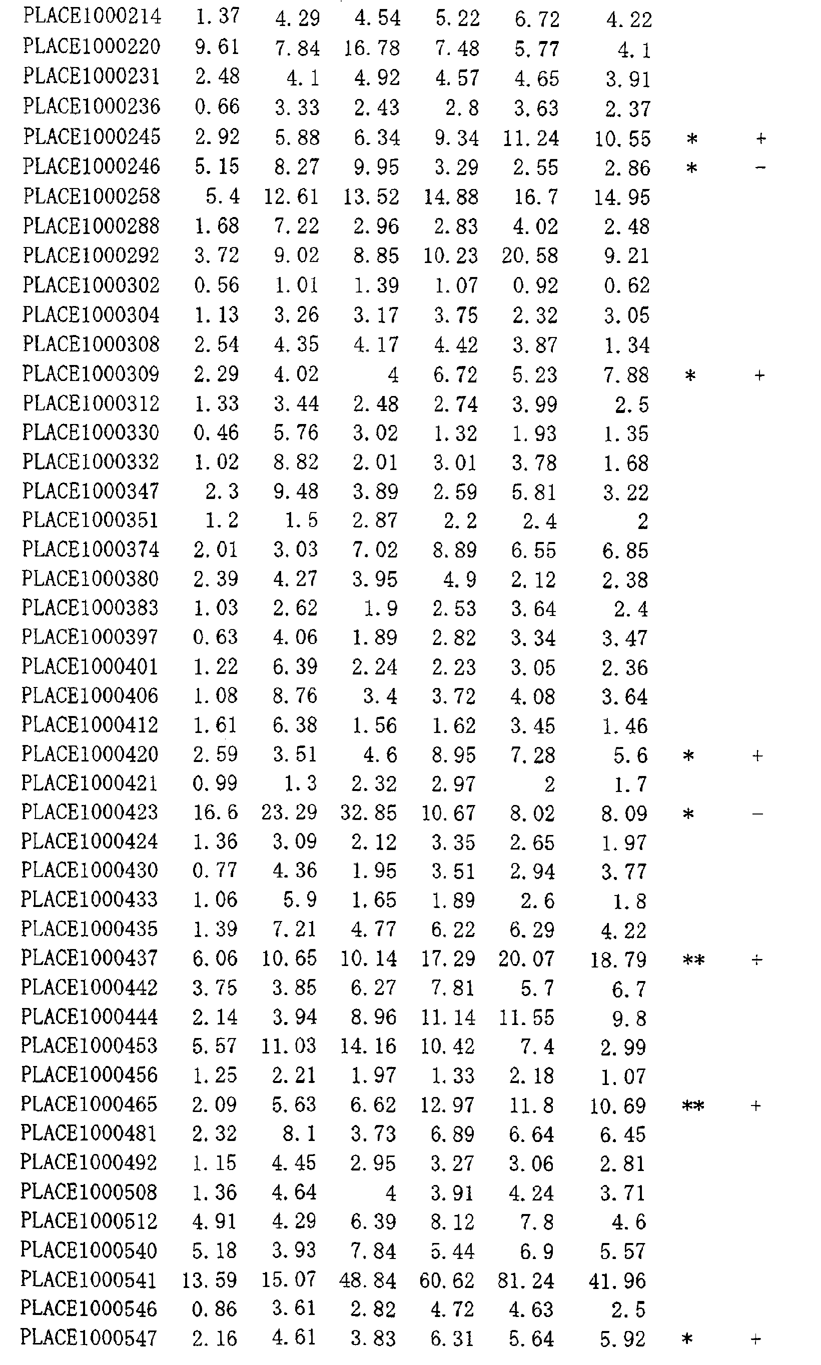

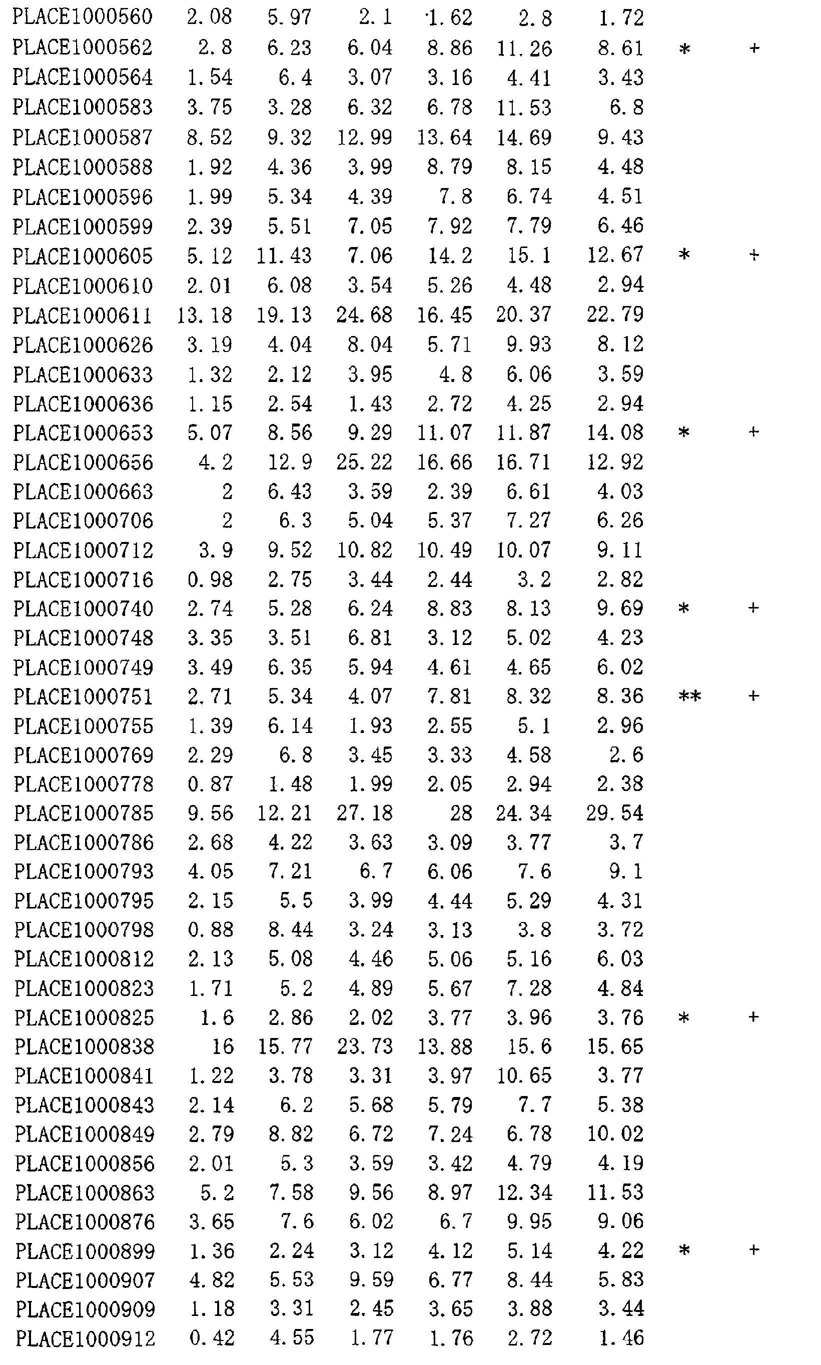

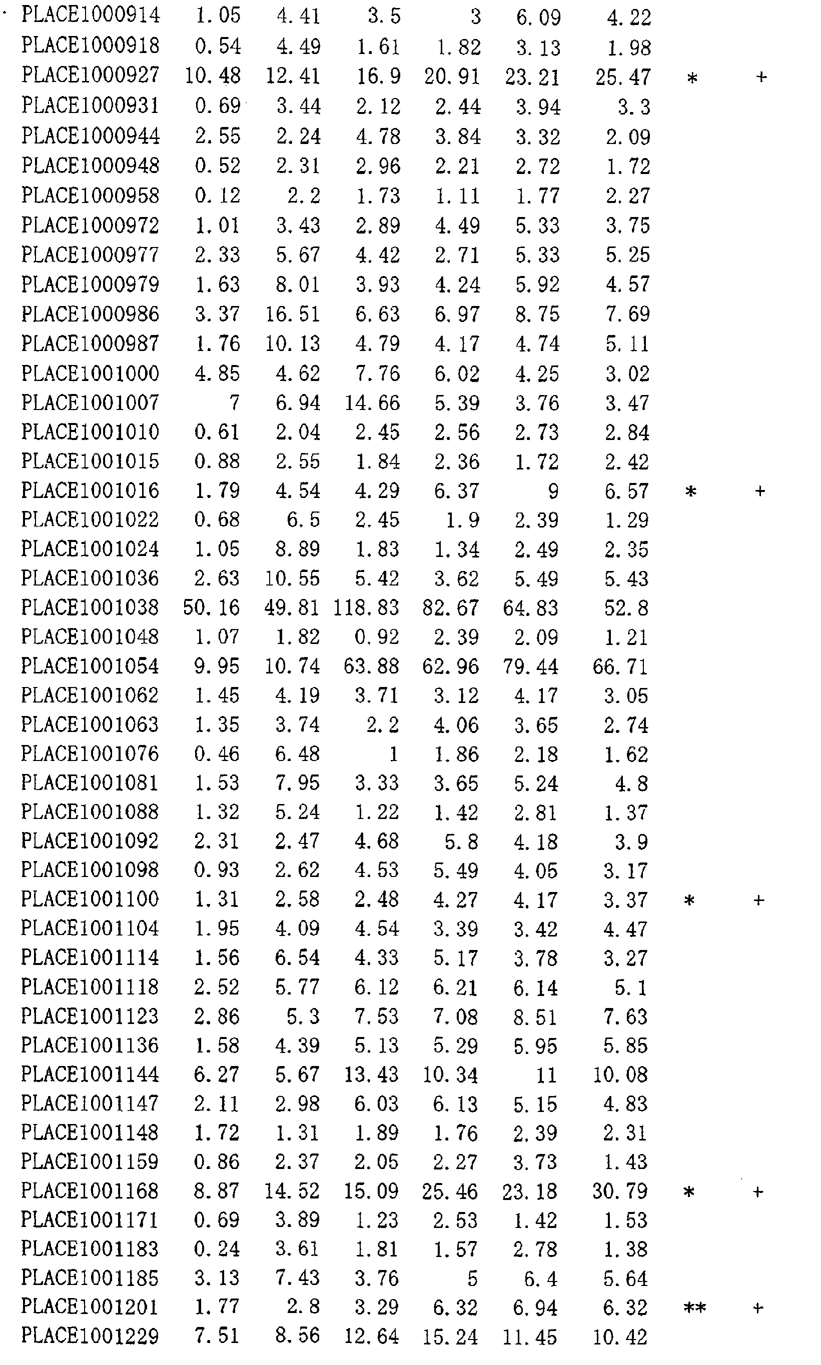

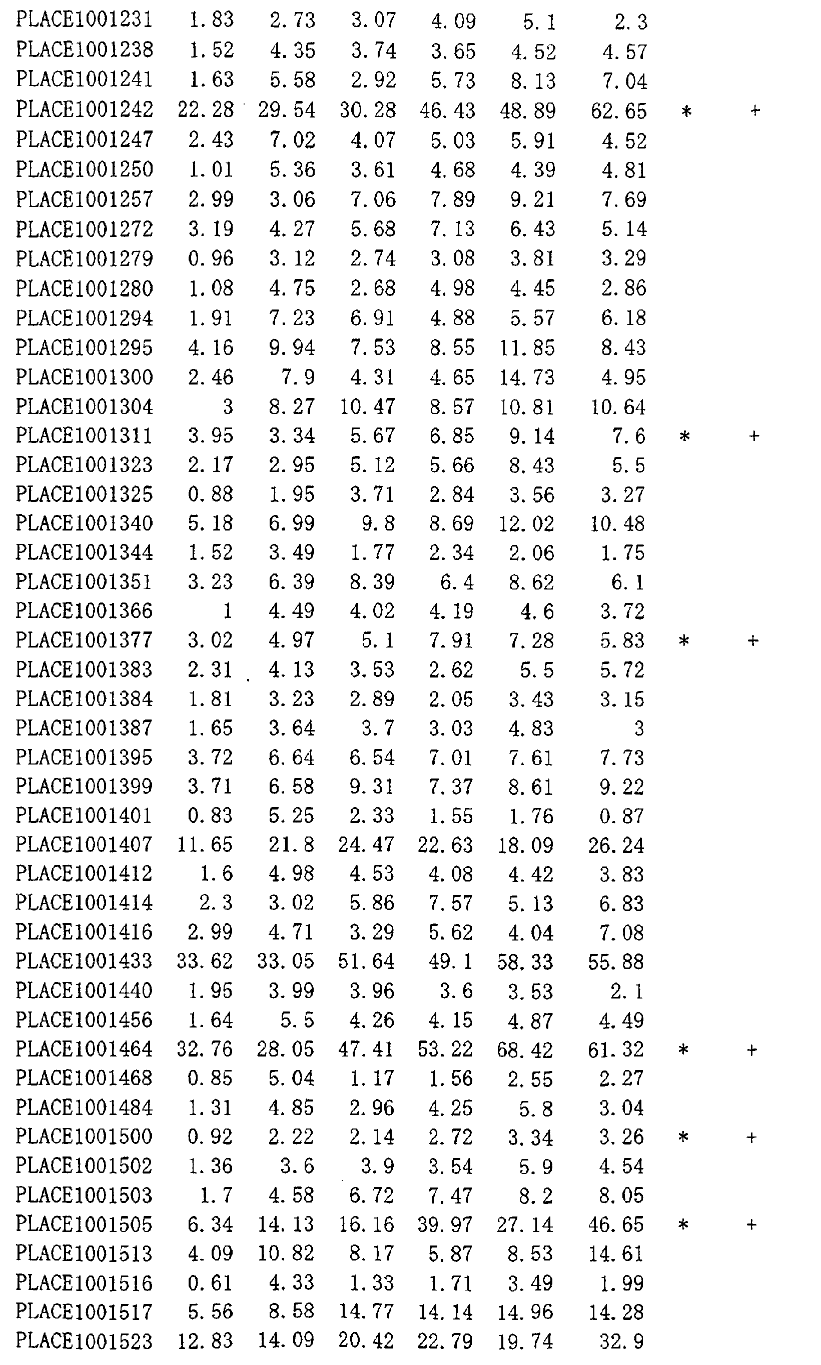

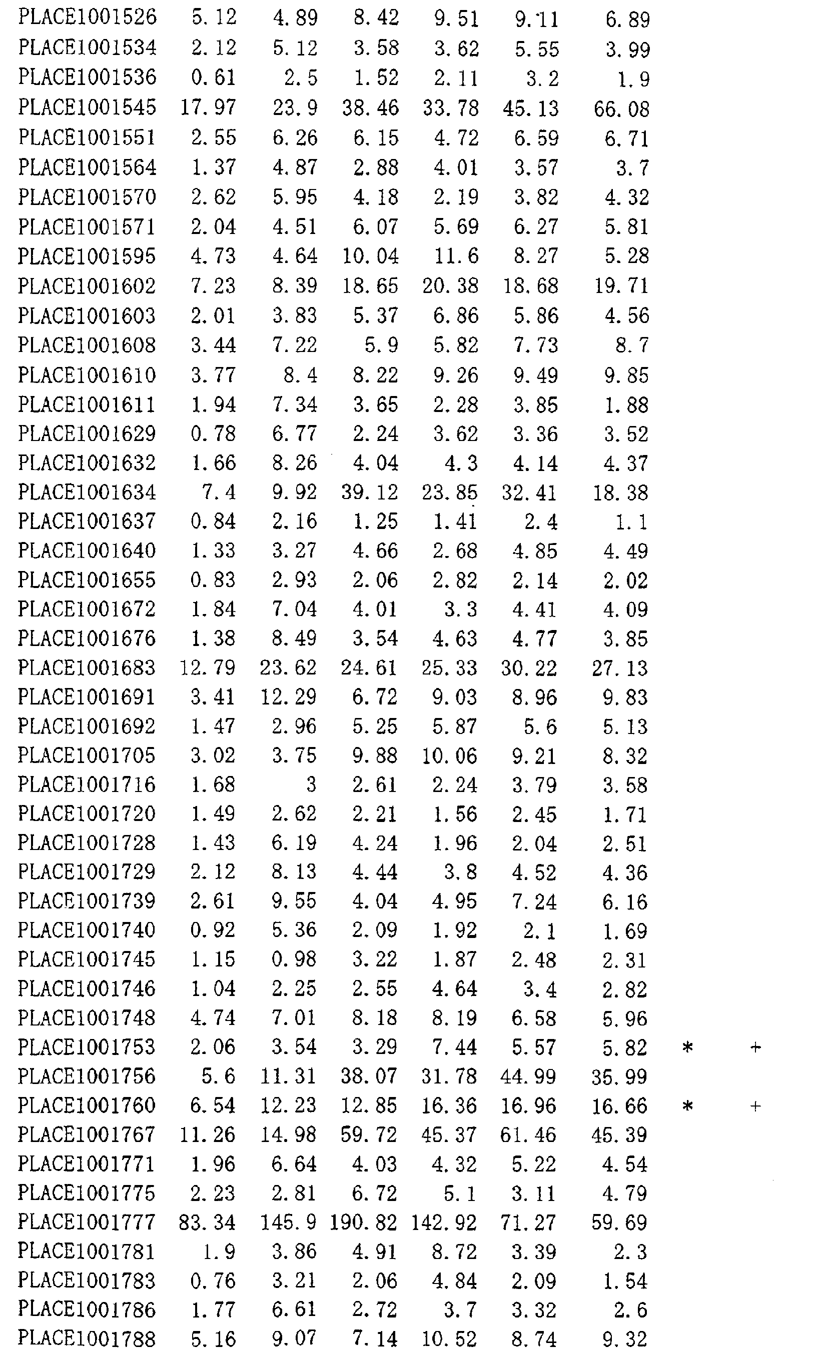

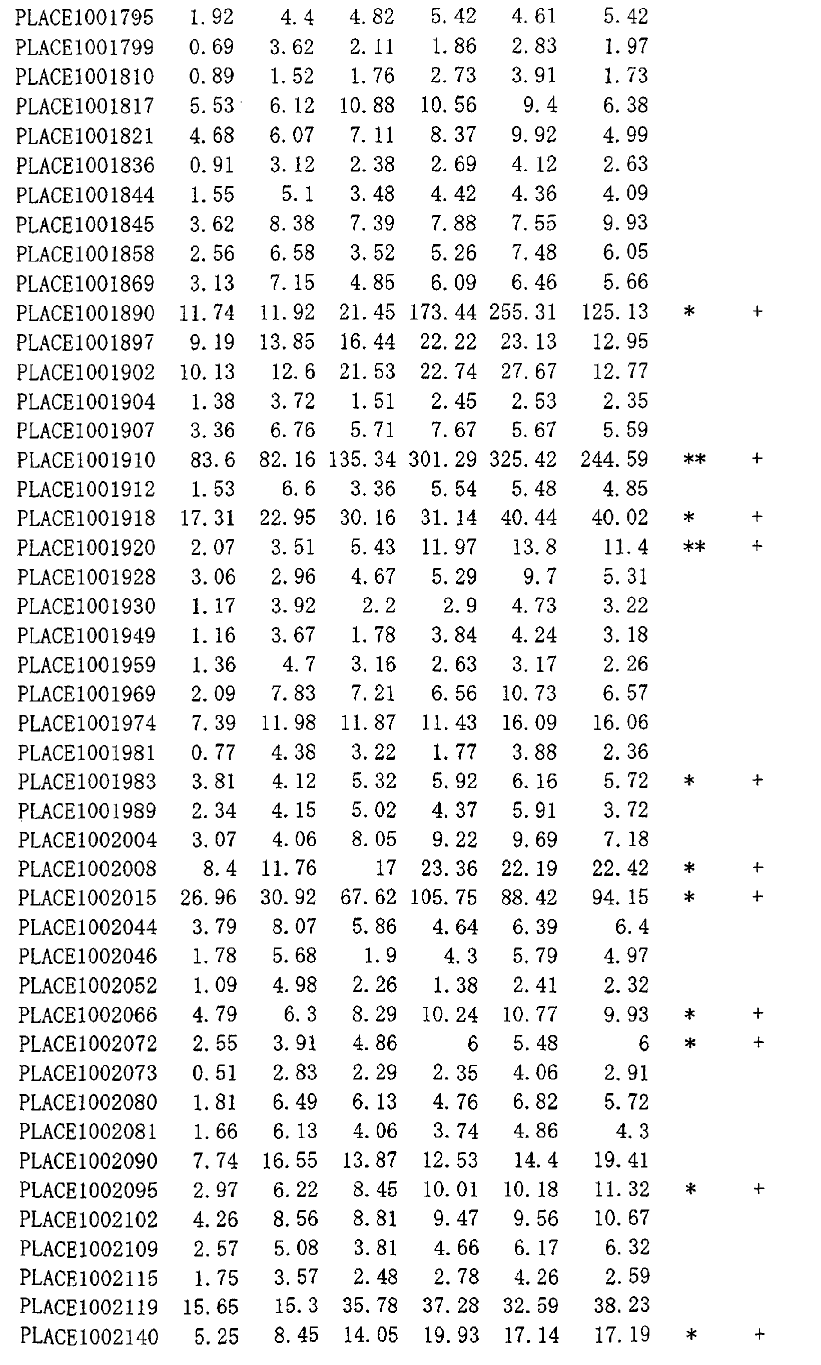

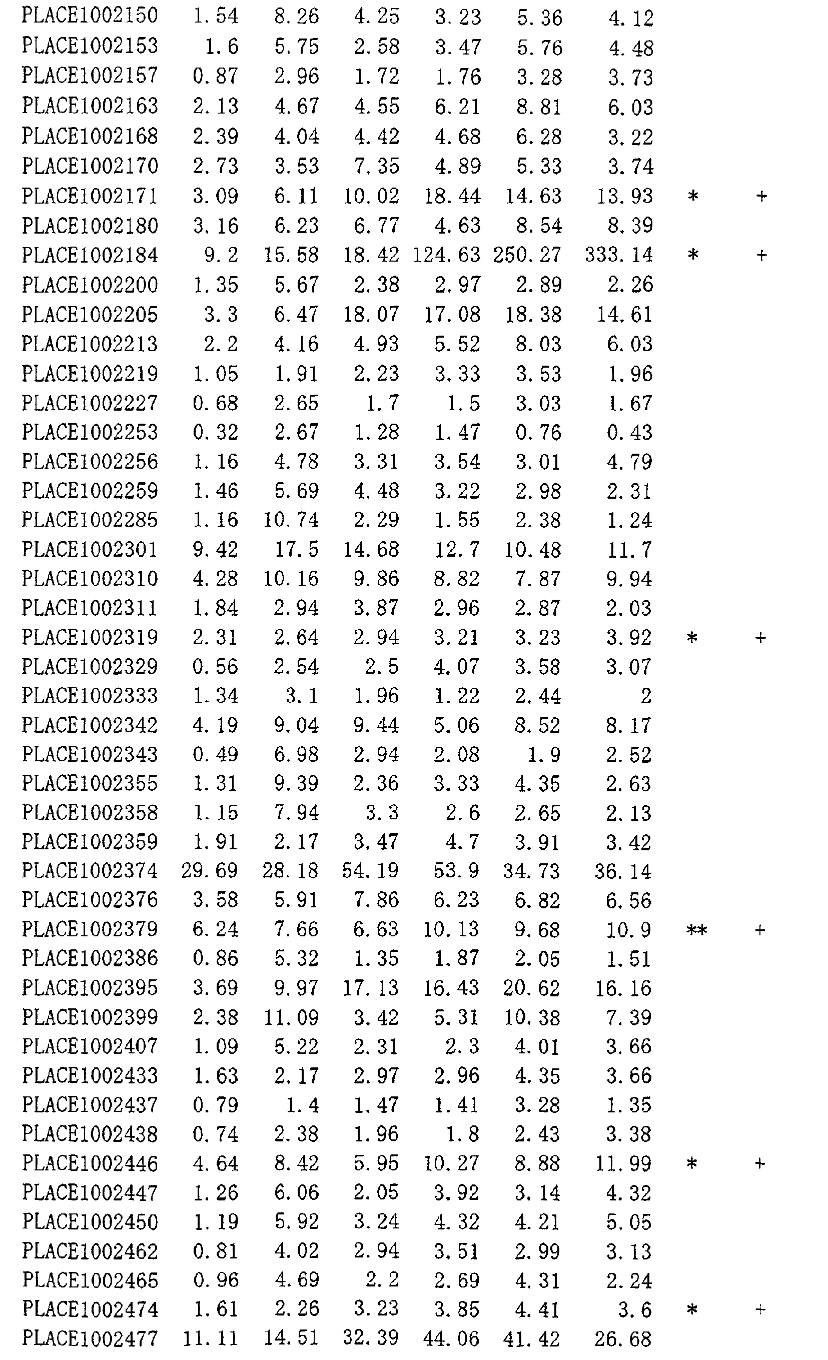

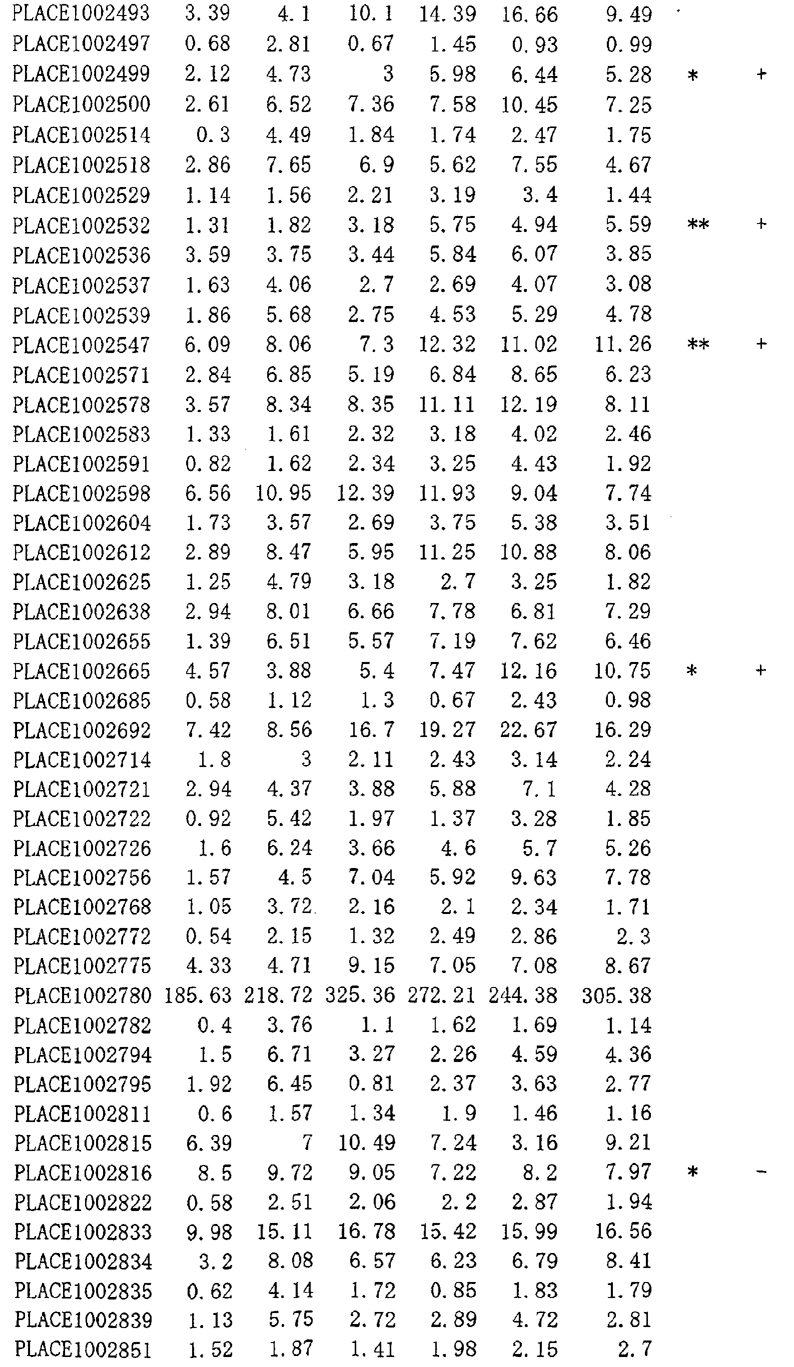

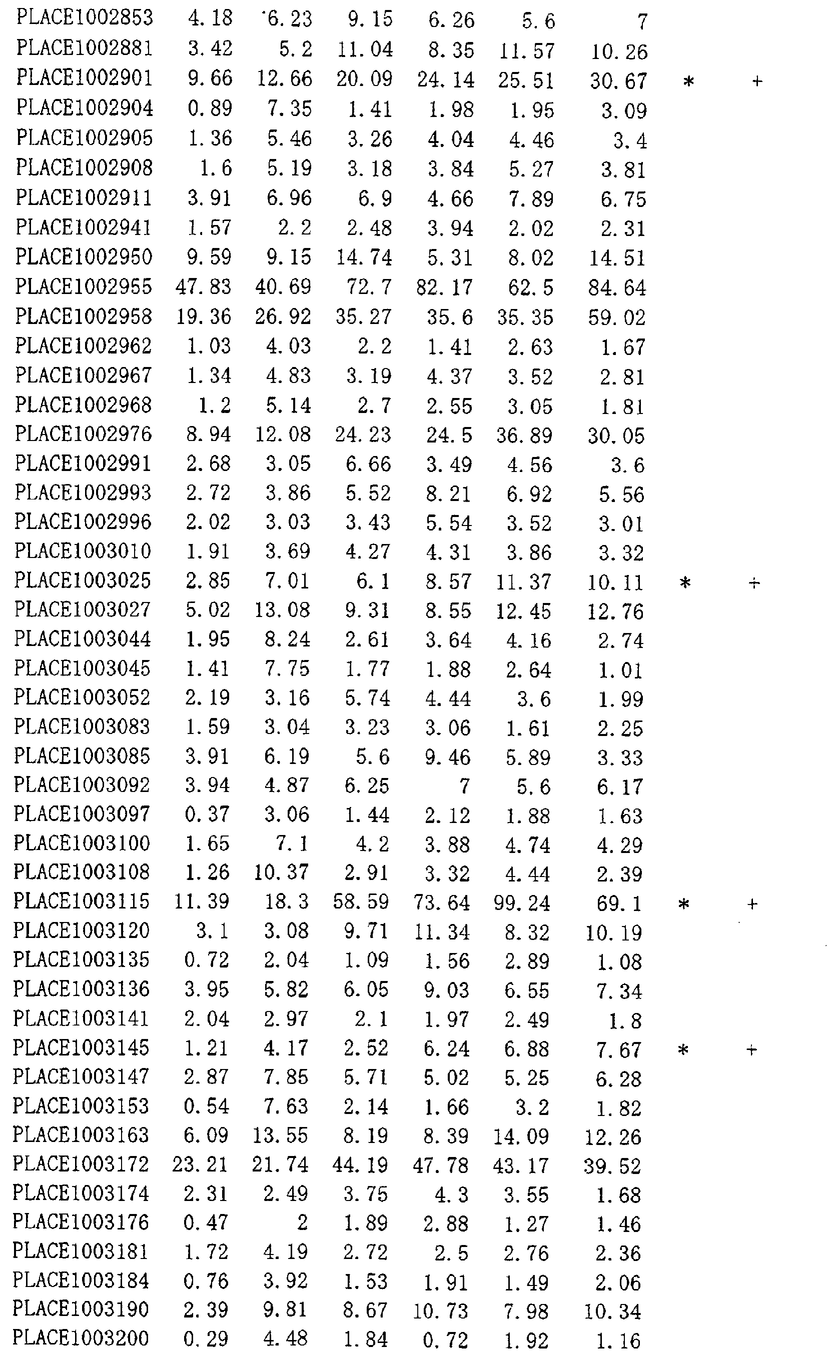

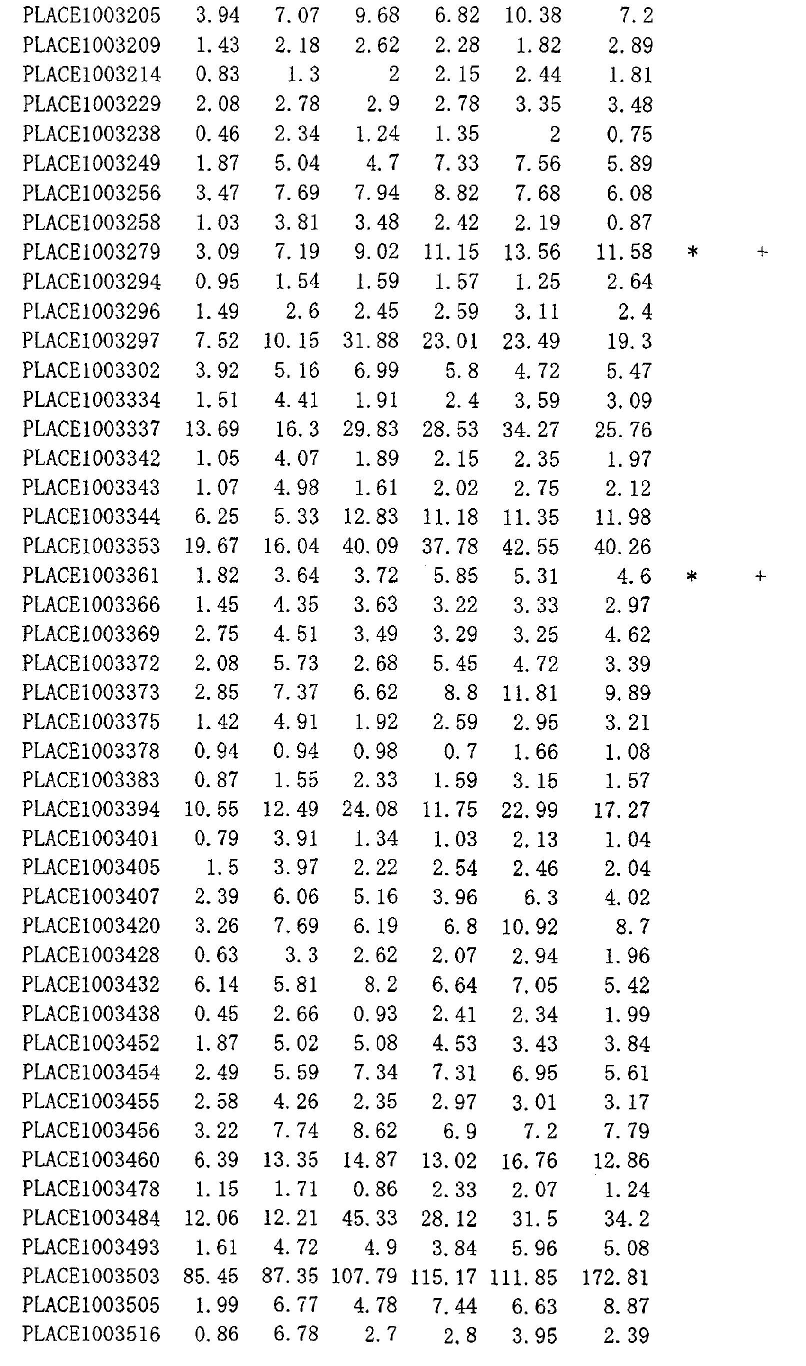

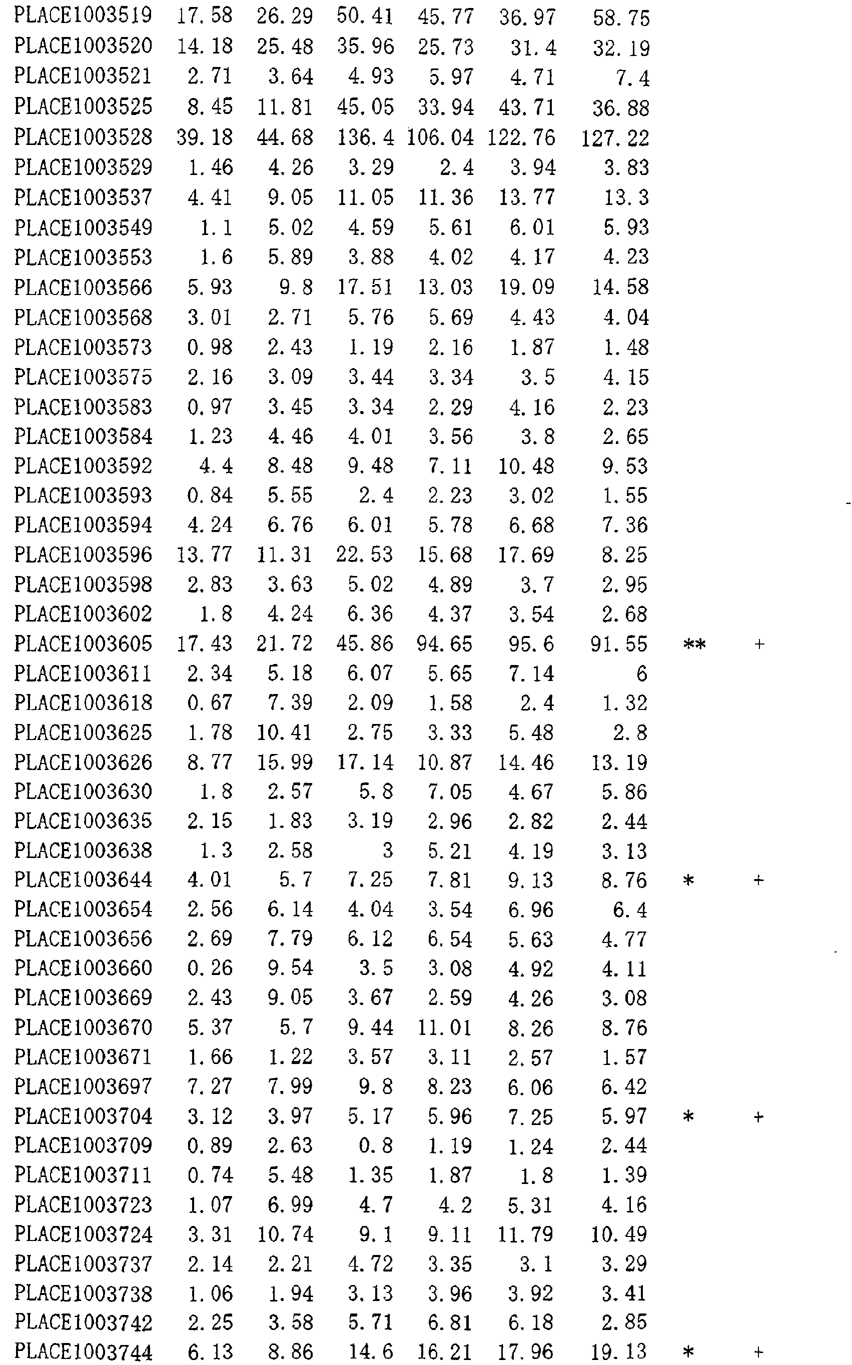

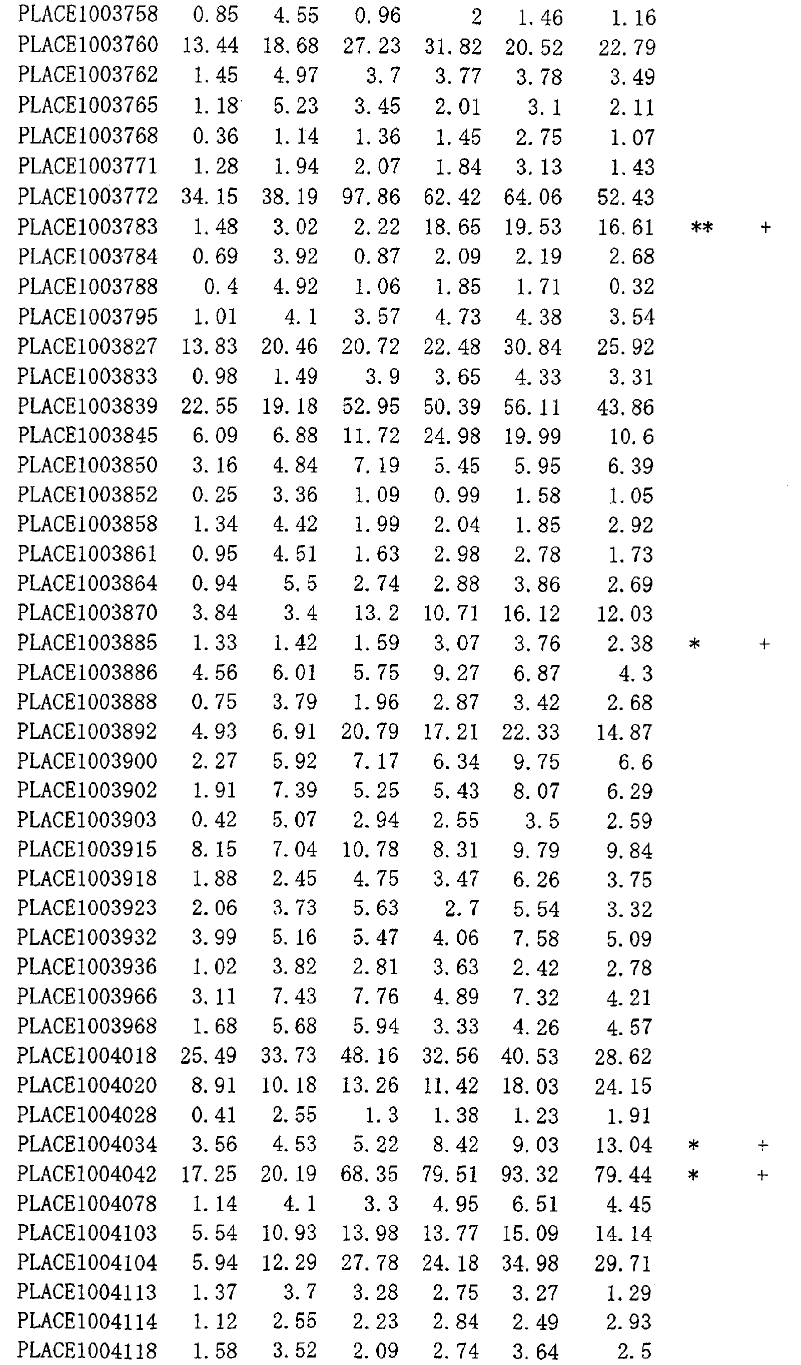

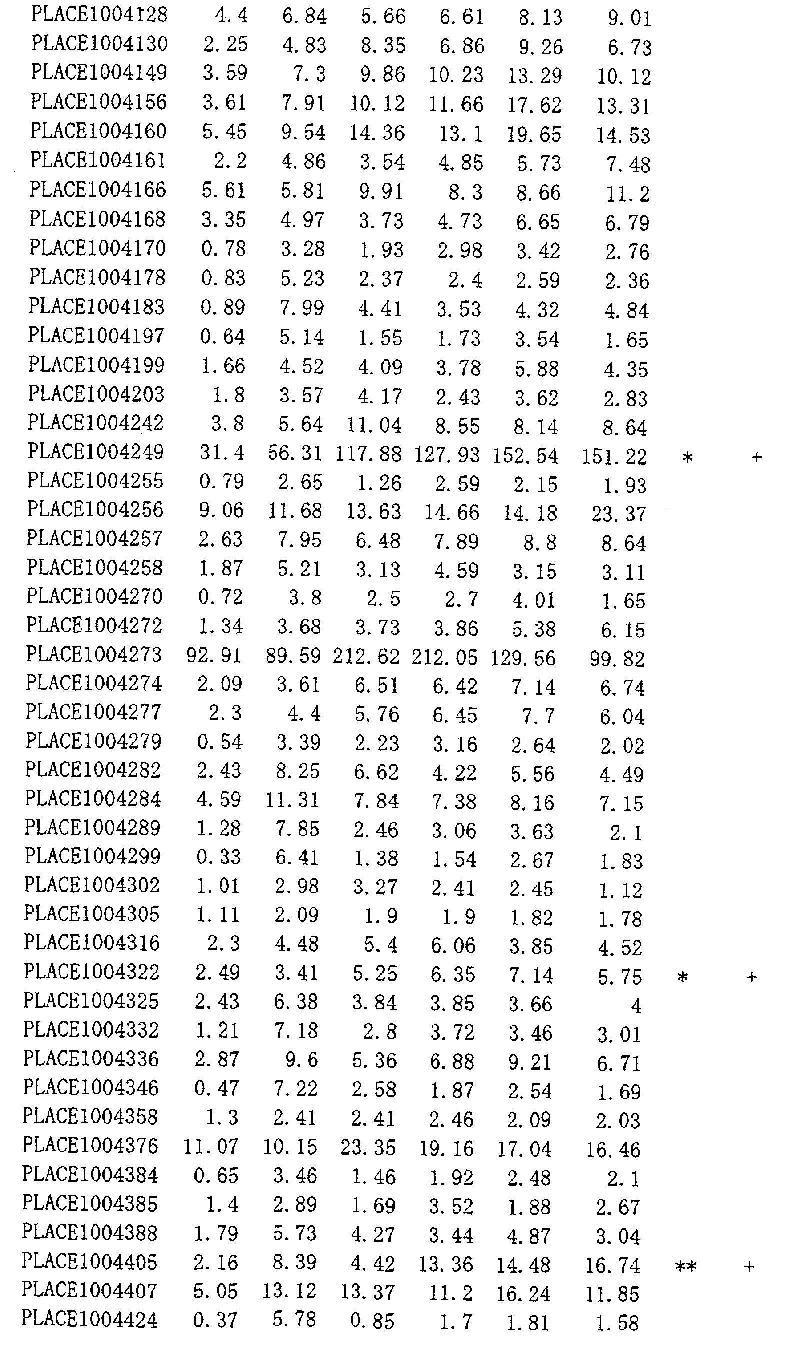

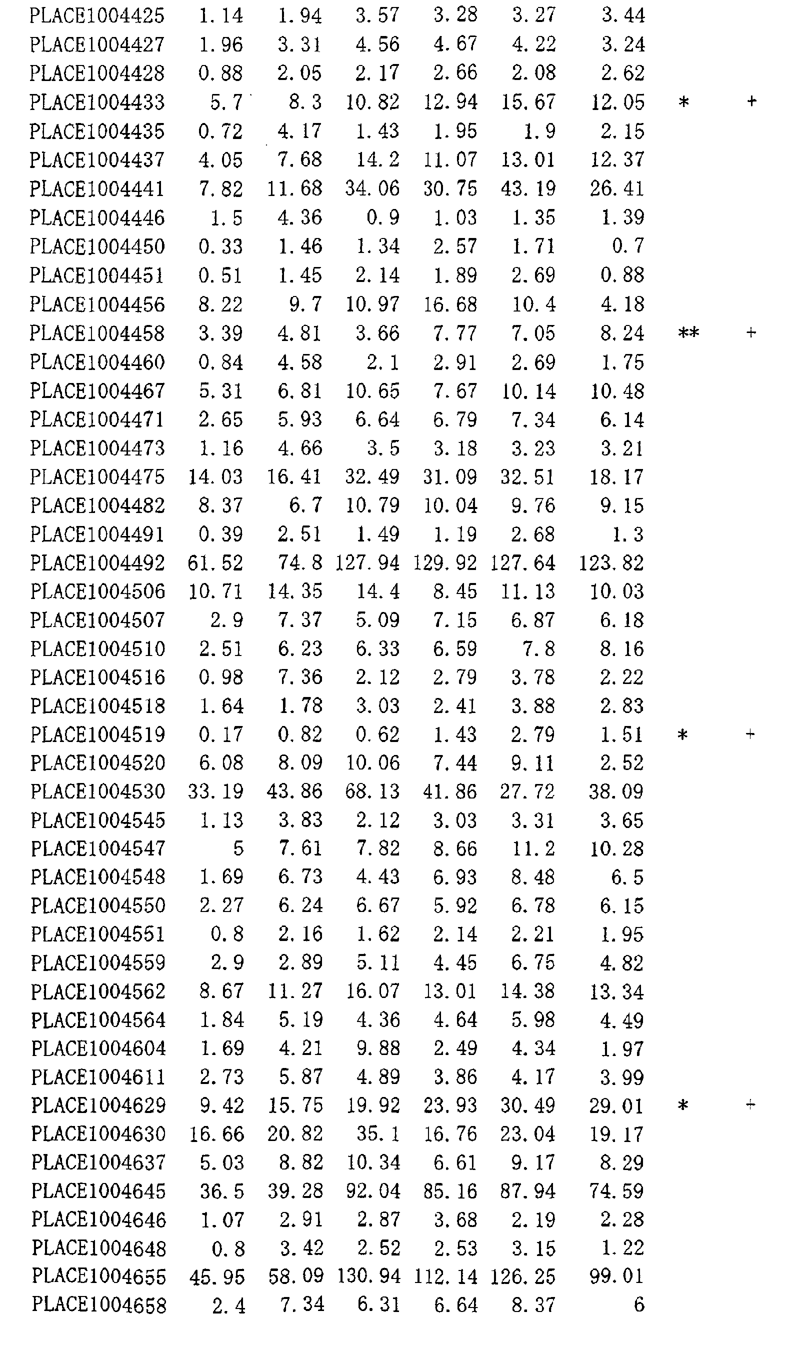

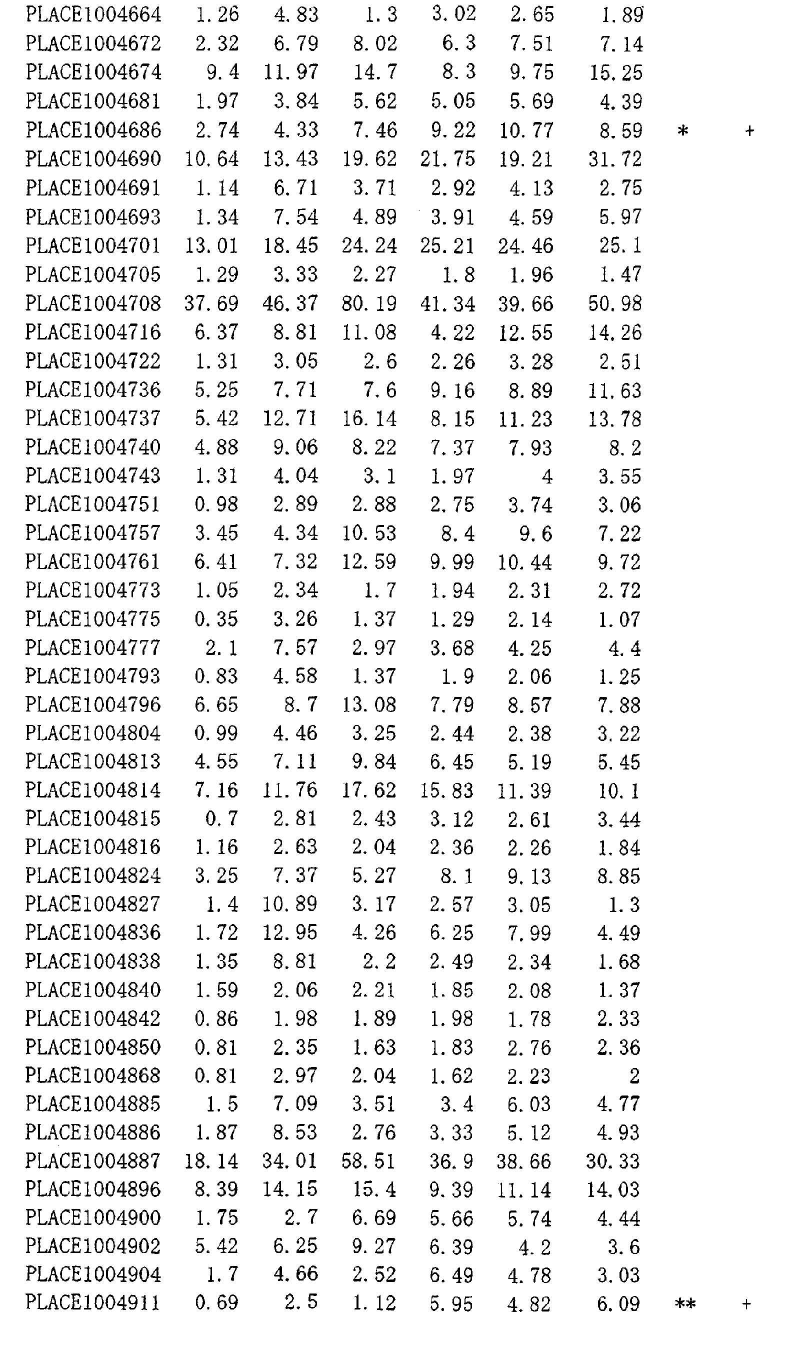

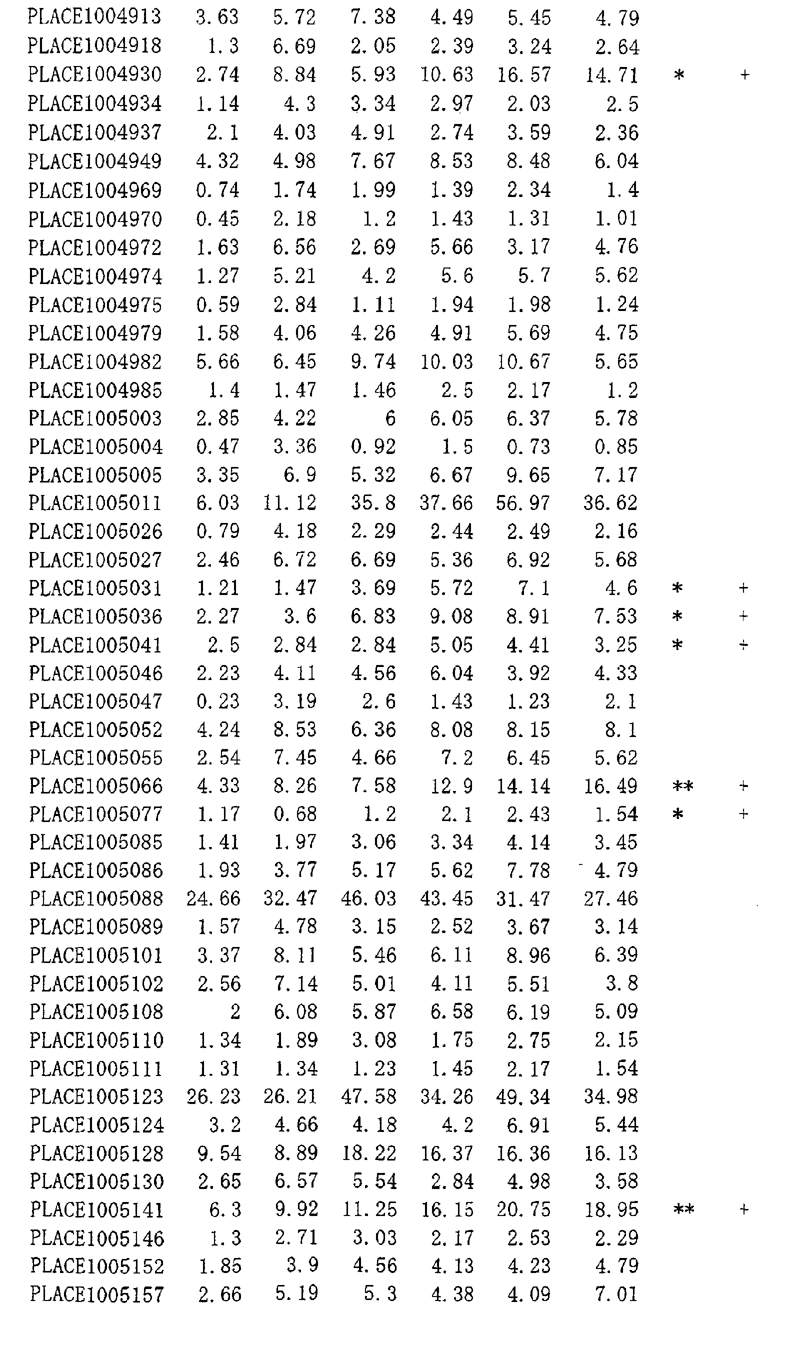

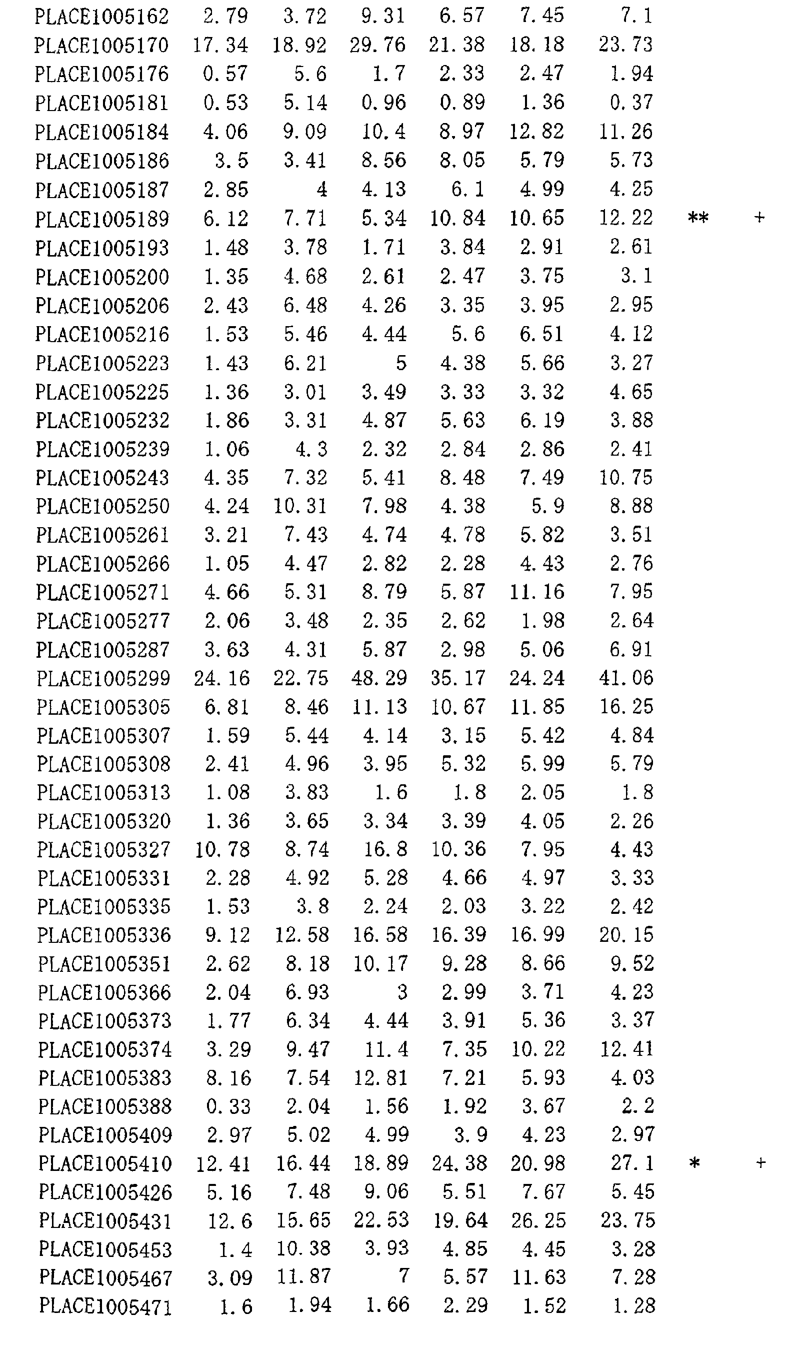

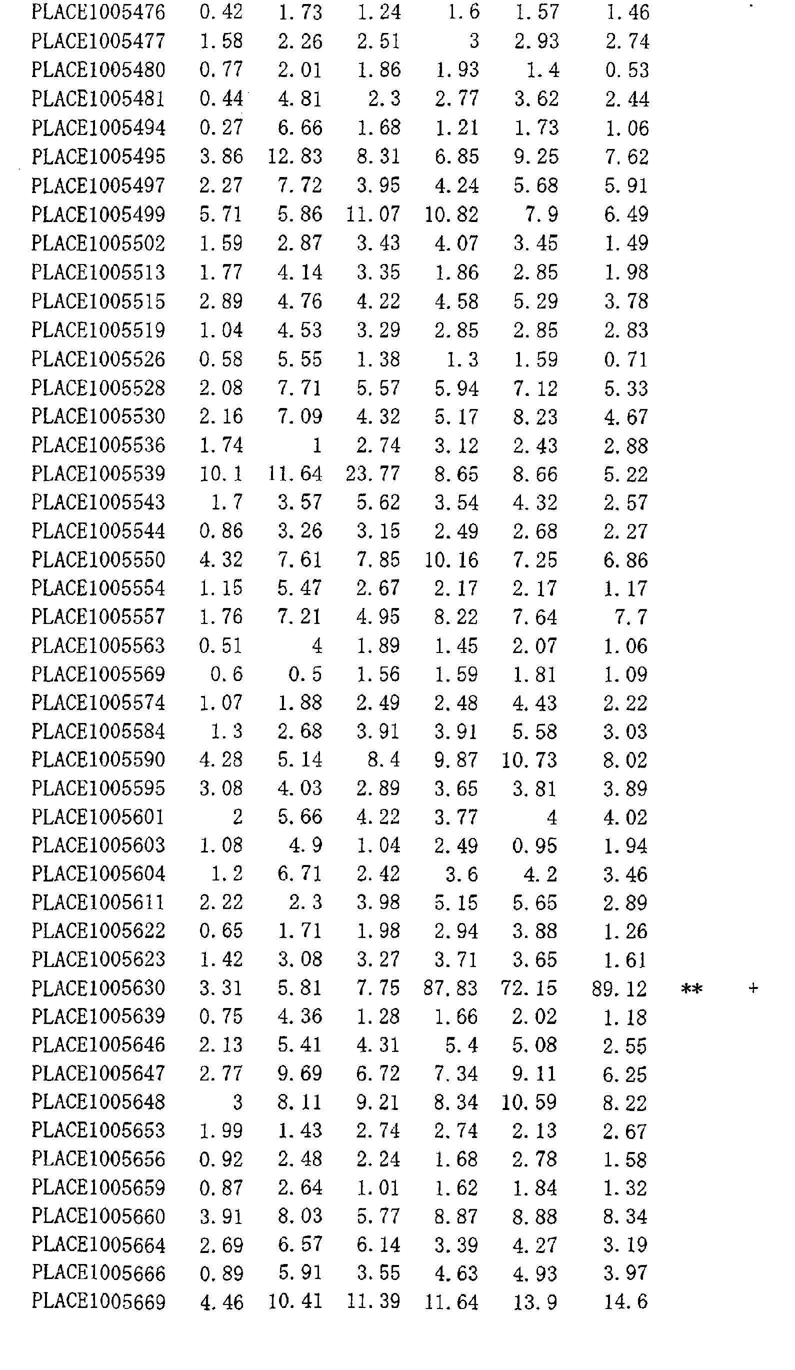

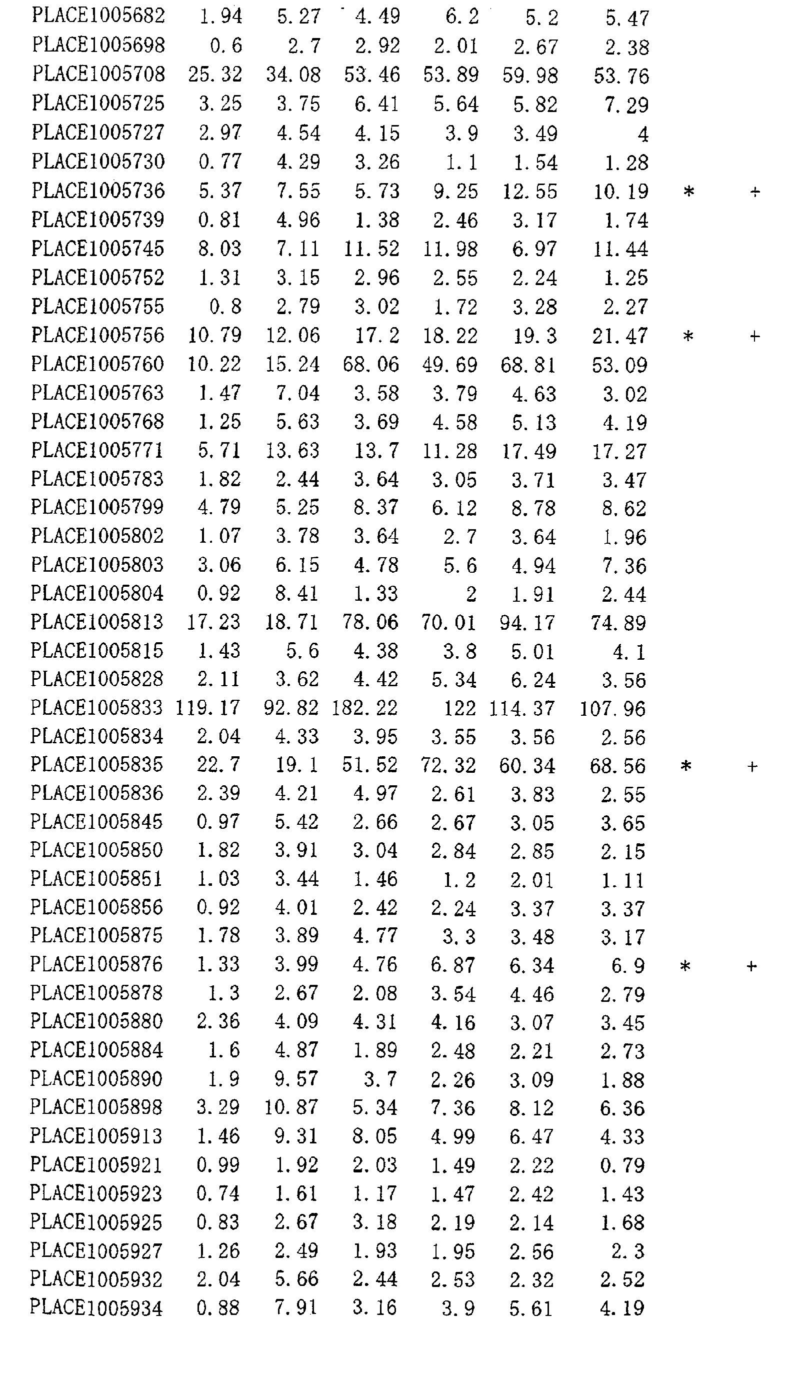

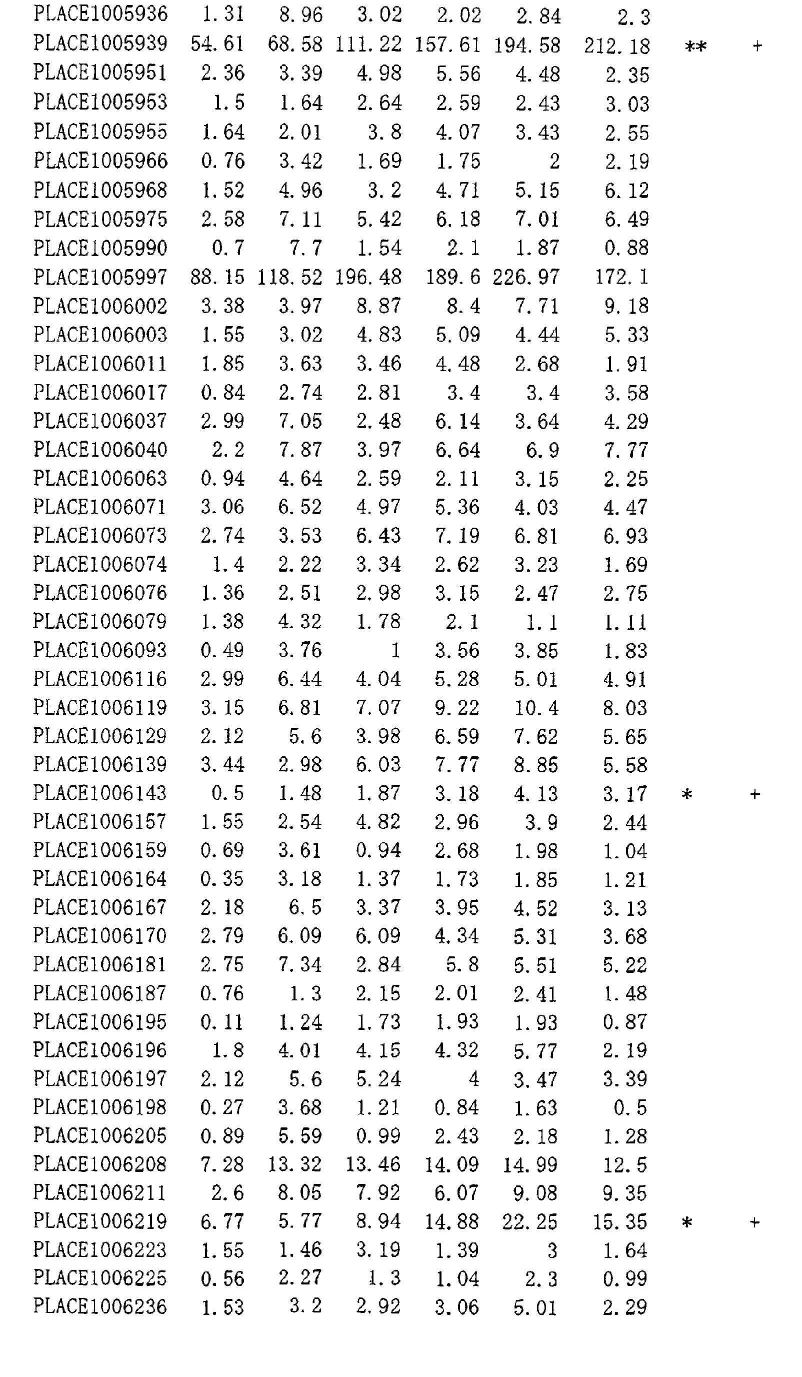

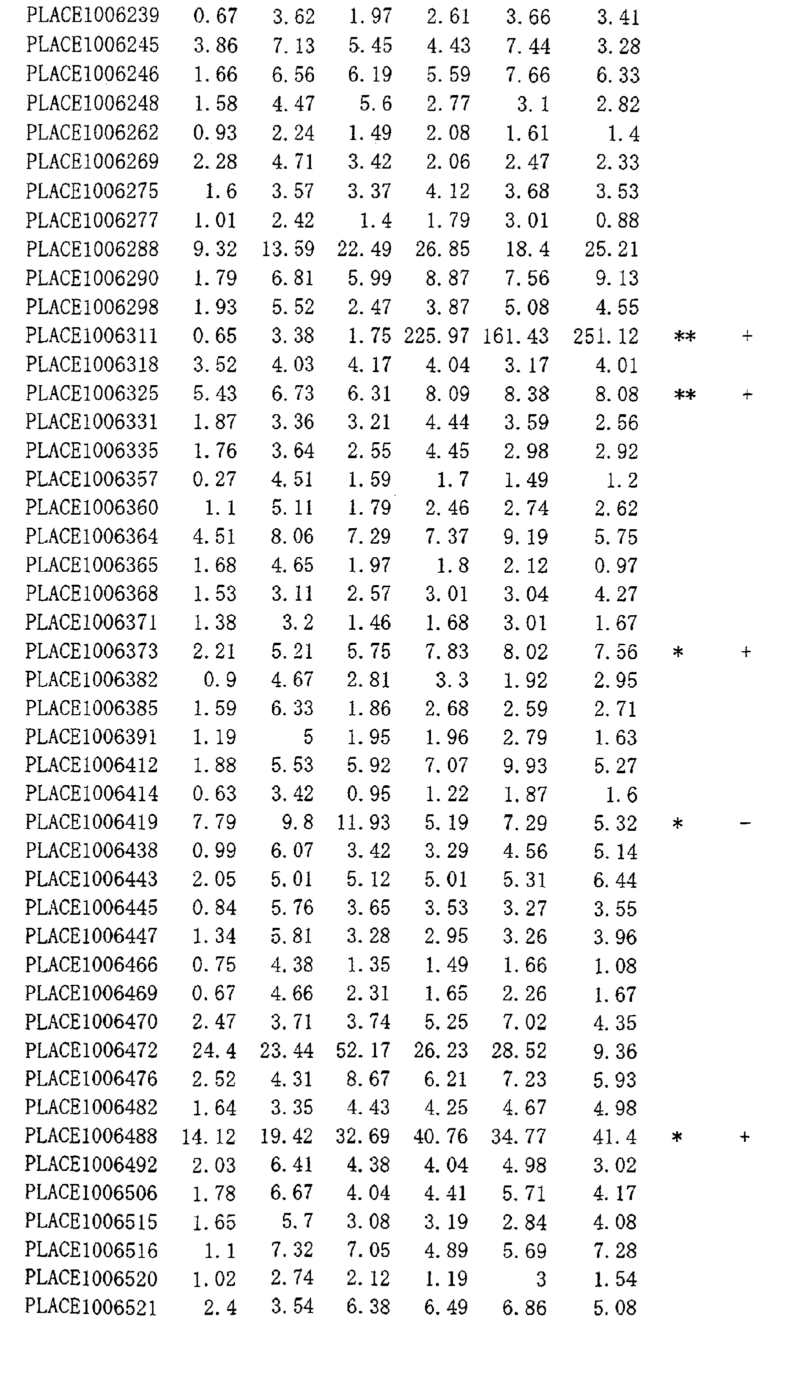

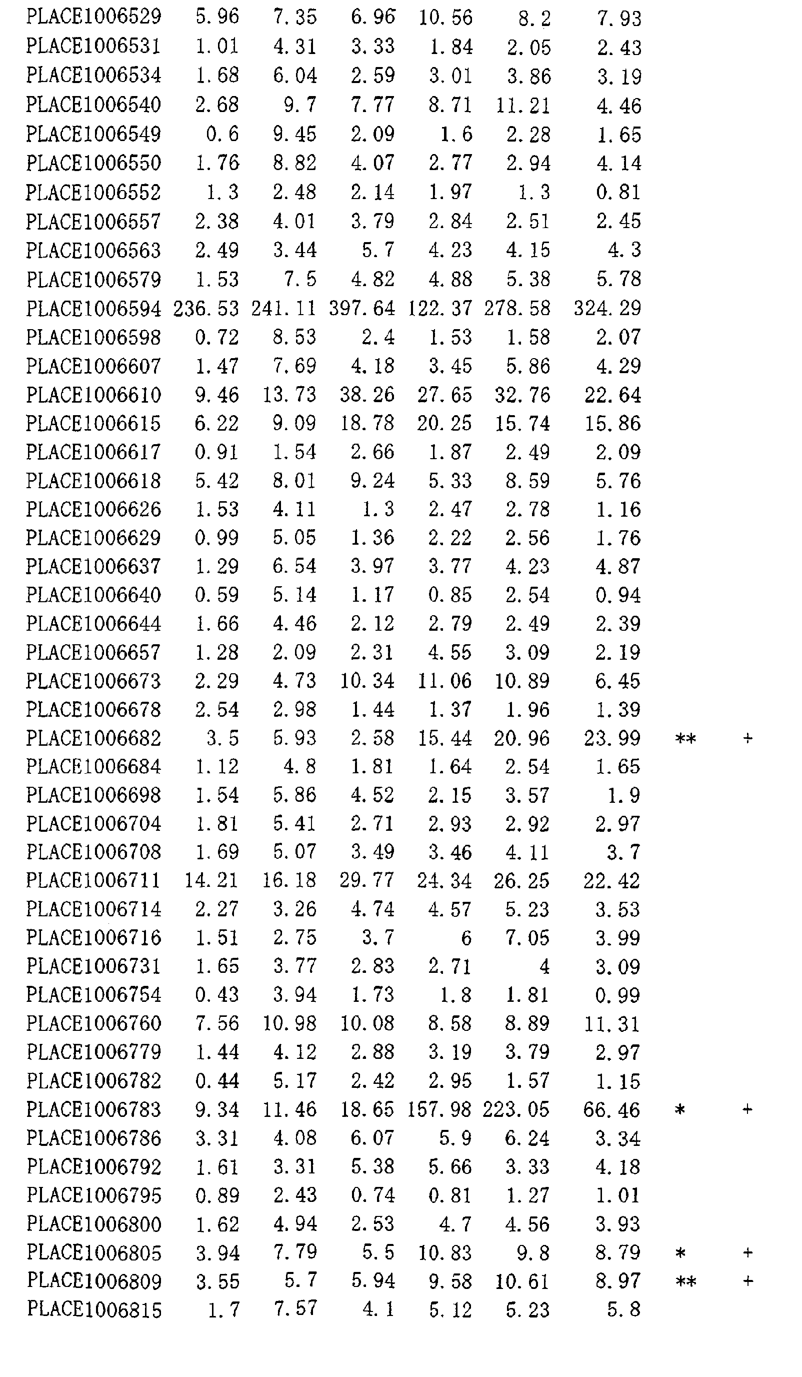

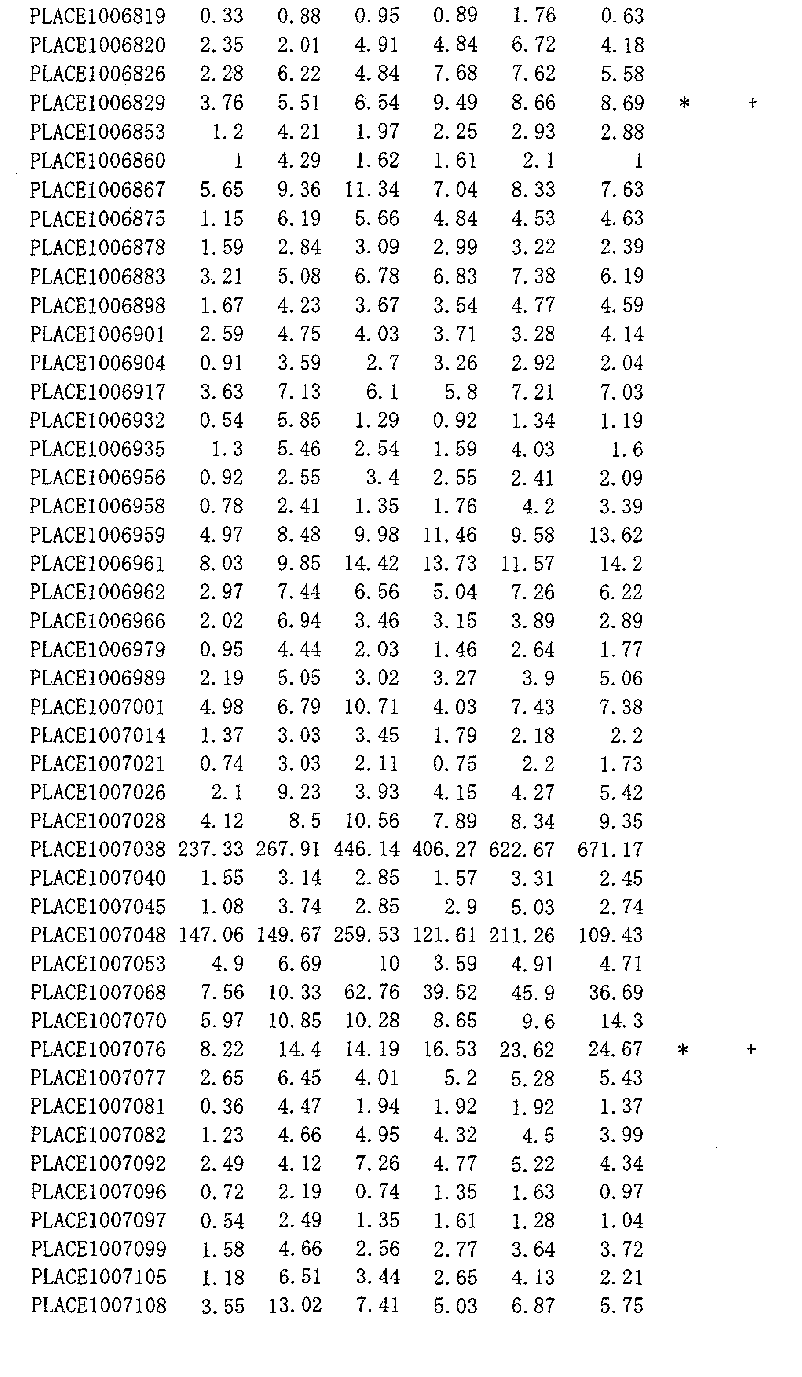

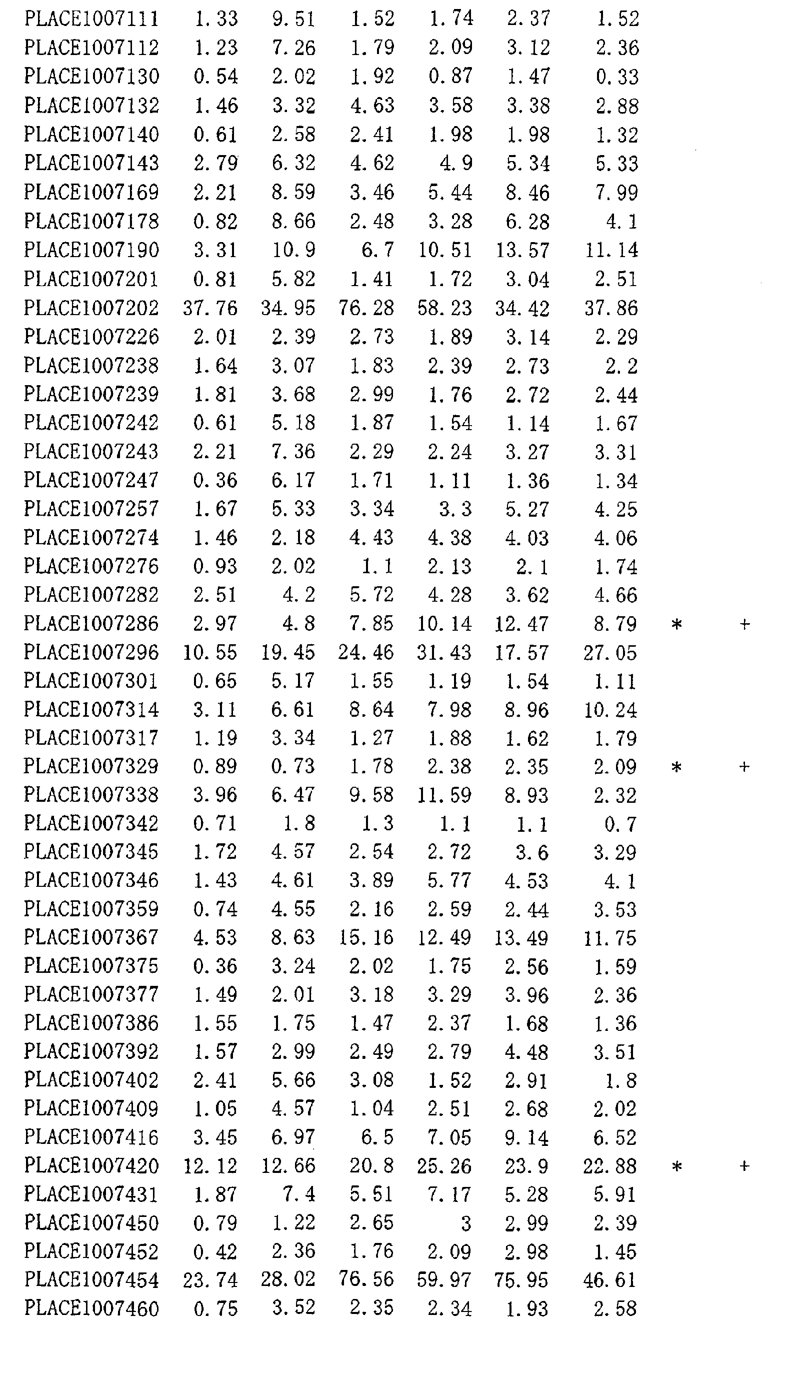

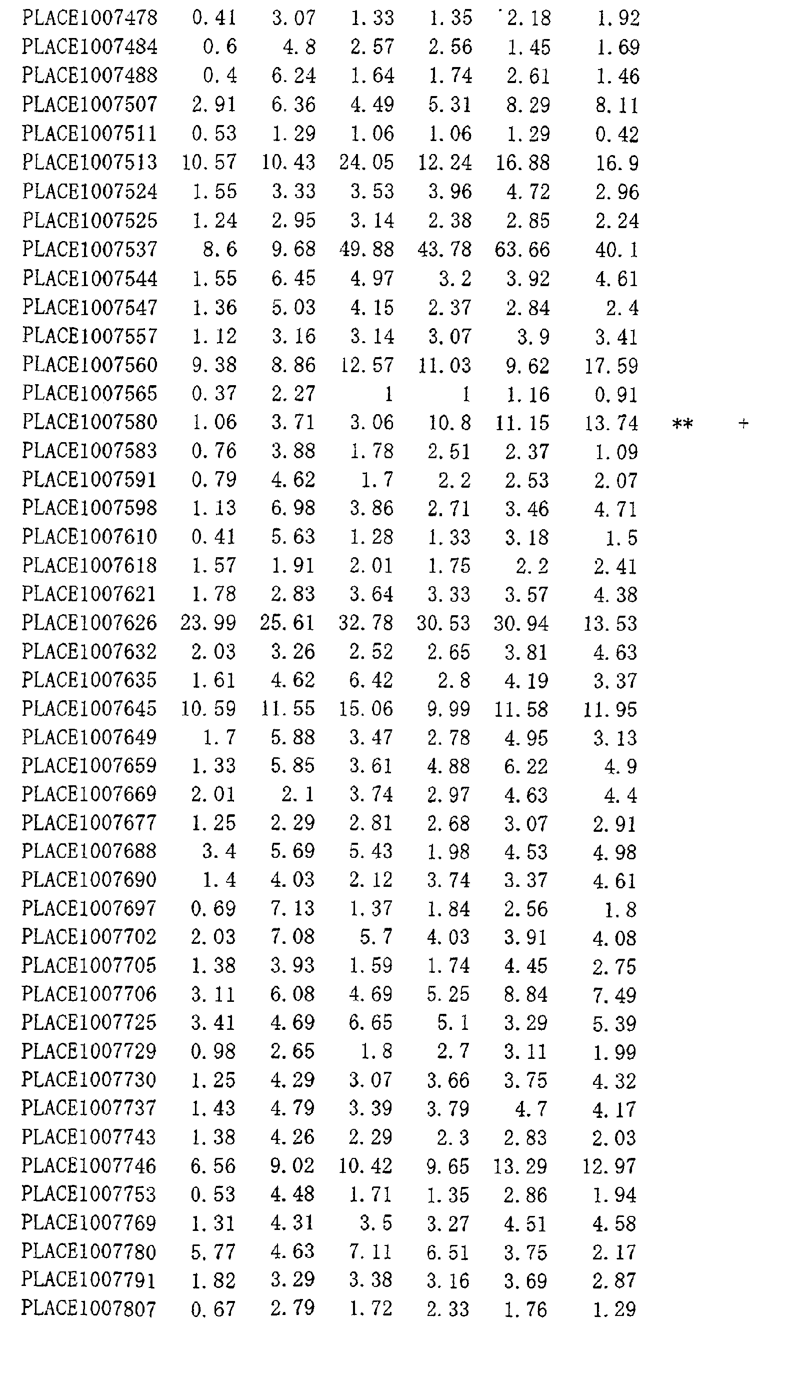

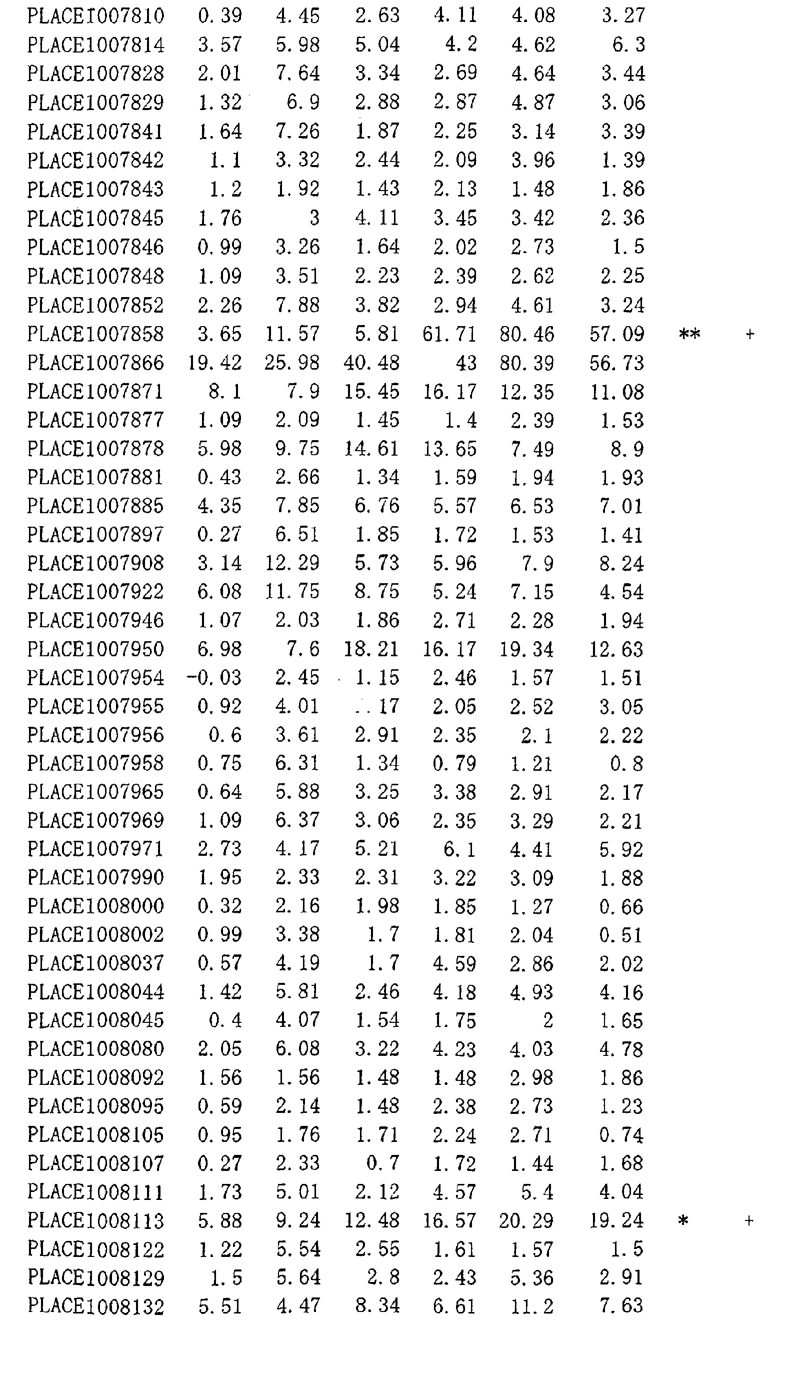

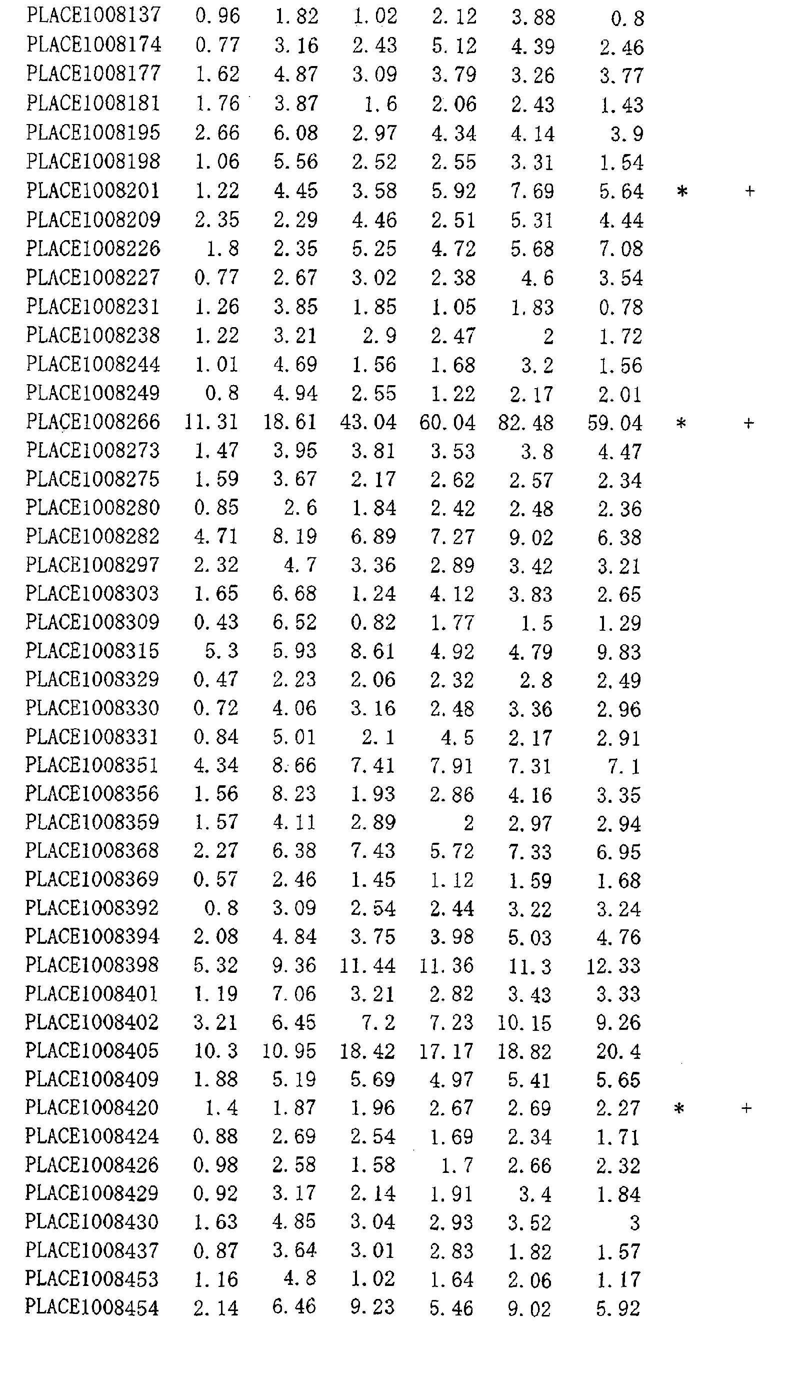

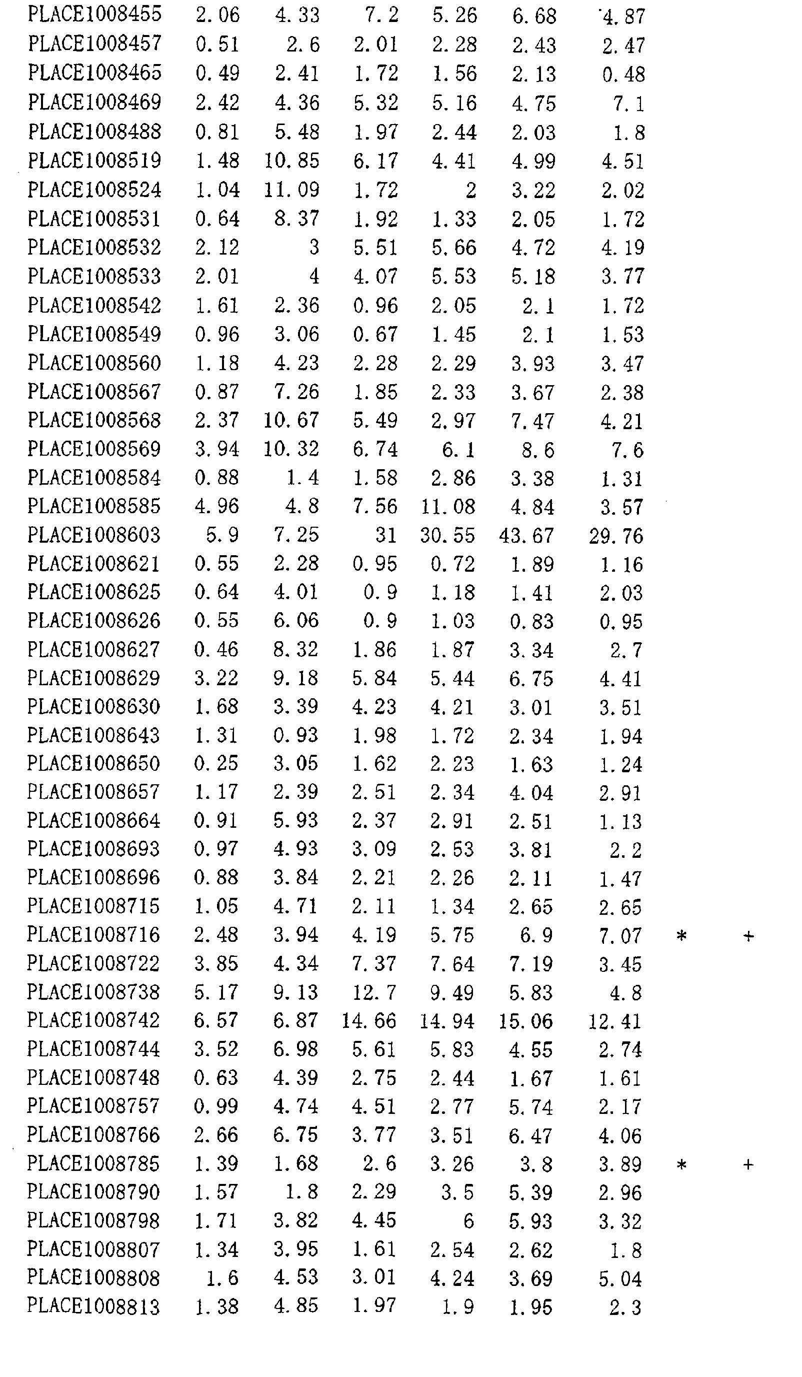

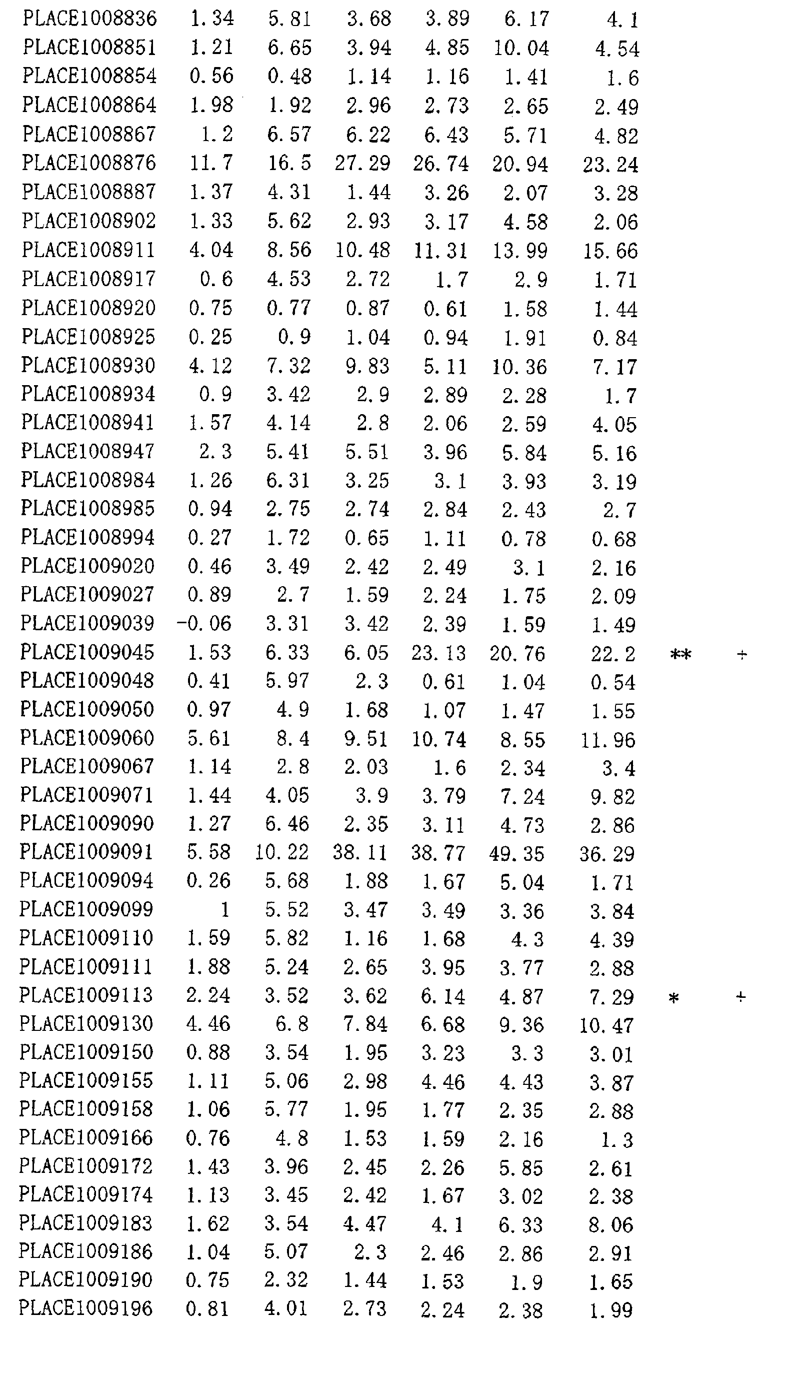

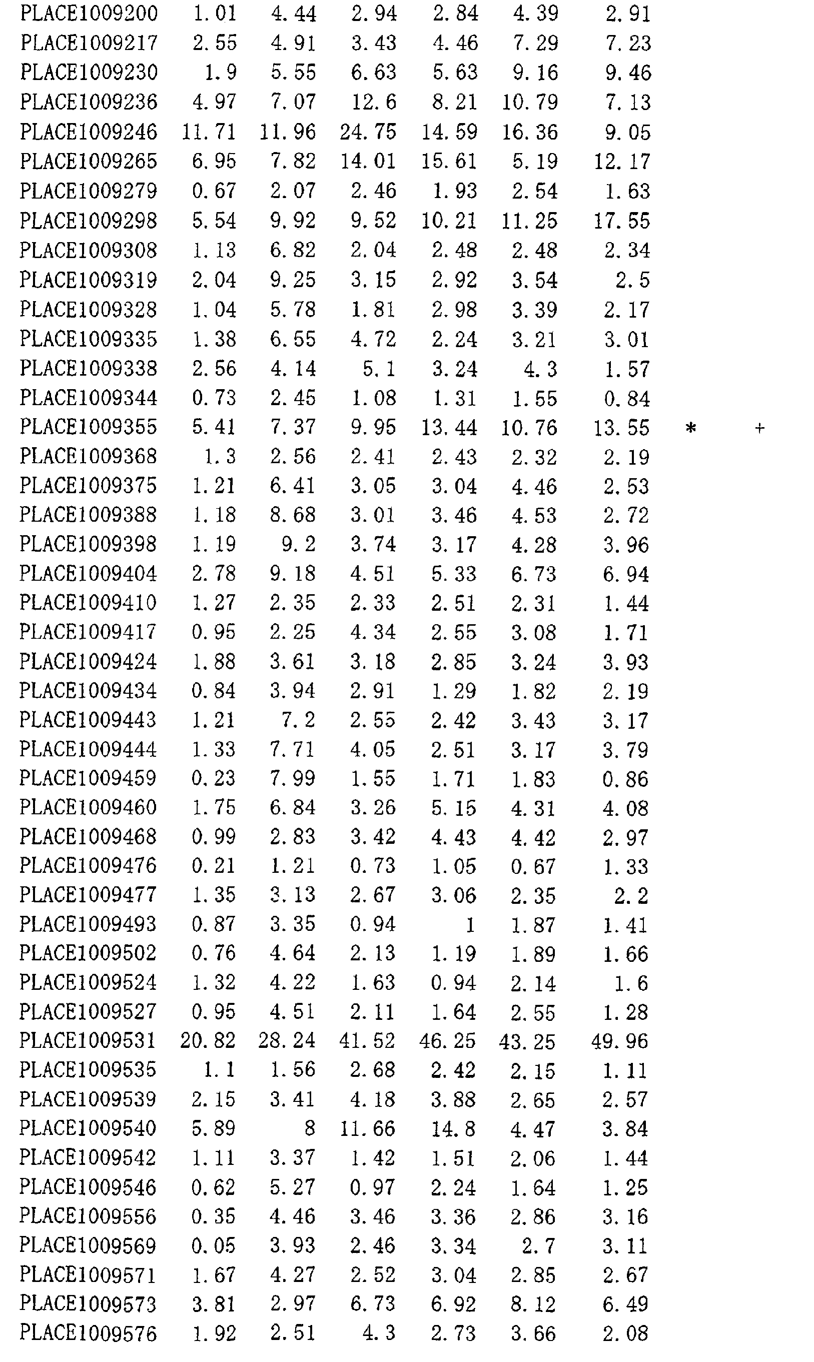

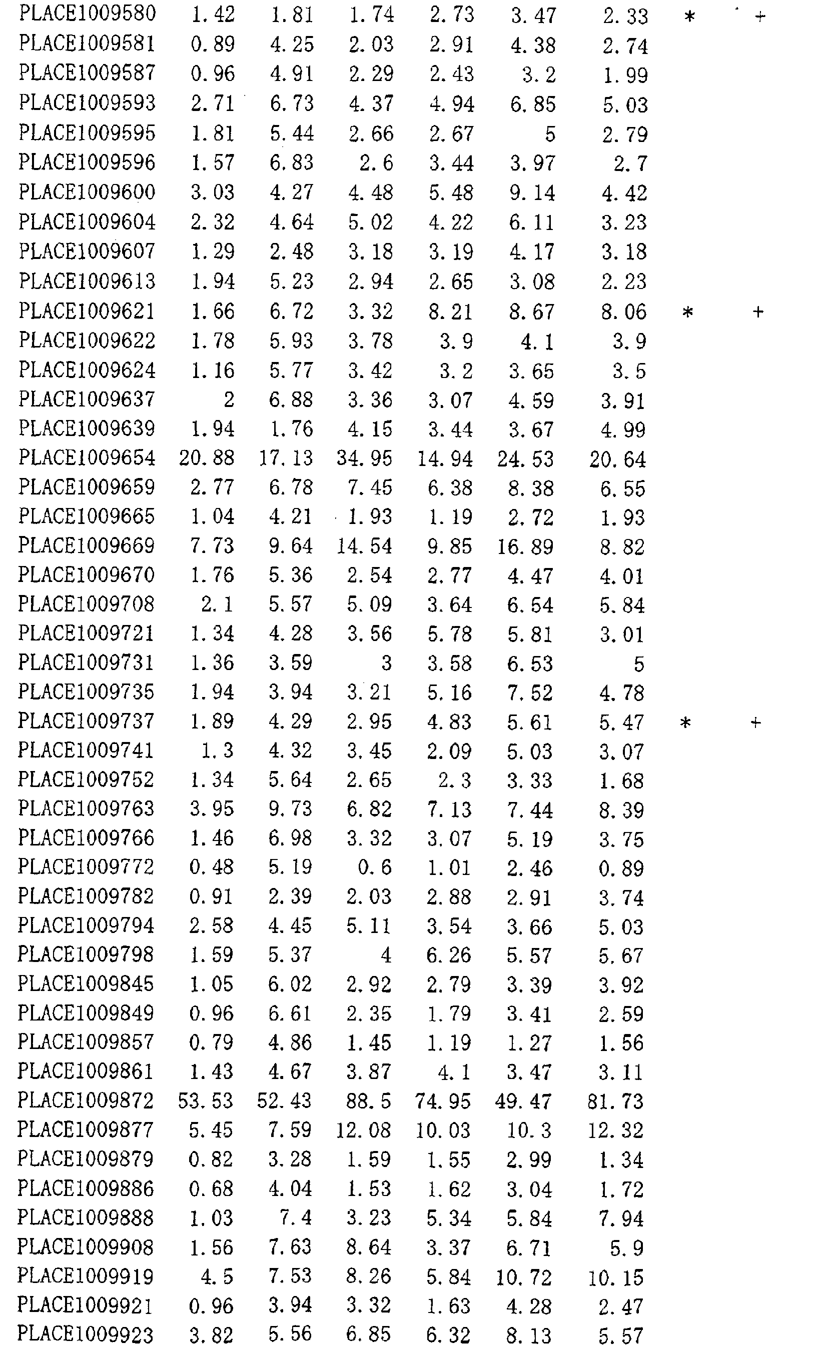

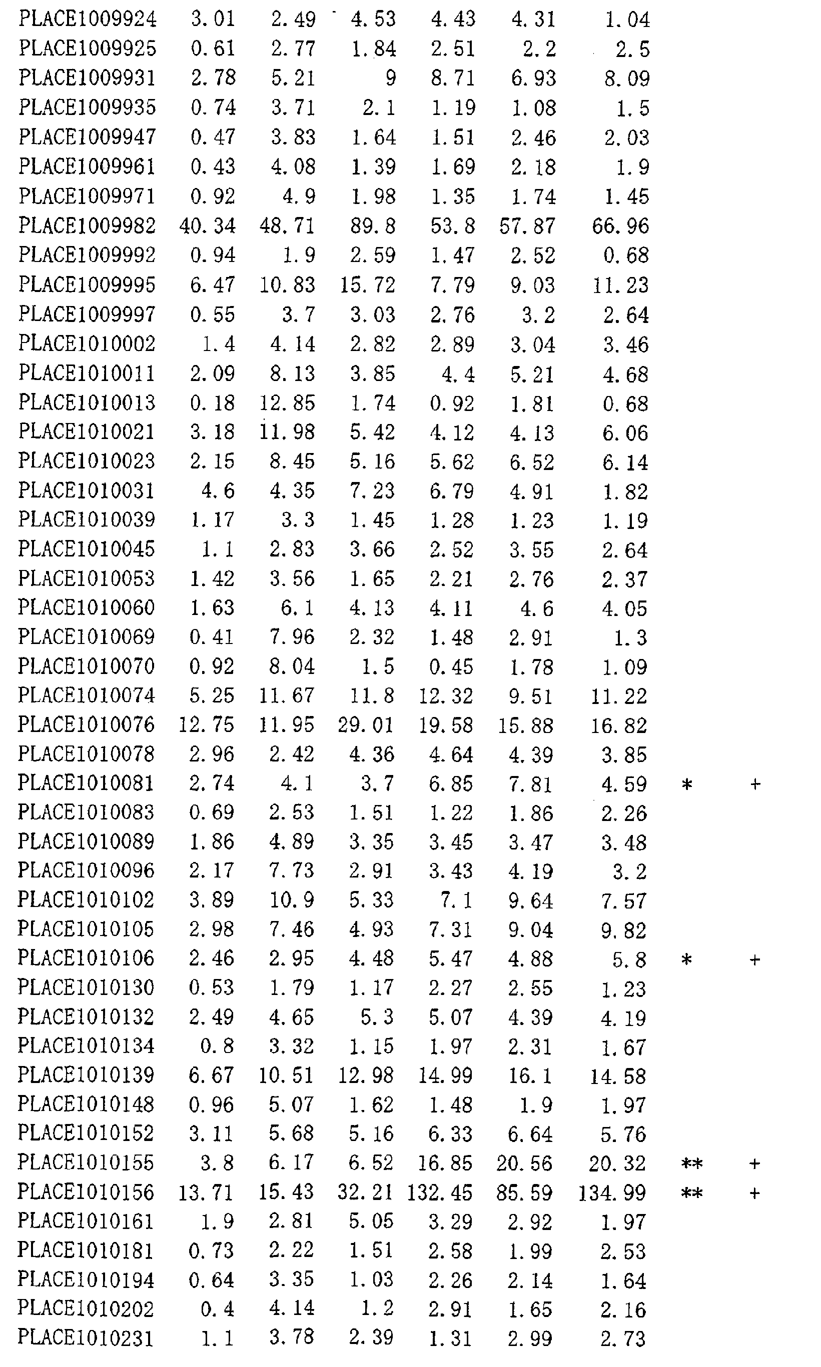

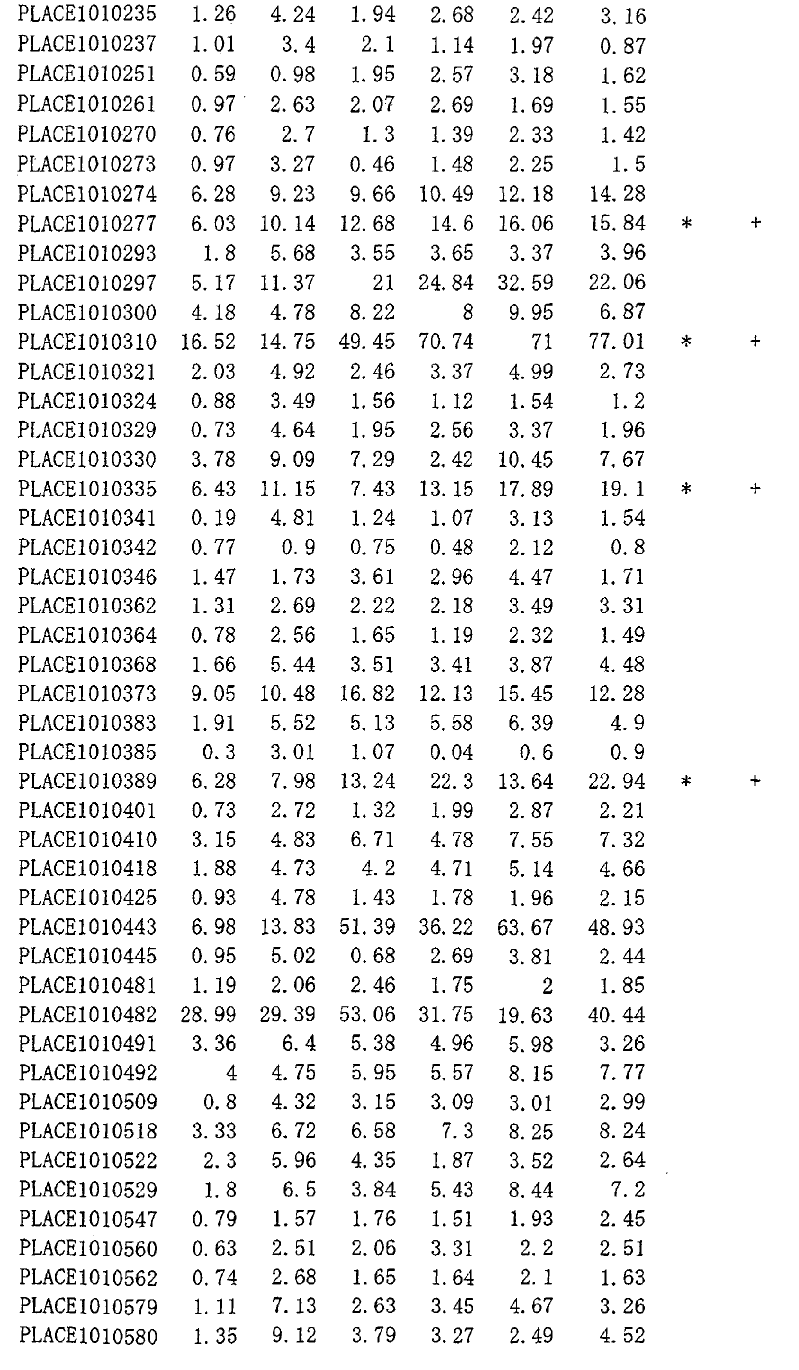

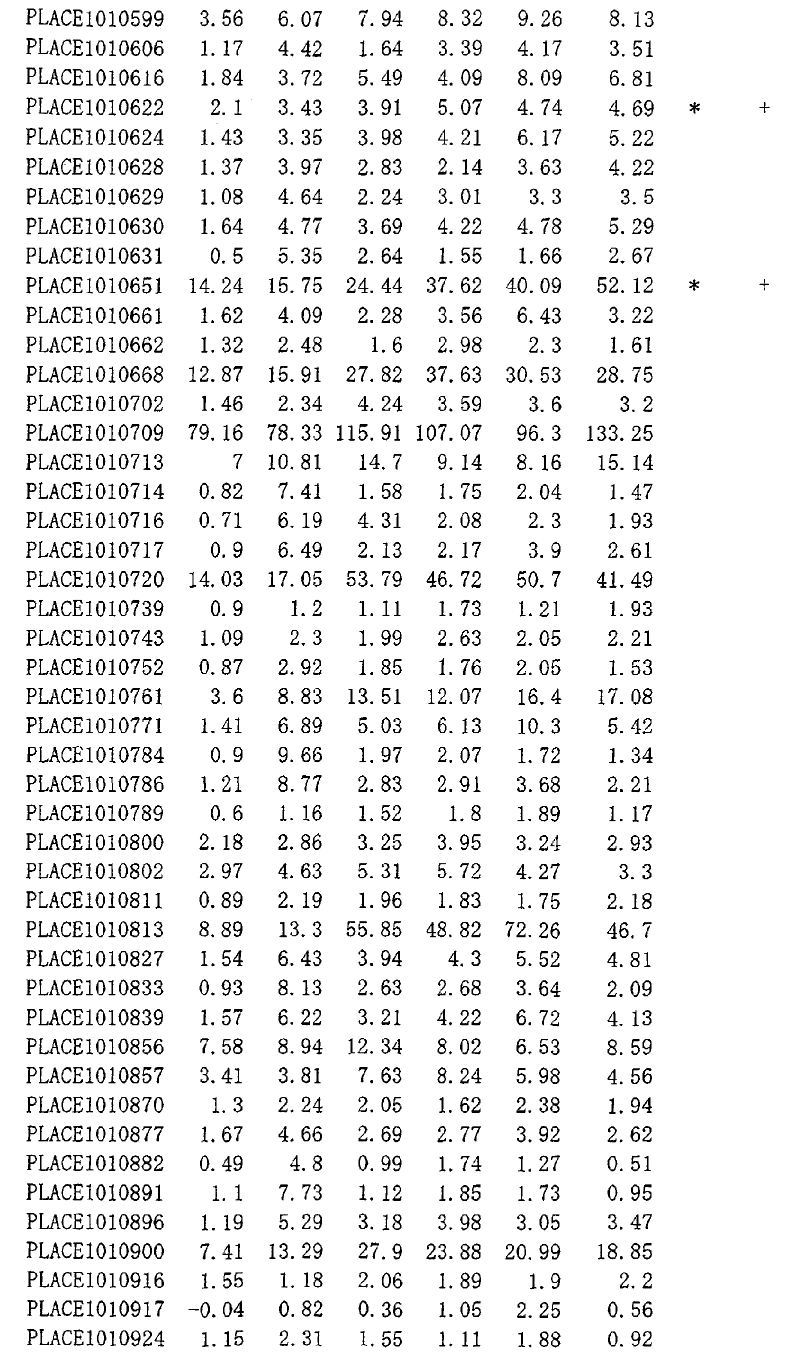

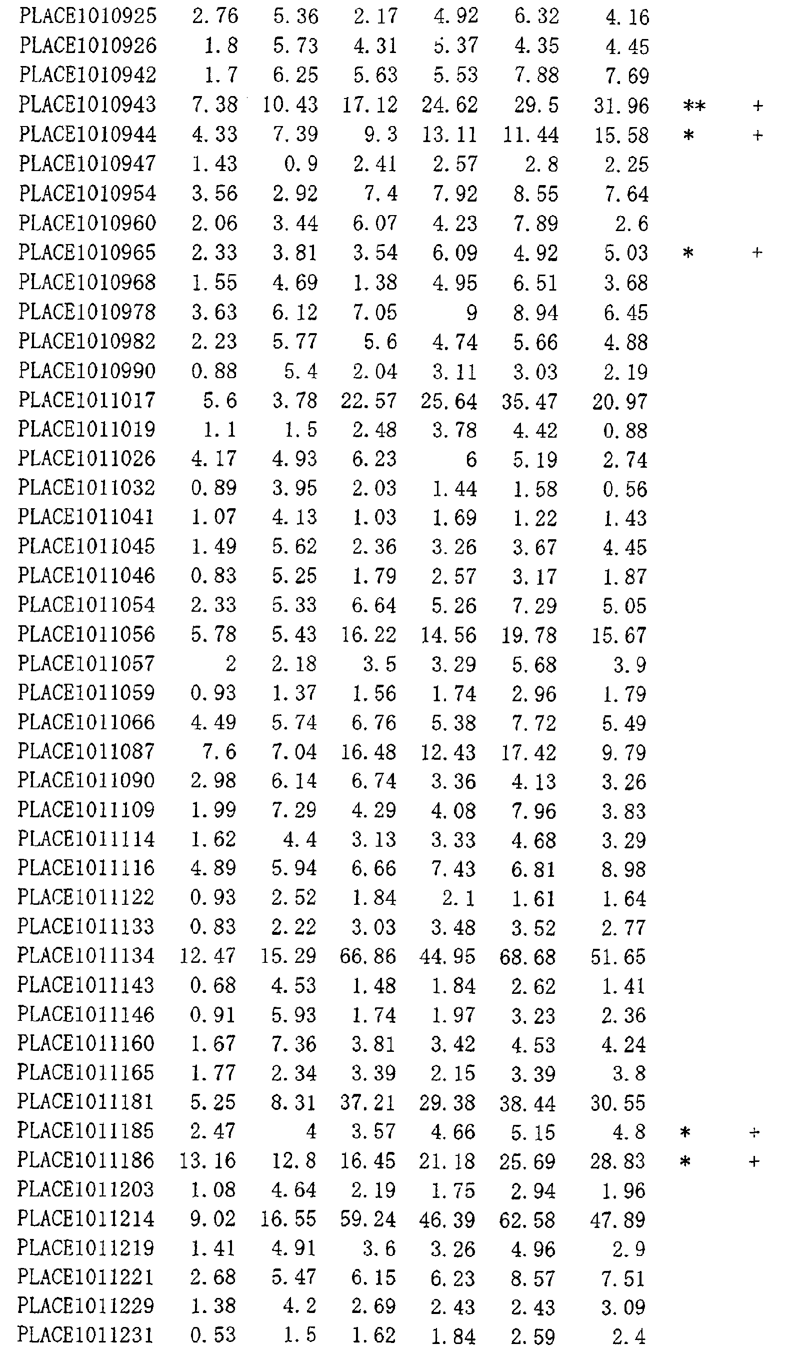

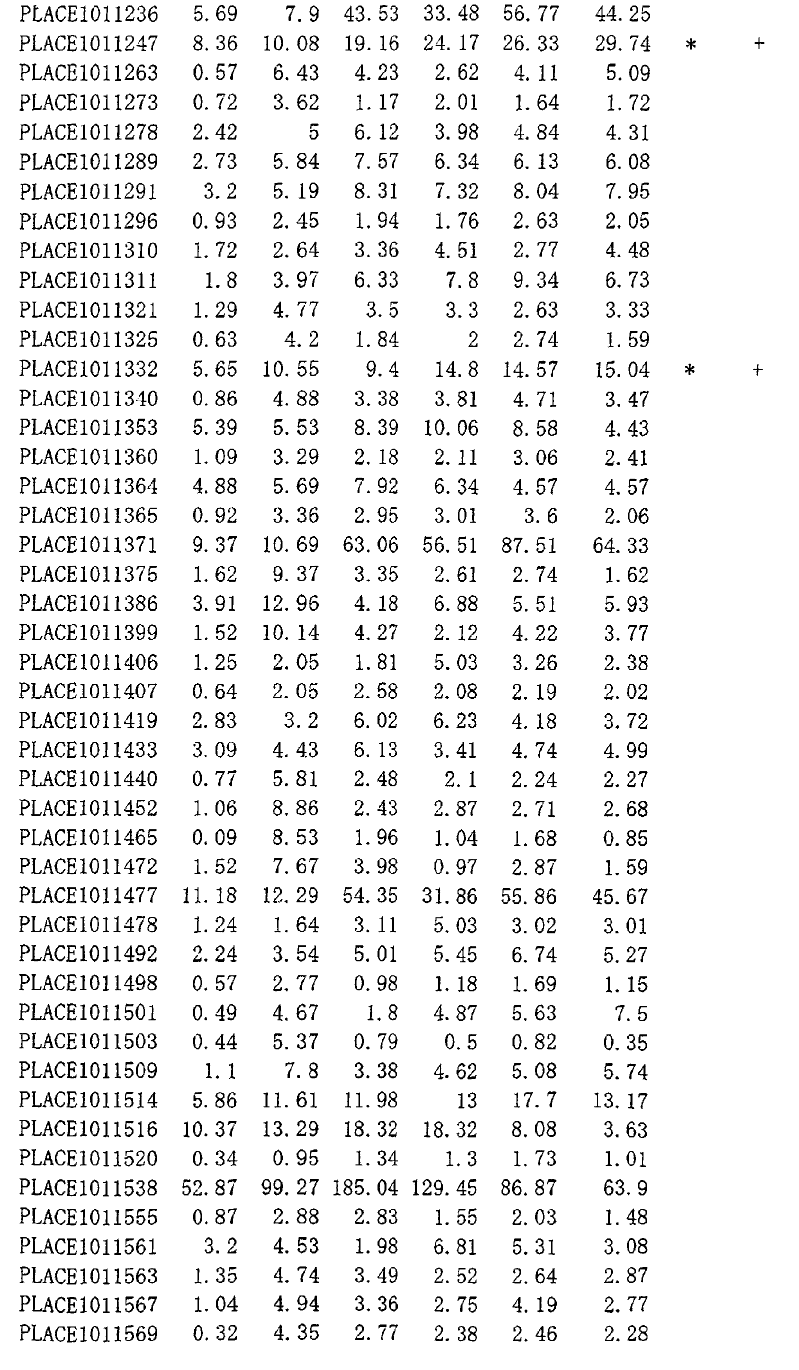

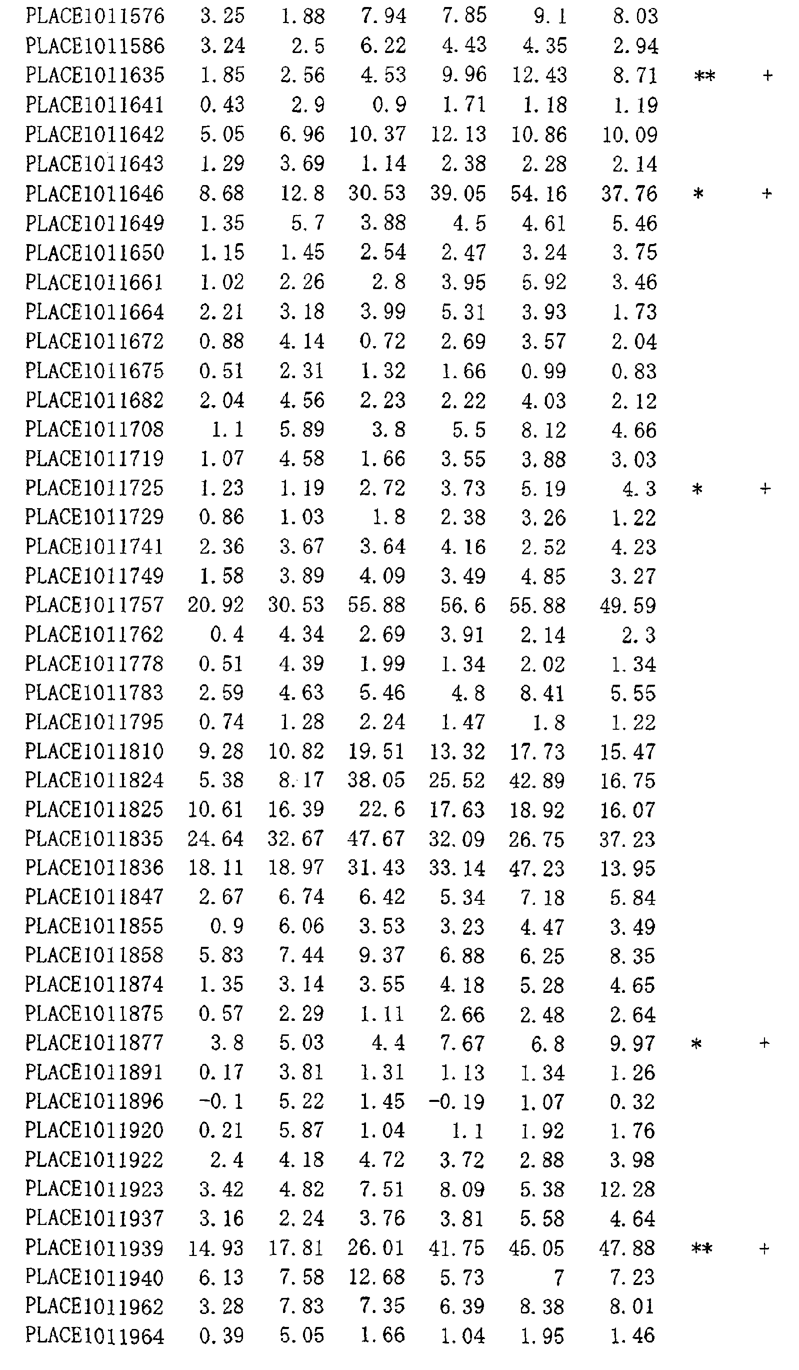

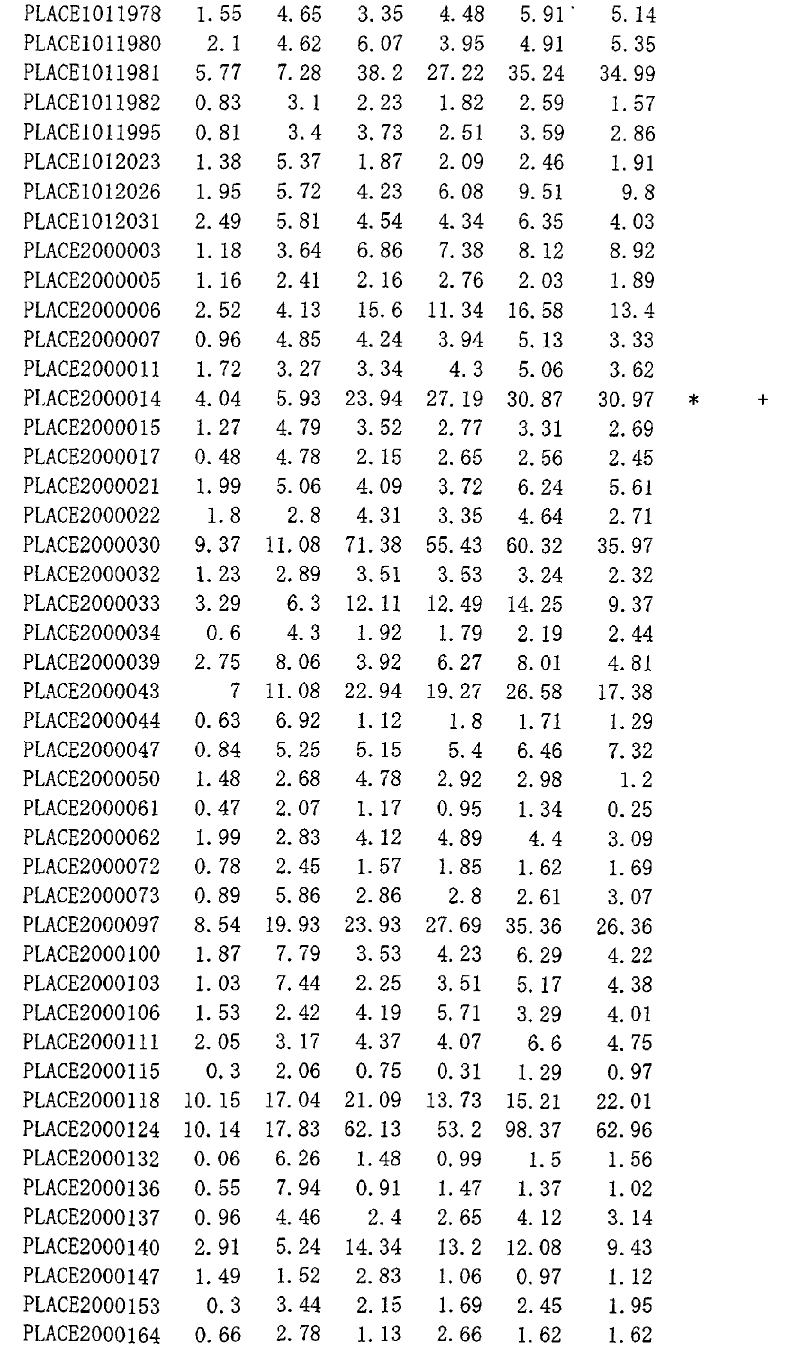

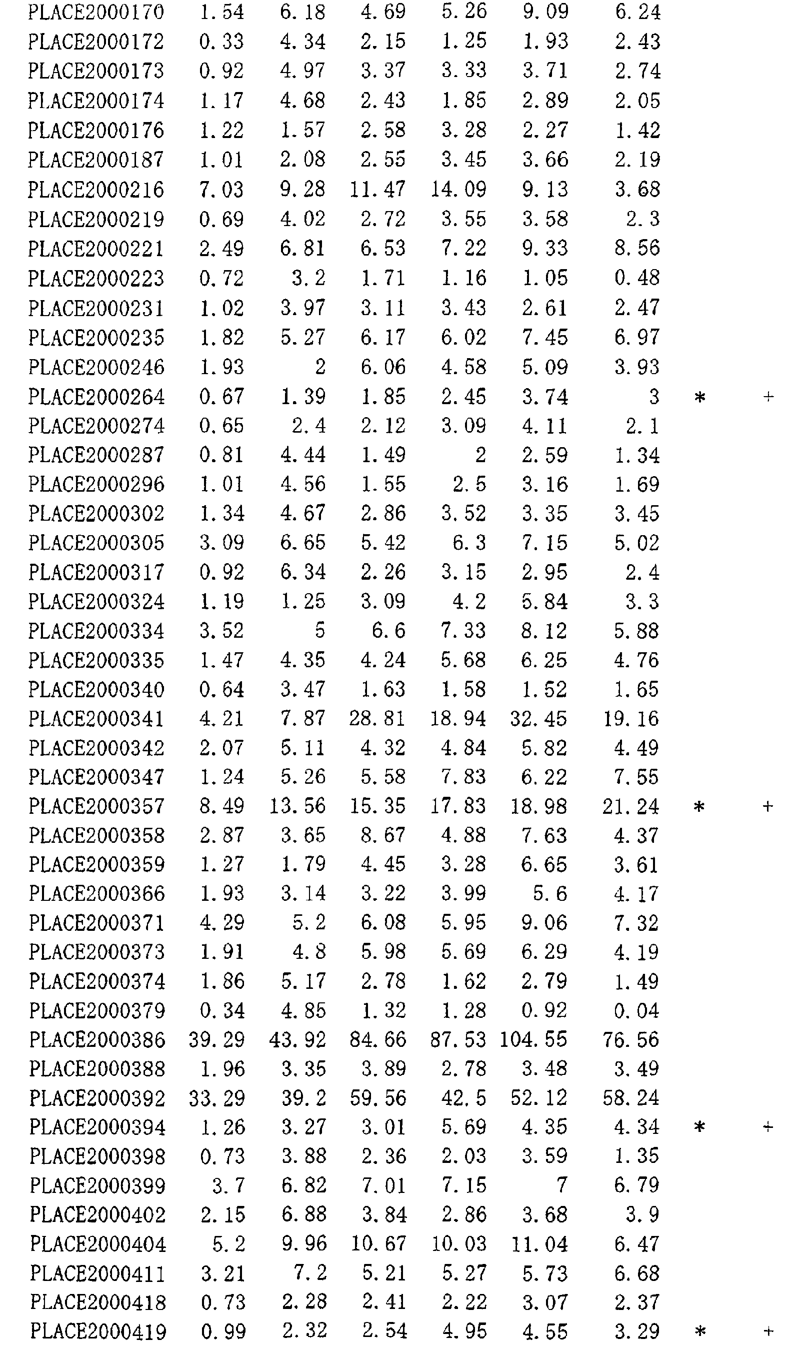

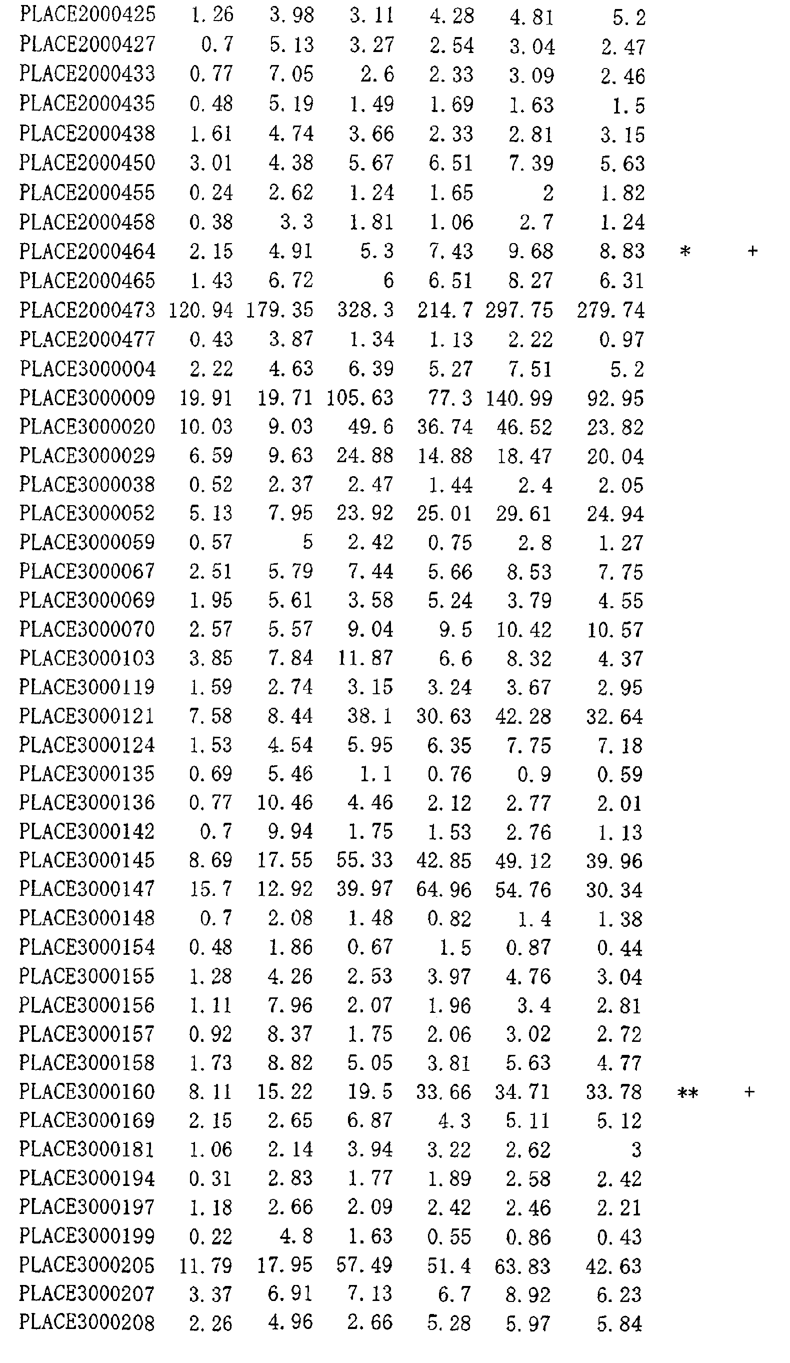

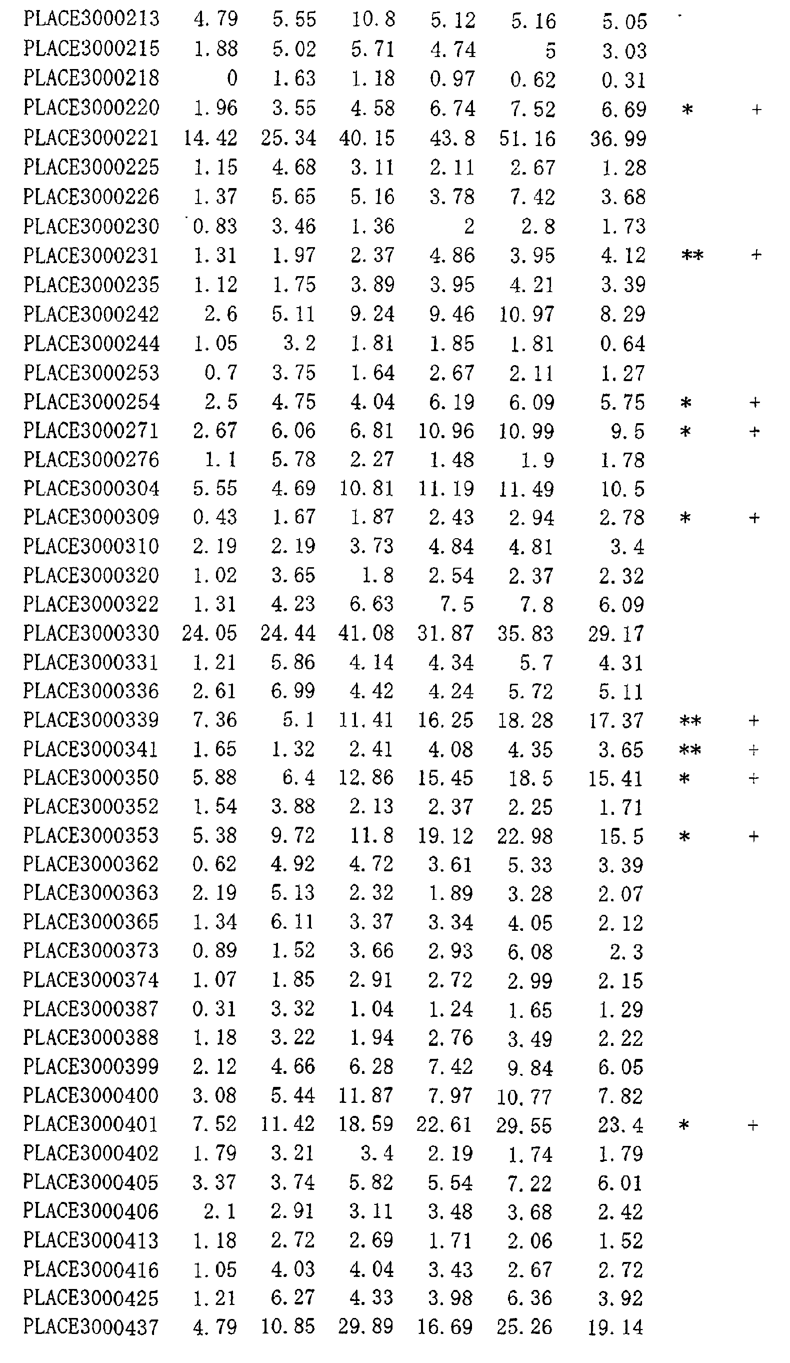

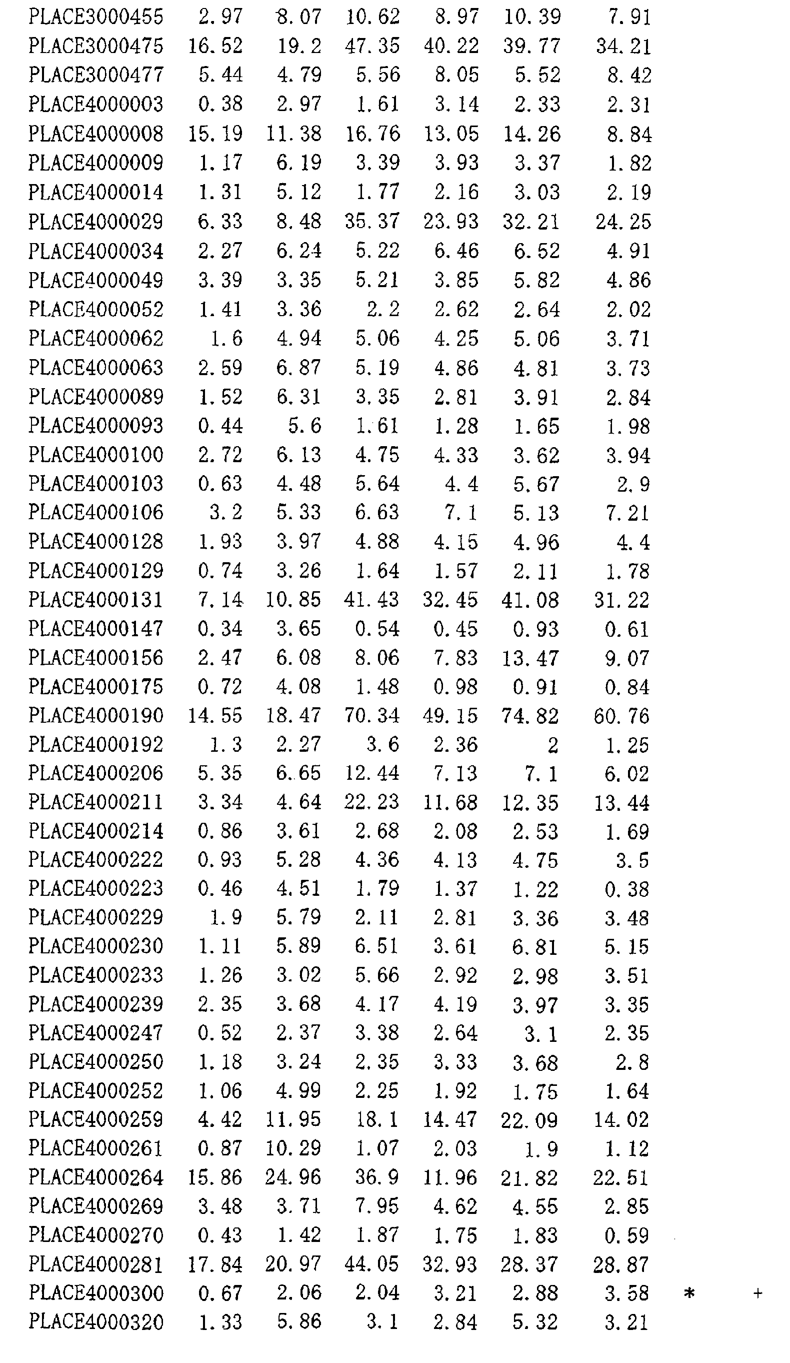

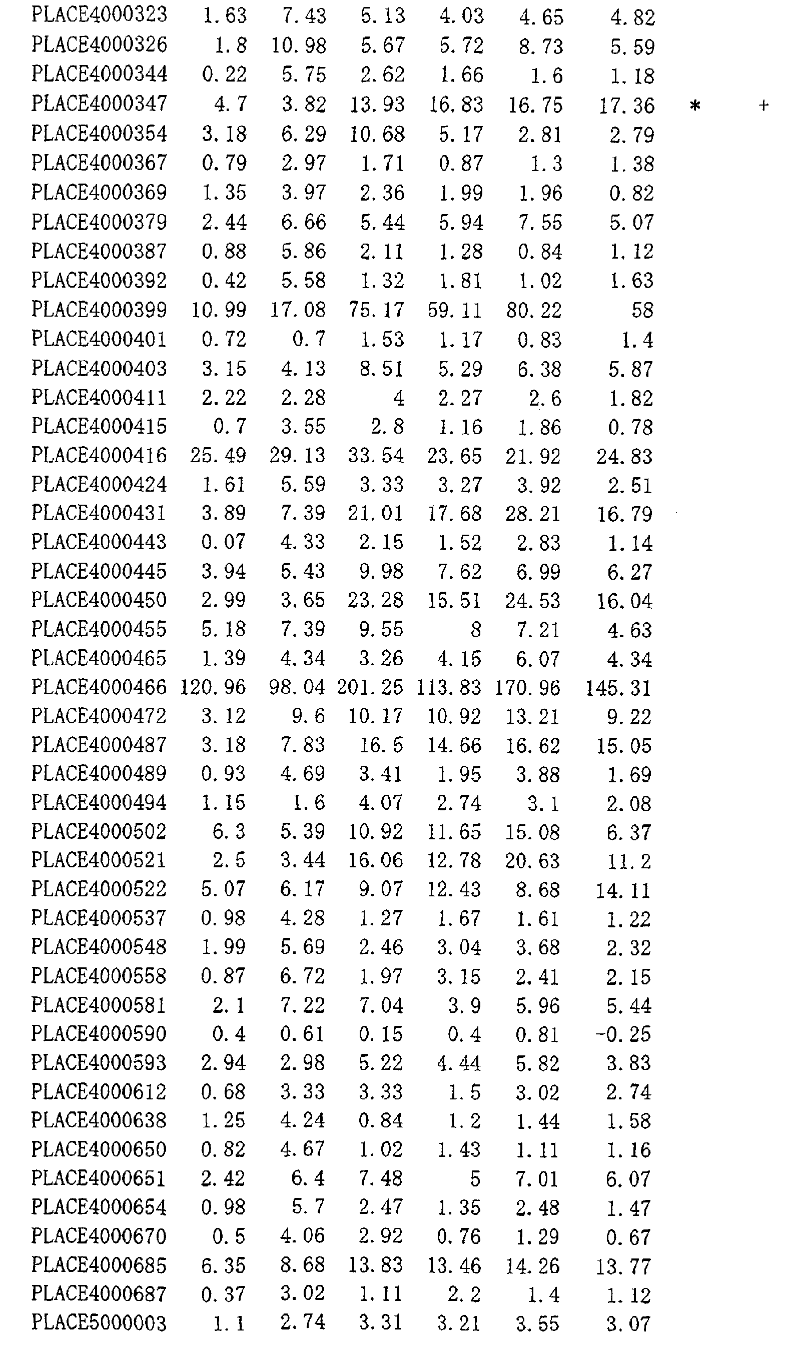

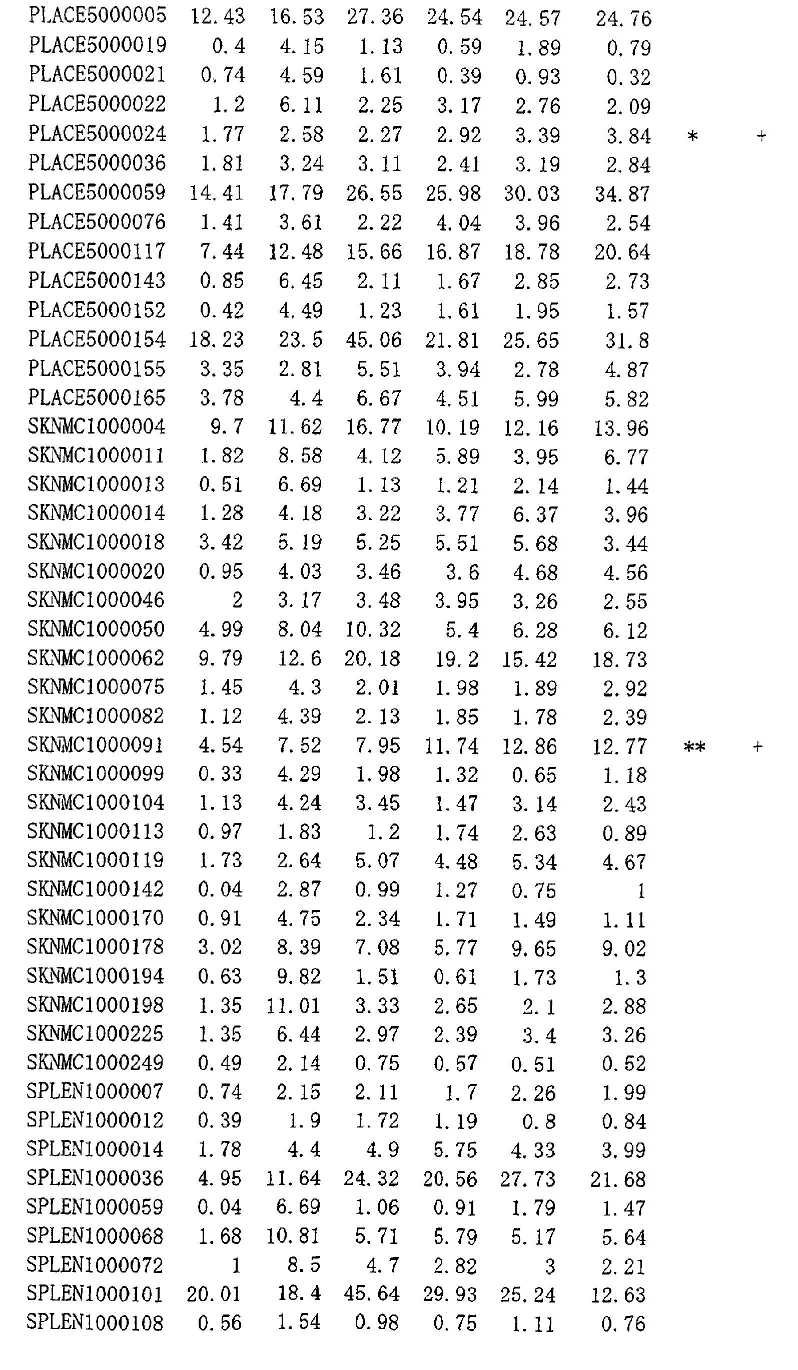

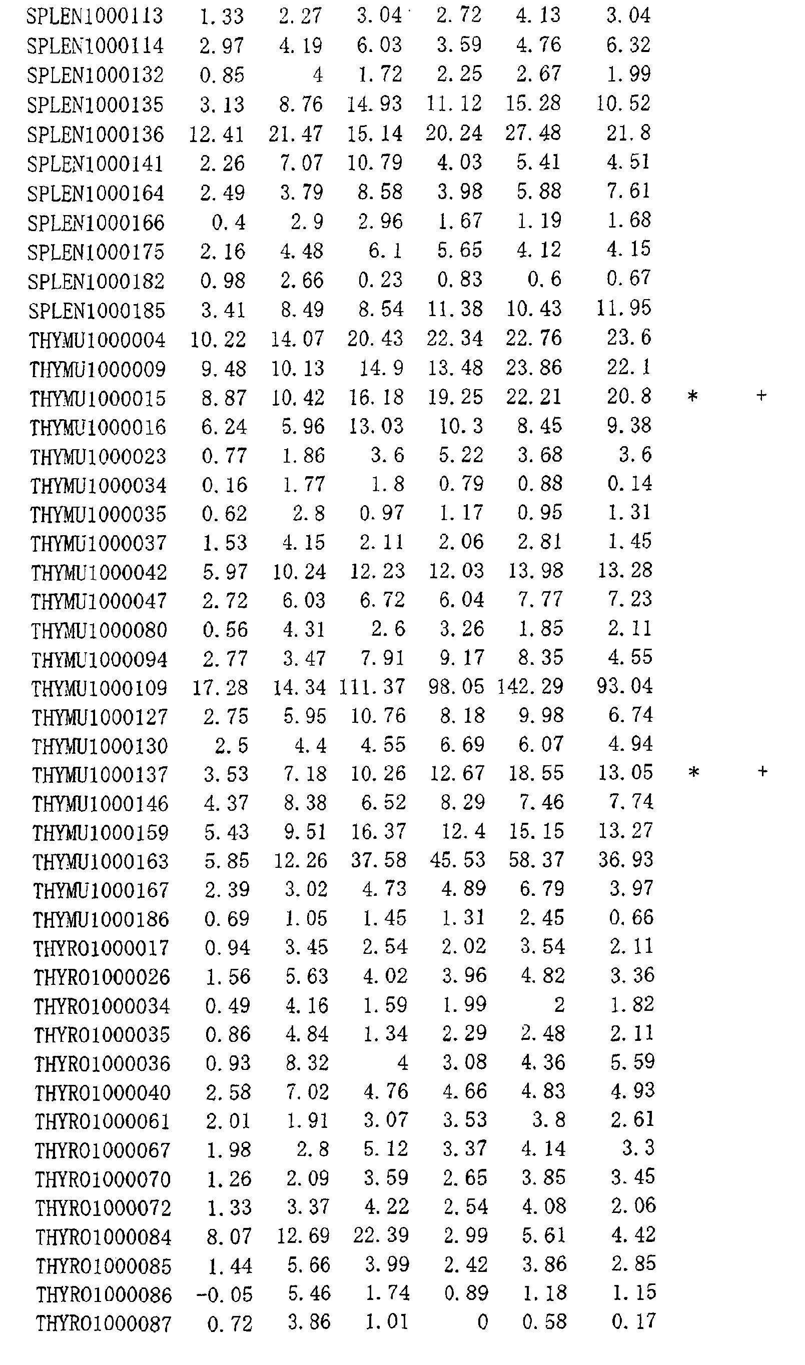

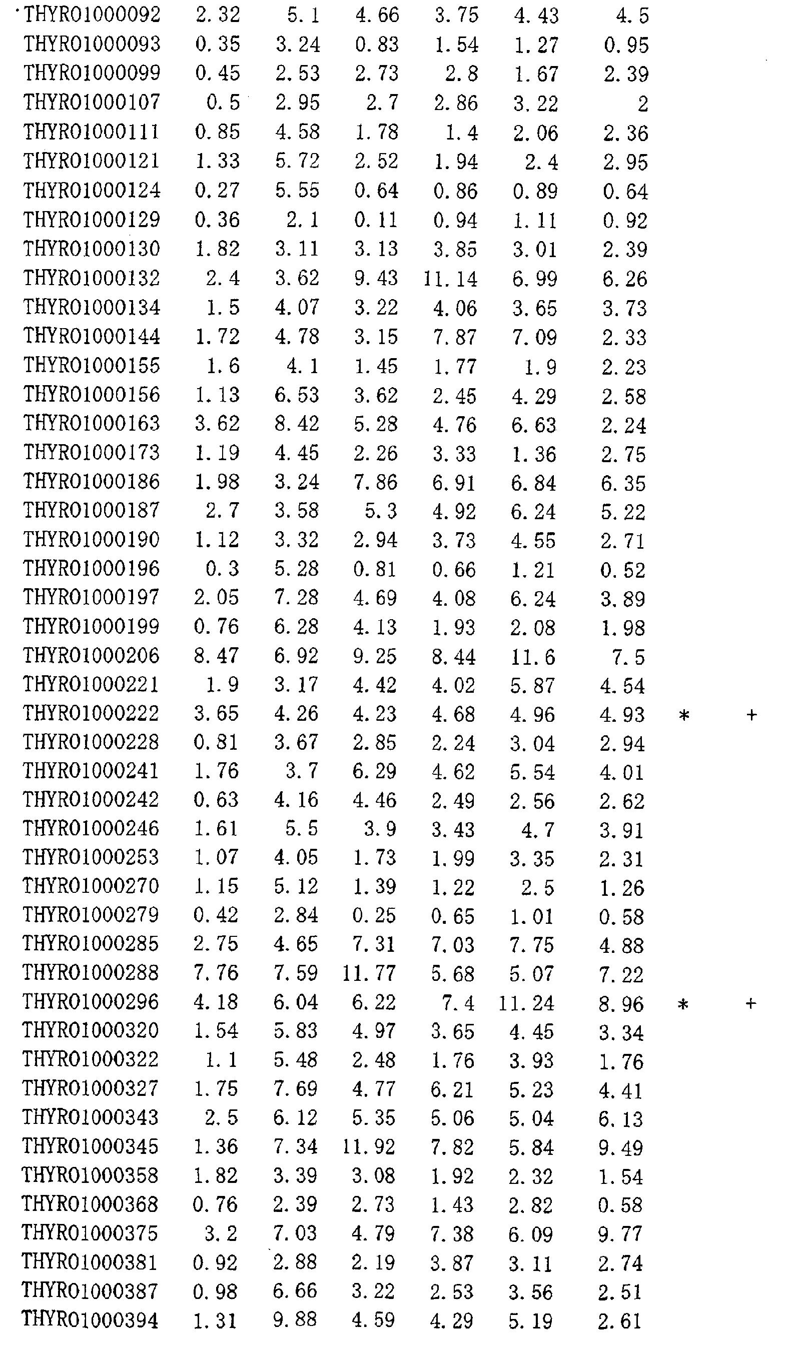

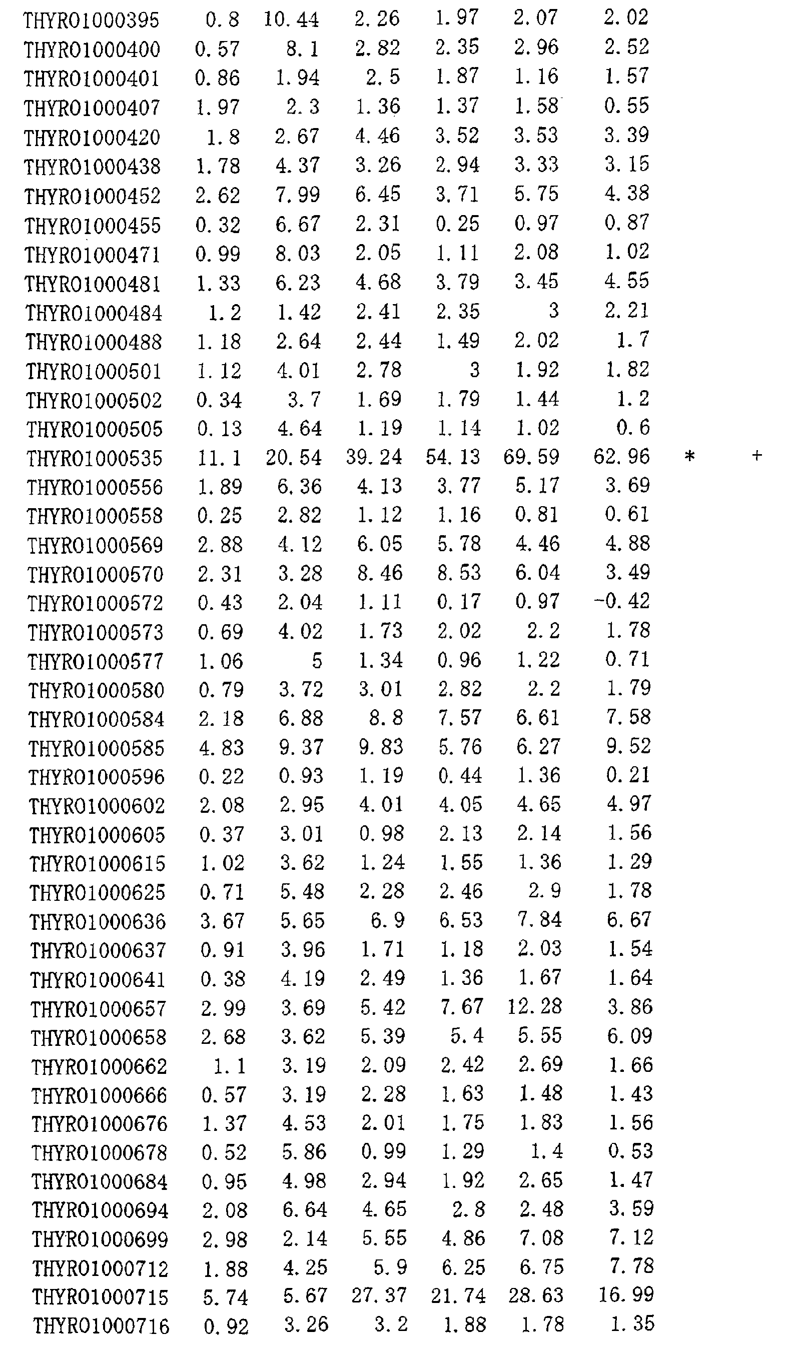

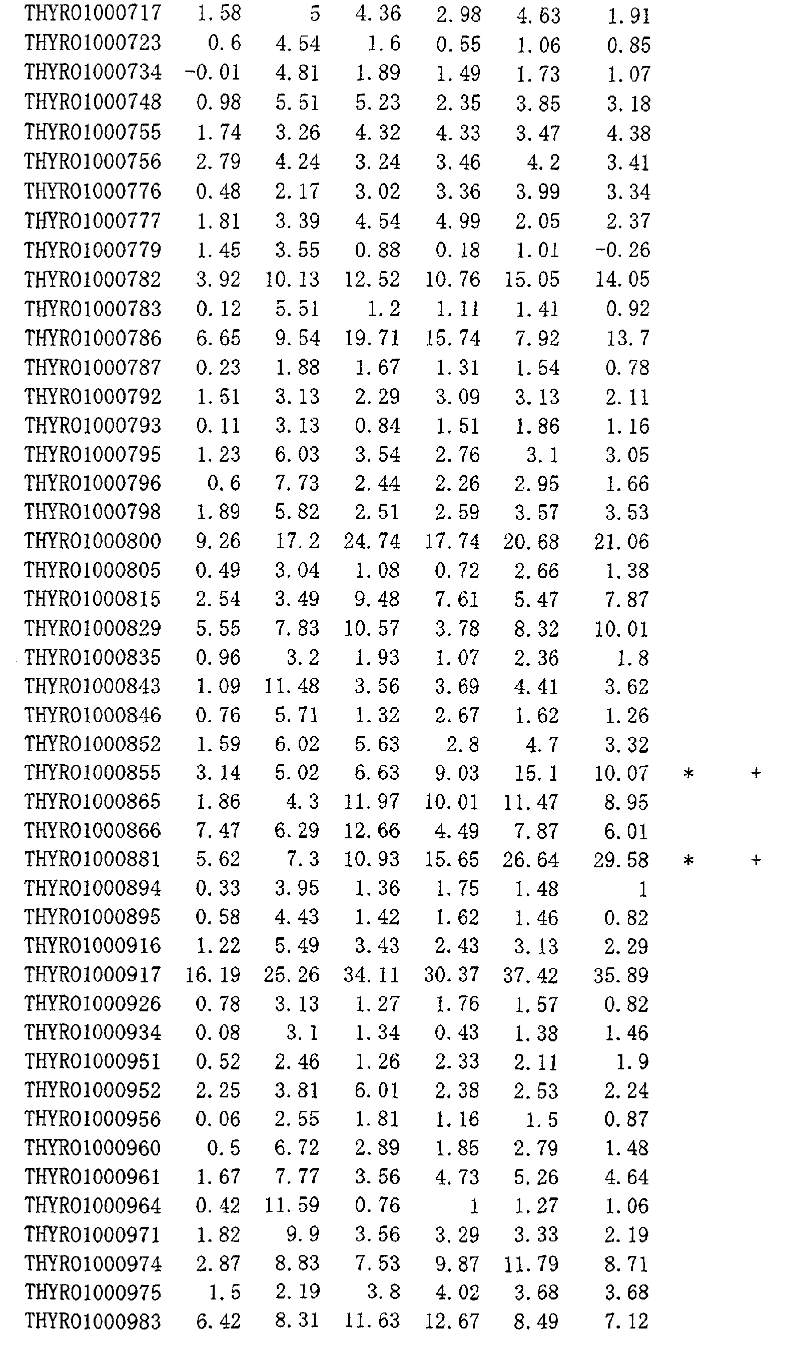

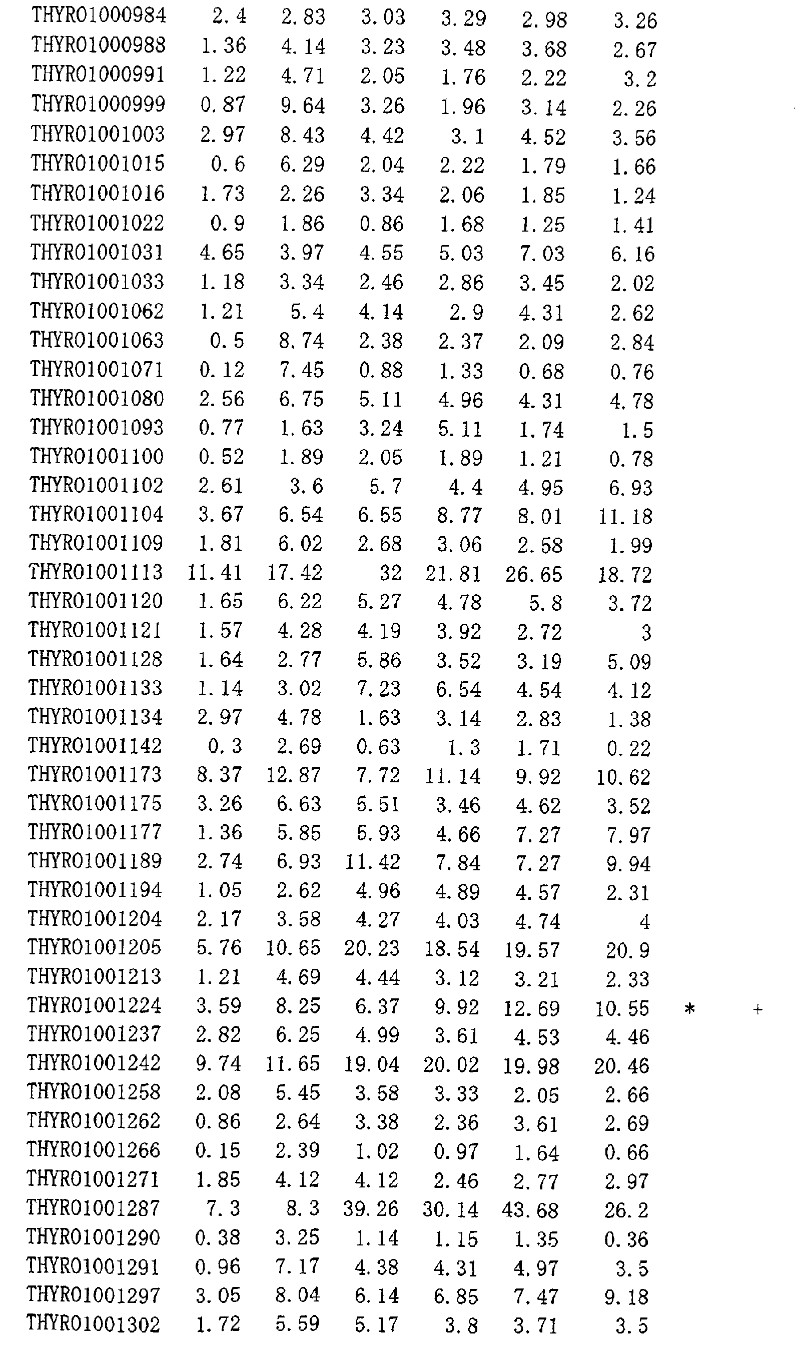

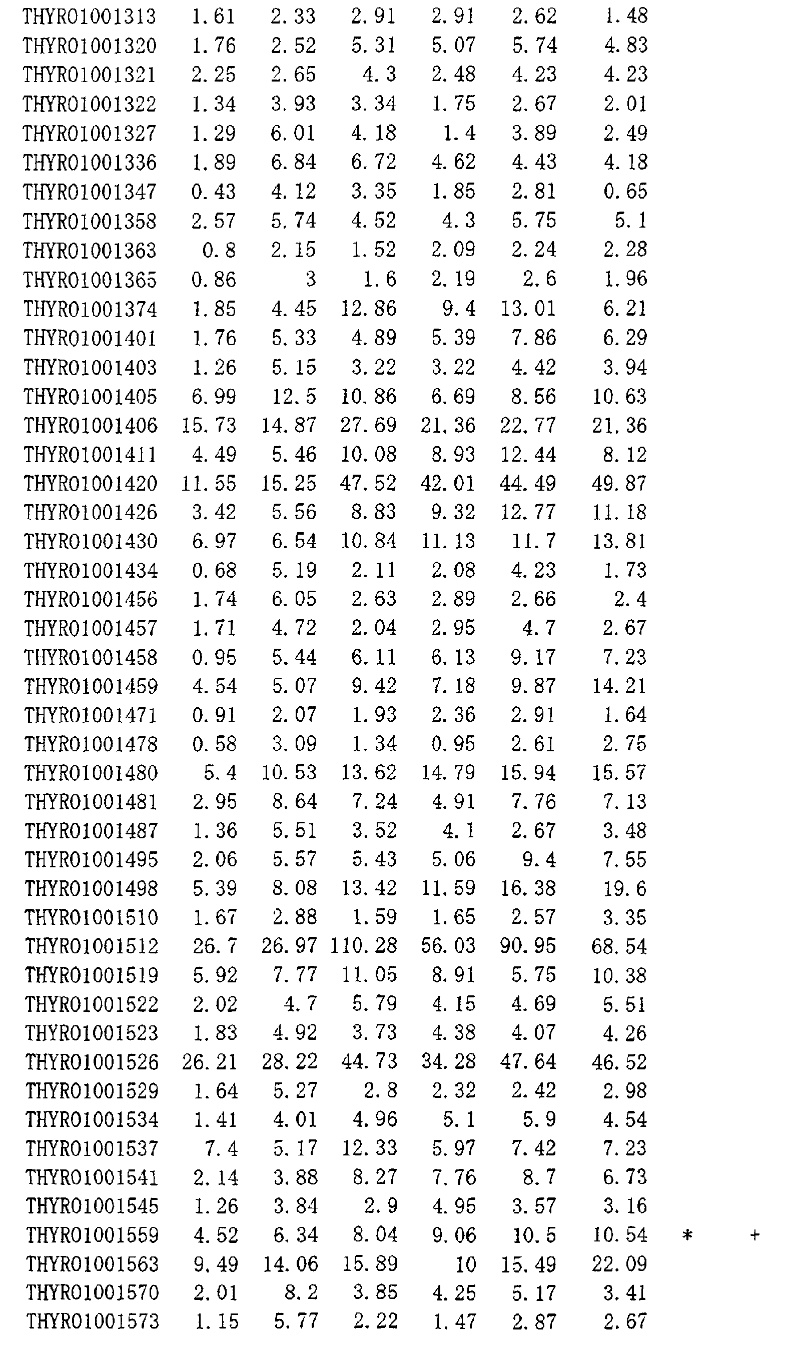

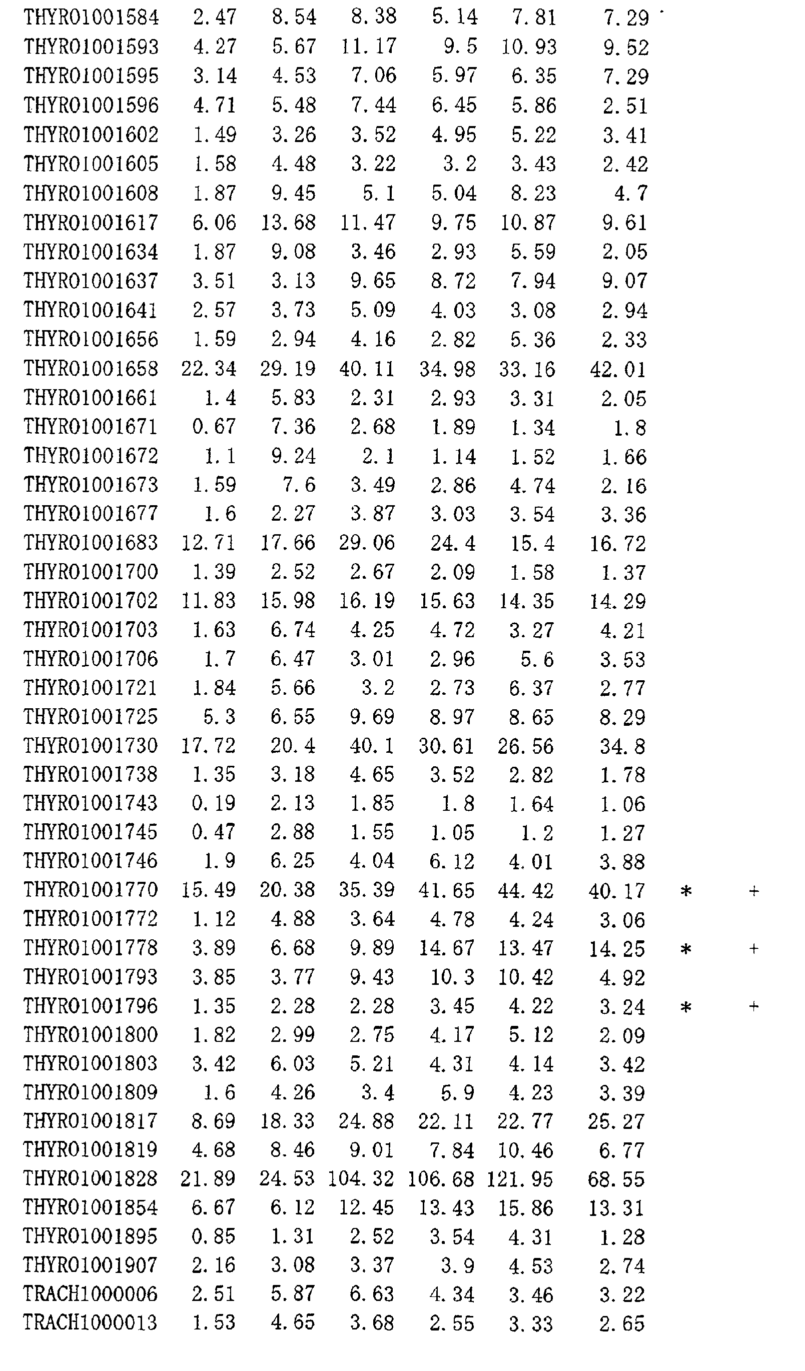

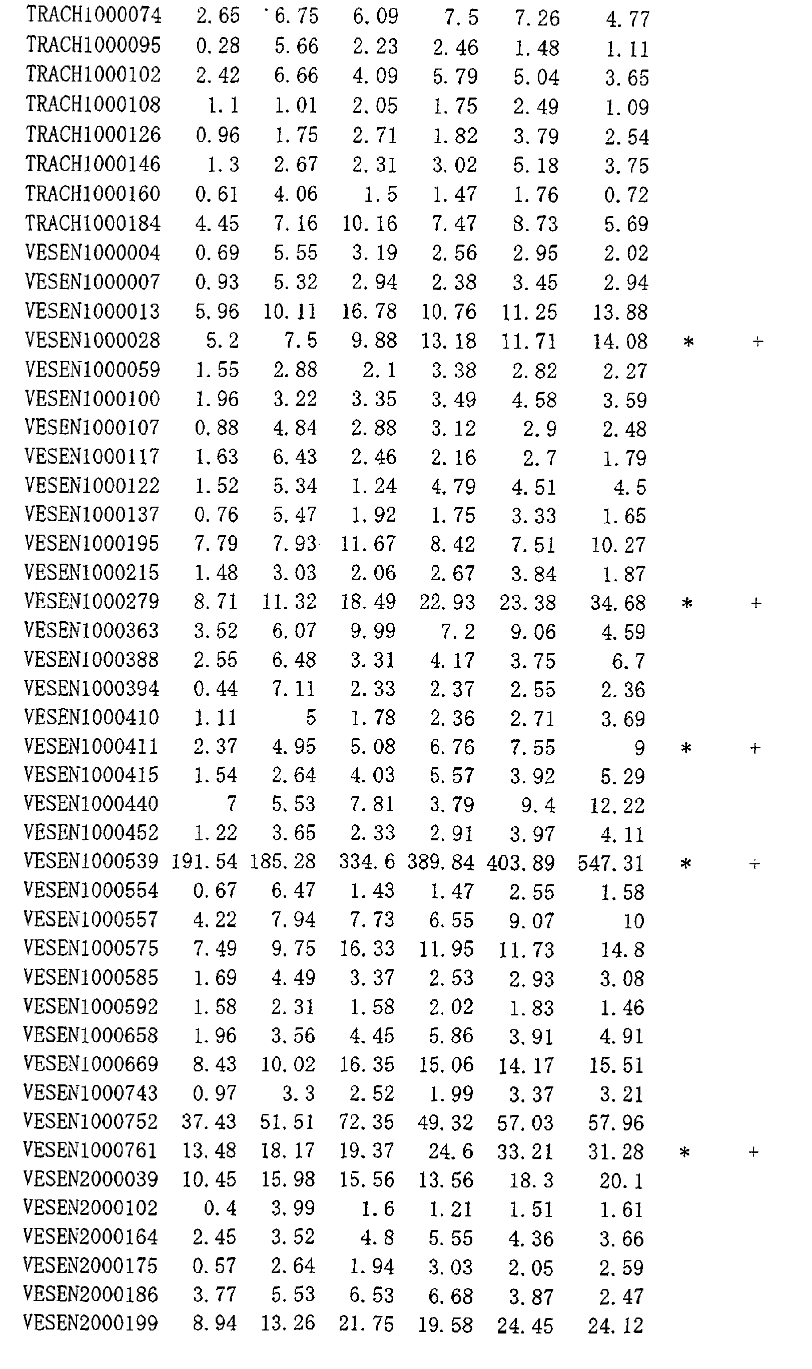

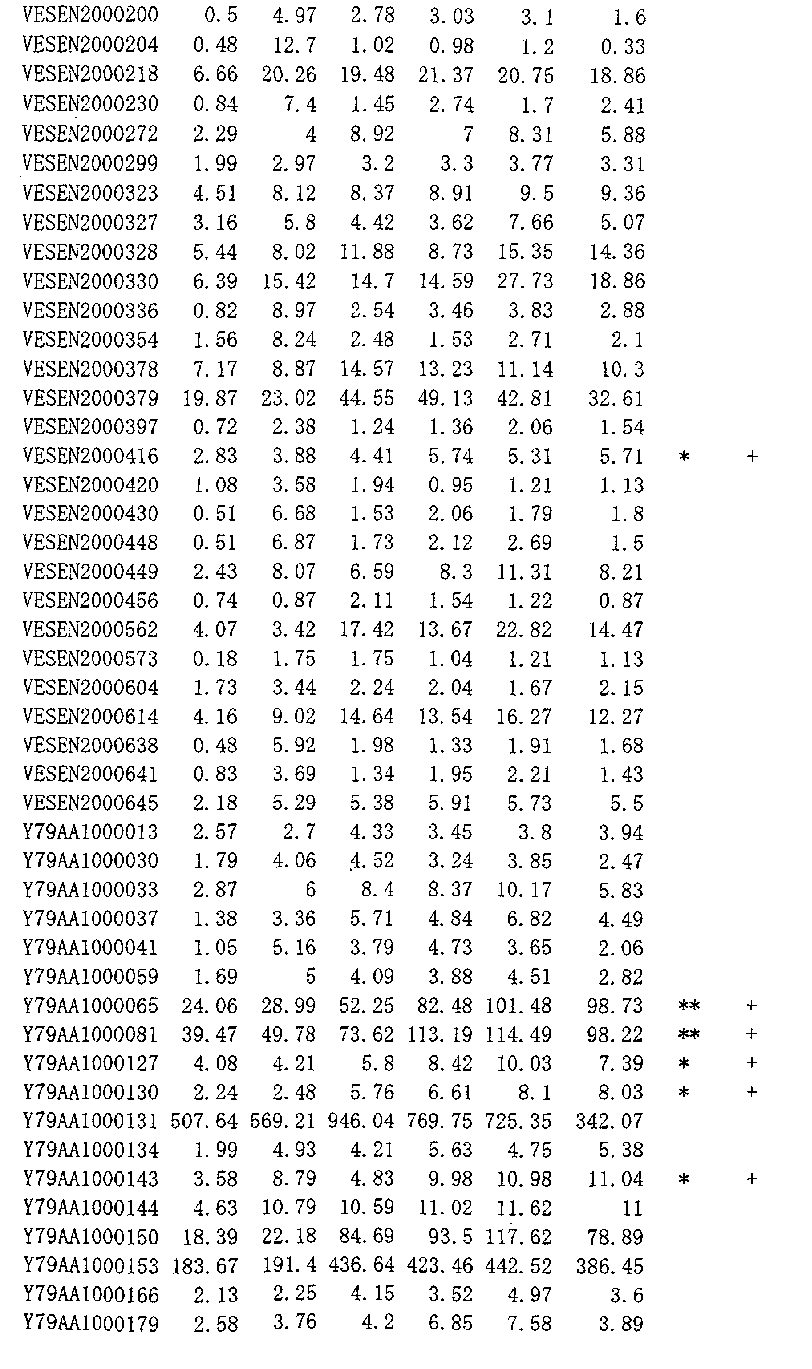

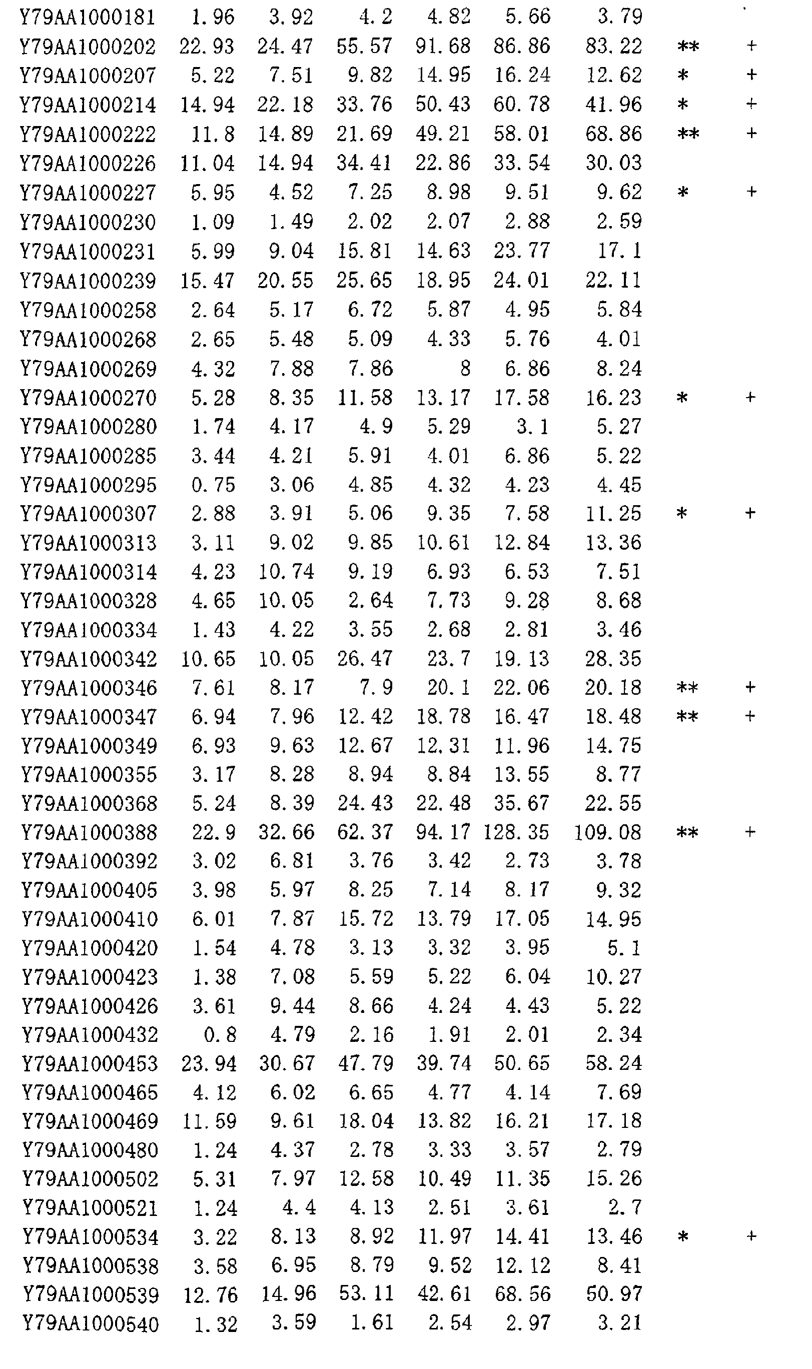

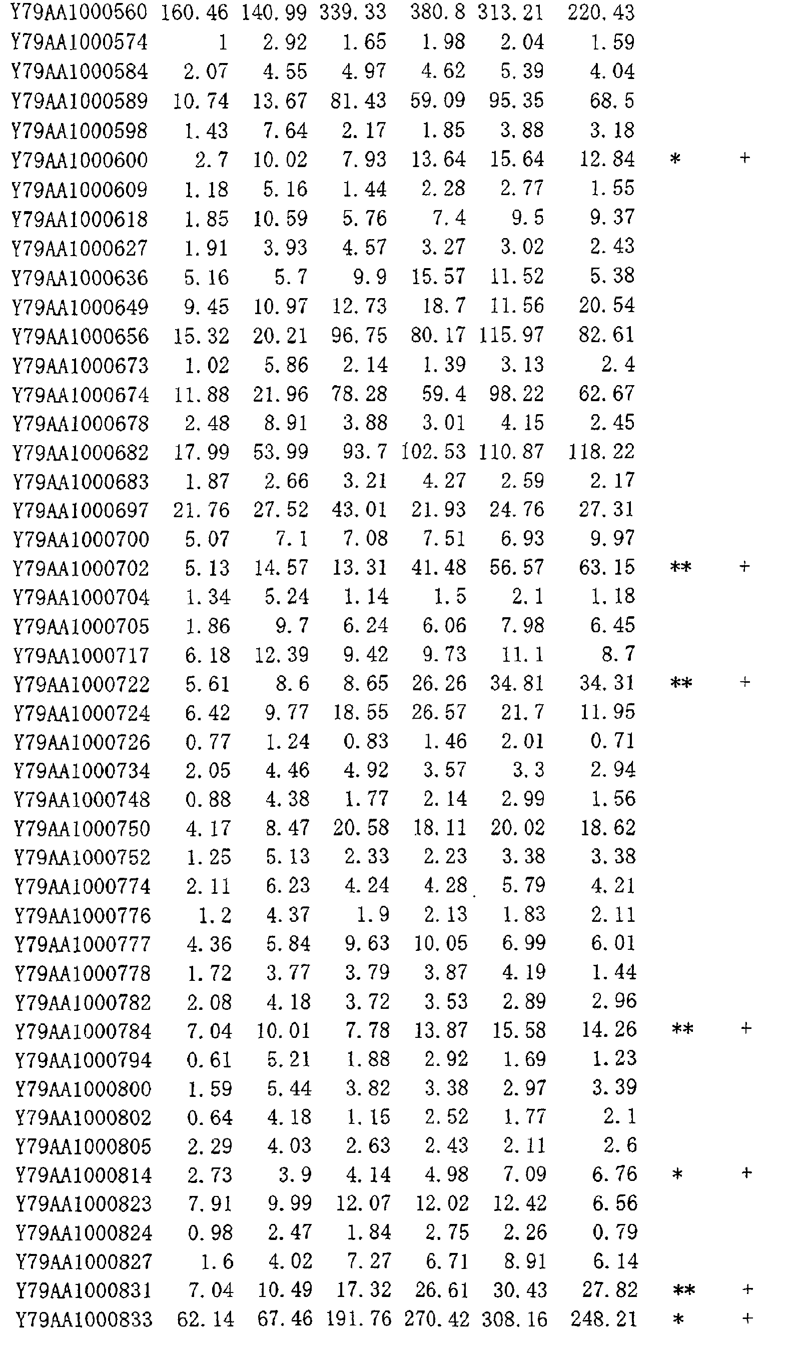

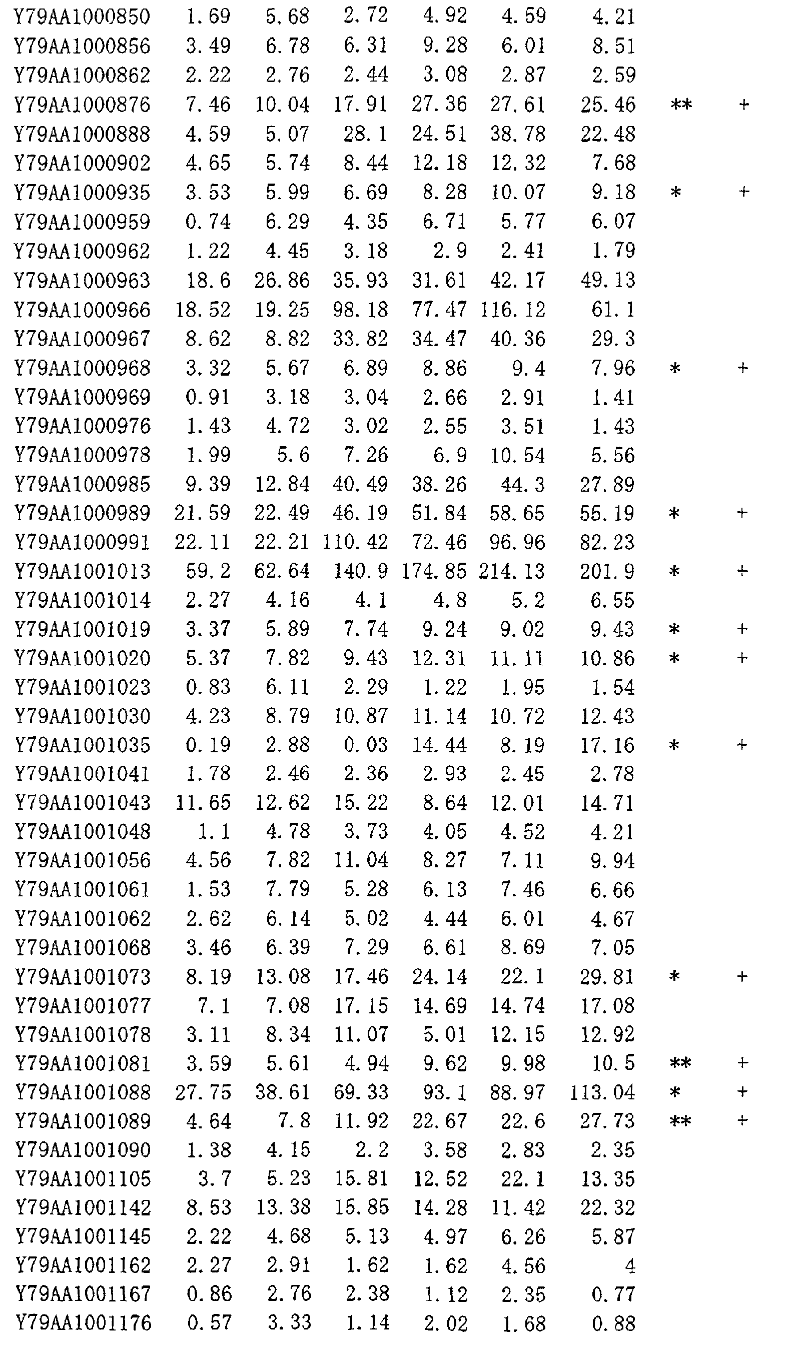

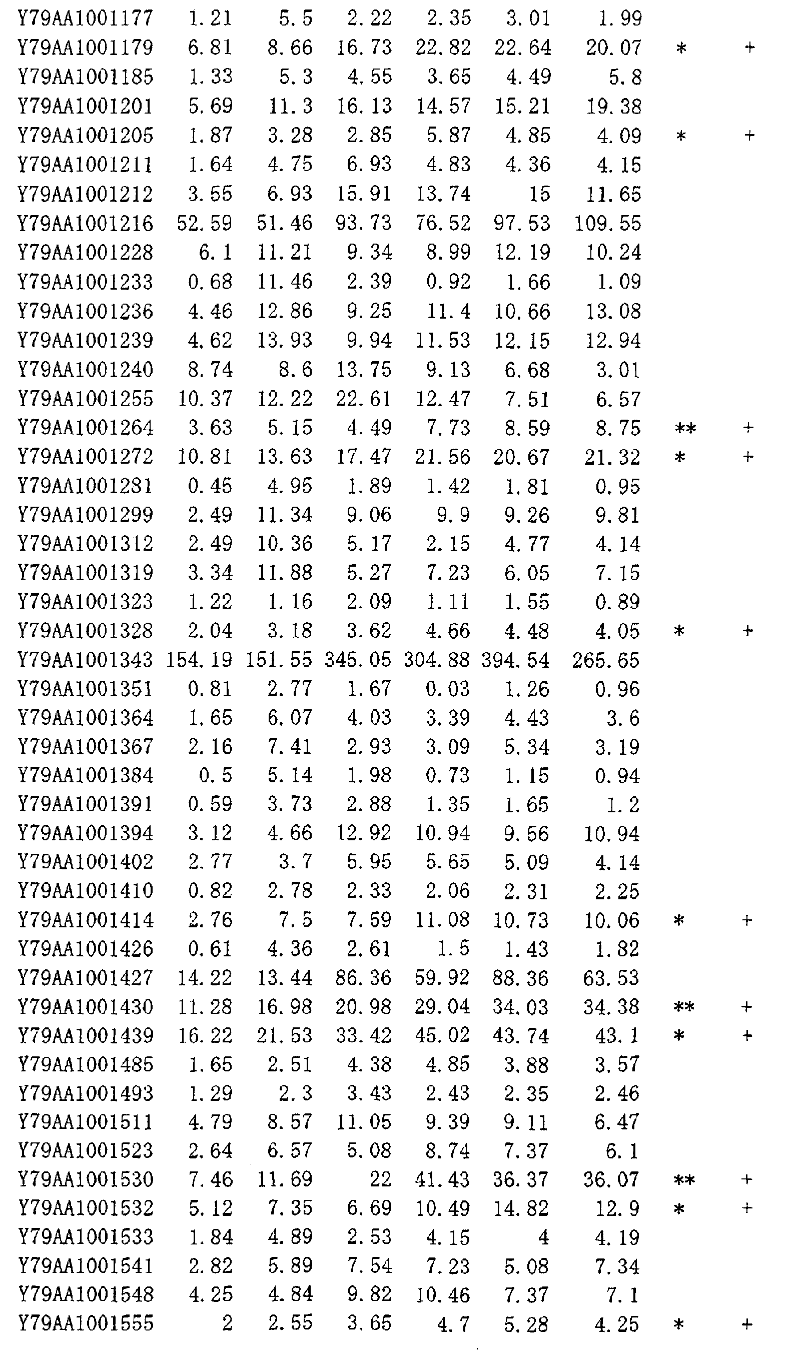

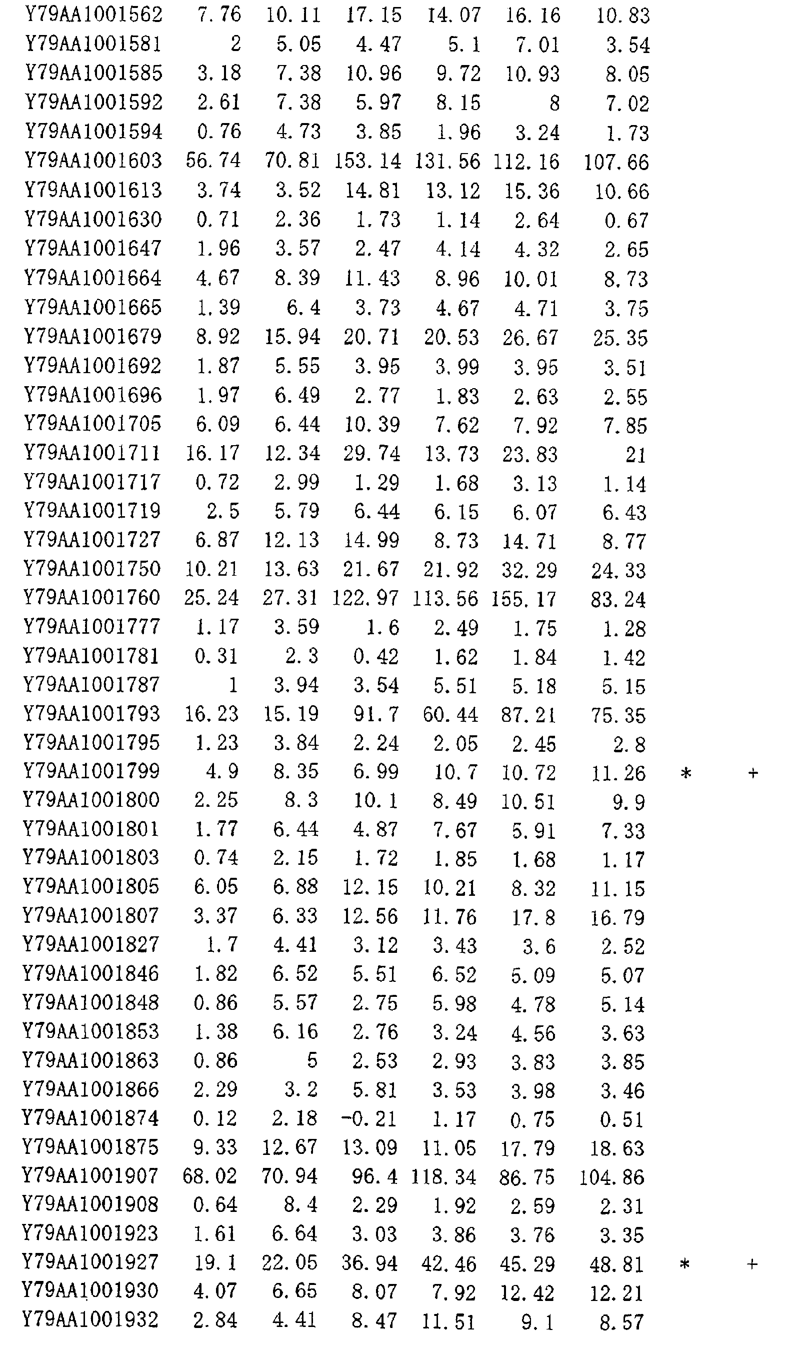

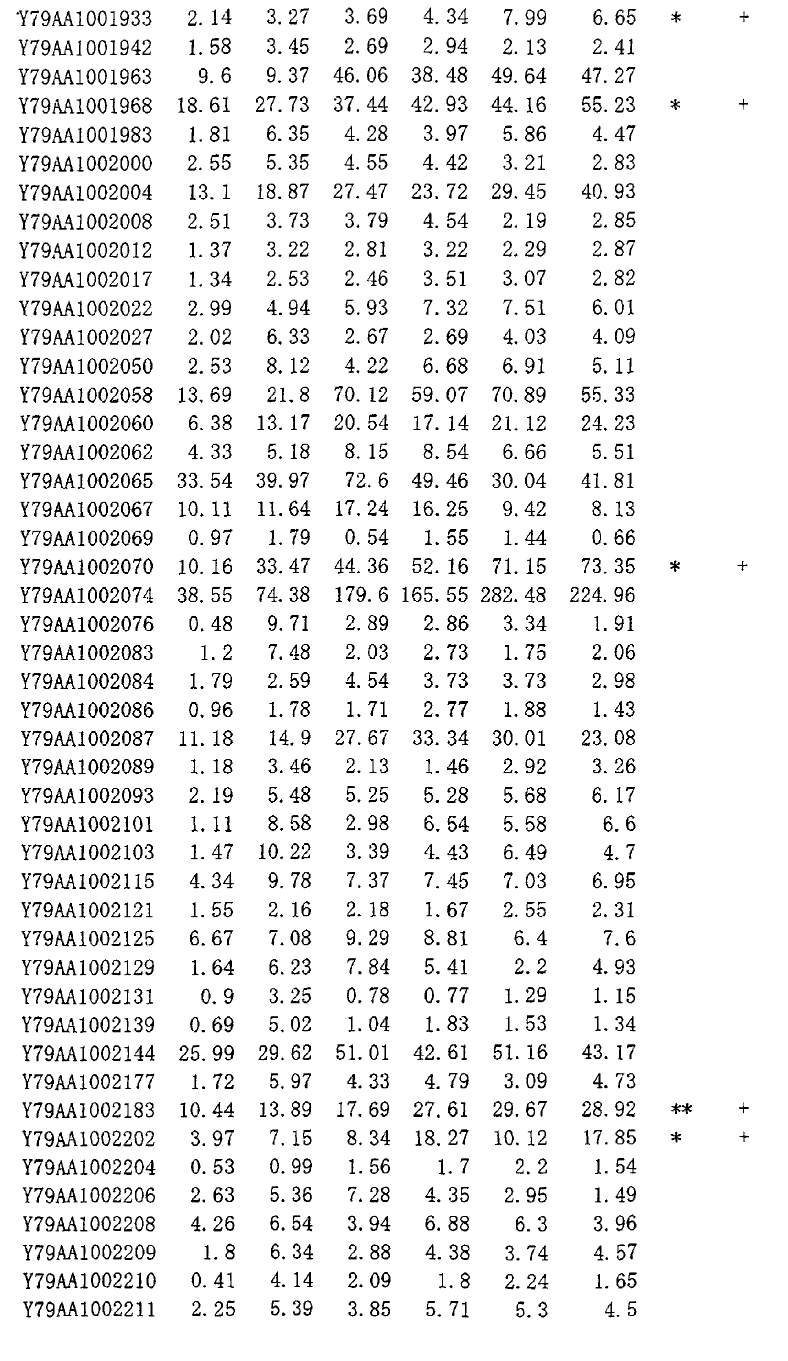

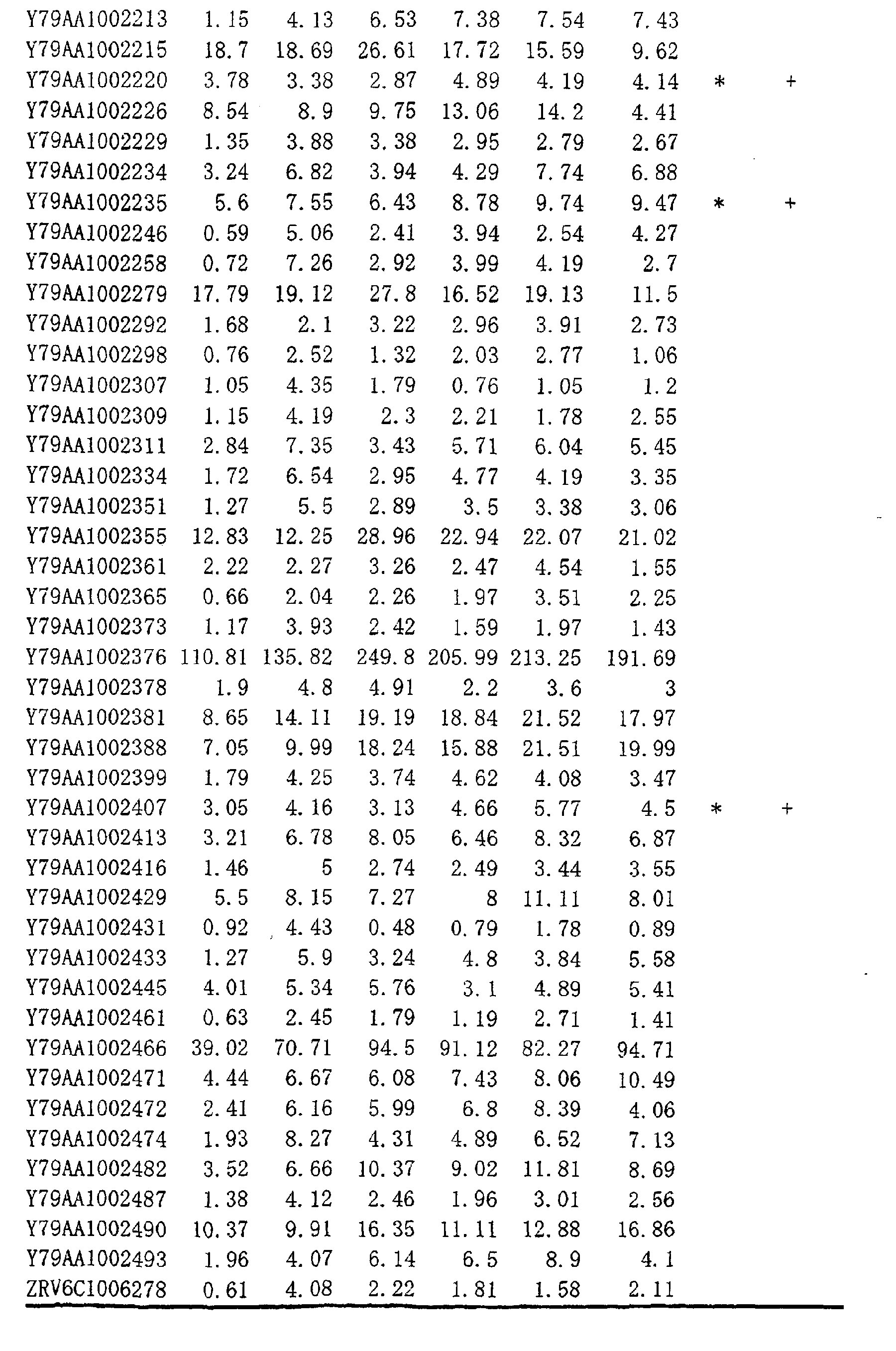

## Claims

1. An isolated polynucleotide selected from the group consisting of
(a) a polynucleotide comprising a coding region of the nucleotide sequence set forth in any one of the following SEQ ID NOs: SEQ ID NO: 1, 3, · · · 347, and, 349;
(b) a polynucleotide comprising a nucleotide sequence encoding a protein comprising the amino acid sequence set forth in any one of the following SEQ ID NOs: SEQ ID NO: 2, 4, · · · 348, and, 350;
(c) a polynucleotide comprising a nucleotide sequence encoding a protein comprising an amino acid sequence selected from the amino acid sequences of (b), in which one or more amino acids are substituted, deleted, inserted, and/or added, wherein said protein is functionally equivalent to the protein comprising said amino acid sequence selected from the amino acid sequences of (b);
(d) a polynucleotide that hybridizes with a polynucleotide comprising a nucleotide sequence selected from the nucleotide sequences of (a), and that comprises a nucleotide sequence encoding a protein functionally equivalent to the protein encoded by the nucleotide sequence selected from the nucleotide sequences of (a);
(e) a polynucleotide comprising a nucleotide sequence encoding a partial amino acid sequence of a protein encoded by the polynucleotide of (a) to - (d);
(f) a polynucleotide comprising a nucleotide sequence with at least 70% identity to the nucleotide sequence of (a).

2. A substantially pure protein encoded by the polynucleotide of claim 1.

3. Use of an oligonucleotide as a primer for synthesizing the polynucleotide comprising the nucleotide sequence set forth in any one of SEQ ID NOs: 370-540 or the complementary strand thereof, wherein said oligonucleotide is complementary to said polynucleotide or the complementary strand thereof and comprises at least 15 nucleotides.

4. A primer set for synthesizing polynucleotides, the primer set comprising an oligo-dT primer and an oligonucleotide complementary to the complementary strand of the polynucleotide comprising the nucleotide sequence set forth in any one of SEQ ID NOs: 370-540, wherein said oligonucleotide comprises at least 15 nucleotides.

5. A primer set for synthesizing polynucleotides, the primer set comprising a combination of an oligonucleotide comprising a nucleotide sequence complementary to the complementary strand of the polynucleotide comprising a 5'-end nucleotide sequence and an oligonucleotide comprising a nucleotide sequence complementary to the polynucleotide comprising a 3'-end nucleotide sequence, wherein said oligonucleotides comprise at least 15 nucleotides and wherein said combination of 5'-end nucleotide sequence/3'-end nucleotide sequence is selected from the group consisting of: SEQ ID NO: 391/SEQ ID NO: 541, · · · and SEQ ID NO: 540/SEQ ID NO: 679

6. A polynucleotide which can be synthesized with the primer set of claim 4 or 5.

7. A polynucleotide comprising a coding region in the polynucleotide of claim 6.

8. A substantially pure protein encoded by polynucleotide of claim 7.

9. A partial peptide of the protein of claim 8.

10. An antibody against the protein or peptide of any one of claims 2, 8, and 9.

11. A vector comprising the polynucleotide of claim 1 or 7.

12. A transformant carrying the polynucleotide of claim 1 or 7, or the vector of claim 11.

13. A transformant expressively carrying the polynucleotide of claim 1 or 7, or the vector of claim 11.

14. A method for producing the protein or peptide of any one of claims 2, 8, and 9, comprising culturing the transformant of claim 13 and recovering the expression product.

15. An oligonucleotide comprising the nucleotide sequence of claim 1 (a) or the nucleotide sequence complementary to the complementary strand thereof, wherein said oligonucleotide comprises 15 nucleotides or more.

16. Use of the oligonucleotide of claim 15 as a primer for synthesizing a polynucleotide.

17. Use of the oligonucleotide of claim 15 as a probe for detecting a gene.

18. An antisense polynucleotide against the polynucleotide of claim 1, or the portion thereof.

19. A method for synthesizing a polynucleotide, the method comprising:
a) synthesizing a complementary strand using a cDNA library as a template, and using the primer set of claim 4 or 5, or the primer of claim 16; and
b) recovering the synthesized product.

20. The method of claim 19, wherein the cDNA library is obtainable by oligo-capping method.

21. The method of claim 19, wherein the complementary strand is obtainable by PCR.

22. A method for detecting the polynucleotide of claim 1, the method comprising:
a) incubating a target polynucleotide with the oligonucleotide of claim 15 under the conditions where hybridization occurs, and
b) detecting the hybridization of the target polynucleotide with the oligonucleotide of claim 15.

23. A database of polynucleotides and/or proteins, the database comprising information on at least one sequence selected from the nucleotide sequences of claim 1 (a) and/or the amino acid sequences of claim 1(b), or a medium on which the database is stored.
